(19) Europäisches Patentamt / European Patent Office / Office européen des brevets

(11) **EP 2 479 283 B1**

(12) **EUROPEAN PATENT SPECIFICATION**

(45) Date of publication and mention of the grant of the patent:
**22.06.2016 Bulletin 2016/25**

(51) Int Cl.:
***C12Q 1/68*** *(2006.01)*

(21) Application number: **11009931.4**

(22) Date of filing: **17.04.2007**

(54) **Methods and nucleic acids for the detection of colorectal cell proliferative disorders**

Verfahren und Nukleinsäuren zur Detektion kolorektaler proliferativer Zellerkrankungen

Procédés et acides nucléiques pour la détection de troubles prolifératifs cellulaires colorectaux

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **17.04.2006 PCT/US2006/014131**
**27.06.2006 EP 06090119**

(43) Date of publication of application:
**25.07.2012 Bulletin 2012/30**

(62) Document number(s) of the earlier application(s) in accordance with Art. 76 EPC:
**07724317.8 / 2 013 360**

(73) Proprietor: **Epigenomics AG**
**10829 Berlin (DE)**

(72) Inventors:
- **Lofton-Day, Catherine**
  **Seattle**
  **WA 98103 (US)**
- **Ebert, Matthias**
  **81735 München (DE)**

(74) Representative: **Zwicker, Jörk et al**
**ZSP Patentanwälte PartG mbB**
**Radlkoferstrasse 2**
**81373 München (DE)**

(56) References cited:
**WO-A-03/042661 WO-A-2005/000087**
**WO-A2-2005/001140 WO-A2-2005/001141**
**US-B1- 6 783 933**

- **EBERT MATTHIAS ET AL: "Genome scanning analysis identifies ALX4 gene methylation as a potential marker for colorectal and other gastrointestinal adcnocarcinomas", GASTROENTEROLOGY, vol. 130, no. 4, Suppl. 2, April 2006 (2006-04), page A54, XP008152874, & DIGESTIVE DISEASE WEEK MEETING/107TH ANNUAL MEETING OF THE AMERICAN-GASTROENTEROLOGICAL-ASSOCIATION; LOS ANGELES, CA, USA; MAY 19 -24, 2006 ISSN: 0016-5085**
- **ZOU HONGZHI ET AL: "Selection of methylated genes as candidate markers for colorectal cancer screening", PROCEEDINGS OF THE ANNUAL MEETING OF THE AMERICAN ASSOCIATION FOR CANCER RESEARCH, AMERICAN ASSOCIATION FOR CANCER RESEARCH, US, vol. 47, 1 April 2006 (2006-04-01), page 13, XP008152874, ISSN: 0197-016x**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 479 283 B1

SEQUENCE LISTING

<110> Epigenomics AG

<120> METHODS AND NUCLEIC ACIDS FOR THE DETECTION OF PRE-CANCEROUS COLORECTAL LESIONS.

<130> 536-27EPT2

<160> 20

<210> 1
<211> 52626
<212> DNA
<213> Homo Sapiens

<400> 1

```
ggaggaagga gaaggagttt attgcaaata acaacaacca aacagtttgg gctgggccag      60
caaagggtgt tctgggagtg cccggctacc tctgcactcc attttgcccc atgcctgggc     120
ccctacatct ccttcccatc cgtgttcctc ctctcccaaa aaagatgtaa gtgcaataac     180
ttactttaaa aggcaagaac gtttctttta atattttgct ataatgtagt tacatggtgg     240
tataggcagt aaaactttat ggaaccgacc tcattttttt ttttacattt ttctggattt     300
ttactgtatt tttaatatat atttttaata ttccacccca ggccattaag ctaaaggaaa     360
agttgcattt atacagggtt aaaatatctt acaatgagaa caataagtaa cggttgaggt     420
tcatgttcct tctagcactc agctttcttt tttcaaccac tgcacaccta aacagatgat     480
tagacattga aaactgtcct tcaaaatcag tagtataaag gcctagctct atgtgtgtga     540
gtgaagaggg aagaaaggag aggccgaggt taggtcatgg aagcaccttt cctccctaca     600
gcatcctgaa agaagtgaag tggggttgat gggtcattgt caccttctta tttttaact      660
ggatagtcac agagtattat cctgggggcc agtttaccaa ccatcctccc ccagctaggc     720
ctgtttgcct ttgcactgga cagtaagggc catgaaacag accaggaacc cccgcgtgtc     780
tttcaggttc tcgaagatgg ccccccactg tctagcccag ggagggaatg actgcataag     840
cagggtacgt agttttcatg gaagcgtccc tcgtctgccc agccgaaatg acaggagatg     900
gggcacggag ctgggtccag tgtgatcaca gctgtgtgtg ctggggaccc accccacact     960
tgctgcacct accccatcgc agtgtctttg agctaacctg gccaccgtgc ttcccgaagc    1020
tggaggcacg gtagtgagcg ctgagaatca agtatagtta aaagaattca aggaaagttc    1080
taaaagccca tgtcctccaa gtgctatttc agccttggag acaagcctct ctgtgtgccc    1140
cccagagccc tcgctcccat gccccaggag tagtgctggc atctggaggg cagtagcagc    1200
accttagtgt caagccttgc catgctgaga cctgtgccca gcagggactg gaggctgtca    1260
gtgtggggag gctgcccatc catctgccgg ctacccacga ctttattttt aagttgccat    1320
ctctgttgag atcttaggca ggtatccaaa ggaaatcctc acctctctta gcttattcgc    1380
atagcaatga accacgtggc aggtagatgc ttctctccac ctgccttcat gagctttagc    1440
tccagatggc cttgggcagc tgccctgact tgctcagtca tcctaggagc tgacacggca    1500
gtggcccccct gctctgcagt gcagaaggtt gggcctagcc acctgtcttg gaagggactg    1560
ggccccaagg ttctcttctg gagaactgag ctgctgagtg aatgcccatg aggcattgga    1620
gaggggagct ccatgcagca ccggccgccc ctggtgccaa cgaagcccag gccctcacac    1680
cctgggtggc acgtcccagg ctcctctctc agcctagtct cagggcaagt acaggtcagg    1740
gcagtcatgt ctccactctg ggccagggtc ttctctgttg tgaagggact ggactaacta    1800
gagggtttct aagcttcctc ccaactcggt ggtcctatca tacttagtgt ggttaggtgg    1860
cccccaaggg ctgggccaag ccacagagga caggcctggg gtacgtggct gcctttgacg    1920
agatgaacag gaatgtttca cattggcatg tttaatccta gacacttcct gggtgtgttg    1980
ggacaggcag ccatagccac agagtcttct aatagacgcc ttttcttctc aagcttttgg    2040
gttgaaattt gcctgacttg ggtctagccc agccagagag cagacgcatg gaggaactga    2100
gtgccggggg ccactgcgga agagccccct taaagctctc actctccctc cctgcctgct    2160
ctggagctac ctgtctgtcc tcctctttgt cctgattccc ttgctctccc tcctggccca    2220
ctctggagct acctggctct cctcctcttt gtcctgattc tctcatgggt gagaaagacc    2280
caaggtgaaa gttccttcct tccccatcct tcctcagcca tgagcatttc agaaggaaag    2340
gggggagaaa tggggaaact gttcccagcc ttaagttctt cctttccctg gctagatttt    2400
```

```
gggggagtct gggtcatttc agcgaacctc agaactagaa aaaacttccc aaaagtcact   2460
ccaaatcctc aaatatttga aacacttaaa gccaagcctc caagaacatt taattcaatg   2520
tccaagaccc tttcacctct ataagatcca ttttagagct acggttcaca aagaggtctg   2580
actcattaag ggaggccaca gccagaccct tcctcagcct ggtttctaga gcagatgaga   2640
acaccaggca ggcgggagac tgtcaccccc aaccccagag accctcattc aacaacacca   2700
agtttaccct ccgcccaacc caggaggcct ttctgtgtcc acgaccctga gcatagtggg   2760
cctggcccta ctccatgaca ggaatcaagg gagaaggtag aaaaggcgag cgcttcaatg   2820
cggggcgctg cctgctggag ggaccccca cctggggtct gacaggcctg cctctggacg   2880
gttactgtgt actcccagct gaggtgaggg ctggggctca gggccagacc caagctacag   2940
gggtcgggca ggcaggcaca gagccccgct tcctggaggc aggaagcctg gtttcaggag   3000
ggcatcctga cagccccatt tgcaatagca aagccacacc gaggagccca ggccaggagc   3060
ccttccgcag gctggagaga gggcagagag tcctggtccc tgtggaagaa gtgtaaggac   3120
ggagtgtgga aagctgtgtg gagaagagca acacctccta gtgcctggag ggacacagag   3180
cccccatctg ccgtggaagg ctctgggcag cgatgacact gacagatatg gcagggagga   3240
cagtcagttt gaggccatag cagggcctgg aggccgagca tggccacacg tcctctagca   3300
aagcctgcac gcagcttttg accctctgcc acccggcccc caagctctag cttatgtgtt   3360
ccgaagttgt gagtccttcc atgccgggac actgctgagc agccccaggg aggggccagg   3420
gtagggcagg gaagagacga gcccctccgc atcctcctgg agggaacctg actgggctct   3480
ggatggagaa caaagctgcc cagacctccc cgctgtacca gatgccaggc aggccggaga   3540
gcgggaggac tgcccgggcc cacgccccac ccggatgcct ccccctctcc caggaccttt   3600
ccttcttcta gcaccatggg agcccttgtg gcatctgtgt tctcagcctc cctcaggtct   3660
ggctcctccc ctatagtgtc cagtgagggg ctggcccgtg tctcccctgg ggactggcat   3720
tgctagatgg cagcaggggt cctcggctcc agctcctgtc atcctctgcc cactctctcc   3780
aggtgtatcc agcctagggg acagccagga tgcagtcagc caggctctgt cagactcgtc   3840
actgttcggg gggaggtgga ggctggcgcc ttggccccag caggtcggat tccgtgtgct   3900
ttcagggaga gaaagatggg agggcaggag gctggctctg cagggcagcc tgtgcttcag   3960
acacacagcc accggcctgg tgaccagggg accccactag gagcgggaag gatggacaag   4020
actgggctgg tgaggctgct gggtctgcat ggaaatctag cattgactca tggtcaacta   4080
ggcagagcag aggagtgggc gggagcaaga aagcgctttc agcttatcat ggatgcgaag   4140
ctgaaaaacg tggccacctg gctttctcca ctgcctgtgg ccgggagcag gggtcagggc   4200
ctcagtgcca gggaggggcc aggggcctgc tgccccctcc ctcccagcag tccacggggc   4260
ctcagacttg gggcggctga aagtgctgag ggtcaggccc ctggcccagg ccaggttcct   4320
aagaggaaag tcgagtggga ggctggggc ctggaaaaca tgggcgtggc ccatggtgtc   4380
ccgaggtggg gacggggcag gggtgccctg tcatgtggcc caggaaatgg ccgcactgtg   4440
ctccttggcc ttcatgcgga gggccgcgat gctcgaggtc ttgcggtccg gctcgccgtt   4500
gagctcgtag ccattgaggc ctgggctgag gctggctgct ccaaacaggc tgcccatgtg   4560
cgtctggccc acgtgactgc cagccccaga cacactcagg aagtcggtga cgctgctggc   4620
cccagagcca gggggtggg catgagggga catgcaggca ggcaccgggt cgcaggggac   4680
cacgcaggct ggcactggtg aggcagcccc gttgttgccg agccaggacg ggttctgaat   4740
ctgggagagg aagggagaga tgtcaccggc tggcgggcag gtgtggtgtg gaaggggctc   4800
gccccttccc ccagagcacc tctcccctca ctgtccatcc ttcccatgaa gcccctcttg   4860
agtgtttagc cttgatggac atccagatac ccctgtgggg gtggctttta caccttggca   4920
tctctgttcc cagggccagg ctagcagcag agggaaattc agttctaaag agtgacctca   4980
ggcttcatta agggtccagt ggaagggtga caccccagca ccccccagct ggcttcctat   5040
ttttagactc aactgtagac ttggcctcca tctgcggatg gcagctgtgt ctcattcacc   5100
tccagctcct ccccaggccc ctcacatcca taggcacctg agcaaggtga gcagacctca   5160
actcggagag ctttggcctc gcaggttccc ttgggccccc accagtagca gggaagatgg   5220
aggggttggg tgaggtgggc ggcagggacc taaacctctc agaaaccaag ggtcagtggc   5280
tgaggttcca gggcctgaac taggaggctt cagaactaac tcaggatgga aggggctgcc   5340
tcaggagtgg acagccagat ctgagaccgg acctcctcgt gccaaagggg cgagggcatg   5400
tgcagggatg agttggccat cttatcttcc atctgtgcca ctgggcttcc cttccctccc   5460
caacgttcac ctggggctcc catcttcctc taccttctag gcacgtataa ggaggtagaa   5520
ttagggtgtg ggggagaccc gggaacctct cagcttatgc acacgggatt cctggtaacc   5580
tcatgtgggc ttctgctggc ctgccctcct gcatgtgtct ggagcacact cgagggaggc   5640
attttaaagc agggagaaag agctcagccc ccatcctgga gggaaccgca tttctctctg   5700
cggtgcaggg gaagggggac cggttggcct ccatcacccc ctctcagaag aggtctgagc   5760
acacactatg tggtgctgta ggtgaggtgg gggtaggagg ggtgaacagg gccctgtgtt   5820
gggttctccc tccagctgtg cgctcaggca gcagagctca aagccaagga gggagctaga   5880
```

```
gttccatcct gggaggacag agggaggctg agtttaggga gggagagggg agaaagagaa   5940
tggaagaaca tagtgcgagc tgatgctgat ggtgcctgtg tgagacaccc catattcctc   6000
agcctcacct ccaggcctgt gccctgccct cctgccccat gaatcctggg ctgggggaga   6060
gggaacagag ggctggtctg gttggagagc cagagaccat ttgctagcac agaaccaggt   6120
cagggagaag taaaggaggc aggggcgccc gtgctactgg ctggctgctg agcagggcca   6180
ggttgggagc tgaggtggag gccatgccag aatgaggcct gagcaggtag ggagcagccg   6240
cttctgctgg gcaggctgag tgggggtcag gagaggactt ggacttggcc aaaaacacat   6300
ctctctaagc atcccatgtg cctgatgtga ctcaatactt ctcatccctt gcatctcaca   6360
ctagaacagg ggactggctg acagacacct agggaaaatg ctctgggggg aggggaggac   6420
agggccccag aagctgagca caggcacaca agctttttga aactgtacaa ttcacaaaat   6480
acttttgcat gattgaattt agaccttaca actgcctgac aacagagctg agcagatgct   6540
tttaccatgt ccaaggtaca ggtgaagaaa ctgaggctca gagatttgag acttgcctaa   6600
agtgagaaag acctggagct ggaaagataa cctaagtccc cggactcaga atcccaagct   6660
cattccaact tggccacgag tcctggagat ttcagagctc gctttctgaa gagaccctcc   6720
gagcggtatc tgagggcctg gtggccttct gcctgccttg gttccgatgc ccacagcaaa   6780
agtggccaaa gctagtggag gaaagagccg ttctcactgc cccaaccttc ccttccctaa   6840
agttcagcca agcctggttc aatgattagt tggatagatt aaccctgggc ctcccacact   6900
ctggtataaa caggcacacc cctggaatga gacggaaggg cagcctggag ccataagtca   6960
tctcggggtg cctgggctct cctccaaggg gctcattctc agagcaccag gggctgggga   7020
tcggtggcgg cagctcaggg cgggacttac ctgggcgtag ttctcagctc gggtgaggag   7080
gggcagctca tatgcagtgg agaagtgggt tcgaacctgc tgcatctgcc caaaacgctc   7140
ccgcttcctc cacttggccc ttcggttctg gaaccagacc tacaagacgc gaaaaagcca   7200
ttgtcaccag ggtgagatgc ccgcctggag gcctcacttt caggcagtgc aaactgttcc   7260
cttgagcccc ccatcagttg cagtgcgttt gggaaacggt gcggccacac ataaatatca   7320
accttggtct ttgtcctcaa gagctgacaa tctagaatca tccactctga gctgggaagg   7380
tatcttaccc ccactagcca tgggcgtgat cctgagccag aacttttatt tttctaagct   7440
tgagaatcta gagcagccaa gtgtcaccac ctataaaaca agtcactggt catcttagca   7500
gtcatctaag cagactcctg tggagaagct gataaaagct acaaatgctc ctccagatgt   7560
gttctcctag acaatcacac acagttttgc cacagttttc caaaaggttc atggctctct   7620
aaggcttatt ggtccagatt cacaatgcct tcctccctgc atcctgtctg cagccttctt   7680
gttggagaca gtctcccagc tcagaactcc cctgtgttaa agggagaaca ggggctctgc   7740
tgggcaacag aggcagtcag ggaagacttc ctggaggagg gacaggagct gggccttgaa   7800
tggttggctg aaaggaggaa agccaaggcc taggtttaga aacagcaagg catactccaa   7860
aaacctaaag aggtcaattt gaaggaagcg gtgggagaga aagaaacaga gtcagatcgc   7920
agggggcctt gactgccaag tgaagggttt ttcccttggt gctgtctgta gaggcagttg   7980
ctataaaatc ttgtgtagaa gcgtggcctg atggtgagag gaagtggagg ccaacccgtc   8040
actgagcaca agtggatggg atgttggcct gattgggagc ccaacggggg aggggaggtc   8100
tctgagtcag ctgtgagcac tggggttttt ggtgacgggg agtattgggt cctttccctt   8160
tgcacgagca tttctggaag gctgagaact ttcccaaggc tcaagagcaa gagctgccct   8220
acccctggcc catgacttag gatctggaga aggggtgatg gggacggggt gggtgagaga   8280
gggagagtga ggagggatca gcttccaggc caggaatggc tttaggctga ttcagagatg   8340
gaccgtggtg ctcctcagag ggaggcctct gccagcttga tgtgcacttg tctgctggct   8400
ttgcagactc agatgcaagt gggcacacct cagacagcac gctgcagacc gggagaacag   8460
agccatgaca atagcctcag ctgcagagcc ataaggagcc ttgccaggcc cttccaggcc   8520
caggcatcaa gaaagccaac tttcctgagg tccagtccct gctctccagg tggaaacatt   8580
tctccatcca tcacgaccaa tgcagctgct gagctaaata taggagtcag ctctcaaaat   8640
tcctctgcct cagagccttt gagcttccta ggaaccactg cgaggagggg agaaggaggt   8700
gtggacgtgc ttcctcacag acgctgcctg gcctctttgg gcatccagtg tagctggcca   8760
atggacaggc tcataggagc actggtattg ccaagggcct cagtttctgc atggcgaatg   8820
gtacctcata gtcgcagcag gggagataga tgggaaatgc agtccaagaa tgtttagagt   8880
acttcccatt tgtttcttta cagctcctga cctttctcac tgtcttttgc tagaggaaag   8940
aggtcaagga agatggggat gtatctcatg ttactgtgag gagggtgggg gtgagcaggg   9000
gtgtacatta ttatgtttct ccactcctgt accaactcac tttcccctcc ctaatcctaa   9060
gcatgtgtgt gcgtgcacga gtgtacccac tagtgtggct acacgcacgt gcatgtgtgc   9120
acacacattg actgggggag gagaagcctc ttccttagaa acggaccctc tccccagttt   9180
ggggtctcag cccctgccct ggcctcccag ggcccctttt cctccaggga ttcctagggt   9240
ccaggccctg gctgcccact gggagccaag gacagatggg gtctcctctc tgtgctgctg   9300
aaatgggatc cctctttatc agtggcctgg gatgggagac tggggccagg atgagagaat   9360
```

```
gaatcagagg agcttgtttt catttcatgt ttaatttcct gcaggcttag ctccgtcttt     9420
ccgctccttc ctcagcctcc cagggttttc tgtgggggaa gctgggggga ggggagaagc     9480
cactggaggc acggagagag atggcttctc tttttctttt tgtcactgtg actgttgggg     9540
ttctccttgt gtctctgtgg gttccaggtc cccatggtgg gactgtggaa gtggaagatg     9600
tccctacagg ctctggaaca gaggatccag ggatgggcgg ggagggaccc cgaagaggga     9660
aggaggtggc cagcgcctgg caaaggggtc tagagtggag ggaggagcca tgtgggggag     9720
tggaatgtgg ggcgcgggct cgctggagct gagtgtgagg gaggaggaga tgtgggttat     9780
ctggggaaga cagatgacca ggcgggggag aggccagcag agctagcacc gtctttggaa     9840
caggagtgga agaaggtcag gctgagggat ttatttgagg gctgaagatg gaaatggtga     9900
gaggcgggtg agcgaccggt gggagcaggg ctgcccacct cctccctcct tcttctcctg     9960
ttcctttgtc ctggggcctt accctgcgca cccagttctg gcttttctgc ccagatccag    10020
acatgagcta ggtgagcctg gccggtcctc aatatcctcc ccagtaaatt ggaggcagca    10080
agggtatcct cacgacccct tctatgctca catctgcagg tcctgaggct tggagtgtcc    10140
atccatttct gacccctggc gccccagggg ctccgagagc tcccttccag atgccacacc    10200
aacccccacg cccgttcctg tgcctcccag ctctgcaccg caggcctgtc tccactgtgc    10260
agccccagga ggacagccaa ccacgaggtc cccactgttc tcccggtctg aatgtcatac    10320
ctcaggacca ccccagcctg ggcccactca gcacaggcct ctcccgagga ccgggctgtg    10380
caaggaacca actgtcttgg cggaggcctc gctaaccgca gcccatctgg ggccagggcg    10440
gggtggccca gggcggggag gcctctgtct tattgtcttc agacaccagc tgcagagatt    10500
ggaatcccag acagcagaga gaggcccctg cagccccctg gagcagggcc aaaccctagg    10560
cttcttccca ggaaaagtcc atcttggggc ccccattgcc ccgtccctgt ccccaccacc    10620
actctacgcc aggcacctgg cttgccacca gctgagtatg ggccctgaac aaacacagca    10680
gaggcaggca gggggttcca tcaccacccc cttacccccc cagggcactg ccaggcttag    10740
ggctcaaccc agaggtctga cgcacatgaa gatcccaccc accctgctgc ctttatacaa    10800
agaaaagcct ctgaacaatc tggcatgtgt ccttgtacag caaactaaag tcaaagaagc    10860
actaggctgc gactcaggag tcccggattc cagtcgcagt gctgctactg agtggctgct    10920
gttaacttca ggttatgact tcccacctat gggcctcttc ttcctcgtct atcaaatggg    10980
aagatggggt gatcatttcc agcactaccg ttctatagtt ctgtggtcct cccactaggt    11040
gataagatat gcagcacgtc agagtttccc tttctgcctt ggctgccagg aagctccaag    11100
ttgaagtttg tgctcaatgg caaccatgaa ccttagccta aatttttttt taccacatta    11160
cctctgcatt tcatgccatt gttcttgttt ttagattgaa caaataaatc tttaattgta    11220
agctacctca actccatttt ggaagtaggt ataagtgtgt gtgtgtgcgc gcgcatgtgt    11280
gtgtgcagag aacactctgt tttagttgta tggtggcaac tgaaaaatgc tctgtgcgcc    11340
tttgttttca tgttttgtac tcaaggcgtc attcttcttt cagtacttag tgaattctac    11400
tcccgggcac tcccatatat ttgttaggaa gcatgcatgc aacccacata tgcaaacaca    11460
aatgtacaca catactgcac agaaaaggaa caggtttatc ccacaccaac acagcaccta    11520
cagggctgtc ccataaccag ctatgcccct ttctctacct acacctgtgg gtcctcccca    11580
atccatttgc tggagatcag gctagatgct aggattcctg tccaaactca ggcatgtaag    11640
gtcatgtgag tggctggttg tccaaattca agtgagatct gggtgtgtga gggcttctgg    11700
attcacagga aagccccagc tgccctgtca tccttcatca aacattcatt aggcactgga    11760
ggggaataaa atggcagtct tgccaggtgc agtggctcat gcctgtaatc ccagcacttt    11820
gggaggctga ggtggtggat cacctgaggt caggagttga agaccagcca accaacatgg    11880
tgaaacccca tctctattaa atatacaaaa attagccggg tgtggtggtg cacacctgta    11940
atcccagcta ctcaggaggc tgaggcagga gaatcgcttg aacccgggag gcagaggttg    12000
cagtgagcca agattgcacc actgcactcc agcctgggcg acagaacaag actctgtcta    12060
aaaaaaaaaa aggcagtctg ggctgggcat ggtggctcac gcctgtaatc tcagcacttt    12120
gggagaccaa agcaggtgca ccacttgagg tcatgagttc aagaccaacc tggccaacat    12180
ggtgaaacct ccatctctac caaaaaataa aaaaaattag ctgggtgtag tggtgcatgc    12240
ctgtagtcac agctacttgg gaggctgagg catgagaatc gcttgaaccc aggaggtgga    12300
ggccgcagtg agccaagatc acaccactgc actccaacct gggtgacaca gtgagatcct    12360
gtctcaaaaa ataaaaaaat aaaaaaaaaa tggcaggctg ccctcaggga acttacagca    12420
actcaaccgt gcagctcttc agcatcactt ctggtagctg cagtttctga acagctacat    12480
atttactgat aaccactagt ccttttccac attagccatt ttggaaaaaa agtttgtaga    12540
ctctattaga gatgcctctg ccttcttaaa cagaaaatgg ttgtaactga tgaatctttt    12600
actgatgcct tagaacaatg ctgctgaaga tgctttcatg agttaattca agagtccctg    12660
gggcctgctg aggattgcag ggttggggca tctctgtcct aacattgaag ggctcagccc    12720
actgagctgc atggcgcttt gctgattaaa aaaaaaaaaa aaaaaaagat ttctctttgt    12780
tctgaagcta ttgatttttc tggcccactg gagagggaaa aactgctatg tggcaacgtt    12840
```

ttatttgcta tttccaacat gctttgggtt ggaaaagcgg actgattatg acatttattt 12900
tgtgttaaat tgaaacttaa acttgagtgt gtgaattttg tcacctgtat tttggctact 12960
catgggtctt ttctttcttt ctgatccttt ctttgctgtg aattcatgct ggtcggggca 13020
gatacagctc tctggacaag ggttgattga tccacggata cttatttttt agacttgatg 13080
ctctgcgttt ggctttctct aattggtggg aatacagtga tgaacaatca gtgcttgatc 13140
tcaaggaaat cacagtttag taggagggga caaacaagta aaataacgta agccagtcaa 13200
caatcctgta aagaagacaa aataggagtt gggcacagtg tctcatgcct ataatcccag 13260
cagtatggga ggctgaggtg agggattact tgaggccagg agtttgagac caacatggac 13320
aacaaagcaa gaccccatct ctacaaaaaa taaaaaaact tatcccaggc gtggtggtgt 13380
atgcctgtgg tcccagctac tcaggaaact gaggcgggag gatccattga gcccacgaag 13440
ttaaggctac agtgagccat gatcacacca ctgtactcca cgctgggtga cagagagaga 13500
cccagtctct ttaaaaaaaa taaacaaaaa tacccaaaag ataaaatggg cccaggcatg 13560
gtggctcatg cctataattc tagcactttg ggaggttgag gtgggatgat cactttagcc 13620
taggagtttg aggctgcagt gagctataat catcttattg cactccagcc tgggtgatag 13680
aatgagaccc tgtctctaaa aagaaaacaa aaaaaattta aaaaattttt aagaagataa 13740
aataggacag tgtaacaaaa ggtgagctgg tttgcttctg ttgtgttggg ttgggttgag 13800
ttctattggg ttgtgttgag tgggttgagt gtgtcaggtt ccattgtgtt gtgttgtgtt 13860
aggttgagtt ctgttggaat gtgttatatt gggttgtgtt gtgttggaag gggctgcatt 13920
aggtttagta tgttgagttc cattgtgttg tgttgggttg agttgagttc tattgggttg 13980
tgttgtgttg ggttgggttg agttctattc ggttgcactg agttgagttg agtgtgtcag 14040
gttccattgt gttgtgttgt gttaggttga gttccattgg aatgtgttat attgggttgt 14100
gttgtgttgg aatgggttga gttgggttga gtgtgttcag ttccattgtg ttgtgctggg 14160
ttgagttgag ttctattggg ttgtgttgtg ttgggttgca ctgagttggg ttgagtgtgt 14220
tgagttccac tgtgttgtgt tgtgttgagt tgagttctat tgggttgtgt tctgttgggt 14280
tgcaatgagt tgggttgagt gttgggttcc actgtattgt actgggttgg gtgttttggt 14340
tgttggctgg taatagggg gctactgctt tagccacagt ggtcaggaag acttctcaga 14400
ggaggtatga gttgggactt gaaaaatgga gggggatgca gccatctagg cagaaggaag 14460
agctaaactg ttctctgttt ctgcccactg ggatgctgtg gacattgtct tcatgtttgt 14520
cctgtttcct gtctaagaga taggccttct agagcaggga caagtaaccc ttctctgtgt 14580
gaaagagcca ggactcttac actgtgtaaa gcccagaaac agccagcagc agccttcctg 14640
cccctctgct ggaggccctc cttcctttgc aaccccccac cacagtgcag aagcccaata 14700
ttaaagagga taaaagcaga gtcgctctag ttgggacagg ggcatgaggg gcaggctagg 14760
agcccccact ttcaggttct caatgaactg aggaaaggaa agccatggtg tggttggtgg 14820
gcagtcagga gaccccaact gcagccaaga gcccagggac aggctctgct ttaccagcct 14880
cactcccagg tggccctcac tgacctgcac gcgggcctca gtgaggtctg tcctcatggc 14940
cagctgttcc cgcgcataca cgtctgggta gtgggtcttc tggaagacct tctccagctc 15000
ctccagctgg tagctggtga aggtggtccg gttccgccgc ttcttgccct tgttgctctc 15060
tgagtcggcc ttctccaatg ggctggggag gtctgagctg gcccggtcct ggggcccctt 15120
cacccccagcc tccttgacac tcaggtagct gctgtccatc cccacagtgt cagagtcagg 15180
gggtaactct ggctcaccca gggagctctc tttagctgag ggaggaggaa acaaagtcag 15240
aaaccaatgg ttgaaccaaa caagagaagg ggaatgtcag ggggagagtg gggcactcgg 15300
gtagccaccc cctgcctttt tcctcttaga gctcattgga tgcgaggtcc ggagacttgg 15360
cttctaattc tggcttgcca ttaacttgct gtgtgacctt gggcaaatct cttccccaat 15420
ctgagcctct tcaccatcat cttagactag gtcccttcca gcaacaatgt tatgtgaacc 15480
taaggcagcc aatctgacct ctgtgacctt taatccccac ctcactgacc ctgagctcct 15540
ctctctagag ggattccctg catcaggact tttctggggc ccgagaattc tgaccccagt 15600
gccatggcct ccctgggtgg aagggaaact cattcagcag ccatgtctgg cccagtgggg 15660
cttggggtga ggaggaccca ggattgaggc agacaggagt gcccctcctt tcctggccta 15720
gtcccaccag gagttcagca ggagctgtgg agactctgcc ccaagagtcc agtgctgagg 15780
agtcaacacc tccttccctg ccccccaacc ctgcacactg gtctcaccta ttgttcaaga 15840
gaagggtctt tcttccttgg gggtgcatgg gattgaggca tgggccacat ttcctgttac 15900
aagcagctcc tgcccactgt ttactgaggc tgcccgggca ccttcatccc cagcacgctg 15960
accccgggcc tgggtgggtg tacatgagca cagctcccag ccactgcgtc tggcagatgg 16020
acccccccacc caccggcaca ttcctagctc aggactgcaa ggacgagtca acccatacac 16080
atcaagaaag aagaggtgga ggctggggtg ggagagtgag gcaagaagcc actttaccac 16140
cacacagcct ggtgagcttc ctaggcagac acactcacct cctggcagag ggtttgggac 16200
aacagtccct accttttttgg caccagggac tggttttgtg gaagataatt ttttccatgg 16260
gtggagaagg ttgtggtggg gcatggtttg gggatgaaac tgttccacct caaatcatca 16320

ggcatcagat tctcttaagg agcacgcaac ctagatccct cacatgcatg gttcacagta 16380
ggattcacgc ctctgtgaga atctaatggc accgctgatc tgacaggagc cagaacttgc 16440
agtaatgctt gctcacctgc cgctcacctc ctgtgtgcgg cccggttact aacaggccat 16500
ggatcggtac tggtccacag cctgggggct aaggacccct ggtttaggtc atggactctt 16560
gatagccccg ccatgaatcc tatggccatg actccccagc tgtgtgctca ggagagctta 16620
cctacccgct atgtgcctca gtttccccct ctataaaatg gggatgaaga tacttctagg 16680
atcttcctca tagggtagct gtgggaaata aagaggctag tgtgctagct atgtagtttt 16740
gccattattg atattcacat ttctagtctg gacttgcaaa cccacagacc ctgtgaacat 16800
tagaggactc cgaccctgga gaagtcctcc aagcatgcag gcccttgggc acaggagcat 16860
gtgcagggcg tgcacagatg cctagggaag ggcagccaac tgggtgtctg cagatgtggg 16920
tgtgaactcc tcgagaactg tattccttgt ctgtaacttg gggaaaagga acttgttgag 16980
tgaaagaaca aacacaatta ataagacgtt tggggagcat ttgacacgac ctttcacgtg 17040
cacgagcttt gatcatcaca acaagcacgt gtggtaggtg tgagcatcag ccactctgac 17100
ccgcggtttc ttcttctgga aataggtcca agcgatgggc attaagtgtc agaatgatgc 17160
ctctagtcca ggtcttctga ctccatccag atcctgaggg ctgaagcttc agactcgtct 17220
cattcctggc tttctgtgca cactggggaa gctgagaggg acatttggcc tgtggcattc 17280
tcgcctccct ctccaaggca tcaaggaaag ggcatagagg tgagtgccca gagtaggcct 17340
gaaagcagct tcaggaggct gctcctgagg gccactggca gtgcagacgc catgggggtg 17400
cacgaccggc ccagcggcat cacgcatgac acctgtgtga gcccaggcac atgcagacag 17460
ccatggcttc agtgacaaca ttctgcaaac tgcagctggc agctgggcca agagctttca 17520
gcaaagatat ttatcttcct ggcagacaaa tacattttat gttttaggtt tttttttttt 17580
tttttagaa accccaccca gcctcacaaa aataaccatc cctgttttgc aacttcctgg 17640
aagccagatg aggggctgag ggaggtgagc actgagccct gagctctgat ccccaggccc 17700
catctcacag ccccatagac gcttgctggg gattgggacc accctgcaca gcaatgggag 17760
ctccatgtta caggaactga gagttggtat ggaccagcct gaactcctag ttttaactgg 17820
ggggaaactg aggccagaga gagaagggaa catgccccaa ttccggacag agcacagcct 17880
ctcagctcct ggcctgccct ctactccctc ccctgcggtc ttaggcctta ttcccaggga 17940
gagaggtagg ggcagaagga acccaggggc ctcatatgac caaggcgtcc tgctctgtcc 18000
atcttgtggg gggcaggacc agcccttgag gatgagggag tgaggatgcg gcctgggggc 18060
agcctctcag cactgagcca gctcttggcc tttcagggga agtggcagaa ggtccagtct 18120
tcatggtgac aagtaagggt ccagttttgc ccacctccag ctgcccactg agaccaagtg 18180
agcaggcaga gatagaggtg gcacagggtg tgcgaagacc accaggccaa gagttgggag 18240
ccctggcctc ggcccccgct cttctcagga cttcagatga cccatttcat ctctggagtc 18300
tcacctacaa atagggagag cggggtagtt gagggtccct cctccccttgc tcaccagcag 18360
ccaggactgt ccctgaggac ctggggaggg gaggagaggg ggagggtgtg cctgtgtcag 18420
agatggaagg aattcagggg agcgctgctc ctggttcctc aaggaagaag gaagcaagcc 18480
ttctaacctg tcttgagtca gcttgcttta aattctgagg ctgggttcct catttagaca 18540
acaaagaaaa attccctgaa gttctgcagc tccactcaac tggtcagaat aacaagcaca 18600
gccctctcag agtctgagtc ctaagacccc aagctcaggc tgtctatgtc cacctctcgc 18660
caccctgcag catctccacc tggcgctggt tccagtctag ggtagcaggg agctcactgc 18720
ccccaggaca gcagaactac tcatctttag acccagccca tgtctgtctc ctgtaggtct 18780
ggttcactga ttccagttct aaccccccag cgcccttccc catgacagcc ctgtccctgc 18840
ggacattggg aggctactgc cacccctctg agtctccctt ccttctgcag ttcccactgc 18900
tgttcctcgg gtggccctga gtttctccgg cctgtgtgag acagagggtg gcagggatga 18960
tatgaggagt ggggctgggc cctgggcaaa gccagcagat tgctctccag ggagatgacc 19020
ccgggctggc tggagtgtca gcatggtagc tgaaggcctg ggtgctggtc agatctgact 19080
ccccgcaagg tgccctcctc agcaagacgg ctgctgaaaa tagaagtccc atggctcagg 19140
ctgactgctc ctgaccaaag gaaaccactg accttggtcc cccaagtccc catctcgggc 19200
ccctgcctgg catgttcctc cttccagctg acctccaaga tccaatgctc ctccaggaag 19260
ccctcccaga ttgtgactgt tttccatcct ctgctcaccc tctccactga ctgcctatgt 19320
ttgctgactc gtaatctcat caagccccga atgtccagct cctcctatag aatgtcagtt 19380
ccccaggggc tgggacctca tgccgtttgt cccttctcag cctgaggctc agcacaggat 19440
aggtcaggtg gcagagatgc agtaagaggt tgctgaggaa taaatgaaca atttctaact 19500
aacaaacatc actgagctct cgctctgtcc aggtatctgt gtgcaccttt ggtgagccga 19560
tgttctatag agcatgtgca gaaaatgtct ctcgagtaaa tgaaccaacc gcctcatcct 19620
cctacccact ggggagaaag tcgggtggga tacattgaaa aagggcacgc aatgacgaat 19680
gccaccattc aaggctgaca gcacaaacag caaaggtcac tgcgggagac aggagagaag 19740
aaagacccag acagaccttg agcgatgtct ccatgcagag agcaagggac agaaggagaa 19800

gcagagacgg gcttcatagg ggcggctcca gaattgaaac caaggtccag aaacccagtc 19860
caggatctgc caggaactgg ctccacaccc accctgccac cacactgggg tagagaagac 19920
cgccagggag ctctcagttt gcctggatgc tgggcaagcc ttcttcagag ccttggggca 19980
cctcgtgctg gcataaggct gagcagcaac agcgcccagc gtgggtccag gccacccgcc 20040
aggggttgga cttctcacat cctccctgga gcagctcctt gtggacccgg aaaggctcta 20100
tgctgatgcc cctcccatga tgagtttgtc ccagggctga ggctatgggg tccaagacaa 20160
tgtagtccca atctgcagct tcccagtctt gatctggaag taaacccctg actacttaga 20220
gccatcaatc aagtagtgaa cctacatcac tgagagcaat gcctgctcca cggagggtgt 20280
tgtgcacgca gtgtggccat catggtatcc agtgctcttc aaacaatcct aggtcagcat 20340
tattgccata gactgctttt gatgaagtgg gaagcattca gttcaaggtt gtattctcaa 20400
tcgggagcca ctgagccctt caacctctgc tggaactccc cctgggggga cccccgtacc 20460
actgggagct gtcgtttcta gtgctgggaa cccctggctg ttggatttca gctggagaaa 20520
tgcatgaagt ggaaagcctt gcttcgttcc cttaacctct acccatctgt cctggttcaa 20580
cccaatggca tcactctaat ctagttttac ctttttaaaa caggaaaacc cttgagaggt 20640
ttccaaaggt gactgatttt tcttttttct ttctttcttt tttttttgg ccgtgattta 20700
aagaatgacc ttctagagcc attttaggga gagggtgctg gtagcatctg tatgtacaga 20760
tgtacacccg agggcctgtg gcacaagtct gccattgtga gtgggtgtgg ggaacatatg 20820
tgtagttgct tgtggatagg tttgtggagt gtgcatgctt gtgtggtcaa gcgcaggcac 20880
acaagtggga tgtgtgtccc cgtgggcagt tgtgtgtggg tgcagatccg cgtgggtggg 20940
tgtggacatg ggtggtgtgg gcctgtgtgg agccgtgcac aggcccatgc atatggcgct 21000
aaggggtctc agtcatttta tcctccatgg ttttgattac atctattttt cctccaaaca 21060
tctgctttgt gggggacatg ccgcttgctc tgttctcggg tgagctggcc ccacacaccc 21120
tgtggcggag tgcacacacc gatgtaatct ccttaaaaaa aacaggccag catgaggccc 21180
tttcatgcta ttgaaagggc actgactaaa cccactgctg tccaaacggt cctgtggggg 21240
tgagggcgga ggttaaagtt tagccattga tcacgtcccc accccacctc atctgacact 21300
ggggtctgtc cagagctcaa cttgaatgtc cctaatttag ctccagtccc aataaataat 21360
gccaaagggg tagtagtgac tgggcttaga tttcagcaca catgcacggg gcacagctgt 21420
ttgcagagac agacagtgtc ccggccctca aggggctgcc aatcttcagc agagggtggg 21480
gtggaaatgg cagtcctcag gctagccagt ggcacaggcc atcctgctca cccacagcca 21540
ccaacccaag ccctgaaatg ctctcgaaat aaggaaagtc tgagaaactg ccccagacta 21600
gaggaggcca aggaaccatg aggactaagt gtaatcaatg tgttctgggg ggaatgctgg 21660
aacagtaaaa ggacattggg ggaagccagt gaaatctgaa taaagtgtgg agtgtagtta 21720
atagtaatat ttaacaaaaa gtgggcaaaa tattctagca gacacttcac ccaagaagat 21780
ctacagggag caaataagca tgaaatatgc tcaacctcat tacccactat ggaaatgcaa 21840
attaaaagca caatgagata ccactatgtg cctatcacag tgggaataag acctgatgat 21900
gccaagttac ggcgatgtat ggagcacccg gaactcccat ccatcacagt gcaaacactt 21960
gggcggtgtc ttatctagtt aaactaacac tgacctcatg acccagcaat cccactccta 22020
ggtatttacc ctacagaaat aatgacttat tttaagagga gaggaaccca aacccatagg 22080
caaatgtctg cagcagctct attcagaatc accccgaaat ggagacaccc ccaagtgtcc 22140
tttgacaggg gaatggagac gcaagctgtg gtctatccag gccatggatc gactccacag 22200
tgaaaagaaa gaactgttga caatgacatg gatgaatctg aaaggtggcc ttagggtctg 22260
taaacaaagc cagtctcaaa tttacatgaa gtgcaactgc actcagctag cacatgaatt 22320
ctgggaaagg caaaactacg gggactctag gcaacagatc actggttgcc aggtgtagga 22380
ggagggggaa ggtttgttta caaagggctt gcactggtgt gatggaacta ttctgtatcc 22440
tgactgtggc agtgaataca tgaatctata catgagtgtg ttaaaactca taaaagtata 22500
taccaaaaag ttttttacag ctgggcgtgg tggcttgcgc ctgtaatctt agcactttga 22560
gaggcagagg cggatggtgg atcacctgag gtcaggagtt tgagaccagc ctggccaaca 22620
cggtgaaacc ccatctctac taaaaataca aaaattcgtc gggcatggtg gcagccacct 22680
ataatcccag ctacttggga gggtgaggca ggagaatcgc ttgaacacgg gaggtgcaga 22740
ttacactgaa ctgagatcat gccattgcac tttagcctgg gcaacaagag caaaactcca 22800
tctcaaaaaa aaaaaaaaaa aatttacagc atgttgtttt caaattttaa aaaatgtaac 22860
taaaaagaaa atacaaaatt gtgttcatat atataaaaca aaaactaaat taaaagttta 22920
tatgccttca cccagaaatc tcacttctag gaatttactc aataaatgat ggtaagcatt 22980
cactagagag aacgttcatc atgtcatagt ttaatatgta ggaaaaccga cagtaatcaa 23040
aatattcaac aataggggat tggttaaata aaggatgata tttccataaa tggaagatcc 23100
ttcctgatag tctttattct gcacgttgtt tcttttttg agacagagtc tcgctttgcc 23160
acccaggctg gagtgcagtg gttcgatctc ggctcactgc aacctcacct cctagattca 23220
aacgattgtc ggacctcggc ctcctgagta gccaggacta caggcatggg ccaccacacc 23280

ccgctcattt ttgtattttt agtagagacg gggtttcctc atgttgccca ggctggtctt 23340
gaactcctgg cctcaaatga tccacccgtc ttggtctccc aaagtgctgg gattacaggc 23400
atggaccacc gcgccaatat taggtattta tttgttcttc tgttttcccc ttgtttattg 23460
tctctctctc tctccctccc atggagcagg gaatatatct gaacatgtgt gctttgttta 23520
ccctgctgtg taacacctag tatcatagct ggcatgcagt agatgctcag taaacatttg 23580
atgaacaaat gaatgagatg atgctaagaa aggatagcgt tttaacacat gcaaagatgt 23640
tcaatttata tcaaataaat ctcattttga tcaaaaagag gaagggagtg gagcatttgt 23700
cctcagaaaa tactacaagt ttccagaagt ggcaatatgg gtctatgggt taattttgtt 23760
tcttttcttt ttttgggggg cggggcgggg ctggtctgaa ttaattttct atttgttttc 23820
ccaggaacag gttgaaatat ctgaaagcag tgattagaat cctggatggt gcatgctctg 23880
agagcgggct gggctctgtg ctgtgggatt atgggaagca agagacaagc accacttgga 23940
gagactcagt taaactggaa ttttagataa acagttaaca ctttaaacaa tataagtata 24000
tcccaaatat ggcacaagaa atacttttat cctacaaatt attcattatt caaatgcaga 24060
tttgactgag taacctggtt ttgttgtttg cttgcttgct ttttgctaaa tctgccaacc 24120
ctgtacatca ggcttatgtg gcattcaggg tcccgaaggc aggactggtc cccaccagac 24180
tcacggactt acacgccagc cttccatcta gcttgaggtt cagagacctg cttccgctct 24240
gcagcgcact gcacgagtat cttcaggctt ccgcactcct gcgggcaccc ttccttctac 24300
ctagcatgtc tctctgcctc tcctgtctgt ggctaactac tactttcacc tggatagctt 24360
tctctgtaac ttttgtgact tttctttcgc attacaaatg taatctatgt tctatatgga 24420
agattaaaaa aaaatcaaga cagaccaaaa gaaaatcact cacacacata cacatacaca 24480
cacacacaca aacacacaca gagaaaacat tactttgtaa acttttgtgt ttttttttga 24540
gatggagtct cgctgttgtc gcccaggctg gagtgcagtg gtgcactctt ggctcagtgc 24600
aacctccgcc tccccggttc aagctattct cctgccgcag cctcctgagt agctgggatt 24660
acaggcaccc agcaccacgc ccggctaatt tttgtactct tagtagagac agggtttcgc 24720
catgttggcc aggctggtct cgaactcctg acctcagatg atctgccttc ctgggcctcc 24780
caaagtgctg ggattacagg cgtgagccac cgtgcccagc ctataaacat tttagtaaaa 24840
tctaaccata ttccgtaccc ctttccatgc tgttctatat tcttctacca caccatcctt 24900
aatggttacg tagcgagaac aatcactatt ttatttaatc catccttttc tgttgggctc 24960
acaggtttct acttgtttga aatcatagca ggtctgtgat ttaaatctct caatacatgc 25020
atggcgattc ccttaggaga agctccaaga actagaaata tgaagttcaa ttatccacat 25080
atctgagggt ctctgagaaa cacggccaag ttgtcttcca gaaacatgta atgtcctcag 25140
ctaccacggt gtcccctccc acttcagagc caataatggt cattatagtg tttttaaaat 25200
ctttgccagc acgatggatg aaagtgagat cttgtctttc cttttttttt ttttattttt 25260
taactatgga acattttatt ttttgttttc ttctgctgtg aattccctct cctgggcttt 25320
caaagctcag ccaagggcca cccttcttcc tccaggacct ccccttgcctc ttaaacactg 25380
aatcccctgc tttacagcac cggctcatgg gcctgctccc tccctagact gtgagctggt 25440
cagagtgggc ctcccagatg tactgctggg gcctgggcct cgtgtgagta agggctcagg 25500
acacacccct ggaattaatg caaatgcctg tgtgtgcctg ggttgaggga gactcctctg 25560
ggctccttct gcctctggtt tggctggggc agctctgcag gcacagctga gggtggccag 25620
tgtgtctgca ggaaaaggcc ccaggccctg gagctgggct ccagacacag gaggtccttg 25680
gtcccctgca cacactgctt gtgtcaggag gctgctgcat ggagtcggaa gaagggacct 25740
ttcaggatga ggggggtcga ggaaaccagc cacagcgcag ggcaggaagg accaaagatg 25800
accccggcag ctccctggcc ctggacaaac ctcttgcctt ctgtttgctc agctcaacaa 25860
cattgcatga attcctgcca gggaggggta tgagaagagg agatggaggg gcacagccct 25920
gtgggaagcc aagactgaag aggagtcccc ggccctggct gcttacccag aaggctaagc 25980
attcaggttc cagggcccaa ctcccagcag gccaagatca agactgcagt tgtgcatgct 26040
ggccaccagg aggcactgca gttccaataa agtcactgga cggaaggtga tgatcctggg 26100
gccccagtgc ctaatgaggt ttctagacgt ttgtttgaag acccaggtcc tagctgggag 26160
gatttgccac tcaagcagta attcagccct actacgcgtc tgccaccatt ccagcactac 26220
aagggacaaa gagatggatg gggcgggtcg ctggccttcc ttgcaggggt ctttcctccc 26280
ctagttctgg gtattttcac attcctgcct cattccagtc tcctcagaat tcccacactg 26340
tggccccagc atcctcattg gacagacggt caaagtgagg cgccgagaga agggacccac 26400
cagtgtcact agtgactgag tgacagacag gctgagacct gggctctttt tcattcctta 26460
agtggcctcc ctttggagac ccatggggat tccttggccc tccagagagg gtggcgcaca 26520
gtgggtgctc aggaaactga ctaacttgca aaagcctttg ccttgcaaaa gctccctagg 26580
acacccctgt cgtttggccc agcggctttc catgtggctg gcagacttgt taccagcttc 26640
cccgtgggtt cttcccgagg caggcatccg ccgaggcagg gtctgccacc ccgtcctcca 26700
ctgctccgcc ctcacacaga agctctctca gttcttcctg caatggcagg ggtgagtggg 26760

```
gaggactgtt tttctcttag tctagagatg gagtgggctt cccagagtcc aggaacgaaa   26820
agggattaat gctgggctct cgcaaaaaca aaaaacaaac aaacaaacta aacaaaacca   26880
ggaactgggc tttgtacttt acaaagtact agggcatcta ttatgccacc agtagggttc   26940
cagcctcttt acaaccgcca ggtttccatt ttatagcccc agaaactgag gccagagggg   27000
gtaaatgcct tgctaagaat caataactgt taagaaggtc caggggtggt ggttcatgcc   27060
tgtaatccca acattttggg aggccgaggt gggagagtca cttgacccca gagttccatc   27120
tagcctgggt gacagaggca gagacctgtc tcaaaaaaaa aagaaaagaa aagaaaagaa   27180
aacaaaaaac tggtaagagg cagagctgag acttgaatct catgcctgga gcatgcctac   27240
tgggccctcc tggggtggat gctgtgggtg ggactgggac aggcagacag gagacagggt   27300
caaggaggaa gggaggaagt cacaggagaa tggctgctct cagctgcctc aagagattgg   27360
ccccccttgca acaaccttca aggctgcccc tgatcaaact gctctgctga agatcaactt   27420
cattgaccat ctgggacctc agatgtggac cagtgccttg tttcagggcc tacacacact   27480
gaagtactta gaggcaaaga ggcatatgag tcatatgtct gcaacttact cttttttttgt   27540
ttgtttgttt ttagacaaag tcttgctctg ttgcccaggc tagagtgcag tggcacaatc   27600
ttgactaact gcaacctctg cctcccgggt tcaagtgatt ccctatgttg gccaggctgg   27660
tctcgaactc ctgacctcag gtgatccacc ttcctcgacc tcccaaagtg ctgggattac   27720
aggcatgagc cactgcgccc agcctgcaac ttactgttaa atgatacagc tgtatatcgt   27780
atgcatgtat atcaatacat acataaacag agcatatgca cagagagaga cacagagaga   27840
gacccagaga gattgaaaat aatgcaaata tggcaaaaca tcaccatttg gggagtcagc   27900
ataaaaggca tctgggaatt ctttgtagtc cacttataac tttaagtgta atactgtatc   27960
aaaataaaaa ggtttttaaa agaaaaggta tgtggactaa tggaaagaag gcaccttccc   28020
taggaacccc tcttgtgaag ctggcatatg taatcatttg aacaggacct ggatgagttt   28080
acctggcaac ctccccgcct tcccctcatc accaccacca ttcataattc tacaaggggc   28140
cttgtacgtt gcaataaaaa tcacaagaca aatgccaact ctgggagcac acattgctcc   28200
ccagccctct ggaagccctc actcccgtgg aagccactgt gacccaatta gaacatgggg   28260
ctcctttaag cagctcttct gagagctgta ggagaccaag gtcaagctac tgttggaact   28320
taaaagttta aaaagtactt taatttttga gtctactgta atttcaactg tcctacagtt   28380
agtccaaatg agtaaagttt ccttgtataa aaaaaagaaa tggtggaaag aaagaaccct   28440
aaactttgtc cagccctcag cccccatggc ccctctgcca aggcctgggt cccctccatc   28500
ccccagcttg tgtctggaat ttgtctttcc tgttctctcc cccaccctgt gtcttgcctt   28560
actgattcat tcagagcttc ccttaggaat catggcctag tggaagagat gcatgaggga   28620
caagcacggt caagtgttcc agggtcccca gggcaaaagt cttgtttccc agacacggca   28680
gggcccacag aaaggagcgg tcaattttgg ttgaaaaggt cggagaaggc ctctgcaatg   28740
gagtggctct tgaattgggt attgccagtc aaggaaggct cggcaaggca tgttgcattt   28800
tatggtaaaa caagatacca agataccatc ccctcacccc aaataggggca aatcccaaat   28860
taccaagctc cttagacagg agctctccaa ccagggctga ttacaaccat gacagcaata   28920
atgtaatgcc ttgtattcgt gttgccctga cccacccact atctcctctg accctcccca   28980
gatcccatga ggtcagtgtt ggtatctctg tttcacagat gagaaaactg aggtccagtg   29040
aagatgaata acttgctcaa gatcacatag ctaggagggg cagactggat ggtcccatgc   29100
caagtccact gcctttcaag gtggtgatga gttatctttc taacatggag ggtcaggca   29160
ggcttcattt tgttctaagg gggcattgct gagccactgg ggtcatttct atctcaaagg   29220
actagggtaa accaagcttc agggtctcca tgagaagccc atgtatgatc taacacttgg   29280
agatcacttc ctgatgcctc ctggggtggg atgggtgttc cagcttcagt ctgggctcta   29340
agtgcccctg tcagcttctc tcctcaagga ggctagaagt ttcccggctc ctcgtcccat   29400
ccccaagctc catctcatgg cgtgggtggt tggatgagac cctgtccccg gcaggaccca   29460
ctgccagaga aacagagtct cccatgctct cccctatccc agcaatacgt ccgcagggtt   29520
cagagacccc actggaccag accctccctt cacagccaag atgcggaggc ccagagcaag   29580
gtcatactgc ctgataatgg aagggccagg acttgaaccc tgcacctcct gcatccagcc   29640
ctgtggtctg tctgcccatt gtccctctca agccccctaa accatgtggc cctgggcgtg   29700
tcccaagaca tctctcactt aattccccca agcctctgtt tcccttctgt agcagaaaga   29760
atcctgcagc tccaccacct tcaccaagac actgtaatat cactttcatc cagaggctgg   29820
cgagatggag gagcttagat cctgcacgag gcaaaatgaa atgattgaga agtctttggg   29880
gtaggacagg cctgggcttg gcccctggct ctgccactta tttgctacat tgattcattc   29940
aacagcattc ctggagcacc agctatgcac tgggcaaagt tccaggtggt gaaaatataa   30000
agcccagtaa aacacagtcc cttccttcaa atggctcgcc tcagtgacag gggccatggt   30060
tacagtgcag ggtggtgacc agggcagggg cacctagccc agccttgggg cgactccaga   30120
tctctgtaga ggaggtgttt tgaagcagca atgttgttgg agaggtctgt tgaagctgtg   30180
tttagctaac gcttcacata agactaggga atgccagttt tccccatgaa agaaatggga   30240
```

gccgactaaa ggagattatt aaggaggctc ccaagccacc ttgtggttct gccacagaag 30300
ggtatccttg gaaaagtgac ttgacctttg tgtgcttcag tttcctcatc tgtaagatga 30360
ggctgagaac atccacatcc cagaatggct gtgaggtctg actgaagtag agtgcaagac 30420
ggcatatccc ctgacccaca cacggcaccc actctatcaa tgggacgttg ccatgatata 30480
ttaacacagt tatgatctgg ggatgaattt ggccgtggca gctgacagcc agggcaacgt 30540
tgctcagcct gcaactgtgt cacccccacc tgcttccttc cccaggtgca tcctctccag 30600
ttcaggtttc ccgagcggca gctcccagaa tgacatttat gaaccataaa cctctgtcaa 30660
aagtagctcc cagggtccct ttcaatgaag ggcccatttc acatacatat aacaaagact 30720
ataaatgggg tgaaacattg gcaactgctg taaaaatcaa atgttaaatt tatttggtta 30780
tatagactta aaaaaacttt taaagagtgg attcacggca gcctctgcct ttagactgtg 30840
ccctcaccaa gggcagggga ggggtgttac gaggcagcgt tatcactcgc cctgcctgtt 30900
gtcactgtct ggcacccaag tacgtgctga attgagctaa tcttaaacaa ttgaatttct 30960
ttcttcttt ctttcttttt ttggagacag ggtcttgctc tgctgcccag gctgaagtgc 31020
agtggtgcga tctcggctca ctgcaacctc cgccttctgg gttcaagcga ttctcctgcc 31080
tcagccttcc gtgtagctga ggctacaggc acatgccgcc acacctggct ttttgtgttt 31140
tagtagagac agggtttcac tcttgttgtc ccggctggtc ttgaactcat gagctcaagc 31200
aatccgcccg cctcagcctc ccgaagtgct aggattacag gcatgagcca ttgcgcctgg 31260
ccaaacaatt gaatttctta gcctgcagaa gcagcatatg atcccagaaa gaatgtgctg 31320
ctttcaccaa catcaaatct tttctttctc aaatgtgctc cttctttaat ttttatttat 31380
gtatttattt atagagacgg agtctcgctc tgtcacccag gctggagtgc attgatgcca 31440
tctcggctaa ctgcaacctc tgccccccag gttcaagcca ttcttctgcc tcagcctccc 31500
gagtagctgg gattaccagc actggtcact gcacctggct aatttttgta cttttagtag 31560
aggtggggtt tcaccatctt ggccaggctg gtcttgaact cctgacctcg agatccaccc 31620
tcctcggcct cccaaagtgc tgggattaca ggtgtgagcc acctcgcctg gcccaaatat 31680
actcattcca atctagagaa tcacttacaa actgaaaaag caacaaggct tttctcccct 31740
tggatgagaa agggaaagta atggctcaat taattataag attgcttttt tttttctgt 31800
tgcctaggct ggagcacagt ggtacaatca cagctcactg cagcctcaac ctcctgggct 31860
caagcaatcc tccctcctca gcctcctgag tagctggtac tatgggcaca caacatcaca 31920
gctggctaat taaatttttt tttttttttt tttttttgta gagacgaggt gtcactatgt 31980
tgcccaggct ggtctcaaac tcctggattc aagcaattgt ctcacctcag cctcccaaag 32040
tgctggaata caggcatgag ccaccatgcc cagtcctagt ttgtacttta ggaaagtcat 32100
caacttcctt aaagttgata agaatgggaa agattcttca atatttgctt tttaaaaaat 32160
ccacatttac aactataaaa tagttaacaa ttcaaaatat ctaaaggata attataccta 32220
aaagttttgg ttggtgtttt tgtataaatg actgggattt ggagtagatg ggttcccagg 32280
attttgccta tcacatatag taaggataaa atataaataa tcaatgtttc agcattcaca 32340
attaacatgt tagttaaaaa tggttctttt ggccccgcat ggtagctcat gcctgtaatc 32400
ccagcacttt gggaagccaa cgtgggcaga ctgcctgagc tcaggagttt gagaccagcc 32460
tggcaacac agtaagaccc ccatctctat aaaaaaaatt tttaaattag ctgggcgtgg 32520
tggcacaagc ctgtagtccc agctactcag gaggctgaag caggagaact gcttgagccc 32580
aggaggttgt ggctgcagtg ggccatgatt tagccattga attccagcct gggtgatagt 32640
gagaccctgt ctgagagaag gaaggaagga aggaaggaag gaaggaagga aggaaggaag 32700
gaaggaagga aggaaggaag gaaggaagca ggcaggcagg gagggaggga gggagggagg 32760
gaaggaagga agaaaatggt tttttcattg acttctgctc atctgaacaa aataaaaaat 32820
tcaaaaaagt ctcttcttta aaatgcatga tgtggccagg tgcagtgtct cacacttgta 32880
atcccagcac tttgggaggc tgaggtgggc ggatcacctg aggtcaggag ttcgagacca 32940
gcctggccaa catggcaaaa ccccatctta aaaaaagaaa aaaaaatata tatataatgc 33000
atggtgtgtc ccttctacca agcaaacctt aatccacagc gaacgtgaga caaatacatc 33060
tcttttccac ctgttactac ttcttggcag gatggatccc ctgcaacggg atagatctcc 33120
tgagaggata ctttgtgtag cacaaaactg ggctctgtgg aatcttctct catggggcta 33180
aatcctgggg gatgcattaa tacagcagag cttgggcaca tacattccct gaggaggcct 33240
ggccctcact gtccacctgc cacatggtac aagtaatatt tccaaactgg cctcttgccc 33300
ctggcttcgc caacactggg ctagtgagga ggctattact cgttctgttt agaagggttt 33360
gtctcctcct tctttaccca caggagctgc tcccacatat cacatggggg tcctgggctg 33420
ggctggcctg gttctcagga tggctctgga gggaggaggg agtggtgtct gggcggtcag 33480
gggacatggt catcatttca gtagatgggc gaagatggtg tagcccacgt acaacatgca 33540
gtgtgctgaa aacccctccc agtgcacccc ctccaccgtc tacagagctc tcacatgctg 33600
gtcaagtctg atgggcacat aggcatgagg gcatccaggc agtgggcatg agccccctgg 33660
gcaggggagg aaaaaggctc tttggcttca ggagatctgg gttcaattca cagcgggaca 33720

gcctcacctt cctcatcttc aaattgagga tactaatatc tcccagtggg tttccacaaa 33780
aagtctcagt ccaggtgtgg ccaagggcaa tgcccaccag gtgctttctt aattgcagat 33840
ggggcccagg aagctggggc ccaggaaggg tgcagaggtt gcctgacatt ccctgagccc 33900
gattctctgt gctcagccct gtgctgatca gagaggaaca cagcagaacc cctgccctgg 33960
ggacaggctg aagttctggg tcaaataaac acagaaaaca cattcagcag ggagagagtt 34020
attccccagc tcatgacaca tgtgtacgag gtgcagcagc ctatggggag gaggggcctg 34080
aggggcctgg gtggcctttg tccctccttg aagcctgccc ccgctgcgtt aggtagagat 34140
ggcagccccc gcccctccca tcactcatcc atgttttaaa agacaatgtc aaccacacag 34200
taatcataga ttcagtaata atttcttcat tttccaactc tatgcatccc agtcaacttt 34260
aactgcctgt caaggtttcc ggccagtctg atagagtcaa ggttccagca aaatcttatc 34320
aatcataaca gctaacattg actgagcact tactatgctc caggaactgt tctaagatcc 34380
ttacctattt atttatttat ttatttattt atttatttat ttagagacag agtttcgctc 34440
ttcttgccca ggctggagtg caatggtgtg gtctcggctc actgcaacct ctgcctcctg 34500
ggttcaagtg attctcctgc ctcagcctcc ctagtagctg ggattacagg cgcccaccac 34560
cacgcccagc taattttttg tatttttagt agagacaggg tttcactatg ttggccaggc 34620
tggtctcaaa ctcctgacct caggcaatcc acctgcctcg gcctcccaaa gtgctgggat 34680
tacagatgtg agcaaccaca cctggctttg tccttgccta ttttacctta tttaagcctc 34740
accctatcac taacaggtag acatgattct tatccccact ttacaggtaa agaaactgag 34800
gcaaagaggt caactaattt gcccaaggtc actcagctat gtgggctgaa atctgtttcc 34860
aggtcctgag cttcatgggc ttctccaaac caaaggcatc tgaagcatca agcagcctgt 34920
gtagcacaat tgtggaatga acagtttcca tgattttcca agaaccctag aaattatgcc 34980
ttagggcaca tggctggaag accaggctca agcaggcctc tgtttagtca cctaggccgg 35040
ggaagttcct aggaagggga gccggggaca gctacttctc tgcagctgtg ctccccttgg 35100
actctgctgt ctagtccctg gagccccagc ccctggttgt gccattcact ggatggccta 35160
gggtagggga cagggagttg caagtgcctc ttttgggaat ctaacagaaa atcttcaaaa 35220
tatgcatggg gccaacagca tggagccact ggatgcttcc ttcttggagc tcctccaagc 35280
tccgctggaa ggattctgga atcatcttaa taaaggaagt ggtcccagat gtgcgcatct 35340
caggaggtgg gtgtatagac cctcagagca caaaagaatt tccccaaagg ccacgtcctc 35400
atgggatggt caactgaagg ggcagagggg cccccgaata tccaggggcc tagccagggc 35460
caagactgtg gctctagagt cctcgccttt ggctctgccc tccacccctt ctcaccagct 35520
ccaggtcccg gaaaacaaat gtgtccttga gctgaatttc tgtgacctgt tggtcactgg 35580
cctgcaggca aggagatgaa cggatggcct acaagggtcc ttgtacattc tgcaatcctg 35640
gacttcacat cagaagatca ttcaggggcc agatagacaa atgcaacaga atagagacca 35700
gatgtaaact ctcaaataca tgcgcaattg attttcaaca aaggtcccaa gaccggtcga 35760
tgagaaaagg gccatctttt ctacaaatgg tgctgggaaa agtggatatc cccataagga 35820
agaatgaagt tggagagcct tatgttacgt catattcaaa attaaagtca tcaaagacct 35880
aaacctaaga gctaaactat aaagctctta aaagaagaca taggcgaaaa tcctcctgac 35940
actggagtta ttgacgattt cttgaatatg acaccaaaag cacagacaac aaaataaaga 36000
aattgaaaaa ttgtacttct tcatcaaaat gtaaaacttc tgtgcatcaa aggacagtat 36060
ccagggagtg aaaggacaat ccatagaatg agaagaaata ttcgcaaatc atgtatctga 36120
aaaggaatta atatccagaa ttcctatgac tgaagaacaa ggaaacaaac aaccccattc 36180
aaaaatgggc aaagtacttg aatacacttc tccaaaggag atacacaaat ggccagacgc 36240
caaaggagaa tactgtatga ttccacctat aagaggtacc taaggccagg tgcggtggct 36300
cacacctgta atcccagcac tttgggaggc tgaggcgggt ggatcacctg aggtcaggag 36360
ttcaagacca gcctggccaa tgtggcaaaa ccccatctct actaaaaaca caaaaattag 36420
gcaggtgtgg tggtgggtgc ctgtaatccc agctacttgg gaggctgagg caagagaatc 36480
gcttgaaccc gggaggtgga gattgcagtg agctgagatt gcaccatttc attccagcct 36540
gggcaacaag agtgaaactc tgtctcaaaa aaaaaaaaaa aaaaaaaagg tacctacagt 36600
aggaaaatcc acagagagag agtagaatag aggtcacttg ggtgggggt caagggtgtg 36660
atggcggttc ccgtttaata ggtatggagt ttctgtttgg gatgatgaaa aagttctgga 36720
aacagtggtg atggttacac aatattgtgg gtgtacttaa tgccactgaa aggttcactt 36780
gtagatgtta aagtggtagc attttatgct atgtatattt taccacaata aaaagttcat 36840
tcttaattaa aataaagatt ttgaatgcaa aaagcaaagg ccatactgaa ccatgcaaag 36900
aacactcagc ctggggtctc actgttttgc tcagagccct ccagtggctt ccccaaagtc 36960
tgaacccttt ggtagaaaat aaatggccca ggggccaggc gcggtggctt atgcttgtaa 37020
tcccagcaat ttgggaggct gaggcgggag gatcacttga gtctaggagt tcaagaccag 37080
cctgggcagc atggagaaac cctgtctcta ctaaaaatac aaaagttaac tgggcgtggt 37140
gacgagcctg tagtcccagc tacttgggag gctgaggtgg gaggatcact taagcctggg 37200

aggcagaggt tgcagtgagc tgagattgcg ccactgcact ccagcctggg tgaccgagtg 37260
agaccttgtc tcaaaaaata ataaaataaa ataaaataaa ataaaaagct ctcttcagct 37320
gtgcccctgc cattcctcag cccacctcac cggcatgcat catgcccctg cactccacac 37380
acttgcttca tgttcttccc ttacccgaag acttagtttg tgctgtttcc tgagtctaga 37440
gggcctcttt catctccttc agctggtgag ctcctattca actttcaagg tccacttcga 37500
atgtccatgc tcaggaagcc ccaatccatt caccttccct ggggattccc atggcatgta 37560
gcacattcta accaggctat gggctctctg agggcaggag ccagttcatt cactgccaaa 37620
cactctggtc cctgcctgac acagaagtgc caggctgaag gatctgttgg atgtaaggag 37680
gagcttttgc caggcgcaca ctcctccatg cattccttct gagctgttcc ccctctttga 37740
actttggctt ccatctgtaa aatggagatt caattcctcc acctaggcct ttggtgagac 37800
tcaggggacc atggatgttc aagtgccctc agattgcata tgccggggtg ggacgggtgg 37860
acggaatcat gggtgtgctg ggaagagccc aggccttgga gccggatgcc ctgctgggaa 37920
cccgggtgag gtcagatggc atcctggcca gaagagaggg ctgcatccct tgcaggtgag 37980
tctcagaatc ccagggtgga ggatgagtga atgggggact aagggaggaa cctcagcagc 38040
tctcaggcag caccctgggc aggtggcagg aatgggggtg ccagcgggca ggacacggaa 38100
ctcactgtag ggaatggggg agacctgagg aggagagttg gggatgaagg gagaaaacca 38160
tttggctaga gccgaccccg gggctgtgg cagtcagacc gcaggcctgt ggccagtggg 38220
gtgagaccca aaagaggagc agtgaggaaa aggggtactc ctccgactgc tagagttgga 38280
gaagaccaag acttgtgcta aatagagcta cccagagcct cgctgctcct acaccccaca 38340
ggaaaccctg caaaaaaaaa aaaatcaata attttctacc agaatgcaag agaacgacaa 38400
agcaggctct caccccaggc ctccattcaa aggaaggatg aacgaagaga gagtggaaag 38460
acttacagag ttcctagcat gttcccctcc tgtaagatcc cttcattcat tcactcagca 38520
acagacattt attgagcatc tactatatag actagctcca ggctctgggg aaagttggaa 38580
tcaaaacaga caaagcctcc tgcggtctct catgtttcct agagggcact gttcccacag 38640
atgctcctca gctccaccct accttgatca gaggttctct catgttcctg aaacttctgg 38700
aaactcatgc agtcattcct cataccagct atgaggccct atgccatgcc tgggacactg 38760
ttttcacctg gtacaggtga ggaagctgtg cccaaggtca cagagtcagg tgtagaactt 38820
caacctgaca ggtgccgaaa tttactcctg acactacttg tcttctgcat ccccggttgt 38880
gaagctgaga agaacgcttt gggggccatg gttttttttt ttttttttt ttttgtgaga 38940
tggatcttgc tctgtcaccc aggctggagt gcagtggcag gatctcggct cactgcaagc 39000
tccgcctccc gggttcacac cattctcctg cctcagtctc ccaagtagct gggactacag 39060
gcgcccgcca ccacgcccgg ctaatttttt gtatttttgg tagagacagg gtttcaccgt 39120
gttagccagg atggtctcaa tctcctgact ttgtgatccg cccgcctagg cctcccaaaa 39180
tgctgggatt acgggcatga gccactgcac cgtgccctgg gggccatgtt tgtaagaccc 39240
tgtctttcat ctccacatca tctgggatgg aattagggtt atctcaagtc taatcaggat 39300
cattctagaa caggatgcct tttaggtttc ttggagccct cggccagcaa gactcgactc 39360
ttccttcctg gctgtctcac tgtggacgag ttacttatct tccatgaacc tcagttttca 39420
tccacgaaat ggggatgata atagtaccaa tctcatagga ttgtgatggt gattaagtga 39480
aatcatgcat aagcagcact ggtgtactgc ctggcgcaca ggcagggacg ctcagttgtt 39540
ctgggctgtc tcatggttct gcagcagcag ccaactctaa gccttctttg ccttaaaacc 39600
cattattgtc tctgtcctgt agatgggaaa actcaggcca ggagccaggc ctggcatccc 39660
agactcttaa cctttggcag actggaccag ccttcccccct ttcctggtac aatgaatggg 39720
gaagagtgtg gggtcctgag gcatcttgag gattctggag ggccaattca cccctcttgg 39780
tgaatatcac agaaccccaa ggcctctgaa accactgagt cccatgaaga aggttcagag 39840
atggggaggc tgaggccaca acaggggagc catgcagtag agctatgtgc atgtttgtct 39900
tccccatttg aaagtgagct ccctccttga agtcagaggc catggagctg gagttgtccc 39960
atctccatgc tgggatgtga gccaggtgtg aatggatcct tgtggccaca tagagacatc 40020
ggggaggctc acatatccag gaggacccca aactctacat acacactcta aaacctgatc 40080
agaaccccac cactatcatt tgcaatatcc tagcaccctc aaagggtata gtgtgagggg 40140
tggggcagag aacctggtgg tgcaggatgt acggggcaga cacactacct ctctccatca 40200
tcacatcctc ctcatactcc cctcccaggt gggacagggc caaagtgcca ttcaacacag 40260
ggagaaatgg agtggcacta gagaagtaag gggctgtta tacatgacta tttccataga 40320
aaaaccaaga gaaagcatgg agggagaaaa ggagaaagtc aagagagaag aagcctcagg 40380
atttccagcc actagagaaa actcattggc ctggacaaat cccaacttcc cccaactttc 40440
ctgcaccctc cccttaagtg gcttccagta agctaccaaa ccctggtggc gcctgcacgc 40500
cccagggcaa gccagcctga ccttctcctc agagcctgag tcagggcttt ccccatctca 40560
gggcccatcc cactgaactg gaatttcaag gtcacttgtc tgtctctgcc tctggacctg 40620
aaggcagggg ctgcatccag agcttcccag aataccccac aaggcttggc atagtgacag 40680

gcaaagtttg gtgagccttg cgtaaatgga atcaagcctc tcagtcccct ttgtctgtga 40740
aataagacat gtgggccagg agatgactgc catctcttcc aacccttgagt gagacccccc 40800
aacatgtcac catgacccat tgtgtgtgtg ctgcagggtc aggggaggta ccagtttcct 40860
cgatgtaacc aaaggccctc agggcctggt ggacagtcct acacagtggg aggagttggt 40920
catctggcct cagctgaccc tggcttgtgg ttttcaagaa cttaggaaaa gaaaacccag 40980
cttggggagc tgggcagaga gcaggagtcc cagcctggtg gggctgcatt agagtcaggt 41040
ccctgcaatc ccaccaggga ttccacgggt gctctcctgc tgtcctctaa tgcccaggag 41100
ctcatggcgg ggtctgcttt acttctaagg agactgaggc agaactgcaa gcatctgccc 41160
tatttgatgc acagagagag tgcaacttac tggacttatt gcaggagtcg catccaacaa 41220
gagagcgggt gagtggattg gattccccaa cactttctgc ctttccaccc attgtagtgg 41280
aaggaggaga actcggtggg ttcctgccct cctactaacc tggctgtggg ggtccatttc 41340
cccttgggcc tcagtttgct catgtgctaa atggggtcag cagtgggcac agacagtgcc 41400
aaggacacgt ctcttccgca gtgagccaca cgggacagag ggtatattcc agagctggtg 41460
gcttgtcaga cggcctcgcc gcagtgtggg tgggctggat ggaccatgtt cgagggcccc 41520
aggatgacgc ggggggtggt tgggccccac cggggacttg ccacagggag attctgcctg 41580
ccactcggtg agtcagctgc tcctgggagc catgaccagg ggcaggaagg ccacaggcca 41640
tgatgcaagg gcgcttagtc attctccctg gggcgcctc tggaggcaga ggctatgcca 41700
cccacagacc cagagaagct ccagagccgt gcacacgggt actcaccctt gcacacctga 41760
ggctcgcaca ctgggggaca cagacacaag tgccagagga cagacatgcc tttcaccgga 41820
ctacacacaa accctcggcc tcgccctgaa acactcagat agcaggacgt ggacccagag 41880
agaatgggca cggcacacac cgctcacatt catgctgtgc acacgctcac acaaacacct 41940
aagctggctc ctgggtgcac ttaaccatgt gggggcaaca ctgcaggaga caccagttct 42000
tcattcattc ttccagccag cgaacatttg ttaagcccaa ctgtacaaaa cacttggaag 42060
gacataagcc gtaacacagg aggcagggac ttggcatgtt ctacctccta ggagaaggtc 42120
cgcccaggga aggtgctcca taaatactgg gatagggaag atgagtggac acaggggcca 42180
tgagcccggg gcctcagctt gagcctggtc acaaagacac ttgcttaccc cctccctatt 42240
tgcatcccag tcctgagccc agaacctgca ttccaccatg gccctgtagg acctctagcc 42300
ggctttgtgt cctgtgatgg ctccccactc aggacctgtg ttagcctcca gggaagcctt 42360
ttccacccac cttaccccag tttctcctgg acaaaaatgc gtgtgggggt gaggagggaa 42420
gacaaagggg gtgaaggtgg gcccagactg ctaccagcac agaaactcca agagggtctt 42480
tggaaattgt caacactcct gcatttcata cagctaaaat tcagaccccg gagcagaaca 42540
tacttctcca aggccatgga ggagtagaga atgcaaaggt ggaggctggg gtgatctaca 42600
ccgcaactgt gggccccggt ccccacgtgt gccgcgctgg acatcagtgt catggtccaa 42660
acctcccaca gcttcccagc cttctctgtt gtggccttca gagccctgga gagccagttg 42720
ttctgctctg acctgggtca ccaggtcctg tccctctacg aggtgcgtgg ggccaacatc 42780
catgctgggc ctccagttca gaatgaaaat ccactcttaa agaatctgct ttaagaatcc 42840
gatgaagcaa gcactggctc cctctctgcc cccaatgtgc agcggcgcca gtgcccagca 42900
gccttgacca agcggtcccg agtacaagcg gcgccttcac cgggaaagat aggtacttgg 42960
cagccggtgg cccctcttac cccgcccgta tctcccagtg acccccctcct caccaaaacg 43020
gcaccctggg agccgcggag cgccacggtg acccttccaa ctgcctctgg agtccaaatc 43080
cgaaccggtc cgcggccctg agtaccaccc tacacacccc agccttggct cctcagagcc 43140
ttgcctctgc gtggctcttc ctcgccgctg agccacaact cccttccatt cctctctttc 43200
ctcggcgctg gctggtgcgg gttggggtca ggtggagaag ccgctctttg ttaaggtgac 43260
agaacgtgct gggggtgggg gccgggggcca gggccggtgc aactaggggg ccgctgccct 43320
ttcctggaca cagtggaagc ttcttccgca tcaccaaatt tttgtcatcc tttctgaggg 43380
acctgcttcc aggcagcacg caagttgttg tcccgggttt actccgcacc cctctactgg 43440
gtgaggaagg agcatcttga atggagatgg gggtgtcccc ggtttataca tctgcagaga 43500
agaggtgtgc cgggctgcac ctctggaggc cgcggtaact gatattagag aagaccccgg 43560
ttgcagctgg gaaggctcac tggctggaaa gaggtgcctc ctccttccag caaagggccc 43620
tgtttggaag ggctgcttct cacctgtcta gtggcaccac aggacggtcg gcttccactc 43680
gaattccccc ggacggtatc atcacatagc cgggtcctcg cagtgttggt ttcccaatcc 43740
gatgactgtc acctcggtga ggacctgtgc tgatggccgg agaaccctgc gctgcgggcg 43800
cacatggcca ggtggcgcct ggcaggcgac gtccgggtgc aggacggcgc tcttaccgcc 43860
ccaccccaaa ccgttgcctg ggcctaggtc cttcggcttc ctgaacaggg gtttgggggg 43920
ctaaggacgc tgaggctccg gggcaggaaa gttctctctg gttaagcgtt ctctcttctc 43980
tccggcatac actcccctac ccacccacct cgcctaccct cggggcgaga ggctcaccaa 44040
ggcagggcgc gcccccccca tgaatcatcc caaggcctct gagccgcggg ggctccgggc 44100
aactatcccc ctcctctcct ggcctcaggc accccagtcc aggggtctgc agagaagccc 44160

gaagcccgga caaacgcgcc ggacgtcaac aacctctcat ccctggcagc agcaaaggcc 44220
aatatatttc catttcttat ttcagtttgc caccaaaaca aagctgcgcg cggctgaggg 44280
caggaaggcg ctgagaccga gaagaaggga cgtcccggag aaagtgcgcc cagctgatct 44340
tagaaaccag agtcctccgg gacttcgccg agattttctg tagggcgttt taatctgttt 44400
tcctactgcg tgccggcgtc gcagcgcgtg cggctcaggg cttggtgact ccggcttagc 44460
ccggcggtcg cggcgaggtt cctggcgcag ccgcttggaa cttcgcatta gaatcgggac 44520
cgcgcaaatg ccctggctga agtgtcaccc tattcaagaa acactgctgt caggaacaaa 44580
atggggtccc cggtgctccg aagtatcttc tgaaattttc ttaaaacaac ttacaaaaaa 44640
tgtttttgct ttaacgtttt acaacgttta aggaaacatg taaatggtct gtttctttat 44700
cgagatggtc gtcctaacta acagtgtaca catacataac aattcttcca actttcctcc 44760
tcagagctaa gcacttcact atatgtaaat tataataaag aaaagattgt gcaagatcat 44820
gcaagtcgat tgacttaaaa tattgagttt taatccaggc cctctgtttt tctatttaac 44880
aacttttgtg tttggaccag actggtgaag caggctatgg aaattaacaa agtaaaaaat 44940
taaaagcatc ttccttcgcc atccctccct ccaaaattaa acaacagtcg ccccttcctg 45000
agcaggcttc agtcccaggc tcgagttttc ctgcgatcac cccacagtca cccacagcag 45060
ctgttgctgc ttctgtcggg ttttcgtttc tgccttcttt gggtcgtctc ttgtatacaa 45120
aacacacccc agttctctaa ctaaattcaa atacgacccc ggcagaattt acacatttcg 45180
tggtgcatgg attgtgtcgg tgcaggggaa ataaataccc tctggtattt aaccactgag 45240
tctaattcga aaaatcggga ctgggcccct aggcggcacc ccaggggctc caacctggcc 45300
cgcgcctccc cagaccttgg cgctgagagc gctgcttttg cgggtgggtg gacggagagg 45360
taacaatctg ctttcaacaa aaacctgtcg ccaccgaatc gaaagcgaaa gggaagggag 45420
aagaggggca gcttcccgca gcggccccgc gtctcgggac aggtagacta ggaccagtac 45480
gcgccggctg cagctccggc ggtcggatcc ggagtttggg ggcgaggcgc cggagtccct 45540
gccctgagta cagggaggct ctctgcgtcg ctgagaaacg tcggatctcc aacccgacat 45600
gtctcctgtg gcccaggcgg cccgccctgg actcgggcaa ggaattcagg aggccaaaga 45660
gcggacccag agtctggagg ggagaatggt gaagtcttgg gttgcaacag aagagggctc 45720
ggtggggct ctttcgtaaa aacgcggtct ccgcacccaa aggacactgc ggagccgcct 45780
taatcgtcgt tcttttagca catttgggga aaggaaggcc caagttgggg tagggacgct 45840
tgcagcccgg gagggtttct ctcaccaact ctttccgcaa cccggtccag acccggccag 45900
cagagactgg aggcggcttc cccgccccgc actccctcgg cttggcccag gctcagctgc 45960
ccggggcacc ttccttgccg agaggcaatt ggactctgcc gggagtgggc ccagaagttg 46020
aggcccagga agaggcctcc gagcttcccc cttttgttag gtgtagttac acacgtgaaa 46080
gaacagcttt cgccccgacc tccagggcta ggaatggttg ccaggtctca gctgcctgcc 46140
tgaagacgga gatcagccag cgccagtact ggcaccaagg ccgcccctgc ctccgacttt 46200
cccaatgctc ctgggagcgt ctaaggattt ttccttgaac tccatttctc cgcttttgag 46260
tatatacact ctcacaactg tgggagagaa tcgttgtcaa tgcatgcctc agttttcctg 46320
cttgctggca gggtagggat cccactccag agtccctcct cctggctact tctgttaagg 46380
gcagagcctc atcttttgta tcctcagttt ctccatctta gacctaacgc gcctaatccc 46440
tttcctagaa ggaccagaca tctcctggct ggggagactc agctgctgag agagggaaag 46500
aacaaacact gacttgctgt ttattgtttg tggctttgcc ccgagcagtg cctccatctt 46560
aggcagcatg gagtggcgtc ctgccggtag ttacaggctg cctttggtcc cagcctgcag 46620
tttatgaccc agagcagtgg cagggacttg ccctgtcctc agaattgggt gctctcgagt 46680
tcccagcggc tcggagatag atagggatgg aaatgtgggt gggggttggg aggggcaggg 46740
gtagcggggg ctcttgctct tgcctcttgc tgtctctgca gaggcacatt tatgacccag 46800
taaggccttt ctgttatcac cccaggatgc agatgaggaa accggggttt gggaaggtgt 46860
ttgctgtcag gtcacactgc cacgggttag gggcagtagc gggtcaagtg gctggccctt 46920
tccgtcagct ccaaaggcta gtgcaagtag aggaagggcc aggctggctg tgggggccca 46980
gcaggacaca gagagagggc tcaggggagg ccaggcctct ccgccttccc tctggacctg 47040
aaggcattta accaagaggt ggggagctcc ttaggctctc ttcttgccta atgtatcagc 47100
ctcttcagag gtctcactgg taattttcat taaaatatga aaattcaatg cagtggagag 47160
agagcatgag ctctctggct atgcaggatg aacctccatg ggtcccctag tctgcgacct 47220
gccctcacct aatagtcctg aatgacaggg acgagctggg gcctgcatcc ctgggctggg 47280
aaggacagcc agtacggaca gggctgagag tctctgctgg cttagcacgg aaagagtgcc 47340
tgtatgagag ctgtgtgtgt ccatgtgcgg agctgtgtgt gtctgtgtgt ggagctgtgt 47400
gtgtccatgt gtggagctgt gtgtgtccgt gtgtggagct gtgggtgtcc gtgtgtggag 47460
ctgtgggtgt ctgtgtgtgg agcagtgggt gtctgtgtgt ggaactgtgt gtgcctgcat 47520
gtggagctgt gggtgtctgt gtgtggagcc ttgggggtct gtgatctgaa tgcctgatct 47580
cattccaact ggaggtgaag aggaaggtgg aggaagtggg gaagggccac tgaatctttc 47640

taaatcttct ctcagttctg tcctatgggt ttgggagtat gtggctcatt cgaggccaca 47700
tgggcatgtg ttcttggggt atgaccacat cggctcctaa agaagatgct ctctgcgtgt 47760
gtgtgtgtag agtgcaattg tagacaagca tatgactgtg aatgtttgtc agcagccatc 47820
tatgaactag ttctgcgcct gtgaaagaag gaacctgaat acgagaaaat atctgccatc 47880
ggctgttttc ccaaagttat tctagccacc aaggcctccg ccgtgtccca gcctctgtcc 47940
ccaggcctag tgcctctcca tcagcctgga tcagggcaaa ttccctgccc ccaactcttc 48000
ccaccagggc cggacctgaa ggccaaggcc tccgggtatg tgctcaggac tcctggccca 48060
gagaaggttt ttccctttct gcgtctttgc ttccccttcc gctttggggt tcaggaaccc 48120
cgattctctg gaagaagaga aggcctaggc tactgagctg ctctggctag agacgtcctt 48180
gagtgtctct ctctcccggg caatggtggc tgtgctctgg ggggcccagg cctgcctcct 48240
gtggctgcgt tcaaggcagc ttggaggtca tttaggccag ctcccccggg ttccccgtgg 48300
gctcaaatca ggacccagac ccttctctct tgccacagct ctgggccacc tgggactcgg 48360
gacttcctgt ctcccttcct actctgtcct aaaccattgt tcagaggcct gccgcctcca 48420
agaatcgccg agggagaagc tccaggggaa ctggcctgcc cgcatcccca ctgccagtag 48480
cgctcagggt cccaggactc gggtcttgcc agaccacagt gtcccctggg accctagggt 48540
ctctggctcc gcactgtgtt gcgccagtgc gcctggattt cccgtgagct ttcggccaac 48600
cacgctccgg gcttcccgcc tggctccgca cctgcggcgc tctcacctgc ggcggcgccg 48660
cccgaggagc ctcccagccg gcgaggagcg aggcttctgc agtcctgctg tcccgcagcc 48720
ccgcagcccc gcagcctcgc agccccgcag ccccgcagcc ccgcagcccc gcagccccgc 48780
agcccagcgc cagagcgctg cgcggagact ccttgccgcc gcacgccctg ggccggtaag 48840
aagcatccgc ttcaaatccc gcccagcggg cagcgcaaag cgagcggttt ggggaaatgc 48900
cgaagggacc gggaaaaaca aactccaagt cgagtttaac agccgaaacg ctccgtgcct 48960
aggccggcgg acaagaagga gacagaacag atcccttggc tccctcgcag cgatcgatcc 49020
cgtttaccga ccagagtcac caccccgccc ccagcccgcc aactcatacc tcccgcaagc 49080
caccagtttc aagggatgcg gaagccaagc accccgtggt ccccagcacc aggtgacccg 49140
cacgtgcact caccgtagca gggaacctgc aaggccgcgc tgtggccgct gctgccttcc 49200
tggagtttga ggctgccgtc cgggggcgtc ttgcaggcgc ctcgctgcaa gtaaagatgc 49260
ggttgcgcgg gcggctgggg ctgcggctgc tgctgctgcg gctgcggctg cggcggcggc 49320
tggggctgcg gggtcgacgg ctggggctgg aacttgttaa aggagccccg cgccccagct 49380
ccactctcca ggggtgtcgc caggtcctgc tgcccagcgc cgtaacgggc ccggctcttg 49440
gcgtccccga atccctgtgc tttggcggcg gccgacagga aagttgtgcc gaacttgtcg 49500
cctccgggaa atgccctaaa aggcgacgag ccctcccgac tctgcgacac cgggctgtag 49560
taggcgtcca tggcagcggc cggcgactcg cagtaagaga cgcaagtctc agcattcatg 49620
cctggcttgc gcaggcggcg ggcggggacg cgagcgaggg cgcgaggacg ccaccgcgcg 49680
ccttggctgg gagtttgggg aggcgaggag gctgtggctg tgcactcctt gcccggcctg 49740
ctcgctctcc ctcctcccct ccacgccggc ctctctctct ctccccctct tctttctctg 49800
tctctctcct ctctctccct cctctctctc tcttcccctc cctccccccc ccttcctctc 49860
tctctccctc tctctctttc ctccctcccc acagctagcc gagggaaaga acggagggca 49920
gtaaaaagat tcagatgttt cggaaagttg accagatctc ccaaaccctc ttaaggtttt 49980
gcagcggaaa aaaaaaataa gtaaattttt ttccctgcct tgcttcttcc tttccccctc 50040
tctcgtcccc tcctcctctc tccttccact ggcccctgcc ccctcctctc cactcttaag 50100
gagatgctac taactcgcta gcggggattc aacccggagg cgcccgaggg cttttccgat 50160
tctaggcggg gctggggaga tgccaggatc cccagggctc ccgaggctag gctgagaatt 50220
ggcggggtgt cggagctttg ccttagggca aagggaaatg caaattttaa tgacaggtta 50280
aaaaaaagcc ccccaaaata aacctaacag gttttgacat tcgaagccag ggggaccacg 50340
ggcctgcgga tgcaagcgag tcctacggac tcctgcgcgc gagctcgcgg gcgccgctgc 50400
gggccttccc gctggcacct tttcctaggt ttccaacctt cctccccatt cccgttccta 50460
cgggagatta acacacacac aagccttcgt cgtccagtgc atggtcactt gcacgtcttg 50520
ttaactattt ccctctgctg taagggcgct gggctgggat gtagcggaat ctgtgctccc 50580
tcaagccctg gagaagccct gggctgggcc cggcttcaga agctttagag cgctgccctc 50640
tgcgctcccg tctagaggcc gcaccgttca cgccccacgt ctgcgcccag gcgaggggat 50700
ttcctgccag gcgaagtaca gagagcggac tctggagacc ccctgacctt gcccgatagt 50760
cgcgctgtca cctgttcact ccttggggtc aggggtcggg gagaggggtg agaccgtctc 50820
ttccctttct ggcctcactg cccccgactg taaaacgaac tgcttggttt tgataggttg 50880
tggtttcacc accactgcga gcctcaaggc ccctggcacc gccacagcaa gggaaaaaac 50940
tttgcgcgtg cagcccctcg atttcgcttc atgacaaccg tgctagagat tggcgccccc 51000
aattttctga tgagctaacg gaggctcaga gaggggcggc cacctggtcc aggtcacaca 51060
gctgggaaat agccgtcgcg ccctggaacc tctgctcagg ctagtccctc caccgacctg 51120

```
gaaggccgtg cggacagcgt agatgcagag gggaaactgg cccccggctg tggaatggtt   51180
caagttgaag gagaccagcg agagcagcct tccttaaacc gctgagaaaa tcgttaagcg   51240
agaacgtacc tctagggagt ctgggagtag ggtgagaggt aaaagtgcaa ggtaaaatta   51300
aagagggcct gagcaccgtg aaccaaggaa tgtgggtcag atgtcaactt tctgcatcta   51360
aggtctccca aagaaaaaat atacctgggg ggtcgcgctt ctgctcccaa tctcatctgg   51420
gctaattctt acccccaccc taccccctta gtatctctgg tccttgctcc gccgccgacc   51480
gcgcgatccc acatctggtt tccatcgccg gcttaccttg aagtgctcgt taataaagat   51540
attccgcaaa ccaagccagg agcgcgcagg ctccagccgc cccccactcc atgcccaatg   51600
ggaaccgcgc ctcactggcc cgcagaccta agccacttga gagtccttta tttattgagc   51660
tcctgttgtg ccaacaatct ggcaatacaa ggcttatcat acccattttg catatgggta   51720
gactgagact caaccaaaat aattttccag ttagcaaaaa gcggagtgct cctgccatca   51780
tcctaaatgc tcctgtccag cggtttcttc aagttcctag aacctgccaa ggtctctagc   51840
ctgtgcactg tagcataggt ggtttcccct acctggaatt ccctaaactc attcctgcac   51900
ttttgtctgg ctaatgattc agagttcagc ctagagcatc ttcctctagg aagacttccc   51960
gtctctccta gtttaagttg acgcttccgt ttgcctcatc tccgtcccag atctcgtggt   52020
tgtctggttg cctggctatg agtgtatctc aaccccacta gttgccccag ggcagcctcc   52080
tacgtgcctg gcacccccaca aatatttgtg gaataaaaga ttaaacctgg accaggcgcg   52140
gtggctcaca cctgtaatcc cagcacttta ggaggccaag gaggatggat cacaaggtca   52200
ggagatcgac accatcctgg ctaacacggt gaaaccctgt ctctactaaa aatacaaaaa   52260
ttagccagac gtgatggcag acgcatgtag tcccagctac ttgggaggct gaggcaggag   52320
aatggcgtga accagggagg cagagcttgc agtgagccga gtttgtgcca ctgcactcca   52380
gcatgggcga cagggcgaca gggtgagact ccgtctcaaa aaaaaaaata aataaataaa   52440
aataaaataa aataaagatt aaacctgggg ctgtaggact ctatcacctc agtgtattcc   52500
aactgctcag tgcagcacca agttctgaga ctctttcttt ggtttccctt agtcaaggac   52560
agctggtcac ccacccaccc cctcttcaaa aatcaagtag cggaattaac agcgtgctgg   52620
accttc                                                              52626
```

<210> 2
<211> 221909
<212> DNA
<213> Homo Sapiens

<400> 2

```
aaaaccccgt ctctactaaa aatacaaaaa ttagccggtt gtggtggcgt gtgcctgtaa     60
tcccagctac ttgggaggct gaggcaagag aattgcttga atccgggagg tggacgttgt    120
agtgagctga gatcatgcca ctgcactcta gcctgggtga cagagggaga ctctgtctca    180
taaataaata aataaaccag gtgcggtggc tcacacctgt aatcccagca ctttgggagg    240
ctgaggcggg gggatcacct caggttagga gtttgagacc agcctggcca acatggtgaa    300
accctgtctc tactaaaaaa gaaaaaaatt acaaaaaaaa aaaaattagc caggtgtggc    360
gggcacctgt aatctcagct actcaagagg ctgaggctgg agaatcactt gaacctggga    420
ggcagaggtt gcagtgagct gagatcgcac cattgcactc cagcctcggc gacgagggca    480
aaactccctc tcaaaaaata aaaaataaaa ataaataaat aaaaacaaaa tttgaggctt    540
aaacaattaa gcttcgtgca tctctttaat ttttcttttt ttttttaac tctaatttca    600
tgacagatta agcttcatgc atctcaaaaa tgtgtagaac cctcattccc agccttccct    660
ccctctctct tgtccttctt tctgatttct tggaatcgga aggaaaattc ggatatccag    720
gaaccatcac acaggaggca cccatgtgat gtgtccaaca tcactccacg caggtgtcct    780
tgggtcctgg gtgcccaggt tcctgtgaat tgtgacccca ggcagaagcg tgagcatccg    840
cagaggaacc cacctaccca aagccaggga acagccaggg ctggcatcct cagggtgact    900
gtgctccagg gcacctctga cttgtgctga gtaaaatgga caaagccagc aatgcaccca    960
taacccagat tcttgagaca ccgctggact ttgggaatcc caggcattag gctcgtggca   1020
gtgtcatcat ggctaggctg gggcagtgt catcatggtg cttgctggcc aagtcaggac   1080
acggcctaag ccggaatctg ctggtgctgg tacaagcggt gagtgggcgt gtgggtgggg   1140
gttgcggtta gggtgtggac atcccagaat ccaggacgct gttcacaatt cgtccatact   1200
ccctgctccc ctcccttaca gcacttccct cccgaggcct cctgagcctc taaccccccag   1260
ataaggaaag gggtggactc agttgaagtc cagtgtccac tccggtccaa tcagctctac   1320
ctggggtgag gggaaactcc tctggctggg ctgctccctc tgcagggctg tggctggaca   1380
gattactcaa gaaaggaagc aggtgggccg gaacgaaccc acggtgccac ataggcctcc   1440
```

```
gttctcatgc atataagttt cccacactca ggcaggaaat gaaaatgctt cttggacccc 1500
caaggagagt aaaggagagg ggaagaggag gaggattttt cggggctgtt agtgacactt 1560
tcagcctcct gtgcattttt gggagggctg caggagaagg caatgctgcg cccctaccca 1620
gaactagcga gcgcttccca actggcctcc cgccttcctc tcgtcctcta cggcctccat 1680
tggcagctgt gcaagggccc ggccctgcct ctgtctctcc acactcgctg tgatacctca 1740
ggcctcccag gctccgccac gcccttccct ctttcgggaa cagctgcctt ccatcacctt 1800
ggctgcctgg ctaattcctg ctcattcctt aaaaccctgt tttgatttcg cctcaataaa 1860
actctgacac caccctccag tcataattag tcacttcttc ctctgtacgt tggaaacacc 1920
gctttatcgt ggagacttta aggatatcca agtagagaga attcttccgt gcaagttttg 1980
catcctctca ccactgcctg cgttacccga gttgtaaagg gcggctccct gtgtctgccc 2040
cgctgcaccg atacaccgag ctgcgcacgg tgcccagcgc agggagaaca aatgatcatc 2100
tgtccaacgc gcccatttac aggtgaggaa actaaggctc caactcaatc gacgcactct 2160
gcccttttga ttaccagaaa agtagcagga caggtgtcct gtcccgccct accccggccc 2220
actaagccgg caccccggct ccgacccccg gctgtgcccg gcgccgccgc ggtgcccggc 2280
gccgccgcct cgcccggcgg ggccgcccgg agcgcccgca cctccgcccg cttccacctg 2340
gccgggcccg ccccgcccgg actcgggact gggaagtgcg gcgactcccg gaaccagcca 2400
ttggcgccag cgcgggggagc tgggggtgca gagctgcggg cgcggcgggc cacgcaggcg 2460
gcccccaccc ccggcctggc ctggtctggt ctggtctgcg ctgccgcgcg gggcgcccc 2520
ctcccaggcc cggcgcccgc cagccccgct ccgccaggtg cagcgcagcg caggggtggg 2580
cgggggtggg gctcggcgcg cacgttcacg gggcggggag ggggcgggtc aggggcggga 2640
ccacagccgg ctgggccggg gttctatgcg catctccggg gaggggcggg gcgggggcgg 2700
ggccggggcg gggcccggtc ggtgcactcc agacggcggg ccgccccctc ttcccgcctt 2760
cctactaccg gcccaggatt agcgccctgg gagcgcgcgc cccgctgcct cgccgccaca 2820
ctttcctggg agcggcggcc acggaggcac catgaagaag tcttactcag gtgggcttcg 2880
cgcccggggt ggggaggggt cggtgtcccg ggaccagcgc tgctcacctg agtgcctgcg 2940
gccgggagtg gcgaggcgcc cccggagctg agcgagtccc cgcggcgggc acactgcagg 3000
tcgagttcct cccaggacag ggccgctgtc gggccgcttt cgacctgagc cgaccgtccc 3060
ctgcgctgtc tccagccctt gctcgagtgt cggaggggct gccctggggg acgctccctc 3120
ttcctcgccc cttgcaccct cgcaggaatc gctgactttc caggtcggcc gggtgctttg 3180
ggtccctgtg cgtctgtgtg ggtgaatggg gtcggggcta ggtggagggg tgtccttggg 3240
ttcagcctct agggctggtg gtccaggccg cagcatcctt tcttcggatt ctcttcggtt 3300
tctcctctac ttagtggggc acgggacggc ctccagatgg gaccgtccag cagcgcccaa 3360
acttggcgac tcgggttcac gttttgcgct caggacgccg cccgcggctg acatcctcac 3420
tccaccctag cagggcccgc cttaagtagg cgaagggggc ctgacttggg cctcaccatg 3480
agccggttca ggagctttca tttcataaga ggctcccgct ctggcactct gaagttccat 3540
ttcacagatg ggcacaccga ggccacaagg gcaggttaag agaaatccag atcttcatta 3600
tttgtctttt ccttcctttt ttttttcctt tgtggttttc tccaaaggat ccaggcaggc 3660
aggatccttt gtcctcctca gtcctcaaat tataaatagc gtctcctctt ctaaatctag 3720
aaaccgtttg tttaatgtcc ctcctttaat tcattcagat agaaaggtac ctgccaagga 3780
tccatgaatt aatttttatt gataggagaa cagaatcatg ggccttagtt ggaggtgaag 3840
gagtcatcgg ggccatcccc ctgcctttgg gccattgtcg gtttcctctg gctttccata 3900
tttaggaacc aaaatgagtg ataaagtacc ttgtctcaaa gaggattact cagaacttcc 3960
ccgaaaatac ccttctgggt cagtagctta gaatttcaac tttcacccgg ttgttgcgaa 4020
caagatggcc ctctacgcac ccggagcaca gctctgtcag gagcgtgggt gggttggtgt 4080
gctggtgggc gggagcgggc ttccaggtgg cacatgcttg gttttggcac cggggcttcc 4140
tccccaacat gctccaaata cttgagattg ttgtggggat cgcgcggtgc atggcccttt 4200
ccaggggatc tctagcccgc ccagggaggg ccctttgccc ttggtttctt acactttacc 4260
tcccactggt gggtccagac agtgggggtg accggggtcc tcccaggtcg tgggtcctgg 4320
gtttttttctg cggagaccct cctgcacaga tgccggaagg gccctgaggc aggcttggag 4380
tctctgcacc tgcggcttac tgggaggtgg ggagaccatc acttgtcctt ttctcctctc 4440
ctccacacac acctacccca cccctcactg ggtttctttt tttttttttt ttttaaaaaa 4500
aaggacagaa ccttttgcag taggtggaag tagagtttgt ggagtgagca ggaaccaact 4560
cttgtacagc agcctcggtg cctccttgtg tttacctaaa ggggcaggcc cctctgagga 4620
tgggggaagg gagggctcag accatttctg gtttgctccc tgcttggcag tggcccaagg 4680
tgtgctgggt cactgtgttg cagttgtatg gagtgggact ggagggggct gatggtgggg 4740
gtggcctctt gccctcattc tgagtgtcct ggagctgtct tggcaccagc cctttttccgg 4800
gtacgggcac tcgcaggagt caggctgacc ccccaacaag ccatcgacac catgaaatta 4860
aggaaagcag caaaagatgt taaaaatccc ccaaggctct gacaatccaa ggacaacagg 4920
```

aaggtcttta ttttttcatc tagactgtgc gggattattg cttccagacc tgtatgtggg 4980
cttcagaata aacggaatga ccataatgtt gttcccggta gacctggttt gaacattcgt 5040
gagcctctga aaaggagaaa gattttagaa ggatgtgttg gggcaatctc aagtaccgtg 5100
ggtctataga cagtcatcga agacacttta tgtgctgggc acacatggcc aagtctgagt 5160
gggtacagaa atggtaatga gatatagtcc ttgcctgcag agaacttaca ttctcaccgg 5220
gagtcaggat gagggcgaga caccagctca ttaacaccct tccctcctga gcagttcagc 5280
ctccaaatac tacaggaatg ccccggaggt tttaggtgga ggtgagattg agctgggcct 5340
tgaggaagga gaagtcttga gtagtggaga ataaaaaggg acttggccag gtgcgcttga 5400
agtcccagct actcaggagg ctgagacacg aggatcgctt gagcccagga ggctactcag 5460
gaggctgaga cacgaggatc gcttgagccc aggaggctac tcaggaggct gagacaggag 5520
gatcgcttga agccaggagt tcaaggctgc agccctgtga cagcgcctgg aaatagacac 5580
ggcactccag cctgggtggc gtcatgagac cccatctgta agaaggtctt ttccaatgcg 5640
gaaggcagca agggcccaca cagggcagct gcaggatcca gggggacact ggggacaggt 5700
tctctctggc agctccactg ctgctccagg ctttcccgat tgcctcaccc ggccttccaa 5760
tggggtggag gtagtggtag tctacaggca gtgtggtcca gtggttacac ttgggggctc 5820
agagccgcca gccccttggg attaaatctc accaggctgc gcctctccct gtggcacaag 5880
gttctcacct catgcatctc atttgcctca tttgtttttt tgtgtgtgtg gtgtttttta 5940
attttattta tttatttatt tttgagacgg agtctcactc tgtctcccag gctggagtgc 6000
aatggcatga tctcagctta ctgcaacctc cgcctcccag gttcaagtga ttctcctgcc 6060
tcagcctcct gagtggctgg gattgtagga acgtgccact gcgcccagct aatttttata 6120
tttttactag agatggggtt tcaccatgtt ggtcaggctg atctcgaact cctgacctcg 6180
tgatctgcct gccttggcct cccaaagtgc tgtgattaca ggcatgagcc accacgccca 6240
gcctgcctca tttgttaaat gggacagtgc tatgttgttg taaaggttaa acgagttcat 6300
atttgaaaaa gctgagaaat gcctgctctg taacctgtta gctgctgtca tgatcatgat 6360
tgtcattgac aggaggatgg agaaggctgg ggttacgcca ttgctaggac gttccagaag 6420
cctccatgat cttcccttac tgtggtcaga gcatggtgca gggtctctcc cccagcccct 6480
cagcactgga gccagctgca ctgtacccgc cctggctctc taggttctga gttttgggac 6540
tccctttgca tactaggtca catggaactt gccgtcattc ccaggctccc ctggaaactc 6600
tctgtgggtg gcagcggcag gaggacttac ccgaggccga tgagccacag aagcgggact 6660
gaggttgcgt cacaccccta ctggcacctg ggaattactc tggagattct tttttattt 6720
tttatttttt tgagatggag tcttgctctg ttgcccaggc tggagtgcag tggcacgatc 6780
tcggttcact gcaacctctg cctcccaggt tcaagcaatt ctcatgcctc agcctcctga 6840
gtagctggga ttacaggcat gtgccaccgt gcccagctaa tttttgtatt ttttagtaga 6900
gatggggttt taccatactg gccaggctgg tctcgaactc cggacctcag gtgatccacc 6960
cgcctcagcc tcccaaagtg ctgggatcac aggcatgagc caccgtgccc agccactctg 7020
gagtttctct gtggctttca ggagattcct gaaggggcac cagccacgat taaaaagctc 7080
tatgctgtcg tgacttctga gtcctgggca gggagggagg ctgcacccac acaggaagca 7140
gcttctgccg tgattcagtg tgctggctcg ccgtggtgga cagggctgca ccctcaccca 7200
gcgccccggg agctgactta cgggaagggc aaggaattgc accctcagag aacacttccc 7260
cagtcagcta acccggtgac aggcgagcgt gggaagggca caagcacagg gtttcttcct 7320
cttctccccc agccccgtct ccgcgcctgt tctcgctgcc tcttgcctcc tcacgtccaa 7380
gcttgtctgc tctggcgcat tcaggggacc tggactggag agaaaaagcc acctcgtacg 7440
gttagtctgt ggctctgtct gacttgtgtc ctccgccgag gcccctcagt tgtgagacag 7500
ctggctgagc ctaagtcctg gggccagccc tctccctagt ctgttgcaaa ggtagaaagg 7560
catgcccagg caggggccac ctgccctgcc cgctcacgcc agaccgcagg cacttgcccg 7620
tcagatctcc ccggcctgcc cagagggatt ctgatagccg agggcaatgc acctggcagc 7680
ggagcattgc gtttgcgaca gtccaccttc ttcctggaac caccccccac aagtgtgacc 7740
agctcaagcc aggacaggta caggggggacc caggattgcc acttaggggc agtggtctgg 7800
aatccaggtc caggtgacat gggagccaac cctctgcttg gagatcccag gtgggtgttt 7860
gcagaggaca tctcacttga ggacccatgg aaggggacac ttcccacccc ctacctggtc 7920
cttgggctgc cctcctctga cactctcctg ccatcccagg cagccctgac agccccatgt 7980
gactgggacc tagaggtggc ccctgtttcc gccccagtgt ttgtccacag gcagtgttgc 8040
gtgggctctg aagtttgcct aagatggacc tgataagggg agttcagtac acatcagtgc 8100
aggcccggcc agggggtgat gatggcagtt cctgggcaga tccagctggc tttagggtag 8160
gaggggctcc agagagaggc tccaggcccc taggggtgtg aacctttggg gtgaggcggg 8220
aagtgacgcg ctgggttcct gcgcctctgt ttgttggagc tattaccttg gaaacatccc 8280
agctctaggc ggttgccagg gatttatggg gatcagtggg tgccaatccc tgggtgaagc 8340
cctcggggct tgtacacagc acacagcgct gtggccccat ggctcacttt gctgtgtgaa 8400

```
gtggctcagt ctccgggaga gtccttagcc aggcaggcct gacggcaact gccaggtcat    8460
ggtgatctgg aatcgctgac caaagggagt cagtcctggt gggtggaggg acttggtggg    8520
cagccatcgt gatgccaggc atggggatcg tgtgtcaccc aggcctgtgg gtgccgagct    8580
tctgtgggag cagtgaagtg cccccgtggc cgatgggctg gtcacccctg cccctttcct    8640
gatgtgacag ttgtgcctgg gccccgggaa atttgccagg aggcctggca ttggcctgct    8700
ctactggagc ggccctgctg agctgtgagc tgcccgtctc cctgaggttc ctagcccatt    8760
tcaactctcc atgtggattt ggtcattcat tccagaagcg tctgccgggc gctgctgagt    8820
gtcaggttct gtttgcctcc tctcctgtac gctggaggct ctgggcacca gctctgcagg    8880
gtggcctggc cttgggtctc cagggtagag catggcactc tctgctccca gcccagtttc    8940
tctctgcttt atctccctga ccccaccctg ggcatgggcc catttgcagc ccctgtgaag    9000
ggagacattg agctgttgct ggcagaggag gggcggctcc tgggcactgc ttgccaatgg    9060
cctctcggca gctctgtgac agctgagctt tgttcccagc ctaatctgac ctggttctcc    9120
aaagcatgac tgttggcagc ctgaaaacct ccaggacagg aaacaggagt ggcgcaggga    9180
atgtaagatg atcccagctt caagtcactg ggcggccctt agacccaggc tgcctgctgt    9240
ctctggcagg aactcaagtc gctggtcatc ctcagagcgg tgttggcccg gggccttcag    9300
tggcctttgt gtctgggtga gaggaaccct ggatggccac tctgccctga gtgtgtgggt    9360
ccccagaagt gctgggttag ggggcacgga gggccagaaa gtcccctttg gagcgctgga    9420
ctctctcgct gactcctacc ccaccccggc ctggggtttc agagaagggg tccaggcagg    9480
agtgtcatct ttctcaatg  ggatgtggc  ttcagtctct gtccaggtgg gtgcattgct    9540
ctctttccgg ctcctcccag gctgcttaaa tgacctgtga cgaggcctgc gccatggccg    9600
gggccctctg cttttcccag cgctgcacct gggaaccagg ggttgggccc cggaagccga    9660
ggatgtccct gaatgccatg cctgggcacc tgtcctggcc aggcgaggcg tgttggggca    9720
gcggctgaga tgggagcagc agcattgcct gccaggtttg agatgggccc cgggtgagtg    9780
tggttaccag gaagggctgg gcagagcagg cgggtgggcc gcccctgggg ccttgttccg    9840
cagatcaacc tgatccgcac cctggggccg gggcttcctt tcacagcctt cccatgaact    9900
atggctcctg tttccatccc aaatcagatc ccagaagagg aataatgtcc atgtaaccca    9960
agccagttgg aagtgggtta catccaaccc ctgtcatctt ccagcttgtt aaggacttga   10020
ctaactcaac ccagagggat ttggagggtt gtctgcaaaa cccactaata acagggaact   10080
ttagctctgt agccacaggg tctacagagg tgtcgggctg tcgggttatt gctgggaggt   10140
gagccggtgg cgtagacacc atggaggcac cggtagtgga agagctgcaa gtgcacacag   10200
aggccggcac cgagggggcgg ggaggaggag gtggtggatt tctcgccagt gttggcatca   10260
tgttgcctcc agcatcagag acccaaggtg tggctgacct tggctggggc gatgagtcat   10320
tggatagcag agccttcctg gtagctgggg gttaacgctt taactgccgg gactttgtat   10380
tatgccgctt cttttttttg ttttgtttgg tttggtttgg tttggttttt gagacggagt   10440
ctcactctgt cacccaggct ggagtgtggt ggctcaatct tggctcactg caacctctgc   10500
ctcctgggtt caagcaattc tcctgcctca gcctcctgaa tagctgggat tacaggcacg   10560
cgccaccatg cctggctaat ttttgtattt ttagtagaga tggggtttcg ccatgttggc   10620
cgggttggtt tcaaactcct gacctcaggt gatccacctg ccccggcctc ccaaagtgct   10680
gggattacag gcatgagcta ccgcgcccag cttgcattta tgcttttttt tttttttttt   10740
tgagatggag tttcgctctt gttgcccagg ctggagtgca aaggcgcgat ctcagctcac   10800
tgcaacctcc acctcccggg ttcaagcgat tctcttgcct cagcctccca agtagctggg   10860
attacaggcc tgcgtcacca tgcccggcta attttgtatt tttagtacag acggggtgtc   10920
cccatgttgg tcaggctggt ctcgaactcc cgacctcagg tgatccgccc gccttggcct   10980
cccaaagtgc tgggattaca ggcgtgagcc accgcacccg gcctatgctt tttattatta   11040
ttatttaatt ttttaaggaa agcaatctaa actgtacact gtatcttttc tttctctttt   11100
gctttttgtt gtcagttttc tgtattactt attgccaaac aacatgaaca ggaaattgaa   11160
aggaaaactt caacaatcat tggctgtagc atttcttctg tgttccattc agtgcttgtc   11220
tcctcaggaa catgacatag ggtttgattt aattatgaaa ggcgtatatt ttcagagttt   11280
tatactcccc atagaatgaa tgttgaattc tgttcttttc acttgaaatt agatctcagg   11340
catggatttc atgattatca ctataatggc tcttcagagt ggacattgca tcttgtaggg   11400
aaccaccctc tccccagctg ctggtgtgga cgctcctcct tgatggtgtg ggtctgggcc   11460
cctgaagggt ggtgtcctga gtaaagatgt tacagtagcc atgcttatag ctttgggcct   11520
ggggctaggt ggagactggc ttcaacccta gagcttgctg tcttaaaata aattcttttt   11580
tttttttttt tgagatggag tctcgctctg ttgcccaggc tggagtgcag tggcgcgatc   11640
tccgctcact gcaagctccg cctcccgggt tcacaccatt ctcctgcctc agcatcccgc   11700
atagctggga ctacaggcac ccgcccccac gcccggctaa tttttgtat  tttttagta    11760
gaggcggggt ttcaccatgt tagccaggat ggtctcaatc ttctgacctc gtgatccact   11820
cgcctcagcc tccaaagtgc tgggataaaa taaattctta gggccgggag cggtggctca   11880
```

cacctgtaat cccagcactt tgagaggccg aggcgggcag atcacaaggt cagaagatcg 11940
agaccatggt gaaaccccgt ctctactaaa aatacaaaaa attagctggg tgcagtggcg 12000
ggcgcctgta gtcccagcta ctcgggaggc tgaggcagga gaatggcatg aacccggaag 12060
gcggagcttg cagtgagccg agattgcgcc agtgcactcc agcctgggcg acagagtgag 12120
accctgtctc ataaataaat aaataaataa ataaataaat aaattcttat acccacttgc 12180
attatttatt attattatta ttaaacaggg tcttgctgtg ttgcccaggt tggagtgcag 12240
tggcgtgatc atgacctccc agtctcgacc tcccagtttc aattggtcct cccttctcag 12300
cctcctgagt agctgggact acaggtgtgc gctgcatgcc tggctaattt tttttttttt 12360
tttttttttg agacagagtc ttactctgtt gcccaggctg gagtgccgtg gtgcaatctc 12420
ggctcactgc aaccccccggc tcctggatta aagcaattct cctgcctcag cctcctgaat 12480
agctgcgatt acaggcatgt gccgccacac ctggctaatt tttgtatttt tagtagggac 12540
ggggtttcac catgttggtc aggctggtct caaactcctg acctcgtgat ccacctgcct 12600
tggcctccca aagtgctggg attacaggtg ggagccaccg cgcctggcat gcctggctaa 12660
tttttaaaaa atttttgtag cagccgggcg cggtggctca tgcctgtaat cctagcactt 12720
tgggaggcca aggaggtcgg gagttcgaga ccagcctgac caacatggag aaaccctgtc 12780
tctactaaaa ctacaaaatt agctgggcat ggtggcacat gcctgtaatc ccagctactc 12840
aggaggctga ggcaggagaa ttgcttgaat ctaggaggcg gggggttgcag tgagctgaga 12900
tcgtgccacg gcactccagc ctgggcaaag agagtgaaac tccgtctcaa aaaatttttg 12960
tttttgtttt tgtagcgaca gggtcttgtt ttgttgttgc ctaggctggt actgaatttc 13020
cagcctcaag cgatcctcct gcctcagcct cccaaagcgc tgggagtaca ggcgtgagtc 13080
actgtgcctg gcctccactt gctttggctg cctttctctg tcttaggtaa ccatacattt 13140
ctccaggtaa ttaagaaaag attgcgcatg atgagtccct tttctggtat ttctgggatt 13200
gcccccaaaa ccagacaagt gttcttacct tgacatttta ttaagacttt aatgaacaga 13260
atgccgtttg ccctcagtct gctggtcccc ctgcgaaggc ccagggcttc cttgtttgaa 13320
aagtcatccc cttgtcgagg tctttgattc cttttcacca cggcagtcat gccgagggtc 13380
cacagagcca cccccttcccc atctcttcct cggcccctca agacgcatct gtacccccgg 13440
agatgtctct ttgttttctt gggccacctc tcctggccgt cactcaccct gctgactcat 13500
tacttgtgca acagtttctt ctggaaccct caggctcctg ccacctaccc tctgcagtcc 13560
ccgttttgtt cctcttgcct cagtcttcct ccccatcacc cctctacctg ttggtctcca 13620
tctctgtcta tggcacatcc ctgtgttata ctctgatggc tggataaagg ttgagtgaaa 13680
cgcatctacc cctttggtga ctggggtgcc cattaatcag aagggatcca aggctggcgt 13740
agccttttcc cgcatcctgg ggtatcgtcc tgtggctctt ccttgttgga gtctgcagtt 13800
ttttgttttt tttttttaa atttttgag atggagtctc gctctgtcgc ccaggctgga 13860
gtgcagtggt gagatcccag ctcactgcaa cctccgcctc ccgggttcaa ccaattctcc 13920
tgcttcagcc tcccaagtag ctgggactac aggcgcccgc caccatgcct ggccaatttt 13980
ggtattttta gtagagacgg ggtttcccta cattggccgg gctggtctcg aactcttgac 14040
ctcaggtgat aggctcgcgt cggcctccca aagtgctgag attacgggca tgagccacca 14100
ggcccggccg ttgagattac cttcataagc caccaggcct ggctgtagtt ggcagttttt 14160
gaccactggt ctcgctccac gtgatctgtg atcttcccct tggcccccctg gccctgccct 14220
gttcatctgc tcacctttct catgcatgcg gtttgttcat agggtatgta atggggtcta 14280
accaggaaat ggaaaccgct tgagcattta tttatttatt tatttattta tttatttatt 14340
tatttttccc aagacaaggt ctcactctgt cacgcaagct gaagtgcagt ggtgtgatca 14400
cagctcatgg cagccttgaa ctcctgagct caagcgatcc tcccttctca gcctcccaag 14460
tagctcggac tataggtgca cactatatgc ctgcctaatt ttttctttta tttttgtaga 14520
gacagggtct tgctttgttg cccaggctgg tcctgaactc ccaacctcaa gcaatcctcc 14580
tgcctcagcc tcccaaagta ctggagctac aggcatgtgc catcacgcct tgctaatttt 14640
tgtatttttt gtgtgtgtgt aaagacaagg tcttactatg ttgcccaggt tggtcttgta 14700
ctcctgggtt tatgtgatcc tcctgcctgg gcctcccaaa gtgctgggat tacaggcggg 14760
agccacagtg cctggtccac ttgagcattt agaacagaag gacttaaagg cggggaacta 14820
gttacacaga tgatgggaga ggtgaaggcc agacagggaa cagagaggca atcagcagga 14880
aatcaaagtg gtgtgggctt atacagccct ctaaaggtat ggatgcaatg attcatttag 14940
aatgagaaaa taaataacag caaattataa attataagca gctgttaaat atcacaaaca 15000
tcactacatg cagaagagta ccagtcaact gtcagtcata tctccaaagt tctatttctc 15060
ccacattttt ggcattactt cttcaatatg atgactttgt aatatcgttt tctatttcga 15120
gaacagaaaa ctgatcaggc tggacacagt ggctcgtgcc tgtaatccca gcactttggg 15180
aggccaaggc aggtggatca cttgaggcca ggagttcaag accggtccgg gcagcgtggc 15240
gaaaccccgt ctctactaaa aatacaaaaa ctagcagggc atggtggcat acacctgtag 15300
tcccagttac ttaggaggct gaggtgggag gattgcttga gcccaggagg tcgaggctgc 15360

agtgagccgt gatcatgcca ctgcactcca gcctgggtga gagagtgaga ccatgtctca 15420
aaaaaaaaaa aaaaacaaca aaaaaaaaca taggctgggc gtggtggctc acgcctgtaa 15480
ttccagcact gtgggaggtt gagatgggcg gatcacttga ggtcaggagt ttgaaaccag 15540
cctggtcaac atggtgaaac cccatctcta ccaaaaacat aaaaaattag ccaggtgtga 15600
tgacgcacgc ctgtaatccc agctacttgg gaggctgagg caggagaatg gcttgaaccc 15660
aggaggcaga ggttgcagtg ggccaagatc gtgccattgc actccagcct gggcaacaga 15720
gtgagactcc atctcaaaaa taaataaata aataaagcga atgaatctcc ttagttgatc 15780
aaattttgtt tttatttttg gtagttggga tgtatacaaa agtggcggtc aacatggagt 15840
tggtggtgct gctacaattg ttttgttcaa gatacacagg aattctggta aatcctgttt 15900
ttttcttttt ttttaaatgt gattcccatc aagaaagaag taggaaaatg ggggagattt 15960
ctaactgcat atgaggcatt tgtgcatgta tttctgacca gagagacttt tctgttttga 16020
ctccacattg aaggaactga atcctttgct tacaacttca cctctggtga tgggagtaat 16080
tttccacaga tttgcttctg gacccgtgca tttcaaatct tgcttctctc cactgcgcac 16140
gtttttccgg cgccaggcac cgcaggatat gttcgtgtct tgtgattttt gattctgcgc 16200
ggtaggtgga attggaacgg tgagtggagc gagtattgct ggaatccgtt tccacaccgg 16260
gatagcgagc agtaactgaa ctatacatgg aaataaatgg gaactacata aatataaccc 16320
gaccaaagcc aaactaaata tctttccagt tcaacttctc cttagcagga gcccccacgt 16380
ggcagcaagc ctccgtttcc acctcacaca agagaaaatg tgacgggcag gaactcaaga 16440
ctggaaatga acggggacgt taattgattg tagggaaaat attttccatt ttgtaaattt 16500
tacaaaaaat gtttgaccct gtggacatgt ggttagggcc cttctcagtg ccttgagagg 16560
gaaccatgca ggtagaggga gggttcttag cattgtgggg gtgacctgga gattggcagc 16620
agggtgccac caccacccct aggtggggat gagagggagg aagtcctgtg accagagtcc 16680
aggggccaga gtcacctgct gggagcccaa acaaaggtgg gactctggca ggagctggaa 16740
ctacacagag gaccacctct cctgctggag aagctgtctg aggcagagga ggtggagagc 16800
tcccgcgccc tcccttgctt ccacctccca ggttcctccc aagcctctcg ttggttgaac 16860
ccagcccaaa gccagcaggc ctggggacct ggacacctga gccagcagag gtcggcgttt 16920
ctcccacaca aaggagggca caagaagtgg gaggcacgga gggggatcag gccaggaccc 16980
acacaggatg tgcaaacagg agctcccctc tcttcccacc ccaaatggca tctgcagccg 17040
gcctcacttc cctgctgtta gacaggaaga ggaaccttgt tcccggcacc agctgccccc 17100
accgcccccc accccacgga ggcccctttc ctctctggcc tttgcttgca atcattggcg 17160
acgctgatgg accatatctt ctggggagtt tggtcatgaa catggtttcc catttgttgt 17220
ggaccaaact gtgcccttcc ccttgaattc ctgcgttgaa gccctaactc ccaatgcagc 17280
tgtaattgaa gatagggctt ttagggaggt aattaaggtt aaatgaagtc atagggcagg 17340
accctaatct aataggattg atgtccttat aagaagaggg agagacacca gagatctctc 17400
ctccccccctc acttgtaaac agaggaaaaa tgacggagga cacagcaggg aggtgggtac 17460
aagccaggaa gaggggcctc gccgggaact aaccctgacg gcaccttgat ccgggacttt 17520
gggcctctca aactgagaaa tacatgtctg ttgtttaagt cactccctcc acagtaatct 17580
ggatggcagc ctgagcttac caaggcaccg tctgcatcag ccagggctct ccagggagac 17640
agccaatagg agatggatgg atggatggat ggctagatag acagacaagc ggacagagag 17700
agagagagac aggcagacac agacagacag gcagacaaat ggctagatag acaagcagac 17760
agagagatag agacagacag acacaaacag gcaaacagac aggcagacaa atggatagat 17820
agatgataga tagccaggca ggcagataga cacagacagg cagacagaca gacagacaga 17880
tgataggttg ggttcagtga ctcttacctg taataaccag cacattggta ggctgaggcc 17940
ggtggatcac ttgaggccag gagtttgaga ccagcctggc taacagggtg aaaccccatc 18000
tctactaaaa atagaaaaat tagctgggcg tggtggtgca tgtctgtaat cccagccact 18060
cgggaggctg aggcacgaga atcacttgaa cctgggaggt ggaggttgca gtgagctgag 18120
atcacgccac tgcactccag cctaggcgac aaagtgagac tccatctcaa aaagaaaaaa 18180
aagatagata gatgaatgga tagatagcta gatagatgac agatggatga taggtagata 18240
gataattgat aaatagatat aaatgaccga tggacaggca gacatagata cattgattag 18300
gtagatgata gatggataga tagaagatag atggtggatg ggtgggtaga taggcagata 18360
gacagttaga aagatagatg atagatagat aggaaggaat ttattagggg aattggctca 18420
taccattgtg gaggccaaga tgtaccacag aaggccgtcc gcaagctgga ggcccaggaa 18480
agccagtatc gtggctcagt ccaagtctgc aggcctcagg actgggaagc tgttgatgta 18540
actcttagtt taaggccaaa ggtttgagag cctggggaac tgctggagaa ctgggagttc 18600
taagtccaag ggcaggagag gaaggcatcc cacctccagg agaaagaggg aattcacctt 18660
ttctctgcct ttttgttgtc tggcaatgga atggtgtcat ccatattgag ggaggatctt 18720
taccactcag cccaccaact cacatgccca tctcctccag aaacaccctc acagacacac 18780
ccagaagcga tgcgttacca gccatccctt aatccagcca aactgacacc tgccgtcagc 18840

caccacacag gctcagaaga atcgtggttg catggtgatg gtctccatcc cgtctaaaga 18900
agttgaagat gaactgttgg cacaagccat cgttctttca ggctccttcc agtcgttcat 18960
tcttttaaaa atgtattcat tgagcacctg ctgcatgcca ggtgctgtga caagcactgg 19020
ggcacagcag tgagcaaaca aggactcttg tttcttggag tttacatgca gtagaaggaa 19080
acagaatcaa tgaattcatc agttctagaa actatcaaaa taggactaag agctgtggtg 19140
agaattaaga taaggtgatt gcatagaaag tgactgatgg ctgctttgga atgagtggtg 19200
aggggtcaca gagatgatga cagctgagtt gagattggga agacaggaag gagccagcag 19260
tgcaaggact aggctggaag caagcttggt gcattccagg aacagcaagg aggccagagg 19320
ggctgcagta gagcaggcga ggaggaggag ggagacgcag gtcaaagagg agcctggggc 19380
cagatggagg accctccctc caaagctcag tcatttcctc cttaaaggag aatgtgttac 19440
ctccaaaagt cacatatgcc agcacagtgt ctggctcatc atatgccttc agtaggcggt 19500
gagcatggct gttcttgtta ttactacatc acaattacta ggaataatgg agtcaccagg 19560
ctttgcattt acagcaatgt gagggagtca gcaagcttcc ctggggaacc catctcccct 19620
cactaagttc ataagagcct gataaccagg tcaatgtagt gagagctata gctgcccagt 19680
gtactggact ctttagccac gggccaggac actttcatct cagcagaaac ctctaagggg 19740
actggggagt gtgctgtgga tgtttctggg ttactcagac ctcctgggga gccttcccca 19800
ccgcagctgc tgcagggagc tcagctgcag tgatgaactg cggctcagcc ttgtttatgg 19860
gaaaacccta catgggagat gggacggttt cctgttggtt ctctcggcct caggttgctt 19920
tcacaagtgc agggattctc acctgggtgt gtgaatgggg ctcaggatgc cccaagggta 19980
tgtggagatg ggggtcagct gtcttctctg atgcctctga acctcctccc cctgcctttc 20040
tgcatctcat tgcttcctgg cctctctcac tttgggtgct ttggaacaac atgcaatcca 20100
atacacaggc tgactttatt attttgtttt gttttatttt atttatttt ggaaacaaag 20160
gctctgtcat tcagcctgga gtgcagcggt gcaattgcaa ctcactgcag ccttgaactt 20220
ctgggctcaa gtgattctct cgcctcagcc tctcaaagtg ctgggattac aggcatgagc 20280
tgctgtgcct ggcctaggct ggctttaaaa gctcctcact ctctctgcat cctgatgata 20340
cctacctcct ggagcaggct cccagccatt ggcttcggat actttgtagc tattattggc 20400
cagttgtggc atgtgaagct gaggctcagg agaagggcag tcctgtccaa ggccacacgg 20460
ctcagtgcta acactcagac ttcagtgttt ttatctccct cctggtcgct tccaccctta 20520
gtgcatctct ggaggcccaa gggagctaaa ggccaggctt tggtcagcta agaatttaaa 20580
aaagctgtct ctgtgctcct ctgtcaatcc atcactccat tatctccccc gacaatgaat 20640
tcagtttgaa tttcagataa acaaggatta atttttagta taagcatatc ccacaacaga 20700
cttgggatat acttatgcct aaaaatgatt cattgtttgc tggccttagt agctcatgcc 20760
tacaatccca gtgctttggg aggctacagt tggagaattg cttgagtcca ggagttcaag 20820
gttacagtga gccatgatca tgccactgag ctccagcttg ggtgaagcag caagatcctt 20880
atctcttcaa aaaattgttg attgtttatc tgaaatccag gtttaactga gcatcctgta 20940
ttttatatga gaaccctata taggtgagac ccagagtccc aatgcccact ggtaagcctt 21000
tgcagggagg ctctgtgggt ccttggctgg cactgatgcc ctgtgatgag cagggccatc 21060
tggcaaggtg gacgcctgct gcccatgcag gcagagcaag gccctgggca ggtatgagct 21120
gatctcacct tccccagcct gggagccaga tactgttggc agcttgccgt caagagaggg 21180
gctggtcacc aagagagggg cggcctccat gggccttctt tggcttttgg ctctccccca 21240
gccctcctcc taccccaaac agaggccaac ggcaccgcac cagaaccagt gtgctgcctc 21300
ctttggttgg cattttgtag ttgggtagac tgtttccttc tcacaggagc tggtggtgcc 21360
ccttccgctt cacagggagg atggctgaga cccctgacct tgacacaggg tgctgttctt 21420
tttttgtttt gagacagagt cttggtcttt tcacccaggt tggagtgcag tggcatgatg 21480
ttggctcact gcagcctcca cctcctgggt tctagcaatt cttctgcctc agcctcctga 21540
gtagctgaga ttacaggcgt gcaccaccac tgagaacaat gccttctaca gtgtttattc 21600
tcctgtagga gcaagttcct aaggcaagga ggttgctccc ctcatggatg aataaattga 21660
ctttgatcca cagccccacg tgtcagcctt gagggtgggg aaaggttgtt ctgtgtctag 21720
gcaggggggtg ggggcaaggc caggagggcc tggtgcccag tgtacctctt caggacacaa 21780
ctccaggtag cagaggccaa ggacagaggg gaggagaaga gggaggtggc cattatctcc 21840
ttatctcagc ttcctttgga gataagcttg agtattttag ttgtccacta agtctggaga 21900
taagtctgga atttaaacct ttgactttga tccagccctt cacagctcct gtgagcaaac 21960
tgagcctcag aaaaagggag tgaagcctgt gcatcctgga cgcccccacc ccaggctcaa 22020
accgccccca ccccaggctc aaaccgcccc catcccagct caaaccgccc ccatcccagg 22080
ctcaaagcgc ccccatccca gctcaaagcg cccccatccc aggctcaaag tgcccccatc 22140
ccaggctcaa agcgccccca ccccaggctc aaaccgcccc catcccagct caaaccgccc 22200
ccatcccagc tcaaaccacc cccaacacag gctcaaacca ccctcaaccc aggctcaaag 22260
cacccccacc ccaggctcaa agcatcccca ccccaggctc aaagtgcccg caacccaggc 22320

```
ttaaagcgcc cccaccccag ctcaaaccgc cctatcccag gctcaaaccg ctcccacccc   22380
aggctcaaac caccccccatc ctaggctcaa accgccccca ccccaggctc aaagtgggaa  22440
ggagccacag ggaaacaaac gggggaggca cggatgacca ggatgagggt ctccatgacc   22500
cacctttccc caggggctat ctcaagagct ttttaaactt ttattatttt ttaatagaga   22560
tggggtcttg ctgtgttgcc caggctgatc tccaactcct agcctcaagc catcctccca   22620
cctcagcctc ccaaactgct gaggttacag gtgtgagcca ctgcacctgg cctctctcag   22680
gagtttcaag aaaaaaaatg ggaatagatt tgtgtgtgtg tgtgtgtgtg tgtgtgtgtg   22740
tgtgtgtgtg tgtgttttga gaaagagttt cgctctgtca cccaggctgg agtgcaatgg   22800
cacgatctcg gctcactgca acctccgcct ctcgggctca agtgcttctt ctgcctcagc   22860
ttcctgagta gctgggatta caaacatgca ggctgatttt gtattttcat tagagacggg   22920
gtttcaccat gttggtcagg ctgatctcga actccggacc tcagatgatt tgcctgcctt   22980
ggcctctcaa agtgctggga ttacaggcct gagccaccgt gcccggccag gaatagattc   23040
ttttctgtag atgacttctg tcattattga aaatggatgt ttatgcacaa ttgaattgtc   23100
ataacgcctc atcctaaaaa gctccttttt gagaaaatgt gacttttgat agaggagaga   23160
ctgtccaggc ctcattcatt gtcagcacaa aggcagagca ggggtgtggc ggtgtcaccc   23220
tcctctgaga cagccccatc cctgctctgc ccaggggttc agaagtctgg tctattcgag   23280
cccacagagg ggtttggagt ggctgtctta catcacaggt ggggtctgta aggatctgaa   23340
atgacctttg agaaggcggg tgtggtgggt gcatttcaat cattaaagta acaacaatga   23400
tacatcaaat agtgtctgct cagattctga gtgcctgttt aaacatttcc atcttgtgac   23460
ttgccagatt ctagcataac aacccaggta aaagaaaaaa tagctgggcg tggtggcttg   23520
tgcctgtaat cccagcgtga ggccaaggca ggaggattgc ttgagaccag gagtttgaga   23580
ccagcctggg ccacctgaga ccccacctct gaaaataaac aaaccaataa aagaccttca   23640
tgctctatgt ctttcttata tctaggttcc ccaaattata tgcattgggg aaagtccatt   23700
aagagaagcc cctggccggg cgtggtggct cacgtctgta atcccagcac tttgggaggc   23760
caaggtggga ggatcacaag gtcaggagtt cgagaccagc ctggccaaca tggtgaaacc   23820
ccatctctac taaaaataca aaaattagcc gggcgtggta gtgcatgctt gtaatcccag   23880
ctacgcagga ggttgaggca ggagaatcac ttgaacctgg gaggtagagg ttgcagtgag   23940
ccaagttggt gccactgcac tccagcctgg gcgacagaaa aagactccat ctctaaataa   24000
ataaataaag ggagagaagc ctctgggctc ctagatacat gggtcagtgt ccacccctgc   24060
aaagctgctg tgcccaaaga ggggctgtac ttggtggaat tggggcaaaa gagcagggag   24120
ggcactgggc agttgacctg gtctccacta ctggaaaggg gctgtgctgc tggtgccctg   24180
agctggttcc acaaggaaga cactgcacag ggcgccctaa gtttgctctt tctgcctagg   24240
ctcacccttt cctctccagg accagggagc cccgctcgtc tcctgtcttc ggcatctgct   24300
ctctggcttg tgagcactgc atttctttct gcttggaacg ccggcccatg gtgggtcccc   24360
ggactcctgc cttctgctgt cttttgccat atctcgtcaa agtgtgtttt atttatttat   24420
tgttattatt ttttgaaaca gagtcttgct ctgtcgccca ggctggagtg caatggtgtg   24480
atctcggctc actgcaacct ccgtctcctg ggttcaagcg attctcctgc cttggcctcc   24540
cgagtagctg ggattacagg cgaatgccac cacgcccggc taattttttgt atttttagta  24600
gagatggggt ttccctatgt tggtcaggct ggtctcgaac tgacctcggg tgactggccc   24660
acgtcagcct cccaaattgc tgggattaca ggcgtgagtc accatgcctg gccaactatt   24720
ataatattga tattataata atatcacagc cagataacgg tgactaacgc ctgtaatccg   24780
aacactttgg gaggctgagg caggcagatc acctgaggtc agttcgagac cagcctggcc   24840
aacgtggtga aaccctgtct ctaccgaata tacaaaaatt agccggagcg tgatggcagg   24900
tgcctgtaat ctcagctact tgggaggcgg aggttgcggt gaactgagat cgtgccactg   24960
cagtccaacc tgggtgacag agcaagactc catctcaaaa gtagtaataa taataataat   25020
aataataata tcacttattg agttttaatt ttgtgccagg cactgtgcta gtacttgcgc   25080
catgtgacct cattgactcg gaacaaccta tgagtaagca ccatctctgt ccccaaggaa   25140
gctgagactc aggaaatgga agtcatctcc cagggcccac agcaagggcc tggcgaggtc   25200
tgcgttcaaa ttctgatcat ctgactccag gctttcctgc ctcctttgcc tcccaaaaac   25260
tcaaatactt tcatctggga tgactccaga cagctagatt tcaattgtct tctctccttc   25320
tgagtatgtt ttattttaaa atcacataaa gataactcac ttctcaactg gagccacgga   25380
atcgttagat gtcttggaga cttttctagc tacctctttg ctggactcag acacaggcct   25440
gaataattag gtccaaggag ctgaactgga gccgctcgtg gccagaggtc ccttcctggg   25500
aacagcagtg gcggcagcgg ctgcgctgag cctggcaatt tgttcaagag caggggatgc   25560
tcaggctgcg ctcgccccct tcttatgaac ggtttgcttt gcgtcttctc tgtgccaagg   25620
gctgccaggc accagatgcc cattttagga aatcagaaaa ataaacagtg taaataaaac   25680
aaaaactcca cccaagaaat ctgagacaga tgctgttttc ccccaccgttt ctccaccccc  25740
atgctgggtt tggagagagt gggccagcat gtttcctacg gataagaggc cctggggtat   25800
```

ttaacctgga tggaacctct tctattttcc ttttgcctgg tgctaggggg cagaaggcag 25860
gggtggtgtc catctggccc agggaggagg atagggcagc ctttgtcgcc ggcatcccct 25920
gcagagatgt ctcagccaga agtgactcca tcctcaggaa cgtgtgtcct gttctttgct 25980
gggcggcccc tccttacccc ccattgtttc cacagtgggg tgtgctagat ttatgggcag 26040
agttctggtt cactctcagc cccggacagc agaatgtccc agtagtgcac tgatggtgtg 26100
tgcgtgggga tgtgtggaac aggggatagt gaccccgtgt gcgttatatg cagtgcggct 26160
tccagtcggg tgtgtgaagg gggtgctcat ggccatcgtg gaagtgagct cagctggggc 26220
tggagggtcc aggctcactg tatgccactc agcagccttg ggagtggggt tcatcctcct 26280
ccccgctcct ctcccctcct ggtcctgggc agcccaccgg agctgcactc tgggccatct 26340
ctcctggtct taaggaggct gtcatttctg ggaggcagat gctggccctt ccatccttgc 26400
ttcctggaga tctgcgccga ggtctgcctt tctgtctgga gggactggcc aggaacccct 26460
gggttttcct ggtggggttt ttgttgctta gcagtgagca gagtgagtgg gcaccaagag 26520
gaggatgctg ggtgaggtca cggacatggc tggggcaggc tgggggtgct ggaggtgaat 26580
tccgcagctg tgcgaccttg ggcagtttgc ccagccctgg gcctcgcttc ctcctctatg 26640
aaatggggcg ggagcacctg cctgctgggg ttgtgggttg ggtgaatcgg agctaaggta 26700
cagctcacag cctgccgtca gaccctcgca agcagaagtg ggtgtccctg ctcagcgtcc 26760
tccggtccct ccttcaaggc tcagctttgt cctcatctcc tccatgaagc ctccctggtt 26820
actccaggag tcacgaccca caccctcttt ggagccttgt gtcacacgca tttctcacgg 26880
tgttgctttc cctggaggat gggttcaact aaaattgggg cccatctggg ggttccctcc 26940
agcaccagag aagctcaggc ttagtagaga tgggtaccct gtcaatacta gttccgcgaa 27000
ggatacactg tgctcattct gctgccagtt gctgagtctg gtgtcctgta gttatggaac 27060
atgtgtacag gcctgggctg ctggacacga aggccagatg ggcagttcac tctgcctccg 27120
ctctgagcct ttagaaacaa gctggctgca cccatttcca tctggtcaca gtttaggagt 27180
ggggttgtgg aatggatgga aggaaggatg agtgggtggg tggggggtg ggtgggtggg 27240
tggatggatg gatggatgat gggtggatgg gttgatggat gggtggatta ggtggacagg 27300
tgaatggatt ggtgggtggg tgggtgggtg aagggctgaa taggtgggtt gggtggatgg 27360
gtggatggat gtgtggaggg gtggtggggt ggatggggtc catgggtgaa tggatggatg 27420
gatgggtgaa tggagtaaat gagtgggtgg atggatggat ggatgctgca agagcctttc 27480
tggagggttc cagggaggaa ctggcatctt tgaccagcag ccagcatggt gatcagatac 27540
agggaggtgt ctgggtctgc ctgatccctt atctcactgt tggtgggact gggctggtta 27600
tgtgtccctg aagctctctg aagggtggct gtatggcatt gcccagggga catccccaga 27660
gtgccgtgct ctacagcaat agggtggccg cctgtgctcc cagggccgct cctcctggca 27720
gcctttcggg tttgcttggc ttagcacctc agcaaatctc ctcgagttag gacttgcctg 27780
ccatgagcct ctccaacaag cagccccagg ccaaggcctg tgtgtgggca gagggagcgc 27840
atgccgcggg aggctgcatt aatctgaggt tctgtttttg ttccctcctg gggcaaaagg 27900
taaaaatagc ctgtcccact ccatccccaa atccagcccg gctgcaccca gccctgggag 27960
tgaagggggc tgggtcagga ctgcccgtga gttgtagacc ctggtgtagg gaaaccctga 28020
ttcataagac gggtggacat ggaggaagga ggagactccg tccagcagag acacgcttgg 28080
gtgcagccca gcttggagtc tgatggacgt gggctcacct gtgtgcaact ctcattcact 28140
gtgtggccac agggaagtga cactctgtgg agactcagat tccttatctg caaaacaggg 28200
gtagtagggc ttgggggagg atgtgtgtga aggtttggcc cagggcccct ccagcagcca 28260
gacagcagtg gctgtcatcc ctccagcgtc cagggttacc tgggggagta gggcctctgt 28320
ccccagatgg gccccgtttc tggctctgga actcacagca ggagcaaagg cgaggacatt 28380
cctggtgtta atcatccacc cggagggaga gcgccagtgg ccttgtgtga cctttgccct 28440
ttgtctctgt ctttaggagg cacgcggacc tccagtggcc ggctccggag gcttggtgac 28500
tccagtggcc caggtaggtg gctcgctccg ctctggcccc acccagctca tgggtgtgtt 28560
tgtgctgggg tcccttcatt ctggtctggg acctgcctcc ccacctggct tccctggatg 28620
agtggggcca cttgcccatt tcccagggag acgcagtctt tgacggcttt ggactcaggt 28680
ggcttccagg cctgtcgacg ctgccctgct ggagatgatc cttgtggggg caggggcagc 28740
cacccccagg ctcagggagc aagcccgtgt gtgcgggaca gaggagagaa caggagatag 28800
atagggctgg atgatttggt ttgagatgct gaggggacag atgtgccatg gacaggtggc 28860
tttggccgtg agcctctcgg tgttggttca gtatctgtgg gctccctgca accttgcagg 28920
atggaagatg ctccaggata gagcagatac gtccggtgtt atctaggagg aaataagtgg 28980
gcctctggca gtggtcactg tgctactcct tgtgcttttt ccatgtgttt ggaaattttc 29040
cacactaacc agtagggaag ggaaacagat gcacccatca gagctctggg gctggccgtc 29100
caggacacga gttacagctt ataggcttga agggactcta ccaacactgg gtgcagcctg 29160
ggcaagggag tgggacagag aggtcagggc tctgcctcca gcccttgccc cccagggctg 29220
agtctgcgcg tcatcttctc cccactgggt gtccctgatc tcccctgcct gaccccagtg 29280

```
tggccatgct agtgcctctg tttctgggag cctacgttgt tcttggctgt cacttccagc   29340
ggccaggctg ggacaggtgc ccatctagtg gcttttctat ttggagagga tgatgggtca   29400
cagcaatgaa tggacttacc tcccctagga gacgaggagc tctgagaaga gcagagatgg   29460
cgtggtcacc tgtgcatatg tgctgggggc caactgggtg ccagacccct ggctgccatc   29520
atggtggcag acaggcaggg actgtgcctt catgaattag ccattctaga ggagacaaag   29580
ttaccaaagg acacggaccc ggagtcaaat ccacacaaat agagcgcctg tggaccaggg   29640
ctgccttcag aacaaggtga gacagccgtc ctgccaggac aaggcgccta cgggggcaag   29700
ggcatgtgcc catctggagc cgccacctcc atgctctaga ccagctccgt ctaatagaac   29760
cttctgtgat ggtgggtctg ttctatacat ctgcactccg ccatcctgta tggtagccgc   29820
ttagccgcct gggtctattg cacactcaaa gtataaatgt ttacgtttac tttattcact   29880
catttacttg catagaatta tttatttatt ttaaatggat ttccacttat atctacgtgt   29940
ggctagtggc tatgaaactg aacagttcca gaccacactg gcacctgaga cctcagaatt   30000
ctagacccaa agcctgcttc tgatcctgag cttgcttctg tggggacagc acagacgcct   30060
cctaagtctg ccttccagag gtggatgcag tgcccgtgga ctgtgcacca ccaggcctgg   30120
acagtggaga gggtggcttt ttacagggag tgggagtgga ggggatgccg tgtccacatt   30180
gagtgcatgt gaggcccctt gcagtacagg atgggactgt atgtgggaag agggggggtga   30240
tgctctggct ggaggcccct gggcagctgc atgattcagc gctgtggtta agaggggctc   30300
cgcccatcgc agtcatgttg ggcactaacc actggacagc ccagccgctg ctgtgtttct   30360
ggggagcttt tctccggctc cgcatctgat ctgcctctcc atctccttcc ctctcattgg   30420
ccagcttctg gtggaatggg tggggagggt cccccagttc ttgtgggggt tgcatcttgg   30480
gaacctggca ccctgggctg catggacaca gcctcccccg gcggttgctt ccctctgctc   30540
agactggctg gttctgtgct gtcagcactt ggcagccacg atcacagctc agcttggaag   30600
cctaaggcat aatttgctga atcccaaagc tgcaagctgg tgatctgggt ttgcgtggga   30660
ctgcctgctg acacacgggg tcctccctct gtgggcctct gtgggctctg gcccgagaca   30720
ccctttcctc atcttcttgg cttctttctt tctccctgcc tcccttttgc taagaccctg   30780
gtccaggcag gagttcattc ctagacgccc cagcatctcc ggcctcccca tttgacactc   30840
cccagacgac tgtctgccat cccatcctcc cagcccagct tctctgtaga tccacatctg   30900
gccaatctcc tatgtctggg actcttgccc tccccagaag gacgaaggct cttctcgagg   30960
ttgtgtttgg gactgggcca gggatgacat ttctggcaaa agcttaaatg acaccatgat   31020
caacgctttg gtcagctcac acactttctt tccttttaaa aaaaaaatgg aattcttaga   31080
gctacaaaac aaaaatgtaa agaataaggt aataaaacac ctgtgtactc accatcccgc   31140
ttaagaagtg agacattgca ggtgtagccg cagccctggg tgaccttctc catcctagcc   31200
ccactcctgc tgcggtgccc gcccctctgg attgggatct ggccccgggc aggcacatct   31260
gtgctgggca gtggtgtgtc tgagaagcct aggtctcctt ttttttttt ttgagataga   31320
gtctcgctct atcgcccagg ctggagtgca gtggcaccat ctcggctcac tgcaacctct   31380
gcctcctggg ttcaagcgat tcttctgcct cagcctcctc agtagccagg attacaggca   31440
cccgccacca tgtctggcta atttttttg tatttttagt agacacaggg tttcaccatg   31500
ttggtcaggc tggtctcata cccctgacct caggtgatcc acctgccttg gcctcccaaa   31560
gtgctgggat tataggtgtg agccaccgca tccggcctgg tctcctcttg catacggtca   31620
tcagcatcgt gtgactgagc agcctaggtc tgtctcctct tgaatgccgt catcagcatc   31680
acatccttgg tgcctcactg gagtcagtgg ctgccctgcc tgtgcccaca acgtgacggc   31740
gtggagactt atccgtgtag atccacgaag ccttggttca cttattttgg cagctagcga   31800
gtgttcctct gtgtggggag gcagagtttg cccgtcccgt ggcccactgg tgacatcgcc   31860
gaaggtcttc tctgttacta gtgctgccgg gaacgtgtgt ttccccaggt ggatttggct   31920
gggctgtgga gatgcgcttc tcagcccttc tagatgttac caccacgccc tcccgagtgg   31980
ccttctggta ggctctccca tgttcgctca ttttctgtgc ctcaggcagc cgcctctctg   32040
actgagcgtc cagcggactt ttgccctgtt tcccacggtg cacggagcat ttcatctcag   32100
tggtgcctga gcttccctcc ctctggccct cagaccccgg ctgggatcct tggagccaac   32160
tcctctgcct gacatcccgt gtcgctaggt cctgctcttc catcttccaa tacctctggg   32220
gtgtgtggcc ttttccccag tgcccccatc atcccttctg tcctaggttg aaccgtgtcc   32280
cccacaaact catgtccacc aaaaacctca gaatgtgacc tgggagttgg agatggggtc   32340
tttgcagatg taagtagtta aggatcttga gatgaggtca ttctggatta gggtgggccc   32400
taaatccaac aactggtgtc cttaaaagaa gcagagaggc cacagacaca ccggggagag   32460
ccacgtgaag gcaggagcag atgatggagt gacgcagccc caggccaggg agcgtctggg   32520
aagaggcaag gaaggacccc ccccccaccc caccccagag cctccctagg gagtgtggcc   32580
ttgctgacac cttgattttg gacctctggc ctccagcatg ggagagaacc cgcttccatt   32640
ttttcaagcc ccacagctgt ggtccattat ggcagccaca ggacactgat gcacctccca   32700
cctggccgct gtggatggac gaccccgccc gcaggaagtc tccctcaagc actgcaggtg   32760
```

```
tctctgccct cacaggggac gcctgcacaa gtaggtgtga aaaaagggcc ctgtgggggc 32820
aaggcctggg ctggtgctcg ctgcctccct tcatcctatg ctagttccca tgcctcttcc 32880
ggcaggagtg cctggctcca cctggtgaat cagagccaaa ctctcctgac aattggctgt 32940
cacttattta actgccacca tccccaggag acagaaaatg ccagaagggt ggggcctgtg 33000
cagtgcctcc tcgctatttc cccagggcac cgtgccacgt tgcctcaatg cacagagtag 33060
ctgctcaaag tgtgtcggac gaacaggtgc cccatgccca gccatgcaga caaaagtgtg 33120
ccacgtggag ggagctggtc gtggaagaga ccaccagcat cttggctgcc cgaggacctt 33180
gcccctgggc tcttgcaggc acccctagtt cattacttcc tctctcaccc atagctaccc 33240
actcttcctc tatttctctg atatcagcct tgccacccac ccaggtttgc aagcaggaaa 33300
tctcagagtt gaccttgacc tctgcctccc cagcaccccc aggtctcaac aggctgccaa 33360
gatctcatca actcgccctc tgcatactga cccttggaca ggctgccccg gtcacggtca 33420
tgtcttggtt gggcctccat catccctcct tggaccagca ggtgccatcc tccccacccc 33480
tgccctgctg tggctccttc ccctccacgg ccaacattta gaggacgatt accacttccc 33540
tccctgtgtg ctggggaccg ggtgatagga tccctccacc cgatcatttc cattagacct 33600
gggccgggtc ttcttactca tagagccctt agtttccgcc cccaaagcct cgagggtgga 33660
ttcgtgctgc cgcccagcct ggccctgata ccgctttcca gcctggagcc ccctgcttct 33720
gcgctcctgg ctctgagtgc tcgggccccc cctggatggg ttgccacttg tcctctggaa 33780
agcagggctt ccatgctcct ggacctccta gctgactcag tgactttggg ccatcttgtc 33840
acaccccttc tatcagcttg gccttggtta tttctgtgtc ctccatagcc tcggaccaga 33900
gccacggcgg gatgctttcc tggtgcgtgg caggcagtca ggaaatattg ctggactgac 33960
cttgatagga gatgcttggg agaattgccc aaaaaactag cttaaaatgc aaatattggc 34020
cttcttttgc gttggctaaa gagtagcatt tctccgtttg cctcccgggt aagaaattga 34080
tggcgaatgc aatgtctcta gggaggagaa gaaggtgaca aaaagggtct gtataacggg 34140
gcacacgcta ggcatttgtg actatttaag tttaaattat aacataaatg cgaaactctt 34200
tgccttggta gcccttccac attgcaagcg ttcactgcca catgtggctg ccatattgac 34260
tacggcagat acagaacatt gacatcattg cggagagttc tatgggggg cgctggttta 34320
gatcatcttc acgttggatc cacaaattca ttgtttatcc agtgaacggt tactgggcac 34380
ctactgagca agcaggatga aagacacccg ctctcggggg agtcttccgt tggctgcaga 34440
gaagcttaaa ctcaggggac ctggggcaaa ctctgcaagg gcccttggcc tctgcacccc 34500
cagacctccc tcgctctgca ggggcagtcg ggggccccct ctaggaatgg agcgccagct 34560
ccagctcctg tcaggctaca gcccagatgg ttggctgagc acgagcatgt gagagtggaa 34620
ttgggcccaa ccgggctgct tctccccgtt gtcttggttt ccttccacgc tgacctggaa 34680
ggactcctaa agcaacaggc ctgcccaggc ggtttgcacc agtaaaccct gcagcggccc 34740
caagggcaga gatagctgga ttgtatttat ttaggaacag ccaaagtctg aggcacaata 34800
aaaagtggag aaagattcca gagaggaatg cagctggcct cagcctggga atccgtcttg 34860
cttgggaggc tgtaggcggg ccaggctggg gccagcctgg agggtcttgg ctggctctgc 34920
tggcaggtac agtagcaggg tcagcaggag ggagtccagg acggagctcc ttcattttta 34980
attcagggaa gcaaacttca gtggttgagt ctgaggatga gcaggaaatg aatggaaaag 35040
tctttttttt ttctttttc tttctaggca gggtctttct ctgtccccca ggctggagtg 35100
cagtggcaca attatagctc actgcagcct caacctgcca ggctcaaggg atcctcctgc 35160
cttagcctcc taagtagctg ggactacagt tgtgcgccac catgactggc taattcttaa 35220
aagaaatgtt ttcaggctag gcatggtgac ccatgcctgt aatcccagta cttttggagc 35280
cgaggtggga ggatcacttg agctcaggtg ctccagacca gcctgggcaa catgacgaga 35340
ccccgtcttg actaaaaata caaaaagtta gctgggcgtg gtggtacagc cctgtagttc 35400
cagctacccg ggaagctgag gctgcagtga gttgtgatca cgtcactgtc ctccagcctg 35460
ggcaaaggga gtaaggccct gtttcaaaaa aaaaaatttt tttttttt gaggtggagt 35520
cttgctctgt tgcccaggct ggagtgcagt ggcatgatct tggctcactg caatctctgc 35580
ctcccaggtt caagtgattc tcctgcctca gcctcccaag tagctgggat tacaggcatg 35640
agccactgtg cctggacttt ttttttttt ttttttttt gtagagatgg gatttctcta 35700
tgttgcccag gctggtcttg aactcttggc ctcaagtgat cctcccactt tggcctccca 35760
aagtgttgga attacaggtg tgagccacca cacccagcag gaaaagtcac tttttaaagc 35820
gagaggttgt gcgtcctcta tagcttggcg ctcatcagca tggaggattt actggaacgt 35880
gtgttaggtg aggggacatg gaagatggca gcaaatccga gcccagagca tcagaaagca 35940
tgctttcagc caaagatgcg ttctctccac ctccgcgcag caccctcccc cagtgtgtac 36000
tcctggagca aggtggggcc gatcctacga agaccctttc ccgaaggccc ctgctgccca 36060
catcagtgtc acctggaccg cctgctccct tttctggatg gctggaccag ccccagggcc 36120
tcctggcctg gtgtggggag cacagtccac cctggggaat gcctggtatg tgtcccctcc 36180
ccactgggta ccaggggatg aagctgtgct tcgtggaagg ggacagaggc gctggtgccc 36240
```

```
aggatggtgc acctggggcg gctgaactgg acctggctcc tactgggccc ctgtggagcc 36300
cagattgttg tgagaagggg tggggaaagg caggtgatcc gcacctttca cttttgtttc 36360
acatgggaac aggggcttat cctgcacaca tattcagttt ggagtatgaa tttccatgaa 36420
gaggaaaatc tctattaggc tctgatgtca ggattgtccg tgagggccgg gccgtgttct 36480
ggggcaagga caggagtgga gggggcaacc agggaaggct tcctaatgga ggaggcattt 36540
gatagcatct tgaaaaagaa gcagttccta ggccaagcca gagactgaag aatgaccacc 36600
taacttcttt tttttttttt tgagatggaa tctcgcgctg tcgcccaggc tggagtgcag 36660
tggcgtgatc taggctcact gcaacctcca cctcccaggt tcaagcaatt ctcctgcctc 36720
agccttctga atagctggga ttacaggcat gcgccaccat gcccagctaa tttttgtatt 36780
tttagtagag acggggtttc accatgttgg tcaggctggt ctggaacccc tgaccttgtg 36840
atccacctgc ctcggcctcc caaagtgctg ggattacagg catgagccac tgcacccggc 36900
cacgaccacc taacttcttt ctacaagatg ccccctggct gacgcaaagc aggttgagtg 36960
agtgtgacac ttccttcctg tctgtgggtt ccggcctttc cagagcccgg ccagtgctat 37020
tggagacaaa gtggccttcg ctcttccttc ccgccctgcc aggcccagcc tgtgccgctg 37080
agccccactt tgcaattaag aggagctgtg aaccagtgat gctgatgtcg cagaaaagtg 37140
gccaggggtc tatgggggt gaaggacagg gatgccgccc tgagaactgt ctggaagggg 37200
cttttgggta acagtgccca ctccagggtg tgggcaggct ggggtggctg ggagctgggc 37260
tcataggctg gcacaaccag agtttcccaa agccgtgggc ctgagggccc tgccttcctg 37320
ggacagcaac ctttggtcca atttgggaaa aggtgggtta aaccaagccc ggctccctca 37380
ctgtaggatt actcagtgcg tttagtatgc aaatgtgcct ctgaatctaa gacaggacca 37440
cagaaacctc ttcagtgttc ctctggaggg agggaccgcg ggggatccac cgcaggggcc 37500
tgggtctgca ctggcctggt gaagtgcccc cttagtgctt acaccatgcg cggtgagagg 37560
ttggccctcg cctcccccac cctctgctgg ccttacctca ccacctctgc actcgcctcc 37620
cagccggctc atgactaact cctgcctctt cagagccaat cggtgctgcc aggttttcca 37680
tttcaagtga tcccccgcct gggccaaaat gtttggattt tcaggcacgg aggggagatg 37740
gacctcaggc caaaccatgc tgggtaaaac tgagggtcaa ggctgtgctc agcaagtgct 37800
ccagccctgg ccgtcaaacc catagtgtgc acagcgtgca ggaggtggct gggtgaatga 37860
actcagtcat agcccggccc ctcactcggg gctgttccca agttcccacc gccagccagg 37920
ctgggtcaga gcctcgtggg gccttcctgg gcttccctgc cccacagcag gagttcgtat 37980
ggagagtctc gagtgaggtt aatgggtttt cttcctcttc tttttttttt tttttaattt 38040
gagacagggt ctcactcttt ttacccagcc agggtgcagt agtgcaatta tagctcacag 38100
cagcctcgaa ctcctggctt caagcaatcc ttccgccgca gcctcccaaa gtgctgagat 38160
tacaggcacg tgccaccttg cccagctggg gtttctgctt tttacttttt ctcttgtttc 38220
taaactcaat acaaagaaag atattacatt tataatcaga aaaaccaaca aggaatatta 38280
tttcaaaatt ggaaaaggga ttaaagaaac aaaccaagaa acctctgcga ttcctgccca 38340
tcggagtcag tggttgttag tggttggctt aacacaatgt tttaggaaga aatagaataa 38400
cagaaagcaa ctccagtgag ctgagccctg gctccgtctg tggcgctgca tcagtttcct 38460
gcagctgctg taacgagtgg ccacaagctg gctgggtcac aacaacgccc agccatcctc 38520
tcacagctct gcaggccaca catccgagat cagggtcacc aggctgaaat cagggtgtcc 38580
actgggcata gccctctgga ggctctaggg caggggttcc cagccccgtg tgtcctatta 38640
ggaactggcc tgcacagcag gaggtgagtg actggcaagc gagcgaagct tcatctgtat 38700
ttacagtcac tccccattgc tcgcattact gcccaagctc tgcctcctgt ctgatcagcc 38760
atggcattgg attcttatag gagagtgaac cgtattgtga acggcacatg tgagggatct 38820
aggttgcgtg cttcttatga gaatctgatg cctgatgatc tgtcaccgtc tcccatcacc 38880
accagatggg accgtctggt tgcaggaaaa taagctcagg gctccaactg attctacatt 38940
atggtgagtt gtataattat ctcattatat attataataa taatagaaat aaagtgcaca 39000
atcaatacaa cgtgcttgtg gccgggtgca gtggctcaca cctgtaatcc cagagtgaaa 39060
ctctgtctca aaaaataaaa aataaaggcc aggcacagtg gcttatgcct gtaatcccag 39120
cactttggga ggccaaggct ggcggatcac gaggtcagga gttcaagatc agcctggcca 39180
atatggtgaa accccgtttc tactaaaaat acaaaaatta gccggacacg gtggcaggcg 39240
tctgtagtcc cagcttcttg ggaggctgag gcagaagaat tgcttgaacc caggtggcgg 39300
aggttgcagt gaactgagat agtgccactg tactccagcc tgggtggcag agcgagactc 39360
tgtctcaata aaataaaata aaataaaata ataaatataa tacacttgaa tcatctcaaa 39420
accatctccc cactccctgg tctgtggaaa aattgtcttc cctgaaaccg atcccaggtg 39480
ccaaaaaggt tgggggccac tgccttcatc gcctgcccct ttgagcctct ggtggctgtg 39540
ggtgtccctt ggcttgtggc tgcatggccc ttaccttcaa caccagcatc tacagatctc 39600
tccgggcccc atctccactt ggccatccct gtctgtgtca aatctccctc tgcccccgtc 39660
ttgtatgagc accgagactg cctttaggga ccacctagac catccagggt gctctcgcca 39720
```

cagcaagatc tgcaactgaa tcacagatgc aaaaactctt gttccaaaca aggtcccatt 39780
tgcaggtcta gggattagga catgctatcc ctggggccat tattcagcct tctctcgtga 39840
ctatgatccc tcccttcctc cccagcctcc cacctcggct gagatgctgt ccccacctcc 39900
ctcactgtct ggctgcctgt ggggctgtcc tccaggctct ttgggaggag gtggttgctt 39960
gatgtctccc agaaggctgg gaatggtcct ccccaccccc caggaagatg tcctttctgc 40020
caaggagatt gcatttgggg tcctatccca ggcagagagc ggacgggact gtggcctgaa 40080
gtggcacttc agggcctttg gctctgtcat ctagccgagg gctgtgcatg ggcctcacct 40140
ggtcacaggt aactcacctg gctctgtggg gcagggattg taaactcaga ggcctgcggg 40200
gtgtgtcgag gtagaggggt gcaaatattg taaatttgtt gggggccttg gggagcagca 40260
cagactgaaa agtgttttgt ctgaagagca gaactgtctt ataccagccc tgtgcggcca 40320
ggcaggaagg cttccgtggt gcggccacgc gtcctccctt tctcaacaga agctgacagt 40380
ctggagtttt cagagagatt gcctgagttc tcagccttgg cacaaacaca cattctcttt 40440
aaacaggctt gggccaacag aaccagcctc cgtgtctcgg gtttgaatga acccttgagg 40500
tgaagcttaa agacagagag gatgcccctg attcccaccc cagggaccca cattgtaaac 40560
ccccacctgc tctgctgagc ccttggcctc agcatggacc ctgaccatgt gctggtgggg 40620
aaccccccagg taagtaagca gcctctgagg accccggatg aacagtgggg aacagcactg 40680
atcccccagt ggggccccga gttcccggag aggaagactc gctccctccc aggggacggc 40740
tagagactca ctgactcatg cgtgtgtggt aggaatcttc caggaagtcc ccgtcccgtt 40800
cgccctctct gcctgggcgg ggacggcaac tgcctctgtc actgcagact aatgggacgg 40860
aggggggtga cttctcaggg ttcctcctgg gcaggtgctc cggaaccttt tctcagcacg 40920
ctggcctggg gcacggccgt tcctcctgcc cagccacgtt ggggtacagg gtgaagaagg 40980
gctggggcca gcccaggaca gaggaaggcg aggcaggcac gcaggaactg ggctttttaa 41040
acccttaagc ccaaggaaat cgtagcatcg cgggacaggg aaaatgaaag actttggaag 41100
tcgtcaggaa tttgactctg tgagttggtt tccaagagtc taagttaagc atctccaagt 41160
ggatattaaa aaggagcagc aagcctcggg gcggcggggg ctggaggagg tggagagagg 41220
aggctgccgg aagccgcact cgggacctct gcagccaccg accagaccgg gcggccggga 41280
ctctgggact ctcgcaggca gacccggtgg tctgccggac tcctcggggc ccacttcggg 41340
ccctctctcc tgcctcctat ttttggattt ctctcctttg ctccctttttt cctcccgttt 41400
tgaagagaca atgctacttc agtttggagc acaaacatat gatcagcaca tggaaatgtg 41460
gtaattcgga tgcattcgtg attgcaacag attgaagaaa ttagaccaga caaagagtgt 41520
ttttagagga ggaggaggag gaggaggagg ctgagagagg gagggcgacg ggggtgagaa 41580
aggggaggcc gcctctgagc gggacgccgg gactcccgcc gctgctaaat atatccgtag 41640
gaatggagag ggaccggatc tcaggtacgc agacagcccc ctccccatcg gccgcccccc 41700
actgccctgg actcggggct ttatttatgc ctgggggaat aagcatgcaa agggcgcttt 41760
tgctcatttt tcacagaaat atttcttttc tttttgacgg ttccaagctc gtgggatgaa 41820
ctccgtctca gaagctaagc tctcctgagt ctggctttta tttaaactcg gctctcctct 41880
ctcctggcta cttccctttc attttccagt gtgccattct tgcatgtcat ggccactgtc 41940
aatgctgagg ctgttggcgc cgaaaccttg ggagggtgtg ggggacctgc ccccctggct 42000
cggtctgggg gcggcggcgc ttggacctgg ctggtggttg gtttgtccac tgtgtggttt 42060
gtttttttgct tccagccaaa gtctgtcttc aaggctgcct cggtctccta cctaaaatag 42120
catttctgtg ccttcctggc agttgattat agagggggaa gctcccggcg ctcagaggag 42180
cctggcgtgt gtgtgcacgt gtgtgtgcat gtgtgtgcgt gtgtccattc acgctggcag 42240
ccccccttgcc gcaggcacag acccatcagg gcgtgaacag gcaaatgaag agaaaggggg 42300
ctcccctgcc tttagttcac cccagctttc caggccctcc ctcccaggcc cagcctcctt 42360
ggtcccttca ctcacatgcg gtgccgtggc tgccatctca cgtggccagc gccgtcatcg 42420
gtaattagca gtgatgacag tttctgccgc tcatgccgcc ctctgagccc agcgctggga 42480
gacatggggt ggctgagcca agctccaggc tttgggtatt ctgaagtgac ctacgggcca 42540
tggagcggga ggtcgctaag agtgtcaacc cctacgtgcc ttgggccctg gtctgttggt 42600
tgccactatg tgtgtgttgg ggggtccctg agaggcctga gagagggagg cccatctcgg 42660
tggctcctct ctaggacgtg tggtcccctc caccactgct cccagggcca catctttttt 42720
tttttttttc tttgagacgg agtctggctc tgttgcccag gctggagtgc agtggcgcga 42780
tctcagctca ctgcaagctc cgcctcccta gttcatgcca ttctcctgcc tcagcctccc 42840
aagcagctgg gactataggc gcccgccata tttttttgta tttttagtag agatggggtt 42900
tcaccgtgtc aaccaggatg gtctcgatct cctgatctcg tgatccgccc gcctcagcct 42960
cccaaagtgc tgggattaca ggcgtgagcc acggcgcccg gcctcagggc cacatcttga 43020
ttgtcacgtt gggactggtg gagcgggtgt gtgttgtgcc gctgagcaca cccagtcggg 43080
cacagcaggc ggacctcagg aggggccag gggagacggc agaaggcttg ggtggcagtg 43140
aaactgcatc ttatctgttt gagctcgggg gctcaggccc gctgggacgg gtgggtgtgg 43200

agcccagcgt cctgctctcc gcccagcagg agccgcaggg cagggagcgc tgagagggac 43260
cttcgtcagg cttgcacagt ggagccgtcc tcggagtgac caccccacca ggcagcctgg 43320
gcagaccaca gtgggtcccc tgcctcctgg gccaccagcc ccaggaggac gtcactccac 43380
ccgcctggct ggcaggggcc tcggggagcc tcgggttcgg ccttcacttt atagatgatc 43440
tagccgaggc ccagatgggt gaccagcagg ggatctgaga gctgagcaga gaggcgtggg 43500
tttgggggtg gtgctccctg gggagctgga agtgatggca ccctcccagg ctcacaaaac 43560
acgggctttt catgaactga acaaaacact ctttgaaagc agccagtgct gtgccccaga 43620
gaccactctg atccctgctg cccttggctg aggacagcac agccagggat gaagcgggga 43680
cctcccctcg ggctgtgcct tgatgaaaga tgctttctac aaaatcccat tcggatgtgt 43740
tttcatgtaa gaaaacgtac atttttagaa actcagttgt aaactggctg ccttccgaat 43800
gaacacgaca gtcccggagc tgtttccctt aatgaggttc tcgtcctggg caaactttag 43860
cgctggtctt taattgtcac tgaactcccg cacagtccct ggggaagtga cttccttcct 43920
gtccctgctg ggtggccttg agcaagccac ttggcagctc tgggccgcag ttccgtcatc 43980
tgcaaggtga ggggtgggag cggcacggcc cctaggcagc gcccccacct cctgcgggtt 44040
tctgcgcgtg tcctgagttt tgtgggcgcc tcttccctca ctgaggggct cttcttctct 44100
gcagggcatg gctcacgccg cgttgtcagg ctgcaagcca ctgcgagagc tgctctgatc 44160
cccaagcatg ctctggcctg aggaggagcc atggggagga cgctgtgcct gtgtgacaga 44220
ggcctgcccg gcagatgccg gcccgctgac ttccagacgt ggctgtgggt gcctcatgcc 44280
ccactgggct ggcgtgtgag gcgtgtccag ctcaggctgc cctacagctg cccaggaagg 44340
gtttgtggga gggggtggtc ctaccctggt gagtccacgg tggtctgcca gccccaggga 44400
aggcttagga gccccaagac ctgccctctt gtccctccct cggcagtgtc tggctccggt 44460
ctggtttgtg aacgggggcc tgggtactct cccgccctcc ctgggggctg tggccttccc 44520
ctgggaaggt gctggagggt ttgtcagcag ggccggcatg agtcatggag tggtgactgg 44580
ccgctgaggc atggaaggcc actccttcca gccaagggtc ggagcttttt ttttgttctg 44640
gaacggcttt gtggctttta caaattaagc agttttatct tgttccaaga gcttgtattt 44700
cagcagggag agagtctcca agaagggaac tttccactgg ggagctctgt tgggctggga 44760
ggctaggaac aggccacgag ggagggctgc aggcagggga agtggccgtc tgtgcatggt 44820
catgaatgca ggcccggggt cctggagggg gcttggccac gctgtcgctg gacgtctgtc 44880
cccaccaggg tccatctgat gcagtacggc acagggtaaa gcccagctcc tcttacgggg 44940
ttcctgggtc atcagtgggc ggggaaccga ggcctcctgg agggcggtgc ctcagccctc 45000
acccctccct cctgtccctc gctgtgctgt ccccgatggc ctctgccatc ccctgggagt 45060
tcttacccct ggacgctcgg tgcctgtccg gtctcaacct ctccagctct gcttttccat 45120
ctccgaggtg agaggcctgg cctgacgcgc tctgcacccg cttcctgccc agccccgtgg 45180
gaactctggt ccctgtagtg gtttcctggg gctgccatca ccaagtgcca caaactgggt 45240
ggctcaaaac accagacatt tcttctctcg ccgttctgga ggccagaagt ccgaaatcac 45300
ggcatcagca ggcctggttc tttccggagg ctccgagggg gagcctgctt ctcgcctctg 45360
gcagcttctg gcggctcccg aaacccttgg cgttccttgg ctcgtggcca caccactcga 45420
cctctgcctc cgttgtcacg gggcctcctt ctgtgcccta aatctccccc tgtctgtctc 45480
tcatacggac agtctttctc ggatttaggg cccactgggt aacccaaaat ggtcccatct 45540
caagatcctt aacttcataa cgtctgcaaa gacccttttt ccaaataggc catgttcata 45600
ggctctgggg tggatgtaaa ttttaggggc cacctttcat ccccctgttg tccccgtgtg 45660
tctggatgca cagcatggtt ccatgtccca gactccttgc tggacccctc tggccctttg 45720
gggtggcctt tggccttttc cactcgtttc tgtgctcctg gtgaggtgtc ggggtcgggc 45780
ttagcccctg caggaattct ggggacagca tggagccggc aactccccac caggcgtctt 45840
cctacctgcc cagagctcat ggttgtgagg ggtctctcct tgtggttttg atttgcaatt 45900
ccccagtggt tagtgatgtt gagcatcttt tcgtgtttat tgctcatttg cacatattct 45960
tgcagaaatg tctgtccaag tcctttacct gtgtttgatt tggatgattg ttgttgctga 46020
gtgttaggag atctgtagat taatatccag atactaatcc catatctgac ctatgatttg 46080
ctgccattcc atggattgcc ttctccccac gttgttagtg ccctttgata tgcaattttt 46140
ttttttttt ttgagatgga gtctgactct gttgcccagg ctggagtgca gtggcatgat 46200
cttggctcac tgcaacctcc gcctccctgg ttccagcgat ttcctgcct cagcctcctg 46260
agtagttggg attacaggca cccgccacca cacccagcta atttttgtat ttttagtaga 46320
gacggggttt cactgtgttg actaagttgg tctcgaactc ctgacctcaa gtaatccacc 46380
cgtctcggcc tcccaaagtg ccggagttac aggcgtgagt caccgcaccc aggctgatat 46440
gcaaatattt taaacttcta tgacgttcca ctttatctat ttgttcttct gttgcctgtg 46500
cttttggcgc catatccaag aaatcattgc caaatgcaac gtcaggaagc ttttcccctg 46560
tgttttcttc taagagtttt gtggttttag ctcttgagtt taggtctttg atgcaagttg 46620
agttgatttt tgcatgtggt gtaagggctg gtccagcctc atgctctggg ctcttgattc 46680

acttctcttc ttttctcacg cccagctggt tccgctgggt ggcggggagg agtggggaag 46740
tcccgggctg ggcctgcact cgatcatccc ctctcaggcc agccagggag tctcagctcc 46800
tgcccaggac ctggctggac gtgctcccta ccgggaaagc ctgggccgtc tttctaggct 46860
gatggcaggg ccagcccggg gcgtcctgag gcctgccctg cggacatgcc ccttgttcta 46920
ggtggtgtgg ctgcccggcc tgcgtgtgag accagctgtc tgtgcttcag gccatggagg 46980
ctgagtgttt ccagcctgtc cccttgctcg gctctccctc tggggaagcc cctgcagccc 47040
attctctgcc tccgcttctg ccatctgtgc ctttgtctgc ttcctgtttg gaggtggtca 47100
tccctggggc cacccctcat gatctggaca caagtctcca tcctgaagcc accacccaaa 47160
cccctgtgcc tcaaacccct cccacccacc acatgggttt ccactgtgac caactcagca 47220
gctgatgaag cttcccttgg ggctctccta gcaacgggga gctggctttc ccggaggcct 47280
ggcctctccc taagtggaag tggggcgtga gggtgtcagc ctttttctgc tgcctggtgc 47340
tctaggttgg cttgtcaccc ctggaagcac ttgccatcct tatacagcac cccacaccca 47400
cctccccgcc tcctacccct tcttccaagg ggtcatctct gcttccctcc ccacccaacc 47460
tcacccatgt ggtccgccca gcaacctttg acccccaaca tgacaaaata aacctccctt 47520
gccggtcact cattcattca ttcagcattg ggtgctccct gtggacttgg cgctggggtc 47580
ccgtggagga caaagccaga cacagtcctt gccctcatgg gactgcacaa gtgcaagacc 47640
acatcagtaa acgtgaaaca caggaagtga caggtgtgac aaaggggacc agtggcagga 47700
cagaacctgc ggttcgtagg accaggtcag gagggctgcc tcgggggggac accttcgggc 47760
tgagcgcaga aggatgaggg gagtaaacca ggctcaaacc cagcaggcag aggcgatcgc 47820
tgcaggcaac cggcaatgtg ttcaaaggcc ctggggcgcg gggggctgag gccggcagca 47880
cggcaggaag taagactggg gttgaaagag actgactgtc atgttgtgaa atatacactt 47940
ggttttcatc tccatttcct ggcacacaac tcctaaaatc cttggaatct ccaaagtgat 48000
gtctttttgg atgctcatga ttgacagacc agctggcagc ttcaggatgg ttcccaggga 48060
agaccaggta gaatcacaag gttcagcacc accccgcaac ctccaggtag gggagagggg 48120
ctgaaggtta agcagatcat cagcggccaa tgattgaatc aatcatgcct tcgtaatgag 48180
gcctccgtga acactcagaa ggatggggtt ccgggagctt ctggatggat gagcatgtgg 48240
aggctcctgg agggtggagc gcctggggag cacatggaag ctctgcgtcc ctcccccata 48300
ccttgcccta cacatctctt cccctgtatc ctttgtaata tcctttataa taaactagta 48360
aattccatga gccccaggaa catgtgtaaa agaaaaaaaa aagaataaac catagctgct 48420
gtagcaagtt aatcctacca gcactggctc tcatctgccg accttacctc tggcaatgcc 48480
tccctctcct gggctcctgg gacccctccg gccccaccac ccctcctgta gtggtctcgc 48540
tggctgctgc cctcaggtta gtttattttt acccggcctg ctcccaccag gggctgaggc 48600
ctccttgctc actccagctt cctggcccgt ctcttgctct gggcaccccc ccactccgaa 48660
cggccagagc ttctgaagcc gctcgctgtg tgcccccgcc tggccccatg catcccgcac 48720
ggtgctcccc agggtctccc actggcaagg gcattggcat agattctggg gctgccttgg 48780
ttcaagccca ccctgacttg gagcctgagg gtccctgagg gggtcctgaa ggccagggca 48840
ggcaaggctg gcatgctctg ggcagtgggg gctgggtatg ggaggggatc gtgggtgggg 48900
agattcctct taaccagcgg cccacgtttg gagactgatt tgggctccag gctacgcctt 48960
gaaattggga cagagagtgg cgcagatctc agcccggaga ctccctctgc ctgtggacgc 49020
cctcatacct ccatccgtgg agccaccctc ctcttttttt ttctaaatgg ggaaactctg 49080
acttgaacac tgaataaaca acacaccctc gaaatcagcg aagaggcact ggctctgaaa 49140
ctttgccatg aaagcttgaa aatgctcttt ggaccgattt gctggaacag acgggtgtga 49200
tggggctgcg gggtttcagg tggcaggagg cgtccccgtg cctagggcat cccgatgctc 49260
agagtttccc cgtgcccagt gtgtccctgt atccagggca tccccatgcc cagggtgtcc 49320
ccgtgcctag tgtgtccctg tatccagggc atccccatgc ccagggtttc cccgtgccca 49380
gggtgtccct gtatccagag catccccatg cccagggtgt ccccgtaccc agggtgtccc 49440
cgttcccagg gcgtcctgtg cccagggtgt ccccgtgccc agtgtgtccc tgtatccagg 49500
gcgtccccat gcccagggtg tccccatgcc caggcactgc ctctccccac tttcctaatc 49560
tccctccacc aacaggtgtc agctgtgaaa tgcctgctgt gtgtgtcctt tggttgagtt 49620
catcttgtgc tcttaagttc ctgttagaca caggcaggct gctttagttc ttctggcaaa 49680
tgttttgttg ttgttgtttg atacggggtc tcactccgtc acccaggctg gagtgcagtg 49740
gcgccatctc gcctcactgc aacctccgcc tcccaggttc aagcgattct cctgcctcag 49800
cctcccaaat agctgggatg acaggcgtcc gccaccatgc ctggttaatt tttgtatttt 49860
tagtagagat ggggtttcac catgttggtc aggctggtcc cgatctcctg acctcaagtg 49920
atccacccag cttggcctcc caaagtgctg ggattacagg catgagccac cgtgcctggc 49980
ccgtctggtg aatgtttatt gaggcctact atgtgcagcc cctctcttag gtgcttggcc 50040
tgtaatgagg agcaacaaag acacagtcgc tggtcgcttg ttacttaaca gttggaactg 50100
agaggcagtc cacaaatgag caaatccata ggtggtttta gatgaggact gtgaagaagc 50160

taagaacagg ttgctggggg tgcatatctg tgtgtgtggc gaggggatta atggaagccc 50220
tttctgagtg gtgacactga gctgaagctg taatgaccag aaggacccag ccaggccaaa 50280
atccaaggga agagcaacct ggcaaaggaa acagcaggtg tgaaggcccg aggctggagc 50340
ctggggaagg tgggagtggc tggagtgtgt tttcagagac gggccatggt gacaccaatg 50400
cgacaggcag ggccaggtcg tgccgggcct tggaggccct ggtgagggct tgattttatt 50460
ctcactatgg tgggacctca gtgccctgtg gctgctgaga atggccaggg gcaggcaggg 50520
aggcctgcta gggaggaggc tgctgcaggc cctgcagctg gagagggagg atcaggatct 50580
ctttagagag gaagttggag agggaggatc tctttgattg ctggatggac ccacctgcct 50640
ggctgccccc gtcagcatcc agcagaggcc actggatgct gacaggtggc cggccctgcc 50700
cagccttgca ctgccctgtg ctgccctggt ggctgccggc tttaggagga gcgcttttgg 50760
tgtctgagcc cagtggtcct ggctgtggtt tggtacactt ggccgtgggt gaggccaagg 50820
tggtttggat cctgggaagg ttgggtacct ggcagcctca aggcaggagc tggggtgttg 50880
gaggaggaag cacgctcatc cctcgttcct tcctgtttcg tgggatgctg agtctctggg 50940
cagggacatg gtgagccctg gtgtgatgca cttaacacct gctggtgccc ccatccccaa 51000
gcaggaaccc tggtgcctgg tgcctgagga ctgctgtcca ccgtcccacc atccttcagg 51060
tttcttccca gcctctgact cggggcaagg ttcagatatc cagaacattc ttggctggct 51120
cttggagctg gggtgaggca gaacctccct cccatattta cagcaccagc caaggcttcg 51180
gggtttggac accatccctg ggaggggtga gcaggacaag tgaggtctcc agacttgggg 51240
tggcttcgga ctggccaggc aggtgggtgg gggctgcagt ccatgccccc gctccaggca 51300
acacagtcga gctgcagccc ccgctccatc cccagctggg gctccctgtg ctcaccttct 51360
gcttccttcc tctccctcgg aatgggggt ggacactcca agactagcct cccagccact 51420
ctccctgcct cctgcctcct ctccttcggg acagacccct gtgatcgctg ggtgttcctg 51480
atgctctgtc ccctctgcgt cctgtgccgt gggtgagaga gggcttcggg gggacttccc 51540
cctcttggag cacctgctgc tccgggtgcc tctgggggcg ggcccccacc tcacattgtg 51600
gagctggtgt aggaaggaag gtgccgggag ccagctccaa ttcacgtgct ggctgtggcc 51660
ctcaaggtgt ggatggcagg aggtggtgag acagggactc attgatcatg ggactgacac 51720
cccctcatgt tgatgtgggc cctgagccat aatctcacca gctcctgagt tttggcgctg 51780
ctcttggcga tggcgtggac tcagccaggc tctcgggcag cctttcctgg attcttcctc 51840
cgttctcccc tgggcagtgt ttctgttcac ctggaaagga tgggtaggac ttggagacaa 51900
cagggattca aagaagtcgg gagtggggc acctgtgcag ctcaccctgc agaacagaca 51960
atttggttaa agcctaaagc ctgctgtcag gagcctcgct tgcctctcag agagcacatg 52020
gcttgaccat cacggggact gggggagagg cacaggcatg cctgtccggc agcagaaggg 52080
aaccccgggc ctgggcctct ccattctgtg gcgttgtctt tcctcagagt tgcactgtct 52140
ccttcactgt ccctgcctgg ccccagtgct catgtggctt gtggccctca gtctgggctt 52200
ggtctcccgc aattcaccct gcccagcccc acacagcttg tccctccagc tgggaatcgg 52260
agctcacctg gagtcccagg cagcagattc gatgcagtga tgcagggctg tcagccatgc 52320
cagcaggccc tgctgctcac agagggatgg gtattgctgt tgacaataat taaagaacta 52380
cggcttcagg ctgggtacag tggctcacac ctgcaatccc agaactttgg gaggccgagg 52440
caggagaatc aattgaatcc aggagttcca gaccaggctg ggcaacatag caaggctttg 52500
tctttacaaa aaataaaaaa taataataat aataaaaact acagcttcag aatgattgac 52560
ttcagcatgt ttataaagtc aagggtcttc agtgtcctgg gcctatgtca ctgcgtcaga 52620
gggagagagg aagaggctgg ggtgatgggc actggggctt atgctcctcc tggaatgggt 52680
ggtcttggac tgtgcctgtg tgatatgttt gtcgagcggg ggcggtgtcc actgaggact 52740
ggatgttgag gtgtgggtgg ggggaggtta tgaagcactg ggcatttccc gtgcaccagt 52800
gccccgagat tgcagagagg ggacccgccc agcctgggca gggaggcggg tggcagcgag 52860
gaaggggaga ggaactcggg tgcagaaagc ctttcctcca gcaggtggcg ccgcaagctc 52920
tcgagagtcc agctcctgcg gggtttgcat tttcactgaa tttgtgggca gaggctgcct 52980
gggtgtagtt tccgccaact gtgactgctc actgcgggcg aggggaagtg gagtgcagag 53040
aatgcaccgg agggcagatg accagccccc gcacaggcac ggggtgcct ccttccccgc 53100
acaggcacgg ggggcccgag cccttggggt tcctgtccta actgggcggg gaaagggagg 53160
gttggaagct gagttagggg gaaagggggt ggtctcctgc catctctttt attttatttt 53220
tcctattttt ggttgatagg tttcaatctt ttttacattt ttccaacttt ttgccgtaaa 53280
acttttcaaa catagaaaag atgaaagagg gctgagtgtg gtggctcatg actgtaatcc 53340
cagcactttg ggaggttgag gtaggaggat cacttgagcc caggagttca agaccagccc 53400
gggcaatata gtgagatccc atctctataa aggatacaaa aaattagtca ggcatggtgg 53460
tgcaggcttg gagtcccagc tactcggaag gttgaggtga gaggattgct tgaacctggg 53520
aggttgaggc tgcagtgagc tgggattgtg ccaatgcact ccagcctggg cgacagaaca 53580
aaaccctgtc tcaaaaacaa aacaaaacaa aaccacaaaa aagaaaccat gaaagattct 53640

aaagtaaaca cccatgcacc taccacctag attccacagc aaccattttg ctgtttgctt 53700
caccacaaat ccctctggcc accagcatcc acgctggttt tgatgcactt aaaataagct 53760
gcagaaatca gcacacaaca tcctgtcaac acctcagttt gcatttcatt aactagagtc 53820
cggcatttgt ctgtgattat ttttgttttt ctttgaagta aaatgtatat accctgagat 53880
gcacaggtgt gaaacgttcc gttctttgaa ttttaacaaa ggtatccacg tgtatcccag 53940
cctgaccaga acatagaaca tcactatcac cccagtaagt tcctcatgcc cctccaggtg 54000
catcacagtc cccaccttcc ccagggcaac cacaattgtg cttttttttcc accacgaaat 54060
aattttgcca gtttgagtgt ttcttagagc tacccatgtt gctgcaggtg ccagcgattt 54120
gttctgtttt attgctgagt aacgtccact gtgtgaacat tctgtgggtt tttagtcatt 54180
ctcttgttga aagacccctg gctgtttcca tctggaggct cttatgagta aacctgttac 54240
gctggttctt cttgtacaag tttttctgtg catatgggcg ttcatttatt ctgtagaaac 54300
acctagagta ggtatagggt aggtatacat ttagttttat aagaaactgc cagatctttt 54360
cccaaagtgg cccttccatt tcacactccc accagcaatg gatacgagtt ctagttgctc 54420
cacatcctgg ccaacatttg gtgctgtcac tctgtgaaat cttagtcatt ctggtgggcg 54480
tgtagtggta tcatctcatt atggctttaa tttgctttgg cctgatgact aatgcggttg 54540
agcacatttt catgattggc tcttcatata tttttccttt gtgaaatgtc ttttcaaatc 54600
tgttgctcat tttaaaattg ggttgcttga ctttttcttc tggatactga tcttttgtca 54660
gatacgtgtt ttacaaatat tttattccag tctgtggctt gcctgttcat tttctttcct 54720
ttttcaagaa caaggtctcg ctgtattgcc caagcaggtc tgaaactcct gggctcaagc 54780
tgtcctcctg cctctgcctc cccaagagcc ggttttacag gcatgagcca ccatgcccgg 54840
cctgttcatt ttcttagtgg tattttccaa taagcaaaag tgtgtcactt taatttttat 54900
gaagtctaat ttaacaaact cctttctttg tggttctctc tctgtgttct ttgtaagaaa 54960
cctttgccta ccctgccaaa ttgcaaagat atttatgttt tcctttagaa ggcttagagt 55020
tatatagctt ttatgaatta acttttgcat atggtgtgag gtagagggtc agggttcatt 55080
ttgttgtcgt tttatgtgtt tattcagttg ttccaaactt catttttttt taaagctttt 55140
atttgtgtatt ttcttggtga cttatgaggt cgcacaattt tccaagatcc ttgttggtca 55200
tttacgtatt ttcctttgta aaattatctg tccacgtctt acttcttttt aaaattatgt 55260
tgtttcaaga tcgagtgatt tctgaagcct tcattttata atgcttgaaa gaatgaacac 55320
cttgttcccc agctacaggc ttctgctctt tgtcctgcag ctgcacagtg gggtaagact 55380
tgggttttgt tttgttgctg ctttgttgtt tcttcatttg actgtttccc catgggtctc 55440
atttaaaaaa aaaattggcc gggcacagtg gctcacgcct gtaatcccag cactttggga 55500
ggccgaggcg ggcagatcac gaggtcagga gatcaagacc atcctggcta acacggtgaa 55560
accccatctc tactaaaaat acaaaaaatt agccgggcgt ggtggcgggc gcctgtagtc 55620
ccagataatc gggaggccga ggcaggaaaa tggtgtgaac ctgggaggcg gagcttgcag 55680
tgagccgaga ttgcaccact gcactccagc ctaggtgaca gagcaagact ctgtctcaaa 55740
aaaaaaaaaa aaaaaaaatg ttgtttcata tgttttttat atattctgga tataagtcct 55800
ttatcagata tatgtatcat aagttttttt ttccacattg ctcctttcct tttggttttc 55860
ttaatgatgt tatttaatga gcaagagtat tttgttttca taaagtccaa tttatcaact 55920
ttttccagtt tcgatttatg atttttgtga cctatggagg aaatctttgc ctacactaag 55980
gtcaaaagaa gttctcttat tttctagaaa ttttatagtt gtatattcta tgattccttt 56040
tattttttaa ttttttaaat ttttacattt tttaacctat tatagccgtc aggatgttgt 56100
atgatccatt ttaaatcagt ttttgtattt ggtttgaggt cagggttaat tcttgttttt 56160
ctatactcat atccagttct agcaccgttt gttgaaaaga cataccttac tctattgaat 56220
tacctgacac ctttgctaaa aatcattgac tgtatatgtg gccctatatt agtatatgta 56280
cgtatataca tgtaggccct gtgttgactg tatgtgtggt cctatattag tatatgtacg 56340
tatatacata ttggccctat gttgactgta tatgtggtcc tgtattagta tatacatatc 56400
agccctatat tgactgtata tgtggtcctg tattagtata tgtacatata tacatgtagg 56460
ccctatgttg actgtatatg tggtcctata ttagtatatg tacatttata catgtaggcc 56520
ctgtgttgac tgtatatgtg gtcctgtatt agtatatgta catatataca tataggcccc 56580
gtgttgactg tatatgtggt cctgtattag tatatgtaca tatatacatg taggccccat 56640
gttgactgta tatgtggtcc tgtattagta tatgtacata tatacatata ggccccgtgt 56700
tgactgtata tgtggtcctg tattagtata tgtacatata tacatgtagg ccccatgttg 56760
actgtatatg tggtcttgta ttagtatatg tacatatata catgtaggcc ctatgttgac 56820
tgtatatgta gtcctatatt agtatatgta catatataca tgtaggccct atgttgactg 56880
tatatgtagt cctatattag tatatgtaca tatatacatg taggccccat gttgactgta 56940
tatgtggtcc tgtattagta tatgtacata tatacatgta ggccctatgt tgactgtata 57000
tgtggtcctg tattagtata tgtacatata tacatgtagg ccctatgttg actgtatatg 57060
tggtcctgta ttagtatatg tacatatata catgtaggcc ctatgttgac tgtatatgtg 57120

gtcctgtatt agtatatgta catatataca tgtaggccct atgttgactg tatatatata 57180
tgtggtcctg tattagtata tgtacatata tacatgtagg ccccgtgttg actgtatatg 57240
tggtcctgta ttagtatatg tacatatata catgtaggtc ctatgttgac tgtatatgtg 57300
gtcctatatt agtatatgta ctgtatacat gtaggtccta tgttgactgt atatgtggtc 57360
ctatattagt atatgtactg tatacatgta ggccctgtgt tgagacacta ttgtgttttc 57420
ttgatatttt gtccattcct ttgctgatgc cacatcttga ttaatgtggc tttacagtaa 57480
gtcttaatat aaagtctctc aaatatctaa aatattactt tgaatattct aggtcctttg 57540
agtttcctta taaatttaag aagtagcttg ttgatgtcta caaaaatgca gtgtgaattt 57600
tggttgagat tgtgttgaat ctgtagatca attaaggaag aattggcatt ttaacaatat 57660
tgagtctttt gtatagatgg tatatctccg ttttaaaggt ccttttgtat ttcttttatt 57720
tctctactga aaaaaaatct ctacagaatt acattgtttt gtagttttca agaaaaagat 57780
cttgcatgcc atttgttaaa tttattccta agaattttac ttttggcact attttaagct 57840
gaaatagatt ttaaatttca tttttaatta tttgctgcta gtatgtaaaa atacaataga 57900
tttttgtatg taaatcttat atcctatgat cttttccaaa gtcatttatt actctggtaa 57960
tggttttgta ggtcttttag gatttaaata catagtttta cttctttctg atcttgatgc 58020
cttgtttttt tttcttgctt tattgcactg gttagagctg ccagttcagt attgaatagt 58080
agtgattagt ggacatcctt gcctgtgcca aacttataat gttaacttta gatttgtcat 58140
atatgacctt tgtcagattg aggaaattcc cttctattct taatttgctg aaattttta 58200
acatgaatgg gtatgatttt agtaaatgct ttttctgcat ctatggaaat gatcattgtt 58260
ttgtctttta ttcgtttaat aggataaatt acaatgattg atttttgccc cccgtttttt 58320
ttttttttt ttttaaagaa acagagtctc actcttgccc aggctgtagc acagtggtgc 58380
catcgtagct cactgcagcc tgaactcctg gacttaagca atcctcccac ctcagcctcc 58440
taagtagcag ggactatagg cacatgccac catgccaagc taatttttaa tttttaaaat 58500
tttttgtaca gatggggtct cattgtgttg ctcaggctgg tctgcaattc ttgagcttaa 58560
gcaatactcc cacctctgcc tcccaaagtg ctgagattac aggtgtgagc cactgtgcag 58620
gcctgatttt ttaatattga accaaccttg catttctgga agaagtccaa cttgcttatg 58680
agatatcatc ctttttatat attgctaaat ttgatttgcc cagtttttaa ggatttttgc 58740
gtgttgattc atgagggata ttggtgtggt attttctttg tttgttagtt tgtttcggta 58800
atgtctttgt cagcctcatt ttggcttcat aaaattagtt tggaagtgtt cccttctctt 58860
tgttttctgt gttttcataa ggttgatgtt gtttcttcct taaatgtttg atagaatcca 58920
ccaagaaaac cgtctggacc tagaatttc tactaggaaa ggtgattgtt tgtttttaat 58980
cacaaattca atttaatgta tagaaagagt tattcaggtt tttttacttc cccttgtctt 59040
acttttggta agttgtactt tgaaggaatt tgtccctttc aggtaagttg ttgaatttac 59100
tcatttaaag ttgttcgtaa tacaagcata cctagggttt gcttccagac ctcggcaata 59160
aagcaaatat tgcaataaag cacgtcacac aaactttttg gtttcccagt acatataaaa 59220
gttatgttta ggccaggcat ggtggctcat gcctgtaatc ctagcccttt gggaggccaa 59280
ggtgggcgga tcatttgagg ttaggagttt gagaacagcc tggccaacat ggtgaaacct 59340
catctctatt agaaatacaa aaattagctg ggtgtggtgg cacatgcctg tagtcccagc 59400
tgcttgggag gctgaggtgg gagaattgct cgaacctggg aggtggaggt tgcggtgagc 59460
tgagatcaca ccactgtact ccagactggg tgacagggcg agactccatc tcaaaaaaaa 59520
aagaaaaaag ttatgtttac actatactat agtctattaa agtgtataag atcattatgt 59580
ctttttgcta aaaaatgcta ataatcatct gagccttcag caagccataa tctttttctt 59640
ggtagaaggt ctgcctcaat gttgatggct gctgaccagg tggtggttgc tgaaagttga 59700
aggtagctgt ggcagcagtt tctttgtttc ttttttcttt ttcttttttt ttgagacaga 59760
gtcttgctct attgcccagg ctggagtgca gtggcacgat ttcagctcac tgcaacctct 59820
gcctcccggg ttcaagcaat tctcctgcct cagcctcccg agtagctggg actacaggtg 59880
cctgccacca cgcctggcta atttttgtat ttttagtaga gacagggttt caccttgttg 59940
gccaggctgg tctcaaactc ctgacctcag gtgatccacc tgccttggcc tcccaaagtg 60000
ctgggattac aggcgtgagc cactgcacct ggccggcagt ttcttaactg aagtttttga 60060
catgggttga ctctttcttt catgaaagat ttttctgtgg cacgcattat gtttgatagc 60120
atcttaccca caatagagct tccttcaaaa ttagagtcaa ttctttcaac cctggccacg 60180
gattgatcaa ataagttgat gtaatattct aaatcttttg ttgtcatttc aacaatgttc 60240
acagcatctt cgcctggagt agatcccatc tccaggaacc actttgtctg ctcatccata 60300
agaagcaact cctcatctgt tcaagtttga tcatgggatt gcagcaatcc agtcccatct 60360
ccaggctcca cttctaattc tagttctctg ctatttccac cacatctgca gtgacttcct 60420
ctgctgaagt cttaaaccgc tcatccatga gggttggaat caacttcttc caaactcctg 60480
ttaatgttga tatttcaacc tcttcttgtg aatcacaagt gttcttaatg gcatctagaa 60540
tggggaatcc tttccagaag gttttcaatt tactttgccc agatccatta gaggaatcac 60600

```
taactatggc agatactgct ttataaaata tatttcttaa ataataagac ttgaaaatca   60660
aatttatccc ttctccatgg gctgcagaat gggtgttaca ttagcaggca tgaaaacaac   60720
attcatcttt acatacatct ccgtcagagc tcttgggtca caaggtgcat tgtcaatgag   60780
caataatatt ttgaaaggat tcttttttcc tgagcagtag gtctcaatga tttttttttt   60840
tttttgatgg agtctcgctg tgtcacccag aatggaatgc ggtggcatga tctcggctca   60900
ttgtaacctc tgcctcccgg gttcaagtga ttcttgtgcc tcagcctcct gagtagttgg   60960
gattacaggc acacaccacc atgcttggct agtttttgta ttttttgtag aaacagggtt   61020
tcaccatgtt ggccaggctg gtctcgaact cctgacctca ggtgatccac ctgcctcagc   61080
ctctcaaagc attgggacta caggcatgag ccactgcacc gggccctatt tgtttttttt   61140
ttgttgttgt tttttaattg agacagagtc tcgctttgtt gcctagactg gagtgcagca   61200
gcgctatctc agctcaccgc agcctccgcc tcccaggttc aagtgattct cctgcctcag   61260
cctcctgagt agctgggact gcaggtgtgc actaccatgc ctggctaatt tttgtatttt   61320
taatagagat gaggtttcac caccatgttg gccaggctgg tcttgaactc ctgaactcag   61380
gtgatttgcc tgccttggcc tcccaaaatg ctgggattac agctgtgagc caccgtgctt   61440
ggcccttttt tgtttttttg ttttttttca aattgagaca gagtctccct ctgttgccca   61500
ggctggagta cagtggcacg atctcggctc actgcaacct ccgcctcctg ggctcaagcg   61560
gttctcctgc ctcagcctcc caagtagctg gaattacagg cgtgtgacac catgcctgaa   61620
taatttttgt atttttagta gagatgcggt ttcaccatgt tggccaggct ggtctcgaac   61680
tcctgacctc aagtgatccc cccgccccag ccttcaaaag tgctgggatt acaggcgtga   61740
gttgccgcac caggcctcaa tgggttttta atattcagta aaccatgctg taaacagatg   61800
tgctgtcaag tagggcctgt tattccattt ctgaagcata gacagaatag atttggcaga   61860
attcttaagg gccctaggat ttttggaacg gtcaatgagt attggcttta acttacggtc   61920
aacagctgcg ttagcctctc atgagagtca gcctgtcctt tgaagcttta aagacaggca   61980
ttgacttctc ccctctagct atgaaaatcc tagatgacat cttcttccag tagaaggtgg   62040
gacatctatg ctgaaattct gttgcttagt gtagccacct tcatcagtgc tctgagctag   62100
atcttctggg taacttgctg ccgcttctcc atcagcacct gctgcttcac cttgcacttt   62160
tacattatag agatggcttc tttccttaaa cttcatgacc tgacctctgc tagcctcaaa   62220
ctttccttct ccagcttcct cacttctctt agccttcata ccattgaaga gagttagggc   62280
tttgccctgg attaggcttt tgtttaaggg aatgttgtgg ctgatttgat cttctatcca   62340
gaccactcaa actttcttca tatcagcatt aaggctgttt tgctttcttc ttcttcatgt   62400
gtgcactgga atcgcacttt taatttcttt caataacttt ttcttggcat tcacaacttg   62460
gctagctatt tggcataaga gatctcactt ttgcctgtct tggctttcga cacgccttcc   62520
tcactcagct taatcatttc tagcttttga tttaaagtga gagacgggtg actcttcctt   62580
tcacttgaac acttagaggc cattgtaggg ttattaattg gcctaatttc aatattgttg   62640
agtctcaggg aatagggatg cctgagcaga gggagagaga gcggggaaca gccagtcagt   62700
ggagcagtca gaacatacaa cgtttattga ataagttcac tgtcttatct ggatgtggct   62760
cgtcatgccc caaaaacaac tgcaataatg gcattgaaga tcactgatca ctgggcgcgg   62820
tggcccatgc ctgtaatccc agcactttgg gaggccgagg caggtggatc acctgaggtc   62880
aggagtttga gaccagcctg gccaacatgg tgaaacctcg tctctactaa aaataccaaa   62940
attagccagg cgtggtggcg ggtgcctgta gtcccagcta ctggggaagc tggggcagaa   63000
gagtcgcttg aacctgggag gtggaggttg cagtgagccg agatcacacc actgcactcc   63060
actgcactcc agcctgggca acagagcgag actccatctc aaaaaaaaaa aaaaagggcc   63120
gggcgcagtg gctcacgcct gtaatcccag cactttggga ggccgaggcg ggcagatcat   63180
gaggtcaggg gattgagacc atcctggcta acacgatgaa accccgtctc tactaaagat   63240
acgaaaaaaa tagccgggcg tggtggtggg tgcctgtagt cccagctact cgggaggctg   63300
aggcaggaga atggcgtgaa cccgggaggc ggagcttgca gtgagccgag atcgcgccgc   63360
tgcactccag acagagcaag actctgtctc aaaaaaaaaa aaacaaagaa agaaaaaaga   63420
aaaaagagat cacagatcac catagcagat ataataataa tgaaaaagtt tgaactattt   63480
caagaatcat caaactccta cacagagaca cagtgttaac atgttgttgg aaaaatggtg   63540
ccagcagact tacttgatgc agggctgcca cgaaccttca atttgtaaaa agtgcagtat   63600
ctgtgaagca caatgaaatg aggtgtgcct gtgttccttg tcactgtaat gtcggtagga   63660
tccgtaagga aaacacttca ttcctttat tgtgacttta tgttcgccat ctccctctca   63720
tcagcagcct ggctaggagg ttatcaatct agatcttttt aaagaaccag cattggactc   63780
cccttttctg gtgtccattt ccttgagttt ctctgttatc ttcattactt ccttccttgt   63840
acttgcttca gcgcattagt cagggcccag tcaggaaaca aaaaccacac cggcaatttg   63900
aatagcaact tgtaatataa agtgttctta actgtaagag gtagttaact attaaaggga   63960
tcacaaaggg gtgacattgt gatataagaa agatgtattt gggctgggct cggtgcagtg   64020
gctcatgttt gtaatcccag cactttggga ggctgaggca ggcggatccc ttgagctcag   64080
```

gagtttgaga ccagcctggg caacatggtg aaacctcatg tctaccaaaa atacaaaaaa 64140
ttatcctggc gtggcggtgt gtgcctgcgg ttccagctac ttgggaggtt gaggtgagag 64200
gatagtttga gcccgggaag gcagacgttg tggtgagccg agattgcact actgcactcc 64260
agcctggatg acagagggag actctgtctc aaaaatcaat gtatctgtct gtctgtctgt 64320
ctatctatct atctatctat ctatctatct atctatctag tctctgtccc ccaactttgt 64380
ttggcatgta gctcctaaaa ctcttgaaat ctccaaagtg atgggtgtct ttttgtttgc 64440
taaggaggcg actggtggct ggaggctcct ggacagcctg gggataaggg ctggttgcca 64500
ggggacccag ccttgtgatt agaaggctag aacttgcagc ctttcccatc cacctcctgc 64560
gaggcaagag gggatgaagg ttgagttgat catcaatggc caaagatttc atgtcatgct 64620
tatgtagtga agcctccctg aaaacccaaa aggatgaggt ttgtagagtt tcaggttgtt 64680
gaatacctgc aggtgctggg agggcagtgt accctcccgt ggaagctcca tgccgcttcc 64740
ttcatacctt tcttatgcat ctcttccagt tggctgttca tttgtatcct ttgaaatatc 64800
atttgtaata agtcagcaat tttaagtaaa ctgatttcct gggttctgtg agtcactcaa 64860
gcaaattatt gaacctgaga agggagttgt gaagacctca aatttgtagc caagtcagac 64920
agaagttatg ggtaacctgg tgtcttacca cttgcgactg atctctgaag ttgagggcag 64980
tcttgtgaga tggaaccctt aacctatggg atctgcacta actctggttg gtgtcagaat 65040
tgaactgaat tgcaggacac ccagttgggg actgctgagt attggagaat tgcttggggg 65100
tcggcgggag gaaaccacac atctggtgtc agagctgaag cactgagtgt tgtgaatgag 65160
agtggagaga tagagtttgt tttgccttat gcaagaggac tctaaggagt atagaaagag 65220
caaatatgag aagcagctac tttagaagaa aacataggag gaaagcttcg tgacattgga 65280
gttggcaatg ctttcttgga tgtgacaccg aaaatacaac aaaagaaata aatgataaat 65340
tggacttcac caaaacttaa aacttttgtg catcaaggaa tactaccaat ggcgtgaaaa 65400
ggaaatccac agaatggaag aaagtatttg aaaatcgtat atccgataaa ggactagtat 65460
ccagaatata taaagaactc ctacaactaa gccacaaaac aaaaaaaaac aacccagtga 65520
acagacattt ttccacagaa gacataccaa tggccaagaa gcacaggaag agatgcgcag 65580
tgtcactaat catcagggaa atgaaaatca aagccacaat aaaatatgat ttcacaccct 65640
ttaaggtggc tgttagccaa aacatggata acagcaagtg ttggcaagga tatggagaaa 65700
ttggagcccc gtgcactgct ggtgggaatg taaaatggtg cagctgctgt ggaaaagata 65760
taacaattgc tcaaaaaatt aaactcagaa tgaccataac atccagtgat tccacttcta 65820
gatgtatact ccaaagaact gacagcaggg acttgaacag gtgtgctaca ttcttgtttg 65880
tagcagcact tttcccataa ccaaaagatg caggcaaccc aggtgtccac cagcggatga 65940
atgaacagac aaaatgtggt ccatctgtac agtggaatat tattctgcct taaaaaggaa 66000
ggagaggcca ggtgtggtgg ctcacgcctg taatcccagc gctttggaag gccgaggtgg 66060
gcagatcact tgaggtcagg agtttgagac tagcctggcc agcatggcga aaccctgtct 66120
ctgctaaaaa tacaaaaatt agccaggcat gggggcatgt gcctgtaatc ccagctacta 66180
gggaggctga ggcaggagaa tcgcttgaac tagggaggtg gaggttgcag tgagccgaga 66240
ccatgccact gcactccagc ttgggcgaca gagcaagact gcctcaaaaa aaaaaaaaaa 66300
aaaaaaaaaa aaggagattc tgacacatgc tatagcatag atgagccttg aagacatgct 66360
aagtgacaga agccagacat gaaaagacaa atactgtatg atttcacttt tatgaggtcc 66420
ctagagtagt caaattcaga gagacagaaa gtgggatgga ggttgccaga ggctggggga 66480
agggagaatg aatgggaagt tggtgtttaa tggggataga attgcagttt gggaagatga 66540
gatggatggt ggtgatggtt acacaacagt gtgaatgtgc ttcatgccac caaactgcac 66600
acctgaaaat ggttaaaatg gtaaattttc tgtgatggat actttaaggc atgtatgcaa 66660
acacacagaa gcagttacta ccactggggt tgatggaaaa gaaaccaaga actcggagcg 66720
tcctccacac cttgccacgt cttacgttca ggccttgttg gagagtgtgt gactgccaca 66780
gaaacctgca gcctactggt aacaaaaagg ttcttgccag aaggtgtaga aagagtggat 66840
ctgcaggatc agcttactgg gtagccgaga aacctactag agggtatggg tgggcaggag 66900
ctgggtgtct gagaagtttg ctgaggtccc attgggctag agctggggtg ctggagaaac 66960
tcagtacaga gcaggcccca ctgggcacac gccactggca gctgtgcctt cagaggaaaa 67020
agagaccctg gaaccaggaa gagaagctgc tacctcagca aggcctggca gctccccaca 67080
gtgttgtccc ctgtggatag agcctgacat gactgtattt ggccaaggag aaagactcat 67140
aaggcctcgg tccagtatca cggagcgggg cctggcagga tgtatgcaga gttaagaggc 67200
aatacatggg taactggcat atttttccag attctttgtt ttttttgttg ttttgttttg 67260
ttttgttttg ttttgttttg ttttgagaga gcatcctgct ctgttgccca ggctggagtg 67320
cagtggcatg atctaggctc actgcaacct ccacctcccg ggttcaagcc attctcctgc 67380
ctcagccttc taagtagctg gagatgccca ccatgcccag ctaatttttg tatttttagt 67440
agagacaggg tttcaccatg ttggccaggc tggtcttgaa ctcctgatct caggtgatcc 67500
acctgccttg gcctcccaaa gtgctgggat tacaggcgtg agctacactg cacctggccc 67560

```
atttttccag attcttaaag cagatcctta ggtcttttt ttttttttt ttttttttt 67620
tttacttctt ttctaatata agcacttaca gtttccctct aagcactagc ttcagcctac 67680
gtattttagt atgctgtgtt ttcattatca tttcatttga aatattgtaa aatttttctt 67740
gtgatttcct ctttgactca tgggttattt agaagtatgt tgtttaattt gcaaatagtc 67800
aaggctcctc tgagtatctt agagctattg atttctaatt taatcttact ttggccagac 67860
aagcatattt tgcatgcttt taaaatttga gacttatggc ccagaatgtg tatcttggtg 67920
aatgcactgt tttcacttga gaaaaaaata tgtatatata ttctagactt cctgagtata 67980
gagctttata actgctgatt aggtcaattg ctagttacca gtgttcttca gattatctga 68040
tgcttgtttc tcagatgttg agagaatggt gtttgctatg gcctacctgt ttgtgtcccc 68100
caaaattcat atgttggagc ttaatctctg gtgcagtggt gttgggaatt ggggcatcag 68160
ggaagtattg gctcatgagg gcagagtcat cgtgaatggg attagtccct tctgccatgt 68220
ggggacacag caggaaaagg acatctctga agcagggagc tagctctcac cagacactga 68280
gcaccctgat tttggacttc ccagcctcta gaactgtgag cactacaatc tgtttataaa 68340
ttacccagcc taaggcattt tgttttaaca tcctgaatga actgaggtgg tgttaaaatc 68400
tccaactatg catccaggcg cagtggctca cacctgtaat cccagcactt caggaggcca 68460
aggcgggtgg atcacctgag gtcaggagtt tgagaccagc ctggacaaca tggtgaaacc 68520
ttgtctctac taaaaataca aaaattagct gggcgtggtg gcaggctcct gtaatcccag 68580
ctactcggga ggctgaggca ggagaatcgc ttgaacccag gaggtggagg ttgcagtgag 68640
ccgagattgc accattgtac tccagcctgg gcaacaagag tgaaactccg tctcaaaaaa 68700
aaaaaaaaag agtctccacc tatgattgtg gaattgccca tttctccctt taattctttc 68760
aggttttata tcatgtattt tgaagctcct ttggttaggt gcatacacat ttatgattat 68820
aatggtgtct cgatgaactg atcatttaac catgatgaat gtattccttt atctcgtaat 68880
aattttatct tggactcaac tttattgata tttatgtagc ctctccagcc ttcttaaaca 68940
tgctgtttgg gatgcataag aaggctggag aggcttatgg agaaacttat gatggtttga 69000
cttacagttt ttcaacttta ccatggtgcg aacgtggtat gtatacagta gaaactgtac 69060
tttaagtatg tgtgaaacca ttgtgtttct cactttcagt acagtattca ataacttaca 69120
tgagatagtc aacactttat tataaaatag gctttgtgtt tgttgatttt gctcaactgt 69180
aggctgaagt aagtgttcta atcatgttaa ggtaggctag gctaagctag aatgttcggt 69240
aagttagatg tattaagtgc atttttgaga tgatatttat tttccactta tgatgggttt 69300
gtcaagacat aactccatca tcattcaagc agcatcagta tgtctttttc caaacagaaa 69360
atttcaacct gtgtttatac tgaaacagca tcttttgtag atagtatata gttgagtctt 69420
gctattttat tcattctgac agcctccgcc ttttaattgg aatattcagg ctatttctgt 69480
ttaacatagt tatagatatg actgggttta gagctactac cttgctcttt gatatttatt 69540
tctttcatct tgctttcttg gcttctttta tgttaattaa gtatttttta gaattctatt 69600
ttaatttatg ttttttttt ttttttttt ttaagacaga gtctcactct gttgcccagg 69660
ctggagtgca atagcatgat cttggctcac tgcaacctct gcctcctggg ttcaagtgat 69720
tctcctgcct cagcttccct agtatactgg gattacagac gcctgccacc acggccggct 69780
aatttttgta tttttagtaa agacggggtt tcaccatgct ggccaggctg gcctcgaact 69840
cctgacctca ggcgatccgc ccaccctggc ctcccaaagt gctgggatta caagcgtgag 69900
ccactgagcc cggcctgttt tgacttttta attgtatctc tttgtattgc tattttagta 69960
gttactttat agattctatt gcacattctt agcttttcac agtctactta gaattaacat 70020
tgtttttctt catggaaaaa aagtgtaaga actaggcaat cctatgggtt catttaccac 70080
tttccatcct gtatgtgatg gttgttagat atttactaca tccatacaca ttgtaaacta 70140
gacaatacaa taaggaaatt tttgctttaa acagccccat gcattttaaa gaaattaaaa 70200
gtaaaacaga atcttttcta tttgcccatg tatttgctat atcctgtgct cttcattcct 70260
tcctgaagag ctgagttccc atctggtctc atttcccatg atcttggaga acttgcttca 70320
cggtttttgt agtgcaggtt tgcagacagc taattccctt tcattttcac ttatctgcaa 70380
atctttattt tgctgtcatt ctttcttttt ttatttttat ttttattttt gagacagagt 70440
ctcactctgt ctccccacag gctacagtgc aatggtgtga tttcaaaact cctgcccaat 70500
ctcaactcac caaccttctc ctcccgggct caaacgattc tcctgcctca gcctcccaag 70560
tagctgggac tacaggcatg tgccatcata cccagctaat ttttgtattt ttagtagaga 70620
tgggatttca ccatgatggc caggctggtc tcgaactcct gacctcaagt gatctgcctg 70680
ctttggcctc ctaaaatgct gggattatag gtgtgagcca ctgtgcccag cctgctgtca 70740
ttcttgaacc ataatttcac tggttataga attctgagtt gtcagttttt tctttcagga 70800
ctttaaaata tagggttcca gtgtctttgg cctccgttgt ttctgtcaag gaatcagcat 70860
tcatctagct attcccctgt agaccacatg ttgtttttcc ctggatgctt ttaagacttt 70920
ttatccaagt ttgattgctc agtacctacc tctgggcaaa aaagcctcca gaaaccagaa 70980
acttatctag ttttattccc ctctgtcagg tgtagactct actccagctt ctacctactt 71040
```

gagatattct atactgactc caggctgttg ttattttgtt ctttcaaata ttttgtccag 71100
agctataatg ttttctgcag aattgaccag atagcagcta ctctctgtga ccaggagcct 71160
tgatcttcca gacttgctga tgggctcaca aagctcctgc ccgtggccct cagttggcag 71220
tggcctctct gccttgaagt tgccatgagt gtgtaaaacc tacaggctca accttcctca 71280
ttagtacagt ccagaaaagt ggaattcctg gtctgggtgg ctatggtgcc ctagaccact 71340
ggctagacca gggtggtgac tgccagctgg ccgaatgtgg ggtctccagc ctagcaccct 71400
tggactctac cccaggagtt atggctgttt cagggccagg ggcctggcct cagcccctgt 71460
cccccttctc ttctcctcaa gttgcccagc atcaggtccc aaccagcatc ttttgcagac 71520
agggtgggtc ggctcctcac tagaggggca gggttgggcg ggaagcctgt tgagccctcc 71580
ttcctgtgca gcccacagcc ctggggttag ctccagaggg gtgcagaaag gagcccccaa 71640
aggcagaaac tcacccttct ctctgttctt gtgcttccag ggataaagga gaggtggtcc 71700
tagctggggg gctaccagtg gcatgcagga ggcccacatg accactgttt gctggggcct 71760
ttccttctct gtgagagaga cgagagagag gagagagagt gtgagtcagg gtagtgtctt 71820
cttccagtaa cgcagacttc tgcacgtgtt ctctaggaga aatcccgtca ctcgctagag 71880
ctctagatct ggaagaagct ctgcctctct tctctcctcc agtgcagtgt gtgtgtgtgt 71940
gtgtgtgtgt gtgtccccac atgcattaca cccttctaca gaggaaataa ttcccgtcca 72000
agtgcaaagg ttgggattgg agcaggcagc atgtgggcta aaggagcaga gaggaggaaa 72060
cgggggcggg ggtgagggtg ggaacaggag gtgggtagat gccacgaggc atgccttcca 72120
caactagaag ggctggtgga gatgaccaga atgctccaga cagacctttt atcaaacttc 72180
caaaattcaa tcttgtttca tgaagcctga ggaccctgca gagtgaaaga caaggaaggg 72240
ctttcctgca ggggggtgtg tgtgcctgtg tgtgcctgtg tgctgtgtgc ccccatgtgt 72300
gtgcacgtgt ggacctgtgt ggtgtgtgcc tgcacatgtg tgcacgtgtg gacctgtgtg 72360
gtgtgtgctg agtgtgtata tgtgtgtgcc tgtgtgctgt gtatgcactt gcacatgtgt 72420
acacagatct cattttcctt ttttctggct tactaagtgc cagaaagaat ttccgtgagc 72480
ctctccaccc ttcccaaggc tgcacatgga gggacttgcg caggcacatg tgcgcgtgca 72540
cacaacacac acacacacac acacacacac acacacacac acacacgagt caggggcgag 72600
cactcctgtg gattgtacct aggcctggca ggaaaccggg ttctgggaaa ctgtaggccc 72660
cacctttggc agaaaaccat gtttttgttg ctgctcgttg aagggaacat taagtggggc 72720
tggggacagt ttatgttgag aagagcaaat tttgcctatg gcactgggga cattgagtgg 72780
ctcctgtccc gggctctgga gggcttggga gacacgtctg cagctctggg catctctcaa 72840
ggtcacgggc ggccacactc aaggttaggc cctcctctgt gtgagctgcg gtcagcagga 72900
aggctgacct cagaggcggc cccagggccc ttggaggccc tgcccctgag acatgggcca 72960
aggtcagaga gccgagaagg ctgctgctga ggactctggt tggcgcccag cccagcggcc 73020
cttcaggcgg gccctgtgca ggggctgatg aagtcagctg ggaaatctgt gaaataaagc 73080
acaggtcccc caggcttgcc cagagccccg gcatcagatg aggtgcagag aagggaactg 73140
agctgccaag tgtgaacagg agagaggagc cccaggacca cacaggggcc ccgacatggc 73200
aggtgcttgg tcagcagcgg tgctgccctt actgctggac gtgggaaaat gcactgacac 73260
ttgctgacca gagccggggt gagcatctgg gtgaccctgc agatgagaca aaataagggc 73320
tgccgtgggg gaggggccct tgccgggcca ccctgctgag gccctgtgtt actgtcctgc 73380
ggctgtcata acaaaggacc acaaatgagg tgactcaaaa catccgaaat cggccacgtg 73440
cggtggctca tgcctgtaat cccagcactt tgggaggccg aggcggttgg atcacgaggt 73500
caggagatcg agaccatcct ggctaacatg gtgaaacccc gtctctacta aaaatacaaa 73560
aaaaaaaaaa aaattagccg ggcgtggtgg tgggtgcctg tagtcccagc tgctcaggag 73620
gctgaggcag gagaatggca tgaaccttgg aggtggaggt tgcagtgagc cgagatcgcg 73680
ccattgcact ccagcctggg tgacagagca agactccgtc tcaaaaacaa aaacaaaaac 73740
aaaaacaaaa acatccgaaa tgtcttccct aaagtttctg gagccagaaa tcccagaccg 73800
aggtgtctga aggttcattc ctcctggggg ttccgaggga gactgtccca tgcctttctc 73860
cgagctctgc ggcggccagc aacccctggt attcctcggc ttgcagctcg gcctaccccc 73920
cacctggcct tcaccctgtg tctcttcttg tgtctctgtc caaactgccc tctctgttgt 73980
ctttttaatt tatttttagg gacaggttct cattctgtcc tccaggctgg agtgcagtgg 74040
cgtgatcatg gctcactgca gcctcagcct cccaggctca agcaatcctc ccacctcagc 74100
cttccaagta gctgggacac aggcgcacgc caccatgcct ggctaatttt ttaatttttt 74160
tgtagagacg agggcccagg ctggtctcaa actcctgcgc tcaggcaatg agactttgcc 74220
tcccaaaaca ctgggattag agacatgagc tgctgtgccc agcctacctc tgtcttataa 74280
agacacctgt cattggattt agggcccact ctgattcaat atgccctcaa ataacccaat 74340
tacatcttca aagaccctat gtccatataa agtcacattc ataggtactg gggggtcagg 74400
acttgcatat gtcttttggg ggaacacaat tcaacccaca atgggtctca cccacagaac 74460
gaacggcctc tccagggtgt gcgcaggtgg gtgaggggcc caggagaggg acctgcgtgg 74520

caagagggct tgtgttcacg tcagagattc cagaaggaga ctggatgggg tggctgcatg 74580
gagcaagggg ctacagccac tgtctccaag tagttggtgg gttgtctggt agaaagggag 74640
catttatttt gagttccccc aagatatgaa gctagtacca ggtacctgga aggtgctagc 74700
gagtagagct tgactcttta cagaaagagg tgtttttctc acaatgttca cttgaggacg 74760
gtggaacagg ctatttcaca aggtagtgag ttccccatgc ctggaagcat tcaagcaggg 74820
gccatgtggg agttgttcag gagtgcatag aagggacctc agaggtccct gccaatacca 74880
tctttgcttg tcttatttta cgacaacttg aatttcagaa tgtaatgtta ttggggatat 74940
gtctactaga ggggcctcca gcatcaggcc ccacacacta tgcatcattt ttagggcagg 75000
gaatgagagc aggctccatt ctttttggtt gtctgtgagg accaggaggc ttgctgctga 75060
ggcagcttcc tgtggaaaca gtgtctcagc cccgcaatcc attcatcgga gctccaacat 75120
cttaatgaaa cactgtgtca gatgggttct gggcatgtgt gtgcagattg tcacaagtcc 75180
atgagaatgc acacacgcac attgcatgtg tgtgcacaca tgtatgcaca catgcacaag 75240
aacacgaaga cccgaggcag gtatagtccg tgagacagat ccctctaggc tgaaagactc 75300
cccacaaggg tccctagaac aaggctccag cctcggcagt ccctcccact tctggtggga 75360
ggatctccac agagaagaag ggtcaagaag cacagctctt gatgctcacg gtgtaccaag 75420
ctctgcttta agcttgtgtc ttattttta cttatttagc aaatctgtcg ctcttgtttt 75480
gtgccagatg ctgttctcag cactttacaa atattgatgc atgtttgttt catgacaacc 75540
ctgtgaagta ggtagtgtta ccccgtttta tagatgagga aacaggcgta gagggataaa 75600
gggacttgcc caaggtctgt ctctacctta cacgcacatg aacgctcact catgcggcag 75660
gcgctagttt ccctctgatg tgagtcaggc cttgagctgg ttggggtctg ggtcccactt 75720
tggttgtgtt ttgctgtggg caccaaggaa gcttctagca gcctcatctc cctggtatga 75780
cctagctcca gggactggat ttccccaaat gctcctgccc agcctggagc ctcctttcca 75840
gctgggggag ggctgaacga cccctcactc ttggaggctg ccggagcccc tcctgcctgc 75900
atgcggccgc tgccgggtgg acaggcctgc ttgtgggttg aggctcccag cagctggggt 75960
ggatcatttc tcctcccaag aagctttggg agaagcagct gaggggggca gcgtcctcct 76020
gaggccaatt ccctgcctcc cccagcgcag gcgcgaagcc atctttgctt gtgtgtttgc 76080
cacagacagt ggagggcggt gcaatcgtga cgcctgcttg ttgcactttg cgtccttgag 76140
gacgttgacc cctggccggt gctgggcgct tgctcttcag tcatcttgcc ctgacctggc 76200
gtggcaggcc cgagcactgc tcttttacaa ggaaacaggc tcagaggcct tgagtcactt 76260
gcccgggctc ccccagccaa taattggctg agccaggatt tccatccagc tctgtgactc 76320
cagaagtcca agttcttccc atcacgtcag ggccaggaaa ggattagaat gcttttctac 76380
gtgccgatta tttggatgat ctgtgcagct tagctcgttg tagttgctca ataaaaatgt 76440
ggaatgaatg ggcctatatg tcccattgtg tgactcagag ccaatgactc tggctgatcc 76500
tcctgttttc attccgtggc ccagcatctc cctttggggt gtgtgacctt ggaagcagtg 76560
actctctcca tcccaccttg aatggtgtct gcgccgcagc aggggagcag ctggggattg 76620
ccagcgcctg ttgctggtgg gctgtgcgcc agatcccgtc ttggatgtgt gggtgattgt 76680
cagccacatg tgtgtgaagg gctgtgcgtg taggatttgt gcaaatgtgc ccctccctgc 76740
tgccagccca tgggaggctg tcaagattct gcccctgcac attagcccac actctctttg 76800
tggcccactg ctgtggctgt tcccttgtgt cacgtggctg caggcacagc cccccaagtgg 76860
atggggatcg tgggctgaat gtgcatcaga tgctcctgct tcttggtggg tggagaccct 76920
caacccatgc accgtggctg ccgcatctga aaggccttcc atggtgaagg acttggacct 76980
gagcaaccct ccgcagaggt cttagaagtg ctccctggcc gggcgcggtg gctcacacct 77040
gtaatcccag cactttggga ggccgaggtg ggcggatcac aaggtcagga gatcgagacc 77100
atcctggcta acacggtgaa accccgtctc tactaaaaat acaaaaaatt agccgggcgt 77160
ggtggcgggt gcctgtagtc ccagctactc gggaggctga ggcaggagaa tggcgtgaac 77220
ccaggaggcg gagcttgcag tgagccgaga tcgcgtcact gcactccagc ctgggcgaca 77280
gagcgagact ccgtctcaaa aaaaaaaaaa aaatgaagtg ctcccagctc ttcctcctgt 77340
ggtgaggttc tgagtttcca tcctcagaag aaaacctggc tggggagggc atctctagac 77400
ccggacataa acgccaggag accagggctg ccctctcagg tgaccaatca cagagactca 77460
gggaaagtca cgtttggaag aggcgacact gtgtctcagc ctcattcatt tctccatcct 77520
acagacactg cctggtgccc agcccgcacc gcgaggcttc ctaagcagct ctgggcaccg 77580
tgcacctggc atggcctcca gccccatctc cctgccttgg gagacccggt ggggactccg 77640
ggctgagctc ctgtggctgt ggaaccttgg tggtttattg tggtggctga gaacagggac 77700
gtggggttgg acgggttgag tgtaactccc agtggctccg tgaactgcct gggagccttg 77760
ggcagctctt tctcactctg tgcctccgct tcctgatgga aaaatggggg ccaggggggga 77820
gcgaccttga aggtattcgt gggaaccaag cgaggtgata aatgtagtgt ccttggtaga 77880
gaactggaca cagagtccac ccccatgaag gggctgctcc ctcctccttc tatgtttcct 77940
cttgttctta tttctcctct tgtatcttct cttcctgctt tccctccccc tcccctcccc 78000

ccccacatgc aggcacactc catttggtgc cccaactgcc tttctccatt gtccttctgt 78060
aagtggttct ctaagtggga actcaggcct ggaaagggtg ggcaccctgt ctggtgggct 78120
ctggcaggcc aggcgctgag cagcctcagg agacacaacc ctaggaaaag gctcatgcag 78180
gctggctgtc atgacggact gttggtggga gaggggaagg cgccgtggta ggggaagctt 78240
tggaaacatc cactagtggc cttatttatg actcaggaca cctgccaggt cactagaaca 78300
cagctgagga agctgtcggc cagacgtcca gaagcatcaa cctggtggct gggctgtggg 78360
gggacctctc tggacaggca gacaaggtgt cggaggaagg ggctgacact cactcagtgt 78420
ctgtcatgca ctgagggcac aaagggcagg atggagagct ggcccacgcc acctgtggaa 78480
tcttccaggt gtgggggcag atggggaagc aagggggctt gaagacacat gcgctcatta 78540
aatgtcctta aatcccaaga agaagaacaa ggacacatcc acaggggcag ggctgaaagt 78600
ctgatgcatg gtgttttggg gtctgctcct gctgcaacag ctacagtggc tccctcttac 78660
ctttctcttc cctccattct gttgtgaaaa tgttcaaaac acatggtcaa gttgaaagca 78720
ttttacaggg aacacattca gccatcacct agattctacc actaacacca agctacacct 78780
gctttatcac ctcaccgtcc atctatccct ccttcgcctg gccatgaatc cattttattt 78840
ttattttttg gtgcatttca aagtaatttc aggtcatcct gattatctct ggaatcactg 78900
tcagaaattc cctcccttcc cctcccctct cctttccttt ccttttctga gatgggatct 78960
tgctaggtca cctaggctga agtgcggtgg tgcagtcata gcacactgca gcctccaact 79020
cccgagctca agcaatcctc ctgcctcagc ctcctgagta gctgggaccg caggcatcca 79080
ccaccacacc cagcttcatt ttcaaaattt ctcaccaact cctcactcct tgtattcacc 79140
caggaccact tctcacaaag ccagggtgct gcaggagggg tcagaacccc aagccctaat 79200
cctggctctg tcattaacac tgtgcgaccc tcagcaaatc attttgcctg tctaggtcct 79260
gttttcattc ctatgaaacg agggcgttaa actgtatgtt ataaataatg agggctacca 79320
gttaccaagc tcctatttta ggccaggcac ttttcacatg ttattatgag tcctttcaat 79380
ctaggtattg ttctcaatgg acagcttagt caacggaagc tcagagaggt ggtgtaactt 79440
gccaaaagtc ccactaccca gtgaatgtcc ccacggggtc tgcacccagg agtctgacac 79500
agagcccagg cctcagcacc tggcgatgtt ttgggggtgt gagcagccca gcctactctg 79560
ggcacgtgtt tacttgctgt tccttctgcc tcatgtttgt gtttgtcccc tctgaagctc 79620
agactgttat tttccagttc caaataccaa aactggctag gcacggtggc tcatgcctgt 79680
aatcccagca ctttgggaga ccaaggcggg tggatcactt gaagtcagga gttcaagacc 79740
agcctggcca acatggtaaa accccatctc tacaaaaaat acaaaaatta gccagacatg 79800
gtggcgtgca cctgtagtcc cagctactca aggagaaccc agctacccag ctactgaggc 79860
acgagaatca cttaaacctg ggaggtggag gttgcagtga gccgagatcg caccactcct 79920
ctctaggcta ggcaacaagt gtgagactct gtctcaaaaa taataaataa ataaaataaa 79980
ataaaaatta aaaccaaatg ccaaaaccaa cccacaaaat aaaccttggt taaagtagcc 80040
tgttcatttt gctttaacta tgtatattgc accagattct gccaggggct cagcgtataa 80100
agatgagacc cctgccctgc cagacaggtg ggatgatggc cataccttga gttagtcaag 80160
gcaccatctg gctgtgtaac aaaagctaaa acatcagagg ctgagcagca ctcggaccca 80220
cgtcccagtc caagtgtcag tcgttatggg ggcccaggac agcccggacc tagttcattc 80280
ctttggtgca tcctcgacac acagcttcca tctcttgagc tgagatggtt ccagctcctg 80340
ccgtcatgtc tgcactccag ccagagggaa gggaagaagg gagaggggaa agggcaagtg 80400
tccctcttta tttttaaggg catgacctga aagttaaaca cttcagctcc ttcccactgg 80460
ccagaaccca gttgtatggc cgctcctgtc atcagggagg ctgagcaatg tattcttcag 80520
ctgggcttcc ctgtgcacaa ccaaaacttc agttactata gaaaaaaggg agctcagata 80580
ttggggaaac aactagcatc tgtaccacat gcactgatag ctctctttgt atgaacagag 80640
ctgtggcagg ccctatgcca gggagaaagt aagattggaa aagagcttac caaggaggtg 80700
gcatttgcac tgtgcttaag gggcaagaaa aacgtcttcc aatcaggagc cacaaatgct 80760
tggctgaagt gctactgctc tttcatcctg gagctggaac agacgtcacc agtcaatcat 80820
gatggctgct gggtgcactg gctaacatct ataatcccag cactttgtga ggctgagggt 80880
gggaagattg cttggggcca ggagtttgag accagtttgg gcaaattgca agaccctgtc 80940
tctgcaaaaa aatataaaat gtagctgagt gtggtggcac ctgtagaccc agccccagct 81000
actcgagagg ctgagatggg aggatcgctt gggcctagga gttcgaggct gcagtgagct 81060
atgattgcac cactgcactc cagcctgggt gacagagcag gacctgtctc taaaaacaat 81120
aaaataaaat caaagtttta aaatggaaaa aaaatcatga gcacataact ccagatgtat 81180
cctcaagata gcaaacccgg gtgggcgcag tggctcatgc ctgtaatcct ggcactttgg 81240
gaggccaagg tgggtggatc acctgaggtc aggagtttga gactagcctg gccaacagcc 81300
ccatctgtac tacaaataca aaattagctg ggcatggtgg tgggcacctg tgatcccagc 81360
tacttggaag gctgaggcag gagaattgct tgaacccagg aggcggaggt tgcagtgagc 81420
caagatcaca ccattgcact ccagcctgga gaaaaaaagc aaaactctgt ctcaaaaaaa 81480

```
aaaaaaaaaa gcaaacccag ggccaggtct gtagccacat ggccaccttc ttccctgagc 81540
atggaggcag cctcagaaac ctgccccaca tcacatcagt gggcagttga ttgggtcagc 81600
ctgccagttg aaccctattt gccacagtca ttctccatag taaggggcat tctagacata 81660
cccccccttcc cagggaagaa taagccgtag ctgcctgagc gttttatcca gtggtgtgca 81720
tgtcttttcc caactctgtg tttagtgatg tcctgtttgt agcttgaaat ttgccacagt 81780
gggtgtgttt acaccacagg aattggcaaa cactacatat caggccttac ctcctgctcc 81840
ctactagggt aggttgtttt ttttttgttg tttttgtttt ttgtttcttt ttttttttga 81900
gacggagtct cgctctgtcg cccaggctgg cgtggagtga tgcgatctcc gctcactgca 81960
agctccgcct ccctggttca caccattctc ctgcctgagc ttccagaata gctgggacta 82020
caggcgcccg ccaccacacc cggctaattt tttgtatttt tagtagagac ggggtttcac 82080
cgtgttagcc aggatggtct cgatctcctg acctcgcaat ctgccctcct cggcctccca 82140
aaatgctggc attatgccac tgcgcccggc ctagggtagg ttgttagaaa tgtaccagcc 82200
tgccagtttc agccttgttt gtctttcatc cttttttttt tttttttttt ttttgagacg 82260
gggtctctct ctgttgcccc ccaggctgga gtgcagtggt gtgatcctgg ctcactgcaa 82320
cctctgcctc ctgggatcaa gtgattctcc tgcctcagcc tcccaagtag ctgggattac 82380
aggtgcccac caccacgtcc agctaaattt tgtattttta gtagagatgg ggtttcacca 82440
cattggccag gctggtatcg aactccttac ctaaggtgat ccacccgcct tggcctccca 82500
aagtgctggg attacaggcg tgagccaccg cactgggcct ctttcgttct ttcttaatga 82560
aaatcttcct gaaagatttg cctactgatg tctgtgtcaa taggtactgc ctgcgggaca 82620
gagcctgccc tccttttgaa ggtctttctc tgtctgggtg accatctgca tgtcactccc 82680
tgctgctcag aactaaagat gtagctatgc ctggggtagg ttgggaaaac ctgtgggaat 82740
cacagagctt gctgtcatgc tgtgttctgg atacctgaag cttggcaggc aggtgggatc 82800
gagggtgggc attaggaaag tgatgtggct ctataggaag gggacatagg tcctgagatt 82860
ttaggtcccc aattggacaa agctgttttt tttttctgga taaatgttct agcttattca 82920
taatcgagtg gtcagttttc ttttcttttc ttttcttttt ttttttgaga tggagtctcg 82980
ctctttcgcc caggctggag tgcagtggcg ctatctcggc tcactgcaag ctccgcctcc 83040
caggttcacg ccattctccc gcctcagcct ccggagtagc tgggactaca ggcgcctgcc 83100
accgcgtccg gctaattttt tgtattttta gtagagacag ggtttcaccg tgttagccag 83160
gatggtctcg atctcttgac ctcgtgatcc gcccgcctcg gcctcccaaa gtgctgggat 83220
tacaggcgtg agccaccgcg cccggccaag tggtcagttt tctcagctgc cgggtcttgc 83280
acaccttgct ggctctggag accatatgca ccatgtagac cccatttgtt tcctaccctc 83340
agtgttgggg gaggggccaa gggcctgtcg atgtgttgac acctggccat tcactggcct 83400
tgaatgtcca cattcacctc cttgaaatcg atgccccgta tcggaggctc catgtggatt 83460
tgggggcaga ggcctgtact ccagatggtg gggtggcctt caccaatcag gcagattccc 83520
tgccctgccc tgccctttgg gctctgtagc tccagatgtg tgctcaagat ctgtggccac 83580
atggatggga caagccacag agactcagac tccgtggtcc ctgtgaccag agtagccgtg 83640
gccctgtctg ggaatgttcc caccctgttg ctgaaggtga gaaatttctc tgtgctggag 83700
aacagctgtg ctgagaagtt ggcccatctg gcttgcagga ctgctggggc aagggaggtc 83760
tgctgccggt ctcttccttc ctcctctgac tgggtgctcc ttgtctaggt tccttgcagc 83820
aaaagaattc tctaatttat tttgttccct tattgagtct ctactccatg aagcccacct 83880
cctaatccca gctgactggc tcccacctcc tcaagaggaa ggccaggggc agacgaggct 83940
gaaggaggct gactcccctg tgccttcgca gtctgagctg atgctgctgt atgttcacag 84000
ggctccttca gcttttaaaa aggcttcaac acgttcgtca cacgcttgtg gctctcctgg 84060
atgccacact aactcagtaa ggcctgagtt agaggctgtt cctgaggctg tgaaaataga 84120
agaagggtcc agaggagaat cccttcatct gacgatccca ggaaggcttc ctagaagaag 84180
cagcctctga gctgagacct gaagacagat caggcctgag tcaggccagg gagggtgtga 84240
ggggcccacc aggcagaggg aacaggatag ggcaggcaca gaggtctggc acctggcttg 84300
tggggaagtg tgagcccctg ggcatgacca aggcggggag aagtggcgtg agaggaggca 84360
gatgtggctg gcaggagatc ggcggccccg gccttgctga gctccactct gtatgcacca 84420
ggtgacagag gctgcccagg acggtgggca gggaagtgac aggtcacatg gtcatcccac 84480
caacagccgg gagtccagct tgggggggacc caagaccctg gggaaggcac gagtcagggg 84540
cctgctagag ccaggagcta gtgggagaag gaaacagtgt tgcttatgaa ttataaacag 84600
acactcccgc aactttctct gacgtgatac aatctcaaag tgggccaccc agaggtggga 84660
aggtgacttc tggctctcca ggccttgggc ctgtcttttt tttttttttt tttttttttt 84720
tgagatggag tctcactctg ttgcccaggc tggagtgcag tggtgtaatc ttggctcact 84780
accacctctg cctcctgcgt tcaagcaatc ctcctgcctc agcctcctga gtagctggga 84840
ttacaggcac ctgccaccac acccggctaa tttttgtatt tttagtagag atggtgtttc 84900
accatgttgg ccaggctggt ctcgaattcc tgacctcagg tgatccgccc accttggcct 84960
```

cccaaagtgc tgggattaca ggcatgagcc actgtgccca gccgagccca tcttcaagtt 85020
ctccctggcc ctcagtgttt ctgtattagt tcacccttttt ggctccctgc ttttaagagt 85080
ctcccttaag actgtcccca agtggacagg tccccagaac caaggagcag ccttaggagt 85140
ccccagttac agacactcca gggggtccgt gtggccctca gcctctggcc tggttccggc 85200
agccttggtc ctttgtgctg tctctcacat agcctggcca gagctggcca ggtcccttgg 85260
ctgggccgag tcatcgtggc cagtgactca tgcaggggga gaaaagtgag gagggggaac 85320
agagtgcgag gggactgtg ttaataacaa acccattagg tccctgaggt cacttcacag 85380
gagggacaaa tggttcattt aggttgatct ggctgccggc tggcttccag agacactcag 85440
ggtggggaag atttcattat ctggggaaag caataccaga ttgtctggag gggcccagcc 85500
agcgcacccc gcgcttcctg tctgcctccc acgcagagcg ttctgttccc aaggcggcag 85560
gccaagagcg cgagccgagg ctcaggtgca aagaggggtg cggcatgggg gcactgcagg 85620
gcacggggtg catgcatttt tatttatttt tttactgagt gttagggtaa tttgttaatg 85680
gggtatattt ggtgacataa aaccttctgt cgattcatgg cagtctggaa cggagtggaa 85740
gcgtcccact cctggggaac ccccacacac atttggctgt gaacgtcggt gcctttgcac 85800
tggagcctct ttggagtgtg ctgggcagag cggagaggtt cctcttctct cttctccatc 85860
atcctgtgga ggtgtcaaca tgtagccaga gatggtcagt ggcagtgagg agagggggc 85920
aggaagagac cagggctggg ggcttgccag cccacagttc taccctgaa cacccatggc 85980
ctccctgttc aggtcagcga ggctggggc agagacgggc aggggtcccc caatgttggg 86040
catctccccg ctccctcata ctctggattt gccctctgga gaaagctctg gagcacccag 86100
aagtcctggg gctgctggct gaggtgggga gcagagccca gctgtgcctg gaggctcccc 86160
acttccactc agcttccagg gacagtctca gctgtgcagg cccaggccgc tgaggctggc 86220
tccagaaatt tcttcacaac caccactgga tggaaaacca gttctctaaa gggccccaga 86280
gccagttttg tttcttcttc cacaggttcc catagttaga gcatcgggtc tttgttagag 86340
atgcagaaga gacgatgtcc ccagccctta tgtggttcgg tctccactgt ggatgttggt 86400
gatcttagtc tccttctcaa aaccaagagg gctgatgccc cacattccag gcccccagaa 86460
agagccgcca cccacccaca ccgctacatc ttctttccac ttttctgttc cggaaagagt 86520
agagtggata tgcgctcctg tagaaatcca tctacagcct gactgcagat gtatttgct 86580
cccttattga gtctctgctc catgaagcct gccttgtttg tacctctttc tctctctttc 86640
tctctttctt ccctctcttc cttcccttcc gttcccttcc tttttctttc cttcctgtct 86700
tattctttcc tctttctttc tttcctctct ctctctttcc ccctttctct gtctttccta 86760
tcttttcttt ctctctcttt cctcttttttt gaacagccaa agatagaatg aatggcatag 86820
gtaggtactg agctccctgt aactggaggt gttcaagtgt agggtggatg ggcatttata 86880
gggcaggcat tgaggaagga tctccctggg attgtggatg gcaggacagg ccctgtggag 86940
ccttctgagc gggtgcagaa gagggagggc atgttcctgc tccctgccct tcccgggttg 87000
ccaggccgcc actcccaacc tccaccctct ttccttccac ctgctgggcc ttacgggatg 87060
ggcagaggca ttgttagaac atggagccgc ggtccacaga gggccagcag ggaggagagc 87120
tccctgggga ccacgtcgcc tcagtcactg tggtgacttt gaaaataaat gctttcttct 87180
ctgcttgagt ccctgttgtt cctgctgacg tggaactagg gcaggctggt gactcagccc 87240
tcatccctgc actgcatcgg tggcaagttg cctccagcct cagaccccac agagcagggt 87300
gccttcttca gaaaaacagt agggaggcca ggacaccaag ggccccaggg acagcctgtt 87360
tctcctggct ggcatgagtt ctagccaagc ctcctggtac cctgggggtg aggaggtacc 87420
agctgtgtgc tttagtcact ggctgctttg aggctgggtc ggtgccaggc tggccactcc 87480
ttcttgtccc ctagtgtgac tttgcagagc tcccggaagg gtcagagggt ttttttccat 87540
cactggtgtc catgggatac tgtgtcccca tctcattttt gtactccagt ctcagacggg 87600
tgctaggaag ttggagactt tagccatttc acgactgtgt cttgcggggc tggagtagga 87660
acaggagtag gaggaacgct ctggtcatgg gactgtgtga agagagaagg atcaggaaga 87720
aaacatgagc tatcatgtct gggttatacc tcagcagagc cgctggtggc ctcccaggca 87780
tctgaattta ctaatcacaa agctcaccag gtgtgcaaat aaacaaatgc tgttcaggag 87840
gcacagccag ggttctgctg gagagggatc atcttggagg ggtcatactt tccacccagt 87900
ccttcagtcc ttctgtcctg tgtgtgtgca gcccctgacc accaccatcc ctgcccctgc 87960
cacttgctcc cagggttcca tcaggttgga tgttattgca ataaaatgta gcaacagaag 88020
gtccaggcag tgctgcagtc cctggtggta tcatcagagc cagcaggcag cagggctggg 88080
gaactccaag ctgattcagt cccaagtgcc ggagcgggga gctcggatga ggtgggggtg 88140
gggaaccggg gagccatgga gcagagcatt tgctgtgctg aaggccagga gcctccttgg 88200
gagatcatga atttagaaca ctaatacttt gtttaaaaag tcccctgcct tggagataag 88260
agctcaggcc gctgtggtca ggttttgctg taaatgttct gcacaaagca ggctgggggg 88320
atcactggga agtctgggga acagattggt catgtgcttc tttgagtgaa accttatggt 88380
tgcattgaag cggccaggca gagggtgtga tggggtgtgg tagctgcaca ccggctcagt 88440

caggctgaat ggggaaatgc aaactcaggt ggcctgaaag agacttagtg gtccacagcc 88500
tggtggctgc tcccagtaca ggtgatgtaa agagagaagg ggttccgggg gcttggccca 88560
ggtggaaacc atgcactgtc tgtgatcctg ggcatgtcga ggctggagca ggtctgcttg 88620
gtggctggca gaacgcacat gaagaatcca tgtgtgttct gaacgtcccc tctgcccctt 88680
tgggctcatg ccccatcagg agtctcccga ctctgccatt ctgcaaagga aactgcgctt 88740
tgtcattgca caggatcgaa caggtgtgtg tggagtccct aaagccactg tctcaagggg 88800
tctcatggga caatgggaga gttgctgtga tgtttaagac taaagtgatg gctgggcgca 88860
gtggctcacg tctgtaatcc cagcactttg ggaggctgag gaggatggat cacttgaggc 88920
caggagttcg agactaccct ggccaacacg gtgaaacccc atctctacta aaaaaacaaa 88980
aattagccgg gcatggtagc gggtgcctgt aatcccagct actcgggagg ctgaggcagg 89040
agaattgctc cgatctggga ggcggaggtt gcagtgagcc gagatcacgc cactacacta 89100
cagcccgggc gacagagtga gactgtctaa aataataata atgctaataa taaataaaat 89160
aataataata aaataataaa agcaaggact tgaacaaata tttgcacacc catgtttgta 89220
gcagctttac tctcaattgc tcaaatgtgg aaggaatgga tatacaaagt gtgtgtacac 89280
acagtggaat atcattcagc cttcaaaagg aacggaattc tgtcactaca tgtgaacctt 89340
gaataatgaa ccttgaagac ctcatgctac gtaagcctgt ctcacaagga caaatattgt 89400
atgatttcac ttatgtgaag tcaaattcat agagaaattg gaatgacggt tgccagcagc 89460
taggggaagg aaggatgggg agttattgtt taatgaggac agagtttcag tttgggaagt 89520
tggaagaagt ttcggaggtg gatggtgttg atgggtgtac aatgtgaatg tacttaatgt 89580
cactaacatg gaaacttggt taaaatggta aatttgtctt ttttgttttt gttttttttt 89640
tttttgagac ggagtctcgc tctgtcgccc aggctggagt gcactggcgt gatcttggct 89700
cactgtaacc tccgcctccc aggttcaggc gattctcgtg cctcagcctc ccagagagct 89760
gggattatag gcgtttgcca ccacgcccgg ctgatttttg tatttttagt agagacaggg 89820
tttcaccatg ttggccaggc tggtcttgaa ctcctgacct caggtgatcc acccaccttg 89880
gccttccaaa gtgctgggat tacaggcgtg agccatggtg gctggccaaa aatggtaaat 89940
tttatattac atatatttta tcacagtaaa aatgcattgc tctattagaa aaatgataga 90000
tttttacaat atgaattaaa ttacaagtta tgaaggaaaa attaagcaaa acattaaaaa 90060
attacttata agtaattata tttaaaacaa aagataataa atacacaact ttcatcacct 90120
cttaggtttt ttgcaacatt taactattgt ctgtcctgtt gcggttatgt tcttgaggtt 90180
gtatttgtat gatagaaaca ctatattcgt ggtgactaaa catctcttct cagtctcacc 90240
tccaatgatg tcatacagat aacttgaaat tggccggctg ggcacgatgg ctcatgcctg 90300
taatcccagc actttgggag gccaaggcag gcagatcatc tgagttcaaa accagcctgg 90360
ccagcagggt gaatcctcat ctctagtaaa aatacaaaaa ttagccagga gtggtggcgg 90420
gtgcctgtaa tccgagctac ttgggaggct gaggcaggag aatcacttga acctgggagg 90480
tggagtttgc agtgagccag gattgcccca ctgcactcca gaccgggtga cagagtgaga 90540
ccctgtcaaa aaagaaaaga aaagaaagaa attggccaag gcaggaatat tgacaccaca 90600
ggaataggca agcgctacaa atcgggaaac tgagtgttgt tgcttttgct ggccccaggt 90660
ggccggttgt tgaccgttaa ctggcacact actggaggag ggaggttcag actgattcgc 90720
agagagacta gaggtagaaa cgcaccatgg tttggatgct gagggtgagg gaaagagagg 90780
agtcaacagt ggtgcccgga gacttggctt gagcaactag gtggatggta gcaccgtttc 90840
ctaagatgag ggctgtgggg aacttgaggg gctgtggaag gctctgccac tgacggaccc 90900
agggctcggc agctctggga ggcctacttt gtgtcaggcc acgcagagat aagaccatct 90960
gggccctgcc cttgggaagt ctctgtctat gaggacacag cctgccacct aatggggtgt 91020
cccgtctgga gcctcagtgt tgctgggaga aagcccctca ccttccttcg ctctccgagc 91080
ctcttttgct tccctgccag ctgagcctct ccatggaggc tgtgcctgga gggggggtcat 91140
ggatatggat gtgtgcgcca aacgctgctt cctcgtggat ttcctcccca ctccgttccc 91200
acgctttgta agcttcattt ccccggggcc tgacctgtgg ggtttctctc cagacctgct 91260
ccttcccatt tccagaaagg cgtgcagggg tttcctatgg ctctcttccc tgtccccatc 91320
ccccttccct tgggacagtg atctgctgga cgggtgggct acccacaggg tttgtgattg 91380
cttagacatc tttgctgtat gaagctttga cctcaaggca tgatgcagat gggaataagg 91440
cacgagtttc tcctggctgc tgtaacaact gtcgaaactg ggtggcttat agcaagggga 91500
atgtgtcctc tcacagttct ggaggccaga agtctgaaat caaggtgcca gcagggccag 91560
cttcctctga agactccagg ggaaaacctt tctttgcctc ttccagcttc tggtggcccc 91620
aggtgttcct tggcctgtgg ctgcatcact ccggtctctg cctctgtctt cacaaggcct 91680
ttttctgtgt ctctgtactt tcttttctgt ctcttagaag gacacagcat tgaatttagg 91740
gcccacctta tcttgagatc cttgcctaaa ttctttctgc agagaccctt aaacagaatc 91800
aggtcacatt tgaggttcca agtgggcata tctttgtttt ggggggggatc accggcccac 91860
tgtaggtctc gttgtgcact gaagacgcag gcggtccctt tggccaggcc tgcagtcggt 91920

cttgccttct tttgtgaccg tggaagaatg caccttgctg gatgcacagg cctttatagt 91980
tcttttgttc ttgccaaatt tcagctcagt ttcctctctg ctgccggcgt gcatcttctc 92040
tgcccagtgc cccttctggg actgtgtgtc caggccctga ggccctagaa aaggggggtg 92100
gagggcctcc tcactatcct gtagcaatga ggcctcatta aaatcctgga gccccagact 92160
ggacccagcc cttagctatg atcaaaggac agctcgggat atgattaaag ggctagagaa 92220
agatgattta ggctgagaaa gaggaggcca aggagcaact ggattgtcct tgaatgtgga 92280
aaaggtaatt atgacttgaa gggtggtgcc cagctgtttc ccatctcctc tgaggacagg 92340
aaaaaaaagg aaataaatga tgtacaatta gagcagttgg ttagatacga gggtgaactt 92400
cctggctggg agtatggcca agccctgatt tgggtttcct gaaagagctc ggggaacctc 92460
cttacctgga ggtttctagg cctggtcatg tggctctgag tgattacatt gaaggggcag 92520
tggctggggc tggagccggt gggctctgag cacagtcacg aaggcatgcg cactttctgg 92580
ctgctctcct gccattgtcc cttgggttcc cattttggtg aggcgtgcac cctggcggcg 92640
ctccccaggg aactaaatat gtttgctgca ctgtgcgtgg agatggagaa tgtacaattg 92700
gctgaccctg tgctaatctg gtggaactcc atgccagccc tgggaaagaa caattgcatg 92760
ggtgtgtcca cactcaccag gtgcttttta gaaaaacact cgagaataat gctgtggctt 92820
aggatggctg ttgtgccgga cccggcatct tcccaggggg gctgtgttgt tgggctgagt 92880
ttcttaggta ctggaccccc aaatccccaa atacggcgtg gacaggtggc ccagtagggg 92940
ctggactatc cgataggccc aggtgctgga gttcagacaa gacataccct ggcctggcgt 93000
ggaagatacg gggtgctatt aatggcagca atggctgcat ttctgaaacc cgggctccca 93060
ggccgacgag ggtgtgcacg catctgaaat gtctgtggtt ttgcagttcc catgtccaca 93120
aactcacttg gttgaaaata gttcaaaata tccaaagcat gagggaggga gtgcctgctt 93180
ttcttaaaaa ggaaggactt gatttcatct acttaaaaag ccacccaaac ctagaacatt 93240
ttccgcaaga gaccccctgc cccccgcctc tccagaatgg ctggagagtc tcagcactcc 93300
tgcacatttg ggatatttca gagggggtgg ggaggggcaa gtgggcagcg agcgacctca 93360
gacccaggat gagctgtcag gcgctccccg gccacacatt caagggaccg gagtgcagtt 93420
gtagcgttgc ggcctgctgc ttcgggggtg ggggtgttgt tccatgctgt gaattctcac 93480
atggcccctg actctgggca gaggccgagg gtctaaggga cggggtgaca gggagagcat 93540
gcaggagtgg gtttctggct ttccagggcg agtggaagaa gcgcctctct ctcttgtagg 93600
tgacagacct ggggggccct tcttgaggat gagagcctgt tgcttctcaa gttctgtgtc 93660
taacccaggt ccccaggtct accccagccc ctcggccctg cctgccttgt ggatgatata 93720
gtttaagggt agagaccgct ggcctggagg gaaggctagg cctcaggtta gggcccagaa 93780
gggagggaga agcccttggg gcagctccct ttctgctcac tcactgccta gctccttcct 93840
tcacaccttc cttcggaaac gtctgctcct gacaaggtct acttcctgct ctcaggaggc 93900
ccttattgtg gaggaaggga ggcgtcgccc gtccctggct tctctgacag ccgtgttcca 93960
tccccgccct gtgccccttc tcccggacag tgccttctcc agggctcacc caggagggtg 94020
cagcggtggc ccccggggcg gtggtcgtgg tgggggtgtt agctgcaggg gtgccctcgg 94080
tgggtgggag ttggtggcct ctcgctggtg ccatgggact cgcatgttcg ccctgcgccc 94140
ctcggctctt gagcccacag gccgggatcc tgcctgccag ccgcgtgcgc tgccgtttaa 94200
cccttgcagg cgcagagcgc gcggcggcgg tgacagagaa ctttgtttgg ctgcccaaat 94260
acagcctcct gcagaaggac cctgcgcccg gggaagggga ggaatctctt cccctctggg 94320
cgcccgccct cctcgccatg gcccggcctc cacatccgcc cacatctggc cgcagcgggg 94380
cgcccggggg gaggggctga ggccgcgtct ctcgccgtcc cctgggcgcg ggccaggcgg 94440
ggaggagggg ggcgctccgg tcgtgtgccc aggactgtcc cccagcggcc actcgggccc 94500
cagcccccca ggcctggcct tgacaggcgg gcggagcagc cagtgcgaga cagggaggcc 94560
ggtgcgggtg cgggaacctg atccgcccgg gaggcggggg cggggcgggg gcgcagcgcg 94620
cggggagggg ccggcgcccg ccttcctccc ccattcattc agctgagcca gggggcctag 94680
gggctcctcc ggcggctagc tctgcactgc aggagcgcgg gcgcggcgcc ccagccagcg 94740
cgcagggccc gggccccgcc gggggcgctt cctcgccgct gccctccgcg cgacccgctg 94800
cccaccagcc atcatgtcgg accccgcggt caacgcgcag ctggatggga tcatttcgga 94860
cttcgaaggt gggtgctggg ctggctgctg cggccgcgga cgtgctggag aggaccctgc 94920
gggtgggcct ggcgcgggac gggggtgcgc tgaggggaga cgggagtgcg ctgaggggag 94980
acgggacccc taatccaggc gccctcccgc tgagagcgcc gcgcgccccc ggccccgtgc 95040
ccgcgccgcc tacgtggggg accctgttag gggcacccgc gtagaccctg cgcgccctca 95100
caggaccctg tgctcgttct gcgcactgcc gcctgggttt ccttcctttt attgttgttt 95160
gtgtttgcca agcgacagcg acctcctcga gggctcgcga ggctgcctcg gaactctcca 95220
ggacgcacag tttcactctg ggaaatccat cggtcccctc cctttggctc tccccggcgg 95280
ctctcgggcc ccgcttggac ccggcaacgg gatagggagg tcgttcctca cctccgactg 95340
agtggacagc cgcgtcctgc tcgggtggac agccctcccc tcccccacgc cagtttcggg 95400

gccgccaagt tgtgcagccc gtgggccggg agcaccgaac ggacacagcc caggtcgtgg 95460
cagggtctag agtgggatgt cccatggccc ccatccaggc ctggggatat cctcatccgc 95520
ctcccagaat cgggccgtgg gggacagaag gggcctgcgt gcgggcaggg agagtatttt 95580
ggctctctcc tgtcttcggg gtttacaaag tgtgttggga cttgcggggc tgctctgtcc 95640
aagcctgggt ctggcgtccg cgtctctgag cctgtgagtg cgtgcgcttt cctgcgtcct 95700
cttgactgcc ggtgctgggg ctctgcgtcc tgcgtccgcg ggagtaaata cagcaggcga 95760
aggggaagct cacacaatgg tctccagcgc tctggggcag ggcttctgag gggcgggcct 95820
gcctctgccg ggacctggag cccccgcccc tcggagaggc tcctaggctg acttgggcag 95880
agccctctgg tgggccggga gggggaaagg ctgtgttgaa atgagcaaac tgtccaggtg 95940
tcaggccaag ctgggaggtg accagcctga ggtcctcccc gctccatggc cagaaccagg 96000
gctgacatct gggtgtcctg agcccagctg cccacacggc ccacctgggg tcagccctat 96060
ctgagtgggg gaggcggggc ctcctggggg accagaactt tggctggacg ccaagcagag 96120
tgccagtggc tgttcttcag ggctgggcct gaggagggtg tggggcggcg aagggacggg 96180
agggggttgt gatccagtgg ccactggcgc tgtgcagagt gtgagctgga aacatcgtag 96240
ttactttgtc agcttagtgg tgaaagccct ttttcaggct ctatcccttt gcatccctgc 96300
ttcccagagg gaggggaggt ctgggtctgc agagctggga gggcttgctg ttcccgcccc 96360
cctccccccac aacacctcct catctggaca tctttgggca catgctcata ctggggtctc 96420
cctaggtcca ctgtgttccg ttgagcctcc tgcagtcccc gagtgaatgt gacctccctg 96480
cccctgcctc tttgcaactc ctccctgcga ccgctcctcc aggggccttc cttgtcccaa 96540
atgtccaagt ggcacgactt agccggtctg accactttcc agtaagccct tatggagaga 96600
ggccctgtgt tgtgcagagc tctcctcctg cctgcgggat cgaggtctct gctctcagtt 96660
cctaacagaa agtgtcgggc ccccagtggg atttctgggg aagaactctc gtgtctcaac 96720
gggagccctg tggcgggagg ggaggccagg gtttggggtt gtgttcgttg tacagctgtc 96780
accatttgca ctatgaaagt tgttagtgcc ccttccttgg gtctctgggt gtaactccac 96840
ccttgccccc atgtgcctcc atctggagct gcttctgcgg ctgtctccca agccagtttt 96900
gtgaccctgt aatttagtcc aagacaatgg gctcattgag accatcctgg tgcagcagtt 96960
ggcaatcctt tggctctggg ggaaggtttc tcagtctcgg ggagtggggc ctcaatctgc 97020
tggttccctg tgtttatcag tctccccctt gtgtgtcctg aatggttttg ctgggaattc 97080
tggtcttaga gccatcaggt ggcccgagtc gataggcgtg agagagtgtg tgtgtgcatg 97140
agtgcgcatg tgcatggggg ctgacctggg gtatggaaag gtggccctcc ctggtgccca 97200
aggagcctgg agtatagttg gagggtgtgg gggtgtgtat atgggagttg gacaaccttg 97260
ggtggacaga cagacgtggg gaagggatga ttgaaggagg tggaggagag agtgtgattc 97320
agcccagcca ggggtgatgt ggacaggcag cttccgaatc agggtagaga aaagtcacca 97380
ctagctagca ggggagaagt cagtatggag gaggcggacc ttgagggaga gtaggaattg 97440
gattgcaaga ggaaggagag ccttctggcc agcagcagcc agcagcagtg ggggaggctg 97500
gaatgagctg gctggagagg gggctggggc ataaggaggg gcctgcctgt gaagatcata 97560
tgggccaggc tgcggagggc caggcatgcc cgccgggagt gcagctggtc cacgggaagc 97620
atctggagtg gctgggaatg ggcgcaggag cagcgccgtg ggagcacagg tctctttccc 97680
ggggcggctc acctggtgtc ttggttcctg caaggtaggc cgaaagggtg gggaggaaac 97740
tgccagctcc ttacagcgct gggatggtgg ccccagggtt cctgaggcca gcggatgtgg 97800
gtgcctgtca ccatgtgggt tgctgagggg cggagactcc aggggccacc ccaaagcagg 97860
acgagctctg agccacggca tctctggggg cagttttcca atcgagcaga cgtctaggcc 97920
tggaatcctg taacagaggc cacagggccc tgatcagggt gttctgggag gcttagaact 97980
agtggcagta tacagggtag acggcaagtg acctggcatg gggaaagagg caggtgccca 98040
ggccggcaca gcacacccgt aaggaacagg tagacgggaa gccgtccgtg ggcctgtgtg 98100
tgtgctcgga gttaaaattc tgccaatgtc ccatgtcctg ggcacatcta ccccctccct 98160
ggggagcacc tttctcttca ccttttcctt ccccgcctgt ccccttcacc cagggccttc 98220
cccatccctc ccgtcttggg gaccgagggc accatggctt tatgttccat caccgatgag 98280
ttgcacaggg attcagtcct tccgctgttc aggccgggtc cttcaggctc agggttccca 98340
ggaatggaga gggtatcagt gtcttccatg gactcaaact tcccgcatcc cgcctttgct 98400
ccccccttcaa gataggtctc cgagcttcaa ggttttaggg ctctgtggag gccgccacgt 98460
agcagcaagg agaatgtttt gtatttggct gatgagattt ttagagtctc attttctact 98520
tttccactgt acaaacgggc ctccaggcga ctgcagcacc cgccactgcc cgtaataggg 98580
tgacaagagg gatgaccctt tcctctttct cccttctggt tggtggaggc acggggctgg 98640
cggacggcat gtgctttcgt gaattcaggc caaatctgtt atcgcaaaca cgattacaac 98700
tcgggtcttt gtgtaacaaa agcctttcca agtaccagct gttggcctgc tttgctcagc 98760
ggtgtttgct gtaaccagat ttgcacaccg agaaagaatc caaaagtcct tgatgtttgt 98820
tgaaacaatc tggcccagga cccacgtgct cagatcccag agctgtgtgg catctgagct 98880

tctcccgagc ccccactgtc gcccgaggag gacccccaga tctgtgttct ggaggcagag 98940
caggctgtgg gacgggcttc tgggtgggaa ggaccatgtg gatatgcctt cttgtctgag 99000
agtcccaaca cctccgggac gtgggagctg gcgcgctggc aggattcagg tgcctctttc 99060
ctctctagag aaaaaggccc cgttgcttgg taataggtgc agacctgtcc ctaattaatg 99120
ccagtaggcc tcttgcgtga tgaaccctgc ctttcagcca agactcaagg catcctgtga 99180
atactgcctc tctgcagttt gagcttttgt ggtgggaggc aggagccatg gggagtgggg 99240
gcaggcctct tacacgggtc ccacagccac tggcagcact gacttgatgc tctttgagtt 99300
cagagcccag ggccagacag acccactgtc ccgactacga gttggttcat ttagaggggg 99360
gcggacacag cacccaggca gcagatgcac tgtgatcagc cttgcagcgg ggctgtgggt 99420
tctctgggct ggatgtccgg gaagaggcag gtggaggtaa acgccaggac accccctgcag 99480
tgactgggtg actgcaggct ggaaatgctt tctgtgggct gtggctgtcc aggaaggttt 99540
tgaatggggc tagtggacag agtttgcatc cagaggggca gtgctttgga ggagtgaggg 99600
gtatggcagt gtagggatgc ccaggccgcc ctcactctgc cattggaaag ctgggcggct 99660
tcggtttctt cagtttcttc gcctgtttcc ctgcctgcaa agtggggtta gaaacagctc 99720
tctttgaggg ttgctggggg actctgagat gcagcccatg gcgctgagca cgggtcctgc 99780
ctcctacggg tgtggtgggt gtcgcggctg gtgtggcatc tgggcgggaa aagggggcat 99840
ttgcaaagga caggtaggtt tggatgctta aatatgcaga tctggggatg ggaggtctca 99900
ggcaagggcc tgtgtgatgc cactgtatga atgaggccgg acagcatggt caccactaat 99960
tatggaaagc acggcagaca ctagtgccag tagcgcccgc ctggggggtcc ctctgagccc 100020
gacaggtact caggctccca gtgcagccat gggacccccc ttttcctgcc tggctgacat 100080
tctggaggct ggcgtcatgt tggctgcagg ttcctcttac tgggaggttg tcctggtctt 100140
ccctgccacc atctacccc acccccaccc tggagtgtca cagatgggtg gaccatatgc 100200
ccatctgcag ggccaagcgc tgacaatgag gtgagcactt gctggatgac cggaccatga 100260
ggacagcggg ggcgctggca gcctgcgttc ggctctgcag gctggccttt ccctgaaaag 100320
cggttgtggc agagtgtgca tcccagggag tggtgcctgc tgccctgaac attctcatcc 100380
agcaggcctc tgcaagggcc cagtcaagtg tggtgcagag ctgggctggc agctggaaca 100440
gtctcatctt gtctagtggt gagaccaagg gctgatgggc caaggctgcg tgctgcttgc 100500
ccagcccgtg gacccatcca ctgagcaccc ccataccccct acactgggcc cggggtgtca 100560
ggaggtggag gggggactgg gtgcctggaa aaacaagccc accgagagcc tgagccccca 100620
ggggtcagta tcaggcagtc accacggtgc ccggtacaca gtggccacct ggtctcgtcg 100680
gctgtcttgg tggtggagag cgtgcatggt ggccgtacgt gatgggcatc acccagggta 100740
atacagatat gcagactgtg catttgtctg taggagccag ggacttctca gcctggcaac 100800
ctgtgacctc tgcctacccg ttgggagaga ggtgctgttc cctgatagcc tggtgccggg 100860
agagcagaac tagggtctcc tcctcctttc tccacctccc atttcttaca tgcccaggca 100920
tcaggggagg tcacactcag ggatgagtct tggccactgg gttcagggcc tcactgttgc 100980
catctcttcc ctttgccagc cagccaaatg cccgtccatc atcgtgccgg ggatggggct 101040
ggcctgctcc tctgaagtct ccttggtgta tagcctaggc tggagccccg gacgattccg 101100
ccacccccctc ccccgacttt ttctttttat tttgattcat cccagaaacc cagccaaaaa 101160
ccaagttcct gtgctctccc ttgacaaagc cactgaggaa aaatggtcaa acactgcctt 101220
tctttccaaa gccctcttgg tttttcccaa atttggaggc tctgagttgc ttatgagacc 101280
aaagggtccg gaactctctg gaacacagtc ccagaagtgg gaggatttgt catctctcca 101340
gccaaacaga cccaccagtt ggcccctaga cacccccagg cgcctggggc atttctgggc 101400
tcaggctgtt taatttggtc tgcagagccc cgaggctccg tttagactgg gccagcagac 101460
agttctagag acttcaagtt tgttgtcgtt tttaaaagtc ctgccaatgc ccagaaactt 101520
agagggacaa gaagatgacc tcgatgtcag ccgggaccat aaacagtgag gcccgccccc 101580
ccccccaccc atgcctgcct ccactgctcc cgtagctggg tctccagggg attgcccgtg 101640
gccatggggg aggccgggaa tgcggggctc agactctact gggcttctcg tattttttttg 101700
tttttccttg tccatcttct ttctcggaaa tttctgaata tccccctaca gcctttggca 101760
ttctgtcact cctggcatct ctgggccaac tgggaaagcc atcatttgtc caatatccag 101820
aaaatcaagg ggcctggcct taaatacatt gcagtgccct cctccccaca tggcatcgaa 101880
tttcctgtgt cctcaaagcc aggagcctgt gccttggatg tgggcgatgg agccttgcag 101940
acacctggag cggctttcct gggagagtct ccctcgcctt cccatccacc cgcttcccct 102000
cccctgcccc aagctctggt cacagcctcg gaggggtcat ctgagtgtag gttgcctcca 102060
ggtgtacagt tggagggggg cagttacccc acatccccct gtctgctagg ggcaccccca 102120
gggactcagg tcttgaggga cagcggaagg gtggggtggg gagtaggccg ttgtctcgtg 102180
ccacctgggt gttatgtaga ctctgaatgg gcagttcctg ggagctggtt tttccttctc 102240
acccccttcta cagtgctgcc cctctgaggg gctggagctc tctggagccc tttgcagcct 102300
gtggcaggtc tttctgattc tgtgcctggt ttcgcagcct ccaacccata gccagtggca 102360

tgaagaacat tcctggctgt ctctacctgt ggcctttgca ggcagcagtt ccctggggca 102420
gggcctgggg gtcctggcac ctcccttgtgg gacccccttgg gtatgaggga gggagccacc 102480
tgggccacag atagaccccc ttcctgtatc caggaggttc aggatggacc tgtctcatct 102540
ttctttctgg tgggaagaga gcattcctgg ccatggggaa tgtggcctgg acagagccac 102600
ctagcctctc ccctccccca ggcagggccc tgaaagatgg agatggctct gctcctgcca 102660
ggtcaacagc cagcagtctg gtggatttcc atgcagtcca ggcaggatct ccttctgtcc 102720
ccacccccca ccccatgtgc aggaagctgg ttccaagcag ggacccttgg ccccgctttc 102780
cacgtgggtg ggcagtttcc ctgcgagggg caggccaggc ccctgcccta tgtcccccag 102840
agaccgatgt tatctacagt aagagcatac aagtcttttt ggctccaaac caggcctgga 102900
ggggtggtct tctcctctgt cctctgcctc ttgcccccca ttaaaggtca gggaatggag 102960
tgctctgtat gtttgctggg gttgggtttt ttcctttggt ttaaaatgaa gggctggaat 103020
ccctggtgca gactgcacgg tgagaggtgc tccttgggat ccactcgagg agcattactg 103080
ctgaatgccc tgccaggcca ggagcatcgc tgggggtggg agaggcagtt ggcttatttg 103140
tttatccaac agccatccat gggtacccca agggccagct ctgttctagg cactggggt 103200
ctaggcatgg gctcaggaga aggggtagaa cgggagggct tcctggagga aggcttccta 103260
accagagacc ggggtaggag tttgccaggc aggtgatgct ggccagcttc tcttgccatt 103320
ttccttttct tttttctttt ttctttcttt cttttttttt gagacagagt ttcgctcttg 103380
ttgcccaggc tgcagtgcag tggcgtgatc tcggctcact gcatcctccg cctcctgggt 103440
tcaagcgatt ctcctgcctc agcctcctga gtagctgaga ctccaggcac gcaccaccac 103500
gcccagctaa ttttttgtat ttttagtaga gacaaggttt catcctgttg gccaggctgg 103560
tctcaaactc cttacctcag gtgatccgcc caccttggct tcccaaagtg ctgggattac 103620
aggtgtgagc cactgcgctc ggccttctct tgccatttct gcagactcat agtgtgtgtg 103680
gaagactgta tgtctgtgcc tggcacagct gccctcggca ctcaggtggg aggtggggga 103740
gagggctgtt ggtgccagca caggtcatgg catgagacag gctcccgcag ccaagcgggg 103800
tgcaccgcag cattggaggg gccctgctgc gcacgagatc caggctctgc atctgtcctc 103860
ctccccagga ggcctggagg gctgggaggg accagggtgg tgaaataacc caggtgggag 103920
agaggcactg ttggagaggc agagggagct gcaggtttgt agcagcgagc tttaaaccct 103980
gggcctccac cagaaaacct cagaaagggc tgctttgctt ttttttctaa actacaaaag 104040
aaacccttgg ccgcctgggc cccttggggt cgggtggggg aggagatcct tcattcgcac 104100
ctttgcaggc ccagtactgt gcagagggag gagggtggag gcaaaggcga gggaggaggg 104160
tggaagcaaa ggcggggtcc ctccctagcc ccgctgccct gcctggttgc tgctctcaga 104220
cccctactcc tggcccggaa acatggaatc ttgggacagc tggggtaggg ggcacagaac 104280
ggagaccctg gtgagggagg aactctttct ccagccctgg ctgtggccac agcaaggcca 104340
ggctgggagg acctcactca aatatttaca tctctcctaa tatttaaatg atgaagcagg 104400
ctcctgcaca cagctgccct ggagccagct gttgcctgcc ctaccctccc tctctccctc 104460
cctctttctt tccctccctc tttctctccc tccctccttc tctccctccc tccctcttgc 104520
attctttgtc tctgacccaa gtccaggccc ttccagctgt tctctcctta cccacaggca 104580
cctcctgtga ctgagcttct ccttgttatt tttcataacc atttccttta attgagcacc 104640
tcctaggtgc caggccctgg gctggtactt tgtatacaag gtgtctttta atccccacga 104700
gagcccaaca aggtagattg ggctgctgag gctccagcgc tggaaggccg gcccgggtcc 104760
tcccagtccg cacctgcatc catccatcac tcccccactg tggagacctg ttccctggcc 104820
cctggctgag cacgatggaa atctctgtct actagtcctt ggagagtggg gatggctgag 104880
ggtcccgacc ctggggtctg acgggggctg cctctgcccc acttcagcct ggctgctctt 104940
ccactcaatg actgagtttg caaaaccaga ataacagtgc tacctggctg gtactttggg 105000
tggctgggag gcgtgggaga gggtgcacgg aggggctgag cgaggtgtca gcatcgtggg 105060
gatctccgag ggtagccttc ccaggcccag ggcggagtgt aggcagaggg cgcgatggcc 105120
cagcgtgccg gctcacatct ggccttgccc ttgcccacac ggcaatgtgg gggagtcatc 105180
agcctctcag tgcctcagtt tcccatctgt gcaatgggga caagagtaaa tcactgtctt 105240
caggtatggt aagcactcat acacattgac tattgcagaa tgaacacagt gagcccttct 105300
taggcttcta ctacatgcca ggcagggtgc caggccctag tggagagggg acaatgatgg 105360
gcaagctggc tcctgccctc aagggctgtc gagcagggag gaaggcggcg gctgccctga 105420
ctgtgacccg gagtggggtg attcgcaaag aggctggcgg gaggctggct gggggctggc 105480
tgggggctgc tccctctcct gcctgtgttc cgtggcaggg tattaaactg gctttttca 105540
tggatttccg aggccctgac tgtgggacct cgggcaggtt ttgggacctt gtgactttgt 105600
tcccgcttgt cattcaggcc tttgtcctca gcagtgccca gcacacggcg acttctgcat 105660
gtccttcagt gtgtgactga ccctggagct gagacttggc agctgaggct ggacattgtc 105720
cttggataac tcccggggag gtgggggcag ggtggggctg tgagctggca actccgagtc 105780
cctagcagcc tccgaatgga ggctttcctc ttgtaggatt ctcggggagg aaggagggga 105840

```
tgggggagat gaggcttgtg agtcttcagg agggcagagg ttcacacctg cagcgcccca 105900
gaaacactcc ggggtgccca ggcttaccca gccctcctga gccagaacca ccaagagttg 105960
gggaccagca gtccattttt tagcaagatc cagaaatgat tctcctgaac agtccgagcc 106020
aagaatgaca tatctggaat cagaacatca gaactacaga ggaatcgaga gaacgggccc 106080
aaaatgtgta gggacctcag ccacatggcg ccacatggag gaaagcaccc aacatcatca 106140
ctgtatgaac ctgtttaaac acacacacac acacacacac acacacacac acacacacac 106200
acactcccca gtaaagagtg gaaagacatt tgtcagaata ttgatggaag ttacctgggt 106260
tgtgggttta tagatttttt ccctttgttt ttgtgcattt tgcaaatttt cggctttgtg 106320
cctaaattcc ttttacagtc aggaacaaag caatagatgt tattttttaa agttcatggg 106380
ctcgtgcccg taatcccaac actctgggag gctgaggcag gaggattcct taagcccagg 106440
agtttgagac cagcctgggc aacataggga gaccccccacc ttttttttt tttttttttg 106500
acagagtttc actgtgtccc aggctgaagt ggcggtggca cgttcttggt tcactgcaac 106560
ctctgcctcc caggttcagg tgattctcat gcctcagcct cctgagtagc tggattacag 106620
acactcatat ttgtaggatt tgagaatcct acaagaggaa agcctccatt cggaggctgc 106680
tggggacttg gagttgccag ctcacatatt ttttagtaga gacagggttt caccatgttg 106740
accaggctgg tctcaaactc ctgtcctcaa gtgatccatc catctcggcc tcccaaggtt 106800
ccaggattac aggcgtgagc cactgcgccc ggccatggga gaccccctct taaaacaaga 106860
ataaaaataa aaataaataa aagtttactg tcaaaaagaa aaacacatca cccaacctaa 106920
tctcccccta ggattgagag atgaggaagc agaggccgga aagtgtcaca ttccctggtc 106980
gctggcagtc aggggacctg aggcctgggc tggttttctc gtgctccagg cctggggtgt 107040
caggtggagc ccctgctttg cccaaaggtc taggtgcctc tgccagcact gcttgcttct 107100
gcatttttgg ccacaaggaa tgtgggccaa aggcagggtc cttgacctct gcccaggagg 107160
gtgaagtggg ctggtcctcc ttgtccctgc tccaaggccc ctgcggctgt gcctttggaa 107220
actgctggcc cgagctagtg tgcggagctg ggacagacct caccgtggcc ctcgtggggt 107280
cattccatgt ccgggctgga agggatcttt cggtccagcc cttcctgttc ctcatcagga 107340
aactgagacc agagaggtca tctgcctggc ccggggtggg gtgtagatgg cgcttgtgac 107400
tcctgcatgc cacgtgcaga cagtccctca gctgtgggct tcccacgcaa gcagccatgc 107460
tggggataac ttggctgaaa cctgcgattt aatgaagaga agaatgactc ctcctgcagc 107520
cctgtgcccc acaggctccc tcctgccccc acccctcctg cagaggggcc tcatgctggt 107580
gggtgggtcc ctgttgctgc aatgggccca ggcagtttct gggcagtgga ggcttggctg 107640
tcttcctgcc caatgtcccc tgtagcccac gctgtccggg aagggcgggg aagcactcag 107700
ccactgggga cttcacaaga tgggtatctg ggagatgctg attggcaggc caggctcccg 107760
tggcccacag gcaggtcaga ggtcacagtg ctgagctggg ctgagttggc agctcagggc 107820
agtgggggtg ggggtacctt gctcagccat gggcctctct ggcacaaggg tttggagttt 107880
actgtaaaca actccttcag tttttccttc tctcataggc caccagcaga attgaggggc 107940
cctctgttga tgaggtgtgg cctgggaggg gctagttgtc tgtccacctg atccttgcgg 108000
acaggtgacc acaggcttcc tgccccaggg ggctggggga aagatcgggg aggcctctct 108060
ggcttcccca cttggaagga aaatacgggc ataggggaga cactttacag ggacacattt 108120
cagccagtac cctctggctg tgctggggc acagtgcctg ccctgcccac aagaggggtc 108180
agttctctcc aggatttaac ttcagccttc agtgcagggc agatgagcac tcagaggccc 108240
agaggactca tatcacgaga gagaagctgt gaccacacag gcactgaagg ttaagcagaa 108300
gagtgggaca cacgggtgac ctgaaagtcc cagggtcagt gtggttagcc ccagatagct 108360
ggggagcttt gggtaccagg cttagcaggg gaggagggcc cccgtgacag tttgctgggg 108420
ctgctgcaac aaagccccac gaactttggg ggctgaagat ggttctagag gccagaagtc 108480
caaaatcaag gtgtcgacca ggctgatcct ttggggcctc tgcgggaggc tccggcccag 108540
gcctctctcc cagcttctgg tggctcccgg gtgctcctgg gcatgtagat gcgtcacgcc 108600
actccctgct ccgtcttcac atggcctttt ctctgtgtct ctgtgtctcg taaggatgct 108660
tgtcattgca tttagagccc accctaaatc caggatactc tcctcttgag atcctttatt 108720
gcatctgcaa agaccctatt ccaactcctg tcacattcac aggtccgagg gctagggctc 108780
tggcatgtct tttgggggtc actattcagt ccactgcggc agctgatctt aggggaagca 108840
gaatgttcta agcttggggt gagcggaggg aacgctgctg ggtggggagt gggagaccag 108900
gctcggagac accgcggatc tcctctgtct gatgagggcg tgatgaggtt ctcatctcag 108960
gtcctctgaa tggcggctgc ttctgaggag acccttttccc ctggatctgc ccctccagaa 109020
acctccactt aggtccaggt cccaaggtgg atggtaagag gcggctgtcc ctgccacact 109080
tgatgggtga gtgtgtgaac ggggctggga gcaggacact tgtcacttct gtggacagat 109140
tccagaggct catgaatctg ctgctgggga aggtgcctga tctagcctga ccctccaccc 109200
ccacctcccc cttcccgccc cccttcacca gccctgttcc cagagcttca cttccccaaa 109260
ggcaggcgcc gtttcctgga cttctcagca gctacccccct accccagacc cctctcttgt 109320
```

aggcccctct ggtctcagcg cctactcctc cctgggcttc tgcccctcta ccgtgaggct 109380
tctgggcaga agtgtggggc cccctcgctg tgtctggctc tcgcggtata ccaggggtcc 109440
tcccaggggc ccgccctgcc cgctaggttt ccagggagct tttcaagttc ttagatgggc 109500
ttgatggagt ttcccaatgc gttgtctcag agcaggaggg gccccggctg agtccgagag 109560
tcccctcagt cacaccctgg gctgtttggg gcagccaggc agcccgtctt ctggaggtgc 109620
tgcctctctc tggttacccc ttctggaatc ttctgcgtcc agttgcggcc cctcctacac 109680
agcacaccca ccaccccctt ttacctgtga ctcttctttg tcaacctctt catgtctttg 109740
agaatggaga ccaggtcctc ctcaccctgg agttcagcag aacctaaacc aggcagatcc 109800
gtgtaggttc acgtgggggc tctgtggcca ctgtggctct gccgtcacct ccagcccagc 109860
ttggcgcgct ttggatcatg tcattcagtg ccggtcctcc aaacctctcc cctggctctg 109920
caggtctggg aagcagctgg ctcctgggtg cagctgtgac cggagcctga actctgctcc 109980
ctggggtcac cgtcccctgt aattaggctg cagctcatat tggctcgggc agaggccatc 110040
ggctgcccgc cttggggcca caacacagag agccccgtga ttaatggagg ctccacctgg 110100
gccaagtggc ccctggtgtt aggcgctttt tttttttttt ttttttttt gcattttta 110160
agtgcttgta acatttcccc taatggacaa tgagacgagg agggagggag gggtggcttc 110220
tctgcccagg cagatcagga atggtcaccg cctgtgtgtg cagtgacagg tctgggaaca 110280
gacaagggag agcttgtccg ggaggctgga ccctcacacc gatgctgcct gtgcctggca 110340
gctctgtccc gggcctcaga acagacactg accaacacgc tcctcagggt ctccaggtcc 110400
gggtcctggt ccccggcaga gcttcccatc catgggaaga agcaccgagc agccttgctg 110460
gctcctcgca gggagctggg atgcatttct aagagcagca tgcgtgaagc ccggggtctg 110520
ggcaaaggag tgtttgggga agagtctgct gttgttggag ggtcggaagt agaggggaag 110580
gaggggcacg cggtaggctg aggaggtggc tggcgaccct gtggtgggga cgaggggggct 110640
atgagcagac caggagccag catcacgttt ctctgtctcc agaatcgagg acggagagct 110700
gccagagagg ccctggcggg gtgtctgggg ccacctaaag ggcgtgggtg gattaaagct 110760
gaggaaagcc tcattttggg ggactccgta cttaaccttt gaggccctca gagctgcagc 110820
ctgtggtttt tcgaagtcaa tcacctccca gggcctcgct cctgccttca gcgacagcca 110880
tttatcaggg agtctttgac ccaaggagac ctgaaaggtc atctggtccc tctcctaccc 110940
ccaggcaggc cacacctaga ccctcccaca cagatccgtc ccacccttct gctgccttcc 111000
accagggacg gagggtgggc ggccctccca cccaggccct ggcggcccca ccccagccag 111060
gaaattcttc ctctttatct gcctcacatc tcttcccctg cggcttccgc ccatttcccc 111120
tcaatctgct cctagtggag cagccagcag ctggtttaga acccgggggt gggggagcg 111180
ctagaccagt gcagcacatc tgagagtgtg ccagaggtgc tcccattccc atagctgatt 111240
tagtaaagaa aagaggaagt ccagcaactt agcgccactt taaagtccgc ctggaatgac 111300
cctgtggctg ccggaaccag gggcacgcgg tagatgacgg gagcaggcac agagaggcct 111360
tcgtcccgga tcagtcgctg ttgccctctt ggaggctggg ctgcctttca agaagaggac 111420
ccaaggccct gtgagcggtg gcctcctctg gagcctttac tgtggtcctg gttctgctct 111480
gcgtaaagag agatggcttg aagcagaagc tgctacgtgg ctgtcccagg caggcgcccg 111540
tagtcccagc tactcgggag gcggaggcag gagaatggca tgaacccggg aggcggagct 111600
tgcagtgagc agagatggca ccactgcacc gcagcctggg cgacagagca agactccgtc 111660
ttaaaaaaaa aaaaaaaaaa aaggagtaga gaaatctttc ccagaaggcc tgcagtcacc 111720
tccccttcat gtttcagagg taaaaaaaaa aaaaactgca catttcgctt ttttttttt 111780
tttttgagac ggagtctcgc tctgtcgccc aggctggagt gcagtggcgc gatctcggct 111840
cgctgcaagc tccgcctccc gggttcacgc cattctcctg cctcagcctc ccgagtagct 111900
gggactacag gcgcccgcca ccacgcccgg ctaatttttt gtgtttttag tagagacggg 111960
gtttcactgt gttagccagg atggtctcga tctcctgacc tcgtgatctg cccgcctctg 112020
cctcccaaag tgctgggatt acaggcgtga gccgccgcgc ccggccacac gtttctgttc 112080
ttgaacaggt ggctccctga ccctgggtgt ggttactcca agaccttgct ctggtggggc 112140
acaattctgg ccccaggtag ggcgaccgta taacttactg tcccttgggg gacacgttag 112200
tgaaagggac ccttgaatga ctacggtggg acaacagatg tgagctgggt ttgtcccagg 112260
aaggctggat gtatgggcct cttgagccag aggagacaac agcagaaagc tctgcggctg 112320
atgggaccga gcaggagaag ggccggcgga ggctggggca acaacgcact tgcccactgt 112380
gaagggcctg tgtgagctga tggctgatga gagggcagtg gggcgaggct gcctcctgcc 112440
aggctagacc agctccagga cacccatatc tccttttcag aaccggtgac tcagtggcta 112500
gaaggggctt cagaattgac gggacactga gggagtcgaa tggaatggct ggagtggcca 112560
cagcaggctc taggcctcct cccgtttctt ttctgtgttc tgttcctggg gtctatatcc 112620
tggggttgct gtaacagagt gccacaacct gggggcttac aaacatagct tccggcggct 112680
cctggagatt cctggcgttc cttggcctgc agacgcatca ccatcatctc tgatctctgg 112740
cccctgtctc cacgtggctt tctccccctg tctgtgtgtc tttctccttt tctgtctttt 112800

ataaagatgc tcaccattgg atttagggcc cactaaattc agaatgatct caccttgaga 112860
cccttacctt aattacatct gcaaagatcc gttttccaaa tgtgatcatg ttcgcaggtt 112920
ccaggagtta ggacttaaga catatcgttt tgggggacac tgttgaaccc actacattta 112980
ataaacccag agttggtgcc aatgtcccct gcggacacca tagcagattc tggaggttcc 113040
ttgtaaagga atccgtagga cttcttttcc cttctggagg gatgagactg tagctgggct 113100
ggggaaagct gctggttcaa gctggggctg gttcagctgg tgctggcttg ccaggttgtt 113160
agagagcttt atccagggag aaattaacat cacaaagttg ggggataaga gaaggtttgg 113220
aatcccaagg gctggctgtt gaggtcagct gcccacccct ttgtgttcca tcttacaggg 113280
acccaggcct gccagtgctc cgtttctgga aggaatgctg tgggctgctg tgccccatag 113340
gcagggtgga gggtcccctt gcagccagcg agctgctctt cctgtagcgt gcagagggaa 113400
ggaggaggct tgtttatttc tcttggcctc agccgaaaat ccaaccacag gctctggcac 113460
taggcagacc tgctcattct cacatcatca ttgcagagtg agccaggcct cttgggtggg 113520
gctgtgatga gcaggaagga cctccggagt gcctgggact tcttgggccc agggttgcca 113580
gaggcagaga gaaagaagcc tcctcctaac acttgagcaa agatagtcat cagaatccat 113640
cagagtggcc tcctgtggct cttccctggg tgctgacccc cctggtgcat ttgaagtttt 113700
cagggatact attgacgctc cctgcacctc catccagggg ttagtggaaa tggttctttt 113760
cctccttgtc cagaggtact gtggatgatg accccaggtc ccagacctca gtcattgggc 113820
cacgtgtgca gataagcctg ggcagtcagt aacctttggc caccaagctg gcctcctgcg 113880
gactgggccg gcactggagg cgggtcttag ctgctagcac tgtggcccag ccaggcctgg 113940
ccttgggaag gcagaggggg aggggactgg gaaagggatt gagctggggg tggggtggta 114000
ccctggttct tccctcccag acctttggca ggtttgaact atctggctgc ggctctctaa 114060
ggagggagcg cctcctccgc cttgatgtgg actttgcctt ctgttcacgt tgtccctgtc 114120
cctaaacaaa tgtggctctc tgtggggagc ctgggggccg ccctccactg acactgctgt 114180
ttcctggctt ccctttgcag gctccgctca gcttcactgc gcctattttt aagcctttcc 114240
ttttggacgg ttttgcttcc tgagaacaca cccctcccca tcaccccaac tgtgtcctcc 114300
tccctatccg gagcctgctc cacctgcccc aggagccacc tccctcctcc accaagcagc 114360
ccaggccaat tctggagggc taatttgtgt ccctccagaa gcaggtcctg agacagggat 114420
ttcagtggga ggggacacca ggggatgcct gagtgtggga atgggccagg cagggaatcc 114480
agccaataaa gggtgcctca ccagctcatt gtgaccacag gtggctgccc agcccccata 114540
ccgggcagca tggggagact gcagaccgtg cccccagcct tgtcccacct ggggttgggt 114600
aagggagctg gggtttttat acttcagttc ttggccaagg actgcactgc tcactccttc 114660
cagccttccg agggcttggg cactaggaaa ggctctgggt ggagacacgg aaataggcca 114720
tgtgaagtca gtgggcgtga ttggtagcag taaggccagg acagccttgg tcaccagcaa 114780
ggtgaccaca catttcagca tgcacagccc ccacagacac acacccagga gatggtaatt 114840
acaccagcaa cagtcacccc tgccgcgtgc catgcctgca cccagggaat cttatttttt 114900
atttatttat ttatttttat ttgagatgga gttgcgctct tgttgcccag gatagagtgc 114960
aatggcacaa tctcggctca ccacaacctc cgcctcctgg gttcaagcta ttctgcctca 115020
gcctcccgag tagctgggat tacaggcatg caccaccacg cctgactatt tttgtatttt 115080
tagtagagat ggggtttctc cattttggtc aggctggtct cgaactcctg acctcaggtg 115140
atccacccac ctcagcctcc caaagtgtta ggattacagg catgagccac cgcgcctggc 115200
ctcaggggca ccttattacc ccatcacctc ctacagcagg tgacactgag gacactgagg 115260
ctcagagtag actgtggtta tgtcactctc ctgtttggtt ctcctgctct cagccctgaa 115320
ataaagagca cggtccctgt gcaggcccat acgtcctgtc tccagccctg gagcagtcct 115380
taagctcctt gaaggcacac caggcctttg ggcctcgtcc cccaagtcct ggataaatag 115440
gaggtgaagg agcctttgca actggacccc actctgccag cgccaaggcc ctgcctgact 115500
acccacccca ccctacacag atcagggacc tgtgccaagg tccagtgtca caggagagtg 115560
aagacaagga ggtgcccatg gcgtacatgc tgctggactc actctgtaga tcctaagggt 115620
tatggggagt tatctgccag gtgaggtttt cccgtggctt tttcctgcag gctcagggta 115680
gcatacaggg aggtgcttcc ctactccttg gcaccatctg tccggcttcc ctgagccctg 115740
accctggctc ctagatagcc agtgtttctt cagggacaga acctgctttg ctgctgctca 115800
gaggagaccc agtttgacca gggctagcgt tcctggaacc tgagcctgga attccaccct 115860
gccctgggca gaggccacgt tgctccctcc tacgagagct gagctgcgca tgagattatt 115920
ctgaccttgg gctctaccat ttctttgcat gacatttgtc ccaggctgga gcagggctct 115980
gtggctcgct tggtgttccc tccccagact gaggcctgat atgtacacca ggaagtgctt 116040
gatctggttt caggagctgg gagcggtttg ccaggcagcc tgtggtcaga gcaggggcct 116100
taacaatggt cccttgtgcg gcacattcct ctctaaatag gcccaggtcc aggctgctcc 116160
tgtttcgacc ccgccatggg cctggagggg aaagtttgca tctggagggt ggcccactgt 116220
ggcctctttt tgtgtagcag ctgagaccgt aggtgcttga gaataggaga agccctgtgc 116280

ccatcctggg ctgtgagatg tttttccaga ggtggggatg gggatgggcc agcatgcagt 116340
tacccacacc ctccagcttg ttggcatcaa ggtctgtgta tgtgtgtgtt tttttttttt 116400
taattttgtt tttccaacat ggagtcttgc catttgccca ggctggtctc ggactcctgg 116460
ctcaagcgat cctctagcct caggttccca aagtgctggg attataggtg tgagccacca 116520
tgcccagcca gtaccaaggt gtgtttttta agagcaatat tggccgggtg cagtggctca 116580
cgcctgtaat cgcagcactt tgggaggccg aggcaggcgg atcatgaggt cagcagattg 116640
agaccatcct ggctaacacg gtgaaatcgt gtctctacta aaaatacaaa aaaattagcc 116700
agacgtggtg gccgggcacc tgtagtccca gctactcggg aggctgaggc aggagaatgg 116760
cgtgaacctg ggaggtggag cttgcggtga gccgagattg cgccactgca ctccagtctg 116820
ggcgacagag cgagactgtc tcaaaaaaaa aaaaaaaaaa gaacaatatc gatggtttta 116880
ttccctgaac atgctaagtg atccccatgt agtataagca tctcagaagg caggaaaatg 116940
taatgaacaa cacccaccgc cattcattcc atccagagat cgttattttc tgcccgggtg 117000
tgagtgtgtg tgtgcgtgca aactgcacat gtgtgtatga aatggaatta ggatctttgt 117060
atacatcttg tgtcctgcta tttttactta atattctcgg catttctcat cttcaaaaat 117120
gtgattttaa tgatggtgca ggcctttgac atatgggttt agaaaatctg cggaaacccg 117180
gactgtttcc aggttttgtc agtttaatgc tgatgcaaat gtcctcgtac gtaaatcttt 117240
ctgtacttct gattatttcc ttgggataaa ttcccagaag tgggccaaag cacatgaacg 117300
gtcttcaggc ttttggagca agttgagatc ggtggggatt ggagaaggga cccagggagg 117360
cttttgggag cttgcgagat tggagtctgg ctttagggga cttgaattca cagatgaagg 117420
agatggctgg caaaggtggg aacctcctgc tctatgggtg ggggggtgac actgctttac 117480
cttccagtga cagcatgggg atcctcccaa catgacctgg ctccgcatct ttctgacact 117540
gtgcacggtc cacagttgtg atgctcagct gggagcctct tctgtgtcct tcagttgcat 117600
taactccttg cagacagagg aggaagctgt ccggtgctcc tccctgctgc gtggtccttt 117660
tgaatctgcc cagttgggcc tgtggatctg tgcagtcacg tacggggttc taaaatctgc 117720
aaaatgacaa gctcacgaac actctcgctt ctgtgggtga atgtggatac cccttggctg 117780
cgtctccctc cacagggaag atggagacgt gtaaagtgac aaggacctgc taccccctggg 117840
caggttatgc cggttccact gtgaggatgg tggcaggggg cttcccagga ggcctgcaca 117900
ttgggaagag aggggcgacc gtgtgtgact gcaggaccca gggcgcagtc ttaggactct 117960
gatgggggag gagatgctgg ggagccctgt tctgccagcc tggtgacaga catctcctaa 118020
tgtttcccgg ctgggtacaa ctcacaatgt cctccgtggg gaggcaattg cccatgaggc 118080
atccctggca gggatgatct tgtttcattg ccacatatcc cagcaatgaa acaagaatct 118140
agagtgatgt gattcctccg atgacttctc caaccatctg ggtttttgt gcgactccaa 118200
gaagggggaa ggaggcagct ctcgctcctc tgggctcctg atgtggcagc tctggaatgg 118260
ccacgtgtcc gttggcccac agtgggccat ttgtcgaatg tggttgtggt gggattgggg 118320
ctgggctgga gagtgggtga ccacaccagc tagcctcacc ctgggccact gtgcagccag 118380
gggtttcatc ttcccagggg tctcgttgtg agtgtcctgg ggctgctgga acaaattact 118440
ggaaaccaag tggcccaaca aagattgatt cgctcatggt tctggaggcc acaagtttga 118500
catcaaggta ttggcaggac cacactccct ccgacagctc gaggggagga cccttccttg 118560
cctcctccag cttgcgttgg cccaggcgct ccttggctct ggcagcatct gcagtctctg 118620
ctctcatcac gtagccctcc ccaccgtgtc tctttgtgtc ccaagtcccc ttctgccttt 118680
ctcctataag gacaccgagc actggattta gagcccatcc caaatccagg atagtctcat 118740
cccgggatcc ctaatcacat ctgcaaagac cctttttcca aataaggtca cattcgtggg 118800
ttcttggggt taggtattag acatgtcttt ttgggggtca ctagtcaacc ctctacagaa 118860
cctcagacga aagtgaatct tcacaggctg aaatgggggt gaaggtctag tgcgtccctc 118920
ctccttggga gctccctcag ggcttgtgcc tatcctgtga tcctgtgatc cagccttttt 118980
tttgagatga agtcttgctt tgtcgcccag gctggagtac tgtggcacga tctgggctca 119040
ctgcaacctc tgcctcccga atttaagtga ttcttctgcc tcagccttcc aagtagctgg 119100
gattacaggc gcccaccccc atgcctagct aattttttgt gtttttagta gagacagggt 119160
tttaccatgt tggccaggct ggtttcgaac tcctgacctc aggtgatccg cctgccttgg 119220
gcctgccttg gcctcccaaa gtgctgggat tacaggcatg agccaccacg cctggcattt 119280
agtaggtttt ggttgttttt ttttttgag acagagtctc actctgtcat ccaggctgga 119340
gtgtagtggt gtgatcatgg ctcactgcag ccttgatctc ctggcctcaa gtgatcctct 119400
cacctcagcc tctcgagtag ctgggattac aggcatatgc caccacccct ggccaatttt 119460
tgtattgttt agtggagtcg gggctttgcc atgttgccca ggtttgtctt gaactcctgg 119520
gctcaagcga tctgtccacc ttggcttccc aaagtgttgg ggttacaggc gtgagccacc 119580
acgcctggct tagtaggttt tctagaagcg cctgaggcct ggtgaaggca tggtgtcatg 119640
gagtagagaa cattcaactc tgttcccggg gagccggaac acacccctcg tagccaatct 119700
caacagtcat gacgtggcca ggatgacatg gggagggggt ggtgtggact gtgtgggagg 119760

ccagctcccc gggaaggggg ctggagtggg cttggctttg aggaggattc ccaagaggag 119820
gaggaggcgg tggcatgctc gtttgggggg atggcgtgag ccagggtggg cacctcttca 119880
tttaggtgtc accttcttga cattccagcc gggacatggt ttccttgggg aagatggaca 119940
cccccagagt ggggcatctc ctggggaaga tggacacctc cggaatgggg cgcctcctgt 120000
tctcactgtt cttctagcac acagttgtat tctcttgcag ttctggaggc tgtgagatct 120060
cctggctgtt atctagaaca aactcgcctt tgagcagcgc cgtcctgatg gtggggtggc 120120
ccctgcgggt ggaacagccc ctgctgtctc agagcttact gagagctagt gggcatggga 120180
gtggtgctgg accgaaacac ccagagggat catagagcca ggcagtaagc acagaggcca 120240
gctagtgggg agggagggac ccatccatga aaagacacca agcattgaaa gagaggagag 120300
gatggttggg gaccggcaca catgtgcatt ttgcgcagat ggccaaggtg gtgaggcgcc 120360
agccacgcag acaactggag gaaggaggag tgccctaggg gaggggccag ctccggttct 120420
agaatgatcc gcttgtgcac gtgggcctgg ctggaataaa tcattgcatc cgcaccgtag 120480
actcaatcgt aagctcacac cagggcagcc tctgctttgg tgcagtgtct tcgatcatga 120540
gttggccatg gtgggggtga ctccaagggg actcccaagg aggggttca ccggatctcc 120600
cagggaaggt catttgtttt cttgggttgg acctgggaca aggccttctc ctgtctagtt 120660
tctgttctcc aacttgaccg cacggaagga tgctgaggac acctggaagc cggcccctgc 120720
tctacagcac ccataggaca gaggcacacc atgtggggac agtttggaca gtgctcagga 120780
ggcagggagc agatcagagg accgcgggag gccctgggaa ccgcacgagg acccaggcat 120840
tgagctctgc actttctggc atcagcccga tgtacagtct ggctctgtgt ctaccccgta 120900
cttcactttc cccatctgta cgggggcggt aataatattc tttacagccc aggtttaagc 120960
gggtgtagat gtgatgagct tggtaagccc atctcattac attcagttct gggcattgaa 121020
ccctggactt tctggctggt agaagtggac attcaacctt tccttggggc cctggggctt 121080
ctgcagaatc agaacacagc cagcagtttg catgcagggt gcagagggac agagggacac 121140
catgtcggga gcccacacct ggcacacccc tgactctggg gctcagtgtc ctgctgccct 121200
gcggccacgt gatcagctag aggtcatggc tggacccata ggcgaataca gcctgtggat 121260
ttgattttgt ttggccctgc ggtgttttat tttattttta gttttagttt gtggagacag 121320
agtctcgctc tgtcagtcag gctgagtgca gtgacacgat ctcagctctg caacctctgc 121380
ctcccgggtt caagcgattc tcctgcctca gcctcctgag tagctgggac tataggcgag 121440
tgtcaccatg cccagctaat tttttatttt tagtaaagat ggggtttcac cacatcagcc 121500
aggctgctca gtgttttaaa aagggcttgt tttgctttgt gccagcattt aaagatcagg 121560
agatatcata taaagatcca gatttctact ctcttgagaa gtgaacagct ctggccacgc 121620
cgggcccata tcctctaggg cagtgacgga gctgagacag ggcctgtgac aactgagagt 121680
tctctggagt ccacacggct ccttgcccag cttctggtgg aggttgagct tagtgaccca 121740
ggatttcgtc tgtgtccacc aagaaggaga ggtgcctgtg gcttagagag ggcgcccaca 121800
ggagtgcctt gcccaggaag ctggcagctg aggcggccag ggttaggggg ttgggcttcc 121860
tcttcccctc cccagtgcct gagtgcattt ggaggaagag agacgggtga ggggaaggca 121920
ctgaaattgg tcctggagcg ctattattgg gggctgggga ccagctgtca tccctttcc 121980
tgtgggcagg ggaggcagtg gtgggatccc atgagacccg gactctgtgg cctggtgagg 122040
cagggagcgg ctggggagac actgtgggtc tccacagggg tgcgctgtgg gtctggcctg 122100
gggaaggagc tggtgaatgt gcctgccatg tgggtccctc cctgctctgg ccctgataac 122160
caggagcggc tggctctcat ttggagtgcg gttgtgggga ctgtggaggt ggcagcttcc 122220
caggtaccat ccccactgcc tccgagggag cagaccctgg tggagaggca ggatggctgc 122280
agggcgggag ctggtggttg gcatcactgc acgggcttgt agtagtggat gggaagatgc 122340
aggagaggct gatgactgag aaagttggtt cctgtggact aagctggggg agccggacaa 122400
ccaagtggtt agaaccttgg gctctggtat cagcctgatg tacagtctgg ctctgtgtct 122460
accccgtgcc ccactttccc catctgtact gggggtactg gtattcttta catcccaggg 122520
ttaagcggct gcagatgtga tgagctcggt aggcccggcg cctcgccggc actgaaagct 122580
ccagtgtccg cccttagtgt cattgatggg gttttgttcc tgctgcggtt ctgattggtt 122640
acagttgcgg tcacttatta ttggaggcat gtcccccgct gtggctgatg gtagaggtgt 122700
gtttgctggt ggtgtcccag tgtgtgcctt ggctggaggt gaggagggat aaaaacagcc 122760
gcgacaagaa ggaaatcaag gccgggcacg gtggcgcacg cctgtaatcc cagcactttg 122820
ggaggccgag gcgggtggat cgcttgaggt caggagtccg agaccagact gaccaacatg 122880
gtgaaacccc atctctacta aaaatacaaa aattagccag gcatggtggc ccgtgcctat 122940
aatcccagct actggggagg ctgaggtagg agaatcgctt gaacccggga ggtgccgctg 123000
cactccagcc agggcaacag agcaagactc catctcaaaa aaaaaaagaa ggaaataaaa 123060
gtcttctctg gaactagaac gttcctatgt agacgtagct ggaggggggag gctgtgtggt 123120
ttgcgccgtg gccatggcaa gacacgacgg gcagcatctt cccactccag cctgcatctc 123180
tgccccagaa ctcatgatgg ttctcctagg atggggctgt cccttccgct tgctgagaca 123240

tgaaggacgg gaagagaccc ccggccctca ccgccgcatc gcctgccttc ctctgctgct 123300
ccttagcagg aaacatgccg gagtgttccc tagccatcca ttcaccaatt gcatcccctc 123360
tctttatttt tcagccttga aaagatcttt tgaggtcgag gaggtcgaga cacccaactc 123420
caccccaccc cggagggtcc agactcccct actccgagcc actgtggcca gctccaccca 123480
gaaattccag gacctgggcg tgaagaactc agaaccctcg gcccgccatg tggactccct 123540
aagccaacgc tcccccaagg cgtccctgcg gagggtggag ctctcgggcc ccaaggcggc 123600
cgagccggtg tcccggcgca ctgagctgtc cattgacatc tcgtccaagc aggtggagaa 123660
cgccggggcc atcggcccgt cccggttcgg gctcaagagg gccgaggtgt tgggccacaa 123720
gacgccagaa ccggcccctc ggaggacgga gatcaccatc gtcaaacccc aggagtcagc 123780
ccaccggagg atggagcccc ctgcctccaa ggtccccgag gtgcccactg cccctgccac 123840
cgacgcagcc cccaagaggg tggagatcca gatgcccaag cctgctgagg cgcccaccgc 123900
ccccagccca gcccagacct tggagaattc agagcctgcc cctgtgtctc agctgcagag 123960
caggctggag cccaagcccc agccccctgt ggctgaggct acaccccgga gccaggaggg 124020
tgagtcgcag agcgctaggt cttggatgct gtggtaatgg ggggcctggt tgctggcttt 124080
gccacagaat cttggaggaa gaagctggat atggggtgga gggtgctacc ctggagaccc 124140
agaaagaccg gaatgcatgg gggtgggggt ctgcaagctc aggagaggtg gctctctctg 124200
tctgatggga acatgccaag atgccccaag cgggacttga atgagagttt ggaaagattt 124260
tctgggtttc aaggagcctc ctttatgggt caccgtgggc cttgctggga cctctgtctg 124320
ctgtgtgtcc ctggagggcc tgggaggctt agagaatgag gagccacgca ggaaccaggg 124380
caggctcatg caggggtgta ggacagggtg gagttgagac ccctagaagg gctgtcacat 124440
tctggtggct cctgcttgag gaattgcagg ctgcctggat atgcccagca gaggccgagg 124500
gcttggggaa gggaggaggg acgtatgcct cctgaggctt gcattggagc ccatggtctg 124560
gtcctcctcc cccaggaggg atggttgtgg cagagggagg ggaacatgag gagcacctta 124620
gcctgcagga caaaattctc agggcactgg gaaggggtgc ctggaccagg gggctgtgtg 124680
atagctgcac acggttttat ttgggggtgc ccttctggcg agaggagaga ggccatgggg 124740
ctccagcagc gtcctgggag ggctcaagcc cacagccgtg ctgacatgga gcttacaggc 124800
aggggattgg tgccagtcct gtgggacaca gggcctgttt ggaaatgcca ggctgtggga 124860
catggttggg tcccttctca gaagcgtgcc ccctggcccc agcagggatc gtggctccat 124920
ggggctatag taaggtgcca gctgggttag aagcccctca agagccaggt ctgattctgg 124980
gcacaggatc agaactcagt aatgctcagt aagccttctt taggttggac aaacagcaca 125040
gtctgggttt ttgatttagc tgctgcttct tatttctaac cgtggtgaaa tacacaacat 125100
acaatgtgca attttagcca ctttgcagtg tacgtttcag cggcattcag tacattcacg 125160
gtgttgcgca gctgccaccg ctgtccatct ccggtaccct tttcatcctc ccaaactgag 125220
ctccgtcccc gttagaccaa gctccccatg cccctccccc agctgctggc agccaccatt 125280
ctatttttctg tctttattat gaatttattt gaccactcta ggaaccttgt gtgtgtggaa 125340
aaatacaata actgcccatt ttgtgtctgg ctcatttcac tcagcatgac gtcctaaagg 125400
ttcatccgtc gtgctgtagc atggggcaga atttccttcc tttcagaggc tgaataatat 125460
tctgttgtgc gatgcacttg tgttgtgtag ctgttcctcc atcggtggac ccttgggttg 125520
cttccacatc tctctaagtc cctctttcca tcttctttga cttttctttg actttttttga 125580
gacagggtct tgctctgtcc tccaggctgg cgtgcagtgg tgtgatcact gcttactgca 125640
acctctgccc cctgggctca agtgattccc cagcctcagc ctcccaagta cctaggacca 125700
caggttgcgc gccaccatgc ctggctaatt tttaaatttt ttttgtagag acaggttctc 125760
actaggttgc ccaggctggt ctcaaactcc tgggttcaaa tgagcctcct gcctcggcct 125820
cccaaagtgt tgggattata ggcatgagcc acccaacccc tttttggttt ttctttttga 125880
atgagagttc tccagtggtc agtgggcttg gggggctggga ggatgtgggg cctggagagg 125940
gtgtgggttt ggggggagac cagtgctcct catgctttca tgggcatgtg gagtgcacat 126000
ttgtcttggc gggtctgggg cctgggactc tgcatttcct acagaggggg catcggaaca 126060
cactgagtag ccagaggcta gtgggccttg gaaggtcctt tccggccctc agtgtgcgag 126120
tcccgtgttc cctctcccat ctctctaagc ttgactctga agagccaccc tgccccagcc 126180
acccagactc agtgacagtg gttgctggtg gcatggtgtc aggccaaagg catgggcagt 126240
ttgcagagtg gcaggaggct gtttcctccc ctgccttctg ttggtgcagg acctttgttt 126300
gacgtgccgg atacaccccc atcctgggtt gatgtgttca cactcagctg agtcaaacag 126360
gatgtggctg gggaggcggt tgtcaccgag ccatctaaat ctcggtgatg gctggtgctg 126420
gatgcacagg gacgtggtcc tggctctggg ggacaggtag ggggatgtcc atggggatgt 126480
acaagaacat tctcctccag gggcagacac agtttagccc ctaaattgtg ccagagactg 126540
tgccgggagc caggcccagg gacacaacct gcgggctctg ctttctggag ctcaccgagc 126600
cgtgagcagg atggctggga cacaaggagt gtccagggag cttccccagc agtggaagta 126660
caatttcatc aggccaggaa ggggagagaa aggcaggcca cgtggaggca gcagcgttca 126720

gagctgccag gaacctggcg ctctgggggg acggtggctt tctccatgga ataggatgta 126780
cacgttggag ccggggcatg ggagaaaacc atcagcatcc cagacccggc cacccacgga 126840
cagggacctg tgcaggtggg agtccagctc acgtggagag gtccgtgggc tgggctgaca 126900
gtcctgaggg cctaagcaag tccaggtgga ggaaatgcag ggacagacga gggcagggcc 126960
ttcccaatgg aggagtgctc ttgaaatccg atgggagtgg cttctcgggg ggcccaggcc 127020
tgagccccac tagttcttcc tccgaggcac cacctgcttg cttctccgtc ctccctctct 127080
gtgtcacgac cgttctggac acggatccag ttctctccaa ctcagcttcc tgcccatctt 127140
tccttcaggc ccatcccctt ctgcccctcc atgtggggct gggctgggag ttccctgagc 127200
cccgacatgc acagctctga ccaatctctg cggcccctct gtcccccgag caccagggat 127260
ccagctggca ccatggggaa aggaatcatg gagggagttc cgtgccctct gagcagacgc 127320
ccagcccctt ggcttctgcc tgcgggaggg catctgtgct gtggtttgtc tccaccccag 127380
agctgtgggt gcagggcagg gtctcctggg gccccagctg aattgctccc agactgactt 127440
ccccaaagca cagcactgct gagatctctt cccgtgctga gccttctctg ctcactcacc 127500
ataggcccct ccacggttga ttcactctcc tgctcagctc acacctctct ccttcccagc 127560
ttctcctgct ttggtcttcc cttggtctct cacacctttc ctcacaaccc ctttgccctg 127620
acctcctgaa ccctgacctc tgcttctctt tacatcactg ccttggtgaa ctcctatgca 127680
tccctcaaaa cctggtccaa atacctcgtg tttggcttgg gatataaatg atttgtgtat 127740
aaactttctt tcctctgcta gactttttttt tttaaatgtg aaagtagttt ttcagatcat 127800
aaaaagtaat cgagtaccct ttctttaaaa tgcaggaagt acagaaatga gaaagaacct 127860
aaaattcacc tgtaagccag cgatatccat gcttgaggtt ttgctgtata gtcttcaaaa 127920
ttcctttcat gcccccacta gattctgagg gtagggctgt tgtctttgtt tttttttgtg 127980
tttttttttt gtttttttgtt ttttgagatg gagtctctct ctatcaccca ggctggagtg 128040
cagtggcatg atctcagctt actgcaacct ctgcctccca ggttcaagcg attcttctgc 128100
ctcagcctcc cgagtagctg ggactatagg cgtgggctac cacgcctggc taacttttgt 128160
atttttagta gagacggggt ttcaccatat aggccaggct ggtctcgaac tcctgacctc 128220
gtgacccacc cacctcagct tccctgacct cccaaagtgc tgggatcatg ccaccgtgcc 128280
cagcctgtct tggtttttat aaagaatccc ataaggggcc aggtgcagtg gctcatgcct 128340
gtaaatccag cactttggag gccgaagcag gcagattgct tgagcccagg agtttgggac 128400
tagcctgggc aacatagaga aaccctatct ttacaaaaaa tacaaaaaat tagccaggca 128460
tgatagcatg cgcttgtagt cccagctact ggggaggttg aggtgggagg accacctaag 128520
catgggaggt tgaggctgcg gtgagctgag attgtgccac tgcactccag cctaggagac 128580
agagtgagac cctgtctcaa aaaaaaaaaa aaaaaaaaaa aaaatcccat aagataccaa 128640
gtaagtgact tggacataat aggtgtgcca tgaaattatg ctgaaggagt gagccactgg 128700
tggtgagttg ggatggcttt tttgtggttc ttgtaggcag atgtagtgac ccccatgggt 128760
cgtccctaga gaccaagcag aaactttcct ggtcaacttt acccccagga cacctggtct 128820
ctcctctgga ggttgtgctg gagggctcag tgggtgggtg ttggcttcag tgtgggggtt 128880
ctggaaggag tcagcatttc aggagggagt gtgggcacct agatggttga aggggaggga 128940
gaggaagaga ggcgctgcag agggcagagg catccctgcc tgctcctccg ccccagggcc 129000
tggtgaggtc tgagctttat gctggtgggg tggattctcg gtttccgagg ctctggccct 129060
gcccctggac ttggcaccct ccttgctgtc ctcccacaga agggcgctca tcctttgggg 129120
tcatccctgg tcctctcagg ctctgggtgg ggacggcctt gcctcctctc ctggatccct 129180
gctcttttcc tgccccccaa catgggtgcc tctgggtgga gtggggtggg gctgggctgc 129240
agcacccagt actgagcctg caagggctgg ctgctggctc agagcccccc ccaccagagt 129300
tacccccctc cggctcctca gccacaccag ccaagtttct ccagagctgc cttataaggc 129360
gggtgctcag cgctcgttca tgggtctggt tctctgcctt cttgggatgc tcactggggg 129420
caagagggag gccaggccca gtgcccagct cagttctggg catgtcccag ctctgcccca 129480
tgagtcaact ggttctgaac tatcattcca cccctcccag cctcaatttc ttgatctgtc 129540
aaagaggcat ctgcagaggt ggctggggc tgagctctca tgtatccacc caccccaaga 129600
accaagaagg gagtccacag tggttctggg gccacttgtg aacatggatg tgaaattggg 129660
gcggggtaga catggcagca tgtctcagta gttttcctgt gacccagagg gtctggaaat 129720
ctttgtcatg tgggtggagc gttgagggag caggggtca ggcccccccg agctgatgat 129780
ggtgggcgtg gctcttcgca tacttggtcc gcaccgggcc gtgtgttcca ggggcagaac 129840
agggaggaat ctcagcacag acagcagggc tgccagtaca gcgttgagta tagggttaat 129900
caaagcacag ggctccctgg aaccacccaa cacagagcag tgaagaggtg gctttgggga 129960
aagtggggtc agagctgagc ctgagtgggg ggtggggagg gcgggcatca gcctgcaagg 130020
gagggacacc tactgggccc cagaagttcc tggtggggat cagggactgt gaagttgggg 130080
agagggtgac cactgtgatt tagggaggaa gggccccata gacagcccag cttctggccc 130140
cgtgtttaca ggacacccca cataggctct gagcctatcc ctggactgtg gactccctga 130200

ggcagggtgt gcaggttcgt ccagtaccag gtgtaaggca gggggccttg gtaagcgtcg 130260
agggactcac tggtgtctga tgaagcagtg agtttggatc gtgtcatgcc tcgggtcctg 130320
agcctgcagg atttttctgt ggggctgtgt gcttctgctt ctggaggtgt ccagaaggca 130380
ctgacgttcc tgccgggatg ccttactcgg aaggtcccgc tgctcccacc tcctcccatt 130440
ttcctgagct gcttgcagag ggcacccagg tccccttta ctttgggatt gggtctcttt 130500
ctctttcccc ttctaagaac aagtaccctc cttctccgaa ctgtccctgg agcctctcag 130560
acctaggtct agagtgggaa gtcccgcatg tgctggggga acaggaatca atgcgggagg 130620
attggccagg cccggtgtgt ctccttcgtg gtgggacggg gtggggccag agaacagagc 130680
ctggctccag agcccaggaa gagaacccct cccctcgccg gcctgccaag caccgtgcag 130740
tggtgagagg aaaggagggc tggtccggga atgcagcggg tgccgagcag ctgtggaata 130800
ccttaaatag agcgcagctg cggggaaggg tggggccagg aggcaggcgc tgggggcggt 130860
ttctctgctc cgccccctgc agctctgtgg ccctgaccac ccagcctggc cccaggatgg 130920
agccccggcc cccagacaca tggcactggg tgaggccgcc tctcagcact tgtcccagga 130980
agtcagagca gctgccagaa gagagaggag ctgtgtctgt acccgccgcc tcccaggccc 131040
gggacagctg ccgggattga gggtctgcac aatggcgagc ctgtccggag gggcgcgccg 131100
cggccagaca gccatgctct gtgcggtgtg gacagagggc acattcagcc ctggaggagc 131160
gcactcagac tccccgcctc ggggcccaaa gtgtgggcag cccctaacca gaccctggat 131220
tccagcagac agcctgggag gatgccatgt ggtccaccct gacacactgt ctcacctgtg 131280
acctcatttc tgagagctgc agggaggcgc ttcctcagtt ggactccact tcctctccgg 131340
ggttttctgt gggtgttata agtgactctg tctccttcag tccaaggggg gctcctgggg 131400
tatgggctgt aactgacagg ctagagtagc ccatggccag ggctgagctt ccttcccgtt 131460
ctctctgaac actgggccca ggaaggggtt aaagtggaga tgctctgatt aaagttgagg 131520
gaggcatgga gctcccctga ccctgtcccc ccatcccact gctgctactg ggtgtactct 131580
gtggagcccc aggactctta aagaacccag agtggagatt cccggtctca tccagtgcag 131640
gtgaggggga aagtgatttg tccacagtta catggctacc atcagcacac ctgggtcaag 131700
ctcgattcca ctccacggcc cctgtctgaa acctggaact ggaaatggcc ctggaaatca 131760
ctgattccat ctgccgcctt ccaaaatggc ccgcagaggt taagatatag actcctgtaa 131820
tgccttgccc actcgtgtgt ggggaaatgg cagaggagca ccggctttgc cctcacactg 131880
cctgggctgc cgcccctgcc ctgcctagtg gctttcctct ctggtccgtt ttctcattgt 131940
ggaagtgggc acaattcttc ctgtgcagcg tggttagagt ggacagtgat gattcgtgaa 132000
gcagggtgga gcgcctgggg gattcgtgtt aaacaagcac ataaacaaaa gccaacacaa 132060
cccttactta atcagcaagc aggccttgtc tgaagcccct gggcagcacc tcatgccatc 132120
cctaggttcc aggccggtgg tgtcgccccg tgtctctctc acccgcaagc tggtgtttag 132180
aagccctgct ctgtgttctg gctcccattc tccctgtgac ctttcccttt cttcatccta 132240
aaaagcaatg cacgacgggg cgtggtggcg ggcacctgta gtcccagctg cttgggaggc 132300
tgaggcagga gaatcacttg aacccaggag gcggaggttg cagtgagctg agattgtgcc 132360
agtgcagtcc agcctgggca acagaacaag actcccatct gggaaaaaaa aaaaaaagcg 132420
atgcccagtc attcttagat agaaaatgaa gatcacatga aatttggaaa gaagtttttc 132480
tctcccattc cccaactctc tcccaagggg tggccatgtt gacagctggg tgtggcattc 132540
tcctgaattt tattcctgca gagtcattca gggcttgtgt aaaaaaagtc aatgtgtaaa 132600
tatagacttt tcaagaacac aaaggaaatc agacttctgc taccagcttt tattttttcc 132660
atttaacaac actgttgcca cttgtagatg tagcacgttc ctttttcttt ttcttttttt 132720
tttttttga cagagcgtgg cccaggctgg agtgcagtgg tgcgatctca gctcactgtg 132780
gctcactgta acctccacct ttcgccttcc gctttcaagc gattctcctg cctcagcctc 132840
ccaagtagct gaaatcacag gtgcttgcca ccacacctgg ctaattttg tatttttagt 132900
agagacgggg tttcaccatg ttggccaggc tgctcttgaa ctcctgacct tgagatccgc 132960
ccacctcggc ctcccaaagt agcacgttcc ttttaatggt tgtgtcatca gctgtcccat 133020
ggccctgcca ggcttgtcca gcttcccact gtgattgaga ggctgcaaca gatgttctcg 133080
cacttgtaca tgtgcacaca tgactgaagg atgagctctt agtgaatttc tgtgtcaagg 133140
atgtgtgcat ttaagttttg atctgctggg cacagtggct cacacctgta atcccagcac 133200
tttgggaggc caaggcgggc tgatcacttg aggtcaggag ttcaagacca gcctggccaa 133260
catggtgaaa ccccatctct actaaaaata caaaaattag ctgggcatgg tggcttgtgc 133320
ctgtagtccc agttactcag gaggctaagg caggagactt gcttgaaccc agcaggcaga 133380
ggttgcagtg agccaagatc gtgccattgc actccagcct gggtgacaga gcgagactcc 133440
ctatcaaaaa aaaaaaaaaa aaaaaaaaat tgatccttgt tcctgaattg gcctctaaag 133500
tggttactag aatttttag gacccctccc ctcgtcgacc tgctcagctg tctgctgggc 133560
cctgtgcagt gatggtccag cagggaggtc ctatgcccct taccctggc tgagggccaa 133620
atgctctcca attggtgttt tattttgtgc atctcggtca agcgagaggg attatctctt 133680

cgtatgttat aggccagggc cctgtgttct cttttcattt ggaaggtgta tccttttctt 133740
gttgaaattt tttaaaaaga gttctttgta agccaggcgt gatggctaac acctataatc 133800
ccagcacttt gggaggctga agtgggcgga ttgcttgagc ttaggagttc gagaccagcc 133860
taggcaacat ggcaaaaccg catctctgca aaaattagct gggtgaggtg gtgcgcctgt 133920
agtcccggct actcgggagg ctgatgtggg aggatcattt gagtctggga gtttgaggct 133980
gctgtgagct gtggtcatac cactgcactc cagcctgggt gacagagtga gatcctgtct 134040
aaaaaaaaaa aagagttatt tgtacatctt tatcacagca ggtacgagta tcttgtcccc 134100
agctgcctac ttttgatttt gtttctggga tgttcttcta cacagaacat tttaatcctg 134160
atattgtcaa gtctctgctt tccctttag cttttggaat tcacatcctg tttagaggtc 134220
tcctgcgttc tgaaattata ggaaagaatt tcacccacgt tttcttctag tacttctgtg 134280
gtttcatttt tacatgttga aatcatgaat ccattggaat gtattttggt ttcagcagcg 134340
aggtaggggt agatggtatt gcttggatac acagcagcga gcttggtctg gagcgggtgg 134400
ggcgtgtgtg tgtgtgtgtg tgtgtgtgtg tgtgtgtgct atttttatca gttttggtgt 134460
ctgcattgtg ctggcttagc aaaaacaatt tggatgcatt tcttttaccc tctgccttct 134520
ggaacatttt aagcagcaac ataataatct ctctgttaaa acttttcaag aattcactcg 134580
tgaaaccatc tgagcctgat actttgggg gagtctagca ctttaaaaac ttaaactttt 134640
tccatgggtc tgtgagtttg ggagtttttc tttcttctta aaattcatta ctattttttt 134700
cctagaaaag tgtcttttag cttatttaaa atccgtcagc ttattccgag ggggttgctt 134760
tagctcgctt agtgaggaca gtgtgcagcg cggtgattga catgattgat cagcagagca 134820
cgatcgatca gtcggtgtgc aaacatgact gactgtggcc agcgtcctgg gatgctccat 134880
gttgaggcac atgtgtgcag tgcagggtga tgtgtattct gagaggggat gaagccacag 134940
gacccctgca cccttaggag agagcccccc tccccaaggc tgcagtcttg agtaggtagt 135000
gacgggactg ggtctagggg gctgagtggt cccagcaggc caagggcttc aggaattatg 135060
agaagtttgg agttgcccag agagcagctt tcaaaaccag tctggactgc agcctccaga 135120
aagacatgcg attgacacca cgaaccagga cacacctata tatggggtgg gggtggggat 135180
gggatgtatt aatgaaacca aatttccgtg atggaattct tacctttacc atgcgtgtgc 135240
tacactcagt attttctttt tttttttttt tttcccctct ctcttttttt ctttttgttt 135300
gtttgttttg gagagaggga aggctttttt tcattttttt ttaaacagct ttattgagat 135360
ccaagtaagc tactatacaa tccaccaatt tatttattta tttatttttt tgagacggag 135420
tttcactctt gtcgcctagg ctggagtgca gtggcgccat ctcggctgac tgcaacctcc 135480
gcctcctggg ttcaaccaat tctcctgcct cagcctcccg agtagctgag attacagaca 135540
tgtaccacta cgcccggcta attttgtatt tttagtagag acggggtttc tccatgttga 135600
ggctggtctc gaactcctga cctcaggtga tccacccgcc tcagcctccc aaagtgctgg 135660
gattacaggc atgagtcacc gcgtggccca atccacccat ttaaagtgca taattccatg 135720
gttttcagtc tattcagaga gtagtgcaac catcgtcacc acaaccaatt ttagaaaatt 135780
gaaaatttga gaaatttaga aaattttttt cttaagaaga aaccccgtag cctttagcta 135840
tcaatcctgc cctcctcctc catatttcct tcctatctgt gtggattctt tttttttttt 135900
tttttttttt tgagataggg tctcactctg tcacccaggc tggggtgcaa tggctcaatc 135960
tctgctcact gcaacctcca cctcccaggc tcaagccatc ctcccaccat ggcctcccga 136020
gtagctgaga ctacaggcct gtgccaccat gcccggctaa tttttgtatt ttttgtagag 136080
atggggtttc accatgttgc ccaggctggt cttgaactcc tgggctcaaa tgatcctcct 136140
gccttggcct cccaaagttc tggaattaca ggcgtgagct actgcgcctg gccgattcta 136200
gacatttctt gtacagagaa ttgcacgttt ggcctttgtg tctggcttct ccacttggtg 136260
tcatgtttcc aaggtccatc cacattgtgg caggtgccag tgcgtccctg agtaatattc 136320
caccatggca tggaccattc cattgtggac agacatgttg ccttcatcag ctgagggaca 136380
ttgggttgct gccaccgctg gtgagtgtat tcttttcaaa ttaaaaaaaa aatgctggtc 136440
accacccact agatcgtttt cttggcccac agggtgggac aagccggcct gggtgagggc 136500
caggaggtgg ccaggtggag gcggctggag cggccctggg ttgcctctgg gcgcagcagg 136560
cagaggtgca atggcagagt ctgagcaggt tccctgggcg gatctccatg ccggtaatgt 136620
ttgtttattc aagaagtctc ggccaggcgt ggtgcctcac acctgtaatc ccagcacttt 136680
gggaagttga ggcaggcaga tcacctgagg tcaggagttc gagaccagcc tggccaacat 136740
ggcaaaaccc cgtctctact aaaaatataa aaattagctg ggtgtggtgg cgtgtacctg 136800
tagtcccagc tactcggtag gctgaggcag gagaatctgc ttgattccag gaggcagagc 136860
ttgaacctag gaggtggagg ttgcagtgag ccaaaattgc gccactgcac tccagcctgg 136920
gtgacagagt gagactatgt ctcaaaaaaa aaaaaaaaaa gaaaaaaaaa gaaatctcca 136980
gtgctgccct gtgcccggca ccgaagccga gggaaggcag cattagtatg agctcccagc 137040
ccaccggct cctgtctgga gctgacgtgc gaccaaggca cacgagggaa gcatggcgag 137100
gtggacggtg cggaggcata aggagcaaaa taaggcagat gcagtggctc agtggctcct 137160

ggtggctgcg aggagcgtgg gcaggacagc cccaggaggg cacctggaga gcagggccgg 137220
ctggggcaag gtgtgtcagg cacttgtcag gagcaaaatg taaggggaca ccaaagagct 137280
caggaatcaa cacagatagt atttaatgca gtatttttca atatcgaaat gatgcatagt 137340
aatctctatg atgaacaaat gttggaattt taaattaaga caggcccagc cctgcacgtg 137400
cacagccctg tcagagtttg ttcagcgtgg aatttgattt gtttaaaatg ttgcttcaaa 137460
atattgtttg attcctggat tttttggcac tgccttaact tttgcacctg gacgaaggcc 137520
tcaccctaat cccagctgga gagggtcaag ggctggtgaa tttggctttg gacctgcaga 137580
gcttgtggta cctgagaggt ggggccagga acaggaagga gctggtgaga acggcacccg 137640
gtatgggatg gggccagatc agagcagcac tgtggctctt tccaacccgt ggttttgctg 137700
ctggatgctt ggcgttggtg ctgagccccc ggccctctga acggagtgtg atgaataacg 137760
gggccgggga gctcttgggc ccctccctga agcagcagtg aagccaggga catgccggca 137820
gcaggcaggg agagggacgt ctggtctgtg cctggagcca gctgccccat ggaggagctg 137880
ggtgaggatg cggtgggagg aagaccttcc cagtcctgca tcctccgaag cataactgag 137940
tgcttgtgtg gcttggggt cactctggtc cgaggacagt cttgctttgc ttagccaggc 138000
tttgaagagc caccgggata cggacagcag ccctgctggg tggcaggttg tccaccctcg 138060
cccactcaac gctggaactg gatgcagagc acctggatgg ccagtcccag agtgtctctg 138120
acactcccag tcacctcatc ccttagacca ggataagatg cgggatccca acctcgagcc 138180
tcccatgggc tccattccat gtgctcccat aagccactgg gcccacctcc tggctccagg 138240
gctgtctggc catttggcat cttggtttcc tcatctgtac aatgaaaata atagtcccag 138300
ccttctcgag gagttacaag atgcctttcg tgtgaaaaac tgggatttgg taccagacta 138360
tctggatttg aattttggcc ctacccctcc taacctgtgg ctttgggcca atctggcctc 138420
cctgtcctca gtttccccat ttatagagcc atctcattga gttgctggga ggatgaacat 138480
aaggaagtgt actcatgtgc ccagagcatt gtgattttac ttattattac caaacgaggc 138540
acacaataag tgcctaatag atgctcagtg tcatttcacg gattctgtgt gccttgtgtg 138600
tctccagcag ccaagctctg gaggtgggga agctgttgac gagttgcacg aggtaattag 138660
aaagaggagg ccacaggtgc aagggtttgg gtgacacctg tggacagaga gaggccgatg 138720
gaccctggca gaggggaagg gggcccttgc acatagaaaa tgtttcctgc atccaagatc 138780
cttgtgactc tggaacagag gcagcagagg tctgcaaatt ccaaatcata aacccagaaa 138840
gcatagaaca ttcgtagaga ggaccagaaa catcatatat cttagtgtgt atagaatttt 138900
ccacatagcc aaacaaaagc acgtttctaa ctgaagctgt tggagagttc ctgaggactt 138960
tataccagac aagggccggt aagtggaagt tcaatacgca ggagccattc cttttgttca 139020
caaatgtttg ccgtcttttc tgtctatatc cgcattcatg tgagggcact cataggtcca 139080
ccgtccagcc ccatgctttg tttgctacca aaaaatttct taagaattcc tcagctgcaa 139140
atcctcctac cggagggggg gttccaggaa cccagccttg ctcggggttg gggcacctca 139200
gcacctgctg cacaggcgtt ggtcattaaa ggtcatgtgc cgtatccgtg ggccctcggg 139260
gtctggtggc acctgggcac acagtccagt ggcggcctcc tacctgccag acactgggag 139320
tggggggctc ctcattcctg ttccctggcg gtggggggag tgccccgagc tgtcacacat 139380
cagaacccac cgactcacac gttgcggcac cttacagccc cttgacagcc tctcagcctg 139440
agagaccatc aaactgagtt cctggagagg gtggctgttg aggcctggac acaggtgtgg 139500
acaggatagc cctgcctcat ctcggagtcc cgctccggtt cagcctcttg tgggtctgaa 139560
gctcaggctg ggccagtccc tgcccctcag atggccggaa aggcagccca gccccacggc 139620
ctccttgtca ggctctgaga gccgcctggc ctccagctct gctctttttt tttcttaaga 139680
tggagtttta ctctgttgcc caggctggag tgcagtggtg ccatcttggc tcactgcaat 139740
ctccacctcc tgggttcaag caattctcct gcctcagcct cctgagtagc tgggactaca 139800
ggcgcgtgtc accatggcag gctagttttt gtatttttag cagggaccgg ttttcaccat 139860
gttggccagg ctggtcttga actcctgacc tcaagtgatc tgcccacctc ggcttcccaa 139920
agtgctggga ttacaggtgt gagccgccac gcccagcctc cagctctgcc ttgattttct 139980
cttaaggccg tgaggatgtc cacgcctcct cctttcctca cggcccattg atgtctccag 140040
atgctctgca aagccaccct cccctcggga gctccccagc tggtgacggg ggacctttg 140100
gggatcactg cccaggtagt catgtaggtc tgtgggccgt gcttccaggc tggaccgaga 140160
cccggtgcat ggggtgcttc tggtctgagc tgaggcccct ggaagccaca gccaggaggc 140220
agcaagggac ggctgtcatg tcccctctcc cctgtctccc agggtccctg cgggacagat 140280
ttccctgcct gagcctcctg gttgcccata ccctggcctc ccaaagccca gcctgaagcc 140340
ttaggcaccc tacaacctgc ttcctctagc agcctgccct gtctcttctc tgccgatgtt 140400
cctgaaactt tcctgcctct ccttacctct tggccagaga gagactggag accctgtggg 140460
aacccccgag gggagggaga aggccgctgt ttgaacggag gtggtctttc cctctgcccc 140520
tccccaggcg ggtcacactt gcaggcccag cctcctgctg tggcagcgct tggggtgtcg 140580
gctgcctcct gggcacgagt tgggagctga gggaggcgct ttcaggaccg gagtggcaac 140640

ccagctggag gaggagtaac aggcccgtgg tcagcagcag ccgccgcagg gaccccggcg 140700
ctgtacctgt gccccagctg acccctcccg cttccctagg gtgggccggg gatggtggtc 140760
tgttgcccag cccaggaaga agaggggccg taatcagcag cagccacctc aggggacccc 140820
agcgctgtac ctgcgaccca gctgacccct ccccccttccc cagggctggc tgggaatggt 140880
ggtctgttgc ccagcctagg aggaagaggg gctgtggctt ccactctaga ggtctctctc 140940
ccggtccctc ccccggtgct ggtctaactg caggcctgcc ttaggccgtg ccggcacctg 141000
ctgactgacg cttagcggat gctgatagga gagggctggg atggggaagc agcaagtccc 141060
ggtcactgtg ggctcgggtt acagatggtt gaggcacctc ctggccatcc ccaggaagca 141120
aggccccacg cagcctccga ctcacccact gcccgtgtgt atttcaggag gctgcatccc 141180
ccaaggcccc caccaggtaa cttcattggg ctgggtgtcg ctgcaaaacc acccattggg 141240
tgcggtggtc ttctggtggg gggcatggct ggggaggatg cggtggagga gaagccagct 141300
ccgtggttgg ggggagggcg gactatccgt ttcctgtctg tcccctgaac tggagctcat 141360
ggtccctccc ccaggtgcct gggccatgga agaggtcaca gccctgcctt attcctgtgg 141420
cagcctaggg gagccctggc cagtcccgtg tctcctggga gggcgacagc ctggtcaggc 141480
tgcagggcag gactggaatg cagacccctg cttggccatg ctgttgcctg gctgctctgt 141540
ctggggacct tctgttcagg aagagagaag cgggccggac agcccggctc agtgattgga 141600
agctcagctc cccatgttca gtccagcagg aagtttgcct cctcggaaca cttgcgtgct 141660
tctgcatgtg tcttcttggc tggtggttct gcgaggcccc tactgggctg cgtcatgtcc 141720
tctggccacc ctggccttgg tgcctggccc ctgtgaaaca gagatacctg ggcagtcccc 141780
cacttgtgct tgcaagcctg tgtggcttca gtgatggctg attctgctcc acccagagga 141840
cctgggaatc ctcagcccaa ctgtccagag gagtctttct cagtggggag agcctgagct 141900
gtcctcctgg tggacagtcc tttagatctc aggcggactt ccctgccggc cgttcctcct 141960
gctgcccctg ctcctctgtc ccccatctgt ctcccaccct ctcccctgca gcagccagct 142020
ggcacgggga cctccccttc cctctgggat tgtgaatgag acgactttcc tggtccttct 142080
gggtctagat ctttctggca acagtcagtg aaacccttgt ctcaggtggg aggagaaggc 142140
tcagtatgtg gtcacaacaa cggtcctaac cacatcactc tcaaacgctg gctttcactt 142200
acccccttgcc ctgcccaaca gttactttca tcatcccatt ttccagatta ggaaatcaag 142260
gcgcagagag cttcagccac ttgcctgagg cccccacagt taagtgtctg gatttgaacc 142320
ctggagcagc ctggctgctg agctgtcctg gagacggggt actgtgcagt ggtcggggta 142380
ggggtggagc tctgccgtcg ctgactcccg ggctacctgg agtgataaca cagcccagtg 142440
tctgggcccc tgcctgagac tttggctgga tgccaggagc ccgagacctt ccagctcttt 142500
cctgtgtaag gcgccatggg gtgaaatgaa agaaaacccc agttagaagg tgtccccctc 142560
ttccttcagt ccctcagctg ccctggattt tgaaataaga caccgcccgt ctgtggggtg 142620
agcaggaaca gattggaacc gcatttctct ccccccagtc acagctgcca ggagctcatc 142680
tttctctgtc tcctccccct gccgcctctc cccacaagac tgggctggat gctgctggga 142740
aagtcaacag gaagtctttt ggctgcggtg gaggtggggg tctgtgcttc cctggggacc 142800
gagatgaggg ctccaggttg gctccggcaa gagcagggag gctcacgtct ctgcctgctg 142860
gttttgcttc ctgtcctggg agaaggtatc cacatgtccc agaatgcacc ctctgcgtgt 142920
acagggatgg cttttgggcc attcacctga ggtttattga tctttctctt tggaactgga 142980
gtggctcctc cacggtggat tgggctgagc tctctgcctc ggccgacgtc tttccccagg 143040
cacttcctct caccgtgcca gggtgcaggt tgctagtgga tgggtcttca ccctcgggga 143100
ttggcagctg ccacgctcat ttcacagtcg agacaccctg gccgaaggtt ctgctaagct 143160
ccatggagtg tagggatttt ttttgagacg gtctctctct gtcgcccagg ctggagttca 143220
atggcccaat gtcccgctca ctgtgatctc gacctcctag gctcagcagt cctcccatct 143280
cagcctacca agtagctggg actacaggtg tgtgccacca tgcctggtta atttttttaat 143340
tttaattttt ttttgtagaa atggagtctt actatatttc ccaggctgaa ctccaactcc 143400
tgggcttaaa tgatcctcct gccttggcct gccaaagtgc tgggattcca ggcatgagcc 143460
attgtgctca gctggggttt tcctttaacc cttcctggcc cttcccaggt gcaggacccg 143520
ggcctcagcc ttatgtgttc ttgaacagct ctgggctctg tcctgtacct cctgggcgcg 143580
tggtcaggcc tctcgtgaca tcagcaggag actcaggtga agaagcaggc agaacggaat 143640
gcttgctcac ccagcctcgg ggccaggcgg ggcggccagg acagtagcag tggctggtgt 143700
ggcttcctgg gaggtcagcc cattggcgtg gccagcgaat ggggaggggga gcttctcccc 143760
ttgtctcccc tcatttctgg tctctgggcc tccagggcca ggtctccaga gttctaggtg 143820
gaggatggag gtcagggaag ctggggtaag agcgggctgg gggtgggtgg tcaggcggat 143880
cctggaaaac catcctctgc aggtcccgct ttccccaggg agcactcagg gctggtcccg 143940
gggagccggg ctaatggtca cctcggatgg tgcaggggtc catccccatg aaaccgggcc 144000
catccccatg aaactgggcc cacagaagct tttggttccg gttctgttct gccccctggc 144060
tcttgcttcc aaagtaggac tcaggatagc tctgaattcc ctgccgaaat gcagcttcct 144120

gggccctaac ccagccctcc agaaccagag tctctggggt gggccctctg cttgtctagc 144180
agcctccagg ggacacttgt gcctgccaga gcaagtctaa taagcactgc tgtaaacccc 144240
tcgtccccac agccttctgg ttcgtctccc ttttcctgct gggatctgca tcaggattaa 144300
atgccaacat cagggatcag gttatacttg tgaaccctga gacaatacct gcatcttttt 144360
ttttcttgag gctgtcttgc tcgctctgtc atccaggctg aagtacaatg gtgtgatatc 144420
cactcatggc aacctccacc tccagggttc aagcaattct cctgtctcag cctcctgagt 144480
agctgggact acaggctcac accaccacac ctggctaatt tttgtatttt tagtagagat 144540
ggagtttcac catgttggcc aggctggtct cgaactcctg gcctcaagcg atccgcccgc 144600
ctcagcctcc caaagtgctg ggactacagg cgtgagccac tgcgcccggc ctgataccta 144660
tatcttgacc aagctgctgt ggaaacattg agttcccctt gatagttttg tccacagagt 144720
gaatgtttag gaaccggcga ggcatctctc acccccaccc agatggcggc ctggaggcag 144780
cgtttctgac cctgtgatgg gatttgtctc tcttgtccaa gagactttag agatgtggcc 144840
cctgaatgtg ggggcccctg ttggggtggg aacgggagct ggccctggcc attgtccagt 144900
gaccccatct cagtgcctcg atcctagcac cctcgatctc agcgcctccc cttccccggc 144960
tccgcgcgga gatcacgtgg cttgcgctga gcaatcctcc actgccccg tcgcgttcct 145020
gctgcaggga agcctttcca gggagccagc tgacacattt ggcctccatt atttgttttt 145080
gaacaggaat gaaaggagag agtgagtaag aaagtggcag gaggtggggg ttgcagagta 145140
ggggaaggtc agagagaggg gaggaaagat ctggaaagtc caggcatgtg ctctggaagc 145200
aggtgccagc gatgagctca tctggagggt ggccgcccgc cctgagccct gcctggggcc 145260
agcagggaac actctgccag ggcatgtcgc tgggagcctg tccttggctc actttctgct 145320
gctttgagcg ctgagcagat ttgtttccat tttagggaca acgcaaggag gctgtcattc 145380
cacccctcac cttattaata tttcatgaat cataagtgac cacttactgc gtcctgacgt 145440
actccaggcg ctgggctcga agctttatgc gcttcatggg atgtatctgc actatcgccg 145500
tagaaaacgg ttcctgttac tactgcagag aaggcaccga ggcacagaga ggttgaggaa 145560
ctggcctcag gccacacagc tagaagcggc tgaccctggc tgtgaacaca gggctgcttg 145620
actccaaagc ctgagctggt ttgcagcgag gctcatggtt tctgtgccac tgtttgagcc 145680
tctggtcgag ccccttggct cagtgggggg cccagtgacc taccagctgt tccattatat 145740
gttgtggaca cctccacggc ccacctgggt gggagggaag gggcttcagg aggtgggagg 145800
ggtacaggga acacagtcca gcctgcttgg gcccaatacc ttgaccttca aggtacccct 145860
tgagggtagc atgaggctaa gctcttgttc tcctccagga ctctgttcct gtgagggcct 145920
cggggtccct tgcttttttc tggggccctt gctggtcagg atcacagaga agctctttgc 145980
tgttgatctt tctgcctctg tatcttttgg caagccattt tatgactcag ttgctttgtc 146040
tgtaaaatgg gttgaataac attgtcctgc ctcggagggt ttttgggagg atcaaatgag 146100
acctgttgca cagaagccct ttgtaagcca tgctgactgg cggtcgccgg gcccccaagt 146160
gaatgggcat ccgggcactg agtgtgtgga ctcagtactc ggaaggaggg gctggggggt 146220
acctagaact cctgggaggc ttggacccct gctctgctca gattctgagc ttctccttgg 146280
ccagtttaat tcccatcagt agggtgaggg taaccaggac ttacgcatag ttgggcgaag 146340
tcgagtgacc acatgtggaa gtctagcgtc cttcatgcaa agtgggcagg cggccaaagt 146400
gagctctgct ccagtccccc cacccccggg gctgttcctg gcgccctgcc ctcccccaga 146460
gccccactga cctgcaccat gtgacccggg cctgggaaga tgggcccagc tgtgggaggt 146520
cgtgcgggct ggggactgag agtgccggag gagggcctcc caggaaaacc cagctgcaca 146580
ggaagccggg ccagcggcct ccggaagcct ctgggctttg caagctggac tccccagatc 146640
tgtggctgtg gggccctctc ctgcctgcag ctgagagccc ttgacccgca cctgagggct 146700
tcagaggtcc gggaggggga tgtgaccgtg tccccagggt gaagcccacc cgagtgtcac 146760
tctggataca gccctcacct cttcctggcc ccctctctgg tcatggggac gctgcctggg 146820
gggggttccc ttacatggaa ggagctgcag ctgagctggc gggcctcgca cacccccaag 146880
gccgcctgac ttccagcctc caagtggggt tgcattcacc actcaggacc tggagcctca 146940
gccagagagg gaccctccac tgcctctttc tctgtctctt gggaaaggta gggtctttgt 147000
gccccctccc acccagggac ctggcatgtt gggaggctaa gaggaagctg ccccccgctc 147060
atctaccccc caaccctccc agccagcaga gttttacctg tgtgcttaga atctgtgcgt 147120
ggagccagaa gtcacaaggt tggcccagct gtgtctgacc ctggttgtgc ggtcttggac 147180
agtcccagcc ctcctggagc tgagcatcct gatctgtaga ggagaggctg ctgtgccggc 147240
cccgcagggc cctgtgcaga ggggagtgtg tgcggctgtg agttcaggcc atgccctcag 147300
actggaggat aggatcggaa cagtgggtca ggatggccag agggtcccag cctcaggagg 147360
aggcctccac acctcccagc acctccacac caggaaatgc agccaggccc ccaggcctcc 147420
catgtcagga agcagcaccc cgccccccac ctctggttgc ctgggcctga gaagttgagc 147480
actcctcccg aatccgagtg ccctctgacc cctgcttggg tagttgtccc tcctgaactg 147540
accgcctgtc ctacacagtc ccctgtggtt tgcccgacag ggtggccagc gagtcccctg 147600

cacagcacac ctcccccagg agtgctgcct gtgatgatgg tgtaaagggt gcccccccga 147660
gcccccggag gcatgcagtg gcgcaacagc gtgagcccag gagccccggt gagctcctgg 147720
atggagaatc tgttcagatc actccccagc ctaaaccttc cctgggtgtc tgctgtgctt 147780
ggggcgctga ctcatcgtgg ggcggcctgt gagaccctac gtcacctggc cactcccctc 147840
agcccctgtt cactgcaccg gtcccctggc ctggctgctc ctggaacatg agggctcatc 147900
ctcacggaag aatattcact tgttccctcc tggaaagtcc ctccctagac ctcctggccg 147960
cctcattcag gtcttgctga agcgctgcca tcttggagag gcctccctgt cacccgcagt 148020
taaaacagcc ctcccatgcc tgtgggtggc tttctatggg gcatggatga gtattggaga 148080
gtcttgccgt ttatttgttc atatgccctc tgggagggca ggggacactg acttgtgttg 148140
tgggtgcctt gcatgtcacg ggtgctcagt gaatgcctgt taagcattag gggagcacaa 148200
aggagggaag cctctgtcca actggagcag tcagggaaga cttcctggag gaagtggtat 148260
ctggtctgct tagaatgtgg cgatttgtct gtatgcagac attagattag agggcaaagg 148320
ccattatcat aacttagcag acagctacaa gcagctaagg aattgctatg acatggactg 148380
ctcgtgattg gagctaagaa ggagtcagag acagaggtcc ctgtgctctg gcgtgagtcc 148440
ttgggggagg ctgagctccc agaatgtggg gccgcctgtg cagaggccgg aggtgggaga 148500
cacacctgtg agtgccggtg agtgtggggt gagccactgg ggctggatca gaagatggga 148560
atccctaaac ccaccaacca gccttggtgc cctgggagtg acaggagacg gggcttctgg 148620
gggactaacc cagcacaagt ggacgagatg ggatgcaggg gaaaggacaa agctctctga 148680
ggctttggga agctgagtta gggtagagcc atgacagggt gagttccaga agcaacacaa 148740
gaaaaaatag ctagactggg ttttcccttc actgtgggag atgccccacc tcacttcccc 148800
cgtcctccgg gctctggcaa tgggctgggc acagtgaatg ggttccatct gcaggagtgg 148860
tcacacccct tgggcacagc tgcctcatac actgtattga gatgggatcc gaggccggga 148920
agaacagctt cttctgtagg ttgacattgt cagccttcgg ggagggaagg acggcagggg 148980
gcctggagca tctccttttg ccatccacac tctgtgcctg cctgcagggt cttgagccct 149040
gtctgttctg cctcagtcct ggtcaggaac ttgttggagc ccatagggc agtcaggctg 149100
gaacaggagg agaccaggcc ggcaggggag ccgagagggg gccctggaca gcacccaggg 149160
ctgagtcatc ctgggcctgc ccgacttgct atttggagct gttcccgctt gctcatcatg 149220
tgcaaggtcc tgatccactc gggtctgttt aaccagcgcc cctccccagg cccagttagc 149280
ctggttttcg ttcgattcat ttattgtcat cagtgccatc cttgcaccac gtaactagcc 149340
tttgcagcat gccagccacc aagctcaggg ccctctgtgc atctcctgta accctcacag 149400
caacccggga ggcacagaga ggctgagtcg ctggcccata gtcacacagc aggtatgggg 149460
cattgccagg atttagccgg atctctgact ccagggcctt tccagggcct tgggtgggac 149520
actggcaagg aagcaaggta tgaacactgg ccctgccttg gacagagctc ggggacggtg 149580
gcctggcagg cccagctgac acagtgaccc tggtgcaggt tcaaccattg gcaaagtgtt 149640
caagtacaca cagaatctca actggggaga caggcgaagc ggtcaaggga gggctgtgag 149700
tgacaggtgg acactgtacc tactggcaga tcagctgacc ttggtgcctt ctggagcctt 149760
tcaacttcct tatccagaaa aagaggaact aacagttcca cccccaaact gggtgggagt 149820
accacattcg aaattgaagc aaagctggtc gtgtcacagg ctcggcagat gttagctgtg 149880
caggcaactt agtgaccatt tgaagtaggc tgtgcggcgg catcctcatc ttgcagatgt 149940
agtccagaga ggttagggca ctaaccctgg gtcacacagc gacagctcag gctacagaga 150000
gcagtgctgg atggcagggt gttggcagct tcaggcacag gctgggaggg ctgtggggtc 150060
agcagaggga gggcacactt cccagctggt ggggttccct tgtgcctcag tactcatgtg 150120
ggcccaaagg agctggaagg agggaggtta gagccaccca tgggttcaga agctgataag 150180
caggttattg actctgaatt tgcctcatcg tatgtaaatt ggggacaaat ttggggattt 150240
aatgatataa tgtaagaagc agacatgtca cagccctggt tatacagtca gtgtataata 150300
agtggtaagc cgtcagagga ggcagccggt gagaatggga gcatctcagc agccctccgc 150360
cccctgctcc tggttgcaga tgtacctgtt cttggctatt tgtgccccag ctccgtgtcc 150420
tccggtgtgt gtgaggccaa gctcctgggg tggggacttg gggtgtgtc tgcagctcct 150480
gaggccaaga ccaaggctga ggccgaggct gaggctgagg ccaccttggt gaggaggaaa 150540
ttgcaggtgg agaagctcgt tgaccgtgac cttgatctga ccgtaatttt gatggtgccg 150600
atgccgtcag cacgcaggcc tcctgccctc gccacgactg gctcctgcag cccacctggc 150660
tgagaggtgt gctggctctc gcaggttgca aaatggggac atcaccgtcc gcctgggcta 150720
cagcgtgctc gccgattgca tttggggagg gactgagggc tgattgtgtt gtggggatgt 150780
tggcagagaa tcagagaggg catccaaaaa ggccaacagt tcggtgggga gaggaggcgc 150840
aaggctggtt cctgttttct gggctttgat cgaaagggaa caggggacac atctggggac 150900
agtctccttc ctggaggaag aagggcttag ggtgctggtg tggagtttgc acagatgggg 150960
ataatgagct ggagggtgct gggcaggagc tggccagtaa taggctgacg agggaggccc 151020
tgctggctgg gcctggtttt caagtgagca tcctgagctg cggggaccca gggggtctta 151080

gagacctcag agctgagact cgggaccaca gaggggtggg ctgtgctcag ggcaacacag 151140
cggatggggc agatgttgcg aaacctgtct ctctgcctct ctttctcctg ctacagaact 151200
ggatcctccc agctcatggc tgtcaaatcc tttagatgct tctgggttcc atgggcgcct 151260
tacactggct tccctgtagg acagcagacc ctgggggttg gctctgatgg tattcaccat 151320
ggggtacccc atgcaagcac agtgctttca cgcccagcct cccagcctgt ggagtaggcc 151380
tgtgtctggc cagcctcact ttgtggccag aggagggaga cagtggttct ggagacagga 151440
gctggcctgg ggctgccctc ccagcccctg ttctcccagc tcatctcctt gccttgcggg 151500
tgctggccac cctccccccg ccagctccat ccccagcttt ctctgcccga gccgtgacct 151560
gtcagtaggg ggcagtggag gccttgaaag tccttggaga ggactttgaa gcagttttta 151620
caaatctgtc cctggaaagt ctctcctgtc acacccccag cggctccctc cctcctaaca 151680
tttttcctgg ggttgtcatg cagcgggggg tggaggaacc cactaggccg tggatagagc 151740
agagccatgt tgacacaaac cctggctgaa cccacatgct ccctacagcc agcggccgat 151800
cactggaacc agggaagtga cctcatagca agggcccgca gagcagccat cacgcattcc 151860
cgatcttggt ttctggaccc agctctggaa aagaagccag atcctagcca cttggagctg 151920
cctagggaag accccagcct ggtgccggct gacagctcac tggaactttt catctgcttg 151980
gccaggtgat gccaaggggc agaggttaga agtgctaaga agcaggccga gggtggtggc 152040
tcacgcatgt aatcccaaca ctttgggagg ccaaggcggg tgaatccctt aaggtcagga 152100
gttcaagacc aggctggtca acatggcgaa accccatctc tactgaaaat acaaaaatta 152160
gccaggtatg gtggtgcacg cttgtaatcc cagctactct ggaggctgag gcaggagaat 152220
ctcttgaacc cgggaggcag aggttgcagt gagccgagac tgtgctactg cactccagcc 152280
tgggcaacag agcaatactc cgtatcaaaa aaaaaaaaaa gaagaagaac aagaagtgct 152340
aagaagcatc tgtcttcctg attgaaaagc aatctttgct gggcgtggtg gctcacgcct 152400
gtaatcccag ctttgggagg ctgcggtggg agagtcgctt gaggccaggc gttcgagacc 152460
agtctgggca acagaggtgc taggggtagt ggcgcttgcc tgtagtccca gctcctcagg 152520
aggctcaggt aggaggattg cttgagttag gagtttgagg ctgcagtgag ctatgaccac 152580
accactgtac tccagctcct gggtgacagg atgagaccct gtctcaaaaa acaaaacaca 152640
aacaaaaaac caacgtttat cttttgtgat tttaaaaatc acacctactt gtgacaaaat 152700
aattaaatta gaaaatgtaa aggaaatgat caataataat tgcgtcctcc agacaaacac 152760
tgttaacatg ttcacgtttc cttctagtct ttttctacat agacactcaa tacttattaa 152820
tttgttttga actaaagatg gttttctctg ctttgtatga gaaccacctc agccaggcac 152880
ggtggttcac gtctataatc ccagaaagct gaggcaggca gattgcttga gcccacgagt 152940
ttgagaccag cctgggcaac atggcgaaac tccatctcta caaaaaaaca caaaaattag 153000
cctggtgtgg tggcacatgc ctgtgctccc agctcctcag gaggctgagg caggagaata 153060
gcttgagccc gggaggtaga ggttgcagtg agccaagatc gtgccactgc attccagcct 153120
aggcaacggg agtgaaatgc tgtctcaaaa aaaaaaaaaa aaaaaaaaaa gttgccacgt 153180
tgtcacctct ctcagggatt ttcatccttt ctacttttcg cccaagactt gccctgtaaa 153240
aggatgaggc caaatagcca tgggagggtg gtactggatg ttcaaaaaaa tgtttcgtgg 153300
ctggcgggac ttagaaacag gttggggagg tttcagcaga gggtggaccc cagctggggg 153360
tgggggcag caggggggcc cctcacatgc tctctctgcg tgtttcatgg ggctgcctgt 153420
gaggccgtgt gaaccagagc ccggctggga agccacatgc tacctggact ctgacctttc 153480
ctggcagcat gaccttagcc aatacgctca actatgttcc cctctgtaaa atgggctaat 153540
agaaatgtcc actcgagaca tgatgagaaa caaaggtgtt catagagcaa agtgcttaga 153600
acggtcctgg catgacagga gcccacatgt cagtgctgcc gttggcatcg tcatcatcac 153660
ggcggcattg gctgctttgt ttctcccttg tccctgtcac cttcttccct cagcaggctc 153720
tcattcactt gttcatttat tcatggccga ggccccgtct gtgtcaggcc tggtgctgag 153780
aacgggggta gagagatggg ggctgtgccc tgctccctga cccaagggtt ccctttgcaa 153840
tgtgaagttc ttggagactg gaccctcagg ctgttccacc agcccctcct caggccagct 153900
taacccttcc gtgctcgttt tctgagctgt tagaggaaga gagcgggagg aaggatggag 153960
tggggttttt gtggggactg agcggggggt gtgtgaggcc ctggaaatgg tgcctgcggc 154020
actcacgcag cattagcttt tgctgcagtt gctgtttgga gaatattctt ggatttcagg 154080
acactgatga cagaaggtgg aacgcttttt ctggaagcca tcaagagggg accaccgcgg 154140
cggctgagca gagttgctga cttgtgagct gtggcctgac cacagtcact gttctcatga 154200
gcgaggcccc ctgtggtccc cctcccagga atttctggcc ccttggcagg ggacagttca 154260
gggctggggg tgggtagtgg tgtctgcagg ccctcttgga gtcagagaat cccctgtcgc 154320
cgtggcgggg ctgttccctc ctgcccctcc cctcccgctg tgctctcagc tgctccgaca 154380
ggatgctttt ggcctgagag acagaaaccc caactctgaa tggcttcagc cctcaagaga 154440
atttggagct tttttgtgca aaatgagatg ttctgagcat cgaggatgct gtgggggatc 154500
ctccatccca ccatctccag ccgtggtgtc cctctcggtc acaggatgct gcagccacgt 154560

tgcagggaag gagagcctcc cttcctgtga gcctagggag cccctccatg cagccccttc 154620
actggccatt gcctgagacc cgacctggtg gctcctgccc caaggagcag aaagggggtt 154680
cgctaggtca gggtggggct gggtgcgagg accccccccg gccaacaggg tctgcttagg 154740
tgtccgtcct gttggggcag cagggaaggg acccacatgt ggcactggga cacggagagg 154800
cttttgtgcc tgtgacggcc aaaagggatg gcgccccagc cacgttccca cccaccccga 154860
ggcccagcat ggccatgccg gaaatagcag catcgtcccc ctcactgtcc tggcttgctt 154920
tcaataggcc agaggggtgg ggctggagcg ggcgtctgac ctgtggctgg atctagctgc 154980
ccgtggacag gctgtgagtg aatctgcaga ggaagggaga tgggaatgcc gggaccttct 155040
cctgcacctc ccgaaccccc gttccctgtg agttagatgg ggagtgcacc cctgccctcc 155100
cctcctgggt atcccctgcg ccccccgccc caggcacttg atgttgcctg ttcattttcc 155160
ctctctgccc ctcccgctgg cccttcccat atggtcctaa ttaggacctt ccgcccgttg 155220
gtggatgggc agagagggga gcttgctgtg gggcctcccc cgatcggggg acttcccagc 155280
acagagcttt ctggcctctg cagggacgct gtgtgtgcct gtgtgagtct ggcgtgttag 155340
aagcgctaag ctgcagctgt tctgtgcgtt tttcacattt ctcacttctc aaaggccctg 155400
ggtttggtgt ctgtgggctg gggatctgtg ttggggtctc caggaggccc ttggtgggag 155460
aggcagggcc ccttcccctt taatctgggc aaccttgctt tggcctgctg tctgcggaaa 155520
gtgccctagg atacggtggc aggactggct ggggttctgg ctccaggccc agccccagcc 155580
cctgtcagac ttaccctcct tgtgcctccg ttcaccctgt ccacccgcag ggcctgcagg 155640
agcatcctga gagttggtag ggtggcctgc ccggcgtccg cacagggtct gctggtgggt 155700
ggctgaggct gcggtgaggt ctccatggct ccctcgtcct gcacatcctt cccttgttgt 155760
gttgtctcgc tagcatatga gcccgtactg cctctgagtc tgcagcctgt tttccttacc 155820
aggtggctaa gcagggtggc agccagggtg gcagagtccc attggcctgc aggggaccct 155880
ctgtgggctg caggtagtcc cctgtggttg aaggtgccct ggcaggacct gcaccatgac 155940
ccccgtcaaa gctcacgtcc aaggcatttg tgggcacccc cgccccagac gtgagggttg 156000
gcatcttctc ttgcacgtga agcaaaggcc tgtttgaagg agcgtgtgga agcctgggtg 156060
tgatgaaggc aaggaccggt ggctcacccc tcttgcacct ggaccttggg tggcaggcct 156120
ggggccttca gtccttaatt caggagggta gccagtcgct ggaactaaga gtgaacttca 156180
gctgttgttg ccatagctgg agggaagagg ggaaaggagg cgctgcaggg gagcagagac 156240
ctcacccttc ctctgccgac atcaggctgc cggtgctgga cggggccctg gcaaccgtgg 156300
caggagtggt gatgtccgat gatggtaaca agggcttcct gaggaccccg agctgtctta 156360
agggctcttt acctggagta agtatcccct ttaatcctcc caacaacact gtgaaattga 156420
ttctgttgtt attcccattt caccgatgag gaacccacag cccagagatg ttaagtaacc 156480
tgcccaagcc tccctctgag catggcagag ggagggtccg aatgcggaaa gtctggcttc 156540
agtgccccca tccctaacca ctgtcattta cctcttcggc cagccctctg attgcaaaac 156600
ctgaggccca gaggagtgca gtgacttggc cagcttcgca cggcaggtta gtggcagagc 156660
tgagcagtgt ctcccatgcc ctgattctgt ggcttcttga ggtggctcac ccctaaacac 156720
gccccccatg gtggctctgt ttcctcctct cctctgaacc tgccagcctg tgctcaggca 156780
gggggagaag tagagcaacc atgccgggcg ggtcgtcgtg ccccagtgcc gcctgcccta 156840
cacctgattg gcagcagagt ggcccctcct tggtgtggtc tggaggtgcc agtggtcact 156900
catcagcaca gcccagtctc acacatgtca ctcccaaagg atgttgacac agaatgaagc 156960
agctagggga ggcggacgct ttggcacgag ggtggcgttt ctggtaatcc gtagtgctgt 157020
tgtctgtggc tgcctttggc tggcatctca cctcctcctg gctgtcctgc agccacctga 157080
gggaggtcag catgtcgcct tcatcccagg acagtgtgaa tgcagcagcg caagcttgcc 157140
agcgccgcac tgcgggatct ggaggccttt gctcctgagc cctgcctctg tgtaatccct 157200
aaagcacgga cttcttaggg gcttccattc ctccacctgg aaaaacctta caccgtttag 157260
ggcttgaggc agggctagag catggagacg ttgctggagg accaagggag aactgagcgc 157320
gtgttctctt ggcctcagcc tcgtgtgtgt gtttatgcat gggtactttg ccatccacac 157380
tggcttccac ggctgggtgg gagttgagtt gagctcggcg gctgcctttc caccctgcgg 157440
gcctggttgg ggagtgtgag gatgcctgct ggaagccaac gtgcacgaaa gagttaatgg 157500
tggatcccat ttcctcagcc ccctctccag ctttcccagg gaaagggcag gaacagagag 157560
gccttatctt cagaggaagg ggtgggcggg gacacagcgc tgttcggcac agtcccctct 157620
gagccaccct gcatgtttca ttccaccgac ttctgcagcc cagctgttca gagcggatcc 157680
agaaggtgga gagagggttt ccaataagca gaggatggag tgaatgtgag attcattact 157740
gggatttggc aaagcaaaga gcctcacttt ctccccgcag atgggacggg ggcggggctg 157800
gagccagcgg ggtgggggta tccagcaaga gctccttccc atcctcccac ttcctttgcc 157860
atgggccccc tcctccacgc agaccctcag cctggaatga tcagtgtcca gcttttctcg 157920
cccctctcct gccccgctgg agggagtgac ctggcccctg gccccggcca cctacacctc 157980
ctgttttgct gcactagcct cgtggtaggg gctgcggcgg aggtgggcaa aagccgcttc 158040

cgggcagcca gcccccatcc tgggagtgac tgaaagtcct tgggggttcc tgcagggtga 158100
gctgcccatt ctggccctgg taaggacacc caggagaggg ccagggtgcc tgtatttggg 158160
gcacagcata aagaacagcc gtaagtgttt ctcaggcagg agggtgccga agcattggga 158220
agctgggatt tagaagcatt gtttggggtg gtgagtggaa gatgcaggac acagattcac 158280
gcgattgtgc agatcacctg gcctcactcc ctcggtttac agtggaggaa actagaggcc 158340
gagagaggtg aagtgagttc cctggggtca cacagcctag ccaggcccag ggcgccgacc 158400
tcctgctctg ccttcccgtg cccattcggt tggcatcagc atccctgcct tgacaccccc 158460
gtgtggcagc tgctgtgatt ctgtgactcc tcctcacccc accccagtgg ggccctgcga 158520
gggaaagaag aatgccctat gcagaagtgg ttggaaagca agaagcctgg agagaagtga 158580
ggcctctgtg gtttggcctg agtccaggac tcatggcctg gttcccaggg atggagcacc 158640
gacccctccc ttctacaccc ccaccccacg cccccaggag ctccctatgg ggaagccgga 158700
atggacctgg gctcagagat tgtaaattca tcttgctgga tgtgtggctg cctttatgta 158760
accaactctc tcacggagaa gccaggggat ggctctcagt actctcgggg gtctctcagt 158820
gaggtgggag gacctcgaga ggtcaggagg ctgctcaaga ccttctgccc ctcagggacc 158880
aggtgagggc ttgtcgactg ctctgacctc ctgatgcttc tggtggggag acctcagagg 158940
ggcctgccgt caccatcgcc agtggccctg gcctgcccag tcatccttct ggggtggcca 159000
aactccatgc tgcaaaagcc caagtgaacc ccagtagtgg cctgggagaa tcatctgtca 159060
cgagccaagg atggagtttc tggggaggac tttctgctgc cccccaaccc ctttcgggta 159120
cattctcctg tagccccact ccctgtaaga aagtcaggtg cctcccgaag cttctccctg 159180
gccccaggcc agaggtgaca gtgggggccc cttgcttgcg ctggtgactg tggctggttg 159240
ttggtgcctg gccctgccct gcactgtgca cccattgaag gcggcatcat ccgggcctct 159300
ccggtggcca tgcatgggtg agcagcccac gacacatctg cttccagcat cgtgtgggcc 159360
ggggctttca gaggggctga gggcttggat ggccctgcag gggggtgctc ctggccttgt 159420
gaccgaaagg agctgaaggt ttggagaagc cgttgggccg aggtcagttc ttatagattt 159480
ggcgaggagg gaacagatgc tcacggagct gctccaggca ctgcacacac tgcctcactc 159540
tgcgctgaca gccaagaggc tggttccccg ccgaccccca ttttacagat aatgtgctcc 159600
cccagttcct tagtggtgga gccaggattc gaacccacgc aggcagcctc ctggcctgt 159660
agtcctaccc ttcatctccc tccaggacct atgtcttggg gttggcagga taatgactcc 159720
cagagttgag cacatcctaa tccccagcac ctgggactac gttaggtgac atggcagggc 159780
cagctaaggt cactaatcag ctggtcttaa aatagggaga tgatcctgga tgatccacac 159840
gggcccgctg tcatcacaag agtttttaaa agcgcaatag accatctgag gggtgcagtg 159900
ggagaggctt ggaaggagga aggaggccac aagccaagaa atgcaggtga acagaaaatg 159960
caggaaaacg cagtctcccc tgcagcctcg ggagggtgcg cagccctgtg gacaccttcc 160020
ctgtagccta gtgaggtgga gtgtgggact aatctgtgtt gtttgacatc acaagtgtgt 160080
ggtcatttgc tacagcagtg atgggaattg aatgccatta ggcagcagtg cttctatccc 160140
ggaagaatta ggataaattc ccctgtggat ctcaagctct gggagattct gggctcctct 160200
ggactgctcc agttctgcaa tcataggaaa ggtctcccat gtctgtttgg gagaagaaaa 160260
ccccgtagca aatccatttc cccttttgac aaatgaaggc gagggtgtgg gctggacaca 160320
tgtttccttt ttgtggattt tttttgtttt aacacttaga attctgtagc tgaaggagac 160380
tttggggggt tgtctgcctg atttgcaagt gaagaaatgg aggcccaggg aagttaagtg 160440
actggcccag ggtcatgcag agtcatgctg gaatatcaag ctagatagcc cagttcctgc 160500
tccaaagtgc tttcctccct ggttggggag gagggttgga gggagtggac cccgagcaac 160560
agcagcattg gcaggggttg gaagagtagc tactgtatcc aggtgctgga gcctctggcc 160620
acttcctgca ggaaccaggt gacccaccat ggcacaggcc tctccctgtc tatctgcagc 160680
cctgggggcc ccagggacca gaggacactc tggcctgagg gctggggcct gagagaagag 160740
atggaagtac ccctagtctg gcctgccccc gtggcgtcct gcaggcatat ccggcagccc 160800
aggggtccta gagaacttgg ctggtgggcg tccccacctt gccgcagctg caccctgtgg 160860
ccagcagctt gcaggacttg ggctgttgcc tgtggccctg aaacagtcat tttctgagct 160920
gagtggggat caggtgagca gatccccaca ccttaactgc tgtgatcttt ctgtcctgga 160980
ggctggtggg cagcctggtt ggtgcttcat ggcactttat gaccggccct gtcaccaggc 161040
tcgtgagttg ctcccctcct ttcgggcatc ctgaacggct tccctgagag ccccgccctg 161100
ggccttcgcc tctgcaggcc cccgctgcct ggggcgcctc cttcccctgg aagctggcc 161160
tctttgggga ctcattaaga agggccttgc cgagtgctgc caaattgccc catctggtcc 161220
ccatttgttc cacttaccac cctattttaa cttttatgtc tcctccttgg ctctaagatc 161280
tgagcaggca gggcctgtga ggcaccccag gtcccctgat gctgccagca cccaggggac 161340
tgttagtaca tatgcaggga gggaaggaat gaaggaagag atgacttggg gagtcctcca 161400
gttcccattt gtaactggca cattgcgaca aaacactcat ggcattttac ctttttcata 161460
tacctttttt ttaaatttat ttttatttga gatggagtct cgctccgtca cctaggctgg 161520

agtgcagtag cacaatcttg gctcactgca acctccacct ctcgggttca agcgagtctc 161580
ctgcctcagc ctcctaagta gctgggatta taggcgccca ccaccacgcc cggcgaattt 161640
ttgtattttt agtagagatg gggtttcacc aagttggcca ggctggtctc gaactcccga 161700
cttcaggtga tccgcctgcc ttcgcctccc aaagtgttgg gattacaggc gtgagccact 161760
gcacctggct ttcgtatatc tataaaagag aatttgccca gaaaatcttt cagatgcacc 161820
tttagggttt taaggcacag atacatgaag cttgtcagtg agcagctgct gcttattcct 161880
ttttctcttc caattcagag cggcctgcgt cccaagcgga cagctgctcc cttcctcttg 161940
gctccctgct gcccacgctc ccacagaagc agggagaaga ggcaggaagg tgacgcgtga 162000
tcatccgctg ggcgcccgca cagtgccgtc tgcacaggtg ccatctcctt tacttgtcac 162060
tctcctcgct gagccctttc cccttttgc agatgagaga gcattgcctc ccagaggtta 162120
agaaatgggc ccagagtcac tcagctgatc agcgtgcagc cagatttgaa ccacatcctg 162180
ctgatctcca tgcctgcagg cctggcctga tgagatggac agctgggtgg tggagacctt 162240
gagaccctgg atggctccca aatagggcct gtagctcctg gacagggaga gtctggcaca 162300
aagggtttgt ggagtgagct gggggagaag gtggcttagg gtccctgtct ctgaacaaac 162360
agatggaggg gctattccag cgtttctca gcctctggtg tgctgggctc ctggttctgg 162420
gaggagagaa tggagctggc ctccattctg ggctgggctg gtttgggccc aattcagaca 162480
agcttcttag tttgccatga gtgacacttt tgcctgccct ggtgtctctg ccaaaaatat 162540
gaagggtttc agaaccccca ccccggcctg cttccctccc agacttggag tgggtctggg 162600
gctgacaaca gtctcatgcc agatagcctg agcagccacc agtgccatca ggtctgacac 162660
tgcggcccct gccccggagg gaaaaacaag gccctctctg gggatttc tgccagcttg 162720
tgaaaacagg cctggagtca gccagcgccc ccatgcttag acacttcgtg tggttttcaa 162780
agtgctttta tgttgctagc tcatatgatc ttccccaaaa ccccataagg gagggcagga 162840
agtttgagca acagcctgtc aagcacccat acagcaggtg gtttcattgc cctccattta 162900
cagacaagta aactgagctt cagggtggct accgcttgtc tcccaagcca cccagccatt 162960
tagccacgga agaggtcatg actttttaca ccccagtgaa gctgaagatt tttctttccg 163020
tttttaaaaa tgtttactcc ccagggcagt gaacacctgg tgcctctgtc tagcgcaaga 163080
ggtgaacgtt tgccacttcc gtttctttcc tcctaccaga cctgaccctc cttttgtgac 163140
gggaacatcc tgcaggcagt ctgtcctgct ttcatagact ttgagaaacg gggtccaaat 163200
tgccggctgt gggtgggaca ggcgacactg cagtgaagag gtggagtatt tttgtagcgg 163260
ggaagaaata catttcctct accctcttag gttcagtgtt tgggggtctg tgaattaaac 163320
tcactcaaga taaattaatg agagaaaaga cagatttaat cacatacgta tgggatttca 163380
ctgaaaaacg tgactcaaaa gagcaggtag aaggtggggc ccgtgactgg tccgaaggta 163440
gtgagttatc tcagttgatt gttcacagtc agttacagat tagactcctt gttccactgt 163500
ctcccctctt cccacttgtt ttgttttttt tgagtcagag tcttgcactg ttgcctgggc 163560
tggagtgcaa tggcacgatc tcggctcact ctaacctccg cctcccgggt tcaagcgatt 163620
cccctgcctc agcctcccga gtagctagga ttacaggcac ccaccaccac gcctggctaa 163680
tttttgtat ttttagtaga gacagggttt cactatgttg gccaggctgg tctcaaactc 163740
ctgacctcgt gatctgccta cctcggcctc ccaaagtggt gggattacag gcatgagcca 163800
ctgcacccag ccaactagtc ttaaaaaaaa aaaaaaaaaa aaaagaactt agggcccgta 163860
tactatctta ataggaaaaa aggaaggggt gaaagcacat attatgggcc gggtgcgatg 163920
gctcacgcct gtaatcccgg cacttcggga ggccgaggca ggtggatcac ctgaggtcgg 163980
gaattcaaga ccagcctggc caacatggtg aaaccccgtc tctactaaaa atacaaaaat 164040
tagccgggta tggtggtgca cacctgtaat cccagctact tgggaagctg aggcaggaga 164100
attgcttgaa cccacgaggc ggaggttgca gtgagctgag atcataccat tgcattccag 164160
tctgggcaac agagtgagac tgtgtcttaa aaaaacaaat aaaaaaaaaa aagcacatat 164220
tatggcaaaa caaatgagtt tttggaaaga taaataggcc cttaggagct gagatgggag 164280
atgtgaagtc ttgtgacaat gtcttcttag gtgtggtgtg gaaactactc acatcttcca 164340
gggaagagtc agttgctccc agggagcaga tttgggcctg tataccatct taatagggga 164400
aatggaaggg gcaaaagcac atattatggg ctgggtgcaa tgtgcccagt gcaggaggca 164460
gacgtggttt atggttgctg ttgaaaccag tgcagggccc gattgctttg tattgagtgt 164520
tgaggatcct ggttttgtat cagccatgag tttgcagctc tgggacacac ttgatgttgg 164580
ctcaagggga aatgaggttg gaggggcagg gtggttggat tggatgtggg cctggggcca 164640
ttgctgatct acaaagtggt catcacacgg gaataataga gctggctaca gaccacaacc 164700
agcaatatta tttaaaagta aggtcacact tttaataata agtcacattt taagttatta 164760
tcaatttata aaacacactg ttggtataat gatagctttt caagaaggat ataaaaaggc 164820
atgttgggct gggtgcagtg actcacgcct ataatcccag cactttgaga ggctgaggtc 164880
ggcggatcac ttcaagccag gagtttgaga ccagcctggc caacatggtg aaaccctgtc 164940
tctgctaaaa atacaaaaaa aaattagctt ggcgtggcgg cgcatgcttg taatcccagc 165000

tacgggggag gctgtggcag aagaatcact taaaacctgg gaggtggagg ttgcagtgag 165060
ctgagatcgc gccactacac tccagcctgg gcaacagagc cagactccgt ctcaaaaaaa 165120
aagaaaaaaa agaaaaaaag taaaaaggca tgttgaaggt acctgcttgt ggaaagttgc 165180
aacttgacgg gaggtagggc gagcacttct tagaaaggtg gaaatgggcc ctcctgctac 165240
agagacccac aaacatagac tgaggcctgc agcctaggag actcaaaaga ggcaaaggtg 165300
aataagagtc aaggtctcca agtcagggtc tgactctggg ggtgggggtc acatatgatg 165360
gaggatgtca ggagggcttc ctggaggtgg tgacatttac caggggggat tttaggtggg 165420
gtttgcgaga cagtaggacc tggctgaaga gcgtaggctc cttagacagg agtgggaatt 165480
caggcttgaa aatgagttgg ccagattgca ggggtcctcg aatgcaaggg aagaatttca 165540
actttaacag aggagcctgg gaagctgttt gggaaaagga gtgataaagc gggaggggtc 165600
ctccagcaga gccctgcatg ggcaggaggg agcgagatgg gaggtgggtg ttggtaaggg 165660
atgatcccag tggtccaggg gagcagtcat agggtggaaa ctaaccttgt ggggtctggg 165720
caggaaggga ctgatttgag acaccgggaa gaaacagcca gtaggacttt ggtgagaggg 165780
ctgggggtcg gggagggggg tgtggagagt gggagcttga gtctctgaaa acagacagtg 165840
ttccaagggg gagacctgag gcaagatgtg gagaggcagc tgagctggga agctggaacg 165900
gaggtgtggt cactccagga gactaagtca gggggcgagg ggacccttgg gagctatcta 165960
gaccccagca ctagggaata cggaggggag aaggagggga gaagaaccag ctggtgcgtg 166020
tcaggcactg ccctcgaggt cagatggggt ggggctggat actggccatg aggatttttt 166080
caagagctgg gttaatagtg agacgggcgg acaccagatg gctcgggcta agaagtaagt 166140
gggcggtggg aagctagaag ccgtggtgta gacctacttt gagaggttgg gctgttcaga 166200
cagggagaaa gttcacagtt tgaggaggta gataggtcaa aggaagaggg tttgcttgtt 166260
tttgagattg gggcatcctg aacgttctcc caggccaagt gagaagaaac cagaagagga 166320
acatgtcacc caaatgtgct gaaacagccc agcagctgcg ctgcctcaca gaaaatgtgc 166380
agaggcccct ctgtcccacc atgcctgcct ggggacggcc ttcactcggg ctactcaggg 166440
tttccccagc ctgccaaccc aagggtggca ggagctgtgg gtgctcccag cccctctca 166500
aggttggtct gtgggtagaa atgtgggctg cctcggggcg tcacagctac aaatgcatac 166560
cagccctcag aaccacattc ctgctcccca gccctttcct ccgcaccccc atgcagaagc 166620
gcggccgcca gctcacaaag gatagggagg gatattgctc ttggcatttg atgggaagca 166680
tctgctgcat cccattgggg tgttgcccag gatggattgg aaaagagttg gcaggaaggc 166740
tgagctctgt gctcacaacc tggcttggtg gtggccgagg agcttggcag gagcagagtg 166800
caggacctgg gaactggggg ttggtgcatg tgtgcacgca cgtgtgtgtg tgtgtgcgtg 166860
cgtgctgggt gggtagggag gaagctgtga aaccacatcc cctcctctct gctgctgtgt 166920
tgctgtgtgt ttcagcagca cgtgggtgtc accacacttc ctagcaggtg tcaacctcca 166980
agactgttct gggctcttct cccagttggc tgagttggag gtgggagtcc caactgtccc 167040
ctgtggcttc cagagtggga ccttgctgtg ggataggctg gccaatggtg ctccctcccc 167100
tgtgaccctt ctgttgggtg ggtcacgagg aaggactgtg ggtgttgccc acagacaggt 167160
ggacatgtgg caaggacacc ttgggacctt ctttctgacg ccccttgaag gggcacttt 167220
ctcagctttg agatgagtct ctgtggatgt gggaagttca ctatctcaag agcagcagcc 167280
ttggaaaatc caacacagaa ccccgagtag gggcgggaag gggtcctgtc ccgctcactg 167340
gctgcctggc agagttctgc acaaggaagc gcctgtgttg ctgtgggcgg aggaatggac 167400
tgagggctac attcgcttcc tgttgccgct gtaactgctt atcacaaact cagtggctta 167460
aagcaacaga ggctccttcc tttacagtgc taagggtcag aagccgatca gtctcaccgg 167520
actaaagtca aggtgttggc agaatccatt cctgcctctt ccagctttgg gtgggaggct 167580
ctgctggagt tccttggctt gcggctgcat ccctccagcc tctgcctcca tcctcctaca 167640
gcctcctcct tctctgcagt cagatctccc tctgccttcc tctttttttt ttttgagacg 167700
gagtcaccca ggctggagtg cagtggcaca atcttggctc actgcagcct ccgcctcctg 167760
ggttcaagcg attctcctgc ctcagcttcc cgagtagctg ggattacagg catgtgctac 167820
tacacctggc taatttttgt atttttagta gagacagggt tttgccatgt tggccaggct 167880
ggtcttgaac tcctgacctc aggtgatctg cctgcctcag cctcccaaag tgctgggatt 167940
gcagccatga gccatcacac ctggcctgcc tccctcttaa aggacgcttg tgatttgggg 168000
cccacctggg taatctcttc atctcaacat cttcagttac atctacagag tccctgttgc 168060
cacatgaggt aacacagttt gggaagggag agttattcag cctaccctag ggcctgtgg 168120
tgtatctcag ggcccttctg attttaagat ataaagcaag aaaacaaact ggctcaaggg 168180
gaaaaaagga cacgttgaat tctgttgctt taaatgtata tttttttatt gtgctaaaat 168240
gcacagaaca taaaatttgc cattagtaac actgagtaca ttcacagtgt cgtgcaacca 168300
tcagcactgt ctagcgccag aactttttca tcaccccaaa gggaaacccc gtatccatga 168360
aggactcact ccccattcgc cctctccagc ccttggcagc caccagaatg ctttctgtct 168420
ccataaattc atttttaata agtgcaattc tgtgtgactt taaaataaat aaacatgagc 168480

acgatgagtt gcttattgga aggatatcca tgcggggagg ccggcgtgtg gagtgcgtag 168540
gcctccggac gggcaggagt tgaaggggcg tggatgtgcc gccctctcct ccccttgctc 168600
tttccttggg gtcactgcct gagtatccct ctttgcaaat ggccccaaat aatgtctcag 168660
cccccacgtc tgcatcgcct cctagcttca ggaccctcca ccaaaaaaca ttccaagctt 168720
cagactcact cctgggaaaa ttccaatggc ctcactctcc cttttgagcc agccagatcc 168780
catggcctgt ggcgggctgc ctttgagtcc tgagcacctg tgagctaggg aagcaggaca 168840
ggcacaccca gggaagggga agagtcgtcg tcagtcacag taattgatat ctttggaatc 168900
gtctaagaga tacttagcgt gtgcctaaaa cattcatttc tttttttgtt tgtttttttga 168960
gacgaagtct cgctctgtcg cccaggctgg agtgcagtgg cgtgatctca gctcaccgca 169020
acctcctcct cccgggttca agcgattctc ctgcctcaac ctcctgagtg gctgggacta 169080
caggcacaca ctaccacgcc tggcgaattt ttgtattttt agtagtgact gggtttcacc 169140
atgttggtca ggctggtctt gaactcctga cctcaggcaa tgtgctcgcc ttggcctccc 169200
aaagtgctgg gatgacaggt gtgagccaac acacctggcc acattcattt cttaggagaa 169260
gctctgcatt ttctgaaagt gaaatgtccc caggcgggtg acactgatgg tgggatctgt 169320
ggcctcacca gtgtcttatg agagatgccg ctgtgtgttt caccaagctg tatcctattg 169380
tcctctaaat gtggcttgta gacatggtgc cgagaatgat ctgggaggtt caaatcataa 169440
tatggtccaa atcaacagct ctctgatgac ctggtattat cctaggatca aattgagact 169500
atccgaggat gggcgtggtg gctcacgcgt gtaatctcag cacttcagga ggctgaggcg 169560
ggcggatcaa ttgaggtctg tagtttgaga ccagcctggc caacatggtg aaaccccatc 169620
tctactaaaa atacaaaaag tagccgccgg acatggtggt gtacgcctgt agtcccagct 169680
acttgggagg ctgaggcggg agaattgctt gaactcagga gacggaggtt gcagtgagcc 169740
aagatcacgc cattgcgctc cagcctgggt gacagagcaa gactctgttc cccggcaacc 169800
aaaaaaaaaa aaaaaaattg agactctccg agctgcactg tccagcatga tagccactgg 169860
ctgcacgggg ccatttgaac acctgaaatg tggtgattcc aagtggagag gtgccgtgtg 169920
tgtgaagtgc actctgggtt tcagagtcct agcaaggggg aaagaaaaga atatcaaata 169980
tctgtcattt ttacattgat tacatgttga attgataata ttttgggctc tgttaagtga 170040
atgatattaa aattaatatc acctgttctt acatttttta atgatttcct ttttcttttc 170100
ttttcttttt ttttttaaat atggggtctc acactgttgc ccaggctgga gtgcagtggc 170160
acgatctcgg ctcactgcaa cctttgcctc ccaggtttga gcgattctcc tgcctcagcc 170220
tcccaagtag ctgggattac aggtggctgc taccatgcct ggctaatttt tgtattttta 170280
gtagagacag ggttttgcca tgttggccag gctggttttg aactcctgac ctcaggtgat 170340
ctgcccacct cagcctccca aagtgctggt atgacaggcg tgagccactg tgcctggctt 170400
gttcttacat tttttattgt aggtgctgga aaatgtaagg ttgcgtctgt ggcccatgtg 170460
ccgtttctgc tggagggttg gaccgagggc cagccgagaa ggctctgggg ctgctctacc 170520
tgaatgctga gtgtgtgctg agcggtagag aactttgaga agtgctgaag gacagaggct 170580
ttgtgtgggg gatgcagccc caggccggag atgccttctc tgtgctctgg agaccctcat 170640
ttggtgacag caagaggaac cactgcaaga ggggcggcca cctcagcaaa gcacccagga 170700
tgctggcctc ccagctcagg aatccagagg cagaggaggg gaggaggctg ggcttggagg 170760
agagcagggt cttgggccct gggaaaagcc tggggggcca gaaagctggg ggacagattg 170820
aggccagagg caaagagaag aaggttcgga gaggagggcc aaggaagaag ggttctgccc 170880
acggggaggg agaggcccac ggggcagggg cgtggtggga gctgcatcct cgaggctttc 170940
tgttgaccct gacacctggc cagaaaggag ttccttctgg catcctttgt cagcggtttc 171000
tggagctgcc ctttaggggc cagtgatgag ggtgccctgg gctgggctgc agacagtgga 171060
aaagacaggc tcttctcact cccagtgctg gggaaattag cttcttggag gaagggggag 171120
ctgtattttc tgagtggccg cacttcctgg cctagacgtg tgtgtcttgc tgtaattttg 171180
gcgactggtg ttggtggtgc ccattttatg gatggggaag gtcaaattcc acagatattg 171240
agtgacaggc ccagggcctt ggatttcagt gtttgactca gaacccaggt ctctgatctg 171300
ccaccagcct ccctcccatt cagatgggaa cagggactcc gggcatgcag tcttctccca 171360
gacactcccc cggctctggc ctggcgcccg gagaccagta gcagcagctc cccacggccg 171420
ttcactgtga gggtggggag ggccgtggga ccaggaggcc ttagggggaa agttggccat 171480
gaggagtggg aagcgacggg tcagatgcca gtgactgctg gctggggagc cgctggacgt 171540
ggctggcctg tttggccagt gtggcgggtg ccccggggct tcagtcaccc ccagccacgt 171600
gtgggggtgc gggatgggaa aggtaagagt gtgtggagcc cagtcagcac tcaacatgag 171660
gtcccgtggg ggctgcctga gggcgtagct tccagaattc agcccctggc cctcagaagg 171720
tgtcaccaaa ggcttctttc catttgagtg aatccctccc agcccccagc aagccctcgg 171780
aacgagccca ctcccaggcg ctctctccta gtgtgggagt ggccacgccc ctgctgggag 171840
tgactcactt gggttcagag gtcgtggcac tgagatgggt ctggcagatc ccagcgtcca 171900
ggcccagccc ctatagtgtc agctccctcc tctggggacc cccttgcttg tgcccctctg 171960

ggtcccagca catcccaggc ctgcagggag ggggagagga agagactgac tcactggcca 172020
ggtcccccag gggctggaga ggctggagag gcaggagctg gatcagatct gaatccagag 172080
gctctcggag gaagagctca gggtgagctg cgcccccatc ccctgcccct cctctcctgc 172140
tccttctccc ttccatggtc ccagccagca agcacctggg gtagagggga caaacccagg 172200
tggctgtgtt ccagccctgg ctgcaggtct gaatggcttt ctggggtggc tggccatgct 172260
ccctgagagc ccagctgtgg cgatgtctga gcaggtaggt gggggagcac ctaggaagca 172320
ggggtgtcag gcagagcaca aggagagagg gtgtccaggt cagtttcagg acctggctga 172380
gaggaggggg ctcctcacgg gcaccgcctc tggcaagcac agggacaagg gcaaggacgg 172440
catggccaga ggtccctggg agcctcttcc cctctcttct tcctagcagc tccccctcac 172500
tcttcccagg gaccctgtca ctttccttta gcgtgtggca gctccttggc gtccctcccg 172560
tgccttcagg ttgcttctgc gccgggcctg ccgctgggcg cccctatctc tgcctgcccc 172620
ctcctcctgc tcccctcgcc ctgccccctt ggagcaattc cccaccgagc ctcccttccc 172680
aggcagtcga ggtccctccc tacctctgcc ccgcgctctg ggaggctcct tgttccgcga 172740
ccacaaagcc cctttgatcc tctgctcggc tctgagccat gtgacccggt gggcgggccg 172800
cggctctcgg cgcgtccagc gcagcccgac gttccgctgc tggggtgagt cctgctcctt 172860
tgttcttccc agccttgcac cactggctcg gggctctca ggtggcgcgg ccgcgaggcg 172920
gaccctgatg gccatggtgg cggtgccggg agccacgctg tccctgggcc ccggcccgag 172980
gccggcagga ccgagcgggg tccccaggag aggggtggcg gggagctcga tctccacgcg 173040
gggaccagat tttcggcctc aaaatagaag aatagggctt tgtgtggtca cagctatctc 173100
tttgtaaata tttggccaac taagctgagt ggctaagttc tcctgctgcc cggagcttct 173160
tggaacatgt ttccttttcg caaggggttt ccctggcttc caggagggcc aggaagaaat 173220
tcgaattggc caccgctttc tctaaaatca ctccgctcaa gttatcaccc ctctgggctc 173280
ccgaagaccg gctggctgga ggctggagat agtctcaatg ctcgaaatgc cgtaaccgaa 173340
gctccccgcg gcgccggcac tgggatccag ggagctgctg ctacagcgca gctctggatt 173400
cctggatgtg ttggatatgt gcagggcgtt cctgggagga gcggggaggg agggtgctgc 173460
tggcggggct ggtctgcgtg tgctttgctt ctctacaatg gcatgctgcg tgtcggccat 173520
gcagaggcat gtcagtgagc aggggctgag ggatctccct aacggacctg ctttcagagg 173580
gtcttttcat gctgggagaa ccccagagac taaatcatgc agccaacggg gtggtccccg 173640
gcctcaaagc agggaggggc gaggagcttt gtaggcaatg ccatctgctc ctgaaacgcc 173700
gtcccagtga ctctggggac tgactcagcc tccagcctgc tgcacttcat ccctggcccc 173760
tctctctttg ctttttcatg tgaaatctgc tgtgttttgg tcagaggttt ggggaacagc 173820
tctctgctca tctaagataa gtttgtaatt cctttcctgg agaaaaatat gccacccgga 173880
agcaggttga gcagtggttt tctggcaggt ctgttcgggg ctgtggagac agcacctgct 173940
ggatcaggat ggagttggaa tttggtttgg atcccacatg agaaaacccg ttggaagagg 174000
aggaagcaga aaaaggcccc attccctaat aatgctgtgg gttttcctcc tccattcatt 174060
gccaccttgc cttgaaactt aatggggcca cgtgtgtttc catgaatgtc attctctttg 174120
gccaactccc ccggcagccc caggagctcc ttttcaggaa aaggaagaag gtgtttgctt 174180
agcatctagt atacgagtcc ttgttggaaa ggtgggattc ttgtttctcc ttgggtctct 174240
tttaggctga gatgtggtca aggaaggcct gtggtgtggc tcgccttttt taatgctcgt 174300
ctctggagac ctgagttgct gctgactccc agcttgcccc ttctgtcccc tggccatttc 174360
tgtgtcgggg tggctgagac actattgcat tggccacttt gtgttcccac tagcccccgc 174420
ccctgtaggc tgtcattgtg atggtgatgg ggccaattca gctgcggtgg ggaggaccca 174480
ctgtgtgcgc ctggggggtg gcgtggggca ctctgggatg agaagatgcg acttctgccc 174540
tgagagccct gctttaatgg aaggcggggg catagttgca ggcagaagcc atcaagcact 174600
gtggtcagac cagcattcca tgtcaaaaat ggccttgttg gccgggcgca gtggctcatg 174660
cctgtaatcc caacacttca ggaggccaag gcaggcagat cacctgaggt caagagttcg 174720
agaccagcct ggccaacatg gtgaaaccct gtctctacta aaaatactgg ctaatccggg 174780
catggtggcg cgtgcctgta atggcagcta ctcgggaggc tgagacagga gaatcacttg 174840
aacctgggag gtggaggttg cagtgagctg agattgtgct actgcactcc agtctgggca 174900
acaagagtga aactctgtct caaaaaaaaa aaaaaaaagc cttatctggg cctgggatct 174960
gtcctccgaa cactgggtcc actgtgctcc acaaggactt tcaccccgga agccctagac 175020
ccacttgggc aaccagagtc tctgggttga ccactcccca gccctggcca gcctcctggg 175080
ccaccgtatt ctcccaactt cctgttgtgc cacggctggt ccaagctgct cttatcagac 175140
aatgcgaggc acttccacag gaggggacaa ccccgtcctt aggagcccct tctccctgcc 175200
cccaggcctg gtgcagtgtg tggcttccct gccggtgtca aaggtctcaa ggccccttta 175260
agaagcctgt caccaccacc agcagcttcc gttatctggt tcttctgtac atgtaagcct 175320
ttcccataca ccccggatgt ttagggcttc cgccctctct ccctggccgg gagtgtggga 175380
agagggcttt atttcaatgc ttgagagtag ggtcaatggc cagggtaagc tgtgggggct 175440

cctccccgcc ggcccctctc tgcagccctc accttaacac agagcatcga taccacctgg 175500
gctttgggaa gacctggagg ctgaccaccc tggaggctcc cagcgtccca cagccactct 175560
ttggggaaga ggcttcctga gggacttgtg gcccttggct gggactctag tcccagagtt 175620
ctgccttccg gcgccgagct ttgatttgtt tattgagtgc cagcccggtt cccggacagg 175680
tagggacctg ttcccggagc tcagcagagc cttgggaagc tttcccagga gggctcccga 175740
gggtggcggc catgcccagt ccgctgtacg tgtgagtttg tttgatgagg aattggctgg 175800
gaaggcgcgg gtctaattac aggcccatga gggtccctct cgcccccacc caggaagcag 175860
cggagatcct ggcacactca gcacctgggc ctgtgcctgc ccagctgctg gtggaatcat 175920
gggagccttg ctccggggcc tgtagagcag cagtgggaac cttccccacc tcagcccctc 175980
ccagagttgg ctgttcaaac tcgccgccgc tcagcctctt cctcctctct ttcatcaacc 176040
ctgggaatcg cacactgccc accccaccgg gccccggagc gggtgcagga agagagagct 176100
taacacccag aggtctgggc cttctgcagg cgcctgagct gctgtggcct gagctgggtt 176160
ccctgctgga accatcacag tagacctaat gatctgcctg caggagttgc ttcatcccag 176220
ccgactccgg ggctgcggga aggggctggg ccttggtttt gctgggggtg gggggtcagt 176280
gcaaagggcc tgaaaagtgg cagaggtgat ggcccatctg tcctccccct ggccggccgg 176340
cctaaacgcg attggctctc tttctttttt ttttttccct ccctttccc tataaataac 176400
actaaatgcg atttgtcaat gcattcattt gggttcagta gttggaccat gggcagaagc 176460
actccagatt tgtcactttc cctaagctaa gggtggggaa caccctctgt gagatcttgg 176520
ctcctgagcg cccaagactg ctcttggaac cccaactctg ggtcggtctc aggggactga 176580
cgttgatgga gttgctgaca ttgacctccg gcctctgcat ggccttcagg aagggggccc 176640
ttcgctctct gtcccgtcga gcctggctgg cagcactccc ttctcaggcc tgtgcccccg 176700
gcagggcctg ctgcccccgc cctgctctgt gggtgccagg ctccaagcac caggtgactc 176760
ggtcactgaa tggtcactga acagtcatcc ttgctggtgt ccccagtgga atgtgcatgg 176820
acgtcctccc tcccccagat ccttgtcttt tatctctcag cctttgttcc aggggccacc 176880
acaggaccag gaggtacgca gacgtgggtg gcacatccac tgggcagatt cagccctgaa 176940
cctgcacgcc ccctccaagg gggcccccag cgggcggggt tgatggtgcc gtctctctct 177000
ctgtcactag aacggacatg aggattgtgt cttgtcgatc cacgtcctga gttcccagac 177060
gtcataggtg cttgctcaac gagtgtttga atgaatggtc agcattgtgt gaccgcagat 177120
tttcaacttt tatccaaata gtttgtggtt tctgtgaggc tatcccgaga cagtctcaca 177180
agaatctcca ttctccctgc acgctccgca caaggtctcc ttggggggtt tttcagaaaa 177240
agggccaaag agcagcattt cctcggggat ttgaacacga tctcccccgg ggcaggggac 177300
tgccctggaa gattcccggc accgcttccc atgcgccacg tgactaggag ggtcttgggg 177360
caggatggtg tcttttgtgc cttgtgtgct ggggccggag gaaacactgc ccgtggccgg 177420
tgtcatctgc cagccctgaa gactaatgat gggctggaca gtggccccat tgagtgggag 177480
ggagaatcgc tgctgtctgt agctgggggg ccgggtgggt gggagccgag gccagggaga 177540
acagcgcaga cctggcacgg agaggcctgg aggaggagca gggggtctca gccacccagg 177600
gtgcagggac gccacacgag gagcaggtgt ggtgtgcgtc ctcctgcggg ttctccacct 177660
cttggcttcg ttacctgcac taggctttgt ggtgagaggg cacagagtgg aggaaggtgc 177720
cgtggctagg gcaggccaca cgctgtgcta atggattgct gttacctgtc gcctctgtgg 177780
ttttgctgtc aggatcctcc agcaccaggt ccccaccacc aggtatgggc cccacaggca 177840
ctactggctc cccgaagcca gggacagacc ccatagacaa tactgagtcc ctgcagccag 177900
ggatgggccc ccacggccag taccagatct cagggaccag caccgggtac ccacagccag 177960
ggacaggcct ccatggccag taccaggtct cagggaccag caccgggtac ccacagccag 178020
ggacgggccc ccatggccag taccagatct cagggaccag caccgggtac ccacagccag 178080
ggacgggtcc ccatggccag taccaggtct cagggaccag cgctgggtcc ccacagccaa 178140
ggacaagtcc cccacggccaa taccagatcc cagtgaccag cactagatgt ccacagccag 178200
agatgggccc ctatggccag taccaggtct cagggaccag cgctgggacc ctggatgact 178260
gaaggagggc acggttgact aaagctgcag gagccagcct ggccatcctg aggcactaag 178320
ccatcactgc ggtctcaggg gactcagact cctctacttg tcttcttgaa ttctggaatc 178380
agacacacct gggtccagcc tttgcccccg gggctgtgtg cctgggatgg ttgcctcacc 178440
tccctgaagc tcagtgagat ggggacggct gcacagggcc cggcacagat ggcacccgat 178500
ggcacagggt cacctgcttg catccataca gcctcggccc tcgctctggc tgcgtaccga 178560
gagtcccatg cccagacctc tgctgagtct ccaagctcct ggtcatgcct caggcctccc 178620
gtgagaagca tcactgcatg gaccatgcct cgggacctca ccttgggctg ggaagcttgc 178680
tccatctccc caagtctccc ctagaggcgc cccatgtccc atcactctca ctgcttgagg 178740
ggtcagatag gggaacccag tgtgggaggc ccagaggtgc cggagcagac gttaagcgag 178800
ttccgtgttt atgcgctggg ccttggcaga caccctgaat gagcctcttc cccaggaagg 178860
agattggggt cccagtccaa gttgtggcct caaaccctgt gaccaagagt ggagaaaagg 178920

caagacccct cctcccagtc ttccagaaca gccgatagtg gtggaaacat ggggttcaca 178980
ctgaatttcc ttgtaacttg ttgaagagaa tttagcccac attttcaggg gtggaggtct 179040
ggccgatttg gcagtgtgcc cgtccccctg gccacagttt actctggacc gggcaccctg 179100
gtgccctccc tcagtgacgc agtgaagtca ctatgacgtg tttagttggt gagaaagtgc 179160
ctcctgtaat gttttactct tcttagaggg tgaataagcc ttgtttatta taaataatta 179220
tttacagaat acatgaggtt taacctaact ctgggccacg gcagcgtccc ctgggtaaac 179280
ccacgcgcca ccctagctag agcctgtgcc cccacaggct tcctggactc accctgaagg 179340
ggggctggat tttctgcctg tcccgatgag agggctgcag gacagtcccc agcgagccct 179400
tctgagggct gtaacctctt cccctggtat tttcctgctc gccaggaatg tttcaagctt 179460
ccagcagaag cctacccacc atagtcgctt tccaccctgc tcaccatccc tcctgccctc 179520
actcattcag cagaccccgt gcacagccca gtgcaggact cagggtggga aggtgtcccg 179580
ggtgggctgg agcaaggttc ccagccaggg cggggtccca aggtgggtcc acagccagga 179640
ctgtgatttg ggaggcccct ccctgccttg ttccagcccc ggcaccgccg ctgtccttgt 179700
gctcagtgga gactcactgg ctccaggctc taaaacctca gcgaggtctg cagacaggga 179760
ggagccccac tcgagggtag agtgactgcc gggtccattt gcctgttacg aaccacgtgg 179820
cctcagacct gccattcctc ctctcctaga gctatggtct tttgtctgaa aaatggataa 179880
tgatggttac catgtcaggg accattttga ggacaaacgg ggatgaaact gaagaggctc 179940
agcacggaac ctggacacag aagtgctaat tgtgtgttac cagctgcggt ggctgccaag 180000
gcccaaccac atggcagcca cgggctgagt ggcgggcggg aagtggggag caggagcatg 180060
caagatgggc attcctgaag tttctgatcc agtgagataa gtacacaccc agctctaaga 180120
tggagtgtcc caggaaggtg gagactgcaa aggccttgtg gcgcaggagt gtgagctgca 180180
cctctgggct ctctagaatt tcaccagagc tcagggagga ggtcagctca gatatagggg 180240
caacgggaga gatgggagtg agaaaatggc ttggcgtctg gaagtggagc ctggaaaggt 180300
gggcgtgggg acagaggagg gccttggggg ccatgggccc atgcgtccag gaggtggagg 180360
cagtgaacgg ggccacggtc ggccccacac gtggcaccac cagccctgct gagcctgcac 180420
ctgctcctca ggccccctga gtgccactac cacctggcac cccttggtct gattcctgct 180480
tcctgcccgc ctgggaagag tgagaccact tgcctaaagt cccttagctg tccttaacca 180540
ttctggctgc ctcctggccc tgtccagggt tagccgttga cctgaactgc ctggtgccac 180600
ctcccccagac tcctggggcc ttgccctggc accaacagcc acctccatct tcatcattca 180660
ctcgcacatt cacccagcag tgctttttgg gcatccactc agcaatatca aggcgctgag 180720
atgcggctgt ggttaagacc cgggcccggt ctgtgaggac ctcgcagttg ccatttcccc 180780
agagcacagc cccagccagg cacactgtgg ggtccagaaa gagaaactaa tacattgtgt 180840
tcgtccattt tgagttgcta taaggaatag ccgaggctgg ggggttattt gactcacggt 180900
tctgcaggct gtacaagtgg tgccagcatc tgcttggctt ctggggaggc cccaggaagc 180960
ttttcctcat ggtagaaggt gaagggggag ctggcacgtc acatggcgag agagagaact 181020
cccagactct tttttttttt gaaatggagt ctcgctgtgt cagccaggct ggagtgcagt 181080
ggcacaatcc tcggctcact gcaacctcag cctcctgagt agctgggatt acaggcgcat 181140
gccaccgcgc ccggctaatt tttttgtatt tttactagag atggggtttc accatgttgg 181200
tcgggctggt cccaaactcc taaccttgtg atccacccac cttggcctcc caaagtgctg 181260
ggattacagg tgtgagccac ctcacccagc cctcccagac tctatagcaa tcagatctcc 181320
cggactttgt gaccgtgggg agggcaccaa accatccatg agggatccac ccccatgacc 181380
caagccccac cccccccagg ccttacctcc aacatcaggg atcacatttc agcatgagat 181440
ttggaggggga caacgtccaa accatatcac acgtgacctc tctgtgcgtg cgtcagtttc 181500
tacatctggt taagcagtag aagctccttc tctgttcctt gttgagagag cggtggggga 181560
gaaatgactc agggatggga ggctggtccc ccgaggggcc ctggtgtttg tggagtggga 181620
gtgcgggaag gcccctacgt tgtgtgtgcc gtgctgggct ggctcctcac agctcaccgt 181680
ggtgctcccc gaggactgct ggggaggaaa gagctaatta acttggggac ccagctgctg 181740
aagttatcat taaacttgag cagtccaggg gcccctctcc ttctgggtcc tatgtcaccc 181800
agcacagtgg ggcttaattg aattttttct catcccccaa ggctcctggc tggcgtggct 181860
ggggtttggg atacggggag cgagagggct tgggcctctt ccagatttgg agtgggttca 181920
ccagaaatgg cccaggaaac cgcgggggcc acccccagca gctgggggct cctgtggtca 181980
tttcctcttg gtcctgttga gcagccctgg cccctggagc cctcagcaag ggatgtgggg 182040
gcctgccagg ccagggtcct cactgctgcc gctatgcaca gctgcatctt gcatccgggg 182100
agtggggtgc cgagtggggt gtttgggagg caggttttcc cttgtccctt ctgcccagag 182160
accttgggga tgagttgagt gagacccttc cggcttctga ctggctgtgt ttctgttgcc 182220
atcccccact ccttttaagt ggaggtttat gacttccttc tttatttaaa agggtcattt 182280
agtgacttag cagatcccag tctgtgttac ccagggccct tttaaaaatc tgtggggagg 182340
ctgggcttgg tggctcacac ctctaatccc agcactttgg gaggctgagg cgggtggatc 182400

acctgaagtc aggagttcga gaccagcctg gccaacatgg agaaacccta cctctagtaa 182460
aaatacaaaa aattggctgg gtgtggtggc acgtgcctgt aatcccagtt actcgggagg 182520
ctgaggcagg agaatcactc aaatccagga ggcggaggtt gcagtgagcc gagatcacac 182580
cactgcactc cagcctggac gacagagtga gactctgtct caaaaaaata aaaataaata 182640
aataaaaata aaaatccatg gggggacctc tgtgtccatt ggctggtgtg aggagagggt 182700
gaggagctgc agagaaggta ccaggctgag gaccgccccc acccttgggc tacagaattg 182760
aaggagggga cacatgaaag atgttcctag actcgggctg gaggctgggg acatccgagg 182820
gcaaggacga cgctgtccct gctcttgttt gcagaggagt gcacagggct caccagcttc 182880
ttgttgctgt ttaagtgata gctttatgga gatagaattc acatactgtg acgttgacct 182940
ttttaaaatc tacagttcaa tggttttgct gtattcacaa agtgattgta ttagggtcat 183000
ccagagaaac agaaccaata gaaggtgtgt acgtgtgttt agataaagag atttattatt 183060
atgaggagtt ggctcacctc attacagagg ttggcaagtg ccaagattgg cagagtaaat 183120
cagcaggctg gagacacggg agagcccatg ttgcggctct ggtgtccacc tgaaggccca 183180
agacccagga gagccaatgg cgtcgttcca gtcccgagac ccaggaaaag cgcatgtgtc 183240
agtttgagtc tgaagcagga aaaatctgat gttccaggtt gaaggcggtt aagcgggaag 183300
aattctctct cactcaggcc ttgtgtcctc ctattcagac cttcaggcct ttgtcacaaa 183360
attacaaaga cctgatcaga acaaaatctg agaaaaaacc ctttccttct atcggttttg 183420
gattcattgg ctgggaccct ggaaattaga ctgacagaga ttaacaggaa acaagcataa 183480
caattcaagt aagttttact tgacaccgag ccttcataag gaaatgaaga cccagagaaa 183540
ccgttagcct gagcggtttt tgttttgttt tgagacaaag tctcactctg tcgcacgtcc 183600
aggctggaga gcagtggccc gatcacggct cactgcagcc ttgacttcct gggctcaagc 183660
tggggcccag ctgccggccc cgggagctgc tctgaaccca ctcttaagtc ccagctcaga 183720
ctcccaagta gctgggacta caggcatgtg ccaccccgcc tggctaattt tttatttttt 183780
gtagagacag ggtcccacta tgttgcccgg gctggtctca aactcctggg ctcgagcaat 183840
cctcctgcct tggcctccca aagtgctggg gttacaggtg tgagccactg tgcccggcct 183900
aggcctgaac agtttttatt tgttttgctt gtttttttaaa tttgtttgtt gcttgttttg 183960
agcagttttt ttgtttattt cttttgcttg tcttttgccc aggctggagt gcagtggcat 184020
gatctctgct cactgcaacc tctgtctcct gggttcaagc cattctcatg cctcagcctc 184080
ccaagtagct gcgactacag gcgtgtgcca ccacacccgg ctgatttttg tatttttagt 184140
agggatgggg ttttaccatg ttggccaggc tgttcttcaa ctcctgacct caagtgatct 184200
ggccaccttg gccttccaaa gtgctgggat tgtaggtgtg agctgctgtg cctagcctag 184260
gcctgagcgt ttttatgctg ggtttgatga agagtggaaa gtcgtgggaa actgtgacag 184320
agcaacaaaa gatgagctga acagtgagcc cgtgggggtc tcggcaagac ctgtgtactg 184380
gggcccctct cagatcctct gtctctggag atgaggctgt tcctttccgc tgggtgtagg 184440
gagggcacct cttacgtgag gggcttatga tctacttcag agaaggggca ggtcagtgag 184500
ttcttcttgg acctgccatt tcttaaattc cttcagctga aaatatttac tatgccaagg 184560
cactgtatct tggagtgtgc gttccaagcc ctgttgcctt caactgatcg ggtgaggccc 184620
acccccatca gagcacacag tctgctttac ccaggctcct gattgcaccc ttttcctgtt 184680
tgccccgaga atactcacca gcagtgcttg tggctgcagc gttcacccca agataaattt 184740
gccgggaagt atcttgcttt tattattatt ttcacatcac tctagtatat cgactttgga 184800
aacaaaagac atcattctgt ttatagcatt ccatttttag tagtggtatt tcctttaaaa 184860
aaaaatggta attctcagct gggtgtggtg gctcatgcct gtaatcctag cacttgggaa 184920
agctgaggca ggctgatcac ctgaggccag gagttggaga ccagcctggc caacatggtg 184980
aaatcccatc tctactaaaa atacaaaaat tagccaggcg tggtggtagg cacctgtaat 185040
cccagctact tgggaggccg aggccggaga attgcttgaa cccgggaggc agaggttgca 185100
gtgagccgag atcgcgccat tgcattccag cctggacgac agagcaagat tccatctcaa 185160
aaaataaata aataaataaa atttaaaaaa aaatagtaat tctcgatcgc tgaaaatgtc 185220
aaatcctaga gaacacagca ttcctacaag tgatgttaac attgttcttg atcagttgtt 185280
ggctgaagat tcatttgatg aatctgattt ttccagaata gacgactcag atgactctgt 185340
tgttttgctt agaaataact gcaggaacaa tttaaaaaat tttttttctt tttaattttt 185400
ttgtttttttg agacggactc tctctgttgc ccaggctgga gtgcagtggc acgatctcgg 185460
cttactgcaa cctctgcctc ctgggttcaa gtgattctcc agtcttagcc tcctgagttt 185520
agctgggatt acaaggtgcc tgccaccaca cgtggctaat tttttgtatt tttagtagag 185580
acggtgtttc accaggttga ccaggctagt ctcgaacccc tgacctcaaa taattggccc 185640
atgtcggctt cccaaagcgc tgggattaca ggcgtgagcc accacgcctg gccagttttt 185700
atatttaatt ttcacacaga gcatccagtc agatttgctt cagcctcaaa gagcgtgttt 185760
atgtaaaatt aagtgagtgc tggcagtgag ctgaactttc taaaaggtaa aagggttaaa 185820
tattaaactc atccaaaaac accttcacag acacactcag aataatgttt gaccggttac 185880

ctaggtgccc catagcccag tccagttgac acagaaattt tttttttttt ttttgagacg 185940
gagtctcgct ctgtcgccca ggctggagtg cagtggcgcg atcttggctc actgcaagct 186000
tcgcctccca ggttcacgcc attctcctgc ctcagcctcc cgagtagctg ggactacagg 186060
tgcccaccac cacacccggc taattttttt ttttgtattt ttagtagaga tggggtttca 186120
ccgtggtctc aatctcctga cctcgtgatc cacccgcctc agcctcccaa agtgctggga 186180
ttacaggcat gagccactgc gcccggctga cacaggaaat taaccatcac agttacacag 186240
ccatcgccat taatcccagc acatttcatc atccccaaaa gagaccctgc ccccattagc 186300
caatccccat tcccaactcc gggctctcag cccctagcca ccgctcatct gccttcgtct 186360
ctatgaattc acctgttcta gatagtttgt ataagtggaa tcgtacagta tgtggccttg 186420
gtgtctggct cctttcactt ggtagaatat tttcaaggct catccacatt gtagcaggtg 186480
ctggtgcttc attctttttc atggccgaat aatattccat cgtggataga ccacactgtg 186540
tatctcttca tctgctgatg gacatttggg ttgtttctgc tttttggcat aattctgttg 186600
tgaagaattc tgcagtgagt gctggtctac aggtttttgt gtgaacatgt tttcatttct 186660
cttgggtaat tgcaagatca tgtggtttag ccaaccagcc tttaagccat gtgtctgctg 186720
agggacccag gcctggtggg ggtgcattgc tgtgcttggc cagccaggtg ctgcgagtcc 186780
gtgtcgcagc catttctctg tgctgtgctc tgaggttgtc tcgccggcac cgggcactgt 186840
gagccccagg aggccaaagc cccctttgtt ttatcttgag tccaggcctc gctccatgaa 186900
ctaggatgaa tggacattga atcaacgcag ggatgggtgg ggctggggtg gggacgcaga 186960
gtgaccccgg gggcctgcag ctgggtcctg gctgctgact tccagtttcc tttgcctgtt 187020
ggagcccaga acaagtcaga aaggggaaat gcaaagttcg agtgtctttt ctgtggtaga 187080
ctggcaggcc tgggtgggtg agccacttgc agctagttgc agcgtgagag aggaagtggg 187140
aggggaggga gagggtgagt ttggtgtaac tcacccctgg aaggaggctg ggctgaggct 187200
gggggaagtg acttttattt ttctgagagg attcttccct ggggccaact tcaaaccaag 187260
ggagacaaga ccttcctggg ggaggctgga agtgctgggg cccccaggtg ggcaggtctc 187320
ccagggccaa cgtcttgggg aagctataag ttaccatgtt tctggactgt cacacacaca 187380
cacacacaca cacacacaca cacacacaca cacacacaca cacgagtaaa ctgtaacaca 187440
aatgtgtgcc catgaaagtg cccactttaa accaccacac agcctgtaaa gcgaaggccc 187500
tggcccgcat ctgggcaccc actgcccagc cagccggtag gcagtttgca ctgtagtctg 187560
ttaccatccc tcagagatgc agccgaggag cccagctggc ttggagggcc cagtgggcgc 187620
agcctcagga aagtggtcag gcccgggcca ggccccttcc ccctcccagc cttgggagaa 187680
attaccatat gaatgtagct ccttcccgga gttcgggctg gaagggcctg ggaggggaca 187740
ggctcccacc ccaggagctt ccaggttccg gctgctgcct gtgttagctg cgtgagcgtg 187800
agcgagccag gggtcactac tgggtggaag gaagaagcgg gcatttcttc caggaggtca 187860
ccgttggagt cccacagtag gtcaacccca ggccctgggt cctcgggagg aggagaaaga 187920
agagaagcag gaggtgtgga gggtaggcca ggaccagaag gcacacaggg tcggggcagt 187980
gtgggagacc ctgggggctc cctcatttaa ccagctaaca tcacagcgtt cataagaaca 188040
cgctattact gcagttttat tactttgctc tgatttcaga aggcacaact gatgacttta 188100
gggaacggag aatctgagga aggcctcgca ggagagggtc agatgcgatc gagtaaatgt 188160
gagccgtctg taaactgtag agtgcacgca cagcaccggt cagccctggg gtcagtgttc 188220
ccctggtgct cagctcggcc tctgcacctt ttagtgctcg gctgggctct gtgggggtca 188280
gggctgggtc tggtacatta gaggacttct accaagcccc tggcagtgtc tcagttacta 188340
aatccagttt gccagttact aaatccgctc tgccggttcc cgaatcactc ttccgctggg 188400
gccggccatg tcacgtcagg cttttcatgc aggacggcag atggatagac gctgggacca 188460
agtcatcctt gggccgcgag acgtctgacc ttctgctggt gtctcagaag atgctagctc 188520
tccgtggcat gtcttccacg cggtgcttgg ggaccagtgg cctctaaggg gcccccctgc 188580
ctcagcgtgg ggagtggcct ggccaaggtc tcagtgtctg gttggagcca ggcataggct 188640
caggtgtact ggctggaggc ctcctgccca tgggctctgc ctgtcctgtg tccctggtcc 188700
ttactgtgtc ttctgtgttc ctgggagaga ccccaggggc cctgagccca gcactggggt 188760
tggggagtgg gcggggaaga gggtggggat tagggtccct ggctgcagaa cagccaggcc 188820
ccaagtcccc actccactcc cctcccaccg ggcgcctcat cccacactga ccatttcctg 188880
aagaaaattt cctgaaccgg cctcccaccc cggctgtccc aggaagccca tccatccttc 188940
cgccttaatg ctttgtggct ccagaaggat ggtggaagcc ggaaatcatg agtctcattc 189000
acaggcggcc acacaccgaa gccgcggaga cgccgtggga aaggactctg gccagcgcgg 189060
ccggcacaga gggagcggtg ttaccggggt agacgggaac cgtggctgcc cccatgctgg 189120
tctgggagag acgaggggcc ggacgaaagg acagcagact ggccattcat tcatttattc 189180
attcactcat tcacagcgtc ttactgggtg cttcggacgt gtaaggtttt gtgggtagtg 189240
aatgcgacag accccagaca gcaatcggag ggccacgtgt gtaagaactg agggacctag 189300
gctgggcgtg gtgtctcaag cctgtaatct cagcactttg ggaggcagag gcgggcggat 189360

tacgaggtca ggagatcgag accatcctgg ctaacacggt gaaaccccgt ttctactaaa 189420
aatataaaaa attagccggg cgtggtggcg ggcgcctgta atcccagcta ctcaggaggc 189480
tgaggcagga gaatggcgtg aacccgggag gcagagcttg cagtgagccc agatagtgcc 189540
actgcactcc agcctgggcg acagagtgag actccatctc aaaaaaaaca aaacaaaaca 189600
aaacaaaaca aaaactgagg aacccagtgt agatgagcta tcccgggaaa acaagtggga 189660
gttctaggaa cagcatgggg aagggatggt cccggcaggg aggacctctg tgggtagcct 189720
tgttggaaga gctgaaaggc agccagagag gctgtgcgtg gatggaagct gaagagtagg 189780
ttaggggcgt gtgttttgtt aacaaagact tcaagcattt actgaccagg cacaacacta 189840
tgtgcttcat aaataaatat ctcatttgat ccccagcatt cttatgagac agatgttatt 189900
atcccatttc acatgtaaga acattgaggc acagagtggt taagttactt gctcagggtc 189960
acacagcacg tcggtggtag gcaggattct ggagccacag gaatctggta tatcccaggt 190020
gcaatggaaa ccagtcacgg cttcatagag ggaaatcatc agattagtaa attttacaca 190080
cgcccccctgg tgctttgagg aggagggatt cggacagggg cagctaatgg tggtggctgt 190140
gagatgtgtt gggttaacat tgatgagagt ttgggatgga tgagtggatt cggagtaatg 190200
gaaatggagg gcccaggccc ggcccctgga gatctagctg caatagctgt tggctggaga 190260
ggctgttcac ttagatgggg gatggggtgg gaggcaggtg agatgagagc tccagagttc 190320
agcctggggc acggtcattg ggttcaagat gccttcagat gttggggctt gacagtgaca 190380
gtggctgcag atacccagga cccagcagag ggcctggcac agcggggccc agggacacgt 190440
ctgtgagtca ggctggcact caagtgtgaa tctggacaaa gaaggtggca ttgttggcat 190500
tgaatgctca ggggctgggt gaagtcagct agggagaggg gctctcaagg caccaggact 190560
gactccgtgg gtgagggcgg aggaggagac ggcagaggct gagagaatag ccagagacag 190620
gaggaaggtc agcagagcca tgcacagaag cctggagaag atttgaggaa aggaagggct 190680
gggtggtcgt cattccctgc agctaggagg caagggtgac ccctgtgtgt ttcattcttc 190740
agaggattgg tggctccatc tggtcagaac cccaagtcca gagatgcatc tgagcttcat 190800
gccatggtgc aggcagcagg agccatggag caggcctgcg ggtcacggc ctgcctgctg 190860
cttcctggtg tctctaccct gagctcaggg gttgcttgga tgccctgtgc tgcatccccc 190920
caggcggtgt tccagagagg atgaaagtgg ctatctcaag gtgtgaccag gtagcggtcc 190980
atgcacactg ctttttcctg gggagagtct gggatggaag ggtatgtgtg tgtttctgag 191040
actccttgaa taggctccat gtacctatcc acccatctta ccacccaccc acccatccac 191100
tcactcaccc atccacgcac ccacccatcc acccatccac tcatccacct gtcaacccat 191160
ctatccactc attcacccat ccactcatcc acctatctac agtcattcac tcatccaccc 191220
atccactcat ttacccatct atccatccac tcatccacct gcccatccac tcacccaccc 191280
atccactcat ccacccatcc acccatctat ctatgcactc atccacccat ccactcatcc 191340
acctatccac tcattcactc attcactcat ccacccatcc actcattcac ccatctatcc 191400
atccactcat ccacccatcc actcattcac tcatctgtcc atccactcat ctacccatcc 191460
actcattcac ccatttatcc atccactcaa ccatccactc atccacccat ccactcatca 191520
tccgtccact cacccatcta tccacccatg tatccatcca ctcatccacc catccactca 191580
tctgtccatc cacttatcca cccatctact cacccaccca tccactcacc catccatcta 191640
cccatcctct catctatcca tccactcatc tacccatcca cttatccatc catccactca 191700
tccgtccatc cactcatcca cgcatccact cacccatcca tctacccatc cactcatcca 191760
cccatccact tgtctactca tccactcacc cacccatcca ctcatccacc cattcactca 191820
tctactcatc cactcatcca tctatccact catccaccaa ttcactcatc cacctatcta 191880
ctcatccacc catccatcca gtcattcatc catttgctca ttcatccgcc aacccatcca 191940
ttcacctgcc tgttcatcca cctacccatc cattcatcca tctgtccatc catccaccca 192000
cccactcacc cattcacaca cccatacacc catccattta tccaaccatc catccccatc 192060
cttacttcag aaagcatttg aagttgctgg ggtgtgcact tggaaaatgc gtcataagct 192120
ttcataggcc tctctttggc ttcctcactt ctttgattgg tctttctttc ggacatgcag 192180
gattctgagc agctcttctg ggaggtgggt gcagtccagg taggctgcct gcaaagctag 192240
gaccgaggga gccacatcca aggcactgtg ggttcccaag gcgatggcac tgtgggccca 192300
ctgcttccag acctcctcaa ctcccaggct cctgagcagg aggactcctg gggcagggcc 192360
tggagacggg aagagaaaac aattccagtc tctcaactct tgtcagtcca ggtgggtgct 192420
gcatggattg gggcacctg gaacagttgg ccccttcacc cctttccccc acctttccag 192480
cccctctggc tataatcaga cagttgtgtg cgatgtcttt ctccctcctg aaggggtcat 192540
ttagtcaggc tcctactcat ggcccaggaa tgtgcccctc cctgcctgtc ccctaccccc 192600
aagcaggccg tgcctccagc cccctgctgt ctctgaaggg cctgggcatg gggcagcctc 192660
tgcagcatcc ccagggcccg agctgatctg cacagcagaa gggaattggg ggaaagcttt 192720
ttttctaccc agcacctggc agggggccac cctgttgcac cccagagacc cttgtctaag 192780
ttttgttatt tcagatgatt tcataagttg ggggaggcgg agccccagcc cccacagccc 192840

ctgttgttgc ctcactgctg agctctgctg tgtatctgct gacaggtctg ctgatagcag 192900
aaggggctgg agagagggaa gctctgggcc ctgccctccc caacccctgc ttaggggaca 192960
tccctgataa gaggaaattc cagcctgtga cagatggcca gtgtccaccc acccacgggg 193020
gaacttttct ttctctttgt aagtttgcag atttgccagg aactaaagga atctctggtt 193080
gacaggccac tgtggacatg gctggagggc tggcacgggc tggatcttgg ggttgctttg 193140
aggtaggggc cgctcctgct ggaaggttcc ttggggtggg gaaggcatca gggccattcc 193200
ctgaccctcc ctctgcagtg gatccacttc agagcctgag acaaggcaag ggggcagcag 193260
aaaacagaat ataaaatagc ccccccacc acacaccgca ccagaagtca gaggattcat 193320
ccttggcctg ccctccctgc agcctaagtt ctgagagtgg ctctttagga agtgaatggt 193380
gatggtttgc aggtcagagc ttgctctggc ccaagattga tgtctcaata ggccgcggag 193440
cctgggtgaa aaaactcagg ctagggaggt ggtgggttgc aaatgaaaga acagaggagg 193500
gcctcttcca gaaagcggcg ggtggtgtga ggagcccttg gaccttgtcc aaatttgact 193560
ttaccgactg gggccaattg aaaccgtgct ggcagaaagg caaaactgct tgagattcct 193620
tcctccacaa tagaggagag gtttgcttga aagggtttgg cgagggaaaa gctggaggaa 193680
ccttggccgg cagtaaaagt ggggaccccc gccccctgcc tcgggggcgc ctcggcccct 193740
gaatgaatga aagaggaggc tggatggaga cagactcaca ccacctgtcc cttccaggtc 193800
ctgcaaagga gcacacaggg ttcttgttgg ctcttccacc ttgttagtca ccaagaagga 193860
gaaaacacca acaggggaac tgtaaagctg cagttttgtt taagccccat agtggaaacc 193920
ggaaagagct ccagctcgga gaaaaatgca gctctttgcc tttgcaaatc cgtgtctttc 193980
tctgggaggc cagagccatt gtgctcgatt tctaagcagg gtttcagagt gtctcaggag 194040
gggaaggcag agtctcagat ttccggcatc ccaggtccta accaggcttt gaggagacgc 194100
ttcctgtccc atacaaagtg tgaagtgtaa tttgatttgg agttagttaa caaattagat 194160
gagctgtgtg cattttaaaa attcagtcct tgatccccca cccacacaca tttgcacaat 194220
ttggtcattt atattcagtg tttccatctt tagaagagaa caggaagtcc ccagtgacat 194280
ttatggagtg tttgtagtag cttgggtagg tcagtaggaa cacaatgcag tgaaaagcag 194340
agttgagatg ggaaaatatc ctgttttcag agccccccag ggatgacatt ccttatcgtc 194400
tctgtccacc cgctctggag tccactccgc aaacagcaac ttgttctcac ttgagagctt 194460
cctcctgccg gagctcaggg atggagagca gctggcctca gtcttctcct cttacctctt 194520
tgtttctcag actcatcttc caccttgaat aattgttttt aacctgctct ccctcttctc 194580
tagctgtaga acccagaact ttgtgtgtgt ggtcaaaagt ttagagagtg ctgcaggtaa 194640
tgctgagata accctattaa attctgtaag ttctcatcac accaagtttg tttgtttttt 194700
ttaattctca ttttatttta tttcatttta tagggatggg gtctcgctac gttgcccagg 194760
ctggtcttga actcctgggc tcaaacgatc ctcccgcctc ggcctcccag agtgccaagt 194820
tattttttta actgggatcc acgggcctag ggattctttg atgaaccgtt caaaatacgt 194880
gtgtgtctgt ctgtctgtct tcaatgtata cttttctagg gagagtcttt acttttcacc 194940
agaggtttgt ggcacacaca tgtcctgtac tactgtatca gattgtgaat tccttacatg 195000
ttaaagactg cttcaaatct gtccggaagt ctcggctaat aaacatttaa ataaatgaac 195060
ttcaaattcc catgtgccac cactgttttc attctgacta ctctcaagcc tgtagtgtca 195120
tctttttgtt actgtgaagt agacactcaa taaatacttg tcaaatgaat caatgttatg 195180
gtcctggtcc actaggaccc caggatcatc gtctaccatt attgtctata gtttctttac 195240
catcttgttc cagagcagtt ctttctctga ggtctgctca cctctgcacg tgtgtctagt 195300
ttccagccct gtcgccttac cattctcttt cctttacctg catttcctga actgttaata 195360
gttaacaata gcttctccct ccacccactc actcatacct gaagtgagaa gtcatcattg 195420
aggtctctgt ggattttgtc agcccaacct ggggcggcct cctggaggtg gagttttcag 195480
atgagggatg gacattttgg tgtctgctgg gtagatctac ctgggagctt tggcaggggc 195540
cccagggcag ctctgcctct ttattttggc tgtgggaccc ctggtgcact agggtttctc 195600
aagtaagcat ctgtgtcctg ggagagccat aggcaggcct gagagggccc ctggaatctg 195660
ggacccccag cccggcccag tagctggtac catcatcttt cttgcaaggg aagttatgga 195720
aactaagatg ctaatgtgct ttgataggcc tggggtggct ctaagccctg ggaagagccc 195780
tggcaacttt ccctggctgg cgggtagaat cttctcctgg cagttcaggg ctctgcctcc 195840
agctccctgg gggagagcaa ctgagggagg agctggaggc caaggcctca gtgctgagct 195900
gcctgcaccg cagctgcacc ttctgggtcc tggctgcagg aaccccatac ttctccagcc 195960
tttccatggt tcccgaagac ctggcctggg gagggggtca gcagcctggg gctcacctac 196020
ctggcttcaa atcctggctc taccgcttac tcgctttgcg accctcataa ttgacaaaag 196080
ccctctcggt ctcggtttcc ttatctgtga aatgggacaa taacagtggc taccttatag 196140
gattgtgtga tgatccatca gttataatag ctcctgagca gcgtttagag taacacctgg 196200
ggctcggtgc agtggcttat gtctataatc ccagcacttt gggaggccaa ggtgggagga 196260
tcgcttgagg ccaggagttt gaaaccagcc tgaccaacat tagtgagacc cactctctaa 196320

```
aaaaaaaaat tagagtaacg cctggcacag agagccccca gtaaacctcg gctgctgtga  196380
tgagctttgt ttcttcattt gttcattcat tcaccagact ttttgccggg gcttgccgtg  196440
agccctacgc tgctctgaag aaagccgggc tggggtgagc gtgatggatg aggtgtctgg  196500
cttcacaaac cctgtgtggg ggggttgtgg tgagaggaaa ccgcacacat cattattcag  196560
ttaccatcgt ggggagactg tctggacgca gagagcaggc tctgtgtggg agcggggagg  196620
gcaagccctg gttgcgaggc agggcttccc caggattcag cagggatctg aaggactttg  196680
caggcaccca gggagatagg agaggaggag ggagcagccc tggccggaca ctgtcctcct  196740
agcattgcct gcatcaggga ctcactcagc tttaagaagc ccctttgtgg gggacaggga  196800
gcatctgtta gtttatagga cctgaagtgc ccccatgggc tcaagtttct gggaaggcct  196860
gcaggtggcc gtagggctgc cgcaggggtg ctggccccag ggtctggatt caggggagcc  196920
tgcagaggga gggcagctgg aggctgctcc agtgtgcatt gttacgaggc aaagtaagga  196980
gactgctggg cccacgctgg gccggggtgg atggaggcaa ggaagtcttc gccggggcaa  197040
gggcaccagc tgtagatgcc ggcagctttc tcctggacac gggcctggaa ggctgacagg  197100
gtgtggtgag tgccaccggc tcccctgccg tggcctggtc agtggcttca caggcctccg  197160
tgggcaggag gaggatgacc ttgcattctg cttggccacc attggcagtg acagacaagg  197220
tgtgtgggga tgtggccatt tcggtccgtg ctctgagagc caggtgtggg ttgggtttgg  197280
ggaagacagg ggaatggcat tgacttgacc ctgagcactt tgtcctgggg tgcaggcccg  197340
gctgtcactc ccctggagct gggaatggca ttgggcggtg gctcagggtc ccttgcccca  197400
gcggcaccat ctctgcattt ggccagcaag gctgatggga cttgtgtgga catttctggg  197460
acgtagacca ggcagcatga cacacaggga ggggtaggca gagaagggcg gcccctgggt  197520
cttggtttcc aagtcaggct ttgcaagcct gctgtgtcaa gccctctctc ggcaccaggc  197580
caggtgggcc agggtcagcc ctggggctgt gatgagtgtg agtgccccta aagtgtgtca  197640
tgtccaatag ctgcctcctc tctgggcact gcctcccagc cagatatcct acagcccccg  197700
tcccagccac agacagcaat ctgattccct catcctcggc aggtcctggg gttggggtaa  197760
gaatttgtca ctaagtgcag atgttcccag agggtgctgg ctcagggctg cgtggattac  197820
agccctttca tccctgcctt agacccacct gcaactctgg agaaaagatg gcccagtgga  197880
cgcctgcggg gaggagggag ctggtcccca ggatgagcag atttgggggc agtccccatc  197940
acgggaccgg tgacctactg ccaccagcgc cagtgaatgg cagaggggcc ctggcataag  198000
gcctgggttc agaatgggaa cctgcagaga aactgtcaag gtctcccgcc acctgacacc  198060
tccgcctggc tgccacccta gaaacaccct tgctgcaaga agatgggggga gtttgtctgg  198120
ggcatggtct ggcgacacca acttgagaac ctcaggccta agtgttggcc tctgggcctc  198180
agctgtcctc tgagctgtaa agctggctcc tgggcacttg tggaaaacac tggactcttt  198240
atttatttt actttacatt ctgggataca tgtgcagaac gtgcaggttt gttacatagg  198300
aatacaagtg ccatagtggt ttgctgcacc cattggactc ttcaatccag ctccactaga  198360
aatgactgcc ttctttccct tcttccgtcc ctccctcctc ccctcccttt tttccttcct  198420
tccctgtttc cttccccctc cctccttccc ttctttccct tcctctcccg tcctcatggg  198480
gatataattt ccatattaca ccatccgccg attcagaaag cacagttgag tggcattttt  198540
tcacaacctt ctataagcat cacctctaat tccagaacgt tttcctcacc ccaaaaggaa  198600
acgccatcct cggtacacag tcactccccc tcccccagcc cctggaaatc accaatctgc  198660
gctttgtccc tacagatgta cttattctga acatatgact agaaccctaa aagatgtgac  198720
tttttgtgct tggtttcttg cagttggaat gttttcagag ttcatctgtg ttggcgcacg  198780
ctcgtgcctt tttatggctg agaaatagtc cgctgtgttg actcagcacg ttgggtttct  198840
ccacgcatct gttggcggat gtgttagtta tttctggagc cgggtgtgtt gtgctactca  198900
cgggctgcac taggagctgg ggatatcact gagtaagcca cagcctcggg cacccaggag  198960
ctcgcagagc aggagctggg agggaggtca ggaggtgcat ggcctgtttg ccaggctgga  199020
gagacttgca aaaagtcact ttgagccaga aaccagagcc tgaggcctct gactcccagc  199080
tgcggagccg ggagggaagc tctgcatttt gctttgactg tggggtgacg tgtcagttac  199140
tgtccaaatc agaacctttc tgagagtcga cgtgggtact gctgataatt agtctggata  199200
cccgaaagaa tggaaagcag ggacccagac agatccttgc acaaccgtgt tcgtagcagg  199260
gcgaggcctg gaggtgcgat ggggatctca acggggcagc tgccgtcttg ctggagccca  199320
cggtctttgg ggggggtcac aggtgttaac cgcatatcat acaagcagga gtgtgaaatt  199380
tcagggttgc caaattcagt gagggtcttg tgctggcctg gccacttaca gtggcatttc  199440
aggaaaaaaa atcactctat ggacctcagt ttccccatta gtaaagagta ggttggactg  199500
gaattctgta ccatccagta tagttgccat ttatgaaatg tggctattta aatttaaata  199560
aaaatagaaa ttccgttcct cagttggact agctcagtgt caagtgctca gaagtcactt  199620
gtgaacacac tgtggcaatt cctcaaaaaa cgaaaactag aaccaccgta gcatcccata  199680
attccacttc tgggtgtgga ttcaaaagaa tgcacagcat cttaaagagc tagagatatt  199740
tgtacaccca cattcatagc ggctctgttc acagtagcca gcgggtagaa acaacccgaa  199800
```

tgcccgcggt caaatgaatg gagaaatcaa aggcgggaca ggccgggcgt ggtggctcac 199860
gcctgtaatc tcagcacttt gggaggccga ggcgggtgga tcatctgagg tcacaagttc 199920
aagaccagcc tgaccagcat ggagaaaccc cgtctctact aaaacaaaat tagccgggca 199980
tggtggcggg tgcctgtaat cccagctact tgagaggctg aggcaggaga attgcttgaa 200040
cctggggagg cagaggtttc ggtgagccga gatcgcgcca ttgcactcca gcctgggcaa 200100
caagagcaaa actctgtcta aaaaaaaaaa aaaaaagggt ggaacataca aggaatttga 200160
ctcagccgta aagaggaagt taggatacct gcaacagcaa gggtgaacct ttcgggtatt 200220
acgctaagtg aaataatttg gtcacaaaag gacaatcctg taggattcca cttataccag 200280
gtccgtagag tagtcagatt cagaaaaaca gcagaatgga ggttgccaga ggcttggggg 200340
atggggctgg ggagttgttt aatgaggaca gtttgtttta caagatgaaa aagttctgga 200400
gatttgttac acaacaatgc gaatgtactt aacaccactg gactgcacac ttcaaaatag 200460
tgaccgtggc aaacgttatg acaattaaaa ggaaaagtca cgtggccagt ggctaccacg 200520
ctgagcagcg caggcctcgg acggtgtcgt ggtcacagac aatggagtgg gtgatcttta 200580
ccattgtttc cagctgtctg agcaccatgg tgtcctgggg ccaggatgaa agtgtctctg 200640
ggtatggggg tctctgcttt tcccacttgg gagtcctgaa ggcagacccc aaagctcacc 200700
tggccaggta ggagagcgag gcgggctctt gcattgctct ctgcgtgcgt gcctgcctgc 200760
gggccagccg gggcctgcaa ggccgcctct cgagggaggg gttgcaggaa tggctctcac 200820
ctgtgcagaa ggcaagccac tgccctacgt caatggcaaa gatggggctc ggctgcgttg 200880
tattattatt tctcaagcgg ggcccctggt gggcaggatg tttggtttga tttgagtttc 200940
tgcagtgagt gctggtgcct gtggagggac ccagctgcag ctgagccggc ccctccctcg 201000
ctctgggctc caggggcgtt gggggcgaga aaggagagtt cagggggcag cgcctccccc 201060
caggtcctgc tggggagggt tgagggatgc tgggggaggg tgggggcggg taggggctgg 201120
tcccagtccc aggcaaaatg gctctcagac ccacaggcag atggaagcga ttttcggtca 201180
cgtgggtggg gactgaactg ctgccccgca aggcaaggcc ccatgtctgg cttcaggaag 201240
cggccccaaa gccacatggt ccatggccag atgcagggct gtgtctttgt ttatttaaag 201300
gtcctggaag cccaggggag agtatttctg aagggggagc aggagcagga gcaaagccct 201360
gaaggcttcc aggctgcgtg gcctgggtgg gggccacgtg ggggcacaga gagaggagcc 201420
ctggcctcca ggtccgcctg tgggtattgc tctcagcagt ggagatgtgt ggaccccccc 201480
ttggcatttc ccaacaagga attatattgt tcctttgttc cccaattccc ataagatagg 201540
aattcctgtg ccctgtggag gctgagaaga cggggaggtg cctgtgagga taaaccagcc 201600
cccagtttct ggcttccaga gtcttttcct ctctcacctc tgatgcctca ggcctgggga 201660
gcctccctcc gacaccctct gaccgctcca gtgaaatagg ccgagaggga tggcttgaat 201720
cggctgggga tgcaggctgc agccaggggg ctcagtgggc atttcctgag ttcggttctt 201780
gccccgcaag tggaagggca gttgaactga agtctcccac tgcccctcct ggcctggaag 201840
cctgcctccc accttctggg cagagtgagg agtgggcgtg ggccgggagg gctgcctcct 201900
gtctgggagg gctgctcact tgcctgtcac gggggatgag ccccctgcag cctcccgtgc 201960
tgtggccctg gctcccacgc cggctccact cccctgtggc tgcccctgct gggtgcccac 202020
cggtcccagg gaaaagccac tcccctcccg gaggactggc gggagggcc ctgcctggga 202080
gcgtgccgtg tggtccctgt cttagcgcag gatgcctgag gccagatcct ccctgggagg 202140
caggatgggg caaaagtctc cccgcaggca gagcagggcc tggatgggcc caagcaggga 202200
cgggggcaca gaacagcagc cgcccagcct gcgtgggagc tgagggccac caagggcagc 202260
tccagcctgt ggggactagc agaggcacca cctctgtgac cagcgagttg tctgcacctg 202320
catgcgcatg caccagtgag tgtggcgagt ctgaggtgtg ctgggacctg gcccgtgtac 202380
cagccagccc tgatgggaac cagccccatc ctgggtaccc ctggaacccc actgtgtggg 202440
agcacctggg tcctcagcag gcgtcttccg ggcgtccgcc cccagcccca ggcctctgac 202500
ccgtgtgtgg tgtcttaaca gcagccagga gcagggccat cgaagggacc tacagacaga 202560
agaggcttta gtgatttcgg gaagttcagc tccacgtgcc ccacataccc aagagtaaaa 202620
cttggtcttg gcctgtgggt gtgcagcccg tggcgctgg ccaggcacac aagacggccg 202680
cctgggtcag tccaggggga ggcagatgtg gtgcaggggc ccccctcctc catgagccca 202740
ggaacagctc tgcatccagg cacccgccct gcacccagca cttttccttc tcccaggact 202800
gctggctcgt tctatgggat tgaacacaca actccatggg gaccgacgct ggttctatcc 202860
ccctttctta caagaccatc catggggatg tgggaggcgg ggacacacgc tggttctatc 202920
cccgtttctt acaagaccat ccctcctgtg tcttggctcg tggatcggct tctaaggcct 202980
ttgagtggca gcaggcggta cagcttatct gagtagaggt gaaggagagg gccaggtcca 203040
cctgggtggc ccgaggcagt gtggagggcc aggagaggag agcaccctcc acccttgtct 203100
gagcagccct ccagggtacg gagacaggag ttcagggtca gccctgcctt ggagcgcagg 203160
gggcccccag cttggcacag ctcctagaag aggagatgaa aaagcctggc cttgggagtc 203220
tggggatgga gctttgctgg ggaggcagcg tggctgcagg atggcagctt tttagaggac 203280

accacggcca gggacatcca cccgggggaa ttctcagggg tcgcctgggc aagtcgctta 203340
gcctttctga gcctcagtgc ctcagtttcc ccatccaagg atgggtgtga caaccacctg 203400
gcaggcgcct gtgtgtgagc ccctgttccg ctctaactcc tctgctgttc cttccccagc 203460
cactgaggcg gctcccagct gcgttggcga catggccgac acccccagag atgccgggct 203520
caagcaggcg cctgcatcac ggaacgagaa ggccccggtg gacttcggct acgtggggat 203580
tgactccatc ctggagcaga tgcgccggaa ggccatgaag cagggcttcg agttcaacat 203640
catggtggtc ggtgagtcct caccttgcat gccactcaac caatgccgga aaccagcctc 203700
agcccccagg gcggcccaca agcctttggg cttggtcttc ccttctgtaa aatggggcag 203760
caaccctgac ctcaaggacg gattgccagt gacattgcgt gtgcatgcat gcgcctggga 203820
ggagcccaca gcctccagcg gctcctcaga ggggcctgtc ctgcacatcc agggcccctg 203880
agatgacttt ggggagccca ggccggcctg gagcaggtcc tgatgagtgg tcgctgcctc 203940
tgagcaatga cgtcagcttg tgcagagggt tctgtggcag ctgtcccctc actgttgctc 204000
cagcctggcc ctccaggctc cccaccgcac cccaccgcac cccggccaga ggcttcccag 204060
cagctcagct tcagctctgc cctggaattg gggcctcgtg gcttccacac cccctggtgg 204120
ccccggagag ctggaggtca ccacgggagg tcgtcgatca gcacgacctg ggcagggtga 204180
gggggccagg ctggagtcta cagtgggccg cctgttgttt ccaaagcccc ccaggtccca 204240
ttcaaacctc acagggaccc tgtgaagggg gcgtcccgtt ttccaggcag aagcgaggct 204300
cagggaaggc tgccaaggtt ccttagctca cagggcagag aaccgcagcc accgttttga 204360
ctcccagaac tgtgttctgc ccactccacg gggtccaggg aggggtctgg ggaagtgccc 204420
ccgccaggtc acaccctaaa caaaggtgag cttgcggggt ttctgcaagg tgtctgggca 204480
cctctcaggc cgggcctgtt tctctgacct ttgccagccg tgcacgtcct tcctgcccag 204540
ctctgtggac gcaaacccac acctgccagc ccgacacctt tctttcctct tctgcactga 204600
tctgggccct ggctcccctg gggtcttggt gtcctctctg cttcctgtag cctcatggga 204660
cccggctgct catggccctg ctgtgctcac agctgcctgg aagctggcag cctgcttcaa 204720
gggagggcgg aggtgtgggg tcccctccta cggcccccac cctgttctgc ctgttgtggg 204780
ggacgtgctt gtaccctgag gattcagggc tgcctcccgg gaagatggct ggagagaaag 204840
agaggggaga ggcccaaagg cagtggccag gctgggactc atctctgtgg gctacggagg 204900
ggctgccctg ctccctagcc agggacaggg tgagaaggac ctgggaacct ctgcgtccca 204960
gccatggcac aggccttctt ccggagcttt ggccccaagg agagggagag gcagcccagc 205020
agccctcgcc ttccgtggct gccactgtga cagccttgcc ctctgggaat ggtgcgcagg 205080
aggccacttc acctgtctgg gcctcttcgc cctttcaacc ctgagaagaa cagtggtcct 205140
gattccccac aaaatcactc cctcccaggg atgccgagtg aggaacaaga cgtggggcag 205200
ctctgctctc ctccctcctc cctcccatgg cagctgtgcc cacctgcaga ggtgcaggca 205260
aggtggaggg gcacctgttt ggaggtccac agaccccggg gagaatccca gctttgtcct 205320
aactcagcgg ggtgaggctt gtgtgtcttc ccctgggctc ctcctctgtg agccttagct 205380
tcctcgtcca taaaatggga tcatcccata aagtgagatc aagcgagcta ggccccgggc 205440
agggtcccca acaggttgtg gttgttcgag aatttgcagc agtggtgatg gtggttgtgg 205500
tgatgaggat gatgggggtg gtaatgacgg tgatgatgat ggggtgatgg tgctagtgga 205560
gatgatgggg atgatggtga tggtggtggt gatagtggtg ggatggtagt ggtgatgtgg 205620
gtgatggtga ttgtgatggt ggtggtggtg gtgatggagg tgatggtgat agtgatcatg 205680
agggtgatgg ggatggtggt ggtggtgatg gtgacagtgg tgggatggta gtgatgatgt 205740
gggtgatggt gatggtggtg atggtggtgg tggtgattgt ggtggtggtg gtgatgatgg 205800
tggtgatgat ggtgattgtg gtgatggtga tggtggtaat tgtgatggtg gtggtggtga 205860
tggtggtgat gatggtgatt gtggtgatgg tggttatgat ggtggtaatt gtgatggtgg 205920
tggtgatggt ggtgatgggg atgatgatgg tgattgtgat gggggtggtg gtggtgattg 205980
tgatggtggt gatgtgggtg gtggtggtga ttgtgatggt ggtgatgtgg gtggtggtgg 206040
tgattgtgat ggtggtggtg gtggttgtga tggtggtgat gtgggtggtg gtggtgattg 206100
tgatggtggt gatgtgggtg gtggtggtga ttgtgatggt ggtggcgatg gtggttgtga 206160
tggtggtgat gtgggtggtg gtgattgtga tggttgtgat tgtgatggtg gtggtggtga 206220
tggtgatgat ggtggtgatt gtgatggtgg tgatgggagt gatgctggtg attgtggtgg 206280
tgaagggggt gatggtggtg attgtgatgg tggtgaaggg ggtgatggtg gtgattgtga 206340
tggtggtgaa gggggtgatg gtggtgattg tggtggtggt aatggtgatg gtggtaaagg 206400
ggtgatggtg gtgattgtgg tgatgtgggt ggtggtggtg atgtgggtgg tggtggtgat 206460
tgtgatgggg gtgatgatgg taatgtgggt gatggtgatt gtggtggtgg tggtgatggt 206520
gatgattgtg atggtggtga agggggtgat ggtggtgatt gtgatggtgg tgattgtgat 206580
ggtgatgtgg gtgatggtgg tgttgatggg ggtgatgatg ttagtagtgg tggtgatagt 206640
gatggggatg atggtggtgt tgatggtgat agtggtggga tggtagtggt gatgtggttg 206700
atgatggtgc tggtgctgat gattgtgatg acagtggtgg tggtgatgag ggtgacggaa 206760

gtggtgatga tggcgggagt gatgggatga tggtaatagc agtgatggta ttgtgatgat 206820
ggtgctgatg gggatggtgg tgatgataag ggtgatgatg atggtgatga taataataaa 206880
agtaacaata ataaagggat acaccttgca gtatcccagt tggcatctcc tagatctaag 206940
gtgtcgtggc tggaacattg acataactcc tgacctgagg ttggccagga cacagacctg 207000
gtggacagag agagatggac gaggagtgtc cgtccttggg ctactaaaca gttcaggcca 207060
gggattccca gcaccagcct ctgtcctccc aggagcccac acttttccta gaaaggtgtt 207120
atcaaagtca gaacatcagt gtaatttagg gtgtttttttg tgttgtgttt ttttgagaca 207180
gggtctcatt gttgcccagg ctggagtgca gtggcatggt cttgactcac tgcagcctcc 207240
acatcctggg ttcaagtgat tctcgtgcct caggcatgtg ccaccatacc tggctaattt 207300
ttgtgttttt tggtagagac agggtttcac catgttggcc aagctggtct cgaactcctg 207360
acctcaagta atccaccctc ctcagcctcc caaagtgctg ggattacagg cgtgagccat 207420
ctcgcctggc cgatttttgt tttttaaaa gacagggtct ctgtcactct ggagtggcaa 207480
tgatcctagc tcactgcagc ctcgcactcc tgggcttgag caattctcct acttcagcct 207540
cctgcgtagc tgggactata ggtgctcgcc actacaccta gccaatgttt acattttgat 207600
gtagagacag ggtctctctg tgttgcccag gctggtcttg aactcctggg ctcaagcaat 207660
cctcctgcct cagcctccca aagtgctggg actacaggca tgagccacca cacctggcct 207720
agggtatttt ttttggtgtg ttgctagcgt agcccaaggc agctctgtgc agggtaaagc 207780
aatggggctc cgagtctgga ggggtgtgtg tgtgtgtgtg tgtgtgtgtg tgtgtgtgcg 207840
cgcacgcgcg cgcgtgttat atgtgatttg tttgtgtgtg ttgtgagagt tgtgtgtgag 207900
gtatgtgtaa gaggtatgtt tgaggtgagt tgtgtgtgtg ttgtgagttg tgcatgtgaa 207960
gtgtgtgtgt tgtgaggtgt gtgtatgagg tgtatgtgag ttgtgtgtgt tgtgttgcat 208020
atgaatgcta cttcctcatc ctcgtttgtc tcagtcacct gtggactggc tgtctgcaca 208080
cacggtcact gtccctcgag tggcaccacc ctgcaggcca gcgcccccta gcacatggcc 208140
ccacagaccc catgggacat gcgtgagcct gcctgatgct cacaccagct ccacggggga 208200
aggactgttt atcccatttt gcagatcagg aaactgagac acggtgaggt caggtgactt 208260
gcccaggccc tggccggtca gggaggagca gtttgaacac ctgggctttg gttgcaacca 208320
ccctggtgaa ctgcaggaat cccggccacc tggtgcaccc tgggcagccc tgggctcctc 208380
cgccagcccg gcctctgtcc tgtccaaaat ctgagccacc aggagacctc ctgtccagaa 208440
agcacaaggg gcacaagaga tgccgactct cccaggcccg gctcacctct caggagagga 208500
ctgtggcctg ggttgtgctg gcatcagagc ccggctgtgt gggtttacac agtcactgga 208560
cacccggctc gagggtgaag tcctcggggg ctgcttggca gggatattgc cgggaggtag 208620
cccaggcact gctgaatctc agactggaga gcctcgtgcc tgggtgggag gggccccatg 208680
tgcagaccct gctggtctct ccggagagac ccctgaccgg cctcccatcc tctccccagg 208740
gcagagcggc ttgggtaaat ccaccttaat caacaccctc ttcaaatcca aaatcagccg 208800
gaagtcggtg cagcccacct cagaggagcg catccccaag accatcgaga tcaagtccat 208860
cacgcacggt cagtggccgg gagtgggctg gggggtgcagg acgcccctgc cttcctggag 208920
cacaggggtt gggggtcaag accatcacac acagtcagtg gccaggggcg ggctgggggt 208980
gcaggactcc tctgccttcc tggagcacag agtgggggggt caagaccatc acacacagtc 209040
agtggccggg agtgggctgg ggatgcagga cgcccctgcc ttcctggagc acaggggttg 209100
ggggtcaaga ccatcacaca cggtcagtgg ctggggatgg gctggggatg tgggataccc 209160
atgccttcct ggagcactgg tgcggtgtga gggtgcccga gtcaggcaat cccaggtccc 209220
caagacgggg actcgctgtg cccaccatcc ccagcatgtt gagcatgttg gggctctggc 209280
tgtgatgggt cagtgtcccg gacccctgtg ggccccgtca cactgagctc cccacagaca 209340
actgagtttt ccaccaaaag tctgacaggc aaggggctcc caggggaacc gtcaccggcc 209400
tgtgccggag ttggctgagg aaggccatta atttcccggt gtctccttgg ttgtcatcgc 209460
tgcctacctg gggttgcccc ggggtcagtc aggtgtgggg tgtctgtgag ggtcttccgt 209520
ctcccctctg actctgcgtc cgtggctctg tgcagatatt gaggagaaag gcgtccggat 209580
gaagctgaca gtgattgaca caccagggtt cggggaccac atcaacaacg agaactggtg 209640
aggcccctcc aggggagga gcactagcgg gggcttcagg gctccctgga ccccacccag 209700
agtcagccct agaggtttcc cggcccgcgg gggtgcaggg cccacctcct gggacctgac 209760
atgctgccaa agccgcacgt ctcaggggcc tgaaaattct ggaccaaagc agaattacct 209820
ggggaatctt gctgtcccca actcatcacc tgaggccagc ccggggaggc tggtttggga 209880
ggccgagggt aaggctgtgg catctccgct cagcaagcca gacctcccag ggcatgcatt 209940
tcattggaag gtggggtgtt gggctctgag tcctgggaat gcacgacctg cttggggagg 210000
gcatctcatg tgcctgctga ctcgtcccca tcccccacgc agctggcagc ccatcatgaa 210060
gttcatcaat gaccagtacg agaaatacct gcaggaggag gtcaacatca accgcaagaa 210120
gcgcatcccg gacacccgcg tccactgctg cctctacttc atccccgcca ccggccactc 210180
gtacgtccct gcagtgtcgg cgtcctcatg ccgggtgccc tgggttccct ctgtcccctg 210240

ggactgcaag acggtgctgt ctgccctgct gctgggtgca gaggggctga gtcaggggcc 210300
atggccgcca gctatgaagt tgggggtgtg ccatgtctgc acctccaaag cctcacactg 210360
acccctatgc aagtgtcctg cccagccccc atccttcagc tgcgtcttgg ggagctgagg 210420
gtcaggcagg ccctgggctg ttctggagcc tggtggtctg tgtcctgcct tctcaggcca 210480
ggctctggca ggtggagtgg gagcccccag ccaagcaccc gtagcggtga ctgaccttct 210540
catccgggcc ctgcctcatg ggacccctta gagggtttgg tgcgttctta gacgggaagc 210600
acagagatca gtgctggccc cttccagggc tggacggggc tgctgttggt gttggtgaga 210660
gccctttcct cccgccggct ctgtccgcac ctgacgtcgc ccctagagat cagcaagggc 210720
gggaggctgc atgtaccctg gcaggtggac tcttcatggc ctcacggacc ctctgtcctc 210780
agctccctgt gcaccccctg ccagacacct gtggctcacc ctgagccagg gctgggagga 210840
ggaggaccag aggccagcag gagtggctgg tggaggcagc cgtgggtggc ttgtctgcat 210900
gggtgggtgg agaaggtcct tcgcacctgc tgtcccagat gtacttccct gggcggagga 210960
agcccagggg tttggggaaa ccatactctg tgggcaccag gtgcaggaag gagcctggct 211020
ggtgggcagc gggcctcacc cagagtctgt gggaaaaccc aagccggagt tttcgagccc 211080
accctctggc atcatcctct gtgggccagc aggagtcagg ggcagggggc cagcccgctg 211140
tccatgggcc tctcctgaac tgaacatctg ccctcccagg cagaacggct cacctgcggt 211200
cctgtggcag ctgttccagc tgctccaagc tttgcctcag ctgaggctga tggcaggagg 211260
cagaagcctc tcctgatgcc agctgggcaa gccgagggcg ggcgggcggt ctgtggcact 211320
tctgccactc cccagcactg accctttggc tgggactcca gctaggcatg ttgcctggtc 211380
ccgcccaagg cgtccagtcc catggggcag gggcgggcct tggccatctc tgcttaaact 211440
tctgcccgga gcgggcttct ccccagccat gtgtgtgacc gatttattct cactgtggga 211500
aaagctcggc tccagaactg gggaagatgg tgccccagtt ccagggccct gccagcccct 211560
gccacccaca gctttcccca catcagaccc caggctcact ggggccaagc aggcaagggg 211620
agcagcccgc ctggtgcagg ggagcagccc gcctggtgca ggggcaccgc acactggtgc 211680
tagcttctat ttctcttgcc tctcccctcc cagccaaagc cccacagcaa gacggctgct 211740
gagccaacga cagcccttct gtgtggacat gtaggggtgt ggttgggacc ccagaactca 211800
tggacgaggg tgcttggagc tggccctggc ccgcctggcc tgtgcagtgg tgtcacctgt 211860
gtctgggaca cacccgtgtc cttcctctgc cccacactca cagcgtggcc gactcggccc 211920
ggggccctgt ccttgccagc agggaccccc gtgagccccg agccagcacc tgagagtgtc 211980
gacacctgct tgggggtggg tgccccccca ctgccccgct cccccagatg agcctcacgc 212040
acatccctct gcttcagcct caggcccctg gacatcgagt ttatgaaacg cctgagcaag 212100
gtggtcaaca tcgtccctgt catcgccaag gcggacacac tcaccctgga ggagagggtc 212160
cacttcaaac agcgggtagg gttccatctc tacttgcccc agcccttctg tgcaacctgg 212220
agacgctgca ggcctggcag gggccacccc gtctaaagga gtcacactgt cagggagacc 212280
gaaccgctgg tcactctccc cagcgggtgg ctggcctggt ggtccctggc ccaggtggct 212340
gctgggggcc gtccctgaga tggcctctcc agctccccca tatctcaaaa gcagctttgt 212400
gggagtgcca attctccagc agagtctctg aggggggttt tggagaagac ctggaccagg 212460
cagatggtga atctctcttc cagggagtga ggccgagcag gggccgtgcg acatggccca 212520
ccttgtttta tgcccccccct tttttttttt gagaaagggt ctggctcagt tgccaaggtt 212580
gcaatgtagt ggcacaatca tggctcactg cagcctccac ctcccaggct cagtccccga 212640
gtagctggaa ttacagacgc acaccaccat gtctggctaa tttttgtatt tttagtagag 212700
acgaggtttt gccatgttgc ccaggctggt ctctaaccct tgggctcaag ggatcctcct 212760
gcctcagcct cccaaagtgc tggggttaca ggcatgagcc acctcacctc gccctgcctg 212820
gtttttatta gatggaagag aagctgggcc aggtgtggtg gctcacgcct gtaatcccag 212880
cactttggga ggccgaggcg ggcagatcac ttgaggtcag gagtttgagg ccaacatgat 212940
gaaaccccat ctctactaaa aatacaaaaa tattagtcgg gcatggtggc gggcgcctgt 213000
agtcccagct acttgggagg ctgaggcagg agaatctctt gaacccggga ggcggaggct 213060
tcagtgagcc gagattgagc cactgcactc cagcctgggt gacagagcga gactccatct 213120
caaaaaaaaa aaaaaaaaaa aaagtttcag gagggatggt ctctgccacc ttcttctccg 213180
ccaccgtctg aggcccctgc tgtgggatag ggaggggcat ccaagcaaga gggcagccct 213240
gggctcttcc tcctgacagg gcagcacatc agagccacat ggggtgcctt tcaaaatgaa 213300
accgaggtgc ctgggctgcg gcaccacatc tgcctcggtg ggtgtgggtg gggcctgtaa 213360
cctactccgt cctgggggcca ggtgtcaggg accttcccag catgtgcagg ggaagacagt 213420
ccacacaaag tgggtgtgtc tgccggaggc tacacacggg ctgtggtctg tgcctgggca 213480
ggcgagcagc cgctcactgg gatgtttctg ttgcacgtac ccatgtcggg ctgctggcag 213540
ggagctgaga ggttactgca ggcggggccc ctgctggaac tggggactgt gacgagggtt 213600
ttttaggaag ggtttcagga ggggaggtct cggtaaccct gagtactttc ttggggctgt 213660
gatgagcagg agaagagagg tgggaggtgc tcaggacgat gaccttaagc ccctggggag 213720

ttgcagcgtc gctcaggaca gcaggtgcac ctgcagctgc ccctcttccc tccaggaggc 213780
acaggagttg gaggtgattg gtgtcacagc cccccagagc ctgcccttga acccgagcct 213840
ggggcagcac acagtgtgga ggtcatgtgg caaacaccag tgggtgggta gagcttgcca 213900
cagggatggg cccatctctc tccctcctta tcccagatca ccgcagacct gctgtccaac 213960
ggcatcgacg tgtacccccca gaaggaattt gatgaggact cggaggaccg gctggtgaac 214020
gagaagttcc gggtgagtgg atccactagg atgttgttcc caggggaccc ccagttcctg 214080
ctgaagggtg ggttgggggt ttggaggacc ttaagccatg aaattatatt cttggggcca 214140
gagggcactg agcccaggtg tctgtaccca gtgctgtcag gctgaggctc tcgtttttgg 214200
gggaccccag gctcagggca gctcctcccg ggggcccagg ggagggaatt gccttcccgc 214260
acatgtgtaa ccaataccgt ctgcccgttc cccaggagat gatcccattt gctgtggtgg 214320
gcagtgacca cgagtaccag gtcaacggca agaggatcct tgggaggaag accaagtggg 214380
gtaccatcga aggtactcgc cgcaggcgcc ggggctccag acagatggga agacagtctc 214440
ttctgctgac ccagagcctg tgggccacgc ctgagccagg gactcgtgga acctcatcca 214500
ctgccttgcc ccacccagag ggaggggtct ccttgtcccc attcaaccct tgggaaagtg 214560
agagggcaag tggcctgtgc agatgcctcc accccagacc tccccccggg cagggaaaga 214620
ttcagggaga caggagctgg gatgggggcc gccaagttct ggatcctggt gcaggctctt 214680
tcataggcac ctgtgtggcc ctggacaaat cgcccaacct ctctgggcct agaaaagggg 214740
tgggctgtag ctctgtgagc cagatggacc ctcggcccct taccctgctg aagttcctgg 214800
gaccctcaca ccttagtctc ttgagtggct gttgtaacca attagcacaa acgaggggac 214860
ttaaaacaac acaaacgtat tttcacagtt ccagaggcca gaggtccgaa atcaggggct 214920
gtttccttct gaggccctga ggaccagcct catccatgcc ttcctccttc tggtggcgcc 214980
tggcattcct agttgtattg taaaggcatc acatgctcta atctctgccc cattgtcaca 215040
gggccttctt ccctgtgtct ctgtcccgtc cctcctcttc cttttttttt tttttttttt 215100
ttgtttgaga cggagtctcg ttctgtcacc caggctggag tgcagtgttg caatctcagc 215160
tcactgcaac ctctgcctcc tgggttcaag taattctcct gcctcagcct cccgagtatc 215220
tgggattaca gtcacgtacc acccacctga cttaattttt gtatttttag tagagacagg 215280
atttcgccat gttggccagg ctggtcttaa actcctgacc tcaggtgatc tgcccacctt 215340
gcttggcctc ccaaagtgct gggattacag gaatgagcca ccgagcctgc cctctttcct 215400
tcttgtcttt ggacaaggat gcctgtcttt ggacttaggg cctaccctaa gtggatgatc 215460
ccatttggag atccttaatg aatctataaa gaaagacgtt ttccaagtaa ggtcacaatc 215520
atgggttcca aggcctggac cttgaccatg tctttgaatg gatgggagcc cctgggggggc 215580
cacagttcaa cccactcctg gcggtgacac cccgggaggg atgggcccga acgtttcctt 215640
cccctttcctc tctctgtccc ttcatttctc attagaatgg aagaggggaa ggtgcagagg 215700
gaaatgcagc aggaaaagcc actttgttct gggagagcac ttggctgaaa ggcccagtag 215760
agcaggaagc acaagtctct taatcttcca gggcctcagt tttcatcatc cacaaagtgg 215820
gtgcagtgtg ccaagatttt agtgagttga gagactgtcc caaagaccac agagcttttt 215880
gggaagctgt tgctctaaaa aaatggtcat aatgacaatt accaggaggc atcagacact 215940
cctgtgccac tggctagaca tgggttatct cgtttcatgt ccgaagctcc ccgcacccac 216000
cctccttgca gagttgaaga ggtgctgtga gcagaaaacc tgccaaggga cccagaacgg 216060
gaggcggctc tagaatctac agctccagcc ctgcaccaga ctccctcgag atgggagttc 216120
atgcctggag gagggtgtgg agaggcacac gggcctttcc cctgtctgca ctcctcccct 216180
accaccaccc ctgccgggcc caccctgcgg gagctgccca gctgggtgca gcctgggtgt 216240
tttccagtat ccgcctgtgg tgtcaggctg gcttgcctcc ccttggcccc tctgcctgca 216300
gttcctctct tcctctctgc ccaaagagtt cttgctgttt aggaaacttt ttatgaaagg 216360
caggtttggg agagttaggg atggatgggc ctgggacggg ccggcggctt tgctatgggc 216420
gtgcctctgc tgcaccctgg tggccaacct tgagtaccgc aggtggccgg tgacagaaac 216480
tgtcaggaaa tgcacaagag agaggtcccc gcacctctct gacctgtgcg ttgttgagca 216540
ccactggctc cagctgagga agagtctgga acctgtcagg acagttgtga ggtggtgggg 216600
cccactttcc agcagtctta tatgaggacc ctggaaatgt gccttcagca ctgctgggtt 216660
tttatatcag gggccccatg agttcatagc tgacctgaca cccggactag taagggctgt 216720
gccttgttct tgcctcccac ttggtgaaca gcatcttgag gtatctttcc catccttaga 216780
aaccaccccca agtattgtca atagaaatgt ggggtgtaat tgatcacgcc atgaactcat 216840
ttaatcctca tgtccctgag aggcgggtga cgttattatc cacattttat ggatgaggaa 216900
actgagggaa tgagagtaac ttgcccgagc cagggagtgg cgatcacaca ctccaactcc 216960
agcctgagag ccccattctt cctgccggct ctcgctgccc gtcagtgtta gatgagagtg 217020
ctgctggtca gtctgcagga ggttctagtg tggacaccgc cctacacagg gccgtggcag 217080
gagggcctag gcaaaggcaa ccggcatagc actgagagca cttgaggggct gggcaaggtg 217140
gaggccccgg ccctgcctgc tggcctacca agtaaaaact agacattttt aacttgggga 217200

```
aaagccatgt ggctctgcga tggggcagcc tggagccagg tgtatgggtt ctgcatcccg  217260
ctccagcctt atgcactgtg gagctgagac agacacagtt cttgcgttct ctatgcctca  217320
ttttcttttc ttttcttttt ctttttcttt tttttttttt ttgagacgaa gtttcactct  217380
tttgcccagg ctggagtgcc gtggcgtgat cttggctcac cacaacctcc acctcctggg  217440
ttccagtgat tctcctgcct cagcctcctg agtagctggg attacaggcg cctggtacca  217500
ggcctggcta atttttgtat ttttaacaga gacgagattt catcatgttg gccaggctgg  217560
tctcgaactc ctgacctcgt gatccaccca cctcagcctc ccaaagtgct gggattgcag  217620
acgtgagccg tggtgcccag cgtctacgcc ccattttcgt aagataatgg ggataacaaa  217680
ggaatcacaa gagacacttg caggtgtgtg aagacgaagt tcatcacatg ggagggcacc  217740
gggtgtcttt atttgccttc cctgcccggc tccccacctc cagtgatgcc gttcactgcc  217800
ctccttgtta aaaggccttc tgttatataa cagttacaca tgccaggcac cgcgtgttca  217860
tataggagtt acgaagcaca gcaataaaat gaccactctg agatggccac cagctcgagc  217920
ccgagggcac ttgtgggtgc ccgtggtctc ctgcccagtc tccctgagaa gtggccactg  217980
tcccgagctt gggcttgtat gtattgtgtg aagtgcacat gtccctgagc aatgtttagc  218040
attgctgatt cttgagcttg tgaatggtcg tccacagtac gtagcccctg tgtgtgtccc  218100
tgtgtgtgtg tgctctgagc gcacacaagg tggatggggc cgtgggggta acaccctgtg  218160
ggccacaggt tttctttaaa atcccagctc cgattcctgc ttcatcccat tcccagcagc  218220
ccagccccct gcctgtcttc aggcccctgc tgggacgtcc tgctctccct ggtcacccca  218280
gtggtgcggg ggcccactgc cctggcttct ccactgtggc ttccttccat gctcctattt  218340
ggcctggtca ctgtgccatc tcccattagc caggaacttt ctggagagca ggagctgagc  218400
tgtccaggag caggagctgg tgctggtcac tccagtatcc cccagactga gggcagtgcc  218460
aggtgtccta tactcagctg cagagcaggt ggttcccctg ccctcctgcc catggggctg  218520
ccctcagact ctgcttttgg caccagcatt tctgggcagg gggagagagg tggggtccgg  218580
gcagagtttc tccactggga cattaaagcc cctcctgtct cctctcctag ttgaaaacac  218640
cacacactgt gagtttgcct acctgcggga ccttctcatc aggtgagaga cagggtgctt  218700
gggtggggct gacggcttca cccctaagag ggccctacag cgggtggggg cagtgggtgt  218760
ggggccgaag ccctgggcag agtgggtgcc ccctgccacc tgcctgccct gccctcggga  218820
agatcttgga agcccatctt ctgaaaaaga aaacccactg ggaaatgaag caggcagtga  218880
agatcctttt caacccctgc gaagttggct ggatgggaag gctgagcttt gatccactgt  218940
ggtctggccc gttagagctg cccggtggcc actaagggcc cagtgaggcc tcatgtgcag  219000
gccctgccgg cagcgtgggg cgtctccagc tccgatccca ctggcctctg acccctaccc  219060
ctttcacctg gctgaggctt ctccttgttt cccttgagtc caggcaagga ggggaagccc  219120
tgatggagag ggtgctgggt gggtggtccc tggagtgtgg gccctggcct gggcaggttc  219180
ttcctcagca agcagctggc atggatgatg ggaatgttat caagaggagg ggcctccagg  219240
ttggcggggg caggggtggg gggattccgg gagccgttct gccattgtgg ggatactggg  219300
ctttccaacc tccctccaga ttattcctcc tgagctgcag aggaatgaaa atccgagccc  219360
aggacttttt tttcctgggg acaagggcaa cccagccctt tgccccaggc cacctgcctg  219420
ccacaggcca tagtcctgga ggccggtggt cacccactgc ttccaccctg ccagcaccct  219480
ggacaccggc ctggagcaac aatgctccag ccctgtccct ctgagtctat gcactgtccc  219540
tgggccctgg acccagggcc atagggtgga cccagaggga gacacatgca ctggaatatg  219600
tgtgttctga ccgagtctgg gcttaccagg gggactgacc cttcccccaa aacgtgtacc  219660
agatctggtc caacatggtg ggcctggggg accccatggg gagccaagca gtcgggatgg  219720
ggagtggggg tgggggcagg cgggcctgag tccgaggcag gccgagcagg gcccctgccc  219780
cgctgccccc accccgctgc gcccacctca ctgacccgcc cgccccccac ccccacagga  219840
cgcacatgca gaacatcaag gacatcacca gcagcatcca cttcgaggcg taccgtgtga  219900
agcgcctcaa cgagggcagc agcgccatgg ccaacggcat ggaggagaag gagccagaag  219960
ccccggagat gtagacgcca ccctgcccac ccccgggatc ctgcccccaa gtcatttccg  220020
tcccccccca ggccctccca ccaccccatt ttattttata tgatttctc catttgtcat  220080
cgttccccac cccttcgaca tgctgccagg aaacaaggga aggggcctcc ctccgagtga  220140
gtcagtgatg aggccgcggc ctccccgagg ttgtggggag gctgcactgg agccacaggc  220200
aggggtgaga gcacccactg aattgacatg accctctgtc cccaggcctg gctccccgag  220260
ggctcagaag agcagcttcg gtgtgcagat catccgtctg tgtggggttc tcagtgccgg  220320
aggccttggg gtgggggcca ggcctcgcac ttgcagagga gcccagtggg ctgcacgctc  220380
ccctccatcc ccatcggccc tgtcccctgg agtgtgtcag agcccagggg agaatgcagc  220440
ccaccaggag cacctggacc ccctgcccgc cacatggtgt ggccatcact cagcccctac  220500
ccctgccctg ctcctaaggg tagaaaactc cagggtcccc tgccaccgac tgcccagcca  220560
ctccaagccc cctggcagct gcccctcctg gagcagaaag tgcctttatc tcagccatcc  220620
gcagactgct tggccagatg cggggacagg ctggaatgag ggaggcgtct tcatctccct  220680
```

```
gccatccccc tctcacgcca cccccgcccc caccgggctg caggtgctgc tgatgcgctg 220740
ggatctgatt gaggataaaa aggaaggaga gatgacccct accccctcat cccccagttt 220800
tgaaaaggtc taagcaagtg agtctggtgg aggagctgag ggagggagcc atggaaggtg 220860
ccagaaggaa ggttggcggg ggcacgtgtg ggccgtggct tgggctggtc agagtggcgt 220920
gagctgcccg gcgcctgccc tgcccaagtg accagggaag tgtgtgtgtg tccatgtgta 220980
tgcgtgtccg tctgtctgtc tagtgtctgg gtttggccca agactgggct gtagttacat 221040
taatgcccag ccagccaccc ctgccactca cccttcctgg cccaggcctt gctgactctc 221100
tgagctgggg aggtgggagg ccaggcgagc ctgactctgt tgatctaccc gtgcctgggc 221160
ccctcccctc agagcccatg gtaacgaacc cctagaaagg agagaacggg cgtcaggggt 221220
gcacagtcca cagctgaaga gcaaggtttc gtggcagcac ggcccggccc ctcaccctct 221280
gtccccacga ggggacccat gggggctgtc tttgcagggc acagatgacc aaagtccctt 221340
cctgcttcct gttacctgtc ttgctcctgg ggagaaagag gggcctgatg agactccact 221400
caggtgcaca catcaccagg tgcatctgca ggcaccgggc tggctgcttg cagccaggag 221460
aaggtcagcg agaaggagtg tatgagtgtg agtgtgtgtg catggaagtt ggggcactgg 221520
gcgtctgact ccctccccac ccaagagagg aaggacccct caccaccccc actggtgaga 221580
cagtttactt tgccaacttg ccatgttttt gccaaaacca agattttgaa ggaaatgagt 221640
ggccagcgcc agggcccagg ccatgtggcc tgcccagcct caatgtcact tggtggcggg 221700
gtggggtggg ggtgggcagc agcatcccag ccttgagatg cttcactttc cttctctgta 221760
accagacttt gaaaaattgt tcgtttcatc aggctctgtt cctcaatggc cttttgctac 221820
gtgcctcccg agaaatttgt ctttttgtat aaatgacaaa gtgttgaaaa tgtatttcct 221880
gaaataaatg tttcaaatgc agaaaccca 221909
```

<210> 3
<211> 52626
<212> DNA
<213> Artificial Sequence

<220>
<223> chemically treated genomic DNA (Homo sapiens)

<400> 3

```
ggaggaagga gaaggagttt attgtaaata ataataatta aatagtttgg gttgggttag 60
taaagggtgt tttgggagtg ttcggttatt tttgtatttt attttgtttt atgtttgggt 120
ttttatattt ttttttattt cgtgtttttt ttttttttaaa aaagatgtaa gtgtaataat 180
ttattttaaa aggtaagaac gttttttttta atattttgtt ataatgtagt tatatggtgg 240
tataggtagt aaaattttat ggaatcgatt ttattttttt ttttatattt ttttggattt 300
ttattgtatt tttaatatat atttttaata ttttatttta ggttattaag ttaaaggaaa 360
agttgtattt atatagggtt aaaatatttt ataatgagaa taataagtaa cggttgaggt 420
ttatgttttt tttagtattt agtttttttt ttttaattat tgtatattta aatagatgat 480
tagatattga aaattgtttt ttaaaattag tagtataaag gtttagtttt atgtgtgtga 540
gtgaagaggg aagaaaggag aggtcgaggt taggttatgg aagtattttt tttttttata 600
gtattttgaa agaagtgaag tggggttgat gggttattgt tatttttttta tttttttaatt 660
ggatagttat agagtattat tttgggggtt agtttattaa ttattttttt ttagttaggt 720
ttgtttgttt ttgtattgga tagtaagggt tatgaaatag attaggaatt ttcgcgtgtt 780
ttttaggttt tcgaagatgg tttttttattg tttagtttag ggagggaatg attgtataag 840
tagggtacgt agtttttatg gaagcgtttt tcgtttgttt agtcgaaatg ataggagatg 900
gggtacggag ttgggtttag tgtgattata gttgtgtgtg ttggggattt attttatatt 960
tgttgtattt attttatcgt agtgtttttg agttaatttg gttatcgtgt ttttcgaagt 1020
tggaggtacg gtagtgagcg ttgagaatta agtatagtta aaagaattta aggaaagttt 1080
taaaagttta tgttttttaa gtgttatttt agttttggag ataagttttt ttgtgtgttt 1140
tttagagttt tcgtttttat gttttaggag tagtgttggt atttggaggg tagtagtagt 1200
atttagtgt taagttttgt tatgttgaga tttgtgttta gtagggattg gaggttgtta 1260
gtgtggggag gttgtttatt tatttgtcgg ttatttacga ttttattttt aagttgttat 1320
ttttgttgag attttaggta ggtatttaaa ggaaattttt atttttttta gtttattcgt 1380
atagtaatga attacgtggt aggtagatgt ttttttttat ttgtttttat gagttttagt 1440
tttagatggt tttgggtagt tgttttgatt tgtttagtta ttttaggagt tgatacggta 1500
gtggtttttt gttttgtagt gtagaaggtt gggtttagtt atttgttttg gaagggattg 1560
```

```
ggttttaagg ttttttttg gagaattgag ttgttgagtg aatgtttatg aggtattgga 1620
gaggggagtt ttatgtagta tcggtcgttt ttggtgttaa cgaagtttag gtttttatat 1680
tttgggtggt acgttttagg ttttttttt agtttagttt tagggtaagt ataggttagg 1740
gtagttatgt ttttattttg ggttagggtt ttttttgttg tgaagggatt ggattaatta 1800
gagggttttt aagttttttt ttaattcggt ggttttatta tatttagtgt ggttaggtgg 1860
tttttaaggg ttgggttaag ttatagagga taggtttggg gtacgtggtt gtttttgacg 1920
agatgaatag gaatgtttta tattggtatg tttaatttta gatatttttt gggtgtgttg 1980
ggataggtag ttatagttat agagtttttt aatagacgtt ttttttttt aagtttttgg 2040
gttgaaattt gtttgatttg ggtttagttt agttagagag tagacgtatg gaggaattga 2100
gtgtcggggg ttattgcgga agagtttttt taaagttttt atttttttt tttgtttgtt 2160
ttggagttat ttgtttgttt tttttttgt tttgattttt ttgttttttt ttttggttta 2220
ttttggagtt atttggtttt ttttttttt gttttgattt ttttatgggt gagaaagatt 2280
taaggtgaaa gtttttttt tttttatttt tttttagtta tgagtatttt agaaggaaag 2340
gggggagaaa tggggaaatt gttttagtt ttaagttttt ttttttttg gttagatttt 2400
gggggagttt gggttatttt agcgaatttt agaattagaa aaaatttttt aaaagttatt 2460
ttaaattttt aaatatttga aatatttaaa gttaagtttt taagaatatt taatttaatg 2520
tttaagattt ttttattttt ataagattta ttttagagtt acggtttata aagaggtttg 2580
atttattaag ggaggttata gttagatttt tttttagttt ggtttttaga gtagatgaga 2640
atattaggta ggcgggagat tgttattttt aattttagag atttttattt aataatatta 2700
agtttatttt tcgtttaatt taggaggttt ttttgtgttt acgattttga gtatagtggg 2760
tttggtttta ttttatgata ggaattaagg gagaaggtag aaaaggcgag cgttttaatg 2820
cggggcgttg tttgttggag ggatttttta tttggggttt gataggtttg tttttggacg 2880
gttattgtgt atttttagtt gaggtgaggg ttggggttta gggttagatt taagttatag 2940
gggtcgggta ggtaggtata gagtttcgtt ttttggaggt aggaagtttg gttttaggag 3000
ggtattttga tagtttttatt tgtaatagta aagttatatc gaggagttta ggttaggagt 3060
tttttcgtag gttggagaga gggtagagag ttttggtttt tgtggaagaa gtgtaaggac 3120
ggagtgtgga aagttgtgtg gagaagagta atattttttta gtgtttggag ggatatagag 3180
tttttatttg tcgtggaagg ttttgggtag cgatgatatt gatagatatg gtagggagga 3240
tagttagttt gaggttatag tagggtttgg aggtcgagta tggttatacg tttttagta 3300
aagtttgtac gtagtttttg atttttttgtt attcggtttt taagttttag tttatgtgtt 3360
tcgaagttgt gagttttttt atgtcgggat attgttgagt agttttaggg aggggttagg 3420
gtagggtagg gaagagacga gttttttcgt atttttttgg agggaatttg attgggtttt 3480
ggatggagaa taaagttgtt tagatttttt cgttgtatta gatgttaggt aggtcggaga 3540
gcgggaggat tgttcgggtt tacgttttat tcggatgttt ttttttttt taggattttt 3600
tttttttta gtattatggg agtttttgtg gtatttgtgt ttttagtttt ttttaggttt 3660
ggtttttttt ttatagtgtt tagtgagggg ttggttcgtg tttttttgg ggattggtat 3720
tgttagatgg tagtaggggt tttcggtttt agtttttgtt attttttgtt tatttttttt 3780
aggtgtattt agtttagggg atagttagga tgtagttagt taggttttgt tagattcgtt 3840
attgttcggg gggaggtgga ggttggcgtt ttggttttag taggtcggat ttcgtgtgtt 3900
tttagggaga gaaagatggg agggtaggag gttggttttg tagggtagtt tgtgttttag 3960
atatatagtt atcggtttgg tgattagggg attttattag gagcgggaag gatggataag 4020
attgggttgg tgaggttgtt gggtttgtat ggaaatttag tattgattta tggttaatta 4080
ggtagagtag aggagtgggc gggagtaaga aagcgttttt agtttattat ggatgcgaag 4140
ttgaaaaacg tggttatttg gtttttttta ttgtttgtgg tcgggagtag gggttagggt 4200
tttagtgtta gggaggggtt aggggtttgt tgtttttttt tttttagtag tttacggggt 4260
tttagatttg gggcggttga aagtgttgag ggttaggttt ttggtttagg ttaggttttt 4320
aagaggaaag tcgagtggga ggttgggggt ttggaaaata tgggcgtggt ttatggtgtt 4380
tcgaggtggg gacggggtag gggtgttttg ttatgtggtt taggaaatgg tcgtattgtg 4440
ttttttggtt tttatgcgga gggtcgcgat gttcgaggtt ttgcggttcg gttcgtcgtt 4500
gagttcgtag ttattgaggt ttgggttgag gttggttgtt ttaaataggt tgtttatgtg 4560
cgtttggttt acgtgattgt tagttttaga tatatttagg aagtcggtga cgttgttggt 4620
tttagagtta ggggggtggg tatgagggga tatgtaggta ggtatcgggt cgtaggggat 4680
tacgtaggtt ggtattggtg aggtagtttc gttgttgtcg agttaggacg ggttttgaat 4740
ttgggagagg aagggagaga tgttatcggt tggcgggtag gtgtggtgtg gaaggggttc 4800
gtttttttt ttagagtatt ttttttttta ttgtttattt tttttatgaa gttttttttg 4860
agtgtttagt tttgatggat atttagatat ttttgtgggg gtggttttta tattttggta 4920
tttttgtttt tagggttagg ttagtagtag agggaaattt agttttaaag agtgatttta 4980
ggttttatta agggtttagt ggaagggtga tattttagta ttttttagtt ggttttttat 5040
```

ttttagattt aattgtagat ttggttttta tttgcggatg gtagttgtgt tttatttatt 5100
tttagttttt ttttaggttt tttatattta taggtatttg agtaaggtga gtagatttta 5160
attcggagag ttttggtttc gtaggttttt ttgggttttt attagtagta gggaagatgg 5220
aggggttggg tgaggtgggc ggtagggatt taaatttttt agaaattaag ggttagtggt 5280
tgaggtttta gggtttgaat taggaggttt tagaattaat ttaggatgga aggggttgtt 5340
ttaggagtgg atagttagat ttgagatcgg attttttcgt gttaaagggg cgagggtatg 5400
tgtagggatg agttggttat tttatttttt atttgtgtta ttgggttttt tttttttttt 5460
taacgtttat ttggggtttt tatttttttt tattttttag gtacgtataa ggaggtagaa 5520
ttagggtgtg ggggagattc gggaattttt tagtttatgt atacgggatt tttggtaatt 5580
ttatgtgggt ttttgttggt ttgttttttt gtatgtgttt ggagtatatt cgagggaggt 5640
atttaaagt agggagaaag agtttagttt ttattttgga gggaatcgta tttttttttg 5700
cggtgtaggg gaagggggat cggttggttt ttattatttt tttttagaag aggtttgagt 5760
atatattatg tggtgttgta ggtgaggtgg gggtaggagg ggtgaatagg gttttgtgtt 5820
gggttttttt tttagttgtg cgtttaggta gtagagttta aagttaagga gggagttaga 5880
gtttatttt gggaggatag agggaggttg agtttaggga gggagagggg agaaagagaa 5940
tggaagaata tagtgcgagt tgatgttgat ggtgtttgtg tgagatattt tatatttttt 6000
agttttattt ttaggtttgt gttttgtttt tttgttttat gaattttggg ttgggggaga 6060
gggaatagag ggttggtttg gttggagagt tagagattat ttgttagtat agaattaggt 6120
tagggagaag taaaggaggt aggggcgttc gtgttattgg ttggttgttg agtagggtta 6180
ggttgggagt tgaggtggag gttatgttag aatgaggttt gagtaggtag ggagtagtcg 6240
tttttgttgg gtaggttgag tgggggttag gagaggattt ggatttggtt aaaaatatat 6300
tttttaagt attttatgtg tttgatgtga tttaatattt tttatttttt gtattttata 6360
ttagaatagg ggattggttg atagatattt agggaaaatg ttttgggggg aggggaggat 6420
agggttttag aagttgagta taggtatata agttttttga aattgtataa tttataaaat 6480
atttttgtat gattgaattt agattttata attgtttgat aatagagttg agtagatgtt 6540
tttattatgt ttaaggtata ggtgaagaaa ttgaggttta gagatttgag atttgtttaa 6600
agtgagaaag atttggagtt ggaaagataa tttaagtttt cggatttaga attttaagtt 6660
tattttaatt tggttacgag ttttggagat tttagagttc gttttttgaa gagatttttc 6720
gagcggtatt tgagggtttg gtggtttttt gtttgttttg gtttcgatgt ttatagtaaa 6780
agtggttaaa gttagtggag gaaagagtcg ttttattgt tttaattttt tttttttaa 6840
agtttagtta agtttggttt aatgattagt tggatagatt aattttgggt tttttatatt 6900
ttggtataaa taggtatatt tttggaatga gacggaaggg tagtttggag ttataagtta 6960
tttcggggtg tttgggtttt tttttaaggg gtttattttt agagtattag gggttgggga 7020
tcggtggcgg tagtttaggg cgggatttat ttgggcgtag tttttagttc gggtgaggag 7080
gggtagttta tatgtagtgg agaagtgggt tcgaatttgt tgtatttgtt taaaacgttt 7140
tcgttttttt tatttggttt ttcggttttg gaattagatt tataagacgc gaaaaagtta 7200
ttgttattag ggtgagatgt tcgtttggag gttttatttt taggtagtgt aaattgtttt 7260
tttgagtttt ttattagttg tagtgcgttt gggaaacggt gcggttatat ataaatatta 7320
atttggttt ttgtttttaa gagttgataa tttagaatta tttattttga gttgggaagg 7380
tattttattt ttattagtta tgggcgtgat tttgagttag aatttttatt tttttaagtt 7440
tgagaattta gagtagttaa gtgttattat ttataaaata agttattggt tattttagta 7500
gttatttaag tagattttttg tggagaagtt gataaaagtt ataaatgttt ttttagatgt 7560
gtttttttag ataattatat atagttttgt tatagttttt taaaaggttt atggtttttt 7620
aaggtttatt ggtttagatt tataatgttt tttttttgt attttgtttg tagttttttt 7680
gttggagata gttttttagt ttagaatttt tttgtgttaa agggagaata ggggttttgt 7740
tgggtaatag aggtagttag ggaagatttt ttggaggagg gataggagtt gggttttgaa 7800
tggttggttg aaaggaggaa agttaaggtt taggtttaga aatagtaagg tatattttaa 7860
aaatttaaag aggttaattt gaaggaagcg gtgggagaga aagaaataga gttagatcgt 7920
agggggtttt gattgttaag tgaagggttt ttttttttggt gttgtttgta gaggtagttg 7980
ttataaaatt ttgtgtagaa gcgtggtttg atggtgagag gaagtggagg ttaattcgtt 8040
attgagtata agtggatggg atgttggttt gattgggagt ttaacggggg aggggaggtt 8100
tttgagttag ttgtgagtat tggggttttt ggtgacgggg agtattgggt tttttttttt 8160
tgtacgagta tttttggaag gttgagaatt tttttaaggt ttaagagtaa gagttgtttt 8220
atttttggtt tatgatttag gatttggaga aggggtgatg gggacggggt gggtgagaga 8280
gggagagtga ggagggatta gtttttaggt taggaatggt tttaggttga tttagagatg 8340
gatcgtggtg ttttttagag ggaggttttt gttagtttga tgtgtatttg tttgttggtt 8400
ttgtagattt agatgtaagt gggtatattt tagatagtac gttgtagatc gggagaatag 8460
agttatgata atagttttag ttgtagagtt ataaggagtt ttgttaggtt tttttaggtt 8520

```
taggtattaa gaaagttaat ttttttgagg tttagttttt gtttttagg tggaaatatt 8580
tttttattta ttacgattaa tgtagttgtt gagttaaata taggagttag tttttaaaat 8640
ttttttgttt tagagttttt gagtttttta ggaattattg cgaggagggg agaaggaggt 8700
gtggacgtgt tttttttatag acgttgtttg gttttttttgg gtatttagtg tagttggtta 8760
atggataggt ttataggagt attggtattg ttaagggttt tagtttttgt atggcgaatg 8820
gtattttata gtcgtagtag gggagataga tgggaaatgt agtttaagaa tgtttagagt 8880
atttttttatt tgttttttta tagtttttga ttttttttat tgtttttttgt tagaggaaag 8940
aggttaagga agatggggat gtattttatg ttattgtgag gagggtgggg gtgagtaggg 9000
gtgtatatta ttatgttttt ttatttttgt attaatttat tttttttttt ttaattttaa 9060
gtatgtgtgt gcgtgtacga gtgtatttat tagtgtggtt atacgtacgt gtatgtgtgt 9120
atatatattg attgggggag gagaagtttt tttttttagaa acggattttt tttttagttt 9180
ggggttttag tttttgtttt ggttttttag ggtttttttt tttttaggga tttttagggt 9240
ttaggttttg gttgtttatt gggagttaag gatagatggg gttttttttt tgtgttgttg 9300
aaatgggatt ttttttttatt agtggtttgg gatgggagat tggggttagg atgagagaat 9360
gaattagagg agtttgtttt tattttatgt ttaattttttt gtaggtttag tttcgttttt 9420
tcgttttttt tttagttttt tagggttttt tgtgggggaa gttgggggga ggggagaagt 9480
tattggaggt acggagagag atggtttttt tttttttttt tgttattgtg attgttgggg 9540
tttttttttgt gtttttgtgg gttttaggtt tttatggtgg gattgtggaa gtggaagatg 9600
tttttatagg ttttggaata gaggatttag ggatgggcgg ggagggattt cgaagaggga 9660
aggaggtggt tagcgtttgg taaaggggtt tagagtggag ggaggagtta tgtgggggag 9720
tggaatgtgg ggcgcgggtt cgttggagtt gagtgtgagg gaggaggaga tgtgggttat 9780
ttggggaaga tagatgatta ggcgggggag aggttagtag agttagtatc gtttttggaa 9840
taggagtgga agaaggttag gttgagggat ttatttgagg gttgaagatg gaaatggtga 9900
gaggcgggtg agcgatcggt gggagtaggg ttgtttattt tttttttttt tttttttttg 9960
ttttttttgtt ttggggtttt attttgcgta tttagttttg gtttttttgt ttagatttag 10020
atatgagtta ggtgagtttg gtcggttttt aatatttttt ttagtaaatt ggaggtagta 10080
agggtatttt tacgattttt tttatgttta tatttgtagg ttttgaggtt tggagtgttt 10140
atttattttt gatttttggc gttttagggg tttcgagagt ttttttttag atgttatatt 10200
aatttttacg ttcgtttttg tgtttttttag ttttgtatcg taggtttgtt tttattgtgt 10260
agtttagga ggatagttaa ttacgaggtt tttattgttt tttcggtttg aatgttatat 10320
tttaggatta ttttagtttg ggtttattta gtataggttt ttttcgagga tcgggttgtg 10380
taaggaatta attgttttgg cggaggtttc gttaatcgta gtttatttgg ggttagggcg 10440
gggtggttta gggcggggag..gtttttgttt tattgttttt agatattagt tgtagagatt 10500
ggaattttag atagtagaga gaggtttttg tagtttttttg gagtagggtt aaattttagg 10560
ttttttttta ggaaaagttt attttggggt ttttattgtt tcgtttttgt ttttattatt 10620
attttacgtt aggtatttgg tttgttatta gttgagtatg ggttttgaat aaatatagta 10680
gaggtaggta gggggtttta ttattatttt tttatttttt tagggtattg ttaggtttag 10740
ggtttaattt agaggtttga cgtatatgaa gattttattt attttgttgt ttttatataa 10800
agaaaagttt ttgaataatt tggtatgtgt ttttgtatag taaattaaag ttaaagaagt 10860
attaggttgc gatttaggag tttcggattt tagtcgtagt gttgttattg agtggttgtt 10920
gttaatttta ggttatgatt ttttatttat gggttttttt tttttcgttt attaaatggg 10980
aagatggggt gattattttt agtattatcg ttttatagtt ttgtggtttt tttattaggt 11040
gataagatat gtagtacgtt agagtttttt tttttgtttt ggttgttagg aagttttaag 11100
ttgaagtttg tgtttaatgg taattatgaa ttttagttta aatttttttt tattatatta 11160
tttttgtatt ttatgttatt gtttttgttt ttagattgaa taaataaatt tttaattgta 11220
agttatttta attttatttt ggaagtaggt ataagtgtgt gtgtgtgcgc gcgtatgtgt 11280
gtgtgtagag aatattttgt tttagttgta tggtggtaat tgaaaaatgt tttgtgcgtt 11340
tttgttttta tgttttgtat ttaaggcgtt attttttttt tagtatttag tgaattttat 11400
tttcgggtat ttttatatat ttgttaggaa gtatgtatgt aatttatata tgtaaatata 11460
aatgtatata tatattgtat agaaaaggaa taggtttatt ttatattaat atagtattta 11520
tagggttgtt ttataattag ttatgttttt tttttttattt atatttgtgg gtttttttta 11580
atttatttgt tggagattag gttagatgtt aggattttttg tttaaattta ggtatgtaag 11640
gttatgtgag tggttggttg tttaaattta agtgagattt gggtgtgtga gggtttttgg 11700
atttatagga aagttttagt tgttttgtta tttttttatta aatatttatt aggtattgga 11760
ggggaataaa atggtagttt tgttaggtgt agtggtttat gtttgtaatt ttagtatttt 11820
gggaggttga ggtggtggat tatttgaggt taggagttga agattagtta attaatatgg 11880
tgaaatttta tttttattaa atatataaaa attagtcggg tgtggtggtg tatatttgta 11940
atttagtta tttaggaggt tgaggtagga gaatcgtttg aattcgggag gtagaggttg 12000
```

```
tagtgagtta agattgtatt attgtatttt agtttgggcg atagaataag attttgttta   12060
aaaaaaaaaa aggtagtttg ggttgggtat ggtggtttac gtttgtaatt ttagtatttt   12120
gggagattaa agtaggtgta ttatttgagg ttatgagttt aagattaatt tggttaatat   12180
ggtgaaattt ttatttttat taaaaaataa aaaaaattag ttgggtgtag tggtgtatgt   12240
ttgtagttat agttatttgg gaggttgagg tatgagaatc gtttgaattt aggaggtgga   12300
ggtcgtagtg agttaagatt atattattgt attttaattt gggtgatata gtgagatttt   12360
gttttaaaaa ataaaaaaat aaaaaaaaaa tggtaggttg tttttaggga atttatagta   12420
atttaatcgt gtagtttttt agtattattt ttggtagttg tagttttga atagttatat   12480
atttattgat aattattagt tttttttat attagttatt ttggaaaaaa agtttgtaga   12540
ttttattaga gatgttttg tttttttaaa tagaaaatgg ttgtaattga tgaatttttt   12600
attgatgttt tagaataatg ttgttgaaga tgtttttatg agttaattta agagttttg   12660
gggtttgttg aggattgtag ggttggggta tttttgtttt aatattgaag ggtttagttt   12720
attgagttgt atggcgtttt gttgattaaa aaaaaaaaaa aaaaaaagat tttttttgt   12780
tttgaagtta ttgatttttt tggtttattg gagagggaaa aattgttatg tggtaacgtt   12840
ttatttgtta tttttaatat gttttgggtt ggaaaagcgg attgattatg atatttattt   12900
tgtgttaaat tgaaatttaa atttgagtgt gtgaattttg ttatttgtat tttggttatt   12960
tatgggtttt tttttttttt ttgatttttt ttttgttgtg aatttatgtt ggtcggggta   13020
gatatagttt tttggataag ggttgattga tttacggata tttatttttt agatttgatg   13080
ttttgcgttt ggtttttttt aattggtggg aatatagtga tgaataatta gtgtttgatt   13140
ttaaggaaat tatagtttag taggagggga taaataagta aaataacgta agttagttaa   13200
taattttgta aagaagataa aataggagtt gggtatagtg ttttatgttt ataattttag   13260
tagtatggga ggttgaggtg agggattatt tgaggttagg agtttgagat taatatggat   13320
aataaagtaa gatttatttt ttataaaaaa taaaaaaatt tattttaggc gtggtggtgt   13380
atgtttgtgg ttttagttat ttaggaaatt gaggcgggag gatttattga gtttacgaag   13440
ttaaggttat agtgagttat gattatatta ttgtatttta cgttgggtga tagagagaga   13500
tttagttttt ttaaaaaaaa taaataaaaa tatttaaaag ataaaatggg tttaggtatg   13560
gtggtttatg tttataattt tagtattttg ggaggttgag gtgggatgat tattttagtt   13620
taggagtttg aggttgtagt gagttataat tattttattg tattttagtt tgggtgatag   13680
aatgagattt tgtttttaaa aagaaaataa aaaaaattta aaaaattttt aagaagataa   13740
aataggatag tgtaataaaa ggtgagttgg tttgtttttg ttgtgttggg ttgggttgag   13800
ttttattggg ttgtgttgag tgggttgagt gtgttaggtt ttattgtgtt gtgttgtgtt   13860
aggttgagtt ttgttggaat gtgttatatt gggttgtgtt gtgttggaag ggttgtatt   13920
aggtttagta tgttgagttt tattgtgttg tgttgggttg agttgagttt tattgggttg   13980
tgttgtgttg ggttgggttg agttttattc ggttgtattg agttgagttg agtgtgttag   14040
gttttattgt gttgtgttgt gttaggttga gttttattgg aatgtgttat attgggttgt   14100
gttgtgttgg aatgggttga gttgggttga gtgtgtttag ttttattgtg ttgtgttggg   14160
ttgagttgag ttttattggg ttgtgttgtg ttgggttgta ttgagttggg ttgagtgtgt   14220
tgagttttat tgtgttgtgt tgtgttgagt tgagttttat tgggttgtgt tttgttgggt   14280
tgtaatgagt tgggttgagt gttgggtttt attgtattgt attgggttgg gtgttttggt   14340
tgttggttgg taatagggggg gttattgttt tagttatagt ggttaggaag atttttttaga   14400
ggaggtatga gttgggattt gaaaaatgga gggggatgta gttatttagg tagaaggaag   14460
agttaaattg tttttgttt ttgtttattg ggatgttgtg gatattgttt ttatgtttgt   14520
tttgtttttt gtttaagaga taggtttttt agagtaggga taagtaattt tttttgtgt   14580
gaaagagtta ggattttat attgtgtaaa gtttagaaat agttagtagt agttttttg   14640
ttttttgtt ggaggtttt ttttttttgt aatttttat tatagtgtag aagtttaata   14700
ttaaagagga taaaagtaga gtcgttttag ttgggatagg ggtatgaggg gtaggttagg   14760
agtttttatt tttaggtttt taatgaattg aggaaaggaa agttatggtg tggttggtgg   14820
gtagttagga gattttaatt gtagttaaga gtttagggat aggttttgtt ttattagttt   14880
tatttttagg tggttttat tgatttgtac gcgggtttta gtgaggtttg tttttatggt   14940
tagttgtttt cgcgtatata cgtttgggta gtgggttttt tggaagattt tttttagttt   15000
ttttagttgg tagttggtga aggtggttcg gtttcgtcgt tttttgtttt tgttgttttt   15060
tgagtcggtt ttttttaatg ggttggggag gtttgagttg gttcggtttt ggggtttttt   15120
tattttagtt tttttgatat ttaggtagtt gttgtttatt tttatagtgt tagagttagg   15180
gggtaatttt ggtttattta gggagttttt tttagttgag ggaggaggaa ataaagttag   15240
aaattaatgg ttgaattaaa taagagaagg ggaatgttag ggggagagtg gggtattcgg   15300
gtagttattt tttgtttttt ttttttaga gtttattgga tgcgaggttc ggagatttgg   15360
tttttaattt tggtttgtta ttaatttgtt gtgtgatttt gggtaaattt ttttttaat   15420
ttgagttttt ttattattat tttagattag gttttttta gtaataatgt tatgtgaatt   15480
```

```
taaggtagtt aatttgattt ttgtgatttt taatttttat tttattgatt ttgagttttt    15540
tttttagag ggattttttg tattaggatt tttttggggt tcgagaattt tgattttagt    15600
gttatggttt ttttgggtgg aagggaaatt tatttagtag ttatgtttgg tttagtgggg    15660
tttggggtga ggaggattta ggattgaggt agataggagt gttttttttt ttttggttta    15720
gtttattag gagtttagta ggagttgtgg agattttgtt ttaagagttt agtgttgagg    15780
agttaatatt tttttttttg tttttaatt ttgtatattg gttttattta ttgtttaaga    15840
gaagggtttt tttttttgg gggtgtatgg gattgaggta tgggttatat tttttgttat    15900
aagtagtttt tgtttattgt ttattgaggt tgttcgggta tttttatttt tagtacgttg    15960
atttcgggtt tgggtgggtg tatatgagta tagtttttag ttattgcgtt tggtagatgg    16020
atttttattt tatcggtata tttttagttt aggattgtaa ggacgagtta atttatatat    16080
attaagaaag aagaggtgga ggttggggtg ggagagtgag gtaagaagtt attttattat    16140
tatatagttt ggtgagtttt ttaggtagat atatttattt tttggtagag ggtttgggat    16200
aatagttttt attttttgg tattagggat tggttttgtg gaagataatt tttttatgg    16260
gtggagaagg ttgtggtggg gtatggtttg gggatgaaat tgttttattt taaattatta    16320
ggtattagat tttttaagg agtacgtaat ttagattttt tatatgtatg gtttatagta    16380
ggatttacgt ttttgtgaga atttaatggt atcgttgatt tgataggagt tagaatttgt    16440
agtaatgttt gtttatttgt cgtttatttt ttgtgtgcgg ttcggttatt aataggttat    16500
ggatcggtat tggtttatag tttgggggtt aaggattttt ggtttaggtt atggattttt    16560
gatagtttcg ttatgaattt tatggttatg attttttagt tgtgtgttta ggagagttta    16620
tttattcgtt atgtgtttta gttttttttt ttataaaatg gggatgaaga tatttttagg    16680
attttttta tagggtagtt gtgggaaata aagaggttag tgtgttagtt atgtagtttt    16740
gttattattg atatttatat ttttagtttg gatttgtaaa tttatagatt ttgtgaatat    16800
tagaggattt cgatttttgga gaagtttttt aagtatgtag gtttttgggt ataggagtat    16860
gtgtagggcg tgtatagatg tttagggaag ggtagttaat tgggtgtttg tagatgtggg    16920
tgtgaatttt tcgagaattg tattttttgt ttgtaatttg gggaaaagga atttgttgag    16980
tgaaagaata aatataatta ataagacgtt tggggagtat ttgatacgat tttttacgtg    17040
tacgagtttt gattattata ataagtacgt gtggtaggtg tgagtattag ttattttgat    17100
tcgcggtttt tttttttgga aataggttta agcgatgggt attaagtgtt agaatgatgt    17160
ttttagttta ggttttttga ttttatttag attttgaggg ttgaagtttt agattcgttt    17220
tatttttggt tttttgtgta tattggggaa gttgagaggg atatttggtt tgtggtattt    17280
tcgtttttt ttttaaggta ttaaggaaag ggtatagagg tgagtgttta gagtaggttt    17340
gaaagtagtt ttaggaggtt gtttttgagg gttattggta gtgtagacgt tatgggggtg    17400
tacgatcggt ttagcggtat tacgtatgat atttgtgtga gtttaggtat atgtagatag    17460
ttatggtttt agtgataata ttttgtaaat tgtagttggt agttgggtta agagttttta    17520
gtaaagatat ttattttttt ggtagataaa tatattttat gtttaggtt ttttttttt    17580
tttttagaa attttattta gttttataaa aataattatt tttgttttgt aatttttgg    17640
aagttagatg aggggttgag ggaggtgagt attgagtttt gagttttgat ttttaggttt    17700
tattttatag ttttatagac gtttgttggg gattgggatt attttgtata gtaatgggag    17760
ttttatgtta taggaattga gagttggtat ggattagttt gaatttttag ttttaattgg    17820
ggggaaattg aggttagaga gagaagggaa tatgttttaa tttcggatag agtatagttt    17880
tttagtttt ggtttgtttt ttattttttt ttttgcggtt ttaggtttta tttttaggga    17940
gagaggtagg ggtagaagga atttaggggt tttatatgat taaggcgttt tgttttgttt    18000
attttgtggg gggtaggatt agtttttgag gatgagggag tgaggatgcg gtttgggggt    18060
agtttttag tattgagtta gtttttggtt ttttagggga agtggtagaa ggtttagttt    18120
ttatggtgat aagtaagggt ttagttttgt ttattttag ttgtttattg agattaagtg    18180
agtaggtaga gatagaggtg gtatagggtg tgcgaagatt attaggttaa gagttgggag    18240
ttttggtttc ggttttcgtt tttttagga ttttagatga tttattttat ttttggagtt    18300
ttatttataa atagggagag cggggtagtt gagggttttt tttttttgt ttattagtag    18360
ttaggattgt ttttgaggat ttggggaggg gaggagaggg ggagggtgtg tttgtgttag    18420
agatggaagg aatttagggg agcgttgttt ttggtttttt aaggaagaag gaagtaagtt    18480
ttttaatttg ttttgagtta gttttgttta aattttgagg ttgggttttt tatttagata    18540
ataaagaaaa atttttgaa gttttgtagt tttatttaat tggttagaat aataagtata    18600
gtttttttag agtttgagtt ttaagatttt aagtttaggt tgtttatgtt tatttttcgt    18660
tattttgtag tatttttatt tggcgttggt tttagtttag ggtagtaggg agtttattgt    18720
ttttaggata gtagaattat ttatttttag atttagttta tgtttgtttt ttgtaggttt    18780
ggtttattga ttttagtttt aatttttag cgtttttttt tatgatagtt ttgtttttgc    18840
ggatattggg aggttattgt tattttttg agtttttttt tttttgtag tttttattgt    18900
tgtttttcgg gtggttttga gtttttcgg tttgtgtgag atagagggtg gtagggatga    18960
```

```
tatgaggagt ggggttgggt tttgggtaaa gttagtagat tgtttttttag ggagatgatt   19020
tcgggttggt tggagtgtta gtatggtagt tgaaggtttg ggtgttggtt agatttgatt   19080
tttcgtaagg tgtttttttt agtaagacgg ttgttgaaaa tagaagtttt atggtttagg   19140
ttgattgttt ttgattaaag gaaattattg attttggttt tttaagtttt tatttcgggt   19200
ttttgtttgg tatgtttttt tttttagttg atttttaaga tttaatgttt ttttaggaag   19260
ttttttttaga ttgtgattgt tttttatttt ttgtttattt tttttattga ttgtttatgt   19320
ttgttgattc gtaattttat taagtttcga atgtttagtt ttttttatag aatgttagtt   19380
ttttaggggt tgggatttta tgtcgtttgt ttttttttag tttgaggttt agtataggat   19440
aggttaggtg gtagagatgt agtaagaggt tgttgaggaa taaatgaata atttttaatt   19500
aataaatatt attgagtttt cgttttgttt aggtatttgt gtgtattttt ggtgagtcga   19560
tgttttatag agtatgtgta gaaaatgttt ttcgagtaaa tgaattaatc gttttatttt   19620
tttatttatt ggggagaaag tcgggtggga tatattgaaa aagggtacgt aatgacgaat   19680
gttattattt aaggttgata gtataaatag taaaggttat tgcgggagat aggagagaag   19740
aaagatttag atagatttg agcgatgttt ttatgtagag agtaagggat agaaggagaa   19800
gtagagacgg gttttatagg ggcggtttta gaattgaaat taaggtttag aaatttagtt   19860
taggatttgt taggaattgg ttttatattt attttgttat tatattgggg tagagaagat   19920
cgttagggag tttttagttt gtttggatgt tgggtaagtt ttttttagag ttttggggta   19980
tttcgtgttg gtataaggtt gagtagtaat agcgtttagc gtgggtttag gttattcgtt   20040
aggggttgga tttttttatat tttttttgga gtagtttttt gtggattcgg aaaggtttta   20100
tgttgatgtt tttttatga tgagtttgtt ttagggttga ggttatgggg tttaagataa   20160
tgtagtttta atttgtagtt ttttagtttt gatttggaag taaatttttg attatttaga   20220
gttattaatt aagtagtgaa tttatattat tgagagtaat gtttgtttta cggagggtgt   20280
tgtgtacgta gtgtggttat tatggtattt agtgtttttt aaataatttt aggttagtat   20340
tattgttata gattgttttt gatgaagtgg gaagtattta gtttaaggtt gtatttttaa   20400
tcgggagtta ttgagttttt taatttttgt tggaattttt tttgggggga ttttcgtatt   20460
attgggagtt gtcgtttta gtgttgggaa tttttggttg ttggatttta gttggagaaa   20520
tgtatgaagt ggaaagtttt gtttcgtttt tttaattttt atttatttgt tttggtttaa   20580
tttaatggta ttattttaat ttagttttat ttttttaaaa taggaaaatt tttgagaggt   20640
ttttaaaggt gattgatttt tttttttttt tttttttttt ttttttttgg tcgtgattta   20700
aagaatgatt ttttagagtt attttaggga gagggtgttg gtagtatttg tatgtataga   20760
tgtatattcg agggtttgtg gtataagttt gttattgtga gtgggtgtgg ggaatatatg   20820
tgtagttgtt tgtggatagg tttgtggagt gtgtatgttt gtgtggttaa gcgtaggtat   20880
ataagtggga tgtgtgtttt cgtgggtagt tgtgtgtggg tgtagattcg cgtgggtggg   20940
tgtggatatg ggtggtgtgg gtttgtgtgg agtcgtgtat aggtttatgt atatggcgtt   21000
aaggggtttt agttatttta ttttttatgg ttttgattat atttattttt tttttaaata   21060
tttgttttgt gggggatatg tcgtttgttt tgttttcggg tgagttggtt ttatatattt   21120
tgtggcggag tgtatatatc gatgtaattt ttttaaaaaa aataggttag tatgaggttt   21180
ttttatgtta ttgaaagggt attgattaaa tttattgttg tttaaacggt tttgtggggg   21240
tgagggcgga ggttaaagtt tagttattga ttacgttttt attttatttt atttgatatt   21300
ggggtttgtt tagagtttaa tttgaatgtt tttaatttag ttttagtttt aataaataat   21360
gttaaagggg tagtagtgat tgggtttaga ttttagtata tatgtacggg gtatagttgt   21420
ttgtagagat agatagtgtt tcggttttta aggggttgtt aatttttagt agagggtggg   21480
gtggaaatgg tagtttttag gttagttagt ggtataggtt attttgttta tttatagtta   21540
ttaatttaag ttttgaaatg ttttcgaaat aaggaaagtt tgagaaattg ttttagatta   21600
gaggaggtta aggaattatg aggattaagt gtaattaatg tgttttgggg ggaatgttgg   21660
aatagtaaaa ggatattggg ggaagttagt gaaatttgaa taaagtgtgg agtgtagtta   21720
atagtaatat ttaataaaaa gtgggtaaaa tattttagta gatattttat ttaagaagat   21780
ttatagggag taaataagta tgaaatatgt ttaattttat tatttattat ggaaatgtaa   21840
attaaaagta taatgagata ttattatgtg tttattatag tgggaataag atttgatgat   21900
gttaagttac ggcgatgtat ggagtattcg gaatttttat ttattatagt gtaaatattt   21960
gggcggtgtt ttatttagtt aaattaatat tgattttatg atttagtaat tttattttta   22020
ggtatttatt ttatagaaat aatgatttat tttaagagga gaggaattta aatttatagg   22080
taaatgtttg tagtagtttt atttagaatt atttcgaaat ggagatattt ttaagtgttt   22140
tttgataggg gaatggagac gtaagttgtg gtttatttag gttatggatc gattttatag   22200
tgaaaagaaa gaattgttga taatgatatg gatgaatttg aaaggtggtt ttagggtttg   22260
taaataaagt tagttttaaa tttatatgaa gtgtaattgt atttagttag tatatgaatt   22320
ttgggaaagg taaaattacg ggatttttag gtaatagatt attggttgtt aggtgtagga   22380
ggagggggaa ggtttgttta taaagggttt gtattggtgt gatggaatta ttttgtattt   22440
```

```
tgattgtggt agtgaatata tgaatttata tatgagtgtg ttaaaattta taaaagtata   22500
tattaaaaag ttttttatag ttgggcgtgg tggtttgcgt ttgtaatttt agtattttga   22560
gaggtagagg cggatggtgg attatttgag gttaggagtt tgagattagt ttggttaata   22620
cggtgaaatt ttatttttat taaaaatata aaaattcgtc gggtatggtg gtagttattt   22680
ataattttag ttatttggga gggtgaggta ggagaatcgt ttgaatacgg gaggtgtaga   22740
ttatattgaa ttgagattat gttattgtat tttagtttgg gtaataagag taaaatttta   22800
ttttaaaaaa aaaaaaaaaa aatttatagt atgttgtttt taaattttaa aaaatgtaat   22860
taaaaagaaa atataaaatt gtgtttatat atataaaata aaaattaaat taaaagttta   22920
tatgttttta tttagaaatt ttatttttag gaatttattt aataaatgat ggtaagtatt   22980
tattagagag aacgtttatt atgttatagt ttaatatgta ggaaaatcga tagtaattaa   23040
aatatttaat aataggggat tggttaaata aaggatgata tttttataaa tggaagattt   23100
tttttgatag tttttatttt gtacgttgtt tttttttttg agatagagtt tcgttttgtt   23160
atttaggttg gagtgtagtg gttcgatttc ggtttattgt aattttattt tttagattta   23220
aacgattgtc ggatttcggt tttttgagta gttaggatta taggtatggg ttattatatt   23280
tcgtttattt ttgtattttt agtagagacg gggtttttttt atgttgttta ggttggtttt   23340
gaatttttgg ttttaaatga tttattcgtt ttggtttttt aaagtgttgg gattataggt   23400
atggattatc gcgttaatat taggtattta tttgtttttt tgtttttttt ttgtttattg   23460
tttttttttt tttttttttt atggagtagg gaatatattt gaatatgtgt gttttgttta   23520
ttttgttgtg taatatttag tattatagtt ggtatgtagt agatgtttag taaatatttg   23580
atgaataaat gaatgagatg atgttaagaa aggatagcgt tttaatatat gtaaagatgt   23640
ttaatttata ttaaataaat tttattttga ttaaaaagag gaagggagtg gagtatttgt   23700
ttttagaaaa tattataagt ttttagaagt ggtaatatgg gtttatgggt taattttgtt   23760
tttttttttt ttttgggggg cggggcgggg ttggtttgaa ttaatttttt atttgttttt   23820
ttaggaatag gttgaaatat ttgaaagtag tgattagaat tttggatggt gtatgttttg   23880
agagcgggtt gggttttgtg ttgtgggatt atgggaagta agagataagt attatttgga   23940
gagatttagt taaattggaa ttttagataa atagttaata ttttaaataa tataagtata   24000
ttttaaatat ggtataagaa atatttttat ttttataaatt atttattatt taaatgtaga   24060
tttgattgag taatttggtt ttgttgtttg tttgtttgtt ttttgttaaa tttgttaatt   24120
ttgtatatta ggtttatgtg gtatttaggg tttcgaaggt aggattggtt tttattagat   24180
ttacggattt atacgttagt tttttattta gtttgaggtt tagagatttg ttttcgtttt   24240
gtagcgtatt gtacgagtat ttttaggttt tcgtattttt gcgggtattt ttttttttat   24300
ttagtatgtt tttttgtttt ttttgtttgt ggttaattat tattttttatt tggatagttt   24360
tttttgtaat ttttgtgatt tttttttcgt attataaatg taatttatgt tttatatgga   24420
agattaaaaa aaaattaaga tagattaaaa gaaaattatt tatatatata tatatatata   24480
tatatatata aatatatata gagaaaatat tattttgtaa atttttgtgt ttttttttga   24540
gatggagttt cgttgttgtc gtttaggttg gagtgtagtg gtgtattttt ggtttagtgt   24600
aatttttcgtt ttttcggttt aagttatttt tttgtcgtag tttttttgagt agttgggatt   24660
ataggtattt agtattacgt tcggttaatt tttgtatttt tagtagagat agggtttcgt   24720
tatgttggtt aggttggttt cgaatttttg attttagatg atttgttttt ttgggttttt   24780
taaagtgttg ggattatagg cgtgagttat cgtgtttagt ttataaatat tttagtaaaa   24840
tttaattata tttcgtattt ttttttatgt tgttttatat tttttttatta tattattttt   24900
aatggttacg tagcgagaat aattattatt ttatttaatt tattttttttt tgttgggttt   24960
ataggttttt atttgtttga aattatagta ggtttgtgat ttaaattttt taatatatgt   25020
atggcgattt ttttaggaga agttttaaga attagaaata tgaagtttaa ttatttatat   25080
atttgagggt ttttgagaaa tacggttaag ttgtttttta gaaatatgta atgtttttag   25140
ttattacggt gttttttttt attttagagt taataatggt tattatagtg tttttaaaat   25200
ttttgttagt acgatggatg aaagtgagat tttgtttttt tttttttttt ttttattttt   25260
taattatgga atattttatt ttttgttttt ttttgttgtg aatttttttt tttgggtttt   25320
taaagtttag ttaagggtta tttttttttt tttaggattt tttttgtttt ttaaatattg   25380
aattttttgt tttatagtat cggtttatgg gtttgttttt tttttagatt gtgagttggt   25440
tagagtgggt tttttagatg tattgttggg gtttgggttt cgtgtgagta agggtttagg   25500
atatattttt ggaattaatg taaatgtttg tgtgtgtttg ggttgaggga gattttttttg   25560
ggtttttttt gtttttggtt tggttggggt agttttgtag gtatagttga gggtggttag   25620
tgtgtttgta ggaaaaggtt ttaggttttg gagttgggtt ttagatatag gaggtttttg   25680
gtttttttgta tatattgttt gtgttaggag gttgttgtat ggagtcggaa gaagggattt   25740
tttaggatga ggggggtcga ggaaattagt tatagcgtag ggtaggaagg attaaagatg   25800
atttcggtag ttttttggtt ttggataaat tttttgtttt ttgtttgttt agtttaataa   25860
tattgtatga atttttgtta gggaggggta tgagaagagg agatggaggg gtatagtttt   25920
```

```
gtgggaagtt aagattgaag aggagttttc ggttttggtt gtttatttag aaggttaagt   25980
atttaggttt tagggtttaa tttttagtag gttaagatta agattgtagt tgtgtatgtt   26040
ggttattagg aggtattgta gttttaataa agttattgga cggaaggtga tgattttggg   26100
gttttagtgt ttaatgaggt ttttagacgt ttgtttgaag atttaggttt tagttgggag   26160
gatttgttat ttaagtagta atttagtttt attacgcgtt tgttattatt ttagtattat   26220
aagggataaa gagatggatg gggcgggtcg ttggtttttt ttgtaggggt tttttttttt   26280
ttagttttgg gtatttttat atttttgttt tattttagtt tttttagaat ttttatattg   26340
tggttttagt atttttattg gatagacggt taaagtgagg cgtcgagaga agggatttat   26400
tagtgttatt agtgattgag tgatagatag gttgagattt gggttttttt ttattttttta   26460
agtggttttt ttttggagat ttatggggat tttttggttt tttagagagg gtggcgtata   26520
gtgggtgttt aggaaattga ttaatttgta aaagtttttg ttttgtaaaa gtttttttagg   26580
atatttttgt cgtttggttt agcggttttt tatgtggttg gtagatttgt tattagtttt   26640
ttcgtgggtt tttttcgagg taggtattcg tcgaggtagg gtttgttatt tcgtttttta   26700
ttgtttcgtt tttatataga agttttttta gtttttttttg taatggtagg ggtgagtggg   26760
gaggattgtt tttttttttag tttagagatg gagtgggttt tttagagttt aggaacgaaa   26820
agggattaat gttgggtttt cgtaaaaata aaaaataaat aaataaatta aataaaatta   26880
ggaattgggt tttgtatttt ataaagtatt agggtattta ttatgttatt agtagggttt   26940
tagtttttttt ataatcgtta ggttttttatt ttatagtttt agaaattgag gttagagggg   27000
gtaaatgttt tgttaagaat taataattgt taagaaggtt taggggtggt ggtttatgtt   27060
tgtaatttta atattttggg aggtcgaggt gggagagtta tttgatttta gagttttatt   27120
tagtttgggt gatagaggta gagatttgtt ttaaaaaaaa aagaaaagaa aagaaaagaa   27180
aataaaaaat tggtaagagg tagagttgag atttgaattt tatgtttgga gtatgtttat   27240
tgggtttttt tggggtggat gttgtgggtg ggattgggat aggtagatag gagatagggt   27300
taaggaggaa gggaggaagt tataggagaa tggttgtttt tagttgtttt aagagattgg   27360
tttttttgta ataattttta aggttgtttt tgattaaatt gttttgttga agattaattt   27420
tattgattat ttgggatttt agatgtggat tagtgttttg ttttagggtt tatatatatt   27480
gaagtattta gaggtaaaga ggtatatgag ttatatgttt gtaatttatt tttttttttgt   27540
ttgtttgttt ttagataaag ttttgttttg ttgtttaggt tagagtgtag tggtataatt   27600
ttgattaatt gtaattttttg tttttcgggt ttaagtgatt ttttatgttg gttaggttgg   27660
tttcgaattt ttgattttag gtgatttatt tttttcgatt ttttaaagtg ttgggattat   27720
aggtatgagt tattgcgttt agtttgtaat ttattgttaa atgatatagt tgtatatcgt   27780
atgtatgtat attaatatat atataaatag agtatatgta tagagagaga tatagagaga   27840
gatttagaga gattgaaaat aatgtaaata tggtaaaata ttattatttg gggagttagt   27900
ataaaaggta tttgggaatt ttttgtagtt tatttataat tttaagtgta atattgtatt   27960
aaaataaaaa ggttttttaaa agaaaaggta tgtggattaa tggaaagaag gtattttttt   28020
taggaatttt ttttgtgaag ttggtatatg taattatttg aataggattt ggatgagttt   28080
atttggtaat tttttcgttt ttttttttatt attattatta tttataattt tataaggggt   28140
tttgtacgtt gtaataaaaa ttataagata aatgttaatt ttgggagtat atattgtttt   28200
ttagtttttt ggaagttttt attttcgtgg aagttattgt gatttaatta gaatatgggg   28260
ttttttttaag tagttttttt gagagttgta ggagattaag gttaagttat tgttggaatt   28320
taaaagttta aaaagtattt taatttttga gtttattgta attttaattg ttttatagtt   28380
agtttaaatg agtaaagttt ttttgtataa aaaaaagaaa tggtggaaag aaagaatttt   28440
aaattttgtt tagtttttag tttttatggt tttttttgtta aggtttgggt ttttttttatt   28500
ttttagtttg tgtttggaat ttgttttttt tgtttttttt tttattttgt gttttgtttt   28560
attgatttat ttagagtttt ttttaggaat tatggtttag tggaagagat gtatgaggga   28620
taagtacggt taagtgtttt agggttttta gggtaaaagt tttgtttttt agatacggta   28680
gggtttatag aaaggagcgg ttaattttgg ttgaaaaggt cggagaaggt ttttgtaatg   28740
gagtggtttt tgaattgggt attgttagtt aaggaaggtt cggtaaggta tgttgtattt   28800
tatggtaaaa taagatatta agatattatt tttttatttt aaataggggta aattttaaat   28860
tattaagttt tttagatagg agttttttaa ttagggttga ttataattat gatagtaata   28920
atgtaatgtt ttgtattcgt gttgttttga tttatttatt attttttttg atttttttta   28980
gattttatga ggttagtgtt ggtatttttg ttttatagat gagaaaattg aggtttagtg   29040
aagatgaata atttgtttaa gattatatag ttaggagggg tagattggat ggttttatgt   29100
taagtttatt gtttttttaag gtggtgatga gttatttttt taatatggag gggttaggta   29160
ggttttattt tgttttaagg gggtattgtt gagttattgg ggttattttt attttaaagg   29220
attagggtaa attaagtttt agggttttta tgagaagttt atgtatgatt taatatttgg   29280
agattatttt ttgatgtttt ttggggtggg atgggtgttt tagttttagt ttgggtttta   29340
agtgtttttg ttagtttttt tttttaagga ggttagaagt ttttcggttt ttcgttttat   29400
```

ttttaagttt tattttatgg cgtgggtggt tggatgagat tttgttttcg gtaggattta 29460
ttgttagaga aatagagttt tttatgtttt tttttatttt agtaatacgt tcgtagggtt 29520
tagagatttt attggattag attttttttt tatagttaag atgcggaggt ttagagtaag 29580
gttatattgt ttgataatgg aagggttagg atttgaattt tgtatttttt gtatttagtt 29640
ttgtggtttg tttgtttatt gttttttta agtttttaa attatgtggt tttgggcgtg 29700
ttttaagata tttttattt aatttttta agtttgtt tttttttgt agtagaaaga 29760
attttgtagt tttattattt ttattaagat attgtaatat tatttttatt tagaggttgg 29820
cgagatggag gagtttagat tttgtacgag gtaaaatgaa atgattgaga agtttttggg 29880
gtaggatagg tttgggtttg gtttttggtt ttgttattta tttgttatat tgatttattt 29940
aatagtattt ttggagtatt agttatgtat tgggtaaagt tttaggtggt gaaaatataa 30000
agtttagtaa aatatagttt tttttttaa atggttcgtt ttagtgatag gggttatggt 30060
tatagtgtag ggtggtgatt agggtagggg tatttagttt agttttgggg cgatttaga 30120
tttttgtaga ggaggtgttt tgaagtagta atgttgttgg agaggtttgt tgaagttgtg 30180
tttagttaac gttttatata agattaggga atgttagttt ttttatgaa agaaatggga 30240
gtcgattaaa ggagattatt aaggaggttt ttaagttatt ttgtggtttt gttatagaag 30300
ggtattttg gaaaagtgat ttgattttg tgtgttttag ttttttatt tgtaagatga 30360
ggttgagaat atttatattt tagaatggtt gtgaggtttg attgaagtag agtgtaagac 30420
ggtatatttt ttgatttata tacggtattt attttattaa tgggacgttg ttatgatata 30480
ttaatatagt tatgatttgg ggatgaattt ggtcgtggta gttgatagtt agggtaacgt 30540
tgtttagttt gtaattgtgt tatttttatt tgtttttttt tttaggtgta tttttttag 30600
tttaggtttt tcgagcggta gttttagaa tgatatttat gaattataaa tttttgttaa 30660
aagtagtttt tagggttttt tttaatgaag ggtttatttt atatatatat aataaagatt 30720
ataaatgggg tgaaatattg gtaattgttg taaaaattaa atgttaaatt tatttggtta 30780
tatagattta aaaaaatttt taaagagtgg atttacggta gtttttgttt ttagattgtg 30840
ttttattaa gggtagggga ggggtgttac gaggtagcgt tattattcgt tttgtttgtt 30900
gttattgttt ggtatttaag tacgtgttga attgagttaa ttttaaataa ttgaattttt 30960
ttttttttt ttttttttt ttggagatag ggttttgttt tgttgtttag gttgaagtgt 31020
agtggtgcga tttcggttta ttgtaatttt cgtttttgg gtttaagcga tttttttgtt 31080
ttagtttttc gtgtagttga ggttataggt atatgtcgtt atatttggtt ttttgtgttt 31140
tagtagagat agggttttat ttttgttgtt tcggttggtt ttgaatttat gagtttaagt 31200
aattcgttcg ttttagtttt tcgaagtgtt aggattatag gtatgagtta ttgcgtttgg 31260
ttaaataatt gaatttttta gtttgtagaa gtagtatatg atttagaaa gaatgtgttg 31320
tttttattaa tattaaattt tttttttt aaatgtgttt ttttttaat ttttatttat 31380
gtatttattt atagagacgg agtttcgttt tgttatttag gttggagtgt attgatgtta 31440
tttcggttaa ttgtaatttt tgttttttag gtttaagtta ttttttgtt ttagtttttc 31500
gagtagttgg gattattagt attggttatt gtatttggtt aatttttgta tttttagtag 31560
aggtggggtt ttattatttt ggttaggttg gttttgaatt tttgatttcg agatttattt 31620
ttttcggttt ttttaaagtgt tgggattata ggtgtgagtt atttcgtttg gtttaaatat 31680
atttatttta atttagagaa ttatttataa attgaaaaag taataaggtt ttttttttt 31740
tggatgagaa agggaaagta atggtttaat taattataag attgtttttt ttttttttgt 31800
tgtttaggtt ggagtatagt ggtataatta tagtttattg tagttttaat tttttgggtt 31860
taagtaattt ttttttttta gtttttgag tagttggtat tatgggtata taatattata 31920
gttggttaat taaatttttt ttttttttt tttttttgta gagacgaggt gttattatgt 31980
tgtttaggtt ggttttaaat ttttggattt aagtaattgt tttattttag ttttttaaag 32040
tgttggaata taggtatgag ttattatgtt tagttttagt ttgtatttta ggaaagttat 32100
taattttttt aaagttgata agaatgggaa agattttta atatttgttt tttaaaaaat 32160
ttatatttat aattataaaa tagttaataa tttaaaatat ttaaaggata attatattta 32220
aaagttttgg ttggtgtttt tgtataaatg attgggattt ggagtagatg ggtttttagg 32280
atttgttta ttatatatag taaggataaa atataaataa ttaatgtttt agtatttata 32340
attaatatgt tagttaaaaa tggttttttt ggtttcgtat ggtagtttat gtttgtaatt 32400
ttagtatttt gggaagttaa cgtgggtaga ttgtttgagt ttaggagttt gagattagtt 32460
tgggtaatat agtaagattt ttatttttat aaaaaaaatt tttaaattag ttgggcgtgg 32520
tggtataagt ttgtagtttt agttatttag gaggttgaag taggagaatt gtttgagttt 32580
aggaggttgt ggttgtagtg ggttatgatt tagttattga attttagttt gggtgatagt 32640
gagattttgt ttgagagaag gaaggaagga aggaaggaag gaaggaagga aggaaggaag 32700
gaaggaagga aggaaggaag gaaggaagta ggtaggtagg gagggaggga gggagggagg 32760
gaaggaagga agaaaatggt ttttttattg atttttgttt atttgaataa aataaaaaat 32820
ttaaaaaagt ttttttttta aaatgtatga tgtggttagg tgtagtgttt tatatttgta 32880

```
atttagtat tttgggaggt tgaggtgggc ggattatttg aggttaggag ttcgagatta 32940
gtttggttaa tatggtaaaa ttttatttta aaaaaagaaa aaaaaatata tatataatgt 33000
atggtgtgtt ttttttatta agtaaatttt aatttatagc gaacgtgaga taaatatatt 33060
ttttttttat ttgttattat tttttggtag gatggatttt ttgtaacggg atagattttt 33120
tgagaggata ttttgtgtag tataaaattg ggttttgtgg aatttttttt tatggggtta 33180
aatttggggg gatgtattaa tatagtagag tttgggtata tatatttttt gaggaggttt 33240
ggttttattt gtttatttgt tatatggtat aagtaatatt tttaaattgg ttttttgttt 33300
ttggtttcgt taatattggg ttagtgagga ggttattatt cgttttgttt agaagggttt 33360
gttttttttt ttttatttta taggagttgt ttttatatat tatatggggg ttttgggttg 33420
ggttggtttg gtttttagga tggttttgga gggaggaggg agtggtgttt gggcggttag 33480
gggatatggt tattatttta gtagatgggc gaagatggtg tagtttacgt ataatatgta 33540
gtgtgttgaa aatttttttt agtgtatttt ttttatcgtt tatagagttt ttatatgttg 33600
gttaagtttg atgggtatat aggtatgagg gtatttaggt agtgggtatg agttttttgg 33660
gtaggggagg aaaaaggttt tttggtttta ggagatttgg gtttaattta tagcgggata 33720
gttttatttt ttttattttt aaattgagga tattaatatt ttttagtggg tttttataaa 33780
aagttttagt ttaggtgtgg ttaagggtaa tgtttattag gtgttttttt aattgtagat 33840
ggggtttagg aagttggggt ttaggaaggg tgtagaggtt gtttgatatt ttttgagttc 33900
gatttttttgt gtttagtttt gtgttgatta gagaggaata tagtagaatt tttgttttgg 33960
ggataggttg aagttttggg ttaaataaat atagaaaata tatttagtag ggagagagtt 34020
atttttttagt ttatgatata tgtgtacgag gtgtagtagt ttatggggag gaggggtttg 34080
aggggtttgg gtggttttttg ttttttttttg aagtttgttt tcgttgcgtt aggtagagat 34140
ggtagttttc gtttttttta ttatttattt atgttttaaa agataatgtt aattatatag 34200
taattataga tttagtaata attttttttat tttttaattt tatgtatttt agttaatttt 34260
aattgtttgt taaggttttc ggttagtttg atagagttaa ggttttagta aaattttatt 34320
aattataata gttaatattg attgagtatt tattatgttt taggaattgt tttaagattt 34380
ttatttattt atttatttat ttatttattt atttatttat ttagagatag agtttcgttt 34440
tttttgttta ggttggagtg taatggtgtg gtttcggttt attgtaattt ttgttttttg 34500
ggtttaagtg atttttttgt tttagttttt ttagtagttg ggattatagg cgtttattat 34560
tacgtttagt taatttttttg tatttttagt agagataggg ttttattatg ttggttaggt 34620
tggttttaaa tttttgattt taggtaattt atttgtttcg gtttttttaaa gtgttgggat 34680
tatagatgtg agtaattata tttggttttg tttttgttta ttttatttta tttaagtttt 34740
atttattat taataggtag atatgatttt tatttttatt ttataggtaa agaaattgag 34800
gtaaagaggt taattaattt gtttaaggtt atttagttat gtgggttgaa atttgttttt 34860
aggttttgag ttttatgggt tttttttaaat taaaggtatt tgaagtatta agtagtttgt 34920
gtagtataat tgtggaatga atagtttttta tgatttttta agaattttag aaattatgtt 34980
ttagggtata tggttggaag attaggttta agtaggtttt tgtttagtta tttaggtcgg 35040
ggaagttttt aggaagggga gtcgggggata gttatttttt tgtagttgtg ttttttttgg 35100
atttgttgt ttagtttttg gagttttagt ttttggttgt gttatttatt ggatggttta 35160
gggtagggga tagggagttg taagtgtttt ttttgggaat ttaatagaaa atttttaaaa 35220
tatgtatggg gttaatagta tggagttatt ggatgttttt tttttggagt tttttaagt 35280
ttcgttggaa ggattttgga attattttaa taaaggaagt ggttttagat gtgcgtattt 35340
taggaggtgg gtgtatagat ttttagagta taaaagaatt tttttaaagg ttacgttttt 35400
atgggatggt taattgaagg ggtagagggg ttttcgaata tttaggggtt tagttagggt 35460
taagattgtg gttttagagt tttcgttttt ggttttgttt tttatttttt tttattagtt 35520
ttaggtttcg gaaaataaat gtgtttttga gttgaatttt tgtgatttgt tggttattgg 35580
tttgtaggta aggagatgaa cggatggttt ataagggttt ttgtatattt tgtaattttg 35640
gattttatat tagaagatta tttaggggtt agatagataa atgtaataga atagagatta 35700
gatgtaaatt tttaaatata tgcgtaattg atttttaata aaggttttaa gatcggtcga 35760
tgagaaaagg gttatttttt ttataaatgg tgttgggaaa agtggatatt tttataagga 35820
agaatgaagt tggagagttt tatgttacgt tatatttaaa attaaagtta ttaaagattt 35880
aaatttaaga gttaaattat aaagttttta aaagaagata taggcgaaaa ttttttttgat 35940
attggagtta ttgacgattt tttgaatatg atattaaaag tatagataat aaaataaaga 36000
aattgaaaaa ttgtattttt ttattaaaat gtaaaatttt tgtgtattaa aggatagtat 36060
ttagggagtg aaaggataat ttatagaatg agaagaaata ttcgtaaatt atgtatttga 36120
aaaggaatta atatttagaa tttttatgat tgaagaataa ggaaataaat aattttattt 36180
aaaaatgggt aaagtatttg aatatatttt tttaaaggag atatataaat ggttagacgt 36240
taaaggagaa tattgtatga ttttatttat aagaggtatt taaggttagg tgcggtggtt 36300
tatatttgta attttagtat tttgggaggt tgaggcgggt ggattatttg aggttaggag 36360
```

tttaagatta gtttggttaa tgtggtaaaa ttttattttt attaaaaata taaaaattag 36420
gtaggtgtgg tggtgggtgt ttgtaatttt agttatttgg gaggttgagg taagagaatc 36480
gtttgaattc gggaggtgga gattgtagtg agttgagatt gtattatttt attttagttt 36540
gggtaataag agtgaaattt tgttttaaaa aaaaaaaaaa aaaaaaaagg tatttatagt 36600
aggaaaattt atagagagag agtagaatag aggttatttg ggtgggggt taagggtgtg 36660
atggcggttt tcgtttaata ggtatggagt ttttgtttgg gatgatgaaa aagttttgga 36720
aatagtggtg atggttatat aatattgtgg gtgtatttaa tgttattgaa aggtttattt 36780
gtagatgtta aagtggtagt attttatgtt atgtatattt tattataata aaaagtttat 36840
ttttaattaa aataaagatt ttgaatgtaa aaagtaaagg ttatattgaa ttatgtaaag 36900
aatatttagt ttggggtttt attgttttgt ttagagtttt ttagtggttt ttttaaagtt 36960
tgaatttttt ggtagaaaat aaatggttta ggggttaggc gcggtggttt atgtttgtaa 37020
ttttagtaat ttgggaggtt gaggcgggag gattatttga gtttaggagt ttaagattag 37080
tttgggtagt atggagaaat tttgttttta ttaaaaatat aaaagttaat tgggcgtggt 37140
gacgagtttg tagttttagt tatttgggag gttgaggtgg gaggattatt taagtttggg 37200
aggtagaggt tgtagtgagt tgagattgcg ttattgtatt ttagtttggg tgatcgagtg 37260
agattttgtt ttaaaaaata ataaaataaa ataaaataaa ataaaaagtt tttttagtt 37320
gtgtttttgt tattttttag tttattttat cggtatgtat tatgttttg tattttatat 37380
atttgtttta tgtttttttt ttattcgaag atttagtttg tgttgttttt tgagtttaga 37440
gggtttttt tattttttt agttggtgag tttttattta atttttaagg tttatttcga 37500
atgtttatgt ttaggaagtt ttaatttatt tattttttt ggggattttt atggtatgta 37560
gtatatttta attaggttat gggtttttg agggtaggag ttagtttatt tattgttaaa 37620
tattttggtt tttgtttgat atagaagtgt taggttgaag gatttgttgg atgtaaggag 37680
gagtttttgt taggcgtata tttttttatg tattttttt gagttgtttt tttttttga 37740
attttggttt ttatttgtaa aatggagatt taatttttt atttaggttt ttggtgagat 37800
ttaggggatt atggatgttt aagtgttttt agattgtata tgtcggggtg ggacgggtgg 37860
acggaattat gggtgtgttg ggaagagttt aggttttgga gtcggatgtt ttgttgggaa 37920
ttcgggtgag gttagatggt attttggtta gaagagaggg ttgtattttt tgtaggtgag 37980
ttttagaatt ttagggtgga ggatgagtga atgggggatt aagggaggaa ttttagtagt 38040
ttttaggtag tattttgggt aggtggtagg aatgggggtg ttagcgggta ggatacggaa 38100
tttattgtag ggaatggggg agatttgagg aggagagttg gggatgaagg gagaaaatta 38160
tttggttaga gtcgatttcg ggggttgtgg tagttagatc gtaggtttgt ggttagtggg 38220
gtgagattta aaagaggagt agtgaggaaa aggggtattt tttcgattgt tagagttgga 38280
gaagattaag atttgtgtta aatagagtta tttagagttt cgttgttttt atattttata 38340
ggaaattttg taaaaaaaaa aaaattaata atttttttatt agaatgtaag agaacgataa 38400
agtaggtttt tattttaggt ttttatttaa aggaaggatg aacgaagaga gagtggaaag 38460
atttatagag tttttagtat gtttttttt tgtaagattt ttttatttat ttatttagta 38520
atagatattt attgagtatt tattatatag attagtttta ggttttgggg aaagttggaa 38580
ttaaaataga taaagttttt tgcggttttt tatgtttttt agagggtatt gtttttatag 38640
atgtttttta gttttatttt attttgatta gaggtttttt tatgttttg aaatttttgg 38700
aaatttatgt agttattttt tatattagtt atgaggtttt atgttatgtt tgggatattg 38760
tttttatttg gtataggtga ggaagttgtg tttaaggtta tagagttagg tgtagaattt 38820
taatttgata ggtgtcgaaa tttatttttg atattatttg tttttgtat tttcggttgt 38880
gaagttgaga agaacgtttt gggggttatg gttttttttt ttttttttt ttttgtgaga 38940
tggattttgt tttgttattt aggttggagt gtagtggtag gatttcggtt tattgtaagt 39000
ttcgtttttc gggtttatat tattttttg ttttagtttt ttaagtagtt gggattatag 39060
gcgttcgtta ttacgttcgg ttaatttttt gtatttttgg tagagatagg gttttatcgt 39120
gttagttagg atggttttaa ttttttgatt ttgtgattcg ttcgtttagg ttttttaaaa 39180
tgttgggatt acgggtatga gttattgtat cgtgttttgg gggttatgtt tgtaagattt 39240
tgttttttat ttttatatta tttgggatgg aattagggtt attttaagtt taattaggat 39300
tattttagaa taggatgttt tttaggtttt ttggagtttt cggttagtaa gattcgattt 39360
tttttttttg gttgttttat tgtggacgag ttatttattt tttatgaatt ttagttttta 39420
tttacgaaat ggggatgata atagtattaa ttttatagga ttgtgatggt gattaagtga 39480
aattatgtat aagtagtatt ggtgtattgt ttggcgtata ggtagggacg tttagttgtt 39540
ttgggttgtt ttatggtttt gtagtagtag ttaattttaa gttttttttg ttttaaaatt 39600
tattattgtt tttgttttgt agatgggaaa atttaggtta ggagttaggt ttggtatttt 39660
agatttttaa tttttggtag attggattag tttttttttt tttttggtat aatgaatggg 39720
gaagagtgtg gggttttgag gtattttgag gatttggag ggttaattta tttttttttgg 39780
tgaatattat agaattttaa ggtttttgaa attattgagt tttatgaaga aggtttagag 39840

atggggaggt tgaggttata ataggggagt tatgtagtag agttatgtgt atgtttgttt 39900
tttttatttg aaagtgagtt ttttttttga agttagaggt tatggagttg gagttgtttt 39960
atttttatgt tgggatgtga gttaggtgtg aatggatttt tgtggttata tagagatatc 40020
ggggaggttt atatatttag gaggatttta aattttatat atatatttta aaatttgatt 40080
agaattttat tattattatt tgtaatattt tagtattttt aaagggtata gtgtgagggg 40140
tggggtagag aatttggtgg tgtaggatgt acggggtaga tatattattt tttttatta 40200
ttatattttt tttatatttt ttttttaggt gggatagggt taaagtgtta tttaatatag 40260
ggagaaatgg agtggtatta gagaagtaag ggggttgtta tatatgatta tttttataga 40320
aaaattaaga gaaagtatgg agggagaaaa ggagaaagtt aagagagaag aagtttttagg 40380
atttttagtt attagagaaa atttattggt ttggataaat ttttaatttt tttaattttt 40440
ttgtattttt tttttaagtg gtttttagta agttattaaa ttttggtggc gtttgtacgt 40500
tttagggtaa gttagtttga tttttttttt agagtttgag ttagggtttt ttttatttta 40560
gggtttattt tattgaattg gaattttaag gttatttgtt tgtttttgtt tttggatttg 40620
aaggtagggg ttgtatttag agtttttttag aatattttat aaggtttggt atagtgatag 40680
gtaaagtttg gtgagttttg cgtaaatgga attaagtttt ttagtttttt ttgtttgtga 40740
aataagatat gtgggttagg agatgattgt tatttttttt aattttgagt gagatttttt 40800
aatatgttat tatgatttat tgtgtgtgtg ttgtagggtt aggggaggta ttagtttttt 40860
cgatgtaatt aaaggttttt agggtttggt ggatagtttt atatagtggg aggagttggt 40920
tatttggttt tagttgattt tggtttgtgg tttttaagaa tttaggaaaa gaaaatttag 40980
tttggggagt tgggtagaga gtaggagttt tagtttggtg gggttgtatt agagttaggt 41040
ttttgtaatt ttattaggga ttttacgggt gttttttttgt tgttttttaa tgtttaggag 41100
tttatggcgg ggtttgtttt atttttaagg agattgaggt agaattgtaa gtatttgttt 41160
tatttgatgt atagagagag tgtaatttat tggatttatt gtaggagtcg tatttaataa 41220
gagagcgggt gagtggattg gatttttttaa tatttttttgt tttttttattt attgtagtgg 41280
aaggaggaga attcggtggg tttttgtttt tttattaatt tggttgtggg ggtttatttt 41340
tttttgggtt ttagtttgtt tatgtgttaa atggggttag tagtgggtat agatagtgtt 41400
aaggatacgt ttttttcgta gtgagttata cgggatagag ggtatatttt agagttggtg 41460
gtttgttaga cggtttcgtc gtagtgtggg tgggttggat ggattatgtt cgagggtttt 41520
aggatgacgc ggggggtggt tgggttttat cggggatttg ttatagggag attttgtttg 41580
ttattcggtg agttagttgt ttttgggagt tatgattagg ggtaggaagg ttataggtta 41640
tgatgtaagg gcgtttagtt atttttttttg ggggcgtttt tggaggtaga ggttatgtta 41700
tttatagatt tagagaagtt ttagagtcgt gtatacgggt atttattttt gtatatttga 41760
ggttcgtata ttgggggata tagatataag tgttagagga tagatatgtt ttttatcgga 41820
ttatatataa attttcggtt tcgttttgaa atatttagat agtaggacgt ggatttagag 41880
agaatgggta cggtatatat cgtttatatt tatgttgtgt atacgtttat ataaatattt 41940
aagttggttt ttgggtgtat ttaattatgt gggggtaata ttgtaggaga tattagtttt 42000
ttatttattt ttttagttag cgaatatttg ttaagtttaa ttgtataaaa tatttggaag 42060
gatataagtc gtaatatagg aggtagggat ttggtatgtt ttattttttta ggagaaggtt 42120
cgtttaggga aggtgtttta taaatattgg gatagggaag atgagtggat ataggggtta 42180
tgagttcggg gttttagttt gagtttggtt ataaagatat ttgtttattt tttttttattt 42240
tgtattttag ttttgagttt agaatttgta ttttattatg gttttgtagg atttttagtc 42300
ggttttgtgt tttgtgatgg tttttttattt aggatttgtg ttagtttttta gggaagtttt 42360
ttttatttat tttattttag ttttttttgg ataaaaatgc gtgtgggggt gaggagggaa 42420
gataaagggg gtgaaggtgg gtttagattg ttattagtat agaaatttta agagggtttt 42480
tggaaattgt taatattttt gtattttata tagttaaaat ttagatttcg gagtagaata 42540
tattttttta aggttatgga ggagtagaga atgtaaaggt ggaggttggg gtgatttata 42600
tcgtaattgt gggtttcggt ttttacgtgt gtcgcgttgg atattagtgt tatggtttaa 42660
atttttttata gtttttttagt ttttttttgtt gtggtttttta gagtttttgga gagttagttg 42720
ttttgttttg atttgggtta ttaggttttg tttttttacg aggtgcgtgg ggttaatatt 42780
tatgttgggt ttttagttta gaatgaaaat ttatttttaa agaatttgtt ttaagaattc 42840
gatgaagtaa gtattggttt ttttttttgtt tttaatgtgt agcggcgtta gtgtttagta 42900
gttttgatta agcggtttcg agtataagcg gcgtttttat cgggaaagat aggtatttgg 42960
tagtcggtgg tttttttttat ttcgttcgta tttttagtg atttttttttt tattaaaacg 43020
gtattttggg agtcgcggag cgttacggtg attttttttaa ttgttttttgg agtttaaatt 43080
cgaatcggtt cgcggttttg agtattattt tatatatttt agttttggtt ttttagagtt 43140
ttgtttttgc gtggtttttt ttcgtcgttg agttataatt ttttttattt tttttttttt 43200
ttcggcgttg gttggtgcgg gttggggtta ggtggagaag tcgttttttg ttaaggtgat 43260
agaacgtgtt gggggtgggg gtcggggtta gggtcggtgt aattaggggg tcgttgtttt 43320

tttttggata tagtggaagt ttttttcgta ttattaaatt tttgttattt tttttgaggg 43380
atttgttttt aggtagtacg taagttgttg tttcgggttt atttcgtatt tttttattgg 43440
gtgaggaagg agtattttga atggagatgg gggtgttttc ggtttatata tttgtagaga 43500
agaggtgtgt cgggttgtat ttttggaggt cgcggtaatt gatattagag aagatttcgg 43560
ttgtagttgg gaaggtttat tggttggaaa gaggtgtttt tttttttag taaagggttt 43620
tgtttggaag ggttgttttt tatttgttta gtggtattat aggacggtcg gtttttattc 43680
gaatttttc ggacggtatt attatatagt cgggttttcg tagtgttggt tttttaattc 43740
gatgattgtt atttcggtga ggatttgtgt tgatggtcgg agaattttgc gttgcgggcg 43800
tatatggtta ggtggcgttt ggtaggcgac gttcgggtgt aggacggcgt ttttatcgtt 43860
ttattttaaa tcgttgtttg ggtttaggtt tttcggtttt ttgaataggg gtttgggggg 43920
ttaaggacgt tgaggtttcg gggtaggaa gtttttttg gttaagcgtt tttttttt 43980
ttcggtatat attttttat ttatttattt cgtttatttt cggggcgaga ggtttattaa 44040
ggtagggcgc gttttttta tgaattattt taaggttttt gagtcgcggg ggtttcgggt 44100
aattattttt ttttttttt ggtttaggt attttagttt aggggtttgt agagaagttc 44160
gaagttcgga taaacgcgtc ggacgttaat aattttttat ttttggtagt agtaaaggtt 44220
aatatatttt tattttttat tttagtttgt tattaaaata aagttgcgcg cggttgaggg 44280
taggaaggcg ttgagatcga gaagaaggga cgtttcggag aaagtgcgtt tagttgattt 44340
tagaaattag agtttttcgg gatttcgtcg agattttttg tagggcgttt taatttgttt 44400
ttttattgcg tgtcggcgtc gtagcgcgtg cggtttaggg tttggtgatt tcggtttagt 44460
tcggcggtcg cggcgaggtt tttggcgtag tcgtttggaa tttcgtatta gaatcgggat 44520
cgcgtaaatg ttttggttga agtgttattt tatttaagaa atattgttgt taggaataaa 44580
atgggttttt cggtgtttcg aagtattttt tgaaattttt ttaaaataat ttataaaaaa 44640
tgttttgtt ttaacgtttt ataacgttta aggaaatatg taaatggttt gtttttttat 44700
cgagatggtc gttttaatta atagtgtata tatatataat aattttttta attttttttt 44760
ttagagttaa gtattttatt atatgtaaat tataataaag aaaagattgt gtaagattat 44820
gtaagtcgat tgatttaaaa tattgagttt taatttaggt tttttgtttt tttatttaat 44880
aatttttgtg tttggattag attggtgaag taggttatgg aaattaataa agtaaaaaat 44940
taaaagtatt ttttttcgtt attttttttt ttaaaattaa ataatagtcg tttttttttg 45000
agtaggtttt agttttaggt tcgagttttt ttgcgattat tttatagtta tttatagtag 45060
ttgttgttgt ttttgtcggg ttttcgtttt tgtttttttt gggtcgtttt ttgtatataa 45120
aatatatttt agtttttaa ttaaatttaa atacgatttc ggtagaattt atatatttcg 45180
tggtgtatgg attgtgtcgg tgtaggggaa ataaatattt tttggtattt aattattgag 45240
tttaattcga aaaatcggga ttgggttttt aggcggtatt ttaggggttt taatttggtt 45300
cgcgtttttt tagattttgg cgttgagagc gttgtttttg cgggtgggtg gacggagagg 45360
taataatttg tttttaataa aaatttgtcg ttatcgaatc gaaagcgaaa gggaagggag 45420
aagaggggta gtttttcgta gcggtttcgc gtttcgggat aggtagatta ggattagtac 45480
gcgtcggttg tagtttcggc ggtcggattc ggagtttggg ggcgaggcgt cggagttttt 45540
gttttgagta tagggaggtt ttttgcgtcg ttgagaaacg tcggattttt aattcgatat 45600
gtttttgtg gtttaggcgg ttcgttttgg attcgggtaa ggaatttagg aggttaaaga 45660
gcggatttag agtttggagg ggagaatggt gaagttttgg gttgtaatag aagagggttc 45720
ggtgggggtt ttttcgtaaa aacgcggttt tcgtatttaa aggatattgc ggagtcgttt 45780
taatcgtcgt ttttttagta tatttgggga aaggaaggtt taagttgggg tagggacgtt 45840
tgtagttcgg gagggttttt tttattaatt tttttcgtaa ttcggtttag attcggttag 45900
tagagattgg aggcggtttt ttcgtttcgt attttttcgg tttggtttag gtttagttgt 45960
tcggggtatt tttttgtcg agaggtaatt ggattttgtc gggagtgggt ttagaagttg 46020
aggtttagga agaggtttc gagtttttt tttttgttag gtgtagttat atacgtgaaa 46080
gaatagtttt cgtttcgatt tttagggtta ggaatggttg ttaggtttta gttgtttgtt 46140
tgaagacgga gattagttag cgttagtatt ggtattaagg tcgtttttgt tttcgatttt 46200
tttaatgttt ttgggagcgt ttaaggattt ttttttgaat tttatttttt cgtttttgag 46260
tatatatatt tttataattg tgggagagaa tcgttgttaa tgtatgtttt agttttttg 46320
tttgttggta gggtagggat tttattttag agtttttttt tttggttatt tttgttaagg 46380
gtagagtttt atttttgta tttttagttt ttttatttta gatttaacgc gtttaatttt 46440
ttttttagaa ggattagata ttttttggtt ggggagattt agttgttgag agagggaaag 46500
aataaatatt gatttgttgt ttattgtttg tggttttgtt tcgagtagtg tttttatttt 46560
aggtagtatg gagtggcgtt ttgtcggtag ttataggttg tttttggttt tagtttgtag 46620
tttatgattt agagtagtgg tagggatttg ttttgttttt agaattgggt gttttcgagt 46680
ttttagcggt tcggagatag atagggatgg aaatgtgggt gggggttggg aggggtaggg 46740
gtagcggggg tttttgtttt tgttttttgt tgtttttgta gaggtatatt tatgatttag 46800

```
taaggttttt ttgttattat tttaggatgt agatgaggaa atcggggttt gggaaggtgt 46860
ttgttgttag gttatattgt tacgggttag gggtagtagc gggttaagtg gttggttttt 46920
ttcgttagtt ttaaaggtta gtgtaagtag aggaagggtt aggttggttg tgggggttta 46980
gtaggatata gagagagggt ttaggggagg ttaggttttt tcgtttttt tttggatttg 47040
aaggtattta attaagaggt ggggagtttt ttaggttttt tttttgttta atgtattagt 47100
ttttttagag gttttattgg taatttttat taaaatatga aaatttaatg tagtggagag 47160
agagtatgag ttttttggtt atgtaggatg aatttttatg ggtttttag tttgcgattt 47220
gtttttattt aatagtttg aatgataggg acgagttggg gtttgtattt ttgggttggg 47280
aaggatagtt agtacggata gggttgagag tttttgttgg tttagtacgg aaagagtgtt 47340
tgtatgagag ttgtgtgtgt ttatgtgcgg agttgtgtgt gtttgtgtgt ggagttgtgt 47400
gtgtttatgt gtggagttgt gtgtgttcgt gtgtggagtt gtgggtgttc gtgtgtggag 47460
ttgtgggtgt ttgtgtgtgg agtagtgggt gtttgtgtgt ggaattgtgt gtgtttgtat 47520
gtggagttgt gggtgtttgt gtgtggagtt ttgggggttt gtgatttgaa tgtttgattt 47580
tattttaatt ggaggtgaag aggaaggtgg aggaagtggg gaagggttat tgaatttttt 47640
taaatttttt tttagtttg ttttatgggt ttgggagtat gtggtttatt cgaggttata 47700
tgggtatgtg tttttggggt atgattatat cggtttttaa agaagatgtt ttttgcgtgt 47760
gtgtgtgtag agtgtaattg tagataagta tatgattgtg aatgtttgtt agtagttatt 47820
tatgaattag ttttgcgttt gtgaaagaag gaatttgaat acgagaaaat atttgttatc 47880
ggttgttttt ttaaagttat tttagttatt aaggttttcg tcgtgtttta gtttttgttt 47940
ttaggtttag tgtttttta ttagtttgga ttagggtaaa tttttttgttt ttaattttttt 48000
ttattagggt cggatttgaa ggttaaggtt ttcgggtatg tgtttaggat ttttggttta 48060
gagaaggttt ttttttttt gcgtttttgt tttttttttc gttttggggt ttaggaattt 48120
cgatttttg gaagaagaga aggtttaggt tattgagttg ttttggttag agacgttttt 48180
gagtgttttt tttttcggg taatggtggt tgtgttttgg ggggtttagg tttgtttttt 48240
gtggttgcgt ttaaggtagt ttggaggtta tttaggttag tttttttcggg tttttcgtgg 48300
gtttaaatta ggatttagat tttttttttt tgttatagtt ttgggttatt tgggattcgg 48360
gatttttgt ttttttttt attttgtttt aaattattgt ttagaggttt gtcgttttta 48420
agaatcgtcg agggagaagt tttaggggaa ttggtttgtt cgtattttta ttgttagtag 48480
cgtttagggt tttaggattc gggtttttgtt agattatagt gtttttggg atttagggt 48540
ttttggtttc gtattgtgtt gcgttagtgc gtttggattt ttcgtgagtt ttcggttaat 48600
tacgtttcgg gtttttcgtt tggtttcgta tttgcggcgt ttttatttgc ggcggcgtcg 48660
ttcgaggagt tttttagtcg gcgaggagcg aggtttttgt agttttgttg tttcgtagtt 48720
tcgtagtttc gtagtttcgt agtttcgtag tttcgtagtt tcgtagtttc gtagtttcgt 48780
agtttagcgt tagagcgttg cgcggagatt ttttgtcgtc gtacgttttg ggtcggtaag 48840
aagtattcgt tttaaatttc gtttagcggg tagcgtaaag cgagcggttt ggggaaatgt 48900
cgaagggatc gggaaaaata aattttaagt cgagtttaat agtcgaaacg tttcgtgttt 48960
aggtcggcgg ataagaagga gatagaatag atttttggt tttttcgtag cgatcgattt 49020
cgtttatcga ttagagttat tatttcgttt ttagttcgtt aatttatatt tttcgtaagt 49080
tattagtttt aagggatgcg gaagttaagt atttcgtggt ttttagtatt aggtgattcg 49140
tacgtgtatt tatcgtagta gggaatttgt aaggtcgcgt tgtggtcgtt gttgtttttt 49200
tggagtttga ggttgtcgtt cgggggcgtt ttgtaggcgt ttcgttgtaa gtaaagatgc 49260
ggttgcgcgg gcggttgggg ttgcggttgt tgttgttgcg gttgcggttg cggcggcggt 49320
tggggttgcg gggtcgacgg ttggggttgg aatttgttaa aggagtttcg cgttttagtt 49380
ttatttttta ggggtgtcgt taggttttgt tgtttagcgt cgtaacgggt tcggtttttg 49440
gcgttttcga atttttgtgt tttggcggcg gtcgatagga aagttgtgtc gaatttgtcg 49500
ttttcgggaa atgttttaaa aggcgacgag ttttttcgat tttgcgatat cgggttgtag 49560
taggcgttta tggtagcggt cggcgattcg tagtaagaga cgtaagtttt agtatttatg 49620
tttggtttgc gtaggcggcg ggcggggacg cgagcgaggg cgcgaggacg ttatcgcgcg 49680
ttttggttgg gagtttgggg aggcgaggag gttgtggttg tgtatttttt gttcggtttg 49740
ttcgtttttt ttttttttt ttacgtcggt ttttttttt ttttttttt tttttttttg 49800
ttttttttt ttttttttt ttttttttt ttttttttt ttttttttt ttttttttt 49860
ttttttttt ttttttttt ttttttttt atagttagtc gagggaaaga acggagggta 49920
gtaaaaagat ttagatgttt cggaaagttg attagatttt ttaaattttt ttaaggtttt 49980
gtagcggaaa aaaaaaataa gtaaattttt tttttgttt tgtttttttt ttttttttt 50040
tttcgttttt ttttttttt tttttttatt ggtttttgtt ttttttttt tatttttaag 50100
gagatgttat taattcgtta gcggggattt aattcggagg cgttcgaggg ttttttcgat 50160
tttaggcggg gttggggaga tgttaggatt tttagggttt tcgaggttag gttgagaatt 50220
ggcggggtgt cggagttttg ttttagggta aagggaaatg taaattttaa tgataggtta 50280
```

```
aaaaaaagtt ttttaaaata aatttaatag gttttgatat tcgaagttag ggggattacg    50340
ggtttgcgga tgtaagcgag ttttacggat ttttgcgcgc gagttcgcgg gcgtcgttgc    50400
gggtttttc gttggtattt tttttaggt ttttaatttt ttttttatt ttcgtttta       50460
cgggagatta atatatatat aagttttcgt cgtttagtgt atggttattt gtacgttttg    50520
ttaattattt ttttttgttg taagggcgtt gggttgggat gtagcggaat ttgtgttttt    50580
ttaagttttg gagaagtttt gggttgggtt cggttttaga agttttagag cgttgttttt    50640
tgcgttttcg tttagaggtc gtatcgttta cgttttacgt ttgcgtttag gcgaggggat    50700
tttttgttag gcgaagtata gagagcggat tttggagatt ttttgatttt gttcgatagt    50760
cgcgttgtta tttgtttatt ttttgggtt aggggtcggg gagaggggtg agatcgtttt     50820
ttttttttt ggttttattg ttttcgattg taaaacgaat tgtttggttt tgataggttg     50880
tggttttatt attattgcga gttttaaggt ttttggtatc gttatagtaa gggaaaaaat    50940
tttgcgcgtg tagtttttcg atttcgtttt atgataatcg tgttagagat tggcgttttt    51000
aatttttga tgagttaacg gaggtttaga gaggggcggt tatttggttt aggttatata     51060
gttgggaaat agtcgtcgcg ttttggaatt tttgtttagg ttagtttttt tatcgatttg    51120
gaaggtcgtg cggatagcgt agatgtagag gggaaattgg ttttcggttg tggaatggtt    51180
taagttgaag gagattagcg agagtagttt tttttaaatc gttgagaaaa tcgttaagcg    51240
agaacgtatt tttagggagt ttgggagtag ggtgagaggt aaaagtgtaa ggtaaaatta    51300
aagagggttt gagtatcgtg aattaaggaa tgtgggttag atgttaattt tttgtattta    51360
aggtttttta aagaaaaaat atatttgggg ggtcgcgttt ttgtttttaa ttttatttgg    51420
gttaattttt atttttattt tattttttta gtatttttgg tttttgtttc gtcgtcgatc    51480
gcgcgatttt atatttggtt tttatcgtcg gtttattttg aagtgttcgt taataaagat    51540
atttcgtaaa ttaagttagg agcgcgtagg ttttagtcgt tttttatttt atgtttaatg    51600
ggaatcgcgt tttattggtt cgtagattta agttatttga gagtttttta tttattgagt    51660
ttttgttgtg ttaataattt ggtaatataa ggtttattat atttattttg tatatgggta    51720
gattgagatt taattaaaat aatttttag ttagtaaaaa gcggagtgtt tttgttatta     51780
ttttaaatgt ttttgtttag cggttttttt aagtttttag aatttgttaa ggtttttagt    51840
ttgtgtattg tagtataggt ggtttttttt atttggaatt ttttaaattt atttttgtat    51900
ttttgtttgg ttaatgattt agagtttagt ttagagtatt tttttttagg aagatttttc    51960
gtttttttta gtttaagttg acgttttcgt ttgttttatt ttcgttttag atttcgtggt    52020
tgtttggttg tttggttatg agtgtatttt aattttatta gttgttttag ggtagttttt    52080
tacgtgtttg gtattttata aatatttgtg gaataaaaga ttaaatttgg attaggcgcg    52140
gtggtttata tttgtaattt tagtatttta ggaggttaag gaggatggat tataaggtta    52200
ggagatcgat attattttgg ttaatacggt gaaattttgt ttttattaaa aatataaaaa    52260
ttagttagac gtgatggtag acgtatgtag ttttagttat ttgggaggtt gaggtaggag    52320
aatggcgtga attagggagg tagagtttgt agtgagtcga gtttgtgtta ttgtatttta    52380
gtatgggcga tagggcgata gggtgagatt tcgttttaaa aaaaaaaata aataaataaa    52440
aataaaataa aataaagatt aaatttgggg ttgtaggatt ttattatttt agtgtatttt    52500
aattgtttag tgtagtatta agttttgaga tttttttttt ggtttttttt agttaaggat    52560
agttggttat ttatttattt tttttttaaa aattaagtag cggaattaat agcgtgttgg    52620
attttt                                                               52626
```

<210> 4
<211> 52626
<212> DNA
<213> Artificial Sequence

<220>
<223> chemically treated genomic DNA (Homo sapiens)

<400> 4

```
gaaggtttag tacgttgtta atttcgttat ttgatttttg aagaggggggt gggtgggtga    60
ttagttgttt ttgattaagg gaaattaaag aaagagtttt agaatttggt gttgtattga    120
gtagttggaa tatattgagg tgatagagtt ttatagtttt aggtttaatt tttattttat    180
tttattttta tttatttatt ttttttttg agacggagtt ttattttgtc gttttgtcgt     240
ttatgttgga gtgtagtggt ataaattcgg tttattgtaa gttttgtttt tttggtttac    300
gttattttttt tgttttagtt ttttaagtag ttgggattat atgcgtttgt tattacgttt   360
ggttaatttt tgtattttta gtagagatag ggttttatcg tgttagttag gatggtgtcg    420
```

```
atttttgat tttgtgattt atttttttg gttttttaaa gtgttgggat tataggtgtg      480
agttatcgcg tttggtttag gtttaatttt ttattttata aatatttgtg gggtgttagg    540
tacgtaggag gttgttttgg ggtaattagt ggggttgaga tatatttata gttaggtaat    600
tagataatta cgagatttgg gacggagatg aggtaaacgg aagcgttaat ttaaattagg    660
agagacggga agttttttta gaggaagatg ttttaggttg aattttgaat tattagttag    720
ataaaagtgt aggaatgagt ttagggaatt ttaggtaggg gaaattattt atgttatagt    780
gtataggtta gagattttgg taggttttag gaatttgaag aaatcgttgg ataggagtat    840
ttaggatgat ggtaggagta tttcgttttt tgttaattgg aaaattattt tggttgagtt    900
ttagtttatt tatatgtaaa atgggtatga taagttttgt attgttagat tgttggtata    960
ataggagttt aataaataaa ggatttttaa gtggtttagg tttgcgggtt agtgaggcgc   1020
ggtttttatt gggtatggag tgggggggcgg ttggagtttg cgcgtttttg gtttggtttg   1080
cggaatattt ttattaacga gtattttaag gtaagtcggc gatggaaatt agatgtggga   1140
tcgcgcggtc ggcggcggag taaggattag agatattaag gggtagggt gggggtaaga   1200
attagtttag atgagattgg gagtagaagc gcgatttttt aggtatattt tttttttggg   1260
agatttaga tgtagaaagt tgatatttga tttatatttt ttggtttacg gtgtttaggt   1320
ttttttaat tttattttgt atttttattt tttatttat ttttagattt tttagaggta    1380
cgttttcgtt taacgatttt tttagcggtt taaggaaggt tgttttcgtt ggtttttttt   1440
aatttgaatt attttatagt cgggggttag tttttttttt gtatttacgt tgttcgtacg   1500
gtttttttagg tcggtggagg gattagtttg agtagaggtt ttagggcgcg acggttattt  1560
tttagttgtg tgatttggat taggtggtcg tttttttttg agttttcgtt agtttattag   1620
aaaattgggg gcgttaattt ttagtacggt tgttatgaag cgaaatcgag gggttgtacg   1680
cgtaaagttt tttttttgt tgtggcggtg ttaggggttt tgaggttcgt agtggtggtg    1740
aaattataat ttattaaaat taagtagttc gttttatagt cgggggtagt gaggttagaa   1800
agggaagaga cggttttatt ttttttttcg atttttgatt ttaaggagtg aataggtgat   1860
agcgcgatta tcgggtaagg ttaggggggt tttagagttc gtttttttgta tttcgtttgg  1920
taggaaattt tttcgtttgg gcgtagacgt ggggcgtgaa cggtgcggtt tttagacggg   1980
agcgtagagg gtagcgtttt aaagtttttg aagtcgggtt tagtttaggg tttttttagg   2040
gtttgaggga gtatagattt cgttatattt tagtttagcg tttttatagt agagggaaat   2100
agttaataag acgtgtaagt gattatgtat tggacgacga aggtttgtgt gtgtgttaat   2160
ttttcgtagg aacgggaatg gggaggaagg ttggaaattt aggaaaaggt gttagcggga   2220
aggttcgtag cggcgttcgc gagttcgcgc gtaggagttc gtaggattcg tttgtattcg   2280
taggttcgtg gttttttttgg tttcgaatgt taaaatttgt taggtttatt ttgggggggtt 2340
tttttttaat ttgttattaa aatttgtatt ttttttttgtt ttaaggtaaa gtttcgatat  2400
ttcgttaatt tttagtttag tttcgggagt tttggggatt ttggtatttt tttagtttcg   2460
tttagaatcg gaaaagtttt cgggcgtttt cgggttgaat tttcgttagc gagttagtag   2520
tattttttta agagtggaga ggagggggta ggggttagtg gaaggagaga ggaggagggg   2580
acgagagagg gggaaaggaa gaagtaaggt agggaaaaaa atttatttat tttttttttt   2640
cgttgtaaaa ttttaagagg gtttgggaga tttggttaat ttttcgaaat atttgaattt   2700
ttttattgtt tttcgttttt tttttcggtt agttgtgggg agggaggaaa gagagagagg   2760
gagagagaga ggaagggggg gggagggagg ggaagagaga gagaggaggg agagagagga   2820
gagagataga gaaagaagag ggggagagag agagaggtcg gcgtggaggg gaggagggag   2880
agcgagtagg tcgggtaagg agtgtatagt tatagttttt tcgttttttt aaatttttag   2940
ttaaggcgcg cggtggcgtt ttcgcgtttt cgttcgcgtt ttcgttcgtc gtttgcgtaa   3000
gttaggtatg aatgttgaga tttgcgtttt ttattgcgag tcgtcggtcg ttgttatgga   3060
cgtttattat agttcggtgt cgtagagtcg ggagggttcg tcgttttttta gggtattttt  3120
cggaggcgat aagttcggta taattttttt gtcggtcgtc gttaaagtat agggattcgg   3180
ggacgttaag agtcgggttc gttacggcgt tgggtagtag gatttggcga tatttttgga   3240
gagtggagtt ggggcgcggg gttttttttaa taagttttag ttttagtcgt cgatttcgta  3300
gttttagtcg tcgtcgtagt cgtagtcgta gtagtagtag tcgtagtttt agtcgttcgc   3360
gtaatcgtat ttttatttgt agcgaggcgt ttgtaagacg ttttcggacg gtagttttaa   3420
atttaggaa ggtagtagcg gttatagcgc ggttttgtag gtttttttgtt acggtgagtg   3480
tacgtgcggg ttatttggtg ttggggatta cggggtgttt ggttttcgta tttttttgaaa  3540
ttggtggttt gcgggaggta tgagttggcg ggttgggggc ggggtggtga ttttggtcgg   3600
taaacgggat cgatcgttgc gagggagtta agggatttgt tttgtttttt ttttgttcgt   3660
cggtttaggt acggagcgtt tcggttgtta aattcgattt ggagtttgtt tttttcggtt   3720
ttttcggtat ttttttaaat cgttcgtttt gcgttgttcg ttgggcggga tttgaagcgg   3780
atgtttttta tcggtttagg gcgtgcggcg gtaaggagtt ttcgcgtagc gttttggcgt   3840
tgggttgcgg ggttgcgggg ttgcggggtt gcggggttgc ggggttgcga ggttgcgggg   3900
```

ttgcggggtt gcgggatagt aggattgtag aagtttcgtt tttcgtcggt tgggaggttt 3960
ttcgggcggc gtcgtcgtag gtgagagcgt cgtaggtgcg gagttaggcg ggaagttcgg 4020
agcgtggttg gtcgaaagtt tacgggaaat ttaggcgtat tggcgtaata tagtgcggag 4080
ttagagattt tagggtttta ggggatattg tggtttggta agattcgagt tttgggattt 4140
tgagcgttat tggtagtggg gatgcgggta ggttagtttt tttggagttt tttttcggc 4200
gatttttgga ggcggtaggt ttttgaataa tggtttagga tagagtagga agggagatag 4260
gaagtttcga gttttaggtg gtttagagtt gtggtaagag agaagggttt gggttttgat 4320
ttgagtttac ggggaattcg ggggagttgg tttaaatgat ttttaagttg ttttgaacgt 4380
agttatagga ggtaggtttg ggttttttag agtatagtta ttattgttcg ggagagagag 4440
atatttaagg acgtttttag ttagagtagt ttagtagttt aggttttttt tttttttaga 4500
gaatcggggt ttttgaattt taaagcggaa ggggaagtaa agacgtagaa agggaaaaat 4560
tttttttggg ttaggagttt tgagtatata ttcggaggtt ttggttttta ggttcggttt 4620
tggtgggaag agttgggggt agggaatttg ttttgattta ggttgatgga gaggtattag 4680
gtttggggat agaggttggg atacggcgga ggttttggtg gttagaataa ttttgggaaa 4740
atagtcgatg gtagatattt tttcgtattt aggttttttt ttttataggc gtagaattag 4800
tttatagatg gttgttgata aatatttata gttatatgtt tgtttataat tgtattttat 4860
atatatatac gtagagagta tttttttag gagtcgatgt ggttatattt taagaatata 4920
tgtttatgtg gtttcgaatg agttatatat ttttaaattt ataggataga attgagagaa 4980
gatttagaaa gatttagtgg ttttttttta ttttttttat ttttttttt attttagtt 5040
ggaatgagat taggtattta gattatagat ttttaaggtt ttatatatag atatttatag 5100
ttttatatgt aggtatatat agtttatat atagatattt attgttttat atatagatat 5160
ttatagtttt atatacggat atttatagtt ttatatacgg atatatatag ttttatatat 5220
ggatatatat agtttatat atagatatat atagtttcgt atatggatat atatagtttt 5280
tatataggta tttttttcgt gttaagttag tagagatttt tagttttgtt cgtattggtt 5340
gtttttttta gtttagggat gtaggtttta gttcgttttt gttatttagg attattaggt 5400
gagggtaggt cgtagattag ggatttatg gaggtttatt ttgtatagtt agagagttta 5460
tgttttttt ttattgtatt gaatttttat attttaatga aaattattag tgagattttt 5520
gaagaggttg atatattagg taagaagaga gtttaaggag ttttttattt tttggttaaa 5580
tgtttttagg tttagaggga aggcggagag gtttggtttt ttttgagttt tttttttgtg 5640
ttttgttggg tttttatagt tagtttggtt ttttttttat ttgtattagt ttttggagtt 5700
gacggaaagg gttagttatt tgattcgtta ttgtttttaa ttcgtggtag tgtgatttga 5760
tagtaaatat ttttttaaat ttcggttttt ttatttgtat tttggggtga taatagaaag 5820
gttttattgg gttataaatg tgtttttgta gagatagtaa gaggtaagag taagagtttt 5880
cgttattttt gtttttttta atttttattt atatttttat ttttatttat tttcgagtcg 5940
ttgggaattc gagagtattt aattttgagg atagggtaag tttttgttat tgttttgggt 6000
tataaattgt aggttgggat taaaggtagt ttgtaattat cggtaggacg ttattttatg 6060
ttgtttaaga tggaggtatt gttcggggta aagttataaa taataaatag taagttagtg 6120
tttgtttttt tttttttta gtagttgagt tttttagtt aggagatgtt tggtttttt 6180
aggaaaggga ttaggcgcgt taggtttaag atggagaaat tgaggatata aaagatgagg 6240
ttttgttttt aatagaagta gttaggagga gggattttgg agtgggattt ttattttgtt 6300
agtaagtagg aaaattgagg tatgtattga taacgatttt tttttatagt tgtgagagtg 6360
tatatattta aaagcggaga aatggagttt aaggaaaaat ttttagacgt ttttaggagt 6420
attgggaaag tcggaggtag gggcggtttt ggtgttagta ttggcgttgg ttgattttcg 6480
tttttaggta ggtagttgag atttggtaat tatttttagt tttggaggtc ggggcgaaag 6540
ttgttttttt acgtgtgtaa ttatatttaa taaaaggggg aagttcggag gtttttttt 6600
gggttttaat ttttgggttt attttcggta gagtttaatt gtttttcggt aaggaaggtg 6660
tttcgggtag ttgagtttgg gttaagtcga gggagtgcgg ggcggggaag tcgtttttag 6720
tttttgttgg tcgggtttgg atcgggttgc ggaaagagtt ggtgagagaa atttttttcgg 6780
gttgtaagcg tttttatttt aatttgggtt ttttttttt taaatgtgtt aaaagaacga 6840
cgattaaggc ggtttcgtag tgttttttgg gtgcggagat cgcgttttta cgaaagagtt 6900
tttatcgagt ttttttttgt tgtaatttaa gattttatta ttttttttt tagattttgg 6960
gttcgttttt tggtttttg aatttttgt tcgagtttag ggcgggtcgt ttgggttata 7020
ggagatatgt cgggttggag attcgacgtt ttttagcgac gtagagagtt tttttgtatt 7080
tagggtaggg atttcggcgt ttcgttttta aatttcggat tcgatcgtcg gagttgtagt 7140
cggcgcgtat tggttttagt ttatttgttt cgagacgcgg ggtcgttgcg ggaagttgtt 7200
ttttttttt tttttttttc gttttcgatt cggtggcgat aggtttttgt tgaaagtaga 7260
ttgttatttt ttcgtttatt tattcgtaaa agtagcgttt ttagcgttaa ggtttgggga 7320
ggcgcgggtt aggttggagt ttttggggtg tcgtttaggg gtttagtttc gatttttcga 7380

```
attagattta gtggttaaat attagagggt atttattttt tttgtatcga tataatttat     7440
gtattacgaa atgtgtaaat tttgtcgggg tcgtatttga atttagttag agaattgggg     7500
tgtgttttgt atataagaga cgatttaaag aaggtagaaa cgaaaattcg atagaagtag     7560
taatagttgt tgtgggtgat tgtggggtga tcgtaggaaa attcgagttt gggattgaag     7620
tttgtttagg aaggggcgat tgttgtttaa ttttggaggg agggatggcg aaggaagatg     7680
tttttaattt tttattttgt taatttttat agtttgtttt attagtttgg tttaaatata     7740
aaagttgtta aatagaaaaa tagagggttt ggattaaaat ttaatatttt aagttaatcg     7800
atttgtatga ttttgtataa tttttttttt attataattt atatatagtg aagtgtttag     7860
ttttgaggag gaaagttgga agaattgtta tgtatgtgta tattgttagt taggacgatt     7920
atttcgataa agaaatagat tatttatatg tttttttaaa cgttgtaaaa cgttaaagta     7980
aaaatatttt ttgtaagttg ttttaagaaa attttagaag atatttcgga gtatcgggga     8040
ttttattttg tttttgatag tagtgttttt tgaatagggt gatattttag ttagggtatt     8100
tgcgcggttt cgattttaat gcgaagtttt aagcggttgc gttaggaatt tcgtcgcgat     8160
cgtcgggtta agtcggagtt attaagtttt gagtcgtacg cgttgcgacg tcggtacgta     8220
gtaggaaaat agattaaaac gttttataga aaatttcggc gaagtttcgg aggattttgg     8280
tttttaagat tagttgggcg tatttttttc gggacgtttt ttttttttcgg ttttagcgtt    8340
tttttgtttt tagtcgcgcg tagttttgtt ttggtggtaa attgaaataa gaaatggaaa     8400
tatattggtt tttgttgttg ttagggatga gaggttgttg acgttcggcg cgtttgttcg     8460
ggtttcgggt ttttttgtag atttttggat tggggtgttt gaggttagga gaggaggggg     8520
atagttgttc ggagttttcg cggtttagag gttttgggat gatttatggg gggggcgcgt     8580
tttgttttgg tgagtttttc gtttcgaggg taggcgaggt gggtgggtag gggagtgtat     8640
gtcggagaga agagagaacg tttaattaga gagaattttt tgttttcgga gttttagcgt     8700
ttttagtttt ttaaattttt gtttaggaag tcgaaggatt taggtttagg taacggtttg     8760
gggtggggcg gtaagagcgt cgttttgtat tcggacgtcg tttgttaggc gttatttggt     8820
tatgtgcgtt cgtagcgtag ggtttttcgg ttattagtat aggtttttat cgaggtgata     8880
gttatcggat tgggaaatta atattgcgag gattcggtta tgtgatgata tcgttcgggg     8940
gaattcgagt ggaagtcgat cgttttgtgg tgttattaga taggtgagaa gtagtttttt     9000
taaatagggt tttttgttgg aaggaggagg tatttttttt tagttagtga gtttttttag     9060
ttgtaatcgg ggtttttttt aatattagtt atcgcggttt ttagaggtgt agttcggtat     9120
attttttttt tgtagatgta taaatcgggg atatttttat ttttatttaa gatgtttttt     9180
ttttatttag tagaggggtg cggagtaaat tcgggataat aatttgcgtg ttgtttggaa     9240
gtaggttttt tagaaaggat gataaaaatt tggtgatgcg gaagaagttt ttattgtgtt     9300
taggaaaggg tagcggtttt ttagttgtat cggttttggt ttcggttttt atttttagta     9360
cgttttgtta ttttaataaa gagcggtttt tttatttgat tttaattcgt attagttagc     9420
gtcgaggaaa gagaggaatg gaagggagtt gtggtttagc ggcgaggaag agttacgtag     9480
aggtaaggtt ttgaggagtt aaggttgggg tgtgtagggt ggtatttagg gtcgcggatc     9540
ggttcggatt tggattttag aggtagttgg aagggttatc gtggcgtttc gcggttttta     9600
gggtgtcgtt ttggtgagga gggggttatt gggagatacg ggcggggtaa gaggggttat     9660
cggttgttaa gtatttattt ttttcggtga aggcgtcgtt tgtattcggg atcgtttggt     9720
taaggttgtt gggtattggc gtcgttgtat attgggggta gagagggagt tagtgtttgt     9780
tttatcggat ttttaaagta gatttttttaa gagtggattt ttattttgaa ttggaggttt    9840
agtatggatg ttggttttac gtatttcgta gagggatagg atttggtgat ttaggttaga     9900
gtagaataat tggtttttta gggttttgaa ggttataata gagaaggttg ggaagttgtg     9960
ggaggtttgg attatgatat tgatgtttag cgcggtatac gtggggatcg ggtttatag     10020
ttgcggtgta gattatttta gtttttattt ttgtattttt tatttttttta tggttttgga   10080
gaagtatgtt ttgtttcggg gtttgaattt tagttgtatg aaatgtagga gtgttgataa   10140
tttttaaaga tttttttgga gtttttgtgt tggtagtagt ttgggtttat ttttattttt   10200
tttgtttttt tttttttattt ttatacgtat ttttgtttag gagaaattgg ggtaaggtgg  10260
gtggaaaagg tttttttgga ggttaatata ggttttgagt ggggagttat tataggatat   10320
aaagtcggtt agaggtttta tagggttatg gtggaatgta ggttttgggt ttaggattgg   10380
gatgtaaata gggagggggt aagtaagtgt ttttgtgatt aggtttaagt tgaggtttcg   10440
ggtttatggt ttttgtgttt atttattttt tttattttag tatttatgga gtattttttt   10500
tgggcggatt tttttttagg aggtagaata tgttaagttt ttgttttttg tgttacggtt   10560
tatgtttttt taagtgtttt gtatagttgg gtttaataaa tgttcgttgg ttggaagaat   10620
gaatgaagaa ttggtgtttt ttgtagtgtt gtttttatat ggttaagtgt atttaggagt   10680
tagtttaggt gtttgtgtga gcgtgtgtat agtatgaatg tgagcggtgt gtgtcgtgtt   10740
tattttttttt gggtttacgt tttgttattt gagtgtttta gggcgaggtc gagggtttgt  10800
gtgtagttcg gtgaaaggta tgtttgtttt ttggtatttg tgtttgtgtt ttttagtgtg   10860
```

```
cgagttttag gtgtgtaagg gtgagtattc gtgtgtacgg ttttggagtt tttttgggtt    10920
tgtgggtggt atagtttttg tttttagagg cgtttttagg gagaatgatt aagcgttttt    10980
gtattatggt ttgtggtttt tttgtttttg gttatggttt ttaggagtag ttgatttatc    11040
gagtggtagg tagaattttt ttgtggtaag ttttcggtgg ggtttaatta tttttcgcgt    11100
tattttgggg ttttcgaata tggtttattt agtttattta tattgcggcg aggtcgtttg    11160
ataagttatt agttttggaa tatatttttt gtttcgtgtg gtttattgcg gaagagacgt    11220
gtttttggta ttgtttgtgt ttattgttga ttttatttag tatatgagta aattgaggtt    11280
taaggggaaa tggattttta tagttaggtt agtaggaggg taggaattta tcgagttttt    11340
ttttttttat tataatgggt ggaaaggtag aaagtgttgg ggaatttaat ttatttattc    11400
gtttttttgt tggatgcgat ttttgtaata agtttagtaa gttgtatttt ttttgtgtat    11460
taaatagggt agatgtttgt agttttgttt tagttttttt agaagtaaag tagatttcgt    11520
tatgagtttt tgggtattag aggatagtag gagagtattc gtggaatttt tggtgggatt    11580
gtagggattt gattttaatg tagttttatt aggttgggat ttttgttttt tgtttagttt    11640
tttaagttgg gttttttttt tttaagtttt tgaaaattat aagttagggt tagttgaggt    11700
tagatgatta atttttttta ttgtgtagga ttgtttatta ggttttgagg gtttttggtt    11760
atatcgagga aattggtatt tttttgattt ttgtagtata tatataatgg gttatggtga    11820
tatgttgggg ggttttattt aaggttggaa gagatggtag ttatttttg gtttatatgt    11880
tttattttat agataaaggg gattgagagg tttgatttta tttacgtaag gtttattaaa    11940
ttttgtttgt tattatgtta agttttgtgg ggtattttgg gaagttttgg atgtagtttt    12000
tgtttttagg tttagaggta gagatagata agtgattttg aaattttagt ttagtgggat    12060
gggttttgag atggggaaag ttttgattta ggttttgagg agaaggttag gttggtttgt    12120
tttggggcgt gtaggcgtta ttagggtttg gtagtttatt ggaagttatt taaggggagg    12180
gtgtaggaaa gttgggggaa gttgggattt gtttaggtta atgagttttt tttagtggtt    12240
ggaaattttg aggttttttt ttttttgatt tttttttttt ttttttttat gttttttttt    12300
ggttttttta tggaaatagt tatgtataat agttttttta ttttttttagt gttattttat    12360
tttttttttgt gttgaatggt attttggttt tgttttattt gggaggggag tatgaggagg    12420
atgtgatgat ggagagaggt agtgtgtttg tttcgtatat tttgtattat taggtttttt    12480
gttttatttt ttatattata ttttttgagg gtgttaggat attgtaaatg atagtggtgg    12540
ggttttgatt aggttttaga gtgtgtatgt agagtttggg gttttttttgg atatgtgagt    12600
tttttcgatg tttttatgtg gttataagga tttatttata tttggtttat attttagtat    12660
ggagatggga taattttagt tttatggttt ttgattttaa ggagggagtt tatttttaaa    12720
tggggaagat aaatatgtat atagttttat tgtatggttt ttttgttgtg gttttagttt    12780
ttttattttt gaattttttt tatgggattt agtggtttta gaggttttgg ggttttgtga    12840
tatttattaa gaggggtgaa ttggtttttt agaattttta agatgtttta ggattttata    12900
ttttttttta tttattgtat taggaaaggg ggaaggttgg tttagtttgt taaaggttaa    12960
gagtttggga tgttaggttt ggtttttggt ttgagttttt ttatttatag gatagagata    13020
ataatgggtt ttaaggtaaa gaaggtttag agttggttgt tgttgtagaa ttatgagata    13080
gtttagaata attgagcgtt tttgtttgtg cgttaggtag tatattagtg ttgtttatgt    13140
atgattttat ttaattatta ttataatttt atgagattgg tattattatt attttattt    13200
cgtggatgaa aattgaggtt tatggaagat aagtaattcg tttatagtga gatagttagg    13260
aaggaagagt cgagttttgt tggtcgaggg ttttaagaaa tttaaaaggt attttgtttt    13320
agaatgattt tgattagatt tgagataatt ttaattttat tttagatgat gtggagatga    13380
aagatagggt tttataaata tggtttttag ggtacggtgt agtggtttat gttcgtaatt    13440
ttagtatttt gggaggttta ggcgggcgga ttataaagtt aggagattga gattattttg    13500
gttaatacgg tgaaattttg tttttattaa aaatataaaa aattagtcgg gcgtggtggc    13560
gggcgtttgt agttttagtt atttgggaga ttgaggtagg agaatggtgt gaattcggga    13620
ggcggagttt gtagtgagtc gagattttgt tattgtattt tagtttgggt gatagagtaa    13680
gatttatttt ataaaaaaaa aaaaaaaaaa aaaaattatg gtttttaaag cgtttttttt    13740
agttttataa tcggggatgt agaagataag tagtgttagg agtaaatttc ggtatttgtt    13800
aggttgaagt tttatatttg attttgtgat tttgggtata gtttttttat ttgtattagg    13860
tgaaaatagt gttttaggta tggtataggg ttttatagtt ggtatgagga atgattgtat    13920
gagtttttag aagttttagg aatatgagag aatttttgat taaggtaggg tggagttgag    13980
gagtatttgt gggaatagtg tttttttagga aatatgagag atcgtaggag gttttgtttg    14040
ttttgatttt aatttttttt agagtttgga gttagtttat atagtagatg tttaataaat    14100
gtttgttgtt gagtgaatga atgaagggat tttataggag gggaatatgt taggaatttt    14160
gtaagttttt ttattttttt ttcgtttatt ttttttttga atggaggttt ggggtgagag    14220
tttgttttgt cgtttttttg tattttggta gaaaattatt gatttttttt tttttgtagg    14280
gtttttttgtg gggtgtagga gtagcgaggt tttgggtagt tttatttagt ataagttttg    14340
```

gtttttttta attttagtag tcggaggagt attttttttt tttattgttt tttttttggg 14400
ttttatttta ttggttatag gtttgcggtt tgattgttat agttttcggg gtcggtttta 14460
gttaaatggt ttttttttt tatttttaat ttttttttt aggtttttt tattttttat 14520
agtgagtttc gtgttttgtt cgttggtatt tttatttttg ttatttgttt agggtgttgt 14580
ttgagagttg ttgaggtttt tttttagtt ttttatttat ttattttta ttttgggatt 14640
ttgagattta tttgtaaggg atgtagtttt tttttttggt taggatgtta tttgatttta 14700
ttcgggtttt tagtagggta ttcggtttta aggtttgggt ttttttagt atatttatga 14760
tttcgtttat tcgttttatt tcggtatatg taatttgagg gtatttgaat atttatggtt 14820
ttttgagttt tattaaaggt ttaggtggag gaattgaatt tttattttat agatggaagt 14880
taaagtttaa agaggggggaa tagtttagaa ggaatgtatg gaggagtgtg cgtttggtaa 14940
aagtttttt ttatatttaa tagattttt agtttggtat ttttgtgtta ggtagggatt 15000
agagtgtttg gtagtgaatg aattggtttt tgttttttaga gagtttatag tttggttaga 15060
atgtgttata tgttatggga atttttaggg aaggtgaatg gattggggtt ttttgagtat 15120
ggatattcga agtggatttt gaaagttgaa taggagttta ttagttgaag gagatgaaag 15180
aggtttttta gatttaggaa atagtataaa ttaagttttc gggtaaggga agaatatgaa 15240
gtaagtgtgt ggagtgtagg ggtatgatgt atgtcggtga ggtgggttga ggaatggtag 15300
gggtatagtt gaagagagtt ttttatttta ttttatttta ttttattatt ttttgagata 15360
aggttttatt cggttattta ggttggagtg tagtggcgta attttagttt attgtaattt 15420
ttgtttttta ggtttaagtg atttttttat tttagttttt taagtagttg ggattatagg 15480
ttcgttatta cgtttagtta atttttgtat ttttagtaga gatagggttt ttttatgttg 15540
tttaggttgg ttttgaattt ttagatttaa gtgatttttt cgttttagtt ttttaaattg 15600
ttgggattat aagtataagt tatcgcgttt ggtttttggg ttatttattt tttattaaag 15660
ggtttagatt ttggggaagt tattggaggg ttttgagtaa aatagtgaga ttttaggttg 15720
agtgtttttt gtatggttta gtatggtttt tgtttttttgt atttaaaatt tttattttaa 15780
ttaagaatga atttttttatt gtggtaaaat atatatagta taaaatgtta ttattttaat 15840
atttataagt gaattttttta gtggtattaa gtatatttat aatattgtgt aattattatt 15900
attgttttta gaattttttt attattttaa atagaaattt tatatttatt aaacgggaat 15960
cgttattata tttttgattt tttatttaag tgatttttat tttattttttt ttttgtggat 16020
ttttttattg taggtatttt tttttttttt ttttttttt gagatagagt tttatttttg 16080
ttgtttaggt tggaatgaaa tggtgtaatt ttagtttatt gtaatttta tttttcgggt 16140
ttaagcgatt tttttgtttt agttttttaa gtagttggga ttataggtat ttattattat 16200
atttgtttaa tttttgtgtt tttagtagag atggggtttt gttatattgg ttaggttggt 16260
tttgaatttt tgattttagg tgatttattc gttttagttt tttaaagtgt tgggattata 16320
ggtgtgagtt atcgtatttg gttttaggta tttttttatag gtggaattat atagtatttt 16380
tttttggcgt ttggttattt gtgtattttt tttggagaag tgtatttaag tattttgttt 16440
atttttgaat ggggttgttt gttttttttgt tttttagtta taggaatttt ggatattaat 16500
ttttttttag atatatgatt tgcgaatatt ttttttttatt ttatggattg ttttttttatt 16560
ttttggatat tgtttttttga tgtatagaag ttttatattt tgatgaagaa gtataatttt 16620
ttaattttt tattttgttg tttgtgtttt tggtgttata tttaagaaat cgttaataat 16680
tttagtgtta ggaggatttt cgtttatgtt tttttttaag agttttatag tttagttttt 16740
aggtttaggt ttttgatgat tttaatttttg aatatgacgt aatataaggt tttttaattt 16800
tatttttttt tatggggata tttatttttt ttagtattat ttgtagaaaa gatggttttt 16860
tttttatcga tcggttttgg gatttttgtt gaaaattaat tgcgtatgta tttgagagtt 16920
tatatttggt ttttattttg ttgtatttgt ttatttggtt tttgaatgat tttttgatgt 16980
gaagtttagg attgtagaat gtataaggat ttttgtaggt tattcgttta tttttttgtt 17040
tgtaggttag tgattaatag gttatagaaa tttagtttaa ggatatattt gtttttcggg 17100
atttggagtt ggtgagaagg ggtggagggt agagttaaag gcgaggattt tagagttata 17160
gttttggttt tggttaggtt tttggatatt cgggggtttt tttgtttttt tagttgatta 17220
ttttatgagg acgtggtttt tggggaaatt tttttgtgtt ttgagggttt atatatttat 17280
tttttgagat gcgtatattt gggattattt tttttattaa gatgatttta gaattttttt 17340
agcggagttt ggaggagttt taagaaggaa gtatttagtg gttttatgtt gttggtttta 17400
tgtatatttt gaagattttt tgttagattt ttaaaagagg tatttgtaat tttttgtttt 17460
ttattttagg ttatttagtg aatggtataa ttaggggttg gggttttagg gattagatag 17520
tagagtttaa ggggagtata gttgtagaga agtagttgtt ttcggttttt tttttttagga 17580
attttttcgg tttaggtgat taaatagagg tttgtttgag tttggttttt tagttatgtg 17640
ttttaaggta taattttttag ggtttttgga aaattatgga aattgtttat tttataattg 17700
tgttatatag gttgtttgat gttttagatg tttttggttt ggagaagttt atgaagttta 17760
ggatttggaa atagatttta gtttatatag ttgagtgatt ttgggtaaat tagttgattt 17820

ttttgtttta gttttttat ttgtaaagtg gggataagaa ttatgtttat ttgttagtga 17880
tagggtgagg tttaaataag gtaaaatagg taaggataaa gttaggtgtg gttgtttata 17940
tttgtaattt tagtattttg ggaggtcgag gtaggtggat tgtttgaggt taggagtttg 18000
agattagttt ggttaatata gtgaaatttt gtttttatta aaaatataaa aaattagttg 18060
ggcgtggtgg tgggcgtttg taattttagt tattagggag gttgaggtag gagaattatt 18120
tgaatttagg aggtagaggt tgtagtgagt cgagattata ttattgtatt ttagtttggg 18180
taagaagagc gaaattttgt ttttaaataa ataaataaat aaataaataa ataaataaat 18240
aggtaaggat tttagaatag tttttggagt atagtaagtg tttagttaat gttagttgtt 18300
atgattgata agattttgtt ggaattttga ttttattaga ttggtcggaa attttgatag 18360
gtagttaaag ttgattggga tgtatagagt tggaaaatga agaaattatt attgaattta 18420
tgattattgt gtggttgata ttgtttttta aaatatggat gagtgatggg aggggcgggg 18480
gttgttattt ttatttaacg tagcgggggt aggttttaag gagggataaa ggttatttag 18540
gtttttagg tttttttttt ttataggttg ttgtatttcg tatatatgtg ttatgagttg 18600
gggaataatt tttttttgt tgaatgtgtt ttttgtgttt atttgattta gaattttagt 18660
ttgtttttag ggtaggggtt ttgttgtgtt tttttttgat tagtataggg ttgagtatag 18720
agaatcgggt ttagggaatg ttaggtaatt tttgtatttt ttttgggttt tagtttttttg 18780
ggttttattt gtaattaaga aagtatttgg tgggtattgt ttttggttat atttggattg 18840
agatttttttg tggaaattta ttgggagata ttagtatttt taatttgaag atgaggaagg 18900
tgaggttgtt tcgttgtgaa ttgaatttag attttttgaa gttaaagagt tttttttttt 18960
ttttgtttag gggggtttatg tttattgttt ggatgttttt atgtttatgt gtttattaga 19020
tttgattagt atgtgagagt tttgtagacg gtggaggggg tgtattggga ggggttttta 19080
gtatattgta tgttgtacgt gggttatatt attttcgttt atttattgaa atgatgatta 19140
tgttttttga tcgtttagat attattttt tttttttta gagttatttt gagaattagg 19200
ttagtttagt ttaggatttt tatgtgatat gtgggagtag tttttgtggg taaagaagga 19260
ggagataaat ttttttaaat agaacgagta atagtttttt tattagttta gtgttggcga 19320
agttaggggt aagaggttag tttggaaata ttatttgtat tatgtggtag gtggatagtg 19380
agggttaggt ttttttaggg aatgtatgtg tttaagtttt gttgtattaa tgtatttttt 19440
aggatttagt tttatgagag aagattttat agagtttagt tttgtgttat ataaagtatt 19500
tttttaggag atttatttcg ttgtagggga tttattttgt taagaagtag taataggtgg 19560
aaaagagatg tatttgtttt acgttcgttg tggattaagg tttgtttggt agaagggata 19620
tattatgtat tatatatata tttttttttt ttttttttaag atggggtttt gttatgttgg 19680
ttaggttggt ttcgaatttt tgatttttagg tgattcgttt attttagttt tttaaagtgt 19740
tgggattata agtgtgagat attgtatttg gttatattat gtattttaaa gaagagattt 19800
ttttgaattt tttattttgt ttagatgagt agaagttaat gaaaaaatta tttttttttt 19860
tttttttttt tttttttttt tttttttttg tttgtttgtt tttttttttt tttttttttt 19920
tttttttttt tttttttttt tttttttttt tttttttttt tttttttttt ttttagatag 19980
ggttttatta ttatttaggt tggaatttaa tggttaaatt atggtttatt gtagttataa 20040
ttttttgggt ttaagtagtt tttttgtttt agtttttttga gtagttggga ttataggttt 20100
gtgttattac gtttagttaa tttaaaaatt ttttttatag agatgggggt tttattgtgt 20160
tgtttaggtt ggttttaaat ttttgagttt aggtagtttg tttacgttgg ttttttaaag 20220
tgttgggatt ataggtatga gttattatgc ggggttaaaa gaattatttt taattaatat 20280
gttaattgtg aatgttgaaa tattgattat ttatatttta tttttattat atgtgatagg 20340
taaaattttg ggaatttatt tattttaaat tttagttatt tatataaaaa tattaattaa 20400
aatttttagg tataattatt ttttagatat tttgaattgt taattatttt atagttgtaa 20460
atgtggattt tttaaaaagt aaatattgaa gaattttttt tattttttatt aattttaagg 20520
aagttgatga ttttttttaaa gtataaatta ggattgggta tggtggttta tgtttgtatt 20580
ttagtatttt gggaggttga ggtgagataa ttgtttgaat ttaggagttt gagattagtt 20640
tgggtaatat agtgatattt cgtttttata aaaaaaaaaa aaaaaaaaaa aatttaatta 20700
gttagttgtg atgttgtgtg tttatagtat tagttattta ggaggttgag gagggaggat 20760
tgtttgagtt taggaggttg aggttgtagt gagttgtgat tgtattattg tgttttagtt 20820
taggtaatag aaaaaaaaaa agtaatttta taattaattg agttattatt tttttttttt 20880
tatttaaggg gagaaaagtt ttgttgtttt tttagtttgt aagtgatttt ttagattgga 20940
atgagtatat ttgggttagg cgaggtggtt tatatttgta attttagtat tttgggaggt 21000
cgaggagggt ggatttcgag gttaggagtt taagattagt ttggttaaga tggtgaaatt 21060
ttattttttat taaaagtata aaaattagtt aggtgtagtg attagtgttg gtaattttag 21120
ttattcggga ggttgaggta gaagaatggt ttgaatttgg ggggtagagg ttgtagttag 21180
tcgagatggt attaatgtat tttagtttgg gtgatagagc gagatttcgt ttttataaat 21240
aaatatataa ataaaaatta aagaaggagt atatttgaga aagaaaagat ttgatgttgg 21300

```
tgaaagtagt atattttttt tgggattata tgttgttttt gtaggttaag aaatttaatt 21360
gtttggttag gcgtaatggt ttatgtttgt aattttagta tttcgggagg ttgaggcggg 21420
cggattgttt gagtttatga gtttaagatt agtcgggata ataagagtga aattttgttt 21480
ttattaaaat ataaaaagtt aggtgtggcg gtatgtgttt gtagttttag ttatacggaa 21540
ggttgaggta ggagaatcgt ttgaatttag aaggcggagg ttgtagtgag tcgagatcgt 21600
attattgtat tttagtttgg gtagtagagt aagattttgt ttttaaaaaa agaaagaaag 21660
aaagaaagaa atttaattgt ttaagattag tttaatttag tacgtatttg ggtgttagat 21720
agtgataata ggtagggcga gtgataacgt tgtttcgtaa tatttttttt ttgtttttgg 21780
tgagggtata gtttaaaggt agaggttgtc gtgaatttat tttttaaaag tttttttaag 21840
tttatataat taaataaatt taatatttga tttttatagt agttgttaat gttttatttt 21900
atttatagtt tttgttatat gtatgtgaaa tgggtttttt attgaaaggg attttgggag 21960
ttatttttga tagaggttta tggtttataa atgttatttt gggagttgtc gttcgggaaa 22020
tttgaattgg agaggatgta tttggggaag gaagtaggtg ggggtgatat agttgtaggt 22080
tgagtaacgt tgttttggtt gttagttgtt acggttaaat ttatttttag attataattg 22140
tgttaatata ttatggtaac gttttattga tagagtgggt gtcgtgtgtg ggttagggga 22200
tatgtcgttt tgtattttat tttagttaga ttttatagtt attttgggat gtggatgttt 22260
ttagttttat tttatagatg aggaaattga agtatataaa ggttaagtta tttttttaag 22320
gatatttttt tgtggtagaa ttataaggtg gtttgggagt tttttaata atttttttta 22380
gtcggttttt atttttttta tggggaaaat tggtattttt tagttttatg tgaagcgtta 22440
gttaaatata gttttaatag atttttttaa taatattgtt gttttaaaat atttttttta 22500
tagagatttg gagtcgtttt aaggttgggt taggtgtttt tgttttggtt attattttgt 22560
attgtaatta tggtttttgt tattgaggcg agttatttga aggaagggat tgtgttttat 22620
tgggttttat atttttatta tttggaattt tgtttagtgt atagttggtg ttttaggaat 22680
gttgttgaat gaattaatgt agtaaataag tggtagagtt aggggttaag tttaggtttg 22740
ttttatttta aagattttt aattatttta ttttgtttcg tgtaggattt aagtttttt 22800
atttcgttag tttttggatg aaagtgatat tatagtgttt tggtgaaggt ggtggagttg 22860
taggattttt tttgttatag aagggaaata gaggtttggg ggaattaagt gagagatgtt 22920
ttgggatacg tttagggtta tatggtttag ggggtttgag agggataatg ggtagataga 22980
ttatagggtt ggatgtagga ggtgtagggt ttaagttttg gtttttttat tattaggtag 23040
tatgattttg ttttgggttt tcgtattttg gttgtgaagg gagggtttgg tttagtgggg 23100
tttttgaatt ttgcggacgt attgttggga taggggagag tatgggagat tttgtttttt 23160
tggtagtggg ttttgtcggg gatagggttt tatttaatta tttacgttat gagatggagt 23220
ttggggatgg gacgaggagt cgggaaattt ttagtttttt tgaggagaga agttgatagg 23280
ggtatttaga gtttagattg aagttggaat atttatttta ttttaggagg tattaggaag 23340
tgatttttaa gtgttagatt atatatgggt tttttatgga gattttgaag tttggtttat 23400
tttagttttt tgagatagaa atgatttag tggtttagta atgtttttt agaataaaat 23460
gaagtttgtt tgatttttt atgttagaaa gataatttat tattatttg aaaggtagtg 23520
gatttggtat gggattattt agtttgtttt ttttagttat gtgattttga gtaagttatt 23580
tatttttatt ggattttagt tttttattt gtgaaataga gatattaata ttgattttat 23640
gggatttggg gagggttaga ggagatagtg ggtgggttag ggtaatacga atataaggta 23700
ttatattatt gttgttatgg ttgtaattag ttttggttgg agagttttg tttaaggagt 23760
ttggtaattt gggatttgtt ttatttgggg tgaggggatg gtattttggt attttgtttt 23820
attataaaat gtaatatgtt ttgtcgagtt ttttttgatt ggtaatattt aatttaagag 23880
ttattttatt gtagaggttt ttttcgattt ttttaattaa aattgatcgt tttttttgt 23940
gggtttttgtc gtgtttggga aataagattt ttgttttggg gattttggaa tatttgatcg 24000
tgtttgtttt ttatgtattt tttttattag gttatgattt ttaagggaag ttttgaatga 24060
attagtaagg taagatatag ggtgggggag agaataggaa agataaattt tagatataag 24120
ttgggggatg gaggggattt aggttttggt agaggggtta tgggggttga gggttggata 24180
aagtttaggg ttttttttt ttattatttt ttttttttat ataaggaaat tttatttatt 24240
tggattaatt gtaggatagt tgaaattata gtagatttaa aaattaaagt atttttttaaa 24300
tttttaagtt ttaatagtag tttgattttg gttttttata gtttttagaa gagttgttta 24360
aaggagtttt atgttttaat tgggttatag tggtttttac gggagtgagg gtttttagag 24420
ggttggggag taatgtgtgt ttttagagtt ggtatttgtt ttgtgatttt tattgtaacg 24480
tataaggttt tttgtagaat tatgaatggt ggtggtgatg aggggaaggc ggggaggttg 24540
ttaggtaaat ttatttaggt tttgtttaaa tgattatata tgttagtttt ataagagggg 24600
tttttaggga aggtgttttt tttttattag tttatatatt tttttttta aaaatttttt 24660
tattttgata tagtattata tttaaagtta taagtggatt ataaagaatt tttagatgtt 24720
ttttatgttg atttttaaa tggtgatgtt ttgttatatt tgtattattt ttaattttt 24780
```

tgggtttttt tttgtgtttt tttttgtgta tatgttttgt ttatgtatgt attgatatat 24840
atgtatacga tatatagttg tattatttaa tagtaagttg taggttgggc gtagtggttt 24900
atgtttgtaa ttttagtatt ttgggaggtc gaggaaggtg gattatttga ggttaggagt 24960
tcgagattag tttggttaat atagggaatt atttgaattc gggaggtaga ggttgtagtt 25020
agttaagatt gtgttattgt attttagttt gggtaataga gtaagatttt gtttaaaaat 25080
aaataaataa aaaaagagta agttgtagat atatgattta tatgtttttt tgtttttaag 25140
tattttagtg tgtgtaggtt ttgaaataag gtattggttt atatttgagg ttttagatgg 25200
ttaatgaagt tgatttttag tagagtagtt tgattagggg tagttttgaa ggttgttgta 25260
agggggttaa ttttttgagg tagttgagag tagttatttt tttgtgattt tttttttttt 25320
tttttgattt tgttttttgt ttgtttgttt tagttttatt tatagtattt attttaggag 25380
ggtttagtag gtatgtttta ggtatgagat ttaagtttta gttttgtttt ttattagttt 25440
tttgtttttt tttttttttt tttttttttt ttttgagata ggtttttgtt tttgttattt 25500
aggttagatg gaattttggg gttaagtgat ttttttattt cggtttttta aaatgttggg 25560
attataggta tgaattatta tttttggatt tttttaatag ttattgattt ttagtaaggt 25620
atttattttt tttggtttta gtttttgggg ttataaaatg gaaatttggc ggttgtaaag 25680
aggttggaat tttattggtg gtataataga tgttttagta ttttgtaaag tataaagttt 25740
agtttttggt tttgtttagt ttgtttgttt gtttttttgtt tttgcgagag tttagtatta 25800
attttttttc gtttttggat tttgggaagt ttattttatt tttagattaa gagaaaaata 25860
gttttttttta tttattttttg ttattgtagg aagaattgag agagttttttg tgtgagggcg 25920
gagtagtgga ggacggggtg gtagattttg tttcggcgga tgtttgtttc gggaagaatt 25980
tacggggaag ttggtaataa gtttgttagt tatatggaaa gtcgttgggt taaacgatag 26040
gggtgtttta gggagttttt gtaaggtaaa ggtttttgta agttagttag tttttgagt 26100
atttattgtg cgttattttt tttggagggt taaggaattt ttatgggttt ttaaagggag 26160
gttatttaag gaatgaaaaa gagtttaggt tttagtttgt ttgttattta gttattagtg 26220
atattggtgg gttttttttt tcggcgtttt attttgatcg tttgtttaat gaggatgttg 26280
gggttatagt gtgggaattt tgaggagatt ggaatgaggt aggaatgtga aaatatttag 26340
aattagggga ggaaagattt ttgtaaggaa ggttagcgat tcgttttatt tattttttttg 26400
tttttgtag tgttggaatg gtggtagacg cgtagtaggg ttgaattatt gtttgagtgg 26460
taaatttttt tagttaggat ttgggttttt aaataaacgt ttagaaattt tattaggtat 26520
tggggtttta ggattattat ttttcgttta gtgattttat tggaattgta gtgtttttttg 26580
gtggttagta tgtataattg tagttttgat tttggtttgt tgggagttgg gttttggaat 26640
ttgaatgttt agttttttgg gtaagtagtt agggtcgggg attttttttt agttttggtt 26700
ttttataggg ttgtgttttt ttattttttt tttttatatt ttttttttggt aggaatttat 26760
gtaatgttgt tgagttgagt aaatagaagg taagaggttt gtttagggtt agggagttgt 26820
cggggttatt tttggttttt tttgttttgc gttgtggttg gtttttttcga ttttttttat 26880
tttgaaaggt tttttttttc gattttatgt agtagttttt tgatataagt agtgtgtgta 26940
ggggattaag gattttttgt gtttggagtt tagttttagg gtttggggtt tttttttgta 27000
gatatattgg ttattttttag ttgtgtttgt agagttgttt tagttaaatt agaggtagaa 27060
ggagtttaga ggagtttttt ttaatttagg tatatatagg tatttgtatt aattttaggg 27120
gtgtgttttg agtttttatt tatacgaggt ttaggtttta gtagtatatt tgggaggttt 27180
attttgatta gtttatagtt tagggaggga gtaggtttat gagtcggtgt tgtaaagtag 27240
gggatttagt gtttaagagg taagggaggt tttggaggaa gaagggtggt ttttggttga 27300
gttttgaaag tttaggagag ggaatttata gtagaagaaa ataaaaaata aaatgtttta 27360
tagttaaaaa ataaaaaaaa aaaaaggaaa gataagattt tatttttatt tatcgtgttg 27420
gtaaagattt taaaaatatt ataatgatta ttattggttt tgaagtggga ggggatatcg 27480
tggtagttga ggatattata tgtttttgga agataatttg gtcgtgtttt ttagagattt 27540
ttagatatgt ggataattga attttatatt tttagttttt ggagtttttt ttaagggaat 27600
cgttatgtat gtattgagag atttaaatta tagatttgtt atgattttaa ataagtagaa 27660
atttgtgagt ttaatagaaa aggatggatt aaataaaata gtgattgttt tcgttacgta 27720
attattaagg atggtgtggt agaagaatat agaatagtat ggaaaggggt acggaatatg 27780
gttagatttt attaaaatgt ttataggttg ggtacggtgg tttacgtttg taattttagt 27840
attttgggag gtttaggaag gtagattatt tgaggttagg agttcgagat tagtttggtt 27900
aatatggcga aattttgttt ttattaagag tataaaaatt agtcgggcgt ggtgttgggt 27960
gtttgtaatt ttagttattt aggaggttgc ggtaggagaa tagtttgaat cggggaggcg 28020
gaggttgtat tgagttaaga gtgtattatt gtattttagt ttgggcgata atagcgagat 28080
tttattttaa aaaaaaatat aaaagtttat aaagtaatgt ttttttttgtg tgtgtttgtg 28140
tgtgtgtgtg tatgtgtatg tgtgtgagtg attttttttt ggtttgtttt gatttttttt 28200
taatttttta tatagaatat agattatatt tgtaatgcga aagaaaagtt ataaaagtta 28260

```
tagagaaagt tatttaggtg aaagtagtag ttagttatag ataggagagg tagagagata 28320
tgttaggtag aaggaagggt gttcgtagga gtgcggaagt ttgaagatat tcgtgtagtg 28380
cgttgtagag cggaagtagg tttttgaatt ttaagttaga tggaaggttg gcgtgtaagt 28440
tcgtgagttt ggtggggatt agttttgttt tcgggatttt gaatgttata taagtttgat 28500
gtatagggtt ggtagattta gtaaaaagta agtaagtaaa taataaaatt aggttattta 28560
gttaaatttg tatttgaata atgaataatt tgtaggataa aagtattttt tgtgttatat 28620
ttgggatata tttatattgt ttaaagtgtt aattgtttat ttaaaatttt agtttaattg 28680
agttttttta agtggtgttt gtttttgtt tttataatt ttatagtata gagtttagtt 28740
cgtttttaga gtatgtatta tttaggattt taattattgt ttttagatat tttaatttgt 28800
ttttgggaaa ataaatagaa aattaattta gattagtttc gtttcgtttt ttaaaaaaag 28860
aaaagaaata aaattaattt atagatttat attgttattt ttggaaattt gtagtatttt 28920
ttgaggataa atgttttatt tttttttttt ttttgattaa aatgagattt atttgatata 28980
aattgaatat ttttgtatgt gttaaaacgt tatttttttt tagtattatt ttatttattt 29040
gtttattaaa tgtttattga gtatttattg tatgttagtt atgatattag gtgttatata 29100
gtagggtaaa taaagtatat atgtttagat atattttttg ttttatggga gggagagaga 29160
gagagataat aaataagggg aaaatagaag aataaataaa tatttaatat tggcgcggtg 29220
gtttatgttt gtaattttag tatttggga gattaagacg ggtggattat ttgaggttag 29280
gagtttaaga ttagtttggg taatatgagg aaatttcgtt tttattaaaa atataaaaat 29340
gagcggggtg tggtggttta tgtttgtagt tttggttatt taggaggtcg aggttcgata 29400
atcgtttgaa tttaggaggt gaggttgtag tgagtcgaga tcgaattatt gtattttagt 29460
ttgggtggta aagcgagatt ttgttttaaa aaaagaaata acgtgtagaa taaagattat 29520
taggaaggat tttttattta tggaaatatt atttttttatt taattaattt tttattgttg 29580
aatattttga ttattgtcgg tttttttata tattaaatta tgatatgatg aacgtttttt 29640
ttagtgaatg tttattatta tttattgagt aaatttttag aagtgagatt tttgggtgaa 29700
ggtatataaa tttttaattt agtttttgtt ttatatatat gaatataatt ttgtattttt 29760
tttttagtta tattttttaa aatttgaaaa taatatgttg taaatttttt tttttttttt 29820
ttgagatgga gttttgtttt tgttgtttag gttaaagtgt aatggtatga ttttagttta 29880
gtgtaatttg tatttttcgt gtttaagcga tttttttgtt ttattttttt aagtagttgg 29940
gattataggt ggttgttatt atgttcgacg aatttttgta tttttagtag agatggggtt 30000
ttatcgtgtt ggttaggttg gttttaaatt tttgatttta ggtgatttat tattcgtttt 30060
tgtttttttaa agtgttaaga ttataggcgt aagttattac gtttagttgt aaaaaatttt 30120
ttggtatata tttttatgag ttttaatata tttatgtata gatttatgta tttattgtta 30180
tagttaggat atagaatagt tttattatat tagtgtaagt tttttgtaaa taaatttttt 30240
tttttttttt atatttggta attagtgatt tgttgtttag agttttcgta gttttgtttt 30300
ttttagaatt tatgtgttag ttgagtgtag ttgtatttta tgtaaatttg agattggttt 30360
tgtttataga ttttaaggtt atttttttaga tttatttatg ttattgttaa tagttttttt 30420
tttttattgt ggagtcgatt tatggtttgg atagattata gtttgcgttt ttattttttt 30480
gttaaaggat atttgggggt gtttttattt cggggtgatt ttgaatagag ttgttgtaga 30540
tatttgttta tgggtttggg tttttttttt tttaaaataa gttattattt ttgtagggta 30600
aatatttagg agtgggattg ttgggttatg aggttagtgt tagtttaatt agataagata 30660
tcgtttaagt gtttgtattg tgatggatgg gagtttcggg tgttttatat atcgtcgtaa 30720
tttggtatta ttaggtttta tttttattgt gataggtata tagtggtatt ttattgtgtt 30780
tttaatttgt atttttatag tgggtaatga ggttgagtat attttatgtt tatttgtttt 30840
ttgtagattt ttttgggtga agtgtttgtt agaatatttt gtttattttt tgttaaatat 30900
tattattaat tatattttat attttattta gattttattg gtttttttta atgttttttt 30960
attgttttag tatttttttt agaatatatt gattatattt agtttttatg gtttttttggt 31020
tttttttagt ttggggtagt tttttagatt tttttattt cgagagtatt ttagggtttg 31080
ggttggtggt tgtgggtgag taggatggtt tgtgttattg gttagtttga ggattgttat 31140
ttttatttta ttttttgttg aagattggta gtttttttgag ggtcgggata ttgtttgttt 31200
ttgtaaatag ttgtgtttcg tgtatgtgtg ttgaaattta agtttagtta ttattatttt 31260
tttggtatta tttattggga ttggagttaa attagggata tttaagttga gttttggata 31320
gattttagtg ttagatgagg tggggtgggg acgtgattaa tggttaaatt ttaattttcg 31380
tttttatttt tataggatcg tttggatagt agtgggttta gttagtgttt ttttaatagt 31440
atgaaagggt tttatgttgg tttgtttttt ttaaggagat tatatcggtg tgtgtatttc 31500
gttatagggt gtgtggggtt agtttattcg agaatagagt aagcggtatg tttttttataa 31560
agtagatgtt tggaggaaaa atagatgtaa ttaaaattat ggaggataaa atgattgaga 31620
tttttttagcg ttatatgtat gggtttgtgt acggttttat ataggtttat attatttatg 31680
tttatattta tttacgcgga tttgtattta tatataattg tttacgggga tatatatttt 31740
```

atttgtgtgt ttgcgtttga ttatataagt atgtatattt tataaattta tttataagta 31800
attatatata tgttttttat atttatttat aatggtagat ttgtgttata ggttttcggg 31860
tgtatatttg tatatataga tgttattagt attttttttt taaaatggtt ttagaaggtt 31920
atttttaaa ttacggttaa aaaaaaaaag aaagaaagaa aaaagaaaaa ttagttattt 31980
ttggaaattt tttaagggtt tttttgtttt aaaaaggtaa aattagatta gagtgatgtt 32040
attgggttga attaggatag atgggtagag gttaagggaa cgaagtaagg ttttttattt 32100
tatgtatttt tttagttgaa atttaatagt taggggtttt tagtattaga aacgatagtt 32160
tttagtggta cgggggtttt tttaggggga gttttagtag aggttgaagg gtttagtggt 32220
tttcgattga gaatataatt ttgaattgaa tgttttttat tttattaaaa gtagtttatg 32280
gtaataatgt tgatttagga ttgtttgaag agtattggat attatgatgg ttatattgcg 32340
tgtataatat ttttcgtgga gtaggtattg tttttagtga tgtaggttta ttatttgatt 32400
gatggtttta agtagttagg ggtttatttt tagattaaga ttgggaagtt gtagattggg 32460
attatattgt tttggatttt atagttttag ttttgggata aatttattat gggaggggta 32520
ttagtataga gttttttcgg gtttataagg agttgtttta gggaggatgt gagaagttta 32580
atttttggcg ggtggtttgg atttacgttg ggcgttgttg ttgtttagtt ttatgttagt 32640
acgaggtgtt ttaaggtttt gaagaaggtt tgtttagtat ttaggtaaat tgagagtttt 32700
ttggcggttt tttttatttt agtgtggtgg tagggtgggt gtggagttag tttttggtag 32760
attttggatt gggtttttgg attttggttt taattttgga gtcgttttta tgaagttcgt 32820
ttttgttttt ttttttgttt tttgtttttt gtatggagat atcgtttaag gtttgtttgg 32880
gttttttttt ttttttgttt ttcgtagtga tttttgttgt ttgtgttgtt agttttgaat 32940
ggtggtattc gttattgcgt gttttttttt aatgtatttt attcgatttt tttttagtg 33000
ggtaggagga tgaggcggtt ggtttattta ttcgagagat attttttgta tatgttttat 33060
agaatatcgg tttattaaag gtgtatatag atatttggat agagcgagag tttagtgatg 33120
tttgttagtt agaaattgtt tatttatttt ttagtaattt tttattgtat ttttgttatt 33180
tgatttattt tgtgttgagt tttaggttga gaagggataa acggtatgag gttttagttt 33240
ttggggaatt gatattttat aggaggagtt ggatattcgg ggtttgatga gattacgagt 33300
tagtaaatat aggtagttag tggagagggt gagtagagga tggaaaatag ttataatttg 33360
ggagggtttt ttggaggagt attggatttt ggaggttagt tggaaggagg aatatgttag 33420
gtaggggttc gagatgggga tttgggggat taaggttagt ggtttttttt ggttaggagt 33480
agttagtttg agttatggga tttttatttt tagtagtcgt tttgttgagg agggtatttt 33540
gcggggagtt agatttgatt agtatttagg tttttagtta ttatgttgat attttagtta 33600
gttcggggtt attttttttgg agagtaattt gttggttttg tttagggttt agttttattt 33660
tttatattat ttttgttatt ttttgtttta tataggtcgg agaaatttag ggttattcga 33720
ggaatagtag tgggaattgt agaaggaagg gagatttaga ggggtggtag tagtttttta 33780
atgttcgtag ggatagggtt gttatgggga agggcgttgg ggggttagaa ttggaattag 33840
tgaattagat ttataggaga tagatatggg ttgggtttaa agatgagtag ttttgttgtt 33900
ttgggggtag tgagtttttt gttattttag attggaatta gcgttaggtg gagatgttgt 33960
agggtggcga gaggtggata tagatagttt gagtttgggg ttttaggatt tagattttga 34020
gagggttgtg tttgttattt tgattagttg agtggagttg tagaatttta gggaattttt 34080
ttttgttgtt taaatgagga atttagtttt agaatttaaa gtaagttgat ttaagatagg 34140
ttagaaggtt tgtttttttt ttttttgagg aattaggagt agcgtttttt tgaatttttt 34200
ttatttttga tataggtata tttttttttt tttttttttt ttttaggttt ttagggatag 34260
ttttggttgt tggtgagtaa gggaggaggg atttttaatt atttcgtttt ttttatttgt 34320
aggtgagatt ttagagatga aatgggttat ttgaagtttt gagaagagcg ggggtcgagg 34380
ttagggtttt taatttttgg tttggtggtt ttcgtatatt ttgtgttatt tttatttttg 34440
tttgtttatt tggttttagt gggtagttgg aggtgggtaa aattggattt ttatttgtta 34500
ttatgaagat tggatttttt gttatttttt ttgaaaggtt aagagttggt ttagtgttga 34560
gaggttgttt ttaggtcgta tttttatttt tttattttta agggttggtt ttgtttttta 34620
taagatggat agagtaggac gttttggtta tatgaggttt ttgggttttt tttgtttttta 34680
tttttttttt tgggaataag gtttaagatc gtaggggagg gagtagaggg taggttagga 34740
gttgagaggt tgtgttttgt tcggaattgg ggtatgtttt tttttttttt tggttttagt 34800
tttttttttag ttaaaattag gagtttaggt tggtttatat taatttttag tttttgtaat 34860
atggagtttt tattgttgtg tagggtggtt ttaattttta gtaagcgttt atggggttgt 34920
gagatggggt ttggggatta gagtttaggg tttagtgttt atttttttta gttttttatt 34980
tggtttttag gaagttgtaa aatagggatg gttatttttg tgaggttggg tggggttttt 35040
aaaaaaaaaa aaaaaaaatt taaaatataa aatgtatttg tttgttagga agataaatat 35100
ttttgttgaa agtttttggt ttagttgtta gttgtagttt gtagaatgtt gttattgaag 35160
ttatggttgt ttgtatgtgt ttgggtttat ataggtgtta tgcgtgatgt cgttgggtcg 35220

gtcgtgtatt tttatggcgt ttgtattgtt agtggttttt aggagtagtt ttttgaagtt 35280
gtttttaggt ttattttggg tatttatttt tatgtttttt ttttgatgtt ttggagaggg 35340
aggcgagaat gttataggtt aaatgttttt tttagttttt ttagtgtgta tagaaagtta 35400
ggaatgagac gagtttgaag ttttagtttt taggatttgg atggagttag aagatttgga 35460
ttagaggtat tattttgata tttaatgttt atcgtttgga tttattttta gaagaagaaa 35520
tcgcgggtta gagtggttga tgtttatatt tattatacgt gtttgttgtg atgattaaag 35580
ttcgtgtacg tgaaaggtcg tgttaaatgt tttttaaacg ttttattaat tgtgtttgtt 35640
ttttatttta ataagttttt tttttttaag ttatagataa ggaatatagt tttcgaggag 35700
tttatattta tatttgtaga tatttagttg gttgtttttt tttaggtatt tgtgtacgtt 35760
ttgtatatgt ttttgtgttt aagggtttgt atgtttggag gattttttta gggtcggagt 35820
tttttaatgt ttatagggtt tgtgggtttg taagtttaga ttagaaatgt gaatattaat 35880
aatggtaaaa ttatatagtt agtatattag tttttttatt ttttatagtt attttatgag 35940
gaagatttta gaagtatttt tatttttatt ttatagaggg ggaaattgag gtatatagcg 36000
ggtaggtaag tttttttgag tatatagttg gggagttatg gttataggat ttatggcggg 36060
gttattaaga gtttatgatt taaattaggg gtttttagtt tttaggttgt ggattagtat 36120
cgatttatgg tttgttagta atcgggtcgt atataggagg tgagcggtag gtgagtaagt 36180
attattgtaa gttttggttt ttgttagatt agcggtgtta ttagattttt atagaggcgt 36240
gaattttatt gtgaattatg tatgtgaggg atttaggttg cgtgtttttt aagagaattt 36300
gatgtttgat gatttgaggt ggaatagttt tatttttaaa ttatgtttta ttataatttt 36360
ttttatttat ggaaaaaatt atttttata aaattagttt ttggtgttaa aaaggtaggg 36420
attgttgttt taaattttt gttaggaggt gagtgtgttt gtttaggaag tttattaggt 36480
tgtgtggtgg taaagtggtt ttttgtttta tttttttatt ttagtttta ttttttttt 36540
tttgatgtgt atgggttgat tcgtttttgt agttttgagt taggaatgtg tcggtgggtg 36600
gggggtttat ttgttagacg tagtggttgg gagttgtgtt tatgtatatt tatttaggtt 36660
cggggttagc gtgttgggga tgaaggtgtt cgggtagttt tagtaaatag tgggtaggag 36720
ttgtttgtaa taggaaatgt ggtttatgtt ttaattttat gtatttttaa ggaagaaaga 36780
tttttttttt gaataatagg tgagattagt gtgtagggtt gggggtagg gaaggaggtg 36840
ttgatttttt agtattggat ttttggggta gagtttttat agtttttgtt gaatttttgg 36900
tgggattagg ttaggaaagg aggggtattt ttgtttgttt taattttggg ttttttttat 36960
tttaagtttt attgggttag atatggttgt tgaatgagtt ttttttttat ttagggaggt 37020
tatggtattg gggttagaat tttcgggttt tagaaaagtt ttgatgtagg gaattttttt 37080
agagagagga gtttagggtt agtgaggtgg ggattaaagg ttatagaggt tagattggtt 37140
gtttaggtt tatataatat tgttgttgga agggatttag tttaagatga tggtgaagag 37200
gtttagattg ggaagagat ttgtttaagg ttatatagta agttaatggt aagttagaat 37260
tagaagttaa gttttcggat ttcgtattta atgagtttta agaggaaaaa ggtagggggt 37320
ggttattcga gtgtttttatt ttttttttga tatttttttt tttttgtttg gtttaattat 37380
tggtttttga ttttgttttt ttttttttta gttaaagaga gtttttttggg tgagttagag 37440
ttatttttttg attttgatat tgtggggatg gatagtagtt atttgagtgt taaggaggtt 37500
ggggtgaagg ggttttagga tcgggttagt ttagattttt ttagtttatt ggagaaggtc 37560
gatttagaga gtaataaggg taagaagcgg cggaatcgga ttattttttat tagttattag 37620
ttggaggagt tggagaaggt tttttagaag atttattatt tagacgtgta tgcgcgggaa 37680
tagttggtta tgaggataga ttttattgag gttcgcgtgt aggttagtga gggttatttg 37740
ggagtgaggt tggtaaagta gagtttgttt ttgggttttt ggttgtagtt ggggtttttt 37800
gattgtttat taattatatt atggtttttt tttttttagt ttattgagaa tttgaaagtg 37860
ggggttttta gtttgttttt tatgtttttg ttttaattag agcgattttg tttttatttt 37920
ttttaatatt gggtttttgt attgtggtgg ggggttgtaa aggaaggagg gtttttagta 37980
gaggggtagg aaggttgttg ttggttgttt ttgggtttta tatagtgtaa gagttttggt 38040
tttttttatat agagaagggt tatttgtttt tgttttagaa ggtttatttt ttagatagga 38100
aataggataa atatgaagat aatgtttata gtattttagt gggtagaaat agagaatagt 38160
ttagtttttt tttttgttta gatggttgta ttttttttta tttttttaagt tttaatttat 38220
atttttttttg agaagttttt ttgattattg tggttaaagt agtagttttt ttattattag 38280
ttaataatta aaatatttaa tttagtataa tatagtggaa tttaatattt aatttaattt 38340
attgtaattt aatagaatat aatttaatag aatttaattt aatataatat aatatagtgg 38400
aatttaatat atttaattta atttagtgta atttaatata atataattta atagaattta 38460
atttaattta gtataatata atggaattga atatatttaa tttaatttaa tttattttaa 38520
tataatataa tttaatataa tatattttaa tggaatttaa tttaatataa tataatataa 38580
tggaatttga tatatttaat ttaatttagt gtaatcgaat agaatttaat ttaatttaat 38640
ataatataat ttaatagaat ttaatttaat ttaatataat ataatggaat ttaatatatt 38700

aaatttaatg tagttttttt taatataata taatttaata taatatattt taatagaatt 38760
taatttaata taatataata taatggaatt tgatatattt aatttattta atataattta 38820
atagaattta atttaattta atataataga agtaaattag tttatttttt gttatattgt 38880
tttattttat tttttaaaa atttttaaa ttttttttgt tttttttta gagatagggt 38940
tttattttat tatttaggtt ggagtgtaat aagatgatta tagtttattg tagttttaaa 39000
tttttaggtt aaagtgatta ttttatttta atttttaaa gtgttagaat tataggtatg 39060
agttattatg tttgggttta ttttattttt tgggtatttt tgtttatttt ttttaaagag 39120
attgggtttt tttttgttat ttagcgtgga gtatagtggt gtgattatgg tttattgtag 39180
ttttaatttc gtgggtttaa tggatttttt cgttttagtt ttttgagtag ttgggattat 39240
aggtatatat tattacgttt gggataagtt tttttatttt ttgtagagat ggggttttgt 39300
tttgttgttt atgttggttt taaatttttg gttttaagta atttttattt ttagtttttt 39360
atattgttgg gattataggt atgagatatt gtgtttaatt tttattttgt ttttttata 39420
ggattgttga ttggtttacg ttattttatt tgtttgtttt tttttattaa attgtgattt 39480
ttttgagatt aagtattgat tgtttattat tgtattttta ttaattagag aaagttaaac 39540
gtagagtatt aagtttaaaa aataagtatt cgtggattaa ttaattttg tttagagagt 39600
tgtatttgtt tcgattagta tgaatttata gtaaagaaag gattagaaag aaagaaaaga 39660
tttatgagta gttaaaatat aggtgataaa atttatatat ttaagtttaa gttttaattt 39720
aatataaaat aaatgttata attagttcgt ttttttaatt taaagtatgt tggaaatagt 39780
aaataaaacg ttgttatata gtagtttttt ttttttagt gggttagaaa aattaatagt 39840
tttagaataa agagaaattt ttttttttt ttttttttta attagtaaag cgttatgtag 39900
tttagtgggt tgagtttttt aatgttagga tagagatgtt ttaattttgt aatttttagt 39960
aggttttagg gatttttgaa ttaatttatg aaagtatttt tagtagtatt gttttaaggt 40020
attagtaaaa gatttattag ttataattat tttttgttta agaaggtaga ggtattttta 40080
atagagttta taaatttttt ttttaaaatg gttaatgtgg aaaaggatta gtggttatta 40140
gtaaatatgt agttgtttag aaattgtagt tattagaagt gatgttgaag agttgtacgg 40200
ttgagttgtt gtaagttttt tgagggtagt ttgttatttt tttttattt ttttattttt 40260
tgagatagga ttttattgtg ttatttaggt tggagtgtag tggtgtgatt ttggtttatt 40320
gcggttttta tttttgggt ttaagcgatt tttatgtttt agtttttaa gtagttgtga 40380
ttataggtat gtattattat atttagttaa ttttttttat tttttggtag agatggaggt 40440
tttattatgt tggttaggtt ggttttgaat ttatgatttt aagtggtgta tttgttttgg 40500
ttttttaaag tgttgagatt ataggcgtga gttattatgt ttagtttaga ttgttttttt 40560
ttttttaga tagagttttg ttttgtcgtt taggttggag tgtagtggtg taattttggt 40620
ttattgtaat ttttgttttt cgggtttaag cgattttttt gttttagttt tttgagtagt 40680
tgggattata ggtgtgtatt attatattcg gttaattttt gtatatttaa tagagatggg 40740
gttttattat gttggttggt tggtttttaa tttttgattt taggtgattt attattttag 40800
ttttttaaag tgttgggatt ataggtatga gttattgtat ttggtaagat tgttatttta 40860
ttttttttta gtgtttaatg aatgtttgat gaaggatgat agggtagttg gggttttttt 40920
gtgaatttag aagtttttat atatttagat tttatttgaa tttggataat tagttattta 40980
tatgatttta tatgtttgag tttggatagg aattttagta tttagtttga tttttagtaa 41040
atggattggg gaggatttat aggtgtaggt agagaaaggg gtatagttgg ttatgggata 41100
gttttgtagg tgttgtgttg gtgtgggata aatttgtttt tttttgtgt agtatgtgtg 41160
tatatttgtg tttgtatatg tgggttgtat gtatgttttt taataaatat atgggagtgt 41220
tcgggagtag aatttattaa gtattgaaag aagaatgacg ttttgagtat aaaatatgaa 41280
aataaaggcg tatagagtat tttttagttg ttattatata attaaaatag agtgtttttt 41340
gtatatatat atgcgcgcgt atatatatat atttatattt atttttaaaa tggagttgag 41400
gtagtttata attaaagatt tatttgttta atttaaaaat aagaataatg gtatgaaatg 41460
tagaggtaat gtggtaaaaa aaaatttagg ttaaggttta tggttgttat tgagtataaa 41520
ttttaatttg gagtttttttg gtagttaagg tagaaaggga aattttgacg tgttgtatat 41580
tttattattt agtgggagga ttatagaatt atagaacggt agtgttggaa atgattattt 41640
tattttttta tttgatagac gaggaagaag aggtttatag gtgggaagtt ataatttgaa 41700
gttaatagta gttatttagt agtagtattg cgattggaat tcgggatttt tgagtcgtag 41760
tttagtgttt ttttgatttt agtttgttgt ataaggatat atgttagatt gtttagaggt 41820
ttttttttgt ataaaggtag tagggtgggt gggattttta tgtgcgttag atttttgggt 41880
tgagttttaa gtttggtagt gttttggggg ggtaaggggg tggtgatgga atttttttgtt 41940
tgtttttgtt gtgtttgttt agggtttata tttagttggt ggtaagttag gtgtttggcg 42000
tagagtggtg gtggggatag ggacggggta atgggggttt taagatggat ttttttggg 42060
aagaagttta gggtttggtt ttgttttagg gggttgtagg ggtttttttt tgttgtttgg 42120
gattttaatt tttgtagttg gtgtttgaag ataataagat agaggttttt tcgttttggg 42180

ttatttcgtt ttggttttag atgggttgcg gttagcgagg ttttcgttaa gatagttggt 42240
tttttgtata gttcggtttt cgggagaggt ttgtgttgag tgggtttagg ttggggtggt 42300
tttgaggtat gatatttaga tcgggagaat agtggggatt tcgtggttgg ttgttttttt 42360
ggggttgtat agtggagata ggtttgcggt gtagagttgg gaggtatagg aacgggcgtg 42420
ggggttggtg tggtatttgg aagggagttt tcggagtttt tggggcgtta ggggttagaa 42480
atggatggat attttaagtt ttaggatttg tagatgtgag tatagaaggg gtcgtgagga 42540
tatttttgtt gtttttaatt tattggggag gatattgagg atcggttagg tttatttagt 42600
ttatgtttgg atttgggtag aaaagttaga attgggtgcg tagggtaagg ttttaggata 42660
aaggaatagg agaagaagga gggaggaggt gggtagtttt gtttttatcg gtcgtttatt 42720
cgtttttat tatttttatt tttagttttt aaataaattt tttagtttga ttttttttta 42780
tttttgtttt aaagacggtg ttagttttgt tggttttttt ttcgtttggt tatttgtttt 42840
ttttagataa tttatatttt tttttttttt atatttagtt ttagcgagtt cgcgttttat 42900
attttatttt tttatatggt tttttttttt attttagatt tttttgttag gcgttggtta 42960
tttttttttt ttttcggggt ttttttttcgt ttatttttgg atttttttgtt ttagagtttg 43020
tagggatatt ttttattttt atagttttat tatggggatt tggaatttat agagatataa 43080
ggagaatttt aatagttata gtgataaaaa gaaaaagaga agttattttt tttcgtgttt 43140
ttagtggttt tttttttttt tttagttttt tttatagaaa attttgggag gttgaggaag 43200
gagcggaaag acggagttaa gtttgtagga aattaaatat gaaatgaaaa taagtttttt 43260
tgatttattt ttttattttg gttttagttt ttttattttag gttattgata aagagggatt 43320
ttattttagt agtatagaga ggagatttta tttgtttttg gtttttagtg ggtagttagg 43380
gtttggattt taggaatttt tggaggaaaa ggggttttgg gaggttaggg taggggttga 43440
gattttaaat tggggagagg gttcgttttt aaggaagagg tttttttttt tttagttaat 43500
gtgtgtgtat atatgtacgt gcgtgtagtt atattagtgg gtatattcgt gtacgtatat 43560
atatgtttag gattagggag gggaaagtga gttggtatag gagtggagaa atataataat 43620
gtatattttt gtttattttt attttttta tagtaatatg agatatattt ttattttttt 43680
tgattttttt ttttagtaa aagatagtga gaaaggttag gagttgtaaa gaaataaatg 43740
ggaagtattt taaatatttt tggattgtat tttttattta ttttttttgt tgcgattatg 43800
aggtattatt cgttatgtag aaattgaggt ttttggtaat attagtgttt ttatgagttt 43860
gtttattggt tagttatatt ggatgtttaa agaggttagg tagcgtttgt gaggaagtac 43920
gtttatattt tttttttttt ttttcgtagt ggtttttagg aagtttaaag gttttgaggt 43980
agaggaattt tgagagttga tttttatatt tagtttagta gttgtattgg tcgtgatgga 44040
tggagaaatg tttttatttg gagagtaggg attggatttt aggaaagttg gtttttttga 44100
tgtttgggtt tggaagggtt tggtaaggtt ttttatggtt ttgtagttga ggttattgtt 44160
atggttttgt tttttcggtt tgtagcgtgt tgtttgaggt gtgtttattt gtatttgagt 44220
ttgtaaagtt agtagataag tgtatattaa gttggtagag gttttttttt gaggagtatt 44280
acggtttatt tttgaattag tttaaagtta tttttggttt ggaagttgat tttttttttat 44340
tttttttttt ttatttattt cgtttttatt attttttttt tagattttaa gttatgggtt 44400
aggggtaggg tagtttttgt ttttgagttt tgggaaagtt tttagttttt tagaaatgtt 44460
cgtgtaaagg aaaaggattt aatatttttc gttattaaaa attttagtgt ttatagttga 44520
tttagagatt tttttttttt cgttgggttt ttaattaggt taatatttta tttatttgtg 44580
tttagtgacg ggttggtttt tatttttttt tattattagg ttacgttttt atataagatt 44640
ttatagtaat tgtttttata gatagtatta agggaaaaat tttttatttg gtagttaagg 44700
ttttttgcga tttgattttg tttttttttt ttttatcgtt ttttttaaat tgattttttt 44760
aggtttttgg agtatgtttt gttgttttta aatttaggtt ttggtttttt ttttttttagt 44820
taattattta aggtttagtt tttgtttttt ttttaggaag tttttttttga ttgtttttgt 44880
tgtttagtag agtttttgtt ttttttttaa tataggggag ttttgagttg ggagattgtt 44940
tttaataaga aggttgtaga taggatgtag ggaggaaggt attgtgaatt tggattaata 45000
agttttagag agttatgaat tttttggaaa attgtggtaa aattgtgtgt gattgtttag 45060
gagaatatat ttggaggagt atttgtagtt tttattagtt tttttatagg agtttgttta 45120
gatgattgtt aagatgatta gtgatttgtt ttataggtgg tgatatttgg ttgttttaga 45180
tttttaagtt tagaaaaata aaagtttggg tttaggatta cgtttatggt tagtgggggt 45240
aagatatttt tttagtttag agtggatgat tttagattgt tagtttttga ggataaagat 45300
taaggttgat atttatgtgt ggtcgtatcg tttttttaaac gtattgtaat tgatgggggg 45360
tttaagggaa tagtttgtat tgtttgaaag tgaggttttt aggcgggtat tttattttgg 45420
tgataatggt tttttcgcgt tttgtaggtt tggttttaga atcgaagggt taagtggagg 45480
aagcgggagc gttttgggta gatgtagtag gttcgaattt atttttttat tgtatatgag 45540
ttgttttttt ttattcgagt tgagaattac gtttaggtaa gtttcgtttt gagttgtcgt 45600
tatcgatttt tagtttttgg tgttttgaga atgagttttt tggaggagag tttaggtatt 45660

tcgagatgat ttatggtttt aggttgtttt ttcgttttat tttaggggtg tgtttgttta 45720
tattagagtg tgggaggttt agggttaatt tatttaatta attattgaat taggtttggt 45780
tgaattttag ggaagggaag gttggggtag tgagaacggt ttttttttt attagttttg 45840
gttatttttg ttgtgggtat cggaattaag gtaggtagaa ggttattagg tttttagata 45900
tcgttcggag ggttttttta gaaagcgagt tttgaaattt ttaggattcg tggttaagtt 45960
ggaatgagtt tgggattttg agttcgggga tttaggttat ttttttagtt ttaggttttt 46020
tttattttag gtaagtttta aatttttgag ttttagtttt tttatttgta ttttggatat 46080
ggtaaaagta tttgtttagt tttgttgtta ggtagttgta aggtttaaat ttaattatgt 46140
aaaagtattt tgtgaattgt atagttttaa aaagtttgtg tgtttgtgtt tagtttttgg 46200
ggttttgttt ttttttttt ttagagtatt ttttttaggt gtttgttagt tagtttttttg 46260
ttttagtgtg agatgtaagg gatgagaagt attgagttat attaggtata tgggatgttt 46320
agagagatgt gtttttggtt aagtttaagt ttttttttga tttttattta gtttgtttag 46380
tagaagcggt tgttttttat ttgtttaggt tttattttgg tatggttttt attttagttt 46440
ttaatttggt tttgtttagt agttagttag tagtacgggc gtttttgttt tttttatttt 46500
tttttgattt ggttttgtgt tagtaaatgg tttttggttt tttaattaga ttagtttttt 46560
gttttttttt ttttagttta ggatttatgg ggtaggaggg tagggtatag gtttggaggt 46620
gaggttgagg aatatggggt gttttatata ggtattatta gtattagttc gtattatgtt 46680
tttttatttt ttttttttt ttttttttt taaatttagt ttttttttgt tttttttagga 46740
tggaatttta gttttttttt tggttttgag ttttgttgtt tgagcgtata gttggaggga 46800
gaatttaata tagggttttg tttatttttt ttatttttat tttatttata gtattatata 46860
gtgtgtgttt agattttttt tgagaggggg tgatggaggt taatcggttt ttttttttt 46920
gtatcgtaga gagaaatgcg gttttttttta ggatgggggt tgagtttttt tttttttgttt 46980
taaaatgttt ttttcgagtg tgttttagat atatgtagga gggtaggtta gtagaagttt 47040
atatgaggtt attaggaatt tcgtgtgtat aagttgagag gttttcgggt ttttttttata 47100
ttttaatttt atttttttat acgtgtttag aaggtagagg aagatgggag ttttaggtga 47160
acgttgggga gggaagggaa gtttagtggt atagatggaa gataagatgg ttaatttatt 47220
tttgtatatg ttttcgtttt tttggtacga ggaggttcgg ttttagattt ggttgtttat 47280
ttttgaggta gtttttttta ttttgagtta gttttgaagt tttttagttt aggttttgga 47340
attttagtta ttgatttttg gtttttgaga ggtttaggtt tttgtcgttt atttttattta 47400
atttttttat ttttttttgtt attggtgggg gtttaaggga atttgcgagg ttaaagtttt 47460
tcgagttgag gtttgtttat tttgtttagg tgtttatgga tgtgaggggt ttggggagga 47520
gttggaggtg aatgagatat agttgttatt cgtagatgga ggttaagttt atagttgagt 47580
ttaaaaatag gaagttagtt gggggggtgtt ggggtgttat ttttttattg gatttttaat 47640
gaagtttgag gttatttttt agaattgaat ttttttttgt tgttagtttg gttttgggaa 47700
tagagatgtt aaggtgtaaa agttattttt ataggggtat ttggatgttt attaaggtta 47760
aatatttaag aggggtttta tgggaaggat ggatagtgag gggagaggtg ttttgggggga 47820
aggggcgagt ttttttttata ttatatttgt tcgttagtcg gtgatatttt ttttttttt 47880
ttttagattt agaattcgtt ttggttcggt aataacgggg ttgttttatt agtgttagtt 47940
tgcgtggttt tttgcgattc ggtgtttgtt tgtatgtttt tttatgttta ttttttttggt 48000
tttggggtta gtagcgttat cgattttttg agtgtgtttg gggttggtag ttacgtgggt 48060
tagacgtata tgggtagttt gtttggagta gttagtttta gtttaggttt taatggttac 48120
gagtttaacg gcgagtcgga tcgtaagatt tcgagtatcg cggtttttcg tatgaaggtt 48180
aaggagtata gtgcggttat tttttgggtt atatgatagg gtatttttgt ttcgttttta 48240
tttcgggata ttatgggtta cgtttatgtt ttttaggttt ttagtttttt attcgatttt 48300
tttttttagga atttggtttg ggttaggggt ttgattttta gtatttttag tcgttttaag 48360
tttgaggttt cgtggattgt tgggagggag gggggtagtag gtttttggtt tttttttggt 48420
attgaggttt tgattttttgt tttcggttat aggtagtgga gaaagttagg tggttacgtt 48480
ttttagtttc gtatttatga taagttgaaa gcgttttttt gttttcgttt atttttttgt 48540
tttgtttagt tgattatgag ttaatgttag atttttatgt agatttagta gttttattag 48600
tttagttttg tttatttttt tcgtttttag tggggttttt tggttattag gtcggtggtt 48660
gtgtgtttga agtataggtt gttttgtaga gttagttttt tgttttttta ttttttttt 48720
tttgaaagta tacggaattc gatttgttgg ggttaaggcg ttagttttta ttttttttcg 48780
aatagtgacg agtttgatag agtttggttg attgtatttt ggttgttttt taggttggat 48840
atatttggag agagtgggta gaggatgata ggagttggag tcgaggattt ttgttgttat 48900
ttagtaatgt tagtttttag gggagatacg ggttagtttt ttattggata ttatagggga 48960
ggagttagat ttgagggagg ttgagaatat agatgttata agggttttta tggtgttaga 49020
agaaggaaag gttttgggag agggggaggt attcgggtgg ggcgtgggtt cgggtagttt 49080
tttcgttttt cggtttgttt ggtatttggt atagcgggga ggtttgggta gttttgtttt 49140

ttatttagag tttagttagg ttttttttag gaggatgcgg aggggttcgt ttttttttg 49200
ttttatttg gttttttttt ggggttgttt agtagtgttt cggtatggaa ggatttataa 49260
tttcggaata tataagttag agtttggggg tcgggtggta gagggttaaa agttgcgtgt 49320
aggttttgtt agaggacgtg tggttatgtt cggtttttag gttttgttat ggttttaaat 49380
tgattgtttt ttttgttata tttgttagtg ttatcgttgt ttagagtttt ttacggtaga 49440
tgggggtttt gtgttttttt aggtattagg aggtgttgtt tttttttata tagtttttta 49500
tatttcgttt ttatattttt tttataggga ttaggatttt ttgttttttt tttagtttgc 49560
ggaagggttt ttggtttggg tttttcggtg tggttttgtt attgtaaatg gggttgttag 49620
gatgtttttt tgaaattagg tttttttgttt ttaggaagcg gggttttgtg tttgtttgtt 49680
cgatttttgt agtttgggtt tggttttgag ttttagtttt tattttagtt gggagtatat 49740
agtaatcgtt tagaggtagg tttgttagat tttaggtggg gggttttttt agtaggtagc 49800
gtttcgtatt gaagcgttcg ttttttttat ttttttttt gattttttgtt atggagtagg 49860
gttaggttta ttatgtttag ggtcgtggat atagaaaggt tttttgggtt gggcggaggg 49920
taaatttggt gttgttgaat gagggttttt ggggttgggg gtgatagttt ttcgtttgtt 49980
tggtgttttt atttgtttta gaaattaggt tgaggaaggg tttggttgtg gttttttttta 50040
atgagttaga tttttttgtg aatcgtagtt ttaaaatgga ttttatagag gtgaaagggt 50100
tttggatatt gaattaaatg tttttggagg tttggtttta agtgttttaa atatttgagg 50160
atttggagtg atttttggga agtttttttt agttttgagg ttcgttgaaa tgatttagat 50220
tttttaaaa tttagttagg gaaaggaaga atttaaggtt gggaatagtt tttttatttt 50280
ttttttttt ttttttgaaa tgtttatggt tgaggaagga tggggaagga aggaattttt 50340
atttgggtt tttttttattt atgagagaat taggataaag aggaggagag ttaggtagtt 50400
ttagagtggg ttaggaggga gagtaaggga attaggataa agaggaggat agataggtag 50460
ttttagagta ggtagggagg gagagtgaga gttttaaggg ggtttttttcg tagtggtttt 50520
cggtatttag tttttttatg cgtttgtttt ttggttgggt tagatttaag ttaggtaaat 50580
tttaatttaa aagtttgaga agaaaaggcg tttattagaa gattttgtgg ttatggttgt 50640
ttgttttaat atatttagga agtgtttagg attaaatatg ttaatgtgaa atatttttgt 50700
ttatttcgtt aaaggtagtt acgtatttta ggtttgtttt ttgtggtttg gtttagtttt 50760
tggggggttat ttaattatat taagtatgat aggattatcg agttgggagg aagtttagaa 50820
attttttagt tagtttagtt tttttataat agagaagatt ttggtttaga gtggagatat 50880
gattgttttg atttgtattt gttttgagat taggttgaga gaggagtttg ggacgtgtta 50940
tttagggtgt gagggtttgg gtttcgttgg tattaggggc ggtcggtgtt gtatggagtt 51000
ttttttttta atgttttatg ggtatttatt tagtagttta gtttttttaga agagaatttt 51060
ggggtttagt tttttttaag ataggtggtt aggtttaatt ttttgtattg tagagtaggg 51120
ggttattgtc gtgttagttt ttaggatgat tgagtaagtt agggtagttg tttaaggtta 51180
tttggagtta aagtttatga aggtaggtgg agagaagtat ttatttgtta cgtggtttat 51240
tgttatgcga ataagttaag agaggtgagg attttttttg gatatttgtt taagatttta 51300
atagagatgg taatttaaaa ataaagtcgt gggtagtcgg tagatggatg ggtagttttt 51360
ttatattgat agtttttagt ttttgttggg tataggtttt agtatggtaa ggtttgatat 51420
taaggtgttg ttattgtttt ttagatgtta gtattatttt tggggtatgg gagcgagggt 51480
tttggggggt atatagagag gtttgttttt aaggttgaaa tagtatttgg aggatatggg 51540
ttttagaat tttttttgaa ttttttttaat tatatttgat ttttagcgtt tattatcgtg 51600
tttttagttt cgggaagtac ggtggttagg ttagtttaaa gatattgcga tggggtaggt 51660
gtagtaagtg tggggtgggt ttttagtata tatagttgtg attatattgg atttagtttc 51720
gtgttttatt ttttgttatt tcggttgggt agacgaggga cgtttttatg aaaattacgt 51780
atttgtttta tgtagttatt ttttttttgg gttagatagt gggggggttat tttcgagaat 51840
ttgaaagata cgcgggggtt tttggtttgt tttatggttt ttattgttta gtgtaaaggt 51900
aaataggttt agttggggga ggatggttgg taaattggtt tttaggataa tatttgtga 51960
ttatttagtt aaaaaataag aaggtgataa tgatttatta attttatttt atttttttta 52020
ggatgttgta gggaggaaag gtgtttttat gatttaattt cggttttttt ttttttttt 52080
ttttatttat atatatagag ttaggttttt atattattga ttttgaagga tagtttttaa 52140
tgtttaatta tttgtttagg tgtgtagtgg ttgaaaaaag aaagttgagt gttagaagga 52200
atatgaattt taatcgttat ttattgtttt tattgtaaga tattttaatt ttgtataaat 52260
gtaatttttt ttttagttta atggtttggg gtggaatatt aaaaatatat attaaaaata 52320
tagtaaaaat ttagaaaaat gtaaaaaaaa aaatgaggtc ggttttataa agttttattg 52380
tttatattat tatgtaatta tattatagta aaatattaaa agaaacgttt ttgtttttta 52440
aagtaagtta ttgtatttat atttttttttg ggagaggagg aatacggatg ggaaggagat 52500
gtaggggttt aggtatgggg taaaatggag tgtagaggta gtcgggtatt tttagaatat 52560
tttttgttgg tttagtttaa attgtttggt tgttgttatt tgtaataaat ttttttttt 52620

tttttt 52626

<210> 5
<211> 221909
<212> DNA
<213> Artificial Sequence

<220>
<223> chemically treated genomic DNA (Homo sapiens)

<400> 5

```
aaaatttcgt ttttattaaa aatataaaaa ttagtcggtt gtggtggcgt gtgtttgtaa      60
ttttagttat ttgggaggtt gaggtaagag aattgtttga attcgggagg tggacgttgt     120
agtgagttga gattatgtta ttgtatttta gtttgggtga tagagggaga ttttgtttta     180
taaataaata aataaattag gtgcggtggt ttatatttgt aattttagta ttttgggagg     240
ttgaggcggg gggattattt taggttagga gtttgagatt agtttggtta atatggtgaa     300
atttgtttt tattaaaaaa gaaaaaaatt ataaaaaaaa aaaaattagt taggtgtggc     360
gggtatttgt aattttagtt atttaagagg ttgaggttgg agaattattt gaatttggga     420
ggtagaggtt gtagtgagtt gagatcgtat tattgtattt tagtttcggc gacgagggta     480
aaattttttt ttaaaaaata aaaaataaaa ataaataaat aaaaataaaa tttgaggttt     540
aaataattaa gtttcgtgta tttttttaat tttttttttt ttttttaat tttaatttta      600
tgatagatta agttttatgt attttaaaaa tgtgtagaat ttttattttt agtttttttt     660
tttttttttt tgtttttttt tttgattttt tggaatcgga aggaaaattc ggatatttag     720
gaattattat ataggaggta tttatgtgat gtgtttaata ttattttacg taggtgtttt     780
tgggttttgg gtgtttaggt ttttgtgaat tgtgatttta ggtagaagcg tgagtattcg     840
tagaggaatt tatttattta aagttaggga atagttaggg ttggtatttt tagggtgatt     900
gtgttttagg gtatttttga tttgtgttga gtaaaatgga taaagttagt aatgtattta     960
taatttagat ttttgagata tcgttggatt ttgggaattt taggtattag gttcgtggta    1020
gtgttattat ggttaggttg gggtagtgt tattatggtg tttgttggtt aagttaggat    1080
acggtttaag tcggaatttg ttggtgttgg tataagcggt gagtgggcgt gtgggtgggg    1140
gttgcggtta gggtgtggat attttagaat ttaggacgtt gtttataatt cgtttatatt    1200
ttttgttttt ttttttata gtattttttt ttcgaggttt tttgagtttt taattttttag    1260
ataaggaaag gggtggattt agttgaagtt tagtgtttat ttcggtttaa ttagttttat    1320
ttggggtgag gggaaatttt tttggttggg ttgtttttttt tgtagggttg tggttggata    1380
gattatttaa gaaaggaagt aggtgggtcg gaacgaattt acggtgttat ataggttttc    1440
gttttatgt atataagttt tttatattta ggtaggaaat gaaaatgttt tttggatttt     1500
taaggagagt aaaggagagg ggaagaggag gaggattttt cggggttgtt agtgatattt    1560
ttagtttttt gtgtattttt gggagggttg taggagaagg taatgttgcg ttttattta     1620
gaattagcga gcgtttttta attggttttt cgtttttttt tcgtttttta cggtttttat    1680
tggtagttgt gtaagggttc ggtttttgttt ttgttttttt atattcgttg tgatatttta    1740
ggtttttttag gtttcgttac gtttttttttt ttttcgggaa tagttgtttt ttattatttt  1800
ggttgtttgg ttaattttttg tttattttttt aaaattttgt tttgatttcg ttttaataaa  1860
atttgatat tatttttttag ttataattag ttattttttt ttttgtacgt tggaaatatc    1920
gttttatcgt ggagatttta aggatattta agtagagaga attttttcgt gtaagttttg    1980
tatttttttta ttattgtttg cgttattcga gttgtaaagg gcggtttttt gtgtttgttt  2040
cgttgtatcg atatatcgag ttgcgtacgg tgtttagcgt agggagaata aatgattatt    2100
tgtttaacgc gtttatttat aggtgaggaa attaaggttt taatttaatc gacgtatttt    2160
gttttttttga ttattagaaa agtagtagga taggtgtttt gtttcgtttt atttcggttt  2220
attaagtcgg tatttcggtt tcgattttcg gttgtgttcg gcgtcgtcgc ggtgttcggc    2280
gtcgtcgttt cgttcggcgg ggtcgttcgg agcgttcgta ttttcgttcg ttttttatttg  2340
gtcgggttcg tttcgttcgg attcgggatt gggaagtgcg gcgattttcg gaattagtta    2400
ttggcgttag cgcgggggagt tgggggtgta gagttgcggg cgcggcgggt tacgtaggcg  2460
gtttttattt tcggtttggt ttggtttggt ttggtttgcg ttgtcgcgcg ggggcgtttt    2520
tttttaggtt cggcgttcgt tagtttcgtt tcgttaggtg tagcgtagcg taggggtggg    2580
cgggggtggg gttcggcgcg tacgtttacg gggcggggag ggggcgggtt aggggcggga    2640
ttatagtcgg ttgggtcggg gttttatgcg tattttcggg gaggggcggg gcgggggcgg    2700
ggtcggggcg gggttcggtc ggtgtatttt agacggcggg tcgttttttt ttttcgtttt    2760
```

```
tttattatcg gtttaggatt agcgttttgg gagcgcgcgt ttcgttgttt cgtcgttata   2820
ttttttttggg agcggcggtt acggaggtat tatgaagaag ttttatttag gtgggtttcg   2880
cgttcggggt ggggaggggt cggtgtttcg ggattagcgt tgtttatttg agtgtttgcg   2940
gtcgggagtg gcgaggcgtt ttcggagttg agcgagtttt cgcggcgggt atattgtagg   3000
tcgagttttt tttaggatag ggtcgttgtc gggtcgtttt cgatttgagt cgatcgtttt   3060
ttgcgttgtt tttagttttt gttcgagtgt cggaggggtt gttttggggg acgttttttt   3120
tttttcgttt tttgtatttt cgtaggaatc gttgattttt taggtcggtc gggtgttttg   3180
ggtttttgtg cgtttgtgtg ggtgaatggg gtcggggtta ggtggagggg tgtttttggg   3240
tttagttttt agggttggtg gtttaggtcg tagtattttt ttttcggatt tttttcggtt   3300
ttttttttat ttagtggggt acgggacggt ttttagatgg gatcgtttag tagcgtttaa   3360
atttggcgat tcgggtttac gttttgcgtt taggacgtcg ttcgcggttg atattttttat   3420
tttattttag tagggttcgt tttaagtagg cgaagggggt ttgatttggg ttttattatg   3480
agtcggttta ggagttttta ttttataaga ggttttcgtt ttggtatttt gaagttttat   3540
tttatagatg ggtatatcga ggttataagg gtaggttaag agaaatttag atttttatta   3600
tttgtttttc ttttttttt ttttttttt tgtggttttt tttaaaggat ttaggtaggt   3660
aggatttttt gttttttttta gtttttaaat tataaatagc gtttttttttt ttaaatttag   3720
aaatcgtttg tttaatgttt tttttttaat ttatttagat agaaaggtat ttgttaagga   3780
tttatgaatt aatttttatt gataggagaa tagaattatg ggttttagtt ggaggtgaag   3840
gagttatcgg ggttattttt ttgtttttgg gttattgtcg gtttttttttg gtttttttata   3900
tttaggaatt aaaatgagtg ataaagtatt ttgttttaaa gaggattatt tagaattttt   3960
tcgaaaatat ttttttgggt tagtagttta gaattttaat ttttattcgg ttgttgcgaa   4020
taagatggtt ttttacgtat tcggagtata gttttgttag gagcgtgggt gggttggtgt   4080
gttggtgggc gggagcgggt ttttaggtgg tatatgtttg gttttggtat cggggttttt   4140
tttttaatat gttttaaata tttgagattg ttgtggggat cgcgcggtgt atggtttttt   4200
ttaggggatt tttagttcgt ttagggaggg tttttttgttt ttggtttttt atattttatt   4260
ttttattggt gggtttagat agtgggggtg atcggggttt ttttaggtcg tgggttttgg   4320
gtttttttttg cggagatttt tttgtataga tgtcggaagg gttttgaggt aggtttggag   4380
tttttgtatt tgcggtttat tgggaggtgg ggagattatt atttgttttt ttttttttttt   4440
ttttatatat atttatttta tttttttattg ggtttttttt tttttttttt ttttaaaaaa   4500
aaggatagaa tttttttgtag taggtggaag tagagtttgt ggagtgagta ggaattaatt   4560
tttgtatagt agtttcggtg tttttttgtg tttatttaaa ggggtaggtt tttttgagga   4620
tgggggaagg gagggtttag attattttttg gtttgttttt tgtttggtag tggtttaagg   4680
tgtgttgggt tattgtgttg tagttgtatg gagtgggatt ggaggggggtt gatggtgggg   4740
gtggtttttt gttttttattt tgagtgtttt ggagttgttt tggtattagt ttttttttcgg   4800
gtacgggtat tcgtaggagt taggttgatt ttttaataag ttatcgatat tatgaaatta   4860
aggaaagtag taaaagatgt taaaaatttt ttaaggtttt gataatttaa ggataatagg   4920
aaggttttta tttttttatt tagattgtgc gggattattg tttttagatt tgtatgtggg   4980
ttttagaata aacggaatga ttataatgtt gttttcggta gatttggttt gaatattcgt   5040
gagtttttga aaaggagaaa gattttagaa ggatgtgttg gggtaatttt aagtatcgtg   5100
ggtttataga tagttatcga agatatttta tgtgttgggt atatatggtt aagtttgagt   5160
gggtatagaa atggtaatga gatatagttt ttgtttgtag agaatttata tttttatcgg   5220
gagttaggat gagggcgaga tattagttta ttaatatttt ttttttttga gtagtttagt   5280
ttttaaatat tataggaatg tttcggaggt tttaggtgga ggtgagattg agttgggttt   5340
tgaggaagga gaagttttga gtagtggaga ataaaaaggg atttggttag gtgcgtttga   5400
agttttagtt atttaggagg ttgagatacg aggatcgttt gagtttagga ggttatttag   5460
gaggttgaga tacgaggatc gtttgagttt aggaggttat ttaggaggtt gagataggag   5520
gatcgtttga agttaggagt ttaaggttgt agttttgtga tagcgtttgg aaatagatac   5580
ggtattttag tttgggtggc gttatgagat tttatttgta agaaggtttt ttttaatgcg   5640
gaaggtagta agggtttata tagggtagtt gtaggattta gggggatatt ggggataggt   5700
tttttttggt agttttattg ttgttttagg tttttttcgat tgttttattc ggttttttaa   5760
tggggtggag gtagtggtag tttataggta gtgtggttta gtggttatat ttgggggttt   5820
agagtcgtta gtttttttggg attaaatttt attaggttgc gttttttttt gtggtataag   5880
gtttttattt tatgtatttt atttgtttta tttgtttttt tgtgtgtgtg gtgtttttta   5940
atttatttta tttatttatt tttgagacgg agttttattt tgttttttag gttggagtgt   6000
aatggtatga ttttagttta ttgtaatttt cgtttttttag gtttaagtga ttttttttgtt   6060
ttagtttttt gagtggttgg gattgtagga acgtgttatt gcgtttagtt aatttttata   6120
tttttattag agatggggtt ttattatgtt ggttaggttg atttcgaatt tttgatttcg   6180
tgatttgttt gttttggttt tttaaagtgt tgtgattata ggtatgagtt attacgttta   6240
```

```
gtttgtttta tttgttaaat gggatagtgt tatgttgttg taaaggttaa acgagtttat 6300
atttgaaaaa gttgagaaat gtttgttttg taatttgtta gttgttgtta tgattatgat 6360
tgttattgat aggaggatgg agaaggttgg ggttacgtta ttgttaggac gttttagaag 6420
tttttatgat ttttttttat tgtggttaga gtatggtgta gggttttttt tttagttttt 6480
tagtattgga gttagttgta ttgtattcgt tttggttttt taggttttga gttttgggat 6540
tttttttgta tattaggtta tatggaattt gtcgttattt ttaggttttt ttggaaattt 6600
tttgtgggtg gtagcggtag gaggatttat tcgaggtcga tgagttatag aagcgggatt 6660
gaggttgcgt tatattttta ttggtatttg ggaattattt tggagatttt ttttttattt 6720
tttattttttt tgagatggag ttttgttttg ttgtttaggt tggagtgtag tggtacgatt 6780
tcggtttatt gtaattttttg tttttaggt ttaagtaatt tttatgtttt agtttttttga 6840
gtagttggga ttataggtat gtgttatcgt gtttagttaa tttttgtatt ttttagtaga 6900
gatggggttt tattatattg gttaggttgg tttcgaattt cggattttag gtgatttatt 6960
cgttttagtt ttttaaagtg ttgggattat aggtatgagt tatcgtgttt agttattttg 7020
gagttttttt gtggttttta ggagattttt gaaggggtat tagttacgat taaaaagttt 7080
tatgttgtcg tgatttttga gttttgggta gggagggagg ttgtatttat ataggaagta 7140
gtttttgtcg tgatttagtg tgttggttcg tcgtggtgga tagggttgta ttttttattta 7200
gcgtttcggg agttgattta cgggaagggt aaggaattgt atttttagag aatatttttt 7260
tagttagtta attcggtgat aggcgagcgt gggaagggta taagtatagg gttttttttt 7320
ttttttttt agtttcgttt tcgcgtttgt tttcgttgtt ttttgttttt ttacgtttaa 7380
gtttgtttgt tttggcgtat ttaggggatt tggattggag agaaaaagtt atttcgtacg 7440
gttagtttgt ggttttgttt gatttgtgtt tttcgtcgag gttttttagt tgtgagatag 7500
ttggttgagt ttaagttttg gggttagttt ttttttttagt ttgttgtaaa ggtagaaagg 7560
tatgtttagg taggggttat ttgttttgtt cgtttacgtt agatcgtagg tatttgttcg 7620
ttagattttt tcggtttgtt tagagggatt ttgatagtcg agggtaatgt atttggtagc 7680
ggagtattgc gtttgcgata gtttattttt tttttggaat tattttttat aagtgtgatt 7740
agtttaagtt aggataggta tagggggatt taggattgtt atttaggggt agtggtttgg 7800
aatttaggtt taggtgatat gggagttaat tttttgtttg gagattttag gtgggtgttt 7860
gtagaggata ttttatttga ggatttatgg aaggggatat tttttatttt ttatttggtt 7920
tttgggttgt ttttttttga tattttttttg ttattttagg tagttttgat agttttatgt 7980
gattgggatt tagaggtggt ttttgttttc gttttagtgt ttgtttatag gtagtgttgc 8040
gtgggttttg aagtttgttt aagatggatt tgataagggg agtttagtat atattagtgt 8100
aggttcggtt agggggtgat gatggtagtt tttgggtaga tttagttggt tttagggtag 8160
gaggggtttt agagagaggt tttaggtttt taggggtgtg aatttttggg gtgaggcggg 8220
aagtgacgcg ttgggttttt gcgtttttgt ttgttggagt tattattttg gaaatatttt 8280
agttttaggc ggttgttagg gatttatggg gattagtggg tgttaatttt tgggtgaagt 8340
tttcggggtt tgtatatagt atatagcgtt gtggttttat ggtttatttt gttgtgtgaa 8400
gtggtttagt tttcgggaga gtttttagtt aggtaggttt gacggtaatt gttaggttat 8460
ggtgatttgg aatcgttgat taaagggagt tagttttggt gggtggaggg atttggtggg 8520
tagttatcgt gatgttaggt atggggatcg tgtgttattt aggtttgtgg gtgtcgagtt 8580
tttgtgggag tagtgaagtg ttttcgtggt cgatgggttg gttattttttg tttttttttt 8640
gatgtgatag ttgtgtttgg gtttcgggaa atttgttagg aggtttggta ttggtttgtt 8700
ttattggagc ggttttgttg agttgtgagt tgttcgtttt tttgaggttt ttagtttatt 8760
ttaatttttt atgtggattt ggttatttat tttagaagcg tttgtcgggc gttgttgagt 8820
gttaggtttt gtttgttttt tttttttgtac gttggaggtt ttgggtatta gttttgtagg 8880
gtggtttggt tttgggtttt tagggtagag tatggtattt tttgttttta gtttagtttt 8940
tttttgtttt attttttttga ttttattttg ggtatgggtt tatttgtagt ttttgtgaag 9000
ggagatattg agttgttgtt ggtagaggag gggcggtttt tgggtattgt ttgttaatgg 9060
tttttcggta gttttgtgat agttgagttt tgtttttagt ttaatttgat ttggtttttt 9120
aaagtatgat tgttggtagt ttgaaaattt ttaggatagg aaataggagt ggcgtaggga 9180
atgtaagatg attttagttt taagttattg ggcggttttt agatttaggt tgtttgttgt 9240
ttttggtagg aatttaagtc gttggttatt tttagagcgg tgttggttcg gggtttttag 9300
tggtttttgt gtttgggtga gaggaatttt ggatggttat tttgttttga gtgtgtgggt 9360
ttttagaagt gttgggttag ggggtacgga gggttagaaa gttttttttg gagcgttgga 9420
ttttttcgtt gatttttatt ttatttcggt ttggggtttt agagaagggg tttaggtagg 9480
agtgttattt tttttaatg gggatgtggt tttagttttt gtttaggtgg gtgtattgtt 9540
ttttttttcgg ttttttttag gttgtttaaa tgatttgtga cgaggtttgc gttatggtcg 9600
gggtttttttg ttttttttag cgttgtattt gggaattagg ggttgggttt cggaagtcga 9660
ggatgttttt gaatgttatg tttgggtatt tgttttggtt aggcgaggcg tgttggggta 9720
```

```
gcggttgaga tgggagtagt agtattgttt gttaggtttg agatgggttt cgggtgagtg     9780
tggttattag gaagggttgg gtagagtagg cgggtgggtc gtttttgggg ttttgtttcg     9840
tagattaatt tgattcgtat tttggggtcg gggttttttt ttatagtttt tttatgaatt     9900
atggttttg ttttatttt aaattagatt ttagaagagg aataatgttt atgtaattta       9960
agttagttgg aagtgggtta tatttaattt ttgttatttt ttagtttgtt aaggatttga    10020
ttaatttaat ttagagggat ttggagggtt gtttgtaaaa tttattaata atagggaatt    10080
ttagttttgt agttataggg tttatagagg tgtcgggttg tcgggttatt gttgggaggt    10140
gagtcggtgg cgtagatatt atggaggtat cggtagtgga agagttgtaa gtgtatatag    10200
aggtcggtat cgagggcgg ggaggaggag gtggtggatt tttcgttagt gttggtatta     10260
tgttgttttt agtattagag atttaaggtg tggttgattt tggttggggc gatgagttat    10320
tggatagtag agtttttttg gtagttgggg gttaacgttt taattgtcgg gattttgtat    10380
tatgtcgttt tttttttttg ttttgtttgg tttggtttgg tttggttttt gagacggagt    10440
tttattttgt tatttaggtt ggagtgtggt ggtttaattt tggtttattg taatttttgt    10500
tttttgggtt taagtaattt ttttgtttta gtttttttgaa tagttgggat tataggtacg   10560
cgttattatg tttggttaat ttttgtattt ttagtagaga tggggtttcg ttatgttggt    10620
cgggttggtt ttaaattttt gattttaggt gatttatttg tttcggtttt ttaaagtgtt    10680
gggattatag gtatgagtta tcgcgtttag tttgtattta tgtttttttt tttttttttt    10740
tgagatggag tttcgttttt gttgtttagg ttggagtgta aaggcgcgat tttagtttat    10800
tgtaattttt atttttcggg tttaagcgat tttttttgttt tagtttttta agtagttggg   10860
attataggtt tgcgttatta tgttcggtta attttgtatt tttagtatag acggggtgtt    10920
tttatgttgg ttaggttggt ttcgaatttt cgattttagg tgattcgttc gttttggttt    10980
tttaaagtgt tgggattata ggcgtgagtt atcgtattcg gtttatgttt tttattatta    11040
ttatttaatt ttttaaggaa agtaatttaa attgtatatt gtattttttt tttttttttt    11100
gtttttttgtt gttagttttt tgtattattt attgttaaat aatatgaata ggaaattgaa   11160
aggaaaattt taataattat tggttgtagt attttttttg tgttttattt agtgtttgtt    11220
tttttaggaa tatgatatag ggtttgattt aattatgaaa ggcgtatatt tttagagttt    11280
tatatttttt atagaatgaa tgttgaattt tgtttttttt atttgaaatt agattttagg    11340
tatggatttt atgattatta ttataatggt tttttagagt ggatattgta ttttgtaggg    11400
aattattttt tttttagttg ttggtgtgga cgtttttttt tgatggtgtg ggtttgggtt    11460
tttgaagggt ggtgttttga gtaaagatgt tatagtagtt atgtttatag ttttgggttt    11520
ggggttaggt ggagattggt tttaatttta gagtttgttg ttttaaaata aatttttttt    11580
tttttttttt tgagatggag tttcgttttg ttgtttaggt tggagtgtag tggcgcgatt    11640
ttcgtttatt gtaagtttcg tttttcgggt ttatattatt tttttgtttt agtatttcgt    11700
atagttggga ttataggtat tcgtttttac gttcggttaa tttttttgtat tttttttagta  11760
gaggcggggt tttattatgt tagttaggat ggttttaatt ttttgatttc gtgatttatt    11820
cgttttagtt ttttaaagtgt tgggataaaa taaattttta gggtcgggag cggtggttta   11880
tatttgtaat tttagtattt tgagaggtcg aggcgggtag attataaggt tagaagatcg    11940
agattatggt gaaatttcgt ttttattaaa aatataaaaa attagttggg tgtagtggcg    12000
ggcgtttgta gttttagtta ttcgggaggt tgaggtagga gaatggtatg aattcggaag    12060
gcggagtttg tagtgagtcg agattgcgtt agtgtatttt agtttgggcg atagagtgag    12120
attttgtttt ataaataaat aaataaataa ataaataaat aaatttttat atttatttgt    12180
attatttatt attattatta ttaaataggg ttttgttgtg ttgtttaggt tggagtgtag    12240
tggcgtgatt atgatttttt agtttcgatt ttttagtttt aattggtttt tttttttttag   12300
ttttttgagt agttgggatt ataggtgtgc gttgtatgtt tggttaattt tttttttttt    12360
ttttttttg agatagagtt ttattttgtt gtttaggttg gagtgtcgtg gtgtaatttc     12420
ggtttattgt aattttcggt ttttggatta aagtaatttt tttgttttag tttttttgaat   12480
agttgcgatt ataggtatgt gtcgttatat ttggttaatt tttgtatttt tagtagggac    12540
ggggttttat tatgttggtt aggttggttt taaattttttg atttcgtgat ttatttgttt   12600
tggtttttta aagtgttggg attataggtg ggagttatcg cgtttggtat gtttggttaa    12660
tttttaaaaa atttttgtag tagtcgggcg cggtggttta tgtttgtaat tttagtattt    12720
tgggaggtta aggaggtcgg gagttcgaga ttagtttgat taatatggag aaattttgtt    12780
tttattaaaa ttataaaatt agttgggtat ggtggtatat gtttgtaatt ttagttattt    12840
aggaggttga ggtaggagaa ttgtttgaat ttaggaggcg gggttgtag tgagttgaga     12900
tcgtgttacg gtattttagt ttgggtaaag agagtgaaat ttcgttttaa aaaattttg     12960
tttttgtttt tgtagcgata gggttttgtt ttgttgttgt ttaggttggt attgaatttt    13020
tagttttaag cgattttttt gttttagttt tttaaagcgt tgggagtata ggcgtgagtt    13080
attgtgtttg gtttttattt gttttggttg tttttttttg ttttaggtaa ttatatattt    13140
ttttaggtaa ttaagaaaag attgcgtatg atgagttttt tttttggtat ttttgggatt    13200
```

```
gtttttaaaa ttagataagt gtttttattt tgatatttta ttaagatttt aatgaataga    13260
atgtcgtttg tttttagttt gttggttttt ttgcgaaggt ttagggtttt tttgtttgaa    13320
aagttatttt tttgtcgagg tttttgattt tttttttatta cggtagttat gtcgagggtt    13380
tatagagtta tttttttttt attttttttt cggtttttta agacgtattt gtattttcgg    13440
agatgttttt ttgttttttt gggttatttt ttttggtcgt tatttatttt gttgatttat    13500
tatttgtgta atagtttttt ttggaatttt taggtttttg ttatttattt tttgtagttt    13560
tcgttttgtt tttttttgttt tagttttttt ttttattatt tttttatttg ttggttttta    13620
tttttgttta tggtatattt ttgtgttata ttttgatggt tggataaagg ttgagtgaaa    13680
cgtatttatt tttttggtga ttggggtgtt tattaattag aagggattta aggttggcgt    13740
agtttttttt cgtattttgg ggtatcgttt tgtggttttt ttttgttgga gtttgtagtt    13800
ttttgttttt ttttttttaa atttttttgag atggagtttc gttttgtcgt ttaggttgga    13860
gtgtagtggt gagattttag tttattgtaa ttttcgtttt tcgggtttaa ttaatttttt    13920
tgttttagtt ttttaagtag ttgggattat aggcgttcgt tattatgttt ggttaatttt    13980
ggtattttta gtagagacgg ggttttttta tattggtcgg gttggtttcg aatttttgat    14040
tttaggtgat aggttcgcgt cggtttttta aagtgttgag attacgggta tgagttatta    14100
ggttcggtcg ttgagattat ttttataagt tattaggttt ggttgtagtt ggtagttttt    14160
gattattggt ttcgttttac gtgatttgtg attttttttt tggtttttttg gttttgtttt    14220
gtttatttgt ttattttttt tatgtatgcg gtttgtttat agggtatgta atggggttta    14280
attaggaaat ggaaatcgtt tgagtattta tttatttatt tatttattta tttatttatt    14340
tattttttttt aagataaggt tttattttgt tacgtaagtt gaagtgtagt ggtgtgatta    14400
tagtttatgg tagttttgaa tttttgagtt taagcgattt ttttttttta gtttttttaag    14460
tagttcggat tataggtgta tattatatgt ttgtttaatt ttttttttta tttttgtaga    14520
gatagggttt tgttttgttg tttaggttgg ttttgaattt ttaattttaa gtaatttttt    14580
tgttttagtt ttttaaagta ttggagttat aggtatgtgt tattacgttt tgttaatttt    14640
tgtatttttt gtgtgtgtgt aaagataagg ttttattatg ttgtttaggt tggttttgta    14700
tttttgggtt tatgtgattt ttttgtttgg gtttttttaaa gtgttgggat tataggcggg    14760
agttatagtg tttggtttat ttgagtattt agaatagaag gatttaaagg cggggaatta    14820
gttatataga tgatgggaga ggtgaaggtt agatagggaa tagagaggta attagtagga    14880
aattaaagtg gtgtgggttt atatagtttt ttaaaggtat ggatgtaatg atttatttag    14940
aatgagaaaa taaataatag taaattataa attataagta gttgttaaat attataaata    15000
ttattatatg tagaagagta ttagttaatt gttagttata tttttaaagt tttatttttt    15060
ttatattttt ggtattattt ttttaatatg atgattttgt aatatcgttt tttatttcga    15120
gaatagaaaa ttgattaggt tggatatagt ggttcgtgtt tgtaatttta gtattttggg    15180
aggttaaggt aggtggatta tttgaggtta ggagtttaag atcggttcgg gtagcgtggc    15240
gaaatttcgt ttttattaaa aatataaaaa ttagtagggt atggtggtat atatttgtag    15300
ttttagttat ttaggaggtt gaggtgggag gattgtttga gtttaggagg tcgaggttgt    15360
agtgagtcgt gattatgtta ttgtatttta gtttgggtga gagagtgaga ttatgtttta    15420
aaaaaaaaaa aaaaataata aaaaaaaata taggttgggc gtggtggttt acgtttgtaa    15480
ttttagtatt gtgggaggtt gagatgggcg gattatttga ggttaggagt ttgaaattag    15540
tttggttaat atggtgaaat tttatttttta ttaaaaatat aaaaaattag ttaggtgtga    15600
tgacgtacgt ttgtaatttt agttatttgg gaggttgagg taggagaatg gtttgaattt    15660
aggaggtaga ggttgtagtg ggttaagatc gtgttattgt attttagttt gggtaataga    15720
gtgagatttt attttaaaaa taaataaata aataaagcga atgaattttt ttagttgatt    15780
aaattttgtt tttatttttg gtagttggga tgtatataaa agtggcggtt aatatggagt    15840
tggtggtgtt gttataattg ttttgtttaa gatatatagg aattttggta aattttgttt    15900
tttttttttt ttttaaatgt gatttttatt aagaaagaag taggaaaatg ggggagattt    15960
ttaattgtat atgaggtatt tgtgtatgta tttttgatta gagagatttt tttgttttga    16020
ttttatattg aaggaattga atttttttgtt tataatttta tttttggtga tgggagtaat    16080
tttttataga tttgtttttg gattcgtgta ttttaaattt tgtttttttt tattgcgtac    16140
gttttttcgg cgttaggtat cgtaggatat gttcgtgttt tgtgattttt gattttgcgc    16200
ggtaggtgga attggaacgg tgagtggagc gagtattgtt ggaattcgtt tttatatcgg    16260
gatagcgagt agtaattgaa ttatatatgg aaataaatgg gaattatata aatataattc    16320
gattaaagtt aaattaaata ttttttttagt ttaatttttt tttagtagga gtttttacgt    16380
ggtagtaagt tttcgttttt attttatata agagaaaatg tgacgggtag gaatttaaga    16440
ttggaaatga acggggacgt taattgattg tagggaaaat atttttttatt ttgtaaattt    16500
tataaaaaat gtttgatttt gtggatatgt ggttagggtt ttttttagtg ttttgagagg    16560
gaattatgta ggtagaggga gggtttttag tattgtgggg gtgatttgga gattggtagt    16620
agggtgttat tattattttt aggtggggat gagagggagg aagttttgtg attagagttt    16680
```

agggggttaga gttatttgtt gggagtttaa ataaaggtgg gattttggta ggagttggaa 16740
ttatatagag gattattttt tttgttggag aagttgtttg aggtagagga ggtggagagt 16800
tttcgcgttt tttttgtttt ttattttttta ggttttttttt aagtttttcg ttggttgaat 16860
ttagtttaaa gttagtaggt ttggggattt ggatatttga gttagtagag gtcggcgttt 16920
tttttatata aaggagggta taagaagtgg gaggtacgga gggggattag gttaggattt 16980
atataggatg tgtaaatagg agttttttttt ttttttttatt ttaaatggta tttgtagtcg 17040
gttttatttt tttgttgtta gataggaaga ggaattttgt tttcggtatt agttgttttt 17100
atcgtttttt attttacgga ggtttttttt tttttttggtt tttgtttgta attattggcg 17160
acgttgatgg attatatttt ttggggagtt tggttatgaa tatggttttt tatttgttgt 17220
ggattaaatt gtgttttttt ttttgaattt ttgcgttgaa gttttaattt ttaatgtagt 17280
tgtaattgaa gatagggttt ttagggaggt aattaaggtt aaatgaagtt atagggtagg 17340
attttaattt aataggattg atgtttttat aagaagaggg agagatatta gagatttttt 17400
ttttttttttt atttgtaaat agaggaaaaa tgacggagga tatagtaggg aggtgggtat 17460
aagttaggaa gaggggtttc gtcgggaatt aattttgacg gtattttgat tcgggatttt 17520
gggtttttta aattgagaaa tatatgtttg ttgtttaagt tatttttttt atagtaattt 17580
ggatggtagt ttgagtttat taaggtatcg tttgtattag ttagggtttt ttagggagat 17640
agttaatagg agatggatgg atggatggat ggttagatag atagataagc ggatagagag 17700
agagagagat aggtagatat agatagatag gtagataaat ggttagatag ataagtagat 17760
agagagatag agatagatag atataaatag gtaaatagat aggtagataa atggatagat 17820
agatgataga tagttaggta ggtagataga tatagatagg tagatagata gatagataga 17880
tgataggttg ggtttagtga tttttatttg taataattag tatattggta ggttgaggtc 17940
ggtggattat ttgaggttag gagtttgaga ttagtttggt taatagggtg aaattttatt 18000
tttattaaaa atagaaaaat tagttgggcg tggtggtgta tgtttgtaat tttagttatt 18060
cgggaggttg aggtacgaga attatttgaa tttgggaggt ggaggttgta gtgagttgag 18120
attacgttat tgtattttag tttaggcgat aaagtgagat tttattttaa aaagaaaaaa 18180
aagatagata gatgaatgga tagatagtta gatagatgat agatggatga taggtagata 18240
gataattgat aaatagatat aaatgatcga tggataggta gatatagata tattgattag 18300
gtagatgata gatggataga tagaagatag atggtggatg ggtgggtaga taggtagata 18360
gatagttaga aagatagatg atagatagat aggaaggaat ttattagggg aattggttta 18420
tattattgtg gaggttaaga tgtattatag aaggtcgttc gtaagttgga ggtttaggaa 18480
agttagtatc gtggtttagt ttaagtttgt aggttttagg attgggaagt tgttgatgta 18540
atttttagtt taaggttaaa ggtttgagag tttggggaat tgttggagaa ttgggagttt 18600
taagtttaag ggtaggagag gaaggtattt tatttttagg agaaagaggg aatttatttt 18660
ttttttgttt ttttgttgtt tggtaatgga atggtgttat ttatattgag ggaggatttt 18720
tattatttag tttattaatt tatatgttta ttttttttag aaatattttt atagatatat 18780
ttagaagcga tgcgttatta gttatttttt aatttagtta aattgatatt tgtcgttagt 18840
tattatatag gtttagaaga atcgtggttg tatggtgatg gtttttattt cgtttaaaga 18900
agttgaagat gaattgttgg tataagttat cgttttttta ggttttttttt agtcgtttat 18960
tttttaaaa atgtatttat tgagtatttg ttgtatgtta ggtgttgtga taagtattgg 19020
ggtatagtag tgagtaaata aggatttttg ttttttggag tttatatgta gtagaaggaa 19080
atagaattaa tgaatttatt agttttagaa attattaaaa taggattaag agttgtggtg 19140
agaattaaga taaggtgatt gtatagaaag tgattgatgg ttgttttgga atgagtggtg 19200
aggggttata gagatgatga tagttgagtt gagattggga agataggaag gagttagtag 19260
tgtaaggatt aggttggaag taagtttggt gtattttagg aatagtaagg aggttagagg 19320
ggttgtagta gagtaggcga ggaggaggag ggagacgtag gttaaagagg agtttggggt 19380
tagatggagg attttttttt taaagtttag ttattttttt tttaaaggag aatgtgttat 19440
ttttaaaagt tatatatgtt agtatagtgt ttggtttatt atatgttttt agtaggcggt 19500
gagtatggtt gtttttgtta ttattatatt ataattatta ggaataatgg agttattagg 19560
ttttgtattt atagtaatgt gagggagtta gtaagttttt ttggggaatt tatttttttt 19620
tattaagttt ataagagttt gataattagg ttaatgtagt gagagttata gttgtttagt 19680
gtattggatt ttttagttac gggttaggat atttttattt tagtagaaat ttttaagggg 19740
attggggagt gtgttgtgga tgtttttggg ttatttagat tttttgggga gttttttttta 19800
tcgtagttgt tgtagggagt ttagttgtag tgatgaattg cggtttagtt ttgtttatgg 19860
gaaaatttta tatgggagat gggacggttt tttgttggtt ttttcggttt taggttgttt 19920
ttataagtgt agggattttt atttgggtgt gtgaatgggg tttaggatgt tttaagggta 19980
tgtggagatg ggggttagtt gtttttttttg atgttttttga atttttttttt tttgtttttt 20040
tgtattttat tgtttttttgg tttttttttat tttgggtgtt ttggaataat atgtaattta 20100
atatataggt tgattttatt attttgtttt gttttatttt attttatttt ggaaataaag 20160

gttttgttat ttagtttgga gtgtagcggt gtaattgtaa tttattgtag ttttgaattt 20220
ttgggtttaa gtgattttt cgttttagtt ttttaaagtg ttgggattat aggtatgagt 20280
tgttgtgttt ggtttaggtt ggttttaaaa gttttttatt ttttttgtat tttgatgata 20340
tttatttttt ggagtaggtt tttagttatt ggtttcggat attttgtagt tattattggt 20400
tagttgtggt atgtgaagtt gaggtttagg agaagggtag ttttgtttaa ggttatacgg 20460
tttagtgtta atatttagat tttagtgttt ttattttttt tttggtcgtt tttattttta 20520
gtgtattttt ggaggtttaa gggagttaaa ggttaggttt tggttagtta agaatttaaa 20580
aaagttgttt ttgtgttttt ttgttaattt attattttat tatttttttc gataatgaat 20640
ttagtttgaa ttttagataa ataaggatta atttttagta taagtatatt ttataataga 20700
tttgggatat atttatgttt aaaaatgatt tattgtttgt tggttttagt agtttatgtt 20760
tataatttta gtgttttggg aggttatagt tggagaattg tttgagttta ggagtttaag 20820
gttatagtga gttatgatta tgttattgag ttttagtttg ggtgaagtag taagattttt 20880
atttttttaa aaaattgttg attgtttatt tgaaatttag gtttaattga gtattttgta 20940
ttttatatga gaattttata taggtgagat ttagagtttt aatgtttatt ggtaagtttt 21000
tgtagggagg ttttgtgggt ttttggttgg tattgatgtt ttgtgatgag tagggttatt 21060
tggtaaggtg gacgtttgtt gtttatgtag gtagagtaag gttttgggta ggtatgagtt 21120
gatttttattt tttttagttt gggagttaga tattgttggt agtttgtcgt taagagaggg 21180
gttggttatt aagagagggg cggtttttat gggttttttt tggtttttgg ttttttttta 21240
gtttttttttt tattttaaat agaggttaac ggtatcgtat tagaattagt gtgttgtttt 21300
ttttggttgg tattttgtag ttgggtagat tgtttttttt ttataggagt tggtggtgtt 21360
tttttcgttt tatagggagg atggttgaga tttttgattt tgatataggg tgttgttttt 21420
tttttgtttt gagatagagt tttggttttt ttatttaggt tggagtgtag tggtatgatg 21480
ttggtttatt gtagttttta tttttttgggt tttagtaatt tttttgtttt agttttttga 21540
gtagttgaga ttataggcgt gtattattat tgagaataat gtttttttata gtgtttattt 21600
ttttgtagga gtaagttttt aaggtaagga ggttgttttt tttatggatg aataaattga 21660
ttttgattta tagttttacg tgttagtttt gagggtgggg aaaggttgtt ttgtgtttag 21720
gtagggggtg ggggtaaggt taggagggtt tggtgtttag tgtatttttt taggatataa 21780
ttttaggtag tagaggttaa ggatagaggg gaggagaaga gggaggtggt tattattttt 21840
ttattttagt tttttttgga gataagtttg agtattttag ttgtttatta agtttggaga 21900
taagtttgga atttaaattt ttgattttga tttagttttt tatagttttt gtgagtaaat 21960
tgagttttag aaaaagggag tgaagtttgt gtattttgga cgtttttatt ttaggtttaa 22020
atcgtttttta ttttaggttt aaatcgtttt tattttagtt taaatcgttt ttattttagg 22080
tttaaagcgt ttttatttta gtttaaagcg tttttatttt aggtttaaag tgtttttatt 22140
ttaggtttaa agcgttttta ttttaggttt aaatcgtttt tattttagtt taaatcgttt 22200
ttattttagt ttaaattatt tttaatatag gtttaaatta tttttaattt aggtttaaag 22260
tatttttatt ttaggtttaa agtattttta ttttaggttt aaagtgttcg taatttaggt 22320
ttaaagcgtt tttattttag tttaaatcgt tttattttag gtttaaatcg tttttatttt 22380
aggtttaaat tatttttatt ttaggtttaa atcgttttta ttttaggttt aaagtgggaa 22440
ggagttatag ggaaataaac gggggaggta cggatgatta ggatgagggt ttttatgatt 22500
tattttttttt taggggttat tttaagagtt ttttaaattt ttattatttt ttaatagaga 22560
tggggttttg ttgtgttgtt taggttgatt tttaattttt agttttaagt tattttttta 22620
ttttagtttt ttaaattgtt gaggttatag gtgtgagtta ttgtatttgg tttttttttag 22680
gagttttaag aaaaaaaatg ggaatagatt tgtgtgtgtg tgtgtgtgtg tgtgtgtgtg 22740
tgtgtgtgtg tgtgttttga gaaagagttt cgttttgtta tttaggttgg agtgtaatgg 22800
tacgatttcg gtttattgta attttcgttt ttcgggttta agtgtttttt ttgttttagt 22860
tttttgagta gttgggatta taaatatgta ggttgatttt gtatttttat tagagacggg 22920
gttttattat gttggttagg ttgatttcga atttcggatt ttagatgatt tgtttgtttt 22980
ggttttttaa agtgttggga ttataggttt gagttatcgt gttcggttag gaatagattt 23040
tttttttgtag atgattttg ttattattga aaatggatgt ttatgtataa ttgaattgtt 23100
ataacgtttt attttaaaaa gtttttttttt gagaaaatgt gatttttgat agaggagaga 23160
ttgtttaggt tttatttatt gttagtataa aggtagagta ggggtgtggc ggtgttattt 23220
ttttttgaga tagttttatt tttgttttgt ttaggggttt agaagtttgg tttattcgag 23280
tttatagagg ggtttggagt ggttgtttta tattataggt ggggtttgta aggatttgaa 23340
atgattttg agaaggcggg tgtggtgggt gtattttaat tattaaagta ataataatga 23400
tatattaaat agtgtttgtt tagattttga gtgtttgttt aaatattttt attttgtgat 23460
ttgttagatt ttagtataat aatttaggta aaagaaaaaa tagttgggcg tggtggtttg 23520
tgtttgtaat tttagcgtga ggttaaggta ggaggattgt ttgagattag gagtttgaga 23580
ttagtttggg ttatttgaga ttttattttt gaaaataaat aaattaataa aagattttta 23640

```
tgttttatgt ttttttata tttaggtttt ttaaattata tgtattgggg aaagtttatt 23700
aagagaagtt tttggtcggg cgtggtggtt tacgtttgta attttagtat tttgggaggt 23760
taaggtggga ggattataag gttaggagtt cgagattagt ttggttaata tggtgaaatt 23820
ttatttttat taaaaatata aaaattagtc gggcgtggta gtgtatgttt gtaattttag 23880
ttacgtagga ggttgaggta ggagaattat ttgaatttgg gaggtagagg ttgtagtgag 23940
ttaagttggt gttattgtat tttagtttgg gcgatagaaa aagattttat ttttaaataa 24000
ataaataaag ggagagaagt ttttgggttt ttagatatat gggttagtgt ttatttttgt 24060
aaagttgttg tgtttaaaga ggggttgtat ttggtggaat tggggtaaaa gagtagggag 24120
ggtattgggt agttgatttg gtttttatta ttggaaaggg gttgtgttgt tggtgttttg 24180
agttggtttt ataaggaaga tattgtatag ggcgttttaa gtttgttttt tttgtttagg 24240
tttatttttt tttttttagg attagggagt ttcgttcgtt ttttgttttc ggtatttgtt 24300
ttttggtttg tgagtattgt attttttttt gtttggaacg tcggtttatg gtgggttttc 24360
ggatttttgt tttttgttgt tttttgttat atttcgttaa agtgtgtttt atttatttat 24420
tgttattatt ttttgaaata gagttttgtt ttgtcgttta ggttggagtg taatggtgtg 24480
atttcggttt attgtaattt tcgtttttttg ggtttaagcg atttttttgt tttggttttt 24540
cgagtagttg ggattatagg cgaatgttat tacgttcggt taatttttgt atttttagta 24600
gagatggggt ttttttatgt tggttaggtt ggtttcgaat tgatttcggg tgattggttt 24660
acgttagttt tttaaattgt tgggattata ggcgtgagtt attatgtttg gttaattatt 24720
ataatattga tattataata atattatagt tagataacgg tgattaacgt ttgtaattcg 24780
aatattttgg gaggttgagg taggtagatt atttgaggtt agttcgagat tagtttggtt 24840
aacgtggtga aattttgttt ttatcgaata tataaaaatt agtcggagcg tgatggtagg 24900
tgtttgtaat tttagttatt tgggaggcgg aggttgcggt gaattgagat cgtgttattg 24960
tagtttaatt tgggtgatag agtaagattt tattttaaaa gtagtaataa taataataat 25020
aataataata ttatttattg agttttaatt ttgtgttagg tattgtgtta gtatttgcgt 25080
tatgtgattt tattgattcg gaataattta tgagtaagta ttatttttgt ttttaaggaa 25140
gttgagattt aggaaatgga agttattttt tagggtttat agtaagggtt tggcgaggtt 25200
tgcgtttaaa ttttgattat ttgattttag gttttttttgt ttttttttgtt ttttaaaaat 25260
ttaaatattt ttatttggga tgattttaga tagttagatt ttaattgttt tttttttttt 25320
tgagtatgtt ttattttaaa attatataaa gataatttat tttttaattg gagttacgga 25380
atcgttagat gttttggaga ttttttttagt tattttttttg ttggatttag atataggttt 25440
gaataattag gtttaaggag ttgaattgga gtcgttcgtg gttagaggtt tttttttggg 25500
aatagtagtg gcggtagcgg ttgcgttgag tttggtaatt tgtttaagag taggggatgt 25560
ttaggttgcg ttcgtttttt ttttatgaac ggtttgtttt gcgtttttttt tgtgttaagg 25620
gttgttaggt attagatgtt tattttagga aattagaaaa ataaatagtg taaataaaat 25680
aaaaatttta tttaagaaat ttgagataga tgttgttttt tttatcgttt ttttattttt 25740
atgttgggtt tggagagagt gggttagtat gtttttttacg gataagaggt tttggggtat 25800
ttaatttgga tggaattttt tttattttttt ttttgtttgg tgttaggggg tagaaggtag 25860
gggtggtgtt tatttggttt agggaggagg atagggtagt ttttgtcgtc ggtatttttt 25920
gtagagatgt tttagttaga agtgatttta tttttaggaa cgtgtgtttt gtttttttgtt 25980
gggcggtttt tttttatttt ttattgtttt tatagtgggg tgtgttagat ttatgggtag 26040
agttttggtt tatttttagt ttcggatagt agaatgtttt agtagtgtat tgatggtgtg 26100
tgcgtgggga tgtgtggaat aggggatagt gatttcgtgt gcgttatatg tagtgcggtt 26160
tttagtcggg tgtgtgaagg gggtgtttat ggttatcgtg gaagtgagtt tagttggggt 26220
tggagggttt aggtttattg tatgttattt agtagttttg ggagtggggt ttattttttt 26280
tttcgttttt tttttttttt ggttttgggt agtttatcgg agttgtattt tgggttattt 26340
tttttggttt taaggaggtt gttatttttg ggaggtagat gttggttttt ttattttttgt 26400
tttttggaga tttgcgtcga ggtttgtttt tttgtttgga gggattggtt aggaattttt 26460
gggttttttt ggtggggttt ttgttgttta gtagtgagta gagtgagtgg gtattaagag 26520
gaggatgttg ggtgaggtta cggatatggt tggggtaggt tgggggtgtt ggaggtgaat 26580
ttcgtagttg tgcgattttg ggtagtttgt ttagttttgg gtttcgtttt ttttttttatg 26640
aaatggggcg ggagtatttg tttgttgggg ttgtgggttg ggtgaatcgg agttaaggta 26700
tagtttatag tttgtcgtta gattttcgta agtagaagtg ggtgtttttg tttagcgttt 26760
ttcggttttt tttttaaggt ttagttttgt ttttattttt tttatgaagt ttttttggtt 26820
attttaggag ttacgattta tattttttttt ggagttttgt gttatacgta tttttttacgg 26880
tgttgttttt tttggaggat gggtttaatt aaaattgggg tttatttggg ggtttttttt 26940
agtattagag aagtttaggt ttagtagaga tgggtatttt gttaatatta gtttcgcgaa 27000
ggatatattg tgtttatttt gttgttagtt gttgagtttg gtgttttgta gttatggaat 27060
atgtgtatag gtttgggttg ttggatacga aggttagatg ggtagtttat tttgttttcg 27120
```

ttttgagttt ttagaaataa gttggttgta tttattttta tttggttata gtttaggagt 27180
ggggttgtgg aatggatgga aggaaggatg agtgggtggg tggggggtg ggtgggtggg 27240
tggatggatg gatggatgat gggtggatgg gttgatggat gggtggatta ggtggatagg 27300
tgaatggatt ggtgggtggg tgggtgggtg aagggttgaa taggtgggtt gggtggatgg 27360
gtggatggat gtgtggaggg gtggtggggt ggatggggtt tatgggtgaa tggatggatg 27420
gatgggtgaa tggagtaaat gagtgggtgg atggatggat ggatgttgta agagtttttt 27480
tggagggttt tagggaggaa ttggtatttt tgattagtag ttagtatggt gattagatat 27540
agggaggtgt ttgggtttgt ttgatttttt attttattgt tggtgggatt gggttggtta 27600
tgtgtttttg aagtttttg aagggtggtt gtatggtatt gtttagggga tatttttaga 27660
gtgtcgtgtt ttatagtaat agggtggtcg tttgtgtttt tagggtcgtt tttttggta 27720
gtttttcggg tttgtttggt ttagtatttt agtaaatttt ttcgagttag gatttgtttg 27780
ttatgagttt ttttaataag tagttttagg ttaaggtttg tgtgtgggta gagggagcgt 27840
atgtcgcggg aggttgtatt aatttgaggt tttgtttttg ttttttttg ggtaaaagg 27900
taaaaatagt ttgttttatt ttattttaa atttagttcg gttgtattta gttttgggag 27960
tgaagggggt tgggttagga ttgttcgtga gttgtagatt ttggtgtagg gaaattttga 28020
tttataagac gggtggatat ggaggaagga ggagatttcg tttagtagag atacgtttgg 28080
gtgtagttta gtttggagtt tgatggacgt gggtttattt gtgtgtaatt tttatttatt 28140
gtgtggttat agggaagtga tattttgtgg agatttagat tttttatttg taaaataggg 28200
gtagtagggt ttgggggagg atgtgtgtga aggtttggtt tagggttttt ttagtagtta 28260
gatagtagtg gttgttattt ttttagcgtt tagggttatt tgggggagta gggtttttgt 28320
ttttagatgg gtttcgtttt tggttttgga atttatagta ggagtaaagg cgaggatatt 28380
tttggtgtta attatttatt cggagggaga gcgttagtgg ttttgtgtga tttttgtttt 28440
ttgtttttgt ttttaggagg tacgcggatt tttagtggtc ggtttcggag gtttggtgat 28500
tttagtggtt taggtaggtg gttcgtttcg ttttggtttt atttagttta tgggtgtgtt 28560
tgtgttgggg ttttttttatt ttggtttggg atttgttttt ttatttggtt tttttggatg 28620
agtggggtta tttgtttatt ttttagggag acgtagtttt tgacggtttt ggatttaggt 28680
ggtttttagg tttgtcgacg ttgttttgtt ggagatgatt tttgtggggg taggggtagt 28740
tatttttagg tttagggagt aagttcgtgt gtgcgggata gaggagagaa taggagatag 28800
atagggttgg atgatttggt ttgagatgtt gaggggatag atgtgttatg gataggtggt 28860
tttggtcgtg agtttttcgg tgttggttta gtatttgtgg gtttttttgta attttgtagg 28920
atggaagatg ttttaggata gagtagatac gttcggtgtt atttaggagg aaataagtgg 28980
gtttttggta gtggttattg tgttattttt tgtgtttttt ttatgtgttt ggaaattttt 29040
tatattaatt agtagggaag ggaaatagat gtatttatta gagttttggg gttggtcgtt 29100
taggatacga gttatagttt ataggtttga agggatttta ttaatattgg gtgtagtttg 29160
ggtaagggag tgggatagag aggttagggt tttgttttta gtttttgttt tttagggttg 29220
agtttgcgcg ttattttttt tttattgggt gtttttgatt tttttgtttt gattttagtg 29280
tggttatgtt agtgtttttg tttttgggag tttacgttgt ttttggttgt tatttttagc 29340
ggttaggttg ggataggtgt ttatttagtg gtttttttat ttggagagga tgatgggtta 29400
tagtaatgaa tggatttatt ttttttagga gacgaggagt tttgagaaga gtagagatgg 29460
cgtggttatt tgtgtatatg tgttgggggt taattgggtg ttagattttt ggttgttatt 29520
atggtggtag ataggtaggg attgtgtttt tatgaattag ttattttaga ggagataaag 29580
ttattaaagg atacggattc ggagttaaat ttatataaat agagcgtttg tggattaggg 29640
ttgtttttag aataaggtga gatagtcgtt ttgttaggat aaggcgttta cggggggtaag 29700
ggtatgtgtt tatttggagt cgttattttt atgttttaga ttagtttcgt ttaatagaat 29760
tttttgtgat ggtgggtttg ttttatatat ttgtatttcg ttattttgta tggtagtcgt 29820
ttagtcgttt gggtttattg tatatttaaa gtataaatgt ttacgtttat tttatttatt 29880
tatttatttg tatagaatta tttatttatt ttaaatggat ttttatttat atttacgtgt 29940
ggttagtggt tatgaaattg aatagtttta gattatattg gtatttgaga ttttagaatt 30000
ttagatttaa agtttgtttt tgattttgag tttgtttttg tggggatagt atagacgttt 30060
tttaagtttg tttttttagag gtggatgtag tgttcgtgga ttgtgtatta ttaggtttgg 30120
atagtggaga gggtggtttt ttatagggag tgggagtgga ggggatgtcg tgtttatatt 30180
gagtgtatgt gaggtttttt gtagtatagg atgggattgt atgtgggaag agggggggtga 30240
tgttttggtt ggaggttttt gggtagttgt atgatttagc gttgtggtta agaggggttt 30300
cgtttatcgt agttatgttg ggtattaatt attggatagt ttagtcgttg ttgtgttttt 30360
ggggagtttt ttttcggttt cgtatttgat ttgttttttt attttttttt tttttattgg 30420
ttagtttttg gtggaatggg tggggagggt tttttagttt ttgtgggggt tgtattttgg 30480
gaatttggta ttttgggttg tatggatata gttttttttcg gcggttgttt tttttttgttt 30540
agattggttg gttttgtgtt gttagtattt ggtagttacg attatagttt agtttggaag 30600

tttaaggtat aatttgttga attttaaagt tgtaagttgg tgatttgggt ttgcgtggga 30660
ttgtttgttg atatacgggg tttttttttt gtgggttttt gtgggttttg gttcgagata 30720
tttttttttt attttttgg tttttttttt ttttttgtt ttttttttgt taagattttg 30780
gtttaggtag gagtttattt ttagacgttt tagtattttc ggttttttta tttgatattt 30840
tttagacgat tgtttgttat tttatttttt tagtttagtt tttttgtaga tttatatttg 30900
gttaattttt tatgtttggg atttttgttt tttttagaag gacgaaggtt tttttcgagg 30960
ttgtgtttgg gattgggtta gggatgatat ttttggtaaa agtttaaatg atattatgat 31020
taacgttttg gttagtttat atattttttt tttttttaaa aaaaaaatgg aatttttaga 31080
gttataaaat aaaaatgtaa agaataaggt aataaaatat ttgtgtattt attatttcgt 31140
ttaagaagtg agatattgta ggtgtagtcg tagttttggg tgattttttt tattttagtt 31200
ttatttttgt tgcggtgttc gttttttttgg attgggattt ggtttcgggt aggtatattt 31260
gtgttgggta gtggtgtgtt tgagaagttt aggttttttt tttttttttt ttgagataga 31320
gtttcgtttt atcgtttagg ttggagtgta gtggtattat ttcggtttat tgtaattttt 31380
gtttttttggg tttaagcgat ttttttgttt tagtttttttt agtagttagg attataggta 31440
ttcgttatta tgtttggtta atttttttttg tatttttagt agatataggg ttttattatg 31500
ttggttaggt tggttttata tttttgattt taggtgattt atttgttttg gttttttaaa 31560
gtgttgggat tataggtgtg agttatcgta ttcggtttgg tttttttttg tatacggtta 31620
ttagtatcgt gtgattgagt agtttaggtt tgtttttttt tgaatgtcgt tattagtatt 31680
atatttttgg tgttttattg gagttagtgg ttgttttgtt tgtgtttata acgtgacggc 31740
gtggagattt attcgtgtag atttacgaag ttttggttta tttattttgg tagttagcga 31800
gtgttttttt gtgtggggag gtagagtttg ttcgtttcgt ggtttattgg tgatatcgtc 31860
gaaggttttt tttgttatta gtgttgtcgg gaacgtgtgt ttttttaggt ggatttggtt 31920
gggttgtgga gatgcgtttt ttagtttttt tagatgttat tattacgttt tttcgagtgg 31980
tttttttggta ggtttttttta tgttcgttta tttttttgtgt tttaggtagt cgtttttttg 32040
attgagcgtt tagcggattt ttgttttgtt ttttacggtg tacggagtat tttattttag 32100
tggtgtttga gttttttttt ttttggtttt tagatttcgg ttgggatttt tggagttaat 32160
tttttttgttt gatatttcgt gtcgttaggt tttgtttttt tattttttaa tatttttggg 32220
gtgtgtggtt ttttttttag tgtttttatt attttttttg ttttaggttg aatcgtgttt 32280
tttataaatt tatgtttatt aaaaatttta gaatgtgatt tgggagttgg agatggggtt 32340
tttgtagatg taagtagtta aggattttga gatgaggtta ttttggatta gggtgggttt 32400
taaatttaat aattggtgtt tttaaaagaa gtagagaggt tatagatata tcggggagag 32460
ttacgtgaag gtaggagtag atgatggagt gacgtagttt taggttaggg agcgtttggg 32520
aagaggtaag gaaggatttt tttttttattt tattttagag tttttttttagg gagtgtggtt 32580
ttgttgatat tttgattttg gatttttggt ttttagtatg ggagagaatt cgtttttattt 32640
tttttaagtt ttatagttgt ggtttattat ggtagttata ggatattgat gtatttttta 32700
tttggtcgtt gtggatggac gatttcgttc gtaggaagtt ttttttaagt attgtaggtg 32760
tttttgtttt tataggggac gtttgtataa gtaggtgtga aaaaagggtt ttgtgggggt 32820
aaggtttggg ttggtgttcg ttgttttttt ttattttatg ttagttttta tgtttttttc 32880
ggtaggagtg tttggtttta tttggtgaat tagagttaaa ttttttttgat aattggttgt 32940
tatttattta attgttatta tttttaggag atagaaaatg ttagaagggt ggggtttgtg 33000
tagtgttttt tcgttatttt tttagggtat cgtgttacgt tgttttaatg tatagagtag 33060
ttgtttaaag tgtgtcggac gaataggtgt tttatgttta gttatgtaga taaaagtgtg 33120
ttacgtggag ggagttggtc gtggaagaga ttattagtat tttggttgtt cgaggatttt 33180
gtttttgggt ttttgtaggt atttttagtt tattattttt tttttttattt atagttattt 33240
attttttttt tattttttttg atattagttt tgttatttat ttaggtttgt aagtaggaaa 33300
ttttagagtt gattttgatt tttgtttttt tagtattttt aggttttaat aggttgttaa 33360
gattttatta attcgttttt tgtatattga tttttggata ggttgtttcg gttacggtta 33420
tgttttggtt gggtttttat tatttttttt tggattagta ggtgttattt tttttatttt 33480
tgttttgttg tggttttttt tttttttacgg ttaatattta gaggacgatt attatttttt 33540
tttttgtgtg ttggggatcg ggtgatagga ttttttttatt cgattatttt tattagattt 33600
gggtcgggtt tttttattta tagagttttt agttttcgtt tttaaagttt cgagggtgga 33660
ttcgtgttgt cgtttagttt ggttttgata tcgtttttta gtttggagtt ttttgttttt 33720
gcgtttttgg ttttgagtgt tcgggttttt tttggatggg ttgttatttg ttttttggaa 33780
agtagggttt ttatgttttt ggatttttta gttgatttag tgattttggg ttattttgtt 33840
atattttttt tattagtttg gttttggtta tttttgtgtt ttttatagtt tcggattaga 33900
gttacggcgg gatgtttttt tggtgcgtgg taggtagtta ggaaatattg ttggattgat 33960
tttgatagga gatgtttggg agaattgttt aaaaaattag tttaaaatgt aaatattggt 34020
ttttttttgc gttggttaaa gagtagtatt ttttcgtttg tttttcgggt aagaaattga 34080

tggcgaatgt aatgttttta gggaggagaa gaaggtgata aaaagggttt gtataacggg 34140
gtatacgtta ggtatttgtg attatttaag tttaaattat aatataaatg cgaaattttt 34200
tgttttggta gtttttttat attgtaagcg tttattgtta tatgtggttg ttatattgat 34260
tacggtagat atagaatatt gatattattg cggagagttt tatggggggg cgttggttta 34320
gattattttt acgttggatt tataaattta ttgtttattt agtgaacggt tattgggtat 34380
ttattgagta agtaggatga aagatattcg ttttcggggg agtttttcgt tggttgtaga 34440
gaagtttaaa tttaggggat ttggggtaaa ttttgtaagg gtttttggtt tttgtatttt 34500
tagattttt tcgttttgta ggggtagtcg gggttttttt ttaggaatgg agcgttagtt 34560
ttagtttttg ttaggttata gtttagatgg ttggttgagt acgagtatgt gagagtggaa 34620
ttgggtttaa tcgggttgtt ttttttcgtt gttttggttt ttttttacgt tgatttggaa 34680
ggatttttaa agtaataggt ttgtttaggc ggtttgtatt agtaaatttt gtagcggttt 34740
taagggtaga gatagttgga ttgtatttat ttaggaatag ttaaagtttg aggtataata 34800
aaaagtggag aaagatttta gagaggaatg tagttggttt tagtttggga attcgttttg 34860
tttgggaggt tgtaggcggg ttaggttggg gttagtttgg agggttttgg ttggttttgt 34920
tggtaggtat agtagtaggg ttagtaggag ggagtttagg acggagtttt tttattttta 34980
atttagggaa gtaaatttta gtggttgagt ttgaggatga gtaggaaatg aatggaaaag 35040
ttttttttt ttttttttt tttttaggta gggttttttt ttgtttttta ggttggagtg 35100
tagtggtata attatagttt attgtagttt taatttgtta ggtttaaggg atttttttgt 35160
tttagttttt taagtagttg ggattatagt tgtgcgttat tatgattggt taatttttaa 35220
aagaaatgtt tttaggttag gtatggtgat ttatgtttgt aattttagta tttttggagt 35280
cgaggtggga ggattatttg agtttaggtg ttttagatta gtttgggtaa tatgacgaga 35340
tttcgttttg attaaaaata taaaaagtta gttgggcgtg gtggtatagt tttgtagttt 35400
tagttattcg ggaagttgag gttgtagtga gttgtgatta cgttattgtt ttttagtttg 35460
ggtaaaggga gtaaggtttt gttttaaaaa aaaaaatttt ttttttttt gaggtggagt 35520
tttgttttgt tgtttaggtt ggagtgtagt ggtatgattt tggtttattg taatttttgt 35580
tttttaggtt taagtgattt ttttgtttta gtttttaag tagttgggat tataggtatg 35640
agttattgtg tttggatttt ttttttttt ttttttttt gtagagatgg gatttttta 35700
tgttgtttag gttggttttg aatttttggt tttaagtgat tttttattt tggtttttta 35760
aagtgttgga attataggtg tgagttatta tatttagtag gaaaagttat tttttaaagc 35820
gagaggttgt gcgtttttta tagtttggcg tttattagta tggaggattt attggaacgt 35880
gtgttaggtg aggggatatg gaagatggta gtaaattcga gtttagagta ttagaaagta 35940
tgtttttagt taaagatgcg ttttttttat tttcgcgtag tatttttttt tagtgtgtat 36000
ttttggagta aggtggggtc gatttacga agattttttt tcgaaggttt ttgttgttta 36060
tattagtgtt atttggatcg tttgtttttt tttttggatg gttggattag ttttagggtt 36120
ttttggtttg gtgtggggag tatagtttat tttggggaat gtttggtatg tgtttttttt 36180
ttattgggta ttaggggatg aagttgtgtt tcgtggaagg ggatagaggc gttggtgttt 36240
aggatggtgt atttggggcg gttgaattgg atttggtttt tattgggttt ttgtggagtt 36300
tagattgttg tgagaagggg tggggaaagg taggtgattc gtatttttta tttttgtttt 36360
atatgggaat aggggtttat tttgtatata tatttagttt ggagtatgaa tttttatgaa 36420
gaggaaaatt tttattaggt tttgatgtta ggattgttcg tgagggtcgg gtcgtgtttt 36480
ggggtaagga taggagtgga gggggtaatt agggaaggtt ttttaatgga ggaggtattt 36540
gatagtattt tgaaaaagaa gtagttttta ggttaagtta gagattgaag aatgattatt 36600
taattttttt ttttttttt tgagatggaa tttcgcgttg tcgtttaggt tggagtgtag 36660
tggcgtgatt taggtttatt gtaattttta ttttttaggt ttaagtaatt tttttgtttt 36720
agtttttga atagttggga ttataggtat gcgttattat gtttagttaa tttttgtatt 36780
tttagtagag acggggtttt attatgttgg ttaggttggt ttggaatttt tgattttgtg 36840
atttatttgt ttcggttttt taaagtgttg ggattatagg tatgagttat tgtattcggt 36900
tacgattatt taattttttt ttataagatg tttttttggtt gacgtaaagt aggttgagtg 36960
agtgtgatat tttttttttg tttgtgggtt tcggtttttt tagagttcgg ttagtgttat 37020
tggagataaa gtggttttcg tttttttttt tcgttttgtt aggtttagtt tgtgtcgttg 37080
agttttattt tgtaattaag aggagttgtg aattagtgat gttgatgtcg tagaaaagtg 37140
gttaggggtt tatggggggt gaaggatagg gatgtcgttt tgagaattgt ttggaagggg 37200
tttttgggta atagtgttta ttttagggtg tgggtaggtt ggggtggttg ggagttgggt 37260
ttataggttg gtataattag agttttttaa agtcgtgggt ttgagggttt tgttttttg 37320
ggatagtaat ttttggttta atttgggaaa aggtgggtta aattaagttc ggtttttta 37380
ttgtaggatt atttagtgcg tttagtatgt aaatgtgttt ttgaatttaa gataggatta 37440
tagaaatttt tttagtgttt ttttggaggg agggatcgcg ggggatttat cgtaggggtt 37500
tgggtttgta ttggtttggt gaagtgtttt tttagtgttt atattatgcg cggtgagagg 37560

```
ttggttttcg ttttttttat tttttgttgg ttttatttta ttatttttgt attcgttttt    37620
tagtcggttt atgattaatt tttgtttttt tagagttaat cggtgttgtt aggtttttta    37680
ttttaagtga tttttcgttt gggttaaaat gtttggattt ttaggtacgg aggggagatg    37740
gattttaggt taaattatgt tgggtaaaat tgagggttaa ggttgtgttt agtaagtgtt    37800
ttagttttgg tcgttaaatt tatagtgtgt atagcgtgta ggaggtggtt gggtgaatga    37860
atttagttat agttcggttt tttattcggg gttgttttta agtttttatc gttagttagg    37920
ttgggttaga gtttcgtggg gtttttttgg gttttttttgt tttatagtag gagttcgtat    37980
ggagagtttc gagtgaggtt aatgggtttt tttttttttt tttttttttt tttttaattt    38040
gagatagggt tttatttttt ttatttagtt agggtgtagt agtgtaatta tagtttatag    38100
tagtttcgaa tttttggttt taagtaattt tttcgtcgta gtttttttaaa gtgttgagat    38160
tataggtacg tgttattttg tttagttggg gtttttgttt tttatttttt tttttgtttt    38220
taaatttaat ataaagaaag atattatatt tataattaga aaaattaata aggaatatta    38280
ttttaaaatt ggaaaaggga ttaaagaaat aaattaagaa atttttgcga tttttgttta    38340
tcggagttag tggttgttag tggttggttt aatataatgt tttaggaaga aatagaataa    38400
tagaaagtaa ttttagtgag ttgagttttg gtttcgtttg tggcgttgta ttagtttttt    38460
gtagttgttg taacgagtgg ttataagttg gttgggttat aataacgttt agttattttt    38520
ttatagtttt gtaggttata tattcgagat tagggttatt aggttgaaat tagggtgttt    38580
attgggtata gtttttttgga ggttttaggg taggggtttt tagtttcgtg tgttttatta    38640
ggaattggtt tgtatagtag gaggtgagtg attggtaagc gagcgaagtt ttatttgtat    38700
ttatagttat tttttattgt tcgtattatt gtttaagttt tgtttttttgt ttgattagtt    38760
atggtattgg atttttatag gagagtgaat cgtattgtga acggtatatg tgagggattt    38820
aggttgcgtg tttttttatga gaatttgatg tttgatgatt tgttatcgtt ttttattatt    38880
attagatggg atcgtttggt tgtaggaaaa taagtttagg gttttaattg attttatatt    38940
atggtgagtt gtataattat tttattatat attataataa taatagaaat aaagtgtata    39000
attaatataa cgtgtttgtg gtcgggtgta gtggtttata tttgtaattt tagagtgaaa    39060
ttttgtttta aaaaataaaa aataaaggtt aggtatagtg gtttatgttt gtaattttag    39120
tattttggga ggttaaggtt ggcggattac gaggttagga gtttaagatt agtttggtta    39180
atatggtgaa atttcgtttt tattaaaaat ataaaaatta gtcggatacg gtggtaggcg    39240
tttgtagttt tagttttttg ggaggttgag gtagaagaat tgtttgaatt taggtggcgg    39300
aggttgtagt gaattgagat agtgttattg tattttagtt tgggtggtag agcgagattt    39360
tgttttaata aaataaaata aaataaaata ataaatataa tatatttgaa ttattttaaa    39420
attatttttt tatttttttgg tttgtggaaa aattgttttt tttgaaatcg atttaggtg    39480
ttaaaaaggt tgggggttat tgtttttatc gtttgttttt ttgagttttt ggtggttgtg    39540
ggtgtttttt ggtttgtggt tgtatggttt ttatttttaa tattagtatt tatagatttt    39600
ttcgggtttt atttttattt ggttattttt gtttgtgtta aattttttttt tgttttcgtt    39660
ttgtatgagt atcgagattg tttttaggga ttatttagat tatttagggt gttttcgtta    39720
tagtaagatt tgtaattgaa ttatagatgt aaaaattttt gttttaaata aggttttatt    39780
tgtaggttta gggattagga tatgttattt ttggggttat tatttagttt tttttcgtga    39840
ttatgatttt tttttttttt tttagttttt tatttcggtt gagatgttgt ttttattttt    39900
tttattgttt ggttgtttgt ggggttgttt tttaggtttt ttgggaggag gtggttgttt    39960
gatgtttttt agaaggttgg gaatggtttt ttttattttt taggaagatg ttttttttgt    40020
taaggagatt gtatttgggg ttttatttta ggtagagagc ggacgggatt gtggtttgaa    40080
gtggtatttt agggttttttg gttttgttat ttagtcgagg gttgtgtatg ggttttattt    40140
ggttataggt aatttatttg gttttgtggg gtagggattg taaatttaga ggtttgcggg    40200
gtgtgtcgag gtagaggggt gtaaatattg taaatttgtt gggggttttg gggagtagta    40260
tagattgaaa agtgttttgt ttgaagagta gaattgtttt atattagttt tgtgcggtta    40320
ggtaggaagg ttttcgtggt gcggttacgc gttttttttt ttttaataga agttgatagt    40380
ttggagtttt tagagagatt gtttgagttt ttagttttgg tataaatata tatttttttt    40440
aaataggttt gggttaatag aattagtttt cgtgtttcgg gtttgaatga atttttgagg    40500
tgaagtttaa agatagagag gatgtttttg atttttattt tagggattta tattgtaaat    40560
ttttatttgt tttgttgagt ttttggtttt agtatggatt ttgattatgt gttggtgggg    40620
atttttttagg taagtaagta gtttttgagg atttcggatg aatagtgggg aatagtattg    40680
atttttttagt ggggtttcga gttttcggag aggaagattc gttttttttt aggggacggt    40740
tagagattta ttgatttatg cgtgtgtggt aggaattttt taggaagttt tcgtttcgtt    40800
cgtttttttt gtttgggcgg ggacggtaat tgtttttgtt attgtagatt aatgggacgg    40860
agggggtga ttttttaggg tttttttttgg gtaggtgttt cggaattttt ttttagtacg    40920
ttggtttggg gtacggtcgt tttttttgtt tagttacgtt ggggtatagg gtgaagaagg    40980
gttggggtta gtttaggata gaggaaggcg aggtaggtac gtaggaattg ggttttttaa    41040
```

```
atttttaagt ttaaggaaat cgtagtatcg cgggataggg aaaatgaaag attttggaag    41100
tcgttaggaa tttgattttg tgagttggtt tttaagagtt taagttaagt atttttaagt    41160
ggatattaaa aaggagtagt aagtttcggg gcggcggggg ttggaggagg tggagagagg    41220
aggttgtcgg aagtcgtatt cgggattttt gtagttatcg attagatcgg gcggtcggga    41280
ttttgggatt ttcgtaggta gattcggtgg tttgtcggat ttttcggggt ttatttcggg    41340
tttttttttt tgttttttat ttttggattt ttttttttttg tttttttttt tttttcgttt    41400
tgaagagata atgttatttt agtttggagt ataaatatat gattagtata tggaaatgtg    41460
gtaattcgga tgtattcgtg attgtaatag attgaagaaa ttagattaga taaagagtgt    41520
ttttagagga ggaggaggag gaggaggagg ttgagagagg gagggcgacg gggtgagaa    41580
aggggaggtc gtttttgagc gggacgtcgg gattttcgtc gttgttaaat atattcgtag    41640
gaatggagag ggatcggatt ttaggtacgt agatagtttt tttttatcg gtcgtttttt    41700
attgttttgg attcggggtt ttatttatgt ttgggggaat aagtatgtaa agggcgtttt    41760
tgtttatttt ttatagaaat attttttttt tttttgacgg ttttaagttc gtgggatgaa    41820
tttcgtttta gaagttaagt ttttttgagt ttggttttta tttaaattcg gttttttttt    41880
tttttggtta tttttttttt atttttagt gtgttatttt tgtatgttat ggttattgtt    41940
aatgttgagg ttgttggcgt cgaaatttg ggagggtgtg ggggatttgt tttttggtt    42000
cgggtttggg gcggcggcgt ttggatttgg ttggtggttg gtttgtttat tgtgtggttt    42060
gtttttgtt tttagttaaa gtttgttttt aaggttgttt cggtttttta tttaaaatag    42120
tatttttgtg ttttttttggt agttgattat agagggggaa gttttcggcg tttagaggag    42180
tttggcgtgt gtgtgtacgt gtgtgtgtat gtgtgtgcgt gtgtttattt acgttggtag    42240
ttttttttgtc gtaggtatag atttattagg gcgtgaatag gtaaatgaag agaaaggggg    42300
ttttttttgtt tttagtttat tttagttttt taggtttttt tttttaggtt tagttttttt    42360
ggttttttta tttatatgcg gtgtcgtggt tgttatttta cgtggttagc gtcgttatcg    42420
gtaattagta gtgatgatag tttttgtcgt ttatgtcgtt ttttgagttt agcgttggga    42480
gatatggggt ggttgagtta agttttaggt tttgggtatt ttgaagtgat ttacgggtta    42540
tggagcggga ggtcgttaag agtgttaatt tttacgtgtt ttgggttttg gtttgttggt    42600
tgttattatg tgtgtgttgg ggggtttttg agaggtttga gagagggagg tttatttcgg    42660
tggttttttt ttaggacgtg tggttttttt tattattgtt tttagggtta tatttttttt    42720
tttttttttt tttgagacgg agtttggttt tgttgtttag gttggagtgt agtggcgcga    42780
ttttagttta ttgtaagttt cgtttttttta gtttatgtta ttttttttgtt ttagtttttt    42840
aagtagttgg gattataggc gttcgttata ttttttttgta tttttagtag agatggggtt    42900
ttatcgtgtt aattaggatg gtttcgattt tttgatttcg tgattcgttc gttttagttt    42960
tttaaagtgt tgggattata ggcgtgagtt acggcgttcg gttttagggt tatattttga    43020
ttgttacgtt gggattggtg gagcgggtgt gtgttgtgtc gttgagtata tttagtcggg    43080
tatagtaggc ggattttagg aggggggttag gggagacggt agaaggtttg ggtggtagtg    43140
aaattgtatt ttatttgttt gagttcgggg gtttaggttc gttgggacgg gtgggtgtgg    43200
agtttagcgt tttgtttttc gtttagtagg agtcgtaggg tagggagcgt tgagagggat    43260
tttcgttagg tttgtatagt ggagtcgttt tcggagtgat tattttatta ggtagtttgg    43320
gtagattata gtgggttttt tgttttttgg gttattagtt ttaggaggac gttattttat    43380
tcgtttggtt ggtaggggtt tcggggagtt tcgggttcgg tttttatttt atagatgatt    43440
tagtcgaggt ttagatgggt gattagtagg ggatttgaga gttgagtaga gaggcgtggg    43500
tttgggggtg gtgtttttttg gggagttgga agtgatggta ttttttttagg tttataaaat    43560
acgggttttt tatgaattga ataaaatatt ttttgaaagt agttagtgtt gtgttttaga    43620
gattatttttg atttttgttg tttttggttg aggatagtat agttagggat gaagcgggga    43680
ttttttttcg ggttgtgttt tgatgaaaga tgttttttat aaaattttat tcggatgtgt    43740
ttttatgtaa gaaaacgtat attttagaa atttagttgt aaattggttg tttttcgaat    43800
gaatacgata gtttcggagt tgtttttttt aatgaggttt tcgttttggg taaattttag    43860
cgttggtttt taattgttat tgaattttcg tatagttttt ggggaagtga tttttttttt    43920
gtttttgttg ggtggttttg agtaagttat ttggtagttt tgggtcgtag tttcgttatt    43980
tgtaaggtga ggggtgggag cggtacggtt tttaggtagc gtttttattt tttgcgggtt    44040
tttgcgcgtg ttttgagttt tgtgggcgtt tttttttttta ttgaggggtt tttttttttt    44100
gtagggtatg gtttacgtcg cgttgttagg ttgtaagtta ttgcgagagt tgttttgatt    44160
tttaagtatg ttttggtttg aggaggagtt atggggagga cgttgtgttt gtgtgataga    44220
ggtttgttcg gtagatgtcg gttcgttgat ttttagacgt ggttgtgggt gttttatgtt    44280
ttattgggtt ggcgtgtgag gcgtgtttag tttaggttgt tttatagttg tttaggaagg    44340
gtttgtggga gggggtggtt ttattttggt gagtttacgg tggtttgtta gttttaggga    44400
aggtttagga gttttaagat ttgttttttt gttttttttt cggtagtgtt tggtttcggt    44460
ttggtttgtg aacgggggtt tgggtatttt ttcgtttttt ttgggggttg tggttttttt    44520
```

ttgggaaggt gttggagggt ttgttagtag ggtcggtatg agttatggag tggtgattgg 44580
tcgttgaggt atggaaggtt attttttttta gttaagggtc ggagtttttt ttttgttttg 44640
gaacggtttt gtggttttta taaattaagt agttttattt tgttttaaga gtttgtattt 44700
tagtagggag agagttttta agaagggaat tttttattgg ggagttttgt tgggttggga 44760
ggttaggaat aggttacgag ggagggttgt aggtagggga agtggtcgtt tgtgtatggt 44820
tatgaatgta ggttcggggt tttggagggg gtttggttac gttgtcgttg gacgtttgtt 44880
tttattaggg tttatttgat gtagtacggt atagggtaaa gtttagtttt ttttacgggg 44940
tttttgggtt attagtgggc ggggaatcga ggttttttgg agggcggtgt tttagttttt 45000
attttttttt tttgtttttc gttgtgttgt tttcgatggt ttttgttatt ttttgggagt 45060
ttttattttt ggacgttcgg tgtttgttcg gttttaattt ttttagtttt gtttttttat 45120
tttcgaggtg agaggtttgg tttgacgcgt tttgtattcg tttttttgttt agtttcgtgg 45180
gaattttggt ttttgtagtg gtttttttggg gttgttatta ttaagtgtta taaattgggt 45240
ggtttaaaat attagatatt tttttttttcg tcgttttgga ggttagaagt tcgaaattac 45300
ggtattagta ggtttggttt ttttcggagg tttcgagggg gagtttgttt ttcgtttttg 45360
gtagttttttg gcggttttcg aaattttttgg cgtttttttgg ttcgtggtta tattattcga 45420
tttttgtttt cgttgttacg gggtttttttt ttgtgtttta aattttttttt tgtttgtttt 45480
ttatacggat agtttttttc ggatttaggg tttattgggt aatttaaaat ggttttattt 45540
taagattttt aattttataa cgtttgtaaa gatttttttt ttaaataggt tatgtttata 45600
ggttttgggg tggatgtaaa ttttaggggt tatttttttat tttttttgttg ttttcgtgtg 45660
tttggatgta tagtatggtt ttatgtttta gattttttttgt tggatttttt tggtttttttg 45720
gggtggtttt tggtttttttt tattcgtttt tgtgtttttg gtgaggtgtc ggggtcgggt 45780
ttagttttttg taggaatttt ggggatagta tggagtcggt aatttttttat taggcgtttt 45840
tttatttgtt tagagtttat ggttgtgagg ggtttttttt tgtggttttg atttgtaatt 45900
ttttagtggt tagtgatgtt gagtattttt tcgtgtttat tgtttatttg tatatatttt 45960
tgtagaaatg tttgtttaag ttttttattt gtgtttgatt tggatgattg ttgttgttga 46020
gtgttaggag atttgtagat taatatttag atattaattt tatatttgat ttatgatttg 46080
ttgttatttt atggattgtt tttttttttac gttgttagtg tttttttgata tgtaattttt 46140
tttttttttt ttgagatgga gtttgatttt gttgtttagg ttggagtgta gtggtatgat 46200
tttggtttat tgtaatttttc gtttttttgg ttttagcgat tttttttgttt tagttttttg 46260
agtagttggg attataggta ttcgttatta tatttagtta atttttgtat ttttagtaga 46320
gacggggttt tattgtgttg attaagttgg tttcgaattt ttgattttaa gtaatttatt 46380
cgtttcggtt ttttaaagtg tcggagttat aggcgtgagt tatcgtattt aggttgatat 46440
gtaaatattt taaattttta tgacgtttta ttttatttat ttgttttttt gttgtttgtg 46500
tttttggcgt tatatttaag aaattattgt taaatgtaac gttaggaagt ttttttttttg 46560
tgttttttttt taagagtttt gtggttttag tttttgagtt taggttttttg atgtaagttg 46620
agttgatttt tgtatgtggt gtaagggttg gtttagtttt atgttttggg tttttgattt 46680
attttttttt ttttttttacg tttagttggt ttcgttgggt ggcggggagg agtggggaag 46740
tttcgggttg ggtttgtatt cgattatttt tttttaggtt agttagggag ttttagtttt 46800
tgtttaggat ttggttggac gtgtttttta tcgggaaagt ttgggtcgtt tttttaggtt 46860
gatggtaggg ttagttcggg gcgttttgag gtttgttttg cggatatgtt ttttgtttta 46920
ggtggtgtgg ttgttcggtt tgcgtgtgag attagttgtt tgtgttttag gttatggagg 46980
ttgagtgttt ttagtttgtt tttttgttcg gtttttttttt tggggaagtt tttgtagttt 47040
atttttttgtt ttcgtttttg ttatttgtgt ttttgtttgt tttttgtttg gaggtggtta 47100
tttttggggt tattttttat gatttggata taagttttta ttttgaagtt attatttaaa 47160
tttttgtgtt ttaaattttt tttatttatt atatgggttt ttattgtgat taatttagta 47220
gttgatgaag tttttttttgg ggtttttttta gtaacgggga gttggttttt tcggaggttt 47280
ggtttttttt taagtggaag tggggcgtga gggtgttagt ttttttttgt tgtttggtgt 47340
tttaggttgg tttgttattt ttggaagtat ttgttatttt tatatagtat tttatattta 47400
tttttttcgtt ttttattttt ttttttaagg ggttattttt gtttttttttt ttatttaatt 47460
ttatttatgt ggttcgttta gtaatttttg atttttaata tgataaaata aattttttttt 47520
gtcggttatt tatttattta tttagtattg ggtgtttttt gtggatttgg cgttggggtt 47580
tcgtggagga taaagttaga tatagttttt gtttttatgg gattgtataa gtgtaagatt 47640
atattagtaa acgtgaaata taggaagtga taggtgtgat aaaggggatt agtggtagga 47700
tagaatttgc ggttcgtagg attaggttag gagggttgtt tcggggggat attttcgggt 47760
tgagcgtaga aggatgaggg gagtaaatta ggtttaaatt tagtaggtag aggcgatcgt 47820
tgtaggtaat cggtaatgtg tttaaaggtt ttggggcgcg gggggttgag gtcggtagta 47880
cggtaggaag taagattggg gttgaaagag attgattgtt atgttgtgaa atatatattt 47940
ggtttttatt tttatttttt ggtatataat ttttaaaatt tttggaattt ttaaagtgat 48000

gtttttttgg atgtttatga ttgatagatt agttggtagt tttaggatgg tttttaggga 48060
agattaggta gaattataag gtttagtatt atttcgtaat ttttaggtag gggagagggg 48120
ttgaaggtta agtagattat tagcggttaa tgattgaatt aattatgttt tcgtaatgag 48180
gttttcgtga atatttagaa ggatggggtt tcgggagttt ttggatggat gagtatgtgg 48240
aggtttttgg agggtggagc gtttggggag tatatggaag ttttgcgttt tttttttata 48300
ttttgtttta tatatttttt tttttgtatt ttttgtaata tttttataa taaattagta 48360
aattttatga gttttaggaa tatgtgtaaa agaaaaaaaa aagaataaat tatagttgtt 48420
gtagtaagtt aattttatta gtattggttt ttatttgtcg attttatttt tggtaatgtt 48480
ttttttttt gggtttttgg gatttttttcg gttttattat ttttttgta gtggtttcgt 48540
tggttgttgt ttttaggtta gtttattttt attcggtttg tttttattag gggttgaggt 48600
tttttgttt attttagttt tttggttcgt tttttgtttt gggtattttt ttatttcgaa 48660
cggttagagt ttttgaagtc gttcgttgtg tgttttcgtt tggttttatg tatttcgtac 48720
ggtgtttttt agggtttttt attggtaagg gtattggtat agatttgggg gttgttttgg 48780
tttaagttta ttttgatttg gagtttgagg gtttttgagg gggttttgaa ggttagggta 48840
ggtaaggttg gtatgttttg ggtagtgggg gttgggtatg ggaggggatc gtgggtgggg 48900
agattttttt taattagcgg tttacgtttg gagattgatt tgggttttag gttacgtttt 48960
gaaattggga tagagagtgg cgtagatttt agttcggaga tttttttgt ttgtggacgt 49020
ttttatattt ttattcgtgg agttattttt ttttttttt ttttaaatgg ggaaattttg 49080
atttgaatat tgaataaata atatattttc gaaattagcg aagaggtatt ggttttgaaa 49140
ttttgttatg aaagtttgaa aatgtttttt ggatcgattt gttggaatag acgggtgtga 49200
tggggttgcg gggttttagg tggtaggagg cgttttcgtg tttagggtat ttcgatgttt 49260
agagtttttt cgtgtttagt gtgtttttgt atttagggta tttttatgtt tagggtgttt 49320
tcgtgtttag tgtgtttttg tatttagggt atttttatgt ttagggtttt ttcgtgttta 49380
gggtgttttt gtatttagag tatttttatg tttagggtgt tttcgtattt agggtgtttt 49440
cgtttttagg gcgttttgtg tttagggtgt tttcgtgttt agtgtgtttt tgtatttagg 49500
gcgtttttat gtttagggtg tttttatgtt taggtattgt ttttttttat ttttttaatt 49560
ttttttatt aataggtgtt agttgtgaaa tgtttgttgt gtgtgttttt tggttgagtt 49620
tattttgtgt ttttaagttt ttgttagata taggtaggtt gttttagttt ttttggtaaa 49680
tgttttgttg ttgttgtttg atacggggtt ttatttcgtt atttaggttg gagtgtagtg 49740
gcgttatttc gttttattgt aattttcgtt ttttaggttt aagcgatttt tttgttttag 49800
ttttttaaat agttgggatg ataggcgttc gttattatgt ttggttaatt tttgtatttt 49860
tagtagagat ggggttttat tatgttggtt aggttggttt cgattttttg atttaagtg 49920
atttatttag tttggttttt taaagtgttg ggattatagg tatgagttat cgtgtttggt 49980
tcgtttggtg aatgtttatt gaggtttatt atgtgtagtt tttttttag gtgtttggtt 50040
tgtaatgagg agtaataaag atatagtcgt tggtcgtttg ttatttaata gttggaattg 50100
agaggtagtt tataaatgag taaatttata ggtggtttta gatgaggatt gtgaagaagt 50160
taagaatagg ttgttggggg tgtatatttg tgtgtgtggc gaggggatta atggaagttt 50220
tttttgagtg gtgatattga gttgaagttg taatgattag aaggatttag ttaggttaaa 50280
atttaaggga agagtaattt ggtaaaggaa atagtaggtg tgaaggttcg aggttggagt 50340
ttggggaagg tgggagtggt tggagtgtgt ttttagagac gggttatggt gatattaatg 50400
cgataggtag ggttaggtcg tgtcgggttt tggaggtttt ggtgagggtt tgatttttatt 50460
tttattatgg tgggatttta gtgttttgtg gttgttgaga atggttaggg gtaggtaggg 50520
aggtttgtta gggaggaggt tgttgtaggt tttgtagttg gagagggagg attaggattt 50580
ttttagagag gaagttggag agggaggatt tttttgattg ttggatggat ttatttgttt 50640
ggttgttttc gttagtattt agtagaggtt attggatgtt gataggtggt cggttttgtt 50700
tagttttgta ttgttttgtg ttgttttggt ggttgtcggt tttaggagga gcgtttttgg 50760
tgtttgagtt tagtggtttt ggttgtggtt tggtatattt ggtcgtgggt gaggttaagg 50820
tggtttggat tttgggaagg ttgggtattt ggtagtttta aggtaggagt tggggtgttg 50880
gaggaggaag tacgtttatt tttcgttttt ttttgtttcg tgggatgttg agtttttggg 50940
tagggatatg gtgagtttg gtgtgatgta tttaatattt gttggtgttt ttattttttaa 51000
gtaggaattt tggtgtttgg tgtttgagga ttgttgttta tcgttttatt attttttagg 51060
ttttttttta gtttttgatt cggggtaagg tttagatatt tagaatattt ttggttggtt 51120
tttggagttg gggtgaggta gaattttttt tttatattta tagtattagt taaggtttcg 51180
gggtttggat attatttttg ggaggggtga gtaggataag tgaggttttt agatttgggg 51240
tggtttcgga ttggttaggt aggtgggtgg gggttgtagt ttatgttttc gttttaggta 51300
atatagtcga gttgtagttt tcgttttatt tttagttggg gtttttttgtg tttatttttt 51360
gttttttttt tttttttcgg aatgggggt ggatatttta agattagttt tttagttatt 51420
ttttttgttt tttgtttttt tttttcggg atagattttt gtgatcgttg ggtgtttttg 51480

atgttttgtt ttttttgcgt tttgtgtcgt gggtgagaga gggtttcggg gggatttttt 51540
ttttttggag tatttgttgt ttcgggtgtt tttgggggcg ggtttttatt ttatattgtg 51600
gagttggtgt aggaaggaag gtgtcgggag ttagttttaa tttacgtgtt ggttgtggtt 51660
tttaaggtgt ggatggtagg aggtggtgag atagggattt attgattatg ggattgatat 51720
tttttatgt tgatgtgggt tttgagttat aattttatta gtttttgagt tttggcgttg 51780
tttttggcga tggcgtggat ttagttaggt tttcgggtag ttttttttgg attttttttt 51840
cgtttttttt tgggtagtgt ttttgtttat ttggaaagga tgggtaggat ttggagataa 51900
tagggattta aagaagtcgg gagtgggggt atttgtgtag tttattttgt agaatagata 51960
atttggttaa agtttaaagt ttgttgttag gagtttcgtt tgtttttttag agagtatatg 52020
gtttgattat tacggggatt gggggagagg tataggtatg tttgttcggt agtagaaggg 52080
aatttcgggt ttgggttttt ttattttgtg gcgttgtttt tttttagagt tgtattgttt 52140
tttttattgt ttttgtttgg ttttagtgtt tatgtggttt gtggttttta gtttgggttt 52200
ggtttttcgt aatttatttt gtttagtttt atatagtttg ttttttttagt tgggaatcgg 52260
agtttatttg gagttttagg tagtagattc gatgtagtga tgtagggttg ttagttatgt 52320
tagtaggttt tgttgtttat agagggatgg gtattgttgt tgataataat taaagaatta 52380
cggttttagg ttgggtatag tggtttatat ttgtaatttt agaattttgg gaggtcgagg 52440
taggagaatt aattgaattt aggagtttta gattaggttg ggtaatatag taaggttttg 52500
tttttataaa aaataaaaaa taataataat aataaaaatt atagttttag aatgattgat 52560
tttagtatgt ttataaagtt aagggttttt agtgttttgg gtttatgtta ttgcgttaga 52620
gggagagagg aagaggttgg ggtgatgggt attggggttt atgttttttt tggaatgggt 52680
ggttttggat tgtgtttgtg tgatatgttt gtcgagcggg ggcggtgttt attgaggatt 52740
ggatgttgag gtgtgggtgg ggggaggtta tgaagtattg ggtatttttc gtgtattagt 52800
gtttcgagat tgtagagagg ggattcgttt agtttgggta gggaggcggg tggtagcgag 52860
gaaggggaga ggaattcggg tgtagaaagt ttttttttta gtaggtggcg tcgtaagttt 52920
tcgagagttt agtttttgcg gggtttgtat ttttattgaa tttgtgggta gaggttgttt 52980
gggtgtagtt ttcgttaatt gtgattgttt attgcgggcg aggggaagtg gagtgtagag 53040
aatgtatcgg agggtagatg attagttttc gtataggtac ggggggtgttt tttttttcgt 53100
ataggtacgg ggggttcgag tttttggggt ttttgtttta attgggcggg gaaagggagg 53160
gttggaagtt gagttagggg gaaagggggt ggttttttgt tatttttttt attttatttt 53220
ttttattttt ggttgatagg ttttaatttt ttttatattt ttttaatttt ttgtcgtaaa 53280
atttttttaaa tatagaaaag atgaaagagg gttgagtgtg gtggtttatg attgtaattt 53340
tagtattttg ggaggttgag gtaggaggat tatttgagtt taggagttta agattagttc 53400
gggtaatata gtgagatttt atttttataa aggatataaa aaattagtta ggtatggtgg 53460
tgtaggtttg gagttttagt tattcggaag gttgaggtga gaggattgtt tgaatttggg 53520
aggttgaggt tgtagtgagt tgggattgtg ttaatgtatt ttagtttggg cgatagaata 53580
aaattttgtt ttaaaaataa aataaaataa aattataaaa aagaaattat gaaagatttt 53640
aaagtaaata tttatgtatt tattatttag attttatagt aattattttg ttgtttgttt 53700
tattataaat ttttttggtt attagtattt acgttggttt tgatgtattt aaaataagtt 53760
gtagaaatta gtatataata ttttgttaat attttagttt gtattttatt aattagagtt 53820
cggtatttgt ttgtgattat ttttgttttt ttttgaagta aaatgtatat attttgagat 53880
gtataggtgt gaaacgtttc gtttttttgaa ttttaataaa ggtatttacg tgtattttag 53940
tttgattaga atatagaata ttattattat tttagtaagt tttttatgtt tttttaggtg 54000
tattatagtt tttatttttt ttagggtaat tataattgtg tttttttttt attacgaaat 54060
aatttttgtta gtttgagtgt tttttagagt tatttatgtt gttgtaggtg ttagcgattt 54120
gttttgtttt attgttgagt aacgtttatt gtgtgaatat tttgtgggtt tttagttatt 54180
tttttgttga aagattttttg gttgttttta tttggaggtt tttatgagta aatttgttac 54240
gttggttttt tttgtataag tttttttgtg tatatgggcg tttatttatt ttgtagaaat 54300
atttagagta ggtatagggt aggtatatat ttagttttat aagaaattgt tagatttttt 54360
tttaaagtgg tttttttatt ttatattttt attagtaatg gatacgagtt ttagttgttt 54420
tatattttgg ttaatatttg gtgttgttat tttgtgaaat tttagttatt ttggtgggcg 54480
tgtagtggta ttattttatt atggttttaa tttgttttgg tttgatgatt aatgcggttg 54540
agtatatttt tatgattggt tttttatata tttttttttt gtgaaatgtt tttttaaatt 54600
tgttgtttat tttaaaattg ggttgtttga tttttttttt tggatattga ttttttgtta 54660
gatacgtgtt ttataaatat tttattttag tttgtggttt gtttgtttat tttttttttt 54720
tttttaagaa taaggtttcg ttgtattgtt taagtaggtt tgaaattttt gggtttaagt 54780
tgtttttttg tttttgtttt tttaagagtc ggttttatag gtatgagtta ttatgttcgg 54840
tttgtttatt tttttagtgg tattttttaa taagtaaaag tgtgttattt taatttttat 54900
gaagtttaat ttaataaatt tttttttttg tggtttttttt tttgtgtttt ttgtaagaaa 54960

```
tttttgttta ttttgttaaa ttgtaaagat atttatgttt ttttttagaa ggtttagagt 55020
tatatagttt ttatgaatta atttttgtat atggtgtgag gtagagggtt agggtttatt 55080
ttgttgtcgt tttatgtgtt tatttagttg ttttaaattt tattttttt taaagttttt 55140
atttgtatt tttttggtga tttatgaggt cgtataattt tttaagattt ttgttggtta 55200
tttacgtatt tttttttgta aaattatttg tttacgtttt attttttttt aaaattatgt 55260
tgttttaaga tcgagtgatt tttgaagttt ttattttata atgtttgaaa gaatgaatat 55320
tttgtttttt agttataggt ttttgttttt tgttttgtag ttgtatagtg gggtaagatt 55380
tgggttttgt tttgttgttg ttttgttgtt tttttatttg attgtttttt tatgggtttt 55440
atttaaaaaa aaaattggtc gggtatagtg gtttacgttt gtaattttag tattttggga 55500
ggtcgaggcg ggtagattac gaggttagga gattaagatt attttggtta atacggtgaa 55560
attttatttt tattaaaaat ataaaaaatt agtcgggcgt ggtggcgggc gtttgtagtt 55620
ttagataatc gggaggtcga ggtaggaaaa tggtgtgaat ttgggaggcg gagtttgtag 55680
tgagtcgaga ttgtattatt gtattttagt ttaggtgata gagtaagatt ttgttttaaa 55740
aaaaaaaaaa aaaaaaaatg ttgttttata tgttttttat atattttgga tataagtttt 55800
ttattagata tatgtattat aagtttttt ttttatattg ttttttttt tttggttttt 55860
ttaatgatgt tatttaatga gtaagagtat tttgttttta taaagtttaa tttattaatt 55920
ttttttagtt tcgatttatg attttgtga tttatggagg aaatttttgt ttatattaag 55980
gttaaaagaa gtttttttat tttttagaaa ttttatagtt gtatatttta tgattttttt 56040
tattttttaa ttttttaaat ttttatattt tttaatttat tatagtcgtt aggatgttgt 56100
atgatttatt ttaaattagt ttttgtattt ggtttgaggt tagggttaat ttttgttttt 56160
ttatatttat atttagtttt agtatcgttt gttgaaaaga tatattttat tttattgaat 56220
tatttgatat ttttgttaaa aattattgat tgtatatgtg gttttatatt agtatatgta 56280
cgtatatata tgtaggtttt gtgttgattg tatgtgtggt tttatattag tatatgtacg 56340
tatatatata ttggttttat gttgattgta tatgtggttt tgtattagta tatatatatt 56400
agtttttatat tgattgtata tgtggttttg tattagtata tgtatatata tatatgtagg 56460
ttttatgttg attgtatatg tggttttata ttagtatatg tatatttata tatgtaggtt 56520
ttgtgttgat tgtatatgtg gttttgtatt agtatatgta tatatatata tataggtttc 56580
gtgttgattg tatatgtggt tttgtattag tatatgtata tatatatatg taggttttat 56640
gttgattgta tatgtggttt tgtattagta tatgtatata tatatatata ggtttcgtgt 56700
tgattgtata tgtggttttg tattagtata tgtatatata tatatgtagg ttttatgttg 56760
attgtatatg tggttttgta ttagtatatg tatatatata tatgtaggtt ttatgttgat 56820
tgtatatgta gttttatatt agtatatgta tatatatata tgtaggtttt atgttgattg 56880
tatatgtagt tttatattag tatatgtata tatatatatg taggttttat gttgattgta 56940
tatgtggttt tgtattagta tatgtatata tatatatgta ggttttatgt tgattgtata 57000
tgtggttttg tattagtata tgtatatata tatatgtagg ttttatgttg attgtatatg 57060
tggttttgta ttagtatatg tatatatata tatgtaggtt ttatgttgat tgtatatgtg 57120
gttttgtatt agtatatgta tatatatata tgtaggtttt atgttgattg tatatatata 57180
tgtggttttg tattagtata tgtatatata tatatgtagg tttcgtgttg attgtatatg 57240
tggttttgta ttagtatatg tatatatata tatgtaggtt ttatgttgat tgtatatgtg 57300
gttttatatt agtatatgta ttgtatatat gtaggtttta tgttgattgt atatgtggtt 57360
ttatattagt atatgtattg tatatatgta ggttttgtgt tgagatatta ttgtgttttt 57420
ttgatatttt gtttattttt ttgttgatgt tatattttga ttaatgtggt tttatagtaa 57480
gtttaatat aaagtttttt aaatatttaa aatattattt tgaatatttt aggtttttttg 57540
agttttttta taaatttaag aagtagtttg ttgatgttta taaaaatgta gtgtgaattt 57600
tggttgagat tgtgttgaat ttgtagatta attaaggaag aattggtatt ttaataatat 57660
tgagtttttt gtatagatgg tatattttcg ttttaaaggt ttttttgtat ttttttttatt 57720
ttttattgaa aaaaaaattt ttatagaatt atattgtttt gtagttttta agaaaaagat 57780
tttgtatgtt atttgttaaa tttattttta agaattttat ttttggtatt attttaagtt 57840
gaaatagatt ttaaatttta tttttaatta tttgttgtta gtatgtaaaa atataataga 57900
tttttgtatg taaattttat attttatgat tttttttaaa gttatttatt attttggtaa 57960
tggttttgta ggttttttag gatttaaata tatagtttta tttttttttg attttgatgt 58020
tttgtttttt tttttgttt tattgtattg gttagagttg ttagtttagt attgaatagt 58080
agtgattagt ggatattttt gtttgtgtta aatttataat gttaatttta gatttgttat 58140
atatgatttt tgttagattg aggaaatttt tttttatttt taatttgttg aaatttttta 58200
atatgaatgg gtatgatttt agtaaatgtt ttttttgtat ttatggaaat gattattgtt 58260
ttgtttttta ttcgtttaat aggataaatt ataatgattg atttttgttt ttcgtttttt 58320
tttttttttt ttttaaagaa atagagtttt atttttgttt aggttgtagt atagtggtgt 58380
tatcgtagtt tattgtagtt tgaatttttg gatttaagta attttttat tttagttttt 58440
```

taagtagtag ggattatagg tatatgttat tatgttaagt taatttttaa tttttaaaat 58500
tttttgtata gatggggttt tattgtgttg tttaggttgg tttgtaattt ttgagtttaa 58560
gtaatatttt tatttttgtt ttttaaagtg ttgagattat aggtgtgagt tattgtgtag 58620
gtttgatttt ttaatattga attaattttg tatttttgga agaagtttaa tttgtttatg 58680
agatattatt ttttttatat attgttaaat ttgatttgtt tagtttttaa ggatttttgc 58740
gtgttgattt atgagggata ttggtgtggt atttttttttg tttgttagtt tgtttcggta 58800
atgtttttgt tagttttatt ttggttttat aaaattagtt tggaagtgtt tttttttttt 58860
tgtttttttgt gtttttataa ggttgatgtt gtttttttttt taaatgtttg atagaattta 58920
ttaagaaaat cgtttggatt tagaattttt tattaggaaa ggtgattgtt tgtttttaat 58980
tataaattta atttaatgta tagaaagagt tatttaggtt tttttatttt tttttgtttt 59040
atttttggta agttgtattt tgaaggaatt tgtttttttt aggtaagttg ttgaatttat 59100
ttatttaaag ttgttcgtaa tataagtata tttagggttt gtttttagat ttcggtaata 59160
aagtaaatat tgtaataaag tacgttatat aaatttttttg gtttttagt atatataaaa 59220
gttatgttta ggttaggtat ggtggtttat gtttgtaatt ttagtttttt gggaggttaa 59280
ggtgggcgga ttatttgagg ttaggagttt gagaatagtt tggttaatat ggtgaaattt 59340
tatttttatt agaaatataa aaattagttg ggtgtggtgg tatatgtttg tagttttagt 59400
tgtttgggag gttgaggtgg gagaattgtt cgaatttggg aggtggaggt tgcggtgagt 59460
tgagattata ttattgtatt ttagattggg tgatagggcg agatttatt ttaaaaaaaa 59520
aagaaaaaag ttatgtttat attatattat agtttattaa agtgtataag attattatgt 59580
ttttttgtta aaaaatgtta ataattattt gagtttttag taagttataa tttttttttt 59640
ggtagaaggt ttgttttaat gttgatggtt gttgattagg tggtggttgt tgaaagttga 59700
aggtagttgt ggtagtagtt tttttgtttt tttttttttt tttttttttt ttgagataga 59760
gtttgtttt attgtttagg ttggagtgta gtggtacgat tttagtttat tgtaattttt 59820
gtttttcggg tttaagtaat tttttgttt tagtttttcg agtagttggg attataggtg 59880
tttgttatta cgtttggtta atttttgtat ttttagtaga gatagggttt tattttgttg 59940
gttaggttgg ttttaaattt ttgattttag gtgatttatt tgttttggtt ttttaaagtg 60000
ttgggattat aggcgtgagt tattgtattt ggtcggtagt tttttaattg aagtttttga 60060
tatgggttga tttttttttt tatgaaagat ttttttgtgg tacgtattat gtttgatagt 60120
atttatttta taatagagtt tttttaaaa ttagagttaa ttttttttaat tttggttacg 60180
gattgattaa ataagttgat gtaatatttt aaatttttttg ttgttatttt aataatgttt 60240
atagtatttt cgtttggagt agatttatt tttaggaatt attttgtttg tttatttata 60300
agaagtaatt ttttatttgt ttaagtttga ttatgggatt gtagtaattt agtttttattt 60360
ttaggtttta tttttaattt tagttttttg ttatttttat tatatttgta gtgatttttt 60420
ttgttgaagt tttaaatcgt ttatttatga gggttggaat taattttttt taaattttttg 60480
ttaatgttga tattttaatt ttttttttgtg aattataagt gtttttaatg gtatttagaa 60540
tggggaattt tttttagaag gtttttaatt tattttgttt agatttatta gaggaattat 60600
taattatggt agatattgtt ttataaaata tattttttaa ataataagat ttgaaaatta 60660
aatttatttt ttttttatgg gttgtagaat gggtgttata ttagtaggta tgaaaataat 60720
atttattttt atatatattt tcgttagagt ttttgggtta taaggtgtat tgttaatgag 60780
taataatatt ttgaaaggat tttttttttt tgagtagtag gttttaatga tttttttttt 60840
tttttgatgg agtttcgttg tgttatttag aatggaatgc ggtggtatga tttcggttta 60900
ttgtaatttt tgtttttcgg gtttaagtga tttttgtgtt ttagtttttt gagtagttgg 60960
gattataggt atatattatt atgtttggtt agtttttgta tttttttgtag aaatagggtt 61020
ttattatgtt ggttaggttg gtttcgaatt tttgatttta ggtgatttat ttgttttagt 61080
tttttaaagt attgggatta taggtatgag ttattgtatc gggttttatt tgtttttttt 61140
ttgttgttgt tttttaattg agatagagtt tcgttttgtt gtttagattg gagtgtagta 61200
gcgttatttt agtttatcgt agttttcgtt ttttaggttt aagtgatttt tttgttttag 61260
tttttttgagt agttgggatt gtaggtgtgt attattatgt ttggttaatt tttgtatttt 61320
taatagagat gaggttttat tattatgttg gttaggttgg ttttgaattt ttgaatttag 61380
gtgatttgtt tgttttggtt ttttaaaatg ttgggattat agttgtgagt tatcgtgttt 61440
ggtttttttt tgtttttttg ttttttttta aattgagata gagtttttttt ttgttgttta 61500
ggttggagta tagtggtacg atttcggttt attgtaattt tcgtttttttg ggtttaagcg 61560
gttttttttgt tttagttttt taagtagttg gaattatagg cgtgtgatat tatgtttgaa 61620
taatttttgt atttttagta gagatgcggt tttattatgt tggttaggtt ggtttcgaat 61680
ttttgatttt aagtgatttt ttcgttttag tttttaaaag tgttgggatt ataggcgtga 61740
gttgtcgtat taggttttaa tgggttttta atatttagta aattatgttg taaatagatg 61800
tgttgttaag tagggtttgt tattttattt ttgaagtata gatagaatag atttggtaga 61860
atttttaagg gttttaggat ttttggaacg gttaatgagt attggtttta atttacggtt 61920

aatagttgcg ttagtttttt atgagagtta gtttgttttt tgaagtttta aagataggta 61980
ttgatttttt ttttttagtt atgaaaattt tagatgatat ttttttttag tagaaggtgg 62040
gatatttatg ttgaaatttt gttgtttagt gtagttattt ttattagtgt tttgagttag 62100
atttttttggg taatttgttg tcgttttttt attagtattt gttgttttat tttgtatttt 62160
tatattatag agatggtttt tttttttaaa ttttatgatt tgatttttgt tagttttaaa 62220
tttttttttt ttagtttttt tatttttttt agtttttata ttattgaaga gagttagggt 62280
tttgttttgg attaggtttt tgtttaaggg aatgttgtgg ttgatttgat tttttattta 62340
gattatttaa atttttttta tattagtatt aaggttgttt tgtttttttt ttttttatgt 62400
gtgtattgga atcgtatttt taattttttt taataatttt tttttggtat ttataatttg 62460
gttagttatt tggtataaga gattttattt ttgtttgttt tggttttcga tacgtttttt 62520
ttatttagtt taattatttt tagtttttga tttaaagtga gagacgggtg attttttttt 62580
ttatttgaat atttagaggt tattgtaggg ttattaattg gtttaatttt aatattgttg 62640
agttttaggg aatagggatg tttgagtaga gggagagaga gcgggggaata gttagttagt 62700
ggagtagtta gaatatataa cgtttattga ataagtttat tgttttattt ggatgtggtt 62760
cgttatgttt taaaaataat tgtaataatg gtattgaaga ttattgatta ttgggcgcgg 62820
tggtttatgt ttgtaatttt agtatttggg gaggtcgagg taggtggatt atttgaggtt 62880
aggagtttga gattagtttg gttaatatgg tgaaatttcg tttttattaa aaatattaaa 62940
attagttagg cgtggtggcg ggtgtttgta gttttagtta ttggggaagt tggggtagaa 63000
gagtcgtttg aatttgggag gtggaggttg tagtgagtcg agattatatt attgtatttt 63060
attgtatttt agtttgggta atagagcgag attttatttt aaaaaaaaaa aaaaagggtc 63120
gggcgtagtg gtttacgttt gtaattttag tatttgggga ggtcgaggcg ggtagattat 63180
gaggttaggg gattgagatt attttggtta atacgatgaa atttcgtttt tattaaagat 63240
acgaaaaaaa tagtcgggcg tggtggtggg tgtttgtagt tttagttatt cgggaggttg 63300
aggtaggaga atggcgtgaa ttcgggaggc ggagtttgta gtgagtcgag atcgcgtcgt 63360
tgtattttag atagagtaag attttgtttt aaaaaaaaaa aaataaagaa agaaaaaaga 63420
aaaaagagat tatagattat tatagtagat ataataataa tgaaaaagtt tgaattattt 63480
taagaattat taaattttta tatagagata tagtgttaat atgttgttgg aaaaatggtg 63540
ttagtagatt tatttgatgt agggttgtta cgaattttta atttgtaaaa agtgtagtat 63600
ttgtgaagta taatgaaatg aggtgtgttt gtgtttttttg ttattgtaat gtcggtagga 63660
ttcgtaagga aaatatttta ttttttttat tgtgatttta tgttcgttat tttttttttta 63720
ttagtagttt ggttaggagg ttattaattt agattttttt aaagaattag tattggattt 63780
tttttttttg gtgtttattt ttttgagttt ttttgttatt tttattattt ttttttttgt 63840
atttgtttta gcgtattagt tagggtttag ttaggaaata aaaattatat cggtaatttg 63900
aatagtaatt tgtaatataa agtgttttta attgtaagag gtagttaatt attaaaggga 63960
ttataaaggg gtgatattgt gatataagaa agatgtattt gggttgggtt cggtgtagtg 64020
gtttatgttt gtaattttag tatttgggga ggttgaggta ggcggatttt ttgagtttag 64080
gagtttgaga ttagtttggg taatatggtg aaattttatg tttattaaaa atataaaaaa 64140
ttattttggc gtggcggtgt gtgtttgcgg ttttagttat ttgggaggtt gaggtgagag 64200
gatagtttga gttcgggaag gtagacgttg tggtgagtcg agattgtatt attgtatttt 64260
agtttggatg atagagggag attttgtttt aaaaattaat gtatttgttt gtttgtttgt 64320
ttatttattt atttatttat ttatttattt atttatttag tttttgtttt ttaattttgt 64380
ttggtatgta gtttttaaaa tttttgaaat ttttaaagtg atgggtgttt ttttgtttgt 64440
taaggaggcg attggtggtt ggaggttttt ggatagtttg gggataaggg ttggttgtta 64500
ggggatttag ttttgtgatt agaaggttag aatttgtagt ttttttttatt tattttttgc 64560
gaggtaagag gggatgaagg ttgagttgat tattaatggt taaagatttt atgttatgtt 64620
tatgtagtga agttttttttg aaaatttaaa aggatgaggt ttgtagagtt ttaggttgtt 64680
gaatatttgt aggtgttggg agggtagtgt atttttttcgt ggaagtttta tgtcgttttt 64740
tttatatttt ttttatgtat ttttttttagt tggttgttta tttgtatttt ttgaaatatt 64800
atttgtaata agttagtaat tttaagtaaa ttgatttttt gggttttgtg agttatttaa 64860
gtaaattatt gaatttgaga agggagttgt gaagatttta aatttgtagt taagttagat 64920
agaagttatg ggtaatttgg tgttttatta tttgcgattg atttttgaag ttgagggtag 64980
ttttgtgaga tggaattttt aatttatggg atttgtatta attttggttg gtgttagaat 65040
tgaattgaat tgtaggatat ttagttgggg attgttgagt attggagaat tgtttggggg 65100
tcggcgggag gaaattatat atttggtgtt agagttgaag tattgagtgt tgtgaatgag 65160
agtggagaga tagagtttgt tttgttttat gtaagaggat tttaaggagt atagaaagag 65220
taaatatgag aagtagttat tttagaagaa aatataggag gaaagtttcg tgatattgga 65280
gttggtaatg ttttttttgga tgtgatatcg aaaatataat aaaagaaata aatgataaat 65340
tggattttat taaaatttaa aatttttgtg tattaaggaa tattattaat ggcgtgaaaa 65400

ggaaatttat agaatggaag aaagtatttg aaaatcgtat attcgataaa ggattagtat 65460
ttagaatata taaagaattt ttataattaa gttataaaat aaaaaaaaat aatttagtga 65520
atagatattt ttttatagaa gatatattaa tggttaagaa gtataggaag agatgcgtag 65580
tgttattaat tattagggaa atgaaaatta aagttataat aaaatatgat tttatatttt 65640
ttaaggtggt tgttagttaa aatatggata atagtaagtg ttggtaagga tatggagaaa 65700
ttggagtttc gtgtattgtt ggtgggaatg taaaatggtg tagttgttgt ggaaaagata 65760
taataattgt ttaaaaaatt aaatttagaa tgattataat atttagtgat tttattttta 65820
gatgtatatt ttaaagaatt gatagtaggg atttgaatag gtgtgttata tttttgtttg 65880
tagtagtatt tttttataa ttaaaagatg taggtaattt aggtgtttat tagcggatga 65940
atgaatagat aaaatgtggt ttatttgtat agtggaatat tattttgttt taaaaaggaa 66000
ggagaggtta ggtgtggtgg tttacgtttg taattttagc gttttggaag gtcgaggtgg 66060
gtagattatt tgaggttagg agtttgagat tagtttggtt agtatggcga aattttgttt 66120
ttgttaaaaa tataaaaatt agttaggtat gggggtatgt gtttgtaatt ttagttatta 66180
gggaggttga ggtaggagaa tcgtttgaat tagggaggtg gaggttgtag tgagtcgaga 66240
ttatgttatt gtattttagt ttgggcgata gagtaagatt gttttaaaaa aaaaaaaaaa 66300
aaaaaaaaaa aaggagattt tgatatatgt tatagtatag atgagtttg aagatatgtt 66360
aagtgataga agttagatat gaaaagataa atattgtatg atttatttt tatgaggttt 66420
ttagagtagt taaatttaga gagatagaaa gtgggatgga ggttgttaga ggttgggga 66480
agggagaatg aatgggaagt tggtgtttaa tggggataga attgtagttt gggaagatga 66540
gatggatggt ggtgatggtt atataatagt gtgaatgtgt tttatgttat taaattgtat 66600
atttgaaaat ggttaaaatg gtaaattttt tgtgatggat atttaaggt atgtatgtaa 66660
atatatagaa gtagttatta ttattggggt tgatggaaaa gaaattaaga attcggagcg 66720
ttttttatat tttgttacgt tttacgttta ggttttgttg gagagtgtgt gattgttata 66780
gaaatttgta gtttattggt aataaaaagg tttttgttag aaggtgtaga aagagtggat 66840
ttgtaggatt agtttattgg gtagtcgaga aatttattag agggtatggg tgggtaggag 66900
ttgggtgttt gagaagtttg ttgaggtttt attgggttag agttggggtg ttggagaaat 66960
ttagtataga gtaggtttta ttgggtatac gttattggta gttgtgtttt tagaggaaaa 67020
agagattttg gaattaggaa gagaagttgt tattttagta aggtttggta gtttttttata 67080
gtgttgtttt ttgtggatag agtttgatat gattgtattt ggttaaggag aaagatttat 67140
aaggtttcgg tttagtatta cggagcgggg tttggtagga tgtatgtaga gttaagaggt 67200
aatatatggg taattggtat atttttttag atttttttgtt tttttgttg ttttgttttg 67260
ttttgttttg ttttgttttg ttttgagaga gtattttgtt ttgttgttta ggttggagtg 67320
tagtggtatg atttaggttt attgtaattt ttatttttcg ggtttaagtt atttttttgt 67380
tttagttttt taagtagttg gagatgttta ttatgtttag ttaattttg tatttttagt 67440
agagataggg ttttattatg ttggttaggt tggttttgaa tttttgattt taggtgattt 67500
atttgttttg gttttttaaa gtgttgggat tataggcgtg agttatattg tatttggttt 67560
attttttag atttttaaag tagattttta ggtttttttt ttttttttt ttttttttt 67620
tttatttttt ttttaatata agtatttata gttttttttt aagtattagt tttagtttac 67680
gtattttagt atgttgtgtt tttattatta ttttatttga aatattgtaa aattttttt 67740
gtgatttttt ttttgattta tgggttattt agaagtatgt tgtttaattt gtaaatagtt 67800
aaggtttttt tgagtatttt agagttattg atttttaatt taattttatt ttggttagat 67860
aagtatattt tgtatgtttt taaaatttga gatttatggt ttagaatgtg tatttggtg 67920
aatgtattgt ttttatttga gaaaaaaata tgtatatata ttttagattt tttgagtata 67980
gagttttata attgttgatt aggttaattg ttagttatta gtgtttttta gattatttga 68040
tgtttgtttt ttagatgttg agagaatggt gtttgttatg gtttatttgt ttgtgttttt 68100
taaaatttat atgttggagt ttaattttg gtgtagtggt gttgggaatt ggggtattag 68160
ggaagtattg gtttatgagg gtagagttat cgtgaatggg attagttttt tttgttatgt 68220
ggggatatag taggaaaagg atatttttga agtagggagt tagtttttat tagatattga 68280
gtattttgat tttggatttt ttagttttta gaattgtgag tattataatt tgtttataaa 68340
ttatttagtt taaggtattt tgttttaata ttttgaatga attgaggtgg tgttaaaatt 68400
tttaattatg tatttaggcg tagtggttta tatttgtaat tttagtattt taggaggtta 68460
aggcgggtgg attatttgag gttaggagtt tgagattagt ttggataata tggtgaaatt 68520
ttgtttttat taaaaatata aaaattagtt gggcgtggtg gtaggttttt gtaattttag 68580
ttattcggga ggttgaggta ggagaatcgt ttgaatttag gaggtggagg ttgtagtgag 68640
tcgagattgt attattgtat tttagtttgg gtaataagag tgaaatttcg ttttaaaaaa 68700
aaaaaaaaag agtttttatt tatgattgtg gaattgttta ttttttttt taattttttt 68760
aggttttata ttatgtattt tgaagttttt ttggttaggt gtatatatat ttatgattat 68820
aatggtgttt cgatgaattg attatttaat tatgatgaat gtattttttt atttcgtaat 68880

aattttattt tggatttaat tttattgata tttatgtagt ttttttagtt tttttaaata 68940
tgttgtttgg gatgtataag aaggttggag aggtttatgg agaaatttat gatggtttga 69000
tttatagttt tttaatttta ttatggtgcg aacgtggtat gtatatagta gaaattgtat 69060
tttaagtatg tgtgaaatta ttgtgttttt tatttttagt atagtattta ataatttata 69120
tgagatagtt aatattttat tataaaatag gttttgtgtt tgttgatttt gtttaattgt 69180
aggttgaagt aagtgtttta attatgttaa ggtaggttag gttaagttag aatgttcggt 69240
aagttagatg tattaagtgt atttttgaga tgatatttat tttttattta tgatgggttt 69300
gttaagatat aattttatta ttatttaagt agtattagta tgttttttt taaatagaaa 69360
atttaattt gtgtttatat tgaaatagta tttttgtag atagtatata gttgagtttt 69420
gttattttat ttattttgat agttttcgtt ttttaattgg aatatttagg ttatttttgt 69480
ttaatatagt tatagatatg attgggttta gagttattat tttgtttttt gatatttatt 69540
tttttattt tgttttttg gttttttta tgttaattaa gtatttttta gaattttatt 69600
ttaatttatg ttttttttt ttttttttt ttaagataga gttttatttt gttgtttagg 69660
ttggagtgta atagtatgat tttggtttat tgtaattttt gtttttggg tttaagtgat 69720
tttttgttt tagttttttt agtatattgg gattatagac gtttgttatt acggtcggtt 69780
aatttttgta tttttagtaa agacggggtt ttattatgtt ggttaggttg gtttcgaatt 69840
tttgatttta ggcgattcgt ttattttggt tttttaaagt gttgggatta taagcgtgag 69900
ttattgagtt cggtttgttt tgattttta attgtatttt tttgtattgt tattttagta 69960
gttattttat agatttatt gtatattttt agttttttat agtttattta gaattaatat 70020
tgtttttttt tatggaaaaa aagtgtaaga attaggtaat tttatgggtt tatttattat 70080
tttttattt gtatgtgatg gttgttagat atttattata tttatatata ttgtaaatta 70140
gataatataa taaggaaatt tttgttttaa atagttttat gtattttaaa gaaattaaaa 70200
gtaaaataga attttttta tttgtttatg tatttgttat attttgtgtt ttttattttt 70260
ttttgaagag ttgagttttt atttggtttt atttttatg attttggaga atttgtttta 70320
cggtttttgt agtgtaggtt tgtagatagt taattttttt ttattttat ttatttgtaa 70380
atttttattt tgttgttatt ttttttttt ttatttttat tttattttt gagatagagt 70440
tttattttgt tttttatag gttatagtgt aatggtgtga ttttaaaatt tttgtttaat 70500
tttaatttat taattttttt ttttcgggtt taaacgattt ttttgtttta gtttttttaag 70560
tagttgggat tataggtatg tgttattata tttagttaat ttttgtattt ttagtagaga 70620
tgggatttta ttatgatggt taggttggtt tcgaattttt gattttaagt gatttgtttg 70680
ttttggtttt ttaaaatgtt gggattatag gtgtgagtta ttgtgtttag tttgttgtta 70740
tttttgaatt ataattttat tggttataga attttgagtt gttagttttt tttttttagga 70800
ttttaaaata tagggtttta gtgtttttgg ttttcgttgt ttttgttaag gaattagtat 70860
ttatttagtt atttttttgt agattatatg ttgtttttt ttggatgttt ttaagatttt 70920
ttatttaagt ttgattgttt agtatttatt tttgggtaaa aaagttttta gaaattagaa 70980
atttatttag ttttattttt ttttgttagg tgtagatttt attttagttt ttatttattt 71040
gagatatttt atattgattt taggttgttg ttattttgtt tttttaaata ttttgtttag 71100
agttataatg ttttttgtag aattgattag atagtagtta tttttttgtga ttaggagttt 71160
tgattttta gatttgttga tgggtttata aagtttttgt tcgtggtttt tagttggtag 71220
tggtttttt gttttgaagt tgttatgagt gtgtaaaatt tataggttta atttttttta 71280
ttagtatagt ttagaaaagt ggaattttg gtttgggtgg ttatggtgtt ttagattatt 71340
ggttagatta gggtggtgat tgttagttgg tcgaatgtgg ggtttttagt ttagtatttt 71400
tggattttat tttaggagtt atggttgttt tagggttagg ggtttggttt tagtttttgt 71460
ttttttttt tttttttaa gttgtttagt attaggtttt aattagtatt ttttgtagat 71520
agggtgggtc ggttttttat tagaggggta gggttgggcg ggaagtttgt tgagtttttt 71580
tttttgtgta gtttatagtt ttggggttag ttttagaggg gtgtagaaag gagtttttaa 71640
aggtagaaat ttattttttt ttttgttttt gtgtttttag ggataaagga gaggtggttt 71700
tagttggggg gttattagtg gtatgtagga ggtttatatg attattgttt gttggggttt 71760
ttttttttt gtgagagaga cgagagagag gagagagagt gtgagttagg gtagtgtttt 71820
tttttagtaa cgtagatttt tgtacgtgtt ttttaggaga aatttcgtta ttcgttagag 71880
ttttagattt ggaagaagtt ttgtttttt ttttttttt agtgtagtgt gtgtgtgtgt 71940
gtgtgtgtgt gtgtttttat atgtattata ttttttttata gaggaaataa ttttcgttta 72000
agtgtaaagg ttgggattgg agtaggtagt atgtgggtta aaggagtaga gaggaggaaa 72060
cgggggcggg ggtgagggtg ggaataggag gtgggtagat gttacgaggt atgtttttta 72120
taattagaag ggttggtgga gatgattaga atgttttaga tagatttttt attaaatttt 72180
taaaatttaa ttttgtttta tgaagtttga ggattttgta gagtgaaaga taaggaaggg 72240
tttttttgta ggggggtgtg tgtgtttgtg tgtgtttgtg tgttgtgtgt ttttatgtgt 72300
gtgtacgtgt ggatttgtgt ggtgtgtgtt tgtatatgtg tgtacgtgtg gatttgtgtg 72360

gtgtgtgttg agtgtgtata tgtgtgtgtt tgtgtgttgt gtatgtattt gtatatgtgt 72420
atatagattt tatttttttt ttttttggtt tattaagtgt tagaaagaat tttcgtgagt 72480
ttttttattt tttttaaggt tgtatatgga gggatttgcg taggtatatg tgcgcgtgta 72540
tataatatat atatatatat atatatatat atatatatat atatacgagt taggggcgag 72600
tatttttgtg gattgtattt aggtttggta ggaaatcggg ttttgggaaa ttgtaggttt 72660
tatttttggt agaaaattat gtttttgttg ttgttcgttg aagggaatat taagtggggt 72720
tggggatagt ttatgttgag aagagtaaat tttgtttatg gtattgggga tattgagtgg 72780
tttttgtttc gggttttgga gggtttggga gatacgtttg tagttttggg tattttttaa 72840
ggttacgggc ggttatattt aaggttaggt ttttttttgt gtgagttgcg gttagtagga 72900
aggttgattt tagaggcggt tttagggttt ttggaggttt tgtttttgag atatgggtta 72960
aggttagaga gtcgagaagg ttgttgttga ggattttggt tggcgtttag tttagcggtt 73020
ttttaggcgg gttttgtgta ggggttgatg aagttagttg ggaaatttgt gaaataaagt 73080
ataggttttt taggtttgtt tagagtttcg gtattagatg aggtgtagag aagggaattg 73140
agttgttaag tgtgaatagg agagaggagt tttaggatta tataggggtt tcgatatggt 73200
aggtgtttgg ttagtagcgg tgttgttttt attgttggac gtgggaaaat gtattgatat 73260
ttgttgatta gagtcggggt gagtatttgg gtgattttgt agatgagata aaataagggt 73320
tgtcgtgggg gaggggtttt tgtcgggtta ttttgttgag gttttgtgtt attgttttgc 73380
ggttgttata ataaaggatt ataaatgagg tgatttaaaa tattcgaaat cggttacgtg 73440
cggtggttta tgtttgtaat tttagtattt tgggaggtcg aggcggttgg attacgaggt 73500
taggagatcg agattatttt ggttaatatg gtgaaatttc gtttttatta aaaatataaa 73560
aaaaaaaaaa aaattagtcg ggcgtggtgg tgggtgtttg tagttttagt tgtttaggag 73620
gttgaggtag gagaatggta tgaattttgg aggtggaggt tgtagtgagt cgagatcgcg 73680
ttattgtatt ttagtttggg tgatagagta agatttcgtt ttaaaaataa aaataaaaat 73740
aaaaataaaa atattcgaaa tgtttttttt aaagtttttg gagttagaaa ttttagatcg 73800
aggtgtttga aggtttattt tttttggggg tttcgaggga gattgtttta tgtttttttt 73860
cgagttttgc ggcggttagt aatttttggt atttttcggt ttgtagttcg gtttattttt 73920
tatttggttt ttattttgtg tttttttttg tgtttttgtt taaattgttt tttttgttgt 73980
ttttttaatt tatttttagg gataggtttt tattttgttt tttaggttgg agtgtagtgg 74040
cgtgattatg gtttattgta gttttagttt tttaggttta agtaattttt ttattttagt 74100
tttttaagta gttgggatat aggcgtacgt tattatgttt ggttaatttt ttaatttttt 74160
tgtagagacg agggtttagg ttggttttaa atttttgcgt ttaggtaatg agattttgtt 74220
ttttaaaata ttgggattag agatatgagt tgttgtgttt agtttatttt tgttttataa 74280
agatatttgt tattggattt agggtttatt ttgatttaat atgtttttaa ataatttaat 74340
tatattttta aagattttat gtttatataa agttatattt ataggtattg gggggttagg 74400
atttgtatat gtttttttggg ggaatataat ttaatttata atgggtttta tttatagaac 74460
gaacggtttt tttagggtgt gcgtaggtgg gtgaggggtt taggagaggg atttgcgtgg 74520
taagagggtt tgtgtttacg ttagagattt tagaaggaga ttggatgggg tggttgtatg 74580
gagtaagggg ttatagttat tgtttttaag tagttggtgg gttgtttggt agaaagggag 74640
tatttatttt gagttttttt aagatatgaa gttagtatta ggtatttgga aggtgttagc 74700
gagtagagtt tgattttta tagaaagagg tgtttttttt ataatgttta tttgaggacg 74760
gtggaatagg ttattttata aggtagtgag tttttttatgt ttggaagtat ttaagtaggg 74820
gttatgtggg agttgtttag gagtgtatag aagggatttt agaggttttt gttaatatta 74880
tttttgtttg ttttatttta cgataatttg aattttagaa tgtaatgtta ttggggatat 74940
gtttattaga ggggttttta gtattaggtt ttatatatta tgtattattt ttagggtagg 75000
gaatgagagt aggttttatt ttttttggtt gtttgtgagg attaggaggt ttgttgttga 75060
ggtagttttt tgtggaaata gtgttttagt ttcgtaattt atttatcgga gttttaatat 75120
tttaatgaaa tattgtgtta gatgggtttt gggtatgtgt gtgtagattg ttataagttt 75180
atgagaatgt atatacgtat attgtatgtg tgtgtatata tgtatgtata tatgtataag 75240
aatacgaaga ttcgaggtag gtatagttcg tgagatagat ttttttaggt tgaaagattt 75300
tttataaggg tttttagaat aaggttttag tttcggtagt ttttttttatt tttggtggga 75360
ggatttttat agagaagaag ggttaagaag tatagttttt gatgtttacg gtgtattaag 75420
ttttgtttta agtttgtgtt ttatttttta tttatttagt aaatttgtcg tttttgtttt 75480
gtgttagatg ttgtttttag tattttataa atattgatgt atgtttgttt tatgataatt 75540
ttgtgaagta ggtagtgtta tttcgtttta tagatgagga aataggcgta gagggataaa 75600
gggatttgtt taaggtttgt ttttatttta tacgtatatg aacgtttatt tatgcggtag 75660
gcgttagttt tttttgatg tgagttaggt tttgagttgg ttggggtttg ggttttattt 75720
tggttgtgtt ttgttgtggg tattaaggaa gtttttagta gttttatttt tttggtatga 75780
tttagtttta gggattggat ttttttaaat gtttttgttt agtttggagt tttttttta 75840

gttgggggag ggttgaacga ttttttattt ttggaggttg tcggagtttt ttttgtttgt 75900
atgcggtcgt tgtcgggtgg ataggtttgt ttgtgggttg aggtttttag tagttggggt 75960
ggattatttt tttttttaag aagttttggg agaagtagtt gagggggtta gcgttttttt 76020
gaggttaatt ttttgttttt tttagcgtag gcgcgaagtt atttttgttt gtgtgtttgt 76080
tatagatagt ggagggcggt gtaatcgtga cgtttgtttg ttgtatttg cgtttttgag 76140
gacgttgatt tttggtcggt gttgggcgtt tgttttttag ttattttgtt ttgatttggc 76200
gtggtaggtt cgagtattgt ttttttataa ggaaataggt ttagaggttt tgagttattt 76260
gttcgggttt ttttagttaa taattggttg agttaggatt tttatttagt tttgtgattt 76320
tagaagttta agtttttttt attacgttag ggttaggaaa ggattagaat gttttttttac 76380
gtgtcgatta tttggatgat ttgtgtagtt tagttcgttg tagttgttta ataaaaatgt 76440
ggaatgaatg ggtttatatg ttttattgtg tgatttagag ttaatgattt tggttgattt 76500
ttttgttttt atttcgtggt ttagtatttt tttttggggt gtgtgatttt ggaagtagtg 76560
atttttttta ttttattttg aatggtgttt gcgtcgtagt aggggagtag ttggggattg 76620
ttagcgtttg ttgttggtgg gttgtgcgtt agatttcgtt ttggatgtgt gggtgattgt 76680
tagttatatg tgtgtgaagg gttgtgcgtg taggatttgt gtaaatgtgt ttttttttgt 76740
tgttagttta tgggaggttg ttaagatttt gtttttgtat attagtttat attttttttg 76800
tggtttattg ttgtggttgt ttttttgtgt tacgtggttg taggtatagt ttttaagtgg 76860
atggggatcg tgggttgaat gtgtattaga tgtttttgtt ttttggtggg tggagatttt 76920
taatttatgt atcgtggttg tcgtatttga aaggtttttt atggtgaagg atttggattt 76980
gagtaatttt tcgtagaggt tttagaagtg ttttttggtc gggcgcggtg gtttatattt 77040
gtaattttag tattttggga ggtcgaggtg ggcggattat aaggttagga gatcgagatt 77100
attttggtta atacggtgaa atttcgtttt tattaaaaat ataaaaaatt agtcgggcgt 77160
ggtggcgggt gtttgtagtt ttagttattc gggaggttga ggtaggagaa tggcgtgaat 77220
ttaggaggcg gagtttgtag tgagtcgaga tcgcgttatt gtattttagt ttgggcgata 77280
gagcgagatt tcgttttaaa aaaaaaaaaa aaatgaagtg tttttagttt ttttttttgt 77340
ggtgaggttt tgagttttta tttttagaag aaaatttggt tggggagggt atttttagat 77400
tcggatataa acgttaggag attagggttg tttttttagg tgattaatta tagagattta 77460
gggaaagtta cgtttggaag aggcgatatt gtgttttagt tttatttatt tttttatttt 77520
atagatattg tttggtgttt agttcgtatc gcgaggtttt ttaagtagtt ttgggtatcg 77580
tgtatttggt atggttttta gtttttatttt tttgttttgg gagattcggt ggggatttcg 77640
ggttgagttt ttgtggttgt ggaattttgg tggtttattg tggtggttga gaatagggac 77700
gtggggttgg acgggttgag tgtaattttt agtggtttcg tgaattgttt gggagttttg 77760
ggtagttttt ttttattttg tgttttcgtt ttttgatgga aaaatggggg ttagggggga 77820
gcgatttga aggtattcgt gggaattaag cgaggtgata aatgtagtgt ttttggtaga 77880
gaattggata tagagtttat ttttatgaag ggggttgttt ttttttttt tatgtttttt 77940
tttgttttta ttttttttt tgtatttttt tttttgttt ttttttttt ttttttttt 78000
ttttatatgt aggtatattt tatttggtgt tttaattgtt ttttttattt gtttttttgt 78060
aagtggtttt ttaagtggga atttaggttt ggaaagggtg ggtattttgt ttggtgggtt 78120
ttggtaggtt aggcgttgag tagttttagg agatataatt ttaggaaaag gtttatgtag 78180
gttggttgtt atgacggatt gttggtggga gaggggaagg cgtcgtggta ggggaagttt 78240
tggaaatatt tattagtggt tttatttatg atttaggata tttgttaggt tattagaata 78300
tagttgagga agttgtcggt tagacgttta gaagtattaa tttggtggtt gggttgtggg 78360
gggatttttt tggataggta gataaggtgt cggaggaagg ggttgatatt tatttagtgt 78420
ttgttatgta ttgagggtat aaagggtagg atggagagtt ggtttacgtt atttgtggaa 78480
tttttaggt gtgggggtag atggggaagt aagggggttt gaagatatat gcgtttatta 78540
aatgttttta aattttaaga agaagaataa ggatatattt ataggggtag ggttgaaagt 78600
ttgatgtatg gtgttttggg gtttgttttt gttgtaatag ttatagtggt tttttttat 78660
ttttttttt tttttatttt gttgtgaaaa tgtttaaaat atatggttaa gttgaaagta 78720
ttttataggg aatatattta gttattattt agattttatt attaatatta agttatattt 78780
gtttattat tttatcgttt atttattttt ttttcgtttg gttatgaatt tattttattt 78840
ttattttttg gtgtatttta aagtaatttt aggttatttt gattattttt ggaattattg 78900
ttagaaattt ttttttttt ttttttttt ttttttttt tttttttga gatgggattt 78960
tgttaggtta tttaggttga agtgcggtgg tgtagttata gtatattgta gtttttaatt 79020
ttcgagttta agtaattttt ttgttttagt tttttgagta gttgggatcg taggtattta 79080
ttattatatt tagttttatt tttaaaattt tttattaatt ttttattttt tgtatttatt 79140
taggattatt ttttataaag ttagggtgtt gtaggagggg ttagaatttt aagttttaat 79200
tttggttttg ttattaatat tgtgcgattt ttagtaaatt attttgtttg tttaggtttt 79260
gtttttattt ttatgaaacg agggcgttaa attgtatgtt ataaataatg agggttatta 79320

gttattaagt ttttatttta ggttaggtat tttttatatg ttattatgag ttttttttaat 79380
ttaggtattg tttttaatgg atagtttagt taacggaagt ttagagaggt ggtgtaattt 79440
gttaaaagtt ttattattta gtgaatgttt ttacggggtt tgtatttagg agtttgatat 79500
agagtttagg ttttagtatt tggcgatgtt ttgggggtgt gagtagttta gtttattttg 79560
ggtacgtgtt tatttgttgt ttttttgtt ttatgtttgt gtttgttttt tttgaagttt 79620
agattgttat tttttagttt taaatattaa aattggttag gtacggtggt ttatgtttgt 79680
aattttagta ttttgggaga ttaaggcggg tggattattt gaagttagga gtttaagatt 79740
agtttggtta atatggtaaa attttatttt tataaaaaat ataaaaatta gttagatatg 79800
gtggcgtgta tttgtagttt tagttattta aggagaattt agttatttag ttattgaggt 79860
acgagaatta tttaaatttg ggaggtggag gttgtagtga gtcgagatcg tattattttt 79920
ttttaggtta ggtaataagt gtgagatttt gttttaaaaa taataaataa ataaaataaa 79980
ataaaaatta aaattaaatg ttaaaattaa tttataaaat aaattttggt taaagtagtt 80040
tgtttatttt gttttaatta tgtatattgt attagatttt gttaggggtt tagcgtataa 80100
agatgagatt tttgttttgt tagataggtg ggatgatggt tatattttga gttagttaag 80160
gtattatttg gttgtgtaat aaaagttaaa atattagagg ttgagtagta ttcggattta 80220
cgttttagtt taagtgttag tcgttatggg ggtttaggat agttcggatt tagtttattt 80280
ttttggtgta ttttcgatat atagttttta ttttttgagt tgagatggtt ttagtttttg 80340
tcgttatgtt tgtattttag ttagagggaa gggaagaagg gagaggggaa agggtaagtg 80400
tttttttta tttttaaggg tatgatttga aagttaaata ttttagtttt tttttattgg 80460
ttagaattta gttgtatggt cgtttttgtt attagggagg ttgagtaatg tattttttag 80520
ttgggttttt ttgtgtataa ttaaaatttt agttattata gaaaaaaggg agtttagata 80580
ttggggaaat aattagtatt tgtattatat gtattgatag tttttttgt atgaatagag 80640
ttgtggtagg ttttatgtta gggagaaagt aagattggaa aagagtttat taaggaggtg 80700
gtatttgtat tgtgtttaag gggtaagaaa aacgtttttt aattaggagt tataaatgtt 80760
tggttgaagt gttattgttt ttttattttg gagttggaat agacgttatt agttaattat 80820
gatggttgtt gggtgtattg gttaatattt ataattttag tattttgtga ggttgagggt 80880
gggaagattg tttggggtta ggagtttgag attagtttgg gtaaattgta agattttgtt 80940
tttgtaaaaa aatataaaat gtagttgagt gtggtggtat ttgtagattt agttttagtt 81000
attcgagagg ttgagatggg aggatcgttt gggtttagga gttcgaggtt gtagtgagtt 81060
atgattgtat tattgtattt tagtttgggt gatagagtag gatttgtttt taaaaataat 81120
aaaataaaat taaagtttta aaatggaaaa aaaattatga gtatataatt ttagatgtat 81180
ttttaagata gtaaattcgg gtgggcgtag tggtttatgt ttgtaatttt ggtattttgg 81240
gaggttaagg tgggtggatt atttgaggtt aggagtttga gattagtttg gttaatagtt 81300
ttatttgtat tataaatata aaattagttg ggtatggtgg tgggtatttg tgatttttagt 81360
tatttggaag gttgaggtag gagaattgtt tgaatttagg aggcggaggt tgtagtgagt 81420
taagattata ttattgtatt ttagtttgga gaaaaaaagt aaaattttgt tttaaaaaaa 81480
aaaaaaaaaa gtaaatttag ggttaggttt gtagttatat ggttattttt tttttgagt 81540
atggaggtag ttttagaaat ttgttttata ttatattagt gggtagttga ttgggttagt 81600
ttgttagttg aattttattt gttatagtta ttttttatag taaggggtat tttagatata 81660
ttttttttt tagggaagaa taagtcgtag ttgtttgagc gttttattta gtggtgtgta 81720
tgtttttttt taattttgtg tttagtgatg ttttgtttgt agtttgaaat ttgttatagt 81780
gggtgtgttt atattatagg aattggtaaa tattatatat taggttttat tttttgtttt 81840
ttattagggt aggttgtttt tttttgttg tttttgtttt ttgttttttt tttttttga 81900
gacggagttt cgttttgtcg tttaggttgg cgtggagtga tgcgattttc gtttattgta 81960
agtttcgttt ttttggttta tattattttt ttgtttgagt ttttagaata gttgggatta 82020
taggcgttcg ttattatatt cggttaattt tttgtatttt tagtagagac ggggttttat 82080
cgtgttagtt aggatggttt cgattttttg atttcgtaat ttgttttttt cggtttttta 82140
aaatgttggt attatgttat tgcgttcggt ttagggtagg ttgttagaaa tgtattagtt 82200
tgttagtttt agtttgttt gttttttatt ttttttttt ttttttttt ttttgagacg 82260
gggttttttt ttgttgtttt ttaggttgga gtgtagtggt gtgattttgg tttattgtaa 82320
tttttgtttt ttgggattaa gtgatttttt tgttttagtt ttttaagtag ttgggattat 82380
aggtgtttat tattacgttt agttaaattt tgtattttta gtagagatgg ggttttatta 82440
tattggttag gttggtatcg aatttttttat ttaaggtgat ttattcgttt tggtttttta 82500
aagtgttggg attataggcg tgagttatcg tattgggttt ttttcgtttt tttttaatga 82560
aaattttttt gaaagatttg tttattgatg tttgtgttaa taggtattgt ttgcgggata 82620
gagtttgttt ttttttgaa ggtttttttt tgtttgggtg attatttgta tgttattttt 82680
tgttgtttag aattaaagat gtagttatgt ttggggtagg ttgggaaaat ttgtgggaat 82740
tatagagttt gttgttatgt tgtgttttgg atatttgaag tttggtaggt aggtgggatc 82800

```
gagggtgggt attaggaaag tgatgtggtt ttataggaag gggatatagg ttttgagatt   82860
ttaggttttt aattggataa agttgttttt tttttttgga taaatgtttt agtttattta   82920
taatcgagtg gttagttttt tttttttttt tttttttttt tttttgaga tggagtttcg   82980
ttttttcgtt taggttggag tgtagtggcg ttatttcggt ttattgtaag tttcgttttt   83040
taggtttacg ttattttttc gttttagttt tcggagtagt tgggattata ggcgtttgtt   83100
atcgcgttcg gttaattttt tgtattttta gtagagatag ggttttatcg tgttagttag   83160
gatggtttcg attttttgat ttcgtgattc gttcgtttcg gtttttttaaa gtgttgggat   83220
tataggcgtg agttatcgcg ttcggttaag tggttagttt ttttagttgt cgggttttgt   83280
atattttgtt ggttttggag attatatgta ttatgtagat tttatttgtt ttttattttt   83340
agtgttgggg gaggggttaa gggtttgtcg atgtgttgat atttggttat ttattggttt   83400
tgaatgttta tatttatttt tttgaaatcg atgtttcgta tcggaggttt tatgtggatt   83460
tgggggtaga ggtttgtatt ttagatggtg gggtggtttt tattaattag gtagattttt   83520
tgttttgttt tgttttttgg gttttgtagt tttagatgtg tgtttaagat ttgtggttat   83580
atggatggga taagttatag agatttagat ttcgtggttt ttgtgattag agtagtcgtg   83640
gttttgtttg ggaatgtttt tattttgttg ttgaaggtga gaaatttttt tgtgttggag   83700
aatagttgtg ttgagaagtt ggtttatttg gtttgtagga ttgttggggt aagggaggtt   83760
tgttgtcggt tttttttttt tttttttgat tgggtgtttt ttgtttaggt tttttgtagt   83820
aaaagaattt tttaatttat tttgtttttt tattgagttt ttattttatg aagtttattt   83880
tttaatttta gttgattggt ttttattttt ttaagaggaa ggttaggggt agacgaggtt   83940
gaaggaggtt gatttttttg tgtttcgta gtttgagttg atgttgttgt atgtttatag   84000
ggttttttta gtttttaaaa aggttttaat acgttcgtta tacgtttgtg gttttttttgg   84060
atgttatatt aatttagtaa ggtttgagtt agaggttgtt tttgaggttg tgaaaataga   84120
agaagggttt agaggagaat tttttattt gacgatttta ggaaggtttt ttagaagaag   84180
tagtttttga gttgagattt gaagatagat taggtttgag ttaggttagg gagggtgtga   84240
ggggtttatt aggtagaggg aataggatag ggtaggtata gaggtttggt atttggtttg   84300
tggggaagtg tgagttttttg ggtatgatta aggcggggag aagtggcgtg agaggaggta   84360
gatgtggttg gtaggagatc ggcggtttcg gttttgttga gttttatttt gtatgtatta   84420
ggtgatagag gttgtttagg acggtgggta gggaagtgat aggttatatg gttattttat   84480
taatagtcgg gagtttagtt tggggggatt taagattttg gggaaggtac gagttagggg   84540
tttgttagag ttaggagtta gtgggagaag gaaatagtgt tgtttatgaa ttataaatag   84600
atattttcgt aattttttttt gacgtgatat aattttaaag tgggttattt agaggtggga   84660
aggtgatttt tggtttttta ggttttgggt ttgttttttt tttttttttt tttttttttt   84720
tgagatggag ttttattttg ttgtttaggt tggagtgtag tggtgtaatt ttggtttatt   84780
attattttttg ttttttgcgt ttaagtaatt tttttgtttt agtttttttga gtagttggga   84840
ttataggtat ttgttattat attcggttaa tttttgtatt tttagtagag atggtgtttt   84900
attatgttgg ttaggttggt ttcgaatttt tgattttagg tgattcgttt atttttggttt   84960
tttaaagtgt tgggattata ggtatgagtt attgtgttta gtcgagttta tttttaagtt   85020
tttttttggtt tttagtgttt ttgtattagt ttattttttt ggtttttttgt ttttaagagt   85080
ttttttttaag attgttttta agtggatagg tttttagaat taaggagtag ttttaggagt   85140
ttttagttat agatatttta gggggttcgt gtggttttta gtttttggtt tggtttcggt   85200
agttttggtt ttttgtgttg tttttttatat agtttggtta gagttggtta ggtttttttgg   85260
ttgggtcgag ttatcgtggt tagtgattta tgtaggggga gaaaagtgag gagggggaat   85320
agagtgcgag gggggattgtg ttaataataa atttattagg tttttgaggt tattttatag   85380
gagggataaa tggtttattt aggttgattt ggttgtcggt tggtttttag agatatttag   85440
ggtggggaag atttttattat ttggggaaag taatattaga ttgtttggag gggtttagtt   85500
agcgtatttc gcgtttttttg tttgtttttt acgtagagcg ttttgttttt aaggcggtag   85560
gttaagagcg cgagtcgagg tttaggtgta aagaggggtg cggtatgggg gtattgtagg   85620
gtacggggtg tatgtatttt tatttatttt tttattgagt gttagggtaa tttgttaatg   85680
gggtatattt ggtgatataa aatttttttgt cgatttatgg tagtttggaa cggagtggaa   85740
gcgttttatt tttggggaat ttttatatat atttggttgt gaacgtcggt gtttttgtat   85800
tggagttttt ttggagtgtg ttgggtagag cggagaggtt tttttttttt tttttttatt   85860
atttgtgga ggtgttaata tgtagttaga gatggttagt ggtagtgagg agagggggggt   85920
aggaagagat tagggttggg ggtttgttag tttatagttt tatttttgaa tatttatggt   85980
ttttttgttt aggttagcga ggttgggggt agagacgggt aggggttttt taatgttggg   86040
tattttttcg tttttttata ttttggattt gttttttgga gaaagttttg gagtatttag   86100
aagttttggg gttgttggtt gaggtgggga gtagagttta gttgtgtttg gaggtttttt   86160
atttttattt agtttttagg gatagtttta gttgtgtagg tttaggtcgt tgaggttggt   86220
tttagaaatt tttttataat tattattgga tggaaaatta gtttttttaaa gggttttaga   86280
```

gttagttttg tttttttttt tataggtttt tatagttaga gtatcgggtt tttgttagag 86340
atgtagaaga gacgatgttt ttagttttta tgtggttcgg tttttattgt ggatgttggt 86400
gattttagtt tttttttaa aattaagagg gttgatgttt tatattttag gtttttagaa 86460
agagtcgtta tttatttata tcgttatatt ttttttttat tttttgttt cggaaagagt 86520
agagtggata tgcgttttttg tagaaattta tttatagttt gattgtagat gtattttgtt 86580
ttttattga gtttttgttt tatgaagttt gtttgcttg tattttttt tttttttttt 86640
tttttttttt tttttttttt tttttttttc gttttttttt tttttttttt tttttgttt 86700
tatttttttt tttttttttt tttttttttt tttttttttt tttttttttt gtttttttta 86760
tttttttttt tttttttttt tttttttttt gaatagttaa agatagaatg aatggtatag 86820
gtaggtattg agtttttgt aattggaggt gtttaagtgt agggtggatg ggtatttata 86880
gggtaggtat tgaggaagga tttttttggg attgtggatg gtaggatagg ttttgtggag 86940
tttttgagc gggtgtagaa gagggagggt atgttttgt tttttgtttt tttcgggttg 87000
ttaggtcgtt atttttaatt tttattttt tttttttat ttgttgggtt ttacgggatg 87060
ggtagaggta ttgttagaat atggagtcgc ggtttataga gggttagtag ggaggagagt 87120
tttttgggga ttacgtcgtt ttagttattg tggtgatttt gaaaataaat gttttttttt 87180
ttgtttgagt ttttgttgtt tttgttgacg tggaattagg gtaggttggt gatttagttt 87240
ttatttttgt attgtatcgg tggtaagttg tttttagttt tagattttat agagtagggt 87300
gtttttttta gaaaaatagt agggaggtta ggatattaag ggttttaggg atagtttgtt 87360
tttttggtt ggtatgagtt ttagttaagt tttttggtat tttgggggtg aggaggtatt 87420
agttgtgtgt tttagttatt ggttgttttg aggttgggtc ggtgttaggt tggttatttt 87480
ttttgtttt ttagtgtgat tttgtagagt tttcggaagg gttagagggt tttttttat 87540
tattggtgtt tatgggatat tgtgttttta ttttattttt gtattttagt tttagacggg 87600
tgttaggaag ttggagattt tagttatttt acgattgtgt tttgcggggt tggagtagga 87660
ataggagtag gaggaacgtt ttggttatgg gattgtgtga agagagaagg attaggaaga 87720
aaatatgagt tattatgttt gggttatatt ttagtagagt cgttggtggt tttttaggta 87780
tttgaattta ttaattataa agtttattag gtgtgtaaat aaataaatgt tgtttaggag 87840
gtatagttag ggttttgttg gagagggatt atttggaggg ggttatattt tttatttagt 87900
tttttagttt ttttgttttg tgtgtgtgta gtttttgatt attattattt ttgtttttgt 87960
tatttgtttt tagggtttta ttaggttgga tgttattgta ataaaatgta gtaatagaag 88020
gtttaggtag tgttgtagtt tttggtggta ttattagagt tagtaggtag tagggttggg 88080
gaattttaag ttgatttagt tttaagtgtc ggagcgggga gttcggatga ggtgggggtg 88140
gggaatcggg gagttatgga gtagagtatt tgttgtgttg aaggttagga gtttttttgg 88200
gagattatga atttagaata ttaatatttt gtttaaaaag tttttgttt tggagataag 88260
agtttaggtc gttgtggtta ggttttgttg taaatgtttt gtataaagta ggttgggggg 88320
attattggga agtttgggga atagattggt tatgtgtttt tttgagtgaa attttatggt 88380
tgtattgaag cggttaggta gagggtgtga tggggtgtgg tagttgtata tcggtttagt 88440
taggttgaat ggggaaatgt aaatttaggt ggtttgaaag agatttagtg gtttatagtt 88500
tggtggttgt ttttagtata ggtgatgtaa agagagaagg ggtttcgggg gtttggttta 88560
ggtggaaatt atgtattgtt tgtgattttg ggtatgtcga ggttggagta ggtttgtttg 88620
gtggttggta gaacgtatat gaagaattta tgtgtgtttt gaacgttttt tttgtttttt 88680
tgggtttatg ttttattagg agtttttcga ttttgttatt ttgtaaagga aattgcgttt 88740
tgttattgta taggatcgaa taggtgtgtg tggagttttt aaagttattg ttttaagggg 88800
ttttatggga taatgggaga gttgttgtga tgtttaagat taaagtgatg gttgggcgta 88860
gtggtttacg tttgtaattt tagtattttg ggaggttgag gaggatggat tatttgaggt 88920
taggagttcg agattatttt ggttaatacg gtgaaatttt atttttatta aaaaaataaa 88980
aattagtcgg gtatggtagc gggtgtttgt aattttagtt attcgggagg ttgaggtagg 89040
agaattgttt cgatttggga ggcggaggtt gtagtgagtc gagattacgt tattatatta 89100
tagttcgggc gatagagtga gattgtttaa aataataata atgttaataa taaataaaat 89160
aataataata aaataataaa agtaaggatt tgaataaata tttgtatatt tatgtttgta 89220
gtagttttat ttttaattgt ttaaatgtgg aaggaatgga tatataaagt gtgtgtatat 89280
atagtggaat attatttagt ttttaaaagg aacggaattt tgttattata tgtgaatttt 89340
gaataatgaa ttttgaagat tttatgttac gtaagtttgt tttataagga taaatattgt 89400
atgattttat ttatgtgaag ttaaatttat agagaaattg gaatgacggt tgttagtagt 89460
taggggaagg aaggatgggg agttattgtt taatgaggat agagttttag tttgggaagt 89520
tggaagaagt ttcggaggtg gatggtgttg atgggtgtat aatgtgaatg tatttaatgt 89580
tattaatatg gaaatttggt taaaatggta aatttgtttt ttttgttttt gttttttttt 89640
tttttgagac ggagtttcgt tttgtcgttt aggttggagt gtattggcgt gattttggtt 89700
tattgtaatt ttcgtttttt aggtttaggc gattttcgtg ttttagtttt ttagagagtt 89760

gggattatag gcgtttgtta ttacgttcgg ttgattttg tatttttagt agagataggg 89820
ttttattatg ttggttaggt tggttttgaa tttttgattt taggtgattt atttattttg 89880
gtttttaaa gtgttgggat tataggcgtg agttatggtg gttggttaaa aatggtaaat 89940
tttatattat atatatttta ttatagtaaa aatgtattgt tttattagaa aaatgataga 90000
tttttataat atgaattaaa ttataagtta tgaaggaaaa attaagtaaa atattaaaaa 90060
attatttata agtaattata tttaaaataa aagataataa atatataatt tttattattt 90120
tttaggtttt ttgtaatatt taattattgt ttgttttgtt gcggttatgt ttttgaggtt 90180
gtatttgtat gatagaaata ttatattcgt ggtgattaaa tatttttttt tagttttatt 90240
tttaatgatg ttatatagat aatttgaaat tggtcggttg ggtacgatgg tttatgtttg 90300
taattttagt attttgggag gttaaggtag gtagattatt tgagtttaaa attagtttgg 90360
ttagtagggt gaatttttat ttttagtaaa aatataaaaa ttagttagga gtggtggcgg 90420
gtgtttgtaa ttcgagttat ttgggaggtt gaggtaggag aattatttga atttgggagg 90480
tggagtttgt agtgagttag gattgtttta ttgtatttta gatcgggtga tagagtgaga 90540
ttttgttaaa aaagaaaaga aaagaaagaa attggttaag gtaggaatat tgatattata 90600
ggaataggta agcgttataa atcgggaaat tgagtgttgt tgtttttgtt ggttttaggt 90660
ggtcggttgt tgatcgttaa ttggtatatt attggaggag ggaggtttag attgattcgt 90720
agagagatta gaggtagaaa cgtattatgg tttggatgtt gagggtgagg gaaagagagg 90780
agttaatagt ggtgttcgga gatttggttt gagtaattag gtggatggta gtatcgtttt 90840
ttaagatgag gggttgtggg aatttgaggg gttgtggaag gttttgttat tgacggattt 90900
agggttcgcgt agttttggga ggtttatttt gtgttaggtt acgtagagat aagattattt 90960
gggttttgtt tttgggaagt ttttgtttat gaggatatag tttgttattt aatggggtgt 91020
ttcgtttgga gttttagtgt tgttgggaga aagtttttta ttttttttcg tttttcgagt 91080
ttttttgtt tttttgttag ttgagttttt ttatggaggt tgtgtttgga gggggttat 91140
ggatatggat gtgtgcgtta aacgttgttt tttcgtggat ttttttttta tttcgttttt 91200
acgttttgta agttttattt tttcggggtt tgatttgtgg ggttttttttt tagatttgtt 91260
ttttttattt tttagaaagg cgtgtagggg ttttttatgg ttttttttttt tgtttttatt 91320
ttttttttt tgggatagtg atttgttgga cgggtgggtt atttataggg tttgtgattg 91380
tttagatatt tttgttgtat gaagttttga ttttaaggta tgatgtagat gggaataagg 91440
tacgagtttt ttttggttgt tgtaataatt gtcgaaattg ggtggtttat agtaagggga 91500
atgtgttttt ttatagtttt ggaggttaga agtttgaaat taaggtgtta gtagggttag 91560
ttttttttga agatttaggg ggaaaatttt tttttgtttt ttttagtttt tggtggtttt 91620
aggtgttttt tggtttgtgg ttgtattatt tcggtttttg tttttgtttt tataaggttt 91680
tttttgtgt ttttgtattt ttttttttgt tttttagaag gatatagtat tgaatttagg 91740
gtttatttta ttttgagatt tttgtttaaa tttttttttgt agagattttt aaatagaatt 91800
aggttatatt tgaggtttta agtgggtata tttttgtttt ggggggggatt atcggtttat 91860
tgtaggtttc gttgtgtatt gaagacgtag gcggtttttt tggttaggtt tgtagtcggt 91920
tttgtttttt tttgtgatcg tggaagaatg tattttgttg gatgtatagg tttttatagt 91980
tttttgttt ttgttaaatt ttagtttagt ttttttttttg ttgtcggcgt gtattttttt 92040
tgtttagtgt tttttttggg attgtgtgtt taggttttga ggttttagaa aagggggggtg 92100
gagggttttt ttattatttt gtagtaatga ggtttttatta aaattttgga gttttagatt 92160
ggatttagtt tttagttatg attaaaggat agttcgggat atgattaaag ggttagagaa 92220
agatgattta ggttgagaaa gaggaggtta aggagtaatt ggattgtttt tgaatgtgga 92280
aaaggtaatt atgatttgaa gggtggtgtt tagttgtttt ttattttttt tgaggatagg 92340
aaaaaaaagg aaataaatga tgtataatta gagtagttgg ttagatacga gggtgaattt 92400
tttggttggg agtatggtta agttttgatt tgggtttttt gaaagagttc ggggaatttt 92460
tttatttgga ggtttttagg tttggttatg tggttttgag tgattatatt gaagggtag 92520
tggttggggt tggagtcggt gggttttgag tatagttacg aaggtatgcg tatttttttgg 92580
ttgtttttt gttattgttt tttgggtttt tattttggtg aggcgtgtat tttggcggcg 92640
tttttaggg aattaaatat gtttgttgta ttgtgcgtgg agatggagaa tgtataattg 92700
gttgattttg tgttaatttg gtggaatttt atgttagttt tgggaaagaa taattgtatg 92760
ggtgtgttta tatttattag gtgtttttta gaaaaatatt cgagaataat gttgtggttt 92820
aggatggttg ttgtgtcgga ttcggtattt ttttaggggg gttgtgttgt tgggttgagt 92880
tttttaggta ttggattttt aaatttttaa atacggcgtg gataggtggt ttagtagggg 92940
ttggattatt cgataggttt aggtgttgga gtttagataa gatatatttt ggtttggcgt 93000
ggaagatacg gggtgttatt aatggtagta atggttgtat ttttgaaatt cgggtttttta 93060
ggtcgacgag ggtgtgtacg tatttgaaat gtttgtggtt ttgtagtttt tatgtttata 93120
aatttatttg gttgaaaata gtttaaaata tttaaagtat gagggaggga gtgtttgttt 93180
tttttaaaaa ggaaggattt gattttattt atttaaaaag ttatttaaat ttagaatatt 93240

tttcgtaaga gattttttgt ttttcgtttt tttagaatgg ttggagagtt ttagtatttt 93300
tgtatatttg ggatatttta gagggggtgg ggaggggtaa gtgggtagcg agcgatttta 93360
gatttaggat gagttgttag gcgtttttcg gttatatatt taagggatcg gagtgtagtt 93420
gtagcgttgc ggtttgttgt ttcgggggtg ggggtgttgt tttatgttgt gaatttttat 93480
atggtttttg attttgggta gaggtcgagg gtttaaggga cggggtgata gggagagtat 93540
gtaggagtgg gtttttggtt ttttagggcg agtggaagaa gcgttttttt tttttgtagg 93600
tgatagattt ggggggtttt ttttgaggat gagagtttgt tgttttttaa gttttgtgtt 93660
taatttaggt ttttaggttt attttagttt ttcggttttg tttgttttgt ggatgatata 93720
gtttaagggt agagatcgtt ggtttggagg gaaggttagg ttttaggtta gggtttagaa 93780
gggagggaga agtttttggg gtagtttttt ttttgtttat ttattgttta gttttttttt 93840
ttatattttt tttcggaaac gtttgttttt gataaggttt atttttttgtt tttaggaggt 93900
ttttattgtg gaggaaggga ggcgtcgttc gtttttggtt tttttgatag tcgtgtttta 93960
ttttcgtttt gtgttttttt tttcggatag tgtttttttt agggtttatt taggagggtg 94020
tagcggtggt tttcggggcg gtggtcgtgg tgggggtgtt agttgtaggg gtgttttcgg 94080
tgggtgggag ttggtggttt ttcgttggtg ttatgggatt cgtatgttcg ttttgcgttt 94140
ttcggttttt gagtttatag gtcgggattt tgtttgttag tcgcgtgcgt tgtcgtttaa 94200
tttttgtagg cgtagagcgc gcggcggcgg tgatagagaa ttttgtttgg ttgtttaaat 94260
atagtttttt gtagaaggat tttgcgttcg gggaagggga ggaatttttt tttttttggg 94320
cgttcgtttt tttcgttatg gttcggtttt tatattcgtt tatatttggt cgtagcgggg 94380
cgttcggggg gaggggttga ggtcgcgttt ttcgtcgttt tttgggcgcg ggttaggcgg 94440
ggaggagggg ggcgtttcgg tcgtgtgttt aggattgttt tttagcggtt attcgggttt 94500
tagtttttta ggtttggttt tgataggcgg gcggagtagt tagtgcgaga tagggaggtc 94560
ggtgcgggtg cgggaatttg attcgttcgg gaggcggggg cggggcgggg gcgtagcgcg 94620
cggggagggg tcggcgttcg tttttttttt ttatttattt agttgagtta gggggtttag 94680
gggtttttttc ggcggttagt tttgtattgt aggagcgcgg gcgcggcgtt ttagttagcg 94740
cgtagggttc gggtttcgtc gggggcgttt tttcgtcgtt gtttttcgcg cgattcgttg 94800
tttattagtt attatgtcgg atttcgcggt taacgcgtag ttggatggga ttatttcgga 94860
tttcgaaggt gggtgttggg ttggttgttg cggtcgcgga cgtgttggag aggattttgc 94920
gggtgggttt ggcgcgggac gggggtgcgt tgagggggaga cgggagtgcg ttgaggggag 94980
acgggatttt taatttaggc gtttttttcgt tgagagcgtc gcgcgttttc ggtttcgtgt 95040
tcgcgtcgtt tacgtggggg attttgttag gggtattcgc gtagatttgg cgcgttttta 95100
taggattttg tgttcgtttt gcgtattgtc gtttgggttt tttttttttt attgttgttt 95160
gtgtttgtta agcgatagcg atttttcga gggttcgcga ggttgtttcg gaatttttta 95220
ggacgtatag ttttattttg ggaaatttat cggttttttt tttttggttt ttttcggcgg 95280
ttttcgggtt tcgtttggat tcggtaacgg gatagggagg tcgtttttta ttttcgattg 95340
agtggatagt cgcgttttgt tcgggtggat agtttttttt ttttttacgt tagtttcggg 95400
gtcgttaagt tgtgtagttc gtgggtcggg agtatcgaac ggatatagtt taggtcgtgg 95460
tagggtttag agtgggatgt tttatggttt ttatttaggt ttggggatat ttttattcgt 95520
tttttagaat cgggtcgtgg gggatagaag gggtttgcgt gcgggtaggg agagtatttt 95580
ggtttttttt tgttttcggg gtttataaag tgtgttggga tttgcggggt tgttttgttt 95640
aagtttgggt ttggcgttcg cgtttttgag tttgtgagtg cgtgcgtttt tttgcgtttt 95700
tttgattgtc ggtgttgggg ttttgcgttt tgcgttcgcg ggagtaaata tagtaggcga 95760
aggggaagtt tatataatgg tttttagcgt tttggggtag ggtttttgag gggcgggttt 95820
gtttttgtcg ggatttggag ttttcgtttt tcggagaggt ttttaggttg atttgggtag 95880
agtttttttgg tgggtcggga gggggaaagg ttgtgttgaa atgagtaaat tgtttaggtg 95940
ttaggttaag ttgggaggtg attagtttga ggttttttttc gttttatggt tagaattagg 96000
gttgatattt gggtgttttg agtttagttg tttatacggt ttatttgggg ttagttttat 96060
ttgagtgggg gaggcggggt tttttggggg attagaattt tggttggacg ttaagtagag 96120
tgttagtggt tgttttttag ggttgggttt gaggagggtg tggggcggcg aagggacggg 96180
agggggttgt gatttagtgg ttattggcgt tgtgtagagt gtgagttgga aatatcgtag 96240
ttattttgtt agtttagtgg tgaaagtttt tttttaggtt ttattttttt gtatttttgt 96300
tttttagagg gaggggaggt ttgggtttgt agagttggga gggtttgttg ttttcgtttt 96360
tttttttat aatattttt tatttggata tttttgggta tatgtttata ttggggtttt 96420
tttaggttta ttgtgtttcg ttgagttttt tgtagttttc gagtgaatgt gatttttttg 96480
tttttgtttt tttgtaattt tttttttgcga tcgttttttt aggggttttt tttgttttaa 96540
atgtttaagt ggtacgattt agtcggtttg attatttttt agtaagtttt tatggagaga 96600
ggttttgtgt tgtgtagagt tttttttttg tttgcgggat cgaggttttt gtttttagtt 96660
tttaatagaa agtgtcgggt ttttagtggg atttttgggg aagaattttc gtgttttaac 96720

gggagttttg tggcgggagg ggaggttagg gtttggggtt gtgttcgttg tatagttgtt 96780
attatttgta ttatgaaagt tgttagtgtt ttttttttgg gtttttgggt gtaattttat 96840
ttttgttttt atgtgttttt atttggagtt gtttttgcgg ttgtttttta agttagtttt 96900
gtgattttgt aatttagttt aagataatgg gtttattgag attattttgg tgtagtagtt 96960
ggtaattttt tggttttggg ggaaggtttt ttagtttcgg ggagtggggt tttaatttgt 97020
tggtttttg tgtttattag ttttttttt gtgtgtttg aatggtttg ttgggaattt 97080
tggttttaga gttattaggt ggttcgagtc gataggcgtg agagagtgtg tgtgtgtatg 97140
agtgcgtatg tgtatggggg ttgatttggg gtatggaaag gtggtttttt ttggtgttta 97200
aggagtttgg agtatagttg gagggtgtgg gggtgtgtat atgggagttg gataattttg 97260
ggtggataga tagacgtggg gaagggatga ttgaaggagg tggaggagag agtgtgattt 97320
agtttagtta ggggtgatgt ggataggtag ttttcgaatt agggtagaga aaagttatta 97380
ttagttagta ggggagaagt tagtatggag gaggcggatt ttgagggaga gtaggaattg 97440
gattgtaaga ggaaggagag ttttttggtt agtagtagtt agtagtagtg ggggaggttg 97500
gaatgagttg gttggagagg gggttggggt ataaggaggg gtttgtttgt gaagattata 97560
tgggttaggt tgcggagggt taggtatgtt cgtcgggagt gtagttggtt tacgggaagt 97620
atttggagtg gttgggaatg ggcgtaggag tagcgtcgtg ggagtatagg ttttttttc 97680
ggggcggttt atttggtgtt ttggttttg taaggtaggt cgaaagggtg gggaggaaat 97740
tgttagtttt ttatagcgtt gggatggtgg ttttagggtt tttgaggtta gcggatgtgg 97800
gtgtttgtta ttatgtgggt tgttgagggg cggagatttt aggggttatt ttaaagtagg 97860
acgagttttg agttacggta tttttggggg tagtttttta atcgagtaga cgtttaggtt 97920
tggaattttg taatagaggt tatagggttt tgattagggt gttttgggag gtttagaatt 97980
agtggtagta tatagggtag acggtaagtg atttggtatg gggaaagagg taggtgttta 98040
ggtcggtata gtatattcgt aaggaatagg tagacgggaa gtcgttcgtg ggtttgtgtg 98100
tgtgttcgga gttaaaattt tgttaatgtt ttatgtttg ggtatattta ttttttttt 98160
ggggagtatt ttttttttta ttttttttt tttcgtttgt ttttttatt tagggtttt 98220
tttattttt tcgttttggg gatcgagggt attatggttt tatgttttat tatcgatgag 98280
ttgtataggg atttagtttt ttcgttgttt aggtcgggtt ttttaggttt agggttttta 98340
ggaatggaga gggtattagt gtttttatg gatttaaatt tttcgtattt cgtttttgtt 98400
ttttttttaa gataggtttt cgagttttaa ggttttaggg ttttgtggag gtcgttacgt 98460
agtagtaagg agaatgtttt gtatttggtt gatgagattt ttagagtttt atttttatt 98520
ttttattgt ataaacgggt ttttaggcga ttgtagtatt cgttattgtt cgtaataggg 98580
tgataagagg gatgattttt ttttttttt ttttttggt tggtggaggt acggggttgg 98640
cggacggtat gtgttttcgt gaatttaggt taaatttgtt atcgtaaata cgattataat 98700
tcgggttttt gtgtaataaa agttttttta agtattagtt gttggtttgt tttgtttagc 98760
ggtgtttgtt gtaattagat ttgtatatcg agaaagaatt taaaagtttt tgatgtttgt 98820
tgaaataatt tggtttagga tttacgtgtt tagattttag agttgtgtgg tatttgagtt 98880
tttttcgagt ttttattgtc gttcgaggag gatttttaga tttgtgtttt ggaggtagag 98940
taggttgtgg gacgggtttt tgggtgggaa ggattatgtg gatatgtttt tttgtttgag 99000
agttttaata ttttcgggac gtgggagttg gcgcgttggt aggatttagg tgttttttt 99060
tttttagag aaaaaggttt cgttgtttgg taataggtgt agatttgttt ttaattaatg 99120
ttagtaggtt ttttgcgtga tgaattttgt tttttagtta agatttaagg tattttgtga 99180
atattgtttt tttgtagttt gagtttttgt ggtgggaggt aggagttatg gggagtgggg 99240
gtaggttttt tatacgggtt ttatagttat tggtagtatt gatttgatgt tttttgagtt 99300
tagagtttag ggttagatag atttattgtt tcgattacga gttggtttat ttagaggggg 99360
gcggatatag tatttaggta gtagatgtat tgtgattagt tttgtagcgg ggttgtgggt 99420
tttttgggtt ggatgttcgg gaagaggtag gtggaggtaa acgttaggat atttttgtag 99480
tgattgggtg attgtaggtt ggaaatgttt tttgtgggtt gtggttgttt aggaaggttt 99540
tgaatggggt tagtggatag agtttgtatt tagaggggta gtgttttgga ggagtgaggg 99600
gtatggtagt gtagggatgt ttaggtcgtt tttattttgt tattggaaag ttgggcggtt 99660
tcggtttttt tagtttttc gtttgttttt ttgtttgtaa agtggggtta gaaatagttt 99720
tttttgaggg ttgttggggg attttgagat gtagtttatg gcgttgagta cgggttttgt 99780
tttttacggg tgtggtgggt gtcgcggttg gtgtggtatt tgggcgggaa aagggggtat 99840
ttgtaaagga taggtaggtt tggatgttta aatatgtaga tttggggatg ggaggtttta 99900
ggtaagggtt tgtgtgatgt tattgtatga atgaggtcgg atagtatggt tattattaat 99960
tatggaaagt acggtagata ttagtgttag tagcgttcgt ttgggggttt ttttgagttc 100020
gataggtatt taggttttta gtgtagttat gggatttttt ttttttttgtt tggttgatat 100080
tttggaggtt ggcgttatgt tggttgtagg ttttttttat tgggaggttg ttttggtttt 100140
ttttgttatt atttattttt atttttattt tggagtgtta tagatgggtg gattatatgt 100200

ttatttgtag ggttaagcgt tgataatgag gtgagtattt gttggatgat cggattatga 100260
ggatagcggg ggcgttggta gtttgcgttc ggttttgtag gttggttttt ttttgaaaag 100320
cggttgtggt agagtgtgta ttttagggag tggtgtttgt tgttttgaat atttttattt 100380
agtaggtttt tgtaagggtt tagttaagtg tggtgtagag ttgggttggt agttggaata 100440
gttttatttt gtttagtggt gagattaagg gttgatgggt taaggttgcg tgttgtttgt 100500
ttagttcgtg gatttattta ttgagtattt ttatattttt atattgggtt cggggtgtta 100560
ggaggtggag gggggattgg gtgtttggaa aaataagttt atcgagagtt tgagttttta 100620
ggggttagta ttaggtagtt attacggtgt tcggtatata gtggttattt ggtttcgtcg 100680
gttgttttgg tggtggagag cgtgtatggt ggtcgtacgt gatgggtatt atttagggta 100740
atatagatat gtagattgtg tatttgtttg taggagttag ggattttta gtttggtaat 100800
ttgtgatttt tgtttattcg ttgggagaga ggtgttgttt tttgatagtt tggtgtcggg 100860
agagtagaat tagggttttt tttttttttt tttatttttt atttttata tgtttaggta 100920
ttaggggagg ttatatttag ggatgagttt tggttattgg gtttagggtt ttattgttgt 100980
tattttttt ttttgttagt tagttaaatg ttcgtttatt atcgtgtcgg ggatggggtt 101040
ggtttgtttt tttgaagttt ttttggtgta tagtttaggt tggagtttcg gacgatttcg 101100
ttattttttt tttcgatttt ttttttttat tttgatttat tttagaaatt tagttaaaaa 101160
ttaagttttt gtgttttttt ttgataaagt tattgaggaa aaatggttaa atattgtttt 101220
tttttttaaa gtttttttgg ttttttttaa atttggaggt tttgagttgt ttatgagatt 101280
aaagggttcg gaatttttttg gaatatagtt ttagaagtgg gaggatttgt tatttttta 101340
gttaaataga tttattagtt ggtttttaga tatttttagg cgttttgggt atttttgggt 101400
ttaggttgtt taatttggtt tgtagagttt cgaggtttcg tttagattgg gttagtagat 101460
agttttagag atttaagtt tgttgtcgtt tttaaaagtt ttgttaatgt ttagaaattt 101520
agagggataa gaagatgatt tcgatgttag tcgggattat aaatagtgag gttcgttttt 101580
tttttatttt atgtttgttt ttattgtttt cgtagttggg tttttagggg attgttcgtg 101640
gttatggggg aggtcgggaa tgcggggttt agattttatt gggtttttcg tattttttg 101700
tttttttttg tttatttttt ttttcggaaa tttttgaata tttttttata gtttttggta 101760
ttttgttatt tttggtattt ttgggttaat tgggaaagtt attatttgtt taatatttag 101820
aaaattaagg ggtttggttt taaatatatt gtagtgtttt tttttttata tggtatcgaa 101880
tttttttgtgt ttttaaagtt aggagtttgt gttttggatg tgggcgatgg agttttgtag 101940
atatttggag cggttttttt gggagagttt ttttcgtttt tttatttatt cgtttttttt 102000
tttttgtttt aagttttggt tatagtttcg gaggggttat ttgagtgtag gttgttttta 102060
ggtgtatagt tggagggggg tagttatttt atattttttt gtttgttagg ggtattttta 102120
gggatttagg ttttgaggga tagcggaagg gtggggtggg gagtaggtcg ttgtttcgtg 102180
ttatttgggt gttatgtaga ttttgaatgg gtagtttttg ggagttggtt tttttttttt 102240
atttttttta tagtgttgtt tttttgaggg gttggagttt tttggagttt tttgtagttt 102300
gtggtaggtt tttttgattt tgtgtttggt ttcgtagttt ttaatttata gttagtggta 102360
tgaagaatat ttttggttgt ttttatttgt ggtttttgta ggtagtagtt ttttggggta 102420
gggtttgggg gttttggtat tttttttgtgg gatttttttgg gtatgaggga gggagttatt 102480
tgggttatag atagattttt tttttgtatt taggaggttt aggatggatt tgttttattt 102540
tttttttttgg tgggaagaga gtatttttgg ttatggggaa tgtggtttgg atagagttat 102600
ttagtttttt tttttttttta ggtagggttt tgaaagatgg agatggtttt gtttttgtta 102660
ggttaatagt tagtagtttg gtggattttt atgtagttta ggtaggattt ttttttgttt 102720
ttatttttta ttttatgtgt aggaagttgg ttttaagtag ggatttttgg tttcgttttt 102780
tacgtgggtg ggtagttttt ttgcgagggg taggttaggt ttttgtttta tgttttttag 102840
agatcgatgt tatttatagt aagagtatat aagttttttt ggttttaaat taggtttgga 102900
ggggtggttt ttttttttgt tttttgtttt ttgtttttta ttaaaggtta gggaatggag 102960
tgttttgtat gtttgttggg gttgggtttt tttttttggt ttaaaatgaa gggttggaat 103020
ttttggtgta gattgtacgg tgagaggtgt tttttgggat ttattcgagg agtattattg 103080
ttgaatgttt tgttaggtta ggagtatcgt tgggggtggg agaggtagtt ggtttatttg 103140
tttatttaat agttatttat gggtatttta agggttagtt ttgttttagg tattgggggt 103200
ttaggtatgg gtttaggaga aggggtagaa cgggagggtt ttttggagga aggtttttta 103260
attagagatc ggggtaggag tttgttaggt aggtgatgtt ggttagtttt ttttgttatt 103320
ttttttttt ttttttttt ttttttttt ttttttttt gagatagagt ttcgtttttg 103380
ttgtttaggt tgtagtgtag tggcgtgatt tcggtttatt gtatttttcg ttttttgggt 103440
ttaagcgatt tttttgtttt agttttttga gtagttgaga ttttaggtac gtattattac 103500
gtttagttaa tttttttgtat ttttagtaga gataaggttt tattttgttg gttaggttgg 103560
ttttaaattt tttattttag gtgattcgtt tattttggtt ttttaaagtg ttgggattat 103620
aggtgtgagt tattgcgttc ggtttttttt tgttattttt gtagatttat agtgtgtgtg 103680

gaagattgta tgtttgtgtt tggtatagtt gttttcggta tttaggtggg aggtggggga 103740
gagggttgtt ggtgttagta taggttatgg tatgagatag gttttcgtag ttaagcgggg 103800
tgtatcgtag tattggaggg gttttgttgc gtacgagatt taggttttgt atttgttttt 103860
tttttagga ggtttggagg gttgggaggg attagggtgg tgaaataatt taggtgggag 103920
agaggtattg ttggagaggt agagggagtt gtaggtttgt agtagcgagt tttaaatttt 103980
gggtttttat tagaaaattt tagaaagggt tgttttgttt tttttttaa attataaaag 104040
aaatttttgg tcgtttgggt tttttggggt cgggtggggg aggagatttt ttattcgtat 104100
ttttgtaggt ttagtattgt gtagagggag gagggtggag gtaaaggcga gggaggaggg 104160
tggaagtaaa ggcggggttt ttttttagtt tcgttgtttt gtttggttgt tgtttttaga 104220
tttttatttt tggttcggaa atatggaatt ttgggatagt tggggtaggg ggtatagaac 104280
ggagattttg gtgagggagg aatttttttt ttagttttgg ttgtggttat agtaaggtta 104340
ggttgggagg attttattta aatatttata tttttttttaa tatttaaatg atgaagtagg 104400
tttttgtata tagttgtttt ggagttagtt gttgtttgtt ttattttttt tttttttttt 104460
tttttttttt tttttttttt tttttttttt tttttttttt tttttttttt ttttttttgt 104520
atttttttgtt tttgatttaa gtttaggttt ttttagttgt ttttttttta tttataggta 104580
ttttttgtga ttgagttttt ttttgttatt ttttataatt attttttttta attgagtatt 104640
ttttaggtgt taggttttgg gttggtattt tgtatataag gtgtttttta atttttacga 104700
gagtttaata aggtagattg ggttgttgag gttttagcgt tggaaggtcg gttcgggttt 104760
ttttagttcg tatttgtatt tatttattat ttttttattg tggagatttg tttttttggtt 104820
tttggttgag tacgatggaa atttttgttt attagttttt ggagagtggg gatggttgag 104880
ggtttcgatt ttggggtttg acggggggttg tttttgtttt atttagttt ggttgttttt 104940
ttatttaatg attgagtttg taaaattaga ataatagtgt tatttggttg gtattttggg 105000
tggttgggag gcgtgggaga gggtgtacgg aggggttgag cgaggtgtta gtatcgtggg 105060
gattttcgag ggtagttttt ttaggtttag ggcggagtgt aggtagaggg cgcgatggtt 105120
tagcgtgtcg gtttatattt ggttttgttt ttgtttatac ggtaatgtgg gggagttatt 105180
agttttttag tgttttagtt ttttatttgt gtaatgggga taagagtaaa ttattgtttt 105240
taggtatggt aagtatttat atatattgat tattgtagaa tgaatatagt gagttttttt 105300
taggttttta ttatatgtta ggtagggtgt taggttttag tggagagggg ataatgatgg 105360
gtaagttggt ttttgttttt aagggttgtc gagtagggag gaaggcggcg gttgttttga 105420
ttgtgattcg gagtggggtg attcgtaaag aggttggcgg gaggttggtt gggggttggt 105480
tgggggttgt tttttttttt gtttgtgttt cgtggtaggg tattaaattg gtttttttta 105540
tggattttcg aggttttgat tgtgggattt cgggtaggtt ttgggatttt gtgattttgt 105600
tttcgtttgt tatttaggtt tttgttttta gtagtgttta gtatacggcg atttttgtat 105660
gtttttttagt gtgtgattga ttttggagtt gagatttggt agttgaggtt ggatattgtt 105720
tttggataat tttcgggggag gtgggggtag ggtggggttg tgagttggta atttcgagtt 105780
tttagtagtt ttcgaatgga ggtttttttt ttgtaggatt ttcggggagg aaggagggga 105840
tgggggagat gaggtttgtg agtttttagg agggtagagg tttatatttg tagcgtttta 105900
gaaatatttc ggggtgttta ggtttattta gtttttttga gttagaatta ttaagagttg 105960
gggattagta gtttattttt tagtaagatt tagaaatgat ttttttgaat agttcgagtt 106020
aagaatgata tatttggaat tagaatatta gaattataga ggaatcgaga gaacgggttt 106080
aaaatgtgta ggattttag ttatatggcg ttatatggag gaaagtattt aatattatta 106140
ttgtatgaat ttgtttaaat atatatatat atatatatat atatatatat atatatatat 106200
atatttttta gtaaagagtg gaaagatatt tgttagaata ttgatggaag ttatttgggt 106260
tgtgggttta tagatttttt ttttttgttt ttgtgtattt tgtaaatttt cggttttgtg 106320
tttaaatttt ttttatagtt aggaataaag taatagatgt tattttttaa agtttatggg 106380
ttcgtgttcg taattttaat attttgggag gttgaggtag gaggattttt taagtttagg 106440
agtttgagat tagtttgggt aatataggga gatttttatt tttttttttt tttttttttg 106500
atagagtttt attgtgtttt aggttgaagt ggcggtggta cgtttttggt ttattgtaat 106560
ttttgttttt taggtttagg tgatttttat gttttagttt tttgagtagt tggattatag 106620
atatttatat ttgtaggatt tgagaatttt ataagaggaa agtttttatt cggaggttgt 106680
tggggatttg gagttgttag tttatatatt ttttagtaga gatagggttt tattatgttg 106740
attaggttgg ttttaaattt ttgtttttaa gtgatttatt tatttcggtt ttttaaggtt 106800
ttaggattat aggcgtgagt tattgcgttc ggttatggga gatttttttt taaaataaga 106860
ataaaaataa aaataaataa aagtttattg ttaaaaagaa aaatatatta tttaatttaa 106920
ttttttttta ggattgagag atgaggaagt agaggtcgga aagtgttata ttttttggtc 106980
gttggtagtt aggggatttg aggtttgggt tggtttttttc gtgttttagg tttggggtgt 107040
taggtggagt ttttgttttg tttaaaggtt taggtgtttt tgttagtatt gtttgttttt 107100
gtatttttgg ttataaggaa tgtgggttaa aggtagggtt tttgattttt gtttaggagg 107160

gtgaagtggg ttggtttttt ttgtttttgt tttaaggttt ttgcggttgt gtttttggaa 107220
attgttggtt cgagttagtg tgcggagttg ggatagattt tatcgtggtt ttcgtggggt 107280
tattttatgt tcgggttgga agggattttt cggtttagtt ttttttgttt tttattagga 107340
aattgagatt agagaggtta tttgtttggt tcggggtggg gtgtagatgg cgtttgtgat 107400
ttttgtatgt tacgtgtaga tagtttttta gttgtgggtt ttttacgtaa gtagttatgt 107460
tggggataat ttggttgaaa tttgcgattt aatgaagaga agaatgattt tttttgtagt 107520
tttgtgtttt ataggttttt ttttgttttt attttttttg tagaggggtt ttatgttggt 107580
gggtgggttt ttgttgttgt aatgggttta ggtagttttt gggtagtgga ggtttggttg 107640
tttttttgtt taatgttttt tgtagtttac gttgttcggg aagggcgggg aagtatttag 107700
ttattgggga ttttataaga tgggtatttg ggagatgttg attggtaggt taggttttcg 107760
tggtttatag gtaggttaga ggttatagtg ttgagttggg ttgagttggt agtttagggt 107820
agtgggggtg ggggtatttt gtttagttat gggttttttt ggtataaggg tttggagttt 107880
attgtaaata atttttttag tttttttttt ttttataggt tattagtaga attgaggggt 107940
tttttgttga tgaggtgtgg tttgggaggg gttagttgtt tgtttatttg atttttgcgg 108000
ataggtgatt ataggttttt tgttttaggg ggttggggga aagatcgggg aggttttttt 108060
ggttttttta tttggaagga aaatacgggt ataggggaga tattttatag ggatatattt 108120
tagttagtat tttttggttg tgttggggggt atagtgtttg ttttgtttat aagaggggtt 108180
agtttttttt aggatttaat tttagttttt agtgtagggt agatgagtat ttagaggttt 108240
agaggattta tattacgaga gagaagttgt gattatatag gtattgaagg ttaagtagaa 108300
gagtgggata tacgggtgat ttgaaagttt tagggttagt gtggttagtt ttagatagtt 108360
ggggagtttt gggtattagg tttagtaggg gaggagggtt ttcgtgatag tttgttgggg 108420
ttgttgtaat aaagttttac gaattttggg ggttgaagat ggttttagag gttagaagtt 108480
taaaattaag gtgtcgatta ggttgatttt ttggggtttt tgcgggaggt ttcggtttag 108540
gttttttttt tagtttttgg tggttttcgg gtgtttttgg gtatgtagat gcgttacgtt 108600
atttttttgtt tcgtttttat atggtttttt ttttgtgttt ttgtgtttcg taaggatgtt 108660
tgttattgta tttagagttt attttaaatt taggatattt ttttttttgag atttttttatt 108720
gtatttgtaa agattttatt ttaatttttg ttatatttat aggttcgagg gttagggttt 108780
tggtatgttt tttgggggtt attatttagt ttattgcggt agttgatttt aggggaagta 108840
gaatgtttta agtttggggt gagcggaggg aacgttgttg ggtggggagt gggagattag 108900
gttcggagat atcgcggatt ttttttgttt gatgagggcg tgatgaggtt tttattttag 108960
gtttttttgaa tggcggttgt ttttgaggag attttttttt ttggatttgt tttttttagaa 109020
atttttattt aggtttaggt tttaaggtgg atggtaagag gcggttgttt ttgttatatt 109080
tgatgggtga gtgtgtgaac ggggttggga gtaggatatt tgttattttt gtggatagat 109140
tttagaggtt tatgaatttg ttgttgggga aggtgtttga tttagtttga tttttttattt 109200
ttattttttt tttttcgttt ttttttatta gttttgtttt tagagtttta ttttttttaaa 109260
ggtaggcgtc gtttttttgga tttttttagta gttatttttt atttagatt ttttttttttgt 109320
aggttttttt ggtttagcg tttattttttt tttgggtttt tgtttttttta tcgtgaggtt 109380
tttgggtaga agtgtggggt tttttcgttg tgtttggttt tcgcggtata ttaggggttt 109440
ttttaggggt tcgttttgtt cgttaggttt ttagggagtt ttttaagttt ttagatgggt 109500
ttgatggagt tttttaatgc gttgttttag agtaggaggg gtttcggttg agttcgagag 109560
ttttttttagt tatattttgg gttgtttggg gtagttaggt agttcgtttt ttggaggtgt 109620
tgtttttttt tggttatttt ttttggaatt ttttgcgttt agttgcggtt ttttttatat 109680
agtatattta ttattttttt ttatttgtga ttttttttttg ttaatttttt tatgtttttg 109740
agaatggaga ttaggttttt tttattttgg agtttagtag aatttaaatt aggtagattc 109800
gtgtaggttt acgtgggggt tttgtggtta ttgtggtttt gtcgttattt ttagtttagt 109860
ttggcgcgtt ttggattatg ttatttagtg tcggtttttt aaattttttt tttggttttg 109920
taggtttggg aagtagttgg tttttgggtg tagttgtgat cggagtttga attttgtttt 109980
ttggggttat cgttttttgt aattaggttg tagtttatat tggttcgggt agaggttatc 110040
ggttgttcgt tttggggtta taatatagag agtttcgtga ttaatggagg ttttatttgg 110100
gttaagtggt ttttggtgtt aggcgttttt tttttttttt tttttttttt gtattttttta 110160
agtgtttgta atattttttt taatggataa tgagacgagg agggagggag gggtggtttt 110220
tttgtttagg tagattagga atggttatcg tttgtgtgtg tagtgatagg tttgggaata 110280
gataagggag agtttgttcg ggaggttgga tttttatatc gatgttgttt gtgtttggta 110340
gttttgtttc gggttttaga atagatattg attaatacgt tttttagggt ttttaggttc 110400
gggttttggt tttcggtaga gttttttatt tatgggaaga agtatcgagt agtttttgttg 110460
gtttttcgta gggagttggg atgtattttt aagagtagta tgcgtgaagt tcggggtttg 110520
ggtaaaggag tgtttgggga agagtttgtt gttgttggag ggtcggaagt agaggggaag 110580
gaggggtacg cggtaggttg aggaggtggt tggcgatttt gtggtgggga cgaggggtt 110640

atgagtagat taggagttag tattacgttt ttttgttttt agaatcgagg acggagagtt 110700
gttagagagg ttttggcggg gtgtttgggg ttatttaaag ggcgtgggtg gattaaagtt 110760
gaggaaagtt ttattttggg ggatttcgta tttaattttt gaggttttta gagttgtagt 110820
ttgtggtttt tcgaagttaa ttattttta gggtttcgtt tttgttttta gcgatagtta 110880
tttattaggg agtttttgat ttaaggagat ttgaaaggtt atttggtttt tttttattt 110940
ttaggtaggt tatatttaga tttttttata tagattcgtt ttattttttt gttgtttttt 111000
attagggacg gagggtgggc ggttttttta tttaggtttt ggcggtttta ttttagttag 111060
gaaatttttt tttttattt gttttatatt tttttttttg cggttttcgt ttattttttt 111120
ttaatttgtt tttagtggag tagttagtag ttggtttaga attcggggt gggggagcg 111180
ttagattagt gtagtatatt tgagagtgtg ttagaggtgt ttttattttt atagttgatt 111240
tagtaaagaa aagaggaagt ttagtaattt agcgttattt taaagttcgt ttggaatgat 111300
tttgtggttg tcggaattag gggtacgcgg tagatgacgg gagtaggtat agagaggttt 111360
tcgtttcgga ttagtcgttg ttgttttttt ggaggttggg ttgtttttta agaagaggat 111420
ttaaggtttt gtgagcggtg gtttttttg gagtttttat tgtggttttg gttttgtttt 111480
gcgtaaagag agatggtttg aagtagaagt tgttacgtgg ttgttttagg taggcgttcg 111540
tagttttagt tattcgggag gcggaggtag gagaatggta tgaattcggg aggcggagtt 111600
tgtagtgagt agagatggta ttattgtatc gtagtttggg cgatagagta agatttcgtt 111660
ttaaaaaaaa aaaaaaaaaa aaggagtaga gaaatttttt ttagaaggtt tgtagttatt 111720
ttttttttat gttttagagg taaaaaaaaa aaaaattgta tatttcgttt tttttttttt 111780
tttttgagac ggagtttcgt tttgtcgttt aggttggagt gtagtggcgc gatttcggtt 111840
cgttgtaagt ttcgttttc gggtttacgt tatttttttg ttttagtttt tcgagtagtt 111900
gggattatag gcgttcgtta ttacgttcgg ttaatttttt gtgtttttag tagagacggg 111960
gttttattgt gttagttagg atggtttcga tttttttgatt tcgtgatttg ttcgtttttg 112020
tttttaaag tgttgggatt ataggcgtga gtcgtcgcgt tcggttatac gtttttgttt 112080
ttgaataggt ggtttttga ttttgggtgt ggttatttta agattttgtt ttggtggggt 112140
ataattttgg ttttaggtag ggcgatcgta taatttattg tttttgggg gatacgttag 112200
tgaaagggat ttttgaatga ttacggtggg ataatagatg tgagttgggt ttgttttagg 112260
aaggttggat gtatgggttt tttgagttag aggagataat agtagaaagt tttgcggttg 112320
atgggatcga gtaggagaag ggtcggcgga ggttggggta ataacgtatt tgtttattgt 112380
gaagggtttg tgtgagttga tggttgatga gagggtagtg gggcgaggtt gtttttttgtt 112440
aggttagatt agttttagga tatttatatt ttttttttag aatcggtgat ttagtggtta 112500
gaaggggttt tagaattgac gggatattga gggagtcgaa tggaatggtt ggagtggtta 112560
tagtaggttt taggtttttt ttcgtttttt ttttgtgttt tgtttttggg gtttatattt 112620
tggggttgtt gtaatagagt gttataattt gggggtttat aaatatagtt ttcggcggtt 112680
tttggagatt tttggcgttt tttggtttgt agacgtatta ttattatttt tgattttttgg 112740
ttttgtttt tacgtggttt tttttttttg tttgtgtgtt tttttttttt tttgttttttt 112800
ataaagatgt ttattattgg atttagggtt tattaaattt agaatgattt tattttgaga 112860
tttttatttt aattatattt gtaaagattc gtttttttaaa tgtgattatg ttcgtaggtt 112920
ttaggagtta ggatttaaga tatatcgttt tgggggatat tgttgaattt attatattta 112980
ataaatttag agttggtgtt aatgtttttt gcggatatta tagtagattt tggaggtttt 113040
ttgtaaagga attcgtagga tttttttttt tttttggagg gatgagattg tagttgggtt 113100
ggggaaagtt gttggtttaa gttggggttg gtttagttgg tgttggtttg ttaggttgtt 113160
agagagtttt atttagggag aaattaatat tataaagttg gggggataaga gaaggtttgg 113220
aattttaagg gttggttgtt gaggttagtt gtttattttt ttgtgtttta ttttataggg 113280
atttaggttt gttagtgttt cgtttttgga aggaatgttg tgggttgttg tgttttatag 113340
gtagggtgga gggttttttt gtagttagcg agttgttttt tttgtagcgt gtagagggaa 113400
ggaggaggtt tgtttatttt ttttggtttt agtcgaaaat ttaattatag gttttggtat 113460
taggtagatt tgtttatttt tatattatta ttgtagagtg agttaggttt tttgggtggg 113520
gttgtgatga gtaggaagga ttttcggagt gtttgggatt ttttgggttt agggttgtta 113580
gaggtagaga gaaagaagtt tttttttaat atttgagtaa agatagttat tagaatttat 113640
tagagtggtt ttttgtggtt ttttttttggg tgttgatttt tttggtgtat ttgaagtttt 113700
tagggatatt attgacgttt tttgtatttt tatttagggg ttagtggaaa tggttttttt 113760
ttttttttgtt tagaggtatt gtggatgatg attttaggtt ttagatttta gttattgggt 113820
tacgtgtgta gataagtttg ggtagttagt aatttttggt tattaagttg gtttttttgcg 113880
gattgggtcg gtattggagg cgggttttag ttgttagtat tgtggtttag ttaggtttgg 113940
ttttgggaag gtagaggggg aggggattgg gaaagggatt gagttggggg tggggtggta 114000
ttttggtttt tttttttag attttggta ggtttgaatt atttggttgc ggttttttaa 114060
ggagggagcg ttttttcgt tttgatgtgg attttgtttt ttgtttacgt tgtttttgtt 114120

```
tttaaataaa tgtggttttt tgtggggagt ttgggggtcg ttttttattg atattgttgt 114180
tttttggttt ttttttgtag gtttcgttta gttttattgc gtttattttt aagttttttt 114240
ttttggacgg ttttgttttt tgagaatata ttttttttta ttattttaat tgtgtttttt 114300
tttttattcg gagtttgttt tatttgtttt aggagttatt tttttttttt attaagtagt 114360
ttaggttaat tttggagggt taatttgtgt ttttttagaa gtaggttttg agatagggat 114420
tttagtggga ggggatatta ggggatgttt gagtgtggga atgggttagg tagggaattt 114480
agttaataaa gggtgtttta ttagtttatt gtgattatag gtggttgttt agtttttata 114540
tcgggtagta tggggagatt gtagatcgtg tttttagttt tgttttattt ggggttgggt 114600
aagggagttg gggtttttat attttagttt ttggttaagg attgtattgt ttattttttt 114660
tagtttttcg agggtttggg tattaggaaa ggtttgggt ggagatacgg aaataggtta 114720
tgtgaagtta gtgggcgtga ttggtagtag taaggttagg atagttttgg ttattagtaa 114780
ggtgattata tattttagta tgtatagttt ttatagatat atatttagga gatggtaatt 114840
atattagtaa tagttatttt tgtcgcgtgt tatgtttgta tttagggaat tttatttttt 114900
atttatttat ttatttttat ttgagatgga gttgcgtttt tgttgtttag gatagagtgt 114960
aatggtataa tttcggttta ttataatttt cgtttttttgg gtttaagtta ttttgtttta 115020
gtttttcgag tagttgggat tataggtatg tattattacg tttgattatt tttgtatttt 115080
tagtagagat ggggtttttt tattttggtt aggttggttt cgaatttttg atttaggtg 115140
atttatttat tttagttttt taaagtgtta ggattatagg tatgagttat cgcgtttggt 115200
tttaggggta ttttattatt ttattatttt ttatagtagg tgatattgag gatattgagg 115260
tttagagtag attgtggtta tgttattttt ttgtttggtt tttttgtttt tagttttgaa 115320
ataaagagta cggtttttgt gtaggtttat acgttttgtt tttagttttg gagtagtttt 115380
taagtttttt gaaggtatat taggtttttg ggtttcgttt tttaagtttt ggataaatag 115440
gaggtgaagg agtttttgta attggatttt atttgttag cgttaaggtt ttgtttgatt 115500
atttatttta ttttatatag attagggatt tgtgttaagg tttagtgtta taggagagtg 115560
aagataagga ggtgtttatg gcgtatatgt tgttggattt attttgtaga ttttaagggt 115620
tatggggagt tatttgttag gtgaggtttt ttcgtggttt ttttttgtag gtttagggta 115680
gtatataggg aggtgttttt ttatttttttg gtattatttg ttcggttttt ttgagttttg 115740
attttggttt ttagatagtt agtgtttttt tagggataga atttgttttg ttgttgttta 115800
gaggagattt agtttgatta gggttagcgt ttttggaatt tgagtttgga attttatttt 115860
gttttgggta gaggttacgt tgtttttttt tacgagagtt gagttgcgta tgagattatt 115920
ttgattttgg gtttattat ttttttgtat gatatttgtt ttaggttgga gtagggtttt 115980
gtggttcgtt tggtgttttt tttttagatt gaggtttgat atgtatatta ggaagtgttt 116040
gatttggttt taggagttgg gagcggtttg ttaggtagtt tgtggttaga gtaggggttt 116100
taataatggt tttttgtgcg gtatattttt ttttaaatag gtttaggttt aggttgtttt 116160
tgtttcgatt tcgttatggg tttggagggg aaagtttgta tttggagggt ggtttattgt 116220
ggtttttttt tgtgtagtag ttgagatcgt aggtgtttga gaataggaga agttttgtgt 116280
ttattttggg ttgtgagatg ttttttttaga ggtggggatg gggatgggtt agtatgtagt 116340
tatttatatt ttttagtttg ttggtattaa ggtttgtgta tgtgtgtgtt tttttttttt 116400
taattttgtt tttttaatat ggagttttgt tatttgttta ggttggtttc ggattttttgg 116460
tttaagcgat tttttagttt taggttttta aagtgttggg attataggtg tgagttatta 116520
tgtttagtta gtattaaggt gtgtttttta agagtaatat tggtcgggtg tagtggttta 116580
cgtttgtaat cgtagtattt tgggaggtcg aggtaggcgg attatgaggt tagtagattg 116640
agattatttt ggttaatacg gtgaaatcgt gtttttatta aaaatataaa aaaattagtt 116700
agacgtggtg gtcgggtatt tgtagtttta gttattcggg aggttgaggt aggagaatgg 116760
cgtgaatttg ggaggtggag tttgcggtga gtcgagattg cgttattgta ttttagtttg 116820
ggcgatagag cgagattgtt ttaaaaaaaa aaaaaaaaaa gaataatatc gatggtttta 116880
tttttgaat atgttaagtg atttttatgt agtataagta ttttagaagg taggaaaatg 116940
taatgaataa tatttatcgt tatttatttt atttagagat cgttattttt tgttcgggtg 117000
tgagtgtgtg tgtgcgtgta aattgtatat gtgtgtatga aatggaatta ggattttgt 117060
atatattttg tgttttgtta tttttattta atattttcgg tattttttat ttttaaaaat 117120
gtgattttaa tgatggtgta ggtttttgat atatgggttt agaaaatttg cggaaattcg 117180
gattgttttt aggtttgtt agtttaatgt tgatgtaaat gttttcgtac gtaaattttt 117240
ttgtattttt gattattttt ttgggataaa tttttagaag tgggttaaag tatatgaacg 117300
gtttttaggt ttttggagta agttgagatc ggtggggatt ggagaaggga tttagggagg 117360
tttttgggag tttgcgagat tggagtttgg ttttagggga tttgaattta tagatgaagg 117420
agatggttgg taaaggtggg aatttttttgt tttatgggtg ggggggtgat attgttttat 117480
tttttagtga tagtatgggg attttttttaa tatgatttgg tttcgtattt ttttgatatt 117540
gtgtacggtt tatagttgtg atgtttagtt gggagttttt tttgtgtttt ttagttgtat 117600
```

taattttttg tagatagagg aggaagttgt tcggtgtttt tttttgttgc gtgggttttt 117660
tgaatttgtt tagttgggtt tgtggatttg tgtagttacg tacggggttt taaaatttgt 117720
aaaatgataa gtttacgaat attttcgttt ttgtgggtga atgtggatat tttttggttg 117780
cgtttttttt tatagggaag atggagacgt gtaaagtgat aaggatttgt tatttttggg 117840
taggttatgt cggttttatt gtgaggatgg tggtaggggg tttttttagga ggtttgtata 117900
ttgggaagag aggggcgatc gtgtgtgatt gtaggattta gggcgtagtt ttaggatttt 117960
gatggggggag gagatgttgg ggagttttgt tttgttagtt tggtgataga tattttttaa 118020
tgtttttcgg ttgggtataa tttataatgt ttttcgtggg gaggtaattg tttatgaggt 118080
atttttggta gggatgattt tgttttattg ttatatattt tagtaatgaa ataagaattt 118140
agagtgatgt gatttttttcg atgatttttt taattatttg gggttttttgt gcgattttaa 118200
gaagggggaa ggaggtagtt ttcgtttttt tgggtttttg atgtggtagt tttggaatgg 118260
ttacgtgttc gttggtttat agtgggttat ttgtcgaatg tggttgtggt gggattgggg 118320
ttgggttgga gagtgggtga ttatattagt tagttttatt ttgggttatt gtgtagttag 118380
gggtttttatt tttttagggg tttcgttgtg agtgttttgg ggttgttgga ataaattatt 118440
ggaaattaag tggtttaata aagattgatt cgtttatggt tttggaggtt ataagtttga 118500
tattaaggta ttggtaggat tatatttttt tcgatagttc gaggggagga tttttttttg 118560
ttttttttag tttgcgttgg tttaggcgtt ttttggtttt ggtagtattt gtagtttttg 118620
tttttattac gtagtttttt ttatcgtgtt tttttgtgtt ttaagttttt ttttgttttt 118680
tttttataag gatatcgagt attggattta gagtttattt taaatttagg atagttttat 118740
ttcgggattt ttaattatat ttgtaaagat tttttttttta aataaggtta tattcgtggg 118800
tttttggggt taggtattag atatgtttt.ttgggggtta ttagttaatt ttttatagaa 118860
ttttagacga aagtgaattt ttataggttg aaatgggggt gaaggtttag tgcgtttttt 118920
ttttttggga gttttttttag ggtttgtgtt tattttgtga ttttgtgatt tagttttttt 118980
tttgagatga agttttgttt tgtcgtttag gttggagtat tgtggtacga tttgggttta 119040
ttgtaatttt tgtttttcga atttaagtga tttttttgtt ttagtttttt aagtagttgg 119100
gattataggc gtttattttt atgtttagtt aatttttttgt gtttttagta gagatagggt 119160
tttattatgt tggttaggtt ggtttcgaat ttttgatttt aggtgattcg tttgttttgg 119220
gtttgttttg gtttttttaaa gtgttgggat tataggtatg agttattacg tttggtattt 119280
agtaggtttt ggttgttttt tttttttgag atagagtttt attttgttat ttaggttgga 119340
gtgtagtggt gtgattatgg tttattgtag ttttgatttt ttggttttaa gtgatttttt 119400
tattttagtt tttcgagtag ttgggattat aggtatatgt tattattttt ggttaatttt 119460
tgtattgttt agtggagtcg gggttttgtt atgttgttta ggtttgtttt gaatttttgg 119520
gtttaagcga tttgtttatt ttggtttttt aaagtgttgg ggttataggc gtgagttatt 119580
acgtttggtt tagtaggttt tttagaagcg tttgaggttt ggtgaaggta tggtgttatg 119640
gagtagagaa tatttaattt tgttttcggg gagtcggaat atatttttcg tagttaattt 119700
taatagttat gacgtggtta ggatgatatg gggagggggt ggtgtggatt gtgtgggagg 119760
ttagtttttc gggaaggggg ttggagtggg tttggttttg aggaggattt ttaagaggag 119820
gaggaggcgg tggtatgttc gtttgggggg atggcgtgag ttagggtggg tatttttttta 119880
tttaggtgtt atttttttga tattttagtc gggatatggt tttttttgggg aagatggata 119940
ttttagagt ggggtatttt ttggggaaga tggatatttt cggaatgggg cgtttttttgt 120000
ttttattgtt tttttagtat atagttgtat tttttttgtag ttttggaggt tgtgagattt 120060
tttggttgtt atttagaata aattcgtttt tgagtagcgt cgttttgatg gtggggtggt 120120
ttttgcgggt ggaatagttt ttgttgtttt agagtttatt gagagttagt gggtatggga 120180
gtggtgttgg atcgaaatat ttagagggat tatagagtta ggtagtaagt atagaggtta 120240
gttagtgggg agggagggat ttatttatga aaagatatta agtattgaaa gagaggagag 120300
gatggttggg gatcggtata tatgtgtatt ttgcgtagat ggttaaggtg gtgaggcgtt 120360
agttacgtag ataattggag gaaggaggag tgttttaggg gaggggttag tttcggtttt 120420
agaatgattc gtttgtgtac gtgggtttgg ttggaataaa ttattgtatt cgtatcgtag 120480
atttaatcgt aagtttatat tagggtagtt tttgttttgg tgtagtgttt tcgattatga 120540
gttggttatg gtgggggtga ttttaagggg atttttaagg agggggttta tcggattttt 120600
tagggaaggt tatttgtttt tttgggttgg atttgggata aggttttttt ttgtttagtt 120660
tttgtttttt aatttgatcg tacggaagga tgttgaggat atttggaagt cggtttttgt 120720
tttatagtat ttataggata gaggtatatt atgtggggat agtttggata gtgtttagga 120780
ggtagggagt agattagagg atcgcgggag gttttgggaa tcgtacgagg atttaggtat 120840
tgagttttgt atttttttggt attagttcga tgtatagttt ggttttgtgt ttatttcgta 120900
ttttattttt tttatttgta cgggggcggt aataatattt tttatagttt aggtttaagc 120960
gggtgtagat gtgatgagtt tggtaagttt atttattat atttagtttt gggtattgaa 121020
ttttggattt tttggttggt agaagtggat atttaatttt tttttggggt tttggggttt 121080

ttgtagaatt agaatatagt tagtagtttg tatgtagggt gtagagggat agagggatat 121140
tatgtcggga gtttatattt ggtatatttt tgattttggg gtttagtgtt ttgttgtttt 121200
gcggttacgt gattagttag aggttatggt tggatttata ggcgaatata gtttgtggat 121260
ttgattttgt ttggttttgc ggtgttttat tttattttta gttttagttt gtggagatag 121320
agtttcgttt tgttagttag gttgagtgta gtgatacgat tttagttttg taatttttgt 121380
ttttcgggtt taagcgattt ttttgtttta gtttttttgag tagttgggat tataggcgag 121440
tgttattatg tttagttaat tttttatttt tagtaaagat ggggttttat tatattagtt 121500
aggttgttta gtgttttaaa aagggtttgt tttgttttgt gttagtattt aaagattagg 121560
agatattata taaagattta gatttttatt tttttgagaa gtgaatagtt ttggttacgt 121620
cgggtttata ttttttaggg tagtgacgga gttgagatag ggtttgtgat aattgagagt 121680
tttttggagt ttatacggtt ttttgtttag tttttggtgg aggttgagtt tagtgattta 121740
ggatttcgtt tgtgtttatt aagaaggaga ggtgtttgtg gtttagagag ggcgtttata 121800
ggagtgtttt gtttaggaag ttggtagttg aggcggttag ggttaggggg ttgggttttt 121860
ttttttttt tttagtgttt gagtgtattt ggaggaagag agacgggtga ggggaaggta 121920
ttgaaattgg ttttggagcg ttattattgg gggttgggga ttagttgtta ttttttttt 121980
tgtgggtagg ggaggtagtg gtgggatttt atgagattcg gatttgtgg tttggtgagg 122040
tagggagcgg ttggggagat attgtgggtt tttatagggg tgcgttgtgg gtttggtttg 122100
gggaaggagt tggtgaatgt gtttgttatg tgggtttttt tttgttttgg ttttgataat 122160
taggagcggt tggtttttat ttggagtgcg gttgtgggga ttgtggaggt ggtagttttt 122220
taggtattat ttttattgtt ttcgagggag tagattttgg tggagaggta ggatggttgt 122280
agggcgggag ttggtggttg gtattattgt acgggtttgt agtagtggat gggaagatgt 122340
aggagaggtt gatgattgag aaagttggtt tttgtggatt aagttggggg agtcggataa 122400
ttaagtggtt agaattttgg gttttggtat tagtttgatg tatagtttgg ttttgtgttt 122460
atttcgtgtt ttattttttt tatttgtatt gggggtattg gtatttttta tattttaggg 122520
ttaagcggtt gtagatgtga tgagttcggt aggttcggcg tttcgtcggt attgaaagtt 122580
ttagtgttcg tttttagtgt tattgatggg gttttgtttt tgttgcggtt ttgattggtt 122640
atagttgcgg ttatttatta ttggaggtat gtttttcgtt gtggttgatg gtagaggtgt 122700
gtttgttggt ggtgttttag tgtgtgtttt ggttggaggt gaggagggat aaaaatagtc 122760
gcgataagaa ggaaattaag gtcgggtacg gtggcgtacg tttgtaattt tagtattttg 122820
ggaggtcgag gcgggtggat cgtttgaggt taggagttcg agattagatt gattaatatg 122880
gtgaaatttt atttttatta aaaatataaa aattagttag gtatggtggt tcgtgtttat 122940
aatttagtt attggggagg ttgaggtagg agaatcgttt gaattcggga ggtgtcgttg 123000
tattttagtt agggtaatag agtaagattt tattttaaaa aaaaaaagaa ggaaataaaa 123060
gttttttttg gaattagaac gtttttatgt agacgtagtt ggagggggag gttgtgtggt 123120
ttgcgtcgtg gttatggtaa gatacgacgg gtagtatttt tttattttag tttgtatttt 123180
tgttttagaa tttatgatgg ttttttttagg atggggttgt ttttttcgtt tgttgagata 123240
tgaaggacgg gaagagattt tcggttttta tcgtcgtatc gtttgttttt ttttgttgtt 123300
ttttagtagg aaatatgtcg gagtgttttt tagttattta tttattaatt gtattttttt 123360
tttttatttt ttagttttga aaagattttt tgaggtcgag gaggtcgaga tatttaattt 123420
tattttattt cggagggttt agattttttt atttcgagtt attgtggtta gttttattta 123480
gaaattttag gatttgggcg tgaagaattt agaattttcg gttcgttatg tggatttttt 123540
aagttaacgt tttttaagg cgtttttgcg gagggtggag ttttcgggtt ttaaggcggt 123600
cgagtcggtg tttcggcgta ttgagttgtt tattgatatt tcgtttaagt aggtggagaa 123660
cgtcggggtt atcggttcgt ttcggttcgg gtttaagagg gtcgaggtgt tgggttataa 123720
gacgttagaa tcggtttttc ggaggacgga gattattatc gttaaatttt aggagttagt 123780
ttatcggagg atggagtttt ttgtttttaa ggttttcgag gtgtttattg tttttgttat 123840
cgacgtagtt tttaagaggg tggagattta gatgtttaag tttgttgagg cgtttatcgt 123900
ttttagttta gtttagattt tggagaattt agagtttgtt tttgtgtttt agttgtagag 123960
taggttggag tttaagtttt agttttttgt ggttgaggtt atatttcgga gttaggaggg 124020
tgagtcgtag agcgttaggt tttggatgtt gtggtaatgg ggggtttggt tgttggtttt 124080
gttatagaat tttggaggaa gaagttggat atggggtgga gggtgttatt ttggagattt 124140
agaaagatcg gaatgtatgg gggtgggggt ttgtaagttt aggagaggtg gtttttttg 124200
tttgatggga atatgttaag atgttttaag cgggatttga atgagagttt ggaaagattt 124260
tttgggtttt aaggagtttt ttttatgggt tatcgtgggt tttgttggga tttttgtttg 124320
ttgtgtgttt ttggagggtt tgggaggttt agagaatgag gagttacgta ggaattaggg 124380
taggtttatg taggggtgta ggatagggtg gagttgagat ttttagaagg gttgttatat 124440
tttggtggtt tttgtttgag gaattgtagg ttgtttggat atgtttagta gaggtcgagg 124500
gtttggggaa gggaggaggg acgtatgttt tttgaggttt gtattggagt ttatggtttg 124560

gtttttttt tttaggaggg atggttgtgg tagagggagg ggaatatgag gagtatttta 124620
gtttgtagga taaaattttt agggtattgg gaaggggtgt ttggattagg gggttgtgtg 124680
atagttgtat acggttttat ttgggggtgt ttttttggcg agaggagaga ggttatgggg 124740
ttttagtagc gttttgggag ggtttaagtt tatagtcgtg ttgatatgga gtttataggt 124800
aggggattgg tgttagtttt gtgggatata gggtttgttt ggaaatgtta ggttgtggga 124860
tatggttggg ttttttttta gaagcgtgtt ttttggtttt agtagggatc gtggttttat 124920
ggggttatag taaggtgtta gttgggttag aagtttttta agagttaggt ttgattttgg 124980
gtataggatt agaatttagt aatgtttagt aagtttttttt taggttggat aaatagtata 125040
gtttgggttt ttgatttagt tgttgttttt tatttttaat cgtggtgaaa tatataatat 125100
ataatgtgta attttagtta ttttgtagtg tacgttttag cggtatttag tatatttacg 125160
gtgttgcgta gttgttatcg ttgtttattt tcggtatttt ttttattttt ttaaattgag 125220
tttcgttttc gttagattaa gtttttatg ttttttttt agttgttggt agttattatt 125280
ttattttttg tttttattat gaatttattt gattatttta ggaatttttgt gtgtgtggaa 125340
aaatataata attgtttatt ttgtgtttgg tttattttat ttagtatgac gttttaaagg 125400
tttattcgtc gtgttgtagt atggggtaga atttttttt ttttagaggt tgaataatat 125460
tttgttgtgc gatgtatttg tgttgtgtag ttgttttttt atcggtggat ttttgggttg 125520
tttttatatt tttttaagtt tttttttta ttttttttga ttttttttg atttttttga 125580
gatagggttt tgttttgttt tttaggttgg cgtgtagtgg tgtgattatt gtttattgta 125640
atttttgttt tttgggttta agtgattttt tagttttagt tttttaagta tttaggatta 125700
taggttgcgc gttattatgt ttggttaatt tttaaatttt ttttgtagag ataggttttt 125760
attaggttgt ttaggttggt tttaaatttt tgggtttaaa tgagtttttt gtttcggttt 125820
tttaaagtgt tgggattata ggtatgagtt atttaatttt tttttggttt ttttttttga 125880
atgagagttt tttagtggtt agtgggtttg ggggttggga ggatgtgggg tttggagagg 125940
gtgtgggttt ggggggagat tagtgttttt tatgttttta tgggtatgtg gagtgtatat 126000
ttgttttggc gggtttgggg tttgggattt tgtatttttt atagaggggg tatcggaata 126060
tattgagtag ttagaggtta gtgggttttg gaaggttttt ttcggttttt agtgtgcgag 126120
tttcgtgttt ttttttttat ttttttaagt ttgattttga agagttattt tgttttagtt 126180
atttagattt agtgatagtg gttgttggtg gtatggtgtt aggttaaagg tatgggtagt 126240
ttgtagagtg gtaggaggtt gttttttttt ttgtttttttg ttggtgtagg atttttgttt 126300
gacgtgtcgg atatattttt attttgggtt gatgtgttta tatttagttg agttaaatag 126360
gatgtggttg gggaggcggt tgttatcgag ttatttaaat ttcggtgatg gttggtgttg 126420
gatgtatagg gacgtggttt tggttttggg ggataggtag ggggatgttt atggggatgt 126480
ataagaatat tttttttttag gggtagatat agtttagttt ttaaattgtg ttagagattg 126540
tgtcgggagt taggtttagg gatataattt gcgggttttg tttttttggag tttatcgagt 126600
cgtgagtagg atggttggga tataaggagt gtttagggag ttttttttagt agtggaagta 126660
taattttatt aggttaggaa ggggagagaa aggtaggtta cgtggaggta gtagcgttta 126720
gagttgttag gaatttggcg ttttgggggg acggtggttt tttttatgga ataggatgta 126780
tacgttggag tcggggtatg ggagaaaatt attagtattt tagattcggt tatttacgga 126840
tagggatttg tgtaggtggg agtttagttt acgtggagag gttcgtgggt tgggttgata 126900
gttttgaggg tttaagtaag tttaggtgga ggaaatgtag ggatagacga gggtagggtt 126960
tttttaatgg aggagtgttt ttgaaattcg atgggagtgg tttttcgggg ggtttaggtt 127020
tgagttttat tagttttttt ttcgaggtat tatttgtttg ttttttcgtt ttttttttt 127080
gtgttacgat cgttttggat acggatttag ttttttttaa tttagttttt tgtttatttt 127140
ttttttaggt ttatttttt ttgttttttt atgtggggtt gggttgggag tttttgagt 127200
ttcgatatgt atagttttga ttaattttg cggtttttt gtttttcgag tattagggat 127260
ttagttggta ttatggggaa aggaattatg gagggagttt cgtgtttttt gagtagacgt 127320
ttagtttttt ggtttttgtt tgcgggaggg tatttgtgtt gtggtttgtt tttattttag 127380
agttgtgggt gtagggtagg gtttttttggg gttttagttg aattgttttt agattgattt 127440
ttttaaagta tagtattgtt gagattttttt ttcgtgttga gttttttttg tttatttatt 127500
ataggttttt ttacggttga tttatttttt tgtttagttt atattttttt ttttttttagt 127560
ttttttttgtt ttggtttttt tttggttttt tatattttttt tttataattt ttttgttttg 127620
attttttgaa ttttgatttt tgtttttttt tatattattg ttttggtgaa tttttatgta 127680
ttttttaaaa tttggtttaa atatttcgtg tttggtttgg gatataaatg atttgtgtat 127740
aaattttttt ttttttgtta gatttttttt tttaaatgtg aaagtagttt tttagattat 127800
aaaaagtaat cgagtatttt ttttttaaaa tgtaggaagt atagaaatga gaaagaattt 127860
aaaatttatt tgtaagttag cgatatttat gtttgaggtt ttgttgtata gtttttaaaa 127920
tttttttttat gtttttatta gatttgaggg gtagggttgt tgtttttgtt ttttttttgtg 127980
ttttttttt gtttttgtt ttttgagatg gagttttttt ttattattta ggttggagtg 128040

tagtggtatg atttagttt attgtaattt ttgtttttta ggtttaagcg attttttttgt 128100
tttagttttt cgagtagttg ggattatagg cgtgggttat tacgtttggt taatttttgt 128160
atttttagta gagacggggt tttattatat aggttaggtt ggtttcgaat ttttgatttc 128220
gtgatttatt tattttagtt tttttgattt tttaaagtgt tgggattatg ttatcgtgtt 128280
tagtttgttt tggtttttat aaagaatttt ataaggggtt aggtgtagtg gtttatgttt 128340
gtaaatttag tattttggag gtcgaagtag gtagattgtt tgagtttagg agtttgggat 128400
tagtttgggt aatatagaga aattttattt ttataaaaaa tataaaaaat tagttaggta 128460
tgatagtatg cgtttgtagt tttagttatt ggggaggttg aggtgggagg attatttaag 128520
tatgggaggt tgaggttgcg gtgagttgag attgtgttat tgtattttag tttaggagat 128580
agagtgagat tttgttttaa aaaaaaaaaa aaaaaaaaaa aaaattttat aagatattaa 128640
gtaagtgatt tggatataat aggtgtgtta tgaaattatg ttgaaggagt gagttattgg 128700
tggtgagttg ggatggtttt tttgtggttt ttgtaggtag atgtagtgat ttttatgggt 128760
cgtttttaga gattaagtag aaattttttt ggttaatttt atttttagga tatttggttt 128820
tttttttgga ggttgtgttg gagggtttag tgggtgggtg ttggttttag tgtgggggtt 128880
ttggaaggag ttagtatttt aggagggagt gtgggtattt agatggttga aggggaggga 128940
gaggaagaga ggcgttgtag agggtagagg tatttttgtt tgttttttcg ttttagggtt 129000
tggtgaggtt tgagttttat gttggtgggg tggattttcg gttttcgagg ttttggtttt 129060
gtttttggat ttggtatttt ttttgttgtt tttttataga agggcgttta ttttttgggg 129120
ttatttttgg ttttttttagg ttttgggtgg ggacggtttt gttttttttt ttggattttt 129180
gttttttttt tgttttttaa tatgggtgtt tttgggtgga gtggggtggg gttgggttgt 129240
agtatttagt attgagtttg taagggttgg ttgttggttt agagtttttt ttattagagt 129300
tatttttttt cggtttttta gttatattag ttaagttttt ttagagttgt tttataaggc 129360
gggtgtttag cgttcgttta tgggtttggt tttttgtttt tttgggatgt ttattggggg 129420
taagagggag gttaggttta gtgtttagtt tagttttggg tatgttttag ttttgtttta 129480
tgagttaatt ggttttgaat tattatttta ttttttttag ttttaatttt ttgatttgtt 129540
aaagaggtat ttgtagaggt ggttgggggt tgagttttta tgtatttatt tattttaaga 129600
attaagaagg gagtttatag tggttttggg gttatttgtg aatatggatg tgaaattggg 129660
gcggggtaga tatggtagta tgttttagta gtttttttgt gatttagagg gtttggaaat 129720
ttttgttatg tgggtggagc gttgagggag tagggggtta ggtttttttcg agttgatgat 129780
ggtgggcgtg gtttttcgta tatttggttc gtatcgggtc gtgtgtttta ggggtagaat 129840
agggaggaat tttagtatag atagtagggt tgttagtata gcgttgagta tagggttaat 129900
taaagtatag ggtttttttgg aattatttaa tatagagtag tgaagaggtg gttttgggga 129960
aagtggggtt agagttgagt ttgagtgggg ggtggggagg gcgggtatta gtttgtaagg 130020
gagggatatt tattgggttt tagaagtttt tggtggggat tagggattgt gaagttgggg 130080
agagggtgat tattgtgatt tagggaggaa gggttttata gatagtttag tttttggttt 130140
cgtgtttata ggatatttta tataggtttt gagtttattt ttggattgtg gatttttttga 130200
ggtagggtgt gtaggttcgt ttagtattag gtgtaaggta gggggttttg gtaagcgtcg 130260
agggatttat tggtgtttga tgaagtagtg agtttggatc gtgttatgtt tcgggttttg 130320
agtttgtagg atttttttgt ggggttgtgt gtttttgttt ttggaggtgt ttagaaggta 130380
ttgacgtttt tgtcgggatg ttttattcgg aaggtttcgt tgttttttatt tttttttatt 130440
tttttgagtt gtttgtagag ggtatttagg ttttttttta ttttgggatt gggttttttt 130500
tttttttttt ttttaagaat aagtattttt ttttttcgaa ttgtttttgg agtttttttag 130560
atttaggttt agagtgggaa gtttcgtatg tgttggggga ataggaatta atgcgggagg 130620
attggttagg ttcggtgtgt ttttttcgtg gtgggacggg gtggggttag agaatagagt 130680
ttggttttag agtttaggaa gagaattttt tttttcgtcg gtttgttaag tatcgtgtag 130740
tggtgagagg aaaggagggt tggttcggga atgtagcggg tgtcgagtag ttgtggaata 130800
ttttaaatag agcgtagttg cggggaaggg tggggttagg aggtaggcgt tgggggcggt 130860
tttttttgttt cgtttttttgt agtttgtgg ttttgattat ttagtttggt tttaggatgg 130920
agtttcggtt tttagatata tggtattggg tgaggtcgtt ttttagtatt tgttttagga 130980
agttagagta gttgttagaa gagagaggag ttgtgtttgt attcgtcgtt ttttaggttc 131040
gggatagttg tcgggattga gggtttgtat aatggcgagt ttgttcggag gggcgcgtcg 131100
cggttagata gttatgtttt gtgcggtgtg gatagagggt atatttagtt ttggaggagc 131160
gtatttagat ttttcgtttc ggggtttaaa gtgtgggtag tttttaatta gattttggat 131220
tttagtagat agtttgggag gatgttatgt ggtttatttt gatatattgt tttatttgtg 131280
attttatttt tgagagttgt agggaggcgt ttttttagtt ggattttatt tttttttcgg 131340
ggttttttgt gggtgttata agtgattttg ttttttttag tttaaggggg gtttttgggg 131400
tatgggttgt aattgatagg ttagagtagt ttatggttag ggttgagttt tttttttcgtt 131460
tttttttgaat attgggttta ggaaggggtt aaagtggaga tgttttgatt aaagttgagg 131520

gaggtatgga gttttttga ttttgttttt ttattttatt gttgttattg ggtgtatttt 131580
gtggagtttt aggattttta aagaatttag agtggagatt ttcggtttta tttagtgtag 131640
gtgaggggga aagtgatttg tttatagtta tatggttatt attagtatat ttgggttaag 131700
ttcgatttta ttttacggtt tttgtttgaa atttggaatt ggaaatggtt ttggaaatta 131760
ttgattttat ttgtcgtttt ttaaaatggt tcgtagaggt taagatatag atttttgtaa 131820
tgttttgttt attcgtgtgt ggggaaatgg tagaggagta tcggttttgt ttttatattg 131880
tttgggttgt cgtttttgtt ttgtttagtg gttttttttt ttggttcgtt tttttattgt 131940
ggaagtgggt ataatttttt ttgtgtagcg tggttagagt ggatagtgat gattcgtgaa 132000
gtagggtgga gcgtttgggg gattcgtgtt aaataagtat ataaataaaa gttaatataa 132060
tttttattta attagtaagt aggttttgtt tgaagttttt gggtagtatt ttatgttatt 132120
tttaggtttt aggtcggtgg tgtcgtttcg tgtttttttt attcgtaagt tggtgtttag 132180
aagttttgtt ttgtgttttg gtttttattt tttttgtgat ttttttttt ttttatttta 132240
aaaagtaatg tacgacgggg cgtggtggcg ggtatttgta gttttagttg tttgggaggt 132300
tgaggtagga gaattatttg aatttaggag gcggaggttg tagtgagttg agattgtgtt 132360
agtgtagttt agtttgggta atagaataag atttttattt gggaaaaaaa aaaaaaagcg 132420
atgtttagtt atttttagat agaaaatgaa gattatatga aatttggaaa gaagtttttt 132480
tttttttattt tttaattttt ttttaagggg tggttatgtt gatagttggg tgtggtattt 132540
ttttgaattt tattttttgta gagttattta gggtttgtgt aaaaaaagtt aatgtgtaaa 132600
tatagatttt ttaagaatat aaaggaaatt agattttttgt tattagtttt tatttttttt 132660
atttaataat attgttgtta tttgtagatg tagtacgttt ttttttttt ttttttttt 132720
tttttttga tagagcgtgg tttaggttgg agtgtagtgg tgcgatttta gtttattgtg 132780
gtttattgta attttttattt ttcgtttttc gtttttaagc gatttttttg ttttagtttt 132840
ttaagtagtt gaaattatag gtgtttgtta ttatatttgg ttaattttttg tatttttagt 132900
agagacgggg ttttattatg ttggttaggt tgtttttgaa tttttgattt tgagattcgt 132960
ttatttcggt tttttaaagt agtacgtttt ttttaatggt tgtgttatta gttgttttat 133020
ggttttgtta ggtttgttta gtttttttatt gtgattgaga ggttgtaata gatgttttcg 133080
tatttgtata tgtgtatata tgattgaagg atgagttttt agtgaatttt tgtgttaagg 133140
atgtgtgtat ttaagttttg atttgttggg tatagtggtt tatatttgta attttagtat 133200
tttgggaggt taaggcgggt tgattatttg aggttaggag tttaagatta gtttggttaa 133260
tatggtgaaa ttttattttt attaaaaata taaaaattag ttgggtatgg tggtttgtgt 133320
ttgtagtttt agttatttag gaggttaagg taggagattt gtttgaattt agtaggtaga 133380
ggttgtagtg agttaagatc gtgttattgt attttagttt gggtgataga gcgagatttt 133440
ttattaaaaa aaaaaaaaaa aaaaaaaaat tgatttttgt ttttgaattg gtttttaaag 133500
tggttattag aattttttag gatttttttt ttcgtcgatt tgtttagttg tttgttgggt 133560
tttgtgtagt gatggtttag tagggaggtt ttatgttttt tatttttggt tgagggttaa 133620
atgtttttta attggtgttt tattttgtgt atttcggtta agcgagaggg attatttttt 133680
cgtatgttat aggttagggt tttgtgtttt ttttttattt ggaaggtgta tttttttttt 133740
gttgaaattt tttaaaaaga gtttttttgta agttaggcgt gatggttaat atttataatt 133800
ttagtatttt gggaggttga agtgggcgga ttgtttgagt ttaggagttc gagattagtt 133860
taggtaatat ggtaaaatcg tattttttgta aaaattagtt gggtgaggtg gtgcgtttgt 133920
agtttcggtt attcgggagg ttgatgtggg aggattattt gagtttggga gtttgaggtt 133980
gttgtgagtt gtggttatat tattgtattt tagtttgggt gatagagtga gattttgttt 134040
aaaaaaaaaa aagagttatt tgtatatttt tattatagta ggtacgagta ttttgttttt 134100
agttgtttat ttttgatttt gtttttggga tgttttttta tatagaatat tttaattttg 134160
atattgttaa gtttttgttt ttttttttag tttttggaat ttatattttg tttagaggtt 134220
ttttgcgttt tgaaattata ggaaagaatt ttatttacgt tttttttttag tatttttgtg 134280
gttttatttt tatatgttga aattatgaat ttattggaat gtattttggt tttagtagcg 134340
aggtaggggt agatggtatt gtttggatat atagtagcga gtttggtttg gagcgggtgg 134400
ggcgtgtgtg tgtgtgtgtg tgtgtgtgtg tgtgtgtgtt atttttatta gttttggtgt 134460
ttgtattgtg ttggtttagt aaaaataatt tggatgtatt tttttttattt tttgtttttt 134520
ggaatatttt aagtagtaat ataataattt ttttgttaaa atttttttaag aatttattcg 134580
tgaaattatt tgagtttgat atttttgggg gagtttagta ttttaaaaat ttaaattttt 134640
tttatgggtt tgtgagtttg ggagtttttt ttttttttta aaatttatta ttattttttt 134700
tttagaaaag tgttttttag tttatttaaa attcgttagt ttatttcgag gggttgttt 134760
tagttcgttt agtgaggata gtgtgtagcg cggtgattga tatgattgat tagtagagta 134820
cgatcgatta gtcggtgtgt aaatatgatt gattgtggtt agcgttttgg gatgttttat 134880
gttgaggtat atgtgtgtag tgtagggtga tgtgtatttt gagaggggat gaagttatag 134940
gatttttgta tttttaggag agagtttttt tttttaaggt tgtagttttg agtaggtagt 135000

gacgggattg ggtttagggg gttgagtggt tttagtaggt taagggtttt aggaattatg 135060
agaagtttgg agttgtttag agagtagttt ttaaaattag tttggattgt agtttttaga 135120
aagatatgcg attgatatta cgaattagga tatatttata tatggggtgg gggtggggat 135180
gggatgtatt aatgaaatta aattttcgtg atggaatttt tatttttatt atgcgtgtgt 135240
tatatttagt attttttttt tttttttttt tttttttttt tttttttttt ttttttgttt 135300
gtttgttttg gagagaggga aggttttttt ttattttttt ttaaatagtt ttattgagat 135360
ttaagtaagt tattatataa tttattaatt tatttattta tttatttttt tgagacggag 135420
ttttattttt gtcgtttagg ttggagtgta gtggcgttat ttcggttgat tgtaattttc 135480
gtttttttggg tttaattaat ttttttgttt tagtttttcg agtagttgag attatagata 135540
tgtattatta cgttcggtta attttgtatt tttagtagag acggggtttt tttatgttga 135600
ggttggtttc gaatttttga ttttaggtga tttattcgtt ttagtttttt aaagtgttgg 135660
gattataggt atgagttatc gcgtggttta atttatttat ttaaagtgta taattttatg 135720
gtttttagtt tatttagaga gtagtgtaat tatcgttatt ataattaatt ttagaaaatt 135780
gaaaatttga gaaatttaga aaattttttt tttaagaaga aatttcgtag tttttagtta 135840
ttaattttgt tttttttttt tatatttttt ttttatttgt gtggattttt tttttttttt 135900
tttttttttt tgagataggg ttttattttg ttatttaggt tggggtgtaa tggtttaatt 135960
tttgtttatt gtaattttta ttttttaggt ttaagttatt tttttattat ggtttttcga 136020
gtagttgaga ttataggttt gtgttattat gttcggttaa tttttgtatt ttttgtagag 136080
atgggggtttt attatgttgt ttaggttggt tttgaatttt tgggtttaaa tgattttttt 136140
gttttggttt tttaaagttt tggaattata ggcgtgagtt attgcgtttg gtcgatttta 136200
gatatttttt gtatagagaa ttgtacgttt ggtttttgtg tttggttttt ttatttggtg 136260
ttatgttttt aaggtttatt tatattgtgg taggtgttag tgcgtttttg agtaatattt 136320
tattatggta tggattattt tattgtggat agatatgttg tttttattag ttgagggata 136380
ttgggttgtt gttatcgttg gtgagtgtat ttttttttaaa ttaaaaaaaa aatgttggtt 136440
attatttatt agatcgtttt tttggtttat agggtgggat aagtcggttt gggtgagggt 136500
taggaggtgg ttaggtggag gcggttggag cggttttggg ttgtttttgg gcgtagtagg 136560
tagaggtgta atggtagagt ttgagtaggt tttttgggcg gatttttatg tcggtaatgt 136620
ttgtttattt aagaagtttc ggttaggcgt ggtgttttat atttgtaatt ttagtatttt 136680
gggaagttga ggtaggtaga ttatttgagg ttaggagttc gagattagtt tggttaatat 136740
ggtaaaattt cgtttttatt aaaaatataa aaattagttg ggtgtggtgg cgtgtatttg 136800
tagttttagt tattcggtag gttgaggtag gagaatttgt ttgattttag gaggtagagt 136860
ttgaatttag gaggtggagg ttgtagtgag ttaaaattgc gttattgtat tttagtttgg 136920
gtgatagagt gagattatgt tttaaaaaaa aaaaaaaaaa gaaaaaaaaa gaaattttta 136980
gtgttgtttt gtgttcggta tcgaagtcga gggaaggtag tattagtatg agtttttagt 137040
ttatcgggtt tttgtttgga gttgacgtgc gattaaggta tacgagggaa gtatggcgag 137100
gtggacggtg cggaggtata aggagtaaaa taaggtagat gtagtggttt agtggttttt 137160
ggtggttgcg aggagcgtgg gtaggatagt tttaggaggg tatttggaga gtagggtcgg 137220
ttgggggtaag gtgtgttagg tatttgttag gagtaaaatg taaggggata ttaaagagtt 137280
taggaattaa tatagatagt atttaatgta gtattttttta atatcgaaat gatgtatagt 137340
aatttttatg atgaataaat gttggaattt taaattaaga taggtttagt tttgtacgtg 137400
tatagttttg ttagagtttg tttagcgtgg aatttgattt gtttaaaatg ttgttttaaa 137460
atattgtttg atttttggat tttttggtat tgttttaatt tttgtatttg gacgaaggtt 137520
ttattttaat tttagttgga gagggttaag ggttggtgaa tttggttttg gatttgtaga 137580
gtttgtggta tttgagaggt ggggttagga ataggaagga gttggtgaga acggtattcg 137640
gtatgggatg gggttagatt agagtagtat tgtggttttt tttaattcgt ggttttgttg 137700
ttggatgttt ggcgttggtg ttgagttttc ggttttttga acggagtgtg atgaataacg 137760
gggtcgggga gtttttgggt ttttttttga agtagtagtg aagttaggga tatgtcggta 137820
gtaggtaggg agagggacgt ttggtttgtg tttggagtta gttgttttat ggaggagttg 137880
ggtgaggatg cggtgggagg aagatttttt tagttttgta tttttcgaag tataattgag 137940
tgtttgtgtg gtttgggggt tatttggtt cgaggatagt tttgttttgt ttagttaggt 138000
tttgaagagt tatcgggata cggatagtag ttttgttggg tggtaggttg tttattttcg 138060
tttatttaac gttggaattg gatgtagagt atttggatgg ttagttttag agtgtttttg 138120
atatttttag ttatttttatt ttttagatta ggataagatg cgggatttta atttcgagtt 138180
ttttatgggt tttattttat gtgtttttat aagttattgg gtttattttt tggttttagg 138240
gttgtttggt tatttggtat tttggttttt ttatttgtat aatgaaaata atagttttag 138300
ttttttcgag gagttataag atgtttttcg tgtgaaaaat tgggatttgg tattagatta 138360
tttggatttg aattttggtt ttattttttt taatttgtgg ttttgggtta atttggtttt 138420
tttgttttta gttttttat ttatagagtt atttattga gttgttggga ggatgaatat 138480

aaggaagtgt atttatgtgt ttagagtatt gtgattttat ttattattat taaacgaggt 138540
atataataag tgtttaatag atgtttagtg ttattttacg gattttgtgt gttttgtgtg 138600
tttttagtag ttaagttttg gaggtgggga agttgttgac gagttgtacg aggtaattag 138660
aaagaggagg ttataggtgt aagggtttgg gtgatatttg tggatagaga gaggtcgatg 138720
gattttggta gaggggaagg gggtttttgt atatagaaaa tgttttttgt atttaagatt 138780
tttgtgattt tggaatagag gtagtagagg tttgtaaatt ttaaattata aatttagaaa 138840
gtatagaata ttcgtagaga ggattagaaa tattatatat tttagtgtgt atagaatttt 138900
ttatatagtt aaataaaagt acgtttttaa ttgaagttgt tggagagttt ttgaggattt 138960
tatattagat aagggtcggt aagtggaagt ttaatacgta ggagttattt tttttgttta 139020
taaatgtttg tcgttttttt tgtttatatt cgtatttatg tgagggtatt tataggttta 139080
tcgtttagtt ttatgttttg tttgttatta aaaaattttt taagaatttt ttagttgtaa 139140
atttttttat cggagggggg gttttaggaa tttagttttg ttcggggttg ggtattttta 139200
gtatttgttg tataggcgtt ggttattaaa ggttatgtgt cgtattcgtg ggttttcggg 139260
gtttggtggt atttgggtat atagtttagt ggcggttttt tatttgttag atattgggag 139320
tgggggtttt tttattttg tttttggcg gtggggggag tgtttcgagt tgttatatat 139380
tagaatttat cgatttatac gttgcggtat tttatagttt tttgatagtt ttttagtttg 139440
agagattatt aaattgagtt tttggagagg gtggttgttg aggtttggat ataggtgtgg 139500
ataggatagt tttgttttat ttcggagttt cgtttcggtt tagttttttg tgggtttgaa 139560
gtttaggttg ggttagtttt tgttttttag atggtcggaa aggtagttta gttttacggt 139620
tttttgtta ggttttgaga gtcgtttggt ttttagtttt gtttttttt ttttttaaga 139680
tggagtttta ttttgttgtt taggttggag tgtagtggtg ttattttggt ttattgtaat 139740
ttttattttt tgggtttaag taattttttt gttttagttt tttgagtagt tgggattata 139800
ggcgcgtgtt attatggtag gttagttttt gtatttttag tagggatcgg ttttttattat 139860
gttggttagg ttggttttga atttttgatt ttaagtgatt tgtttatttc ggtttttttaa 139920
agtgttggga ttataggtgt gagtcgttac gtttagtttt tagttttgtt ttgatttttt 139980
tttaaggtcg tgaggatgtt tacgtttttt ttttttttta cggtttattg atgtttttag 140040
atgttttgta aagttatttt tttttcggga gtttttttagt tggtgacggg ggattttttg 140100
gggattattg tttaggtagt tatgtaggtt tgtgggtcgt gtttttaggt tggatcgaga 140160
ttcggtgtat ggggtgtttt tggtttgagt tgaggttttt ggaagttata gttaggaggt 140220
agtaagggac ggttgttatg tttttttttt tttgtttttt agggttttg cgggatagat 140280
tttttttgttt gagtttttttg gttgtttata ttttggtttt ttaaagttta gtttgaagtt 140340
ttaggtattt tataatttgt ttttttttagt agtttgtttt gttttttttt tgtcgatgtt 140400
tttgaaattt ttttgttttt ttttattttt tggttagaga gagattggag attttgtggg 140460
aattttcgag gggagggaga aggtcgttgt ttgaacggag gtggtttttt tttttgtttt 140520
tttttaggcg ggttatattt gtaggtttag tttttttgttg tggtagcgtt tggggtgtcg 140580
gttgtttttt gggtacgagt tgggagttga gggaggcgtt tttaggatcg gagtggtaat 140640
ttagttggag gaggagtaat aggttcgtgg ttagtagtag tcgtcgtagg gatttcggcg 140700
ttgtatttgt gtttttagttg attttttttcg tttttttagg gtgggtcggg gatggtggtt 140760
tgttgtttag tttaggaaga agaggggtcg taattagtag tagttatttt aggggatttt 140820
agcgttgtat ttgcgattta gttgattttt tttttttttt tagggttggt tgggaatggt 140880
ggtttgttgt ttagtttagg aggaagaggg gttgtggttt ttattttaga ggtttttttt 140940
tcggtttttt tttcggtgtt ggtttaattg taggtttgtt ttaggtcgtg tcggtatttg 141000
ttgattgacg tttagcggat gttgatagga gagggttggg atggggaagt agtaagtttc 141060
ggttattgtg ggttcgggtt atagatggtt gaggtatttt ttggttattt ttaggaagta 141120
aggttttacg tagttttcga tttatttatt gttcgtgtgt attttaggag gttgtatttt 141180
ttaaggtttt tattaggtaa ttttattggg ttgggtgtcg ttgtaaaatt atttattggg 141240
tgcggtggtt ttttggtggg gggtatggtt ggggaggatg cggtggagga gaagttagtt 141300
tcgtggttgg ggggagggcg gattattcgt tttttgtttg ttttttgaat tggagtttat 141360
ggtttttttt ttaggtgttt gggttatgga agaggttata gttttgtttt atttttgtgg 141420
tagtttaggg gagttttggt tagtttcgtg ttttttggga gggcgatagt ttggttaggt 141480
tgtagggtag gattggaatg tagatttttg tttggttatg ttgttgtttg gttgttttgt 141540
ttggggattt tttgtttagg aagagagaag cgggtcggat agttcggttt agtgattgga 141600
agtttagttt tttatgttta gtttagtagg aagtttgttt tttcggaata tttgcgtgtt 141660
tttgtatgtg tttttttggt tggtggtttt gcgaggtttt tattgggttg cgttatgttt 141720
tttggttatt ttggttttgg tgtttggttt ttgtgaaata gagatatttg ggtagttttt 141780
tatttgtgtt tgtaagtttg tgtggtttta gtgatggttg attttgtttt atttagagga 141840
tttgggaatt tttagtttaa ttgtttagag gagttttttt tagtggggag agtttgagtt 141900
gtttttttgg tggatagttt tttagatttt aggcggattt ttttgtcggt cgtttttttt 141960

```
gttgttttg ttttttgtt ttttatttgt tttttatttt tttttttgta gtagttagtt 142020
ggtacgggga ttttttttt tttttgggat tgtgaatgag acgatttttt tggtttttt 142080
gggtttagat tttttggta atagttagtg aaatttttgt tttaggtggg aggagaaggt 142140
ttagtatgtg gttataataa cggttttaat tatattattt ttaaacgttg gtttttattt 142200
atttttgtt ttgtttaata gttattttta ttattttatt ttttagatta ggaaattaag 142260
gcgtagagag ttttagttat ttgtttgagg tttttatagt taagtgtttg gatttgaatt 142320
ttggagtagt ttggttgttg agttgttttg gagacggggt attgtgtagt ggtcggggta 142380
ggggtggagt tttgtcgtcg ttgattttcg ggttatttgg agtgataata tagtttagtg 142440
tttgggtttt tgtttgagat tttggttgga tgttaggagt tcgagatttt ttagtttttt 142500
tttgtgtaag gcgttatggg gtgaaatgaa agaaaatttt agttagaagg tgttttttttt 142560
ttttttagt tttttagttg ttttggattt tgaaataaga tatcgttcgt ttgtggggtg 142620
agtaggaata gattggaatc gtatttttttt tttttagtt atagttgtta ggagtttatt 142680
tttttttgtt ttttttttt gtcgtttttt tttataagat tgggttggat gttgttggga 142740
aagttaatag gaagtttttt ggttgcggtg gaggtggggg tttgtgtttt tttggggatc 142800
gagatgaggg ttttaggttg gtttcggtaa gagtagggag gtttacgttt ttgtttgttg 142860
gttttgtttt ttgttttggg agaaggtatt tatatgtttt agaatgtatt ttttgcgtgt 142920
atagggatgg tttttgggtt atttatttga ggtttattga ttttttttt tggaattgga 142980
gtggtttttt tacggtggat tgggttgagt tttttgtttc ggtcgacgtt ttttttagg 143040
tattttttt tatcgtgtta gggtgtaggt tgttagtgga tgggttttta ttttcgggga 143100
ttggtagttg ttacgtttat tttatagtcg agatattttg gtcgaaggtt ttgttaagtt 143160
ttatggagtg tagggatttt ttttgagacg gttttttttt gtcgtttagg ttggagttta 143220
atggtttaat gtttcgttta ttgtcatttc gatttttag gtttagtagt tttttattt 143280
tagtttatta agtagttggg attataggtg tgtgttatta tgtttggtta atttttaat 143340
tttaatttt ttttgtagaa atggagtttt attatatttt ttaggttgaa ttttaatttt 143400
tgggtttaaa tgattttttt gttttggttt gttaaagtgt tgggatttta ggtatgagtt 143460
attgtgttta gttggggttt ttttttaatt ttttttggtt ttttttaggt gtaggattcg 143520
ggttttagtt ttatgtgttt ttgaatagtt ttgggttttg ttttgtattt tttgggcgcg 143580
tggttaggtt tttcgtgata ttagtaggag atttaggtga agaagtaggt agaacggaat 143640
gtttgtttat ttagtttcgg ggttaggcgg ggcggttagg atagtagtag tggttggtgt 143700
ggtttttggg gaggttagtt tattggcgtg gttagcgaat ggggaggggga gttttttttt 143760
ttgttttttt ttatttttgg tttttgggtt tttagggtta ggtttttaga gttttaggtg 143820
gaggatggag gttagggaag ttggggtaag agcgggttgg gggtgggtgg ttaggcggat 143880
tttggaaaat tatttttttgt aggtttcgtt ttttttaggg agtatttagg gttggtttcg 143940
gggagtcggg ttaatggtta tttcggatgg tgtaggggtt tatttttatg aaatcgggtt 144000
tatttttatg aaattgggtt tatagaagtt tttggtttcg gttttgtttt gtttttttggt 144060
ttttgttttt aaagtaggat ttaggatagt tttgaatttt ttgtcgaaat gtagtttttt 144120
gggttttaat ttagtttttt agaattagag tttttggggt gggtttttttg tttgtttagt 144180
agttttttagg ggatatttgt gtttgttaga gtaagtttaa taagtattgt tgtaaatttt 144240
tcgtttttat agtttttttgg ttcgtttttt ttttttttgtt gggatttgta ttaggattaa 144300
atgttaatat tagggattag gttatatttg tgaattttga gataatattt gtattttttt 144360
ttttttgag gttgttttgt tcgttttgtt atttaggttg aagtataatg gtgtgatatt 144420
tatttatggt aatttttatt tttagggttt aagtaatttt tttgttttag ttttttgagt 144480
agttgggatt ataggtttat attattatat ttggttaatt tttgtatttt tagtagagat 144540
ggagttttat tatgttggtt aggttggttt cgaatttttg gttttaagcg attcgttcgt 144600
tttagttttt taaagtgttg ggattatagg cgtgagttat tgcgttcggt ttgatattta 144660
tattttgatt aagttgttgt ggaaatattg agtttttttt gatagttttg tttatagagt 144720
gaatgtttag gaatcggcga ggtatttttt atttttattt agatggcggt ttggaggtag 144780
cgtttttgat tttgtgatgg gatttgtttt ttttgtttaa gagattttag agatgtggtt 144840
tttgaatgtg ggggtttttg ttggggtggg aacgggagtt ggttttggtt attgtttagt 144900
gattttattt tagtgtttcg attttagtat tttcgatttt agcgtttttt tttttttcggt 144960
ttcgcgcgga gattacgtgg tttgcgttga gtaatttttt attgttttcg tcgcgttttt 145020
gttgtaggga agtttttttta gggagttagt tgatatattt ggtttttatt atttgttttt 145080
gaataggaat gaaaggagag agtgagtaag aaagtggtag gaggtggggg ttgtagagta 145140
ggggaaggtt agagagaggg gaggaaagat ttggaaagtt taggtatgtg ttttggaagt 145200
aggtgttagc gatgagttta tttggagggt ggtcgttcgt tttgagtttt gtttggggtt 145260
agtagggaat attttgttag ggtatgtcgt tgggagtttg tttttggttt attttttgtt 145320
gttttgagcg ttgagtagat ttgtttttat tttagggata acgtaaggag gttgttattt 145380
tattttttat tttattaata ttttatgaat tataagtgat tatttattgc gttttgacgt 145440
```

```
attttaggcg ttgggttcga agttttatgc gttttatggg atgtatttgt attatcgtcg 145500
tagaaaacgg tttttgttat tattgtagag aaggtatcga ggtatagaga ggttgaggaa 145560
ttggttttag gttatatagt tagaagcggt tgattttggt tgtgaatata gggttgtttg 145620
attttaaagt ttgagttggt ttgtagcgag gtttatggtt tttgtgttat tgtttgagtt 145680
tttggtcgag ttttttggtt tagtgggggg tttagtgatt tattagttgt tttattatat 145740
gttgtggata tttttacggt ttatttgggt gggagggaag gggttttagg aggtgggagg 145800
ggtataggga atatagttta gtttgtttgg gtttaatatt ttgattttta aggtattttt 145860
tgagggtagt atgaggttaa gtttttgttt ttttttagga ttttgttttt gtgagggttt 145920
cggggttttt tgtttttttt tggggggtttt gttggttagg attatagaga agttttttgt 145980
tgttgatttt tttgtttttg tatttttttgg taagttattt tatgatttag ttgttttgtt 146040
tgtaaaatgg gttgaataat attgttttgt ttcggagggt ttttgggagg attaaatgag 146100
atttgttgta tagaagtttt ttgtaagtta tgttgattgg cggtcgtcgg gtttttaagt 146160
gaatgggtat tcgggtattg agtgtgtgga tttagtattc ggaaggaggg gttggggggt 146220
atttagaatt tttgggaggt ttggattttt gttttgttta gattttgagt ttttttttgg 146280
ttagtttaat ttttattagt agggtgaggg taattaggat ttacgtatag ttgggcgaag 146340
tcgagtgatt atatgtggaa gtttagcgtt ttttatgtaa agtgggtagg cggttaaagt 146400
gagttttgtt ttagtttttt tatttttcggg gttgtttttg gcgttttgtt ttttttttaga 146460
gttttattga tttgtattat gtgattcggg tttgggaaga tgggtttagt tgtgggaggt 146520
cgtgcgggtt ggggattgag agtgtcggag gagggttttt taggaaaatt tagttgtata 146580
ggaagtcggg ttagcggttt tcggaagttt ttgggttttg taagttggat tttttagatt 146640
tgtggttgtg gggttttttt ttgtttgtag ttgagagttt ttgattcgta tttgagggtt 146700
ttagaggttc gggaggggga tgtgatcgtg tttttagggt gaagtttatt cgagtgttat 146760
tttggatata gtttttattt ttttttggtt ttttttttgg ttatggggac gttgtttggg 146820
gggggttttt ttatatggaa ggagttgtag ttgagttggc gggtttcgta tatttttaag 146880
gtcgtttgat ttttagtttt taagtggggt tgtatttatt atttaggatt tggagtttta 146940
gttagagagg gatttttttat tgtttttttt tttgtttttt gggaaaggta gggtttttgt 147000
gttttttttt atttagggat ttggtatgtt gggaggttaa gaggaagttg tttttcgttt 147060
atttattttt taattttttt agttagtaga gttttatttg tgtgtttaga atttgtgcgt 147120
ggagttagaa gttataaggt tggtttagtt gtgtttgatt ttggttgtgc ggttttggat 147180
agttttagtt tttttggagt tgagtatttt gatttgtaga ggagaggttg ttgtgtcggt 147240
ttcgtagggt tttgtgtaga ggggagtgtg tgcggttgtg agtttaggtt atgtttttag 147300
attggaggat aggatcggaa tagtgggtta ggatggttag agggtttttag ttttaggagg 147360
aggtttttat atttttttagt atttttatat taggaaatgt agttaggttt ttaggttttt 147420
tatgttagga agtagtattt cgttttttat ttttggttgt ttgggtttga gaagttgagt 147480
atttttttcg aattcgagtg tttttttgatt tttgtttggg tagttgtttt ttttgaattg 147540
atcgtttgtt ttatatagtt ttttgtggtt tgttcgatag ggtggttagc gagtttttttg 147600
tatagtatat ttttttttagg agtgttgttt gtgatgatgg tgtaaagggt gtttttttcga 147660
gttttcggag gtatgtagtg gcgtaatagc gtgagtttag gagtttcggt gagtttttgg 147720
atggagaatt tgtttagatt atttttttagt ttaaattttt tttgggtgtt tgttgtgttt 147780
ggggcgttga tttatcgtgg ggcggtttgt gagattttac gttatttggt tattttttttt 147840
agtttttgtt tattgtatcg gtttttttggt ttggttgttt ttggaatatg agggtttatt 147900
tttacggaag aatatttatt tgtttttttt tggaaagttt tttttttagat tttttggtcg 147960
ttttatttag gttttgttga agcgttgtta ttttggagag gtttttttgt tattcgtagt 148020
taaaatagtt ttttttatgtt tgtgggtggt tttttatggg gtatggatga gtattggaga 148080
gttttgtcgt ttatttgttt atatgttttt tgggagggta ggggatattg atttgtgttg 148140
tgggtgtttt gtatgttacg ggtgtttagt gaatgtttgt taagtattag gggagtataa 148200
aggagggaag tttttgttta attggagtag ttagggaaga tttttttggag gaagtggtat 148260
ttggtttgtt tagaatgtgg cgatttgttt gtatgtagat attagattag agggtaaagg 148320
ttattattat aatttagtag atagttataa gtagttaagg aattgttatg atatggattg 148380
ttcgtgattg gagttaagaa ggagttagag atagaggttt ttgtgttttg gcgtgagttt 148440
ttgggggagg ttgagttttt agaatgtggg gtcgtttgtg tagaggtcgg aggtgggaga 148500
tatatttgtg agtgtcggtg agtgtggggt gagttattgg ggttggatta gaagatggga 148560
atttttaaat ttattaatta gttttggtgt tttgggagtg ataggagacg gggtttttgg 148620
gggattaatt tagtataagt ggacgagatg ggatgtaggg gaaaggataa agttttttga 148680
ggttttggga agttgagtta gggtagagtt atgatagggt gagttttaga agtaatataa 148740
gaaaaaatag ttagattggg ttttttttttt attgtgggag atgttttatt ttattttttt 148800
cgtttttcgg gttttggtaa tgggttgggt atagtgaatg ggttttattt gtaggagtgg 148860
ttatattttt tgggtatagt tgttttatat attgtattga gatgggattc gaggtcggga 148920
```

agaatagttt tttttgtagg ttgatattgt tagttttcgg ggagggaagg acggtagggg 148980
gtttggagta tttttttttg ttatttatat tttgtgtttg tttgtagggt tttgagtttt 149040
gtttgttttg ttttagtttt ggttaggaat ttgttggagt ttataggggt agttaggttg 149100
gaataggagg agattaggtc ggtaggggag tcgagagggg gttttggata gtatttaggg 149160
ttgagttatt ttgggtttgt tcgatttgtt atttggagtt gttttcgttt gtttattatg 149220
tgtaaggttt tgatttattc gggtttgttt aattagcgtt tttttttagg tttagttagt 149280
ttggttttcg ttcgatttat ttattgttat tagtgttatt tttgtattac gtaattagtt 149340
tttgtagtat gttagttatt aagtttaggg ttttttgtgt atttttttgta atttttatag 149400
taattcggga ggtatagaga ggttgagtcg ttggtttata gttatatagt aggtatgggg 149460
tattgttagg atttagtcgg atttttgatt ttagggtttt tttagggttt tgggtgggat 149520
attggtaagg aagtaaggta tgaatattgg ttttgttttg gatagagttc ggggacggtg 149580
gtttggtagg tttagttgat atagtgattt tggtgtaggt ttaattattg gtaaagtgtt 149640
taagtatata tagaatttta attggggaga taggcgaagc ggttaaggga gggttgtgag 149700
tgataggtgg atattgtatt tattggtaga ttagttgatt ttggtgtttt ttggagtttt 149760
ttaatttttt tatttagaaa aagaggaatt aatagtttta tttttaaatt gggtgggagt 149820
attatattcg aaattgaagt aaagttggtc gtgttatagg ttcggtagat gttagttgtg 149880
taggtaattt agtgattatt tgaagtaggt tgtgcggcgg tatttttatt ttgtagatgt 149940
agtttagaga ggttagggta ttaattttgg gttatatagc gatagtttag gttatagaga 150000
gtagtgttgg atgggagggt gttggtagtt ttaggtatag gttgggaggg ttgtggggtt 150060
agtagaggga gggtatattt tttagttggt ggggtttttt tgtgttttag tatttatgtg 150120
ggtttaaagg agttggaagg agggaggtta gagttattta tgggtttaga agttgataag 150180
taggttattg attttgaatt tgttttatcg tatgtaaatt ggggataaat ttggggattt 150240
aatgatataa tgtaagaagt agatatgtta tagttttggt tatatagtta gtgtataata 150300
agtggtaagt cgttagagga ggtagtcggt gagaatggga gtattttagt agtttttcgt 150360
tttttgtttt tggttgtaga tgtatttgtt tttggttatt tgtgttttag tttcgtgttt 150420
ttcggtgtgt gtgaggttaa gtttttgggg tggggatttg ggggtgtgtt tgtagttttt 150480
gaggttaaga ttaaggttga ggtcgaggtt gaggttgagg ttattttggt gaggaggaaa 150540
ttgtaggtgg agaagttcgt tgatcgtgat tttgatttga tcgtaatttt gatggtgtcg 150600
atgtcgttag tacgtaggtt ttttgttttc gttacgattg gtttttgtag tttatttggt 150660
tgagaggtgt gttggttttc gtaggttgta aaatggggat attatcgttc gtttgggtta 150720
tagcgtgttc gtcgattgta tttggggagg gattgagggt tgattgtgtt gtggggatgt 150780
tggtagagaa ttagagaggg tatttaaaaa ggttaatagt tcggtgggga gaggaggcgt 150840
aaggttggtt tttgtttttt gggttttgat cgaaagggaa taggggatat atttggggat 150900
agtttttttt ttggaggaag aagggtttag ggtgttggtg tggagtttgt atagatgggg 150960
ataatgagtt ggagggtgtt gggtaggagt tggttagtaa taggttgacg agggaggttt 151020
tgttggttgg gtttggtttt taagtgagta ttttgagttg cggggattta gggggtttta 151080
gagattttag agttgagatt cgggattata gaggggtggg ttgtgtttag ggtaatatag 151140
cggatggggt agatgttgcg aaatttgttt ttttgttttt tttttttttg ttatagaatt 151200
ggattttttt agtttatggt tgttaaattt tttagatgtt tttgggtttt atgggcgttt 151260
tatattggtt tttttgtagg atagtagatt ttggggggttg gttttgatgg tatttattat 151320
ggggtatttt atgtaagtat agtgttttta cgtttagttt tttagtttgt ggagtaggtt 151380
tgtgtttggt tagttttatt ttgtggttag aggagggaga tagtggtttt ggagatagga 151440
gttggtttgg ggttgttttt ttagtttttg tttttttagt ttattttttt gttttgcggg 151500
tgttggttat tttttttttcg ttagttttat ttttagtttt ttttgttcga gtcgtgattt 151560
gttagtaggg ggtagtggag gttttgaaag tttttggaga ggattttgaa gtagtttttta 151620
taaatttgtt tttggaaagt ttttttttgtt atatttttag cggttttttt tttttttaata 151680
ttttttttgg ggttgttatg tagcgggggg tggaggaatt tattaggtcg tggatagagt 151740
agagttatgt tgatataaat tttggttgaa tttatatgtt ttttatagtt agcggtcgat 151800
tattggaatt agggaagtga ttttatagta agggttcgta gagtagttat tacgtatttt 151860
cgattttggt ttttggattt agttttggaa aagaagttag atttagttta tttggagttg 151920
tttagggaag atttagttt ggtgtcggtt gatagtttat tggaattttt tatttgtttg 151980
gttaggtgat gttaaggggt agaggttaga agtgttaaga agtaggtcga gggtggtggt 152040
ttacgtatgt aattttaata ttttgggagg ttaaggcggg tgaatttttt aaggttagga 152100
gtttaagatt aggttggtta atatggcgaa attttatttt tattgaaaat ataaaaatta 152160
gttaggtatg gtggtgtacg tttgtaattt tagttatttt ggaggttgag gtaggagaat 152220
tttttgaatt cgggaggtag aggttgtagt gagtcgagat tgtgttattg tattttagtt 152280
tgggtaatag agtaatattt cgtattaaaa aaaaaaaaaa gaagaagaat aagaagtgtt 152340
aagaagtatt tgtttttttg attgaaaagt aatttttgtt gggcgtggtg gtttacgttt 152400

gtaattttag ttttgggagg ttgcggtggg agagtcgttt gaggttaggc gttcgagatt 152460
agtttgggta atagaggtgt taggggtagt ggcgtttgtt tgtagtttta gtttttttagg 152520
aggtttaggt aggaggattg tttgagttag gagtttgagg ttgtagtgag ttatgattat 152580
attattgtat tttagttttt gggtgatagg atgagatttt gttttaaaaa ataaaatata 152640
aataaaaaat taacgtttat tttttgtgat tttaaaaatt atatttattt gtgataaaat 152700
aattaaatta gaaaatgtaa aggaaatgat taataataat tgcgtttttt agataaatat 152760
tgttaatatg tttacgtttt tttttagttt tttttttatat agatatttaa tatttattaa 152820
tttgttttga attaaagatg gtttttttttg ttttgtatga gaattatttt agttaggtac 152880
ggtggtttac gtttataatt ttagaaagtt gaggtaggta gattgtttga gtttacgagt 152940
ttgagattag tttgggtaat atggcgaaat tttattttta taaaaaaata taaaaattag 153000
tttggtgtgg tggtatatgt ttgtgttttt agttttttag gaggttgagg taggagaata 153060
gtttgagttc gggaggtaga ggttgtagtg agttaagatc gtgttattgt attttagttt 153120
aggtaacggg agtgaaatgt tgttttaaaa aaaaaaaaaa aaaaaaaaaa gttgttacgt 153180
tgttattttt tttagggatt tttattttt ttatttttcg tttaagattt gttttgtaaa 153240
aggatgaggt taaatagtta tgggagggtg gtattggatg tttaaaaaaa tgtttcgtgg 153300
ttggcgggat ttagaaatag gttggggagg ttttagtaga gggtggattt tagttggggg 153360
tggggggtag taggggggtt ttttatatgt ttttttttgcg tgttttatgg ggttgtttgt 153420
gaggtcgtgt gaattagagt tcggttggga agttatatgt tatttggatt ttgatttttt 153480
ttggtagtat gattttagtt aatacgttta attatgtttt tttttgtaaa atgggttaat 153540
agaaatgttt attcgagata tgatgagaaa taaaggtgtt tatagagtaa agtgtttaga 153600
acggttttgg tatgatagga gtttatatgt tagtgttgtc gttggtatcg ttattattac 153660
ggcggtattg gttgtttttgt ttttttttttg tttttgttat tttttttttt tagtaggttt 153720
ttatttattt gtttatttat ttatggtcga ggtttcgttt gtgttaggtt tggtgttgag 153780
aacgggggta gagagatggg ggttgtgttt tgttttttga tttaagggtt tttttgtaa 153840
tgtgaagttt ttggagattg gattttttagg ttgttttatt agttttttttt taggttagtt 153900
taattttttc gtgttcgttt tttgagttgt tagaggaaga gagcgggagg aaggatggag 153960
tggggttttt gtggggattg agcggggggt gtgtgaggtt ttggaaatgg tgtttgcggt 154020
atttacgtag tattagtttt tgttgtagtt gttgtttgga gaatattttt ggattttagg 154080
atattgatga tagaaggtgg aacgtttttt ttggaagtta ttaagagggg attatcgcgg 154140
cggttgagta gagttgttga tttgtgagtt gtggtttgat tatagttatt gtttttatga 154200
gcgaggtttt ttgtggtttt tttttttagga atttttggtt ttttggtagg ggatagttta 154260
gggttggggg tgggtagtgg tgtttgtagg tttttttgga gttagagaat tttttgtcgt 154320
cgtggcgggg ttgttttttt ttgttttttt tttttcgttg tgtttttagt tgtttcgata 154380
ggatgttttt ggtttgagag atagaaattt taattttgaa tggttttagt ttttaagaga 154440
atttggagtt tttttgtgta aaatgagatg ttttgagtat cgaggatgtt gtggggggatt 154500
ttttatttta ttatttttag tcgtggtgtt tttttcggtt ataggatgtt gtagttacgt 154560
tgtagggaag gagagttttt tttttttgtga gtttagggag tttttttatg tagttttttt 154620
attggttatt gtttgagatt cgatttggtg gtttttgttt taaggagtag aaagggggtt 154680
cgttaggtta gggtggggtt gggtgcgagg atttttttcg gttaataggg tttgtttagg 154740
tgttcgtttt gttggggtag tagggaaggg atttatatgt ggtattggga tacggagagg 154800
tttttgtgtt tgtgacggtt aaaagggatg gcgttttagt tacgttttta tttatttcga 154860
ggtttagtat ggttatgtcg gaaatagtag tatcgttttt tttattgttt tggtttgttt 154920
ttaataggtt agagggggtgg ggttggagcg ggcgtttgat ttgtggttgg atttagttgt 154980
tcgtggatag gttgtgagtg aatttgtaga ggaagggaga tgggaatgtc ggattttttt 155040
tttgtatttt tcgaattttc gtttttttgtg agttagatgg ggagtgtatt tttgtttttt 155100
tttttttgggt atttttttgcg tttttcgttt taggtatttg atgttgtttg tttatttttt 155160
tttttttgttt ttttcgttgg ttttttttat atggttttaa ttaggatttt tcgttcgttg 155220
gtggatgggt agagaggggga gtttgttgtg gggttttttt cgatcggggg atttttttagt 155280
atagagtttt ttggtttttg tagggacgtt gtgtgtgttt gtgtgagttt ggcgtgttag 155340
aagcgttaag ttgtagttgt tttgtgcgtt ttttatattt tttattttttt aaaggttttg 155400
ggtttggtgt ttgtgggttg gggatttgtg ttggggtttt taggaggttt ttggtgggag 155460
aggtagggtt tttttttttt taatttgggt aattttgttt tggtttgttg tttgcggaaa 155520
gtgttttagg atacggtggt aggattggtt ggggttttgg ttttaggttt agttttagtt 155580
tttgttagat ttattttttt tgtgtttttcg tttattttgt ttattcgtag ggtttgtagg 155640
agtattttga gagttggtag ggtggtttgt tcggcgttcg tatagggttt gttggtgggt 155700
ggttgaggtt gcggtgaggt ttttatggtt ttttcgtttt gtatattttt tttttgttgt 155760
gttgtttcgt tagtatatga gttcgtattg tttttgagtt tgtagtttgt tttttttatt 155820
aggtggttaa gtagggtggt agttagggtg gtagagtttt attggtttgt aggggatttt 155880

ttgtgggttg taggtagttt tttgtggttg aaggtgtttt ggtaggattt gtattatgat 155940
tttcgttaaa gtttacgttt aaggtatttg tgggtatttt cgttttagac gtgagggttg 156000
gtattttttt ttgtacgtga agtaaaggtt tgtttgaagg agcgtgtgga agtttgggtg 156060
tgatgaaggt aaggatcggt ggtttatttt ttttgtattt ggattttggg tggtaggttt 156120
ggggttttta gtttttaatt taggagggta gttagtcgtt ggaattaaga gtgaatttta 156180
gttgttgttg ttatagttgg agggaagagg ggaaaggagg cgttgtaggg gagtagagat 156240
tttatttttt ttttgtcgat attaggttgt cggtgttgga cggggttttg gtaatcgtgg 156300
taggagtggt gatgttcgat gatggtaata agggtttttt gaggatttcg agttgtttta 156360
agggtttttt atttggagta agtatttttt ttaatttttt taataatatt gtgaaattga 156420
ttttgttgtt atttttattt tatcgatgag gaatttatag tttagagatg ttaagtaatt 156480
tgtttaagtt tttttttgag tatggtagag ggagggttcg aatgcggaaa gtttggtttt 156540
agtgttttta tttttaatta ttgttattta ttttttcggt tagtttttg attgtaaaat 156600
ttgaggttta gaggagtgta gtgatttggt tagtttcgta cggtaggtta gtggtagagt 156660
tgagtagtgt tttttatgtt ttgattttgt ggttttttga ggtggtttat ttttaaatac 156720
gtttttatg gtggttttgt ttttttttt tttttgaatt tgttagtttg tgtttaggta 156780
gggggagaag tagagtaatt atgtcgggcg ggtcgtcgtg ttttagtgtc gtttgtttta 156840
tatttgattg gtagtagagt ggttttttt tggtgtggtt tggaggtgtt agtggttatt 156900
tattagtata gtttagtttt atatatgtta tttttaaagg atgttgatat agaatgaagt 156960
agttagggga ggcggacgtt ttggtacgag ggtggcgttt ttggtaattc gtagtgttgt 157020
tgtttgtggt tgtttttggt tggtatttta tttttttttg gttgttttgt agttatttga 157080
gggaggttag tatgtcgttt ttattttagg atagtgtgaa tgtagtagcg taagtttgtt 157140
agcgtcgtat tgcgggattt ggaggttttt gtttttgagt tttgttttg tgtaattttt 157200
aaagtacgga tttttaggg gttttattt ttttatttgg aaaaatttta tatcgtttag 157260
ggtttgaggt agggttagag tatggagacg ttgttggagg attaagggag aattgagcgc 157320
gtgttttttt ggttttagtt tcgtgtgtgt gtttatgtat gggtatttg ttatttatat 157380
tggtttttac ggttgggtgg gagttgagtt gagttcggcg gttgtttttt tattttgcgg 157440
gtttggttgg ggagtgtgag gatgtttgtt ggaagttaac gtgtacgaaa gagttaatgg 157500
tggattttat tttttagtt tttttttag tttttttagg gaaagggtag gaatagagag 157560
gttttatttt tagaggaagg ggtgggcggg gatatagcgt tgttcggtat agtttttttt 157620
gagttatttt gtatgtttta ttttatcgat ttttgtagtt tagttgttta gagcggattt 157680
agaaggtgga gagagggttt ttaataagta gaggatggag tgaatgtgag atttattatt 157740
gggatttggt aaagtaaaga gttttatttt tttttcgtag atgggacggg ggcggggttg 157800
gagttagcgg ggtgggggta tttagtaaga gttttttttt atttttttat ttttttgtt 157860
atgggttttt tttttacgt agattttag tttggaatga ttagtgttta gtttttttcg 157920
ttttttttt gtttcgttgg agggagtgat ttggtttttg gtttcggtta tttatatttt 157980
ttgttttgtt gtattagttt cgtggtaggg gttgcggcgg aggtgggtaa aagtcgtttt 158040
cgggtagtta gtttttattt tgggagtgat tgaaagtttt tgggggtttt tgtagggtga 158100
gttgtttatt ttggttttgg taaggatatt taggagaggg ttagggtgtt tgtatttggg 158160
gtatagtata aagaatagtc gtaagtgttt tttaggtagg agggtgtcga agtattggga 158220
agttgggatt tagaagtatt gtttggggtg gtgagtggaa gatgtaggat atagatttac 158280
gcgattgtgt agattatttg gttttatttt ttcggtttat agtggaggaa attagaggtc 158340
gagagaggtg aagtgagttt tttggggtta tatagtttag ttaggtttag ggcgtcgatt 158400
ttttgttttg ttttttcgtg tttattcggt tggtattagt atttttgttt tgatattttc 158460
gtgtggtagt tgttgtgatt ttgtgatttt tttttatttt atttagtgg ggttttgcga 158520
gggaaagaag aatgttttat gtagaagtgg ttggaaagta agaagtttgg agagaagtga 158580
ggtttttgtg gtttggtttg agtttaggat ttatggtttg gtttttaggg atggagtatc 158640
gattttttt ttttatattt ttattttacg tttttaggag ttttttatgg ggaagtcgga 158700
atggatttgg gtttagagat tgtaaattta ttttgttgga tgtgtggttg tttttatgta 158760
attaattttt ttacggagaa gttaggggat ggtttttagt attttcgggg gtttttttagt 158820
gaggtgggag gatttcgaga ggttaggagg ttgtttaaga tttttgttt tttagggatt 158880
aggtgagggt ttgtcgattg ttttgatttt ttgatgtttt tggtggggag attttagagg 158940
ggtttgtcgt tattatcgtt agtggttttg gtttgtttag ttattttttt ggggtggtta 159000
aattttatgt tgtaaaagtt taagtgaatt ttagtagtgg tttgggagaa ttatttgtta 159060
cgagttaagg atggagtttt tggggaggat tttttgttgt tttttaattt ttttcgggta 159120
tattttttg tagttttatt ttttgtaaga aagttaggtg tttttcgaag ttttttttttg 159180
gttttaggtt agaggtgata gtgggggttt tttgtttgcg ttggtgattg tggttggttg 159240
ttggtgtttg gttttgtttt gtattgtgta tttattgaag gcggtattat tcgggttttt 159300
tcggtggtta tgtatgggtg agtagtttac gatatatttg tttttagtat cgtgtgggtc 159360

ggggttttta gaggggttga gggtttggat ggttttgtag gggggtgttt ttggttttgt 159420
gatcgaaagg agttgaaggt ttggagaagt cgttgggtcg aggttagttt ttatagattt 159480
ggcgaggagg gaatagatgt ttacggagtt gttttaggta ttgtatatat tgttttattt 159540
tgcgttgata gttaagaggt tggtttttcg tcgattttta ttttatagat aatgtgtttt 159600
tttagttttt tagtggtgga gttaggattc gaatttacgt aggtagtttt ttgggtttgt 159660
agttttattt tttattttt tttaggattt atgttttggg gttggtagga taatgatttt 159720
tagagttgag tatattttaa tttttagtat ttgggattac gttaggtgat atggtagggt 159780
tagttaaggt tattaattag ttggttttaa aatagggaga tgattttgga tgatttatac 159840
gggttcgttg ttattataag agtttttaaa agcgtaatag attatttgag gggtgtagtg 159900
ggagaggttt ggaaggagga aggaggttat aagttaagaa atgtaggtga atagaaaatg 159960
taggaaaacg tagttttttt tgtagtttcg ggagggtgcg tagttttgtg gatatttttt 160020
ttgtagttta gtgaggtgga gtgtgggatt aatttgtgtt gtttgatatt ataagtgtgt 160080
ggttatttgt tatagtagtg atgggaattg aatgttatta ggtagtagtg tttttatttc 160140
ggaagaatta ggataaattt ttttgtggat tttaagtttt gggagatttt gggttttttt 160200
ggattgtttt agttttgtaa ttataggaaa ggttttttat gtttgtttgg gagaagaaaa 160260
tttcgtagta aatttatttt ttttttgat aaatgaaggc gagggtgtgg gttggatata 160320
tgttttttt ttgtggattt tttttgtttt aatatttaga attttgtagt tgaaggagat 160380
tttgggggt tgtttgtttg atttgtaagt gaagaaatgg aggtttaggg aagttaagtg 160440
attggtttag ggttatgtag agttatgttg gaatattaag ttagatagtt tagtttttgt 160500
tttaaagtgt ttttttttt ggttggggag gagggttgga gggagtggat ttcgagtaat 160560
agtagtattg gtaggggttg gaagagtagt tattgtattt aggtgttgga gtttttggtt 160620
attttttgta ggaattaggt gatttattat ggtataggtt tttttttgtt tatttgtagt 160680
tttgggggtt ttagggatta gaggatattt tggtttgagg gttggggttt gagagaagag 160740
atggaagtat ttttagtttg gtttgttttc gtggcgtttt gtaggtatat tcggtagttt 160800
aggggtttta gagaatttgg ttggtgggcg tttttatttt gtcgtagttg tattttgtgg 160860
ttagtagttt gtaggatttg ggttgttgtt tgtggttttg aaatagttat tttttgagtt 160920
gagtggggat taggtgagta gatttttata ttttaattgt tgtgattttt ttgttttgga 160980
ggttggtggg tagtttggtt ggtgttttat ggtattttat gatcggtttt gttattaggt 161040
tcgtgagttg ttttttttt ttcgggtatt ttgaacggtt tttttgagag tttcgttttg 161100
ggttttcgtt tttgtaggtt ttcgttgttt ggggcgtttt tttttttgg aagttgggtt 161160
tttttgggga tttattaaga agggttttgt cgagtgttgt taaattgttt tatttggttt 161220
ttatttgttt tatttattat tttattttaa tttttatgtt tttttttgg ttttaagatt 161280
tgagtaggta gggtttgtga ggtattttag gtttttttgat gttgttagta tttaggggat 161340
tgttagtata tatgtaggga gggaaggaat gaaggaagag atgatttggg gagtttttta 161400
gtttttattt gtaattggta tattgcgata aaatatttat ggtattttat ttttttata 161460
tattttttt ttaaatttat ttttatttga gatggagttt cgtttcgtta tttaggttgg 161520
agtgtagtag tataatttg gtttattgta atttttattt ttcgggttta agcgagtttt 161580
ttgttttagt tttttaagta gttgggatta taggcgttta ttattacgtt cggcgaattt 161640
ttgtattttt agtagagatg gggttttatt aagttggtta ggttggtttc gaatttcga 161700
ttttaggtga ttcgtttgtt ttcgtttttt aaagtgttgg gattataggc gtgagttatt 161760
gtatttggtt ttcgtatatt tataaaagag aatttgttta gaaaattttt tagatgtatt 161820
tttagggttt taaggtatag atatatgaag tttgttagtg agtagttgtt gtttattttt 161880
ttttttttt taatttagag cggtttgcgt tttaagcgga tagttgtttt ttttttttg 161940
gttttttgtt gtttacgttt ttatagaagt agggagaaga ggtaggaagg tgacgcgtga 162000
ttattcgttg ggcgttcgta tagtgtcgtt tgtataggtg ttattttttt tatttgttat 162060
tttttcgtt gagtttttt tttttttgt agatgagaga gtattgtttt ttagaggtta 162120
agaaatgggt ttagagttat ttagttgatt agcgtgtagt tagatttgaa ttatattttg 162180
ttgattttta tgtttgtagg tttggtttga tgagatggat agttgggtgg tggagatttt 162240
gagattttgg atggttttta aatagggttt gtagttttg gatagggaga gtttggtata 162300
aagggtttgt ggagtgagtt gggggagaag gtggtttagg gtttttgttt ttgaataaat 162360
agatggaggg gttattttag cgttttttta gtttttggtg tgttgggttt ttggttttgg 162420
gaggagagaa tggagttggt ttttattttg ggttgggttg gtttgggttt aatttagata 162480
agtttttag tttgttatga gtgatatttt tgtttgtttt ggtgttttg ttaaaaatat 162540
gaagggtttt agaattttta tttcggtttg ttttttttt agatttggag tgggtttggg 162600
gttgataata gttttatgtt agatagtttg agtagttatt agtgttatta ggtttgatat 162660
tgcggttttt gtttcggagg gaaaaataag gtttttttg ggggattttt tgttagtttg 162720
tgaaaatagg tttggagtta gttagcgttt ttatgtttag atatttcgtg tggtttttaa 162780
agtgttttta tgttgttagt ttatatgatt tttttaaaa ttttataagg gagggtagga 162840

agtttgagta atagtttgtt aagtatttat atagtaggtg gttttattgt tttttattta 162900
tagataagta aattgagttt tagggtggtt atcgtttgtt ttttaagtta tttagttatt 162960
tagttacgga agaggttatg atttttttata ttttagtgaa gttgaagatt ttttttttcg 163020
tttttaaaaa tgtttatttt ttagggtagt gaatatttgg tgtttttgtt tagcgtaaga 163080
ggtgaacgtt tgttattttc gttttttttt ttttattaga tttgattttt tttttgtgac 163140
gggaatattt tgtaggtagt ttgttttgtt tttatagatt ttgagaaacg gggtttaaat 163200
tgtcggttgt gggtgggata ggcgatattg tagtgaagag gtggagtatt tttgtagcgg 163260
ggaagaaata tattttttttt atttttttag gtttagtgtt tgggggtttg tgaattaaat 163320
ttatttaaga taaattaatg agagaaaaga tagatttaat tatatacgta tgggatttta 163380
ttgaaaaacg tgatttaaaa gagtaggtag aaggtggggt tcgtgattgg ttcgaaggta 163440
gtgagttatt ttagttgatt gtttatagtt agttatagat tagatttttt gttttattgt 163500
ttttttttttt tttatttgtt ttgttttttt tgagttagag ttttgtattg ttgtttgggt 163560
tggagtgtaa tggtacgatt tcggtttatt ttaattttcg tttttcgggt ttaagcgatt 163620
tttttgtttt agtttttcga gtagttagga ttataggtat ttattattac gtttggttaa 163680
tttttttgtat ttttagtaga gatagggttt tattatgttg gttaggttgg ttttaaattt 163740
ttgatttcgt gatttgttta tttcggtttt ttaaagtggt gggattatag gtatgagtta 163800
ttgtatttag ttaattagtt ttaaaaaaaa aaaaaaaaaa aaaagaattt agggttcgta 163860
tattatttta ataggaaaaa aggaaggggt gaaagtatat attatgggtc gggtgcgatg 163920
gtttacgttt gtaatttcgg tatttcggga ggtcgaggta ggtggattat ttgaggtcgg 163980
gaatttaaga ttagtttggt taatatggtg aaatttcgtt tttattaaaa atataaaaat 164040
tagtcgggta tggtggtgta tatttgtaat tttagttatt tgggaagttg aggtaggaga 164100
attgtttgaa tttacgaggc ggaggttgta gtgagttgag attatattat tgtattttag 164160
tttgggtaat agagtgagat tgtgttttaa aaaaataaat aaaaaaaaaa aagtatatat 164220
tatggtaaaa taaatgagtt tttggaaaga taaataggtt tttaggagtt gagatgggag 164280
atgtgaagtt ttgtgataat gtttttttag gtgtggtgtg gaaattattt atattttta 164340
gggaagagtt agttgttttt agggagtaga tttgggtttg tatattattt taatagggga 164400
aatggaaggg gtaaaagtat atattatggg ttgggtgtaa tgtgtttagt gtaggaggta 164460
gacgtggttt atggttgttg ttgaaattag tgtagggttc gattgttttg tattgagtgt 164520
tgaggatttt ggttttgtat tagttatgag tttgtagttt tgggatatat ttgatgttgg 164580
tttaagggga aatgaggttg gagggtaggg gtggttggat tggatgtggg tttggggtta 164640
ttgttgattt ataaagtggt tattatacgg gaataataga gttggttata gattataatt 164700
agtaatatta tttaaaagta aggttatatt tttaataata agttatattt taagttatta 164760
ttaatttata aaatatattg ttggtataat gatagttttt taagaaggat ataaaaaggt 164820
atgttgggtt gggtgtagtg atttacgttt ataattttag tattttgaga ggttgaggtc 164880
ggcggattat tttaagttag gagtttgaga ttagtttggt taatatggtg aaattttgtt 164940
tttgttaaaa atataaaaaa aaattagttt ggcgtggcgg cgtatgtttg taattttagt 165000
tacgggggag gttgtggtag aagaattatt taaaatttgg gaggtggagg ttgtagtgag 165060
ttgagatcgc gttattatat tttagtttgg gtaatagagt tagatttcgt tttaaaaaaa 165120
aagaaaaaaa agaaaaaaag taaaaaggta tgttgaaggt atttgtttgt ggaaagttgt 165180
aatttgacgg gaggtagggc gagtattttt tagaaaggtg gaaatgggtt tttttgttat 165240
agagatttat aaatatagat tgaggtttgt agtttaggag atttaaaaga ggtaaaggtg 165300
aataagagtt aaggttttta agttagggtt tgattttggg ggtgggggtt atatatgatg 165360
gaggatgtta ggagggtttt ttggaggtgg tgatatttat taggggggat tttaggtggg 165420
gtttgcgaga tagtaggatt tggttgaaga gcgtaggttt tttagatagg agtgggaatt 165480
taggtttgaa aatgagttgg ttagattgta ggggttttcg aatgtaaggg aagaatttta 165540
atttaatag aggagtttgg gaagttgttt gggaaaagga gtgataaagc gggaggggtt 165600
ttttagtaga gttttgtatg ggtaggaggg agcgagatgg gaggtgggtg ttggtaaggg 165660
atgattttag tggtttaggg gagtagttat agggtggaaa ttaatttgt ggggtttggg 165720
taggaaggga ttgatttgag atatcgggaa gaaatagtta gtaggatttt ggtgagaggg 165780
ttgggggtcg gggaggggg tgtggagagt gggagtttga gtttttgaaa atagatagtg 165840
ttttaagggg gagatttgag gtaagatgtg gagaggtagt tgagttggga agttggaacg 165900
gaggtgtggt tattttagga gattaagtta gggggcgagg ggatttttgg gagttattta 165960
gattttagta ttagggaata cggaggggag aaggagggga gaagaattag ttggtgcgtg 166020
ttaggtattg ttttcgaggt tagatggggt ggggttggat attggttatg aggattttt 166080
taagagttgg gttaatagtg agacgggcgg atattagatg gttcgggtta agaagtaagt 166140
gggcggtggg aagttagaag tcgtggtgta gatttatttt gagaggttgg gttgtttaga 166200
tagggagaaa gtttatagtt tgaggaggta gataggttaa aggaagaggg tttgtttgtt 166260
tttgagattg gggtattttg aacgtttttt taggttaagt gagaagaaat tagaagagga 166320

```
atatgttatt taaatgtgtt gaaatagttt agtagttgcg ttgttttata gaaaatgtgt 166380
agaggttttt ttgttttatt atgtttgttt ggggacggtt tttattcggg ttatttaggg 166440
tttttttagt ttgttaattt aagggtggta ggagttgtgg gtgtttttag ttttttttta 166500
aggttggttt gtgggtagaa atgtgggttg tttcggggcg ttatagttat aaatgtatat 166560
tagtttttag aattatattt ttgtttttta gttttttttt tcgtattttt atgtagaagc 166620
gcggtcgtta gtttataaag gatagggagg gatattgttt ttggtatttg atgggaagta 166680
tttgttgtat tttattgggg tgttgtttag gatggattgg aaaagagttg gtaggaaggt 166740
tgagttttgt gtttataatt tggtttggtg gtggtcgagg agtttggtag gagtagagtg 166800
taggatttgg gaattggggg ttggtgtatg tgtgtacgta cgtgtgtgtg tgtgtgcgtg 166860
cgtgttgggt gggtagggag gaagttgtga aattatattt ttttttttt gttgttgtgt 166920
tgttgtgtgt tttagtagta cgtgggtgtt attatatttt ttagtaggtg ttaattttta 166980
agattgtttt gggttttttt tttagttggt tgagttggag gtgggagttt taattgtttt 167040
ttgtggtttt tagagtggga ttttgttgtg ggataggttg gttaatggtg ttttttttt 167100
tgtgattttt ttgttgggtg ggttacgagg aaggattgtg ggtgttgttt atagataggt 167160
ggatatgtgg taaggatatt ttgggatttt ttttttgacg tttttgaag ggggtatttt 167220
tttagttttg agatgagttt ttgtggatgt gggaagttta ttattttaag agtagtagtt 167280
ttggaaaatt taatatagaa tttcgagtag gggcgggaag gggttttgtt tcgtttattg 167340
gttgtttggt agagttttgt ataaggaagc gtttgtgttg ttgtgggcgg aggaatggat 167400
tgagggttat attcgttttt tgttgtcgtt gtaattgttt attataaatt tagtggttta 167460
aagtaataga ggtttttttt tttatagtgt taagggttag aagtcgatta gttttatcgg 167520
attaaagtta aggtgttggt agaatttatt tttgtttttt ttagttttgg gtgggaggtt 167580
ttgttggagt tttttggttt gcggttgtat ttttttagtt tttgttttta tttttttata 167640
gttttttttt tttttgtagt tagattttt tttgtttttt tttttttttt ttttgagacg 167700
gagttattta ggttggagtg tagtggtata attttggttt attgtagttt tcgtttttttg 167760
ggtttaagcg atttttttgt tttagttttt cgagtagttg ggattatagg tatgttatt 167820
tatatttggt taatttttgt atttttagta gagatagggt tttgttatgt tggttaggtt 167880
ggttttgaat ttttgatttt aggtgatttg tttgttttag ttttttaaag tgttgggatt 167940
gtagttatga gttattatat ttggtttgtt tttttttaa aggacgtttg tgatttgggg 168000
tttatttggg taattttttt atttaatat ttttagttat atttatagag tttttgttgt 168060
tatatgaggt aatatagttt gggaagggag agttatttag tttattttag gggtttgtgg 168120
tgtattttag ggtttttttg attttaagat ataaagtaag aaaataaatt ggtttaaggg 168180
gaaaaaagga tacgttgaat tttgttgttt taaatgtata ttttttattt gtgttaaaat 168240
gtatagaata taaaatttgt tattagtaat attgagtata tttatagtgt cgtgtaatta 168300
ttagtattgt ttagcgttag aattttttta ttattttaaa gggaaatttc gtatttatga 168360
aggatttatt ttttattcgt tttttttagt ttttggtagt tattagaatg tttttgttt 168420
ttataaattt atttttaata agtgtaattt tgtgtgattt taaaataaat aaatatgagt 168480
acgatgagtt gtttattgga aggatattta tgcggggagg tcggcgtgtg gagtgcgtag 168540
gttttcggac gggtaggagt tgaaggggcg tggatgtgtc gttttttttt tttttgttt 168600
ttttttggg gttattgttt gagtattttt ttttgtaaat ggttttaaat aatgttttag 168660
tttttacgtt tgtatcgttt tttagtttta ggatttttta ttaaaaaata ttttaagttt 168720
tagatttatt tttgggaaaa ttttaatggt tttattttt tttttgagtt agttagattt 168780
tatggtttgt ggcgggttgt ttttgagttt tgagtatttg tgagttaggg aagtaggata 168840
ggtatattta gggaagggga agagtcgtcg ttagttatag taattgatat ttttggaatc 168900
gtttaagaga tatttagcgt gtgtttaaaa tatttatttt tttttttgtt tgttttttga 168960
gacgaagttt cgttttgtcg tttaggttgg agtgtagtgg cgtgatttta gtttatcgta 169020
attttttttt ttcgggttta agcgattttt ttgttttaat tttttgagtg gttgggatta 169080
taggtatata ttattacgtt tggcgaattt ttgtattttt agtagtgatt gggtttttatt 169140
atgttggtta ggttggtttt gaatttttga ttttaggtaa tgtgttcgtt ttggtttttt 169200
aaagtgttgg gatgataggt gtgagttaat atatttggtt atatttattt tttaggagaa 169260
gttttgtatt ttttgaaagt gaaatgtttt taggcgggtg atattgatgg tgggatttgt 169320
ggttttatta gtgttttatg agagatgtcg ttgtgtgttt tattaagttg tattttattg 169380
ttttttaaat gtggtttgta gatatggtgt cgagaatgat ttgggaggtt taaattataa 169440
tatggtttaa attaatagtt ttttgatgat ttggtattat tttaggatta aattgagatt 169500
attcgaggat gggcgtggtg gtttacgcgt gtaattttag tattttagga ggttgaggcg 169560
ggcggattaa ttgaggtttg tagtttgaga ttagtttggt taatatggtg aaattttatt 169620
tttattaaaa atataaaaag tagtcgtcgg atatggtggt gtacgtttgt agttttagtt 169680
atttgggagg ttgaggcggg agaattgttt gaatttagga gacggaggtt gtagtgagtt 169740
aagattacgt tattgcgttt tagtttgggt gatagagtaa gattttgttt ttcggtaatt 169800
```

aaaaaaaaaa aaaaaaattg agatttttcg agttgtattg tttagtatga tagttattgg 169860
ttgtacgggg ttatttgaat atttgaaatg tggtgatttt aagtggagag gtgtcgtgtg 169920
tgtgaagtgt attttgggtt ttagagtttt agtaaggggg aaagaaaaga atattaaata 169980
tttgttattt ttatattgat tatatgttga attgataata ttttgggttt tgttaagtga 170040
atgatattaa aattaatatt atttgttttt atatttttta atgatttttt tttttttttt 170100
tttttttttt ttttttaaat atggggtttt atattgttgt ttaggttgga gtgtagtggt 170160
acgatttcgg tttattgtaa tttttgtttt ttaggtttga gcgatttttt tgttttagtt 170220
ttttaagtag ttgggattat aggtggttgt tattatgttt ggttaatttt tgtattttta 170280
gtagagatag ggttttgtta tgttggttag gttggttttg aatttttgat tttaggtgat 170340
ttgtttattt tagtttttta aagtgttggt atgataggcg tgagttattg tgtttggttt 170400
gtttttatat tttttattgt aggtgttgga aaatgtaagg ttgcgtttgt ggtttatgtg 170460
tcgtttttgt tggagggttg gatcgagggt tagtcgagaa ggttttgggg ttgttttatt 170520
tgaatgttga gtgtgtgttg agcggtagag aatttttgaga agtgttgaag gatagaggtt 170580
ttgtgtgggg gatgtagttt taggtcggag atgttttttt tgtgttttgg agatttttat 170640
ttggtgatag taagaggaat tattgtaaga ggggcggtta ttttagtaaa gtatttagga 170700
tgttggtttt ttagtttagg aatttagagg tagaggaggg gaggaggttg ggtttggagg 170760
agagtagggt tttgggtttt gggaaaagtt tggggggtta gaaagttggg ggatagattg 170820
aggttagagg taaagagaag aaggttcgga gaggagggtt aaggaagaag ggtttgtttt 170880
acggggaggg agaggtttac ggggtagggg cgtggtggga gttgtatttt cgaggttttt 170940
tgttgatttt gatatttggt tagaaaggag ttttttttgg tatttttgt tagcggtttt 171000
tggagttgtt ttttaggggt tagtgatgag ggtgttttgg gttgggttgt agatagtgga 171060
aaagataggt tttttttatt tttagtgttg gggaaattag ttttttggag gaagggggag 171120
ttgtattttt tgagtggtcg tatttttttgg tttagacgtg tgtgttttgt tgtaatttttg 171180
gcgattggtg ttggtggtgt ttattttatg gatggggaag gttaaatttt atagatattg 171240
agtgataggt ttagggtttt ggatttttagt gtttgattta gaatttaggt ttttgatttg 171300
ttattagttt tttttttatt tagatgggaa tagggatttc gggtatgtag ttttttttta 171360
gatatttttt cggttttggt ttggcgttcg gagattagta gtagtagttt tttacggtcg 171420
tttattgtga gggtggggag ggtcgtggga ttaggaggtt ttagggggaa agttggttat 171480
gaggagtggg aagcgacggg ttagatgtta gtgattgttg gttggggagt cgttggacgt 171540
ggttggtttg tttggttagt gtggcgggtg tttcggggtt ttagttattt ttagttacgt 171600
gtgggggtgc gggatgggaa aggtaagagt gtgtggagtt tagttagtat ttaatatgag 171660
gtttcgtggg ggttgtttga gggcgtagtt tttagaattt agtttttggt ttttagaagg 171720
tgttattaaa ggtttttttt tatttgagtg aatttttttt agtttttagt aagttttcgg 171780
aacgagttta tttttaggcg ttttttttta gtgtgggagt ggttacgttt ttgttgggag 171840
tgatttattt gggtttagag gtcgtggtat tgagatgggt ttggtagatt ttagcgttta 171900
ggtttagttt ttatagtgtt agtttttttt tttggggatt tttttgtttg tgtttttttg 171960
ggttttagta tattttaggt ttgtagggag ggggagagga agagattgat ttattggtta 172020
ggttttttag ggttggagaa ggttggagag gtaggagttg gattagattt gaatttagag 172080
gttttcggag gaagagttta gggtgagttg cgtttttatt ttttgttttt ttttttttgt 172140
tttttttttt ttttatggtt ttagttagta agtatttggg gtagagggga taaatttagg 172200
tggttgtgtt ttagttttgg ttgtaggttt gaatggtttt ttggggtggt tggttatgtt 172260
ttttgagagt ttagttgtgg cgatgtttga gtaggtaggt gggggagtat ttaggaagta 172320
ggggtgttag gtagagtata aggagagagg gtgtttaggt tagttttagg atttggttga 172380
gaggaggggg tttttttacgg gtatcgtttt tggtaagtat agggataagg gtaaggacgg 172440
tatggttaga ggtttttggg agtttttttt tttttttttt ttttagtagt ttttttttat 172500
tttttttagg gatttgtta tttttttttta gcgtgtggta gtttttttggc gtttttttcg 172560
tgtttttagg ttgtttttgc gtcgggtttg tcgttgggcg tttttatttt tgtttgtttt 172620
ttttttttgt ttttttcgtt ttgttttttt ggagtaattt tttatcgagt tttttttttt 172680
aggtagtcga ggtttttttt tatttttgtt tcgcgttttg ggaggttttt tgtttcgcga 172740
ttataaagtt tttttgattt tttgttcggt tttgagttat gtgattcggt gggcgggtcg 172800
cggttttcgg cgcgtttagc gtagttcgac gtttcgttgt tggggtgagt tttgtttttt 172860
tgtttttttt agttttgtat tattggttcg ggggttttta ggtggcgcgg tcgcgaggcg 172920
gattttgatg gttatggtgg cggtgtcggg agttacgttg tttttgggtt tcggttcgag 172980
gtcggtagga tcgagcgggg tttttaggag aggggtggcg gggagttcga tttttacgcg 173040
gggattagat tttcggtttt aaaatagaag aatagggttt tgtgtggtta tagttatttt 173100
tttgtaaata tttggttaat taagttgagt ggttaagttt ttttgttgtt cggagttttt 173160
tggaatatgt tttttttttcg taaggggttt ttttggtttt taggagggtt aggaagaaat 173220
tcgaattggt tatcgttttt tttaaaatta tttcgtttaa gttattattt ttttgggttt 173280

tcgaagatcg gttggttgga ggttggagat agttttaatg ttcgaaatgt cgtaatcgaa 173340
gtttttcgcg gcgtcggtat tgggatttag ggagttgttg ttatagcgta gttttggatt 173400
tttggatgtg ttggatatgt gtagggcgtt tttgggagga gcggggaggg agggtgttgt 173460
tggcggggtt ggtttgcgtg tgttttgttt ttttataatg gtatgttgcg tgtcggttat 173520
gtagaggtat gttagtgagt aggggttgag ggattttttt aacggatttg tttttagagg 173580
gtttttttat gttgggagaa ttttagagat taaattatgt agttaacggg gtggttttcg 173640
gttttaaagt agggaggggc gaggagtttt gtaggtaatg ttatttgttt ttgaaacgtc 173700
gttttagtga ttttggggat tgatttagtt tttagtttgt tgtattttat ttttggtttt 173760
tttttttttg tttttttatg tgaaatttgt tgtgttttgg ttagaggttt ggggaatagt 173820
tttttgttta tttaagataa gtttgtaatt tttttttttgg agaaaaatat gttattcgga 173880
agtaggttga gtagtggttt tttggtaggt ttgttcgggg ttgtggagat agtatttgtt 173940
ggattaggat ggagttggaa tttggtttgg attttatatg agaaaattcg ttggaagagg 174000
aggaagtaga aaaaggtttt atttttttaat aatgttgtgg gttttttttt tttatttatt 174060
gttattttgt tttgaaattt aatggggtta cgtgtgtttt tatgaatgtt atttttttttg 174120
gttaattttt tcggtagttt taggagtttt tttttaggaa aaggaagaag gtgtttgttt 174180
agtatttagt atacgagttt ttgttggaaa ggtgggattt ttgttttttt ttgggttttt 174240
tttaggttga gatgtggtta aggaaggttt gtggtgtggt tcgttttttt taatgttcgt 174300
ttttggagat ttgagttgtt gttgattttt agtttgtttt ttttgttttt tggttatttt 174360
tgtgtcgggg tggttgagat attattgtat tggttatttt gtgtttttat tagttttcgt 174420
ttttgtaggt tgttattgtg atggtgatgg ggttaattta gttgcggtgg ggaggattta 174480
ttgtgtgcgt ttgggggggtg gcgtggggta ttttgggatg agaagatgcg atttttgttt 174540
tgagagtttt gttttaatgg aaggcggggg tatagttgta ggtagaagtt attaagtatt 174600
gtggttagat tagtatttta tgttaaaaat ggttttgttg gtcgggcgta gtggtttatg 174660
tttgtaattt taatatttta ggaggttaag gtaggtagat tatttgaggt taagagttcg 174720
agattagttt ggttaatatg gtgaaatttt gtttttatta aaaatattgg ttaattcggg 174780
tatggtggcg cgtgtttgta atggtagtta ttcgggaggt tgagatagga gaattatttg 174840
aatttgggag gtggaggttg tagtgagttg agattgtgtt attgtatttt agtttgggta 174900
ataagagtga aattttgttt taaaaaaaaa aaaaaaaagt tttatttggg tttgggattt 174960
gtttttcgaa tattgggttt attgtgtttt ataaggattt ttatttcgga agttttagat 175020
ttatttgggt aattagagtt tttgggttga ttattttttta gttttggtta gtttttttggg 175080
.ttatcgtatt tttttaattt tttgttgtgt tacggttggt ttaagttgtt tttattagat 175140
aatgcgaggt atttttatag gaggggataa tttcgttttt aggagttttt tttttttgtt 175200
tttaggtttg gtgtagtgtg tggttttttt gtcggtgtta aaggtttaa ggtttttttta 175260
agaagtttgt tattattatt agtagttttc gttatttggt tttttttgtat atgtaagttt 175320
tttttatata tttcggatgt ttagggtttt cgtttttttt ttttggtcgg gagtgtggga 175380
agagggtttt attttaatgt ttgagagtag ggttaatggt tagggtaagt tgtgggggtt 175440
tttttttcgtc ggtttttttt tgtagttttt attttaatat agagtatcga tattatttgg 175500
gttttgggaa gatttggagg ttgattattt tggaggtttt tagcgtttta tagttatttt 175560
ttggggaaga ggtttttttga gggatttgtg gtttttggtt gggattttag ttttagagtt 175620
ttgtttttcg gcgtcgagtt ttgatttgtt tattgagtgt tagttcggtt ttcggatagg 175680
tagggatttg ttttcggagt ttagtagagt tttgggaagt ttttttagga gggttttcga 175740
gggtggcggt tatgtttagt tcgttgtacg tgtgagtttg tttgatgagg aattggttgg 175800
gaaggcgcgg gtttaattat aggtttatga gggttttttt cgtttttatt taggaagtag 175860
cggagatttt ggtatattta gtatttgggt ttgtgtttgt ttagttgttg gtggaattat 175920
gggagttttg tttcggggtt tgtagagtag tagtgggaat ttttttttatt ttagtttttt 175980
ttagagttgg ttgtttaaat tcgtcgtcgt ttagtttttt ttttttttt tttattaatt 176040
ttgggaatcg tatattgttt attttatcgg gtttcggagc gggtgtagga agagagagtt 176100
taatatttag aggtttgggt tttttgtagg cgtttgagtt gttgtggttt gagttgggtt 176160
ttttgttgga attattatag tagatttaat gatttgtttg taggagttgt tttattttag 176220
tcgatttcgg ggttgcggga aggggttggg ttttggtttt gttgggggtg gggggttagt 176280
gtaaagggtt tgaaaagtgg tagaggtgat ggtttatttg ttttttttt ggtcggtcgg 176340
tttaaacgcg attggttttt ttttttttt ttttttttt ttttttttt tataaataat 176400
attaaatgcg atttgttaat gtatttattt gggtttagta gttggattat gggtagaagt 176460
attttagatt tgttattttt tttaagttaa gggtggggaa tattttttgt gagattttgg 176520
tttttgagcg tttaagattg tttttggaat tttaattttg ggtcggtttt aggggattga 176580
cgttgatgga gttgttgata ttgatttcg gtttttgtat ggtttttagg aagggggttt 176640
ttcgtttttt gtttcgtcga gtttggttgg tagtattttt tttttaggtt tgtgttttcg 176700
gtagggtttg ttgttttcgt tttgttttgt gggtgttagg ttttaagtat taggtgattc 176760

ggttattgaa tggttattga atagttattt ttgttggtgt ttttagtgga atgtgtatgg 176820
acgttttttt ttttttagat ttttgttttt tattttttag tttttgtttt aggggttatt 176880
ataggattag gaggtacgta gacgtgggtg gtatatttat tgggtagatt tagttttgaa 176940
tttgtacgtt ttttttaagg gggtttttag cgggcggggt tgatggtgtc gttttttttt 177000
ttgttattag aacggatatg aggattgtgt tttgtcgatt tacgttttga gtttttagac 177060
gttataggtg tttgtttaac gagtgtttga atgaatggtt agtattgtgt gatcgtagat 177120
ttttaatttt tatttaaata gtttgtggtt tttgtgaggt tatttcgaga tagttttata 177180
agaattttta ttttttttgt acgtttcgta taaggttttt ttgggggttt ttttagaaaa 177240
agggttaaag agtagtattt tttcggggat ttgaatacga tttttttcgg ggtaggggat 177300
tgttttggaa gattttcggt atcgtttttt atgcgttacg tgattaggag ggttttgggg 177360
taggatggtg ttttttgtgt tttgtgtgtt ggggtcggag gaaatattgt tcgtggtcgg 177420
tgttatttgt tagttttgaa gattaatgat gggttggata gtggttttat tgagtgggag 177480
ggagaatcgt tgttgtttgt agttgggggg tcgggtgggt gggagtcgag gttagggaga 177540
atagcgtaga tttggtacgg agaggtttgg aggaggagta gggggtttta gttatttagg 177600
gtgtagggac gttatacgag gagtaggtgt ggtgtgcgtt tttttgcggg ttttttattt 177660
tttggtttcg ttatttgtat taggttttgt ggtgagaggg tatagagtgg aggaaggtgt 177720
cgtggttagg gtaggttata cgttgtgtta atggattgtt gttatttgtc gtttttgtgg 177780
ttttgttgtt aggatttttt agtattaggt ttttattatt aggtatgggt tttataggta 177840
ttattggttt ttcgaagtta gggatagatt ttatagataa tattgagttt ttgtagttag 177900
ggatgggttt ttacggttag tattagattt tagggattag tatcgggtat ttatagttag 177960
ggataggttt ttatggttag tattaggttt tagggattag tatcgggtat ttatagttag 178020
ggacgggttt ttatggttag tattagattt tagggattag tatcgggtat ttatagttag 178080
ggacgggttt ttatggttag tattaggttt tagggattag cgttgggttt ttatagttaa 178140
ggataagttt ttacggttaa tattagattt tagtgattag tattagatgt ttatagttag 178200
agatgggttt ttatggttag tattaggttt tagggattag cgttgggatt ttggatgatt 178260
gaaggagggt acggttgatt aaagttgtag gagttagttt ggttattttg aggtattaag 178320
ttattattgc ggttttaggg gatttagatt tttttatttg tttttttgaa ttttggaatt 178380
agatatattt gggtttagtt tttgttttcg gggttgtgtg tttgggatgg ttgttttatt 178440
tttttgaagt ttagtgagat ggggacggtt gtatagggtt cggtatagat ggtattcgat 178500
ggtatagggt tatttgtttg tatttatata gtttcggttt tcgttttggt tgcgtatcga 178560
gagttttatg tttagatttt tgttgagttt ttaagttttt ggttatgttt taggttttc 178620
gtgagaagta ttattgtatg gattatgttt cgggatttta ttttgggttg ggaagtttgt 178680
tttatttttt taagtttttt ttagaggcgt tttatgtttt attattttta ttgtttgagg 178740
ggttagatag gggaatttag tgtgggaggt ttagaggtgt cggagtagac gttaagcgag 178800
tttcgtgttt atgcgttggg ttttggtaga tattttgaat gagttttttt tttaggaagg 178860
agattggggt tttagtttaa gttgtggttt taaattttgt gattaagagt ggagaaaagg 178920
taagattttt ttttttagtt ttttagaata gtcgatagtg gtggaaatat ggggtttata 178980
ttgaattttt ttgtaatttg ttgaagagaa tttagtttat atttttaggg gtggaggttt 179040
ggtcgatttg gtagtgtgtt cgtttttttg gttatagttt attttggatc gggtattttg 179100
gtgttttttt ttagtgacgt agtgaagtta ttatgacgtg tttagttggt gagaaagtgt 179160
tttttgtaat gttttatttt ttttagaggg tgaataagtt ttgtttatta taaataatta 179220
tttatagaat atatgaggtt taatttaatt ttgggttacg gtagcgtttt ttgggtaaat 179280
ttacgcgtta ttttagttag agtttgtgtt tttataggtt ttttggattt attttgaagg 179340
ggggttggat tttttgtttg tttcgatgag agggttgtag gatagttttt agcgagtttt 179400
tttgagggtt gtaatttttt tttttggtat ttttttgttc gttaggaatg ttttaagttt 179460
ttagtagaag tttatttatt atagtcgttt tttattttgt ttattatttt ttttgttttt 179520
atttatttag tagatttcgt gtatagttta gtgtaggatt tagggtggga aggtgtttcg 179580
ggtgggttgg agtaaggttt ttagttaggg cggggtttta aggtgggttt atagttagga 179640
ttgtgatttg ggaggttttt ttttgttttg ttttagtttc ggtatcgtcg ttgtttttgt 179700
gtttagtgga gatttattgg ttttaggttt taaaatttta gcgaggtttg tagataggga 179760
ggagttttat tcgagggtag agtgattgtc gggtttattt gtttgttacg aattacgtgg 179820
ttttagattt gttatttttt tttttttaga gttatggttt tttgtttgaa aaatggataa 179880
tgatggttat tatgttaggg attattttga ggataaacgg ggatgaaatt gaagaggttt 179940
agtacggaat ttggatatag aagtgttaat tgtgtgttat tagttgcggt ggttgttaag 180000
gtttaattat atggtagtta cgggttgagt ggcgggcggg aagtggggag taggagtatg 180060
taagatgggt atttttgaag tttttgattt agtgagataa gtatatattt agttttaaga 180120
tggagtgttt taggaaggtg gagattgtaa aggttttgtg gcgtaggagt gtgagttgta 180180
tttttgggtt ttttagaatt ttattagagt ttagggagga ggttagttta gatataggggg 180240

taacgggaga gatgggagtg agaaaatggt ttggcgtttg gaagtggagt ttggaaaggt 180300
gggcgtgggg atagaggagg gttttggggg ttatgggttt atgcgtttag gaggtggagg 180360
tagtgaacgg ggttacggtc ggttttatac gtggtattat tagttttgtt gagtttgtat 180420
ttgtttttta ggttttttga gtgttattat tatttggtat tttttggttt gatttttgtt 180480
ttttgttcgt ttgggaagag tgagattatt tgtttaaagt tttttagttg tttttaatta 180540
ttttggttgt tttttggttt tgtttagggt tagtcgttga tttgaattgt ttggtgttat 180600
ttttttagat ttttggggtt ttgttttggt attaatagtt atttttattt ttattattta 180660
ttcgtatatt tatttagtag tgtttttttgg gtatttattt agtaatatta aggcgttgag 180720
atgcggttgt ggttaagatt cgggttcggt ttgtgaggat ttcgtagttg ttattttttt 180780
agagtatagt tttagttagg tatattgtgg ggtttagaaa gagaaattaa tatattgtgt 180840
tcgtttattt tgagttgtta taaggaatag tcgaggttgg ggggttattt gatttacggt 180900
tttgtaggtt gtataagtgg tgttagtatt tgtttggttt ttggggaggt tttaggaagt 180960
ttttttttat ggtagaaggt gaagggggag ttggtacgtt atatggcgag agagagaatt 181020
tttagatttt tttttttttt gaaatggagt ttcgttgtgt tagttaggtt ggagtgtagt 181080
ggtataattt tcggtttatt gtaattttag ttttttgagt agttgggatt ataggcgtat 181140
gttatcgcgt tcggttaatt tttttgtatt tttattagag atggggtttt attatgttgg 181200
tcgggttggt tttaaatttt taattttgtg atttatttat tttggttttt taaagtgttg 181260
ggattatagg tgtgagttat tttatttagt ttttttagat tttatagtaa ttagattttt 181320
cggattttgt gatcgtgggg agggtattaa attatttatg agggatttat ttttatgatt 181380
taagttttat ttttttttagg ttttattttt aatattaggg attatatttt agtatgagat 181440
ttggaggggga taacgtttaa attatattat acgtgatttt tttgtgcgtg cgttagtttt 181500
tatatttggt taagtagtag aagttttttt tttgtttttt gttgagagag cggtggggga 181560
gaaatgattt agggatggga ggttggtttt tcgaggggtt ttggtgtttg tggagtggga 181620
gtgcgggaag gtttttacgt tgtgtgtgtc gtgttgggtt ggttttttat agtttatcgt 181680
ggtgtttttc gaggattgtt ggggaggaaa gagttaatta atttggggat ttagttgttg 181740
aagttattat taaatttgag tagtttaggg gttttttttt ttttgggttt tatgttattt 181800
agtatagtgg ggtttaattg aatttttttt tattttttaa ggtttttggt tggcgtggtt 181860
ggggtttggg atacggggag cgagagggtt tgggtttttt ttagatttgg agtgggttta 181920
ttagaaatgg tttaggaaat cgcgggggtt atttttagta gttgggggtt tttgtggtta 181980
tttttttttg gttttgttga gtagttttgg tttttggagt ttttagtaag ggatgtgggg 182040
gtttgttagg ttagggtttt tattgttgtc gttatgtata gttgtatttt gtattcgggg 182100
agtggggtgt cgagtggggt gtttgggagg taggtttttt tttgtttttt ttgtttagag 182160
atttggggga tgagttgagt gagatttttt cggtttttga ttggttgtgt ttttgttgtt 182220
atttttttatt tttttaagt ggaggtttat gatttttttt tttatttaaa agggttattt 182280
agtgatttag tagattttag tttgtgttat ttagggtttt tttaaaaatt tgtggggagg 182340
ttgggtttgg tggtttatat ttttaatttt agtattttgg gaggttgagg cgggtggatt 182400
atttgaagtt aggagttcga gattagtttg gttaatatgg agaaatttta tttttagtaa 182460
aaatataaaa aattggttgg gtgtggtggt acgtgtttgt aattttagtt attcgggagg 182520
ttgaggtagg agaattattt aaatttagga ggcggaggtt gtagtgagtc gagattatat 182580
tattgtattt tagtttggac gatagagtga gattttgttt taaaaaaata aaaataaata 182640
aataaaaata aaaatttatg gggggatttt tgtgtttatt ggttggtgtg aggagagggt 182700
gaggagttgt agagaaggta ttaggttgag gatcgttttt atttttgggt tatagaattg 182760
aaggagggga tatatgaaag atgtttttag attcgggttg gaggttgggg atattcgagg 182820
gtaaggacga cgttgttttt gtttttgttt gtagaggagt gtatagggtt tattagtttt 182880
ttgttgttgt ttaagtgata gttttatgga gatagaattt atatattgtg acgttgattt 182940
ttttaaaatt tatagtttaa tggttttgtt gtatttataa agtgattgta ttagggttat 183000
ttagagaaat agaattaata gaaggtgtgt acgtgtgttt agataaagag atttattatt 183060
atgaggagtt ggtttatttt attatagagg ttggtaagtg ttaagattgg tagagtaaat 183120
tagtaggttg gagatacggg agagtttatg ttgcggtttt ggtgtttatt tgaaggttta 183180
agatttagga gagttaatgg cgtcgtttta gtttcgagat ttaggaaaag cgtatgtgtt 183240
agtttgagtt tgaagtagga aaaatttgat gttttaggtt gaaggcggtt aagcgggaag 183300
aatttttttt tatttaggtt ttgtgttttt ttatttagat ttttaggttt ttgttataaa 183360
attataaaga tttgattaga ataaaatttg agaaaaaatt tttttttttt atcggttttg 183420
gatttattgg ttgggatttt ggaaattaga ttgatagaga ttaataggaa ataagtataa 183480
taatttaagt aagttttatt tgatatcgag tttttataag gaaatgaaga tttagagaaa 183540
tcgttagttt gagcggtttt tgttttgttt tgagataaag ttttattttg tcgtacgttt 183600
aggttggaga gtagtggttc gattacggtt tattgtagtt ttgatttttt gggtttaagt 183660
tggggtttag ttgtcggttt cgggagttgt tttgaattta tttttaagtt ttagtttaga 183720

ttttaagta gttgggatta taggtatgtg ttatttcgtt tggttaattt tttatttttt 183780
gtagagatag ggttttatta tgttgttcgg gttggtttta aatttttggg ttcgagtaat 183840
tttttttgttt tggtttttta aagtgttggg gttataggtg tgagttattg tgttcggttt 183900
aggtttgaat agtttttatt tgttttgttt gtttttttaaa tttgtttgtt gtttgttttg 183960
agtagttttt ttgtttattt tttttgtttg tttttttgttt aggttggagt gtagtggtat 184020
gatttttgtt tattgtaatt tttgtttttt gggtttaagt tatttttatg ttttagtttt 184080
ttaagtagtt gcgattatag gcgtgtgtta ttatattcgg ttgattttttg tatttttagt 184140
agggatgggg ttttattatg ttggttaggt tgttttttaa tttttgattt taagtgattt 184200
ggttattttg gttttttaaa gtgttgggat tgtaggtgtg agttgttgtg tttagtttag 184260
gtttgagcgt ttttatgttg ggtttgatga agagtggaaa gtcgtgggaa attgtgatag 184320
agtaataaaa gatgagttga atagtgagtt cgtgggggtt tcggtaagat ttgtgtattg 184380
gggttttttt tagatttttt gtttttggag atgaggttgt tttttttcgt tgggtgtagg 184440
gagggtattt tttacgtgag gggtttatga tttattttag agaaggggta ggttagtgag 184500
tttttttttgg atttgttatt ttttaaattt ttttagttga aaatatttat tatgttaagg 184560
tattgtattt tggagtgtgc gttttaagtt ttgttgtttt taattgatcg ggtgaggttt 184620
atttttatta gagtatatag tttgttttat ttaggttttt gattgtattt tttttttgtt 184680
tgtttcgaga atatttatta gtagtgtttg tggttgtagc gtttatttta agataaattt 184740
gtcgggaagt attttgtttt tattattatt tttatattat tttagtatat cgatttttgga 184800
aataaaagat attattttgt ttatagtatt ttatttttag tagtggtatt tttttttaaaa 184860
aaaaatggta atttttagtt gggtgtggtg gtttatgttt gtaattttag tatttgggaa 184920
agttgaggta ggttgattat ttgaggttag gagttggaga ttagtttggt taatatggtg 184980
aaattttatt tttattaaaa atataaaaat tagttaggcg tggtggtagg tatttgtaat 185040
tttagttatt tgggaggtcg aggtcggaga attgtttgaa ttcgggaggt agaggttgta 185100
gtgagtcgag atcgcgttat tgtattttag tttggacgat agagtaagat tttattttaa 185160
aaaataaata aataaataaa atttaaaaaa aaatagtaat tttcgatcgt tgaaaatgtt 185220
aaattttaga gaatatagta tttttataag tgatgttaat attgtttttg attagttgtt 185280
ggttgaagat ttatttgatg aatttgattt ttttagaata gacgatttag atgattttgt 185340
tgttttgttt agaaataatt gtaggaataa tttaaaaaat ttttttttttt tttaattttt 185400
ttgttttttg agacggattt tttttgttgt ttaggttgga gtgtagtggt acgatttcgg 185460
tttattgtaa tttttgtttt ttgggtttaa gtgatttttt agttttagtt ttttgagttt 185520
agttgggatt ataaggtgtt tgttattata cgtggttaat tttttgtatt tttagtagag 185580
acggtgtttt attaggttga ttaggttagt ttcgaatttt tgattttaaa taattggttt 185640
atgtcggttt tttaaagcgt tgggattata ggcgtgagtt attacgtttg gttagttttt 185700
atatttaatt tttatataga gtatttagtt agatttgttt tagttttaaa gagcgtgttt 185760
atgtaaaatt aagtgagtgt tggtagtgag ttgaattttt taaaaggtaa aagggttaaa 185820
tattaaattt atttaaaaat atttttatag atatatttag aataatgttt gatcggttat 185880
ttaggtgttt tatagtttag tttagttgat atagaaattt ttttttttttt ttttgagacg 185940
gagtttcgtt ttgtcgttta ggttggagtg tagtggcgcg attttggttt attgtaagtt 186000
tcgtttttta ggtttacgtt atttttttgt tttagttttt cgagtagttg ggattatagg 186060
tgtttattat tatattcggt taattttttt ttttgtattt ttagtagaga tggggtttta 186120
tcgtggtttt aattttttga tttcgtgatt tattcgtttt agttttttaa agtgttggga 186180
ttataggtat gagttattgc gttcggttga tataggaaat taattattat agttatatag 186240
ttatcgttat taattttagt atattttatt atttttaaaa gagattttgt ttttattagt 186300
taatttttat ttttaatttc gggtttttag tttttagtta tcgtttattt gttttcgttt 186360
ttatgaattt atttgtttta gatagtttgt ataagtggaa tcgtatagta tgtggttttg 186420
gtgtttggtt tttttttattt ggtagaatat ttttaaggtt tatttatatt gtagtaggtg 186480
ttggtgtttt atttttttttt atggtcgaat aatattttat cgtggataga ttatattgtg 186540
tattttttta tttgttgatg gatatttggg ttgtttttgt tttttggtat aattttgttg 186600
tgaagaattt tgtagtgagt gttggtttat aggtttttgt gtgaatatgt ttttattttt 186660
tttgggtaat tgtaagatta tgtggtttag ttaattagtt tttaagttat gtgtttgttg 186720
agggatttag gtttggtggg ggtgtattgt tgtgtttggt tagttaggtg ttgcgagttc 186780
gtgtcgtagt tattttttttg tgttgtgttt tgaggttgtt tcgtcggtat cgggtattgt 186840
gagttttagg aggttaaagt ttttttttgtt ttattttgag tttaggtttc gttttatgaa 186900
ttaggatgaa tggatattga attaacgtag ggatgggtgg ggttggggtg gggacgtaga 186960
gtgatttcgg gggtttgtag ttgggttttg gttgttgatt tttagttttt tttgtttgtt 187020
ggagtttaga ataagttaga aaggggaaat gtaaagttcg agtgtttttt ttgtggtaga 187080
ttggtaggtt tgggtgggtg agttatttgt agttagttgt agcgtgagag aggaagtggg 187140
aggggaggga gagggtgagt ttggtgtaat ttatttttgg aaggaggttg ggttgaggtt 187200

gggggaagtg attttattt ttttgagagg atttttttt ggggttaatt ttaaattaag 187260
ggagataaga tttttttggg ggaggttgga agtgttgggg tttttaggtg ggtaggtttt 187320
ttagggttaa cgttttgggg aagttataag ttattatgtt tttggattgt tatatatata 187380
tatatatata tatatatata tatatatata tatatatata tacgagtaaa ttgtaatata 187440
aatgtgtgtt tatgaaagtg tttattttaa attattatat agtttgtaaa gcgaaggttt 187500
tggttcgtat ttgggtattt attgtttagt tagtcggtag gtagtttgta ttgtagtttg 187560
ttattatttt ttagagatgt agtcgaggag tttagttggt ttggagggtt tagtgggcgt 187620
agttttagga aagtggttag gttcgggtta ggtttttttt ttttttagt tttgggagaa 187680
attattatat gaatgtagtt tttttcgga gttcgggttg gaagggtttg ggaggggata 187740
ggtttttatt ttaggagttt ttaggtttcg gttgttgttt gtgttagttg cgtgagcgtg 187800
agcgagttag gggttattat tgggtggaag gaagaagcgg gtattttttt taggaggtta 187860
tcgttggagt tttatagtag gttaatttta ggttttgggt tttcgggagg aggagaaaga 187920
agagaagtag gaggtgtgga gggtaggtta ggattagaag gtatataggg tcggggtagt 187980
gtgggagatt ttgggggttt ttttatttaa ttagttaata ttatagcgtt tataagaata 188040
cgttattatt gtagttttat tattttgttt tgattttaga aggtataatt gatgatttta 188100
gggaacggag aatttgagga aggtttcgta ggagagggtt agatgcgatc gagtaaatgt 188160
gagtcgtttg taaattgtag agtgtacgta tagtatcggt tagttttggg gttagtgttt 188220
ttttggtgtt tagttcggtt tttgtatttt ttagtgttcg gttgggtttt gtgggggtta 188280
gggttgggtt tggtatatta gaggattttt attaagtttt tggtagtgtt ttagttatta 188340
aatttagttt gttagttatt aaattcgttt tgtcggtttt cgaattattt tttcgttggg 188400
gtcggttatg ttacgttagg ttttttatgt aggacggtag atggatagac gttgggatta 188460
agttattttt gggtcgcgag acgtttgatt ttttgttggt gttttagaag atgttagttt 188520
ttcgtggtat gtttttttacg cggtgtttgg ggattagtgg tttttaaggg gtttttttgt 188580
tttagcgtgg ggagtggttt ggttaaggtt ttagtgtttg gttggagtta ggtataggtt 188640
taggtgtatt ggttggaggt tttttgttta tgggttttgt ttgttttgtg tttttggttt 188700
ttattgtgtt ttttgtgttt ttgggagaga ttttaggggt tttgagttta gtattggggt 188760
tggggagtgg gcggggaaga gggtggggat tagggttttt ggttgtagaa tagttaggtt 188820
ttaagttttt attttatttt ttttttatcg ggcgttttat tttatattga ttattttttg 188880
aagaaaattt tttgaatcgg tttttttattt cggttgtttt aggaagttta tttatttttt 188940
cgttttaatg ttttgtggtt ttagaaggat ggtggaagtc ggaaattatg agtttttattt 189000
ataggcggtt atatatcgaa gtcgcggaga cgtcgtggga aaggattttg gttagcgcgg 189060
tcggtataga gggagcggtg ttatcggggt agacgggaat cgtggttgtt tttatgttgg 189120
tttgggagag acgagggggtc ggacgaaagg atagtagatt ggttatttat ttatttattt 189180
atttatttat ttatagcgtt ttattgggtg tttcggacgt gtaaggtttt gtgggtagtg 189240
aatgcgatag atttagata gtaatcggag ggttacgtgt gtaagaattg agggatttag 189300
gttgggcgtg gtgttttaag tttgtaattt tagtattttg ggaggtagag gcgggcggat 189360
tacgaggtta ggagatcgag attattttgg ttaatacggt gaaatttcgt ttttattaaa 189420
aatataaaaa attagtcggg cgtggtggcg ggcgtttgta attttagtta tttaggaggt 189480
tgaggtagga gaatggcgtg aattcgggag gtagagtttg tagtgagttt agatagtgtt 189540
attgtatttt agtttgggcg atagagtgag attttatttt aaaaaaaata aaataaaata 189600
aaataaaata aaaattgagg aatttagtgt agatgagtta tttcgggaaa ataagtggga 189660
gttttaggaa tagtatgggg aagggatggt ttcggtaggg aggattttttg tgggtagttt 189720
tgttggaaga gttgaaaggt agttagagag gttgtgcgtg gatggaagtt gaagagtagg 189780
ttaggggcgt gtgttttgtt aataaagatt ttaagtattt attgattagg tataatatta 189840
tgtgttttat aaataaatat tttatttgat ttttagtatt tttatgagat agatgttatt 189900
attttatttt atatgtaaga atattgaggt atagagtggt taagttattt gtttagggtt 189960
atatagtacg tcggtggtag gtaggatttt ggagttatag gaatttggta tattttaggt 190020
gtaatggaaa ttagttacgg ttttatagag ggaaattatt agattagtaa attttatata 190080
cgtttttttgg tgttttgagg aggagggatt cggatagggg tagttaatgg tggtggttgt 190140
gagatgtgtt gggttaatat tgatgagagt ttgggatgga tgagtggatt cggagtaatg 190200
gaaatggagg gtttaggttc ggtttttgga gatttagttg taatagttgt tggttggaga 190260
ggttgtttat ttagatgggg gatggggtgg gaggtaggtg agatgagagt tttagagttt 190320
agtttggggt acggttattg ggtttaagat gtttttagat gttggggttt gatagtgata 190380
gtggttgtag atatttagga tttagtagag ggtttggtat agcggggttt agggatacgt 190440
ttgtgagtta ggttggtatt taagtgtgaa tttggataaa gaaggtggta ttgttggtat 190500
tgaatgttta ggggttgggt gaagttagtt agggagaggg gtttttaagg tattaggatt 190560
gatttcgtgg gtgagggcgg aggaggagac ggtagaggtt gagagaatag ttagagatag 190620
gaggaaggtt agtagagtta tgtatagaag tttggagaag atttgaggaa aggaagggtt 190680

gggtggtcgt tattttttgt agttaggagg taagggtgat ttttgtgtgt tttatttttt 190740
agaggattgg tggttttatt tggttagaat tttaagttta gagatgtatt tgagttttat 190800
gttatggtgt aggtagtagg agttatggag taggtttgcg gggttacggt ttgtttgttg 190860
ttttttggtg tttttatttt gagtttaggg gttgtttgga tgttttgtgt tgtatttttt 190920
taggcggtgt tttagagagg atgaaagtgg ttattttaag gtgtgattag gtagcggttt 190980
atgtatattg tttttttttg gggagagttt gggatggaag ggtatgtgtg tgtttttgag 191040
atttttttgaa taggttttat gtatttattt atttatttta ttatttattt atttatttat 191100
ttatttattt atttacgtat ttatttattt atttatttat ttatttattt gttaatttat 191160
ttatttattt atttatttat ttatttattt atttatttat agttatttat ttatttattt 191220
atttatttat ttatttattt atttatttat ttatttattt gtttatttat ttatttattt 191280
atttatttat ttatttattt atttatttat ttatgtattt atttatttat ttatttattt 191340
atttatttat ttatttattt atttatttat ttatttattt atttatttat ttatttattt 191400
atttatttat ttatttattt atttatttat ttatttgttt atttatttat ttatttattt 191460
atttatttat ttatttattt atttatttaa ttatttattt atttatttat ttatttatta 191520
ttcgtttatt tatttattta tttatttatg tatttattta tttatttatt tatttattta 191580
tttgtttatt tatttattta tttatttatt tatttattta tttatttatt tatttattta 191640
tttatttttt tatttattta tttatttatt tatttattta tttatttatt tatttattta 191700
ttcgtttatt tatttattta cgtatttatt tatttattta tttatttatt tatttattta 191760
tttatttatt tgtttattta tttatttatt tatttattta tttatttatt tatttattta 191820
tttatttatt tatttattta tttatttatt tatttattaa tttatttatt tatttattta 191880
tttatttatt tatttattta gttatttatt tatttgttta tttattcgtt aatttattta 191940
tttatttgtt tgtttattta tttatttatt tatttattta tttgtttatt tatttattta 192000
tttatttatt tatttatata tttatatatt tatttattta tttaattatt tatttttatt 192060
tttattttag aaagtatttg aagttgttgg ggtgtgtatt tggaaaatgc gttataagtt 192120
tttataggtt tttttttggt tttttttattt ttttgattgg tttttttttc ggatatgtag 192180
gattttgagt agttttttttg ggaggtgggt gtagtttagg taggttgttt gtaaagttag 192240
gatcgaggga gttatattta aggtattgtg ggtttttaag gcgatggtat tgtgggttta 192300
ttgtttttag attttttttaa tttttaggtt tttgagtagg aggatttttg gggtagggtt 192360
tggagacggg aagagaaaat aatttttagtt ttttaatttt tgttagttta ggtgggtgtt 192420
gtatggattg ggggtatttg gaatagttgg ttttttttatt tttttttttt atttttttag 192480
tttttttggt tataattaga tagttgtgtg cgatgttttt tttttttttg aaggggttat 192540
ttagttaggt ttttatttat ggtttaggaa tgtgtttttt tttgtttgtt ttttattttt 192600
aagtaggtcg tgtttttagt tttttgttgt ttttgaaggg tttgggtatg gggtagtttt 192660
tgtagtattt ttagggttcg agttgatttg tatagtagaa gggaattggg ggaaagtttt 192720
tttttttattt agtatttggt agggggttat tttgttgtat tttagagatt tttgtttaag 192780
ttttgttatt ttagatgatt ttataagttg ggggaggcgg agttttagtt ttttatagttt 192840
ttgttgttgt tttattgttg agttttgttg tgtatttgtt gataggtttg ttgatagtag 192900
aaggggttgg agagagggaa gttttgggtt ttgttttttt taatttttgt ttaggggata 192960
tttttgataa gaggaaattt tagtttgtga tagatggtta gtgtttattt atttacgggg 193020
gaattttttt tttttttttgt aagtttgtag atttgttagg aattaaagga atttttggtt 193080
gataggttat tgtggatatg gttggagggt tggtacgggt tggattttgg ggttgttttg 193140
aggtaggggt cgtttttgtt ggaaggtttt ttggggtggg gaaggtatta gggttatttt 193200
ttgatttttt ttttgtagtg gatttatttt agagtttgag ataaggtaag ggggtagtag 193260
aaaatagaat ataaaatagt ttttttttatt atatatcgta ttagaagtta gaggatttat 193320
ttttggtttg tttttttttgt agtttaagtt ttgagagtgg tttttttagga agtgaatggt 193380
gatggtttgt aggttagagt ttgttttggt ttaagattga tgttttaata ggtcgcggag 193440
tttgggtgaa aaaatttagg ttagggaggt ggtgggttgt aaatgaaaga atagaggagg 193500
gttttttttta gaaagcggcg ggtggtgtga ggagtttttg gattttgttt aaatttgatt 193560
ttatcgattg gggttaattg aaatcgtgtt ggtagaaagg taaaattgtt tgagattttt 193620
tttttttataa tagaggagag gtttgtttga aagggtttgg cgagggaaaa gttggaggaa 193680
ttttggtcgg tagtaaaagt ggggattttc gtttttttgtt tcggggggcgt ttcggttttt 193740
gaatgaatga aagaggaggt tggatggaga tagatttata ttatttgttt tttttaggtt 193800
ttgtaaagga gtatataggg tttttgttgg ttttttttatt ttgttagtta ttaagaagga 193860
gaaaatatta atagggggaat tgtaaagttg tagttttgtt taagttttat agtggaaatc 193920
ggaaagagtt ttagttcgga gaaaaatgta gtttttttgtt tttgtaaatt cgtgtttttt 193980
tttgggaggt tagagttatt gtgttcgatt tttaagtagg gttttagagt gttttaggag 194040
gggaaggtag agttttagat tttcggtatt ttaggtttta attaggtttt gaggagacgt 194100
tttttgtttt atataaagtg tgaagtgtaa tttgatttgg agttagttaa taaattagat 194160

gagttgtgtg tattttaaaa atttagtttt tgattttta tttatatata tttgtataat 194220
ttggttattt atatttagtg tttttatttt tagaagagaa taggaagttt ttagtgatat 194280
ttatggagtg tttgtagtag tttgggtagg ttagtaggaa tataatgtag tgaaaagtag 194340
agttgagatg ggaaaatatt ttgtttttag agtttttag ggatgatatt ttttatcgtt 194400
tttgtttatt cgttttggag tttatttcgt aaatagtaat ttgtttttat ttgagagttt 194460
tttttgtcg gagtttaggg atggagagta gttggtttta gttttttttt tttattttt 194520
tgtttttag atttattttt tattttgaat aattgttttt aatttgtttt tttttttttt 194580
tagttgtaga atttagaatt ttgtgtgtgt ggttaaaagt ttagagagtg ttgtaggtaa 194640
tgttgagata attttattaa attttgtaag tttttattat attaagtttg tttgtttttt 194700
ttaattttta ttttatttta ttttatttta tagggatggg gtttcgttac gttgtttagg 194760
ttggttttga atttttgggt ttaaacgatt ttttcgtttc ggtttttag agtgttaagt 194820
tatttttta attgggattt acgggtttag ggattttttg atgaatcgtt taaaatacgt 194880
gtgtgtttgt ttgtttgttt ttaatgtata ttttttagg gagagttttt atttttatt 194940
agaggtttgt ggtatatata tgttttgtat tattgtatta gattgtgaat tttttatatg 195000
ttaaagattg ttttaaattt gttcggaagt ttcggttaat aaatatttaa ataaatgaat 195060
tttaaatttt tatgtgttat tattgttttt attttgatta tttttaagtt tgtagtgtta 195120
tttttttgtt attgtgaagt agatatttaa taaatatttg ttaaatgaat taatgttatg 195180
gttttggttt attaggattt taggattatc gtttattatt attgtttata gtttttttat 195240
tattttgttt tagagtagtt ttttttttga ggtttgttta tttttgtacg tgtgtttagt 195300
ttttagtttt gtcgttttat tattttttttt tttttatttg tatttttga attgttaata 195360
gttaataata gtttttttttt ttatttattt atttatattt gaagtgagaa gttattattg 195420
aggttttgt ggattttgtt agtttaattt ggggcggttt tttggaggtg gagtttttag 195480
atgagggatg gatattttgg tgtttgttgg gtagatttat ttgggagttt tggtaggggt 195540
tttagggtag ttttgttttt ttattttggt tgtgggattt ttggtgtatt agggtttttt 195600
aagtaagtat ttgtgttttg ggagagttat aggtaggttt gagagggttt ttggaatttg 195660
ggattttag ttcggtttag tagttggtat tattattttt tttgtaaggg aagttatgga 195720
aattaagatg ttaatgtgtt ttgataggtt tggggtggtt ttaagttttg ggaagagttt 195780
tggtaatttt ttttggttgg cgggtagaat tttttttttgg tagtttaggg ttttgttttt 195840
agttttttgg gggagagtaa ttgagggagg agttggaggt taaggtttta gtgttgagtt 195900
gtttgtatcg tagttgtatt ttttgggttt tggttgtagg aattttatat tttttagtt 195960
tttttatggt tttcgaagat ttggtttggg gaggggggtta gtagtttggg gtttatttat 196020
ttggttttaa attttggttt tatcgtttat tcgttttgcg atttttataa ttgataaaag 196080
ttttttcggt ttcggttttt ttatttgtga aatgggataa taatagtggt tattttatag 196140
gattgtgtga tgatttatta gttataatag tttttgagta gcgtttagag taatatttgg 196200
ggttcggtgt agtggtttat gtttataatt ttagtatttt gggaggttaa ggtgggagga 196260
tcgtttgagg ttaggagttt gaaattagtt tgattaatat tagtgagatt tattttttaa 196320
aaaaaaaat tagagtaacg tttggtatag agagttttta gtaaatttcg gttgttgtga 196380
tgagttttgt tttttattt gtttatttat ttattagatt ttttgtcggg gtttgtcgtg 196440
agttttacgt tgttttgaag aaagtcgggt tggggtgagc gtgatggatg aggtgtttgg 196500
ttttataaat tttgtgtggg ggggttgtgg tgagaggaaa tcgtatatat tattatttag 196560
ttattatcgt ggggagattg tttggacgta gagagtaggt tttgtgtggg agcggggagg 196620
gtaagttttg gttgcgaggt agggtttttt taggatttag tagggatttg aaggatttg 196680
taggtattta gggagatagg agaggaggag ggagtagttt tggtcggata ttgtttttt 196740
agtattgttt gtattaggga tttatttagt tttaagaagt tttttgtgg gggatagga 196800
gtatttgtta gtttatagga tttgaagtgt ttttatgggt ttaagttttt gggaaggttt 196860
gtaggtggtc gtagggttgt cgtaggggtg ttggttttag ggtttggatt taggggagtt 196920
tgtagaggga gggtagttgg aggttgtttt agtgtgtatt gttacgaggt aaagtaagga 196980
gattgttggg tttacgttgg gtcggggtgg atggaggtaa ggaagttttc gtcggggtaa 197040
gggtattagt tgtagatgtc ggtagttttt ttttggatac gggtttggaa ggttgatagg 197100
gtgtggtgag tgttatcggt tttttgtcg tggtttggtt agtggtttta taggttttcg 197160
tgggtaggag gaggatgatt ttgtattttg tttggttatt attggtagtg atagataagg 197220
tgtgtgggga tgtggttatt tcggttcgtg ttttgagagt taggtgtggg ttgggtttgg 197280
ggaagatagg ggaatggtat tgatttgatt ttgagtattt tgttttgggg tgtaggttcg 197340
gttgttattt ttttggagtt gggaatggta ttgggcggtg gtttagggtt ttttgtttta 197400
gcggtattat ttttgtattt ggttagtaag gttgatggga tttgtgtgga tattttggg 197460
acgtagatta ggtagtatga tatataggga ggggtaggta gagaagggcg gtttttgggt 197520
tttggttttt aagttaggtt ttgtaagttt gttgtgttaa gtttttttc ggtattaggt 197580
taggtgggtt agggttagtt ttggggttgt gatgagtgtg agtgttttta aagtgtgtta 197640

tgtttaatag ttgttttttt tttgggtatt gtttttagt tagatatttt atagttttcg 197700
ttttagttat agatagtaat ttgatttttt tattttcggt aggttttggg gttggggtaa 197760
gaatttgtta ttaagtgtag atgtttttag agggtgttgg tttagggttg cgtggattat 197820
agttttttta tttttgtttt agatttattt gtaattttgg agaaaagatg gtttagtgga 197880
cgtttgcggg gaggagggag ttggttttta ggatgagtag atttgggggt agtttttatt 197940
acgggatcgg tgatttattg ttattagcgt tagtgaatgg tagaggggtt ttggtataag 198000
gtttgggttt agaatgggaa tttgtagaga aattgttaag gtttttcgtt atttgatatt 198060
ttcgtttggt tgttatttta gaaatatttt tgttgtaaga agatgggggga gtttgtttgg 198120
ggtatggttt ggcgatatta atttgagaat tttaggttta agtgttggtt tttgggtttt 198180
agttgttttt tgagttgtaa agttggtttt tgggtatttg tggaaaatat tggatttttt 198240
atttatttt atttatatt ttgggatata tgtgtagaac gtgtaggttt gttatatagg 198300
aatataagtg ttatagtggt ttgttgtatt tattggattt tttaatttag ttttattaga 198360
aatgattgtt tttttttttt tttttcgttt tttttttttt tttttttttt tttttttttt 198420
ttttgtttt tttttttttt tttttttttt tttttttttt ttttttttcg ttttatggg 198480
gatataattt ttatattata ttattcgtcg atttagaaag tatagttgag tggtattttt 198540
ttataatttt ttataagtat tatttttaat tttagaacgt tttttttatt ttaaaaggaa 198600
acgttatttt cggtatatag ttattttttt tttttagtt tttggaaatt attaatttgc 198660
gtttgtttt tatagatgta tttattttga atatatgatt agaattttaa aagatgtgat 198720
ttttgtgtt tggttttttg tagttggaat gtttttagag tttatttgtg ttggcgtacg 198780
ttcgtgtttt tttatggttg agaaatagtt cgttgtgttg atttagtacg ttgggttttt 198840
ttacgtattt gttggcggat gtgttagtta tttttggagt cgggtgtgtt gtgttattta 198900
cgggttgtat taggagttgg ggatattatt gagtaagtta tagtttcggg tatttaggag 198960
ttcgtagagt aggagttggg agggaggtta ggaggtgtat ggtttgtttg ttaggttgga 199020
gagatttgta aaaagttatt ttgagttaga aattagagtt tgaggttttt gatttttagt 199080
tgcggagtcg ggagggaagt tttgtatttt gttttgattg tggggtgacg tgttagttat 199140
tgtttaaatt agaatttttt tgagagtcga cgtgggtatt gttgataatt agtttggata 199200
ttcgaaagaa tggaaagtag ggatttagat agatttttgt ataatcgtgt tcgtagtagg 199260
gcgaggtttg gaggtgcgat ggggatttta acggggtagt tgtcgttttg ttggagttta 199320
cggtttttgg ggggggttat aggtgttaat cgtatattat ataagtagga gtgtgaaatt 199380
ttagggttgt taaatttagt gagggttttg tgttggtttg gttatttata gtggtatttt 199440
aggaaaaaaa attattttat ggattttagt tttttttatta gtaaagagta ggttggattg 199500
gaattttgta ttatttagta tagttgttat ttatgaaatg tggttattta aatttaaata 199560
aaaatagaaa tttcgttttt tagttggatt agtttagtgt taagtgttta gaagttattt 199620
gtgaatatat tgtggtaatt ttttaaaaaa cgaaaattag aattatcgta gtattttata 199680
atttatttt tgggtgtgga tttaaaagaa tgtatagtat tttaaagagt tagagatatt 199740
tgtatattta tatttatagc ggttttgttt atagtagtta gcgggtagaa ataattcgaa 199800
tgttcgcggt taaatgaatg gagaaattaa aggcgggata ggtcgggcgt ggtggtttac 199860
gtttgtaatt ttagtatttt gggaggtcga ggcgggtgga ttatttgagg ttataagttt 199920
aagattagtt tgattagtat ggagaaattt cgtttttatt aaaataaaat tagtcgggta 199980
tggtggcggg tgtttgtaat tttagttatt tgagaggttg aggtaggaga attgtttgaa 200040
tttggggagg tagaggtttc ggtgagtcga gatcgcgtta ttgtatttta gtttgggtaa 200100
taagagtaaa attttgttta aaaaaaaaaa aaaaaagggt ggaatatata aggaatttga 200160
tttagtcgta aagaggaagt taggatattt gtaatagtaa gggtgaattt ttcgggtatt 200220
acgttaagtg aaataatttg gttataaaag gataattttg taggatttta tttatattag 200280
gttcgtagag tagttagatt tagaaaaata gtagaatgga ggttgttaga ggtttggggg 200340
atggggttgg ggagttgttt aatgaggata gtttgtttta taagatgaaa aagttttgga 200400
gatttgttat ataataatgc gaatgtattt aatattattg gattgtatat tttaaaatag 200460
tgatcgtggt aaacgttatg ataattaaaa ggaaaagtta cgtggttagt ggttattacg 200520
ttgagtagcg taggtttcgg acggtgtcgt ggttatagat aatggagtgg gtgattttta 200580
ttattgtttt tagttgtttg agtattatgg tgttttgggg ttaggatgaa agtgtttttg 200640
ggtatggggg tttttgtttt ttttatttgg gagttttgaa ggtagatttt aaagtttatt 200700
tggttaggta ggagagcgag gcgggttttt gtattgtttt ttgcgtgcgt gtttgtttgc 200760
gggttagtcg gggtttgtaa ggtcgttttt cgagggaggg gttgtaggaa tggtttttat 200820
ttgtgtagaa ggtaagttat tgttttacgt taatggtaaa gatggggttc ggttgcgttg 200880
tattattatt ttttaagcgg ggtttttggt gggtaggatg tttggtttga tttgagtttt 200940
tgtagtgagt gttggtgttt gtggagggat ttagttgtag ttgagtcggt ttttttttcg 201000
ttttgggttt tagggggcgtt gggggcgaga aaggagagtt tagggggtag cgttttttt 201060
taggttttgt tggggagggt tgagggatgt tggggggagg tgggggcggg taggggttgg 201120

ttttagtttt aggtaaaatg gtttttagat ttataggtag atggaagcga ttttcggtta 201180
cgtgggtggg gattgaattg ttgtttcgta aggtaaggtt ttatgtttgg ttttaggaag 201240
cggttttaaa gttatatggt ttatggttag atgtagggtt gtgtttttgt ttatttaaag 201300
gttttggaag tttaggggag agtatttttg aagggggagt aggagtagga gtaaagtttt 201360
gaaggttttt aggttgcgtg gtttgggtgg gggttacgtg ggggtataga gagaggagtt 201420
ttggttttta ggttcgtttg tgggtattgt ttttagtagt ggagatgtgt ggattttttt 201480
ttggtatttt ttaataagga attatattgt ttttttgttt ttttaatttt ataagatagg 201540
aatttttgtg ttttgtggag gttgagaaga cggggaggtg tttgtgagga taaattagtt 201600
tttagttttt ggtttttaga gttttttttt tttttatttt tgatgtttta ggtttgggga 201660
gttttttttc gatatttttt gatcgtttta gtgaaatagg tcgagaggga tggtttgaat 201720
cggttgggga tgtaggttgt agttaggggg tttagtgggt atttttttgag ttcggttttt 201780
gtttcgtaag tggaagggta gttgaattga agttttttat tgtttttttt ggtttggaag 201840
tttgtttttt attttttggg tagagtgagg agtgggcgtg ggtcgggagg gttgtttttt 201900
gtttgggagg gttgtttatt tgtttgttac gggggatgag tttttttgtag tttttcgtgt 201960
tgtggttttg gtttttacgt cggttttatt tttttgtggt tgtttttgtt gggtgtttat 202020
cggttttagg gaaaagttat tttttttttcg gaggattggc gggaggggtt ttgtttggga 202080
gcgtgtcgtg tggtttttgt tttagcgtag gatgtttgag gttagatttt ttttgggagg 202140
taggatgggg taaaagtttt ttcgtaggta gagtagggtt tggatgggtt taagtaggga 202200
cgggggtata gaatagtagt cgtttagttt gcgtgggagt tgagggttat taagggtagt 202260
tttagtttgt ggggattagt agaggtatta tttttgtgat tagcgagttg tttgtatttg 202320
tatgcgtatg tattagtgag tgtggcgagt ttgaggtgtg ttgggatttg gttcgtgtat 202380
tagttagttt tgatgggaat tagttttatt ttgggtattt ttggaatttt attgtgtggg 202440
agtatttggg tttttagtag gcgtttttcg ggcgttcgtt tttagtttta ggtttttgat 202500
tcgtgtgtgg tgttttaata gtagttagga gtagggttat cgaagggatt tatagataga 202560
agaggtttta gtgatttcgg gaagtttagt tttacgtgtt ttatatattt aagagtaaaa 202620
tttggttttg gtttgtgggt gtgtagttcg tgggcgttgg ttaggtatat aagacggtcg 202680
tttgggttag tttaggggga ggtagatgtg gtgtaggggt ttttttttt tatgagttta 202740
ggaatagttt tgtatttagg tattcgtttt gtatttagta tttttttttt ttttaggatt 202800
gttggttcgt tttatgggat tgaatatata attttatggg gatcgacgtt ggttttattt 202860
ttttttttta taagattatt tatgggggatg tgggaggcgg ggatatacgt tggttttatt 202920
ttcgtttttt ataagattat tttttttgtg ttttggttcg tggatcggtt tttaaggttt 202980
ttgagtggta gtaggcggta tagtttattt gagtagaggt gaaggagagg gttaggttta 203040
tttgggtggt tcgaggtagt gtggagggtt aggagaggag agtatttttt atttttgttt 203100
gagtagtttt ttagggtacg gagataggag tttagggtta gttttgtttt ggagcgtagg 203160
gggtttttag tttggtatag tttttagaag aggagatgaa aaagtttggt tttgggagtt 203220
tggggatgga gttttgttgg ggaggtagcg tggttgtagg atggtagttt tttagaggat 203280
attacggtta gggatattta ttcgggggaa tttttagggg tcgtttgggt aagtcgttta 203340
gtttttttga gttttagtgt tttagttttt ttatttaagg atgggtgtga taattatttg 203400
gtaggcgttt gtgtgtgagt ttttgtttcg ttttaatttt tttgttgttt ttttttagt 203460
tattgaggcg gtttttagtt gcgttggcga tatggtcgat atttttagag atgtcgggtt 203520
taagtaggcg tttgtattac ggaacgagaa ggtttcggtg gatttcggtt acgtggggat 203580
tgattttatt ttggagtaga tgcgtcggaa ggttatgaag tagggtttcg agtttaatat 203640
tatggtggtc ggtgagtttt tattttgtat gttatttaat taatgtcgga aattagtttt 203700
agtttttagg gcggtttata agtttttggg tttggttttt ttttttgtaa aatggggtag 203760
taattttgat tttaaggacg gattgttagt gatattgcgt gtgtatgtat gcgtttggga 203820
ggagtttata gtttttagcg gttttttaga ggggtttgtt ttgtatattt agggtttttg 203880
agatgatttt ggggagttta ggtcggtttg gagtaggttt tgatgagtgg tcgttgtttt 203940
tgagtaatga cgttagtttg tgtagaggt tttgtggtag ttgttttttt attgttgttt 204000
tagtttggtt ttttaggttt tttatcgtat tttatcgtat ttcggttaga ggtttttttag 204060
tagtttagtt ttagttttgt tttggaattg gggtttcgtg gtttttatat tttttggtgg 204120
tttcggagag ttggaggtta ttacgggagg tcgtcgatta gtacgatttg ggtagggtga 204180
gggggttagg ttggagttta tagtgggtcg tttgttgttt ttaaagtttt ttaggtttta 204240
tttaaatttt atagggattt tgtgaagggg gcgtttcgtt ttttaggtag aagcgaggtt 204300
tagggaaggt tgttaaggtt ttttagttta tagggtagag aatcgtagtt atcgttttga 204360
tttttagaat tgtgttttgt ttattttacg gggtttaggg aggggtttgg ggaagtgttt 204420
tcgttaggtt atattttaaa taaaggtgag tttgcggggt ttttgtaagg tgtttgggta 204480
ttttttaggt cgggtttgtt tttttgattt ttgttagtcg tgtacgtttt ttttgtttag 204540
ttttgtggac gtaaatttat atttgttagt tcgatatttt tttttttttt tttgtattga 204600

tttgggtttt ggttttttg gggttttggt gtttttttg ttttttgtag ttttatggga 204660
ttcggttgtt tatggttttg ttgtgtttat agttgtttgg aagttggtag tttgttttaa 204720
gggagggcgg aggtgtgggg ttttttttta cggtttttat tttgttttgt ttgttgtggg 204780
ggacgtgttt gtattttgag gatttagggt tgtttttcgg gaagatggtt ggagagaaag 204840
agaggggaga ggtttaaagg tagtggttag gttgggattt atttttgtgg gttacggagg 204900
ggttgttttg tttttagtt agggataggg tgagaaggat ttgggaattt ttgcgtttta 204960
gttatggtat aggtttttt tcggagtttt ggttttaagg agagggagag gtagtttagt 205020
agttttcgtt tttcgtggtt gttattgtga tagttttgtt ttttgggaat ggtgcgtagg 205080
aggttatttt atttgtttgg gttttttcgt ttttttaatt ttgagaagaa tagtggtttt 205140
gattttttat aaaattattt ttttttaggg atgtcgagtg aggaataaga cgtggggtag 205200
ttttgttttt tttttttttt ttttttatgg tagttgtgtt tatttgtaga ggtgtaggta 205260
aggtggaggg gtatttgttt ggaggtttat agatttcggg gagaatttta gttttgtttt 205320
aatttagcgg ggtgaggttt gtgtgttttt ttttgggttt tttttttgtg agttttagtt 205380
ttttcgttta taaaatggga ttattttata aagtgagatt aagcgagtta ggtttcgggt 205440
agggttttta ataggttgtg gttgttcgag aatttgtagt agtggtgatg gtggttgtgg 205500
tgatgaggat gatgggggtg gtaatgacgg tgatgatgat ggggtgatgg tgttagtgga 205560
gatgatgggg atgatggtga tggtggtggt gatagtggtg ggatggtagt ggtgatgtgg 205620
gtgatggtga ttgtgatggt ggtggtggtg gtgatggagg tgatggtgat agtgattatg 205680
agggtgatgg ggatggtggt ggtggtgatg gtgatagtgg tgggatggta gtgatgatgt 205740
gggtgatggt gatggtggtg atggtggtgg tggtgattgt ggtggtggtg gtgatgatgg 205800
tggtgatgat ggtgattgtg gtgatggtga tggtggtaat tgtgatggtg gtggtggtga 205860
tggtggtgat gatggtgatt gtggtgatgg tggttatgat ggtggtaatt gtgatggtgg 205920
tggtgatggt ggtgatgggg atgatgatgg tgattgtgat gggggtggtg gtggtgattg 205980
tgatggtggt gatgtgggtg gtggtggtga ttgtgatggt ggtgatgtgg gtggtggtgg 206040
tgattgtgat ggtggtggtg gtggttgtga tggtggtgat gtgggtggtg gtggtgattg 206100
tgatggtggt gatgtgggtg gtggtggtga ttgtgatggt ggtggcgatg gtggttgtga 206160
tggtggtgat gtgggtggtg gtgattgtga tggttgtgat tgtgatggtg gtggtggtga 206220
tggtgatgat ggtggtgatt gtgatggtgg tgatgggagt gatgttggtg attgtggtgg 206280
tgaagggggt gatggtggtg attgtgatgg tggtgaaggg ggtgatggtg gtgattgtga 206340
tggtggtgaa gggggtgatg gtggtgattg tggtggtggt aatggtgatg gtggtaaagg 206400
ggtgatggtg gtgattgtgg tgatgtgggt ggtggtggtg atgtgggtgg tggtggtgat 206460
tgtgatgggg gtgatgatgg taatgtgggt gatggtgatt gtggtggtgg tggtgatggt 206520
gatgattgtg atggtggtga agggggtgat ggtggtgatt gtgatggtgg tgattgtgat 206580
ggtgatgtgg gtgatggtgg tgttgatggg ggtgatgatg ttagtagtgg tggtgatagt 206640
gatggggatg atggtggtgt tgatggtgat agtggtggga tggtagtggt gatgtggttg 206700
atgatggtgt tggtgttgat gattgtgatg atagtggtgg tggtgatgag ggtgacggaa 206760
gtggtgatga tggcgggagt gatgggatga tggtaatagt agtgatggta ttgtgatgat 206820
ggtgttgatg gggatggtgg tgatgataag ggtgatgatg atggtgatga taataataaa 206880
agtaataata ataaagggat atattttgta gtattttagt tggtattttt tagatttaag 206940
gtgtcgtggt tggaatattg atataatttt tgatttgagg ttggttagga tatagatttg 207000
gtggatagag agagatggac gaggagtgtt cgtttttggg ttattaaata gtttaggtta 207060
gggattttta gtattagttt ttgttttttt aggagtttat attttttta gaaaggtgtt 207120
attaaagtta gaatattagt gtaatttagg gtgtttttg tgttgtgttt ttttgagata 207180
gggttttatt gttgtttagg ttggagtgta gtggtatggt tttgatttat tgtagttttt 207240
atattttggg tttaagtgat tttcgtgttt taggtatgtg ttattatatt tggttaattt 207300
ttgtgttttt tggtagagat agggttttat tatgttggtt aagttggttt cgaatttttg 207360
atttaagta atttattttt tttagttttt taaagtgttg ggattatagg cgtgagttat 207420
ttcgtttggt cgatttttgt tttttaaaa gatagggttt ttgttatttt ggagtggtaa 207480
tgattttagt ttattgtagt ttcgtatttt tgggtttgag taattttttt attttagttt 207540
tttgcgtagt tgggattata ggtgttcgtt attatattta gttaatgttt atattttgat 207600
gtagagatag ggttttttg tgttgtttag gttggttttg aatttttggg tttaagtaat 207660
ttttttgttt tagtttttta aagtgttggg attataggta tgagttatta tatttggttt 207720
agggtatttt ttttggtgtg ttgttagcgt agtttaaggt agttttgtgt agggtaaagt 207780
aatggggttt cgagtttgga ggggtgtgtg tgtgtgtgtg tgtgtgtgtg tgtgtgtgcg 207840
cgtacgcgcg cgcgtgttat atgtgatttg tttgtgtgtg ttgtgagagt tgtgtgtgag 207900
gtatgtgtaa gaggtatgtt tgaggtgagt tgtgtgtgtg ttgtgagttg tgtatgtgaa 207960
gtgtgtgtgt tgtgaggtgt gtgtatgagg tgtatgtgag ttgtgtgtgt tgtgttgtat 208020
atgaatgtta tttttttatt ttcgtttgtt ttagttattt gtggattggt tgtttgtata 208080

```
tacggttatt gtttttcgag tggtattatt ttgtaggtta gcgtttttta gtatatggtt 208140
ttatagattt tatgggatat gcgtgagttt gtttgatgtt tatattagtt ttacggggga 208200
aggattgttt attttatttt gtagattagg aaattgagat acggtgaggt taggtgattt 208260
gtttaggttt tggtcggtta gggaggagta gtttgaatat ttgggttttg gttgtaatta 208320
ttttggtgaa ttgtaggaat ttcggttatt tggtgtattt tgggtagttt tgggtttttt 208380
cgttagttcg gtttttgttt tgtttaaaat ttgagttatt aggagatttt ttgtttagaa 208440
agtataaggg gtataagaga tgtcgatttt tttaggttcg gtttattttt taggagagga 208500
ttgtggtttg ggttgtgttg gtattagagt tcggttgtgt gggtttatat agttattgga 208560
tattcggttc gagggtgaag ttttcggggg ttgtttggta gggatattgt cgggaggtag 208620
tttaggtatt gttgaatttt agattggaga gtttcgtgtt tgggtgggag gggttttatg 208680
tgtagatttt gttggttttt tcggagagat ttttgatcgg tttttttattt tttttttagg 208740
gtagagcggt ttgggtaaat ttattttaat taatattttt tttaaattta aaattagtcg 208800
gaagtcggtg tagtttattt tagaggagcg tatttttaag attatcgaga ttaagtttat 208860
tacgtacggt tagtggtcgg gagtgggttg ggggtgtagg acgtttttgt tttttggag 208920
tataggggtt gggggttaag attattatat atagttagtg gttaggggcg ggttgggggt 208980
gtaggatttt tttgtttttt tggagtatag agtggggggt taagattatt atatatagtt 209040
agtggtcggg agtgggttgg ggatgtagga cgtttttgtt tttttggagt atagggggttg 209100
ggggttaaga ttattatata cggttagtgg ttggggatgg gttggggatg tgggatattt 209160
atgttttttt ggagtattgg tgcggtgtga gggtgttcga gttaggtaat tttaggtttt 209220
taagacgggg attcgttgtg tttattattt ttagtatgtt gagtatgttg gggttttggt 209280
tgtgatgggt tagtgtttcg gatttttgtg ggtttcgtta tattgagttt tttatagata 209340
attgagtttt ttattaaaag tttgataggt aaggggtttt taggggaatc gttatcggtt 209400
tgtgtcggag ttggttgagg aaggttatta atttttcggt gttttttttgg ttgttatcgt 209460
tgtttatttg gggttgtttc ggggttagtt aggtgtgggg tgtttgtgag ggtttttcgt 209520
tttttttttg attttgcgtt cgtggttttg tgtagatatt gaggagaaag gcgttcggat 209580
gaagttgata gtgattgata tattagggtt cggggattat attaataacg agaattggtg 209640
aggtttttttt agggggagga gtattagcgg gggttttagg gtttttttgga ttttatttag 209700
agttagtttt agaggttttt cggttcgcgg gggtgtaggg tttattttttt gggatttgat 209760
atgttgttaa agtcgtacgt tttaggggtt tgaaaatttt ggattaaagt agaattattt 209820
ggggaatttt gttgttttta atttattatt tgaggttagt tcggggaggt tggtttggga 209880
ggtcgagggt aaggttgtgg tattttcgtt tagtaagtta gatttttttag ggtatgtatt 209940
ttattggaag gtggggtgtt gggttttgag ttttgggaat gtacgatttg tttggggagg 210000
gtattttatg tgtttgttga ttcgttttta tttttttacgt agttggtagt ttattatgaa 210060
gtttattaat gattagtacg agaaatattt gtaggaggag gttaatatta atcgtaagaa 210120
gcgtatttcg gatattcgcg tttattgttg tttttatttt attttcgtta tcggttattc 210180
gtacgttttt gtagtgtcgg cgtttttatg tcgggtgttt tgggtttttt ttgttttttg 210240
ggattgtaag acggtgttgt ttgttttgtt gttgggtgta gaggggttga gttaggggtt 210300
atggtcgtta gttatgaagt tgggggtgtg ttatgtttgt atttttaaag ttttatattg 210360
atttttatgt aagtgttttg tttagttttt atttttttagt tgcgttttgg ggagttgagg 210420
gttaggtagg ttttgggttg ttttggagtt tggtggtttg tgttttgttt ttttaggtta 210480
ggttttggta ggtggagtgg gagtttttag ttaagtattc gtagcggtga ttgatttttt 210540
tattcgggtt ttgttttatg ggattttttta gagggtttgg tgcgttttta gacgggaagt 210600
atagagatta gtgttggttt tttttagggt tggacggggt tgttgttggt gttggtgaga 210660
gtttttttttt ttcgtcggtt ttgttcgtat ttgacgtcgt ttttagagat tagtaagggc 210720
gggaggttgt atgtattttg gtaggtggat tttttatggt tttacggatt ttttgttttt 210780
agtttttttgt gtattttttg ttagatattt gtggtttatt ttgagttagg gttgggagga 210840
ggaggattag aggttagtag gagtggttgg tggaggtagt cgtgggtggt ttgtttgtat 210900
gggtgggtgg agaaggtttt tcgtatttgt tgttttagat gtattttttt gggcggagga 210960
agtttagggg tttggggaaa ttatattttg tgggtattag gtgtaggaag gagtttggtt 211020
ggtgggtagc gggttttatt tagagtttgt gggaaaattt aagtcggagt tttcgagttt 211080
atttttttggt attatttttt gtgggttagt aggagttagg ggtagggggt tagttcgttg 211140
tttatgggtt ttttttgaat tgaatatttg ttttttttagg tagaacggtt tatttgcggt 211200
tttgtggtag ttgttttagt tgttttaagt tttgttttag ttgaggttga tggtaggagg 211260
tagaagtttt ttttgatgtt agttgggtaa gtcgagggcg ggcgggcggt ttgtggtatt 211320
tttgttattt tttagtattg attttttggt tgggatttta gttaggtatg ttgtttggtt 211380
tcgtttaagg cgtttagttt tatggggtag gggcgggttt tggttatttt tgtttaaatt 211440
tttgttcgga gcgggttttt ttttagttat gtgtgtgatc gatttatttt tattgtggga 211500
aaagttcggt tttagaattg gggaagatgg tgttttagtt ttagggtttt gttagttttt 211560
```

gttatttata gttttttta tattagattt taggtttatt ggggttaagt aggtaagggg 211620
agtagttcgt ttggtgtagg ggagtagttc gtttggtgta ggggtatcgt atattggtgt 211680
tagtttttat ttttttgtt ttttttttt tagttaaagt tttatagtaa gacggttgtt 211740
gagttaacga tagtttttt gtgtggatat gtaggggtgt ggttgggatt ttagaattta 211800
tggacgaggg tgtttggagt tggttttggt tcgtttggtt tgtgtagtgg tgttatttgt 211860
gtttgggata tattcgtgtt ttttttgt tttatattta tagcgtggtc gattcggttc 211920
ggggttttgt ttttgttagt agggatttc gtgagtttcg agttagtatt tgagagtgtc 211980
gatatttgtt tgggggtggg tgtttttta ttgtttcgtt tttttagatg agttttacgt 212040
atattttttt gtttagttt taggtttttg gatatcgagt ttatgaaacg tttgagtaag 212100
gtggttaata tcgtttttgt tatcgttaag gcggatatat ttattttgga ggagagggtt 212160
tattttaaat agcgggtagg gttttatttt tatttgtttt agttttttg tgtaatttgg 212220
agacgttgta ggtttggtag gggttatttc gtttaaagga gttatattgt tagggagatc 212280
gaatcgttgg ttattttttt tagcgggtgg ttggtttggt ggtttttggt ttaggtggtt 212340
gttgggggtc gtttttgaga tggtttttt agtttttta tattttaaaa gtagttttgt 212400
gggagtgtta atttttagt agagttttg aggggggttt tggagaagat ttggattagg 212460
tagatggtga atttttttt tagggagtga ggtcgagtag gggtcgtgcg atatggttta 212520
ttttgtttta tgttttttt ttttttttt gagaaagggt ttggtttagt tgttaaggtt 212580
gtaatgtagt ggtataatta tggtttattg tagtttttat tttttaggtt tagttttcga 212640
gtagttggaa ttatagacgt atattattat gtttggttaa tttttgtatt tttagtagag 212700
acgaggtttt gttatgttgt ttaggttggt ttttaatttt tgggtttaag ggattttttt 212760
gttttagttt tttaaagtgt tggggttata ggtatgagtt attttatttc gttttgtttg 212820
gtttttatta gatggaagag aagttgggtt aggtgtggtg gtttacgttt gtaattttag 212880
tattttggga ggtcgaggcg ggtagattat ttgaggttag gagtttgagg ttaatatgat 212940
gaaattttat ttttattaaa aatataaaaa tattagtcgg gtatggtggc gggcgtttgt 213000
agttttagtt atttgggagg ttgaggtagg agaatttttt gaattcggga ggcggaggtt 213060
ttagtgagtc gagattgagt tattgtattt tagtttgggt gatagagcga gattttattt 213120
taaaaaaaaa aaaaaaaaaa aaagttttag gagggatggt ttttgttatt tttttttcg 213180
ttatcgtttg aggtttttgt tgtgggatag ggaggggtat ttaagtaaga gggtagtttt 213240
gggttttttt ttttgatagg gtagtatatt agagttatat ggggtgtttt ttaaaatgaa 213300
atcgaggtgt ttgggttgcg gtattatatt tgtttcggtg ggtgtgggtg gggtttgtaa 213360
tttatttcgt tttggggtta ggtgttaggg atttttttag tatgtgtagg ggaagatagt 213420
ttatataaag tgggtgtgtt tgtcggaggt tatatacggg ttgtggtttg tgtttgggta 213480
ggcgagtagt cgtttattgg gatgtttttg ttgtacgtat ttatgtcggg ttgttggtag 213540
ggagttgaga ggttattgta ggcggggttt ttgttggaat tggggattgt gacgagggtt 213600
ttttaggaag ggttttagga ggggaggttt cggtaatttt gagtattttt ttggggttgt 213660
gatgagtagg agaagagagg tgggaggtgt ttaggacgat gattttaagt ttttggggag 213720
ttgtagcgtc gtttaggata gtaggtgtat ttgtagttgt ttttttttt tttaggaggt 213780
ataggagttg gaggtgattg gtgttatagt tttttagagt ttgtttttga attcgagttt 213840
ggggtagtat atagtgtgga ggttatgtgg taaatattag tgggtgggta gagtttgtta 213900
tagggatggg tttatttttt ttttttttta ttttagatta tcgtagattt gttgtttaac 213960
ggtatcgacg tgtattttta gaaggaattt gatgaggatt cggaggatcg gttggtgaac 214020
gagaagtttc gggtgagtgg atttattagg atgttgtttt taggggattt ttagtttttg 214080
ttgaagggtg ggttgggggt ttggaggatt ttaagttatg aaattatatt tttggggtta 214140
gagggtattg agtttaggtg tttgtattta gtgttgttag gttgaggttt tcgtttttgg 214200
gggattttag gtttagggta gttttttcg ggggtttagg ggagggaatt gtttttcgt 214260
atatgtgtaa ttaatatcgt ttgttcgttt tttaggagat gattttattt gttgtggtgg 214320
gtagtgatta cgagtattag gttaacggta agaggatttt tgggaggaag attaagtggg 214380
gtattatcga aggtattcgt cgtaggcgtc ggggtttag atagatggga agatagtttt 214440
ttttgttgat ttagagtttg tgggttacgt ttgagttagg gattcgtgga attttattta 214500
ttgttttgtt ttatttagag ggaggggttt ttttgttttt atttaatttt tgggaaagtg 214560
agagggtaag tggtttgtgt agatgttttt atttagatt ttttttcggg tagggaaaga 214620
tttagggaga taggagttgg gatgggggtc gttaagtttt ggattttggt gtaggttttt 214680
ttataggtat ttgtgtggtt ttggataaat cgtttaattt ttttgggttt agaaaagggg 214740
tgggttgtag ttttgtgagt tagatggatt ttcggttttt tattttgttg aagtttttgg 214800
gatttttata ttttagtttt ttgagtggtt gttgtaatta attagtataa acgaggggat 214860
ttaaaataat ataaacgtat ttttatagtt ttagaggtta gaggttcgaa attaggggtt 214920
gtttttttt gaggttttga ggattagttt tatttatgtt ttttttttt tggtggcgtt 214980
tggtattttt agttgtattg taaaggtatt atatgtttta atttttgttt tattgttata 215040

```
gggttttttt ttttgtgttt ttgtttcgtt tttttttttt tttttttttt tttttttttt  215100
ttgtttgaga cggagtttcg ttttgttatt taggttggag tgtagtgttg taattttagt  215160
ttattgtaat ttttgttttt tgggtttaag taattttttt gttttagttt ttcgagtatt  215220
tgggattata gttacgtatt atttatttga tttaattttt gtatttttag tagagatagg  215280
atttcgttat gttggttagg ttggttttaa atttttgatt ttaggtgatt tgtttatttt  215340
gtttggtttt ttaaagtgtt gggattatag gaatgagtta tcgagtttgt tttttttttt  215400
ttttgttttt ggataaggat gtttgttttt ggatttaggg tttattttaa gtggatgatt  215460
ttatttggag atttttaatg aatttataaa gaaagacgtt ttttaagtaa ggttataatt  215520
atgggtttta aggtttggat tttgattatg tttttgaatg gatgggagtt tttgggggt  215580
tatagtttaa tttattttttg gcggtgatat ttcgggaggg atgggttcga acgttttttt  215640
tttttttttt tttttgtttt tttatttttt attagaatgg aagaggggaa ggtgtagagg  215700
gaaatgtagt aggaaaagtt attttgtttt gggagagtat ttggttgaaa ggtttagtag  215760
agtaggaagt ataagttttt taattttta gggttttagt ttttattatt tataaagtgg  215820
gtgtagtgtg ttaagatttt agtgagttga gagattgttt taaagattat agagtttttt  215880
gggaagttgt tgttttaaaa aaatggttat aatgataatt attaggaggt attagatatt  215940
tttgtgttat tggttagata tgggttattt cgttttatgt tcgaagtttt tcgtatttat  216000
ttttttgta gagttgaaga ggtgttgtga gtagaaaatt tgttaaggga tttagaacgg  216060
gaggcggttt tagaatttat agttttagtt ttgtattaga tttttcgag atgggagttt  216120
atgtttggag gagggtgtgg agaggtatac gggttttttt tttgtttgta tttttttttt  216180
attattattt ttgtcgggtt tattttgcgg gagttgttta gttgggtgta gtttgggtgt  216240
tttttagtat tcgtttgtgg tgttaggttg gtttgttttt ttttggtttt tttgtttgta  216300
gttttttttt tttttttgt ttaaagagtt tttgttgttt aggaaatttt ttatgaaagg  216360
taggtttggg agagttaggg atggatgggt ttgggacggg tcggcggttt tgttatgggc  216420
gtgttttgt tgtattttgg tggttaattt tgagtatcgt aggtggtcgg tgatagaaat  216480
tgttaggaaa tgtataagag agaggtttc gtattttttt gatttgtgcg ttgttgagta  216540
ttattggttt tagttgagga agagtttgga atttgttagg atagttgtga ggtggtgggg  216600
tttattttt agtagtttta tatgaggatt ttggaaatgt gtttttagta ttgttgggtt  216660
tttatattag gggttttatg agtttatagt tgatttgata ttcggattag taagggttgt  216720
gttttgtttt tgttttttat ttggtgaata gtattttgag gtatttttt tatttttaga  216780
aattatttta agtattgtta atagaaatgt ggggtgtaat tgattacgtt atgaatttat  216840
ttaattttta tgtttttgag aggcgggtga cgttattatt tatattttat ggatgaggaa  216900
attgagggaa tgagagtaat ttgttcgagt tagggagtgg cgattatata ttttaatttt  216960
agtttgagag ttttattttt tttgtcggtt ttcgttgttc gttagtgtta gatgagagtg  217020
ttgttggtta gtttgtagga ggttttagtg tggatatcgt tttatatagg gtcgtggtag  217080
gagggtttag gtaaaggtaa tcggtatagt attgagagta tttgagggtt gggtaaggtg  217140
-gaggtttcgg ttttgtttgt tggtttatta agtaaaaatt agatattttt aatttgggga  217200
aaagttatgt ggttttgcga tggggtagtt tggagttagg tgtatgggtt ttgtatttcg  217260
ttttagtttt atgtattgtg gagttgagat agatatagtt tttgcgtttt ttatgtttta  217320
tttttttttt tttttttttt tttttttttt tttttttttt ttgagacgaa gttttatttt  217380
tttgtttagg ttggagtgtc gtggcgtgat tttggtttat tataattttt atttttgggg  217440
ttttagtgat tttttgttt tagtttttg agtagttggg attataggcg tttggtatta  217500
ggtttggtta atttttgtat ttttaataga gacgagattt tattatgttg gttaggttgg  217560
tttcgaattt ttgatttcgt gatttattta ttttagtttt ttaaagtgtt gggattgtag  217620
acgtgagtcg tggtgtttag cgtttacgtt ttattttcgt aagataatgg ggataataaa  217680
ggaattataa gagatatttg taggtgtgtg aagacgaagt ttattatatg ggagggtatc  217740
gggtgttttt atttgttttt tttgttcggt tttttatttt tagtgatgtc gtttattgtt  217800
tttttgtta aaaggttttt tgttatataa tagttatata tgttaggtat cgcgtgttta  217860
tataggagtt acgaagtata gtaataaaat gattattttg agatggttat tagttcgagt  217920
tcgagggtat ttgtgggtgt tcgtggtttt ttgtttagtt tttttgagaa gtggttattg  217980
tttcgagttt gggtttgtat gtattgtgtg aagtgtatat gtttttgagt aatgtttagt  218040
attgttgatt tttgagtttg tgaatggtcg tttatagtac gtagttttg tgtgtgtttt  218100
tgtgtgtgtg tgttttgagc gtatataagg tggatggggt cgtgggggta atattttgtg  218160
ggttataggt tttttttaaa attttagttt cgatttttgt tttattttat ttttagtagt  218220
ttagtttttt gtttgttttt aggtttttgt tgggacgttt tgtttttttt ggttatttta  218280
gtggtgcggg ggtttattgt tttggttttt ttattgtggt tttttttat gttttatttt  218340
ggtttggtta ttgtgttatt ttttattagt taggaatttt ttggagagta ggagttgagt  218400
tgtttaggag taggagttgg tgttggttat tttagtattt tttagattga gggtagtgtt  218460
aggtgtttta tatttagttg tagagtaggt ggttttttg ttttttgtt tatggggttg  218520
```

```
tttttagatt ttgtttttgg tattagtatt tttgggtagg gggagagagg tggggttcgg 218580
gtagagtttt tttattggga tattaaagtt ttttttgttt tttttttag ttgaaaatat 218640
tatatattgt gagtttgttt atttgcggga tttttttatt aggtgagaga tagggtgttt 218700
gggtggggtt gacggtttta tttttaagag ggttttatag cgggtggggg tagtgggtgt 218760
ggggtcgaag ttttgggtag agtgggtgtt ttttgttatt tgtttgtttt gttttcggga 218820
agattttgga agtttatttt ttgaaaaaga aaatttattg ggaaatgaag taggtagtga 218880
agattttttt taatttttgc gaagttggtt ggatgggaag gttgagtttt gatttattgt 218940
ggtttggttc gttagagttg ttcggtggtt attaagggtt tagtgaggtt ttatgtgtag 219000
gttttgtcgg tagcgtgggg cgtttttagt ttcgatttta ttggttttg atttttattt 219060
tttttatttg gttgaggttt ttttttgttt tttttgagtt taggtaagga ggggaagttt 219120
tgatggagag ggtgttgggt gggtggtttt tggagtgtgg gttttggttt gggtaggttt 219180
tttttagta agtagttggt atggatgatg ggaatgttat taagaggagg ggtttttagg 219240
ttggcggggg taggggtggg gggatttcgg gagtcgtttt gttattgtgg ggatattggg 219300
tttttaatt ttttttaga ttattttttt tgagttgtag aggaatgaaa attcgagttt 219360
aggatttttt ttttttgggg ataagggtaa tttagttttt tgttttaggt tatttgtttg 219420
ttataggtta tagttttgga ggtcggtggt tatttattgt ttttatttg ttagtatttt 219480
ggatatcggt ttggagtaat aatgttttag ttttgttttt ttgagtttat gtattgtttt 219540
tgggtttgg atttagggtt atagggtgga tttagaggga gatatatgta ttggaatatg 219600
tgtgttttga tcgagtttgg gtttattagg gggattgatt ttttttttaa aacgtgtatt 219660
agatttggtt taatatggtg ggtttggggg attttatggg gagttaagta gtcgggatgg 219720
ggagtggggg tgggggtagg cgggtttgag ttcgaggtag gtcgagtagg gtttttgttt 219780
cgttgttttt atttcgttgc gtttatttta ttgattcgtt cgtttttttat ttttatagga 219840
cgtatatgta gaatattaag gatattatta gtagtattta tttcgaggcg tatcgtgtga 219900
agcgttttaa cgagggtagt agcgttatgg ttaacggtat ggaggagaag gagttagaag 219960
tttcggagat gtagacgtta ttttgtttat tttcgggatt ttgtttttaa gttattttcg 220020
tttttttta ggttttttta ttattttatt ttattttata tgattttttt tatttgttat 220080
cgtttttttat tttttcgata tgttgttagg aaataaggga aggggttttt tttcgagtga 220140
gttagtgatg aggtcgcggt tttttcgagg ttgtggggag gttgtattgg agttataggt 220200
aggggtgaga gtatttattg aattgatatg atttttttgtt tttaggtttg gtttttcgag 220260
ggtttagaag agtagtttcg gtgtgtagat tattcgtttg tgtggggttt ttagtgtcgg 220320
aggttttggg gtgggggtta ggtttcgtat ttgtagagga gtttagtggg ttgtacgttt 220380
tttttattt ttatcggttt tgtttttttgg agtgtgttag agtttagggg agaatgtagt 220440
ttattaggag tatttggatt ttttgttcgt tatatggtgt ggttattatt tagtttttat 220500
ttttgttttg tttttaaggg tagaaaattt tagggttttt tgttatcgat tgtttagtta 220560
ttttaagttt tttggtagtt gttttttttg gagtagaaag tgtttttatt ttagttattc 220620
gtagattgtt tggttagatg cggggatagg ttggaatgag ggaggcgttt ttattttttt 220680
gttatttttt ttttacgtta ttttcgtttt tatcgggttg taggtgttgt tgatgcgttg 220740
ggatttgatt gaggataaaa aggaaggaga gatgattttt atttttttat tttttagttt 220800
tgaaaaggtt taagtaagtg agtttggtgg aggagttgag ggagggagtt atggaaggtg 220860
ttagaaggaa ggttggcggg ggtacgtgtg ggtcgtggtt tgggttggtt agagtggcgt 220920
gagttgttcg gcgtttgttt tgtttaagtg attagggaag tgtgtgtgtg tttatgtgta 220980
tgcgtgttcg tttgtttgtt tagtgtttgg gtttggttta agattgggtt gtagttatat 221040
taatgtttag ttagttattt ttgttattta ttttttttgg tttaggtttt gttgattttt 221100
tgagttgggg aggtgggagg ttaggcgagt ttgattttgt tgatttattc gtgtttgggt 221160
tttttttttt agagtttatg gtaacgaatt tttagaaagg agagaacggg cgttaggggt 221220
gtatagttta tagttgaaga gtaaggtttc gtggtagtac ggttcggttt tttatttttt 221280
gtttttacga ggggatttat gggggttgtt tttgtagggt atagatgatt aaagtttttt 221340
tttgtttttt gttatttgtt ttgtttttgg ggagaaagag gggtttgatg agatttttatt 221400
taggtgtata tattattagg tgtatttgta ggtatcgggt tggttgtttg tagttaggag 221460
aaggttagcg agaaggagtg tatgagtgtg agtgtgtgtg tatggaagtt ggggtattgg 221520
gcgtttgatt ttttttttat ttaagagagg aaggattttt tattattttt attggtgaga 221580
tagtttattt tgttaatttg ttatgttttt gttaaaatta agattttgaa ggaaatgagt 221640
ggttagcgtt agggtttagg ttatgtggtt tgtttagttt taatgttatt tggtggcggg 221700
gtggggtggg ggtgggtagt agtattttag ttttgagatg ttttattttt tttttttgta 221760
attagatttt gaaaaattgt tcgttttatt aggttttgtt ttttaatggt tttttgttac 221820
gtgtttttcg agaaatttgt ttttttgtat aaatgataaa gtgttgaaaa tgtatttttt 221880
gaaataaatg ttttaaatgt agaaattta 221909
```

<210> 6
<211> 221909
<212> DNA
<213> Artificial Sequence

<220>
<223> chemically treated genomic DNA (Homo sapiens)

<400> 6

```
tgggttttg tatttgaaat atttatttta ggaaatatat ttttaatatt ttgttattta 60
tataaaaaga taaatttttc gggaggtacg tagtaaaagg ttattgagga atagagtttg 120
atgaaacgaa taattttta aagtttggtt atagagaagg aaagtgaagt attttaaggt 180
tgggatgttg ttgtttattt ttattttatt tcgttattaa gtgatattga ggttgggtag 240
gttatatggt ttgggttttg gcgttggtta tttatttttt ttaaaatttt ggttttggta 300
aaaatatggt aagttggtaa agtaaattgt tttattagtg ggggtggtga ggggtttttt 360
ttttttggg tggggaggga gttagacgtt tagtgtttta atttttatgt atatatattt 420
atatttatat atttttttc gttgattttt ttttggttgt aagtagttag ttcggtgttt 480
gtagatgtat ttggtgatgt gtgtatttga gtggagtttt attaggtttt tttttttttt 540
taggagtaag ataggtaata ggaagtagga agggattttg gttatttgtg ttttgtaaag 600
atagttttta tgggtttttt cgtggggata gagggtgagg ggtcgggtcg tgttgttacg 660
aaattttgtt ttttagttgt ggattgtgta tttttgacgt tcgttttttt tttttaggg 720
gttcgttatt atgggttttg aggggagggg tttaggtacg ggtagattaa tagagttagg 780
ttcgtttggt tttttatttt tttagtttag agagttagta aggtttgggt taggaagggt 840
gagtggtagg ggtggttggt tgggtattaa tgtaattata gtttagtttt gggttaaatt 900
tagatattag atagatagac ggatacgtat atatatggat atatatatat ttttttggtt 960
atttgggtag ggtaggcgtc gggtagttta cgttattttg attagtttaa gttacggttt 1020
atacgtgttt tcgttaattt ttttttggt attttttatg gttttttttt ttagtttttt 1080
tattagattt atttgtttag atttttttaa aattggggga tgaggggta ggggttattt 1140
ttttttttt tttattttta attagatttt agcgtattag tagtatttgt agttcggtgg 1200
gggcgggggt ggcgtgagag ggggatggta gggagatgaa gacgtttttt ttatttagt 1260
ttgttttcgt atttggttaa gtagtttgcg gatggttgag ataaaggtat tttttgtttt 1320
aggaggggta gttgttaggg ggtttggagt ggttgggtag tcggtggtag gggattttgg 1380
agttttttat ttttaggagt agggtagggg taggggttga gtgatggtta tattatgtgg 1440
cgggtagggg gtttaggtgt ttttggtggg ttgtattttt ttttgggttt tgatatattt 1500
taggggatag ggtcgatggg gatggagggg agcgtgtagt ttattgggtt tttttgtaag 1560
tgcgaggttt ggtttttatt ttaaggtttt cggtattgag aattttatat agacggatga 1620
tttgtatatc gaagttgttt ttttgagttt tcggggagtt aggtttgggg atagagggtt 1680
atgttaattt agtgggtgtt tttatttttg tttgtggttt tagtgtagtt tttttataat 1740
ttcggggagg tcgcggtttt attattgatt tattcggagg gaggtttttt tttttgtttt 1800
ttggtagtat gtcgaagggg tggggaacga tgataaatgg agaaaattat ataaaataaa 1860
atggggtggt gggagggttt gggggggac ggaaatgatt tgggggtagg atttcggggg 1920
tgggtagggt ggcgtttata ttttcggggt ttttggtttt ttttttttta tgtcgttggt 1980
tatggcgttg ttgttttcgt tgaggcgttt tatacggtac gtttcgaagt ggatgttgtt 2040
ggtgatgttt ttgatgtttt gtatgtgcgt tttgtggggg tgggggcgg gcgggttagt 2100
gaggtgggcg tagcggggtg ggggtagcgg ggtaggggtt ttgttcggtt tgtttcggat 2160
ttaggttcgt ttgtttttat ttttattttt tatttcgatt gtttggtttt ttatggggtt 2220
ttttaggttt attatgttgg attagatttg gtatacgttt tgggggaagg gttagttttt 2280
ttggtaagtt tagattcggt tagaatatat atattttagt gtatgtgttt ttttttgggt 2340
ttattttatg gttttgggtt tagggtttag ggatagtgta tagatttaga gggatagggt 2400
tggagtattg ttgttttagg tcggtgttta gggtgttggt agggtggaag tagtgggtga 2460
ttatcggttt ttaggattat ggtttgtggt aggtaggtgg tttggggtaa agggttgggt 2520
tgtttttgtt tttaggaaaa aaaagttttg ggttcggatt tttatttttt tgtagtttag 2580
gaggaataat ttggagggag gttggaaagt ttagtatttt tataatggta gaacggtttt 2640
cggaattttt ttattttgt tttcgttaat ttggaggttt tttttttga taatattttt 2700
attatttatg ttagttgttt gttgaggaag aatttgttta ggttagggtt tatattttag 2760
ggattattta tttagtattt tttttattag ggttttttt ttttgtttgg atttaaggga 2820
aataaggaga agttttagtt aggtgaaagg ggtaggggtt agaggttagt gggatcggag 2880
```

ttggagacgt tttacgttgt cggtagggtt tgtatatgag gttttattgg gtttttagtg 2940
gttatcgggt agttttaacg ggttagatta tagtggatta aagtttagtt tttttattta 3000
gttaatttcg taggggttga aaaggatttt tattgtttgt tttatttttt agtgggtttt 3060
tttttttaga agatgggttt ttaagatttt ttcgagggta gggtaggtag gtggtagggg 3120
gtatttattt tgtttagggt ttcggtttta tatttattgt ttttattcgt tgtagggttt 3180
ttttaggggt gaagtcgtta gttttattta agtattttgt tttttatttg atgagaaggt 3240
ttcgtaggta ggtaaattta tagtgtgtgg tgtttttaat taggagagga gataggaggg 3300
gttttaatgt tttagtggag aaattttgtt cggattttat tttttttttt ttgtttagaa 3360
atgttggtgt taaaagtaga gtttgagggt agttttatgg gtaggagggt aggggaatta 3420
tttgttttgt agttgagtat aggatatttg gtattgtttt tagtttgggg gatattggag 3480
tgattagtat tagtttttgt ttttggatag tttagttttt gtttttttaga aagtttttgg 3540
ttaatgggag atggtatagt gattaggtta aataggagta tggaaggaag ttatagtgga 3600
gaagttaggg tagtgggttt tcgtattatt ggggtgatta gggagagtag gacgttttag 3660
taggggtttg aagataggta gggggttggg ttgttgggaa tgggatgaag taggaatcgg 3720
agttgggatt ttaaagaaaa tttgtggttt atagggtgtt atttttacgg ttttatttat 3780
tttgtgtgcg tttagagtat atatatatag ggatatatat aggggttacg tattgtggac 3840
gattatttat aagtttaaga attagtaatg ttaaatattg tttagggata tgtgtatttt 3900
atataatata tataagttta agttcgggat agtggttatt ttttagggag attgggtagg 3960
agattacggg tatttataag tgttttcggg ttcgagttgg tggttatttt agagtggtta 4020
ttttattgtt gtgtttcgta atttttatat gaatacgcgg tgtttggtat gtgtaattgt 4080
tatataatag aaggtttttt aataaggagg gtagtgaacg gtattattgg aggtggggag 4140
tcgggtaggg aaggtaaata aagatattcg gtgttttttt atgtgatgaa tttcgttttt 4200
atatatttgt aagtgttttt tgtgattttt ttgttatttt tattatttta cgaaaatggg 4260
gcgtagacgt tgggtattac ggtttacgtt tgtaatttta gtattttggg aggttgaggt 4320
gggtggatta cgaggttagg agttcgagat tagtttggtt aatatgatga aatttcgttt 4380
ttgttaaaaa tataaaaatt agttaggttt ggtattaggc gtttgtaatt ttagttattt 4440
aggaggttga ggtaggagaa ttattggaat ttaggaggtg gaggttgtgg tgagttaaga 4500
ttacgttacg gtattttagt ttgggtaaaa gagtgaaatt tcgttttaaa aaaaaaaaaa 4560
aagaaaaaga aaaagaaaag aaaagaaaat gaggtataga gaacgtaaga attgtgtttg 4620
ttttagtttt atagtgtata aggttggagc gggatgtaga atttatatat ttggttttag 4680
gttgttttat cgtagagtta tatggttttt ttttaagtta aaaatgttta gtttttattt 4740
ggtaggttag taggtagggt cggggttttt attttgttta gtttttaagt gtttttagtg 4800
ttatgtcggt tgtttttgtt taggtttttt tgttacggtt ttgtgtaggg cggtgtttat 4860
attagaattt tttgtagatt gattagtagt atttttattt aatattgacg ggtagcgaga 4920
gtcggtagga agaatggggt ttttaggttg gagttggagt gtgtgatcgt tattttttgg 4980
ttcgggtaag ttattttttat ttttttagtt tttttattta taaaatgtgg ataataacgt 5040
tattcgtttt ttagggatat gaggattaaa tgagtttatg gcgtgattaa ttatatttta 5100
tatttttatt gataatattt ggggtggttt ttaaggatgg gaaagatatt ttaagatgtt 5160
gtttattaag tgggaggtaa gaataaggta tagtttttat tagttcgggt gttaggttag 5220
ttatgaattt atggggtttt tgatataaaa atttagtagt gttgaaggta tatttttagg 5280
gtttttatat aagattgttg gaaagtgggt tttattattt tataattgtt ttgataggtt 5340
ttagattttt ttttagttgg agttagtggt gtttaataac gtataggtta gagaggtgcg 5400
gggatttttt ttttgtgtat tttttgatag tttttgttat cggttatttg cggtatttaa 5460
ggttggttat tagggtgtag tagaggtacg tttatagtaa agtcgtcggt tcgttttagg 5520
tttatttatt tttaattttt ttaaatttgt tttttataaa aagtttttta aatagtaaga 5580
atttttttggg tagagaggaa gagaggaatt gtaggtagag gggttaaggg gaggtaagtt 5640
agtttgatat tataggcgga tattggaaaa tatttaggtt gtatttagtt gggtagtttt 5700
cgtagggtgg gttcggtagg ggtggtggta ggggaggagt gtagataggg gaaaggttcg 5760
tgtgtttttt tatatttttt tttaggtatg aatttttatt tcgagggagt ttggtgtagg 5820
gttggagttg tagattttag agtcgttttt cgttttgggt tttttggtag gtttttttgtt 5880
tatagtattt ttttaatttt gtaaggaggg tgggtgcggg gagtttcgga tatgaaacga 5940
gataatttat gtttagttag tggtatagga gtgtttgatg ttttttggta attgttatta 6000
tgattatttt tttagagtaa tagtttttta aaaagttttg tggtttttgg gatagttttt 6060
taatttatta aaattttggt atattgtatt tattttgtgg atgatgaaaa ttgaggtttt 6120
ggaagattaa gagatttgtg ttttttgttt tattgggttt tttagttaag tgtttttttta 6180
gaataaagtg gttttttttg ttgtattttt ttttgtattt tttttttttt tattttaatg 6240
agaaatgaag ggatagagag aggaagggga aggaaacgtt cgggtttatt tttttcgggg 6300
tgttatcgtt aggagtgggt tgaattgtgg ttttttaggg gtttttattt atttaaagat 6360

```
atggttaagg tttaggtttt ggaatttatg attgtgattt tatttggaaa acgttttttt     6420
ttatagattt attaaggatt tttaaatggg attatttatt tagggtaggt tttaagttta     6480
aagataggta tttttgttta aagataagaa ggaaagaggg taggttcggt ggtttatttt     6540
tgtaatttta gtattttggg aggttaagta aggtgggtag attatttgag gttaggagtt     6600
taagattagt ttggttaata tggcgaaatt ttgtttttat taaaaatata aaaattaagt     6660
taggtgggtg gtacgtgatt gtaattttag atattcggga ggttgaggta ggagaattat     6720
ttgaatttag gaggtagagg ttgtagtgag ttgagattgt aatattgtat tttagtttgg     6780
gtgatagaac gagatttcgt tttaaataaa aaaaaaaaaa aaaaaaaagg aagaggaggg     6840
acgggataga gatataggga agaaggtttt gtgataatgg ggtagagatt agagtatgtg     6900
atgtttttat aatataatta ggaatgttag gcgttattag aaggaggaag gtatggatga     6960
ggttggtttt tagggtttta gaaggaaata gtttttgatt tcggattttt ggtttttgga     7020
attgtgaaaa tacgtttgtg ttgttttaag ttttttcgtt tgtgttaatt ggttataata     7080
gttatttaag agattaaggt gtgagggttt taggaatttt agtagggtaa ggggtcgagg     7140
gtttatttgg tttatagagt tatagtttat ttttttttta ggtttagaga ggttgggcga     7200
tttgtttagg gttatatagg tgtttatgaa agagtttgta ttaggattta gaatttggcg     7260
gtttttattt tagtttttgt tttttgaat ttttttttgt tcgggggag gtttggggtg       7320
gaggtatttg tataggttat ttgtttttt attttttaa gggttgaatg gggataagga       7380
gatttttttt tttgggtggg gtaaggtagt ggatgaggtt ttacgagttt ttggtttagg     7440
cgtggtttat aggttttggg ttagtagaag agattgtttt tttatttgtt tggagtttcg     7500
gcgtttgcgg cgagtatttt cgatggtatt ttatttggtt ttttttttaa ggattttttt     7560
gtcgttgatt tggtattcgt ggttattgtt tattatagta aatgggatta tttttggggg     7620
aacgggtaga cggtattggt tatatatgtg cgggaaggta attttttttt ttgggttttc     7680
gggaggagtt gttttgagtt tggggttttt taaaaacgag agttttagtt tgatagtatt     7740
gggtatagat atttgggttt agtgtttttt ggttttaaga atataatttt atggtttaag     7800
gtttttttaaa tttttaattt atttttagt aggaattggg ggtttttttgg gaataatatt    7860
ttagtggatt tatttattcg gaatttttcg tttattagtc ggtttttcga gtttttatta     7920
aatttttttt gggggtatac gtcgatgtcg ttggatagta ggtttgcggt gatttgggat     7980
aaggagggag agagatgggt ttatttttgt ggtaagtttt atttatttat tggtgtttgt     8040
tatatgattt ttatattgtg tgttgtttta ggttcgggtt taagggtagg ttttgggggg     8100
ttgtgatatt aattattttt aatttttgtg ttttttggag ggaagagggg tagttgtagg     8160
tgtatttgtt gttttgagcg acgttgtaat tttttagggg tttaaggtta tcgttttgag     8220
tattttttat tttttttttt ttgtttatta tagttttaag aaagtattta gggttatcga     8280
gatttttttt tttgaaattt tttttaaaaa attttcgtta tagtttttag ttttagtagg     8340
ggtttcgttt gtagtaattt tttagttttt tgttagtagt tcgatatggg tacgtgtaat     8400
agaaatattt tagtgagcgg ttgttcgttt gtttaggtat agattatagt tcgtgtgtag     8460
ttttcggtag atatatttat tttgtgtgga ttgttttttt ttgtatatgt tgggaaggtt     8520
tttgatattt ggttttagga cggagtaggt tataggtttt atttatattt atcgaggtag     8580
atgtggtgtc gtagtttagg tatttcggtt ttattttgaa aggtatttta tgtggttttg     8640
atgtgttgtt ttgttaggag gaagagttta gggttgtttt tttgtttgga tgtttttttt     8700
tattttatag taggggtttt agacggtggc ggagaagaag gtggtagaga ttattttttt     8760
tgaaattttt tttttttttt tttttttgaa gatggagttt cgttttgtta tttaggttgg     8820
agtgtagtgg tttaatttcg gtttattgaa gttttcgttt ttcgggttta agagattttt     8880
ttgttttagt tttttaagta gttgggatta taggcgttcg ttattatgtt cgattaatat     8940
ttttgtattt ttagtagaga tggggtttta ttatgttggt tttaaatttt tgattttaag     9000
tgatttgttc gtttcggttt tttaaagtgt tgggattata ggcgtgagtt attatatttg     9060
gtttagtttt ttttttattt aataaaaatt aggtagggcg aggtgaggtg gtttatgttt     9120
gtaattttag tattttggga ggttgaggta ggaggatttt ttgagtttaa gggttagaga     9180
ttagtttggg taatatggta aaatttcgtt tttattaaaa atataaaaat tagttagata     9240
tggtggtgtg cgtttgtaat tttagttatt cggggattga gtttgggagg tggaggttgt     9300
agtgagttat gattgtgtta ttatattgta attttggtaa ttgagttaga ttttttttta     9360
aaaaaaaaaa gggggggtat aaaataaggt gggttatgtc gtacggtttt tgttcggttt     9420
tattttttgg aagagagatt tattatttgt ttggtttagg ttttttttaa aatttttttt     9480
agagattttg ttggagaatt ggtattttta taaagttgtt tttgagatat gggggagttg     9540
gagaggttat tttagggacg gtttttagta gttatttggg ttagggatta ttaggttagt     9600
tattcgttgg ggagagtgat tagcggttcg gttttttttga tagtgtgatt tttttagacg    9660
gggtggtttt tgttaggttt gtagcgtttt taggttgtat agaagggttg gggtaagtag     9720
agatggaatt ttattcgttg tttgaagtgg attttttttt ttagggtgag tgtgttcgtt     9780
ttggcgatga tagggacgat gttgattatt ttgtttaggc gttttataaa ttcgatgttt     9840
```

aggggtttga ggttgaagta gagggatgtg cgtgaggttt atttgggggga gcggggtagt 9900
ggggggggtat ttattttttaa gtaggtgtcg atatttttag gtgttggttc ggggtttacg 9960
ggggtttttg ttggtaagga tagggtttcg ggtcgagtcg gttacgttgt gagtgtgggg 10020
tagaggaagg atacgggtgt gttttagata taggtgatat tattgtatag gttaggcggg 10080
ttagggttag ttttaagtat tttcgtttat gagttttggg gttttaatta tatttttata 10140
tgtttatata gaagggttgt cgttggttta gtagtcgttt tgttgtgggg ttttggttgg 10200
gaggggagag gtaagagaaa tagaagttag tattagtgtg cggtgttttt gtattaggcg 10260
ggttgttttt ttgtattagg cgggttgttt tttttgtttg tttggtttta gtgagtttgg 10320
ggtttgatgt ggggaaagtt gtgggtggta ggggttggta gggttttgga attggggtat 10380
tatttttttt agttttggag tcgagttttt tttatagtga gaataaatcg gttatatata 10440
tggttgggga gaagttcgtt tcgggtagaa gtttaagtag agatggttaa ggttcgtttt 10500
tgttttatgg gattggacgt tttgggcggg attaggtaat atgtttagtt ggagttttag 10560
ttaaagggtt agtgttgggg agtggtagaa gtgttataga tcgttcgttc gttttcggtt 10620
tgtttagttg gtattaggag aggtttttgt tttttgttat tagttttagt tgaggtaaag 10680
tttggagtag ttggaatagt tgttatagga tcgtaggtga gtcgttttgt ttgggagggt 10740
agatgtttag tttaggagag gtttatggat agcgggttgg ttttttgttt ttgatttttg 10800
ttggtttata gaggatgatg ttagagggtg ggttcgaaaa tttcggtttg ggttttttta 10860
tagatttggg gtgaggttcg ttgtttatta gttaggtttt tttttgtatt tggtgtttat 10920
agagtatggt ttttttaaat ttttgggttt ttttcgttta gggaagtata tttgggatag 10980
taggtgcgaa ggattttttt tatttattta tgtagataag ttatttacgg ttgtttttat 11040
tagttatttt tgttggtttt tggttttttt tttttttagtt ttggtttagg gtgagttata 11100
ggtgtttggt agggggtgta tagggagttg aggatagagg gttcgtgagg ttatgaagag 11160
tttatttgtt agggtatatg tagtttttcg tttttgttga tttttagggg cgacgttagg 11220
tgcggataga gtcggcggga ggaaagggtt tttattaata ttaatagtag tttcgtttag 11280
ttttggaagg ggttagtatt gatttttgtg tttttcgttt aagaacgtat taaatttttt 11340
aaggggtttt atgaggtagg gttcggatga gaaggttagt tatcgttacg ggtgtttggt 11400
tggggggtttt tattttattt gttagagttt ggtttgagaa ggtaggatat agattattag 11460
gttttagaat agtttagggt ttgtttgatt tttagttttt taagacgtag ttgaaggatg 11520
ggggttgggt aggatatttg tataggggtt agtgtgaggt tttggaggtg tagatatggt 11580
atatttttaa ttttatagtt ggcggttatg gtttttgatt tagttttttt gtatttagta 11640
gtagggtaga tagtatcgtt ttgtagtttt aggggataga gggaatttag ggtattcggt 11700
atgaggacgt cgatattgta gggacgtacg agtggtcggt ggcggggatg aagtagaggt 11760
agtagtggac gcgggtgttc gggatgcgtt ttttgcggtt gatgttgatt ttttttttgta 11820
ggtatttttc gtattggtta ttgatgaatt ttatgatggg ttgttagttg cgtgggggat 11880
ggggacgagt tagtaggtat atgagatgtt ttttttaagt aggtcgtgta tttttaggat 11940
ttagagttta atattttatt ttttaatgaa atgtatgttt tgggaggttt ggtttgttga 12000
gcggagatgt tatagtttta ttttcggttt tttaaattag tttttttcggg ttggttttag 12060
gtgatgagtt ggggatagta agatttttta ggtaattttg ttttggttta gaatttttag 12120
gtttttgaga cgtgcggttt tggtagtatg ttaggtttta ggaggtgggt tttgtatttt 12180
cgcgggtcgg gaaattttta gggttgattt tgggtggggt ttagggagtt ttgaagtttt 12240
cgttagtgtt ttttttttttg gaggggtttt attagttttc gttgttgatg tggttttcga 12300
atttggtgt gttaattatt gttagtttta ttcggacgtt ttttttttta atatttgtat 12360
agagttacgg acgtagagtt agaggggaga cggaagattt ttatagatat tttatatttg 12420
attgatttcg gggtaatttt aggtaggtag cgatgataat taaggagata tcgggaaatt 12480
aatggttttt tttagttaat ttcggtatag gtcggtgacg gtttttttgg gagtttttttg 12540
tttgttagat ttttggtgga aaatttagtt gtttgtgggg agtttagtgt gacggggttt 12600
atagggggttc gggatattga tttattatag ttagagtttt aatatgttta atatgttggg 12660
gatggtgggt atagcgagtt ttcgttttgg ggatttggga ttgtttgatt cgggtatttt 12720
tatatcgtat tagtgtttta ggaaggtatg ggtattttat atttttagtt tatttttagt 12780
tattgatcgt gtgtgatggt tttgattttt aatttttgtg ttttaggaag gtaggggcgt 12840
tttgtatttt tagtttattt tcggttattg attgtgtgtg atggttttga tttttttattt 12900
tgtgttttag gaaggtagag gagttttgta tttttagttc gtttttggtt attgattgtg 12960
tgtgatggtt ttgattttta atttttgtgt tttaggaagg taggggcgtt ttgtattttt 13020
agtttatttt cggttattga tcgtgcgtga tggatttgat ttcgatggtt ttggggatgc 13080
gtttttttga ggtgggttgt atcgattttc ggttgatttt ggatttgaag agggtgttga 13140
ttaaggtgga tttatttaag tcgttttgtt ttggggagag gatgggaggt cggttagggg 13200
tttttttcgga gagattagta gggtttgtat atggggtttt ttttatttag gtacgaggtt 13260
ttttagtttg agatttagta gtgtttgggt tatttttcgg taatattttt gttaagtagt 13320

```
tttcgaggat tttattttcg agtcgggtgt ttagtgattg tgtaaattta tatagtcggg    13380
ttttgatgtt agtataattt aggttatagt tttttttga gaggtgagtc gggtttggga     13440
gagtcggtat tttttgtgtt ttttgtgttt tttggatagg aggttttttg gtggtttaga    13500
ttttggatag gatagaggtc gggttggcgg aggagtttag ggttgtttag ggtgtattag    13560
gtggtcggga tttttgtagt ttattagggt ggttgtaatt aaagtttagg tgtttaaatt    13620
gttttttttt gatcggttag ggtttgggta agttatttga ttttatcgtg ttttagtttt    13680
ttgatttgta aaatgggata aatagttttt ttttcgtgga gttggtgtga gtattaggta    13740
ggtttacgta tgttttatgg ggtttgtggg gttatgtgtt agggggcgtt ggtttgtagg    13800
gtggtgttat tcgagggata gtgatcgtgt gtgtagatag ttagtttata ggtgattgag    13860
ataaacgagg atgaggaagt agtatttata tgtaatataa tatatataat ttatatatat    13920
tttatatata tattttataa tatatatatt ttatatgtat aatttataat atatatataa    13980
tttattttaa atatattttt tatatatatt ttatatataa tttttataat atatataaat    14040
aaattatata taatacgcgc gcgcgtgcgc gtatatatat atatatatat atatatatat    14100
atatattttt ttagattcgg agttttattg ttttattttg tatagagttg ttttgggtta    14160
cgttagtaat atattaaaaa aaatatttta ggttaggtgt ggtggtttat gtttgtagtt    14220
ttagtatttt gggaggttga ggtaggagga ttgtttgagt ttaggagttt aagattagtt    14280
tgggtaatat agagagattt tgtttttata ttaaaatgta aatattggtt aggtgtagtg    14340
gcgagtattt atagttttag ttacgtagga ggttgaagta ggagaattgt ttaagtttag    14400
gagtgcgagg ttgtagtgag ttaggattat tgttatttta gagtgataga gatttgtttt    14460
tttaaaaaaa taaaaatcgg ttaggcgaga tggtttacgt ttgtaatttt agtattttgg    14520
gaggttgagg agggtggatt atttgaggtt aggagttcga gattagtttg gttaatatgg    14580
tgaaattttg tttttattaa aaaatataaa aattagttag gtatggtggt atatgtttga    14640
ggtacgagaa ttatttgaat ttaggatgtg gaggttgtag tgagttaaga ttatgttatt    14700
gtattttagt ttgggtaata atgagatttt gttttaaaaa aatataatat aaaaaatatt    14760
ttaaattata ttgatgtttt gattttgata atattttttt aggaaaagtg tgggtttttg    14820
ggaggataga ggttggtgtt gggaattttt ggtttgaatt gtttagtagt ttaaggacgg    14880
atatttttcg tttatttttt tttgtttatt aggtttgtgt tttggttaat tttaggttag    14940
gagttatgtt aatgttttag ttacgatatt ttagatttag gagatgttaa ttgggatatt    15000
gtaaggtgta ttttttttatt attgttattt ttattattat tattattatt attattattt 15060
ttattattat tattattttt attagtatta ttattataat attattattg ttattattat    15120
tattttatta ttttcgttat tattattatt ttcgttattt ttattattat tattattgtt    15180
attataatta ttagtattag tattattatt aattatatta ttattattat tttattatta    15240
ttattattaa tattattatt attttatta ttattattat tattattaat attattattt     15300
ttattaatat tattattatt tatattatta ttataattat tattattata attattatta    15360
ttattttttt tattattatt ataattatta ttattattat tattattata attattatta    15420
tttatattat tattattatt tttattataa ttattattat tatttatatt attattatta    15480
tttatattat tataattatt attattattt ttttattatt attattatta ttattattat    15540
aattattatt attatttttt ttattattat tataattatt attattattt tttttattat    15600
tattataatt attattatta ttttttttat tattataatt attagtatta tttttattat    15660
tattattata attattatta ttattattat tattattatt attattataa ttataattat    15720
tataattatt attatttata ttattattat tataattatt atcgttatta ttattataat    15780
tattattatt atttatatta ttattattat aattattatt attatttata ttattattat    15840
tataattatt attattatta ttataattat tattattatt tatattatta ttattataat    15900
tattattatt atttatatta ttattattat aattattatt attattttta ttataattat    15960
tattattatt tttattatta ttattattat tattattata attattatta ttataattat    16020
tattattata attattatta ttattattat tattattatt attattataa ttattattat    16080
tattattatt ataattatta ttattattat tattattatt attattatta taattattat    16140
tattattatt attattatta ttattattta tattattatt attattttat tattgttatt    16200
attattatta ttattatttt tattattttt atgattatta ttattattat ttttattatt    16260
attattatta ttattataat tattattatt tatattatta ttattatttt attattatta    16320
ttattattat tattattatt tttattattt ttattagtat tattatttta ttattattat    16380
cgttattatt attttattta tttttattat tataattatt attattattg ttgtaaattt    16440
tcgaataatt ataatttgtt ggggattttg ttcggggttt agttcgtttg attttatttt    16500
atgggatgat tttattttat ggacgaggaa gttaaggttt atagaggagg agtttagggg    16560
aagatatata agttttattt cgttgagtta ggataaagtt gggatttttt tcggggtttg    16620
tggattttta aataggtgtt tttttatttt gtttgtattt ttgtaggtgg gtatagttgt    16680
tatgggaggg aggagggagg agagtagagt tgttttacgt tttgtttttt attcggtatt    16740
tttgggaggg agtgattttg tggggaatta ggattattgt tttttttagg gttgaaaggg    16800
```

```
cgaagaggtt tagataggtg aagtggtttt ttgcgtatta tttttagagg gtaaggttgt 16860
tatagtggta gttacggaag gcgagggttg ttgggttgtt tttttttttt tttggggtta 16920
aagtttcgga agaaggtttg tgttatggtt gggacgtaga ggtttttagg ttttttttat 16980
tttgtttttg gttagggagt agggtagttt tttcgtagtt tatagagatg agttttagtt 17040
tggttattgt ttttgggttt tttttttttt tttttttta gttatttttt cgggaggtag 17100
ttttgaattt ttagggtata agtacgtttt ttataatagg tagaataggg tgggggtcgt 17160
aggaggggat tttatatttt cgtttttttt tgaagtaggt tgttagtttt taggtagttg 17220
tgagtatagt agggttatga gtagtcgggt tttatgaggt tataggaagt agagaggata 17280
ttaagatttt aggggagtta gggtttagat tagtgtagaa gaggaaagaa aggtgtcggg 17340
ttggtaggtg tgggtttgcg tttatagagt tgggtaggaa ggacgtgtac ggttggtaaa 17400
ggttagagaa ataggttcgg tttgagaggt gtttagatat tttgtagaaa tttcgtaagt 17460
ttatttttgt ttagggtgtg atttggcggg ggtatttttt tagatttttt tttggatttc 17520
gtggagtggg tagaatatag ttttgggagt taaaacggtg gttgcggttt tttgttttgt 17580
gagttaagga attttggtag tttttttga gtttcgtttt tgtttggaaa acgggacgtt 17640
tttttttatag ggtttttgtg aggtttgaat gggatttggg gggtttttgga aataataggc 17700
ggtttattgt agattttagt ttggtttttt tattttgttt aggtcgtgtt gatcgacgat 17760
ttttcgtggt gatttttagt ttttcggggt tattaggggg tgtggaagtt acgaggtttt 17820
aatttttaggg tagagttgaa gttgagttgt tgggaagttt ttggtcgggg tgcggtgggg 17880
tgcggtgggg agtttggagg gttaggttgg agtaatagtg aggggatagt tgttatagaa 17940
tttttttgtat aagttgacgt tattgtttag aggtagcgat tatttattag gatttgtttt 18000
aggtcggttt gggtttttta aagttatttt aggggttttg gatgtgtagg ataggttttt 18060
ttgaggagtc gttggaggtt gtgggttttt tttaggcgta tgtatgtata cgtaatgtta 18120
ttggtaattc gtttttgagg ttagggttgt tgttttattt tatagaaggg aagattaagt 18180
ttaaaggttt gtgggtcgtt ttgggggttg aggttggttt tcggtattgg ttgagtggta 18240
tgtaaggtga ggatttatcg attattatga tgttgaattc gaagttttgt tttatggttt 18300
ttcggcgtat ttgttttagg atggagttaa tttttacgta gtcgaagttt atcggggttt 18360
tttcgtttcg tgatgtaggc gtttgtttga gttcggtatt tttgggggtg tcggttatgt 18420
cgttaacgta gttgggagtc gttttagtgg ttggggaagg aatagtagag gagttagagc 18480
ggaatagggg tttatatata ggcgtttgtt aggtggttgt tatatttatt tttggatggg 18540
gaaattgagg tattgaggtt tagaaaggtt aagcgatttg tttaggcgat ttttgagaat 18600
tttttcgggt ggatgttttt ggtcgtggtg tttttttaaaa agttgttatt ttgtagttac 18660
gttgtttttt tagtaaagtt ttattttttag atttttaagg ttaggttttt ttattttttt 18720
ttttaggagt tgtgttaagt tggggggtttt ttgcgtttta aggtagggtt gattttgaat 18780
ttttgttttc gtattttgga gggttgttta gataagggtg gagggtgttt tttttttttg 18840
gtttttttata ttgtttcggg ttatttaggt ggatttggtt ttttttttta tttttattta 18900
gataagttgt atcgtttgtt gttatttaaa ggttttagaa gtcgatttac gagttaagat 18960
ataggaggga tggttttgta agaaacgggg atagaattag cgtgtgtttt cgtttttttat 19020
atttttatgg atggttttgt aagaaagggg gatagaatta gcgtcggttt ttatggagtt 19080
gtgtgtttaa ttttatagaa cgagttagta gttttgggag aaggaaaagt gttgggtgta 19140
gggcgggtgt ttggatgtag agttgttttt gggtttatgg aggaggggggg tttttgtatt 19200
atatttgttt tttttttggat tgatttaggc ggtcgttttg tgtgtttggt tagcgtttac 19260
gggttgtata tttataggtt aagattaagt tttatttttg ggtatgtggg gtacgtggag 19320
ttgaattttt cgaaattatt aaagtttttt ttgtttgtag gtttttttcga tggttttgtt 19380
tttggttgtt gttaagatat tatatacggg ttagaggttt ggggttgggg gcggacgttc 19440
ggaagacgtt tgttgaggat ttaggtgttt ttatatagtg gggttttagg ggtatttagg 19500
atggggttgg tttttattag ggttggttgg tatacgggtt aggttttagt atattttaga 19560
ttcgttatat ttattggtgt atgcgtatgt aggtgtagat aattcgttgg ttatagaggt 19620
ggtgtttttg ttagttttta taggttggag ttgtttttgg tggtttttag tttttacgta 19680
ggttgggcgg ttgttgtttt gtgttttcgt ttttgtttgg gtttatttag gttttgtttt 19740
gtttgcgggg agatttttgt tttattttgt tttttaggga ggatttggtt ttaggtattt 19800
tgcgttaaga tagggattat acggtacgtt tttaggtagg gtttttttcg ttagtttttc 19860
gggaggggag tggttttttt ttgggatcgg tgggtattta gtaggggtag ttatagggga 19920
gtggagtcgg cgtgggagtt agggttatag tacgggaggt tgtagggggt ttattttttcg 19980
tgataggtaa gtgagtagtt tttttagata ggaggtagtt ttttcggttt acgtttattt 20040
tttattttgt ttagaaggtg ggaggtaggt ttttaggtta ggaggggtag tgggagattt 20100
tagtttaatt gttttttttat ttgcggggta agaatcgaat ttaggaaatg tttattgagt 20160
tttttggttg tagtttgtat ttttagtcga tttaagttat ttttttcggt ttattttatt 20220
ggagcggtta gagggtgtcg gagggaggtt ttttaggttt gaggtattag aggtgagaga 20280
```

ggaaaagatt ttggaagtta gaaattgggg gttggtttat ttttataggt attttttcgt 20340
ttttttagtt tttatagggt ataggaattt ttattttatg gaattggggg aataaaggaa 20400
taatataatt ttttgttggg aaatgttaag ggggggttta tatattttta ttgttgagag 20460
taatatttat aggcggattt ggaggttagg gttttttttt ttgtgttttt acgtggtttt 20520
tatttaggtt acgtagtttg gaagttttta gggttttgtt tttgtttttg ttttttttttt 20580
agaaatattt ttttttgggt ttttaggatt tttaaataaa taaagatata gttttgtatt 20640
tggttatgga ttatgtggtt ttggggtcgt tttttgaagt tagatatggg gttttgtttt 20700
gcggggtagt agtttagttt ttatttacgt gatcgaaaat cgtttttatt tgtttgtggg 20760
tttgagagtt attttgtttg ggattgggat tagtttttat tcgtttttat ttttttttag 20820
tattttttaa ttttttttag taggatttgg ggggaggcgt tgttttttga attttttttt 20880
ttcgttttta acgtttttgg agtttagagc gagggagggg tcggtttagt tgtagttggg 20940
tttttttata ggtattagta tttattgtag aaatttaaat taaattaaat attttgttta 21000
ttaggggttt cgtttgagaa ataataatat aacgtagtcg agttttattt ttgttattga 21060
cgtagggtag tggtttgttt tttgtatagg tgagagttat ttttgtaatt tttttttcga 21120
gaggcggttt tgtaggtttc ggttggttcg taggtaggta cgtacgtaga gagtaatgta 21180
agagttcgtt tcgttttttt atttggttag gtgagttttg gggtttgttt ttaggatttt 21240
taagtgggaa aagtagagat ttttatattt agagatattt ttattttggt tttaggatat 21300
tatggtgttt agatagttgg aaataatggt aaagattatt tattttattg tttgtgatta 21360
cgatatcgtt cgaggtttgc gttgtttagc gtggtagtta ttggttacgt gatttttttt 21420
tttaattgtt ataacgtttg ttacggttat tattttgaag tgtgtagttt agtggtgtta 21480
agtatattcg tattgttgtg taataaattt ttagaatttt tttattttgt aaaataaatt 21540
gtttttatta aataattttt tagttttatt ttttaagttt ttggtaattt ttattttgtt 21600
gttttttttga atttgattat tttacggatt tggtataagt ggaattttat aggattgttt 21660
ttttgtgatt aaattatttt atttagcgta atattcgaaa ggtttatttt tgttgttgta 21720
ggtattttaa tttttttttt acggttgagt taaatttttt gtatgtttta tttttttttt 21780
tttttttttt agatagagtt ttgtttttgt tgtttaggtt ggagtgtaat ggcgcgattt 21840
cggtttatcg aaattttgt tttttaggt ttaagtaatt tttttgtttt agtttttttaa 21900
gtagttggga ttataggtat tcgttattat gttcggttaa ttttgtttta gtagagacgg 21960
ggttttttta tgttggttag gttggttttg aatttgtgat tttagatgat ttattcgttt 22020
cggtttttta aagtgttgag attataggcg tgagttatta cgttcggttt gtttcgtttt 22080
tgattttttt atttatttga tcgcgggtat tcgggttgtt tttattcgtt ggttattgtg 22140
aatagagtcg ttatgaatgt gggtgtataa atatttttag tttttttaaga tgttgtgtat 22200
tttttttgaat ttatatttag aagtggaatt atgggatgtt acggtggttt tagttttcgt 22260
tttttgagga attgttatag tgtgtttata agtgattttt gagtatttga tattgagtta 22320
gtttaattga ggaacggaat ttttattttt atttaaattt aaatagttat attttataaa 22380
tggtaattat attggatggt atagaatttt agtttaattt attttttatt aatggggaaa 22440
ttgaggttta tagagtgatt tttttttttg aaatgttatt gtaagtggtt aggttagtat 22500
aagattttta ttgaatttgg taattttgaa atttatatt tttgtttgta tgatatgcgg 22560
ttaatatttg tgattttttt taaagatcgt gggttttagt aagacggtag ttgtttcgtt 22620
gagattttta tcgtattttt aggtttcgtt ttgttacgaa tacggttgtg taaggatttg 22680
tttgggtttt tgttttttat tttttcgggt atttagatta attattagta gtatttacgt 22740
cgatttttag aaaggttttg atttggatag taattgatac gttattttat agttaaagta 22800
aaatgtagag tttttttttc ggtttcgtag ttgggagtta gaggttttag gttttggttt 22860
ttggtttaaa gtgatttttt gtaagttttt ttagtttggt aaataggtta tgtatttttt 22920
gattttttttt ttagtttttg ttttgcgagt ttttgggtgt tcgaggttgt ggtttattta 22980
gtgatatttt tagtttttag tgtagttcgt gagtagtata atatattcgg ttttagaaat 23040
aattaatata ttcgttaata gatgcgtgga gaaatttaac gtgttgagtt aatatagcgg 23100
attatttttt agttataaaa aggtacgagc gtgcgttaat atagatgaat tttgaaaata 23160
ttttaattgt aagaaattaa gtataaaaag ttatattttt tagggtttta gttatatgtt 23220
tagaataagt atatttgtag ggataaagcg tagattggtg attttttaggg gttgggggag 23280
ggggagtgat tgtgtatcga ggatggcgtt tttttttggg gtgaggaaaa cgttttggaa 23340
ttagaggtga tgtttataga aggttgtgaa aaaatgttat ttaattgtgt tttttgaatc 23400
ggcggatggt gtaatatgga aattatattt ttatgaggac gggagaggaa gggaaagaag 23460
ggaaggaggg agggggaagg aaatagggaa ggaaggaaaa aagggagggg aggagggagg 23520
gacggaagaa gggaaagaag gtagttattt ttagtggagt tggattgaag agtttaatgg 23580
gtgtagtaaa ttattatggt atttgtattt ttatgtaata aatttgtacg ttttgtatat 23640
gtattttaga atgtaaagta aaataaaata aagagtttag tgttttttat aagtgtttag 23700
gagttagttt tatagtttag aggatagttg aggtttagag gttaatattt aggtttgagg 23760

```
tttttaagtt ggtgtcgtta gattatgttt tagataaatt tttttatttt tttgtagtaa   23820
gggtgttttt agggtggtag ttaggcggag gtgttaggtg gcgggagatt ttgatagttt   23880
ttttgtaggt ttttattttg aatttaggtt ttatgttagg gttttttttgt tatttattgg   23940
cgttggtggt agtaggttat cggtttcgtg atggggattg tttttaaatt tgtttatttt   24000
ggggattagt tttttttttt tcgtaggcgt ttattgggtt attttttttt tagagttgta   24060
ggtgggttta aggtagggat gaaagggttg taatttacgt agttttgagt tagtattttt   24120
tgggaatatt tgtatttagt gataaatttt tattttaatt ttaggatttg tcgaggatga   24180
gggaattaga ttgttgtttg tggttgggac gggggttgta ggatatttgg ttgggaggta   24240
gtgtttagag aggaggtagt tattggatat gatatatttt aggggtattt atatttatta   24300
tagttttagg gttgattttg gtttatttgg tttggtgtcg agagagggtt tgatatagta   24360
ggtttgtaaa gtttgatttg gaaattaaga tttaggggtc gttttttttt gtttattttt   24420
ttttgtgtgt tatgttgttt ggtttacgtt ttagaaatgt ttatataagt tttattagtt   24480
ttgttggtta aatgtagaga tggtgtcgtt ggggtaaggg atttgagtt atcgtttaat   24540
gttattttta gttttagggg agtgatagtc gggtttgtat tttaggataa agtgtttagg   24600
gttaagttaa tgttattttt ttgttttttt taaatttaat ttatatttgg tttttagagt   24660
acggatcgaa atggttatat ttttatatat tttgtttgtt attgttaatg gtggttaagt   24720
agaatgtaag gttatttttt ttttgtttac ggaggtttgt gaagttattg attaggttac   24780
ggtaggggag tcggtggtat ttattatatt ttgttagttt tttaggttcg tgtttaggag   24840
aaagttgtcg gtatttatag ttggtgtttt tgtttcggcg aagatttttt tgtttttatt   24900
tatttcggtt tagcgtgggt ttagtagttt ttttattttg tttcgtaata atgtatattg   24960
gagtagtttt tagttgtttt tttttgtag gtttttttga atttagattt tggggttagt   25020
atttttgcgg tagttttacg gttatttgta ggttttttta gaaatttgag tttatggggg   25080
tattttaggt tttataaatt aatagatgtt ttttgttttt tataaagggg tttttttaaag   25140
ttgagtgagt ttttgatgta ggtaatgtta ggaggatagt gttcggttag ggttgttttt   25200
tttttttttt ttattttttt gggtgtttgt aaagtttttt agatttttgt tgaattttgg   25260
ggaagttttg tttcgtaatt agggtttgtt tttttcgttt ttatatagag tttgtttttt   25320
gcgtttagat agtttttttta cgatggtaat tgaataatga tgtgtgcggt ttttttttat   25380
tataattttt ttatataggg tttgtgaagt tagatatttt atttattacg tttattttag   25440
ttcggttttt tttagagtag cgtagggttt acggtaagtt tcggtaaaaa gtttggtgaa   25500
tgaatgaata aatgaagaaa taaagtttat tatagtagtc gaggtttatt gggggttttt   25560
tgtgttaggc gttattttaa tttttttttt tagagagtgg gttttattaa tgttggttag   25620
gttggtttta aatttttggt tttaagcgat tttttttattt tggtttttta aagtgttggg   25680
attatagata taagttattg tatcgagttt taggtgttat tttaaacgtt gtttaggagt   25740
tattataatt gatggattat tatataattt tataaggtag ttattgttat tgttttattt   25800
tatagataag gaaatcgaga tcgagagggt ttttgttaat tatgagggtc gtaaagcgag   25860
taagcggtag agttaggatt tgaagttagg taggtgagtt ttaggttgtt gatttttttt   25920
ttaggttagg ttttcgggaa ttatggaaag gttggagaag tatggggttt ttgtagttag   25980
gatttagaag gtgtagttgc ggtgtaggta gtttagtatt gaggttttgg tttttagttt   26040
tttttttagt tgtttttttt tagggagttg gaggtagagt tttgaattgt taggagaaga   26100
ttttattcgt tagttaggga aagttgttag ggtttttttt agggtttaga gttattttag   26160
gtttattaaa gtatattagt attttagttt ttataatttt ttttgtaaga aagatgatgg   26220
tattagttat tgggtcgggt tgggggtttt agattttagg ggttttttta ggtttgttta   26280
tggttttttt aggatataga tgtttatttg agaaatttta gtgtattagg ggttttatag   26340
ttaaaataaa gaggtagagt tgttttgggg tttttgttaa agtttttagg tagatttatt   26400
tagtagatat taaaatgttt atttttttatt tgaaaatttt atttttagga ggtcgtttta   26460
ggttgggttg ataaaattta tagagatttt aatgatgatt ttttatttta ggtatgagtg   26520
agtgggtgga gggagaagtt attgttaatt attaatagtt taggaaatgt aggtaaagga   26580
aagagaatgg taaggcgata gggttggaaa ttagatatac gtgtagaggt gagtagattt   26640
tagagaaaga attgttttgg aataagatgg taaagaaatt atagataata atggtagacg   26700
atgattttgg ggttttagtg gattaggatt ataatattga tttatttgat aagtatttat   26760
tgagtgttta ttttatagta ataaaaagat gatattatag gtttgagagt agttagaatg   26820
aaaatagtgg tggtatatgg gaatttgaag tttatttatt taaatgttta ttagtcgaga   26880
ttttcggata gatttgaagt agtttttaat atgtaaggaa tttataattt gatatagtag   26940
tataggatat gtgtgtgtta taaatttttg gtgaaaagta aagatttttt ttagaaaagt   27000
atatattgaa gatagataga tagatatata cgtattttga acggtttatt aaagaatttt   27060
taggttcgtg gattttagtt aaaaaaataa tttggtattt tgggaggtcg aggcgggagg   27120
atcgtttgag tttaggagtt taagattagt ttgggtaacg tagcgagatt ttattttttat   27180
aaaatgaaat aaaataaaat gagaattaaa aaaaataaat aaatttggtg tgatgagaat   27240
```

```
ttatagaatt taatagggtt attttagtat tatttgtagt attttttaaa tttttgatta   27300
tatatataaa gttttgggtt ttatagttag agaagaggga gagtaggtta aaaataatta   27360
tttaaggtgg aagatgagtt tgagaaataa agaggtaaga ggagaagatt gaggttagtt   27420
gttttttatt tttgagtttc ggtaggagga agtttttaag tgagaataag ttgttgtttg   27480
cggagtggat tttagagcgg gtggatagag acgataagga atgttatttt tggggggttt   27540
tgaaaatagg atattttttt attttaattt tgttttttat tgtattgtgt ttttattgat   27600
ttatttaagt tattataaat attttataaa tgttattggg gattttttgt ttttttttaa   27660
agatggaaat attgaatata aatgattaaa ttgtgtaaat gtgtgtgggt gggggattaa   27720
ggattgaatt tttaaaatgt atatagttta tttaatttgt taattaattt taaattaaat   27780
tatattttat attttgtatg ggataggaag cgttttttta aagtttggtt aggatttggg   27840
atgtcggaaa tttgagattt tgtttttttt ttttgagata ttttgaaatt ttgtttagaa   27900
atcgagtata atggttttgg ttttttagag aaagatacgg atttgtaaag gtaaagagtt   27960
gtattttttt tcgagttgga gtttttttcg gtttttatta tggggtttaa ataaaattgt   28020
agttttatag tttttttgtt ggtgtttttt tttttttggt gattaataag gtggaagagt   28080
taataagaat tttgtgtgtt tttttgtagg atttggaagg gataggtggt gtgagtttgt   28140
ttttatttag tttttttttt tatttattta ggggtcgagg cgttttcgag gtagggggcg   28200
ggggttttta tttttattgt cggttaaggt ttttttagtt tttttttcgt taaatttttt   28260
taagtaaatt ttttttttat tgtggaggaa ggaattttaa gtagttttgt ttttttgtta   28320
gtacggtttt aattggtttt agtcggtaaa gttaaatttg gataaggttt aagggttttt   28380
tatattattc gtcgtttttt ggaagaggtt tttttttgtt tttttatttg taatttatta   28440
tttttttagt ttgagttttt ttatttaggt ttcgcggttt attgagatat taattttggg   28500
ttagagtaag ttttgatttg taaattatta ttatttattt tttaaagagt tatttttaga   28560
atttaggttg tagggagggt aggttaagga tgaatttttt gatttttggt gcggtgtgtg   28620
gtgggggggg ttattttata ttttgttttt tgttgttttt ttgttttgtt ttaggttttg   28680
aagtggattt attgtagagg gagggttagg gaatggtttt gatgtttttt ttattttaag   28740
gaatttttta gtaggagcgg tttttatttt aaagtaattt taagatttag ttcgtgttag   28800
ttttttagtt atgtttatag tggtttgtta attagagatt tttttagttt ttggtaaatt   28860
tgtaaattta taaagagaaa gaaaagtttt ttcgtgggtg ggtggatatt ggttatttgt   28920
tataggttgg aatttttttt tattagggat gttttttaag taggggttgg ggagggtagg   28980
gtttagagtt tttttttttt tagttttttt tgttattagt agatttgtta gtagatatat   29040
agtagagttt agtagtgagg taataatagg ggttgtgggg gttggggttt cgtttttttt   29100
aatttatgaa attatttgaa ataataaaat ttagataagg gtttttgggg tgtaataggg   29160
tggttttttg ttaggtgttg ggtagaaaaa aagttttttt ttaatttttt tttgttgtgt   29220
agattagttc gggttttggg gatgttgtag aggttgtttt atgtttaggt tttttagaga   29280
tagtaggggg ttggaggtac ggtttgtttg ggggtagggg ataggtaggg aggggtatat   29340
ttttgggtta tgagtaggag tttgattaaa tgattttttt aggagggaga aagatatcgt   29400
atataattgt ttgattatag ttagaggggt tggaaaggtg ggggaaaggg gtgaaggggt   29460
taattgtttt aggtgttttt aatttatgta gtatttattt ggattgataa gagttgagag   29520
attggaattg tttttttttt tcgtttttag gttttgtttt aggagttttt ttgtttagga   29580
gtttgggagt tgaggaggtt tggaagtagt gggtttatag tgttatcgtt ttgggaattt   29640
atagtgtttt ggatgtggtt ttttcggttt tagttttgta ggtagtttat ttggattgta   29700
tttatttttt agaagagttg tttagaattt tgtatgttcg aaagaaagat taattaaaga   29760
agtgaggaag ttaaagagag gtttatgaaa gtttatgacg tattttttaa gtgtatattt   29820
tagtaatttt aaatgttttt tgaagtaagg atggggatgg atggttggat aaatggatgg   29880
gtgtatgggt gtgtgaatgg gtgagtgggt gggtggatgg atggatagat ggatgaatgg   29940
atgggtaggt ggatgaatag gtaggtgaat ggatgggttg gcggatgaat gagtaaatgg   30000
atgaatgatt ggatggatgg gtggatgagt agataggtgg atgagtgaat tggtggatga   30060
gtggatagat ggatgagtgg atgagtagat gagtgaatgg gtggatgagt ggatgggtgg   30120
gtgagtggat gagtagataa gtggatgggt ggatgagtgg atgggtagat ggatgggtga   30180
gtggatgcgt ggatgagtgg atggacggat gagtggatgg atggataagt ggatgggtag   30240
atgagtggat ggatagatga gaggatgggt agatggatgg gtgagtggat gggtgggtga   30300
gtagatgggt ggataagtgg atggatagat gagtggatgg gtggatgagt ggatggatat   30360
atgggtggat agatgggtga gtggacggat gatgagtgga tgggtggatg agtggatggt   30420
tgagtggatg gataaatggg tgaatgagtg gatgggtaga tgagtggatg gatagatgag   30480
tgaatgagtg gatgggtgga tgagtggatg gatagatggg tgaatgagtg gatgggtgga   30540
tgagtgaatg agtgaatgag tggataggtg gatgagtgga tgggtggatg agtgtataga   30600
tagatgggtg gatgggtgga tgagtggatg ggtgggtgag tggatgggta ggtggatgag   30660
tggatggata gatgggtaaa tgagtggatg ggtggatgag tgaatgattg tagataggtg   30720
```

gatgagtgga tgggtgaatg agtggataga tgggttgata ggtggatgag tggatgggtg 30780
gatgggtggg tgcgtggatg ggtgagtgag tggatgggtg ggtgggtggt aagatgggtg 30840
gataggtata tggagtttat ttaaggagtt ttagaaatat atatatattt ttttatttta 30900
gatttttttt aggaaaaagt agtgtgtatg gatcgttatt tggttatatt ttgagatagt 30960
tatttttatt ttttttggaa tatcgtttgg ggggatgtag tatagggtat ttaagtaatt 31020
tttgagttta gggtagagat attaggaagt agtaggtagg tcgtgatttc gtaggtttgt 31080
tttatggttt ttgttgtttg tattatggta tgaagtttag atgtattttt ggatttgggg 31140
ttttgattag atggagttat taattttttg aagaatgaaa tatatagggg ttatttttgt 31200
tttttagttg tagggaatga cgattattta gttttttttt tttttaaatt tttttttaggt 31260
ttttgtgtat ggttttgttg attttttttt tgtttttggt tatttttttta gtttttgtcg 31320
tttttttttt cgtttttatt tacggagtta gttttggtgt tttgagagtt tttttttttta 31380
gttgatttta tttagttttt gagtatttaa tgttaataat gttatttttt ttgtttagat 31440
ttatatttga gtgttagttt gatttataga cgtgtttttg ggtttcgttg tgttaggttt 31500
tttgttgggt tttgggtatt tgtagttatt gttattgtta agttttaata tttgaaggta 31560
ttttgaattt aatgatcgtg ttttaggttg aattttggag tttttatttt atttgttttt 31620
tattttattt tttatttaag tgaatagttt ttttagttaa tagttattgt agttagattt 31680
ttaggggtcg ggtttgggtt ttttattttt attatttcga atttatttat ttattttaaa 31740
tttttattaa tgttaattta atatatttta tagttattat tattagttgt ttttgttcga 31800
attttttttt tttaaagtat taggggggcgt gtgtaaaatt tattaatttg atgatttttt 31860
tttatgaagt cgtgattggt ttttattgta tttgggatat attagatttt tgtggtttta 31920
gaattttgtt tattatcgac gtgttgtgtg attttgagta agtaatttaa ttattttgtg 31980
ttttaatgtt tttatatgtg aaatgggata ataatatttg ttttataaga atgttgggga 32040
ttaaatgaga tatttattta tgaagtatat agtgttgtgt ttggttagta aatgtttgaa 32100
gtttttgtta ataaaatata cgtttttaat ttattttttta gtttttattt acgtatagtt 32160
tttttggttg tttttttagtt tttttaataa ggttatttat agaggttttt tttgtcggga 32220
ttattttttt tttatgttgt ttttagaatt tttatttgtt ttttcgggat agtttattta 32280
tattgggttt tttagttttt gttttgtttt gttttgtttt gttttttttg agatggagtt 32340
ttattttgtc gtttaggttg gagtgtagtg gtattatttg ggtttattgt aagttttgtt 32400
tttcgggttt acgttatttt tttgttttag tttttttgagt agttgggatt ataggcgttc 32460
gttattacgt tcggttaatt ttttatattt ttagtagaaa cggggtttta tcgtgttagt 32520
taggatggtt tcgatttttt gatttcgtaa ttcgttcgtt tttgtttttt aaagtgttga 32580
gattataggt ttgagatatt acgtttagtt taggtttttt agtttttata tacgtggttt 32640
ttcgattgtt gtttggggtt tgtcgtattt attatttata aaattttata cgttcgaagt 32700
atttagtaag acgttgtgaa tgagtgaatg aataaatgaa tgaatggtta gtttgttgtt 32760
ttttcgttcg gtttttcgtt tttttttagat tagtatgggg gtagttacgg ttttcgttta 32820
tttcggtaat atcgtttttt ttgtgtcggt cgcgttggtt agagtttttt tttacggcgt 32880
tttcgcggtt tcggtgtgtg gtcgtttgtg aatgagattt atgattttcg gtttttatta 32940
tttttttgga gttataaagt attaaggcgg aaggatggat gggtttttttg ggatagtcgg 33000
ggtgggaggt cggtttagga aattttttttt aggaaatggt tagtgtggga tgaggcgttc 33060
ggtgggaggg gagtggagtg gggatttggg gtttggttgt tttgtagtta gggattttaa 33120
tttttatttt tttttttcgtt tattttttaa ttttagtgtt gggtttaggg tttttggggt 33180
ttttttttagg aatatagaag atatagtaag gattagggat ataggatagg tagagtttat 33240
gggtaggagg tttttagtta gtatatttga gtttatgttt ggttttaatt agatattgag 33300
atttggtta ggttattttt tacgttgagg taggggggtt ttttagaggt tattggtttt 33360
taagtatcgc gtggaagata tgttacggag agttagtatt ttttgagata ttagtagaag 33420
gttagacgtt tcgcggttta aggatgattt ggttttagcg tttatttatt tgtcgttttg 33480
tatgaaaagt ttgacgtgat atggtcggtt ttagcggaag agtgattcgg gaatcggtag 33540
agcggattta gtaattggta aattggattt agtaattgag atattgttag gggtttggta 33600
gaagtttttt aatgtattag atttagtttt gatttttata gagtttagtc gagtattaaa 33660
aggtgtagag gtcgagttga gtattagggg aatattgatt ttagggttga tcggtgttgt 33720
gcgtgtattt tatagtttat agacggttta tatttattcg atcgtatttg attttttttt 33780
gcgaggtttt ttttagattt ttcgtttttt aaagttatta gttgtgtttt ttgaaattag 33840
agtaaagtaa taaaattgta gtaatagcgt gtttttatga acgttgtgat gttagttggt 33900
taaatgaggg agtttttagg gttttttata ttgtttcgat tttgtgtgtt ttttggtttt 33960
ggtttatttt ttatattttt tgtttttttt tttttttttt ttttcgagga tttagggttt 34020
ggggttgatt tattgtggga ttttaacggt gatttttttgg aagaaatgtt cgtttttttt 34080
ttttatttag tagtgatttt tggttcgttt acgtttacgt agttaatata ggtagtagtc 34140
ggaatttgga agtttttggg gtgggagttt gtttttttttt aggtttttttt agttcgaatt 34200

tcgggaagga gttatattta tatggtaatt ttttttaagg ttgggagggg gaaggggttt 34260
ggttcgggtt tgattatttt tttgaggttg cgtttattgg gtttttttaag ttagttgggt 34320
ttttcggttg tatttttgag ggatggtaat agattatagt gtaaattgtt tatcggttgg 34380
ttgggtagtg ggtgtttaga tgcgggttag ggttttcgtt ttataggttg tgtggtggtt 34440
taaagtgggt atttttatgg gtatatattt gtgttatagt ttattcgtgt gtgtgtgtgt 34500
gtgtgtgtgt gtgtgtgtgt gtgtgtgtgt gtgtgtgtga tagtttagaa atatggtaat 34560
ttatagtttt tttaagacgt tggttttggg agatttgttt atttgggggt tttagtattt 34620
ttagtttttt ttaggaaggt tttgtttttt ttggtttgaa gttggtttta gggaagaatt 34680
tttttagaaa aataaaagtt atttttttta gttttagttt agtttttttt taggggtgag 34740
ttatattaaa tttatttttt tttttttttt ttattttttt ttttacgttg taattagttg 34800
taagtggttt atttatttag gtttgttagt ttattataga aaagatattc gaattttgta 34860
tttttttttt ttgatttgtt ttgggtttta ataggtaaag gaaattggaa gttagtagtt 34920
aggatttagt tgtaggtttt cggggttatt ttgcgttttt attttagttt tatttatttt 34980
tgcgttgatt taatgtttat ttattttagt ttatggagcg aggtttggat ttaagataaa 35040
ataaaggggg ttttggtttt ttggggttta tagtgttcgg tgtcggcgag ataattttag 35100
agtatagtat agagaaatgg ttgcgatacg gattcgtagt atttggttgg ttaagtatag 35160
taatgtattt ttattaggtt tgggtttttt agtagatata tggtttaaag gttggttggt 35220
taaattatat gattttgtaa ttatttaaga gaaatgaaaa tatgtttata taaaaatttg 35280
tagattagta tttattgtag aatttttat aatagaatta tgttaaaaag tagaaataat 35340
ttaaatgttt attagtagat gaagagatat atagtgtggt ttatttacga tggaatatta 35400
ttcggttatg aaaaagaatg aagtattagt atttgttata atgtggatga gttttgaaaa 35460
tattttatta agtgaaagga gttagatatt aaggttatat attgtacgat tttatttata 35520
taaattattt agaataggtg aatttataga gacgaaggta gatgagcggt ggttaggggt 35580
tgagagttcg gagttgggaa tggggattgg ttaatggggg tagggttttt tttggggatg 35640
atgaaatgtg ttgggattaa tggcgatggt tgtgtaattg tgatggttaa tttttttgtgt 35700
tagtcgggcg tagtggttta tgtttgtaat tttagtattt tgggaggttg aggcgggtgg 35760
attacgaggt taggagattg agattacggt gaaattttat ttttattaaa aatataaaaa 35820
aaaaaattag tcgggtgtgg tggtgggtat ttgtagtttt agttattcgg gaggttgagg 35880
taggagaatg gcgtgaattt gggaggcgaa gtttgtagtg agttaagatc gcgttattgt 35940
attttagttt gggcgataga gcgagatttc gttttaaaaa aaaaaaaaaa aatttttgtg 36000
ttaattggat tgggttatgg ggtatttagg taatcggtta aatattattt tgagtgtgtt 36060
tgtgaaggtg tttttggatg agtttaatat ttaatttttt tattttttag aaagtttagt 36120
ttattgttag tatttattta attttatata aatacgtttt ttgaggttga agtaaatttg 36180
attggatgtt ttgtgtgaaa attaaatata aaaattggtt aggcgtggtg gtttacgttt 36240
gtaattttag cgttttggga agtcgatatg ggttaattat ttgaggttag ggttcgaga 36300
ttagtttggt taatttggtg aaatatcgtt tttattaaaa atataaaaaa ttagttacgt 36360
gtggtggtag gtattttgta attttagtta aatttaggag gttaagattg gagaattatt 36420
tgaatttagg aggtagaggt tgtagtaagt cgagatcgtg ttattgtatt ttagtttggg 36480
taatagagag agttcgtttt aaaaaataaa aaaattaaaa agaaaaaaaa tttttaaat 36540
tgtttttgta gttattttta agtaaaataa tagagttatt tgagtcgttt attttggaaa 36600
aattagattt attaaatgaa tttttagtta ataattgatt aagaataatg ttaatattat 36660
ttgtaggaat gttgtgtttt ttaggatttg atatttttag cgatcgagaa ttattatttt 36720
tttttaaatt ttatttattt atttattttt tgagatggaa ttttgttttg tcgtttaggt 36780
tggaatgtaa tggcgcgatt tcggtttatt gtaatttttg tttttcgggt ttaagtaatt 36840
tttcggtttc ggttttttaa gtagttggga ttataggtgt ttattattac gtttggttaa 36900
tttttgtatt tttagtagag atgggatttt attatgttgg ttaggttggt ttttaatttt 36960
tggttttagg tgattagttt gttttagttt ttttaagtgt taggattata ggtatgagtt 37020
attatattta gttgagaatt attatttttt tttaaaggaa atattattat taaaaatgga 37080
atgttataaa tagaatgatg tttttttgttt ttaaagtcga tatattagag tgatgtgaaa 37140
ataataataa aagtaagata tttttcggta aatttatttt ggggtgaacg ttgtagttat 37200
aagtattgtt ggtgagtatt ttcggggtaa ataggaaaag ggtgtaatta ggagtttggg 37260
taaagtagat tgtgtgtttt gatgggggtg ggttttattc gattagttga aggtaatagg 37320
gtttggaacg tatattttaa gatatagtgt tttggtatag taaatatttt tagttgaagg 37380
aatttaagaa atggtaggtt taagaagaat ttattgattt gtttttttttt tgaagtagat 37440
tataagtttt ttacgtaaga ggtgtttttt ttatatttag cggaaaggaa tagttttatt 37500
tttagagata gaggatttga gaggggtttt agtatatagg ttttgtcgag atttttacgg 37560
gtttattgtt tagtttattt tttgttgttt tgttatagtt ttttacgatt ttttattttt 37620
tattaaattt agtataaaaa cgtttaggtt taggttaggt atagtagttt atatttataa 37680

ttttagtatt ttggaaggtt aaggtggtta gattatttga ggttaggagt tgaagaatag 37740
tttggttaat atggtaaaat tttattttta ttaaaaatat aaaaattagt cgggtgtggt 37800
ggtatacgtt tgtagtcgta gttatttggg aggttgaggt atgagaatgg tttgaattta 37860
ggagatagag gttgtagtga gtagagatta tgttattgta ttttagtttg ggtaaaagat 37920
aagtaaaaga aataaataaa aaaattgttt aaaataagta ataaataaat ttaaaaaata 37980
agtaaaataa ataaaaattg tttaggttta ggtcgggtat agtggtttat atttgtaatt 38040
ttagtatttt gggaggttaa ggtaggagga ttgttcgagt ttaggagttt gagattagtt 38100
cgggtaatat agtgggattt tgtttttata aaaaataaaa aattagttag gcggggtggt 38160
atatgtttgt agttttagtt atttgggagt ttgagttggg atttaagagt gggtttagag 38220
tagttttcgg ggtcggtagt tgggttttag tttgagttta ggaagttaag gttgtagtga 38280
gtcgtgatcg ggttattgtt ttttagtttg gacgtgcgat agagtgagat tttgttttaa 38340
aataaaataa aaatcgttta ggttaacggt tttttt gggt ttttattttt ttatgaaggt 38400
tcggtgttaa gtaaaattta tttgaattgt tatgtttgtt ttttgttaat ttttgttagt 38460
ttaattttta gggttttagt taatgaattt aaaatcgata gaaggaaagg gttttttttt 38520
agattttgtt ttgattaggt ttttgtaatt ttgtgataaa ggtttgaagg tttgaatagg 38580
aggatataag gtttgagtga gagagaattt ttttcgttta atcgttttta atttggaata 38640
ttagattttt tttgttttag atttaaattg atatatgcgt ttttttt ggg tttcgggatt 38700
ggaacgacgt tattggtttt tttgggtttt gggtttttag gtggatatta gagtcgtaat 38760
atgggttttt tcgtgttttt agtttgttga tttattttgt taattttggt atttgttaat 38820
ttttgtaatg aggtgagtta atttttttata ataataaatt tttttattta aatatacgta 38880
tatatttttt attggttttg ttttttttgga tgattttaat ataattattt tgtgaatata 38940
gtaaaattat tgaattgtag atttaaaaaa ggttaacgtt atagtatgtg aattttattt 39000
ttataaagtt attatttaaa tagtaataag aagttggtga gttttgtgta tttttttgta 39060
aataagagta gggatagcgt cgtttttgtt ttcggatgtt tttagttttt agttcgagtt 39120
taggaatatt ttttatgtgt ttttttttt aatttttgtag tttaagggtg ggggcggttt 39180
ttagtttggt atttttttttg tagtttttta ttttttttttt atattagtta atggatatag 39240
aggtttttttt atggattttt attttttattt atttattttt atttttttga gatagagttt 39300
tattttgtcg tttaggttgg agtgtagtgg tgtgatttcg gtttattgta attttcgttt 39360
tttggatttg agtgattttt ttgttttagt ttttcgagta attgggatta taggtacgtg 39420
ttattatatt tagttaattt tttgtatttt tattagaggt agggtttttt tatgttggtt 39480
aggttggttt cgaattttttg attttaggtg atttattcgt tttagttttt taaagtgttg 39540
ggattagagg tgtgagttat taagtttagt tttttttatag atttttaaaa gggttttggg 39600
taatatagat tgggatttgt taagttatta aatgattttt ttaaataaag aaggaagtta 39660
taaattttta tttaaaagga gtgggggatg gtaatagaaa tatagttagt tagaagtcgg 39720
aagggtttta tttaatttat ttttaaggtt tttgggtaga agggataagg gaaaatttgt 39780
tttttaaata ttttattcgg tattttattt ttcggatgta agatgtagtt gtgtatagcg 39840
gtagtagtga ggattttggt ttggtaggtt tttatatttt ttgttgaggg ttttaggggt 39900
tagggttgtt taataggatt aagaggaaat gattatagga gtttttagtt gttgggggtg 39960
gttttcgcgg tttttttgggt tatttttggt gaatttattt taaatttgga agaggtttaa 40020
gtttttttcgt ttttcgtatt ttaaatttta gttacgttag ttaggagttt tgggggatga 40080
gaaaaaattt aattaagttt tattgtgttg ggtgatatag gatttagaag gagaggggtt 40140
tttggattgt ttaagtttaa tgataatttt agtagttggg tttttaagtt aattagtttt 40200
ttttttttta gtagttttcg gggagtatta cggtgagttg tgaggagtta gtttagtacg 40260
gtatatataa cgtaggggtt ttttcgtatt tttattttat aaatattagg gtttttcggg 40320
ggattagttt ttttattttg agttattttt tttttatcgt ttttttaata aggaatagag 40380
aaggagtttt tattgtttaa ttagatgtag aaattgacgt acgtatagag aggttacgtg 40440
tgatatggtt tggacgttgt ttttttttaaa ttttatgttg aaatgtgatt tttgatgttg 40500
gaggtaaggt ttgggggggg tggggtttgg gttatggggg tggatttttt atggatggtt 40560
tggtgttttt tttacggtta taaagttcgg gagatttgat tgttatagag tttgggaggg 40620
ttgggtgagg tggtttatat ttgtaatttt agtattttgg gaggttaagg tgggtggatt 40680
ataaggttag gagtttggga ttagttcgat taatatggtg aaattttatt tttagtaaaa 40740
atataaaaaa attagtcggg cgcggtggta tgcgtttgta attttagtta tttaggaggt 40800
tgaggttgta gtgagtcgag gattgtgtta ttgtatttta gtttggttga tatagcgaga 40860
ttttatttta aaaaaaaaaa gagtttggga gttttttttt tcgttatgtg acgtgttagt 40920
ttttttttta tttttttatta tgaggaaaag tttttttgggg ttttttttaga agttaagtag 40980
atgttggtat tatttgtata gtttgtagaa tcgtgagtta aataattttt tagtttcggt 41040
tattttttat agtaatttaa aatggacgaa tataatgtat tagttttttt ttttggattt 41100
tatagtgtgt ttggttgggg ttgtgttttg gggaaatggt aattgcgagg tttttataga 41160

tcgggttcgg gttttaatta tagtcgtatt ttagcgtttt gatattgttg agtggatgtt 41220
taaaaagtat tgttgggtga atgtgcgagt gaatgatgaa gatggaggtg gttgttggtg 41280
ttagggtaag gttttaggag tttggggagg tggtattagg tagtttaggt taacggttaa 41340
ttttggatag ggttaggagg tagttagaat ggttaaggat agttaaggga ttttaggtaa 41400
gtggttttat tttttttagg cgggtaggaa gtaggaatta gattaagggg tgttaggtgg 41460
tagtggtatt taggggtttt gaggagtagg tgtaggttta gtagggttgg tggtgttacg 41520
tgtggggtcg atcgtggttt cgtttattgt ttttattttt tggacgtatg ggtttatggt 41580
ttttaaggtt ttttttgtt tttacgttta ttttttttagg ttttattttt agacgttaag 41640
ttattttttt attttattt ttttcgttgt ttttatattt gagttgattt ttttttgag 41700
ttttggtgaa atttagaga gtttagaggt gtagtttata tttttgcgtt ataaggtttt 41760
tgtagttttt atttttttgg gatattttat tttagagttg ggtgtgtatt tattttattg 41820
gattagaaat tttaggaatg tttattttgt atgtttttgt tttttatttt tcgttcgtta 41880
tttagttcgt ggttgttatg tggttgggtt ttggtagtta tcgtagttgg taatatataa 41940
ttagtatttt tgtgtttagg tttcgtgttg agttttttta gttttatttt cgtttgtttt 42000
taaaatggtt tttgatatgg taattattat tatttatttt ttagataaaa gattatagtt 42060
ttaggagagg aggaatggta ggtttgaggt tacgtggttc gtaataggta aatggattcg 42120
gtagttattt tattttcgag tggggttttt ttttgtttgt agatttcgtt gaggttttag 42180
agtttggagt tagtgagttt ttattgagta taaggatagc ggcggtgtcg gggttggaat 42240
aaggtaggga ggggtttttt aaattatagt tttggttgtg gatttatttt gggatttcgt 42300
tttggttggg aattttgttt tagtttattc gggatatttt tttattttga gttttgtatt 42360
gggttgtgta cggggtttgt tgaatgagtg agggtaggag ggatggtgag tagggtggaa 42420
agcgattatg gtgggtaggt ttttgttgga agtttgaaat atttttggcg agtaggaaaa 42480
tattagggga agaggttata gtttttagaa gggttcgttg gggattgttt tgtagttttt 42540
ttatcgggat aggtagaaaa tttagttttt ttttagggtg agtttaggaa gtttgtgggg 42600
gtataggttt tagttagggt ggcgcgtggg tttatttagg ggacgttgtc gtggtttaga 42660
gttaggttaa attttatgta ttttgtaaat aattatttat aataaataag gtttatttat 42720
tttttaagaa gagtaaaata ttataggagg tattttttta ttaattaaat acgttatagt 42780
gattttattg cgttattgag ggagggtatt agggtgttcg gtttagagta aattgtggtt 42840
agggggacgg gtatattgtt aaatcggtta gattttttatt tttgaaaatg tgggttaaat 42900
ttttttttaat aagttataag gaaatttagt gtgaatttta tgtttttatt attatcggtt 42960
gttttggaag attgggagga ggggtttttgt ttttttttta tttttggtta tagggtttga 43020
ggttataatt tggattggga ttttaatttt tttttttgggg aagaggttta tttagggtgt 43080
ttgttaaggt ttagcgtata aatacggaat tcgtttaacg tttgtttcgg tatttttggg 43140
tttttttatat tgggtttttt tatttgattt tttaagtagt gagagtgatg ggatatgggg 43200
cgtttttagg ggagatttgg ggagatggag taagtttttt agtttaaggt gaggtttcga 43260
ggtatggttt atgtagtgat gtttttttacg ggaggtttga ggtatgatta ggagtttgga 43320
gatttagtag aggtttgggt atgggatttt cggtacgtag ttagagcgag ggtcgaggtt 43380
gtatggatgt aagtaggtga ttttgtgtta tcgggtgtta tttgtgtcgg gttttgtgta 43440
gtcgtttttta ttttattgag ttttagggag gtgaggtaat tattttaggt atatagtttc 43500
gggggtaaag gttggattta ggtgtgtttg attttagaat ttaagaagat aagtagagga 43560
gtttgagttt tttgagatcg tagtgatggt ttagtgtttt aggatggtta ggttggtttt 43620
tgtagtttta gttaatcgtg tttttttttta gttatttagg gttttagcgt tggtttttga 43680
gatttggtat tggttatagg ggtttatttt tggttgtgga tatttagtgt tggttattgg 43740
gatttggtat tggtcgtggg gatttgtttt tggttgtggg gatttagcgt tggtttttga 43800
gatttggtat tggttatggg gattcgtttt tggttgtggg tattcggtgt tggtttttga 43860
gatttggtat tggttatggg ggttcgtttt tggttgtggg tattcggtgt tggtttttga 43920
gatttggtat tggttatgga ggtttgtttt tggttgtggg tattcggtgt tggtttttga 43980
gatttggtat tggtcgtggg ggtttatttt tggttgtagg gatttagtat tgtttatggg 44040
gtttgttttt ggtttcgggg agttagtagt gtttgtgggg tttatatttg gtggtgggga 44100
tttggtgttg gaggattttg atagtaaaat tatagaggcg ataggtaata gtaatttatt 44160
agtatagcgt gtggtttgtt ttagttacgg tatttttttt tattttgtgt tttttattta 44220
taaagtttag tgtaggtaac gaagttaaga ggtggagaat tcgtaggagg acgtatatta 44280
tatttgtttt tcgtgtggcg tttttgtatt ttgggtggtt gagatttttt gttttttttt 44340
taggtttttt cgtgttaggt ttgcgttgtt ttttttggtt tcggttttta tttattcggt 44400
tttttagtta tagatagtag cgattttttt ttttatttaa tggggttatt gtttagttta 44460
ttattagttt ttagggttgg tagatgatat cggttacggg tagtgttttt ttcggtttta 44520
gtatataagg tataaaagat attattttgt tttaagattt ttttagttac gtggcgtatg 44580
ggaagcggtg tcgggaattt tttagggtag ttttttgttt cggggggagat cgtgtttaaa 44640

ttttcgagga aatgttgttt tttggttttt tttttgaaaa attttttaag gagattttgt 44700
gcggagcgtg tagggagaat ggagattttt gtgagattgt ttcgggatag ttttatagaa 44760
attataaatt atttggataa aagttgaaaa tttgcggtta tataatgttg attatttatt 44820
taaatattcg ttgagtaagt atttatgacg tttgggaatt taggacgtgg atcgataaga 44880
tataattttt atgttcgttt tagtgataga gagagagacg gtattattaa tttcgttcgt 44940
tgggggtttt tttggagggg gcgtgtaggt ttagggttga atttgtttag tggatgtgtt 45000
atttacgttt gcgtattttt tggttttgtg gtggttttg gaataaaggt tgagagataa 45060
aagataagga tttgggggag ggaggacgtt tatgtatatt ttattgggga tattagtaag 45120
gatgattgtt tagtgattat ttagtgatcg agttatttgg tgtttggagt ttggtattta 45180
tagagtaggg cgggggtagt aggttttgtc gggggtatag gtttgagaag ggagtgttgt 45240
tagttaggtt cgacgggata gagagcgaag ggtttttttt ttgaaggtta tgtagaggtc 45300
ggaggttaat gttagtaatt ttattaacgt tagtttttg agatcgattt agagttgggg 45360
ttttaagagt agttttgggc gtttaggagt taagatttta tagagggtgt tttttatttt 45420
tagtttaggg aaagtgataa atttggagtg tttttgttta tggtttaatt attgaattta 45480
aatgaatgta ttgataaatc gtatttagtg ttatttatag ggaaaaggga gggaaaaaaa 45540
aaaaagaaag agagttaatc gcgtttaggt cggtcggtta gggggaggat agatgggtta 45600
ttatttttgt tatttttttag gtttttgta ttgattttt atttttagta aaattaaggt 45660
ttagtttttt ttcgtagttt cggagtcggt tgggatgaag taatttttgt aggtagatta 45720
ttaggtttat tgtgatggtt ttagtaggga atttagttta ggttatagta gtttaggcgt 45780
ttgtagaagg tttagatttt tgggtgttaa gtttttttt tttgtattcg tttcggggtt 45840
cggtggggtg ggtagtgtgc gatttttagg gttgatgaaa gagaggagga agaggttgag 45900
cggcggcgag tttgaatagt taattttggg aggggttgag gtggggaagg tttttattgt 45960
tgttttatag gtttcggagt aaggttttta tgattttatt agtagttggg taggtatagg 46020
tttaggtgtt gagtgtgtta ggattttcgt tgtttttttgg gtgggggcga gagggatttt 46080
tatgggtttg taattagatt cgcgtttttt tagttaattt tttattaaat aaatttatac 46140
gtatagcgga ttgggtatgg tcgttatttt cgggagtttt tttgggaaag tttttaagg 46200
ttttgttgag tttcgggaat aggtttttat ttgttcggga atcgggttgg tatttaataa 46260
ataaattaaa gttcggcgtc ggaaggtaga attttgggat tagagtttta gttaagggtt 46320
ataagttttt taggaagttt tttttttaaa gagtggttgt gggacgttgg gagtttttag 46380
ggtggttagt ttttaggttt ttttaaagtt taggtggtat cgatgttttg tgttaaggtg 46440
agggttgtag agaggggtcg gcggggagga gtttttatag tttattttgg ttattgattt 46500
tatttttaag tattgaaata aagttttttt tttatatttt cggttaggga gagagggcgg 46560
aagttttaaa tattcggggt gtatgggaaa ggtttatatg tatagaagaa ttagataacg 46620
gaagttgttg gtggtggtga taggtttttt aaaggggttt tgagattttt gatatcggta 46680
gggaagttat atattgtatt aggtttgggg gtagggagaa ggggttttta aggacggggt 46740
tgtttttttt tgtggaagtg tttcgtattg tttgataaga gtagtttgga ttagtcgtgg 46800
tataatagga agttgggaga atacggtggt ttaggaggtt ggttagggtt ggggagtggt 46860
taatttagag atttggttg tttaagtggg tttagggttt tcggggtgaa agtttttgtg 46920
gagtatagtg gatttagtgt tcggaggata gatttaggt ttagataagg ttttttttt 46980
tttttttga gatagagttt tatttttgtt gtttagattg gagtgtagta gtataatttt 47040
agtttattgt aatttttatt ttttaggttt aagtgatttt tttgttttag tttttcgagt 47100
agttgttatt ataggtacgc gttattatgt tcggattagt tagtattttt agtagagata 47160
gggttttatt atgttggtta ggttggtttc gaatttttga ttttaggtga tttgtttgtt 47220
ttggtttttt gaagtgttgg gattataggt atgagttatt gcgttcggtt aataaggtta 47280
tttttgatat ggaatgttgg tttgattata gtgtttgatg gtttttgttt gtaattatgt 47340
tttcgttttt tattaaagta gggtttttag ggtagaagtc gtattttttt attttagagt 47400
gttttacgtt atttttttagg cgtatatagt gggttttttt tatcgtagtt gaattggttt 47460
tattattatt ataatgatag tttatagggg cgggggttag tgggaatata aagtggttaa 47520
tgtaatagtg ttttagttat ttcgatatag aaatggttag gggatagaag gggtaagttg 47580
ggagttagta gtaatttagg tttttagaga cgagtattaa aaaaggcgag ttatattata 47640
ggtttttttt gattatattt tagtttaaaa gagatttaag gagaaataag aattttattt 47700
ttttaataag gattcgtata ttagatgtta agtaaatatt tttttttttt ttttgaaaag 47760
gagtttttgg ggttgtcggg ggagttggtt aaagagaatg atatttatgg aaatatacgt 47820
ggttttatta agttttaagg taaggtggta atgaatggag gaggaaaatt tatagtatta 47880
ttagggaatg gggttttttt ttgttttttt tttttttaac gggttttttt atgtgggatt 47940
taaattaaat tttaatttta ttttgattta gtaggtgttg tttttatagt ttcgaataga 48000
tttgttagaa aattattgtt taatttgttt tcgggtggta tattttttttt taggaaagga 48060
attataaatt tattttagat gagtagagag ttgtttttta aatttttgat taaaatatag 48120

```
tagattttat atgaaaaagt aaagagagag gggttaggga tgaagtgtag taggttggag   48180
gttgagttag tttttagagt tattgggacg gcgttttagg agtagatggt attgtttata   48240
aagtttttcg tttttttttg ttttgaggtc ggggattatt tcgttggttg tatgatttag   48300
tttttggggt ttttttagta tgaaaagatt ttttgaaagt aggttcgtta gggagatttt   48360
ttagtttttg tttattgata tgtttttgta tggtcgatac gtagtatgtt attgtagaga   48420
agtaaagtat acgtagatta gtttcgttag tagtattttt ttttttcgtt ttttttagga   48480
acgttttgta tatatttaat atatttagga atttagagtt gcgttgtagt agtagttttt   48540
tggattttag tgtcggcgtc gcggggagtt tcggttacgg tatttcgagt attgagatta   48600
tttttagttt ttagttagtc ggttttcggg agtttagagg ggtgataatt tgagcggagt   48660
gattttagag aaagcggtgg ttaattcgaa tttttttttg gttttttttgg aagttaggga   48720
aatttttttgc gaaaaggaaa tatgttttaa gaagtttcgg gtagtaggag aatttagtta   48780
tttagtttag ttggttaaat atttataaag agatagttgt gattatataa agttttattt   48840
ttttattttg aggtcgaaaa tttggttttc gcgtggagat cgagtttttc gttatttttt   48900
ttttggggat ttcgttcggt tttgtcggtt tcgggtcggg gtttagggat agcgtggttt   48960
tcggtatcgt tattatggtt attagggttc gtttcgcggt cgcgttattt gagagttttc   49020
gagttagtgg tgtaaggttg ggaagaataa aggagtagga tttattttag tagcggaacg   49080
tcgggttgcg ttggacgcgt cgagagtcgc ggttcgttta tcgggttata tggtttagag   49140
tcgagtagag gattaaaggg gttttgtggt cgcggaataa ggagtttttt agagcgcggg   49200
gtagaggtag ggagggattt cgattgtttg ggaagggagg ttcggtgggg aattgtttta   49260
agggggtagg gcgaggggag taggaggagg gggtaggtag agataggggc gtttagcggt   49320
aggttcggcg tagaagtaat ttgaaggtac gggagggacg ttaaggagtt gttatacgtt   49380
aaaggaaagt gatagggttt ttgggaagag tgagggggag ttgttaggaa gaagagaggg   49440
gaagaggttt ttagggattt ttggttatgt cgtttttgtt tttgttttttg tgtttgttag   49500
aggcggtgtt cgtgaggagt tttttttttt tagttaggtt ttgaaattga tttggatatt   49560
tttttttttt gtgttttgtt tgatattttt gtttttttagg tgtttttttta tttatttgtt   49620
tagatatcgt tatagttggg tttttaggga gtatggttag ttattttaga aagttattta   49680
gatttgtagt tagggttgga atatagttat ttgggtttgt ttttttttatt ttaggtgttt   49740
gttggttggg attatggaag ggagaaggag taggagagga ggggtagggg atgggggcgt   49800
agtttatttt gagttttttt ttcgagagtt tttggattta gatttgattt agtttttgtt   49860
tttttagttt ttttagtttt tgggggattt ggttagtgag ttagtttttt tttttttttt   49920
tttttgtagg tttgggatgt gttgggattt agagggggtat aagtaagggg gtttttagag   49980
gagggagttg atattatagg ggttgggttt ggacgttggg atttgttaga tttattttag   50040
tgttacgatt tttgaattta.agtgagttat ttttagtagg ggcgtggtta tttttatatt   50100
aggagagagc gtttgggagt gggttcgttt cgagggtttg ttgggggttg ggagggattt   50160
atttaaatgg aaagaagttt ttggtgatat tttttgaggg ttaggggttg aattttggaa   50220
gttacgtttt taggtagttt ttacgggatt ttatgttgag tgttgattgg gttttatata   50280
tttttatttt ttttatttcg tatttttata cgtggttggg ggtgattgaa gtttcggggt   50340
attcgttata ttggttaaat aggttagtta cgtttagcgg ttttttagtt agtagttatt   50400
ggtatttgat tcgtcgtttt ttattttttta tggttaattt tttttttaag gtttttttggt   50460
tttacggttt tttttatttt tatagtgaac ggtcgtgggg agttgttgtt attggttttc   50520
gggcgttagg ttagagtcgg gggagtgttt gggagaagat tgtatgttcg gagtttttgt   50580
ttttatttga atgggaggga ggttggtggt agattagaga tttgggtttt gagttaaata   50640
ttgaaattta aggttttggg tttgttattt aatatttgtg gaatttgatt ttttttattt   50700
ataaaatggg tattattaat attagtcgtt aaaattatag taagatatat acgtttaggt   50760
taggaagtgc ggttatttag aaaatatagt tttttttttt tttaagaagt taattttttt   50820
agtattggga gtgagaagag tttgtttttt ttattgtttg tagtttagtt tagggtattt   50880
ttattattgg tttttaaagg gtagttttag aaatcgttga taaaggatgt tagaaggaat   50940
tttttttttgg ttaggtgtta gggttaatag aaagtttcga ggatgtagtt tttattacgt   51000
ttttgtttcg tgggtttttt tttttttcgtg ggtagaattt tttttttttg gttttttttt   51060
tcgaattttt ttttttttgt ttttggtttt aatttgtttt ttagtttttt ggttttttag   51120
gtttttttta gggtttaaga ttttgttttt ttttaagttt agtttttttt tttttttttgt   51180
ttttggattt ttgagttggg aggttagtat tttgggtgtt ttgttgaggt ggtcgttttt   51240
tttgtagtgg tttttttttgt tgttattaaa tgagggtttt tagagtatag agaaggtatt   51300
ttcggtttgg ggttgtattt tttatataaa gtttttgttt tttagtattt tttaaagttt   51360
tttatcgttt agtatatatt tagtatttag gtagagtagt tttagagttt tttcggttgg   51420
ttttcggttt aatttttttag tagaaacggt atatgggtta tagacgtaat tttatatttt   51480
ttagtattta taataaaaaa tgtaagaata agttaggtat agtggtttac gtttgttata   51540
ttagtatttt gggaggttga ggtgggtaga ttatttgagg ttaggagttt aaaattagtt   51600
```

tggttaatat ggtaaaattt tgtttttatt aaaaatataa aaattagtta ggtatggtag 51660
tagttatttg taattttagt tatttgggag gttgaggtag gagaatcgtt taaatttggg 51720
aggtaaaggt tgtagtgagt cgagatcgtg ttattgtatt ttagtttggg taatagtgtg 51780
agattttata tttaaaaaaa aaaagaaaag aaaagaaaaa ggaaattatt aaaaaatgta 51840
agaataggtg atattaattt taatattatt tatttaatag agtttaaaat attattaatt 51900
taatatgtaa ttaatgtaaa aatgatagat atttgatatt tttttttttt tttttgtta 51960
ggattttgaa atttagagtg tattttatat atacggtatt tttttatttg gaattattat 52020
attttaggtg tttaaatggt ttcgtgtagt tagtggttat tatgttggat agtgtagttc 52080
ggagagtttt aattttttttt tttttttttg gttgtcgggg aatagagttt tgttttgtta 52140
tttaggttgg agcgtaatgg cgtgattttg gtttattgta attttcgttt tttgagttta 52200
agtaattttt tcgttttagt tttttaagta gttgggatta taggcgtata ttattatgtt 52260
cggcggttat tttttgtatt tttagtagag atggggtttt attatgttgg ttaggttggt 52320
tttaaattat agattttaat tgattcgttc gttttagttt tttgaagtgt tgagattata 52380
cgcgtgagtt attacgttta ttttcggata gttttaattt gattttagga taatattagg 52440
ttattagaga gttgttgatt tggattatat tatgatttga attttttaga ttattttcgg 52500
tattatgttt ataagttata tttagaggat aataggatat agtttggtga aatatatagc 52560
ggtatttttt ataagatatt ggtgaggtta tagattttat tattagtgtt attcgtttgg 52620
ggatatttta tttttagaaa atgtagagtt ttttttaaga aatgaatgtg gttaggtgtg 52680
ttggtttata tttgttattt tagtattttg ggaggttaag gcgagtatat tgtttgaggt 52740
taggagttta agattagttt gattaatatg gtgaaattta gttattatta aaaatataaa 52800
aattcgttag gcgtggtagt gtgtgtttgt agttttagtt atttaggagg ttgaggtagg 52860
agaatcgttt gaattcggga ggaggaggtt gcggtgagtt gagattacgt tattgtattt 52920
tagtttgggc gatagagcga gatttcgttt taaaaaataa ataaaaaaag aaatgaatgt 52980
tttaggtata cgttaagtat tttttagacg attttaaaga tattaattat tgtgattgac 53040
gacgattttt tttttttttt gggtgtgttt gttttgtttt tttagtttat aggtgtttag 53100
gatttaaagg tagttcgtta taggttatgg gatttggttg gtttaaaagg gagagtgagg 53160
ttattggaat ttttttagga gtgagtttga agtttggaat gttttttggt ggagggtttt 53220
gaagttagga ggcgatgtag acgtgggggt tgagatatta tttggggtta tttgtaaaga 53280
gggatattta ggtagtgatt ttaaggaaag agtaagggga ggagagggcg gtatatttac 53340
gttttttttaa tttttgttcg ttcggaggtt tacgtatttt atacgtcggt tttttcgtat 53400
ggatattttt ttaataagta atttatcgtg tttatgttta tttattttaa agttatatag 53460
aattgtattt attaaaaatg aatttatgga gatagaaagt attttggtgg ttgttaaggg 53520
ttggagaggg cgaatgggga gtgagttttt tatggatacg ggtttttttt ttggggtgat 53580
gaaaaagttt tggcgttaga tagtgttgat ggttgtacga tattgtgaat gtatttagtg 53640
ttattaatgg taaattttat gttttgtgta ttttagtata ataaaaaaat atatatttaa 53700
agtaatagaa tttaacgtgt tttttttttt ttttgagtta gtttgttttt ttgttttata 53760
ttttaaaatt agaagggttt tgagatatat tataggtttt tagggtaggt tgaataattt 53820
ttttttttta aattgtgtta ttttatgtgg taatagggat tttgtagatg taattgaaga 53880
tgttgagatg aagagattat ttaggtgggt tttaaattat aagcgttttt taagagggag 53940
gtaggttagg tgtgatggtt tatggttgta attttagtat tttgggaggt tgaggtaggt 54000
agattatttg aggttaggag tttaagatta gtttggttaa tatggtaaaa ttttgttttt 54060
attaaaaata taaaaattag ttaggtgtag tagtatatgt ttgtaatttt agttattcgg 54120
gaagttgagg taggagaatc gtttgaattt aggaggcgga ggttgtagtg agttaagatt 54180
gtgttattgt attttagttt gggtgatttc gttttaaaaa aaaaaaagag gaaggtagag 54240
ggagatttga ttgtagagaa ggaggaggtt gtaggaggat ggaggtagag gttggaggga 54300
tgtagtcgta agttaaggaa ttttagtaga gtttttttatt taaagttgga agaggtagga 54360
atggattttg ttaatatttt gattttagtt cggtgagatt gatcggtttt tgatttttag 54420
tattgtaaag gaaggagttt ttgttgtttt aagttattga gtttgtgata agtagttata 54480
gcggtaatag gaagcgaatg tagtttttag tttatttttt cgtttatagt aatataggcg 54540
ttttttttgtg tagaatttg ttaggtagtt agtgagcggg ataggatttt ttttcgtttt 54600
tattcggggt tttgtgttgg atttttaag gttgttgttt ttgagatagt gaatttttta 54660
tatttataga gatttatttt aaagttgaga aagtgttttt tttaaggggc gttagaaaga 54720
aggttttaag gtgtttttgt tatatgttta tttgtttgtg ggtaatattt atagtttttt 54780
ttcgtgattt atttaataga agggttatag gggagggagt attattggtt agtttatttt 54840
atagtaaggt tttattttgg aagttatagg ggatagttgg gatttttatt tttaatttag 54900
ttaattggga gaagagttta gaatagtttt ggaggttgat atttgttagg aagtgtggtg 54960
atatttacgt gttgttgaaa tatatagtaa tatagtagta gagaggaggg gatgtggttt 55020
tatagttttt tttttattta tttagtacgt acgtatatat atatatacgt gcgtgtatat 55080

atgtattaat ttttagtttt taggttttgt attttgtttt tgttaagttt ttcggttatt 55140
attaagttag gttgtgagta tagagtttag tttttttgtt aattttttt taatttattt 55200
tgggtaatat tttaatggga tgtagtagat gttttttatt aaatgttaag agtaatattt 55260
tttttatttt tttgtgagtt ggcggtcgcg tttttgtatg ggggtgcgga ggaaagggtt 55320
ggggagtagg aatgtggttt tgagggttgg tatgtatttg tagttgtgac gtttcgaggt 55380
agtttatatt tttatttata gattaatttt gagaaggggt tgggagtatt tatagttttt 55440
gttattttg ggttggtagg ttggggaaat tttgagtagt tcgagtgaag gtcgttttta 55500
ggtaggtatg gtgggataga ggggttttg tatattttt gtgaggtagc gtagttgttg 55560
ggttgtttta gtatatttgg gtgatatgtt ttttttttgg ttttttttta tttggtttgg 55620
gagaacgttt aggatgtttt aattttaaaa ataagtaaat ttttttttt tgatttattt 55680
attttttaa attgtgaatt ttttttttgt ttgaatagtt taattttta aagtaggttt 55740
atattacggt ttttagtttt ttatcgttta tttattttt agttcgagtt atttggtgtt 55800
cgttcgtttt attattaatt tagtttttga aaaaattttt atggttagta tttagtttta 55860
ttttatttga tttcgagggt agtgtttgat acgtattagt tggttttttt ttttttttt 55920
tttttttcgt atttttagt gttggggttt agatagtttt taagggtttt ttcgtttttt 55980
gatttagttt tttggagtga ttatatttc gttttagttt tttagtttag ttgtttttt 56040
atattttgtt ttaggttttt tttttggaat attgtttgtt tttagagatt taagttttta 56100
tttttatat tttttttttc gatttttagt ttttttatta aagttttatt ggttgttttt 56160
tttcggtgtt ttaaattagt ttttttttgt ttagatttta taaggttagt ttttatttta 56220
tgattgtttt tttggattat tgggattatt ttttattaat atttattttt tatttcgttt 56280
tttttgttt atgtagggtt ttgttggagg atttttttcg ttttattatt ttttttttta 56340
aatagttttt taggtttttt tgttaaagtt gaaatttttt ttttgtattc gaggattttt 56400
gtaatttggt taatttattt ttaagtttga atttttattt ttgtttaagg agtttacgtt 56460
ttttagttag gttttattgt ttcgtaaatt ttatttaaaa ttttttttgg taaatgttat 56520
tatttttagg aagttttttt gatatttttt attatatgtg atttttattt ttagagttag 56580
attttgattt ggagattttg atttttattt atttttgttt tttttgagtt ttttaggttg 56640
taggttttag tttatgtttg tgggtttttg tagtaggagg gtttattttt atttttttaa 56700
gaagtgttcg ttttattttt cgttaagttg taatttttta taagtaggta tttttaatat 56760
gtttttttat ttttttttt ttttttttt ttttttgaga cggagtttgg ttttgttgtt 56820
taggttggag tgtagtggcg cgattttagt ttattgtaat ttttattttt taggttttaa 56880
gtgatttttt tgttatagtt tttttcgtag ttgggattat aagtatgcgt cgttacgtta 56940
agttaatttt tttttgtatt tttagtagag atagggtttt attatgttgg ttaggttggt 57000
tttaaatttt tggtttgaag tgattcgtcg attttagttt tttaaagtgt tgggattata 57060
ggcgtgagtt attgtattta gtttaatatg tttttttata ttttttttga aaagttatta 57120
ttatattaat agtgtgtttt ataaattgat aataatttaa aatgtgattt attattaaaa 57180
gtgtgatttt atttttaaat aatattgttg gttgtggttt gtagttagtt ttattatttt 57240
cgtgtgatga ttattttgta gattagtaat ggttttaggt ttatatttaa tttaattatt 57300
ttgtttttt aattttattt ttttttgagt taatattaag tgtgttttag agttgtaaat 57360
ttatggttga tataaaatta ggatttttaa tatttaatat aaagtaatcg ggttttgtat 57420
tggttttaat agtaattata aattacgttt gtttttgta ttgggtatat tgtatttagt 57480
ttataatatg tgtttttgtt tttttattt ttttattaa gatggtatat aggtttaaat 57540
ttgtttttg ggagtaattg attttttttt ggaagatgtg agtagttttt atattatatt 57600
taagaagata ttgttataag attttatatt ttttatttta gtttttaagg gtttatttat 57660
tttttaaaa atttatttgt tttgttataa tatgtgtttt ttttttttta tttgtttttt 57720
taagatatag ttttattttg ttgtttagat tggaatgtaa tggtatgatt ttagtttatt 57780
gtaattttcg tttcgtgggt ttaagtaatt tttttgtttt agtttttaa gtagttggga 57840
ttataggtgt gtattattat attcggttaa tttttgtatt tttagtagag acggggtttt 57900
attatgttgg ttaggttggt tttgaatttt cgattttagg tgatttattt gtttcggttt 57960
ttcgaagtgt cgggattata ggcgtgagtt atcgtattcg gtttataata tgtgttttta 58020
ttttttttt tttttttatt aagatagtat acgggtttta agttttttt ttttttttt 58080
tttttttaag attagttggt tgggtgtagt ggtttatgtt tgtaatttta ttattttggg 58140
aggtcgaggt aggtagatta cgaggttagg agtttgagat tagtttggtt aatatagtga 58200
aattttgttt ttattaaaaa tataaaaaat tagttaggcg tggtggtggg tgtttgtaat 58260
tttagttatt cgggaggttg aggtagggga atcgtttgaa ttcgggaggc ggaggttaga 58320
gtgagtcgag atcgtgttat tgtattttag tttaggtaat agtgtaagat tttgatttaa 58380
aaaaaataaa ataagtggga agaggggaga tagtggaata aggagtttaa tttgtaattg 58440
attgtgaata attaattgag ataatttatt attttcggat tagttacggg ttttattttt 58500
tatttgtttt tttgagttac gtttttagt gaaattttat acgtatgtga ttaaatttgt 58560

```
ttttttttt attaatttat tttgagtgag tttaatttat agatttttaa atattgaatt 58620
taagagggta gaggaaatgt attttttttt cgttataaaa atattttatt tttttattgt 58680
agtgtcgttt gttttattta tagtcggtaa tttggatttc gtttttttaaa gtttatgaaa 58740
gtaggataga ttgtttgtag gatgttttcg ttataaaagg agggttaggt ttggtaggag 58800
gaaagaaacg gaagtggtaa acgtttattt tttgcgttag atagaggtat taggtgttta 58860
ttgttttggg gagtaaatat ttttaaaaac ggaaagaaaa atttttagtt ttattggggt 58920
gtaaaaagtt atgatttttt tcgtggttaa atggttgggt ggtttgggag ataagcggta 58980
gttattttga agtttagttt atttgtttgt aaatggaggg taatgaaatt atttgttgta 59040
tgggtgtttg ataggttgtt gtttaaattt tttgtttttt tttatggggt tttggggaag 59100
attatatgag ttagtaatat aaaagtattt tgaaaattat acgaagtgtt taagtatggg 59160
ggcgttggtt gattttaggt ttgtttttat aagttggtag aaaatttttt agagagggtt 59220
ttgttttttt tttcggggta ggggtcgtag tgttagattt gatggtattg gtggttgttt 59280
aggttatttg gtatgagatt gttgttagtt ttagatttat tttaagtttg ggagggaagt 59340
aggtcggggt gggggttttg aaatttttta tatttttggt agagatatta gggtaggtaa 59400
aagtgttatt tatggtaaat taagaagttt gtttgaattg ggtttaaatt agtttagttt 59460
agaatggagg ttagttttat tttttttttt tagaattagg agtttagtat attagaggtt 59520
gagaaaacgt tggaatagtt tttttatttg tttgtttaga gatagggatt ttaagttatt 59580
ttttttttta gtttatttta taaatttttt gtgttagatt ttttttgttt aggagttata 59640
ggtttattt gggagttatt tagggtttta aggtttttat tatttagttg tttattttat 59700
taggttaggt ttgtaggtat ggagattagt aggatgtggt ttaaatttgg ttgtacgttg 59760
attagttgag tgattttggg tttatttttt aatttttggg aggtaatgtt tttttatttg 59820
taaaaagggg aaagggttta gcgaggagag tgataagtaa aggagatggt atttgtgtag 59880
acggtattgt gcgggcgttt agcggatgat tacgcgttat tttttttgttt tttttttttg 59940
tttttgtggg agcgtgggta gtagggagtt aagaggaagg gagtagttgt tcgtttggga 60000
cgtaggtcgt tttgaattgg aagagaaaaa ggaataagta gtagttgttt attgataagt 60060
tttatgtatt tgtgttttaa aattttaaag gtgtatttga aagatttttt gggtaaattt 60120
tttttttatag atatacgaaa gttaggtgta gtggtttacg tttgtaattt taatattttg 60180
ggaggcgaag gtaggcggat tatttgaagt cgggagttcg agattagttt ggttaatttg 60240
gtgaaatttt atttttatta aaaatataaa aattcgtcgg gcgtggtggt gggcgtttat 60300
aatttagtt atttaggagg ttgaggtagg agattcgttt gaattcgaga ggtggaggtt 60360
gtagtgagtt aagattgtgt tattgtattt tagtttaggt gacggagcga gatttattt 60420
taaataaaaa taaatttaaa aaaaaggtat atgaaaaagg taaaatgtta tgagtgtttt 60480
gtcgtaatgt gttagttata aatgggaatt ggaggatttt ttaagttatt ttttttttta 60540
ttttttttt ttttgtatat gtattaatag tttttttgggt gttggtagta ttaggggatt 60600
tggggtgttt tataggtttt gtttgtttag attttagagt taaggaggag atataaaagt 60660
taaaataggg tggtaagtgg aataaatggg gattagatgg ggtaatttgg tagtattcgg 60720
taaggttttt tttaatgagt ttttaaagag gtttagtttt taggggaagg aggcgtttta 60780
ggtagcgggg gtttgtagag gcgaaggttt agggcggggt ttttagggaa gtcgtttagg 60840
atgttcgaaa ggaggggagt aatttacgag tttggtgata gggtcggtta taaagtgtta 60900
tgaagtatta attaggttgt ttattagttt ttaggataga aagattatag tagttaaggt 60960
gtggggattt gtttatttga tttttattta gtttagaaaa tgattgtttt agggttatag 61020
gtaatagttt aagttttgta agttgttggt tatagggtgt agttgcggta aggtggggac 61080
gtttattagt taagtttttt aggatttttg ggttgtcgga tatgtttgta ggacgttacg 61140
ggggtaggtt agattagggg tatttttatt tttttttttta ggttttagtt tttaggttag 61200
agtgtttttt ggtttttggg gtttttaggg ttgtagatag atagggagag gtttgtgtta 61260
tggtgggtta tttggttttt gtaggaagtg gttagaggtt ttagtatttg gatatagtag 61320
ttattttttt aatttttgtt aatgttgttg ttgttcgggg tttatttttt ttaatttttt 61380
tttttaatta gggaggaaag tatttggag taggaattgg gttatttagt ttgatatttt 61440
agtatgattt tgtatgattt tgggttagtt atttaatttt tttgggtttt tattttttta 61500
tttgtaaatt aggtagataa tttttttaaag tttttttttag ttatagaatt ttaagtgtta 61560
aaataaaaaa aatttataaa aaggaaatat gtgtttagtt tatattttcg tttttatttg 61620
ttaaaagggg aaatggattt gttacggggt tttttttttt taaatagata tgggagattt 61680
tttttatgat tgtagaattg gagtagttta gaggagttta gaatttttta gagtttgaga 61740
tttatagggg aatttatttt aatttttcg ggatagaagt attgttgttt aatggtattt 61800
aatttttatt attgttgtag taaatgatta tatatttgtg atgttaaata atatagatta 61860
gttttatatt ttattttatt aggttatagg gaaggtgttt atagggttgc gtattttttc 61920
gaggttgtag gggagattgc gttttttttgt attttttgtt tatttgtatt ttttggtttg 61980
tggttttttt ttttttttta agtttttttt attgtatttt ttagatggtt tattgcgttt 62040
```

ttaaaaattt ttgtgatgat agcgggttcg tgtggattat ttaggattat ttttttattt 62100
taagattagt tgattagtga ttttagttgg ttttgttatg ttatttaacg tagttttagg 62160
tgttggggat taggatgtgt ttaattttgg gagttattat tttgttaatt ttaagatata 62220
ggttttggag ggagatgaag ggtaggatta taggtttagg aggttgtttg cgtgggttcg 62280
aattttggtt ttattattaa ggaattgggg gagtatatta tttgtaaaat gggggtcggc 62340
ggggaattag ttttttggtt gttagcgtag agtgaggtag tgtgtgtagt gtttggagta 62400
gtttcgtgag tatttgtttt tttttcgtta aatttataag aattgatttc ggtttaacgg 62460
ttttttttaaa tttttagttt ttttcggtta taaggttagg agtatttttt tgtagggtta 62520
tttaagtttt tagttttttt gaaagtttcg gtttatacga tgttggaagt agatgtgtcg 62580
tgggttgttt atttatgtat ggttatcgga gaggttcgga tgatgtcgtt tttaatgggt 62640
gtatagtgta gggtagggtt aggtattaat aattagttat agttattagc gtaagtaagg 62700
ggttttttatt gttattttttg gtttggggtt agggagaagt ttcgggaggt atttgatttt 62760
tttataggga gtggggttat aggagaatgt attcgaaagg ggttgggggg tagtagaaag 62820
ttttttttag aaattttatt tttggttcgt gatagatgat ttttttaggt tattattggg 62880
gtttatttgg gtttttgtag tatggagttt ggttatttta gaaggatgat tgggtaggtt 62940
agggttattg gcgatggtga cggtaggttt ttttgaggtt tttttattag aagtattagg 63000
aggttagagt agtcgataag tttttatttg gtttttgagg ggtagaaggt tttgagtagt 63060
tttttgattt ttcgaggttt ttttatttta ttgagagatt ttcgagagta ttgagagtta 63120
ttttttggtt ttttcgtgag agagttggtt atataaaggt agttatatat ttagtaagat 63180
gaatttataa tttttgagtt taggtttatt tcggtttttt tatagggagt ttttgggggc 63240
gtggggtggg ggtgtagaag ggaggggtcg gtgttttatt tttgggaatt aggttatgag 63300
ttttggattt aggttaaatt atagaggttt tatttttttt taggtttttt gttttttaat 63360
tatttttgta tagggtattt tttttttttt cgtagggttt tattggggtg gggtgaggag 63420
gagttataga attatagtag ttgttatacg gggtgttaa ggtagggatg ttgatgttaa 63480
tcgaatgggt acgggaaggt agagtaggag gtcggcgttt tgggtttggt taggttgtgt 63540
gattttaggg aatttatttt atttttttcg gtttttagtt tttttattg taaatcgagg 63600
gagtgaggtt aggtgatttg tataatcgcg tgaatttgtg ttttgtattt tttatttatt 63660
attttaaata atgtttttaa attttagttt tttaatgttt cggtattttt ttgtttgaga 63720
aatatttacg gttgtttttt atgttgtgtt ttaaatatag gtattttggt ttttttttgg 63780
gtgtttttat tagggttaga atgggtagtt tattttgtag gaatttttaa ggatttttag 63840
ttatttttag gatgggggtt ggttgttcgg aagcggtttt tgtttatttt cgtcgtagtt 63900
tttattacga ggttagtgta gtaaaatagg aggtgtaggt ggtcggggtt aggggttagg 63960
ttattttttt tagcggggta ggagaggggc gagaaaagtt ggatattgat tattttaggt 64020
tgagggtttg cgtggaggag ggggtttatg gtaaaggaag tgggaggatg ggaaggagtt 64080
tttgttggat atttttattt cgttggtttt agtttcgttt tcgttttatt tgcggggaga 64140
aagtgaggtt ttttgttttg ttaaatttta gtaatgaatt ttatatttat tttatttttt 64200
gtttattgga aatttttttt ttatttttttg gattcgtttt gaatagttgg gttgtagaag 64260
tcggtggaat gaaatatgta gggtggttta gaggggattg tgtcgaatag cgttgtgttt 64320
tcgtttattt tttttttttga agataaggtt tttttgtttt tgtttttttt ttgggaaagt 64380
tggagagggg gttgaggaaa tgggatttat tattaatttt ttcgtgtacg ttggttttta 64440
gtaggtattt ttatattttt taattaggtt cgtagggtgg aaaggtagtc gtcgagttta 64500
atttaatttt tatttagtcg tggaagttag tgtggatggt aaagtattta tgtataaata 64560
tatatacgag gttgaggtta agagaatacg cgtttagttt ttttttggtt ttttagtaac 64620
gtttttatgt tttagttttg ttttaagttt taaacggtgt aaggtttttt taggtggagg 64680
aatggaagtt tttaagaagt tcgtgtttta gggattatat agaggtaggg tttaggagta 64740
aaggttttta gatttcgtag tgcggcgttg gtaagtttgc gttgttgtat ttatattgtt 64800
ttgggatgaa ggcgatatgt tgattttttt taggtggttg taggatagtt aggaggaggt 64860
gagatgttag ttaaaggtag ttatagataa tagtattacg gattattaga aacgttattt 64920
tcgtgttaaa gcgttcgttt tttttagttg ttttattttg tgttaatatt ttttgggagt 64980
gatatgtgtg agattgggtt gtgttgatga gtgattattg gtatttttag attatattaa 65040
ggaggggtta ttttgttgtt aattaggtgt agggtaggcg gtattggggt acgacgattc 65100
gttcggtatg gttgttttat tttttttttt gtttgagtat aggttggtag gtttagagga 65160
gaggaggaaa tagagttatt atggggggcg tgtttagggg tgagttattt taagaagtta 65220
tagaattagg gtatgggaga tattgtttag ttttgttatt aatttgtcgt gcgaagttgg 65280
ttaagttatt gtattttttt gggttttagg ttttgtaatt agagggttgg tcgaagaggt 65340
aaatgatagt ggttagggat gggggtattg aagttagatt tttcgtattc ggattttttt 65400
tttgttatgt ttagagggag gtttgggtag gttatttaat atttttgggt tgtgggtttt 65460
ttatcggtga aatgggaata ataatagaat taattttata gtgttgttgg gaggattaaa 65520

ggggatattt attttaggta aagagttttt aagatagttc ggggttttta ggaagttttt 65580
gttattatta tcggatatta ttatttttgt tacggttgtt agggtttcgt ttagtatcgg 65640
tagtttgatg tcggtagagg aagggtgagg tttttgtttt tttgtagcgt tttttttttt 65700
tttttttttt tagttatggt aataatagtt gaagtttatt tttagtttta gcgattggtt 65760
atttttttga attaaggatt gaaggtttta ggtttgttat ttaaggttta ggtgtaagag 65820
gggtgagtta tcggttttttg ttttattat atttaggttt ttatacgttt ttttaaatag 65880
gtttttgttt tacgtgtaag agaagatgtt aatttttacg tttggggcgg gggtgtttat 65940
aaatgttttg gacgtgagtt ttgacggggg ttatggtgta ggttttgtta gggtattttt 66000
aattataggg gattatttgt agtttataga gggtttttttg taggttaatg ggattttgtt 66060
attttggttg ttattttgtt tagttatttg gtaaggaaaa taggttgtag atttagaggt 66120
agtacgggtt tatatgttag cgagataata taataaggga aggatgtgta ggacgaggga 66180
gttatggaga ttttatcgta gttttagtta tttattagta gattttgtgc ggacgtcggg 66240
taggttattt tattaatttt taggatgttt ttgtaggttt tgcgggtgga tagggtgaac 66300
ggaggtataa ggagggtaag tttgataggg gttggggttg ggtttggagt tagaatttta 66360
gttagttttg ttatcgtatt ttagggtatt tttcgtagat agtaggttaa agtaaggttg 66420
tttagattaa aggggaaggg gttttgtttt ttttattaag ggtttttttgg agattttaat 66480
atagattttt agtttataga tattaaattt agggtttttg agaagtgaga aatgtgaaaa 66540
acgtatagaa tagttgtagt ttagcgtttt taatacgtta gatttatata ggtatatata 66600
gcgtttttgt agaggttaga aagttttgtg ttgggaagtt tttcgatcgg gggaggtttt 66660
atagtaagtt tttttttttg tttatttatt aacgggcgga aggttttaat taggattata 66720
tgggaagggt tagcgggagg ggtagagagg gaaaatgaat aggtaatatt aagtgtttgg 66780
ggcggggggc gtaggggata tttaggaggg gagggtaggg gtgtattttt tatttaattt 66840
atagggaacg ggggttcggg aggtgtagga gaaggtttcg gtatttttat tttttttttt 66900
ttgtagattt atttatagtt tgtttacggg tagttagatt tagttatagg ttagacgttc 66960
gtttagttt tatttttttg gtttattgaa agtaagttag gatagtgagg gggacgatgt 67020
tgttattttc ggtatggtta tgttgggttt cggggtgggt gggaacgtgg ttggggcgtt 67080
attttttttg gtcgttatag gtataaaagt tttttcgtgt tttagtgtta tatgtgggtt 67140
ttttttttgt tgttttaata ggacggatat ttaagtagat tttgttggtc ggggggggtt 67200
ttcgtattta gttttatttt gatttagcga atttttttttt tgttttttgg ggtaggagtt 67260
attaggtcgg gttttaggta atggttagtg aaggggttgt atggaggggt tttttaggtt 67320
tataggaagg gaggtttttt tttttgtaa cgtggttgta gtattttgtg atcgagaggg 67380
atattacggt tggagatggt gggatggagg atttttata gtattttcga tgtttagaat 67440
attttatttt gtataaaaaa gttttaaatt tttttgaggg ttgaagttat ttagagttgg 67500
ggtttttgtt ttttaggtta aaagtatttt gtcggagtag ttgagagtat agcgggaggg 67560
gaggggtagg agggaatagt ttcgttacgg cgatagggga tttttttgatt ttaagagggt 67620
ttgtagatat tattatttat ttttagtttt gaattgtttt ttgttaaggg gttagaaatt 67680
tttgggaggg ggattatagg gggtttcgtt tatgagaata gtgattgtgg ttaggttata 67740
gtttataagt tagtaatttt gtttagtcgt cgcggtggtt tttttttgat ggtttttaga 67800
aaaagcgttt tattttttgt tattagtgtt ttgaaattta agaatatttt ttaaatagta 67860
attgtagtaa aagttaatgt tgcgtgagtg tcgtaggtat tatttttagg gttttatata 67920
tttttcgttt agtttttata aaaattttat tttatttttt ttttcgtttt tttttttaa 67980
tagtttagaa aacgagtacg gaagggttaa gttggtttga ggaggggttg gtggaatagt 68040
ttgagggttt agtttttaag aattttatat tgtaaaggga atttttgggt tagggagtag 68100
ggtatagttt ttattttttt attttcgttt ttagtattag gtttgatata gacggggttt 68160
cggttatgaa taaatgaata agtgaatgag agtttgttga gggaagaagg tgatagggat 68220
aagggagaaa taaagtagtt aatgtcgtcg tgatgatgac gatgttaacg gtagtattga 68280
tatgtgggtt tttgttatgt taggatcgtt ttaagtattt tgttttatga atatttttgt 68340
tttttattat gtttcgagtg gatattttta ttagtttatt ttatagaggg gaatatagtt 68400
gagcgtattg gttaaggtta tgttgttagg aaaggttaga gtttaggtag tatgtggttt 68460
tttagtcggg ttttggttta tacggtttta taggtagttt tatgaaatac gtagagagag 68520
tatgtgaggg gtttttttgt tgtttttat ttttagttgg ggtttatttt ttgttgaaat 68580
ttttttaatt tgtttttaag tttcgttagt tacgaaatat ttttttgaat atttagtatt 68640
atttttttat ggttatttgg ttttattttt ttatagggta agttttgggc gaaaagtaga 68700
aaggatgaaa atttttgaga gaggtgataa cgtggtaatt tttttttttt tttttttttt 68760
tttgagatag tattttattt tcgttgttta ggttggaatg tagtggtacg attttggttt 68820
attgtaattt ttattttcg ggtttaagtt attttttgt tttagttttt tgaggagttg 68880
ggagtatagg tatgtgttat tatattaggt taatttttgt gttttttgt agagatggag 68940
tttcgttatg ttgtttaggt tggttttaaa ttcgtgggtt taagtaattt gtttgtttta 69000

gtttttggg attatagacg tgaattatcg tgtttggttg aggtggtttt tatataaagt 69060
agagaaaatt atttttagtt taaaataaat taataagtat tgagtgttta tgtagaaaaa 69120
gattagaagg aaacgtgaat atgttaatag tgtttgtttg gaggacgtaa ttattattga 69180
ttattttttt tatatttttt aatttaatta ttttgttata agtaggtgtg atttttaaaa 69240
ttataaaaga taaacgttgg ttttttgttt gtgttttgtt ttttgagata gggttttatt 69300
ttgttattta ggagttggag tatagtggtg tggttatagt ttattgtagt tttaaatttt 69360
taatttaagt aattttttta tttgagtttt ttgaggagtt gggattatag gtaagcgtta 69420
ttatttttag tatttttgtt gtttagattg gtttcgaacg tttggtttta agcgattttt 69480
ttatcgtagt tttttaaagt tgggattata ggcgtgagtt attacgttta gtaaagattg 69540
ttttttaatt aggaagatag atgtttttta gtattttttg tttttttttt tttttttttt 69600
tttgatacgg agtattgttt tgttgtttag gttggagtgt agtagtatag tttcggttta 69660
ttgtaatttt tgtttttcgg gtttaagaga tttttttgtt ttagttttta gagtagttgg 69720
gattataagc gtgtattatt atatttggtt aatttttgta tttttagtag agatggggtt 69780
tcgttatgtt gattagtttg gttttgaatt tttgatttta agggatttat tcgttttggt 69840
tttttaaagt gttgggatta tatgcgtgag ttattatttt cggtttgttt tttagtattt 69900
ttaattttg ttttttggta ttatttggtt aagtagatga aaagtttag tgagttgtta 69960
gtcggtatta ggttggggtt ttttttaggt agttttaagt ggttaggatt tggttttttt 70020
tttagagttg ggtttagaaa ttaagatcgg gaatgcgtga tggttgtttt gcgggttttt 70080
gttatgaggt tattttttttg gttttagtga tcggtcgttg gttgtaggga gtatgtgggt 70140
ttagttaggg tttgtgttaa tatggttttg ttttatttac ggtttagtgg gtttttttat 70200
ttttcgttgt atgataattt taggaaaaat gttaggaggg agggagtcgt tgggggtgtg 70260
ataggagaga ttttttaggg atagatttgt aaaaattgtt ttaaagtttt ttttaaggat 70320
ttttaaggtt tttattgttt tttattgata ggttacggtt cgggtagaga aagttgggga 70380
tggagttggc ggggggaggg tggttagtat tcgtaaggta aggagatgag ttgggagaat 70440
aggggttggg agggtagttt taggttagtt tttgttttta gaattattgt tttttttttt 70500
tggttataaa gtgaggttgg ttagatatag gtttatttta taggttggga ggttgggcgt 70560
gaaagtattg tgtttgtatg gggtatttta tggtgaatat tattagagtt aatttttagg 70620
gtttgttgtt ttatagggaa gttagtgtaa ggcgtttatg gaatttagaa gtatttaaag 70680
gatttgatag ttatgagttg ggaggattta gttttgtagt aggagaaaga gaggtagaga 70740
gataggtttc gtaatatttg ttttattcgt tgtgttgttt tgagtatagt ttattttttt 70800
gtggtttcga gttttagttt tgaggttttt aagatttttt gggttttcgt agtttaggat 70860
gtttatttga aaattaggtt tagttagtag ggttttttttc gttagtttat tattggttag 70920
tttttgttta gtatttttta gtttattatt tttatttgtg taaattttat attagtattt 70980
taagtttttt ttttttagg aaggagattg tttttagatg tgttttttgt tttttttcga 71040
ttaaagttta gaaaatagga attagtttg cgtttttttt ttttatcgaa ttgttggttt 71100
ttttggatgt ttttttgat tttttgttaa tatttttata atataattag tttttagttt 71160
tttttttaaat gtaatcggcg agtacgttgt agtttaggcg gacggtgatg ttttttatttt 71220
gtaatttgcg agagttagta tattttttag ttaggtgggt tgtaggagtt agtcgtggcg 71280
agggtaggag gtttgcgtgt tgacggtatc ggtattatta aaattacggt tagattaagg 71340
ttacggttaa cgagtttttt tatttgtaat tttttttttta ttaaggtggt tttagtttta 71400
gtttcggttt tagttttggt tttggtttta ggagttgtag atatattttt aagtttttat 71460
tttaggagtt tggttttata tatatcggag gatacggagt tggggtataa atagttaaga 71520
ataggtatat ttgtaattag gagtaggggg cggagggttg ttgagatgtt tttattttta 71580
tcggttgttt tttttgacgg tttattattt attatatatt gattgtataa ttagggttgt 71640
gatatgtttg tttttttatat tatattatta aatttttaaa tttgttttta atttatatac 71700
gatgaggtaa atttagagtt aataatttgt ttattagttt ttgaatttat gggtggtttt 71760
aatttttttt tttttagttt ttttgggttt atatgagtat tgaggtataa gggaatttta 71820
ttagttggga agtgtgtttt ttttttgttg attttatagt ttttttagtt tgtgtttgaa 71880
gttgttaata tttttttatt tagtattgtt ttttgtagtt tgagttgtcg ttgtgtgatt 71940
tagggttagt gttttaattt ttttggatta tatttgtaag atgaggatgt cgtcgtatag 72000
tttattttaa atggttatta agttgtttgt atagttaata tttgtcgagt ttgtgatacg 72060
attagttttg ttttaatttc gaatgtggta tttttattta gtttggggggt ggaattgtta 72120
gttttttttt ttttggataa ggaagttgaa aggttttaga aggtattaag gttagttgat 72180
ttgttagtag gtatagtgtt tatttgttat ttatagtttt tttttgatcg tttcgtttgt 72240
ttttttagtt gagattttgt gtgtatttga atattttgtt aatggttgaa tttgtattag 72300
ggttattgtg ttagttgggt ttgttaggtt atcgttttcg agttttgttt aaggtagggt 72360
tagtgtttat attttgtttt tttgttagtg ttttatttaa ggttttggaa aggttttgga 72420
gttagagatt cggttaaatt ttggtaatgt tttatatttg ttgtgtgatt atgggttagc 72480

gatttagttt ttttgtgttt ttcgggttgt tgtgagggtt ataggagatg tatagagggt 72540
tttgagtttg gtggttggta tgttgtaaag gttagttacg tggtgtaagg atggtattga 72600
tgataataaa tgaatcgaac gaaaattagg ttaattgggt ttggggaggg gcgttggtta 72660
aatagattcg agtggattag gattttgtat atgatgagta agcgggaata gttttaaata 72720
gtaagtcggg taggtttagg atgatttagt tttgggtgtt gtttagggtt ttttttcggt 72780
ttttttgtcg gtttggtttt tttttgtttt agtttgattg tttttatggg ttttaataag 72840
tttttgatta ggattgaggt agaatagata gggtttaaga ttttgtaggt aggtatagag 72900
tgtggatggt aaaaggagat gttttaggtt ttttgtcgtt tttttttttt cgaaggttga 72960
taatgttaat ttatagaaga agttgttttt ttcggtttcg gattttattt taatatagtg 73020
tatgaggtag ttgtgtttaa ggggtgtgat tatttttgta gatggaattt atttattgtg 73080
tttagtttat tgttagagtt cggaggacgg gggaagtgag gtggggtatt ttttatagtg 73140
aagggaaaat ttagtttagt tatttttttt tgtgttgttt ttggaattta ttttgttatg 73200
gttttatttt aatttagttt tttaaagttt tagagagttt tgtttttttt tttgtatttt 73260
atttcgttta tttgtgttgg gttagttttt tagaagtttc gtttttttgtt atttttaggg 73320
tattaaggtt ggttggtggg tttagggatt tttatttttt gatttagttt tagtggttta 73380
ttttatattt atcggtattt ataggtgtgt tttttatttt cggtttttgt ataggcggtt 73440
ttatattttg ggagtttagt ttttttttaag gatttacgtt agagtatagg gatttttgtt 73500
tttgattttt ttttagtttt aattacgagt agtttatgtt atagtaattt tttagttgtt 73560
tgtagttgtt tgttaagtta tgataatggt ttttgttttt taatttaatg tttgtatata 73620
gataaatcgt tatattttaa gtagattaga tattattttt tttaggaagt tttttttgat 73680
tgttttagtt ggatagaggt tttttttttt tgtgtttttt taatgtttaa taggtattta 73740
ttgagtattc gtgatatgta aggtatttat aatataagtt agtgtttttt gtttttttag 73800
agggtatatg aataaataaa cggtaagatt ttttaatatt tatttatgtt ttatagaaag 73860
ttatttatag gtatgggagg gttgttttaa ttgcgggtga tagggaggtt tttttaagat 73920
ggtagcgttt tagtaagatt tgaatgaggc ggttaggagg tttagggagg gatttttttag 73980
gagggaataa gtgaatattt tttcgtgagg atgagttttt atgttttagg agtagttagg 74040
ttaggggatc ggtgtagtga atagggggttg aggggagtgg ttaggtgacg tagggtttta 74100
taggtcgttt tacgatgagt tagcgtttta agtatagtag atatttaggg aaggtttagg 74160
ttggggagtg atttgaatag atttttttatt taggagttta tcggggtttt tgggtttacg 74220
ttgttgcgtt attgtatgtt ttcgggggtt cgggggggta tttttttatat tattattata 74280
ggtagtattt ttgggggagg tgtgttgtgt aggggattcg ttggttattt tgtcgggtaa 74340
attatagggg attgtgtagg ataggcggtt agtttaggag ggataattat ttaagtaggg 74400
gttagagggt attcggattc gggaggagtg tttaattttt taggtttagg taattagagg 74460
tgggggcgg ggtgttgttt tttgatatgg gaggtttggg ggtttggttg tattttttgg 74520
tgtggaggtg ttgggaggtg tggaggtttt tttttgaggt tgggattttt tggttatttt 74580
gatttattgt ttcgatttta ttttttagtt tgagggtatg gtttgaattt atagtcgtat 74640
atattttttt ttgtataggg ttttgcgggg tcggtatagt agtttttttt ttatagatta 74700
ggatgtttag ttttaggagg gttgggattg tttaagatcg tataattagg gttagatata 74760
gttgggttaa ttttgtgatt tttggtttta cgtatagatt ttaagtatat aggtaaaatt 74820
ttgttggttg ggagggttgg ggggtagatg agcggggggt agtttttttt tagtttttta 74880
atatgttagg tttttgggtg ggagggggta taaagatttt attttttttta agagatagag 74940
aaagaggtag tggagggttt tttttttggtt gaggttttag gttttgagtg gtgaatgtaa 75000
ttttatttgg aggttggaag ttaggcggtt ttgggggtgt gcgaggttcg ttagtttagt 75060
tgtagttttt tttatgtaag ggaatttttt ttaggtagcg tttttatgat tagagagggg 75120
gttaggaaga ggtgagggtt gtatttagag tgatattcgg gtgggtttta ttttggggat 75180
acggttatat ttttttttcg gattttgaa gtttttaggt gcgggttaag ggtttttagt 75240
tgtaggtagg agagggtttt atagttatag atttggggag tttagtttgt aaagtttaga 75300
ggttttcgga ggtcgttggt tcggtttttt gtgtagttgg gttttttttgg gaggtttttt 75360
ttcggtattt ttagttttta gttcgtacga tttttttatag ttgggtttat tttttttaggt 75420
tcgggttata tggtgtaggt tagtggggtt ttgggggagg gtagggcgtt aggaatagtt 75480
tcgggggtgg ggggattgga gtagagttta ttttggtcgt ttgtttattt tgtatgaagg 75540
acgttagatt tttatatgtg gttattcgat ttcgtttaat tatgcgtaag ttttggttat 75600
ttttatttta ttgatgggaa ttaaattggt taaggagaag tttagaattt gagtagagta 75660
ggggtttaag tttttttagga gtttttaggta tttttttagtt tttttttttcg agtattgagt 75720
ttatatattt agtgttcgga tgtttattta tttgggggtt cggcgatcgt tagttagtat 75780
ggtttataaa gggtttttgt gtaataggtt ttatttgatt tttttaaaaa tttttcgagg 75840
taggataatg ttatttaatt tattttatag ataaagtaat tgagttataa aatggtttgt 75900
taaaagatat agaggtagaa agattaatag taaagagttt ttttgtgatt ttgattagta 75960

```
aggtttttag aaaaaagtaa gggatttcga ggtttttata ggaatagagt tttggaggag   76020
aataagagtt tagttttatg ttatttttaa ggggtatttt gaaggttaag gtattgggtt   76080
taagtaggtt ggattgtgtt ttttgtattt tttttatttt ttgaagtttt ttttttttta   76140
tttaggtggg tcgtggaggt gtttataata tataatggaa tagttggtag gttattgggt   76200
tttttattga gttaaggggt tcgattagag gtttaaatag tggtatagaa attatgagtt   76260
tcgttgtaaa ttagtttagg ttttggagtt aagtagtttt gtgtttatag ttagggttag   76320
tcgtttttag ttgtgtggtt tgaggttagt tttttaattt ttttgtgttt cggtgttttt   76380
tttgtagtag taataggaat cgtttttttac ggcgatagtg tagatatatt ttatgaagcg   76440
tataaagttt cgagtttagc gtttggagta cgttaggacg tagtaagtgg ttatttatga   76500
tttatgaaat attaataagg tgaggggtgg aatgatagtt tttttgcgtt gtttttaaaa   76560
tggaaataaa tttgtttagc gtttaaagta gtagaaagtg agttaaggat aggtttttag   76620
cgatatgttt tggtagagtg ttttttgttg gttttaggta gggtttaggg cgggcggtta   76680
ttttttagat gagtttatcg ttggtatttg tttttagagt atatgtttgg atttttttaga   76740
tttttttttt tttttttttg attttttttt attttgtaat ttttattttt tgttattttt   76800
ttatttattt ttttttttta tttttgttta aaaataaata atggaggtta aatgtgttag   76860
ttggtttttt ggaaaggttt ttttgtagta ggaacgcgac gggggtagtg gaggattgtt   76920
tagcgtaagt tacgtgattt tcgcgcggag tcggggaagg ggaggcgttg agatcgaggg   76980
tgttaggatc gaggtattga gatggggtta ttggataatg gttagggtta gttttcgttt   77040
ttattttaat aggggttttt atatttaggg gttatatttt taaagttttt tggataagag   77100
agataaattt tattataggg ttagaaacgt tgtttttagg tcgttatttg ggtgggggtg   77160
agagatgttt cgtcggtttt taaatattta ttttgtggat aaaattatta aggggaattt   77220
aatgttttta tagtagtttg gttaagatat aggtattagg tcgggcgtag tggtttacgt   77280
ttgtagtttt agtattttgg gaggttgagg cgggcggatc gtttgaggtt aggagttcga   77340
gattagtttg gttaatatgg tgaaatttta tttttattaa aaatataaaa attagttagg   77400
tgtggtggtg tgagtttgta gttttagtta tttaggaggt tgagatagga gaattgtttg   77460
aattttggag gtggaggttg ttatgagtgg atattatatt attgtatttt agtttggatg   77520
atagagcgag taagatagtt ttaagaaaaa aaaagatgta ggtattgttt tagggtttat   77580
aagtataatt tgatttttga tgttggtatt taattttgat gtagatttta gtaggaaaag   77640
ggagacgaat tagaaggttg tggggacgag gggtttatag tagtgtttat tagatttgtt   77700
ttggtaggta taagtgtttt ttggaggttg ttagataagt agagggttta ttttagagat   77760
tttggttttg gagggttggg ttagggttta ggaagttgta tttcggtagg gaatttagag   77820
ttattttgag ttttattttg gaagtaagag ttagggggta gaatagaatc ggaattaaaa   77880
gtttttgtgg gtttagtttt atggggatgg gttcggtttt atggggatgg atttttgtat   77940
tattcgaggt gattattagt tcggtttttc gggattagtt ttgagtgttt tttggggaaa   78000
gcgggatttg tagaggatgg ttttttagga ttcgtttgat tatttatttt tagttcgttt   78060
ttattttagt ttttttgatt tttatttttt atttagaatt ttggagattt ggttttggag   78120
gtttagagat tagaaatgag gggagataag gggagaagtt ttttttttta ttcgttggtt   78180
acgttaatgg gttgattttt taggaagtta tattagttat tgttattgtt ttggtcgttt   78240
cgtttggttt cgaggttggg tgagtaagta tttcgttttg tttgtttttt tatttgagtt   78300
ttttgttgat gttacgagag gtttgattac gcgtttagga ggtataggat agagtttaga   78360
gttgtttaag aatatataag gttgaggttc gggttttgta tttgggaagg gttaggaagg   78420
gttaaaggaa aattttagtt gagtataatg gtttatgttt ggaattttag tattttggta   78480
ggttaaggta ggaggattat ttaagtttag gagttggagt ttagtttggg aaatatagta   78540
agatttatt ttttataaaaa aaaattaaaa ttaaaaaatt aattaggtat ggtggtatat   78600
atttgtagtt ttagttattt ggtaggttga gatgggagga ttgttgagtt taggaggtcg   78660
agattatagt gagcgggata ttgggttatt gaattttagt ttgggcgata gagagagatc   78720
gttttaaaaa aaatttttat attttatgga gtttagtaga attttcggtt agggtgtttc   78780
gattgtgaaa tgagcgtggt agttgttaat tttcgagggt gaagatttat ttattagtaa   78840
tttgtatttt ggtacggtga gaggaagtgt ttggggaaag acgtcggtcg aggtagagag   78900
tttagtttaa tttatcgtgg aggagttatt ttagttttaa agagaaagat taataaattt   78960
taggtgaatg gtttaaaagt tatttttgta tacgtagagg gtgtatttg ggatatgtgg   79020
atattttttt ttaggatagg aagtaaaatt agtaggtaga gacgtgagtt tttttgtttt   79080
tgtcggagtt aatttggagt ttttatttcg gtttttaggg aagtatagat ttttattttt   79140
atcgtagtta aaagattttt tgttgatttt tttagtagta tttagtttag ttttgtgggg   79200
agaggcggta gggggaggag atagagaaag atgagttttt ggtagttgtg attgggggga   79260
gagaaatgcg gttttaattt gtttttgttt attttataga cgggcggtgt tttattttaa   79320
aatttagggt agttgaggga ttgaaggaag agggggatat tttttaattg gggttttttt   79380
ttattttatt ttatggcgtt ttatatagga aagagttgga aggtttcggg tttttggtat   79440
```

ttagttaaag ttttaggtag gggtttagat attgggttgt gttattattt taggtagttc 79500
gggagttagc gacggtagag ttttattttt atttcgatta ttgtatagta tttcgttttt 79560
aggatagttt agtagttagg ttgttttagg gtttaaattt agatatttaa ttgtgggggt 79620
tttaggtaag tggttgaagt tttttgcgtt ttgatttttt aatttggaaa atgggatgat 79680
gaaagtaatt gttgggtagg gtaaggggta agtgaaagtt agcgtttgag agtgatgtgg 79740
ttaggatcgt tgttgtgatt atatattgag tttttttttt ttatttgaga taagggtttt 79800
attgattgtt gttagaaaga tttagattta gaaggattag gaaagtcgtt ttatttataa 79860
ttttagaggg aaggggaggt tttcgtgtta gttggttgtt gtaggggaga gggtgggaga 79920
tagatggggg atagaggagt aggggtagta ggaggaacgg tcggtaggga agttcgtttg 79980
agatttaaag gattgtttat taggaggata gtttaggttt tttttattga gaaagatttt 80040
tttggatagt tgggttgagg atttttaggt tttttgggtg gagtagaatt agttattatt 80100
gaagttatat aggtttgtaa gtataagtgg gggattgttt aggtattttt gttttatagg 80160
ggttaggtat taaggttagg gtggttagag gatatgacgt agtttagtag gggtttcgta 80220
gaattattag ttaagaagat atatgtagaa gtacgtaagt gtttcgagga ggtaaatttt 80280
ttgttggatt gaatatgggg agttgagttt ttaattattg agtcgggttg ttcggttcgt 80340
tttttttttt ttgaatagaa ggtttttaga tagagtagtt aggtaatagt atggttaagt 80400
aggggtttgt attttagttt tgttttgtag tttgattagg ttgtcgtttt tttaggagat 80460
acgggattgg ttagggtttt tttaggttgt tataggaata aggtagggtt gtgatttttt 80520
ttatggttta ggtatttggg ggagggatta tgagttttag tttaggggat agataggaaa 80580
cggatagttc gttttttttt taattacgga gttggttttt tttttatcgt attttttttta 80640
gttatgtttt ttattagaag attatcgtat ttaatgggtg gttttgtagc gatatttagt 80700
ttaatgaagt tatttggtgg gggttttggg ggatgtagtt ttttgaaata tatacgggta 80760
gtgggtgagt cggaggttgc gtggggtttt gtttttttggg gatggttagg aggtgtttta 80820
attatttgta attcgagttt atagtgatcg ggatttgttg tttttttatt ttagtttttt 80880
tttattagta ttcgttaagc gttagttagt aggtgtcggt acggtttaag gtaggtttgt 80940
agttagatta gtatcggggg agggatcggg agagagattt ttagagtgga agttatagtt 81000
tttttttttt ttaggttggg taatagatta ttattttttag ttagttttgg ggaaggggga 81060
ggggttagtt gggtcgtagg tatagcgttg gggttttttg aggtggttgt tgttgattac 81120
ggtttttttt ttttttgggt tgggtaatag attattattt tcggtttatt ttagggaagc 81180
gggaggggtt agttggggta taggtatagc gtcggggttt ttgcggcggt tgttgttgat 81240
tacgggtttg ttattttttt tttagttggg ttgttatttc ggttttgaaa gcgttttttt 81300
tagtttttaa ttcgtgttta ggaggtagtc gatattttaa gcgttgttat agtaggaggt 81360
tgggtttgta agtgtgattc gtttggggag gggtagaggg aaagattatt ttcgtttaaa 81420
tagcggtttt tttttttttt tcgggggttt ttatagggtt tttagttttt ttttggttaa 81480
gaggtaagga gaggtaggaa agttttagga atatcggtag agaagagata gggtaggttg 81540
ttagaggaag taggttgtag ggtgtttaag gttttaggtt gggttttggg aggttagggt 81600
atgggtaatt aggaggttta ggtagggaaa tttgtttcgt agggattttg ggagataggg 81660
gagaggggat atgatagtcg tttttttgttg tttttttggtt gtggttttta ggggttttag 81720
tttagattag aagtatttta tgtatcgggt ttcggtttag tttggaagta cggtttatag 81780
atttatatga ttatttgggt agtgattttt aaaaggtttt tcgttattag ttggggagtt 81840
ttcgagggga gggtggtttt gtagagtatt tggagatatt aatgggtcgt gaggaaagga 81900
ggaggcgtgg atatttttac ggttttaaga gaaaattaag gtagagttgg aggttgggcg 81960
tggcggttta tatttgtaat tttagtattt tgggaagtcg aggtgggtag attatttgag 82020
gttaggagtt taagattagt ttggttaata tggtgaaaat cggtttttgt taaaaatata 82080
aaaattagtt tgttatggtg atacgcgttt gtagttttag ttatttagga ggttgaggta 82140
ggagaattgt ttgaatttag gaggtggaga ttgtagtgag ttaagatggt attattgtat 82200
tttagtttgg gtaatagagt aaaattttat tttaagaaaa aaaaagagta gagttggagg 82260
ttaggcggtt tttagagttt gataaggagg tcgtggggtt gggttgtttt ttcggttatt 82320
tgaggggtag ggattggttt agtttgagtt ttagatttat aagaggttga atcggagcgg 82380
gatttcgaga tgaggtaggg ttattttgtt tatatttgtg tttaggtttt aatagttatt 82440
tttttttagga atttagtttg atggtttttt aggttgagag gttgttaagg ggttgtaagg 82500
tgtcgtaacg tgtgagtcgg tgggttttga tgtgtgatag ttcggggtat ttttttttatc 82560
gttagggaat aggaatgagg agttttttat ttttagtgtt tggtaggtag gaggtcgtta 82620
ttggattgtg tgtttaggtg ttattagatt tcgagggttt acggatacgg tatatgattt 82680
ttaatgatta acgtttgtgt agtaggtgtt gaggtgtttt aatttcgagt aaggttgggt 82740
ttttggaatt tttttttcgg taggaggatt tgtagttgag gaatttttaa gaaatttttt 82800
ggtagtaaat aaagtatggg gttggacggt ggatttatga gtgtttttat atgaatgcgg 82860
atatagatag aaaagacggt aaatatttgt gaataaaagg aatggttttt gcgtattgaa 82920

ttttattta tcggttttg tttggtataa agtttttagg aatttttaa tagttttagt 82980
tagaaacgtg tttttgtttg gttatgtgga aaattttata tatattaaga tatatgatgt 83040
ttttggtttt ttttacgaat gttttatgtt ttttgggttt atgatttgga atttgtagat 83100
ttttgttgtt tttgttttag agttataagg attttggatg taggaaatat tttttatgtg 83160
taagggtttt tttttttttt gttagggttt atcggttttt ttttgtttat aggtgttatt 83220
taaatttttg tatttgtggt tttttttttt taattatttc gtgtaattcg ttaatagttt 83280
ttttattttt agagtttggt tgttggagat atataaggta tatagaattc gtgaaatgat 83340
attgagtatt tattaggtat ttattgtgtg tttcgtttgg taataataag taaaattata 83400
atgttttggg tatatgagta tattttttta tgtttatttt tttagtaatt taatgagatg 83460
gttttataaa tggggaaatt gaggataggg aggttagatt ggtttaaagt tataggttag 83520
gaggggtagg gttaaaattt aaatttagat agtttggtat taaattttag tttttatac 83580
gaaaggtatt ttgtaatttt tcgagaaggt tgggattatt atttttattg tatagatgag 83640
gaaattaaga tgttaaatgg ttagatagtt ttggagttag gaggtgggtt tagtggttta 83700
tgggagtata tggaatggag tttatgggag gttcgaggtt gggatttcgt attttatttt 83760
ggtttaaggg atgaggtgat tgggagtgtt agagatattt tgggattggt tatttaggtg 83820
ttttgtattt agttttagcg ttgagtgggc gagggtggat aatttgttat ttagtagggt 83880
tgttgttcgt atttcggtgg ttttttaaag tttggttaag taaagtaaga ttgttttcgg 83940
attagagtga tttttaagtt atataagtat ttagttatgt ttcggaggat gtaggattgg 84000
gaaggttttt tttttatcgt atttttattt agttttttta tggggtagtt ggttttaggt 84060
atagattaga cgtttttttt tttgtttgtt gtcggtatgt ttttggtttt attgttgttt 84120
tagggagggg tttaagagtt tttcggtttc gttatttatt atatttcgtt tagagggtcg 84180
ggggtttagt attaacgtta agtatttagt agtaaaatta cgggttggaa agagttatag 84240
tgttgttttg atttggtttt attttatatc gggtgtcgtt tttattagtt ttttttgtt 84300
tttggtttta ttttttaggt attataagtt ttgtaggttt aaagttaaat ttattagttt 84360
ttgatttttt ttagttggga ttagggtgag gttttcgttt aggtgtaaaa gttaaggtag 84420
tgttaaaaaa tttaggaatt aaataatatt ttgaagtaat attttaaata aattaaattt 84480
tacgttgaat aaattttgat agggttgtgt acgtgtaggg ttgggtttgt tttaatttaa 84540
aattttaata tttgtttatt atagagatta ttatgtatta tttcgatatt gaaaaatatt 84600
gtattaaata ttatttgtgt tgatttttga gtttttttggt gtttttttat attttgtttt 84660
tgataagtgt ttgatatatt ttgttttagt cggttttgtt ttttaggtgt tttttttgggg 84720
ttgttttgtt tacgtttttc gtagttatta ggagttattg agttattgta tttgttttat 84780
tttgtttttt atgttttcgt atcgtttatt tcgttatgtt tttttcgtgt gttttggtcg 84840
tacgttagtt ttagatagga gttcggtggg ttgggagttt atattaatgt tgtttttttt 84900
cggtttcggt gtcgggtata gggtagtatt ggagattttt tttttttttt tttttttttt 84960
tttttgaga tatagtttta ttttgttatt taggttggag tgtagtggcg taatttttggt 85020
ttattgtaat ttttattttt taggtttaag ttttgttttt tggaattaag tagatttttt 85080
tgttttagtt tatcgagtag ttgggattat aggtatacgt tattatattt agttaatttt 85140
tatattttta gtagagacgg ggttttgtta tgttggttag gttggtttcg aatttttgat 85200
tttaggtgat ttgtttgttt taattttta aagtgttggg attataggtg tgaggtatta 85260
cgtttggtcg agatttttttg aataaataaa tattatcggt atggagattc gtttagggaa 85320
tttgtttaga ttttgttatt gtatttttgt ttgttgcgtt tagaggtaat ttagggtcgt 85380
tttagtcgtt tttatttggt tatttttttgg tttttattta ggtcggtttg ttttattttg 85440
tgggttaaga aaacgattta gtgggtggtg attagtattt ttttttttaat ttgaaaagaa 85500
tatatttatt agcggtggta gtaatttaat gtttttttagt tgatgaaggt aatatgtttg 85560
tttataatgg aatggtttat gttatggtgg aatattattt agggacgtat tggtatttgt 85620
tataatgtgg atggattttg gaaatatgat attaagtgga gaagttagat ataaaggtta 85680
aacgtgtaat tttttgtata agaaatgttt agaatcggtt aggcgtagta gtttacgttt 85740
gtaattttag aattttggga ggttaaggta ggaggattat ttgagtttag gagtttaaga 85800
ttagtttggg taatatggtg aaattttatt tttataaaaa atataaaaat tagtcgggta 85860
tggtggtata ggtttgtagt tttagttatt cgggaggtta tggtgggagg atggtttgag 85920
tttgggaggt ggaggttgta gtgagtagag attgagttat tgtattttag tttgggtgat 85980
agagtgagat tttattttaa aaaaaaaaaa aaaaaaaaaa agaatttata tagataggaa 86040
ggaaatatgg aggaggaggg taggattgat agttaaaggt tacggggttt ttttttaaga 86100
aaaaaatttt ttaaattttt taaattttta attttttaaa attggttgtg gtgacgatgg 86160
ttgtattatt ttttgaatag attgaaaatt atggaattat gtattttaaa tgggtggatt 86220
gggttacgcg gtgatttatg tttgtaattt tagtattttg ggaggttgag gcgggtggat 86280
tatttgaggt taggagttcg agattagttt taatatggag aaatttcgtt tttattaaaa 86340
atataaaatt agtcgggcgt agtggtatat gtttgtaatt ttagttattc gggaggttga 86400

ggtaggagaa ttggttgaat ttaggaggcg gaggttgtag ttagtcgaga tggcgttatt 86460
gtattttagt ttaggcgata agagtgaaat ttcgttttaa aaaaataaat aaataaataa 86520
attggtggat tgtatagtag tttatttgga ttttaataaa gttgtttaaa aaaaaatgaa 86580
aaaaagtttt tttttttttt aaaataaata aataaaaaga aaaaaagaga gaggggaaaa 86640
aaaaaaaaaa aaagaaaata ttgagtgtag tatacgtatg gtaaaggtaa gaattttatt 86700
acggaaattt ggttttatta atatatttta tttttatttt tattttatat ataggtgtgt 86760
tttggttcgt ggtgttaatc gtatgttttt ttggaggttg tagtttagat tggttttgaa 86820
agttgttttt tgggtaattt taaatttttt ataattttttg aagtttttgg tttgttggga 86880
ttatttagtt ttttagattt agtttcgtta ttatttattt aagattgtag ttttggggag 86940
gggggttttt ttttaagggt gtagggggttt tgtggtttta ttttttttta gaatatatat 87000
tattttgtat tgtatatatg tgttttaata tggagtattt taggacgttg gttatagtta 87060
gttatgtttg tatatcgatt gatcgatcgt gttttgttga ttaattatgt taattatcgc 87120
gttgtatatt gtttttatta agcgagttaa agtaattttt tcggaataag ttgacggatt 87180
ttaaataagt taaaagatat tttttagga aaaaaatagt aatgaatttt aagaagaaag 87240
aaaaattttt aaatttatag atttatggaa aaagtttaag tttttaaagt gttagatttt 87300
tttaaaagta ttaggtttag atggttttac gagtgaattt ttgaaaagtt ttaatagaga 87360
gattattatg ttgttgttta aaatgtttta gaaggtagag ggtaaaagaa atgtatttaa 87420
attgttttttg ttaagttagt ataatgtaga tattaaaatt gataaaaata gtatatatat 87480
atatatatat atatatatat atatacgttt tattcgtttt agattaagtt cgttgttgtg 87540
tatttaagta atattattta tttttatttc gttgttgaaa ttaaaatata ttttaatgga 87600
tttatgattt taatatgtaa aaatgaaatt atagaagtat tagaagaaaa cgtgggtgaa 87660
attttttttt ataattttag aacgtaggag atttttaaat aggatgtgaa ttttaaaagt 87720
taaaagggaa agtagagatt tgataatatt aggattaaaa tgttttgtgt agaagaatat 87780
tttagaaata aaattaaaag taggtagttg gggataagat attcgtattt gttgtgataa 87840
agatgtataa ataatttttt ttttttttta gataggattt tattttgtta tttaggttgg 87900
agtgtagtgg tatgattata gtttatagta gttttaaatt tttagattta aatgattttt 87960
ttatattagt ttttcgagta gtcgggatta taggcgtatt attttattta gttaattttt 88020
gtagagatgc ggttttgtta tgttgtttag gttggtttcg aatttttaag tttaagtaat 88080
tcgtttattt tagtttttta aagtgttggg attataggtg ttagttatta cgtttggttt 88140
ataaagaatt tttttaaaa aattttaata agaaaaggat atattttttta aatgaaaaga 88200
gaatataggg ttttggttta taatatacga agagataatt tttttcgttt gatcgagatg 88260
tataaaataa aatattaatt ggagagtatt tggtttttag ttaggggtaa ggggtatagg 88320
attttttttgt tggattatta ttgtataggg tttagtagat agttgagtag gtcgacgagg 88380
ggaggggttt taaaaaattt tagtaattat tttagaggtt aatttaggaa taaggattaa 88440
ttttttttt ttttttttt ttttgatagg gagtttcgtt ttgttattta ggttggagtg 88500
taatggtacg attttggttt attgtaattt ttgtttgttg ggtttaagta agtttttttgt 88560
tttagttttt tgagtaattg ggattatagg tataagttat tatgtttagt taattttttgt 88620
atttttagta gagatggggt tttattatgt tggttaggtt ggttttgaat ttttgatttt 88680
aagtgattag ttcgttttgg ttttttaaag tgttgggatt ataggtgtga gttattgtgt 88740
ttagtagatt aaaatttaaa tgtatatatt tttgatatag aaatttatta agagtttatt 88800
ttttagttat gtgtgtatat gtataagtgc gagaatattt gttgtagttt tttaattata 88860
gtgggaagtt ggataagttt ggtagggtta tgggatagtt gatgatataa ttattaaaag 88920
gaacgtgtta ttttgggagg tcgaggtggg cggattttaa ggttaggagt ttaagagtag 88980
tttggttaat atggtgaaat ttcgttttta ttaaaaatat aaaaattagt taggtgtggt 89040
ggtaagtatt tgtgatttta gttatttggg aggttgaggt aggagaatcg tttgaaagcg 89100
gaaggcgaaa ggtggaggtt atagtgagtt atagtgagtt gagatcgtat tattgtattt 89160
tagtttgggt tacgttttgt taaaaaaaaa aaaaaagaaa aagaaaaagg aacgtgttat 89220
atttataagt ggtaatagtg ttgttaaatg gaaaaaataa aagttggtag tagaagtttg 89280
attttttttg tgtttttgaa aagtttatat ttatatattg atttttttta tataagtttt 89340
gaatgatttt gtaggaataa aatttaggag aatgttatat ttagttgtta atatggttat 89400
tttttgggag agagttgggg aatgggagag aaaaattttt ttttaaattt tatgtgattt 89460
ttattttttta tttaagaatg attgggtatc gtttttttttt ttttttttta gatgggagtt 89520
ttgttttgtt gtttaggttg gattgtattg gtataatttt agtttattgt aattttcgtt 89580
ttttgggttt aagtgatttt tttgttttag ttttttaagt agttgggatt ataggtgttc 89640
gttattacgt ttcgtcgtgt attgtttttt aggatgaaga aagggaaagg ttatagggag 89700
aatgggagtt agaatataga gtagggtttt taaatattag tttgcgggtg agagagatac 89760
ggggcgatat tatcggtttg gaatttaggg atggtatgag gtgttgttta ggggttttag 89820
ataaggtttg tttgttgatt aagtaagggt tgtgttggtt tttgtttatg tgtttgttta 89880

atacgaattt tttaggcgtt ttattttgtt ttacgaatta ttattgttta ttttaattac 89940
gttgtatagg aagaattgtg tttattttta taatgagaaa acggattaga gaggaaagtt 90000
attaggtagg gtaggggcgg tagtttaggt agtgtgaggg taaagtcggt gttttttttgt 90060
tattttttta tatacgagtg ggtaaggtat tataggagtt tatattttaa tttttgcggg 90120
ttattttgga aggcggtaga tggaattagt gatttttagg gttattttta gttttaggtt 90180
ttagataggg gtcgtggagt ggaatcgagt ttgatttagg tgtgttgatg gtagttatgt 90240
aattgtggat aaattatttt ttttttttatt tgtattggat gagatcggga atttttattt 90300
tgggtttttt aagagttttg gggttttata gagtatattt agtagtagta gtgggatggg 90360
gggatagggt taggggagtt ttatgttttt tttaatttta attagagtat ttttatttta 90420
attttttttt gggtttagtg tttagagaga acgggaagga agtttagttt tggttatggg 90480
ttattttagt ttgttagtta tagtttatat tttaggagtt ttttttggat tgaaggagat 90540
agagttattt ataatattta tagaaaattt cggagaggaa gtggagttta attgaggaag 90600
cgtttttttg tagtttttag aaatgaggtt ataggtgaga tagtgtgtta gggtggatta 90660
tatggtattt ttttaggttg tttgttggaa tttagggttt ggttaggggt tgtttatatt 90720
ttgggtttcg aggcggggag tttgagtgcg ttttttttagg gttgaatgtg ttttttgttt 90780
atatcgtata gagtatggtt gtttggtcgc ggcgcgtttt ttcggatagg ttcgttattg 90840
tgtagatttt taatttcggt agttgtttcg ggtttgggag gcggcgggta tagatatagt 90900
ttttttttt tttggtagtt gttttgattt tttgggataa gtgttgagag gcggttttat 90960
ttagtgttat gtgtttgggg gtcggggttt tattttgggg ttaggttggg tggttagggt 91020
tatagagttg tagggggcgg agtagagaaa tcgtttttag cgtttgtttt ttggttttat 91080
tttttttcgt agttgcgttt tatttaaggt attttatagt tgttcggtat tcgttgtatt 91140
ttcggattag ttttttttt ttttattat tgtacggtgt ttggtaggtc ggcgagggga 91200
ggggtttttt ttttgggttt tggagttagg ttttgttttt tggttttatt tcgttttatt 91260
acgaaggaga tatatcgggt ttggttaatt ttttcgtatt gatttttgtt tttttagtat 91320
atgcgggatt ttttatttta gatttaggtt tgagaggttt tagggatagt tcggagaagg 91380
agggtatttg tttttagaag gggaaagaga aagagattta attttaaagt aaaaggggat 91440
ttgggtgttt tttgtaagta gtttaggaaa atgggaggag gtgggagtag cgggattttt 91500
cgagtaaggt atttcggtag gaacgttagt gttttttgga tatttttaga agtagaagta 91560
tatagtttta tagaaaaatt ttgtaggttt aggattcgag gtatgatacg atttaaattt 91620
attgttttat tagatattag tgagtttttc gacgtttatt aaggtttttt gttttatatt 91680
tggtattgga cgaatttgta tattttgttt tagggagttt atagtttagg gataggttta 91740
gagtttatgt ggggtgtttt gtaaatacgg ggttagaagt tgggttgttt atggggtttt 91800
ttttttttaa attatagtgg ttattttttt tttaatttta tagtttttga tttttattag 91860
gaatttttgg ggtttagtag gtgttttttt tttgtaggtt gatgttcgtt tttttattt 91920
tttatttagg tttagttttg attttatttt ttttaaagtt atttttttat tgttttgtgt 91980
tgggtggttt tagggagttt tgtgttttga ttaattttat atttaacgtt gtattggtag 92040
ttttgttgtt tgtgttgaga tttttttttg ttttgttttt ggaatatacg gttcggtgcg 92100
gattaagtat gcgaagagtt acgtttatta ttattagttc gggggggttt gatttttttgt 92160
tttttaacg ttttatttat atgataaaga tttttagatt ttttgggtta taggaaaatt 92220
attgagatat gttgttatgt ttatttcgtt ttaattttat atttatgttt ataagtggtt 92280
ttagaattat tgtggatttt ttttttggtt tttggggtgg gtggatatat gagagtttag 92340
tttttagtta tttttgtaga tgtttttttg atagattaag aaattgaggt tgggaggggt 92400
ggaatgatag tttagaatta gttgatttat ggggtagagt tgggatatgt ttagaattga 92460
gttgggtatt gggtttggtt tttttttgt ttttagtgag tattttaaga aggtagagaa 92520
ttagatttat gaacgagcgt tgagtattcg ttttataagg tagttttgga gaaatttggt 92580
tggtgtggtt gaggagtcgg agggggtaa ttttggtggg gggggtttg agttagtagt 92640
tagtttttgt aggtttagta ttgggtgttg tagtttagtt ttattttatt ttatttagag 92700
gtatttatgt tggggggtag gaaaagagta gggatttagg agaggaggta aggtcgtttt 92760
tatttagagt ttgagaggat tagggatgat tttaaaggat gagcgttttt ttgtgggagg 92820
atagtaagga gggtgttaag tttaggggta gggttagagt ttcggaaatc gagaatttat 92880
tttattagta taaagtttag attttattag gttttggggc ggaggagtag gtagggatgt 92940
ttttgttttt tgtagcgttt ttttttttt ttttttttt taattattta ggtgtttata 93000
ttttttttg aaatgttgat ttttttaga atttttatat tgaagttaat atttatttat 93060
tgagtttttt agtataattt ttagaggaga gattaggtgt tttgggggta aagttgatta 93120
ggaaagtttt tgtttggttt ttagggacga tttatggggg ttattatatt tgtttataag 93180
aattataaaa aagttatttt aatttattat tagtggttta tttttttagt ataattttat 93240
ggtatattta ttatgtttaa gttatttatt tggtatttta tgggattttt ttttttttt 93300
ttttttttt tgagataggg ttttattttg ttttttaggt tggagtgtag tggtataatt 93360

```
ttagtttatc gtagttttaa ttttttatgt ttaggtggtt tttttatttt aattttttta   93420
gtagttggga ttataagcgt atgttattat gtttggttaa ttttttgtat tttttgtaaa   93480
gatagggttt ttttatgttg tttaggttag ttttaaattt ttgggtttaa gtaatttgtt   93540
tgtttcggtt tttaaagtgt tggatttata ggtatgagtt attgtatttg gtttttttatg  93600
ggattttttta taaaaattaa gataggttgg gtacggtggt atgattttag tattttggga  93660
ggttagggaa gttgaggtgg gtgggttacg aggttaggag ttcgagatta gtttggttta   93720
tatggtgaaa tttcgttttt attaaaaata taaaagttag ttaggcgtgg tagtttacgt   93780
ttatagtttt agttattcgg gaggttgagg tagaagaatc gtttgaattt gggaggtaga   93840
ggttgtagta agttgagatt atgttattgt attttagttt gggtgataga gagagatttt   93900
attttaaaaa ataaaaaata aaaaaaaaat ataaaaaaaa ataaagataa tagttttatt   93960
tttagaattt agtgggggta tgaaaggaat tttgaagatt atatagtaaa attttaagta   94020
tggatatcgt tggtttatag gtgaatttta ggtttttttt tatttttgta tttttttgtat  94080
tttaaagaaa gggtattcga ttattttttta tgatttgaaa aattattttt atatttaaaa  94140
aaaaaagttt agtagaggaa agaaagttta tatataaatt atttatattt taagttaaat   94200
acgaggtatt tggattaggt tttgagggat gtataggagt ttattaaggt agtgatgtaa   94260
agagaagtag aggttagggt ttaggaggtt agggtaaagg ggttgtgagg aaaggtgtga   94320
gagattaagg gaagattaaa gtaggagaag ttgggaagga gagaggtgtg agttgagtag   94380
gagagtgaat taatcgtgga ggggtttatg gtgagtgagt agagaaggtt tagtacggga   94440
agagatttta gtagtgttgt gttttgggga agttagtttg ggagtaattt agttggggtt   94500
ttaggagatt ttgttttgta tttatagttt tggggtggag ataaattata gtatagatgt   94560
tttttcgtag gtagaagtta aggggttggg cgtttgttta gagggtacgg aattttttttt  94620
atgatttttt tttttatggt gttagttgga tttttggtgt tcgggggata gaggggtcgt   94680
agagattggt tagagttgtg tatgtcgggg tttagggaat ttttagttta gttttatatg   94740
gaggggtaga aggggatggg tttgaaggaa agatgggtag gaagttgagt tggagagaat   94800
tggattcgtg tttagaacgg tcgtgatata gagagggagg acggagaagt aagtaggtgg   94860
tgtttcggag gaagaattag tggggtttag gtttgggttt ttcgagaagt tatttttatc   94920
ggattttaag agtatttttt tattgggaag gttttgtttt cgtttgtttt tgtatttttt   94980
ttatttggat ttgtttaggt ttttaggatt gttagtttag tttacggatt tttttacgtg   95040
agttggattt ttatttgtat aggtttttgt tcgtgggtgg tcgggtttgg gatgttgatg   95100
gttttttttt atgtttcggt tttaacgtgt atattttatt ttatggagaa agttatcgtt   95160
tttttagagc gttaggtttt tggtagtttt gaacgttgtt gtttttacgt ggtttgtttt   95220
tttttttttt ttttggtttg atgaaattgt atttttattg ttggggaagt tttttggata   95280
ttttttgtgt tttagttatt ttgtttacgg ttcggtgagt tttagaaagt agagttcgta   95340
ggttgtgttt ttgggtttgg ttttcggtat agtttttggt ataatttagg ggttaaattg   95400
tgtttgtttt tggaggagaa tgtttttgta tatttttatg gatatttttt tatttgtttt   95460
ttagagttag gattacgttt ttgtgtattt agtattagtt attatcgaga tttagatggt   95520
tcggtgataa tcgttttttt agttatattt tgtttgattt agttgagtgt gaatatatta   95580
atttaggatg ggggtgtatt cggtacgtta aataaaggtt ttgtattaat agaaggtagg   95640
ggaggaaata gtttttttgtt attttgtaaa ttgtttatgt ttttggtttg atattatgtt  95700
attagtaatt attgttattg agtttgggtg gttggggtag ggtggttttt tagagttaag   95760
tttagagaga tgggagaggg aatacgggat tcgtatattg agggtcggaa aggatttttt   95820
aaggtttatt agtttttggt tatttagtgt gtttcgatgt tttttttgta ggaaatgtag   95880
agttttaggt tttagattcg ttaagataaa tgtgtatttt atatgtttat gaaagtatga   95940
ggagtattgg ttttttttta aatttatatt tttttttaggt tttatatttt tttagttttt  96000
aagtttattg attattggag aatttttatt taaaaagaaa aattaaaaag gggttgggtg   96060
gtttatgttt ataattttaa tattttggga ggtcgaggta ggaggtttat ttgaatttag   96120
gagtttgaga ttagtttggg taatttagtg agaatttgtt tttataaaaa aaatttaaaa   96180
attagttagg tatggtggcg cgtaatttgt ggttttaggt atttgggagg ttgaggttgg   96240
ggaattattt gagtttaggg ggtagaggtt gtagtaagta gtgattatat tattgtacgt   96300
tagtttggag gatagagtaa gatttgtttt taaaaaagtt aaagaaaagt taaagaagat   96360
ggaaagaggg atttagagag atgtggaagt aatttaaggg tttatcgatg gaggaatagt   96420
tatataatat aagtgtatcg tataatagaa tattatttag tttttgaaag gaaggaaatt   96480
ttgttttatg ttatagtacg acggatgaat ttttaggacg ttatgttgag tgaaatgagt   96540
tagatataaa atgggtagtt attgtatttt tttatatata taaggttttt agagtggtta   96600
aataaattta taataaagat agaaaataga atggtggttg ttagtagttg ggggaggggt   96660
atggggagtt tggtttaacg gggacggagt ttagtttggg aggatgaaaa gggtatcgga   96720
gatggatagc ggtggtagtt gcgtaatatc gtgaatgtat tgaatgtcgt tgaaacgtat   96780
attgtaaagt ggttaaaatt gtatattgta tgttgtgtat tttattacgg ttagaaataa   96840
```

gaagtagtag ttaaattaaa aatttagatt gtgttgtttg tttaatttaa agaaggttta 96900
ttgagtatta ttgagttttg attttgtgtt tagaattaga tttggttttt gaggggtttt 96960
taatttagtt ggtattttat tatagtttta tggagttacg atttttgttg gggttagggg 97020
gtacgttttt gagaagggat ttaattatgt tttatagttt ggtattttta aataggtttt 97080
gtgttttata ggattggtat taattttttg tttgtaagtt ttatgttagt acggttgtgg 97140
gtttgagttt ttttaggacg ttgttggagt tttatggttt tttttttttc gttagaaggg 97200
tatttttaaa taaaatcgtg tgtagttatt atatagtttt ttggtttagg tatttttttt 97260
tagtgttttg agaattttgt tttgtaggtt aaggtgtttt ttatgttttt ttttttttgt 97320
tataattatt tttttggggg gaggaggatt agattatggg ttttaatgta agttttagga 97380
ggtatacgtt tttttttttt tttttaagtt ttcggttttt gttgggtata tttaggtagt 97440
ttgtaatttt ttaagtagga gttattagaa tgtgatagtt tttttagggg ttttaatttt 97500
atttgtttt atatttttgt atgagtttgt tttggttttt gcgtggtttt ttattttta 97560
agtttttag gttttttagg gatatatagt agatagaggt tttagtaagg tttacggtga 97620
tttataaagg aggtttttg aaatttagaa aattttttta aatttttatt taagtttcgt 97680
ttggggtatt ttggtatgtt tttattagat agagagagtt atttttttg agtttgtaga 97740
ttttatttt tatgtatttc ggtttttttg ggtttttagg gtagtatttt ttattttata 97800
tttagtttt tttttaaga ttttgtggta aagttagtaa ttaggttttt tattattata 97860
gtatttaaga tttagcgttt tgcgatttat ttttttggtt tcggggtgta gttttagtta 97920
tagggggttg gggtttgggt tttagtttgt tttgtagttg agatataggg gtaggttttg 97980
aatttttaa ggtttgggtt gggttggggg cggtgggcgt tttagtaggt ttgggtattt 98040
ggatttttat tttttgggg gttgcgtcgg tggtaggggt agtgggtatt tcggggattt 98100
tggaggtagg gggttttatt tttcggtggg ttgatttttg gggtttgacg atggtgattt 98160
tcgtttttcg aggggtcggt tttggcgttt tgtggtttaa tatttcggtt tttttgagtt 98220
cgaatcggga cgggtcgatg gtttcggcgt tttttatttg tttggacgag atgttaatgg 98280
atagtttagt gcgtcgggat atcggttcgg tcgttttggg gttcgagagt tttatttttc 98340
gtagggacgt tttgggggag cgttggttta gggagtttat atggcgggtc gagggttttg 98400
agttttttac gtttaggttt tggaattttt gggtggagtt ggttatagtg gttcggagta 98460
ggggagtttg gatttttcgg ggtggggtgg agttgggtgt ttcgattttt tcgattttaa 98520
aagattttt taaggttgaa aaataaagag aggggatgta attggtgaat ggatggttag 98580
ggaatatttc ggtatgtttt ttgttaagga gtagtagagg aaggtaggcg atgcggcggt 98640
gagggtcggg ggtttttttt cgtttttat gttttagtaa gcggaaggga tagttttatt 98700
ttaggagaat tattatgagt tttggggtag agatgtaggt tggagtggga agatgttgtt 98760
cgtcgtgttt tgttatggtt acggcgtaaa ttatatagtt ttttttttta gttacgttta 98820
tataggaacg ttttagtttt agagaagatt tttattttt tttttttttt tttgagatgg 98880
agttttgttt tgttgttttg gttggagtgt agcggtattt ttcgggttta agcgattttt 98940
ttattttagt tttttagta gttgggatta taggtacggg ttattatgtt tggttaattt 99000
ttgtattttt agtagagatg gggttttatt atgttggtta gtttggtttc ggatttttga 99060
ttttaagcga tttattcgtt tcggtttttt aaagtgttgg gattataggc gtgcgttatc 99120
gtgttcggtt ttgatttttt ttttgtcgcg gttgttttta ttttttttta tttttagtta 99180
aggtatatat tgggatatta ttagtaaata tatttttatt attagttata gcgggggata 99240
tgtttttaat aataagtgat cgtaattgta attaattaga atcgtagtag gaataaaatt 99300
ttattaatga tattaagggc ggatattgga gtttttagtg tcggcgaggc gtcgggttta 99360
tcgagtttat tatatttgta gtcgtttaat tttgggatgt aaagaatatt agtattttta 99420
gtatagatgg ggaaagtggg gtacggggta gatatagagt tagattgtat attaggttga 99480
tattagagtt taaggtttta attatttggt tgttcggttt ttttagttta gtttatagga 99540
attaatttt ttagttatta gtttttttg tattttttta tttattatta taagttcgtg 99600
tagtgatgtt aattattagt tttcgttttg tagttatttt gttttttat tagggtttgt 99660
tttttcggag gtagtgggga tggtatttgg gaagttgtta tttttatagt ttttataatc 99720
gtattttaaa tgagagttag tcgtttttgg ttattagggt tagagtaggg agggatttat 99780
atggtaggta tatttattag tttttttttt aggttagatt tatagcgtat ttttgtggag 99840
atttatagtg tttttttagt cgttttttgt tttattaggt tatagagttc gggttttatg 99900
ggattttatt attgtttttt ttgtttatag gaaaagggat gatagttggt ttttagtttt 99960
taataatagc gttttaggat taattttagt gttttttttt tattcgtttt ttttttttta 100020
aatgtattta ggtattgggg aggggaagag gaagtttaat tttttaattt tggtcgtttt 100080
agttgttagt tttttgggta aggtattttt gtgggcgttt tttttaagtt ataggtattt 100140
ttttttttg gtggatatag acgaaatttt gggttattaa gtttaatttt tattagaagt 100200
tgggtaagga gtcgtgtgga ttttagagaa tttttagttg ttataggttt tgttttagtt 100260
tcgttattgt tttagaggat atgggttcgg cgtggttaga gttgtttatt ttttaagaga 100320

gtagaaattt ggatttttat atgatatttt ttgattttta aatgttggta taaagtaaaa 100380
taagtttttt ttaaaatatt gagtagtttg gttgatgtgg tgaaatttta tttttattaa 100440
aaataaaaaa ttagttgggt atggtgatat tcgtttatag ttttagttat ttaggaggtt 100500
gaggtaggag aatcgtttga attcgggagg tagaggttgt agagttgaga tcgtgttatt 100560
gtatttagtt tgattgatag agcgagattt tgtttttata aattaaaatt aaaaataaaa 100620
taaaatatcg tagggttaaa taaaattaaa tttataggtt gtattcgttt atgggtttag 100680
ttatgatttt tagttgatta cgtggtcgta gggtagtagg atattgagtt ttagagttag 100740
gggtgtgtta ggtgtgggtt ttcgatatgg tgtttttttg ttttttgta ttttgtatgt 100800
aaattgttgg ttgtgttttg attttgtaga agttttaggg ttttaaggaa aggttgaatg 100860
tttattttta ttagttagaa agtttagggt ttaatgttta gaattgaatg taatgagatg 100920
ggtttattaa gtttattata tttatattcg tttaaatttg ggttgtaaag aatattatta 100980
tcgttttcgt atagatgggg aaagtgaagt acggggtaga tatagagtta gattgtatat 101040
cgggttgatg ttagaaagtg tagagtttaa tgtttgggtt ttcgtgcggt ttttagggtt 101100
tttcgcggtt ttttgatttg ttttttgttt tttgagtatt gtttaaattg tttttatatg 101160
gtgtgttttt gttttatggg tgttgtagag taggggtcgg tttttaggtg tttttagtat 101220
tttttcgtgc ggttaagttg gagaatagaa attagatagg agaaggtttt gttttaggtt 101280
taatttaaga aaataaatga ttttttttgg gagattcggt gaattttttt tttgggagtt 101340
tttttggagt tatttttatt atggttaatt tatgatcgaa gatattgtat taaagtagag 101400
gttgttttgg tgtgagttta cgattgagtt tacggtgcgg atgtaatgat ttattttagt 101460
taggtttacg tgtataagcg gattatttta gaatcggagt tggttttttt tttagggtat 101520
tttttttttt tttagttgtt tgcgtggttg gcgttttatt attttggtta tttgcgtaaa 101580
atgtatatgt gtgtcggttt ttaattattt tttttttttt tttaatgttt ggtgtttttt 101640
tatggatggg tttttttttt tttattagtt ggtttttgtg tttattgttt ggttttatga 101700
tttttttggg tgtttcggtt tagtattatt tttatgttta ttagttttta gtaagttttg 101760
agatagtagg ggttgtttta ttcgtagggg ttattttatt attaggacgg cgttgtttaa 101820
aggcgagttt gttttagata atagttagga gattttatag tttttagaat tgtaagagaa 101880
tataattgtg tgttagaaga atagtgagaa taggaggcgt tttatttcgg aggtgtttat 101940
tttttttagg agatgtttta ttttgggggt gtttattttt tttaaggaaa ttatgtttcg 102000
gttggaatgt taagaaggtg atatttaaat gaagaggtgt ttattttggt ttacgttatt 102060
tttttaaacg agtatgttat cgtttttttt ttttttttggg aatttttttt aaagttaagt 102120
ttattttagt ttttttttcg gggagttggt tttttatata gtttatatta tttttttttt 102180
atgttatttt ggttacgtta tgattgttga gattggttac gaggggtgtg tttcggtttt 102240
tcgggaatag agttgaatgt tttttatttt atgatattat gtttttatta ggttttaggc 102300
gtttttagaa aatttattaa gttaggcgtg gtggtttacg tttgtaattt taatattttg 102360
ggaagttaag gtggatagat cgtttgagtt taggagttta agataaattt gggtaatatg 102420
gtaaagtttc gattttatta aataatataa aaattggtta ggggtggtgg tatatgtttg 102480
taattttagt tattcgagag gttgaggtga gaggattatt tgaggttagg agattaaggt 102540
tgtagtgagt tatgattata ttattatatt ttagtttgga tgatagagtg agattttgtt 102600
ttaaaaaaaa aaaataatta aaatttatta aatgttaggc gtggtggttt atgtttgtaa 102660
ttttagtatt ttgggaggtt aaggtaggtt taaggtaggc ggattatttg aggttaggag 102720
tttcgaaatta gtttggttaa tatggtaaaa ttttgttttt attaaaaata taaaaaatta 102780
gttaggtatg ggggtgggcg tttgtaattt tagttatttg gaaggttgag gtagaagaat 102840
tatttaaatt cgggaggtag aggttgtagt gagtttagat cgtgttatag tattttagtt 102900
tgggcgataa agtaagattt tattttaaaa aaaaggttgg attataggat tataggatag 102960
gtataagttt tgagggagtt tttaaggagg agggacgtat tagattttta tttttatttt 103020
agtttgtgaa gatttatttt cgtttgaggt tttgtagagg gttgattagt gatttttaaa 103080
aagatatgtt taatatttaa ttttaagaat ttacgaatgt gattttattt ggaaaaaggg 103140
tttttgtaga tgtgattagg gatttcggga tgagattatt ttggatttgg gatgggtttt 103200
aaatttagtg ttcggtgttt ttataggaga aaggtagaag gggatttggg atataaagag 103260
atacggtggg gagggttacg tgatgagagt agagattgta gatgttgtta gagttaagga 103320
gcgtttgggt taacgtaagt tggaggaggt aaggaagggt ttttttttcg agttgtcgga 103380
gggagtgtgg ttttgttaat attttgatgt taaatttgtg gtttttagaa ttatgagcga 103440
attaattttt gttgggttat ttggttttta gtaatttgtt ttagtagttt taggatattt 103500
ataacgagat ttttgggaag atgaaatttt tggttgtata gtggtttagg gtgaggttag 103560
ttggtgtggt tatttatttt ttagtttagt tttaatttta ttataattat attcgataaa 103620
tggtttattg tgggttaacg gatacgtggt tattttagag ttgttatatt aggagtttag 103680
aggagcgaga gttgtttttt tttttttttt tggagtcgta taaaaatttt agatggttgg 103740
agaagttatc ggaggaatta tattatttta gatttttgtt ttattgttgg gatatgtggt 103800

aatgaaataa gattatttt gttagggatg ttttatgggt aattgtttt ttacggagga 103860
tattgtgagt tgtatttagt cgggaaatat taggagatgt ttgttattag gttggtagaa 103920
tagggttttt tagtattttt tttttatta gagttttaag attgcgtttt gggttttgta 103980
gttatatacg gtcgtttttt ttttttaat gtgtaggttt tttgggaagt tttttgttat 104040
tatttttata gtggaatcgg tataatttgt ttaggggtag taggttttg ttattttata 104100
cgtttttatt ttttttgtgg agggagacgt agttaagggg tatttatatt tatttataga 104160
agcgagagtg ttcgtgagtt tgttattttg tagattttag aatttcgtac gtgattgtat 104220
agatttatag gtttaattgg gtagatttaa aggatttacg tagtagggag gagtatcgga 104280
tagttttttt ttttgtttgt aaggagttaa tgtaattgaa ggatatagaa gaggtttta 104340
gttgagtatt ataattgtgg atcgtgtata gtgttagaaa gatgcggagt taggttatgt 104400
tgggaggatt tttatgttgt tattggaagg taaagtagtg ttattttttt atttatagag 104460
taggaggttt ttattttgt tagttatttt ttttatttgt gaatttaagt tttttaaagt 104520
tagattttaa tttcgtaagt ttttaaaagt tttttgggt ttttttttta atttttatcg 104580
atttaattt gttttaaaag tttgaagatc gtttatgtgt tttggtttat ttttgggaat 104640
ttattttaag gaaataatta gaagtataga aagatttacg tacgaggata tttgtattag 104700
tattaaattg ataaaatttg gaaatagttc gggttttcgt agattttta aatttatatg 104760
ttaaaggttt gtattattat taaaattata tttttgaaga tgagaaatgt cgagaatatt 104820
aagtaaaaat agtaggatat aagatgtata taaagatttt aattttattt tatatatata 104880
tgtgtagttt gtacgtatat atatatttat attcgggtag aaaataacga tttttggatg 104940
gaatgaatgg cggtgggtgt tgtttattat atttttttgt tttttgagat gtttatatta 105000
tatggggatt atttagtatg tttagggaat aaaattatcg atattgtttt tttttttttt 105060
ttttttgag atagtttcgt tttgtcgttt agattggagt gtagtggcgt aatttcggtt 105120
tatcgtaagt tttattttt aggtttacgt tattttttg ttttagtttt tcgagtagtt 105180
gggattatag gtgttcggtt attacgtttg gttaattttt ttgtattttt agtagagata 105240
cgattttatc gtgttagtta ggatggtttt aatttgttga ttttatgatt cgtttgtttc 105300
ggttttttaa agtgttgcga ttataggcgt gagttattgt attcggttaa tattgttttt 105360
aaaaaatata ttttggtatt ggttgggtat ggtggtttat atttataatt ttagtatttt 105420
gggaatttga ggttagagga tcgtttgagt taggagttcg agattagttt gggtaaatgg 105480
taagatttta tgttggaaaa ataaaattaa aaaaaaaaa atatatatat atatagattt 105540
tgatgttaat aagttggagg gtgtgggtaa ttgtatgttg gtttattttt atttttattt 105600
ttggaaaaat atttatagt ttaggatggg tatagggttt tttttatttt taagtattta 105660
cggttttagt tgttatataa aaagaggtta tagtgggtta tttttagat gtaaattttt 105720
tttttaggt ttatggcggg gtcgaaatag gagtagtttg gatttgggtt tatttagaga 105780
ggaatgtgtc gtataaggga ttattgttaa ggtttttgtt ttgattatag gttgtttggt 105840
aaatcgtttt tagtttttga aattagatta agtattttt ggtgtatata ttaggtttta 105900
gtttggggag ggaatattaa gcgagttata gagttttgtt ttagtttggg ataaatgtta 105960
tgtaaagaaa tggtagagtt taaggttaga ataattttat gcgtagttta gttttcgtag 106020
gagggagtaa cgtggttttt gtttagggta gggtggaatt ttaggtttag gttttaggaa 106080
cgttagtttt ggttaaattg ggtttttttt gagtagtagt aaagtaggtt ttgtttttga 106140
agaaatattg gttatttagg agttagggtt agggtttagg gaagtcggat agatggtgtt 106200
aaggagtagg gaagtatttt tttgtatgtt attttgagtt tgtaggaaaa agttacggga 106260
aaattttatt tggtagataa tttttataa tttttaggat ttatagagtg agtttagtag 106320
tatgtacgtt atgggtattt ttttgttttt attttttgt gatattggat tttggtatag 106380
gtttttgatt tgtgtagggt ggggtgggta gttaggtagg gttttggcgt tggtagagtg 106440
gggtttagtt gtaaaggttt ttttattttt tatttattta ggatttgggg gacgaggttt 106500
aaaggtttgg tgtgttttta aggagtttaa ggattgtttt agggttggag ataggacgta 106560
tgggtttgta tagggatcgt gttttttatt ttagggttga gagtaggaga attaaatagg 106620
agagtgatat aattatagtt tattttgagt tttagtgttt ttagtgttat ttgttgtagg 106680
aggtgatggg gtaataaggt gtttttgagg ttaggcgcgg tggtttatgt ttgtaatttt 106740
aatattttgg gaggttgagg tgggtggatt atttgaggtt aggagttcga gattagtttg 106800
attaaaatgg agaaatttta tttttattaa aaatataaaa atagttaggc gtggtggtgt 106860
atgtttgtaa ttttagttat tcgggaggtt gaggtagaat agtttgaatt taggaggcgg 106920
aggttgtggt gagtcgagat tgtgttattg tattttattt tgggtaataa gagcgtaatt 106980
ttattttaaa taaaaataaa taaataaata aaaaataaga ttttttgggt gtaggtatgg 107040
tacgcggtag gggtgattgt tgttggtgta attattattt tttgggtgtg tgtttgtggg 107100
ggttgtgtat gttgaaatgt gtggttattt tgttggtgat taaggttgtt ttggttttat 107160
tgttattaat tacgtttatt gattttatat ggtttatttt cgtgttttta tttagagttt 107220
tttttagtgt ttaagttttc ggaaggttgg aaggagtgag tagtgtagtt tttggttaag 107280

aattgaagta taaaaatttt agttttttta tttaatttta ggtgggataa ggttgggggt 107340
acggtttgta gtttttttat gttgttcggt atgggggttg ggtagttatt tgtggttata 107400
atgagttggt gaggtatttt ttattggttg gatttttgt ttggtttatt tttatattta 107460
ggtattttt ggtgtttttt tttattgaaa tttttgtttt aggatttgtt tttggaggga 107520
tataaattag tttttagaa ttggtttggg ttgtttggtg gaggagggag gtggtttttg 107580
gggtaggtgg agtaggtttc ggatagggag gaggatatag ttggggtgat ggggaggggt 107640
gtgtttttag gaagtaaaat cgtttaaaag gaaaggttta aaaataggcg tagtgaagtt 107700
gagcggagtt tgtaaaggga agttaggaaa tagtagtgtt agtggagggc ggtttttagg 107760
tttttatag agagttatat ttgtttaggg atagggataa cgtgaataga aggtaaagtt 107820
tatattaagg cggaggaggc gtttttttt tagagagtcg tagttagata gtttaaattt 107880
gttaaaggtt tgggagggaa gaattagggt attattttat ttttagttta attttttttt 107940
tagtttttt tttttttgtt tttttaaggt taggtttggt tgggttatag tgttagtagt 108000
taagattcgt ttttagtgtc ggtttagttc gtaggaggtt agtttggtgg ttaaaggtta 108060
ttgattgttt aggtttattt gtatacgtgg tttaatgatt gaggtttggg atttggggtt 108120
attatttata gtatttttgg ataaggagga aaagaattat ttttattaat ttttggatgg 108180
aggtgtaggg agcgttaata gtattttga aaattttaaa tgtattaggg gggttagtat 108240
ttagggaaga gttataggag gttattttga tggattttga tgattatttt tgtttaagtg 108300
ttaggaggag gttttttttt ttttgttttt ggtaatttg ggtttaagaa gttttaggta 108360
tttcggaggt tttttttgtt tattatagtt ttatttaaga ggtttggttt attttgtaat 108420
gatgatgtga gaatgagtag gtttgtttag tgttagagtt tgtggttgga ttttcggttg 108480
aggttaagag aaataaataa gtttttttt tttttttgta cgttatagga agagtagttc 108540
gttggttgta aggggatttt ttattttgtt tatggggtat agtagtttat agtattttt 108600
ttagaaacgg agtattggta ggtttgggtt tttgtaagat ggaatataaa ggggtgggta 108660
gttgatttta atagttagtt tttgggattt taaatttttt tttattttt aattttgtga 108720
tgttaatttt tttttggata aagtttttta ataatttggt aagttagtat tagttgaatt 108780
agttttagtt tgaattagta gtttttttta gtttagttat agttttattt ttttagaagg 108840
gaaaagaagt tttacggatt tttttataag gaatttttag aatttgttat ggtgttcgta 108900
ggggatattg gtattaattt tgggtttatt aaatgtagtg ggtttaatag tgtttttttaa 108960
aacgatatgt tttaagtttt aatttttgga atttgcgaat atgattatat ttggaaaacg 109020
gatttttgta gatgtaatta aggtaagggt tttaaggtga gattattttg aatttagtgg 109080
gttttaaatt taatggtgag tatttttata aaagatagaa aaggagaaag atatatagat 109140
aggggggagaa agttacgtgg agataggggt tagagattag agatgatggt gatgcgtttg 109200
taggttaagg aacgttagga atttttagga gtcgtcggaa gttatgtttg taagttttta 109260
ggttgtggta ttttgttata gtaattttag gatatagatt ttaggaatag aatatagaaa 109320
agaaacggga ggaggtttag agtttgttgt ggttatttta gttattttat tcgatttttt 109380
tagtgtttcg ttaattttga agttttttttt agttattgag ttatcggttt tgaaaaggag 109440
atatgggtgt tttggagttg gtttagtttg gtaggaggta gtttcgtttt attgtttttt 109500
tattagttat tagtttatat aggtttttta tagtgggtaa gtgcgttgtt gttttagttt 109560
tcgtcggttt tttttttgtt cggttttatt agtcgtagag tttttttgttg ttgtttttttt 109620
tggtttaaga ggtttatata tttagttttt ttgggataaa tttagtttat atttgttgtt 109680
ttatcgtagt tatttaaggg ttttttttat taacgtgttt tttaagggat agtaagttat 109740
acggtcgttt tatttggggt tagaattgtg ttttattaga gtaaggtttt ggagtaatta 109800
tatttagggt tagggagtta tttgtttaag aatagaaacg tgtggtcggg cgcggcggtt 109860
tacgtttgta atttagtat tttgggaggt agaggcgggt agattacgag gttaggagat 109920
cgagattatt ttggttaata tagtgaaatt tcgtttttat taaaaatata aaaaattagt 109980
cgggcgtggt ggcgggcgtt tgtagtttta gttattcggg aggttgaggt aggagaatgg 110040
cgtgaattcg ggaggcggag tttgtagcga gtcgagatcg cgttattgta ttttagtttg 110100
ggcgatagag cgagatttcg ttttaaaaaa aaaaaaaaaa agcgaaatgt gtagtttttt 110160
ttttttttat ttttgaaata tgaaggggag gtgattgtag gtttttgggg aaagattttt 110220
ttatttttt tttttttttt tttttttaag acggagtttt gttttgtcgt ttaggttgcg 110280
gtgtagtggt gttattttg tttattgtaa gtttcgtttt tcgggtttat gttatttttt 110340
tgttttcgtt tttcgagtag ttgggattac gggcgtttgt ttgggatagt tacgtagtag 110400
tttttgtttt aagttatttt tttttacgta gagtagaatt aggattatag taaaggtttt 110460
agaggaggtt atcgtttata gggttttggg ttttttttt gaaaggtagt ttagttttta 110520
agagggtaat agcgattgat tcgggacgaa ggttttttttg tgtttgtttt cgttatttat 110580
cgcgtgtttt tggtttcggt agttataggg ttattttagg cggattttaa agtggcgtta 110640
agttgttgga ttttttttt tttttattaa attagttatg ggaatgggag tattttttggt 110700
atattttag atgtgttgta ttggtttagc gtttttttta ttttcgggtt ttaaattagt 110760

tgttggttgt tttattagga gtagattgag gggaaatggg cggaagtcgt aggggaagag 110820
atgtgaggta gataaagagg aagaattttt tggttggggt ggggtcgtta gggtttgggt 110880
gggagggtcg tttatttttc gtttttggtg gaaggtagta gaagggtggg acggatttgt 110940
gtgggagggt ttaggtgtgg tttgtttggg ggtaggagag ggattagatg atttttttagg 111000
ttttttttggg ttaaagattt tttgataaat ggttgtcgtt gaaggtagga gcgaggtttt 111060
gggaggtgat tgatttcgaa aaattatagg ttgtagtttt gagggtttta aaggttaagt 111120
acggagtttt ttaaaatgag gttttttttta gttttaattt atttacgttt tttaggtggt 111180
tttagatatt tcgttagggt tttttttggta gtttttcgtt ttcgatttg gagatagaga 111240
aacgtgatgt tggtttttgg tttgtttata gttttttcgt ttttattata gggtcgttag 111300
ttatttttttt agtttatcgc gtgttttttt ttttttttta ttttcgattt tttaataata 111360
gtagattttt ttttaaatat tttttttgttt agatttcggg ttttacgtat gttgttttta 111420
gaaatgtatt ttagtttttt gcgaggagtt agtaaggttg ttcggtgttt tttttttatgg 111480
atgggaagtt ttgtcgggga ttaggattcg gatttggaga ttttgaggag cgtgttggtt 111540
agtgtttgtt ttgaggttcg ggatagagtt gttaggtata ggtagtatcg gtgtgagggt 111600
ttagtttttc ggataagttt tttttttgttt gtttttagat ttgttattgt atatatatggc 111660
ggtgattatt tttgatttgt ttgggtagag aagttatttt tttttttttt ttcgttttat 111720
tgtttattag gggaaatgtt ataagtattt aaaaaatgta aaaaaaaaaa aaaaaaaaaa 111780
aaagcgttta atattagggg ttatttggtt taggtggagt ttttattaat tacggggttt 111840
tttgtgttgt ggttttaagg cgggtagtcg atggtttttg ttcgagttaa tatgagttgt 111900
agtttaatta taggggacgg tgattttagg gagtagagtt taggtttcgg ttatagttgt 111960
atttaggagt tagttgtttt ttagatttgt agagttaggg gagaggtttg gaggatcggt 112020
attgaatgat atgatttaaa gcgcgttaag ttgggttgga ggtgacggta gagttatagt 112080
ggttatagag tttttacgtg aatttatacg gatttgtttg gtttaggttt tgttgaattt 112140
tagggtgagg aggatttggt ttttattttt aaagatatga agaggttgat aaagaagagt 112200
tataggtaaa agggggtggt gggtgtgttg tgtaggaggg gtcgtaattg gacgtagaag 112260
attttagaag gggtaattag agagaggtag tatttttaga agacgggttg tttggttgtt 112320
ttaaatagtt tagggtgtga ttgaggggat tttcggattt agtcggggtt tttttttgttt 112380
tgagataacg tattgggaaa ttttattaag tttatttaag aatttgaaaa gtttttttgga 112440
aatttagcgg gtagggcggg tttttgggag gatttttggt atatcgcgag agttagatat 112500
agcgaggggg ttttatattt ttgtttagaa gttttacggt agaggggtag aagtttaggg 112560
aggagtaggc gttgagatta gaggggttta taagagaggg gtttggggta gggggtagtt 112620
gttgagaagt ttaggaaacg gcgtttgttt ttggggaagt gaagttttgg gaatagggtt 112680
ggtgaagggg ggcgggaagg gggaggtggg ggtggagggt taggttagat taggtatttt 112740
ttttagtagt agatttatga gtttttggaa tttgtttata gaagtgataa gtgtttttgtt 112800
tttagtttcg tttatatatt tatttattaa gtgtggtagg gatagtcgtt ttttattatt 112860
tattttggga tttggattta agtggaggtt tttggagggg tagatttagg ggaaagggtt 112920
tttttagaag tagtcgttat ttagaggatt tgagatgaga attttattac gtttttatta 112980
gatagaggag attcgcggtg ttttcgagtt tggtttttta tttttttattt agtagcgttt 113040
ttttcgttta ttttaagttt agaatatttt gttttttttta agattagttg tcgtagtgga 113100
ttgaatagtg atttttaaaa gatatgttag agttttagtt ttcggatttg tgaatgtgat 113160
aggagttgga atagggtttt tgtagatgta ataaaggatt ttaagaggag agtattttgg 113220
atttagggtg ggttttaaat gtaatgataa gtatttttac gagatataga gatatagaga 113280
aaaggttatg tgaagacgga gtagggagtg gcgtgacgta tttatatgtt taggagtatt 113340
cgggagttat tagaagttgg gagagaggtt tgggtcggag tttttcgtag aggttttaaa 113400
ggattagttt ggtcgatatt ttgattttgg atttttggtt tttagaatta tttttagttt 113460
ttaaagttcg tggggttttg ttgtagtagt tttagtaaat tgttacgggg gttttttttt 113520
tttgttaagt ttggtattta aagtttttta gttatttggg gttaattata ttgattttgg 113580
gatttttagg ttattcgtgt gttttatttt tttgtttaat ttttagtgtt tgtgtggtta 113640
tagttttttt ttcgtgatat gagtttttttg ggtttttgag tgtttatttg ttttgtattg 113700
aaggttgaag ttaaattttg gagagaattg atttttttttg tgggtagggt aggtattgtg 113760
tttttagtat agttagaggg tattggttga aatgtgtttt tgtaaagtgt tttttttatg 113820
ttcgtatttt ttttttaagt ggggaagtta gagaggtttt ttcgattttt tttttagttt 113880
tttggggtag gaagtttgtg gttatttgtt cgtaaggatt aggtggatag ataattagtt 113940
tttttttaggt tatattttat taatagaggg ttttttaatt ttgttggtgg tttatgagag 114000
aaggaaaaat tgaaggagtt gtttatagta aattttaaat ttttgtgtta gagaggttta 114060
tggttgagta aggtattttt atttttattg ttttgagttg ttaatttagt ttagtttagt 114120
attgtgattt ttgatttgtt tgtgggttac gggagtttgg tttgttaatt agtatttttt 114180
agatatttat tttgtgaagt ttttagtggt tgagtgtttt ttcgtttttt tcggatagcg 114240

tgggttatag gggatattgg gtaggaagat agttaagttt ttattgttta gaaattgttt 114300
gggtttattg tagtaatagg gatttattta ttagtatgag gttttttttgt aggaggggtg 114360
ggggtaggag ggagtttgtg gggtataggg ttgtaggagg agttattttt tttttttatta 114420
aatcgtaggt tttagttaag ttatttttag tatggttgtt tgcgtgggaa gtttatagtt 114480
gagggattgt ttgtacgtgg tatgtaggag ttataagcgt tatttatatt ttatttcggg 114540
ttaggtagat gatttttttg gttttagttt tttgatgagg aataggaagg gttggatcga 114600
aagatttttt ttagttcgga tatggaatga ttttacgagg gttacggtga ggtttgtttt 114660
agtttcgtat attagttcgg gttagtagtt tttaaaggta tagtcgtagg ggttttggag 114720
tagggataag gaggattagt ttattttatt tttttgggta gaggttaagg attttgtttt 114780
tggtttatat tttttgtggt taaaaatgta gaagtaagta gtgttggtag aggtatttag 114840
atttttgggt aaagtagggg ttttatttga tattttaggt ttggagtacg agaaaattag 114900
tttaggtttt aggtttttttg attgttagcg attagggaat gtgatatttt tcggtttttg 114960
tttttttatt ttttaatttt aggggagat taggttgggt gatgtgtttt tttttttgat 115020
agtaaatttt tatttatttt tatttttatt tttgttttaa gagggggttt tttatggtcg 115080
ggcgtagtgg tttacgtttg taattttgga attttgggag gtcgagatgg atggattatt 115140
tgaggatagg agtttgagat tagtttggtt aatatggtga aattttgttt ttattaaaaa 115200
atatgtgagt tggtaatttt aagtttttag tagttttcga atggaggttt tttttttgta 115260
ggatttttaa atttttataaa tatgagtgtt tgtaatttag ttatttagga ggttgaggta 115320
tgagaattat ttgaatttgg gaggtagagg ttgtagtgaa ttaagaacgt gttatcgtta 115380
ttttagtttg ggatatagtg aaattttgtt aaaaaaaaaa aaaaaaaaag gtgggggttt 115440
ttttatgttg tttaggttgg ttttaaattt ttgggtttaa ggaatttttt tgttttagtt 115500
ttttagagtg ttgggattac gggtacgagt ttatgaattt taaaaaataa tatttattgt 115560
tttgtttttg attgtaaaag gaatttaggt ataaagtcga aaatttgtaa aatgtataaa 115620
aataaaggga aaaaatttat aaatttataa tttaggtaat ttttattaat attttgataa 115680
atgttttttt atttttttatt ggggagtgtg tgtgtgtgtg tgtgtgtgtg tgtgtgtgtg 115740
tgtgtgtgtg tttaaatagg tttatatagt gatgatgttg ggtgtttttt tttatgtggc 115800
gttatgtggt tgaggttttt atatattttg ggttcgtttt ttcgattttt ttgtagtttt 115860
gatgttttga ttttagatat gttatttttg gttcggattg tttaggagaa ttattttttgg 115920
attttgttaa aaaatggatt gttggttttt aatttttggt ggttttggtt taggagggtt 115980
gggtaagttt gggtatttcg gagtgttttt ggggcgttgt aggtgtgaat ttttgttttt 116040
ttgaagattt ataagtttta ttttttttat tttttttttt tttttcgaga attttataag 116100
aggaaagttt ttattcggag gttgttaggg attcggagtt gttagtttat agttttattt 116160
tgtttttatt ttttcgggag ttatttaagg ataatgttta gttttagttg ttaagtttta 116220
gttttagggt tagttatata ttgaaggata tgtagaagtc gtcgtgtgtt gggtattgtt 116280
gaggataaag gtttgaatga taagcgggaa taaagttata aggttttaaa atttgttcga 116340
ggttttatag ttagggtttc ggaaatttat gaaaaaagtt agtttaatat tttgttacgg 116400
aatataggta ggagagggag tagtttttag ttagttttta gttagttttt cgttagtttt 116460
tttgcgaatt attttatttc gggttatagt tagggtagtc gtcgtttttt tttttgttcg 116520
atagtttttg agggtaggag ttagtttgtt tattattgtt ttttttttat tagggtttgg 116580
tattttgttt ggtatgtagt agaagtttaa gaagggttta ttgtgtttat tttgtaatag 116640
ttaatgtgta tgagtgttta ttatatttga agatagtgat ttatttttgt ttttattgta 116700
tagatgggaa attgaggtat tgagaggttg atgatttttt tatattgtcg tgtgggtaag 116760
ggtaaggtta gatgtgagtc ggtacgttgg gttatcgcgt tttttgttta tatttcgttt 116820
tgggtttggg aaggttattt tcggagattt ttacgatgtt gatatttcgt ttagtttttt 116880
cgtgtatttt tttttacgtt ttttagttat ttaaagtatt agttaggtag tattgttatt 116940
ttggttttgt aaatttagtt attgagtgga agagtagtta ggttgaagtg gggtagaggt 117000
agttttcgtt agatttttagg gtcgggattt ttagttattt ttatttttta aggattagta 117060
gatagagatt tttatcgtgt ttagttaggg gttagggaat aggtttttat agtgggggag 117120
tgatggatgg atgtaggtgc ggattgggag gattcgggtc ggttttttag cgttggagtt 117180
ttagtagttt aatttatttt gttgggtttt cgtggggatt aaaagatatt ttgtatataa 117240
agtattagtt tagggtttgg tatttaggag gtgtttaatt aaaggaaatg gttatgaaaa 117300
ataataagga gaagtttagt tataggaggt gtttgtgggt aaggagagaa tagttggaag 117360
ggtttggatt tgggttagag ataaagaatg taagagggag ggagggagag aaggagggag 117420
ggagagaaag agggagggaa agaaagaggg agggagagag ggagggtagg gtaggtaata 117480
gttggtttta gggtagttgt gtgtaggagt ttgttttatt atttaaatat taggagagat 117540
gtaaatattt gagtgaggtt tttttagttt ggttttgttg tggttatagt tagggttgga 117600
gaaagagttt tttttttatt agggtttttcg ttttgtgttt tttattttag ttgttttaag 117660
attttatgtt ttcgggttag gagtaggggt ttgagagtag taattaggta gggtagcggg 117720

gttagggagg gatttcgttt ttgttttat ttttttttt cgtttttgtt tttattttt 117780
tttttttgta tagtattggg tttgtaaagg tgcgaatgaa ggattttttt ttttattcga 117840
ttttaagggg tttaggcggt taagggtttt ttttgtagtt tagaaaaaaa agtaaagtag 117900
tttttttga ggtttttgg tggaggttta gggtttaaag ttcgttgtta taaatttgta 117960
gtttttttg ttttttaat agtgtttttt ttttatttgg gttattttat tattttggtt 118020
tttttagtt ttttaggttt tttggggagg aggatagatg tagagtttgg atttcgtgcg 118080
tagtagggtt tttttaatgt tgcggtgtat ttcgtttggt tgcgggagtt tgttttatgt 118140
tatgatttgt gttggtatta atagtttttt tttttatttt ttatttgagt gtcgagggta 118200
gttgtgttag gtatagatat atagtttttt atatatatta tgagtttgta gaaatggtaa 118260
gagaaggtcg agcgtagtgg tttatatttg taattttagt attttgggaa gttaaggtgg 118320
gcggattatt tgaggtaagg agtttgagat tagtttggtt aataggatga aattttgttt 118380
ttattaaaaa tataaaaaat tagttgggcg tggtggtgcg tgtttggagt tttagttatt 118440
taggaggttg aggtaggaga atcgtttgaa tttaggaggc ggaggatgta gtgagtcgag 118500
attacgttat tgtattgtag tttgggtaat aagagcgaaa ttttgtttta aaaaaaaaga 118560
aagaaagaaa aagaaaaaaa gaaaaggaaa atggtaagag aagttggtta gtattatttg 118620
tttggtaaat ttttatttcg gtttttggtt aggaagtttt tttttaggaa gttttttcgt 118680
tttattttt tttttgagtt tatgtttaga tttttagtgt ttagaataga gttggttttt 118740
ggggtattta tggatggttg ttggataaat aaataagtta attgtttttt ttattttag 118800
cgatgttttt ggtttggtag ggtatttagt agtaatgttt ttcgagtgga ttttaaggag 118860
tatttttat cgtgtagttt gtattaggga ttttagtttt ttattttaaa ttaaaggaaa 118920
aaatttaatt ttagtaaata tatagagtat tttattttt gattttaat gggggtaag 118980
aggtagagga tagaggagaa gattattttt ttaggtttgg tttggagtta aaaagatttg 119040
tatgttttta ttgtagataa tatcggtttt tgggggatat agggtagggg tttggtttgt 119100
ttttcgtagg gaaattgttt atttacgtgg aaagcggggt taagggtttt tgtttggaat 119160
tagtttttttg tatatggggt gggggtggg gatagaagga gattttgttt ggattgtatg 119220
gaaatttatt agattgttgg ttgttgattt ggtaggagta gagttatttt tattttttag 119280
ggttttgttt gggggagggg agaggttagg tggttttgtt taggttatat tttttatggt 119340
taggaatgtt tttttttat tagaaagaaa gatgagatag gtttattttg aattttttgg 119400
atataggaag ggggtttatt tgtggtttag gtggtttttt tttttatatt taaggggttt 119460
tataaggagg tgttaggatt tttaggtttt gttttaggga attgttgttt gtaaaggtta 119520
taggtagaga tagttaggaa tgttttttat gttattggtt atgggttgga ggttgcgaaa 119580
ttaggtatag aattagaaag atttgttata ggttgtaaag ggttttagag agttttagtt 119640
ttttagaggg gtagtattgt agaaggggtg agaaggaaaa attagttttt aggaattgtt 119700
tatttagagt ttatataata tttaggtggt acgagataac ggtttatttt ttattttatt 119760
ttttcgttgt tttttaagat ttgagttttt gggggtgttt ttagtagata gggggatgtg 119820
gggtaattgt ttttttttaa ttgtatattt ggaggtaatt tatatttaga tgattttttc 119880
gaggttgtga ttagagtttg gggtagggga ggggaagcgg gtggatggga aggcgaggga 119940
gatttttta ggaaagtcgt tttaggtgtt tgtaaggttt tatcgtttat atttaaggta 120000
taggttttg gttttgagga tataggaaat tcgatgttat gtggggagga gggtattgta 120060
atgtatttaa ggttaggttt tttgattttt tggatattgg ataaatgatg gtttttttag 120120
ttggtttaga gatgttagga gtgatagaat gttaaaggtt gtagggggat atttagaaat 120180
tttcgagaaa gaagatggat aaggaaaaat aaaaaaatac gagaagttta gtagagtttg 120240
agtttcgtat tttcggtttt ttttatggtt acgggtaatt ttttggagat ttagttacgg 120300
gagtagtgga ggtaggtatg ggtggggggg ggggcgggtt ttattgttta tggtttcggt 120360
tgatatcgag gttattttt tgttttttta agtttttggg tattggtagg attttaaaa 120420
acgataataa atttgaagtt tttagaattg tttgttggtt tagtttaaac ggagtttcgg 120480
ggttttgtag attaaattaa atagtttgag tttagaaatg ttttaggcgt ttgggggtgt 120540
ttaggggtta attggtgggt ttgtttggtt ggagagatga taaatttttt tatttttggg 120600
attgtgtttt agagagtttc ggattttttg gttttataag taatttagag tttttaaatt 120660
tgggaaaaat taagagggtt ttggaaagaa aggtagtgtt tgattatttt tttttagtgg 120720
ttttgttaag ggagagtata ggaatttggt ttttggttgg gtttttggga tgaattaaaa 120780
taaaaagaaa aagtcggggg agggggtggc ggaatcgttc ggggttttag tttaggttat 120840
atattaagga gattttagag gagtaggtta gttttatttt cggtacgatg atggacgggt 120900
atttggttgg ttggtaaagg gaagagatgg taatagtgag gttttgaatt tagtggttaa 120960
gatttatttt tgagtgtgat tttttttgat gtttgggtat gtaagaaatg ggaggtggag 121020
aaaggaggag gagattttag ttttgttttt tcggtattag gttattaggg aatagtattt 121080
ttttttaac gggtaggtag aggttatagg ttgttaggtt gagaagtttt tggtttttat 121140
agataaatgt atagtttgta tatttgtatt attttgggtg atgtttatta cgtacggtta 121200

```
ttatgtacgt tttttattat taagatagtc gacgagatta ggtggttatt gtgtatcggg 121260
tatcgtggtg attgtttgat attgattttt gggggtttag gttttcggtg ggtttgtttt 121320
tttaggtatt tagttttttt tttatttttt gatatttcgg gtttagtgta ggggtatggg 121380
ggtgtttagt ggatgggttt acgggttggg taagtagtac gtagttttgg tttattagtt 121440
tttggtttta ttattagata agatgagatt gttttagttg ttagtttagt tttgtattat 121500
atttgattgg gtttttgtag aggtttgttg gatgagaatg tttagggtag taggtattat 121560
tttttgggat gtatattttg ttataatcgt tttttaggga aaggttagtt tgtagagtcg 121620
aacgtaggtt gttagcgttt tcgttgtttt tatggttcgg ttatttagta agtgtttatt 121680
ttattgttag cgtttggttt tgtagatggg tatatggttt atttatttgt gatattttag 121740
ggtgggggtg ggggtagatg gtggtaggga agattaggat aattttttag taagaggaat 121800
ttgtagttaa tatgacgtta gtttttagaa tgttagttag gtaggaaaag gggggtttta 121860
tggttgtatt gggagtttga gtatttgtcg ggtttagagg gatttttagg cgggcgttat 121920
tggtattagt gtttgtcgtg tttttttataa ttagtggtga ttatgttgtt cggttttatt 121980
tatatagtgg tattatatag gtttttgttt gagatttttt atttttagat ttgtatattt 122040
aagtatttaa atttatttgt tttttgtaaa tgtttttttt tttcgtttag atgttatatt 122100
agtcgcgata tttattatat tcgtaggagg taggattcgt gtttagcgtt atgggttgta 122160
ttttagagtt ttttagtaat ttttaaagag agttgttttt aatttattt tgtaggtagg 122220
gaaataggcg aagaaattga agaaatcgaa gtcgtttagt tttttaatgg tagagtgagg 122280
gcggtttggg tatttttata ttgttatatt ttttattttt ttaaagtatt gtttttttgg 122340
atgtaaattt tgtttattag ttttatttaa aatttttttg gatagttata gtttatagaa 122400
agtattttta gtttgtagtt atttagttat tgtaggggtg ttttggcgtt tattttttatt 122460
tgtttttttt cggatattta gtttagagaa tttatagttt cgttgtaagg ttgattatag 122520
tgtatttgtt gtttgggtgt tgtgttcgtt tttttttaaa tgaattaatt cgtagtcggg 122580
atagtgggtt tgtttggttt tgggttttga atttaaagag tattaagtta gtgttgttag 122640
tggttgtggg attcgtgtaa gaggtttgtt tttatttttt atggtttttg tttttttatta 122700
taaaagttta aattgtagag aggtagtatt tataggatgt tttgagtttt ggttgaaagg 122760
tagggtttat tacgtaagag gtttattggt attaattagg gataggtttg tatttattat 122820
taagtaacgg ggtttttttt tttagagagg aaagaggtat ttgaattttg ttagcgcgtt 122880
agtttttacg tttcggaggt gttgggattt ttagataaga aggtatattt atatggtttt 122940
ttttatttag aagttcgttt tatagtttgt tttgttttta gaatatagat ttgggggttt 123000
ttttcgggcg atagtggggg ttcgggagaa gtttagatgt tatatagttt tgggatttga 123060
gtacgtgggt tttgggttag attgttttaa taaatattaa ggatttttgg atttttttttc 123120
ggtgtgtaaa tttggttata gtaaatatcg ttgagtaaag taggttaata gttggtattt 123180
ggaaaggttt ttgttatata aagattcgag ttgtaatcgt gtttgcgata atagatttgg 123240
tttgaattta cgaaagtata tgtcgttcgt tagtttcgtg tttttattaa ttagaaggga 123300
gaaagaggaa agggttattt tttttgttat tttattacgg gtagtggcgg gtgttgtagt 123360
cgtttggagg ttcgtttgta tagtggaaaa gtagaaaatg agattttaaa aattttatta 123420
gttaaatata aaatattttt tttgttgtta cgtggcggtt tttatagagt tttaaaattt 123480
tgaagttcgg agatttattt tgaaggggga gtaaaggcgg gatgcgggaa gtttgagttt 123540
atggaagata ttgatatttt ttttattttt gggaattttg agtttgaagg attcggtttg 123600
aatagcggaa ggattgaatt tttgtgtaat ttatcggtga tggaatataa agttatggtg 123660
ttttcggttt ttaagacggg agggatgggg aaggttttgg gtgaagggga taggcgggga 123720
aggaaaaggt gaagagaaag gtgtttttta gggagggggt agatgtgttt aggatatggg 123780
atattggtag aattttaatt tcgagtatat atataggttt acggacggtt tttcgtttat 123840
ttgtttttta cgggtgtgtt gtgtcggttt gggtatttgt ttttttttt atgttaggtt 123900
atttgtcgtt tattttgtat attgttatta gttttaagtt ttttagaata ttttgattag 123960
ggttttgtgg tttttgttat aggattttag gtttagacgt ttgttcgatt ggaaaattgt 124020
ttttagagat gtcgtggttt agagttcgtt ttgttttggg gtggtttttg gagttttcgt 124080
tttttagtaa tttatatggt gataggtatt tatattcgtt ggttttagga attttggggt 124140
tattatttta gcgttgtaag gagttggtag ttttttttt atttttcgg tttattttgt 124200
aggaattaag atattaggtg agtcgtttcg ggaaagagat ttgtgttttt acggcgttgt 124260
ttttgcgttt atttttagtt attttagatg tttttcgtgg attagttgta ttttcggcgg 124320
gtatgtttgg tttttcgtag tttggtttat atgattttta taggtaggtt ttttttttatg 124380
ttttagtttt tttttagtt agtttatttt agttttttt attgttgttg gttgttgttg 124440
gttagaaggt ttttttttt tttgtaattt aatttttatt tttttttaag gttcgttttt 124500
tttatattga ttttttttt gttagttagt ggtgattttt ttttatttg attcggaagt 124560
tgtttgttta tattattttt ggttgggttg aattatattt ttttttttat ttttttttaat 124620
tatttttttt ttacgtttgt ttgtttattt aaggttgttt aatttttata tatatatttt 124680
```

tatatttttt aattatattt taggtttttt gggtattagg gagggttatt tttttatatt 124740
ttaggttagt ttttatgtat atgcgtattt atgtatatat atattttttt acgtttatcg 124800
attcgggtta tttgatggtt ttaagattag aatttttagt aaaattattt aggatatata 124860
agggggagat tgataaatat agggaattag tagattgagg ttttattttt cgagattgag 124920
aaattttttt ttagagttaa aggattgtta attgttgtat taggatggtt ttaatgagtt 124980
tattgttttg gattaaatta tagggttata aaattggttt gggagatagt cgtagaagta 125040
gttttagatg gaggtatatg ggggtaaggg tggagttata tttagagatt taaggaaggg 125100
gtattaataa tttttatagt gtaaatggtg atagttgtat aacgaatata attttaaatt 125160
ttggtttttt ttttcgttat agggttttcg ttgagatacg agagtttttt tttagaaatt 125220
ttattggggg ttcgatattt tttgttagga attgagagta gagatttcga tttcgtaggt 125280
aggaggagag ttttgtataa tatagggttt tttttttataa gggtttattg gaaagtggtt 125340
agatcggtta agtcgtgtta tttggatatt tgggataagg aaggtttttg gaggagcggt 125400
cgtagggagg agttgtaaag aggtaggggt agggaggtta tatttattcg gggattgtag 125460
gaggtttaac ggaatatagt ggatttaggg agattttagt atgagtatgt gtttaaagat 125520
gtttagatga ggaggtgttg tgggggaggg gggcgggaat agtaagtttt tttagttttg 125580
tagatttaga tttttttttt ttttgggaag tagggatgta aagggataga gtttgaaaaa 125640
gggtttttat tattaagttg ataaagtaat tacgatgttt ttagtttata ttttgtatag 125700
cgttagtggt tattggatta taattttttt tcgtttttttc gtcgttttat attttttttta 125760
ggtttagttt tgaagaatag ttattggtat tttgtttggc gtttagttaa agttttggtt 125820
ttttaggagg tttcgttttt tttatttaga tagggttgat tttaggtggg tcgtgtgggt 125880
agttgggttt aggatattta gatgttagtt ttggttttgg ttatggagcg gggaggattt 125940
taggttggtt attttttagt ttggtttgat atttggatag tttgtttatt ttaatatagt 126000
tttttttttt ttcggtttat tagagggttt tgtttaagtt agtttaggag tttttttcgag 126060
gggcgggggt tttaggtttc ggtagaggta ggttcgtttt ttagaagttt tgttttagag 126120
cgttggagat tattgtgtga gttttttttt cgtttgttgt atttattttc gcggacgtag 126180
gacgtagagt tttagtatcg gtagttaaga ggacgtagga aagcgtacgt atttataggt 126240
ttagagacgc ggacgttaga tttaggtttg gatagagtag tttcgtaagt tttaatatat 126300
tttgtaaatt tcgaagatag gagagagtta aaatattttt tttgttcgta cgtaggtttt 126360
ttttgttttt tacggttcga ttttgggagg cggatgagga tatttttagg tttggatggg 126420
ggttatggga tattttattt tagattttgt tacgatttgg gttgtgttcg ttcggtgttt 126480
tcggtttacg ggttgtataa tttggcggtt tcgaaattgg cgtgggggag gggagggttg 126540
tttattcgag taggacgcgg ttgtttattt agtcggaggt gaggaacgat tttttttattt 126600
cgttgtcggg tttaagcggg gttcgagagt cgtcggggag agttaaaggg aggggatcga 126660
tggatttttt agagtgaaat tgtgcgtttt ggagagtttc gaggtagttt cgcgagtttt 126720
cgaggaggtc gttgtcgttt ggtaaatata aataataata aaaggaagga aatttaggcg 126780
gtagtgcgta gaacgagtat agggttttgt gagggcgcgt agggtttacg cgggtgtttt 126840
taatagggtt ttttacgtag gcggcgcggg tacggggtcg ggggcgcgcg gcgtttttag 126900
cgggagggcg tttggattag gggtttcgtt tttttttagc gtattttcgt tttttttttag 126960
cgtattttcg tttcgcgtta ggtttattcg tagggttttt tttagtacgt tcgcggtcgt 127020
agtagttagt ttagtattta ttttcgaagt tcgaaatgat tttatttagt tgcgcgttga 127080
tcgcggggtt cgatatgatg gttggtgggt agcgggtcgc gcggagggta gcggcgagga 127140
agcgttttcg gcggggttcg ggtttgcgc gttggttggg gcgtcgcgtt cgcgtttttg 127200
tagtgtagag ttagtcgtcg gaggagtttt taggtttttt ggtttagttg aatgaatggg 127260
ggaggaaggc gggcgtcggt ttttttcgc gcgttgcgtt ttcgtttcgt tttcgttttt 127320
cgggcggatt aggttttcgt attcgtatcg gttttttttgt ttcgtattgg ttgtttcgtt 127380
cgtttgttaa ggttaggttt ggggggttgg ggttcgagtg gtcgttgggg gatagttttg 127440
ggtatacgat cggagcgttt tttttttttt cgtttggttc gcgtttaggg gacggcgaga 127500
gacgcggttt tagttttttt tttcgggcgt ttcgttgcgg ttagatgtgg gcggatgtgg 127560
aggtcgggtt atggcgagga gggcgggcgt ttagagggga agagattttt tttttttttc 127620
gggcgtaggg ttttttttgta ggaggttgta tttgggtagt taaataaagt tttttgttat 127680
cgtcgtcgcg cgttttgcgt ttgtaagggt taaacggtag cgtacgcggt tggtaggtag 127740
gatttcggtt tgtgggttta agagtcgagg ggcgtagggc gaatatgcga gttttatggt 127800
attagcgaga ggttattaat ttttatttat cgagggtatt tttgtagtta atatttttat 127860
tacgattatc gtttcggggg ttatcgttgt attttttttgg gtgagttttg gagaaggtat 127920
tgttcgggag aaggggtata gggcggggat ggaatacggt tgttagagaa gttagggacg 127980
ggcgacgttt tttttttttt ataataaggg ttttttgaga gtaggaagta gattttgtta 128040
ggagtagacg ttttcgaagg aaggtgtgaa ggaaggagtt aggtagtgag tgagtagaaa 128100
gggagttgtt ttaagggttt tttttttttt tttgggtttt aatttgaggt ttagtttttt 128160

ttttaggtta gcggttttta tttttaaatt atattattta taaggtaggt agggtcgagg 128220
ggttggggta gatttgggga tttgggttag atatagaatt tgagaagtaa taggttttta 128280
tttttaagaa gggtttttta ggtttgttat ttataagaga gagaggcgtt ttttttattc 128340
gttttggaaa gttagaaatt tatttttgta tgtttttttt gttatttcgt tttttagatt 128400
ttcggttttt gtttagagtt aggggttatg tgagaattta tagtatggaa taatattttt 128460
atttcgaag tagtaggtcg taacgttata attgtatttc ggtttttga atgtgtggtc 128520
ggggagcgtt tgatagttta ttttgggttt gaggtcgttc gttgtttatt tgtttttttt 128580
tatttttttt gaaatatttt aaatgtgtag gagtgttgag atttttagt tattttggag 128640
aggcgggggg taggggttt tttgcggaaa atgttttagg tttgggtggt tttttaagta 128700
gatgaaatta agtttttttt ttttaagaaa agtaggtatt tttttttta tgttttggat 128760
attttgaatt atttttaatt aagtgagttt gtggatatgg gaattgtaaa attatagata 128820
ttttagatgc gtgtatattt tcgtcggttt gggagttcgg gtttagaaa tgtagttatt 128880
gttgttatta atagtatttc gtattttta cgttaggtta gggtatgttt tgtttgaatt 128940
ttagtatttg ggtttatcgg atagtttagt ttttattggg ttatttgttt acgtcgtatt 129000
tggggatttg ggggtttagt atttaagaaa tttagtttaa taatatagtt tttttgggaa 129060
gatgtcgggt tcggtataat agttatttta agttatagta ttatttcga gtgtttttt 129120
aaaaagtatt tggtgagtgt ggatatattt atgtaattgt ttttttttag ggttggtatg 129180
gagttttatt agattagtat agggttagtt aattgtatat tttttattt tacgtatagt 129240
gtagtaaata tatttagttt tttggggagc gtcgttaggg tgtacgtttt attaaaatgg 129300
gaatttaagg gataatggta ggagagtagt tagaaagtgc gtatgttttc gtgattgtgt 129360
ttagagttta tcggttttag ttttagttat tgtttttta atgtaattat ttagagttat 129420
atgattaggt ttagaaattt ttaggtaagg aggtttttcg agtttttta ggaaatttaa 129480
attagggttt ggttatattt ttagttagga agtttatttt cgtatttaat taattgtttt 129540
aattgtatat tatttatttt tttttttttt ttgtttttag aggagatggg aaatagttgg 129600
gtattatttt ttaagttata attatttttt ttatatttaa ggataattta gttgtttttt 129660
ggtttttttt tttttagttt aaattatttt tttttagttt tttaattata tttcgagttg 129720
ttttttgatt atagttaagg gttgggttta gtttggggtt ttaggatttt aatgaggttt 129780
tattgttata ggatagtgag gaggtttttt attttttttt tttagggttt tagggtttgg 129840
atatatagtt ttagaagggg tattgggtag agaagatgta cgtcggtagt agagaggaaa 129900
ttgagttgaa atttggtaag aataaaagaa ttataaaggt ttgtgtattt agtaaggtgt 129960
atttttttac ggttataaaa gaaggtaaga tcgattgtag gtttggttaa agggatcgtt 130020
tgcgttttta gtgtataacg agatttatag tgggtcggtg atttttttta aaataaagat 130080
atgtttattt ggaattttaa atgtgatttg attttgttta agggttttg tagaaagaat 130140
ttaggtaagg attttaagat aaggtgggtt ttaaatttaa tgttgtgttt ttttaagaga 130200
tagaaaagaa agtatagaga tatagaaaaa ggttttgtga agatagaggt agagatcgga 130260
gtgatgtagt tataggttaa ggaatatttg gggttattag aagttggaag aggtaaagaa 130320
aggttttttt ttggagtttt tagaggaagt tggttttgtt ggtattttga ttttagattt 130380
ttggttttta gaattgtgag aggatatatt ttttttgtta taagttattt agtttcgata 130440
gttgttatag tagttaggag aaattcgtgt tttattttta tttgtattat gttttgaggt 130500
taaagtttta tatagtaaag atgtttaagt aattataaat tttgtgggta gtttattcgt 130560
ttagtagatt attgttttaa gggaaggggg atggggatag ggaagagagt tataggaaat 130620
ttttgtacgt ttttttggaa atgggaagga gtaggtttgg agagaaattt tataggttag 130680
gtttcgggga aatgaagttt ataaagcgtg ggaacggagt ggggaggaaa tttacgagga 130740
agtagcgttt ggcgtatata tttatattta tgattttttt ttaggtatag ttttatgga 130800
gaggtttagt tggtagggaa gtaaaagagg ttcggagagc gaaggaaggt gaggggtttt 130860
tttttagtaa tattgaggtt ttagacggga tattttatta ggtggtaggt tgtgttttta 130920
tagatagaga tttttaagg gtagggttta gatggtttta tttttgcgtg gtttgatata 130980
aagtaggttt tttagagttg tcgagttttg ggttcgttag tggtagagtt ttttatagtt 131040
ttttaagttt ttatagtttt ttattttagg aaacggtgtt attatttatt tagttgttta 131100
agttaagttt tcgggtatta ttgttgattt tttttttttt tttattttta gtatttaaat 131160
tatggtgcgt ttttattttt agttttttg cgaattagtt tgaattttt tttttagta 131220
gtgtgttagt taacggttaa taatcggtta tttggggtta gtaaaagtaa taatatttag 131280
tttttcgatt tgtagcgttt gtttattttt gtggtgttaa tatttttgtt ttggttaatt 131340
tttttttttt tttttttttt ttgatagggt tttattttgt tattcggttt ggagtgtagt 131400
ggggtaattt tggtttattg taaattttat tttttaggtt taagtgattt ttttgtttta 131460
gttttttaag tagttcggat tataggtatt cgttattatt tttggttaat ttttgtattt 131520
ttattagaga tgaggattta ttttgttggt taggttggtt ttgaatttag atgatttgtt 131580
tgttttggtt ttttaaagtg ttgggattat aggtatgagt tatcgtgttt agtcggttaa 131640

ttttaagtta tttgtatgat attattggag gtgagattga gaagagatgt ttagttatta 131700
cgaatatagt gtttttatta tataaatata attttaagaa tataatcgta ataggataga 131760
taatagttaa atgttgtaaa aaatttaaga ggtgatgaaa gttgtgtatt tattattttt 131820
tgttttaaat ataattattt ataagtaatt ttttaatgtt ttgtttaatt ttttttttat 131880
aatttgtaat ttaatttata ttgtaaaaat ttattatttt tttaatagag taatgtattt 131940
ttattgtgat aaaatatatg taatataaaa tttattattt ttggttagtt attatggttt 132000
acgtttgtaa ttttagtatt ttggaaggtt aaggtgggtg gattatttga ggttaggagt 132060
ttaagattag tttggttaat atggtgaaat tttgttttta ttaaaaatat aaaaattagt 132120
cgggcgtggt ggtaaacgtt tataatttta gtttttttggg aggttgaggt acgagaatcg 132180
tttgaatttg ggaggcggag gttatagtga gttaagatta cgttagtgta ttttagtttg 132240
ggcgatagag cgagatttcg ttttaaaaaa aaaaaaaata aaaataaaaa agataaattt 132300
attattttaa ttaagttttt atgttagtga tattaagtat atttatattg tatatttatt 132360
aatattattt attttcgaaa ttttttttaa ttttttaaat tgaaatttg ttttttattaa 132420
ataataattt tttattttt tttttttag ttgttggtaa tcgttatttt aatttttttta 132480
tgaatttgat tttatataag tgaaattata taatatttgt ttttgtgaga taggtttacg 132540
tagtatgagg tttttaaggt ttattattta aggtttatat gtagtgatag aatttcgttt 132600
tttttgaagg ttgaatgata ttttattgtg tgtatatata ttttgtatat ttattttttt 132660
tatatttgag taattgagag taaagttgtt ataaatatgg gtgtgtaaat atttgtttaa 132720
gtttttgttt ttattatttt attattatta ttttatttat tattagtatt attattattt 132780
tagatagttt tattttgtcg ttcgggttgt agtgtagtgg cgtgatttcg gtttattgta 132840
attttcgttt tttagatcgg agtaattttt ttgttttagt ttttcgagta gttgggatta 132900
taggtattcg ttattatgtt cggttaattt ttgtttttttt agtagagatg gggttttatc 132960
gtgttggtta gggtagtttc gaatttttgg ttttaagtga tttatttttt ttagtttttt 133020
aaagtgttgg gattatagac gtgagttatt gcgtttagtt attattttag ttttaaatat 133080
tatagtaatt tttttattgt tttatgagat tttttgagat agtggtttta gggattttat 133140
atatatttgt tcgattttgt gtaatgataa agcgtagttt tttttgtaga atggtagagt 133200
cgggagattt ttgatggggt atgagtttaa aggggtagag gggacgttta gaatatatat 133260
ggattttttta tgtgcgtttt gttagttatt aagtagattt gttttagttt cgatatgttt 133320
aggattatag atagtgtatg gtttttattt gggttaagtt ttcggaattt tttttttttt 133380
tatattattt gtattgggag tagttattag gttgtggatt attaagtttt ttttaggtta 133440
tttgagtttg tatttttttta tttagtttga ttgagtcggt gtgtagttat tatattttat 133500
tatattttttt gtttggtcgt tttaatgtaa ttataaggtt ttatttaaag aagtatatga 133560
ttaatttgtt ttttagattt tttagtgatt tttttagttt gttttgtgta gaatatttat 133620
agtaaaattt gattatagcg gtttgagttt ttattttttaa ggtaggggat tttttaaata 133680
aagtattagt gttttaaatt tatgattttt taaggaggtt tttggttttt agtatagtaa 133740
atgttttgtt ttatggtttt tcggtttttt atttttattt tattcgagtt tttcgtttcg 133800
gtatttggga ttgaattagt ttggagtttt ttagttttgt tgtttgttgg ttttgatgat 133860
attattaggg attgtagtat tgtttggatt ttttgttgtt atattttatt gtaataatat 133920
ttaatttgat ggaattttgg gagtaagtgg taggggtagg gatggtggtg gttaggggtt 133980
gtatatatat aggatagaag gattgaagga ttgggtggaa agtatgattt ttttaagatg 134040
atttttttttt agtagaattt tggttgtgtt ttttgaatag tatttgttta tttgtatatt 134100
tggtgagttt tgtgattagt aaatttagat gtttgggagg ttattagcgg ttttgttgag 134160
gtataattta gatatgatag tttatgtttt ttttttgatt tttttttttt tatatagttt 134220
tatgattaga gcgttttttt tatttttgtt tttattttag tttcgtaaga tatagtcgtg 134280
aaatggttaa agtttttaat tttttagtat tcgtttgaga ttggagtata aaaatgagat 134340
ggggatatag tattttatgg atattagtga tggaaaaaaa ttttttgatt ttttcgggag 134400
ttttgtaaag ttatattagg ggataagaag gagtggttag tttggtatcg atttagtttt 134460
aaagtagtta gtgattaaag tatatagttg gtattttttt attttttaggg tattaggagg 134520
tttggttaga atttatgtta gttaggagaa ataggttgtt tttggggttt ttggtgtttt 134580
ggttttttta ttgttttttt gaagaaggta ttttgttttg tggggtttga ggttggaggt 134640
aatttgttat cgatgtagtg tagggatgag ggttgagtta ttagtttgtt ttagttttac 134700
gttagtagga ataataggga tttaagtaga gaagaaagta tttattttta aagttattat 134760
agtgattgag gcgacgtggt ttttagggag tttttttttt tgttggtttt ttgtggatcg 134820
cggttttatg ttttaataat gtttttgttt atttcgtaag gtttagtagg tggaaggaaa 134880
gagggtggag gttgggagtg gcggtttggt aattcgggaa gggtagggag taggaatatg 134940
tttttttttt tttgtattcg tttagaaggt tttatagggt ttgttttgtt atttataatt 135000
ttagggagat tttttttttaa tgtttgtttt ataaatgttt atttatttta tatttgaata 135060
tttttagtta tagggagttt agtatttatt tatgttattt attttatttt tggttgttta 135120

aaaaagagga aagagagaga aagaaaagat aggaaagata gagaaagggg gaaagagaga 135180
gagaggaaag aaagaaagag gaaagaataa gataggaagg aaagaaaaag gaagggaacg 135240
gaagggaagg aagagaggga agaaagagag aaagagagag aaagaggtat aaataaggta 135300
ggttttatgg agtagagatt taataaggga gtaaaatata tttgtagtta ggttgtagat 135360
ggatttttat aggagcgtat atttatttta ttttttcgg aatagaaaag tggaaagaag 135420
atgtagcggt gtgggtgggt ggcggttttt tttggggtt tggaatgtgg ggtattagtt 135480
tttttggttt tgagaaggag attaagatta ttaatattta tagtggagat cgaattatat 135540
aagggttggg gatatcgttt tttttgtatt tttaataaag attcgatgtt ttaattatgg 135600
gaatttgtgg aagaagaaat aaaattggtt ttggggtttt ttagagaatt ggttttttat 135660
ttagtggtgg ttgtgaagaa atttttggag ttagttttag cggtttgggt ttgtatagtt 135720
gagattgttt ttggaagttg agtggaagtg gggagttttt aggtatagtt gggtttttgtt 135780
ttttatttta gttagtagtt ttaggatttt tgggtgtttt agagtttttt ttagagggta 135840
aatttagagt atgagggagc ggggagatgt ttaatattgg gggattttg ttcgtttttg 135900
tttttagttt cgttgatttg aatagggagg ttatgggtgt ttaggggtag aattgtgggt 135960
tggtaagttt ttagttttgg tttttttttg ttttttttt tttattgtta ttgattattt 136020
ttggttatat gttgatattt ttataggatg atggagaaga gagaagagga attttttcgt 136080
tttgtttagt atattttaaa gaggttttag tgtaaaggta tcgacgttta tagttaaatg 136140
tgtgtggggg ttttttagga gtgggacgtt tttatttcgt tttagattgt tatgaatcga 136200
tagaaggttt tatgttatta aatatatttt attaataaat tattttaata tttagtaaaa 136260
aaataaataa aaatgtatgt atttcgtgtt ttgtagtgtt tttatgtcgt attttttttt 136320
gtatttgagt ttcggttcgc gtttttggtt tgtcgttttg ggaatagaac gttttgcgtg 136380
ggaggtagat aggaagcgcg gggtgcgttg gttgggtttt tttagataat ttggtattgt 136440
ttttttttaga taatgaaatt tttttttattt tgagtgtttt tggaagttag tcggtagtta 136500
gattaattta aatgaattat ttgttttttt tgtgaagtga ttttagggat ttaatgggtt 136560
tgttattaat atagtttttt tcgtatttttg tttttttttt ttattttttt ttttttgtat 136620
gagttattgg ttacgatgat tcggtttagt taagggattt ggttagtttt ggttaggtta 136680
tgtgagagat agtataaagg attaaggttg tcggaattag gttagaggtt gagggttata 136740
cggatttttt ggagtgtttg taattgggga tttttaaggt tgtttttttgg ttttggggat 136800
ttgtttattt ggggatagtt ttaagggaga tttttaaaag tagggagtta aaagggtgaa 136860
ttaatataga aatattgagg gttagggaga atttgaagat gggttcggtt gggtatagtg 136920
gtttatgttt gtaattttag tattttggga ggttaaggtg ggcggattat ttgaggttag 136980
gaattcgaga ttagtttggt taatatggtg aaatattatt tttattaaaa atataaaaat 137040
tagtcgggtg tggtggtagg tgtttgtaat tttagttatt taggaggttg aggtaggagg 137100
attgtttgaa cgtaggaggt agaggtggta gtgagttaag attatattat tgtattttag 137160
tttgggtaat agagtgagat tttattttaa aaaaaaaaaa aaaaaaaaaa aaaagatagg 137220
tttaaggttt ggagagttag aagttatttt tttattttg ggtggtttat tttgagattg 137280
tattacgtta gagaaagttg cgggagtgtt tgtttataat ttataagtaa tattgttttt 137340
tttttttatt agtttttggt tttagtaggt ttttgattcg tgtttttttt agggttttgg 137400
gtttttttaa gttggatttt cggttgttgg tgggatgatt atgtgatttg ttattttttt 137460
gtttatcgtt ttgggtagtt tttgttattt ggtgtatata gagtggagtt tagtaaggtc 137520
ggggtcgtcg atttttttgtt agttatattt gttttttttt acgttatttt ttttcgtttt 137580
ggttatgttt aggggtttat atttttttat aagttaggtg ttagattttt gtgtttgttt 137640
tattttgttt tttttgtttg gtgggttttt tatattttttt ttggtttgat ttaggtttga 137700
tttgtttta ggttttagtt tagaggttgt ttttttttagg aagttttttt gggatcgtta 137760
gatgaaggga tttttttttg gatttttttt ttatttttat agttttagga atagttttta 137820
atttaggttt tattgagtta gtgtggtatt taggagagtt ataagcgtgt gacgaacgtg 137880
ttgaagtttt tttaaaagtt gaaggagttt tgtgaatata tagtagtatt agtttagatt 137940
gcgaaggtat aggggagtta gttttttta gtttcgtttg tttttggttt tttttttgag 138000
gaggtgggag ttagttagtt gggattagga ggtgggtttt atggagtaga gatttaataa 138060
gggaataaaa taaattagag aattttttttg ttgtaaggaa tttagataag gagtatttag 138120
ttagaggagg aaggaagaga tcggtagtag atttttttttg ttttagtagt tttgtaagtt 138180
agatgggtta atttttttagt atagttgttt tttagtatag agaaattttt tatttttagt 138240
aatagggtgg gaatattttt agatagggtt acggttattt tggttatagg gattacggag 138300
tttgagtttt tgtggtttgt tttatttatg tggttataga ttttgagtat atatttggag 138360
ttatagagtt taaagggtag ggtagggtag ggaatttgtt tgattggtga aggttatttt 138420
attatttgga gtataggttt ttgtttttaa atttatatgg agttttcgat acggggtatc 138480
gattttaagg aggtgaatgt ggatatttaa ggttagtgaa tggttaggtg ttaatatatc 138540
gataggtttt tggttttttt tttaatattg agggtaggaa ataaatgggg tttatatggt 138600

gtatatggtt tttagagtta gtaaggtgtg taagattcgg tagttgagaa aattgattat 138660
ttggtcgggc gcggtggttt acgtttgtaa ttttagtatt ttgggaggtc gaggcgggcg 138720
gattacgagg ttaagagatc gagattattt tggttaatac ggtgaaattt tgtttttatt 138780
aaaaatataa aaaattagtc ggacgcggtg gtaggcgttt gtagttttag ttatttcgga 138840
ggttgaggcg ggagaatggc gtgaatttgg gaggcggagt ttgtagtgag tcgagatagc 138900
gttattgtat tttagtttgg gcgaaagagc gagattttat tttaaaaaaa aaaagaaaag 138960
aaaagaaaag aaaattgatt attcgattat gaataagtta gaatatttat ttagaaaaaa 139020
aaaatagttt tgtttaattg gggatttaaa attttaggat ttatgttttt tttttataga 139080
gttatattat ttttttaatg tttattttcg attttatttg tttgttaagt tttaggtatt 139140
tagaatatag tatgatagta agttttgtga tttttatagg ttttttttaat ttattttagg 139200
tatagttata tttttagttt tgagtagtac ggagtgatat gtagatggtt atttagatag 139260
agaaagattt ttaaaaggag ggtaggtttt gtttcgtagg tagtatttat tgatatagat 139320
attagtaggt aaattttttta ggaagatttt tattaagaaa gaacgaaaga ggtttagtgc 139380
ggtggtttac gtttgtaatt ttagtatttt gggaggttaa ggcgggtgga ttattttagg 139440
taaggagttc gatattagtt tggttaatgt ggtgaaattt tatttttatt aaaaatataa 139500
aatttagttg gacgtggtgg tgggtatttg taattttagt tatttgggag gttgaggtag 139560
gagaattatt tgattttagg aggtagaggt tgtagtgagt taggattata ttattgtatt 139620
ttagtttggg gggtaataga gagagatttc gttttaaaaa aaaaaaaaaa aaaaaaaagg 139680
atgaaagata aataaggttg aaattggtag gttggtatat ttttaataat ttattttagg 139740
tcgggcgtag tggtataatg ttagtatttt gggaggtcga ggagggtaga ttgcgaggtt 139800
aggagatcga gattattttg gttaatacgg tgaaatttcg tttttattaa aaatataaaa 139860
aattagtcgg gtgtggtggc gggcgtttgt agttttagtt attttggaag tttaggtagg 139920
agaatggtgt gaattaggga ggcggagttt gtagtgagcg gagatcgtat tattttacgt 139980
tagtttgggc gatagagcga gatttcgttt taaaaaaaaa aagaaataaa aaataaaaat 140040
aataaaaaaa aaataattta ttttagtagg gagtaggagg taaggtttga tatgtagtgt 140100
ttgttaattt ttgtggtgta aatatattta ttgtggtaaa ttttaagtta taaataggat 140160
attattaaat atagagttgg gaaaagatat gtatattatt ggataaaacg tttaggtagt 140220
tacggtttat tttttttttgg gaagggggggt atgtttagaa tgttttttat tatggagaat 140280
gattgtggta aatagggttt aattggtagg ttgatttaat taattgttta ttgatgtgat 140340
gtggggtagg tttttgaggt tgtttttatg tttagggaag aaggtggtta tgtggttata 140400
gatttggttt tgggtttgtt tttttttttt ttttttgaga tagagttttg tttttttttt 140460
ttaggttgga gtgtaatggt gtgattttgg tttattgtaa ttttcgtttt ttgggtttaa 140520
gtaatttttt tgttttagtt ttttaagtag ttgggattat aggtgtttat tattatgttt 140580
agttaatttt gtatttgtag tatagatggg gttgttggtt aggttagttt taaatttttg 140640
attttaggtg atttatttat tttggttttt taaagtgtta ggattatagg tatgagttat 140700
tgcgtttatt cgggtttgtt attttgagga tatatttgga gttatgtgtt tatgattttt 140760
tttttatttt aaaattttga ttttatttta ttgtttttag agataggttt tgttttgtta 140820
tttaggttgg agtgtagtgg tgtaattata gtttattgta gtttcgaatt tttaggttta 140880
agcgattttt ttattttagt ttttcgagta gttggggttg ggtttatagg tgttattata 140940
tttagttata ttttatattt ttttgtagag atagggtttt gtaatttgtt taaattggtt 141000
ttaaattttt ggttttaagt aattttttta tttttagttt tataaagtgt tgggattata 141060
gatgttagtt agtgtattta gtagttatta tgattgattg gtgacgtttg ttttagtttt 141120
aggatgaaag agtagtagta ttttagttaa gtatttgtgg tttttgattg gaagacgttt 141180
tttttgtttt ttaagtatag tgtaaatgtt attttttttgg taagtttttt tttaatttta 141240
tttttttttt ggtatagggt ttgttatagt tttgtttata taaagagagt tattagtgta 141300
tgtggtatag atgttagttg tttttttaat atttgagttt tttttttttt atagtaattg 141360
aagttttggt tgtgtatagg gaagtttagt tgaagaatat attgtttagt ttttttgatg 141420
ataggagcgg ttatataatt gggttttggt tagtgggaag gagttgaagt gtttaatttt 141480
taggttatgt ttttaaaaat aaagagggat atttgttttt tttttttttt tttttttttt 141540
ttttttttggt tggagtgtag atatgacggt aggagttgga attattttag tttaagagat 141600
ggaagttgtg tgtcgaggat gtattaaagg aatgaattag gttcgggttg ttttgggttt 141660
ttataacgat tgatatttgg attgggacgt gggttcgagt gttgtttagt ttttgatgtt 141720
ttagtttttg ttatatagtt agatggtgtt ttgattaatt taaggtatgg ttattatttt 141780
atttgtttgg tagggtaggg gttttatttt tatacgttga gtttttggta gaatttggtg 141840
taatatatat agttaaagta aaatgaatag gttattttaa ttaaggttta ttttgtgggt 141900
tggttttggt atttggtttt aatttttatt ttattttatt tatttattat ttttgagata 141960
gagttttata tttgttgttt agtttagaga ggagtggtgc gatttcggtt tattgtaatt 142020
tttatttttt aggtttaagt gattttcgtg ttttagtagt tgggtagttg ggtttttttt 142080

gagtagttgg gattataggt gtacgttatt atgtttggtt aatttttgta ttttttgtag 142140
agatggggtt ttattatgtt ggttaggttg gttttgaatt tttgatttta agtgatttat 142200
tcgttttggt tttttaaagt gttgggatta taggtatgag ttatcgtgtt tagttagttt 142260
tggtatttgg aattggaaaa taatagtttg agttttagag gggataaata taaatatgag 142320
gtagaaggaa tagtaagtaa atacgtgttt agagtaggtt gggttgttta tatttttaaa 142380
atatcgttag gtgttgaggt ttgggttttg tgttagattt ttgggtgtag atttcgtggg 142440
gatatttatt gggtagtggg atttttggta agttatatta ttttttgag ttttcgttga 142500
ttaagttgtt tattgagaat aatatttaga ttgaaaggat ttataataat atgtgaaaag 142560
tgtttggttt aaaataggag tttggtaatt ggtagttttt attatttata atatatagtt 142620
taacgttttc gttttatagg aatgaaaata ggatttagat aggtaaaatg atttgttgag 142680
ggtcgtatag tgttaatgat agagttagga ttagggtttg gggttttgat tttttttgta 142740
gtattttggt tttgtgagaa gtggttttgg gtgaatataa ggagtgagga gttggtgaga 142800
aattttgaaa atgaagttgg gtgtggtggt ggatgtttgc ggttttagtt atttaggagg 142860
ttgaggtagg aggattgttt gagttcggga gttggaggtt gtagtgtgtt atgattgtat 142920
tatcgtattt tagtttaggt gatttagtaa gattttattt tagaaaagga aaggaaagga 142980
gaggggaggg gaagggaggg aatttttgat agtgatttta gagataatta ggatgatttg 143040
aaattatttt gaaatgtatt aaaaaataaa aataaaatgg atttatggtt aggcgaagga 143100
gggatagatg gacggtgagg tgataaagta ggtgtagttt ggtgttagtg gtagaattta 143160
ggtgatggtt gaatgtgttt tttgtaaaat gtttttaatt tgattatgtg ttttgaatat 143220
ttttataata gaatggaggg aagagaaagg taagagggag ttattgtagt tgttgtagta 143280
ggagtagatt ttaaaatatt atgtattaga tttttagttt tgtttttgtg gatgtgtttt 143340
tgtttttttt tttgggattt aaggatattt aatgagcgta tgtgttttta agttttttttg 143400
ttttttttatt tgtttttata tttggaagat tttataggtg gcgtgggtta gtttttttatt 143460
ttgttttttg tgtttttagt gtatgataga tattgagtga gtgttagttt tttttttcga 143520
tattttgttt gtttgtttag agaggttttt ttatagttta gttattaggt tgatgttttt 143580
ggacgtttgg tcgatagttt ttttagttgt gttttagtga tttggtaggt gttttgagtt 143640
ataaataagg ttattagtgg atgtttttaa agtttttttt attacggcgt tttttttttt 143700
tttattaata gttcgttatg atagttagtt tgtatgagtt tttttttagg gttgtgtttt 143760
ttgaggttgt ttagcgtttg gtttgttaga gtttattaga tagggtgttt attttttttta 143820
ggtttgagtt tttatttaga gaattattta tagaaggata atggagaaag gtagttgggg 143880
tattaaatgg agtgtgtttg tatgtggggg gggaggggag ggggagggaa agtaggaaga 143940
gaagatataa gaggagaaat aagaataaga ggaaatatag aaggaggagg agtagttttt 144000
tttatggggg tggattttgt gtttagtttt ttattaagga tattatattt attatttcgt 144060
ttggttttta cgaatatttt taaggtcgtt ttttttttggt ttttattttt ttattaggaa 144120
gcggaggtat agagtgagaa agagttgttt aaggttttta ggtagtttac ggagttattg 144180
ggagttatat ttaattcgtt taattttacg tttttgtttt tagttattat aataaattat 144240
taaggtttta tagttatagg agtttagttc ggagttttta tcgggttttt taaggtaggg 144300
agatggggtt ggaggttatg ttaggtgtac ggtgtttaga gttgtttagg aagtttcgcg 144360
gtgcgggttg ggtattaggt agtgtttgta ggatggagaa atgaatgagg ttgagatata 144420
gtgtcgtttt ttttaaacgt gatttttttt gagtttttgt gattggttat ttgagagggt 144480
agttttggtt ttttggcgtt tatgttcggg tttagagatg ttttttttag ttaggttttt 144540
ttttgaggat ggaaatttag aattttatta taggaggaag agttgggagt attttatttt 144600
tttttttttt ttgagacgga gtttcgtttt gtcgtttagg ttggagtgta gtgacgcgat 144660
ttcggtttat tgtaagtttc gtttttttggg tttacgttat tttttttgttt tagtttttcg 144720
agtagttggg attataggta ttcgttatta cgttcggtta atttttttgta tttttagtag 144780
agacggggtt ttatcgtgtt agttaggatg gtttcgattt tttgatttttg tgattcgttt 144840
atttcggttt tttaaagtgt tgggattata ggtgtgagtt atcgcgttcg gttagggagt 144900
atttttaaga tttttgcgga gggttgttta ggtttaagtt ttttattatg gaaggttttt 144960
tagatgcggt agttacggtg tatgggttga gggtttttat ttattaagaa gtaggagtat 145020
ttgatgtata tttagtttac gatttttatt tatttggggg ttgtgtttgt agttacgtga 145080
tataagggaa tagttatagt agtgggttat aaagagagtg tgggttaatg tgtaggggta 145140
gaattttgat agtttttttat gggttggtag tagggagggg tatatttgta taaattttat 145200
acgtatagtt ttttatatat atgtggttga taattattta tatatttaag acggatttg 145260
gcgtatagtt tattagtaat aggcgttggt aatttttagt tgttttttttg ttgcggcgta 145320
gatattattt aaggtgggat ggagagagtt attgttttta aggttatata ttttaaaggg 145380
agatgttggg ttacggaatg aaaataggag gattagttag agttattggt tttgagttat 145440
ataatgggat atataggttt atttatttta tatttttatt gagtaattat aacgagttaa 145500
gttgtataga ttatttaaat aatcggtacg tagaaaagta ttttaatttt tttttggttt 145560

tgacgtgatg ggaagaattt ggatttttgg agttatagag ttggatggaa attttggttt 145620
agttaattat tggttggggg agttcgggta agtgatttaa ggtttttgag tttgtttttt 145680
tgtaaaagag tagtgttcgg gtttgttacg ttaggttagg gtaagatgat tgaagagtaa 145740
gcgtttagta tcggttaggg gttaacgttt ttaaggacgt aaagtgtaat aagtaggcgt 145800
tacgattgta tcgtttttta ttgtttgtgg taaatatata agtaaagatg gtttcgcgtt 145860
tgcgttgggg gaggtaggga attggtttta ggaggacgtt ggttttttta gttgtttttt 145920
ttaaagtttt ttgggaggag aaatgattta ttttagttgt tgggagtttt aatttataag 145980
taggtttgtt tattcggtag cggtcgtatg taggtaggag gggtttcggt agtttttaag 146040
agtgaggggt cgtttagttt tttttagtt ggaaaggagg ttttaggttg ggtaggagta 146100
tttggggaaa tttagttttt ggagttaggt tatattaggg agatgaggtt gttagaagtt 146160
tttttggtgt ttatagtaaa atataattaa agtgggattt agattttaat tagtttaagg 146220
tttgatttat attagaggga aattagcgtt tgtcgtatga gtgagcgttt atgtgcgtgt 146280
aaggtagaga tagattttgg gtaagttttt ttattttttt acgtttgttt ttttatttat 146340
aaaacggggt aatattattt attttatagg gttgttatga aataaatatg tattaatatt 146400
tgtaaagtgt tgagaatagt atttggtata aaataagagc gatagatttg ttaaataagt 146460
aaaaaataag atataagttt aaagtagagt ttggtatatc gtgagtatta agagttgtgt 146520
tttttgattt ttttttttg tggagatttt tttattagaa gtgggaggga ttgtcgaggt 146580
tggagttttg ttttagggat ttttgtgggg agtttttttag tttagaggga tttgttttac 146640
ggattatatt tgtttcgggt tttcgtgttt ttgtgtatgt gtgtatatat gtgtgtatat 146700
atatgtaatg tgcgtgtgtg tatttttatg gatttgtgat aatttgtata tatatgttta 146760
gaatttattt gatatagtgt tttattaaga tgttggagtt tcgatgaatg gattgcgggg 146820
ttgagatatt gtttttatag gaagttgttt tagtagtaag ttttttggtt tttatagata 146880
attaaaaaga atggagtttg tttttatttt ttgttttaaa aatgatgtat agtgtgtggg 146940
gtttgatgtt ggaggttttt ttagtagata tatttttaat aatattatat tttgaaattt 147000
aagttgtcgt aaaataagat aagtaaagat ggtattggta gggatttttg aggttttttt 147060
tatgtatttt tgaataattt ttatatggtt tttgtttgaa tgtttttagg tatggggaat 147120
ttattatttt gtgaaatagt ttgttttatc gtttttaagt gaatattgtg agaaaaatat 147180
ttttttttgt aaagagttaa gttttattcg ttagtatttt ttaggtattt ggtattagtt 147240
ttatattttg ggggaattta aaataaatgt tttttttta ttagataatt tattaattat 147300
ttggagatag tggttgtagt tttttgtttt atgtagttat tttatttagt tttttttgg 147360
aatttttgac gtgaatataa gttttttttgt tacgtaggtt ttttttttgg gtttttttatt 147420
tatttgcgta tattttggag aggtcgttcg ttttgtgggt gagatttatt gtgggttgaa 147480
ttgtgttttt ttaaaagata tatgtaagtt ttgatttttt agtatttatg aatgtgattt 147540
tatatggata tagggttttt gaagatgtaa ttgggttatt tgagggtata ttgaattaga 147600
gtgggtttta aatttaatga taggtgtttt tataagatag aggtaggttg ggtatagtag 147660
tttatgtttt taattttagt gttttgggag gtaaagtttt attgtttgag cgtaggagtt 147720
tgagattagt ttgggtttc gtttttataa aaaaattaaa aaattagtta ggtatggtgg 147780
cgtgcgtttg tgttttagtt atttggaagg ttgaggtggg aggattgttt gagtttggga 147840
ggttgaggtt gtagtgagtt atgattacgt tattgtattt tagtttggag gatagaatga 147900
gaatttgttt ttaaaaataa attaaaaaga taatagagag ggtagtttgg atagagatat 147960
aagaagagat atagggtgaa ggttaggtgg ggggtaggtc gagttgtaag tcgaggaata 148020
ttaggggttg ttggtcgtcg tagagttcgg agaaaggtat gggatagttt ttttcggaat 148080
ttttaggagg aatgaatttt tagatatttc ggtttgggat ttttggtttt agaaatttta 148140
gggaagatat ttcggatgtt tttgtttttg tttttgtttt tgtttttgag acggagtttt 148200
gttttgttat ttaggttgga gtgtaatggc gcgatttcgg tttattgtaa tttttatttt 148260
taaggtttat gttatttttt tgttttagtt ttttgagtag ttgggattat aggtatttat 148320
tattacgttc ggttaatttt tttttttttt ttgtattttt agtagagacg ggtttttattt 148380
atgttagtta ggatggtttc gatttttga tttcgtgatt taatcgtttc ggttttttaa 148440
agtgttggga ttataggtat gagttatcgt acgtggtcga tttcggatgt tttgagttat 148500
tttatttgtg gtttttttgtt atgatagtcg taggatagta atatagggtt ttagtagggt 148560
ggttcggtaa gggttttttt tttacggtag tttttatttt gtttatttg tagggttatt 148620
tagatgttta tttcggtttt ggttagtaag tgttagtgta ttttttttacg tttagtagta 148680
agggtagtat cgttgttgat taagtatttg ttatgtcggg gtttttgtgt ggttttgggg 148740
ttttttttt ttgtttatat ttggtagttt agtttttttt tttgtatttt atttgatgtc 148800
ggggttttgg gtaagtttgg gggatttgtg ttttatttta tagattttt agttgatttt 148860
attagttttt gtatagggtt cgtttgaagg gtcgttgggt tgggcgttaa ttagagtttt 148920
tagtagtagt tttttcggtt ttttgatttt ggtttatgtt ttaggggtag ggtttttaag 148980
ggttttgggg tcgtttttga ggttagtttt tttgttgatc gtagtttata tagaggaggg 149040

tttaatttg agtgtggtcg ttcgtgattt tgagagatgt ttagagttgt agacgtgttt 149100
tttaagtttt ttagagttcg ggataggagt tatttaatgt ttttagtgtt ataggtaaaa 149160
tttgtttttt ttaatataaa ttgtttttag ttttatttaa tgtttttttt aacgagtagt 149220
aataaaaata tggtttttg ttaaaggtgg ggtttatagt tttttagaat tcggtttttt 149280
gttaggttta ggtataattt ataggagtgt tcgtttttga ttcgtgtgtg tgtgtgtgtg 149340
tgtgtgtgtg tgtgtgtgtg tgtgttgtgt gtacgcgtat atgtgtttgc gtaagttttt 149400
ttatgtgtag ttttgggaag ggtggagagg tttacggaaa tttttttttgg tatttagtaa 149460
gttagaaaaa aggaaaatga gatttgtgta tatatgtgta agtgtatata tagtatatag 149520
gtatatatat atatatattt agtatatatt atataggttt atacgtgtat atatgtgtag 149580
gtatatatta tataggttta tacgtgtata tatatggggg tatatagtat ataggtatat 149640
ataggtatat atattttttt gtaggaaagt tttttttttgt tttttatttt gtagggtttt 149700
taggttttat gaaataagat tgaatttttgg aagtttgata aaaggtttgt ttggagtatt 149760
ttggttattt ttattagttt ttttagttgt ggaaggtatg tttcgtggta tttatttatt 149820
ttttgttttt atttttattt tcgttttcgt tttttttttt ttgttttttt agtttatatg 149880
ttgtttgttt taattttaat ttttgtattt ggacgggaat tattttttttt gtagaagggt 149940
gtaatgtatg tggggatata tatatatata tatatatata tattgtattg gaggagagaa 150000
gagaggtaga gttttttttta gatttagagt tttagcgagt gacgggattt tttttagaga 150060
atacgtgtag aagtttgcgt tattggaaga agatattatt ttgatttata tttttttttt 150120
tttttttcgt ttttttttata gagaaggaaa ggttttagta aatagtggtt atgtgggttt 150180
tttgtatgtt attggtagtt ttttagttag gattattttt tttttatttt tggaagtata 150240
agaatagaga gaagggtgag tttttgtttt tgggggtttt tttttgtatt tttttggagt 150300
taattttagg gttgtgggtt gtataggaag gagggtttaa taggtttttc gtttaatttt 150360
gtttttttag tgaggagtcg atttattttg tttgtaaaag atgttggttg ggatttgatg 150420
ttgggtaatt tgaggagaag agaaggggga taggggttga ggttaggttt ttggttttga 150480
aatagttata atttttgggg tagagtttaa gggtgttagg ttggagattt tatattcggt 150540
tagttggtag ttattatttt ggtttagtta gtggtttagg gtattatagt tatttagatt 150600
aggaatttta tttttttgga ttgtattaat gaggaaggtt gagtttgtag gttttatata 150660
tttatggtaa ttttaaggta gagaggttat tgttaattga gggttacggg taggagtttt 150720
gtgagtttat tagtaagttt ggaagattaa ggtttttggt tatagagagt agttgttatt 150780
tggttaattt tgtagaaaat attatagttt tggataaaat atttgaaaga ataaaataat 150840
aatagtttgg agttagtata gaatatttta agtaggtaga agttggagta gagtttatat 150900
ttgatagagg ggaataaaat tagataagtt tttggttttt ggaggttttt ttgtttagag 150960
gtaggtattg agtaattaaa tttggataaa aagttttaaa agtatttagg gaaaaataat 151020
atgtggttta taggggaata gttagatgaa tgttgatttt ttgatagaaa taacggaggt 151080
taaagatatt ggaattttat attttaaagt tttgaaagaa aaaattgata atttagaatt 151140
ttataattag tgaaattatg gtttaagaat gatagtaggt tgggtatagt ggtttatatt 151200
tataatttta gtattttagg aggttaaagt aggtagatta tttgaggtta ggagttcgag 151260
attagtttgg ttattatggt gaaattttat ttttattaaa aatataaaaa ttagttgggt 151320
atgatggtat atgtttgtag ttttagttat ttgggaggtt gaggtaggag aatcgtttga 151380
gttcgggagg agaaggttgg tgagttgaga ttgggtagga gttttgaaat tatattattg 151440
tattgtagtt tgtggggaga tagagtgaga ttttgtttta aaaataaaaa taaaaataaa 151500
aaaagaaaga atgatagtaa aataaagatt tgtagataag tgaaaatgaa agggaattag 151560
ttgtttgtaa atttgtatta taaaaatcgt gaagtaagtt ttttaagatt atgggaaatg 151620
agattagatg ggaatttagt tttttaggaa ggaatgaaga gtataggata tagtaaatat 151680
atgggtaaat agaaaagatt ttgttttatt tttaattttt ttaaaatgta tggggttgtt 151740
taaagtaaaa atttttttat tgtattgttt agtttataat gtgtatggat gtagtaaata 151800
tttaataatt attatatata ggatggaaag tggtaaatga atttatagga ttgtttagtt 151860
tttatatttt ttttttatga agaaaaataa tgttaatttt aagtagattg tgaaaagtta 151920
agaatgtgta atagaattta taaagtaatt attaaaatag taatataaag agatataatt 151980
aaaaagttaa aataggtcgg gtttagtggt ttacgtttgt aattttagta ttttgggagg 152040
ttagggtggg cggatcgttt gaggttagga gttcgaggtt agtttggtta gtatggtgaa 152100
atttcgtttt tattaaaaat ataaaaatta gtcggtcgtg gtggtaggcg tttgtaattt 152160
tagtatatta gggaagttga ggtaggagaa ttatttgaat ttaggaggta gaggttgtag 152220
tgagttaaga ttatgttatt gtattttagt ttgggtaata gagtgagatt ttgttttaaa 152280
aaaaaaaaaa aaaaaaaaat ataaattaaa atagaatttt aaaaaatatt taattaatat 152340
aaaagaagtt aagaaagtaa gatgaaagaa ataaatatta aagagtaagg tagtagtttt 152400
aaatttagtt atatttataa ttatgttaaa tagaaatagt ttgaatattt taattaaaag 152460
gcggaggttg ttagaatgaa taaaatagta agatttaatt atatattatt tataaaagat 152520

gttgttttag tataaatata ggttgaaatt ttttgtttgg aaaaagatat attgatgttg 152580
tttgaatgat gatggagtta tgttttgata aatttattat aagtggaaaa taaatattat 152640
tttaaaaatg tatttaatat atttaattta tcgaatattt tagtttagtt tagtttattt 152700
taatatgatt agaatattta ttttagttta tagttgagta aaattaataa atataaagtt 152760
tattttataa taaagtgttg attattttat gtaagttatt gaatattgta ttgaaagtga 152820
gaaatataat ggttttatat atatttaaag tatagttttt attgtatata tattacgttc 152880
gtattatggt aaagttgaaa aattgtaagt taaattatta taagtttttt tataagtttt 152940
tttagttttt ttatgtattt taaatagtat gtttaagaag gttggagagg ttatataaat 153000
attaataaag ttgagtttaa gataaaatta ttacgagata aaggaatata tttattatgg 153060
ttaaatgatt agtttatcga gatattatta taattataaa tgtgtatgta tttaattaaa 153120
ggagttttaa aatatatgat ataaaatttg aaagaattaa agggagaaat gggtaatttt 153180
ataattatag gtggagattt tttttttttt tttttgagac ggagttttat ttttgttgtt 153240
taggttggag tataatggtg taatttcggt ttattgtaat ttttattttt tgggtttaag 153300
cgattttttt gttttagttt ttcgagtagt tgggattata ggagtttgtt attacgttta 153360
gttaattttt gtatttttag tagagataag gttttattat gttgtttagg ttggttttaa 153420
atttttgatt ttaggtgatt tattcgtttt ggttttttga agtgttggga ttataggtgt 153480
gagttattgc gtttggatgt atagttggag attttaatat tattttagtt tatttaggat 153540
gttaaaataa aatgttttag gttgggtaat ttataaatag attgtagtgt ttatagtttt 153600
agaggttggg aagtttaaaa ttagggtgtt tagtgtttgg tgagagttag tttttttgttt 153660
tagagatgtt ttttttttgt tgtgttttta tatggtagaa gggattaatt ttatttacga 153720
tgattttgtt tttatgagtt aatatttttt tgatgtttta atttttaata ttattgtatt 153780
agagattaag ttttaatata tgaattttgg gggatataaa taggtaggtt atagtaaata 153840
ttattttttt aatatttgag aaataagtat tagataattt gaagaatatt ggtaattagt 153900
aattgattta attagtagtt ataaagtttt atatttagga agtttagaat atatatatat 153960
attttttttt taagtgaaaa tagtgtattt attaagatat atattttggg ttataagttt 154020
taaattttaa aagtatgtaa aatatgtttg tttggttaaa gtaagattaa attagaaatt 154080
aatagtttta agatatttag aggagttttg attatttgta aattaaataa tatattttta 154140
aataatttat gagttaaaga ggaaattata agaaaaattt tataatattt taaatgaaat 154200
gataatgaaa atatagtata ttaaaatacg taggttgaag ttagtgttta gagggaaatt 154260
gtaagtgttt atattagaaa agaagtaaaa aaaaaaaaaa aaaaaaaaaa aaaaagattt 154320
aaggatttgt tttaagaatt tggaaaaatg ggttaggtgt agtgtagttt acgtttgtaa 154380
ttttagtatt ttgggaggtt aaggtaggtg gattatttga gattaggagt ttaagattag 154440
tttggttaat atggtgaaat tttgttttta ttaaaaatat aaaaattagt tgggtatggt 154500
gggtattttt agttatttag aaggttgagg taggagaatg gtttgaattc gggaggtgga 154560
ggttgtagtg agtttagatt atgttattgt attttagttt gggtaataga gtaggatgtt 154620
tttttaaaat aaaataaaat aaaataaaat aaaataaaat aataaaaaaa ataaagaatt 154680
tggaaaaata tgttagttat ttatgtattg tttttttaatt ttgtatatat tttgttaggt 154740
ttcgtttcgt gatattggat cgaggtttta tgagtttttt tttttggtta aatatagtta 154800
tgttaggttt tatttatagg ggataatatt gtggggagtt gttaggtttt gttgaggtag 154860
tagttttttt ttttggtttt agggtttttt ttttttttga aggtatagtt gttagtggcg 154920
tgtgtttagt ggggtttgtt ttgtattgag tttttttagt attttagttt tagtttaatg 154980
ggattttagt aaatttttta gatatttagt ttttgtttat ttatattttt tagtaggttt 155040
ttcggttatt tagtaagttg attttgtaga tttatttttt ttatattttt tggtaagaat 155100
tttttttgtta ttagtaggtt gtaggttttt gtggtagtta tatatttttt aataaggttt 155160
gaacgtaaga cgtggtaagg tgtggaggac gtttcgagtt tttggttttt tttttattaa 155220
ttttagtggt agtaattgtt tttgtgtgtt tgtatatatg ttttaaagta tttattatag 155280
aaaatttatt attttaatta tttttaggtg tgtagtttgg tggtatgaag tatatttata 155340
ttgttgtgta attattatta ttatttattt tatttttta aattgtaatt ttattttat 155400
taaatattaa tttttttattt attttttttt tttttagttt ttggtaattt ttattttatt 155460
ttttgttttt ttgaatttga ttattttagg gattttataa aagtgaaatt atatagtatt 155520
tgtttttttta tgtttggttt ttgttattta gtatgttttt aaggtttatt tatgttatag 155580
tatgtgttag aatttttttt tttttttttt tttttttttt ttttgaggta gttttgtttt 155640
gtcgtttaag ttggagtgta gtggtatggt ttcggtttat tgtaattttt atttttttag 155700
tttaagcgat tttttttgttt tagttttttt agtagttggg attataggta tatgttttta 155760
tgtttggtta atttttgtat ttttagtaga gatagggttt cgttatgttg gttaggttag 155820
ttttaaattt ttgattttaa gtgatttgtt tatttcggtt ttttaaagcg ttgggattat 155880
aggcgtgagt tattatattt ggtttttttt ttttttttaa ggtagaataa tattttattg 155940
tatagatgga ttatattttg tttgtttatt tattcgttgg tggatatttg ggttgtttgt 156000

atttttggt tatgggaaaa gtgttgttat aaataagaat gtagtatatt tgtttaagtt 156060
tttgttgtta gtttttgga gtatatattt agaagtggaa ttattggatg ttatggttat 156120
tttgagttta atttttgag taattgttat attttttta tagtagttgt attattttat 156180
atttttatta gtagtgtacg gggttttaat tttttatat ttttgttaat atttgttgtt 156240
atttatgttt tggttaatag ttattttaaa gggtgtgaaa ttatatttta ttgtggtttt 156300
gatttttatt tttttgatga ttagtgatat tgcgtatttt tttttgtgtt ttttggttat 156360
tggtatgttt tttgtggaaa aatgtttgtt tattgggttg tttttttttg ttttgtggtt 156420
tagttgtagg agtttttat atattttgga tattagtttt ttatcggata tacgattttt 156480
aaatattttt ttttattttg tggattttt ttttacgtta ttggtagtat tttttgatgt 156540
ataaaagttt taagttttgg tgaagtttaa tttattattt atttttttg ttgtattttc 156600
ggtgttatat ttaagaaagt attgttaatt ttaatgttac gaagtttttt ttttatgttt 156660
tttttaaag tagttgtttt ttatatttgt tttttttata ttttttagag tttttttgta 156720
taaggtaaaa taaattttat tttttattt ttatttataa tatttagtgt tttagttttg 156780
atattagatg tgtggttttt tttcgtcgat ttttaagtaa ttttttaata tttagtagtt 156840
tttaattggg tgttttgtaa tttagtttaa ttttgatatt aattagagtt agtgtagatt 156900
ttataggtta agggttttat tttataagat tgttttttaat tttagagatt agtcgtaagt 156960
ggtaagatat taggttattt ataatttttg tttgatttgg ttataaattt gaggttttta 157020
taattttttt tttaggttta ataatttgtt tgagtgattt atagaattta ggaaattagt 157080
ttatttaaaa ttgttgattt attataaatg atattttaaa ggatataaat gaatagttaa 157140
ttggaagaga tgtataagaa aggtatgaag gaagcggtat ggagttttta cgggagggta 157200
tattgttttt ttagtatttg taggtattta ataatttgaa attttataaa ttttattttt 157260
ttgggttttt agggaggttt tattatataa gtatgatatg aaatttttgg ttattgatga 157320
ttaatttaat ttttattttt ttttgtttcg taggaggtgg atgggaaagg ttgtaagttt 157380
tagtttttta attataaggt tgggtttttt ggtaattagt ttttattttt aggttgttta 157440
ggagttttta gttattagtc gtttttttag taaataaaaa gatatttatt attttggaga 157500
ttttaagagt tttaggagtt atatgttaaa taaagttggg ggatagagat tagatagata 157560
gatagataga tagatagata gatagataga tagatagata gatagatata ttgatttttg 157620
agatagagtt ttttttgtt atttaggttg gagtgtagta gtgtaatttc ggtttattat 157680
aacgtttgtt ttttcgggtt taaattattt ttttatttta attttttaag tagttggaat 157740
cgtaggtata tatcgttacg ttaggataat tttttgtatt tttggtagat atgaggtttt 157800
attatgttgt ttaggttggt tttaaatttt tgagtttaag ggattcgttt gttttagttt 157860
tttaaagtgt tgggattata aatatgagtt attgtatcga gtttagttta aatatatttt 157920
ttttatatta taatgttatt tttttgtgat tttttttaata gttaattatt ttttatagtt 157980
aagaatattt tatattataa gttgttattt aaattgtcgg tgtggttttt gtttttttgat 158040
tgggttttga ttaatgcgtt gaagtaagta taaggaagga agtaatgaag ataatagaga 158100
aatttaagga aatggatatt agaaaagggg agtttaatgt tggtttttta aaaagattta 158160
gattgataat tttttagtta ggttgttgat gagagggaga tggcgaatat aaagttataa 158220
taaaaggaat gaagtgtttt ttttacggat tttatcgata ttatagtgat aaggaatata 158280
ggtatatttt attttattgt gttttataga tattgtattt ttttataaatt gaaggttcgt 158340
ggtagttttg tattaagtaa gtttgttggt attattttttt taataatatg ttaatattgt 158400
gtttttgtgt aggagtttga tgatttttga aatagtttaa atttttttat tattattata 158460
tttgttatgg tgatttgtga ttttttttt ttttttttt tttttgtttt ttttttttg 158520
agatagagtt ttgttttgtt tggagtgtag cggcgcgatt tcggtttatt gtaagtttcg 158580
tttttcgggt ttacgttatt tttttgtttt agtttttcga gtagttggga ttataggtat 158640
ttattattac gttcggttat tttttttcgta tttttagtag agacggggtt ttatcgtgtt 158700
agttaggatg gttttaattt tttgatttta tgatttgttc gtttcggttt tttaaagtgt 158760
tgggattata ggcgtgagtt attgcgttcg gtttttttt ttttttttg agatggagtt 158820
tcgttttgtt gtttaggttg gagtgtagtg gagtgtagtg gtgtgatttc ggtttattgt 158880
aatttttatt ttttaggttt aagcgatttt tttgttttag ttttttttagt agttgggatt 158940
ataggtattc gttattacgt ttggttaatt ttggtatttt tagtagagac gaggttttat 159000
tatgttggtt aggttggttt taaatttttg attttaggtg atttatttgt ttcggttttt 159060
taaagtgttg ggattatagg tatgggttat cgcgtttagt gattagtgat ttttaatgtt 159120
attattgtag ttgtttttgg ggtatgacga gttatattta gataagatag tgaatttatt 159180
taataaacgt tgtatgtttt gattgtttta ttgattggtt gtttttcgtt ttttttttt 159240
ttgtttaggt atttttattt tttgagattt aataatattg aaattaggtt aattaataat 159300
tttataatgg tttttaagtg tttaagtgaa aggaagagtt attcgttttt tattttaaat 159360
taaaagttag aaatgattaa gttgagtgag gaaggcgtgt cgaaagttaa gataggtaaa 159420
agtgagattt tttatgttaa atagttagtt aagttgtgaa tgttaagaaa aagttattga 159480

aagaaattaa aagtgcgatt ttagtgtata tatgaagaag aagaaagtaa aatagtttta 159540
atgttgatat gaagaaagtt tgagtggttt ggatagaaga ttaaattagt tataatattt 159600
ttttaaataa aagtttaatt tagggtaaag ttttaatttt ttttaatggt atgaaggtta 159660
agagaagtga ggaagttgga gaaggaaagt ttgaggttag tagaggttag gttatgaagt 159720
ttaaggaaag aagttatttt tataatgtaa aagtgtaagg tgaagtagta ggtgttgatg 159780
gagaagcggt agtaagttat ttagaagatt tagtttagag tattgatgaa ggtggttata 159840
ttaagtaata gaattttagt atagatgttt tattttttat tggaagaaga tgttatttag 159900
gatttttata gttagagggg agaagttaat gtttgttttt aaagttttaa aggataggtt 159960
gatttttatg agaggttaac gtagttgttg atcgtaagtt aaagttaata tttattgatc 160020
gttttaaaaa ttttagggtt tttaagaatt ttgttaaatt tattttgttt atgttttaga 160080
aatggaataa taggttttat ttgatagtat atttgtttat agtatggttt attgaatatt 160140
aaaaatttat tgaggtttgg tgcggtaatt tacgtttgta attttagtat ttttgaaggt 160200
tggggcgggg ggattatttg aggttaggag ttcgagatta gtttggttaa tatggtgaaa 160260
tcgtattttt attaaaaata taaaaattat ttaggtatgg tgttatacgt ttgtaatttt 160320
agttatttgg gaggttgagg taggagaatc gtttgagttt aggaggcgga ggttgtagtg 160380
agtcgagatc gtgttattgt attttagttt gggtaataga gggagatttt gttttaattt 160440
gaaaaaaaat aaaaaaataa aaaagggtta agtacggtgg tttatagttg taattttagt 160500
atttgggag gttaaggtag gtaaattatt tgagtttagg agtttaagat tagtttggtt 160560
aatatggtgg tgaaatttta tttttattaa aaatataaaa attagttagg tatggtagtg 160620
tatatttgta gttttagtta tttaggaggt tgaggtagga gaattatttg aatttgggag 160680
gcggaggttg cggtgagttg agatagcgtt gttgtatttt agtttaggta ataaagcgag 160740
atttgtttt aattaaaaaa taataataaa aaaaaaataa atagggttcg gtgtagtggt 160800
ttatgtttgt agttttaatg ttttgagagg ttgaggtagg tggattattt gaggttagga 160860
gttcgagatt agtttggtta atatggtgaa attttgtttt tataaaaaat ataaaaatta 160920
gttaagtatg gtggtgtgtg tttgtaattt taattattta ggaggttgag gtataagaat 160980
tatttgaatt cgggaggtag aggttataat gagtcgagat tatgttatcg tattttattt 161040
tgggtgatat agcgagattt tattaaaaaa aaaaaaaaat tattgagatt tattgtttag 161100
gaaaaaagaa tttttttaaa atattattgt ttattgataa tgtattttgt gatttaagag 161160
ttttgacgga gatgtatgta aagatgaatg ttgtttttat gtttgttaat gtaatattta 161220
ttttgtagtt tatggagaag ggataaattt gattttaag ttttattatt taagaaatat 161280
attttataaa gtagtatttg ttatagttag tgattttttt aatggatttg ggtaaagtaa 161340
attgaaaatt ttttggaaag gatttttat tttagatgtt attaagaata tttgtgattt 161400
ataagaagag gttgaaatat taatattaat aggagtttgg aagaagttga ttttaatttt 161460
tatggatgag cggtttaaga ttttagtaga ggaagttatt gtagatgtgg tggaaatagt 161520
agagaattag aattagaagt ggagtttgga gatgggattg gattgttgta attttatgat 161580
taaatttgaa tagatgagga gttgtttttt atggatgagt agataaagtg gtttttggag 161640
atgggattta ttttaggcga agatgttgtg aatattgttg aaatgataat aaaagattta 161700
gaatattata ttaatttatt tgattaattc gtggttaggg ttgaaagaat tgattttaat 161760
tttgaaggaa gttttattgt gggtaagatg ttattaaata taatgcgtgt tatagaaaaa 161820
ttttttatga aagaaagagt taatttatgt taaaaatttt agttaagaaa ttgtcggtta 161880
ggtgtagtgg tttacgtttg taattttagt attttgggag gttaaggtag gtggattatt 161940
tgaggttagg agtttgagat tagtttggtt aataaggtga aattttgttt ttattaaaaa 162000
tataaaaatt agttaggcgt ggtggtaggt atttgtagtt ttagttattc gggaggttga 162060
ggtaggagaa ttgtttgaat tcgggaggta gaggttgtag tgagttgaaa tcgtgttatt 162120
gtattttagt ttgggtaata gagtaagatt ttgttttaaa aaaaaagaaa aagaaaaaag 162180
aaataaagaa attgttgtta tagttatttt taatttttag taattattat ttggttagta 162240
gttattaata ttgaggtaga tttttttatta agaaaaagat tatggtttgt tgaaggttta 162300
gatgattatt agtatttttt agtaaaaaga tataatgatt ttatatattt taatagatta 162360
tagtatagtg taaatataat ttttttttt tttttttgag atggagtttc gttttgttat 162420
ttagtttgga gtatagtggt gtgattttag tttatcgtaa tttttatttt ttaggttcga 162480
gtaatttttt tattttagtt ttttaagtag ttgggattat aggtatgtgt tattatattt 162540
agttaatttt tgtattttta atagagatga ggttttatta tgttggttag gttgttttta 162600
aatttttaat tttaaatgat tcgtttattt tggtttttta aagggttagg attataggta 162660
tgagttatta tgtttggttt aaatataatt tttatatgta ttgggaaatt aaaaagtttg 162720
tgtgacgtgt tttattgtaa tatttgtttt attgtcgagg tttggaagta aatttaggt 162780
atgtttgtat tacgaataat tttaaatgag taaatttaat aatttatttg aaagggataa 162840
atttttttaa agtataattt attaaaagta agataagggg aagtaaaaaa atttgaataa 162900
ttttttttat atattaaatt gaatttgtga ttaaaaataa ataattattt tttttagtag 162960

aaaattttag gtttagacgg tttttttggt ggattttatt aaatatttaa ggaagaaata 163020
atattaattt tatgaaaata tagaaaataa agagaaggga atatttttaa attaatttta 163080
tgaagttaaa atgaggttga taaagatatt atcgaaataa attaataaat aaagaaaata 163140
ttatattaat attttttatg aattaatacg taaaaatttt taaaaattgg gtaaattaaa 163200
tttagtaata tataaaaagg atgatatttt ataagtaagt tggatttttt ttagaaatgt 163260
aaggttggtt taatattaaa aaattaggtt tgtatagtgg tttatatttg taattttagt 163320
attttgggag gtagaggtgg gagtattgtt taagtttaag aattgtagat tagtttgagt 163380
aatataatga gattttattt gtataaaaaa ttttaaaaat taaaaattag tttggtatgg 163440
tggtatgtgt ttatagtttt tgttatttag gaggttgagg tgggaggatt gtttaagttt 163500
aggagtttag gttgtagtga gttacgatgg tattattgtg ttatagtttg ggtaagagtg 163560
agattttgtt tttttaaaaa aaaaaaaaaa aaaaacgggg ggtaaaaatt aattattgta 163620
atttatttta ttaaacgaat aaaagataaa ataatgatta tttttataga tgtagaaaaa 163680
gtatttatta aaattatatt tatttatgtt aaaaaatttt agtaaattaa gaatagaagg 163740
gaattttttt aatttgataa aggttatata tgataaattt aaagttaata ttataagttt 163800
ggtataggta aggatgttta ttaattatta ttatttaata ttgaattggt agttttaatt 163860
agtgtaataa agtaagaaaa aaaaataagg tattaagatt agaaagaagt aaaattatgt 163920
atttaaattt taaaagattt ataaaattat tattagagta ataaatgatt ttggaaaaga 163980
ttataggata taagatttat atataaaaat ttattgtatt tttatatatt agtagtaaat 164040
aattaaaaat gaaatttaaa atttatttta gtttaaaata gtgttaaaag taaaattttt 164100
aggaataaat ttaataaatg gtatgtaaga tttttttttt gaaaattata aaataatgta 164160
attttgtaga gatttttttt tagtagagaa ataaaagaaa tataaaagga tttttaaaac 164220
ggagatatat tatttatata aaagatttaa tattgttaaa atgttaattt ttttttaatt 164280
gatttataga tttaatataa ttttaattaa aatttatatt gtatttttgt agatattaat 164340
aagttatttt ttaaatttat aaggaaattt aaaggattta gaatatttaa agtaatattt 164400
tagatatttg agagatttta tattaagatt tattgtaaag ttatattaat taagatgtgg 164460
tattagtaaa ggaatggata aaatattaag aaaatataat agtgttttaa tatagggttt 164520
atatgtatat agtatatata ttaatatagg attatatata tagttaatat aggatttata 164580
tgtatatagt atatatatta atataggatt atatatatag ttaatatagg atttatatgt 164640
atatatgtat atatattaat ataggattat atatatagtt aatacggggt ttatatgtat 164700
atatgtatat atattaatat aggattatat atatatatat agttaatata gggtttatat 164760
gtatatatgt atatatatta atataggatt atatatatag ttaatatagg gtttatatgt 164820
atatatgtat atatattaat ataggattat atatatagtt aatatagggt ttatatgtat 164880
atatgtatat atattaatat aggattatat atatagttaa tatagggttt atatgtatat 164940
atgtatatat attaatatag gattatatat atagttaata tggggtttat atgtatatat 165000
gtatatatat taatatagga ttatatatat agttaatata gggtttatat gtatatatgt 165060
atatatatta atataggatt atatatatag ttaatatagg gtttatatgt atatatgtat 165120
atatattaat ataagattat atatatagtt aatatggggt ttatatgtat atatgtatat 165180
atattaatat aggattatat atatagttaa tacggggttt atatgtatat atgtatatat 165240
attaatatag gattatatat atagttaata tggggtttat atgtatatat gtatatatat 165300
taatatagga ttatatatat agttaatacg ggtttatat gtatatatgt atatatatta 165360
atataggatt atatatatag ttaatatagg gtttatatgt ataaatgtat atatattaat 165420
ataggattat atatatagtt aatatagggt ttatatgtat atatgtatat atattaatat 165480
aggattatat atatagttaa tatagggttg atatgtatat attaatatag gattatatat 165540
atagttaata tagggttaat atgtatatac gtatatatat taatatagga ttatatatat 165600
agttaatata gggtttatat gtatatacgt atatatatta atatagggtt atatatatag 165660
ttaatgattt ttagtaaagg tgttaggtaa tttaatagag taaggtatgt tttttaata 165720
aacggtgtta gaattggata tgagtataga aaaataagaa ttaattttga ttttaaatta 165780
aatataaaaa ttgatttaaa atggattata taatattttg acggttataa taggttaaaa 165840
aatgtaaaaa tttaaaaaat taaaaaataa aaggaattat agaatatata attataaaat 165900
ttttagaaaa taagagaatt ttttttgatt ttagtgtagg taaagatttt ttttataggt 165960
tataaaaatt ataaatcgaa attggaaaaa gttgataaat tggattttat gaaaataaaa 166020
tatttttgtt tattaaataa tattattaag aaaattaaaa ggaaaggagt aatgtggaaa 166080
aaaaaattta tgatatatat atttgataaa ggatttatat ttagaatata taaaaaatat 166140
atgaaataat attttttttt tttttttttt ttgagataga gttttgtttt gttatttagg 166200
ttggagtgta gtggtgtaat ttcggtttat tgtaagtttc gtttttttagg tttatattat 166260
ttttttgttt cggtttttcg attatttggg attataggcg ttcgttatta cgttcggtta 166320
attttttgta tttttagtag agatggggtt ttatcgtgtt agttaggatg gttttgattt 166380
tttgatttcg tgatttgttc gtttcggttt tttaaagtgt tgggattata ggcgtgagtt 166440

attgtgttcg gttaattttt tttttaaatg agatttatgg ggaaatagtt aaatgaagaa 166500
ataataaagt agtaataaaa taaaatttaa gttttatttt attgtgtagt tgtaggataa 166560
agagtagaag tttgtagttg gggaataagg tgtttatttt tttaagtatt ataaaatgaa 166620
ggttttagaa attattcgat tttgaaataa tataatttta aaaagaagta agacgtggat 166680
agataatttt ataaaggaaa atacgtaaat gattaataag gattttggaa aattgtgcga 166740
ttttataagt tattaagaaa atataaaata aaagttttaa aaaaaaatga agtttggaat 166800
aattgaataa atatataaaa cgataataaa atgaattttg atttttttatt ttatattata 166860
tgtaaaagtt aatttataaa agttatataa ttttaagttt tttaaaggaa aatataaata 166920
tttttgtaat ttggtagggt aggtaaaggt tttttataaa gaatatagag agagaattat 166980
aaagaaagga gtttgttaaa ttagatttta taaaaattaa agtgatatat ttttgtttat 167040
tggaaaatat tattaagaaa atgaataggt cgggtatggt ggtttatgtt tgtaaaatcg 167100
gtttttgggg aggtagaggt aggaggatag tttgagttta ggagttttag atttgtttgg 167160
gtaatatagc gagattttgt ttttgaaaaa ggaaagaaaa tgaataggta agttatagat 167220
tggaataaaa tatttgtaaa atacgtattt gataaaagat tagtatttag aagaaaaagt 167280
taagtaattt aattttaaaa tgagtaatag atttgaaaag atattttata aaggaaaaat 167340
atatgaagag ttaattatga aaatgtgttt aatcgtatta.gttattaggt taaagtaaat 167400
taaagttata atgagatgat attattatac gtttattaga atgattaaga ttttatagag 167460
tgatagtatt aaatgttggt taggatgtgg agtaattaga attcgtattt attgttggtg 167520
ggagtgtgaa atggaagggt tattttggga aaagatttgg tagtttttta taaaattaaa 167580
tgtatattta ttttatattt attttaggtg tttttataga ataaatgaac gtttatatgt 167640
atagaaaaat ttgtataaga agaattagcg taataggttt atttataaga gttttagat 167700
ggaaatagtt aggggttttt taataagaga atgattaaaa atttatagaa tgtttatata 167760
gtggacgtta tttagtaata aaatagaata aatcgttggt atttgtagta atatgggtag 167820
ttttaagaaa tatttaaatt ggtaaaatta tttcgtggtg gaaaaaaagt ataattgtgg 167880
ttgttttggg gaaggtgggg attgtgatgt atttggaggg gtatgaggaa tttattgggg 167940
tgatagtgat gttttatgtt ttggttaggt tgggatatac gtggatattt ttgttaaaat 168000
ttaaagaacg gaacgtttta tatttgtgta ttttagggta tatatatttt attttaaaga 168060
aaaataaaaa taattataga taaatgtcgg attttagtta atgaaatgta aattgaggtg 168120
ttgataggat gttgtgtgtt gatttttgta gtttatttta agtgtattaa aattagcgtg 168180
gatgttggtg gttagaggga tttgtggtga agtaaatagt aaaatggttg ttgtggaatt 168240
taggtggtag gtgtatgggt gtttatttta gaatttttta tggttttttt tttgtggttt 168300
tgttttgttt tgtttttgag atagggtttt gttttgtcgt ttaggttgga gtgtattggt 168360
ataattttag tttattgtag ttttaatttt ttaggtttaa gtaatttttt tattttaatt 168420
tttcgagtag ttgggatttt aagtttgtat tattatgttt gattaatttt ttgtattttt 168480
tatagagatg ggatttttatt atattgttcg ggttggtttt gaattttggg gtttaagtga 168540
tttttttatt ttaatttttt aaagtgttgg gattatagtt atgagttatt atatttagtt 168600
tttttttatt tttttatgt ttgaaaagtt ttacggtaaa aagttggaaa aatgtaaaaa 168660
agattgaaat ttattaatta aaaataggaa aaataaaata aaagagatgg taggagatta 168720
ttttttttt ttttaattta gtttttaatt tttttttttt tcgtttagtt aggataggaa 168780
ttttaagggt tcgggttttt cgtgtttgtg cggggaagga ggtattttcg tgtttgtgcg 168840
ggggttggtt atttgttttt cggtgtattt tttgtatttt attttttttc gttcgtagtg 168900
agtagttata gttggcggaa attatattta ggtagttttt gtttataaat ttagtgaaaa 168960
tgtaaatttc gtaggagttg gattttcgag agtttgcggc gttatttgtt ggaggaaagg 169020
tttttttgtat tcgagttttt tttttttttt tcgttgttat tcgttttttt gtttaggttg 169080
ggcgggtttt ttttttgtaa tttcggggta ttggtgtacg ggaaatgttt agtgttttat 169140
aattttttttt tatttatatt ttaatattta gttttagtg gatatcgttt tcgttcgata 169200
aatatattat ataggtatag tttaagatta tttattttag gaggagtata agttttagtg 169260
tttattattt tagttttttt tttttttttt ttgacgtagt gatataggtt taggatattg 169320
aagattttg attttataaa tatgttgaag ttaattattt tgaagttgta gttttatta 169380
ttattattat tttttatttt ttgtaaagat aaagttttgt tatgttgttt agtttggttt 169440
ggaattttg gatttaattg atttttttgt ttcggttttt taaagttttg ggattgtagg 169500
tgtgagttat tgtatttagt ttgaagtcgt agttttttaa ttattgttaa tagtaatatt 169560
tattttttg tgagtagtag ggtttgttgg tatggttgat agttttgtat tattgtatcg 169620
aatttgttgt ttgggatttt aggtgagttt cgatttttag ttggagggat aagttgtgtg 169680
gggttgggta gggtgaattg cgggagatta agtttagatt gagggttata agttatatga 169740
gtattggggt taggtaggga tagtgaagga gatagtgtaa ttttgaggaa agataacgtt 169800
atagaatgga gaggtttagg ttcgggttt tttttgttg tcggataggt atgtttgtgt 169860
ttttttttta gttttcgtga tggttaagtt atgtgttttt tgagaggtaa gcgaggtttt 169920

tgatagtagg ttttaggttt taattaaatt gtttgttttg tagggtgagt tgtataggtg 169980
tttttatttt cgattttttt gaattttgt tgtttttaag ttttatttat ttttttagg 170040
tgaatagaaa tattgtttag gggagaacgg aggaagaatt taggaaaggt tgttcgagag 170100
tttggttgag tttacgttat cgttaagagt agcgttaaaa tttaggagtt ggtgagatta 170160
tggtttaggg tttatattaa tatgaggggg tgttagtttt atgattaatg agtttttgtt 170220
ttattatttt ttgttattta tattttgagg gttatagtta gtacgtgaat tggagttggt 170280
tttcggtatt ttttttttta tattagtttt ataatgtgag gtgggggttc gtttttagag 170340
gtattcggag tagtaggtgt tttaagaggg ggaagttttt tcgaagtttt tttttattta 170400
cggtatagga cgtagagggg atagagtatt aggaatattt agcgattata ggggtttgtt 170460
tcgaaggaga ggaggtagga ggtagggaga gtggttggga ggttagtttt ggagtgttta 170520
tttttttattt cgagggagag gaaggaagta gaaggtgagt atagggagtt ttagttgggg 170580
atggagcggg ggttgtagtt cgattgtgtt gtttggagcg ggggtatgga ttgtagtttt 170640
tatttatttg tttggttagt tcgaagttat tttaagtttg gagattttat ttgttttgtt 170700
tattttttttt agggatggtg tttaaatttc gaagttttgg ttggtgttgt aaatatggga 170760
gggaggtttt gttttatttt agttttaaga gttagttaag aatgttttgg atatttgaat 170820
tttgtttcga gttagaggtt gggaagaaat ttgaaggatg gtgggacggt ggatagtagt 170880
ttttaggtat taggtattag ggtttttgtt tggggatggg ggtattagta ggtgttaagt 170940
gtattatatt agggtttatt atgtttttgt ttagagattt agtattttac gaaataggaa 171000
ggaacgaggg atgagcgtgt ttttttttt aatattttag tttttgtttt gaggttgtta 171060
ggtatttaat ttttttagga tttaaattat tttggtttta tttacggtta agtgtattaa 171120
attatagtta ggattattgg gtttagatat taaaagcgtt ttttttaaag tcggtagtta 171180
ttagggtagt atagggtagt gtaaggttgg gtagggtcgg ttatttgtta gtatttagtg 171240
gtttttgttg gatgttgacg ggggtagtta ggtaggtggg tttatttagt aattaaagag 171300
atttttttttt tttaattttt tttttaaaga gattttgatt ttttttttt agttgtaggg 171360
tttgtagtag ttttttttt agtaggtttt tttgtttgtt tttggttatt tttagtagtt 171420
atagggtatt gaggttttat tatagtgaga ataaaattaa gtttttatta gggtttttaa 171480
ggttcggtac gatttggttt tgtttgtcgt attggtgtta ttatggttcg tttttgaaaa 171540
tatattttag ttatttttat tttttttagg ttttagtttc gggtttttat atttgttgtt 171600
tttttttgtta ggttgttttt tttttggatt ttggtttggt tgggtttttt tggttattat 171660
agttttagtt tagtgttatt atttagaaag ggtttttatt aattttttcg ttatatatat 171720
agatatgtat ttttagtaat ttgtttttag tttttttata gtttttattt aaaattattt 171780
atggatttgt ttatttgtgg attgtttttt agttttaatt gttaagtaat aagcgattag 171840
cgattgtgtt tttgttgttt tttattatag gttaagtatt taagagaggg gttgtatata 171900
gtaggtttta ataaatattt attagacggg ttaggtacgg tggtttatgt ttgtaatttt 171960
agtattttgg gaggttaagt tgggtggatt atttgaggtt aggagatcgg gattagtttg 172020
attaatatgg tgaaatttta tttttattaa aaatataaaa attaattagg tatggtggcg 172080
gacgtttgtt attttagtta tttgggaggt tgaggtagga gaatcgtttg aatttgggag 172140
gcggaggttg tagtgaggcg agatggcgtt attgtatttt agtttgggtg acggagtgag 172200
atttcgtatt aaataataat aataaaatat ttgttagaag aattaaagta gtttgtttgt 172260
gtttaatagg aatttaagag tataagatga atttaattaa aggatatata tagtaggtat 172320
tttatagttg atatttgttg gtggagggag attaggaaag tggggagagg tagtgtttgg 172380
gtatggggat attttgggta tggggacgtt ttggatatag ggatatattg ggtacgggga 172440
tattttgggt ataggacgtt ttgggaacgg ggatattttg ggtacgggga tattttgggt 172500
atggggatgt tttggatata gggatatttt gggtacgggg aaattttggg tatggggatg 172560
ttttggatat agggatatat taggtacggg gatattttgg gtatggggat gttttggata 172620
tagggatata ttgggtacgg ggaaattttg agtatcggga tgttttaggt acggggacgt 172680
tttttgttat ttgaaatttc gtagttttat tatattcgtt tgttttagta aatcggttta 172740
aagagtattt ttaagttttt atggtaaagt tttagagtta gtgtttttttc gttgatttcg 172800
agggtgtgtt gtttatttag tgtttaagtt agagtttttt tatttagaaa aaaaaagagg 172860
agggtggttt tacggatgga ggtatgaggg cgtttatagg tagagggagt tttcgggttg 172920
agatttgcgt tatttttttgt tttaatttta aggcgtagtt tggagtttaa attagttttt 172980
aaacgtgggt cgttggttaa gaggaatttt tttatttacg attttttttt atatttagtt 173040
tttattgttt agagtatgtt agttttgttt gttttggttt ttaggatttt tttagggatt 173100
tttaggtttt aagttagggt gggtttgaat taaggtagtt ttagaattta tgttaatgtt 173160
tttgttagtg ggagattttg gggagtatcg tgcgggatgt atggggttag gcgggggtat 173220
atagcgagcg gttttagaag ttttggtcgt tcggagtggg ggggtgttta gagtaagaga 173280
cggggttagga agttggagtg agtaaggagg ttttagtttt tggtgggagt aggtcgggta 173340
aaaataaatt aatttgaggg tagtagttag cgagattatt ataggagggg tggtggggtc 173400

ggaggggttt taggagttta ggagagggag gtattgttag aggtaaggtc ggtagatgag 173460
agttagtgtt ggtaggatta atttgttata gtagttatgg tttatttttt tttttttttt 173520
ttatatatgt ttttggggtt tatggaattt attagtttat tataaaggat attataaagg 173580
atatagggga agagatgtgt agggtaaggt atgggggagg gacgtagagt ttttatgtgt 173640
tttttaggcg ttttattttt taggagtttt tatatgttta tttatttaga agttttcgga 173700
attttatttt tttgagtgtt tacggaggtt ttattacgaa ggtatgattg atttaattat 173760
tggtcgttga tgatttgttt aatttttagt tttttttttt tatttggagg ttgcggggtg 173820
gtgttgaatt ttgtgatttt atttggtttt ttttgggaat tattttgaag ttgttagttg 173880
gtttgttaat tatgagtatt taaaaagata ttattttgga gattttaagg attttaggag 173940
ttgtgtgtta ggaaatggag atgaaaatta agtgtatatt ttataatatg atagttagtt 174000
tttttttaatt ttagttttat tttttgtcgt gttgtcggtt ttagttttc gcgttttagg 174060
gtttttgaat atattgtcgg ttgtttgtag cgatcgtttt tgtttgttgg gtttgagttt 174120
ggtttatttt ttttattttt ttgcgtttag ttcgaaggtg ttttttcgag gtagtttttt 174180
tgatttggtt ttacgaatcg taggtttgt tttgttattg gtttttttg ttatatttgt 174240
tattttttgt gttttacgtt tattgatgtg gttttgtatt tgtgtagttt tatgagggta 174300
aggattgtgt ttggttttgt tttttacggg attttagcgt taagtttata gggagtattt 174360
aatgttgaat gaatgaatga gtgatcggta agggaggttt attttgttat gttgggggtt 174420
aaaggttgtt gggcggatta tatgggtgag gttgggtggg gagggaagta gagatgattt 174480
tttggaagaa ggggtaggag gcggggaggt gggtgtgggg tgttgtataa ggatggtaag 174540
tgtttttagg ggtgataagt taatttagag tattaggtag tagaaaaagg ttgatatttt 174600
tacgttttat ttttatttag ggagaggtta ggttttcggg aaagttagtt tttcgttgtt 174660
aggagagttt taagggaagt tttattagtt gttgagttgg ttatagtgga aatttatgtg 174720
gtgggtggga ggggtttgag gtataggggt ttgggtggtg gttttaggat ggagatttgt 174780
gtttagatta tgaggggtgg ttttagggat gattatttt aaataggaag tagataaagg 174840
tatagatggt agaagcggag gtagagaatg ggttgtaggg gttttttag agggagagtc 174900
gagtaagggg ataggttgga aatatttagt ttttatggtt tgaagtatag atagttggtt 174960
ttatacgtag gtcgggtagt tatattattt agaataaggg gtatgttcgt agggtaggtt 175020
ttaggacgtt tcgggttggt tttgttatta gtttagaaag acggtttagg ttttttcggt 175080
agggagtacg tttagttagg ttttgggtag gagttgagat tttttggttg gtttgagagg 175140
ggatgatcga gtgtaggttt agttcgggat tttttatttt tttttcgtta tttagcggaa 175200
ttagttgggc gtgagaaaag aagagaagtg aattaagagt ttagagtatg aggttggatt 175260
agtttttata ttatatgtaa aaattaattt aatttgtatt aaagatttaa atttaagagt 175320
taaaattata aaatttttag aagaaaatat aggggaaaag ttttttgacg ttgtatttgg 175380
taatgatttt ttggatatgg cgttaaaagt ataggtaata gaagaataaa tagataaagt 175440
ggaacgttat agaagtttaa aatatttgta tattagtttg ggtgcggtga tttacgtttg 175500
taatttcggt attttgggag gtcgagacgg gtggattatt tgaggttagg agttcgagat 175560
taatttagtt aatatagtga aatttcgttt ttattaaaaa tataaaaatt agttgggtgt 175620
ggtggcgggt gtttgtaatt ttaattattt aggaggttga ggtaggaaaa tcgttggaat 175680
tagggaggcg gaggttgtag tgagttaaga ttatgttatt gtattttagt ttgggtaata 175740
gagttagatt ttattttaaa aaaaaaaaaa aaaattgtat attaaagggt attaataacg 175800
tggggagaag gtaatttatg gaatggtagt aaattatagg ttagatatgg gattagtatt 175860
tggatattaa tttatagatt ttttaatatt tagtaataat aattatttaa attaaatata 175920
ggtaaaggat ttggatagat atttttgtaa gaatatgtgt aaatgagtaa taaatacgaa 175980
aagatgttta atattattaa ttattgggga attgtaaatt aaaattataa ggagagattt 176040
tttataatta tgagttttgg gtaggtagga agacgtttgg tggggagttg tcggttttat 176100
gttgttttta gaatttttgt aggggttaag ttcgatttcg atattttatt aggagtatag 176160
aaacgagtgg aaaaggttaa aggttatttt aaagggttag aggggtttag taaggagttt 176220
gggatatgga attatgttgt gtatttagat atacggggat aataggggga tgaaaggtgg 176280
tttttaaaat ttatatttat tttagagttt atgaatatgg tttatttgga aaaagggttt 176340
ttgtagacgt tatgaagtta aggatttga gatgggatta ttttgggtta tttagtgggt 176400
tttaaattcg agaaagattg ttcgtatgag agatagatag ggggagattt agggtataga 176460
aggaggtttc gtgataacgg aggtagaggt cgagtggtgt ggttacgagt taaggaacgt 176520
taagggtttc gggagtcgtt agaagttgtt agaggcgaga agtaggtttt ttttcggagt 176580
tttcggaaag aattaggttt gttgatgtcg tgatttcgga tttttggttt ttagaacggc 176640
gagagaagaa atgtttggtg ttttgagtta tttagtttgt ggtatttggt gatggtagtt 176700
ttaggaaatt attataggga ttagagtttt tacggggttg ggtaggaagc gggtgtagag 176760
cgcgttaggt taggtttttt atttcggaga tggaaaagta gagttggaga ggttgagatc 176820
ggataggtat cgagcgttta ggggtaagaa tttttagggg atggtagagg ttatcgggga 176880

tagtatagcg agggatagga gggaggggtg agggttgagg tatcgttttt taggaggttt 176940
cggtttttcg tttattgatg atttaggaat ttcgtaagag gagttgggtt ttattttgtg 177000
tcgtattgta ttagatggat tttggtgggg atagacgttt agcgatagcg tggttaagtt 177060
ttttttagga tttcgggttt gtatttatga ttatgtatag acggttattt tttttgtttg 177120
tagttttttt tcgtggtttg tttttagttt tttagtttaa tagagttttt tagtggaaag 177180
tttttttttt ggagattttt tttttgttga aatataagtt tttggaataa gataaaattg 177240
tttaatttgt aaaagttata aagtcgtttt agaataaaaa aaaagtttcg atttttggtt 177300
ggaaggagtg gtttttttatg ttttagcggt tagttattat tttatgattt atgtcggttt 177360
tgttgataaa ttttttagta tttttttagg ggaaggttat agtttttagg gagggcggga 177420
gagtatttag gttttcgttt ataaattaga tcggagttag atattgtcga gggagggata 177480
agagggtagg ttttggggtt tttaagtttt ttttggggtt ggtagattat cgtggattta 177540
ttagggtagg attatttttt tttataaatt ttttttgggt agttgtaggg tagtttgagt 177600
tggatacgtt ttatacgtta gtttagtggg gtatgaggta tttatagtta cgtttggaag 177660
ttagcgggtc ggtatttgtc gggtaggttt ttgttatata ggtatagcgt tttttttatg 177720
gttttttttt aggttagagt atgtttgggg attagagtag ttttcgtagt ggtttgtagt 177780
ttgataacgc ggcgtgagtt atgttttgta gagaagaaga gtttttttagt gagggaagag 177840
gcgtttataa aatttaggat acgcgtagaa attcgtagga ggtgggggcg ttgtttaggg 177900
gtcgtgtcgt ttttattttt tattttgtag atgacggaat tgcggtttag agttgttaag 177960
tggtttgttt aaggttattt agtagggata ggaaggaagt tattttttta gggattgtgc 178020
gggagtttag tgataattaa agattagcgt taaagtttgt ttaggacgag aattttatta 178080
agggaaatag tttcgggatt gtcgtgttta ttcggaaggt agttagttta taattgagtt 178140
tttaaaaatg tacgtttttt tatatgaaaa tatattcgaa tgggattttg tagaaagtat 178200
tttttattaa ggtatagttc gaggggaggt tttcgtttta tttttggttg tgttgttttt 178260
agttaagggt agtagggatt agagtggttt ttggggtata gtattggttg tttttaaaga 178320
gtgttttgtt tagtttatga aaagttcgtg ttttgtgagt ttgggagggt gttattattt 178380
ttagtttttt agggagtatt atttttaaat ttacgttttt ttgtttagtt tttagatttt 178440
ttgttggtta tttatttggg tttcggttag attatttata aagtgaaggt cgaattcgag 178500
gtttttcgag gtttttgtta gttaggcggg tggagtgacg ttttttttggg gttggtggtt 178560
taggaggtag gggatttatt gtggtttgtt taggttgttt ggtggggtgg ttatttcgag 178620
gacggtttta ttgtgtaagt ttgacgaagg ttttttttag cgtttttttgt tttgcggttt 178680
ttgttgggcg gagagtagga cgttgggttt tatatttatt cgttttagcg ggtttgagtt 178740
ttcgagttta aatagataag atgtagtttt attgttattt aagtttttttg tcgttttttt 178800
tggttttttt ttgaggttcg tttgttgtgt tcgattgggt gtgtttagcg gtataatata 178860
tattcgtttt attagtttta acgtgataat taagatgtgg ttttgaggtc gggcgtcgtg 178920
gtttacgttt gtaattttag tattttggga ggttgaggcg ggcggattac gagattagga 178980
gatcgagatt attttggttg atacggtgaa attttatttt tattaaaaat ataaaaaaat 179040
atggcgggcg tttatagttt tagttgtttg ggaggttgag gtaggagaat ggtatgaatt 179100
agggaggcgg agtttgtagt gagttgagat cgcgttattg tattttagtt tgggtaatag 179160
agttagattt cgttttaaag aaaaaaaaaa aaaaagatgt ggttttggga gtagtggtgg 179220
aggggattat acgttttaga gaggagttat cgagatgggt tttttttttt taggtttttt 179280
agggattttt taatatatat atagtggtaa ttaatagatt agggtttaag gtacgtaggg 179340
gttgatattt ttagcgattt ttcgttttat ggttcgtagg ttattttaga atatttaaag 179400
tttggagttt ggtttagtta ttttatgttt tttagcgttg ggtttagagg gcggtatgag 179460
cggtagaaat tgttattatt gttaattatc gatgacggcg ttggttacgt gagatggtag 179520
ttacggtatc gtatgtgagt gaagggatta aggaggttgg gtttgggagg gagggtttgg 179580
aaagttgggg tgaattaaag gtaggggagt tttttttttt tttatttgtt tgtttacgtt 179640
ttgatgggtt tgtgtttgcg gtaagggggt tgttagcgtg aatggatata cgtatatata 179700
tgtatatata cgtgtatata tacgttaggt tttttgagc gtcgggagtt tttttttttta 179760
taattaattg ttaggaaggt atagaaatgt tattttaggt aggagatcga ggtagttttg 179820
aagatagatt ttggttggaa gtaaaaaata aattatatag tggataaatt aattattagt 179880
taggtttaag cgtcgtcgtt ttagattcga gttagggggg taggtttttt atattttttt 179940
aaggtttcgg cgttaatagt tttagtattg atagtggtta tgatatgtaa gaatggtata 180000
ttggaaaatg aaagggaagt agttaggaga gaggagagtc gagtttaaat aaaagttaga 180060
tttaggagag tttagttttt gagacggagt ttattttacg agtttggaat cgttaaaaag 180120
aaaagaaata tttttgtgaa aaatgagtaa aagcgttttt tgtatgttta tttttttagg 180180
tataaataaa gtttcgagtt tagggtagtg gggggcggtc gatggggagg gggttgtttg 180240
cgtatttgag attcggtttt tttttatttt tacggatata tttagtagcg gcgggagttt 180300
cggcgtttcg tttagaggcg gttttttttt ttttattttc gtcgtttttt tttttttagt 180360

```
tttttttttt ttttttttt tttttaaaaa tattttttgt ttggtttaat ttttttaatt 180420
tgttgtaatt acgaatgtat tcgaattatt atatttttat gtgttgatta tatgtttgtg 180480
ttttaaattg aagtagtatt gtttttttaa aacgggagga aaaagggagt aaaggagaga 180540
aatttaaaaa taggaggtag gagagagggt tcgaagtggg tttcgaggag ttcggtagat 180600
tatcgggttt gtttgcgaga gttttagagt ttcggtcgtt cggtttggtc ggtggttgta 180660
gaggtttcga gtgcggtttt cggtagtttt ttttttttat ttttttagt tttcgtcgtt 180720
tcgaggtttg ttgttttttt ttaatattta tttggagatg tttaatttag atttttggaa 180780
attaatttat agagttaaat ttttgacgat ttttaaagtt ttttattttt tttgtttcgc 180840
gatgttacga ttttttggg tttaagggtt taaaaagttt agtttttgcg tgtttgtttc 180900
gttttttttt gttttgggtt ggtttagtt ttttttatt ttgtatttta acgtggttgg 180960
gtaggaggaa cggtcgtgtt ttaggttagc gtgttgagaa aaggtttcgg agtatttgtt 181020
taggaggaat tttgagaagt tatttttttt cgttttatta gtttgtagtg atagaggtag 181080
ttgtcgtttt cgtttaggta gagagggcga acgggacggg gatttttttgg aagattttta 181140
ttatatacgt atgagttagt gagtttttag tcgtttttttg ggagggagcg agtttttttt 181200
ttcgggaatt cggggtttta ttggggggatt agtgttgttt tttattgttt attcggggtt 181260
tttagaggtt gtttatttat ttgggggggtt tttattagta tatggttagg gtttatgttg 181320
aggttaaggg tttagtagag taggtgggg tttataatgt gggtttttgg ggtgggaatt 181380
aggggtattt tttttgtttt taagttttat tttaagggtt tatttaaatt cgagatacgg 181440
aggttggttt tgttggttta agtttgttta aagagaatgt gtgtttgtgt taaggttgag 181500
aatttaggta attttttga aaattttaga ttgttagttt ttgttgagaa agggaggacg 181560
cgtggtcgta ttacggaagt tttttgttt ggtcgtatag ggttggtata agatagtttt 181620
gtttttttaga taaaatattt tttagtttgt gttgtttttt aaggttttta ataaatttat 181680
aatatttgta ttttttttatt tcgatatatt tcgtaggttt ttgagtttat aatttttgtt 181740
ttatagagtt aggtgagtta tttgtgatta ggtgaggttt atgtatagtt ttcggttaga 181800
tgatagagtt aaaggttttg aagtgttatt ttaggttata gtttcgttcg tttttttgttt 181860
gggataggat tttaaatgta atttttttgg tagaaaggat atttttttgg ggggtgggga 181920
ggattatttt tagtttttttg ggagatatta agtaattatt ttttttttaaa gagtttggag 181980
gatagtttta taggtagtta gatagtgagg gaggtgggga tagtatttta gtcgaggtgg 182040
gaggttgggg aggaagggag ggattatagt tacgagagaa ggttgaataa tggttttagg 182100
gatagtatgt tttaattttt agatttgtaa atgggatttt gtttggaata agagtttttg 182160
tatttgtgat ttagttgtag attttgttgt ggcgagagta ttttggatgg tttaggtggt 182220
ttttaaaggt agtttcggtg tttatataag acgggggtag agggagattt gatatagata 182280
gggatggtta agtggagatg gggttcggag agatttgtag atgttggtgt tgaaggtaag 182340
ggttatgtag ttataagtta agggatattt atagttatta gaggtttaaa ggggtaggcg 182400
atgaaggtag tggtttttaa tttttttggt atttgggatc ggttttaggg aagataattt 182460
ttttatagat tagggagtgg ggagatggtt ttgagatgat ttaagtgtat tatatttatt 182520
attttatttt attttatttt attgagatag agtttcgttt tgttatttag gttggagtat 182580
agtggtatta ttttagttta ttgtaatttt cgttatttgg gtttaagtaa tttttttgtt 182640
ttagtttttt aagaagttgg gattatagac gtttgttatc gtgttcggtt aatttttgta 182700
tttttagtag aaacggggtt ttattatatt ggttaggttg attttgaatt tttgatttcg 182760
tgattcgtta gttttggttt tttaaagtgt tgggattata ggtataagtt attgtgtttg 182820
gtttttattt tttatttttt gagatagagt tttattttgg gattataggt gtgagttatt 182880
gtattcggtt ataagtacgt tgtattgatt gtgtatttta tttttattat tattataata 182940
tataatgaga taattatata atttattata atgtagaatt agttggagtt ttgagtttat 183000
tttttttgtaa ttagacggtt ttatttggtg gtgatgggag acggtgatag attattaggt 183060
attagatttt tataagaagt acgtaattta gattttttat atgtgtcgtt tataatacgg 183120
tttatttttt tataagaatt taatgttatg gttgattaga taggaggtag agtttgggta 183180
gtaatgcgag taatggggag tgattgtaaa tatagatgaa gtttcgttcg tttgttagtt 183240
atttattttt tgttgtgtag gttagttttt aataggatat acggggttgg gaatttttgt 183300
tttagagttt ttagagggtt atgtttagtg gatattttga ttttagtttg gtgattttga 183360
tttcggatgt gtggtttgta gagttgtgag aggatggttg ggcgttgttg tgatttagtt 183420
agtttgtggt tattcgttat agtagttgta ggaaattgat gtagcgttat agacggagtt 183480
agggtttagt ttattggagt tgttttttgt tattttattt tttttttaaaa tattgtgtta 183540
agttaattat taataattat tgatttcgat gggtaggaat cgtagaggtt ttttggtttg 183600
tttttttaat ttttttttta attttgaaat aatattttttt gttggttttt ttgattataa 183660
atgtaatatt ttttttgta ttgagtttag aaataagaga aaaagtaaaa agtagaaatt 183720
ttagttgggt aaggtggtac gtgtttgtaa ttttagtatt ttgggaggtt gcggcggaag 183780
gattgtttga agttaggagt tcgaggttgt tgtgagttat aattgtatta ttgtattttg 183840
```

gttgggtaaa aagagtgaga ttttgtttta aattaaaaaa aaaaaaaaag aagaggaaga 183900
aaatttatta attttattcg agatttttta tacgaatttt tgttgtgggg tagggaagtt 183960
taggaaggtt ttacgaggtt ttgatttagt ttggttggcg gtgggaattt gggaatagtt 184020
tcgagtgagg ggtcgggtta tgattgagtt tatttattta gttatttttt gtacgttgtg 184080
tatattatgg gtttgacggt tagggttgga gtatttgttg agtatagttt tgatttttag 184140
ttttatttag tatggtttgg tttgaggttt attttttttt cgtgtttgaa aatttaaata 184200
ttttggttta ggcgggggat tatttgaaat ggaaaatttg gtagtatcga ttggttttga 184260
agaggtagga gttagttatg agtcggttgg gaggcgagtg tagaggtggt gaggtaaggt 184320
tagtagaggg tgggggaggc gagggttaat tttttatcgc gtatggtgta agtattaagg 184380
gggtatttta ttaggttagt gtagatttag gtttttgcgg tggatttttc gcggtttttt 184440
tttttagagg aatattgaag aggtttttgt ggttttgttt tagatttaga ggtatatttg 184500
tatattaaac gtattgagta attttatagt gagggagtcg ggtttggttt aatttatttt 184560
tttttaaatt ggattaaagg ttgttgtttt aggaaggtag ggtttttagg tttacggttt 184620
tgggaaattt tggttgtgtt agtttatgag tttagttttt agttatttta gtttgtttat 184680
attttggagt gggtattgtt atttaaaagt tttttttaga tagtttttag ggcggtattt 184740
ttgtttttta ttttttatag attttggtt attttttgc gatattagta ttattggttt 184800
atagtttttt ttaattgtaa agtggggttt agcggtatag gttgggtttg gtagggcggg 184860
aaggaagagc gaaggttatt ttgtttttaa tagtattggt cgggttttgg aaaggtcgga 184920
atttatagat aggaaggaag tgttatattt atttaatttg ttttgcgtta gttagggggt 184980
atttgtaga aagaagttag gtggtcgtgg tcgggtgtag tggtttatgt ttgtaatttt 185040
agtattttgg gaggtcgagg taggtggatt ataaggttag gggtttaga ttagtttgat 185100
taatatggtg aaatttcgtt tttattaaaa atataaaaat tagttgggta tggtggcgta 185160
tgtttgtaat tttagttatt tagaaggttg aggtaggaga attgtttgaa tttgggaggt 185220
ggaggttgta gtgagtttag attacgttat tgtattttag tttgggcgat agcgcgagat 185280
tttattttaa aaaaaaaaaa aagaagttag gtggttattt tttagttttt ggtttggttt 185340
aggaattgtt tttttttaa gatgttatta aatgtttttt ttattaggaa gtttttttg 185400
gttgtttttt ttatttttgt ttttgtttta gaatacggtt cggtttttac ggataatttt 185460
gatattagag tttaatagag attttttttt ttatggaaat ttatatttta aattgaatat 185520
gtgtgtagga taagtttttg tttttatgtg aaataaaagt gaaaggtgcg gattatttgt 185580
ttttttttat ttttttttat aataatttgg gttttatagg ggtttagtag gagttaggtt 185640
tagtttagtc gttttaggtg tattattttg ggtattagcg tttttgtttt tttttacgaa 185700
gtatagtttt attttttggt atttagtggg gaggggatat atattaggta ttttttaggg 185760
tggattgtgt tttttatatt aggttaggag gttttggggt tggtttagtt atttagaaaa 185820
gggagtaggc ggtttaggtg atattgatgt gggtagtagg ggttttcggg aaagggtttt 185880
cgtaggatcg gttttatttt gttttaggag tatatattgg ggaagggtgt tgcgcggagg 185940
tggagagaac gtatttttgg ttgaaagtat gttttttgat gttttgggtt cggatttgtt 186000
gttatttttt atgttttttt atttaatata cgttttagta aatttttat gttgatgagc 186060
gttaagttat agaggacgta taattttcg ttttaaaaag tgattttttt tgttgggtgt 186120
ggtggtttat atttgtaatt ttaatatttt gggaggttaa agtgggagga ttatttgagg 186180
ttaagagttt aagattagtt tgggtaatat agagaaattt tatttttata aaaaaaaaaa 186240
aaaaaaaaaa aagtttaggt atagtggttt atgtttgtaa ttttagttat ttgggaggtt 186300
gaggtaggag aattatttga atttgggagg tagagattgt agtgagttaa gattatgtta 186360
ttgtatttta gtttgggtaa tagagtaaga ttttatttta aaaaaaaaaa aaattttttt 186420
ttttgaaata gggttttatt ttttttgttt aggttggagg atagtgacgt gattataatt 186480
tattgtagtt ttagtttttc gggtagttgg aattataggg ttgtattatt acgtttagtt 186540
aatttttgt atttttagtt aagacggggt ttcgttatgt tgtttaggtt ggtttggagt 186600
atttgagttt aagtgatttt tttatttcgg ttttaaaagt attgggatta taggtatggg 186660
ttattatgtt tagtttgaaa atattttttt taagaattag ttagttatgg tggcgtataa 186720
ttgtagtttt agttatttag gaggttaagg taggaggatt ttttgagttt ggtaggttga 186780
ggttgtagtg agttataatt gtgttattgt attttagttt gggggataga gaaagatttt 186840
gtttagaaag aaaaaagaaa aaaaaaagat ttttttattt atttttttgtt tatttttaga 186900
tttaattatt gaagtttgtt tttttgaatt aaaaatgaag gagtttcgtt ttggattttt 186960
ttttgttgat tttgttattg tatttgttag tagagttagt taagattttt taggttggtt 187020
ttagtttggt tcgtttatag tttttttaagt aagacggatt tttaggttga ggttagttgt 187080
attttttttt ggaatttttt tttatttttt attgtgtttt agatttggt tgtttttaaa 187140
taaatataat ttagttattt ttgtttttgg ggtcgttgta gggtttattg gtgtaaatcg 187200
tttgggtagg tttgttgttt taggagtttt tttaggttag cgtggaagga aattaagata 187260
acggggagaa gtagttcggt tgggtttaat tttattttta tatgttcgtg tttagttaat 187320

tatttgggtt gtagtttgat aggagttgga gttggcgttt tatttttaga gggggttttc 187380
gattgttttt gtagagcgag ggaggtttgg gggtgtagag gttaagggtt tttgtagagt 187440
ttgttttagg tttttgagt ttaagttttt ttgtagttaa cggaagattt tttcgagagc 187500
gggtgttttt tattttgttt gtttagtagg tgtttagtaa tcgtttattg gataaataat 187560
gaatttgtgg atttaacgtg aagatgattt aaattagcgt tttttatag aatttttcgt 187620
aatgatgtta atgttttgta tttgtcgtag ttaatatggt agttatatgt ggtagtgaac 187680
gtttgtaatg tggaagggtt attaaggtaa agagtttcgt atttatgtta taatttaaat 187740
ttaaatagtt ataaatgttt agcgtgtgtt tcgttatata gattttttt gttatttttt 187800
ttttttttt agagatattg tattcgttat taatttttta ttcgggaggt aaacggagaa 187860
atgttatttt ttagttaacg taaaagaagg ttaatatttg tattttaagt tagtttttg 187920
ggtaattttt ttaagtattt tttattaagg ttagtttagt aatattttt gattgtttgt 187980
tacgtattag gaaagtattt cgtcgtggtt ttggttcgag gttatggagg atatagaaat 188040
aattaaggtt aagttgatag aaggggtgtg ataagatggt ttaaagttat tgagttagtt 188100
aggaggttta ggagtatgga agttttgttt tttagaggat aagtggtaat ttatttaggg 188160
ggggttcgag tatttagagt taggagcgta gaagtagggg gttttaggtt ggaaagcggt 188220
attagggtta ggttgggcgg tagtacgaat ttattttcga ggttttgggg gcggaaatta 188280
agggttttat gagtaagaag attcggttta ggtttaatgg aaatgatcgg gtggagggat 188340
tttattattc ggtttttagt atatagggag ggaagtggta atcgtttttt aaatgttggt 188400
cgtggagggg aaggagttat agtagggtag gggtggggag gatggtattt gttggtttaa 188460
ggagggatga tggaggttta attaagatat gatcgtgatc ggggtagttt gtttaagggt 188520
tagtatgtag agggcgagtt gatgagattt tggtagtttg ttgagatttg ggggtgttgg 188580
ggaggtagag gttaaggtta attttgagat tttttgtttg taaatttggg tgggtggtaa 188640
ggttgatatt agagaaatag aggaagagtg ggtagttatg ggtgagagag gaagtaatga 188700
attaggggtg tttgtaagag tttaggggta aggttttcgg gtagttaaga tgttggtggt 188760
tttttttacg attagttttt tttacgtggt atatttttgt ttgtatggtt gggtatgggg 188820
tatttgttcg ttcgatatat tttgagtagt tattttgtgt attgaggtaa cgtggtacgg 188880
tgttttgggg aaatagcgag gaggtattgt ataggtttta tttttttggt attttttgtt 188940
ttttggggat ggtggtagtt aaataagtga tagttaattg ttaggagagt ttggttttga 189000
tttattaggt ggagttaggt atttttgtcg gaagaggtat gggaattagt ataggatgaa 189060
gggaggtagc gagtattagt ttaggttttg tttttatagg gttttttttt tatatttatt 189120
tgtgtaggcg tttttgtga gggtagagat atttgtagtg tttgagggag atttttgcg 189180
ggcggggtcg tttatttata gcggttaggt gggaggtgta ttagtgtttt gtggttgtta 189240
taatggatta tagttgtggg gtttgaaaaa atggaagcgg gtttttttt atgttggagg 189300
ttagaggttt aaaattaagg tgttagtaag gttatatttt ttagggaggt tttggggtgg 189360
ggtggggggg gggtttttt ttgtttttt ttagacgttt tttggtttgg ggttgcgtta 189420
ttttattatt tgtttttgtt tttacgtggt tttttcggt gtgtttgtgg ttttttgtt 189480
tttttaagg atattagttg ttggatttag ggtttatttt aatttagaat gattttattt 189540
taagattttt aattatttat atttgtaaag attttatttt taatttttag gttatatttt 189600
gaggttttg gtggatatga gtttgtgggg gatacggttt aatttaggat agaagggatg 189660
atgggggtat tggggaaaag gttatatatt ttagaggtat tggaagatgg aagagtagga 189720
tttagcgata cgggatgtta ggtagaggag ttggttttaa ggattttagt cggggtttga 189780
gggttagagg gagggaagtt taggtattat tgagatgaaa tgtttcgtgt atcgtgggaa 189840
atagggtaaa agttcgttgg acgtttagtt agagaggcgg ttgtttgagg tatagaaaat 189900
gagcgaatat gggagagttt attagaaggt tattcgggag ggcgtggtgg taatatttag 189960
aagggttgag aagcgtattt ttatagttta gttaaattta tttggggaaa tatacgtttt 190020
cggtagtatt agtaatagag aagatttcg gcgatgttat tagtgggtta cgggacgggt 190080
aaattttgtt tttttatata gaggaatatt cgttagttgt taaaataagt gaattaaggt 190140
ttcgtggatt tatacggata agtttttacg tcgttacgtt gtgggtatag gtagggtagt 190200
tattgatttt agtgaggtat taaggatgtg atgttgatga cggtatttaa gaggagatag 190260
atttaggttg tttagttata cgatgttgat gatcgtatgt aagaggagat taggtcggat 190320
gcggtggttt atatttataa ttttagtatt ttgggaggtt aaggtaggtg gattatttga 190380
ggttaggggt atgagattag tttgattaat atggtgaaat tttgtgttta ttaaaaatat 190440
aaaaaaaatt agttagatat ggtggcgggt gtttgtaatt ttggttattg aggaggttga 190500
ggtagaagaa tcgtttgaat ttaggaggta gaggttgtag tgagtcgaga tggtgttatt 190560
gtattttagt ttgggcgata gagcgagatt ttattttaaa aaaaaaaaaa aggagattta 190620
ggttttttag atatattatt gtttagtata gatgtgtttg ttcggggtta gattttaatt 190680
tagaggggcg ggtatcgtag taggagtggg gttaggatgg agaaggttat ttagggttgc 190740
ggttatattt gtaatgtttt attttttaag cgggatggtg agtatatagg tgttttatta 190800

ttttattttt tatattttg ttttgtagtt ttaagaattt tattttttt ttaaaaggaa 190860
agaaagtgtg tgagttgatt aaagcgttga ttatggtgtt atttaagttt ttgttagaaa 190920
tgttattttt ggtttagttt taaatataat ttcgagaaga gttttcgttt ttttggggag 190980
ggtaagagtt ttagatatag gagattggtt agatgtggat ttatagagaa gttgggttgg 191040
gaggatggga tggtagatag tcgtttgggg agtgttaaat ggggaggtcg gagatgttgg 191100
ggcgtttagg aatgaatttt tgtttggatt agggttttag taaaagggag gtagggagaa 191160
agaaagaagt taagaagatg aggaaagggt gtttcgggtt agagtttata gaggtttata 191220
gagggaggat ttcgtgtgtt agtaggtagt tttacgtaaa tttagattat tagtttgtag 191280
ttttgggatt tagtaaatta tgttttaggt ttttaagttg agttgtgatc gtggttgtta 191340
agtgttgata gtatagaatt agttagtttg agtagaggga agtaatcgtc gggggaggtt 191400
gtgtttatgt agtttagggt gttaggtttt taagatgtaa tttttataag aattggggga 191460
ttttttttat ttattttatt agaagttggt taatgagagg gaaggagatg gagaggtaga 191520
ttagatgcgg agtcggagaa aagtttttta gaaatatagt agcggttggg ttgtttagtg 191580
gttagtgttt aatatgattg cgatgggcgg agtttttttt aattatagcg ttgaattatg 191640
tagttgttta ggggttttta gttagagtat tatttttttt tttttatata tagttttatt 191700
ttgtattgta aggggtttta tatgtattta atgtggatac ggtatttttt ttattttat 191760
tttttgtaaa aagttatttt ttttattgtt taggtttggt ggtgtatagt ttacgggtat 191820
tgtatttatt tttggaaggt agatttagga ggcgtttgtg ttgtttttat agaagtaagt 191880
ttaggattag aagtaggttt tgggtttaga attttgaggt tttaggtgtt agtgtggttt 191940
ggaattgttt agttttatag ttattagtta tacgtagata taagtggaaa tttatttaaa 192000
ataaataaat aattttatgt aagtaaatga gtgaataaag taaacgtaaa tatttatatt 192060
ttgagtgtgt aatagattta ggcggttaag cggttattat ataggatggc ggagtgtaga 192120
tgtatagaat agatttatta ttatagaagg ttttattaga cggagttggt ttagagtatg 192180
gaggtggcgg ttttagatgg gtatatgttt ttgttttcgt aggcgttttg ttttggtagg 192240
acggttgttt tattttgttt tgaaggtagt tttggtttat aggcgtttta tttgtgtgga 192300
tttgatttcg ggttcgtgtt ttttggtaat tttgtttttt ttagaatggt taatttatga 192360
aggtatagtt tttgtttgtt tgttattatg atggtagtta ggggtttggt atttagttgg 192420
tttttagtat atatgtatag gtgattacgt tatttttgtt ttttttagag tttttcgttt 192480
tttaggggag gtaagtttat ttattgttgt gatttattat ttttttttaaa tagaaaagtt 192540
attagatggg tatttgtttt agtttggtcg ttggaagtga tagttaagaa taacgtaggt 192600
ttttagaaat agaggtatta gtatggttat attggggtta ggtaggggag attagggata 192660
tttagtgggg agaagatgac gcgtagattt agttttgggg ggtaagggtt ggaggtagag 192720
ttttgatttt tttgttttat ttttttgttt aggttgtatt tagtgttggt agagtttttt 192780
taagtttata agttgtaatt cgtgttttgg acggttagtt ttagagtttt gatgggtgta 192840
tttgtttttt tttttattg gttagtgtgg aaaattttta aatatatgga aaaagtataa 192900
ggagtagtat agtgattatt gttagaggtt tatttatttt tttttagata atatcggacg 192960
tatttgtttt attttggagt atttttttatt ttgtaaggtt gtagggagtt tatagatatt 193020
gaattaatat cgagaggttt acggttaaag ttatttgttt atggtatatt tgttttttta 193080
gtattttaaa ttaaattatt tagttttatt tattttttgt tttttttttt gtttcgtata 193140
tacgggtttg ttttttgagt ttgggggtgg ttgtttttgt ttttataagg attattttta 193200
gtagggtagc gtcgataggt ttggaagtta tttgagttta aagtcgttaa agattgcgtt 193260
tttttgggaa atgggtaagt ggttttattt atttagggaa gttaggtggg gaggtaggtt 193320
ttagattaga atgaagggat tttagtataa atatatttat gagttgggtg gggttagagc 193380
ggagcgagtt atttatttgg gttattggag ttattaagtt ttcggagtcg gttattggag 193440
gttcgcgtgt tttttaaaga tagagataaa gggtaaaggt tatataaggt tattggcgtt 193500
tttttttcgg gtggatgatt aatattagga atgttttcgt ttttgttttt gttgtgagtt 193560
ttagagttag aaacggggtt tatttgggga tagaggtttt attttttttag gtaattttgg 193620
acgttggagg gatgatagtt attgttgttt ggttgttgga ggggttttgg gttaaatttt 193680
tatatatatt ttttttaag ttttattatt tttgttttgt agataaggaa tttgagtttt 193740
tatagagtgt tattttttttg tggttatata gtgaatgaga gttgtatata ggtgagttta 193800
cgtttattag attttaagtt gggttgtatt taagcgtgtt tttgttggac ggagtttttt 193860
ttttttttta tgtttattcg ttttatgaat tagggttttt ttatattagg gtttataatt 193920
tacgggtagt tttgatttag ttttttttat ttttagggtt gggtgtagtc gggttggatt 193980
tggggatgga gtgggatagg ttatttttat tttttgtttt aggagggaat aaaaatagaa 194040
ttttagatta atgtagtttt tcgcggtatg cgtttttttt gtttatatat aggttttggt 194100
ttggggttgt ttgttggaga ggtttatggt aggtaagttt taattcgagg agatttgttg 194160
aggtgttaag ttaagtaaat tcgaaaggtt gttaggagga gcggttttgg gagtataggc 194220
ggttatttta ttgttgtaga gtacggtatt ttggggatgt tttttgggta atgttatata 194280

gttattttt agagagtttt agggatatat aattagttta gttttattaa tagtgagata 194340
agggattagg tagatttaga tattttttg tatttgatta ttatgttggt tgttggttaa 194400
agatgttagt tttttttgg aatttttag aaaggttttt gtagtattta tttatttatt 194460
tatttattta tttattttat ttatttattt atttatttat ttatttatgg atttatttta 194520
ttttattatt tttttatata tttatttatt tatttattta atttatttat ttagtttttt 194580
atttatttat ttatttatta atttatttat ttgtttattt aatttattta tttattaatt 194640
tatttattta ttatttattt atttatttat ttatttattt atttttttat ttatttattt 194700
attttttttt ttatttattt tataatttta tttttaaatt gtgattagat ggaaatgggt 194760
gtagttagtt tgtttttaaa ggtttagagc ggaggtagag tgaattgttt atttggtttt 194820
cgtgtttagt agtttaggtt tgtatatatg ttttataatt ataggatatt agatttagta 194880
attggtagta gaatgagtat agtgtatttt tcgcggaatt agtattgata gggtatttat 194940
ttttattaag tttgagtttt tttggtgttg gagggaattt ttagatgggt tttaatttta 195000
gttgaattta tttttagggg aaagtaatat cgtgagaaat gcgtgtgata taaggtttta 195060
aagagggtgt gggtcgtgat ttttggagta attagggagg ttttatggag gagatgagga 195120
taaagttgag ttttgaagga gggatcggag gacgttgagt agggatattt atttttgttt 195180
gcgagggttt gacggtaggt tgtgagttgt attttagttt cgatttattt aatttataat 195240
tttagtaggt aggtgttttc gttttatttt atagaggagg aagcgaggtt tagggttggg 195300
taaattgttt aaggtcgtat agttgcggaa tttattttta gtatttttag tttgttttag 195360
ttatgttcgt gattttattt agtatttttt ttttggtgtt tatttatttt gtttattgtt 195420
aagtaataaa aattttatta ggaaaattta ggggtttttg gttagttttt ttagatagaa 195480
aggtagattt cggcgtagat ttttaggaag taaggatgga agggttagta tttgtttttt 195540
agaaatgata gtttttttaa gattaggaga gatggtttag agtgtagttt cggtgggttg 195600
tttaggatta ggaggggaga ggagcgggga ggaggatgaa ttttattttt aaggttgttg 195660
agtggtatat agtgagtttg gatttttttag ttttagttga gtttattttt acgatggtta 195720
tgagtatttt ttttatatat tcgattggaa gtcgtattgt atataacgta tacggggtta 195780
ttattttttg ttttatatat ttttacgtat atattattag tgtattattg ggatattttg 195840
ttgttcgggg ttgagagtga attagaattt tgtttataaa tttagtatat tttattgtgg 195900
aaataatggg gggtaaggag gggtcgttta gtaaagaata ggatatacgt ttttgaggat 195960
ggagttattt ttggttgaga tatttttgta ggggatgtcg gcgataaagg ttgttttatt 196020
tttttttttg ggttagatgg atattatttt tgttttttgt tttttagtat taggtaaaag 196080
gaaaatagaa gaggttttat ttaggttaaa tattttaggg tttttttattc gtaggaaata 196140
tgttggttta ttttttttaa atttagtatg ggggtggaga aacggtgggg aaaatagtat 196200
ttgttttaga tttttttgggt ggagtttttg ttttatttat attgtttatt tttttgattt 196260
tttaaaatgg gtatttggtg tttggtagtt tttggtatag agaagacgta aagtaaatcg 196320
tttataagaa gggggcgagc gtagtttgag tatttttttgt ttttgaataa attgttaggt 196380
ttagcgtagt cgttgtcgtt attgttgttt ttaggaaggg atttttggtt acgagcggtt 196440
ttagtttagt tttttggatt taattattta ggtttgtgtt tgagtttagt aaagaggtag 196500
ttagaaaagt ttttaagata tttaacgatt tcgtggtttt agttgagaag tgagttattt 196560
ttatgtgatt ttaaaataaa atatatttag aaggagagaa gataattgaa atttagttgt 196620
ttggagttat tttagatgaa agtatttgag tttttgggag gtaaaggagg taggaaagtt 196680
tggagttaga tgattagaat ttgaacgtag atttcgttag gtttttgttg tgggttttgg 196740
gagatgattt ttattttttg agttttagtt tttttgggga tagagatggt gtttatttat 196800
aggttgtttc gagttaatga ggttatatgg cgtaagtatt agtatagtgt ttggtataaa 196860
attaaaattt aataagtgat attattatta ttattattat tattattatt tttgagatgg 196920
agttttgttt tgttatttag gttggattgt agtggtacga ttttagttta tcgtaatttt 196980
cgttttttaa gtagttgaga ttataggtat ttgttattac gtttcggtta atttttgtat 197040
attcggtaga gatagggttt tattacgttg gttaggttgg tttcgaattg attttaggtg 197100
atttgtttgt tttagttttt taaagtgttc ggattatagg cgttagttat cgttatttgg 197160
ttgtgatatt attataatat taatattata atagttggtt aggtatggtg atttacgttt 197220
gtaattttag taatttggga ggttgacgtg ggttagttat tcgaggttag ttcgagatta 197280
gtttgattaa tatagggaaa ttttattttt attaaaaata taaaaattag tcgggcgtgg 197340
tggtattcgt ttgtaatttt agttattcgg gaggttaagg taggagaatc gtttgaattt 197400
aggagacgga ggttgtagtg agtcgagatt atattattgt attttagttt gggcgataga 197460
gtaagatttt gttttaaaaa ataataataa taaataaata aaatatattt tgacgagata 197520
tggtaaaaga tagtagaagg taggagttcg gggatttatt atgggtcggc gttttaagta 197580
gaaagaaatg tagtgtttat aagttagaga gtagatgtcg aagataggag acgagcgggg 197640
tttttttggtt ttggagagga aagggtgagt ttaggtagaa agagtaaatt tagggcgttt 197700
tgtgtagtgt tttttttgtg gaattagttt agggtattag tagtatagtt ttttttttagt 197760

agtggagatt aggttaattg tttagtgttt tttttgtttt tttgttttaa ttttattaag 197820
tatagttttt ttttgggtat agtagttttg taggggtgga tattgattta tgtatttagg 197880
agtttagagg tttttttttt tttatttatt tatttagaga tggagttttt ttttgtcgtt 197940
taggttggag tgtagtggta ttaatttggt ttattgtaat ttttattttt taggtttaag 198000
tgattttttt gttttaattt tttgcgtagt tgggattata agtatgtatt attacgttcg 198060
gttaattttt gtatttttag tagagatggg gttttattat gttggttagg ttggtttcga 198120
atttttgatt ttgtgatttt tttattttgg ttttttaaag tgttgggatt atagacgtga 198180
gttattacgt tcggttaggg gtttttttta atggattttt tttaatgtat ataatttggg 198240
gaatttagat ataagaaaga tatagagtat gaaggttttt tattggtttg tttattttta 198300
gaggtggggt tttaggtggt ttaggttggt tttaaatttt tggttttaag taattttttt 198360
gttttggttt tacgttggga ttataggtat aagttattac gtttagttat tttttttttt 198420
atttgggttg ttatgttaga atttggtaag ttataagatg gaaatgttta aataggtatt 198480
tagaatttga gtagatatta tttgatgtat tattgttgtt atttaatga ttgaaatgta 198540
tttattatat tcgttttttt aaaggttatt ttagattttt atagatttta tttgtgatgt 198600
aagatagtta ttttaaattt ttttgtgggt tcgaatagat tagattttg aatttttggg 198660
tagagtaggg atggggttgt tttagaggag ggtgatatcg ttatattttt gttttgtttt 198720
tgtgttgata atgaatgagg tttggatagt ttttttttta ttaaaagtta tattttttta 198780
aaaaggagtt ttttaggatg aggcgttatg ataatttaat tgtgtataaa tatttatttt 198840
taataatgat agaagttatt tatagaaaag aatttatttt tggtcgggta cggtggttta 198900
ggtttgtaat tttagtattt tgagaggtta aggtaggtaa attatttgag gttcggagtt 198960
cgagattagt ttgattaata tggtgaaatt tcgtttttaa tgaaaatata aaattagttt 199020
gtatgtttgt aattttagtt atttaggaag ttgaggtaga agaagtattt gagttcgaga 199080
ggcggaggtt gtagtgagtc gagatcgtgt tattgtattt tagtttgggt gatagagcga 199140
aatttttttt taaaatatat atatatatat atatatatat atatatatat atatatataa 199200
atttattttt attttttttt ttgaaatttt tgagagaggt taggtgtagt ggtttatatt 199260
tgtaatttta gtagtttggg aggttgaggt gggaggatgg tttgaggtta ggagttggag 199320
attagtttgg gtaatatagt aagattttat ttttattaaa aaataataaa agtttaaaaa 199380
gtttttgaga tagtttttgg ggaaaggtgg gttatggaga tttttatttt ggttattcgt 199440
gtttttttcg tttgtttttt tgtggttttt ttttattttg agtttggggt gggggcggtt 199500
tgagtttagg atgggggtgg tttgagtttg gggtgggagc ggtttgagtt tgggataggg 199560
cggtttgagt tggggtgggg gcgttttaag tttgggttgc gggtattttg agtttggggt 199620
ggggatgttt tgagtttggg gtgggggtgt tttgagtttg ggttgagggt ggtttgagtt 199680
tgtgttgggg gtggtttgag ttgggatggg ggcggtttga gttgggatgg gggcggtttg 199740
agtttggggt gggggcgttt tgagtttggg atgggggtat tttgagtttg ggatgggggc 199800
gttttgagtt gggatggggg cgttttgagt ttgggatggg ggcggtttga gttgggatgg 199860
gggcggtttg agtttggggt gggggcggtt tgagtttggg gtgggggcgt ttaggatgta 199920
taggttttat tttttttttt tgaggtttag tttgtttata ggagttgtga agggttggat 199980
taaagttaaa ggtttaaatt ttagatttat ttttagattt agtggataat taaaatattt 200040
aagtttattt ttaaaggaag ttgagataag gagataatgg ttattttttt tttttttttt 200100
tttttgtttt tggtttttgt tatttggagt tgtgttttga agaggtatat tgggtattag 200160
gtttttttgg ttttgttttt atttttgtt tagatataga ataatttttt tttattttta 200220
aggttgatac gtggggttgt ggattaaagt taatttattt atttatgagg ggagtaattt 200280
ttttgtttta ggaatttgtt tttataggag aataaatatt gtagaaggta ttgtttttag 200340
tggtggtgta cgtttgtaat tttagttatt taggaggttg aggtagaaga attgttagaa 200400
tttaggaggt ggaggttgta gtgagttaat attatgttat tgtattttaa tttgggtgaa 200460
aagattaaga ttttgtttta aaataaaaaa agaatagtat tttgtgttaa ggttaggggt 200520
tttagttatt ttttttgtga agcggaaggg gtattattag tttttgtgag aaggaaatag 200580
tttatttaat tataaaatgt taattaaagg aggtagtata ttggttttgg tgcggtgtcg 200640
ttggttttg tttggggtag gaggagggtt gggggagagt taaaagttaa agaaggttta 200700
tggaggtcgt tttttttttg gtgattagtt ttttttttga cggtaagttg ttaatagtat 200760
ttggttttta ggttggggaa ggtgagatta gtttatattt gtttagggtt ttgttttgtt 200820
tgtatgggta gtaggcgttt attttgttag atggttttgt ttattatagg gtattagtgt 200880
tagttaagga tttatagagt ttttttgtaa aggtttatta gtgggtattg ggattttggg 200940
ttttatttat atagggtttt tatataaaat ataggatgtt tagttaaatt tggattttag 201000
ataaataatt aataattttt tgaagagata aggatttgt tgttttattt aagttggagt 201060
ttagtggtat gattatggtt tattgtaatt ttgaatttt ggatttaagt aattttttaa 201120
ttgtagtttt ttaaagtatt gggattgtag gtatgagtta ttaaggttag taaataatga 201180
attattttta ggtataagta tattttaagt ttgttgtggg atatgtttat attaaaaatt 201240

aatttttgtt tatttgaaat ttaaattgaa tttattgtcg ggggagataa tggagtgatg 201300
gattgataga ggagtataga gatagttttt ttaaattttt agttgattaa agtttggttt 201360
ttagtttttt tgggttttta gagatgtatt aagggtggaa gcgattagga gggagataaa 201420
aatattgaag tttgagtgtt agtattgagt cgtgtggttt tggataggat tgtttttttt 201480
ttgagtttta gttttatatg ttataattgg ttaataatag ttataaagta ttcgaagtta 201540
atggttggga gtttgtttta ggaggtaggt attattagga tgtagagaga gtgaggagtt 201600
tttaaagtta gtttaggtta ggtatagtag tttatgtttg taattttagt attttgagag 201660
gttgaggcga gagaattatt tgagtttaga agtttaaggt tgtagtgagt tgtaattgta 201720
tcgttgtatt ttaggttgaa tgatagagtt tttgttttta aaataaaata aaataaaata 201780
aaataaaata ataaagttag tttgtgtatt ggattgtatg ttgttttaaa gtatttaaag 201840
tgagagaggt taggaagtaa tgagatgtag aaaggtaggg ggaggaggtt tagaggtatt 201900
agagaagata gttgattttt atttttatat atttttgggg tattttgagt tttatttata 201960
tatttaggtg agaattttg tatttgtgaa agtaatttga ggtcgagaga attaatagga 202020
aatcgtttta ttttttatgt agggtttttt tataaataag gttgagtcgt agtttattat 202080
tgtagttgag ttttttgtag tagttgcggt ggggaaggtt ttttaggagg tttgagtaat 202140
ttagaaatat ttatagtata tttttttagtt tttttagagg tttttgttga gatgaaagtg 202200
ttttggttcg tggttaaaga gtttagtata ttgggtagtt atagttttta ttatattgat 202260
ttggttatta ggtttttatg aatttagtga ggggagatgg gttttttagg gaagtttgtt 202320
gatttttta tattgttgta aatgtaaagt ttggtgattt tattattttt agtaattgtg 202380
atgtagtaat aataagaata gttatgttta tcgtttattg aaggtatatg atgagttaga 202440
tattgtgttg gtatatgtga tttttggagg taatatattt ttttttaagg aggaaatgat 202500
tgagttttgg agggagggtt ttttatttgg ttttaggttt ttttttgatt tgcgtttttt 202560
tttttttttt cgtttgtttt attgtagttt ttttggtttt tttgttgttt ttggaatgta 202620
ttaagtttgt ttttagttta gtttttgtat tgttggtttt tttttgtttt tttaatttta 202680
atttagttgt tattattttt gtgatttttt attatttatt ttaaagtagt tattagttat 202740
tttttatgta attattttat tttaattttt attatagttt ttagttttat tttgatagtt 202800
tttagaattg atgaatttat tgattttgtt tttttttatt gtatgtaaat tttaagaaat 202860
aagagttttt gtttgtttat tgttgtgttt tagtgtttgt tatagtattt ggtatgtagt 202920
aggtgtttaa tgaatatatt tttaaaagaa tgaacgattg gaaggagttt gaaagaacga 202980
tggtttgtgt taatagttta tttttaattt ttttagacgg gatggagatt attattatgt 203040
aattacgatt tttttgagtt tgtgtggtgg ttgacggtag gtgttagttt ggttggatta 203100
agggatggtt ggtaacgtat cgtttttggg tgtgtttgtg agggtgtttt tggaggagat 203160
gggtatgtga gttggtgggt tgagtggtaa agattttttt ttaatatgga tgatattatt 203220
ttattgttag ataataaaaa ggtagagaaa aggtgaattt tttttttttt ttggaggtgg 203280
gatgtttttt ttttttgttt ttggatttag aatttttagt tttttagtag tttttaggt 203340
ttttaaattt ttggttttaa attaagagtt atattaatag ttttttagtt ttgaggtttg 203400
tagatttgga ttgagttacg atattggttt ttttgggttt ttagtttgcg gacggttttt 203460
tgtggtatat tttggttttt ataatggtat gagttaattt ttttaataaa ttttttttta 203520
tttatttatt atttattttt ttaattgttt atttgtttat ttatttattt atttattatt 203580
tatttttat ttatttattt attatttatt taattaatgt atttatgttt gtttgtttat 203640
cggttattta tatttattta ttaattattt atttatttat tatttatttg ttatttattt 203700
agttatttat ttatttattt atttattttt tttttttttt tgagatggag ttttattttg 203760
tcgtttaggt tggagtgtag tggcgtgatt ttagtttatt gtaattttta tttttaggt 203820
ttaagtgatt ttcgtgtttt agtttttcga gtggttggga ttatagatat gtattattac 203880
gtttagttaa tttttttatt tttagtagag atggggtttt atttgttag ttaggttggt 203940
tttaaatttt tggttttaag tgatttatcg gttttagttt attaatgtgt tggttattat 204000
aggtaagagt tattgaattt aatttattat ttgtttgttt gtttgtttgt ttgtttgtgt 204060
ttatttgttt gtttggttat ttattattta tttatttatt tgtttgtttg tttgtttgtt 204120
tgtttgtgtt tgtttgtttt tatttttttg tttgtttgtt tatttagtta tttgtttgtt 204180
tgtttgtttg tgtttgtttg tttttttttt tttttgttcg tttgtttgtt tatttagtta 204240
tttatttatt tatttatttt ttattggttg tttttttgga gagttttggt tgatgtagac 204300
ggtgttttgg taagtttagg ttgttattta gattattgtg gagggagtga tttaaataat 204360
agatatgtat tttttagttt gagaggttta aagtttcgga ttaaggtgtc gttagggtta 204420
gttttcggcg aggttttttt ttttggtttg tatttatttt tttgttgtgt ttttcgttat 204480
tttttttttg tttataagtg aggggggagg agagattttt ggtgtttttt ttttttttta 204540
taaggatatt aattttatta gattagggtt ttgttttatg atttatttta attttaatta 204600
tttttttaaa agttttattt ttaattatag ttgtattggg agttagggtt ttaacgtagg 204660
aatttaaggg gaagggtata gtttggttta taataaatgg gaaattatgt ttatgattaa 204720

```
atttttaga agatatggtt tattagcgtc gttaatgatt gtaagtaaag gttagagagg 204780
aaaggggttt tcgtggggtg gggggcggtg ggggtagttg gtgtcgggaa taaggttttt 204840
tttttgttt aatagtaggg aagtgaggtc ggttgtagat gttatttggg gtgggaagag 204900
aggggagttt ttgtttgtat attttgtgtg ggttttggtt tgattttttt tcgtgttttt 204960
tattttttgt gttttttttt gtgtgggaga aacgtcgatt tttgttggtt taggtgttta 205020
ggtttttagg tttgttggtt ttgggttggg tttaattaac gagaggtttg ggaggaattt 205080
gggaggtgga agtaagggag ggcgcgggag ttttttattt tttttgtttt agatagtttt 205140
tttagtagga gaggtggttt tttgtgtagt tttagttttt gttagagttt tatttttgtt 205200
tgggttttta gtaggtgatt ttggttttttg gattttggtt ataggatttt tttttttttta 205260
tttttattta ggggtggtgg tggtattttg ttgttaattt ttaggttatt tttataatgt 205320
taagaatttt ttttttattt gtatggtttt tttttaaggt attgagaagg gttttaatta 205380
tatgtttata gggttaaata tttttttgtaa aatttataaa atggaaaata tttttttttat 205440
aattaattaa cgttttcgtt tatttttagt tttgagtttt tgttcgttat attttttttt 205500
gtgtgaggtg gaaacggagg tttgttgtta cgtgggggtt tttgttaagg agaagttgaa 205560
ttggaaagat atttagtttg gttttggtcg ggttatattt atgtagtttt tatttatttt 205620
tatgtatagt ttagttattg ttcgttattt cggtgtggaa acggatttta gtaatattcg 205680
ttttatttat cgttttaatt ttatttatcg cgtagaatta aaaattataa gatacgaata 205740
tattttgcgg tgtttggcgt cggaaaaacg tgcgtagtgg agagaagtaa gatttgaaat 205800
gtacgggttt agaagtaaat ttgtggaaaa ttattttttat tattagaggt gaagttgtaa 205860
gtaaaggatt tagtttttttt aatgtggagt taaaatagaa aagtttttttt ggttagaaat 205920
atatgtataa atgttttata tgtagttaga aatttttttt atttttttat ttttttttttg 205980
atgggaatta tatttaaaaa aaaagaaaaa aataggattt attagaattt ttgtgtattt 206040
tgaataaaat aattgtagta gtattattaa ttttatgttg atcgttattt ttgtatatat 206100
tttaattatt aaaaataaaa ataaaatttg attaattaag gagatttatt cgttttattt 206160
atttatttat ttttgagatg gagttttatt ttgttgttta ggttggagtg taatggtacg 206220
attttggttt attgtaattt ttgttttttg ggtttaagtt atttttttgt tttagttttt 206280
taagtagttg ggattatagg cgtgcgttat tatatttggt taatttttta tgtttttggt 206340
agagatgggg ttttattatg ttgattaggt tggttttaaa tttttgattt taagtgattc 206400
gtttatttta atttttttata gtgttggaat tataggcgtg agttattacg tttagtttat 206460
gttttttttt gttgtttttt tttttttttt gagatatggt tttattttttt tatttaggtt 206520
ggagtgtagt ggtatgatta cggtttattg tagtttcgat tttttgggtt taagtaattt 206580
ttttatttta gtttttttaag taattgggat tataggtgta tgttattatg ttttgttagt 206640
ttttgtattt ttagtagaga cggggtttcg ttacgttgtt cggatcggtt ttgaattttt 206700
ggttttaagt gatttatttg ttttggtttt ttaaagtgtt gggattatag gtacgagtta 206760
ttgtgtttag tttgattagt tttttgtttt cgaaatagaa aacgatatta taaagttatt 206820
atattgaaga agtaatgtta aaaatgtggg agaaatagaa ttttggagat atgattgata 206880
gttgattggt attttttttgt atgtagtgat gtttgtgata tttaatagtt gtttataatt 206940
tataatttgt tgttatttat tttttttattt taaatgaatt attgtattta tatttttaga 207000
gggttgtata agtttatatt attttgattt tttgttgatt gtttttttgt tttttgtttg 207060
gtttttattt tttttattat ttgtgtaatt agtttttcgt ttttaagttt ttttgtttta 207120
aatgtttaag tggattaggt attgtggttt tcgtttgtaa ttttagtatt ttgggaggtt 207180
taggtaggag gattatataa atttaggagt ataagattaa tttgggtaat atagtaagat 207240
tttgttttta tatatatata aaaaatataa aaattagtaa ggcgtgatgg tatatgtttg 207300
tagttttagt attttgggag gttgaggtag gaggattgtt tgaggttggg agtttaggat 207360
tagtttgggt aataaagtaa gattttgttt ttataaaaat aaaagaaaaa attaggtagg 207420
tatatagtgt gtatttatag ttcgagttat ttgggaggtt gagaagggag gatcgtttga 207480
gtttaggagt ttaaggttgt tatgagttgt gattatatta ttgtatttta gtttgcgtga 207540
tagagtgaga ttttgttttg ggaaaaataa ataaataaat aaataaataa ataaataaat 207600
aaatgtttaa gcggttttta tttttggtt agattttatt atatatttta tgaataaatc 207660
gtatgtatga gaaaggtgag tagatgaata gggtagggtt agggggttaa ggggaagatt 207720
atagattacg tggagcgaga ttagtggtta aaaattgtta attatagtta ggtttggtgg 207780
tttatgaagg taattttaac ggtcgggttt ggtggtttat gttcgtaatt ttagtatttt 207840
gggaggtcga cgcgagttta ttatttgagg ttaagagttc gagattagtt cggttaatgt 207900
agggaaattt cgtttttatt aaaaatatta aaattggtta ggtatggtgg cgggcgtttg 207960
tagttttagt tatttgggag gttgaagtag gagaattggt tgaattcggg aggcggaggt 208020
tgtagtgagt tgggatttta ttattgtatt ttagtttggg cgatagagcg agattttatt 208080
ttaaaaaatt taaaaaaaaa aaaataaaaa attgtagatt ttaataagga agagttatag 208140
gacgatattt taggatgcgg gaaaaggtta cgttagtttt ggattttttt tgattaatgg 208200
```

gtattttagt tattaaaggg gtagatgcgt tttatttaat ttttatttag ttattagagt 208260
ataatatagg gatgtgttat agatagagat ggagattaat aggtagaggg gtgatgggga 208320
ggaagattga ggtaagagga ataaaacggg gattgtagag ggtaggtggt aggagtttga 208380
gggttttaga agaaattgtt gtataagtaa tgagttagta gggtgagtga cggttaggag 208440
aggtggttta agaaaataaa gagatatttt cgggggtata gatgcgtttt gaggggtcga 208500
ggaagagatg gggaaggggt ggttttgtgg attttcggta tgattgtcgt ggtgaaaagg 208560
aattaaagat ttcgataagg ggatgatttt ttaaataagg aagttttggg ttttcgtagg 208620
gggattagta gattgagggt aaacggtatt ttgtttatta aagttttaat aaaatgttaa 208680
ggtaagaata tttgtttggt tttgggggta attttagaaa tattagaaaa gggatttatt 208740
atgcgtaatt tttttttaat tatttggaga aatgtatggt tatttaagat agagaaaggt 208800
agttaaagta agtggaggtt aggtatagtg atttacgttt gtatttttag cgttttggga 208860
ggttgaggta ggaggatcgt ttgaggttgg aaatttagta ttagtttagg taataataaa 208920
ataagatttt gtcgttataa aaataaaaat aaaaattttt tgagacggag ttttattttt 208980
tttgtttagg ttggagtgtc gtggtacgat tttagtttat tgtaatttc gttttttaga 209040
tttaagtaat ttttttgttt tagtttttttg agtagttggg attataggta tgtgttatta 209100
tgtttagtta attttgtagt tttagtagag atagggtttt tttatgttgg ttaggttggt 209160
ttcgaatttt cgattttttt ggttttttaa agtgttagga ttataggtat gagttatcgc 209220
gttcggttgt tataaaaatt ttttaaaaat tagttaggta tgttaggcgc ggtggttttt 209280
atttgtaatt ttagtatttt gggaggttaa ggtaggtgga ttacgaggtt aggagtttga 209340
gattagtttg attaatatgg tgaaatttcg tttttattaa aaatataaaa attagttagg 209400
tgtggcggta tatgtttgta atcgtagtta tttaggaggt tgaggtagga gaattgtttt 209460
aatttaggag tcgggggttg tagtgagtcg agattgtatt acggtatttt agtttgggta 209520
atagagtaag attttgtttt aaaaaaaaaa aaaaaaaaaa aattagttag gtatgtagcg 209580
tatatttgta gttttagtta tttaggaggt tgagaaggga ggattaattg aaattgggag 209640
gtcgagattg ggaggttatg attacgttat tgtattttaa tttgggtaat atagtaagat 209700
tttgtttaat aataataata ataaataatg taagtgggta taagaattta tttatttatt 209760
tatttattta tttatttatg agatagggtt ttattttgtc gtttaggttg gagtgtattg 209820
gcgtaatttc ggtttattgt aagtttcgtt tttcgggttt atgttatttt tttgttttag 209880
tttttcgagt agttgggatt ataggcgttc gttattgtat ttagttaatt ttttgtattt 209940
ttagtagaga cggggtttta ttatggtttc gatttttttga ttttgtgatt tgttcgtttc 210000
ggttttttaa agtgttggga ttataggtgt gagttatcgt tttcggtttt aagaatttat 210060
tttattttag tattttggag gttgaggcga gtggattacg aggttagaag attgagatta 210120
ttttggttaa tatggtgaaa tttcgttttt attaaaaaaa tataaaaaat tagtcgggcg 210180
tgggggcggg tgtttgtagt tttagttatg cgggatgttg aggtaggaga atggtgtgaa 210240
ttcgggaggc ggagtttgta gtgagcggag atcgcgttat tgtattttag tttgggtaat 210300
agagcgagat tttattttaa aaaaaaaaaa aaaagaattt attttaagat agtaagtttt 210360
agggttgaag ttagttttta tttagtttta ggtttaaagt tataagtatg gttattgtaa 210420
tatttttatt taggatatta ttttttaggg gtttagattt atattattaa ggaggagcgt 210480
ttatattagt agttggggag agggtggttt tttataagat gtaatgttta ttttgaagag 210540
ttattatagt gataattatg aaatttatgt ttgagattta attttaagtg aaaagaatag 210600
aatttaatat ttattttatg gggagtataa aattttgaaa atatacgttt tttataatta 210660
aattaaattt tatgttatgt ttttgaggag ataagtattg aatggaatat agaagaaatg 210720
ttatagttaa tgattgttga agtttttttt ttaatttttt gtttatgttg tttggtaata 210780
agtaatatag aaaattgata ataaaaagta aaagagaaag aaaagatata gtgtatagtt 210840
tagattgttt tttttaaaaa attaaataat aataataaaa agtataggtc gggtgcggtg 210900
gtttacgttt gtaattttag tattttggga ggttaaggcg ggcggattat ttgaggtcgg 210960
gagttcgaga ttagtttgat taatatgggg atatttcgtt tgtattaaaa atataaaatt 211020
agtcgggtat ggtgacgtag gtttgtaatt ttagttattt gggaggttga ggtaagagaa 211080
tcgtttgaat tcgggaggtg gaggttgtag tgagttgaga tcgcgttttt gtattttagt 211140
ttgggtaata agagcgaaat tttattttaa aaaaaaaaaa aaaaaagtat aaatgtaagt 211200
tgggcgcggt agtttatgtt tgtaatttta gtattttggg aggtcggggt aggtggatta 211260
tttgaggtta ggagtttgaa attaattcgg ttaatatggc gaaattttat ttttattaaa 211320
aatataaaaa ttagttaggt atggtggcgc gtgtttgtaa ttttagttat ttaggaggtt 211380
gaggtaggag aattgtttga atttaggagg tagaggttgt agtgagttaa gattgagtta 211440
ttatatttta gtttgggtga tagagtgaga tttcgtttta aaaattaaat taaattaaat 211500
taaataaaat aaaaaaaaga agcggtataa tataaagttt cggtagttaa agcgttaatt 211560
tttagttatt aggaaggttt tgttatttaa tgatttatcg ttttagttaa ggttagttat 211620
attttgggtt tttgatgttg gaggtaatat gatgttaata ttggcgagaa atttattatt 211680

```
tttttttttt cgtttttcgg tgtcggtttt tgtgtgtatt tgtagttttt ttattatcgg 211740
tgtttttatg gtgtttacgt tatcggttta ttttttagta ataattcgat agttcgatat 211800
ttttgtagat tttgtggtta tagagttaaa gttttttgtt attagtgggt tttgtagata 211860
attttttaaa tttttttggg ttgagttagt taagttttta ataagttgga agatgatagg 211920
ggttggatgt aatttatttt taattggttt gggttatatg gatattattt tttttttggg 211980
atttgatttg ggatggaaat aggagttata gtttatggga aggttgtgaa aggaagtttc 212040
ggttttaggg tgcggattag gttgatttgc ggaataaggt tttaggggcg gtttattcgt 212100
ttgttttgtt tagttttttt tggtaattat atttattcgg ggtttatttt aaatttggta 212160
ggtaatgttg ttgtttttat tttagtcgtt gttttaatac gtttcgtttg gttaggatag 212220
gtgtttaggt atggtattta gggatatttt cggttttcgg ggtttaattt ttggttttta 212280
ggtgtagcgt tgggaaaagt agagggtttc ggttatggcg taggtttcgt tataggttat 212340
ttaagtagtt tgggaggagt cggaaagaga gtaatgtatt tatttggata gagattgaag 212400
ttatattttt attgagaaaa gatgatattt ttgtttggat tttttttttg aaattttagg 212460
tcggggtggg gtaggagtta gcgagagagt ttagcgtttt aaaggggatt ttttggtttt 212520
tcgtgttttt taatttagta tttttgggga tttatatatt tagggtagag tggttattta 212580
gggttttttt tatttagata taaaggttat tgaaggtttc gggttaatat cgttttgagg 212640
atgattagcg atttgagttt ttgttagaga tagtaggtag tttgggttta agggtcgttt 212700
agtgatttga agttgggatt attttatatt ttttgcgtta tttttgtttt ttgttttgga 212760
ggtttttagg ttgttaatag ttatgttttg gagaattagg ttagattagg ttgggaataa 212820
agtttagttg ttatagagtt gtcgagaggt tattggtaag tagtgtttag gagtcgtttt 212880
tttttttgtta gtaatagttt aatgtttttt tttatagggg ttgtaaatgg gtttatgttt 212940
agggtggggt tagggagata aagtagagag aaattgggtt gggagtagag agtgttatgt 213000
tttattttgg agatttaagg ttaggttatt ttgtagagtt ggtgtttaga gtttttagcg 213060
tataggagag gaggtaaata gaatttgata tttagtagcg ttcggtagac gtttttggaa 213120
tgaatgatta aatttatatg gagagttgaa atgggttagg aattttaggg agacgggtag 213180
tttatagttt.agtagggtcg ttttagtaga gtaggttaat gttaggtttt ttggtaaatt 213240
tttcggggtt taggtataat tgttatatta ggaaaggggt aggggtgatt agtttatcgg 213300
ttacgggggt attttattgt ttttatagaa gttcggtatt tataggtttg ggtgatatac 213360
gatttttatg tttggtatta cgatggttgt ttattaagtt tttttattta ttaggattga 213420
ttttttttgg ttagcgattt tagattatta tgatttggta gttgtcgtta ggtttgtttg 213480
gttaaggatt ttttcggaga ttgagttatt ttatatagta aagtgagtta tggggttata 213540
gcgttgtgtg ttgtgtataa gtttcgaggg ttttatttag ggattggtat ttattgattt 213600
ttataaattt ttggtaatcg tttagagttg ggatgttttt aaggtaatag ttttaataaa 213660
tagaggcgta ggaatttagc gcgttatttt tcgttttatt ttaaaggttt atatttttag 213720
gggtttggag tttttttttg gagttttttt tattttaaag ttagttggat ttgtttagga 213780
attgttatta ttattttttg gtcgggtttg tattgatgtg tattgaattt tttttattag 213840
gtttatttta ggtaaatttt agagtttacg taatattgtt tgtggataaa tattggggcg 213900
gaaatagggg ttatttttag gttttagtta tatggggttg ttagggttgt ttgggatggt 213960
aggagagtgt tagaggaggg tagtttaagg attaggtagg gggtgggaag tgtttttttt 214020
tatgggtttt taagtgagat gtttttgta aatatttatt tgggattttt aagtagaggg 214080
ttggttttta tgttatttgg atttggattt tagattattg tttttaagtg gtaattttgg 214140
gtttttttgt atttgttttg gtttgagttg gttatatttg tggggggtgg ttttaggaag 214200
aaggtggatt gtcgtaaacg taatgtttcg ttgttaggtg tattgttttc ggttattaga 214260
atttttttgg gtaggtcggg gagatttgac gggtaagtgt ttgcggtttg gcgtgagcgg 214320
gtagggtagg tggtttttgt ttgggtatgt ttttttattt ttgtaataga ttagggagag 214380
ggttggtttt aggatttagg tttagttagt tgttttataa ttgaggggtt tcggcggagg 214440
atataagtta gatagagtta tagattaatc gtacgaggtg gttttttttt tttagtttag 214500
gtttttttgaa tgcgttagag tagataagtt tggacgtgag gaggtaagag gtagcgagaa 214560
taggcgcgga gacggggttg ggggagaaga ggaagaaatt ttgtgtttgt gtttttttta 214620
cgttcgtttg ttatcgggtt agttgattgg ggaagtgttt tttgagggtg taattttttg 214680
tttttttcgt aagttagttt tcggggcgtt gggtgagggt gtagttttgt ttattacggc 214740
gagttagtat attgaattac ggtagaagtt gtttttttgtg tgggtgtagt tttttttttt 214800
gtttaggatt tagaagttac gatagtatag agttttttaa tcgtggttgg tgttttttta 214860
ggaatttttt gaaagttata gagaaatttt agagtggttg ggtacggtgg tttatgtttg 214920
tgattttagt attttgggag gttgaggcgg gtggattatt tgaggttcgg agttcgagat 214980
tagtttggtt agtatggtaa aattttattt ttattaaaaa atataaaaat tagttgggta 215040
cggtggtata tgtttgtaat tttagttatt taggaggttg aggtatgaga attgtttgaa 215100
tttgggaggt agaggttgta gtgaatcgag atcgtgttat tgtattttag tttgggtaat 215160
```

agagtaagat tttattttaa aaaaataaaa aataaaaaaa gaatttttag agtaattttt 215220
aggtgttagt aggggtgtga cgtaatttta gtttcgtttt tgtggtttat cggtttcggg 215280
taagtttttt tgtcgttgtt atttatagag agtttttagg ggagtttggg aatgacggta 215340
agttttatgt gatttagtat gtaaagggag ttttaaaatt tagaatttag agagttaggg 215400
cgggtatagt gtagttggtt ttagtgttga ggggttgggg gagagatttt gtattatgtt 215460
ttgattatag taagggaaga ttatggaggt ttttggaacg ttttagtaat ggcgtaattt 215520
tagttttttt tatttttttg ttaatgataa ttatgattat gatagtagtt aataggttat 215580
agagtaggta ttttttagtt tttttaaata tgaattcgtt taatttttat aataatatag 215640
tattgtttta tttaataaat gaggtaggtt gggcgtggtg gtttatgttt gtaattatag 215700
tattttggga ggttaaggta ggtagattac gaggttagga gttcgagatt agtttgatta 215760
atatggtgaa attttatttt tagtaaaaat ataaaaatta gttgggcgta gtggtacgtt 215820
tttataattt tagttattta ggaggttgag gtaggagaat tatttgaatt tgggaggcgg 215880
aggttgtagt aagttgagat tatgttattg tattttagtt tgggagatag agtgagattt 215940
cgttttaaaa ataaataaat aaataaaatt aaaaaatatt atatatataa aaaaataaat 216000
gaggtaaatg agatgtatga ggtgagaatt ttgtgttata gggagaggcg tagtttggtg 216060
agatttaatt ttaaggggtt ggcggttttg agtttttaag tgtaattatt ggattatatt 216120
gtttgtagat tattattatt tttattttat tggaaggtcg ggtgaggtaa tcgggaaagt 216180
ttggagtagt agtggagttg ttagagagaa tttgttttta gtgttttttt ggattttgta 216240
gttgttttgt gtgggttttt gttgtttttc gtattggaaa agattttttt atagatgggg 216300
ttttatgacg ttatttaggt tggagtgtcg tgtttatttt taggcgttgt tatagggttg 216360
tagttttgaa tttttggttt taagcgattt ttttgtttta gtttttttgag tagtttttttg 216420
ggtttaagcg atttcgtgt tttagtttttt tgagtagttt tttgggttta agcgattttc 216480
gtgttttagt tttttgagta gttgggattt taagcgtatt tggttaagtt ttttttttatt 216540
ttttattatt taagattttt ttttttttaa ggtttagttt aattttattt ttatttaaaa 216600
ttttcggggt atttttgtag tatttggagg ttgaattgtt taggagggaa gggtgttaat 216660
gagttggtgt ttcgttttta ttttgatttt cggtgagaat gtaagttttt tgtaggtaag 216720
gattatattt tattattatt tttgtattta tttagatttg gttatgtgtg tttagtatat 216780
aaagtgtttt cgatgattgt ttatagattt acggtatttg agattgtttt aatatatttt 216840
tttaaaattt tttttttttt tagaggttta cgaatgttta aattaggttt atcgggaata 216900
atattatggt tatttcgttt attttgaagt ttatatatag gtttggaagt aataatttcg 216960
tatagtttag atgaaaaaat aaagattttt ttgttgtttt tggattgtta gagttttggg 217020
ggatttttaa tatttttttgt tgtttttttt aattttatgg tgtcgatggt ttgttggggg 217080
gttagtttga tttttgcgag tgttcgtatt cggaaaaggg ttggtgttaa gatagtttta 217140
ggatatttag aatgagggta agaggttatt tttattatta gttttttttta gttttatttt 217200
atataattgt aatatagtga tttagtatat tttgggttat tgttaagtag ggagtaaatt 217260
agaaatggtt tgagtttttt ttttttttat ttttagaggg gtttgttttt ttaggtaaat 217320
ataaggaggt atcgaggttg ttgtataaga gttggttttt gtttatttta taaattttat 217380
ttttatttat tgtaaaaggt tttgtttttt tttttaaaaa aaaaaaaaaa aaagaaattt 217440
agtgaggggt ggggtaggtg tgtgtggagg agaggagaaa aggataagtg atggtttttt 217500
tatttttttag taagtcgtag gtgtagagat tttaagtttg ttttagggtt ttttcggtat 217560
ttgtgtagga gggttttcgt agaaaaaatt taggatttac gatttgggag gatttcggtt 217620
atttttattg tttggattta ttagtgggag gtaaagtgta agaaattaag ggtaaagggt 217680
ttttttttggg cgggttagag atttttttgga aagggttatg tatcgcgcga tttttataat 217740
aattttaagt atttggagta tgttggggag gaagtttcgg tgttaaaatt aagtatgtgt 217800
tatttggaag ttcgttttcg tttattagta tattaattta tttacgtttt tgatagagtt 217860
gtgtttcggg tgcgtagagg gttattttgt tcgtaataat cgggtgaaag ttgaaatttt 217920
aagttattga tttagaaggg tattttcggg gaagttttga gtaatttttt ttgagataag 217980
gtattttatt atttattttg gtttttaaat atggaaagtt agaggaaatc gataatggtt 218040
taaaggtagg gggatggttt cgatgatttt tttatttttta attaaggttt atgatttgt 218100
tttttttatta ataaaaatta atttatggat ttttggtagg tatttttttta tttgaatgaa 218160
ttaaaggagg gatattaaat aaacggtttt tagatttaga agaggagacg ttatttataa 218220
tttgaggatt gaggaggata aaggattttg tttgtttgga ttttttggag aaaattataa 218280
aggaaaaaaa aaaggaagga aaagataaat aatgaagatt tggatttttt ttaatttgtt 218340
tttgtggttt cggtgtgttt atttgtgaaa tggaatttta gagtgttaga gcgggagttt 218400
tttatgaaat gaaagttttt gaatcggttt atggtgaggt ttaagttagg ttttttttcgt 218460
ttatttaagg cgggttttgt tagggtggag tgaggatgtt agtcgcgggc ggcgttttga 218520
gcgtaaaacg tgaattcgag tcgttaagtt tgggcgttgt tggacggttt tatttggagg 218580
tcgtttcgtg ttttattaag tagaggagaa atcgaagaga attcgaagaa aggatgttgc 218640

```
ggtttggatt attagtttta gaggttgaat ttaaggatat ttttttattt agtttcgatt  218700
ttatttattt atatagacgt atagggattt aaagtattcg gtcgatttgg aaagttagcg  218760
atttttgcga gggtgtaagg ggcgaggaag agggagcgtt ttttagggta gtttttttcga  218820
tattcgagta agggttggag atagcgtagg ggacggtcgg tttaggtcga aagcggttcg  218880
atagcggttt tgttttggga ggaattcgat ttgtagtgtg ttcgtcgcgg ggattcgttt  218940
agtttcgggg gcgtttcgtt attttcggtc gtaggtattt aggtgagtag cgttggtttc  219000
gggatatcga ttttttttta tttcgggcgc gaagtttatt tgagtaagat ttttttatgg  219060
tgttttcgtg gtcgtcgttt ttaggaaagt gtggcggcga ggtagcgggg cgcgcgtttt  219120
tagggcgtta atttgggtc ggtagtagga aggcgggaag agggggcggt tcgtcgtttg  219180
gagtgtatcg atcgggtttc gtttcggttt cgttttcgtt tcgttttttt tcggagatgc  219240
gtatagaatt tcggtttagt cggttgtggt ttcgtttttg attcgttttt ttttcgtttc  219300
gtgaacgtgc gcgtcgagtt ttattttcgt ttatttttgc gttgcgttgt atttggcgga  219360
gcggggttgg cgggcgtcgg gtttgggagg gggcgttttc gcgcggtagc gtagattaga  219420
ttagattagg ttaggtcggg ggtgggggtc gtttgcgtgg ttcgtcgcgt tcgtagtttt  219480
gtatttttag tttttcgcgt tggcgttaat ggttggtttc gggagtcgtc gtattttta  219540
gtttcgagtt cgggcggggc gggttcggtt aggtggaagc gggcggaggt gcgggcgttt  219600
cgggcggttt cgtcgggcga ggcggcggcg tcgggtatcg cggcggcgtc gggtatagtc  219660
gggggtcgga gtcggggtgt cggtttagtg ggtcggggta gggcgggata ggatatttgt  219720
tttgttattt ttttggtaat taaaagggta gagtgcgtcg attgagttgg agtttagtt  219780
tttttatttg taaatgggcg cgttggatag atgattattt gttttttttg cgttgggtat  219840
cgtgcgtagt tcggtgtatc ggtgtagcgg ggtagatata gggagtcgtt ttttataatt  219900
cgggtaacgt aggtagtggt gagaggatgt aaaatttgta cggaagaatt ttttttattt  219960
ggatattttt aaagttttta cgataaagcg gtgtttttaa cgtatagagg aagaagtgat  220020
taattatgat tggagggtgg tgttagagtt ttattgaggc gaaattaaaa tagggtttta  220080
aggaatgagt aggaattagt taggtagtta aggtgatgga aggtagttgt tttcgaaaga  220140
gggaagggcg tggcggagtt tgggaggttt gaggtattat agcgagtgtg gagagataga  220200
ggtagggtcg ggtttttgta tagttgttaa tggaggtcgt agaggacgag aggaaggcgg  220260
gaggttagtt gggaagcgtt cgttagtttt gggtaggggc gtagtattgt ttttttttgt  220320
agtttttta aaaatgtata ggaggttgaa agtgttatta atagtttcga aaaatttttt  220380
ttttttttt ttttttttta ttttttttgg gggtttaaga agtattttta ttttttgttt  220440
gagtgtggga aatttatatg tatgagaacg gaggtttatg tggtatcgtg ggttcgtttc  220500
ggtttatttg tttttttttt tgagtaattt gtttagttat agttttgtag agggagtagt  220560
ttagttagag gagttttttt ttattttagg tagagttgat tggatcggag tggatattgg  220620
atttaattg agtttatttt tttttttatt tgggggttag aggtttagga ggtttcggga  220680
gggaagtgtt gtaagggagg ggagtaggga gtatggacga attgtgaata gcgttttgga  220740
ttttgggatg tttatatttt aatcgtaatt tttatttata cgtttattta tcgtttgtat  220800
tagtattagt agatttcggt ttaggtcgtg ttttgatttg gttagtaagt attatgatga  220860
tattgttttt agtttagtta tgatgatatt gttacgagtt taatgtttgg gatttttaaa  220920
gtttagcggt gttttaagaa tttgggttat gggtgtattg ttggttttgt ttattttatt  220980
tagtataagt tagaggtgtt ttggagtata gttattttga ggatgttagt tttggttgtt  221040
ttttggtttt gggtaggtgg gttttttttgc ggatgtttac gtttttgttt ggggttataa  221100
tttataggaa tttgggtatt taggatttaa ggatatttgc gtggagtgat gttggatata  221160
ttatatgggt gttttttgtg tgatggtttt tggatattcg aattttttttt tcgattttaa  221220
gaaattagaa agaaggataa gagagaggga gggaaggttg ggaatgaggg ttttatatat  221280
ttttgagatg tatgaagttt aatttgttat gaaattagag ttaaaaaaaa aaaagaaaaa  221340
ttaaagagat gtacgaagtt taattgttta agtttaaat tttgttttta tttatttatt  221400
tttatttttt attttttgag agggagtttt gttttcgtcg tcgaggttgg agtgtaatgg  221460
tgcgatttta gtttattgta atttttgttt tttaggttta agtgattttt tagttttagt  221520
tttttgagta gttgagatta taggtgttcg ttatatttgg ttaatttttt ttttttttgta  221580
attttttttt tttttagtag agatagggtt ttattatgtt ggttaggttg gttttaaatt  221640
tttaatttga ggtgattttt tcgttttagt tttttaaagt gttgggatta taggtgtgag  221700
ttatcgtatt tggtttattt atttatttat gagatagagt tttttttttgt tatttaggtt  221760
agagtgtagt ggtatgattt tagtttatta taacgtttat ttttcggatt taagtaattt  221820
ttttgtttta gtttttttaag tagttgggat tataggtata cgttattata atcggttaat  221880
ttttgtattt ttagtagaga cggggtttt                                   221909
```

<210> 7
<211> 52626
<212> DNA
<213> Artificial Sequence

<220>
<223> chemically treated genomic DNA (Homo sapiens)

<400> 7

ggaggaagga gaaggagttt attgtaaata ataataatta aatagtttgg gttgggttag 60
taaagggtgt tttgggagtg tttggttatt tttgtatttt attttgtttt atgtttgggt 120
ttttatattt ttttttatt tgtgtttttt tttttttaaa aaagatgtaa gtgtaataat 180
ttattttaaa aggtaagaat gttttttta atattttgtt ataatgtagt tatatggtgg 240
tataggtagt aaaattttat ggaattgatt ttattttttt ttttatattt ttttggattt 300
ttattgtatt tttaatatat atttttaata ttttatttta ggttattaag ttaaaggaaa 360
agttgtattt atatagggtt aaaatatttt ataatgagaa taataagtaa tggttgaggt 420
ttatgttttt tttagtattt agttttttt ttttaattat tgtatattta aatagatgat 480
tagatattga aaattgtttt ttaaaattag tagtataaag gtttagtttt atgtgtgtga 540
gtgaagaggg aagaaaggag aggttgaggt taggttatgg aagtattttt ttttttttata 600
gtattttgaa agaagtgaag tggggttgat gggttattgt tattttttta tttttaatt 660
ggatagttat agagtattat tttgggggtt agtttattaa ttattttttt ttagttaggt 720
ttgtttgttt ttgtattgga tagtaagggt tatgaaatag attaggaatt tttgtgtgtt 780
ttttaggttt ttgaagatgg tttttattg tttagtttag ggagggaatg attgtataag 840
tagggtatgt agtttttatg gaagtgtttt ttgtttgttt agttgaaatg ataggagatg 900
gggtatggag ttgggtttag tgtgattata gttgtgtgtg ttggggattt attttatatt 960
tgttgtattt attttattgt agtgtttttg agttaatttg gttattgtgt tttttgaagt 1020
tggaggtatg gtagtgagtg ttgagaatta agtatagtta aaagaattta aggaaagttt 1080
taaaagttta tgttttttaa gtgttatttt agttttggag ataagttttt ttgtgtgttt 1140
tttagagttt ttgtttttat gttttaggag tagtgttggt atttggaggg tagtagtagt 1200
attttagtgt taagttttgt tatgttgaga tttgtgttta gtagggattg gaggttgtta 1260
gtgtggggag gttgtttatt tatttgttgg ttatttatga ttttattttt aagttgttat 1320
ttttgttgag attttaggta ggtatttaaa ggaaattttt atttttttta gtttatttgt 1380
atagtaatga attatgtggt aggtagatgt tttttttat ttgtttttat gagttttagt 1440
tttagatggt tttgggtagt tgttttgatt tgtttagtta ttttaggagt tgatatggta 1500
gtggtttttt gttttgtagt gtagaaggtt gggtttagtt atttgttttg gaagggattg 1560
ggttttaagg tttttttttg gagaattgag ttgttgagtg aatgtttatg aggtattgga 1620
gaggggagtt ttatgtagta ttggttgttt ttggtgttaa tgaagtttag gtttttatat 1680
tttgggtggt atgttttagg tttttttttt agtttagttt tagggtaagt ataggttagg 1740
gtagttatgt ttttattttg ggttagggtt tttttttgttg tgaagggatt ggattaatta 1800
gagggttttt aagtttttt ttaatttggt ggttttatta tatttagtgt ggttaggtgg 1860
tttttaaggg ttgggttaag ttatagagga taggtttggg gtatgtggtt gtttttgatg 1920
agatgaatag gaatgtttta tattggtatg tttaatttta gatattttt gggtgtgttg 1980
ggataggtag ttatagttat agagtttttt aatagatgtt tttttttttt aagttttggg 2040
gttgaaattt gtttgatttg ggtttagttt agttagagag tagatgtatg gaggaattga 2100
gtgttggggg ttattgtgga agagtttttt taaagttttt attttttttt tttgtttgtt 2160
ttggagttat ttgtttgttt tttttttgt tttgattttt ttgtttttt ttttggttta 2220
ttttggagtt atttggtttt tttttttttt gttttgattt ttttatgggt gagaaagatt 2280
taaggtgaaa gtttttttt tttttatttt tttttagtta tgagtatttt agaaggaaag 2340
gggggagaaa tggggaaatt gtttttagtt ttaagttttt tttttttttg gttagatttt 2400
gggggagttt gggttatttt agtgaatttt agaattagaa aaaatttttt aaaagttatt 2460
ttaaattttt aaatatttga aatatttaaa gttaagtttt taagaatatt taatttaatg 2520
tttaagattt ttttattttt ataagattta ttttagagtt atggtttata aagaggtttg 2580
atttattaag ggaggttata gttagatttt tttttagttt ggtttttaga gtagatgaga 2640
atattaggta ggtgggagat tgttattttt aattttagag atttttattt aataatatta 2700
agtttatttt ttgtttaatt taggaggttt ttttgtgttt atgattttga gtatagtggg 2760
tttggtttta ttttatgata ggaattaagg gagaaggtag aaaaggtgag tgttttaatg 2820
tggggtgttg tttgttggag ggatttttta tttggggttt gataggtttg tttttggatg 2880
gttattgtgt atttttagtt gaggtgaggg ttggggttta gggttagatt taagttatag 2940
gggttgggta ggtaggtata gagttttgtt ttttggaggt aggaagtttg gttttaggag 3000

ggtattttga tagttttatt tgtaatagta aagttatatt gaggagttta ggttaggagt 3060
tttttgtag gttggagaga gggtagagag ttttggtttt tgtggaagaa gtgtaaggat 3120
ggagtgtgga aagttgtgtg gagaagagta atattttta gtgtttggag ggatatagag 3180
ttttatttg ttgtggaagg ttttgggtag tgatgatatt gatagatatg gtagggagga 3240
tagttagttt gaggttatag tagggtttgg aggttgagta tggttatatg tttttagta 3300
aagtttgtat gtagtttttg atttttgtt atttggtttt taagttttag tttatgtgtt 3360
ttgaagttgt gagtttttt atgttgggat attgttgagt agttttaggg aggggttagg 3420
gtagggtagg gaagagatga gttttttgt atttttttgg agggaatttg attgggtttt 3480
ggatggagaa taaagttgtt tagatttttt tgttgtatta gatgttaggt aggttggaga 3540
gtgggaggat tgtttgggtt tatgttttat ttggatgttt ttttttttt taggattttt 3600
ttttttta gtattatggg agtttttgtg gtatttgtgt ttttagtttt ttttaggttt 3660
ggtttttttt ttatagtgtt tagtgagggg ttggtttgtg tttttttgg ggattggtat 3720
tgttagatgg tagtaggggt ttttggtttt agtttttgtt atttttgtt tattttttt 3780
aggtgtattt agtttaggg atagttagga tgtagttagt taggttttgt tagatttgtt 3840
attgtttggg gggaggtgga ggttggtgtt ttggttttag taggttggat tttgtgtgtt 3900
tttagggaga gaaagatggg agggtaggag gttggttttg tagggtagtt tgtgttttag 3960
atatatagtt attggtttgg tgattagggg attttattag gagtgggaag gatcgataag 4020
attgggttgg tgaggttgtt gggtttgtat ggaaatttag tattgattta tggttaatta 4080
ggtagagtag aggagtgggt gggagtaaga aagtgttttt agtttattat ggatgtgaag 4140
ttgaaaaatg tggttatttg gttttttta ttgtttgtgg ttgggagtag gggttagggt 4200
tttagtgtta gggaggggtt aggggtttgt tgttttttt tttttagtag tttatggggt 4260
tttagatttg gggtggttga aagtgttgag ggttaggttt ttggtttagg ttaggttttt 4320
aagaggaaag ttgagtggga ggttggggt ttggaaaata tgggtgtggt ttatggtgtt 4380
ttgaggtggg gatggggtag gggtgttttg ttatgtggtt taggaaatgg ttgtattgtg 4440
tttttggtt tttatgtgga gggttgtgat gtttgaggtt ttgtggtttg gtttgttgtt 4500
gagtttgtag ttattgaggt ttgggttgag gttggttgtt ttaaataggt tgtttatgtg 4560
tgtttggttt atgtgattgt tagttttaga tatatttagg aagttggtga tgttgttggt 4620
tttagagtta ggggggtggg tatgagggga tatgtaggta ggtattgggt tgtaggggat 4680
tatgtaggtt ggtattggtg aggtagtttt gttgttgttg agttaggatg ggttttgaat 4740
ttgggagagg aagggagaga tgttattggt tggtgggtag gtgtggtgtg gaaggggttt 4800
gtttttttt ttagagtatt tttttttta ttgtttattt tttttatgaa gtttttttg 4860
agtgtttagt tttgatggat atttagatat ttttgtgggg gtggttttta tattttggta 4920
tttttgtttt tagggttagg ttagtagtag agggaaattt agttttaaag agtgatttta 4980
ggttttatta agggtttagt ggaagggtga tattttagta tttttagtt ggtttttat 5040
ttttagattt aattgtagat ttggttttta tttgtggatg gtagttgtgt tttatttatt 5100
tttagttttt ttttaggttt tttatattta taggtatttg agtaaggtga gtagatttta 5160
atttggagag ttttggtttt gtaggttttt ttgggttttt attagtagta gggaagatgg 5220
aggggttggg tgaggtgggt ggtagggatt taaattttt agaaattaag ggttagtggt 5280
tgaggtttta gggtttgaat taggaggttt tagaattaat ttaggatgga aggggttgtt 5340
ttaggagtgg atagttagat ttgagattgg attttttgt gttaaagggg tgagggtatg 5400
tgtagggatg agttggttat tttatttttt atttgtgtta ttgggttttt ttttttttt 5460
taatgtttat ttggggtttt tattttttt tatttttag gtatgtataa ggaggtagaa 5520
ttagggtgtg ggggagattt gggaattttt tagtttatgt atatgggatt tttggtaatt 5580
ttatgtgggt ttttgttggt ttgttttttt gtatgtgttt ggagtatatt tgagggaggt 5640
attttaaagt agggagaaag agtttagttt ttattttgga gggaattgta ttttttttg 5700
tggtgtaggg gaagggggat tggttggttt ttattatttt tttttagaag aggtttgagt 5760
atatattatg tggtgttgta ggtgaggtgg gggtaggagg ggtgaatagg gttttgtgtt 5820
gggttttttt tttagttgtg tgtttaggta gtagagttta aagttaagga gggagttaga 5880
gttttatttt gggaggatag agggaggttg agtttaggga gggagagggg agaaagagaa 5940
tggaagaata tagtgtgagt tgatgttgat ggtgtttgtg tgagatattt tatatttttt 6000
agttttattt ttaggtttgt gttttgtttt tttgttttat gaattttggg ttgggggaga 6060
gggaatagag ggttggtttg gttggagagt tagagattat ttgttagtat agaattaggt 6120
tagggagaag taaaggaggt aggggtgttt gtgttattgg ttggttgttg agtagggtta 6180
ggttgggagt tgaggtggag gttatgttag aatgaggttt gagtaggtag ggagtagttg 6240
tttttgttgg gtaggttgag tggggggttag gagaggattt ggatttggtt aaaaatatat 6300
ttttttaagt attttatgtg tttgatgtga tttaatattt tttattttt gtattttata 6360
ttagaatagg ggattggttg atagatattt agggaaaatg ttttgggggg aggggaggat 6420
agggttttag aagttgagta taggtatata agtttttga aattgtataa tttataaaat 6480

atttttgtat gattgaattt agattttata attgtttgat aatagagttg agtagatgtt 6540
tttattatgt ttaaggtata ggtgaagaaa ttgaggttta gagatttgag atttgtttaa 6600
agtgagaaag atttggagtt ggaaagataa tttaagtttt tggatttaga attttaagtt 6660
tattttaatt tggttatgag ttttggagat tttagagttt gtttttgaa gagatttttt 6720
gagtggtatt tgagggtttg gtggtttttt gtttgttttg gttttgatgt ttatagtaaa 6780
agtggttaaa gttagtggag gaaagagttg tttttattgt tttaattttt tttttttaa 6840
agtttagtta agtttggttt aatgattagt tggatagatt aattttgggt tttttatatt 6900
ttggtataaa taggtatatt tttggaatga gatggaaggg tagtttggag ttataagtta 6960
ttttggggtg tttgggtttt tttttaaggg gtttattttt agagtattag gggttgggga 7020
ttggtggtgg tagtttaggg tgggatttat ttgggtgtag tttttagttt gggtgaggag 7080
gggtagttta tatgtagtgg agaagtgggt ttgaatttgt tgtatttgtt taaaatgttt 7140
ttgttttttt tatttggttt tttggttttg gaattagatt tataagatgt gaaaaagtta 7200
ttgttattag ggtgagatgt ttgtttggag gttttatttt taggtagtgt aaattgtttt 7260
tttgagtttt ttattagttg tagtgtgttt gggaaatggt gtggttatat ataaatatta 7320
atttggttt ttgtttttaa gagttgataa tttagaatta tttattttga gttgggaagg 7380
tattttattt ttattagtta tgggtgtgat tttgagttag aatttttatt tttttaagtt 7440
tgagaattta gagtagttaa gtgttattat ttataaaata agttattggt tattttagta 7500
gttatttaag tagatttttg tggagaagtt gataaaagtt ataaatgttt ttttagatgt 7560
gttttttag ataattatat atagtttgt tatagttttt taaaaggttt atggtttttt 7620
aaggtttatt ggtttagatt tataatgttt tttttttgt attttgttg tagttttttt 7680
gttggagata gtttttagt ttagaatttt tttgtgttaa agggagaata ggggttttgt 7740
tgggtaatag aggtagttag ggaagatttt ttggaggagg gataggagtt gggttttgaa 7800
tggttggttg aaaggaggaa agttaaggtt taggtttaga aatagtaagg tatattttaa 7860
aaatttaaag aggttaattt gaaggaagtg gtgggagaga aagaaataga gttagattgt 7920
agggggtttt gattgttaag tgaagggttt tttttttggt gttgtttgta gaggtagttg 7980
ttataaaatt ttgtgtagaa gtgtggtttg atggtgagag gaagtggagg ttaatttgtt 8040
attgagtata agtggatggg atgttggttt gattgggagt ttaatggggg aggggaggtt 8100
tttgagttag ttgtgagtat tggggttttt ggtgatgggg agtattgggt tttttttttt 8160
tgtatgagta tttttggaag gttgagaatt tttttaaggt ttaagagtaa gagttgtttt 8220
atttttggtt tatgatttag gatttggaga aggggtgatg gggatggggt gggtgagaga 8280
gggagagtga ggagggatta gtttttaggt taggaatggt tttaggttga tttagagatg 8340
gattgtggtg tttttttagag ggaggttttt gttagtttga tgtgtatttg tttgttggtt 8400
ttgtagattt agatgtaagt gggtatattt tagatagtat gttgtagatt gggagaatag 8460
agttatgata atagttttag ttgtagagtt ataaggagtt ttgttaggtt tttttaggtt 8520
taggtattaa gaaagttaat ttttttgagg tttagttttt gtttttttagg tggaaatatt 8580
tttttattta ttatgattaa tgtagttgtt gagttaaata taggagttag tttttaaaat 8640
tttttttgttt tagagttttt gagtttttta ggaattattg tgaggagggg agaaggaggt 8700
gtggatgtgt ttttttatag atgttgtttg gttttttttgg gtatttagtg tagttggtta 8760
atggataggt ttataggagt attggtattg ttaagggttt tagtttttgt atggtgaatg 8820
gtattttata gttgtagtag gggagataga tgggaaatgt agtttaagaa tgtttagagt 8880
atttttattt tgtttttttta tagtttttga ttttttttat tgtttttttgt tagaggaaag 8940
aggttaagga agatggggat gtattttatg ttattgtgag gagggtgggg gtgagtaggg 9000
gtgtatatta ttatgttttt ttatttttgt attaatttat tttttttttt ttaattttaa 9060
gtatgtgtgt gtgtgtatga gtgtatttat tagtgtggtt atatgtatgt gtatgtgtgt 9120
atatatattg attgggggag gagaagtttt tttttttagaa atggattttt tttttagttt 9180
ggggtttttag tttttgtttt ggttttttag ggtttttttt tttttaggga tttttagggt 9240
ttaggttttg gttgtttatt gggagttaag gatagatggg gttttttttt tgtgttgttg 9300
aaatgggatt ttttttttatt agtggtttgg gatgggagat tggggttagg atgagagaat 9360
gaattagagg agtttgtttt tattttatgt ttaatttttt gtaggtttag ttttgttttt 9420
ttgtttttttt tttagttttt tagggttttt tgtgggggaa gttgggggga ggggagaagt 9480
tattggaggt atggagagag atggtttttt tttttttttt tgttattgtg attgttgggg 9540
ttttttttgt gtttttgtgg gttttaggtt tttatggtgg gattgtggaa gtggaagatg 9600
tttttatagg ttttggaata gaggatttag ggatgggtgg ggagggattt tgaagaggga 9660
aggaggtggt tagtgtttgg taaaggggtt tagagtggag ggaggagtta tgtgggggag 9720
tggaatgtgg ggtgtgggtt tgttggagtt gagtgtgagg gaggaggaga tgtgggttat 9780
ttggggaaga tagatgatta ggtgggggag aggttagtag agttagtatt gtttttggaa 9840
taggagtgga agaaggttag gttgagggat ttatttgagg gttgaagatg gaaatggtga 9900
gaggtgggtg agtgattggt gggagtaggg ttgtttattt tttttttttt tttttttttg 9960

tttttttgtt ttggggtttt attttgtgta tttagtttg gttttttgt ttagatttag 10020
atatgagtta ggtgagtttg gttggttttt aatatttttt ttagtaaatt ggaggtagta 10080
agggtatttt tatgattttt tttatgttta tatttgtagg ttttgaggtt tggagtgttt 10140
atttattttt gatttttggt gttttagggg ttttgagagt tttttttag atgttatatt 10200
aatttttatg tttgtttttg tgttttttag ttttgtattg taggtttgtt tttattgtgt 10260
agttttagga ggatagttaa ttatgaggtt tttattgttt ttttggtttg aatgttatat 10320
tttaggatta ttttagtttg ggtttattta gtataggttt tttttgagga ttgggttgtg 10380
taaggaatta attgttttgg tggaggtttt gttaattgta gtttatttgg ggttagggtg 10440
gggtggttta gggtggggag gtttttgttt tattgttttt agatattagt tgtagagatt 10500
ggaattttag atagtagaga gaggtttttg tagtttttg gagtagggtt aaattttagg 10560
tttttttta ggaaaagttt attttggggt ttttattgtt ttgtttttgt ttttattatt 10620
atttatgtt aggtatttgg tttgttatta gttgagtatg ggttttgaat aaatatagta 10680
gaggtaggta gggggtttta ttattatttt tttatttttt tagggtattg ttaggtttag 10740
ggtttaattt agaggtttga tgtatatgaa gattttattt attttgttgt ttttatataa 10800
agaaaagttt ttgaataatt tggtatgtgt ttttgtatag taaattaaag ttaaagaagt 10860
attaggttgt gatttaggag ttttggattt tagttgtagt gttgttattg agtggttgtt 10920
gttaatttta ggttatgatt ttttatttat gggtttttt tttttgttt attaaatggg 10980
aagatggggt gattattttt agtattattg ttttatagtt ttgtggtttt tttattaggt 11040
gataagatat gtagtatgtt agagtttttt tttttgtttt ggttgttagg aagttttaag 11100
ttgaagtttg tgtttaatgg taattatgaa ttttagttta aatttttttt tattatatta 11160
tttttgtatt ttatgttatt gtttttgttt ttagattgaa taaataaatt tttaattgta 11220
agttatttta attttatttt ggaagtaggt ataagtgtgt gtgtgtgtgt gtgtatgtgt 11280
gtgtgtagag aatattttgt tttagttgta tggtggtaat tgaaaaatgt tttgtgtgtt 11340
tttgttttta tgttttgtat ttaaggtgtt attttttttt tagtatttag tgaattttat 11400
ttttgggtat ttttatatat ttgttaggaa gtatgtatgt aatttatata tgtaaatata 11460
aatgtatata tatattgtat agaaaaggaa taggtttatt ttatattaat atagtattta 11520
tagggttgtt ttataattag ttatgttttt tttttattt atatttgtgg gttttttta 11580
atttatttgt tggagattag gttagatgtt aggatttttg tttaaattta ggtatgtaag 11640
gttatgtgag tggttggttg tttaaattta agtgagattt gggtgtgtga gggtttttgg 11700
atttatagga aagttttagt tgttttgtta tttttttatta aatatttatt aggtattgga 11760
ggggaataaa atggtagttt tgttaggtgt agtggtttat gtttgtaatt ttagtatttt 11820
gggaggttga ggtggtggat tatttgaggt taggagttga agattagtta attaatatgg 11880
tgaaatttta tttttattaa atatataaaa attagttggg tgtggtggtg tatatttgta 11940
atttagtta tttaggaggt tgaggtagga gaattgtttg aatttgggag gtagaggttg 12000
tagtgagtta agattgtatt attgtatttt agtttgggtg atagaataag atttgttta 12060
aaaaaaaaaa aggtagtttg ggttgggtat ggtggtttat gtttgtaatt ttagtatttt 12120
gggagattaa agtaggtgta ttatttgagg ttatgagttt aagattaatt tggttaatat 12180
ggtgaaattt ttatttttat taaaaaataa aaaaaattag ttgggtgtag tggtgtatgt 12240
ttgtagttat agttatttgg gaggttgagg tatgagaatt gtttgaattt aggaggtgga 12300
ggttgtagtg agttaagatt atattattgt attttaattt gggtgatata gtgagatttt 12360
gttttaaaaa ataaaaaaat aaaaaaaaaa tggtaggttg tttttaggga atttatagta 12420
atttaattgt gtagtttttt agtattattt ttggtagttg tagtttttga atagttatat 12480
atttattgat aattattagt tttttttat attagttatt ttggaaaaaa agtttgtaga 12540
ttttattaga gatgtttttg tttttttaaa tagaaaatgg ttgtaattga tgaatttttt 12600
attgatgttt tagaataatg ttgttgaaga tgtttttatg agttaattta agagttttg 12660
gggtttgttg aggattgtag ggttggggta tttttgtttt aatattgaag ggtttagttt 12720
attgagttgt atggtgtttt gttgattaaa aaaaaaaaaa aaaaaaagat ttttttttgt 12780
tttgaagtta ttgatttttt tggtttattg gagagggaaa aattgttatg tggtaatgtt 12840
ttatttgtta tttttaatat gttttgggtt ggaaaagtgg attgattatg atatttattt 12900
tgtgttaaat tgaaatttaa atttgagtgt gtgaattttg ttatttgtat tttggttatt 12960
tatgggtttt tttttttttt ttgatttttt ttttgttgtg aatttatgtt ggttggggta 13020
gatatagttt tttggataag ggttgattga tttatggata tttatttttt agatttgatg 13080
ttttgtgttt ggtttttttt aattggtggg aatatagtga tgaataatta gtgtttgatt 13140
ttaaggaaat tatagtttag taggaggga taaataagta aaataatgta agttagttaa 13200
taattttgta aagaagataa aataggagtt gggtatagtg ttttatgttt ataattttag 13260
tagtatggga ggttgaggtg agggattatt tgaggttagg agtttgagat taatatggat 13320
aataaagtaa gattttattt ttataaaaaa taaaaaaatt tattttaggt gtggtggtgt 13380
atgtttgtgg ttttagttat ttaggaaatt gaggtgggag gatttattga gtttatgaag 13440

```
ttaaggttat agtgagttat gattatatta ttgtatttta tgttgggtga tagagagaga    13500
tttagttttt ttaaaaaaaa taaataaaaa tatttaaaag ataaaatggg tttaggtatg    13560
gtggtttatg tttataattt tagtattttg ggaggttgag gtgggatgat tattttagtt    13620
taggagtttg aggttgtagt gagttataat tattttattg tattttagtt tgggtgatag    13680
aatgagattt tgtttttaaa aagaaaataa aaaaaattta aaaaattttt aagaagataa    13740
aataggatag tgtaataaaa ggtgagttgg tttgtttttg ttgtgttggg ttgggttgag    13800
ttttattggg ttgtgttgag tgggttgagt gtgttaggtt ttattgtgtt gtgttgtgtt    13860
aggttgagtt ttgttggaat gtgttatatt gggttgtgtt gtgttggaag ggttgtatt    13920
aggtttagta tgttgagttt tattgtgttg tgttgggttg agttgagttt tattgggttg    13980
tgttgtgttg ggttgggttg agttttattt ggttgtattg agttgagttg agtgtgttag    14040
gttttattgt gttgtgttgt gttaggttga gttttattgg aatgtgttat attgggttgt    14100
gttgtgttgg aatgggttga gttgggttga gtgtgtttag ttttattgtg ttgtgttggg    14160
ttgagttgag ttttattggg ttgtgttgtg ttgggttgta ttgagttggg ttgagtgtgt    14220
tgagttttat tgtgttgtgt tgtgttgagt tgagttttat tgggttgtgt tttgttgggt    14280
tgtaatgagt tgggttgagt gttgggtttt attgtattgt attgggttgg gtgttttggt    14340
tgttggttgg taataggggg gttattgttt tagttatagt ggttaggaag atttttaga    14400
ggaggtatga gttgggattt gaaaaatgga gggggatgta gttatttagg tagaaggaag    14460
agttaaattg tttttgttt ttgtttattg ggatgttgtg gatattgttt ttatgtttgt    14520
tttgtttttt gtttaagaga taggtttttt agagtaggga taagtaattt tttttgtgt    14580
gaaagagtta ggatttttat attgtgtaaa gtttagaaat agttagtagt agttttttg    14640
tttttttgtt ggaggttttt ttttttttgt aatttttat tatagtgtag aagtttaata    14700
ttaaagagga taaaagtaga gttgttttag ttgggatagg ggtatgaggg gtaggttagg    14760
agtttttatt tttaggtttt taatgaattg aggaaaggaa agttatggtg tggttggtgg    14820
gtagttagga gattttaatt gtagttaaga gtttagggat aggttttgtt ttattagttt    14880
tatttttagg tggtttttat tgatttgtat gtgggtttta gtgaggtttg tttttatggt    14940
tagttgtttt tgtgtatata tgtttgggta gtgggttttt tggaagattt tttttagttt    15000
ttttagttgg tagttggtga aggtggtttg gttttgttgt tttttgtttt tgttgttttt    15060
tgagttggtt tttttaatg ggttggggag gtttgagttg gtttggtttt ggggtttttt    15120
tattttagtt tttttgatat ttaggtagtt gttgtttatt tttatagtgt tagagttagg    15180
gggtaatttt ggtttattta gggagttttt tttagttgag ggaggaggaa ataaagttag    15240
aaattaatgg ttgaattaaa taagagaagg ggaatgttag ggggagagtg gggtatttgg    15300
gtagttattt tttgtttttt ttttttaga gtttattgga tgtgaggttt ggagatttgg    15360
ttttaattt tggtttgtta ttaatttgtt gtgtgatttt gggtaaattt tttttttaat    15420
ttgagttttt ttattattat tttagattag gtttttttta gtaataatgt tatgtgaatt    15480
taaggtagtt aatttgattt ttgtgatttt taattttat tttattgatt ttgagttttt    15540
tttttagag ggattttttg tattaggatt tttttggggt ttgagaattt tgattttagt    15600
gttatggttt ttttgggtgg aagggaaatt tatttagtag ttatgtttgg tttagtgggg    15660
tttggggtga ggaggattta ggattgaggt agataggagt gtttttttt ttttggttta    15720
gttttattag gagtttagta ggagttgtgg agattttgtt ttaagagttt agtgttgagg    15780
agttaatatt ttttttttg tttttaatt ttgtatattg gttttattta ttgtttaaga    15840
gaagggtttt ttttttttgg gggtgtatgg gattgaggta tgggttatat tttttgttat    15900
aagtagtttt tgtttattgt ttattgaggt tgtttgggta tttttatttt tagtatgttg    15960
atttgggttt tgggtgggtg tatatgagta tagtttttag ttattgtgtt tggtagatgg    16020
atttttattt tattggtata tttttagttt aggattgtaa ggatgagtta atttatatat    16080
attaagaaag aagaggtgga ggttggggtg ggagagtgag gtaagaagtt atttattat    16140
tatatagttt ggtgagtttt ttaggtagat atatttattt tttggtagag ggtttgggat    16200
aatagttttt atttttttgg tattagggat tggttttgtg gaagataatt tttttatgg    16260
gtggagaagg ttgtggtggg gtatggtttg gggatgaaat tgttttattt taaattatta    16320
ggtattagat ttttttaagg agtatgtaat ttagattttt tatatgtatg gtttatagta    16380
ggatttatgt ttttgtgaga atttaatggt attgttgatt tgataggagt tagaatttgt    16440
agtaatgttt gtttatttgt tgtttatttt ttgtgtgtgg tttggttatt aataggttat    16500
ggattggtat tggtttatag tttgggggtt aaggattttt ggtttaggtt atggattttt    16560
gatagttttg ttatgaattt tatggttatg atttttagt tgtgtgttta ggagagttta    16620
tttatttgtt atgtgtttta gttttttttt ttataaaatg gggatgaaga tatttttagg    16680
attttttta tagggtagtt gtgggaaata aagaggttag tgtgttagtt atgtagtttt    16740
gttattattg atatttatat ttttagtttg gatttgtaaa tttatagatt ttgtgaatat    16800
tagaggattt tgattttgga gaagtttttt aagtatgtag gtttttgggt ataggagtat    16860
gtgtagggtg tgtatagatg tttagggaag ggtagttaat tgggtgtttg tagatgtggg    16920
```

tgtgaatttt ttgagaattg tattttttgt ttgtaatttg gggaaaagga atttgttgag 16980
tgaaagaata aatataatta ataagatgtt tggggagtat ttgatatgat tttttatgtg 17040
tatgagtttt gattattata ataagtatgt gtggtaggtg tgagtattag ttattttgat 17100
ttgtggtttt tttttttgga aataggttta agtgatgggt attaagtgtt agaatgatgt 17160
ttttagttta ggttttttga ttttatttag attttgaggg ttgaagtttt agatttgttt 17220
tatttttggt tttttgtgta tattggggaa gttgagaggg atatttggtt tgtggtattt 17280
ttgttttttt ttttaaggta ttaaggaaag ggtatagagg tgagtgttta gagtaggttt 17340
gaaagtagtt ttaggaggtt gtttttgagg gttattggta gtgtagatgt tatgggggtg 17400
tatgattggt ttagtggtat tatgtatgat atttgtgtga gtttaggtat atgtagatag 17460
ttatggtttt agtgataata ttttgtaaat tgtagttggt agttgggtta agagttttta 17520
gtaaagatat ttattttttt ggtagataaa tatattttat gttttaggtt tttttttttt 17580
tttttagaa atttatttta gttttataaa aataattatt tttgttttgt aatttttttgg 17640
aagttagatg aggggttgag ggaggtgagt attgagtttt gagttttgat ttttaggttt 17700
tattttatag ttttatagat gtttgttggg gattgggatt attttgtata gtaatgggag 17760
ttttatgtta taggaattga gagttggtat ggattagttt gaatttttag ttttaattgg 17820
ggggaaattg aggttagaga gagaagggaa tatgttttaa ttttggatag agtatagttt 17880
tttagttttt ggtttgtttt ttattttttt ttttgtggtt ttaggtttta tttttaggga 17940
gagaggtagg ggtagaagga atttaggggt tttatatgat taaggtgttt tgttttgttt 18000
atttgtgggg gggtaggatt agtttttgag gatgagggag tgaggatgtg gtttgggggt 18060
agtttttttag tattgagtta gtttttggtt ttttaggggga agtggtagaa ggtttagttt 18120
ttatggtgat aagtaagggt ttagttttgt ttatttttag ttgtttattg agattaagtg 18180
agtaggtaga gatagaggtg gtatagggtg tgtgaagatt attaggttaa gagttgggag 18240
ttttggtttt ggtttttgtt tttttagga ttttagatga tttattttat ttttggagtt 18300
ttatttataa atagggagag tggggtagtt gagggttttt ttttttttgt ttattagtag 18360
ttaggattgt ttttgaggat ttggggaggg gaggagaggg ggagggtgtg tttgtgttag 18420
agatggaagg aatttagggg agtgttgttt ttggtttttt aaggaagaag gaagtaagtt 18480
ttttaatttg ttttgagtta gtttgtttta aattttgagg ttgggttttt tatttagata 18540
ataaagaaaa attttttgaa gttttgtagt tttatttaat tggttagaat aataagtata 18600
gtttttttag agtttgagtt ttaagatttt aagtttaggt tgtttatgtt tatttttttgt 18660
tattttgtag tatttttatt tggtgttggt tttagtttag ggtagtaggg agtttattgt 18720
ttttaggata gtagaattat ttatttttag atttagttta tgtttgtttt ttgtaggttt 18780
ggtttattga ttttagtttt aatttttttag tgtttttttt tatgatagtt ttgtttttgt 18840
ggatattggg aggttattgt tattttttttg agtttttttt tttttttgtag ttttttattgt 18900
tgtttttttgg gtggttttga gtttttttgg tttgtgtgag atagagggtg gtagggatga 18960
tatgaggagt ggggttgggt tttgggtaaa gttagtagat tgtttttttag ggagatgatt 19020
ttgggttggt tggagtgtta gtatggtagt tgaaggtttg ggtgttggtt agatttgatt 19080
ttttgtaagg tgtttttttt agtaagatgg ttgttgaaaa tagaagtttt atggtttagg 19140
ttgattgttt ttgattaaag gaaattattg attttggttt tttaagtttt tattttgggt 19200
ttttgtttgg tatgtttttt tttttagttg atttttaaga tttaatgttt ttttaggaag 19260
tttttttaga ttgtgattgt tttttatttt ttgtttattt tttttattga ttgtttatgt 19320
ttgttgattt gtaattttat taagttttga atgtttagtt ttttttatag aatgttagtt 19380
ttttaggggt tgggatttta tgttgtttgt tttttttttag tttgaggttt agtataggat 19440
aggttaggtg gtagagatgt agtaagaggt tgttgaggaa taaatgaata attttaatt 19500
aataaatatt attgagtttt tgttttgttt aggtatttgt gtgtattttt ggtgagttga 19560
tgttttatag agtatgtgta gaaaatgttt tttgagtaaa tgaattaatt gttttatttt 19620
tttatttatt ggggagaaag ttgggtggga tatattgaaa aagggtatgt aatgatgaat 19680
gttattattt aaggttgata gtataaatag taaaggttat tgtgggagat aggagagaag 19740
aaagatttag atagattttg agtgatgttt ttatgtagag agtaagggat agaaggagaa 19800
gtagagatgg gttttatagg ggtggtttta gaattgaaat taaggtttag aaatttagtt 19860
taggatttgt taggaattgg ttttatattt attttgttat tatattgggg tagagaagat 19920
tgttagggag tttttagttt gtttggatgt tgggtaagtt ttttttagag ttttggggta 19980
ttttgtgttg gtataaggtt gagtagtaat agtgtttagt gtgggtttag gttatttgtt 20040
aggggttgga ttttttatat tttttttgga gtagtttttt gtggatttgg aaaggtttta 20100
tgttgatgtt ttttttatga tgagtttgtt ttagggttga ggttatgggg tttaagataa 20160
tgtagtttta atttgtagtt ttttagtttt gatttggaag taaatttttg attatttaga 20220
gttattaatt aagtagtgaa tttatattat tgagagtaat gtttgtttta tggagggtgt 20280
tgtgtatgta gtgtggttat tatggtattt agtgtttttt aaataatttt aggttagtat 20340
tattgttata gattgttttt gatgaagtgg gaagtattta gtttaaggtt gtatttttaa 20400

ttgggagtta ttgagttttt taatttttgt tggaattttt tttgggggga tttttgtatt 20460
attgggagtt gttgttttta gtgttgggaa tttttggttg ttggatttta gttggagaaa 20520
tgtatgaagt ggaaagtttt gttttgtttt tttaattttt atttatttgt tttggtttaa 20580
tttaatggta ttattttaat ttagttttat ttttttaaaa taggaaaatt tttgagaggt 20640
ttttaaaggt gattgatttt tttttttttt tttttttttt tttttttttgg ttgtgattta 20700
aagaatgatt ttttagagtt attttaggga gagggtgttg gtagtatttg tatgtataga 20760
tgtatatttg agggtttgtg gtataagttt gttattgtga gtgggtgtgg ggaatatatg 20820
tgtagttgtt tgtggatagg tttgtggagt gtgtatgttt gtgtggttaa gtgtaggtat 20880
ataagtggga tgtgtgtttt tgtgggtagt tgtgtgtggg tgtagatttg tgtgggtggg 20940
tgtggatatg ggtggtgtgg gtttgtgtgg agttgtgtat aggtttatgt atatggtgtt 21000
aaggggtttt agttatttta tttttttatgg ttttgattat atttattttt tttttaaata 21060
tttgttttgt gggggatatg ttgtttgttt tgtttttggg tgagttggtt ttatatattt 21120
tgtggtggag tgtatatatt gatgtaattt ttttaaaaaa aataggttag tatgaggttt 21180
ttttatgtta ttgaaagggt attgattaaa tttattgttg tttaaatggt tttgtggggg 21240
tgagggtgga ggttaaagtt tagttattga ttatgttttt attttatttt atttgatatt 21300
ggggtttgtt tagagtttaa tttgaatgtt tttaatttag ttttagtttt aataaataat 21360
gttaaagggg tagtagtgat tgggtttaga ttttagtata tatgtatggg gtatagttgt 21420
ttgtagagat agatagtgtt ttggttttta aggggttgtt aatttttagt agagggtggg 21480
gtggaaatgg tagtttttag gttagttagt ggtataggtt attttgttta tttatagtta 21540
ttaatttaag ttttgaaatg tttttgaaat aaggaaagtt tgagaaattg ttttagatta 21600
gaggaggtta aggaattatg aggattaagt gtaattaatg tgttttgggg ggaatgttgg 21660
aatagtaaaa ggatattggg ggaagttagt gaaatttgaa taaagtgtgg agtgtagtta 21720
atagtaatat ttaataaaaa gtgggtaaaa tattttagta gatattttat ttaagaagat 21780
ttatagggag taaataagta tgaaatatgt ttaattttat tatttattat ggaaatgtaa 21840
attaaaagta taatgagata ttattatgtg tttattatag tgggaataag atttgatgat 21900
gttaagttat ggtgatgtat ggagtatttg gaatttttat ttattatagt gtaaatattt 21960
gggtggtgtt ttatttagtt aaattaatat tgattttatg atttagtaat tttattttta 22020
ggtatttatt ttatagaaat aatgatttat tttaagagga gaggaattta aatttatagg 22080
taaatgtttg tagtagtttt atttagaatt attttgaaat ggagatattt ttaagtgttt 22140
tttgataggg gaatggagat gtaagttgtg gtttatttag gttatggatt gattttatag 22200
tgaaaagaaa gaattgttga taatgatatg gatgaatttg aaaggtggtt ttagggtttg 22260
taaataaagt tagttttaaa tttatatgaa gtgtaattgt atttagttag tatatgaatt 22320
ttgggaaagg taaaattatg gggattttag gtaatagatt attggttgtt aggtgtagga 22380
ggaggggaa ggtttgttta taaagggttt gtattggtgt gatggaatta ttttgtattt 22440
tgattgtggt agtgaatata tgaatttata tatgagtgtg ttaaaattta taaaagtata 22500
tattaaaaag ttttttatag ttgggtgtgg tggtttgtgt ttgtaatttt agtattttga 22560
gaggtagagg tggatggtgg attatttgag gttaggagtt tgagattagt ttggttaata 22620
tggtgaaatt ttatttttat taaaaatata aaaatttgtt gggtatggtg gtagttattt 22680
ataattttag ttatttggga gggtgaggta ggagaattgt ttgaatatgg gaggtgtaga 22740
ttatattgaa ttgagattat gttattgtat tttagtttgg gtaataagag taaaatttta 22800
ttttaaaaaa aaaaaaaaaa aatttatagt atgttgtttt taaattttaa aaaatgtaat 22860
taaaaagaaa atataaaatt gtgtttatat atataaaata aaaattaaat taaaagttta 22920
tatgttttta tttagaaatt ttattttag gaatttattt aataaatgat ggtaagtatt 22980
tattagagag aatgtttatt atgttatagt ttaatatgta ggaaaattga tagtaattaa 23040
aatatttaat aataggggat tggttaaata aaggatgata tttttataaa tggaagattt 23100
tttttgatag ttttttatttt gtatgttgtt tttttttttg agatagagtt ttgttttgtt 23160
atttaggttg gagtgtagtg gtttgatttt ggtttattgt aattttattt tttagattta 23220
aatgattgtt ggatttggt tttttgagta gttaggatta taggtatggg ttattatatt 23280
ttgtttattt ttgtattttt agtagagatg gggttttttt atgttgttta ggttggtttt 23340
gaatttttgg ttttaaatga tttatttgtt ttggtttttt aaagtgttgg gattataggt 23400
atggattatt gtgttaatat taggtattta tttgtttttt tgtttttttt ttgtttattg 23460
tttttttttt tttttttttt atggagtagg gaatatattt gaatatgtgt gttttgttta 23520
ttttgttgtg taatatttag tattatagtt ggtatgtagt agatgtttag taaatatttg 23580
atgaataaat gaatgagatg atgttaagaa aggatagtgt tttaatatat gtaaagatgt 23640
ttaatttata ttaaataaat tttattttga ttaaaaagag gaagggagtg gagtatttgt 23700
ttttagaaaa tattataagt ttttagaagt ggtaatatgg gtttatgggt taattttgtt 23760
tttttttttt ttttgggggg tggggtgggg ttggtttgaa ttaatttttt atttgttttt 23820
ttaggaatag gttgaaatat ttgaaagtag tgattagaat tttggatggt gtatgttttg 23880

agagtgggtt gggttttgtg ttgtgggatt atgggaagta agagataagt attatttgga 23940
gagatttagt taaattggaa ttttagataa atagttaata ttttaaataa tataagtata 24000
ttttaaatat ggtataagaa atatttttat tttataaatt atttattatt taaatgtaga 24060
tttgattgag taatttggtt ttgttgtttg tttgtttgtt ttttgttaaa tttgttaatt 24120
ttgtatatta ggtttatgtg gtatttaggg ttttgaaggt aggattggtt tttattagat 24180
ttatggattt atatgttagt tttttattta gtttgaggtt tagagatttg tttttgtttt 24240
gtagtgtatt gtatgagtat ttttaggttt ttgtattttt gtgggtattt ttttttttat 24300
ttagtatgtt tttttgtttt ttttgtttgt ggttaattat tatttttatt tggatagttt 24360
tttttgtaat ttttgtgatt ttttttttgt attataaatg taatttatgt tttatatgga 24420
agattaaaaa aaaattaaga tagattaaaa gaaaattatt tatatatata tatatatata 24480
tatatatata aatatatata gagaaaatat tattttgtaa atttttgtgt ttttttttga 24540
gatggagttt tgttgttgtt gtttaggttg gagtgtagtg gtgtattttt ggtttagtgt 24600
aatttttgtt tttttggttt aagttatttt tttgttgtag tttttttgagt agttgggatt 24660
ataggtattt agtattatgt ttggttaatt tttgtatttt tagtagagat agggttttgt 24720
tatgttggtt aggttggttt tgaattttttg attttagatg atttgttttt ttgggttttt 24780
taaagtgttg ggattatagg tgtgagttat tgtgtttagt ttataaatat tttagtaaaa 24840
tttaattata ttttgtattt ttttttatgt tgttttatat ttttttatta tattattttt 24900
aatggttatg tagtgagaat aattattatt ttatttaatt tattttttttt tgttgggttt 24960
ataggttttt atttgtttga aattatagta ggtttgtgat ttaaattttt taatatatgt 25020
atggtgattt ttttaggaga agttttaaga attagaaata tgaagtttaa ttatttatat 25080
atttgagggt ttttgagaaa tatggttaag ttgtttttta gaaatatgta atgtttttag 25140
ttattatggt gttttttttt attttagagt taataatggt tattatagtg tttttaaaat 25200
ttttgttagt atgatggatg aaagtgagat tttgtttttt tttttttttt ttttattttt 25260
taattatgga atattttatt ttttgttttt ttttgttgtg aatttttttt tttgggtttt 25320
taaagtttag ttaagggtta tttttttttt tttaggattt tttttgtttt ttaaatattg 25380
aattttttgt tttatagtat tggtttatgg gtttgttttt tttttagatt gtgagttggt 25440
tagagtgggt tttttagatg tattgttggg gtttgggttt tgtgtgagta agggtttagg 25500
atatattttt ggaattaatg taaatgtttg tgtgtgtttg ggttgaggga gattttttttg 25560
ggtttttttt gtttttggtt tggttggggt agttttgtag gtatagttga gggtggttag 25620
tgtgtttgta ggaaaaggtt ttaggttttg gagttgggtt ttagatatag gaggttttttg 25680
gtttttttgta tatattgttt gtgttaggag gttgttgtat ggagttggaa gaagggattt 25740
tttaggatga ggggggttga ggaaattagt tatagtgtag ggtaggaagg attaaagatg 25800
attttggtag ttttttggtt ttggataaat tttttgtttt ttgtttgttt agtttaataa 25860
tattgtatga atttttgtta gggaggggta tgagaagagg agatggaggg gtatagtttt 25920
gtgggaagtt aagattgaag aggagttttt ggttttggtt gtttatttag aaggttaagt 25980
atttaggttt tagggtttaa tttttagtag gttaagatta agattgtagt tgtgtatgtt 26040
ggttattagg aggtattgta gttttaataa agttattgga tggaaggtga tgatttttggg 26100
gttttagtgt ttaatgaggt ttttagatgt ttgtttgaag atttaggttt tagttgggag 26160
gatttgttat ttaagtagta atttagtttt attatgtgtt tgttattatt ttagtattat 26220
aagggataaa gagatggatg gggtgggttg ttggtttttt ttgtaggggt tttttttttt 26280
ttagttttgg gtatttttat atttttgttt tatttttagtt tttttagaat ttttatattg 26340
tggttttagt atttttattg gatagatggt taaagtgagg tgttgagaga agggatttat 26400
tagtgttatt agtgattgag tgatagatag gttgagattt gggttttttt ttattttttta 26460
agtggttttt ttttggagat ttatggggat tttttggttt tttagagagg gtggtgtata 26520
gtgggtgttt aggaaattga ttaatttgta aaagttttttg ttttgtaaaa gtttttttagg 26580
atatttttgt tgtttggttt agtggttttt tatgtggttg gtagatttgt tattagtttt 26640
tttgtgggtt ttttttgagg taggtatttg ttgaggtagg gtttgttatt ttgtttttta 26700
ttgttttgtt tttatataga agttttttta gttttttttg taatggtagg ggtgagtggg 26760
gaggattgtt tttttttttag tttagagatg gagtgggttt tttagagttt aggaatgaaa 26820
agggattaat gttgggtttt tgtaaaaata aaaaataaat aaataaatta aataaaatta 26880
ggaattgggt tttgtatttt ataaagtatt agggtattta ttatgttatt agtagggttt 26940
tagttttttt ataattgtta ggtttttatt ttatagtttt agaaattgag gttagagggg 27000
gtaaatgttt tgttaagaat taataattgt taagaaggtt taggggtggt ggtttatgtt 27060
tgtaatttta atattttggg aggttgaggt gggagagtta tttgatttta gagttttatt 27120
tagtttgggt gatagaggta gagatttgtt ttaaaaaaaa aagaaaagaa aagaaaagaa 27180
aataaaaaat tggtaagagg tagagttgag atttgaattt tatgtttgga gtatgtttat 27240
tgggtttttt tggggtggat gttgtgggtg ggattgggat aggtagatag gagatagggt 27300
taaggaggaa gggaggaagt tataggagaa tggttgtttt tagttgtttt aagagattgg 27360

tttttttgta ataattttta aggttgtttt tgattaaatt gttttgttga agattaattt 27420
tattgattat ttgggatttt agatgtggat tagtgttttg ttttagggtt tatatatatt 27480
gaagtattta gaggtaaaga ggtatatgag ttatatgttt gtaatttatt ttttttttgt 27540
ttgtttgttt ttagataaag ttttgttttg ttgtttaggt tagagtgtag tggtataatt 27600
ttgattaatt gtaattttg tttttgggt ttaagtgatt ttttatgttg gttaggttgg 27660
ttttgaattt ttgattttag gtgatttatt ttttttgatt ttttaaagtg ttgggattat 27720
aggtatgagt tattgtgttt agtttgtaat ttattgttaa atgatatagt tgtatattgt 27780
atgtatgtat attaatatat atataaatag agtatatgta tagagagaga tatagagaga 27840
gatttagaga gattgaaaat aatgtaaata tggtaaaata ttattatttg gggagttagt 27900
ataaaaggta tttgggaatt ttttgtagtt tatttataat tttaagtgta atattgtatt 27960
aaaataaaaa ggtttttaaa agaaaaggta tgtggattaa tggaaagaag gtattttttt 28020
taggaatttt ttttgtgaag ttggtatatg taattatttg aataggattt ggatgagttt 28080
atttggtaat ttttttgttt ttttttattt attattatta tttataattt tataaggggt 28140
tttgtatgtt gtaataaaaa ttataagata aatgttaatt ttgggagtat atattgtttt 28200
ttagtttttt ggaagttttt atttttgtgg aagttattgt gatttaatta gaatatgggg 28260
tttttttaag tagttttttt gagagttgta ggagattaag gttaagttat tgttggaatt 28320
taaaagttta aaaagtattt taatttttga gtttattgta attttaattg ttttatagtt 28380
agtttaaatg agtaaagttt ttttgtataa aaaaaagaaa tggtggaaag aaagaatttt 28440
aaattttgtt tagtttttag tttttatggt ttttttgtta aggtttgggt ttttttttatt 28500
ttttagtttg tgtttggaat ttgttttttt tgtttttttt tttattttgt gttttgtttt 28560
attgatttat ttagagtttt ttttaggaat tatggtttag tggaagagat gtatgaggga 28620
taagtatggt taagtgtttt agggttttta gggtaaaagt tttgtttttt agatatggta 28680
gggtttatag aaaggagtgg ttaattttgg ttgaaaaggt tggagaaggt ttttgtaatg 28740
gagtggtttt tgaattgggt attgttagtt aaggaaggtt tggtaaggta tgttgtattt 28800
tatggtaaaa taagatatta agatattatt tttttatttt aaatagggta aattttaaat 28860
tattaagttt tttagatagg agttttttaa ttagggttga ttataattat gatagtaata 28920
atgtaatgtt ttgtatttgt gttgttttga tttatttatt attttttttg atttttttta 28980
gattttatga ggttagtgtt ggtatttttg ttttatagat gagaaaattg aggtttagtg 29040
aagatgaata atttgtttaa gattatatag ttaggagggg tagattggat ggttttatgt 29100
taagtttatt gtttttttaag gtggtgatga gttatttttt taatatggag gggttaggta 29160
ggtttatttt tgttttaagg gggtattgtt gagttattgg ggttattttt attttaaagg 29220
attagggtaa attaagtttt agggttttta tgagaagttt atgtatgatt taatatttgg 29280
agattatttt ttgatgtttt ttggggtggg atgggtgttt tagttttagt ttgggtttta 29340
agtgttttttg ttagtttttt tttttaagga ggttagaagt tttttggttt tttgttttat 29400
ttttaagttt tattttatgg tgtgggtggt tggatgagat tttgttttttg gtaggattta 29460
ttgttagaga aatagagttt tttatgtttt tttttatttt agtaatatgt ttgtagggtt 29520
tagagatttt attggattag attttttttt tatagttaag atgtggaggt ttagagtaag 29580
gttatattgt ttgataatgg aagggttagg atttgaattt tgtatttttt gtatttagtt 29640
ttgtggtttg tttgtttatt gtttttttta agtttttttaa attatgtggt tttgggtgtg 29700
ttttaagata tttttttatt aatttttttta agtttttgtt tttttttttgt agtagaaaga 29760
atttgtagt tttattattt ttattaagat attgtaatat tatttttatt tagaggttgg 29820
tgagatggag gagtttagat tttgtatgag gtaaaatgaa atgattgaga agtttttggg 29880
gtaggatagg tttgggtttg gtttttggtt ttgttattta tttgttatat tgatttattt 29940
aatagtattt ttggagtatt agttatgtat tgggtaaagt tttaggtggt gaaaatataa 30000
agtttagtaa aatatagttt ttttttttaa atggtttgtt ttagtgatag gggttatggt 30060
tatagtgtag ggtggtgatt agggtagggg tatttagttt agttttgggg tgattttaga 30120
tttttgtaga ggaggtgttt tgaagtagta atgttgttgg agaggtttgt tgaagttgtg 30180
tttagttaat gttttatata agattaggga atgttagttt tttttatgaa agaaatggga 30240
gttgattaaa ggagattatt aaggaggttt ttaagttatt ttgtggtttt gttatagaag 30300
ggtattttttg gaaaagtgat ttgatttttg tgtgttttag ttttttttatt tgtaagatga 30360
ggttgagaat atttatattt tagaatggtt gtgaggtttg attgaagtag agtgtaagat 30420
ggtatatttt ttgatttata tatggtattt attttattaa tgggatgttg ttatgatata 30480
ttaatatagt tatgatttgg ggatgaattt ggttgtggta gttgatagtt agggtaatgt 30540
tgtttagttt gtaattgtgt tatttttatt tgtttttttt tttaggtgta ttttttttag 30600
tttaggtttt ttgagtggta gtttttagaa tgatatttat gaattataaa tttttgttaa 30660
aagtagtttt tagggttttt tttaatgaag ggtttatttt atatatatat aataaagatt 30720
ataaatgggg tgaaatattg gtaattgttg taaaaattaa atgttaaatt tatttggtta 30780
tatagattta aaaaaatttt taaagagtgg atttatggta gtttttgttt ttagattgtg 30840

```
tttttattaa gggtagggga ggggtgttat gaggtagtgt tattatttgt tttgtttgtt 30900
gttattgttt ggtatttaag tatgtgttga attgagttaa ttttaaataa ttgaattttt 30960
tttttttttt tttttttttt ttggagatag ggttttgttt tgttgtttag gttgaagtgt 31020
agtggtgtga ttttggttta ttgtaatttt tgtttttttgg gtttaagtga ttttttttgtt 31080
ttagtttttt gtgtagttga ggttataggt atatgttgtt atatttggtt ttttgtgttt 31140
tagtagagat agggttttat ttttgttgtt ttggttggtt ttgaatttat gagtttaagt 31200
aatttgtttg ttttagtttt ttgaagtgtt aggattatag gtatgagtta ttgtgtttgg 31260
ttaaataatt gaattttttta gtttgtagaa gtagtatatg atttagaaa gaatgtgttg 31320
tttttattaa tattaaattt tttttttttt aaatgtgttt ttttttttaat ttttatttat 31380
gtatttattt atagagatgg agttttgttt tgttatttag gttggagtgt attgatgtta 31440
ttttggttaa ttgtaatttt tgtttttttag gtttaagtta ttttttttgtt ttagtttttt 31500
gagtagttgg gattattagt attggttatt gtatttggtt aatttttgta tttttagtag 31560
aggtggggtt ttattatttt ggttaggttg gttttgaatt tttgattttg agatttattt 31620
tttttggttt tttaaagtgt tgggattata ggtgtgagtt attttgtttg gtttaaatat 31680
atttatttta atttagagaa ttatttataa attgaaaaag taataaggtt tttttttttt 31740
tggatgagaa agggaaagta atggtttaat taattataag attgtttttt ttttttttgt 31800
tgtttaggtt ggagtatagt ggtataatta tagtttattg tagttttaat tttttgggtt 31860
taagtaattt tttttttttta gtttttttgag tagttggtat tatgggtata taatattata 31920
gttggttaat taaatttttt tttttttttt ttttttttgta gagatgaggt gttattatgt 31980
tgtttaggtt ggttttaaat ttttggattt aagtaattgt tttattttag ttttttaaag 32040
tgttggaata taggtatgag ttattatgtt tagttttagt ttgtatttta ggaaagttat 32100
taattttttt aaagttgata agaatgggaa agattttttta atatttgttt tttaaaaaat 32160
ttatatttat aattataaaa tagttaataa tttaaaatat ttaaaggata attatattta 32220
aaagttttgg ttggtgtttt tgtataaatg attgggattt ggagtagatg ggtttttagg 32280
attttgttta ttatatatag taaggataaa atataaataa ttaatgtttt agtatttata 32340
attaatatgt tagttaaaaa tggttttttt ggttttgtat ggtagtttat gtttgtaatt 32400
ttagtatttt gggaagttaa tgtgggtaga ttgtttgagt ttaggagttt gagattagtt 32460
tgggtaatat agtaagattt ttattttttat aaaaaaaatt tttaaattag ttgggtgtgg 32520
tggtataagt ttgtagtttt agttatttag gaggttgaag taggagaatt gtttgagttt 32580
aggaggttgt ggttgtagtg ggttatgatt tagttattga attttagttt gggtgatagt 32640
gagattttgt ttgagagaag gaaggaagga aggaaggaag gaaggaagga aggaaggaag 32700
gaaggaagga aggaaggaag gaaggaagta ggtaggtagg gagggaggga gggagggagg 32760
gaaggaagga agaaaatggt tttttttattg atttttgttt atttgaataa aataaaaaat 32820
ttaaaaaagt ttttttttta aaatgtatga tgtggttagg tgtagtgttt tatatttgta 32880
attttagtat tttgggaggt tgaggtgggt ggattatttg aggttaggag tttgagatta 32940
gtttggttaa tatggtaaaa ttttatttta aaaaaagaaa aaaaaatata tatataatgt 33000
atggtgtgtt tttttttatta agtaaatttt aatttatagt gaatgtgaga taaatatatt 33060
ttttttttat ttgttattat tttttggtag gatggatttt ttgtaatggg atagattttt 33120
tgagaggata ttttgtgtag tataaaattg ggttttgtgg aattttttttt tatggggtta 33180
aattttgggg gatgtattaa tatagtagag tttgggtata tatattttttt gaggaggttt 33240
ggtttttatt gtttatttgt tatatggtat aagtaatatt tttaaattgg tttttttgttt 33300
ttggttttgt taatattggg ttagtgagga ggttattatt tgttttgttt agaagggttt 33360
gttttttttt tttttattta taggagttgt ttttatatat tatatggggg ttttgggttg 33420
ggttggtttg gtttttagga tggttttgga gggaggaggg agtggtgttt gggtggttag 33480
gggatatggt tattatttta gtagatgggt gaagatggtg tagtttatgt ataatatgta 33540
gtgtgttgaa aatttttttt agtgtatttt ttttattgtt tatagagttt ttatatgttg 33600
gttaagtttg atgggtatat aggtatgagg gtatttaggt agtgggtatg agtttttttgg 33660
gtaggggagg aaaaaggttt tttggtttta ggagatttgg gtttaattta tagtgggata 33720
gttttatttt ttttattttt aaattgagga tattaatatt ttttagtggg tttttataaa 33780
aagttttagt ttaggtgtgg ttaagggtaa tgtttattag gtgtttttttt aattgtagat 33840
ggggtttagg aagttggggt ttaggaaggg tgtagaggtt gtttgatatt ttttgagttt 33900
gattttttgt gtttagtttt gtgttgatta gagaggaata tagtagaatt tttgttttgg 33960
ggataggttg aagttttggg ttaaataaat atagaaaata tatttagtag ggagagagtt 34020
attttttagt ttatgatata tgtgtatgag gtgtagtagt ttatggggag gaggggtttg 34080
aggggtttgg gtggtttttg tttttttttg aagtttgttt ttgttgtgtt aggtagagat 34140
ggtagttttt gtttttttta ttatttattt atgttttaaa agataatgtt aattatatag 34200
taattataga tttagtaata attttttttat tttttaattt tatgtatttt agttaatttt 34260
aattgtttgt taaggttttt ggttagtttg atagagttaa ggttttagta aaattttatt 34320
```

```
aattataata gttaatattg attgagtatt tattatgttt taggaattgt tttaagattt   34380
ttatttattt atttatttat ttatttattt atttatttat ttagagatag agttttgttt   34440
tttttgttta ggttggagtg taatggtgtg gttttggttt attgtaattt ttgtttttttg  34500
ggtttaagtg atttttttgt tttagttttt ttagtagttg ggattatagg tgtttattat   34560
tatgtttagt taattttttg tatttttagt agagataggg ttttattatg ttggttaggt   34620
tggttttaaa tttttgattt taggtaattt atttgttttg gttttttaaa gtgttgggat   34680
tatagatgtg agtaattata tttggttttg tttttgttta ttttatttta tttaagtttt   34740
atttattat taataggtag atatgatttt tatttttatt ttataggtaa agaaattgag    34800
gtaaagaggt taattaattt gtttaaggtt atttagttat gtgggttgaa atttgttttt   34860
aggttttgag ttttatgggt tttttaaat taaaggtatt tgaagtatta agtagtttgt    34920
gtagtataat tgtggaatga atagttttta tgatttttta agaattttag aaattatgtt   34980
ttagggtata tggttggaag attaggttta agtaggtttt tgtttagtta tttaggttgg   35040
ggaagttttt aggaagggga gttggggata gttattttttt tgtagttgtg tttttttttgg 35100
atttgttgt ttagtttttg gagttttagt ttttggttgt gttatttatt ggatggttta    35160
gggtagggga tagggagttg taagtgtttt ttttgggaat ttaatagaaa atttttaaaa   35220
tatgtatggg gttaatagta tggagttatt ggatgttttt tttttggagt ttttttaagt   35280
tttgttggaa ggatttttgga attattttaa taaaggaagt ggttttagat gtgtgtattt  35340
taggaggtgg gtgtatagat ttttagagta taaaagaatt tttttaaagg ttatgttttt   35400
atgggatggt taattgaagg ggtagagggg tttttgaata tttaggggtt tagttagggt   35460
taagattgtg gtttttagagt ttttgttttt ggttttgttt tttatttttt tttattagtt  35520
ttaggttttg gaaaataaat gtgtttttga gttgaatttt tgtgatttgt tggttattgg   35580
tttgtaggta aggacatgaa tggatggttt ataagggttt ttgtatattt tgtaattttg   35640
gattttatat tagaagatta tttaggggtt agatagataa atgtaataga atagagatta   35700
gatgtaaatt tttaaatata tgtgtaattg atttttaata aaggttttaa gattggttga   35760
tgagaaaagg gttatttttt ttataaatgg tgttgggaaa agtggatatt tttataagga   35820
agaatgaagt tggagagttt tatgttatgt tatatttaaa attaaagtta ttaaagattt   35880
aaatttaaga gttaaattat aaagttttta aaagaagata taggtgaaaa tttttttgat   35940
attggagtta ttgatgattt tttgaatatg atattaaaag tatagataat aaaataaaga   36000
aattgaaaaa ttgtattttt ttattaaaat gtaaaatttt tgtgtattaa aggatagtat   36060
ttagggagtg aaaggataat ttatagaatg agaagaaata tttgtaaatt atgtatttga   36120
aaaggaatta atatttagaa tttttatgat tgaagaataa ggaaataaat aattttattt   36180
aaaaatgggt aaagtatttg aatatatttt tttaaaggag atatataaat ggttagatgt   36240
taaaggagaa tattgtatga ttttatttat aagaggtatt taaggttagg tgtggtggtt   36300
tatatttgta attttagtat tttgggaggt tgaggtgggt ggattatttg aggttaggag   36360
tttaagatta gtttggttaa tgtggtaaaa ttttattttt attaaaaata taaaaattag   36420
gtaggtgtgg tggtgggtgt ttgtaatttt agttatttgg gaggttgagg taagagaatt   36480
gtttgaattt gggaggtgga gattgtagtg agttgagatt gtattatttt attttagttt   36540
gggtaataag agtgaaattt tgttttaaaa aaaaaaaaaa aaaaaaaagg tatttatagt   36600
aggaaaattt atagagagag agtagaatag aggttatttg ggtggggggt taagggtgtg   36660
atggtggttt ttgtttaata ggtatggagt ttttgtttgg gatgatgaaa aagttttgga   36720
aatagtggtg atggttatat aatattgtgg gtgtatttaa tgttattgaa aggtttattt   36780
gtagatgtta aagtggtagt attttatgtt atgtatattt tattataata aaaagtttat   36840
ttttaattaa aataaagatt ttgaatgtaa aaagtaaagg ttatattgaa ttatgtaaag   36900
aatatttagt ttggggtttt attgttttgt ttagagtttt ttagtggttt ttttaaagtt   36960
tgaatttttt ggtagaaaat aaatggttta ggggttaggt gtggtggttt atgtttgtaa   37020
ttttagtaat ttgggaggtt gaggtgggag gattatttga gtttaggagt ttaagattag   37080
tttgggtagt atggagaaat tttgttttta ttaaaaatat aaaagttaat tgggtgtggt   37140
gatgagtttg tagttttagt tatttgggag gttgaggtgg gaggattatt taagtttggg   37200
aggtagaggt tgtagtgagt tgagattgtg ttattgtatt ttagtttggg tgattgagtg   37260
agatttttgtt ttaaaaaata ataaaataaa ataaaataaa ataaaaagtt tttttagtt  37320
gtgtttttgt tattttttag tttattttat tggtatgtat tatgtttttg tattttatat   37380
atttgtttta tgtttttttt ttatttgaag atttagtttg tgttgttttt tgagtttaga   37440
gggttttttt tattttttttt agttggtgag tttttattta atttttaagg tttattttga  37500
atgtttatgt ttaggaagtt ttaatttatt tattttttttt ggggattttt atggtatgta  37560
gtatatttta attaggttat gggtttttttg agggtaggag ttagtttatt tattgttaaa  37620
tattttggtt tttgtttgat atagaagtgt taggttgaag gatttgttgg atgtaaggag   37680
gagtttttgt taggtgtata ttttttatg tatttttttt gagttgtttt ttttttttga    37740
atttggttt ttatttgtaa aatggagatt taatttttttt atttaggttt ttggtgagat  37800
```

ttaggggatt atggatgttt aagtgttttt agattgtata tgttggggtg ggatgggtgg 37860
atggaattat gggtgtgttg ggaagagttt aggttttgga gttggatgtt ttgttgggaa 37920
tttgggtgag gttagatggt attttggtta gaagagaggg ttgtattttt tgtaggtgag 37980
ttttagaatt ttagggtgga ggatgagtga atgggggatt aagggaggaa ttttagtagt 38040
ttttaggtag tattttgggt aggtggtagg aatgggggtg ttagtgggta ggatatggaa 38100
tttattgtag ggaatggggg agatttgagg aggagagttg gggatgaagg gagaaaatta 38160
tttggttaga gttgattttg ggggttgtgg tagttagatt gtaggtttgt ggttagtggg 38220
gtgagattta aaagaggagt agtgaggaaa aggggtattt ttttgattgt tagagttgga 38280
gaagattaag atttgtgtta aatagagtta tttagagttt tgttgttttt atattttata 38340
ggaaattttg taaaaaaaaa aaaattaata attttttatt agaatgtaag agaatgataa 38400
agtaggtttt tattttaggt ttttatttaa aggaaggatg aatgaagaga gagtggaaag 38460
atttatagag tttttagtat gttttttttt tgtaagattt ttttatttat ttatttagta 38520
atagatattt attgagtatt tattatatag attagtttta ggttttgggg aaagttggaa 38580
ttaaaataga taaagttttt tgtggttttt tatgtttttt agagggtatt gtttttatag 38640
atgtttttta gttttatttt attttgatta gaggtttttt tatgtttttg aaatttttgg 38700
aaatttatgt agttattttt tatattagtt atgaggtttt atgttatgtt tgggatattg 38760
ttttatttg gtataggtga ggaagttgtg tttaaggtta tagagttagg tgtagaattt 38820
taatttgata ggtgttgaaa tttatttttg atattatttg tttttttgtat ttttggttgt 38880
gaagttgaga agaatgtttt gggggttatg gttttttttt ttttttttt ttttgtgaga 38940
tggattttgt tttgttattt aggttggagt gtagtggtag gattttggtt tattgtaagt 39000
tttgtttttt gggtttatat tattttttttg ttttagtttt ttaagtagtt gggattatag 39060
gtgtttgtta ttatgtttgg ttaatttttt gtatttttgg tagagatagg gttttattgt 39120
gttagttagg atggttttaa ttttttgatt ttgtgatttg tttgtttagg tttttttaaaa 39180
tgttgggatt atgggtatga gttattgtat tgtgttttgg gggttatgtt tgtaagattt 39240
tgttttttat ttttatatta tttgggatgg aattagggtt attttaagtt taattaggat 39300
tattttagaa taggatgttt tttaggtttt ttggagtttt tggttagtaa gatttgattt 39360
ttttttttg gttgttttat tgtggatgag ttatttattt tttatgaatt ttagttttta 39420
tttatgaaat ggggatgata atagtattaa ttttatagga ttgtgatggt gattaagtga 39480
aattatgtat aagtagtatt ggtgtattgt ttggtgtata ggtagggatg tttagttgtt 39540
ttgggttgtt ttatggtttt gtagtagtag ttaattttaa gtttttttttg ttttaaaatt 39600
tattattgtt tttgttttgt agatgggaaa atttaggtta ggagttaggt ttggtatttt 39660
agattttaa tttttggtag attggattag ttttttttt tttttggtat aatgaatggg 39720
gaagagtgtg gggttttgag gtattttgag gattttggag ggttaattta. ttttttttgg 39780
tgaatattat agaattttaa ggtttttgaa attattgagt tttatgaaga aggtttagag 39840
atggggaggt tgaggttata ataggggagt tatgtagtag agttatgtgt atgtttgttt 39900
tttttatttg aaagtgagtt ttttttttga agttagaggt tatggagttg gagttgtttt 39960
atttttatgt tgggatgtga gttaggtgtg aatggatttt tgtggttata tagagatatt 40020
ggggaggttt atatatttag gaggatttta aatttatat atatatttta aaatttgatt 40080
agaattttat tattattatt tgtaatattt tagtattttt aaagggtata gtgtgagggg 40140
tggggtagag aatttggtgg tgtaggatgt atggggtaga tatattattt tttttttatta 40200
ttatattttt tttatatttt tttttaggt gggataggt taaagtgtta tttaatatag 40260
ggagaaatgg agtggtatta gagaagtaag gggggttgtta tatatgatta tttttataga 40320
aaaattaaga gaaagtatgg agggagaaaa ggagaaagtt aagagagaag aagtttagg 40380
atttttagtt attagagaaa atttattggt ttggataaat ttttaatttt tttaattttt 40440
ttgtattttt tttttaagtg gtttttagta agttattaaa ttttggtggt gtttgtatgt 40500
tttagggtaa gttagtttga ttttttttt agagtttgag ttagggtttt ttttatttta 40560
gggtttattt tattgaattg gaattttaag gttatttgtt tgtttttgtt tttggatttg 40620
aaggtagggg ttgtatttag agttttttag aatattttat aaggtttggt atagtgatag 40680
gtaaagtttg gtgagttttg tgtaaatgga attaagtttt ttagtttttt ttgtttgtga 40740
aataagatat gtgggttagg agatgattgt tattttttttt aattttgagt gagatttttt 40800
aatatgttat tatgatttat tgtgtgtgtg ttgtagggtt aggggaggta ttagtttttt 40860
tgatgtaatt aaaggttttt agggtttggt ggatagtttt atatagtggg aggagttggt 40920
tatttggttt tagttgattt tggtttgtgg tttttaagaa tttaggaaaa gaaaatttag 40980
tttggggagt tgggtagaga gtaggagttt tagtttggtg gggttgtatt agagttaggt 41040
ttttgtaatt ttattaggga ttttatgggt gttttttttgt tgtttttttaa tgtttaggag 41100
tttatggtgg ggtttgtttt atttttaagg agattgaggt agaattgtaa gtatttgttt 41160
tatttgatgt atagagagag tgtaatttat tggatttatt gtaggagttg tatttaataa 41220
gagagtgggt gagtggattg gattttttaa tattttttgt ttttttattt attgtagtgg 41280

aaggaggaga atttggtggg tttttgtttt tttattaatt tggttgtggg ggtttatttt 41340
tttttgggtt ttagtttgtt tatgtgttaa atggggttag tagtgggtat agatagtgtt 41400
aaggatatgt tttttttgta gtgagttata tgggatagag ggtatatttt agagttggtg 41460
gtttgttaga tggttttgtt gtagtgtggg tgggttggat ggattatgtt tgagggtttt 41520
aggatgatgt ggggggtggt tgggttttat tggggatttg ttatagggag attttgtttg 41580
ttatttggtg agttagttgt ttttgggagt tatgattagg ggtaggaagg ttataggtta 41640
tgatgtaagg gtgtttagtt attttttttg ggggtgtttt tggaggtaga ggttatgtta 41700
tttatagatt tagagaagtt ttagagttgt gtatatgggt atttattttt gtatatttga 41760
ggtttgtata ttgggggata tagatataag tgttagagga tagatatgtt ttttattgga 41820
ttatatataa atttttggtt ttgttttgaa atatttagat agtaggatgt ggatttagag 41880
agaatgggta tggtatatat tgtttatatt tatgttgtgt atatgtttat ataaatattt 41940
aagttggttt ttgggtgtat ttaattatgt gggggtaata ttgtaggaga tattagtttt 42000
ttatttattt ttttagttag tgaatatttg ttaagtttaa ttgtataaaa tatttggaag 42060
gatataagtt gtaatatagg aggtagggat ttggtatgtt ttattttttta ggagaaggtt 42120
tgtttaggga aggtgtttta taaatattgg gatagggaag atgagtggat ataggggtta 42180
tgagtttggg gttttagttt gagtttggtt ataaagatat ttgtttattt ttttttttatt 42240
tgtattttag ttttgagttt agaatttgta ttttattatg gttttgtagg atttttagtt 42300
ggttttgtgt tttgtgatgg tttttttattt aggatttgtg ttagttttta gggaagtttt 42360
ttttatttat tttattttag tttttttttgg ataaaaatgt gtgtgggggt gaggagggaa 42420
gataaagggg gtgaaggtgg gtttagattg ttattagtat agaaatttta agagggtttt 42480
tggaaattgt taatattttt gtattttata tagttaaaat ttagattttg gagtagaata 42540
tattttttta aggttatgga ggagtagaga atgtaaaggt ggaggttggg gtgatttata 42600
ttgtaattgt gggttttggt ttttatgtgt gttgtgttgg atattagtgt tatggtttaa 42660
atttttttata gttttttagt tttttttgtt gtggttttta gagttttgga gagttagttg 42720
ttttgttttg atttgggtta ttaggttttg ttttttttatg aggtgtgtgg ggttaatatt 42780
tatgttgggt ttttagttta gaatgaaaat ttattttttaa agaatttgtt ttaagaattt 42840
gatgaagtaa gtattggttt tttttttgtt tttaatgtgt agtggtgtta gtgtttagta 42900
gttttgatta agtggttttg agtataagtg gtgtttttttat tgggaaagat aggtatttgg 42960
tagttggtgg ttttttttttat tttgtttgta tttttttagtg attttttttt tattaaaatg 43020
gtattttggg agttgtggag tgttatggtg atttttttaa ttgtttttgg agtttaaatt 43080
tgaattggtt tgtggttttg agtattattt tatatatttt agttttggtt ttttagagtt 43140
ttgtttttgt gtggtttttt tttgttgttg agttataatt tttttttatt tttttttttt 43200
tttggtgttg gttggtgtgg gttggggtta ggtggagaag ttgtttttttg ttaaggtgat 43260
agaatgtgtt gggggtgggg gttggggtta gggttggtgt aattaggggg ttgttgtttt 43320
tttttggata tagtggaagt ttttttttgta ttattaaatt tttgttattt tttttgaggg 43380
atttgttttt aggtagtatg taagttgttg ttttgggttt attttgtatt tttttattgg 43440
gtgaggaagg agtattttga atggagatgg gggtgttttt ggtttatata tttgtagaga 43500
agaggtgtgt tgggttgtat ttttggaggt tgtggtaatt gatattagag aagattttgg 43560
ttgtagttgg gaaggtttat tggttggaaa gaggtgtttt ttttttttag taaagggttt 43620
tgtttggaag ggttgttttt tatttgttta gtggtattat aggatggttg gtttttattt 43680
gaattttttt ggatggtatt attatatagt tgggtttttg tagtgttggt tttttaattt 43740
gatgattgtt attttggtga ggatttgtgt tgatggttgg agaattttgt gttgtgggtg 43800
tatatggtta ggtggtgttt ggtaggtgat gtttgggtgt aggatggtgt ttttattgtt 43860
ttattttaaa ttgttgtttg ggtttaggtt ttttggtttt ttgaataggg gtttgggggg 43920
ttaaggatgt tgaggttttg ggggtaggaa gttttttttg gttaagtgtt tttttttttt 43980
tttggtatat atttttttat ttatttattt tgtttatttt tggggtgaga ggtttattaa 44040
ggtagggtgt gtttttttta tgaattattt taaggttttt gagttgtggg ggttttgggt 44100
aattattttt tttttttttt ggtttaggt atttagttt aggggtttgt agagaagttt 44160
gaagtttgga taaatgtgtt ggatgttaat aatttttttat ttttggtagt agtaaaggtt 44220
aatatatttt tattttttat tttagtttgt tattaaaata aagttgtgtg tggttgaggg 44280
taggaaggtg ttgagattga gaagaaggga tgttttggag aaagtgtgtt tagttgattt 44340
tagaaattag agttttttgg gattttgttg agattttttg tagggtgttt taatttgttt 44400
ttttattgtg tgttggtgtt gtagtgtgtg tggtttaggg tttggtgatt ttggtttagt 44460
ttggtggttg tggtgaggtt tttggtgtag ttgtttggaa ttttgtatta gaattgggat 44520
tgtgtaaatg ttttggttga agtgttattt tatttaagaa atattgttgt taggaataaa 44580
atgggggtttt tggtgttttg aagtattttt tgaaattttt ttaaaataat ttataaaaaa 44640
tgtttttgtt ttaatgtttt ataatgttta aggaaatatg taaatggttt gttttttat 44700
tgagatggtt gttttaatta atagtgtata tatatataat aatttttttta attttttttt 44760

```
ttagagttaa gtattttatt atatgtaaat tataataaag aaaagattgt gtaagattat    44820
gtaagttgat tgatttaaaa tattgagttt taatttaggt tttttgtttt tttatttaat    44880
aatttttgtg tttggattag attggtgaag taggttatgg aaattaataa agtaaaaaat    44940
taaaagtatt ttttttgtt atttttttt ttaaaattaa ataatagttg ttttttttg       45000
agtaggtttt agttttaggt ttgagttttt ttgtgattat tttatagtta tttatagtag    45060
ttgttgttgt ttttgttggg tttttgtttt tgttttttt gggttgtttt ttgtatataa     45120
aatatatttt agttttttaa ttaaatttaa atatgatttt ggtagaattt atatattttg    45180
tggtgtatgg attgtgttgg tgtaggggaa ataaatattt tttggtattt aattattgag    45240
tttaatttga aaaattggga ttgggttttt aggtggtatt ttaggggttt taatttggtt    45300
tgtgtttttt tagattttgg tgttgagagt gttgtttttg tgggtgggtg gatggagagg    45360
taataatttg tttttaataa aaatttgttg ttattgaatt gaaagtgaaa gggaagggag    45420
aagaggggta gttttttgta gtggtttgt gttttgggat aggtagatta ggattagtat     45480
gtgttggttg tagttttggt ggttggattt ggagtttggg ggtgaggtgt tggagttttt    45540
gttttgagta tagggaggtt ttttgtgttg ttgagaaatg ttggattttt aatttgatat    45600
gtttttttgtg gtttaggtgg tttgttttgg atttgggtaa ggaatttagg aggttaaaga   45660
gtggatttag agtttggagg ggagaatggt gaagttttgg gttgtaatag aagagggttt    45720
ggtgggggtt tttttgtaaa aatgtggttt ttgtatttaa aggatattgt ggagttgttt    45780
taattgttgt ttttttagta tatttgggga aaggaaggtt taacttgggg tagggatgtt    45840
tgtagtttgg gagggttttt tttattaatt ttttttgtaa tttggtttag atttggttag    45900
tagagattgg aggtggtttt tttgttttgt atttttttgg tttggtttag gtttagttgt    45960
ttggggtatt ttttttgttg agaggtaatt ggattttgtt gggagtgggt ttagaagttg    46020
aggtttagga agaggttttt gagttttttt tttttgttag gtgtagttat atatgtgaaa    46080
gaatagtttt tgttttgatt tttagggtta ggaatggttg ttaggtttta gttgtttgtt    46140
tgaagatgga gattagttag tgttagtatt ggtattaagg ttgtttttgt ttttgatttt    46200
tttaatgttt ttgggagtgt ttaaggattt ttttttgaat tttattttt tgtttttgag     46260
tatatatatt tttataattg tgggagagaa ttgttgttaa tgtatgtttt agttttttttg   46320
tttgttggta gggtagggat tttattttag agtttttttt tttggttatt tttgttaagg    46380
gtagagtttt atttttttgta tttttagttt ttttatttta gatttaatgt gtttaatttt   46440
ttttttagaa ggattagata ttttttggtt ggggagattt agttgttgag agagggaaag    46500
aataaatatt gatttgttgt ttattgtttg tggttttgtt ttgagtagtg tttttatttt    46560
aggtagtatg gagtggtgtt ttgttggtag ttataggttg tttttggttt tagtttgtag    46620
tttatgattt agagtagtgg tagggatttg ttttgttttt agaattgggt gtttttgagt    46680
ttttagtggt ttggagatag atagggatgg aaatgtgggt gggggttggg aggggtaggg    46740
gtagtggggg tttttgtttt tgtttttttgt tgtttttgta gaggtatatt tatgatttag   46800
taaggttttt ttgttattat tttaggatgt agatgaggaa attggggttt gggaaggtgt    46860
ttgttgttag gttatattgt tatgggttag gggtagtagt gggttaagtg gttggttttt    46920
tttgttagtt ttaaaggtta gtgtaagtag aggaagggtt aggttggttg tgggggttta    46980
gtaggatata gagagagggt ttaggggagg ttaggttttt ttgttttttt tttggatttg    47040
aaggtattta attaagaggt ggggagtttt ttaggttttt tttttgttta atgtattagt    47100
ttttttagag gttttattgg taatttttat taaaatatga aaatttaatg tagtggagag    47160
agagtatgag ttttttggtt atgtaggatg aatttttatg ggttttttag tttgtgattt    47220
gtttttattt aatagtttg aatgataggg atgagttggg gtttgtattt ttgggttggg     47280
aaggatagtt agtatggata gggttgagag tttttgttgg tttagtatgg aaagagtgtt    47340
tgtatgagag ttgtgtgtgt ttatgtgtgg agttgtgtgt gtttgtgtgt ggagttgtgt    47400
gtgtttatgt gtggagttgt gtgtgtttgt gtgtggagtt gtgggtgttt gtgtgtggag    47460
ttgtgggtgt ttgtgtgtgg agtagtgggt gtttgtgtgt ggaattgtgt gtgtttgtat    47520
gtggagttgt gggtgtttgt gtgtggagtt ttgggggttt gtgatttgaa tgtttgattt    47580
tattttaatt ggaggtgaag aggaaggtgg aggaagtggg gaagggttat tgaatttttt    47640
taaatttttt tttagttttg ttttatgggt ttgggagtat gtggtttatt tgaggttata    47700
tgggtatgtg tttttggggt atgattatat tggtttttaa agaagatgtt ttttgtgtgt    47760
gtgtgtgtag agtgtaattg tagataagta tatgattgtg aatgtttgtt agtagttatt    47820
tatgaattag ttttgtgttt gtgaaagaag gaatttgaat atgagaaaat atttgttatt    47880
ggttgttttt ttaaagttat tttagttatt aaggttttg ttgtgtttta gtttttgttt     47940
ttaggtttag tgtttttttta ttagtttgga ttagggtaaa ttttttgttt ttaatttttt   48000
ttattagggt tggatttgaa ggttaaggtt tttgggtatg tgtttaggat ttttggttta    48060
gagaaggttt ttttttttt gtgttttttgt ttttttttt gttttggggt ttaggaattt     48120
tgatttttg gaagaagaga aggtttaggt tattgagttg ttttggttag agatgttttt     48180
gagtgttttt ttttttggg taatggtggt tgtgttttgg ggggtttagg tttgtttttt     48240
```

```
gtggttgtgt ttaaggtagt ttggaggtta tttaggttag ttttttggg ttttttgtgg 48300
gtttaaatta ggatttagat ttttttttt tgttatagtt ttgggttatt tgggatttgg 48360
gattttttgt ttttttttt attttgtttt aaattattgt ttagaggttt gttgttttta 48420
agaattgttg agggagaagt tttaggggaa ttggtttgtt tgtattttta ttgttagtag 48480
tgtttagggt tttaggattt gggttttgtt agattatagt gtttttggg attttagggt 48540
ttttggtttt gtattgtgtt gtgttagtgt gtttggattt tttgtgagtt tttggttaat 48600
tatgttttgg gtttttgtt tggttttgta tttgtggtgt ttttatttgt ggtggtgttg 48660
tttgaggagt tttttagttg gtgaggagtg aggtttttgt agttttgttg ttttgtagtt 48720
ttgtagtttt gtagttttgt agttttgtag ttttgtagtt ttgtagtttt gtagttttgt 48780
agtttagtgt tagagtgttg tgtggagatt ttttgttgtt gtatgttttg ggttggtaag 48840
aagtatttgt tttaaatttt gtttagtggg tagtgtaaag tgagtggttt ggggaaatgt 48900
tgaagggatt gggaaaaata aattttaagt tgagtttaat agttgaaatg ttttgtgttt 48960
aggttggtgg ataagaagga gatagaatag attttttggt ttttttgtag tgattgattt 49020
tgtttattga ttagagttat tattttgttt ttagtttgtt aatttatatt ttttgtaagt 49080
tattagtttt aagggatgtg gaagttaagt attttgtggt ttttagtatt aggtgatttg 49140
tatgtgtatt tattgtagta gggaatttgt aaggttgtgt tgtggttgtt gttgttttt 49200
tggagtttga ggttgttgtt tgggggtgtt ttgtaggtgt tttgttgtaa gtaaagatgt 49260
ggttgtgtgg gtggttgggg ttgtggttgt tgttgttgtg gttgtggttg tggtggtggt 49320
tggggttgtg gggttgatgg ttggggttgg aatttgttaa aggagttttg tgttttagtt 49380
ttatttttta ggggtgttgt taggttttgt tgtttagtgt tgtaatgggt ttggtttttg 49440
gtgtttttga attttgtgt tttggtggtg gttgatagga aagttgtgtt gaatttgttg 49500
tttttgggaa atgttttaaa aggtgatgag ttttttgat tttgtgatat tgggttgtag 49560
taggtgttta tggtagtggt tggtgatttg tagtaagaga tgtaagtttt agtatttatg 49620
tttggtttgt gtaggtggtg ggtggggatg tgagtgaggg tgtgaggatg ttattgtgtg 49680
ttttggttgg gagtttgggg aggtgaggag gttgtggttg tgtattttttt gtttggtttg 49740
tttgtttttt ttttttttt ttatgttggt tttttttttt tttttttttt tttttttttg 49800
tttttttttt tttttttttt tttttttttt tttttttttt tttttttttt tttttttttt 49860
tttttttttt tttttttttt tttttttttt atagttagtt gagggaaaga atggagggta 49920
gtaaaaagat ttagatgttt tggaaagttg attagatttt ttaaattttt ttaaggtttt 49980
gtagtggaaa aaaaaaataa gtaaattttt tttttgttt tgttttttt tttttttttt 50040
ttttgttttt tttttttttt ttttttattt ggtttttgtt tttttttttt tatttttaag 50100
gagatgttat taatttgtta gtggggattt aatttggagg tgtttgaggg ttttttgat 50160
tttaggtggg gttggggaga tgttaggatt tttagggttt ttgaggttag gttgagaatt 50220
ggtggggtgt tggagttttg ttttagggta aagggaaatg taaattttaa tgataggtta 50280
aaaaaaagtt ttttaaaata aatttaatag gttttgatat ttgaagttag ggggattatg 50340
ggtttgtgga tgtaagtgag ttttatggat ttttgtgtgt gagtttgtgg gtgttgttgt 50400
gggttttttt gttggtattt tttttaggt ttttaatttt ttttttttatt tttgttttta 50460
tgggagatta atatatatat aagtttttgt tgtttagtgt atggttattt gtatgttttg 50520
ttaattattt tttttgttg taagggtgtt gggttgggat gtagtggaat ttgtgttttt 50580
ttaagttttg gagaagtttt gggttgggtt tggttttaga agttttagag tgttgttttt 50640
tgtgttttg tttagaggtt gtattgttta tgttttatgt ttgtgtttag gtgaggggat 50700
ttttgttag gtgaagtata gagagtggat tttggagatt ttttgatttt gtttgatagt 50760
tgtgttgtta tttgtttatt ttttggggtt aggggttggg gagaggggtg agattgtttt 50820
tttttttttt ggtttattg tttttgattg taaaatgaat tgtttggttt tgataggttg 50880
tggttttatt attattgtga gttttaaggt ttttggtatt gttatagtaa gggaaaaaat 50940
tttgtgtgtg tagttttttg attttgtttt atgataattg tgttagagat tggtgttttt 51000
aattttttga tgagttaatg gaggtttaga gaggggtggt tatttggttt aggttatata 51060
gttgggaaat agttgttgtg ttttggaatt tttgtttagg ttagtttttt tattgatttg 51120
gaaggttgtg tggatagtgt agatgtagag gggaaattgg tttttggttg tggaatggtt 51180
taagttgaag gagattagtg agagtagttt tttttaaatt gttgagaaaa ttgttaagtg 51240
agaatgtatt tttagggagt ttgggagtag ggtgagaggt aaaagtgtaa ggtaaaatta 51300
aagagggttt gagtattgtg aattaaggaa tgtgggttag atgttaattt tttgtattta 51360
aggtttttta aagaaaaaat atatttgggg ggttgtgttt ttgtttttaa ttttatttgg 51420
gttaattttt attttattt tattttttta gtatttttgg tttttgtttt gttgttgatt 51480
gtgtgatttt atatttggtt tttattgttg gtttatttg aagtgtttgt taataaagat 51540
atttgtaaa ttaagttagg agtgtgtagg ttttagttgt tttttatttt atgtttaatg 51600
ggaattgtgt tttattggtt tgtagattta agttatttga gagtttttta tttattgagt 51660
ttttgttgtg ttaataattt ggtaatataa ggtttattat atttattttg tatatgggta 51720
```

```
gattgagatt taattaaaat aattttttag ttagtaaaaa gtggagtgtt tttgttatta    51780
ttttaaatgt ttttgtttag tggtttttt aagtttttag aatttgttaa ggtttttagt    51840
ttgtgtattg tagtataggt ggttttttt atttggaatt ttttaaattt atttttgtat    51900
ttttgtttgg ttaatgattt agagtttagt ttagagtatt ttttttagg aagattttt    51960
gttttttta gtttaagttg atgtttttgt ttgttttatt tttgttttag attttgtggt    52020
tgtttggttg tttggttatg agtgtatttt aattttatta gttgttttag ggtagttttt    52080
tatgtgtttg gtattttata aatatttgtg gaataaaaga ttaaatttgg attaggtgtg    52140
gtggtttata tttgtaattt tagtatttta ggaggttaag gaggatggat tataaggtta    52200
ggagattgat attattttgg ttaatatggt gaaattttgt ttttattaaa aatataaaaa    52260
ttagttagat gtgatggtag atgtatgtag ttttagttat ttgggaggtt gaggtaggag    52320
aatggtgtga attagggagg tagagtttgt agtgagttga gtttgtgtta ttgtatttta    52380
gtatgggtga tagggtgata gggtgagatt ttgttttaaa aaaaaaaata aataaataaa    52440
aataaaataa aataaagatt aaatttgggg ttgtaggatt ttattatttt agtgtatttt    52500
aattgtttag tgtagtatta agttttgaga ttttttttt ggttttttt agttaaggat    52560
agttggttat ttatttattt tttttttaaa aattaagtag tggaattaat agtgtgttgg    52620
attttt                                                               52626
```

<210> 8
<211> 52626
<212> DNA
<213> Artificial Sequence

<220>
<223> chemically treated genomic DNA (Homo sapiens)

<400> 8

```
gaaggtttag tatgttgtta attttgttat ttgattttg aagaggggt gggtgggtga      60
ttagttgttt ttgattaagg gaaattaaag aaagagtttt agaatttggt gttgtattga   120
gtagttggaa tatattgagg tgatagagtt ttatagtttt aggtttaatt tttattttat   180
tttattttta tttatttatt ttttttttg agatggagtt ttattttgtt gttttgttgt   240
ttatgttgga gtgtagtggt ataaatttgg tttattgtaa gttttgtttt tttggtttat   300
gttattttt tgttttagtt ttttaagtag ttgggattat atgtgtttgt tattatgttt   360
ggttaatttt tgtattttta gtagagatag ggttttattg tgttagttag gatggtgttg   420
attttttgat tttgtgattt atttttttg gtttttaaa gtgttgggat tataggtgtg     480
agttattgtg tttggtttag gtttaatttt ttattttata aatatttgtg gggtgttagg   540
tatgtaggag gttgttttgg ggtaattagt ggggttgaga tatatttata gttaggtaat   600
tagataatta tgagatttgg gatggagatg aggtaaatgg aagtgttaat ttaaattagg   660
agagatggga agttttttta gaggaagatg ttttaggttg aattttgaat tattagttag   720
ataaaagtgt aggaatgagt ttagggaatt ttaggtaggg gaaattattt atgttatagt   780
gtataggtta gagattttgg taggttttag gaatttgaag aaattgttgg ataggagtat   840
ttaggatgat ggtaggagta ttttgttttt tgttaattgg aaaattattt tggttgagtt   900
ttagtttatt tatatgtaaa atgggtatga taagtttgt attgttagat tgttggtata    960
ataggagttt aataaataaa ggattttaa gtggtttagg tttgtgggtt agtgaggtgt   1020
ggtttttatt gggtatggag tggggggtgg ttggagtttg tgtgttttg gtttggtttg    1080
tggaatattt ttattaatga gtattttaag gtaagttggt gatggaaatt agatgtggga   1140
ttgtgtggtt ggtggtggag taaggattag agatattaag ggggtagggt gggggtaaga   1200
attagtttag atgagattgg gagtagaagt gtgattttt aggtatattt ttttttggg    1260
agattttaga tgtagaaagt tgatatttga tttatatttt ttggtttatg gtgtttaggt   1320
ttttttaat tttattttgt atttttattt tttattttat ttttagattt tttagaggta    1380
tgtttttgtt taatgatttt tttagtggtt taaggaaggt tgtttttgtt ggtttttttt   1440
aatttgaatt attttatagt tggggttag ttttttttt gtatttatgt tgtttgtatg     1500
gtttttagg ttggtggagg gattagtttg agtagaggtt ttagggtgtg atggttattt    1560
tttagttgtg tgatttggat taggtggttg tttttttttg agtttttgtt agtttattag   1620
aaaattgggg gtgttaattt ttagtatggt tgttatgaag tgaaattgag gggttgtatg   1680
tgtaaagttt ttttttttgt tgtggtggtg ttaggggttt tgaggtttgt agtggtggtg   1740
aaattataat ttattaaaat taagtagttt gttttatagt tgggggtagt gaggttagaa   1800
agggaagaga tggttttatt ttttttttg atttttgatt ttaaggagtg aataggtgat    1860
```

```
agtgtgatta ttgggtaagg ttagggggtt tttagagttt gttttttgta ttttgtttgg    1920
taggaaattt ttttgtttgg gtgtagatgt ggggtgtgaa tggtgtggtt tttagatggg    1980
agtgtagagg gtagtgtttt aaagtttttg aagttgggtt tagtttaggg tttttttagg    2040
gtttgaggga gtatagattt tgttatattt tagtttagtg tttttatagt agagggaaat    2100
agttaataag atgtgtaagt gattatgtat tggatgatga aggtttgtgt gtgtgttaat    2160
tttttgtagg aatgggaatg gggaggaagg ttggaaattt aggaaaaggt gttagtggga    2220
aggtttgtag tggtgtttgt gagtttgtgt gtaggagttt gtaggatttg tttgtatttg    2280
taggtttgtg gttttttttgg ttttgaatgt taaaatttgt taggtttatt ttggggggtt    2340
tttttttaat ttgttattaa aatttgtatt tttttttgtt ttaaggtaaa gttttgatat    2400
tttgttaatt tttagtttag ttttgggagt tttggggatt ttggtatttt tttagttttg    2460
tttagaattg gaaaagtttt tgggtgtttt tgggttgaat ttttgttagt gagttagtag    2520
tattttttta agagtggaga ggagggggta ggggttagtg gaaggagaga ggaggagggg    2580
atgagagagg gggaaaggaa gaagtaaggt agggaaaaaa atttatttat tttttttttt    2640
tgttgtaaaa ttttaagagg gtttgggaga tttggttaat tttttgaaat atttgaattt    2700
ttttattgtt ttttgttttt ttttttggtt agttgtgggg agggaggaaa gagagagagg    2760
gagagagaga ggaagggggg gggagggagg ggaagagaga gagaggaggg agagagagga    2820
gagagataga gaaagaagag ggggagagag agagaggttg gtgtggaggg gaggagggag    2880
agtgagtagg ttgggtaagg agtgtatagt tatagttttt ttgttttttt aaatttttag    2940
ttaaggtgtg tggtggtgtt tttgtgtttt tgtttgtgtt tttgtttgtt gtttgtgtaa    3000
gttaggtatg aatgttgaga tttgtgtttt ttattgtgag ttgttggttg ttgttatgga    3060
tgtttattat agtttggtgt tgtagagttg ggagggtttg ttgtttttta gggtattttt    3120
tggaggtgat aagtttggta taattttttt gttggttgtt gttaaagtat agggatttgg    3180
ggatgttaag agttgggttt gttatggtgt tgggtagtag gatttggtga tatttttgga    3240
gagtggagtt ggggtgtggg gttttttttaa taagttttag ttttagttgt tgattttgta    3300
gttttagttg ttgttgtagt tgtagttgta gtagtagtag ttgtagtttt agttgtttgt    3360
gtaattgtat ttttatttgt agtgaggtgt ttgtaagatg tttttggatg gtagttttaa    3420
attttaggaa ggtagtagtg gttatagtgt ggttttgtag gtttttttgtt atggtgagtg    3480
tatgtgtggg ttatttggtg ttggggatta tggggtgttt ggtttttgta tttttttgaaa    3540
ttggtggttt gtgggaggta tgagttggtg ggttgggggt ggggtggtga ttttggttgg    3600
taaatgggat tgattgttgt gagggagtta agggatttgt tttgtttttt ttttgtttgt    3660
tggtttaggt atggagtgtt ttggttgtta aatttgattt ggagtttgtt ttttttggtt    3720
ttttggtat tttttttaaat tgtttgtttt gtgttgtttg ttgggtggga tttgaagtgg    3780
atgtttttta ttggtttagg gtgtgtggtg gtaaggagtt tttgtgtagt gttttggtgt    3840
tgggttgtgg ggttgtgggg ttgtggggtt gtggggttgt ggggttgtga ggttgtgggg    3900
ttgtggggtt gtgggatagt aggattgtag aagttttgtt ttttgttggt tgggaggttt    3960
tttgggtggt gttgttgtag gtgagagtgt tgtaggtgtg gagttaggtg ggaagtttgg    4020
agtgtggttg gttgaaagtt tatgggaaat ttaggtgtat tggtgtaata tagtgtggag    4080
ttagagattt tagggtttta ggggatattg tggtttggta agatttgagt tttgggattt    4140
tgagtgttat tggtagtggg gatgtgggta ggttagtttt tttggagttt tttttttggt    4200
gatttttgga ggtggtaggt ttttgaataa tggtttagga tagagtagga agggagatag    4260
gaagttttga gttttaggtg gtttagagtt gtggtaagag agaagggttt gggttttgat    4320
ttgagtttat ggggaatttg ggggagttgg tttaaatgat ttttaagttg ttttgaatgt    4380
agttatagga ggtaggtttg ggttttttag agtatagtta ttattgtttg ggagagagag    4440
atatttaagg atgtttttag ttagagtagt ttagtagttt aggttttttt tttttttaga    4500
gaattggggt ttttgaattt taaagtggaa ggggaagtaa agatgtagaa agggaaaaat    4560
tttttttggg ttaggagttt tgagtatata tttggaggtt ttggttttta ggtttggttt    4620
tggtgggaag agttggggggt agggaatttg ttttgattta ggttgatgga gaggtattag    4680
gtttggggat agaggttggg atatggtgga ggttttggtg gttagaataa ttttgggaaa    4740
atagttgatg gtagatattt ttttgtattt aggttttttt ttttataggt gtagaattag    4800
tttatagatg gttgttgata aatatttata gttatatgtt tgtttataat tgtattttat    4860
atatatatat gtagagagta ttttttttag gagttgatgt ggttatattt taagaatata    4920
tgtttatgtg gttttgaatg agttatatat ttttaaattt ataggataga attgagagaa    4980
gatttagaaa gatttagtgg ttttttttta ttttttttat tttttttttt atttttagtt    5040
ggaatgagat taggtattta gattatagat ttttaaggtt ttatatatag atatttatag    5100
ttttatatgt aggtatatat agttttatat atagatattt attgttttat atatagatat    5160
ttatagtttt atatatggat atttatagtt ttatatatgg atatatatag ttttatatat    5220
ggatatatat agttttatat atagatatat atagttttgt atatggatat atatagtttt    5280
tatataggta ttttttttgt gttaagttag tagagatttt tagttttgtt tgtattggtt    5340
```

gtttttttta gtttagggat gtaggtttta gtttgttttt gttatttagg attattaggt 5400
gagggtaggt tgtagattag gggatttatg gaggtttatt ttgtatagtt agagagttta 5460
tgtttttttt ttattgtatt gaatttttat attttaatga aaattattag tgagattttt 5520
gaagaggttg atatattagg taagaagaga gtttaaggag tttttttattt tttggttaaa 5580
tgtttttagg tttagaggga aggtggagag gtttggtttt ttttgagttt tttttttgtg 5640
ttttgttggg tttttatagt tagtttggtt ttttttttat ttgtattagt ttttggagtt 5700
gatggaaagg gttagttatt tgatttgtta ttgtttttaa tttgtggtag tgtgatttga 5760
tagtaaatat ttttttaaat tttggttttt ttatttgtat tttggggtga taatagaaag 5820
gtttattgg gttataaatg tgtttttgta gagatagtaa gaggtaagag taagagtttt 5880
tgttattttt gtttttttta atttttattt atatttttat ttttatttat ttttgagttg 5940
ttgggaattt gagagtattt aatttgagg atagggtaag tttttgttat tgttttgggt 6000
tataaattgt aggttgggat taaaggtagt ttgtaattat tggtaggatg ttatttttatg 6060
ttgtttaaga tggaggtatt gtttggggta aagttataaa taataaatag taagttagtg 6120
tttgtttttt ttttttttta gtagttgagt ttttttagtt aggagatgtt tggttttttt 6180
aggaaaggga ttaggtgtgt taggtttaag atggagaaat tgaggatata aaagatgagg 6240
ttttgttttt aatagaagta gttaggagga gggatttggg agtgggattt ttattttgtt 6300
agtaagtagg aaaattgagg tatgtattga taatgatttt tttttatagt tgtgagagtg 6360
tatatattta aaagtggaga aatggagttt aaggaaaaat ttttagatgt ttttaggagt 6420
attgggaaag ttggaggtag gggtggtttt ggtgttagta ttggtgttgg ttgatttttg 6480
tttttaggta ggtagttgag atttggtaat tatttttagt tttggaggtt ggggtgaaag 6540
ttgttttttt atgtgtgtaa ttatatttaa taaaaggggg aagtttggag gttttttttt 6600
gggttttaat ttttgggttt atttttggta gagtttaatt gttttttggt aaggaaggtg 6660
ttttgggtag ttgagtttgg gttaagttga gggagtgtgg ggtggggaag ttgtttttag 6720
tttttgttgg ttgggtttgg attgggttgt ggaaagagtt ggtgagagaa atttttttgg 6780
gttgtaagtg tttttatttt aatttgggtt tttttttttt taaatgtgtt aaaagaatga 6840
tgattaaggt ggttttgtag tgttttttgg gtgtggagat tgtgttttta tgaaagagtt 6900
tttattgagt tttttttttgt tgtaatttaa gattttatta tttttttttt tagattttgg 6960
gtttgttttt tggtttttttg aatttttttgt ttgagtttag ggtgggttgt ttgggttata 7020
ggagatatgt tgggttggag atttgatgtt ttttagtgat gtagagagtt tttttgtatt 7080
tagggtaggg attttggtgt tttgttttta aattttggat ttgattgttg gagttgtagt 7140
tggtgtgtat tggttttagt ttatttgttt tgagatgtgg ggttgttgtg ggaagttgtt 7200
tttttttttt tttttttttt gtttttgatt tggtggtgat aggtttttgt tgaaagtaga 7260
ttgttatttt tttgtttatt tatttgtaaa agtagtgttt ttagtgttaa ggtttgggga 7320
ggtgtgggtt aggttggagt ttttggggtg ttgtttaggg gtttagtttt gatttttttga 7380
attagattta gtggttaaat attagagggt atttattttt tttgtattga tataatttat 7440
gtattatgaa atgtgtaaat tttgttgggg ttgtatttga atttagttag agaattgggg 7500
tgtgttttgt atataagaga tgatttaaag aaggtagaaa tgaaaatttg atagaagtag 7560
taatagttgt tgtgggtgat tgtggggtga ttgtaggaaa atttgagttt gggattgaag 7620
tttgtttagg aaggggtgat tgttgtttaa ttttggaggg agggatggtg aaggaagatg 7680
tttttaattt tttattttgt taattttttat agtttgtttt attagtttgg tttaaatata 7740
aaagttgtta aatagaaaaa tagagggttt ggattaaaat ttaatatttt aagttaattg 7800
atttgtatga ttttgtataa tttttttttt attataattt atatatagtg aagtgtttag 7860
ttttgaggag gaaagttgga agaattgtta tgtatgtgta tattgttagt taggatgatt 7920
atttgataa agaaatagat tatttatatg ttttttttaaa tgttgtaaaa tgttaaagta 7980
aaaatatttt ttgtaagttg ttttaagaaa attttagaag atattttgga gtattgggga 8040
ttttattttg tttttgatag tagtgttttt tgaatagggt gatattttag ttagggtatt 8100
tgtgtggttt tgattttaat gtgaagtttt aagtggttgt gttaggaatt ttgttgtgat 8160
tgttgggtta agttggagtt attaagtttt gagttgtatg tgttgtgatg ttggtatgta 8220
gtaggaaaat agattaaaat gttttataga aaattttggt gaagttttgg aggattttgg 8280
tttttaagat tagttgggtg tatttttttt gggatgtttt tttttttttgg ttttagtgtt 8340
tttttgtttt tagttgtgtg tagttttgtt ttggtggtaa attgaaataa gaaatggaaa 8400
tatattggtt tttgttgttg ttagggatga gaggttgttg atgtttggtg tgtttgtttg 8460
ggttttgggt ttttttgtag atttttggat tggggtgttt gaggttagga gaggaggggg 8520
atagttgttt ggagtttttg tggtttagag gttttgggat gatttatggg gggggtgtgt 8580
tttgttttgg tgagtttttt gttttgaggg taggtgaggt gggtgggtag gggagtgtat 8640
gttggagaga agagagaatg tttaattaga gagaattttt tgtttttgga gttttagtgt 8700
ttttagtttt ttaaattttt gtttaggaag ttgaaggatt taggtttagg taatggtttg 8760
gggtggggtg gtaagagtgt tgttttgtat ttggatgttg tttgttaggt gttatttggt 8820

tatgtgtgtt tgtagtgtag ggttttttgg ttattagtat aggtttttat tgaggtgata 8880
gttattggat tgggaaatta atattgtgag gatttggtta tgtgatgata ttgtttgggg 8940
gaatttgagt ggaagttgat tgttttgtgg tgttattaga taggtgagaa gtagtttttt 9000
taaatagggt tttttgttgg aaggaggagg tatttttttt tagttagtga gtttttttag 9060
ttgtaattgg ggtttttttt aatattagtt attgtggttt ttagaggtgt agtttggtat 9120
attttttttt tgtagatgta taaattgggg atatttttat ttttatttaa gatgtttttt 9180
ttttatttag tagaggggtg tggagtaaat ttgggataat aatttgtgtg ttgtttggaa 9240
gtaggttttt tagaaaggat gataaaaatt tggtgatgtg gaagaagttt ttattgtgtt 9300
taggaaaggg tagtggtttt ttagttgtat tggttttggt tttggttttt atttttagta 9360
tgttttgtta ttttaataaa gagtggtttt tttatttgat tttaatttgt attagttagt 9420
gttgaggaaa gagaggaatg gaagggagtt gtggtttagt ggtgaggaag agttatgtag 9480
aggtaaggtt ttgaggagtt aaggttgggg tgtgtagggt ggtatttagg gttgtggatt 9540
ggtttggatt tggattttag aggtagttgg aagggttatt gtggtgtttt gtggttttta 9600
gggtgttgtt ttggtgagga gggggttatt gggagatatg ggtggggtaa gaggggttat 9660
tggttgttaa gtatttattt tttttggtga aggtgttgtt tgtatttggg attgtttggt 9720
taaggttgtt gggtattggt gttgttgtat attgggggta gagagggagt tagtgtttgt 9780
tttattggat ttttaaagta gatttttttaa gagtggattt ttattttgaa ttggaggttt 9840
agtatggatg ttggttttat gtattttgta gagggatagg atttggtgat ttaggttaga 9900
gtagaataat tggtttttta gggttttgaa ggttataata gagaaggttg ggaagttgtg 9960
ggaggtttgg attatgatat tgatgtttag tgtggtatat gtggggattg gggtttatag 10020
ttgtggtgta gattatttta gtttttattt ttgtattttt tattttttta tggttttgga 10080
gaagtatgtt ttgttttggg gtttgaattt tagttgtatg aaatgtagga gtgttgataa 10140
tttttaaaga ttttttttgga gtttttgtgt tggtagtagt ttgggtttat ttttattttt 10200
tttgtttttt tttttttattt ttatatgtat ttttgtttag gagaaattgg ggtaaggtgg 10260
gtggaaaagg tttttttgga ggttaatata ggttttgagt ggggagttat tataggatat 10320
aaagttggtt agaggtttta tagggttatg gtggaatgta ggttttgggt ttaggattgg 10380
gatgtaaata gggagggggt aagtaagtgt ttttgtgatt aggtttaagt tgaggttttg 10440
ggtttatggt ttttgtgttt atttattttt tttattttag tatttatgga gtattttttt 10500
tgggtggatt ttttttttagg aggtagaata tgttaagttt ttgtttttttg tgttatggtt 10560
tatgtttttt taagtgtttt gtatagttgg gtttaataaa tgtttgttgg ttggaagaat 10620
gaatgaagaa ttggtgtttt ttgtagtgtt gtttttatat ggttaagtgt atttaggagt 10680
tagtttaggt gtttgtgtga gtgtgtgtat agtatgaatg tgagtggtgt gtgttgtgtt 10740
tatttttttt gggtttatgt tttgttattt gagtgtttta gggtgaggtt gagggtttgt 10800
gtgtagtttg gtgaaaggta tgtttgtttt ttggtatttg tgtttgtgtt ttttagtgtg 10860
tgagttttag gtgtgtaagg gtgagtattt gtgtgtatgg ttttggagtt tttttgggtt 10920
tgtgggtggt atagttttttg tttttagagg tgtttttagg gagaatgatt aagtgttttt 10980
gtattatggt ttgtggtttt tttgtttttg gttatggttt ttaggagtag ttgatttatt 11040
gagtggtagg tagaattttt ttgtggtaag tttttggtgg ggtttaatta tttttttgtgt 11100
tattttgggg tttttgaata tggtttattt agtttattta tattgtggtg aggttgtttg 11160
ataagttatt agttttggaa tatatttttt gttttgtgtg gtttattgtg gaagagatgt 11220
gtttttggta ttgtttgtgt ttattgttga ttttatttag tatatgagta aattgaggtt 11280
taaggggaaa tggattttta tagttaggtt agtaggaggg taggaattta ttgagttttt 11340
ttttttttat tataatgggt ggaaaggtag aaagtgttgg ggaatttaat ttatttattt 11400
gttttttttgt tggatgtgat ttttgtaata agtttagtaa gttgtatttt ttttgtgtat 11460
taaatagggt agatgtttgt agttttgttt tagtttttttt agaagtaaag tagattttgt 11520
tatgagtttt tgggtattag aggatagtag gagagtattt gtggaatttt tggtgggatt 11580
gtagggattt gattttaatg tagttttatt aggttgggat ttttgttttt tgtttagttt 11640
tttaagttgg gttttttttt tttaagtttt tgaaaattat aagttagggt tagttgaggt 11700
tagatgatta atttttttta ttgtgtagga ttgtttatta ggttttgagg gtttttggtt 11760
atattgagga aattggtatt tttttgatt ttgtagtata tatataatgg gttatggtga 11820
tatgttgggg ggttttattt aaggttggaa gagatggtag ttattttttg gtttatatgt 11880
tttattttat agataaaggg gattgagagg tttgatttta tttatgtaag gtttattaaa 11940
ttttgtttgt tattatgtta agttttgtgg ggtattttgg gaagttttgg atgtagtttt 12000
tgtttttagg tttagaggta gagatagata agtgattttg aaatttttagt ttagtgggat 12060
gggttttgag atggggaaag ttttgattta ggttttgagg agaaggttag gttggtttgt 12120
tttggggtgt gtaggtgtta ttagggtttg gtagtttatt ggaagttatt taaggggagg 12180
gtgtaggaaa gttgggggaa gttgggattt gtttaggtta atgagttttt tttagtggtt 12240
ggaaattttg aggttttttt ttttttgatt tttttttttt tttttttttat gttttttttt 12300

ggttttttta tggaaatagt tatgtataat agttttttta ttttttagt gttattttat 12360
tttttttgt gttgaatggt attttggttt tgttttattt gggaggggag tatgaggagg 12420
atgtgatgat ggagagaggt agtgtgtttg ttttgtatat tttgtattat taggtttttt 12480
gtttatttt ttatattata ttttttgagg gtgttaggat attgtaaatg atagtggtgg 12540
ggttttgatt aggttttaga gtgtgtatgt agagtttggg gtttttttgg atatgtgagt 12600
tttttgatg tttttatgtg gttataagga tttatttata tttggtttat attttagtat 12660
ggagatggga taattttagt tttatggttt ttgattttaa ggagggagtt tatttttaaa 12720
tggggaagat aaatatgtat atagttttat tgtatggttt ttttgttgtg gttttagttt 12780
ttttattttt gaattttttt tatgggattt agtggtttta gaggttttgg ggttttgtga 12840
tatttattaa gaggggtgaa ttggtttttt agaattttta agatgtttta ggattttata 12900
ttttttttta tttattgtat taggaaaggg ggaaggttgg tttagtttgt taaaggttaa 12960
gagtttggga tgttaggttt ggtttttggt ttgagttttt ttatttatag gatagagata 13020
ataatgggtt ttaaggtaaa gaaggtttag agttggttgt tgttgtagaa ttatgagata 13080
gtttagaata attgagtgtt tttgtttgtg tgttaggtag tatattagtg ttgtttatgt 13140
atgattttat ttaattatta ttataatttt atgagattgg tattattatt atttttattt 13200
tgtggatgaa aattgaggtt tatggaagat aagtaatttg tttatagtga gatagttagg 13260
aaggaagagt tgagttttgt tggttgaggg ttttaagaaa tttaaaaggt attttgtttt 13320
agaatgattt tgattagatt tgagataatt ttaattttat tttagatgat gtggagatga 13380
aagatagggt tttataaata tggtttttag ggtatggtgt agtggtttat gtttgtaatt 13440
ttagtatttt gggaggttta ggtgggtgga ttataaagtt aggagattga gattattttg 13500
gttaatatgg tgaaattttg tttttattaa aaatataaaa aattagttgg gtgtggtggt 13560
gggtgtttgt agttttagtt atttgggaga ttgaggtagg agaatggtgt gaatttggga 13620
ggtggagttt gtagtgagtt gagattttgt tattgtattt tagtttgggt gatagagtaa 13680
gatttatttt ataaaaaaaa aaaaaaaaaa aaaaattatg gtttttaaag tgtttttttt 13740
agttttataa ttggggatgt agaagataag tagtgttagg agtaaatttt ggtatttgtt 13800
aggttgaagt tttatatttg attttgtgat tttgggtata gtttttttat ttgtattagg 13860
tgaaaatagt gttttaggta tggtataggg ttttatagtt ggtatgagga atgattgtat 13920
gagttttttag aagttttagg aatatgagag aatttttgat taaggtaggg tggagttgag 13980
gagtatttgt gggaatagtg tttttttagga aatatgagag attgtaggag gttttgtttg 14040
ttttgatttt aatttttttt agagtttgga gttagtttat atagtagatg tttaataaat 14100
gtttgttgtt gagtgaatga atgaagggat tttataggag gggaatatgt taggaatttt 14160
gtaagttttt ttattttttt tttgtttatt ttttttttga atggaggttt ggggtgagag 14220
tttgttttgt tgttttttttg tattttggta gaaaattatt gatttttttt tttttgtagg 14280
gtttttttgtg gggtgtagga gtagtgaggt tttgggtagt tttatttagt ataagttttg 14340
gttttttttta attttagtag ttggaggagt attttttttt tttattgttt ttttttttggg 14400
ttttatttta ttggttatag gtttgtggtt tgattgttat agtttttggg gttggtttta 14460
gttaaatggt tttttttttt tatttttaat tttttttttt aggttttttt tatttttttat 14520
agtgagtttt gtgttttgtt tgttggtatt tttatttttg ttatttgttt agggtgttgt 14580
ttgagagttg ttgaggtttt ttttttagtt ttttatttat ttatttttta ttttgggatt 14640
ttgagattta tttgtaaggg atgtagtttt tttttttggt taggatgtta tttgatttta 14700
tttgggtttt tagtagggta tttggtttta aggtttgggt tttttttagt atatttatga 14760
ttttgtttat ttgttttatt ttggtatatg taatttgagg gtatttgaat atttatggtt 14820
ttttgagttt tattaaaggt ttaggtggag gaattgaatt tttattttat agatggaagt 14880
taaagtttaa agagggggaa tagtttagaa ggaatgtatg gaggagtgtg tgtttggtaa 14940
aagttttttt ttatatttaa tagatttttt agtttggtat ttttgtgtta ggtagggatt 15000
agagtgtttg gtagtgaatg aattggtttt tgttttttaga gagtttatag tttggttaga 15060
atgtgttata tgttatggga atttttaggg aaggtgaatg gattggggtt ttttgagtat 15120
ggatatttga agtggatttt gaaagttgaa taggagttta ttagttgaag gagatgaaag 15180
aggtttttta gatttaggaa atagtataaa ttaagttttt gggtaaggga agaatatgaa 15240
gtaagtgtgt ggagtgtagg ggtatgatgt atgttggtga ggtgggttga ggaatggtag 15300
gggtatagtt gaagagagtt ttttatttta ttttatttta ttttattatt ttttgagata 15360
aggttttatt tggttattta ggttggagtg tagtggtgta attttagttt attgtaattt 15420
ttgtttttta ggtttaagtg attttttttat tttagttttt taagtagttg ggattatagg 15480
tttgttatta tgtttagtta atttttgtat ttttagtaga gatagggttt ttttatgttg 15540
tttaggttgg ttttgaattt ttagatttaa gtgatttttt tgttttagtt ttttaaattg 15600
ttgggattat aagtataagt tattgtgttt ggtttttggg ttatttattt tttattaaag 15660
ggtttagatt ttggggaagt tattggaggg ttttgagtaa aatagtgaga ttttaggttg 15720
agtgtttttt gtatggttta gtatggtttt tgttttttgt atttaaaatt tttattttaa 15780

ttaagaatga atttttatt gtggtaaaat atatatagta taaaatgtta ttattttaat 15840
atttataagt gaattttta gtggtattaa gtatatttat aatattgtgt aattattatt 15900
attgtttta gaattttttt attattttaa atagaaattt tatatttatt aaatgggaat 15960
tgttattata tttttgattt tttatttaag tgatttttat tttattttt ttttgtggat 16020
tttttattg taggtatttt tttttttttt tttttttttt gagatagagt tttatttttg 16080
ttgtttaggt tggaatgaaa tggtgtaatt ttagtttatt gtaattttta ttttttgggt 16140
ttaagtgatt tttttgtttt agttttttaa gtagttggga ttataggtat ttattattat 16200
atttgtttaa tttttgtgtt tttagtagag atggggtttt gttatattgg ttaggttggt 16260
tttgaatttt tgattttagg tgatttattt gttttagttt tttaaagtgt tgggattata 16320
ggtgtgagtt attgtatttg gttttaggta ttttttatag gtggaattat atagtatttt 16380
tttttggtgt ttggttattt gtgtattttt tttggagaag tgtatttaag tattttgttt 16440
atttttgaat ggggttgttt gtttttttgt tttttagtta taggaatttt ggatattaat 16500
tttttttag atatatgatt tgtgaatatt ttttttatt ttatggattg ttttttatt 16560
ttttggatat tgtttttga tgtatagaag ttttatattt tgatgaagaa gtataatttt 16620
ttaattttt tattttgttg tttgtgtttt tggtgttata tttaagaaat tgttaataat 16680
tttagtgtta ggaggatttt tgtttatgtt ttttttaag agttttatag tttagttttt 16740
aggtttaggt ttttgatgat tttaatttg aatatgatgt aatataaggt tttttaattt 16800
tatttttttt tatggggata tttatttttt ttagtattat ttgtagaaaa gatggttttt 16860
tttttattga ttggttttgg gatttttgtt gaaaattaat tgtgtatgta tttgagagtt 16920
tatatttggt ttttattttg ttgtatttgt ttatttggtt tttgaatgat tttttgatgt 16980
gaagtttagg attgtagaat gtataaggat ttttgtaggt tatttgttta tttttttgtt 17040
tgtaggttag tgattaatag gttatagaaa tttagtttaa ggatatattt gtttttgggg 17100
atttggagtt ggtgagaagg ggtggagggt agagttaaag gtgaggattt tagagttata 17160
gttttggttt tggttaggtt tttggatatt tgggggtttt tttgtttttt tagttgatta 17220
ttttatgagg atgtggtttt tggggaaatt tttttgtgtt ttgagggttt atatatttat 17280
tttttgagat gtgtatattt gggattattt tttttattaa gatgatttta gaattttttt 17340
agtggagttt ggaggagttt taagaaggaa gtatttagtg gttttatgtt gttggtttta 17400
tgtatatttt gaagattttt tgttagattt ttaaaagagg tatttgtaat tttttgtttt 17460
ttattttagg ttatttagtg aatggtataa ttaggggttg gggttttagg gattagatag 17520
tagagtttaa ggggagtata gttgtagaga agtagttgtt tttggttttt tttttttagga 17580
atttttttgg tttaggtgat taaatagagg tttgtttgag tttggttttt tagttatgtg 17640
ttttaaggta taattttag ggtttttgga aaattatgga aattgtttat tttataattg 17700
tgttatatag gttgtttgat gttttagatg tttttggttt ggagaagttt atgaagttta 17760
ggatttggaa atagatttta gtttatatag ttgagtgatt ttgggtaaat tagttgattt 17820
ttttgtttta gttttttat ttgtaaagtg gggataagaa ttatgtttat ttgttagtga 17880
tagggtgagg tttaaataag gtaaaatagg taaggataaa gttaggtgtg gttgtttata 17940
tttgtaattt tagtattttg ggaggttgag gtaggtggat tgtttgaggt taggagtttg 18000
agattagttt ggttaatata gtgaaatttt gtttttatta aaaatataaa aaattagttg 18060
ggtgtggtgg tgggtgtttg taattttagt tattagggag gttgaggtag gagaattatt 18120
tgaatttagg aggtagaggt tgtagtgagt tgagattata ttattgtatt ttagtttggg 18180
taagaagagt gaaattttgt ttttaaataa ataaataaat aaataaataa ataaataaat 18240
aggtaaggat tttagaatag tttttggagt atagtaagtg tttagttaat gttagttgtt 18300
atgattgata agatttttgtt ggaattttga ttttattaga ttggttggaa attttgatag 18360
gtagttaaag ttgattggga tgtatagagt tggaaaatga agaaattatt attgaattta 18420
tgattattgt gtggttgata ttgtttttta aaatatggat gagtgatggg aggggtgggg 18480
gttgttattt ttatttaatg tagtgggggt aggttttaag gagggataaa ggttatttag 18540
gtttttttagg tttttttttt ttataggttg ttgtattttg tatatatgtg ttatgagttg 18600
gggaataatt tttttttgt tgaatgtgtt ttttgtgttt atttgattta gaattttagt 18660
ttgtttttag ggtaggggtt ttgttgtgtt ttttttttgat tagtataggg ttgagtatag 18720
agaattgggt ttagggaatg ttaggtaatt tttgtatttt ttttgggttt tagtttttttg 18780
ggttttattt gtaattaaga aagtatttgg tgggtattgt ttttggttat atttggattg 18840
agatttttttg tggaaattta ttgggagata ttagtatttt taatttgaag atgaggaagg 18900
tgaggttgtt ttgttgtgaa ttgaatttag atttttttgaa gttaaagagt tttttttttt 18960
ttttgtttag gggtttatg tttattgttt ggatgttttt atgtttatgt gtttattaga 19020
tttgattagt atgtgagagt tttgtagatg gtggaggggg tgtattggga ggggttttta 19080
gtatattgta tgttgtatgt gggttatatt atttttgttt atttattgaa atgatgatta 19140
tgtttttttga ttgtttagat attatttttt ttttttttta gagttatttt gagaattagg 19200
ttagtttagt ttaggatttt tatgtgatat gtgggagtag tttttgtggg taaagaagga 19260

ggagataaat ttttttaaat agaatgagta atagtttttt tattagttta gtgttggtga 19320
agttaggggt aagaggttag tttggaaata ttatttgtat tatgtggtag gtggatagtg 19380
agggttaggt ttttttaggg aatgtatgtg tttaagtttt gttgtattaa tgtatttttt 19440
aggatttagt tttatgagag aagattttat agagtttagt tttgtgttat ataaagtatt 19500
tttttaggag atttattttg ttgtagggga tttattttgt taagaagtag taataggtgg 19560
aaaagagatg tatttgtttt atgtttgttg tggattaagg tttgtttggt agaagggata 19620
tattatgtat tatatatata tttttttttt tttttttaag atggggtttt gttatgttgg 19680
ttaggttggt tttgaatttt tgattttagg tgatttgttt attttagttt tttaaagtgt 19740
tgggattata agtgtgagat attgtatttg gttatattat gtattttaaa gaagagattt 19800
ttttgaattt tttattttgt ttagatgagt agaagttaat gaaaaaatta tttttttttt 19860
tttttttttt tttttttttt tttttttttg tttgtttgtt tttttttttt tttttttttt 19920
tttttttttt tttttttttt tttttttttt tttttttttt tttttttttt ttttagatag 19980
ggttttatta ttatttaggt tggaatttaa tggttaaatt atggtttatt gtagttataa 20040
tttttttgggt ttaagtagtt tttttgtttt agttttttga gtagttggga ttataggttt 20100
gtgttattat gtttagttaa tttaaaaatt ttttttatag agatgggggt tttattgtgt 20160
tgtttaggtt ggttttaaat ttttgagttt aggtagtttg tttatgttgg ttttttaaag 20220
tgttgggatt ataggtatga gttattatgt ggggttaaaa gaattatttt taattaatat 20280
gttaattgtg aatgttgaaa tattgattat ttatatttta tttttattat atgtgatagg 20340
taaaatttttg ggaatttatt tattttaaat tttagttatt tatataaaaa tattaattaa 20400
aatttttagg tataattatt ttttagatat tttgaattgt taattatttt atagttgtaa 20460
atgtggattt ttttaaaaagt aaatattgaa gaattttttt tatttttatt aattttaagg 20520
aagttgatga tttttttaaa gtataaatta ggattgggta tggtggttta tgtttgtatt 20580
ttagtatttt gggaggttga ggtgagataa ttgtttgaat ttaggagttt gagattagtt 20640
tgggtaatat agtgatattt tgtttttata aaaaaaaaaa aaaaaaaaaa aatttaatta 20700
gttagttgtg atgttgtgtg tttatagtat tagttattta ggaggttgag gagggaggat 20760
tgtttgagtt taggaggttg aggttgtagt gagttgtgat tgtattattg tgttttagtt 20820
taggtaatag aaaaaaaaaa agtaatttta taattaattg agttattatt tttttttttt 20880
tatttaaggg gagaaaagtt ttgttgtttt tttagtttgt aagtgatttt ttagattgga 20940
atgagtatat ttgggttagg tgaggtggtt tatatttgta attttagtat tttgggaggt 21000
tgaggagggt ggattttgag gttaggagtt taagattagt ttggttaaga tggtgaaatt 21060
ttatttttat taaaagtata aaaattagtt aggtgtagtg attagtgttg gtaattttag 21120
ttatttggga ggttgaggta gaagaatggt ttgaatttgg ggggtagagg ttgtagttag 21180
ttgagatggt attaatgtat tttagtttgg gtgatagagt gagattttgt ttttataaat 21240
aaatatataa ataaaaatta aagaaggagt atatttgaga aagaaaagat ttgatgttgg 21300
tgaaagtagt atattttttt tgggattata tgttgttttt gtaggttaag aaatttaatt 21360
gtttggttag gtgtaatggt ttatgtttgt aattttagta ttttgggagg ttgaggtggg 21420
tggattgttt gagtttatga gtttaagatt agttgggata ataagagtga aattttgttt 21480
ttattaaaat ataaaaagtt aggtgtggtg gtatgtgttt gtagttttag ttatatggaa 21540
ggttgaggta ggagaattgt ttgaatttag aaggtggagg ttgtagtgag ttgagattgt 21600
attattgtat tttagtttgg gtagtagagt aagattttgt ttttaaaaaa agaaagaaag 21660
aaagaaagaa atttaattgt ttaagattag tttaatttag tatgtatttg ggtgttagat 21720
agtgataata ggtagggtga gtgataatgt tgttttgtaa tatttttttt ttgtttttgg 21780
tgagggtata gtttaaaggt agaggttgtt gtgaatttat tttttaaaag tttttttaag 21840
tttatataat taaataaatt taatatttga tttttatagt agttgttaat gttttatttt 21900
atttatagtt tttgttatat gtatgtgaaa tgggtttttt attgaaaggg attttgggag 21960
ttatttttga tagaggttta tggtttataa atgttatttt gggagttgtt gtttgggaaa 22020
tttgaattgg agaggatgta tttggggaag gaagtaggtg gggtgatat agttgtaggt 22080
tgagtaatgt tgttttggtt gttagttgtt atggttaaat ttatttttag attataattg 22140
tgttaatata ttatggtaat gttttattga tagagtgggt gttgtgtgtg ggttagggga 22200
tatgttgttt tgtattttat tttagttaga ttttatagtt attttgggat gtggatgttt 22260
ttagttttat tttatagatg aggaaattga agtatataaa ggttaagtta tttttttaag 22320
gatatttttt tgtggtagaa ttataaggtg gtttgggagt ttttttaata attttttttta 22380
gttggttttt atttttttta tggggaaaat tggtattttt tagttttatg tgaagtgtta 22440
gttaaatata gttttaatag atttttttaa taatattgtt gttttaaaat attttttttta 22500
tagagatttg gagttgtttt aaggttgggt taggtgtttt tgttttggtt attattttgt 22560
attgtaatta tggtttttgt tattgaggtg agttatttga aggaagggat tgtgttttat 22620
tgggttttat atttttatta tttggaattt tgtttagtgt atagttggtg ttttaggaat 22680
gttgttgaat gaattaatgt agtaaataag tggtagagtt aggggttaag tttaggtttg 22740

```
ttttatttta aagatttttt aattatttta ttttgttttg tgtaggattt aagttttttt 22800
attttgttag tttttggatg aaagtgatat tatagtgttt tggtgaaggt ggtggagttg 22860
taggattttt tttgttatag aagggaaata gaggtttggg ggaattaagt gagagatgtt 22920
ttgggatatg tttagggtta tatggtttag ggggtttgag agggataatg ggtagataga 22980
ttatagggtt ggatgtagga ggtgtagggt ttaagttttg gtttttttat tattaggtag 23040
tatgattttg ttttgggttt ttgtattttg gttgtgaagg gagggtttgg tttagtgggg 23100
tttttgaatt ttgtggatgt attgttggga taggggagag tatgggagat tttgtttttt 23160
tggtagtggg ttttgttggg gatagggttt tatttaatta tttatgttat gagatggagt 23220
ttggggatgg gatgaggagt tgggaaattt ttagtttttt tgaggagaga agttgatagg 23280
ggtatttaga gtttagattg aagttggaat atttatttta ttttaggagg tattaggaag 23340
tgatttttaa gtgttagatt atatatgggt tttttatgga gattttgaag tttggtttat 23400
tttagttttt tgagatagaa atgattttag tggtttagta atgttttttt agaataaaat 23460
gaagtttgtt tgattttttt atgttagaaa gataatttat tattatttg aaaggtagtg 23520
gatttggtat gggattattt agtttgtttt ttttagttat gtgattttga gtaagttatt 23580
tatttttatt ggattttagt tttttattt gtgaaataga gatattaata ttgattttat 23640
gggatttggg gagggttaga ggagatagtg ggtgggttag ggtaatatga atataaggta 23700
ttatattatt gttgttatgg ttgtaattag ttttggttgg agagttttg tttaaggagt 23760
ttggtaattt gggatttgtt ttatttgggg tgaggggatg gtattttggt attttgtttt 23820
attataaaat gtaatatgtt ttgttgagtt ttttttgatt ggtaatattt aatttaagag 23880
ttattttatt gtagaggttt tttttgattt ttttaattaa aattgattgt ttttttttgt 23940
gggtttttgtt gtgtttggga aataagattt ttgttttggg gattttggaa tatttgattg 24000
tgtttgtttt ttatgtattt tttttattag gttatgattt ttaagggaag ttttgaatga 24060
attagtaagg taagatatag ggtgggggag agaataggaa agataaattt tagatataag 24120
ttgggggatg gaggggattt aggtttggt agaggggtta tgggggttga gggttggata 24180
aagtttaggg tttttttttt ttattatttt ttttttttat ataaggaaat tttatttatt 24240
tggattaatt gtaggatagt tgaaattata gtagatttaa aaattaaagt atttttaaa 24300
tttttaagtt ttaatagtag tttgattttg gtttttata gtttttagaa gagttgttta 24360
aaggagtttt atgttttaat tgggttatag tggttttat gggagtgagg gtttttagag 24420
ggttggggag taatgtgtgt ttttagagtt ggtatttgtt ttgtgatttt tattgtaatg 24480
tataaggttt tttgtagaat tatgaatggt ggtggtgatg aggggaaggt ggggaggttg 24540
ttaggtaaat ttatttaggt tttgtttaaa tgattatata tgttagtttt ataagagggg 24600
tttttaggga aggtgttttt tttttattag tttatatatt ttttttttta aaaatttttt 24660
tattttgata tagtattata tttaaagtta taagtggatt ataaagaatt tttagatgtt 24720
ttttatgttg atttttttaaa tggtgatgtt ttgttatatt tgtattattt ttaatttttt 24780
tgggtttttt tttgtgtttt tttttgtgta tatgtttgt ttatgtatgt attgatatat 24840
atgtatatga tatatagttg tattatttaa tagtaagttg taggttgggt gtagtggttt 24900
atgtttgtaa ttttagtatt ttgggaggtt gaggaaggtg gattatttga ggttaggagt 24960
ttgagattag tttggttaat atagggaatt atttgaattt gggaggtaga ggttgtagtt 25020
agttaagatt gtgttattgt attttagttt gggtaataga gtaagatttt gtttaaaaat 25080
aaataaataa aaaaagagta agttgtagat atatgattta tatgtttttt tgtttttaag 25140
tattttagtg tgtgtaggtt ttgaaataag gtattggttt atatttgagg ttttagatgg 25200
ttaatgaagt tgattttttag tagagtagtt tgattagggg tagttttgaa ggttgttgta 25260
agggggttaa ttttttgagg tagttgagag tagttatttt tttgtgattt tttttttttt 25320
tttttgattt tgttttttgt ttgtttgttt tagttttatt tatagtattt attttaggag 25380
ggtttagtag gtatgtttta ggtatgagat ttaagtttta gttttgtttt ttattagttt 25440
tttgtttttt tttttttttt tttttttttt ttttgagata ggtttttgtt tttgttattt 25500
aggttagatg gaattttggg gttaagtgat tttttttattt tggtttttta aaatgttggg 25560
attataggta tgaattatta tttttggatt tttttaatag ttattgattt ttagtaaggt 25620
atttattttt tttggtttta gtttttgggg ttataaaatg gaaatttggt ggttgtaaag 25680
aggttggaat tttattggtg gtataataga tgttttagta ttttgtaaag tataaagttt 25740
agtttttggt tttgtttagt ttgtttgttt gtttttttgtt tttgtgagag tttagtatta 25800
atttttttttt gtttttggat tttgggaagt ttattttatt tttagattaa gagaaaaata 25860
gtttttttta tttatttttg ttattgtagg aagaattgag agagttttg tgtgagggtg 25920
gagtagtgga ggatggggtg gtagattttg ttttggtgga tgtttgtttt gggaagaatt 25980
tatggggaag ttggtaataa gtttgttagt tatatggaaa gttgttgggt taaatgatag 26040
gggtgtttta gggagttttt gtaaggtaaa ggtttttgta agttagttag tttttttgagt 26100
atttattgtg tgttattttt tttggagggt taaggaattt ttatgggttt ttaaagggag 26160
gttatttaag gaatgaaaaa gagtttaggt tttagtttgt ttgttattta gttattagtg 26220
```

```
atattggtgg gttttttttt ttggtgtttt attttgattg tttgtttaat gaggatgttg 26280
gggttatagt gtgggaattt tgaggagatt ggaatgaggt aggaatgtga aaatatttag 26340
aattagggga ggaaagattt ttgtaaggaa ggttagtgat ttgttttatt tatttttttg 26400
ttttttgtag tgttggaatg gtggtagatg tgtagtaggg ttgaattatt gtttgagtgg 26460
taaatttttt tagttaggat ttgggttttt aaataaatgt ttagaaattt tattaggtat 26520
tggggtttta ggattattat tttttgttta gtgattttat tggaattgta gtgtttttttg 26580
gtggttagta tgtataattg tagttttgat tttggtttgt tgggagttgg gttttggaat 26640
ttgaatgttt agtttttttgg gtaagtagtt agggttgggg attttttttt agttttggtt 26700
ttttataggg ttgtgttttt ttattttttt tttttatatt tttttttggt aggaatttat 26760
gtaatgttgt tgagttgagt aaatagaagg taagaggttt gtttagggtt agggagttgt 26820
tggggttatt tttggttttt tttgttttgt gttgtggttg gtttttttga ttttttttat 26880
tttgaaaggt tttttttttt gattttatgt agtagttttt tgatataagt agtgtgtgta 26940
ggggattaag gatttttttgt gtttggagtt tagttttagg gtttggggtt ttttttgta 27000
gatatattgg ttatttttag ttgtgtttgt agagttgttt tagttaaatt agaggtagaa 27060
ggagtttaga ggagtttttt ttaatttagg tatatatagg tatttgtatt aattttaggg 27120
gtgtgttttg agtttttatt tatatgaggt ttaggtttta gtagtatatt tgggaggttt 27180
attttgatta gtttatagtt tagggaggga gtaggtttat gagttggtgt tgtaaagtag 27240
gggatttagt gtttaagagg taagggaggt tttggaggaa gaagggtggt ttttggttga 27300
gttttgaaag tttaggagag ggaatttata gtagaagaaa ataaaaaata aaatgtttta 27360
tagttaaaaa ataaaaaaaa aaaaaggaaa gataagattt tatttttatt tattgtgttg 27420
gtaaagattt taaaaatatt ataatgatta ttattggttt tgaagtggga ggggatattg 27480
tggtagttga ggatattata tgtttttgga agataatttg gttgtgtttt ttagagattt 27540
ttagatatgt ggataattga attttatatt tttagttttt ggagtttttt ttaagggaat 27600
tgttatgtat gtattgagag atttaaatta tagatttgtt atgattttaa ataagtagaa 27660
atttgtgagt ttaatagaaa aggatggatt aaataaaata gtgattgttt ttgttatgta 27720
attattaagg atggtgtggt agaagaatat agaatagtat ggaaaggggt atggaatatg 27780
gttagatttt attaaaatgt ttataggttg ggtatggtgg tttatgtttg taattttagt 27840
attttgggag gtttaggaag gtagattatt tgaggttagg agtttgagat tagtttggtt 27900
aatatggtga aattttgttt ttattaagag tataaaaatt agttgggtgt ggtgttgggt 27960
gtttgtaatt ttagttattt aggaggttgt ggtaggagaa tagtttgaat tggggaggtg 28020
gaggttgtat tgagttaaga gtgtattatt gtattttagt ttgggtgata atagtgagat 28080
tttattttaa aaaaaaatat aaaagtttat aaagtaatgt ttttttgtg tgtgtttgtg 28140
tgtgtgtgtg tatgtgtatg tgtgtgagtg attttttttt ggtttgtttt gattttttttt 28200
taatttttta tatagaatat agattatatt tgtaatgtga aagaaaagtt ataaaagtta 28260
tagagaaagt tatttaggtg aaagtagtag ttagttatag ataggagagg tagagagata 28320
tgttaggtag aaggaagggt gtttgtagga gtgtggaagt ttgaagatat ttgtgtagtg 28380
tgttgtagag tggaagtagg tttttgaatt ttaagttaga tggaaggttg gtgtgtaagt 28440
ttgtgagttt ggtggggatt agttttgttt ttgggatttt gaatgttata taagtttgat 28500
gtatagggtt ggtagattta gtaaaaagta agtaagtaaa taataaaatt aggttattta 28560
gttaaatttg tatttgaata atgaataatt tgtaggataa aagtattttt tgtgttatat 28620
ttgggatata tttatattgt ttaaagtgtt aattgtttat ttaaaatttt agtttaattg 28680
agtttttttta agtggtgttt gtttttttgtt ttttataatt ttatagtata gagtttagtt 28740
tgtttttaga gtatgtatta tttaggattt taattattgt ttttagatat tttaatttgt 28800
ttttgggaaa ataaatagaa aattaattta gattagtttt gttttgtttt ttaaaaaaag 28860
aaaagaaata aaattaattt atagatttat attgttattt ttggaaattt gtagtatttt 28920
ttgaggataa atgttttatt tttttttttt ttttgattaa aatgagattt atttgatata 28980
aattgaatat ttttgtatgt gttaaaatgt tatttttttt tagtattatt ttatttattt 29040
gtttattaaa tgtttattga gtatttattg tatgttagtt atgatattag gtgttatata 29100
gtagggtaaa taaagtatat atgtttagat atattttttg ttttatggga gggagagaga 29160
gagagataat aaataagggg aaaatagaag aataaataaa tatttaatat tggtgtggtg 29220
gtttatgttt gtaattttag tattttggga gattaagatg ggtggattat ttgaggttag 29280
gagtttaaga ttagtttggg taatatgagg aaattttgtt tttattaaaa atataaaaat 29340
gagtggggtg tggtggttta tgtttgtagt tttggttatt taggaggttg aggtttgata 29400
attgtttgaa tttaggaggt gaggttgtag tgagttgaga ttgaattatt gtattttagt 29460
ttgggtggta aagtgagatt ttgttttaaa aaaagaaata atgtgtagaa taaagattat 29520
taggaaggat tttttattta tggaaatatt atttttttatt taattaattt tttattgttg 29580
aatattttga ttattgttgg tttttttata tattaaatta tgatatgatg aatgtttttt 29640
ttagtgaatg tttattatta tttattgagt aaatttttag aagtgagatt tttgggtgaa 29700
```

ggtatataaa tttttaattt agtttttgtt ttatatatat gaatataatt ttgtattttt 29760
tttttagtta tattttttaa aatttgaaaa taatatgttg taaatttttt tttttttttt 29820
ttgagatgga gttttgtttt tgttgtttag gttaaagtgt aatggtatga ttttagttta 29880
gtgtaatttg tatttttgt gtttaagtga ttttttgtt ttatttttt aagtagttgg 29940
gattataggt ggttgttatt atgtttgatg aatttttgta tttttagtag agatggggtt 30000
ttattgtgtt ggttaggttg gttttaaatt tttgatttta ggtgatttat tatttgtttt 30060
tgttttttaa agtgttaaga ttataggtgt aagttattat gtttagttgt aaaaaatttt 30120
ttggtatata tttttatgag ttttaatata tttatgtata gatttatgta tttattgtta 30180
tagttaggat atagaatagt tttattatat tagtgtaagt tttttgtaaa taaatttttt 30240
tttttttttt atatttggta attagtgatt tgttgtttag agtttttgta gttttgtttt 30300
ttttagaatt tatgtgttag ttgagtgtag ttgtatttta tgtaaatttg agattggttt 30360
tgtttataga ttttaaggtt atttttaga tttatttatg ttattgttaa tagtttttt 30420
tttttattgt ggagttgatt tatggtttgg atagattata gtttgtgttt ttattttttt 30480
gttaaaggat atttgggggt gtttttattt tggggtgatt ttgaatagag ttgttgtaga 30540
tatttgttta tgggtttggg tttttttttt tttaaaataa gttattattt ttgtagggta 30600
aatatttagg agtgggattg ttgggttatg aggttagtgt tagtttaatt agataagata 30660
ttgtttaagt gtttgtattg tgatggatgg gagttttggg tgttttatat attgttgtaa 30720
tttggtatta ttaggtttta tttttattgt gataggtata tagtggtatt ttattgtgtt 30780
tttaatttgt atttttatag tgggtaatga ggttgagtat attttatgtt tatttgtttt 30840
ttgtagattt ttttgggtga agtgtttgtt agaatatttt gtttattttt tgttaaatat 30900
tattattaat tatattttat attttattta gattttattg gtttttttta atgttttttt 30960
attgttttag tatttttttt agaatatatt gattatattt agtttttatg gttttttggt 31020
ttttttagt ttggggtagt tttttagatt tttttattt tgagagtatt ttagggtttg 31080
ggttggtggt tgtgggtgag taggatggtt tgtgttattg gttagtttga ggattgttat 31140
ttttatttta ttttttgttg aagattggta gttttttgag ggttgggata ttgtttgttt 31200
ttgtaaatag ttgtgttttg tgtatgtgtg ttgaaattta agtttagtta ttattatttt 31260
tttggtatta tttattggga ttggagttaa attagggata tttaagttga gtttggata 31320
gattttagtg ttagatgagg tggggtgggg atgtgattaa tggttaaatt ttaatttttg 31380
tttttatttt tataggattg tttggatagt agtgggttta gttagtgttt ttttaatagt 31440
atgaaagggt tttatgttgg tttgttttt ttaaggagat tatattggtg tgtgtatttt 31500
gttatagggt gtgtggggtt agtttatttg agaatagagt aagtggtatg tttttttataa 31560
agtagatgtt tggaggaaaa atagatgtaa ttaaaattat ggaggataaa atgattgaga 31620
tttttttagtg ttatatgtat gggtttgtgt atggttttat ataggtttat attatttatg 31680
tttatattta tttatgtgga tttgtattta tatataattg tttatgggga tatatatttt 31740
atttgtgtgt ttgtgtttga ttatataagt atgtatattt tataaattta tttataagta 31800
attatatata tgttttttat atttatttat aatggtagat ttgtgttata ggtttttggg 31860
tgtatatttg tatatataga tgttattagt attttttttt taaaatggtt ttagaaggtt 31920
atttttaaa ttatggttaa aaaaaaaaag aaagaaagaa aaaagaaaaa ttagttattt 31980
ttggaaattt tttaagggtt ttttgtttt aaaaaggtaa aattagatta gagtgatgtt 32040
attgggttga attaggatag atgggtagag gttaagggaa tgaagtaagg tttttttattt 32100
tatgtatttt tttagttgaa atttaatagt taggggtttt tagtattaga aatgatagtt 32160
tttagtggta tgggggtttt tttaggggga gttttagtag aggttgaagg gtttagtggt 32220
ttttgattga gaatataatt ttgaattgaa tgttttttat tttattaaaa gtagtttatg 32280
gtaataatgt tgatttagga ttgtttgaag agtattggat attatgatgg ttatattgtg 32340
tgtataatat tttttgtgga gtaggtattg tttttagtga tgtaggttta ttatttgatt 32400
gatggtttta agtagttagg ggtttatttt tagattaaga ttgggaagtt gtagattggg 32460
attatattgt tttggatttt atagttttag ttttgggata aatttattat gggaggggta 32520
ttagtataga gttttttgg gtttataagg agttgtttta gggaggatgt gagaagttta 32580
atttttggtg ggtggtttgg atttatgttg ggtgttgttg ttgtttagtt ttatgttagt 32640
atgaggtgtt ttaaggtttt gaagaaggtt tgtttagtat ttaggtaaat tgagagtttt 32700
ttggtggttt tttttatttt agtgtggtgg tagggtgggt gtggagttag tttttggtag 32760
attttggatt gggtttttgg attttggttt taattttgga gttgttttta tgaagtttgt 32820
ttttgttttt tttttgttt tttgtttttt gtatggagat attgtttaag gtttgtttgg 32880
gttttttttt tttttgttt tttgtagtga tttttgttgt ttgtgttgtt agttttgaat 32940
ggtggtattt gttattgtgt gttttttttt aatgtatttt atttgatttt tttttagtg 33000
ggtaggagga tgaggtggtt ggtttattta tttgagagat atttttgta tatgttttat 33060
agaatattgg tttattaaag gtgtatatag atatttggat agagtgagag tttagtgatg 33120
tttgttagtt agaaattgtt tatttatttt ttagtaattt tttattgtat ttttgttatt 33180

tgatttattt tgtgttgagt tttaggttga gaagggataa atggtatgag gttttagttt 33240
ttggggaatt gatattttat aggaggagtt ggatatttgg ggtttgatga gattatgagt 33300
tagtaaatat aggtagttag tggagagggt gagtagagga tggaaaatag ttataatttg 33360
ggagggtttt ttggaggagt attggatttt ggaggttagt tggaaggagg aatatgttag 33420
gtaggggttt gagatgggga tttgggggat taaggttagt ggtttttttt ggttaggagt 33480
agttagtttg agttatggga tttttatttt tagtagttgt tttgttgagg agggtatttt 33540
gtggggagtt agatttgatt agtatttagg tttttagtta ttatgttgat attttagtta 33600
gtttggggtt atttttttgg agagtaattt gttggttttg tttagggttt agttttattt 33660
tttatattat ttttgttatt ttttgtttta tataggttgg agaaatttag ggttatttga 33720
ggaatagtag tgggaattgt agaaggaagg gagatttaga ggggtggtag tagtttttta 33780
atgtttgtag ggatagggtt gttatgggga agggtgttgg ggggttagaa ttggaattag 33840
tgaattagat ttataggaga tagatatggg ttgggtttaa agatgagtag ttttgttgtt 33900
ttgggggtag tgagtttttt gttattttag attggaatta gtgttaggtg gagatgttgt 33960
agggtggtga gaggtggata tagatagttt gagtttgggg ttttaggatt tagattttga 34020
gagggttgtg tttgttattt tgattagttg agtggagttg tagaatttta gggaattttt 34080
ttttgttgtt taaatgagga atttagtttt agaatttaaa gtaagttgat ttaagatagg 34140
ttagaaggtt tgtttttttt tttttgagg aattaggagt agtgtttttt tgaatttttt 34200
ttatttttga tataggtata ttttttttt ttttttttt ttttaggttt ttagggatag 34260
ttttggttgt tggtgagtaa gggaggaggg attttaatt atttgtttt ttttatttgt 34320
aggtgagatt ttagagatga aatgggttat ttgaagtttt gagaagagtg ggggttgagg 34380
ttagggtttt taatttttgg tttggtggtt tttgtatatt ttgtgttatt tttattttg 34440
tttgtttatt tggttttagt gggtagttgg aggtgggtaa aattggattt ttatttgtta 34500
ttatgaagat tggatttttt gttatttttt ttgaaaggtt aagagttggt ttagtgttga 34560
gaggttgttt ttaggttgta tttttatttt tttattttta agggttggtt ttgtttttta 34620
taagatggat agagtaggat gttttggtta tatgaggttt ttgggttttt tttgtttttta 34680
tttttttttt tgggaataag gtttaagatt gtaggggagg gagtagaggg taggttagga 34740
gttgagaggt tgtgttttgt ttggaattgg ggtatgtttt ttttttttt tggttttagt 34800
tttttttag ttaaaattag gagtttaggt tggtttatat taatttttag tttttgtaat 34860
atggagtttt tattgttgtg tagggtggtt ttaattttta gtaagtgttt atggggttgt 34920
gagatggggt ttggggatta gagtttaggg tttagtgttt attttttta gtttttttatt 34980
tggtttttag gaagttgtaa aatagggatg gttattttg tgaggttggg tggggttttt 35040
aaaaaaaaaa aaaaaaaatt taaaatataa aatgtatttg tttgttagga agataaatat 35100
ttttgttgaa agtttttggt ttagttgtta gttgtagttt gtagaatgtt gttattgaag 35160
ttatggttgt ttgtatgtgt ttgggtttat ataggtgtta tgtgtgatgt tgttgggttg 35220
gttgtgtatt tttatggtgt ttgtattgtt agtggttttt aggagtagtt ttttgaagtt 35280
gtttttaggt ttattttggg tatttatttt tatgtttttt ttttgatgtt ttggagaggg 35340
aggtgagaat gttataggtt aaatgttttt tttagttttt ttagtgtgta tagaaagtta 35400
ggaatgagat gagtttgaag ttttagtttt taggatttgg atggagttag aagatttgga 35460
ttagaggtat tattttgata tttaatgttt attgtttgga tttattttta gaagaagaaa 35520
ttgtgggtta gagtggttga tgtttatatt tattatatgt gtttgttgtg atgattaaag 35580
tttgtgtatg tgaaaggttg tgttaaatgt tttttaaatg ttttattaat tgtgtttgtt 35640
tttttattta ataagttttt tttttttaag ttatagataa ggaatatagt ttttgaggag 35700
tttatattta tatttgtaga tatttagttg gttgtttttt tttaggtatt tgtgtatgtt 35760
ttgtatatgt ttttgtgttt aagggtttgt atgtttggag gattttttta gggttggagt 35820
tttttaatgt ttatagggtt tgtgggtttg taagtttaga ttagaaatgt gaatattaat 35880
aatggtaaaa ttatatagtt agtatattag tttttttatt ttttatagtt attttatgag 35940
gaagatttta gaagtatttt tatttttatt ttatagaggg ggaaattgag gtatatagtg 36000
ggtaggtaag ttttttttgag tatatagttg gggagttatg gttataggat ttatggtggg 36060
gttattaaga gtttatgatt taaattaggg gtttttagtt tttaggttgt ggattagtat 36120
tgatttatgg tttgttagta attgggttgt atataggagg tgagtggtag gtgagtaagt 36180
attattgtaa gttttggttt ttgttagatt agtggtgtta ttagattttt atagaggtgt 36240
gaattttatt gtgaattatg tatgtgaggg atttaggttg tgtgtttttt aagagaattt 36300
gatgtttgat gatttgaggt ggaatagttt tatttttaaa ttatgtttta ttataatttt 36360
ttttatttat ggaaaaaatt atttttttata aaattagttt ttggtgttaa aaaggtaggg 36420
attgttgttt taaatttttt gttaggaggt gagtgtgttt gtttaggaag tttattaggt 36480
tgtgtggtgg taaagtggtt ttttgtttta ttttttttatt ttagttttta tttttttttt 36540
tttgatgtgt atgggttgat ttgtttttgt agttttgagt taggaatgtg ttggtgggtg 36600
gggggtttat ttgttagatg tagtggttgg gagttgtgtt tatgtatatt tatttaggtt 36660

tggggttagt gtgttgggga tgaaggtgtt tgggtagttt tagtaaatag tgggtaggag 36720
ttgtttgtaa taggaaatgt ggtttatgtt ttaattttat gtatttttaa ggaagaaaga 36780
tttttttttt gaataatagg tgagattagt gtgtagggtt ggggggtagg gaaggaggtg 36840
ttgatttttt agtattggat ttttggggta gagtttttat agtttttgtt gaatttttgg 36900
tgggattagg ttaggaaagg aggggtattt ttgtttgttt taattttggg ttttttttat 36960
tttaagtttt attgggttag atatggttgt tgaatgagtt ttttttttat ttagggaggt 37020
tatggtattg ggggttagaat ttttgggttt tagaaaagtt ttgatgtagg gaattttttt 37080
agagagagga gtttagggtt agtgaggtgg ggattaaagg ttatagaggt tagattggtt 37140
gttttaggtt tatataatat tgttgttgga agggatttag tttaagatga tggtgaagag 37200
gtttagattg ggaagagat ttgtttaagg ttatatagta agttaatggt aagttagaat 37260
tagaagttaa gtttttggat tttgtattta atgagtttta agaggaaaaa ggtagggggt 37320
ggttatttga gtgtttttatt ttttttttga tattttttttt tttttgtttg gtttaattat 37380
tggtttttga ttttgttttt ttttttttta gttaaagaga gtttttttggg tgagttagag 37440
ttattttttg attttgatat tgtggggatg gatagtagtt atttgagtgt taaggaggtt 37500
ggggtgaagg ggttttagga ttgggttagt ttagattttt ttagtttatt ggagaaggtt 37560
gatttagaga gtaataaggg taagaagtgg tggaattgga ttatttttat tagttattag 37620
ttggaggagt tggagaaggt tttttagaag atttattatt tagatgtgta tgtgtgggaa 37680
tagttggtta tgaggataga ttttattgag gtttgtgtgt aggttagtga gggttatttg 37740
ggagtgaggt tggtaaagta gagtttgttt ttgggttttt ggttgtagtt ggggtttttt 37800
gattgtttat taattatatt atggtttttt tttttttagt ttattgagaa tttgaaagtg 37860
ggggttttta gtttgttttt tatgtttttg ttttaattag agtgattttg tttttatttt 37920
ttttaatatt gggtttttgt attgtggtgg ggggttgtaa aggaaggagg gtttttagta 37980
gaggggtagg aaggttgttg ttggttgttt ttgggtttta tatagtgtaa gagttttggt 38040
tttttttatat agagaagggt tatttgtttt tgttttagaa ggtttatttt ttagatagga 38100
aataggataa atatgaagat aatgtttata gtattttagt gggtagaaat agagaatagt 38160
ttagtttttt tttttgttta gatggttgta tttttttttta tttttttaagt tttaatttat 38220
attttttttg agaagttttt ttgattattg tggttaaagt agtagttttt ttattattag 38280
ttaataatta aaatatttaa tttagtataa tatagtggaa tttaatattt aatttaattt 38340
attgtaattt aatagaatat aatttaatag aatttaattt aatataatat aatatagtgg 38400
aatttaatat atttaattta atttagtgta atttaatata atataattta atagaattta 38460
atttaattta gtataatata atggaattga atatatttaa tttaatttaa tttattttaa 38520
tataatataa tttaatataa tatattttaa tggaatttaa tttaatataa tataatataa 38580
tggaatttga tatatttaat ttaatttagt gtaattgaat agaatttaat ttaatttaat 38640
ataatataat ttaatagaat ttaatttaat ttaatataat ataatggaat ttaatatatt 38700
aaatttaatg tagttttttt taatataata taatttaata taatatattt taatagaatt 38760
taatttaata taatataata taatggaatt tgatatattt aatttattta atataattta 38820
atagaattta atttaattta atataataga agtaaattag tttatttttt gttatattgt 38880
tttattttat tttttttaaaa atttttttaaa tttttttttgt tttttttttta gagatagggt 38940
tttattttat tatttaggtt ggagtgtaat aagatgatta tagtttattg tagttttaaa 39000
tttttaggtt aaagtgatta ttttatttta atttttttaaa gtgttagaat tataggtatg 39060
agttattatg tttgggttta ttttattttt tgggtatttt tgtttatttt ttttaaagag 39120
attgggtttt tttttgttat ttagtgtgga gtatagtggt gtgattatgg tttattgtag 39180
ttttaatttt gtgggtttaa tggatttttt tgttttagtt ttttgagtag ttgggattat 39240
aggtatatat tattatgttt gggataagtt tttttatttt ttgtagagat ggggttttgt 39300
tttgttgttt atgttggttt taaatttttg gttttaagta atttttttatt ttagtttttt 39360
atattgttgg gattataggt atgagatatt gtgtttaatt tttattttgt tttttttata 39420
ggattgttga ttggtttatg ttattttatt tgtttgtttt tttttattaa attgtgattt 39480
ttttgagatt aagtattgat tgtttattat tgtattttta ttaattagag aaagttaaat 39540
gtagagtatt aagtttaaaa aataagtatt tgtggattaa ttaattttttg tttagagagt 39600
tgtatttgtt ttgattagta tgaatttata gtaaagaaag gattagaaag aaagaaaaga 39660
tttatgagta gttaaaatat aggtgataaa atttatatat ttaagtttaa gttttaattt 39720
aatataaaat aaatgttata attagtttgt tttttttaatt taaagtatgt tggaaatagt 39780
aaataaaatg ttgttatata gtagtttttt ttttttttagt gggttagaaa aattaatagt 39840
tttagaataa agagaaattt tttttttttt tttttttttta attagtaaag tgttatgtag 39900
tttagtgggt tgagtttttt aatgttagga tagagatgtt ttaattttgt aatttttagt 39960
aggttttagg gatttttgaa ttaatttatg aaagtatttt tagtagtatt gttttaaggt 40020
attagtaaaa gatttattag ttataattat tttttgttta agaaggtaga ggtattttta 40080
atagagttta taaattttttt ttttaaaatg gttaatgtgg aaaaggatta gtggttatta 40140

gtaaatatgt agttgtttag aaattgtagt tattagaagt gatgttgaag agttgtatgg 40200
ttgagttgtt gtaagttttt tgagggtagt ttgttatttt tttttatttt ttttattttt 40260
tgagatagga ttttattgtg ttatttaggt tggagtgtag tggtgtgatt ttggtttatt 40320
gtggttttta ttttttgggt ttaagtgatt tttatgtttt agttttttaa gtagttgtga 40380
ttataggtat gtattattat atttagttaa ttttttttat tttttggtag agatggaggt 40440
tttattatgt tggttaggtt ggttttgaat ttatgatttt aagtggtgta tttgttttgg 40500
ttttttaaag tgttgagatt ataggtgtga gttattatgt ttagtttaga ttgttttttt 40560
ttttttttaga tagagttttg ttttgttgtt taggttggag tgtagtggtg taattttggt 40620
ttattgtaat ttttgttttt tgggtttaag tgattttttt gtttttagttt tttgagtagt 40680
tgggattata ggtgtgtatt attatatttg gttaattttt gtatatttaa tagagatggg 40740
gttttattat gttggttggt tggtttttaa tttttgattt taggtgattt attattttag 40800
tttttttaaag tgttgggatt ataggtatga gttattgtat ttggtaagat tgttatttta 40860
ttttttttta gtgtttaatg aatgtttgat gaaggatgat agggtagttg gggttttttt 40920
gtgaatttag aagtttttat atatttagat tttatttgaa tttggataat tagttattta 40980
tatgatttta tatgtttgag tttggatagg aattttagta tttagtttga tttttagtaa 41040
atggattggg gaggatttat aggtgtaggt agagaaaggg gtatagttgg ttatgggata 41100
gttttgtagg tgttgtgttg gtgtgggata aatttgtttt ttttttgtgt agtatgtgtg 41160
tatatttgtg tttgtatatg tgggttgtat gtatgttttt taataaatat atgggagtgt 41220
ttgggagtag aatttattaa gtattgaaag aagaatgatg ttttgagtat aaaatatgaa 41280
aataaaggtg tatagagtat tttttagttg ttattatata attaaaatag agtgtttttt 41340
gtatatatat atgtgtgtgt atatatatat atttatattt atttttaaaa tggagttgag 41400
gtagtttata attaaagatt tatttgttta atttaaaaat aagaataatg gtatgaaatg 41460
tagaggtaat gtggtaaaaa aaaatttagg ttaaggttta tggttgttat tgagtataaa 41520
ttttaatttg gagtttttttg gtagttaagg tagaaaggga aattttgatg tgttgtatat 41580
tttattattt agtgggagga ttatagaatt atagaatggt agtgttggaa atgattattt 41640
tattttttta tttgatagat gaggaagaag aggtttatag gtgggaagtt ataatttgaa 41700
gttaatagta gttatttagt agtagtattg tgattggaat ttgggatttt tgagttgtag 41760
tttagtgttt ttttgatttt agtttgttgt ataaggatat atgttagatt gtttagaggt 41820
ttttttttgt ataaaggtag tagggtgggt gggattttta tgtgtgttag atttttgggt 41880
tgagttttaa gtttggtagt gttttggggg ggtaaggggg tggtgatgga attttttgtt 41940
tgtttttgtt gtgtttgttt agggtttata tttagttggt ggtaagttag gtgtttggtg 42000
tagagtggtg gtggggatag ggatggggta atgggggttt taagatggat tttttttggg 42060
aagaagttta gggtttggtt ttgttttagg gggttgtagg ggttttttttt tgttgtttgg 42120
gattttaatt tttgtagttg gtgtttgaag ataataagat agaggttttt ttgttttggg 42180
ttattttgtt ttggttttag atgggttgtg gttagtgagg tttttgttaa gatagttggt 42240
tttttgtata gtttggtttt tgggagaggt ttgtgttgag tgggtttagg ttggggtggt 42300
tttgaggtat gatatttaga ttgggagaat agtgggggatt ttgtggttgg ttgttttttt 42360
ggggttgtat agtggagata ggtttgtggt gtagagttgg gaggtatagg aatgggtgtg 42420
ggggttggtg tggtatttgg aagggagttt ttggagtttt tggggtgtta ggggttagaa 42480
atggatggat attttaagtt ttaggatttg tagatgtgag tatagaaggg gttgtgagga 42540
tatttttgtt gtttttaatt tattggggag gatattgagg attggttagg tttatttagt 42600
ttatgtttgg atttgggtag aaaagttaga attgggtgtg tagggtaagg ttttaggata 42660
aaggaatagg agaagaagga gggaggaggt gggtagtttt gtttttattg gttgtttatt 42720
tgtttttttat tatttttatt tttagttttt aaataaattt tttagtttga ttttttttta 42780
tttttgtttt aaagatggtg ttagttttgt tggttttttt tttgtttggt tatttgtttt 42840
ttttagataa tttatatttt ttttttttttt atatttagtt ttagtgagtt tgtgttttat 42900
atttatttt tttatatggt ttttttttttt attttagatt tttttgttag gtgttggtta 42960
ttttttttttt tttttggggt tttttttttgt ttatttttgg atttttttgtt ttagagtttg 43020
tagggatatt ttttattttt atagttttat tatggggatt tggaatttat agagatataa 43080
ggagaatttt aatagttata gtgataaaaa gaaaaagaga agttattttt ttttgtgttt 43140
ttagtggttt ttttttttttt tttagttttt tttatagaaa attttgggag gttgaggaag 43200
gagtggaaag atggagttaa gtttgtagga aattaaatat gaaatgaaaa taagtttttt 43260
tgatttattt ttttattttg gttttagttt tttattttag gttattgata aagagggatt 43320
ttattttagt agtatagaga ggagatttta tttgtttttg gtttttagtg ggtagttagg 43380
gtttggattt taggaatttt tggaggaaaa ggggttttgg gaggttaggg taggggttga 43440
gattttaaat tggggagagg gtttgttttt aaggaagagg ttttttttttt tttagttaat 43500
gtgtgtgtat atatgtatgt gtgtgtagtt atattagtgg gtatatttgt gtatgtatat 43560
atatgtttag gattagggag gggaaagtga gttggtatag gagtggagaa atataataat 43620

```
gtatattttt gtttattttt atttttttta tagtaatatg agatatattt ttattttttt 43680
tgattttttt tttttagtaa aagatagtga gaaaggttag gagttgtaaa gaaataaatg 43740
ggaagtattt taaatatttt tggattgtat tttttattta tttttttgt tgtgattatg 43800
aggtattatt tgttatgtag aaattgaggt ttttggtaat attagtgttt ttatgagttt 43860
gtttattggt tagttatatt ggatgtttaa agaggttagg tagtgtttgt gaggaagtat 43920
gtttatattt tttttttttt tttttgtagt ggtttttagg aagtttaaag gttttgaggt 43980
agaggaattt tgagagttga tttttatatt tagtttagta gttgtattgg ttgtgatgga 44040
tggagaaatg tttttatttg gagagtaggg attggatttt aggaaagttg gttttttga 44100
tgtttgggtt tggaagggtt tggtaaggtt ttttatggtt ttgtagttga ggttattgtt 44160
atggttttgt ttttttggtt tgtagtgtgt tgtttgaggt gtgtttattt gtatttgagt 44220
ttgtaaagtt agtagataag tgtatattaa gttggtagag gtttttttt gaggagtatt 44280
atggtttatt tttgaattag tttaaagtta tttttggttt ggaagttgat ttttttttat 44340
ttttttttt ttatttattt tgttttattt attttttttt tagattttaa gttatgggtt 44400
aggggtaggg tagtttttgt ttttgagttt tgggaaagtt tttagtttt tagaaatgtt 44460
tgtgtaaagg aaaaggattt aatattttt gttattaaaa attttagtgt ttatagttga 44520
tttagagatt tttttttttt tgttgggttt ttaattaggt taatatttta tttatttgtg 44580
tttagtgatg ggttggtttt tatttttttt tattattagg ttatgttttt atataagatt 44640
ttatagtaat tgtttttata gatagtatta agggaaaaat tttttatttg gtagttaagg 44700
tttttgtga tttgattttg tttttttttt ttttattgtt ttttttaaat tgattttttt 44760
aggtttttgg agtatgtttt gttgttttta aatttaggtt ttggtttttt tttttttagt 44820
taattattta aggtttagtt tttgtttttt ttttaggaag ttttttttga ttgtttttgt 44880
tgtttagtag agtttttgtt tttttttaa tataggggag ttttgagttg ggagattgtt 44940
tttaataaga aggttgtaga taggatgtag ggaggaaggt attgtgaatt tggattaata 45000
agttttagag agttatgaat tttttggaaa attgtggtaa aattgtgtgt gattgtttag 45060
gagaatatat ttggaggagt atttgtagtt tttattagtt tttttatagg agtttgttta 45120
gatgattgtt aagatgatta gtgatttgtt ttataggtgg tgatatttgg ttgttttaga 45180
tttttaagtt tagaaaaata aaagttttgg tttaggatta tgtttatggt tagtgggggt 45240
aagatatttt tttagtttag agtggatgat tttagattgt tagtttttga ggataaagat 45300
taaggttgat atttatgtgt ggttgtattg tttttttaaat gtattgtaat tgatgggggg 45360
tttaagggaa tagtttgtat tgtttgaaag tgaggttttt aggtgggtat tttattttgg 45420
tgataatggt ttttttgtgt tttgtaggtt tggttttaga attgaagggt taagtggagg 45480
aagtgggagt gttttgggta gatgtagtag gtttgaattt attttttttat tgtatatgag 45540
ttgttttttt ttatttgagt tgagaattat gtttaggtaa gttttgtttt gagttgttgt 45600
tattgatttt tagtttttgg tgttttgaga atgagttttt tggaggagag tttaggtatt 45660
ttgagatgat ttatggtttt aggttgtttt tttgttttat tttaggggtg tgtttgttta 45720
tattagagtg tgggaggttt agggttaatt tatttaatta attattgaat taggtttggt 45780
tgaattttag ggaagggaag gttggggtag tgagaatggt ttttttttt attagttttg 45840
gttattttg ttgtgggtat tggaattaag gtaggtagaa ggttattagg tttttagata 45900
ttgtttggag ggtttttttta gaaagtgagt tttgaaattt ttaggatttg tggttaagtt 45960
ggaatgagtt tgggattttg agtttgggga tttaggttat ttttttagtt ttaggttttt 46020
tttattttag gtaagtttta aatttttgag ttttagtttt tttatttgta ttttggatat 46080
ggtaaaagta tttgtttagt tttgttgtta ggtagttgta aggtttaaat ttaattatgt 46140
aaaagtattt tgtgaattgt atagttttaa aaagtttgtg tgtttgtgtt tagtttttgg 46200
ggttttgttt tttttttttt ttagagtatt ttttttaggt gtttgttagt tagtttttttg 46260
ttttagtgtg agatgtaagg gatgagaagt attgagttat attaggtata tgggatgttt 46320
agagagatgt gttttggtt aagtttaagt ttttttttga tttttattta gtttgtttag 46380
tagaagtggt tgttttttat ttgtttaggt tttattttgg tatggttttt attttagttt 46440
ttaatttggt tttgtttagt agttagttag tagtatgggt gtttttgttt tttttatttt 46500
tttttgattt ggttttgtgt tagtaaatgg tttttggttt tttaattaga ttagtttttt 46560
gtttttttt ttttagttta ggatttatgg ggtaggaggg tagggtatag gtttggaggt 46620
gaggttgagg aatatggggt gttttatata ggtattatta gtattagttt gtattatgtt 46680
tttttatttt tttttttttt tttttttttt taaatttagt ttttttttgt tttttttagga 46740
tggaatttta gttttttttt tggttttgag ttttgttgtt tgagtgtata gttggaggga 46800
gaatttaata tagggttttg tttatttttt ttattttat tttatttata gtattatata 46860
gtgtgtgttt agattttttt tgagaggggg tgatggaggt taattggttt tttttttttt 46920
gtattgtaga gagaaatgtg gtttttttta ggatgggggt tgagtttttt tttttttgttt 46980
taaaatgttt tttttgagtg tgttttagat atatgtagga gggtaggtta gtagaagttt 47040
atatgaggtt attaggaatt ttgtgtgtat aagttgagag gtttttgggt tttttttata 47100
```

ttttaatttt attttttat atgtgtttag aaggtagagg aagatgggag ttttaggtga 47160
atgttgggga gggaagggaa gtttagtggt atagatggaa gataagatgg ttaatttatt 47220
tttgtatatg tttttgtttt tttggtatga ggaggtttgg ttttagattt ggttgtttat 47280
ttttgaggta gtttttttta ttttgagtta gttttgaagt tttttagttt aggttttgga 47340
atttagtta ttgatttttg gtttttgaga ggtttaggtt tttgttgttt atttattta 47400
atttttttat tttttttgtt attggtgggg gtttaaggga atttgtgagg ttaaagtttt 47460
ttgagttgag gtttgtttat tttgtttagg tgtttatgga tgtgaggggt ttggggagga 47520
gttggaggtg aatgagatat agttgttatt tgtagatgga ggttaagttt atagttgagt 47580
ttaaaaatag gaagttagtt gggggtgtt ggggtgttat tttttattg gatttttaat 47640
gaagtttgag gttattttt agaattgaat ttttttttgt tgttagtttg gttttgggaa 47700
tagagatgtt aaggtgtaaa agttattttt ataggggtat ttggatgttt attaaggtta 47760
aatatttaag aggggtttta tgggaaggat ggatagtgag gggagaggtg ttttggggga 47820
aggggtgagt ttttttata ttatatttgt ttgttagttg gtgatatttt tttttttttt 47880
ttttagattt agaatttgtt ttggtttggt aataatgggg ttgttttatt agtgttagtt 47940
tgtgtggttt tttgtgattt ggtgtttgtt tgtatgtttt tttatgttta tttttttggt 48000
tttggggtta gtagtgttat tgattttttg agtgtgtttg gggttggtag ttatgtgggt 48060
tagatgtata tgggtagttt gtttggagta gttagtttta gtttaggttt taatggttat 48120
gagtttaatg gtgagttgga ttgtaagatt ttgagtattg tggtttttttg tatgaaggtt 48180
aaggagtata gtgtggttat tttttgggtt atatgatagg gtattttttgt tttgttttta 48240
ttttgggata ttatgggtta tgtttatgtt ttttaggttt ttagtttttt atttgatttt 48300
tttttagga atttggtttg ggttaggggt ttgattttta gtatttttag ttgttttaag 48360
tttgaggttt tgtggattgt tgggagggag ggggtagtag gttttggtt tttttttggt 48420
attgaggttt tgatttttgt ttttggttat aggtagtgga gaaagttagg tggttatgtt 48480
ttttagtttt gtatttatga taagttgaaa gtgttttttt gttttgttt attttttgt 48540
tttgtttagt tgattatgag ttaatgttag atttttatgt agatttagta gttttattag 48600
tttagttttg tttatttttt ttgtttttag tggggttttt tggttattag gttggtggtt 48660
gtgtgtttga agtataggtt gttttgtaga gttagttttt tgttttttta ttttttttt 48720
tttgaaagta tatggaattt gatttgttgg ggttaaggtg ttagtttta tttttttttg 48780
aatagtgatg agtttgatag agtttggttg attgtatttt ggttgttttt taggttggat 48840
atatttggag agagtgggta gaggatgata ggagttggag ttgaggattt ttgttgttat 48900
ttagtaatgt tagtttttag gggagatatg ggttagtttt ttattggata ttatagggga 48960
ggagttagat ttgagggagg ttgagaatat agatgttata aggttttta tggtgttaga 49020
agaaggaaag gtttgggag agggggaggt atttgggtgg ggtgtgggtt tgggtagttt 49080
ttttgtttt tggtttgttt ggtatttggt atagtgggga ggtttgggta gttttgtttt 49140
ttatttagag tttagttagg tttttttttag gaggatgtgg aggggtttgt tttttttttg 49200
ttttatttg gttttttttt ggggttgttt agtagtgttt tggtatggaa ggatttataa 49260
ttttggaata tataagttag agtttggggg ttgggtggta gagggttaaa agttgtgtgt 49320
aggttttgtt agaggatgtg tggttatgtt tggtttttag gttttgttat ggttttaaat 49380
tgattgtttt ttttgttata tttgttagtg ttattgttgt ttagagtttt ttatggtaga 49440
tgggggtttt gtgttttttt aggtattagg aggtgttgtt ttttttata tagtttttta 49500
tattttgttt ttatattttt tttataggga ttaggatttt ttgtttttt tttagtttgt 49560
ggaagggttt ttggtttggg tttttggtg tggttttgtt attgtaaatg gggttgttag 49620
gatgttttt tgaaattagg tttttgttt ttaggaagtg gggttttgtg tttgtttgtt 49680
tgatttttgt agtttgggtt tggttttgag ttttagtttt tattttagtt gggagtatat 49740
agtaattgtt tagaggtagg tttgttagat tttaggtggg gggttttttt agtaggtagt 49800
gttttgtatt gaagtgtttg tttttttat ttttttttt gatttttgtt atggagtagg 49860
gttaggttta ttatgtttag ggttgtggat atagaaaggt tttttgggtt gggtggaggg 49920
taaatttggt gttgttgaat gagggttttt ggggttgggg gtgatagttt tttgtttgtt 49980
tggtgttttt atttgtttta gaaattaggt tgaggaaggg tttggttgtg gttttttttta 50040
atgagttaga ttttttgtg aattgtagtt ttaaaatgga ttttatagag gtgaaagggt 50100
tttggatatt gaattaaatg tttttggagg tttggtttta agtgttttaa atatttgagg 50160
atttggagtg atttttggga agtttttttt agtttgagg tttgttgaaa tgatttagat 50220
ttttttaaaa tttagttagg gaaaggaaga atttaaggtt gggaatagtt tttttatttt 50280
ttttttttt tttttgaaa tgtttatggt tgaggaagga tggggaagga aggaattttt 50340
atttgggtt tttttattt atgagagaat taggataaag aggaggagag ttaggtagtt 50400
ttagagtggg ttaggaggga gagtaaggga attaggataa agaggaggat agataggtag 50460
ttttagagta ggtagggagg gagagtgaga gttttaaggg ggttttttg tagtggtttt 50520
tggtatttag ttttttatg tgtttgtttt ttggttgggt tagatttaag ttaggtaaat 50580

```
tttaatttaa aagtttgaga agaaaaggtg tttattagaa gattttgtgg ttatggttgt   50640
ttgttttaat atatttagga agtgtttagg attaaatatg ttaatgtgaa atatttttgt   50700
ttattttgtt aaaggtagtt atgtatttta ggtttgtttt ttgtggtttg gtttagtttt   50760
tgggggttat ttaattatat taagtatgat aggattattg agttgggagg aagtttagaa   50820
atttttttagt tagtttagtt tttttataat agagaagatt ttggtttaga gtggagatat   50880
gattgttttg atttgtattt gttttgagat taggttgaga gaggagtttg ggatgtgtta   50940
tttagggtgt gagggtttgg gttttgttgg tattaggggt ggttggtgtt gtatggagtt   51000
ttttttttta atgttttatg ggtatttatt tagtagttta gtttttttaga agagaatttt   51060
ggggtttagt ttttttttaag ataggtggtt aggtttaatt ttttgtattg tagagtaggg   51120
ggttattgtt gtgttagttt ttaggatgat tgagtaagtt agggtagttg tttaaggtta   51180
tttggagtta aagtttatga aggtaggtgg agagaagtat ttatttgtta tgtggtttat   51240
tgttatgtga ataagttaag agaggtgagg atttttttttg gatatttgtt taagatttta   51300
atagagatgg taatttaaaa ataaagttgt gggtagttgg tagatggatg ggtagttttt   51360
ttatattgat agtttttagt ttttgttggg tataggtttt agtatggtaa ggtttgatat   51420
taaggtgttg ttattgtttt ttagatgtta gtattatttt tggggtatgg gagtgagggt   51480
tttgggggggt atatagagag gtttgttttt aaggttgaaa tagtatttgg aggatatggg   51540
ttttagaat tttttttgaa ttttttttaat tatatttgat ttttagtgtt tattattgtg   51600
tttttagttt tgggaagtat ggtggttagg ttagtttaaa gatattgtga tggggtaggt   51660
gtagtaagtg tggggtgggt ttttagtata tatagttgtg attatattgg atttagtttt   51720
gtgttttatt ttttgttatt ttggttgggt agatgaggga tgtttttatg aaaattatgt   51780
atttgtttta tgtagttatt ttttttttgg gttagatagt ggggggttat ttttgagaat   51840
ttgaaagata tgtgggggtt tttggtttgt tttatggttt ttattgttta gtgtaaaggt   51900
aaataggttt agttggggga ggatggttgg taaattggtt tttaggataa tattttgtga   51960
ttatttagtt aaaaaataag aaggtgataa tgatttatta attttatttt atttttttta   52020
ggatgttgta gggaggaaag gtgtttttat gatttaattt tggttttttt tttttttttt   52080
ttttatttat atatatagag ttaggttttt atattattga ttttgaagga tagtttttaa   52140
tgtttaatta tttgtttagg tgtgtagtgg ttgaaaaaag aaagttgagt gttagaagga   52200
atatgaattt taattgttat ttattgtttt tattgtaaga tattttaatt ttgtataaat   52260
gtaatttttt ttttagttta atggtttggg gtggaatatt aaaaatatat attaaaaata   52320
tagtaaaaat ttagaaaaat gtaaaaaaaa aaatgaggtt ggttttataa agttttattg   52380
tttatattat tatgtaatta tattatagta aaatattaaa agaaatgttt ttgtttttta   52440
aagtaagtta ttgtatttat atttttttttg ggagaggagg aatatggatg ggaaggagat   52500
gtaggggttt aggtatgggg taaaatggag tgtagaggta gttgggtatt tttagaatat   52560
tttttgttgg tttagtttaa attgtttggt tgttgttatt tgtaataaat tttttttttt   52620
tttttt                                                               52626
```

<210> 9
<211> 221909
<212> DNA
<213> Artificial Sequence

<220>
<223> chemically treated genomic DNA (Homo sapiens)

<400> 9

```
aaaattttgt ttttattaaa aatataaaaa ttagttggtt gtggtggtgt gtgtttgtaa      60
ttttagttat ttgggaggtt gaggtaagag aattgtttga atttgggagg tggatgttgt     120
agtgagttga gattatgtta ttgtatttta gtttgggtga tagagggaga ttttgtttta     180
taaataaata aataaattag gtgtggtggt ttatatttgt aattttagta ttttgggagg     240
ttgaggtggg gggattattt taggttagga gtttgagatt agtttggtta atatggtgaa     300
atttgttttt tattaaaaaa gaaaaaaatt ataaaaaaaa aaaaattagt taggtgtggt     360
gggtatttgt aattttagtt atttaagagg ttgaggttgg agaattattt gaatttggga     420
ggtagaggtt gtagtgagtt gagattgtat tattgtattt tagttttggt gatgagggta     480
aaattttttt ttaaaaaata aaaaataaaa ataaataaat aaaaataaaa tttgaggttt     540
aaataattaa gttttgtgta tttttttaat tttttttttt ttttttttaat tttaatttta     600
tgatagatta agttttatgt attttaaaaa tgtgtagaat ttttattttt agtttttttt     660
tttttttttt tgtttttttt tttgattttt tggaattgga aggaaaattt ggatatttag     720
```

```
gaattattat ataggaggta tttatgtgat gtgtttaata ttattttatg taggtgtttt     780
tgggttttgg gtgtttaggt ttttgtgaat tgtgatttta ggtagaagtg tgagtatttg     840
tagaggaatt tatttattta aagttaggga atagttaggg ttggtatttt tagggtgatt     900
gtgttttagg gtatttttga tttgtgttga gtaaaatgga taaagttagt aatgtattta     960
taatttagat ttttgagata ttgttggatt ttgggaattt taggtattag gtttgtggta    1020
gtgttattat ggttaggttg gggtagtgt tattatggtg tttgttggtt aagttaggat    1080
atggtttaag ttggaatttg ttggtgttgg tataagtggt gagtgggtgt gtgggtgggg    1140
gttgtggtta gggtgtggat attttagaat ttaggatgtt gtttataatt tgtttatatt    1200
ttttgttttt tttttttata gtattttttt tttgaggttt tttgagtttt taatttttag    1260
ataaggaaag gggtggattt agttgaagtt tagtgtttat tttggtttaa ttagttttat    1320
ttggggtgag gggaaatttt tttggttggg ttgttttttt tgtagggttg tggttggata    1380
gattatttaa gaaaggaagt aggtgggttg gaatgaattt atggtgttat ataggttttt    1440
gtttttatgt atataagttt tttatattta ggtaggaaat gaaaatgttt tttggatttt    1500
taaggagagt aaaggagagg ggaagaggag gaggattttt tggggttgtt agtgatattt    1560
ttagtttttt gtgtattttt gggagggttg taggagaagg taatgttgtg tttttattta    1620
gaattagtga gtgtttttta attggttttt tgtttttttt ttgtttttta tggtttttat    1680
tggtagttgt gtaagggttt ggtttgtttt ttgttttttt atatttgttg tgatatttta    1740
ggtttttttag gttttgttat gttttttttt tttttgggaa tagttgtttt ttattatttt    1800
ggttgtttgg ttaattttttg tttattttt aaaattttgt tttgatttttg ttttaataaa    1860
atttgatat tatttttttag ttataattag ttattttttt ttttgtatgt tggaaatatt    1920
gtttattgt ggagatttta aggatattta agtagagaga atttttttgt gtaagttttg    1980
tattttttta ttattgtttg tgttatttga gttgtaaagg gtggtttttt gtgtttgttt    2040
tgttgtattg atatattgag ttgtgtatgg tgtttagtgt agggagaata aatgattatt    2100
tgtttaatgt gtttatttat aggtgaggaa attaaggttt taatttaatt gatgtatttt    2160
gttttttttga ttattagaaa agtagtagga taggtgtttt gttttgtttt attttggttt    2220
attaagttgg tattttggtt ttgatttttg gttgtgtttg gtgttgttgt ggtgtttggt    2280
gttgttgttt tgtttggtgg ggttgtttgg agtgtttgta ttttgtttg tttttatttg    2340
gttgggtttg ttttgtttgg atttgggatt gggaagtgtg gtgattttg gaattagtta    2400
ttggtgttag tgtggggagt tgggggtgta gagttgtggg tgtggtgggt tatgtaggtg    2460
gtttttattt ttggtttggt ttggtttggt ttggtttgtg ttgttgtgtg ggggtgtttt    2520
tttttaggtt tggtgtttgt tagttttgtt ttgttaggtg tagtgtagtg taggggtggg    2580
tgggggtggg gtttggtgtg tatgtttatg gggtggggag ggggtgggtt aggggtggga    2640
ttatagttgg ttgggttggg gttttatgtg tatttttggg gaggggtggg gtgggggtgg    2700
ggttggggtg gggtttggtt ggtgtatttt agatggtggg ttgttttttt tttttgtttt    2760
tttattattg gtttaggatt agtgttttgg gagtgtgtgt tttgttgttt tgttgttata    2820
ttttttttggg agtggtggtt atggaggtat tatgaagaag ttttatttag gtgggtttttg    2880
tgtttggggt ggggaggggt tggtgttttg ggattagtgt tgtttatttg agtgtttgtg    2940
gttgggagtg gtgaggtgtt tttggagttg agtgagtttt tgtggtgggt atattgtagg    3000
ttgagttttt tttaggatag ggttgttgtt gggttgtttt tgatttgagt tgattgtttt    3060
ttgtgttgtt tttagttttt gtttgagtgt tggaggggtt gttttggggg atgttttttt    3120
tttttttgttt tttgtatttt tgtaggaatt gttgattttt taggttggtt gggtgttttg    3180
ggtttttgtg tgtttgtgtg ggtgaatggg gttggggtta ggtggagggg tgtttttggg    3240
tttagttttt agggttggtg gtttaggttg tagtattttt tttttggatt ttttttggtt    3300
ttttttttat ttagtggggt atggatggt ttttagatgg gattgtttag tagtgtttaa    3360
atttggtgat ttgggtttat gttttgtgtt taggatgttg tttgtggttg atatttttat    3420
tttattttag tagggtttgt tttaagtagg tgaagggggt ttgatttggg ttttattatg    3480
agttggttta ggagttttta ttttataaga ggtttttgtt ttggtatttt gaagttttat    3540
tttatagatg ggtatattga ggttataagg gtaggttaag agaaatttag atttttatta    3600
tttgtttttt ttttttttt ttttttttt tgtggttttt tttaaaggat ttaggtaggt    3660
aggatttttt gttttttttta gtttttaaat tataaatagt gtttttttttt ttaaatttag    3720
aaattgtttg tttaatgttt ttttttttaat ttatttagat agaaaggtat ttgttaagga    3780
tttatgaatt aatttttatt gataggagaa tagaattatg ggttttagtt ggaggtgaag    3840
gagttattgg ggttattttt ttgtttttgg gttattgttg gttttttttg gtttttttata    3900
tttaggaatt aaaatgagtg ataaagtatt ttgttttaaa gaggattatt tagaattttt    3960
ttgaaaatat ttttttgggt tagtagttta gaattttaat ttttatttgg ttgttgtgaa    4020
taagatggtt ttttatgtat ttggagtata gttttgttag gagtgtgggt gggttggtgt    4080
gttggtgggt gggagtgggt ttttaggtgg tatatgtttg gttttggtat tggggttttt    4140
tttttaatat gttttaaata tttgagattg ttgtggggat tgtgtggtgt atggtttttt    4200
```

```
ttaggggatt tttagtttgt ttagggaggg tttttttgttt ttggtttttt atattttatt    4260
ttttattggt gggtttagat agtgggggtg attggggttt ttttaggttg tgggttttgg    4320
gtttttttttg tggagatttt tttgtataga tgttggaagg gttttgaggt aggtttggag    4380
tttttgtatt tgtggtttat tgggaggtgg ggagattatt atttgttttt tttttttttt    4440
ttttatatat atttatttta tttttattg ggtttttttt tttttttttt ttttaaaaaa    4500
aaggatagaa tttttttgtag taggtggaag tagagtttgt ggagtgagta ggaattaatt    4560
tttgtatagt agttttggtg ttttttttgtg tttatttaaa ggggtaggtt tttttgagga    4620
tgggggaagg gagggtttag attattttttg gtttgttttt tgtttggtag tggtttaagg    4680
tgtgttgggt tattgtgttg tagttgtatg gagtgggatt ggaggggggtt gatggtgggg    4740
gtggtttttt gttttttattt tgagtgtttt ggagttgttt tggtattagt tttttttttgg    4800
gtatgggtat ttgtaggagt taggttgatt ttttaataag ttattgatat tatgaaatta    4860
aggaaagtag taaaagatgt taaaaatttt ttaaggtttt gataatttaa ggataatagg    4920
aaggttttta ttttttttatt tagattgtgt gggattattg tttttagatt tgtatgtggg    4980
ttttagaata aatggaatga ttataatgtt gtttttggta gatttggttt gaatatttgt    5040
gagtttttga aaaggagaaa gatttttagaa ggatgtgttg gggtaatttt aagtattgtg    5100
ggtttataga tagttattga agatatttta tgtgttgggt atatatggtt aagtttgagt    5160
gggtatagaa atggtaatga gatatagttt ttgtttgtag agaatttata tttttattgg    5220
gagttaggat gagggtgaga tattagttta ttaatatttt tttttttttga gtagtttagt    5280
ttttaaatat tataggaatg ttttggaggt tttaggtgga ggtgagattg agttgggttt    5340
tgaggaagga gaagttttga gtagtggaga ataaaaaggg atttggttag gtgtgtttga    5400
agttttagtt atttaggagg ttgagatatg aggattgttt gagtttagga ggttatttag    5460
gaggttgaga tatgaggatt gtttgagttt aggaggttat ttaggaggtt gagataggag    5520
gattgtttga agttaggagt ttaaggttgt agttttgtga tagtgtttgg aaatagatat    5580
ggtattttag tttgggtggt gttatgagat tttatttgta agaaggtttt ttttaatgtg    5640
gaaggtagta agggtttata tagggtagtt gtaggattta gggggatatt ggggataggt    5700
ttttttttggt agttttattg ttgttttagg ttttttttgat tgttttattt ggtttttttaa    5760
tggggtggag gtagtggtag tttataggta gtgtggttta gtggttatat ttgggggttt    5820
agagttgtta gtttttttggg attaaatttt attaggttgt gtttttttttt gtggtataag    5880
gtttttattt tatgtatttt atttgtttta tttgtttttt tgtgtgtgtg gtgtttttta    5940
atttatttta tttatttatt tttgagatgg agttttattt tgttttttttag gttggagtgt    6000
aatggtatga ttttagttta ttgtaatttt tgttttttttag gtttaagtga tttttttgtt    6060
ttagtttttt gagtggttgg gattgtagga atgtgttatt gtgtttagtt aatttttata    6120
tttttattag agatggggtt ttattatgtt ggttaggttg attttgaatt tttgattttg    6180
tgatttgttt gttttggttt tttaaagtgt tgtgattata ggtatgagtt attatgttta    6240
gtttgtttta tttgttaaat gggatagtgt tatgttgttg taaaggttaa atgagtttat    6300
atttgaaaaa gttgagaaat gtttgttttg taatttgtta gttgttgtta tgattatgat    6360
tgttattgat aggaggatgg agaaggttgg ggttatgtta ttgttaggat gttttagaag    6420
tttttatgat ttttttttat tgtggttaga gtatggtgta gggttttttt tttagttttt    6480
tagtattgga gttagttgta ttgtatttgt tttggttttt taggttttga gttttgggat    6540
tttttttgta tattaggtta tatggaattt gttgttattt ttaggttttt ttggaaattt    6600
tttgtgggtg gtagtggtag gaggatttat ttgaggttga tgagttatag aagtgggatt    6660
gaggttgtgt tatattttta ttggtatttg ggaattattt tggagatttt tttttttattt    6720
tttatttttt tgagatggag ttttgttttg ttgtttaggt tggagtgtag tggtatgatt    6780
ttggtttatt gtaattttg tttttttaggt ttaagtaatt tttatgtttt agttttttga    6840
gtagttggga ttataggtat gtgttattgt gtttagttaa tttttgtatt ttttagtaga    6900
gatggggttt tattatattg gttaggttgg ttttgaattt tggattttag gtgatttatt    6960
tgttttagtt ttttaaagtg ttgggattat aggtatgagt tattgtgttt agttattttg    7020
gagttttttt gtggtttttta ggagattttt gaaggggtat tagttatgat taaaaagttt    7080
tatgttgttg tgatttttga gttttgggta gggagggagg ttgtatttat ataggaagta    7140
gtttttgttg tgatttagtg tgttggtttg ttgtggtgga tagggttgta tttttattta    7200
gtgttttggg agttgattta tgggaagggt aaggaattgt atttttagag aatatttttt    7260
tagttagtta atttggtgat aggtgagtgt gggaagggta taagtatagg gttttttttt    7320
tttttttttt agttttgttt ttgtgtttgt ttttgttgtt ttttgttttt ttatgtttaa    7380
gtttgtttgt tttggtgtat ttaggggatt tggattggag agaaaaagtt attttgtatg    7440
gttagtttgt ggttttgttt gatttgtgtt ttttgttgag gttttttagt tgtgagatag    7500
ttggttgagt ttaagttttg ggttagttt tttttttagt ttgttgtaaa ggtagaaagg    7560
tatgtttagg taggggttat ttgttttgtt tgtttatgtt agattgtagg tatttgtttg    7620
ttagattttt ttggtttgtt tagagggatt ttgatagttg agggtaatgt atttggtagt    7680
```

```
ggagtattgt gtttgtgata gtttattttt tttttggaat tattttttat aagtgtgatt    7740
agtttaagtt aggataggta taggggggatt taggattgtt atttaggggt agtggtttgg    7800
aatttaggtt taggtgatat gggagttaat tttttgtttg gagattttag gtgggtgttt    7860
gtagaggata ttttatttga ggatttatgg aaggggatat tttttatttt ttatttggtt    7920
tttgggttgt tttttttttga tattttttttg ttattttagg tagttttgat agttttatgt    7980
gattgggatt tagaggtggt ttttgttttt gttttagtgt ttgtttatag gtagtgttgt    8040
gtgggttttg aagtttgttt aagatggatt tgataagggg agtttagtat atattagtgt    8100
aggtttggtt agggggtgat gatggtagtt tttgggtaga tttagttggt tttagggtag    8160
gaggggtttt agagagaggt tttaggtttt taggggtgtg aatttttggg gtgaggtggg    8220
aagtgatgtg ttgggttttt gtgtttttgt ttgttggagt tattattttg gaaatatttt    8280
agttttaggt ggttgttagg gatttatggg gattagtggg tgttaatttt tgggtgaagt    8340
ttttggggtt tgtatatagt atatagtgtt gtggttttat ggtttatttt gttgtgtgaa    8400
gtggtttagt ttttgggaga gtttttagtt aggtaggttt gatggtaatt gttaggttat    8460
ggtgatttgg aattgttgat taaagggagt tagttttggt gggtggaggg atttggtggg    8520
tagttattgt gatgttaggt atggggattg tgtgttattt aggtttgtgg gtgttgagtt    8580
tttgtgggag tagtgaagtg tttttgtggt tgatgggttg gttattttttg ttttttttttt    8640
gatgtgatag ttgtgtttgg gttttgggaa atttgttagg aggtttggta ttggtttgtt    8700
ttattggagt ggttttgttg agttgtgagt tgtttgtttt tttgaggttt ttagtttatt    8760
ttaatttttt atgtggattt ggttatttat tttagaagtg tttgttgggt gttgttgagt    8820
gttaggtttt gtttgttttt ttttttgtat gttggaggtt ttgggtatta gttttgtagg    8880
gtggtttggt tttgggtttt tagggtagag tatggtattt tttgttttta gtttagtttt    8940
tttttgtttt atttttttga ttttattttg ggtatgggtt tatttgtagt ttttgtgaag    9000
ggagatattg agttgttgtt ggtagaggag gggtggtttt tgggtattgt ttgttaatgg    9060
tttttttggta gttttgtgat agttgagttt tgtttttagt ttaatttgat ttggtttttt    9120
aaagtatgat tgttggtagt ttgaaaattt ttaggatagg aaataggagt ggtgtaggga    9180
atgtaagatg atttttagttt taagttattg ggtggttttt agatttaggt tgtttgttgt    9240
ttttggtagg aatttaagtt gttggttatt tttagagtgg tgttggtttg gggtttttag    9300
tggtttttgt gtttgggtga gaggaatttt ggatggttat tttgttttga gtgtgtgggt    9360
ttttagaagt gttgggttag ggggtatgga gggttagaaa gttttttttg gagtgttgga    9420
ttttttttgtt gatttttatt ttattttggt ttggggtttt agagaagggg tttaggtagg    9480
agtgttattt ttttttaatg gggatgtggt tttagttttt gtttaggtgg gtgtattgtt    9540
tttttttttgg ttttttttttag gttgtttaaa tgatttgtga tgaggtttgt gttatggttg    9600
gggtttttttg ttttttttttag tgttgtattt gggaattagg ggttgggttt tggaagttga    9660
ggatgttttt gaatgttatg tttgggtatt tgttttggtt aggtgaggtg tgttggggta    9720
gtggttgaga tgggagtagt agtattgttt gttaggtttg agatgggttt tgggtgagtg    9780
tggttattag gaagggttgg gtagagtagg tgggtgggtt gtttttgggg ttttgttttg    9840
tagattaatt tgatttgtat tttggggttg gggttttttt ttatagtttt tttatgaatt    9900
atggtttttg tttttatttt aaattagatt ttagaagagg aataatgttt atgtaattta    9960
agttagttgg aagtgggtta tatttaattt ttgttatttt ttagtttgtt aaggatttga    10020
ttaatttaat ttagagggat ttggagggtt gtttgtaaaa tttattaata atagggaatt    10080
ttagttttgt agttataggg tttatagagg tgttgggttg ttgggttatt gttgggaggt    10140
gagttggtgg tgtagatatt atggaggtat tggtagtgga agagttgtaa gtgtatatag    10200
aggttggtat tgaggggtgg ggaggaggag gtggtggatt ttttgttagt gttggtatta    10260
tgttgttttt agtattagag atttaaggtg tggttgattt tggttggggt gatgagttat    10320
tggatagtag agttttttttg gtagttgggg gttaatgttt taattgttgg gattttgtat    10380
tatgttgttt ttttttttttg ttttgtttgg tttggtttgg tttggttttt gagatggagt    10440
tttattttgt tatttaggtt ggagtgtggt ggtttaattt tggtttattg taatttttgt    10500
tttttgggtt taagtaattt ttttgtttta gttttttgaa tagttgggat tataggtatg    10560
tgttattatg tttggttaat ttttgtattt ttagtagaga tggggttttg ttatgttggt    10620
tgggttggtt ttaaattttt gattttaggt gatttatttg ttttggtttt ttaaagtgtt    10680
gggattatag gtatgagtta ttgtgtttag tttgtattta tgtttttttt ttttttttttt    10740
tgagatggag ttttgttttt gttgtttagg ttggagtgta aaggtgtgat tttagtttat    10800
tgtaattttt atttttttggg tttaagtgat ttttttgttt tagtttttta agtagttggg    10860
attataggtt tgtgttatta tgtttggtta attttgtatt tttagtatag atggggtgtt    10920
tttatgttgg ttaggttggt tttgaatttt tgattttagg tgatttgttt gttttggttt    10980
tttaaagtgt tgggattata ggtgtgagtt attgtatttg gtttatgttt tttattatta    11040
ttatttaatt ttttaaggaa agtaatttaa attgtatatt gtattttttt ttttttttttt    11100
gtttttttgtt gttagttttt tgtattattt attgttaaat aatatgaata ggaaattgaa    11160
```

aggaaaattt taataattat tggttgtagt atttttttg tgttttattt agtgtttgtt 11220
tttttaggaa tatgatatag ggtttgattt aattatgaaa ggtgtatatt tttagagttt 11280
tatattttt atagaatgaa tgttgaattt tgtttttttt atttgaaatt agattttagg 11340
tatggatttt atgattatta ttataatggt tttttagagt ggatattgta ttttgtaggg 11400
aattattttt tttttagttg ttggtgtgga tgtttttttt tgatggtgtg ggtttgggtt 11460
tttgaagggt ggtgttttga gtaaagatgt tatagtagtt atgtttatag ttttgggttt 11520
ggggttaggt ggagattggt tttaatttta gagtttgttg ttttaaaata aatttttttt 11580
tttttttttt tgagatggag ttttgttttg ttgtttaggt tggagtgtag tggtgtgatt 11640
tttgtttatt gtaagttttg ttttttgggt ttatattatt tttttgtttt agtattttgt 11700
atagttggga ttataggtat ttgtttttat gtttggttaa ttttttgtat ttttttagta 11760
gaggtggggt tttattatgt tagttaggat ggttttaatt ttttgatttt gtgatttatt 11820
tgttttagtt tttaaagtgt tgggataaaa taaattttta gggttgggag tggtggttta 11880
tatttgtaat tttagtattt tgagaggttg aggtgggtag attataaggt tagaagattg 11940
agattatggt gaaattttgt ttttattaaa aatataaaaa attagttggg tgtagtggtg 12000
ggtgtttgta gttttagtta tttgggaggt tgaggtagga gaatggtatg aatttggaag 12060
gtggagtttg tagtgagttg agattgtgtt agtgtatttt agtttgggtg atagagtgag 12120
atttgtttt ataaataaat aaataaataa ataaataaat aaatttttat atttatttgt 12180
attatttatt attattatta ttaaataggg ttttgttgtg ttgtttaggt tggagtgtag 12240
tggtgtgatt atgatttttt agttttgatt ttttagtttt aattggtttt tttttttag 12300
tttttgagt agttgggatt ataggtgtgt gttgtatgtt tggttaattt tttttttttt 12360
tttttttttg agatagagtt ttattttgtt gtttaggttg gagtgttgtg gtgtaatttt 12420
ggtttattgt aatttttggt ttttggatta aagtaatttt tttgttttag tttttttgaat 12480
agttgtgatt ataggtatgt gttgttatat ttggttaatt tttgtatttt tagtagggat 12540
ggggttttat tatgttggtt aggttggttt taaattttg atttgtgat ttatttgttt 12600
tggtttttta aagtgttggg attataggtg ggagttattg tgtttggtat gtttggttaa 12660
tttttaaaaa atttttgtag tagttgggtg tggtggttta tgtttgtaat tttagtattt 12720
tgggaggtta aggaggttgg gagtttgaga ttagtttgat taatatggag aaattttgtt 12780
tttattaaaa ttataaaatt agttgggtat ggtggtatat gtttgtaatt ttagttattt 12840
aggaggttga ggtaggagaa ttgtttgaat ttaggaggtg ggggttgtag tgagttgaga 12900
ttgtgttatg gtattttagt ttgggtaaag agagtgaaat tttgttttaa aaaattttg 12960
tttttgtttt tgtagtgata gggttttgtt ttgttgttgt ttaggttggt attgaatttt 13020
tagttttaag tgattttttt gttttagttt tttaaagtgt tgggagtata ggtgtgagtt 13080
attgtgtttg gtttttattt gttttggttg tttttttttg ttttaggtaa ttatatattt 13140
ttttaggtaa ttaagaaaag attgtgtatg atgagttttt tttttggtat ttttgggatt 13200
gtttttaaaa ttagataagt gtttttattt tgatatttta ttaagatttt aatgaataga 13260
atgttgtttg tttttagttt gttggttttt ttgtgaaggt ttagggtttt tttgtttgaa 13320
aagttatttt tttgttgagg tttttgattt tttttttatta tggtagttat gttgagggtt 13380
tatagagtta tttttttttt attttttttt tggtttttta agatgtattt gtatttttgg 13440
agatgttttt ttgttttttt gggttatttt ttttggttgt tatttatttt gttgatttat 13500
tatttgtgta atagtttttt ttggaatttt taggtttttg ttatttattt tttgtagttt 13560
ttgttttgtt tttttttgttt tagttttttt ttttattatt tttttatttg ttggttttta 13620
tttttgttta tggtatattt ttgtgttata ttttgatggt tggataaagg ttgagtgaaa 13680
tgtatttatt tttttggtga ttggggtgtt tattaattag aagggattta aggttggtgt 13740
agtttttttt tgtattttgg ggtattgttt tgtggttttt ttttgttgga gtttgtagtt 13800
ttttgttttt tttttttttaa atttttgag atggagtttt gttttgttgt ttaggttgga 13860
gtgtagtggt gagattttag tttattgtaa tttttgtttt ttgggtttaa ttaattttt 13920
tgttttagtt ttttaagtag ttgggattat aggtgtttgt tattatgttt ggttaatttt 13980
ggtattttta gtagagatgg ggttttttta tattggttgg gttggtttg aatttttgat 14040
tttaggtgat aggtttgtgt tggtttttta aagtgttgag attatgggta tgagttatta 14100
ggtttggttg ttgagattat ttttataagt tattaggttt ggttgtagtt ggtagttttt 14160
gattattggt tttgttttat gtgatttgtg attttttttt tggtttttg gttttgtttt 14220
gtttatttgt ttattttttt tatgtatgtg gtttgtttat agggtatgta atggggttta 14280
attaggaaat ggaaattgtt tgagtattta tttatttatt tatttattta tttatttatt 14340
tatttttttt aagataaggt tttattttgt tatgtaagtt gaagtgtagt ggtgtgatta 14400
tagtttatgg tagttttgaa tttttgagtt taagtgattt tttttttta gtttttttaag 14460
tagtttggat tataggtgta tattatatgt ttgtttaatt tttttttta tttttgtaga 14520
gatagggttt tgttttgttg tttaggttgg ttttgaattt ttaattttaa gtaatttttt 14580
tgttttagtt ttttaaagta ttggagttat aggtatgtgt tattatgttt tgttaatttt 14640

```
tgtatttttt gtgtgtgtgt aaagataagg ttttattatg ttgtttaggt tggttttgta 14700
tttttgggtt tatgtgattt ttttgtttgg gttttttaaa gtgttgggat tataggtggg 14760
agttatagtg tttggtttat ttgagtattt agaatagaag gatttaaagg tggggaatta 14820
gttatataga tgatgggaga ggtgaaggtt agatagggaa tagagaggta attagtagga 14880
aattaaagtg gtgtgggttt atatagtttt ttaaaggtat ggatgtaatg atttatttag 14940
aatgagaaaa taaataatag taaattataa attataagta gttgttaaat attataaata 15000
ttattatatg tagaagagta ttagttaatt gttagttata tttttaaagt tttatttttt 15060
ttatattttt ggtattattt ttttaatatg atgattttgt aatattgttt tttattttga 15120
gaatagaaaa ttgattaggt tggatatagt ggtttgtgtt tgtaatttta gtattttggg 15180
aggttaaggt aggtggatta tttgaggtta ggagtttaag attggtttgg gtagtgtggt 15240
gaaattttgt ttttattaaa aatataaaaa ttagtagggt atggtggtat atatttgtag 15300
ttttagttat ttaggaggtt gaggtgggag gattgtttga gtttaggagg ttgaggttgt 15360
agtgagttgt gattatgtta ttgtatttta gtttgggtga gagagtgaga ttatgtttta 15420
aaaaaaaaaa aaaaataata aaaaaaaata taggttgggt gtggtggttt atgtttgtaa 15480
ttttagtatt gtgggaggtt gagatgggtg gattatttga ggttaggagt ttgaaattag 15540
tttggttaat atggtgaaat tttattttta ttaaaaatat aaaaaattag ttaggtgtga 15600
tgatgtatgt ttgtaatttt agttatttgg gaggttgagg taggagaatg gtttgaattt 15660
aggaggtaga ggttgtagtg ggttaagatt gtgttattgt attttagttt gggtaataga 15720
gtgagatttt attttaaaaa taaataaata aataaagtga atgaattttt ttagttgatt 15780
aaattttgtt tttatttttg gtagttggga tgtatataaa agtggtggtt aatatggagt 15840
tggtggtgtt gttataattg ttttgtttaa gatatatagg aattttggta aattttgttt 15900
tttttttttt ttttaaatgt gatttttatt aagaaagaag taggaaaatg gggagatttt 15960
ttaattgtat atgaggtatt tgtgtatgta tttttgatta gagagatttt tttgttttga 16020
ttttatattg aaggaattga attttttgtt tataatttta tttttggtga tgggagtaat 16080
tttttataga tttgtttttg gatttgtgta ttttaaattt tgtttttttt tattgtgtat 16140
gtttttttgg tgttaggtat tgtaggatat gtttgtgttt tgtgattttt gattttgtgt 16200
ggtaggtgga attggaatgg tgagtggagt gagtattgtt ggaatttgtt tttatattgg 16260
gatagtgagt agtaattgaa ttatatatgg aaataaatgg gaattatata aatataattt 16320
gattaaagtt aaattaaata tttttttagt ttaatttttt tttagtagga gtttttatgt 16380
ggtagtaagt ttttgttttt attttatata agagaaaatg tgatgggtag gaatttaaga 16440
ttggaaatga atggggatgt taattgattg tagggaaaat atttttttatt ttgtaaattt 16500
tataaaaaat gtttgatttt gtggatatgt ggttagggtt tttttagtg ttttgagagg 16560
gaattatgta ggtagaggga gggtttttag tattgtgggg gtgatttgga gattggtagt 16620
agggtgttat tattattttt aggtggggat gagagggagg aagttttgtg attagagttt 16680
aggggttaga gttatttgtt gggagtttaa ataaaggtgg gattttggta ggagttggaa 16740
ttatatagag gattattttt tttgttggag aagttgtttg aggtagagga ggtggagagt 16800
ttttgtgttt tttttttgttt ttattttta ggtttttttt aagtttttg ttggttgaat 16860
ttagtttaaa gttagtaggt ttggggattt ggatatttga gttagtagag gttggtgttt 16920
tttttatata aaggagggta taagaagtgg gaggtatgga gggggattag gttaggattt 16980
atataggatg tgtaaatagg agtttttttt ttttttatt ttaaatggta tttgtagttg 17040
gttttatttt tttgttgtta gataggaaga ggaattttgt ttttggtatt agttgttttt 17100
attgtttttt attttatgga ggtttttttt tttttggtt tttgtttgta attattggtg 17160
atgttgatgg attatatttt ttggggagtt tggttatgaa tatggttttt tatttgttgt 17220
ggattaaatt gtgttttttt ttttgaattt ttgtgttgaa gttttaattt ttaatgtagt 17280
tgtaattgaa gatagggttt ttagggaggt aattaaggtt aaatgaagtt atagggtagg 17340
attttaattt aataggattg atgtttttat aagaagaggg agagatatta gagatttttt 17400
tttttttttt atttgtaaat agaggaaaaa tgatggagga tatagtaggg aggtgggtat 17460
aagttaggaa gaggggtttt gttgggaatt aattttgatg gtattttgat ttgggatttt 17520
gggtttttta aattgagaaa tatatgtttg ttgtttaagt tatttttttt atagtaattt 17580
ggatggtagt ttgagtttat taaggtattg tttgtattag ttagggtttt ttagggagat 17640
agttaatagg agatggatgg atggatggat ggttagatag atagataagt ggatagagag 17700
agagagagat aggtagatat agatagatag gtagataaat ggttagatag ataagtagat 17760
agagagatag agatagatag atataaatag gtaaatagat aggtagataa atggatagat 17820
agatgataga tagttaggta ggtagataga tatagatagg tagatagata gatagataga 17880
tgataggttg ggtttagtga tttttatttg taataattag tatattggta ggttgaggtt 17940
ggtggattat ttgaggttag gagtttgaga ttagtttggt taatagggtg aaattttatt 18000
tttattaaaa atagaaaaat tagttgggtg tggtggtgta tgtttgtaat tttagttatt 18060
tgggaggttg aggtatgaga attatttgaa tttgggaggt ggaggttgta gtgagttgag 18120
```

```
attatgttat tgtattttag tttaggtgat aaagtgagat tttattttaa aaagaaaaaa 18180
aagatagata gatgaatgga tagatagtta gatagatgat agatggatga taggtagata 18240
gataattgat aaatagatat aaatgattga tggataggta gatatagata tattgattag 18300
gtagatgata gatggataga tagaagatag atggtggatg ggtgggtaga taggtagata 18360
gatagttaga aagatagatg atagatagat aggaaggaat ttattagggg aattggttta 18420
tattattgtg gaggttaaga tgtattatag aaggttgttt gtaagttgga ggtttaggaa 18480
agttagtatt gtggtttagt ttaagtttgt aggttttagg attgggaagt tgttgatgta 18540
atttttagtt taaggttaaa ggtttgagag tttggggaat tgttggagaa ttgggagttt 18600
taagtttaag ggtaggagag gaaggtattt tatttttagg agaaagaggg aatttatttt 18660
tttttgtttt ttttgttgtt tggtaatgga atggtgttat ttatattgag ggaggatttt 18720
tattatttag tttattaatt tatatgttta ttttttttag aaatattttt atagatatat 18780
ttagaagtga tgtgttatta gttatttttt aatttagtta aattgatatt tgttgttagt 18840
tattatatag gtttagaaga attgtggttg tatggtgatg gtttttattt tgtttaaaga 18900
agttgaagat gaattgttgg tataagttat tgttttttta ggtttttttt agttgtttat 18960
tttttaaaa atgtatttat tgagtatttg ttgtatgtta ggtgttgtga taagtattgg 19020
ggtatagtag tgagtaaata aggattttg ttttttggag tttatatgta gtagaaggaa 19080
atagaattaa tgaatttatt agttttagaa attattaaaa taggattaag agttgtggtg 19140
agaattaaga taaggtgatt gtatagaaag tgattgatgg ttgttttgga atgagtggtg 19200
aggggttata gagatgatga tagttgagtt gagattggga agataggaag gagttagtag 19260
tgtaaggatt aggttggaag taagtttggt gtattttagg aatagtaagg aggttagagg 19320
ggttgtagta gagtaggtga ggaggaggag ggagatgtag gttaaagagg agtttggggt 19380
tagatggagg attttttttt taaagtttag ttattttttt tttaaaggag aatgtgttat 19440
ttttaaaagt tatatatgtt agtatagtgt ttggttatt atatgttttt agtaggtggt 19500
gagtatggtt gtttttgtta ttattatatt ataattatta ggaataatgg agttattagg 19560
ttttgtattt atagtaatgt gagggagtta gtaagttttt ttggggaatt tatttttttt 19620
tattaagttt ataagagttt gataattagg ttaatgtagt gagagttata gttgtttagt 19680
gtattggatt ttttagttat gggttaggat atttttattt tagtagaaat ttttaagggg 19740
attggggagt gtgttgtgga tgtttttggg ttatttagat tttttgggga gttttttttta 19800
ttgtagttgt tgtagggagt ttagttgtag tgatgaattg tggtttagtt ttgtttatgg 19860
gaaaatttta tatgggagat gggatggttt tttgttggtt tttttggttt taggttgttt 19920
ttataagtgt agggattttt atttgggtgt gtgaatgggg tttaggatgt tttaagggta 19980
tgtggagatg ggggttagtt gtttttttttg atgtttttga attttttttt tttgtttttt 20040
tgtattttat tgttttttgg tttttttat tttgggtgtt ttggaataat atgtaattta 20100
atatataggt tgattttatt attttgtttt gttttatttt attttatttt ggaaataaag 20160
gtttgttat ttagtttgga gtgtagtggt gtaattgtaa tttattgtag ttttgaattt 20220
ttgggtttaa gtgatttttt tgttttagtt ttttaaagtg ttgggattat aggtatgagt 20280
tgttgtgttt ggtttaggtt ggttttaaaa gtttttttatt ttttttgtat tttgatgata 20340
tttatttttt ggagtaggtt tttagttatt ggttttggat attttgtagt tattattggt 20400
tagttgtggt atgtgaagtt gaggtttagg agaagggtag ttttgtttaa ggttatatgg 20460
tttagtgtta atatttagat tttagtgttt ttattttttt tttggttgtt tttattttta 20520
gtgtattttt ggaggtttaa gggagttaaa ggttaggttt tggttagtta agaatttaaa 20580
aaagttgttt ttgtgttttt ttgttaattt attattttat tatttttttt gataatgaat 20640
ttagtttgaa ttttagataa ataaggatta atttttagta taagtatatt ttataataga 20700
tttgggatat atttatgttt aaaaatgatt tattgtttgt tggttttagt agtttatgtt 20760
tataatttta gtgttttggg aggttatagt tggagaattg tttgagttta ggagtttaag 20820
gttatagtga gttatgatta tgttattgag ttttagtttg ggtgaagtag taagattttt 20880
atttttttaa aaaattgttg attgtttatt tgaaatttag gtttaattga gtattttgta 20940
ttttatatga gaattttata taggtgagat ttagagtttt aatgtttatt ggtaagtttt 21000
tgtagggagg ttttgtgggt ttttggttgg tattgatgtt ttgtgatgag tagggttatt 21060
tggtaaggtg gatgtttgtt gtttatgtag gtagagtaag gttttgggta ggtatgagtt 21120
gattttattt tttttagttt gggagttaga tattgttggt agtttgttgt taagagaggg 21180
gttggttatt aagagagggg tggtttttat gggttttttt tggttttttgg ttttttttta 21240
gtttttttttt tattttaaat agaggttaat ggtattgtat tagaattagt gtgttgtttt 21300
ttttggttgg tattttgtag ttgggtagat tgtttttttt ttataggagt tggtggtgtt 21360
tttttttgttt tatagggagg atggttgaga tttttgattt tgatataggg tgttgttttt 21420
tttttgtttt gagatagagt tttggttttt ttatttaggt tggagtgtag tggtatgatg 21480
ttggtttatt gtagttttta tttttgggt tttagtaatt tttttgtttt agtttttttga 21540
gtagttgaga ttataggtgt gtattattat tgagaataat gtttttttata gtgtttattt 21600
```

ttttgtagga gtaagttttt aaggtaagga ggttgttttt tttatggatg aataaattga 21660
ttttgattta tagttttatg tgttagtttt gagggtgggg aaaggttgtt ttgtgtttag 21720
gtagggggtg ggggtaaggt taggagggtt tggtgtttag tgtatttttt taggatataa 21780
ttttaggtag tagaggttaa ggatagaggg gaggagaaga gggaggtggt tattattttt 21840
ttattttagt tttttttgga gataagtttg agtattttag ttgtttatta agtttggaga 21900
taagtttgga atttaaattt ttgattttga tttagttttt tatagttttt gtgagtaaat 21960
tgagttttag aaaaagggag tgaagtttgt gtattttgga tgtttttatt ttaggtttaa 22020
attgttttta ttttaggttt aaattgtttt tattttagtt taaattgttt ttattttagg 22080
tttaaagtgt ttttatttta gtttaaagtg tttttatttt aggtttaaag tgtttttatt 22140
ttaggtttaa agtgttttta ttttaggttt aaattgtttt tattttagtt taaattgttt 22200
ttattttagt ttaaattatt tttaatatag gtttaaatta tttttaattt aggtttaaag 22260
tatttttatt ttaggtttaa agtattttta ttttaggttt aaagtgtttg taatttaggt 22320
ttaaagtgtt tttattttag tttaaattgt tttattttag gtttaaattg tttttatttt 22380
aggtttaaat tatttttatt ttaggtttaa attgttttta ttttaggttt aaagtgggaa 22440
ggagttatag ggaaataaat gggggaggta tggatgatta ggatgagggt ttttatgatt 22500
tattttttt taggggttat tttaagagtt ttttaaattt ttattatttt ttaatagaga 22560
tggggttttg ttgtgttgtt taggttgatt tttaattttt agttttaagt tattttttta 22620
ttttagtttt ttaaattgtt gaggttatag gtgtgagtta ttgtatttgg tttttttttag 22680
gagttttaag aaaaaaaatg ggaatagatt tgtgtgtgtg tgtgtgtgtg tgtgtgtgtg 22740
tgtgtgtgtg tgtgttttga gaaagagttt tgttttgtta tttaggttgg agtgtaatgg 22800
tatgatttg gtttattgta atttttgttt tttgggttta agtgtttttt ttgttttagt 22860
ttttgagta gttgggatta taaatatgta ggttgatttt gtatttttat tagagatggg 22920
gttttattat gttggttagg ttgattttga attttggatt ttagatgatt tgtttgtttt 22980
ggttttttaa agtgttggga ttataggttt gagttattgt gtttggttag gaatagattt 23040
tttttgtag atgattttg ttattattga aaatggatgt ttatgtataa ttgaattgtt 23100
ataatgtttt attttaaaaa gtttttttt gagaaaatgt gatttttgat agaggagaga 23160
ttgtttaggt tttatttatt gttagtataa aggtagagta ggggtgtggt ggtgttattt 23220
ttttttgaga tagttttatt tttgttttgt ttaggggttt agaagtttgg tttatttgag 23280
tttatagagg ggtttggagt ggttgtttta tattataggt ggggtttgta aggatttgaa 23340
atgattttg agaaggtggg tgtggtgggt gtattttaat tattaaagta ataataatga 23400
tatattaaat agtgtttgtt tagattttga gtgtttgttt aaatattttt attttgtgat 23460
ttgttagatt ttagtataat aatttaggta aaagaaaaaa tagttgggtg tggtggtttg 23520
tgtttgtaat tttagtgtga ggttaaggta ggaggattgt ttgagattag gagtttgaga 23580
ttagtttggg ttatttgaga ttttattttt gaaaataaat aaattaataa aagattttta 23640
tgttttatgt tttttttata tttaggtttt ttaaattata tgtattgggg aaagtttatt 23700
aagagaagtt tttggttggg tgtggtggtt tatgtttgta attttagtat tttgggaggt 23760
taaggtggga ggattataag gttaggagtt tgagattagt ttggttaata tggtgaaatt 23820
ttatttttat taaaaatata aaaattagtt gggtgtggta gtgtatgttt gtaattttag 23880
ttatgtagga ggttgaggta ggagaattat ttgaatttgg gaggtagagg ttgtagtgag 23940
ttaagttggt gttattgtat tttagtttgg gtgatagaaa aagattttat ttttaaataa 24000
ataaataaag ggagagaagt ttttgggttt ttagatatat gggttagtgt ttatttttgt 24060
aaagttgttg tgtttaaaga ggggttgtat ttggtggaat tggggtaaaa gagtagggag 24120
ggtattgggt agttgatttg gtttttatta ttggaaaggg gttgtgttgt tggtgttttg 24180
agttggtttt ataaggaaga tattgtatag ggtgttttaa gtttgttttt tttgtttagg 24240
tttatttttt tttttttagg attagggagt tttgtttgtt ttttgttttt ggtatttgtt 24300
ttttggtttg tgagtattgt attttttttt gtttggaatg ttggtttatg gtgggttttt 24360
ggattttgt tttttgttgt tttttgttat atttgttaa agtgtgtttt atttatttat 24420
tgttattatt ttttgaaata gagttttgtt ttgttgttta ggttggagtg taatggtgtg 24480
atttggttt attgtaattt ttgtttttg ggtttaagtg attttttgt tttggttttt 24540
tgagtagttg ggattatagg tgaatgttat tatgtttggt taatttttgt atttttagta 24600
gagatggggt ttttttatgt tggttaggtt ggttttgaat tgattttggg tgattggttt 24660
atgttagttt tttaaattgt tgggattata ggtgtgagtt attatgtttg gttaattatt 24720
ataatattga tattataata atattatagt tagataatgg tgattaatgt ttgtaatttg 24780
aatattttgg gaggttgagg taggtagatt atttgaggtt agtttgagat tagtttggtt 24840
aatgtggtga aattttgttt ttattgaata tataaaaatt agttggagtg tgatggtagg 24900
tgtttgtaat tttagttatt tgggaggtgg aggttgtggt gaattgagat tgtgttattg 24960
tagtttaatt tgggtgatag agtaagattt tattttaaaa gtagtaataa taataataat 25020
aataataata ttatttattg agttttaatt ttgtgttagg tattgtgtta gtatttgtgt 25080

tatgtgattt tattgatttg gaataattta tgagtaagta ttatttttgt ttttaaggaa 25140
gttgagattt aggaaatgga agttattttt tagggtttat agtaagggtt tggtgaggtt 25200
tgtgtttaaa ttttgattat ttgattttag gttttttttgt ttttttttgtt ttttaaaaat 25260
ttaaatattt ttatttggga tgattttaga tagttagatt ttaattgttt tttttttttt 25320
tgagtatgtt ttattttaaa attatataaa gataatttat tttttaattg gagttatgga 25380
attgttagat gttttggaga tttttttagt tattttttttg ttggatttag atataggttt 25440
gaataattag gtttaaggag ttgaattgga gttgtttgtg gttagaggtt ttttttttggg 25500
aatagtagtg gtggtagtgg ttgtgttgag tttggtaatt tgtttaagag taggggatgt 25560
ttaggttgtg tttgtttttt ttttatgaat ggtttgtttt gtgttttttt tgtgttaagg 25620
gttgttaggt attagatgtt tattttagga aattagaaaa ataaatagtg taaataaaat 25680
aaaaatttta tttaagaaat ttgagataga tgttgttttt tttattgttt ttttattttt 25740
atgttgggtt tggagagagt gggttagtat gttttttatg gataagaggt tttggggtat 25800
ttaatttgga tggaattttt tttattttttt ttttgtttgg tgttaggggg tagaaggtag 25860
gggtggtgtt tatttggttt agggaggagg atagggtagt ttttgttgtt ggtatttttt 25920
gtagagatgt tttagttaga agtgatttta tttttaggaa tgtgtgtttt gttttttgtt 25980
gggtggtttt tttttatttt ttattgtttt tatagtgggg tgtgttagat ttatgggtag 26040
agttttggtt tatttttagt tttggatagt agaatgtttt agtagtgtat tgatggtgtg 26100
tgtgtgggga tgtgtggaat aggggatagt gattttgtgt gtgttatatg tagtgtggtt 26160
tttagttggg tgtgtgaagg gggtgtttat ggttattgtg gaagtgagtt tagttggggt 26220
tggagggttt aggtttattg tatgttattt agtagttttg ggagtggggt ttattttttt 26280
ttttgttttt tttttttttt ggttttgggt agtttattgg agttgtattt tgggttattt 26340
tttttggttt taaggaggtt gttattttttg ggaggtagat gttggttttt ttatttttgt 26400
tttttggaga tttgtgttga ggtttgtttt tttgtttgga gggattggtt aggaattttt 26460
gggttttttt ggtggggttt ttgttgttta gtagtgagta gagtgagtgg gtattaagag 26520
gaggatgttg ggtgaggtta tggatatggt tggggtaggt tgggggtgtt ggaggtgaat 26580
tttgtagttg tgtgattttg ggtagtttgt ttagttttgg gttttgtttt tttttttatg 26640
aaatggggtg ggagtatttg tttgttgggg ttgtgggttg ggtgaattgg agttaaggta 26700
tagtttatag tttgttgtta gatttttgta agtagaagtg ggtgtttttg tttagtgttt 26760
tttggttttt tttttaaggt ttagttttgt ttttattttt tttatgaagt ttttttggtt 26820
attttaggag ttatgattta tattttttttt ggagttttgt gttatatgta ttttttatgg 26880
tgttgttttt tttggaggat gggtttaatt aaaattgggg tttatttggg ggtttttttt 26940
agtattagag aagtttaggt ttagtagaga tgggtatttt gttaatatta gttttgtgaa 27000
ggatatattg tgtttatttt gttgttagtt gttgagtttg gtgttttgta gttatggaat 27060
atgtgtatag gtttgggttg ttggatatga aggttagatg ggtagtttat tttgttttttg 27120
ttttgagttt ttagaaataa gttggttgta tttattttta tttggttata gtttaggagt 27180
ggggttgtgg aatggatgga aggaaggatg agtgggtggg tgggggggtg ggtgggtggg 27240
tggatggatg gatggatgat gggtggatgg gttgatggat gggtggatta ggtggatagg 27300
tgaatggatt ggtgggtggg tgggtgggtg aagggttgaa taggtgggtt gggtggatgg 27360
gtggatggat gtgtggaggg gtggtggggt ggatggggtt tatgggtgaa tggatggatg 27420
gatgggtgaa tggagtaaat gagtgggtgg atggatggat ggatgttgta agagtttttt 27480
tggagggttt tagggaggaa ttggtatttt tgattagtag ttagtatggt gattagatat 27540
agggaggtgt ttgggtttgt ttgatttttt attttattgt tggtgggatt gggttggtta 27600
tgtgttttttg aagtttttttg aagggtggtt gtatggtatt gtttagggga tatttttaga 27660
gtgttgtgtt ttatagtaat agggtggttg tttgtgtttt tagggttgtt ttttttggta 27720
gtttttttggg tttgtttggt ttagtatttt agtaaatttt tttgagttag gatttgtttg 27780
ttatgagttt ttttaataag tagttttagg ttaaggtttg tgtgtgggta gagggagtgt 27840
atgttgtggg aggttgtatt aatttgaggt tttgttttttg tttttttttttg gggtaaaagg 27900
taaaaatagt ttgttttatt ttattttttaa atttagtttg gttgtattta gttttgggag 27960
tgaagggggt tgggttagga ttgtttgtga gttgtagatt ttggtgtagg gaaattttga 28020
tttataagat gggtggatat ggaggaagga ggagattttg tttagtagag atatgtttgg 28080
gtgtagttta gtttggagtt tgatggatgt gggtttattt gtgtgtaatt tttatttatt 28140
gtgtggttat agggaagtga tatttgtgg agatttagat tttttatttg taaaataggg 28200
gtagtagggt ttgggggagg atgtgtgtga aggtttggtt tagggttttt ttagtagtta 28260
gatagtagtg gttgttattt ttttagtgtt tagggttatt tgggggagta gggtttttgt 28320
ttttagatgg gttttgtttt tggttttgga atttatagta ggagtaaagg tgaggatatt 28380
tttggtgtta attatttatt tggagggaga gtgttagtgg ttttgtgtga tttttgtttt 28440
ttgtttttgt ttttaggagg tatgtggatt tttagtggtt ggttttggag gtttggtgat 28500
tttagtggtt taggtaggtg gtttgttttg ttttggtttt atttagttta tgggtgtgtt 28560

tgtgttgggg ttttttatt ttggtttggg atttgttttt ttatttggtt tttttggatg 28620
agtggggtta tttgtttatt ttttagggag atgtagtttt tgatggtttt ggatttaggt 28680
ggtttttagg tttgttgatg ttgttttgtt ggagatgatt tttgtggggg taggggtagt 28740
tatttttagg tttagggagt aagtttgtgt gtgtgggata gaggagagaa taggagatag 28800
atagggttgg atgatttggt ttgagatgtt gaggggatag atgtgttatg gataggtggt 28860
tttggttgtg agtttttgg tgttggttta gtatttgtgg gtttttgta attttgtagg 28920
atggaagatg ttttaggata gagtagatat gtttggtgtt atttaggagg aaataagtgg 28980
gtttttggta gtggttattg tgttattttt tgtgtttttt ttatgtgttt ggaaattttt 29040
tatattaatt agtagggaag ggaaatagat gtatttatta gagttttggg gttggttgtt 29100
taggatatga gttatagttt ataggtttga agggatttta ttaatattgg gtgtagtttg 29160
ggtaagggag tgggatagag aggttagggt tttgtttta gtttttgttt tttagggttg 29220
agtttgtgtg ttattttttt tttattgggt gtttttgatt ttttttgttt gattttagtg 29280
tggttatgtt agtgtttttg tttttgggag tttatgttgt ttttggttgt tatttttagt 29340
ggttaggttg ggataggtgt ttatttagtg gtttttttat ttggagagga tgatgggtta 29400
tagtaatgaa tggatttatt tttttagga gatgaggagt tttgagaaga gtagagatgg 29460
tgtggttatt tgtgtatatg tgttgggggt taattgggtg ttagattttt ggttgttatt 29520
atggtggtag ataggtaggg attgtgtttt tatgaattag ttattttaga ggagataaag 29580
ttattaaagg atatggattt ggagttaaat ttatataaat agagtgtttg tggattaggg 29640
ttgtttttag aataaggtga gatagttgtt ttgttaggat aaggtgttta tgggggtaag 29700
ggtatgtgtt tatttggagt tgttattttt atgttttaga ttagttttgt ttaatagaat 29760
ttttgtgat ggtgggtttg ttttatatat ttgtattttg ttattttgta tggtagttgt 29820
ttagttgttt gggtttattg tatatttaaa gtataaatgt ttatgtttat tttatttatt 29880
tatttatttg tatagaatta tttatttatt ttaaatggat ttttatttat atttatgtgt 29940
ggttagtggt tatgaaattg aatagtttta gattatattg gtatttgaga ttttagaatt 30000
ttagatttaa agtttgtttt tgattttgag tttgtttttg tggggatagt atagatgttt 30060
tttaagtttg tttttttagag gtggatgtag tgtttgtgga ttgtgtatta ttaggtttgg 30120
atagtggaga gggtggtttt ttatagggag tgggagtgga ggggatgttg tgtttatatt 30180
gagtgtatgt gaggtttttt gtagtatagg atgggattgt atgtgggaag agggggggtga 30240
tgttttggtt ggaggttttt gggtagttgt atgatttagt gttgtggtta agagggggttt 30300
tgtttattgt agttatgttg ggtattaatt attggatagt ttagttgttg ttgtgttttt 30360
ggggagtttt tttttggttt tgtatttgat ttgttttttt atttttttt tttttattgg 30420
ttagtttttg gtggaatggg tggggagggt tttttagttt ttgtgggggt tgtattttgg 30480
gaatttggta ttttgggttg tatggatata gttttttttg gtggttgttt tttttgttt 30540
agattggttg gttttgtgtt gttagtattt ggtagttatg attatagttt agtttggaag 30600
tttaaggtat aatttgttga attttaaagt tgtaagttgg tgatttgggt ttgtgtggga 30660
ttgtttgttg atatatgggg ttttttttt gtgggttttt gtgggttttg gtttgagata 30720
ttttttttt attttttgg ttttttttt ttttttgtt tttttttgt taagatttg 30780
gtttaggtag gagtttattt ttagatgttt tagtattttt ggttttttta tttgatattt 30840
tttagatgat tgtttgttat tttattttt tagtttagtt tttttgtaga tttatatttg 30900
gttaattttt tatgtttggg atttttgttt tttttagaag gatgaaggtt ttttttgagg 30960
ttgtgtttgg gattgggtta gggatgatat ttttggtaaa agtttaaatg atattatgat 31020
taatgttttg gttagtttat atattttttt ttttttaaa aaaaaaatgg aatttttaga 31080
gttataaaat aaaaatgtaa agaataaggt aataaaatat ttgtgtattt attattttgt 31140
ttaagaagtg agatattgta ggtgtagttg tagttttggg tgattttttt tattttagtt 31200
ttatttttgt tgtggtgttt gttttttgg attgggattt ggttttgggt aggtatattt 31260
gtgttgggta gtggtgtgtt tgagaagttt aggttttttt ttttttttt ttgagataga 31320
gttttgtttt attgtttagg ttggagtgta gtggtattat tttggtttat tgtaattttt 31380
gtttttgggg tttaagtgat tttttgttt tagttttttt agtagttagg attataggta 31440
tttgttatta tgtttggtta atttttttg tatttttagt agatataggg ttttattatg 31500
ttggttaggt tggttttata tttttgattt taggtgattt atttgttttg gtttttaaa 31560
gtgttgggat tataggtgtg agttattgta tttggtttgg ttttttttg tatatggtta 31620
ttagtattgt gtgattgagt agtttaggtt tgttttttt tgaatgttgt tattagtatt 31680
atatttttgg tgttttattg gagttagtgg ttgttttgtt tgtgtttata atgtgatggt 31740
gtggagattt atttgtgtag atttatgaag ttttggttta tttatttgg tagttagtga 31800
gtgttttttt gtgtggggag gtagagtttg tttgttttgt ggtttattgg tgatattgtt 31860
gaaggttttt tttgttatta gtgttgttgg gaatgtgtgt tttttaggt ggatttggtt 31920
gggttgtgga gatgtgtttt ttagttttt tagatgttat tattatgttt ttttgagtgg 31980
ttttttggta ggtttttta tgtttgttta tttttgtgt tttaggtagt tgtttttttg 32040

attgagtgtt tagtggattt ttgttttgtt ttttatggtg tatggagtat tttattttag 32100
tggtgtttga gttttttttt ttttggtttt.tagattttgg ttgggatttt tggagttaat 32160
tttttttgttt gatattttgt gttgttaggt tttgtttttt tattttttaa tatttttggg 32220
gtgtgtggtt ttttttttag tgttttattt atttttttttg ttttaggttg aattgtgttt 32280
tttataaatt tatgtttatt aaaaatttta gaatgtgatt tgggagttgg agatggggtt 32340
tttgtagatg taagtagtta aggattttga gatgaggtta ttttggatta gggtgggttt 32400
taaatttaat aattggtgtt tttaaaagaa gtagagaggt tatagatata ttggggagag 32460
ttatgtgaag gtaggagtag atgatggagt gatgtagttt taggttaggg agtgtttggg 32520
aagaggtaag gaaggatttt tttttatttt tattttagag tttttttagg gagtgtggtt 32580
ttgttgatat tttgattttg gattttttggt ttttagtatg ggagagaatt tgtttttttatt 32640
tttttaagtt ttatagttgt ggtttattat ggtagttata ggatattgat gtatttttta 32700
tttggttgtt gtggatggat gattttgttt gtaggaagtt tttttttaagt attgtaggtg 32760
tttttgtttt tatagggggat gtttgtataa gtaggtgtga aaaaagggtt ttgtgggggt 32820
aaggtttggg ttggtgtttg ttgttttttt ttattttatg ttagttttta tgtttttttt 32880
ggtaggagtg tttggtttta tttggtgaat tagagttaaa ttttttttgat aattggttgt 32940
tatttattta attgttatta tttttaggag atagaaaatg ttagaagggt ggggtttgtg 33000
tagtgttttt ttgttatttt tttagggtat tgtgttatgt tgttttaatg tatagagtag 33060
ttgtttaaag tgtgttggat gaataggtgt tttatgttta gttatgtaga taaaagtgtg 33120
ttatgtggag ggagttggtt gtggaagaga ttattagtat tttggttgtt tgaggatttt 33180
gtttttgggt ttttgtaggt atttttagtt tattattttt tttttttattt atagttattt 33240
attttttttt tattttttttg atattagttt tgttatttat ttaggtttgt aagtaggaaa 33300
ttttagagtt gattttgatt tttgtttttt tagtattttt aggttttaat aggttgttaa 33360
gattttatta atttgttttt tgtatattga tttttggata ggttgttttg gttatggtta 33420
tgttttggtt gggtttttat tattttttttt tggattagta ggtgttattt tttttattttt 33480
tgttttgttg tggttttttt tttttttatgg ttaatattta gaggatgatt attattttttt 33540
tttttgtgtg ttggggattg ggtgatagga tttttttattt tgattatttt tattagattt 33600
gggttgggtt tttttattta tagagttttt agtttttgtt tttaaagttt tgagggtgga 33660
tttgtgttgt tgtttagttt ggttttgata ttgtttttta gtttggagtt ttttgttttt 33720
gtgtttttgg ttttgagtgt·ttgggttttt tttggatggg ttgttatttg tttttttggaa 33780
agtagggttt ttatgttttt ggattttttta gttgatttag tgattttggg ttattttgtt 33840
atattttttt tattagtttg gttttggtta tttttgtgtt ttttatagtt ttggattaga 33900
gttatggtgg gatgtttttt tggtgtgtgg taggtagtta ggaaatattg ttggattgat 33960
tttgatagga gatgtttggg agaattgttt aaaaaattag tttaaaatgt aaatattggt 34020
tttttttttgt gttggttaaa gagtagtatt tttttgtttg tttttttgggt aagaaattga 34080
tggtgaatgt aatgttttta gggaggagaa gaaggtgata aaaagggttt gtataatggg 34140
gtatatgtta ggtatttgtg attatttaag tttaaattat aatataaatg tgaaattttt 34200
tgttttggta gtttttttat attgtaagtg tttattgtta tatgtggttg ttatattgat 34260
tatggtagat atagaatatt gatattattg tggagagttt tatggggggg tgttggttta 34320
gattattttt atgttggatt tataaattta ttgtttattt agtgaatggt tattgggtat 34380
ttattgagta agtaggatga aagatatttg tttttggggg agttttttgt tggttgtaga 34440
gaagtttaaa tttaggggat ttggggtaaa ttttgtaagg gtttttggtt tttgtatttt 34500
tagattttttt ttgttttgta ggggtagttg ggggtttttt ttaggaatgg agtgttagtt 34560
ttagttttttg ttaggttata gtttagatgg ttggttgagt atgagtatgt gagagtggaa 34620
ttgggtttaa ttgggttgtt tttttttgtt gttttggttt tttttttatgt tgatttggaa 34680
ggatttttaa agtaataggt ttgtttaggt ggtttgtatt agtaaatttt gtagtggttt 34740
taagggtaga gatagttgga ttgtatttat ttaggaatag ttaaagtttg aggtataata 34800
aaaagtggag aaagatttta gagaggaatg tagttggttt tagtttggga atttgttttg 34860
tttgggaggt tgtaggtggg ttaggttggg gttagtttgg agggttttgg ttggttttgt 34920
tggtaggtat agtagtaggg ttagtaggag ggagtttagg atggagtttt tttattttta 34980
atttagggaa gtaaatttta gtggttgagt ttgaggatga gtaggaaatg aatggaaaag 35040
ttttttttttt ttttttttttt tttttaggta gggttttttt ttgtttttta ggttggagtg 35100
tagtggtata attatagttt attgtagttt taatttgtta ggtttaaggg atttttttgt 35160
tttagttttt taagtagttg ggattatagt tgtgtgttat tatgattggt taatttttaa 35220
aagaaatgtt tttaggttag gtatggtgat ttatgtttgt aattttagta tttttggagt 35280
tgaggtggga ggattatttg agtttaggtg ttttagatta gtttgggtaa tatgatgaga 35340
ttttgttttg attaaaaata taaaaagtta gttgggtgtg gtggtatagt tttgtagttt 35400
tagttatttg ggaagttgag gttgtagtga gttgtgatta tgttattgtt ttttagtttg 35460
ggtaaaggga gtaaggtttt gttttaaaaa aaaaaatttt tttttttttt gaggtggagt 35520

tttgttttgt tgtttaggtt ggagtgtagt ggtatgattt tggtttattg taatttttgt 35580
tttttaggtt taagtgattt ttttgtttta gtttttaag tagttgggat tataggtatg 35640
agttattgtg tttggatttt ttttttttt ttttttttt gtagagatgg gatttttta 35700
tgttgtttag gttggtttg aatttttggt tttaagtgat ttttttattt tggtttttta 35760
aagtgttgga attataggtg tgagttatta tatttagtag gaaaagttat tttttaaagt 35820
gagaggttgt gtgtttttta tagtttggtg tttattagta tggaggattt attggaatgt 35880
gtgttaggtg aggggatatg gaagatggta gtaaatttga gtttagagta ttagaaagta 35940
tgtttttagt taaagatgtg tttttttat ttttgtgtag tattttttttt tagtgtgtat 36000
ttttggagta aggtggggtt gattttatga agattttttt ttgaaggttt ttgttgttta 36060
tattagtgtt atttggattg tttgtttttt tttttggatg gttggattag ttttagggtt 36120
ttttggtttg gtgtggggag tatagtttat tttggggaat gtttggtatg tgtttttttt 36180
ttattgggta ttaggggatg aagttgtgtt ttgtggaagg ggatagaggt gttggtgttt 36240
aggatggtgt atttggggtg gttgaattgg atttggtttt tattgggttt ttgtggagtt 36300
tagattgttg tgagaagggg tggggaaagg taggtgattt gtatttttta tttttgtttt 36360
atatgggaat aggggtttat tttgtatata tatttagttt ggagtatgaa tttttatgaa 36420
gaggaaaatt tttattaggt tttgatgtta ggattgtttg tgagggttgg gttgtgtttt 36480
ggggtaagga taggagtgga gggggtaatt agggaaggtt ttttaatgga ggaggtattt 36540
gatagtattt tgaaaaagaa gtagttttta ggttaagtta gagattgaag aatgattatt 36600
taattttttt ttttttttt tgagatggaa ttttgtgttg ttgtttaggt tggagtgtag 36660
tggtgtgatt taggtttatt gtaattttta ttttttaggt ttaagtaatt tttttgtttt 36720
agtttttga atagttggga ttataggtat gtgttattat gtttagttaa tttttgtatt 36780
tttagtagag atggggtttt attatgttgg ttaggttggt ttggaatttt tgatttgtg 36840
atttatttgt tttggttttt taaagtgttg ggattatagg tatgagttat tgtatttggt 36900
tatgattatt taatttttt ttataagatg ttttttggtt gatgtaaagt aggttgagtg 36960
agtgtgatat tttttttttg tttgtgggtt ttggtttttt tagagtttgg ttagtgttat 37020
tggagataaa gtggttttg tttttttttt ttgttttgtt aggtttagtt tgtgttgttg 37080
agttttattt tgtaattaag aggagttgtg aattagtgat gttgatgttg tagaaaagtg 37140
gttaggggtt tatgggggt gaaggatagg gatgttgttt tgagaattgt ttggaagggg 37200
tttttgggta atagtgttta ttttagggtg tgggtaggtt ggggtggttg ggagttgggt 37260
ttataggttg gtataattag agttttttaa agttgtgggt ttgagggttt tgttttttttg 37320
ggatagtaat ttttggttta atttgggaaa aggtgggtta aattaagttt ggttttttta 37380
ttgtaggatt atttagtgtg tttagtatgt aaatgtgttt ttgaatttaa gataggatta 37440
tagaaatttt tttagtgttt ttttggaggg agggattgtg ggggatttat tgtaggggtt 37500
tgggtttgta ttggtttggt gaagtgtttt tttagtgttt atattatgtg tggtgagagg 37560
ttggtttttg ttttttttat tttttgttgg ttttatttta ttattttgt atttgttttt 37620
tagttggttt atgattaatt tttgtttttt tagagttaat tggtgttgtt aggtttttta 37680
ttttaagtga tttttttgttt gggttaaaat gtttggattt ttaggtatgg aggggagatg 37740
gattttaggt taaattatgt tgggtaaaat tgagggttaa ggttgtgttt agtaagtgtt 37800
ttagttttgg ttgttaaatt tatagtgtgt atagtgtgta ggaggtggtt gggtgaatga 37860
atttagttat agtttggttt tttatttggg gttgtttta agtttttatt gttagttagg 37920
ttgggttaga gttttgtggg gtttttttgg gtttttttgt tttatagtag gagtttgtat 37980
ggagagtttt gagtgaggtt aatgggtttt ttttttttt ttttttttt tttttaattt 38040
gagatagggt tttattttt ttatttagtt agggtgtagt agtgtaatta tagtttatag 38100
tagttttgaa tttttggttt taagtaattt ttttgttgta gtttttttaa gtgttgagat 38160
tataggtatg tgttattttg tttagttggg gtttttgttt tttattttt tttttgtttt 38220
taaatttaat ataaagaaag atattatatt tataattaga aaaattaata aggaatatta 38280
ttttaaaatt ggaaaaggga ttaaagaaat aaattaagaa atttttgtga tttttgttta 38340
ttggagttag tggttgttag tggttggttt aatataatgt tttaggaaga aatagaataa 38400
tagaaagtaa ttttagtgag ttgagttttg gttttgtttg tggtgttgta ttagtttttt 38460
gtagttgttg taatgagtgg ttataagttg gttgggttat aataatgttt agttattttt 38520
ttatagtttt gtaggttata tatttgagat tagggttatt aggttgaaat tagggtgttt 38580
attgggtata gtttttggga ggttttaggg taggggtttt tagttttgtg tgttttatta 38640
ggaattggtt tgtatagtag gaggtgagtg attggtaagt gagtgaagtt ttatttgtat 38700
ttatagttat tttttattgt ttgtattatt gtttaagttt tgttttttgt ttgattagtt 38760
atggtattgg atttttatag gagagtgaat tgtattgtga atggtatatg tgagggattt 38820
aggttgtgtg ttttttatga gaatttgatg tttgatgatt tgttattgtt ttttattatt 38880
attagatggg attgtttggt tgtaggaaaa taagtttagg gttttaattg attttatatt 38940
atggtgagtt gtataattat tttattatat attataataa taatagaaat aaagtgtata 39000

attaatataa tgtgtttgtg gttgggtgta gtggtttata tttgtaattt tagagtgaaa 39060
ttttgtttta aaaaataaaa aataaaggtt aggtatagtg gtttatgttt gtaattttag 39120
tattttggga ggttaaggtt ggtggattat gaggttagga gtttaagatt agtttggtta 39180
atatggtgaa attttgtttt tattaaaaat ataaaaatta gttggatatg gtggtaggtg 39240
tttgtagttt tagttttttg ggaggttgag gtagaagaat tgtttgaatt taggtggtgg 39300
aggttgtagt gaattgagat agtgttattg tattttagtt tgggtggtag agtgagattt 39360
tgttttaata aaataaaata aaataaaata ataaatataa tatatttgaa ttattttaaa 39420
attatttttt tattttttgg tttgtggaaa aattgttttt tttgaaattg attttaggtg 39480
ttaaaaaggt tgggggttat tgtttttatt gtttgttttt ttgagttttt ggtggttgtg 39540
ggtgtttttt ggtttgtggt tgtatggttt ttatttttaa tattagtatt tatagatttt 39600
tttgggtttt attttttattt ggttattttt gtttgtgtta aatttttttt tgtttttgtt 39660
ttgtatgagt attgagattg tttttaggga ttatttagat tatttagggt gtttttgtta 39720
tagtaagatt tgtaattgaa ttatagatgt aaaaattttt gttttaaata aggttttatt 39780
tgtaggttta gggattagga tatgttattt ttggggttat tatttagttt ttttttgtga 39840
ttatgatttt tttttttttt tttagttttt tattttggtt gagatgttgt ttttattttt 39900
tttattgttt ggttgtttgt ggggttgttt tttaggtttt ttgggaggag gtggttgttt 39960
gatgtttttt agaaggttgg gaatggtttt ttttattttt taggaagatg tttttttttgt 40020
taaggagatt gtatttgggg ttttatttta ggtagagagt ggatgggatt gtggtttgaa 40080
gtggtatttt agggttttttg gttttgttat ttagttgagg gttgtgtatg ggttttattt 40140
ggttataggt aatttatttg gttttgtggg gtagggattg taaatttaga ggtttgtggg 40200
gtgtgttgag gtagaggggt gtaaatattg taaatttgtt gggggttttg gggagtagta 40260
tagattgaaa agtgttttgt ttgaagagta gaattgtttt atattagttt tgtgtggtta 40320
ggtaggaagg tttttgtggt gtggttatgt gttttttttt ttttaataga agttgatagt 40380
ttggagtttt tagagagatt gtttgagttt ttagttttgg tataaatata tatttttttt 40440
aaataggttt gggttaatag aattagtttt tgtgttttgg gtttgaatga atttttgagg 40500
tgaagtttaa agatagagag gatgtttttg atttttattt tagggattta tattgtaaat 40560
ttttatttgt tttgttgagt ttttggtttt agtatggatt ttgattatgt gttggtgggg 40620
attttttagg taagtaagta gtttttgagg attttggatg aatagtgggg aatagtattg 40680
atttttttagt ggggttttga gtttttggag aggaagattt gttttttttt aggggatggt 40740
tagagattta ttgatttatg tgtgtgtggt aggaattttt taggaagttt ttgttttgtt 40800
tgtttttttt gtttgggtgg ggatggtaat tgtttttgtt attgtagatt aatgggatgg 40860
agggggtga ttttttaggg tttttttttgg gtaggtgttt tggaattttt ttttagtatg 40920
ttggtttggg gtatggttgt tttttttgtt tagttatgtt ggggtatagg gtgaagaagg 40980
gttggggtta gtttaggata gaggaaggtg aggtaggtat gtaggaattg ggttttttaa 41040
atttttaagt ttaaggaaat tgtagtattg tgggataggg aaaatgaaag atttggaag 41100
ttgttaggaa tttgattttg tgagttggtt tttaagagtt taagttaagt atttttaagt 41160
ggatattaaa aaggagtagt aagttttggg gtggtggggg ttggaggagg tggagagagg 41220
aggttgttgg aagttgtatt tgggattttt gtagttattg attagattgg gtggttggga 41280
ttttgggatt tttgtaggta gatttggtgg tttgttggat tttttggggt ttattttggg 41340
tttttttttt tgtttttttat ttttggattt tttttttttg tttttttttt tttttttgttt 41400
tgaagagata atgttatttt agtttggagt ataaatatat gattagtata tggaaatgtg 41460
gtaatttgga tgtatttgtg attgtaatag attgaagaaa ttagattaga taaagagtgt 41520
ttttagagga ggaggaggag gaggaggagg ttgagagagg gagggtgatg ggggtgagaa 41580
aggggaggtt gtttttgagt gggatgttgg gatttttgtt gttgttaaat atatttgtag 41640
gaatggagag ggattggatt ttaggtatgt agatagtttt ttttttattg gttgtttttt 41700
attgttttgg atttggggtt ttatttatgt ttgggggaat aagtatgtaa agggtgtttt 41760
tgtttatttt ttatagaaat attttttttt tttttgatgg ttttaagttt gtgggatgaa 41820
ttttgtttta gaagttaagt ttttttgagt ttggttttta tttaaatttg gttttttttt 41880
tttttggtta tttttttttt atttttagt gtgttatttt tgtatgttat ggttattgtt 41940
aatgttgagg ttgttggtgt tgaaattttg ggagggtgtg ggggatttgt tttttttggtt 42000
tgggtttggg gtggtggtgt ttggatttgg ttggtggttg gtttgtttat tgtgtggttt 42060
gtttttttgtt tttagttaaa gtttgttttt aaggttgttt tggtttttta tttaaaatag 42120
tatttttgtg ttttttttggt agttgattat agagggggaa gtttttggtg tttagaggag 42180
tttggtgtgt gtgtgtatgt gtgtgtgtat gtgtgtgtgt gtgtttattt atgttggtag 42240
tttttttgtt gtaggtatag atttattagg gtgtgaatag gtaaatgaag agaaaggggg 42300
tttttttgtt tttagtttat tttagttttt taggtttttt tttttaggtt tagttttttt 42360
ggtttttttta tttatatgtg gtgttgtggt tgttatttta tgtggttagt gttgttattg 42420
gtaattagta gtgatgatag tttttgttgt ttatgttgtt ttttgagttt agtgttggga 42480

gatatggggt ggttgagtta agttttaggt tttgggtatt ttgaagtgat ttatgggtta 42540
tggagtggga ggttgttaag agtgttaatt tttatgtgtt ttgggttttg gtttgttggt 42600
tgttattatg tgtgtgttgg ggggtttttg agaggtttga gagagggagg tttattttgg 42660
tggttttttt ttaggatgtg tggttttttt tattattgtt tttagggtta tatttttttt 42720
tttttttttt tttgagatgg agtttggttt tgttgtttag gttggagtgt agtggtgtga 42780
ttttagttta ttgtaagttt tgttttttta gtttatgtta tttttttgtt ttagtttttt 42840
aagtagttgg gattataggt gtttgttata tttttttgta tttttagtag agatggggtt 42900
ttattgtgtt aattaggatg gttttgattt tttgattttg tgatttgttt gttttagttt 42960
tttaaagtgt tgggattata ggtgtgagtt atggtgtttg gttttagggt tatattttga 43020
ttgttatgtt gggattggtg gagtgggtgt gtgttgtgtt gttgagtata tttagttggg 43080
tatagtaggt ggattttagg aggggttag gggagatggt agaaggtttg ggtggtagtg 43140
aaattgtatt ttatttgttt gagtttgggg gtttaggttt gttgggatgg gtgggtgtgg 43200
agtttagtgt tttgtttttt gtttagtagg agttgtaggg tagggagtgt tgagagggat 43260
ttttgttagg tttgtatagt ggagttgttt ttggagtgat tattttatta ggtagtttgg 43320
gtagattata gtgggttttt tgtttttttgg gttattagtt ttaggaggat gttattttat 43380
ttgtttggtt ggtaggggtt ttggggagtt ttgggtttgg tttttatttt atagatgatt 43440
tagttgaggt ttagatgggt gattagtagg ggatttgaga gttgagtaga gaggtgtggg 43500
tttgggggtg gtgtttttttg gggagttgga agtgatggta ttttttttagg tttataaaat 43560
atgggttttt tatgaattga ataaaatatt ttttgaaagt agttagtgtt gtgttttaga 43620
gattattttg attttttgttg tttttggttg aggatagtat agttagggat gaagtgggga 43680
ttttttttttg ggttgtgttt tgatgaaaga tgtttttttat aaaattttat ttggatgtgt 43740
ttttatgtaa gaaaatgtat atttttagaa atttagttgt aaattggttg tttttttgaat 43800
gaatatgata gttttggagt tgtttttttt aatgaggttt ttgttttggg taaattttag 43860
tgttggtttt taattgttat tgaattttttg tatagttttt ggggaagtga tttttttttt 43920
gtttttttgttg ggtggttttg agtaagttat ttggtagttt tgggttgtag ttttgttatt 43980
tgtaaggtga ggggtgggag tggtatggtt tttaggtagt gtttttattt tttgtgggtt 44040
tttgtgtgtg ttttgagttt tgtgggtgtt tttttttttta ttgaggggtt tttttttttt 44100
gtagggtatg gtttatgttg tgttgttagg ttgtaagtta ttgtgagagt tgttttgatt 44160
tttaagtatg ttttggtttg aggaggagtt atggggagga tgttgtgttt gtgtgataga 44220
ggtttgtttg gtagatgttg gtttgttgat ttttagatgt ggttgtgggt gttttatgtt 44280
ttattgggtt ggtgtgtgag gtgtgtttag tttaggttgt tttatagttg tttaggaagg 44340
gtttgtggga gggggtggtt ttattttggt gagtttatgg tggtttgtta gttttaggga 44400
aggtttagga gttttaagat ttgttttttt gttttttttt tggtagtgtt tggttttggt 44460
ttggtttgtg aatgggggtt tgggtatttt tttgtttttt ttggggggttg tggttttttt 44520
ttgggaaggt gttggagggt ttgttagtag ggttggtatg agttatggag tggtgattgg 44580
ttgttgaggt atggaaggtt atttttttta gttaagggtt ggagtttttt ttttgttttg 44640
gaatggtttt gtggttttta taaattaagt agttttattt tgttttaaga gtttgtattt 44700
tagtagggag agagttttta agaagggaat tttttattgg ggagttttgt tgggttggga 44760
ggttaggaat aggttatgag ggagggttgt aggtagggga agtggttgtt tgtgtatggt 44820
tatgaatgta ggtttggggt tttggagggg gtttggttat gttgttgttg gatgtttgtt 44880
tttattaggg tttatttgat gtagtatggt atagggtaaa gtttagtttt ttttatgggg 44940
tttttgggtt attagtgggt ggggaattga ggtttttttgg agggtggtgt tttagttttt 45000
attttttttt tttgtttttt gttgtgttgt ttttgatggt ttttgttatt ttttgggagt 45060
ttttattttt ggatgtttgg tgtttgtttg gttttaattt ttttagtttt gttttttttat 45120
ttttgaggtg agaggtttgg tttgatgtgt tttgtatttg tttttttgttt agttttgtgg 45180
gaattttggt ttttgtagtg gtttttttggg gttgttatta ttaagtgtta taaattgggt 45240
ggtttaaaat attagatatt tttttttttg ttgttttgga ggttagaagt ttgaaattat 45300
ggtattagta ggtttggttt tttttggagg ttttgagggg gagtttgttt tttgttttttg 45360
gtagttttttg gtggttttttg aaattttttgg tgttttttttgg tttgtggtta tattatttga 45420
tttttgtttt tgttgttatg gggttttttt ttgtgtttta aatttttttt tgtttgtttt 45480
ttatatggat agtttttttt ggatttaggg tttattgggt aatttaaaat ggttttattt 45540
taagattttt aattttataa tgtttgtaaa gatttttttt ttaaataggt tatgtttata 45600
ggttttgggg tggatgtaaa ttttaggggt tattttttat ttttttgttg tttttgtgtg 45660
tttggatgta tagtatggtt ttatgtttta gatttttttgt tggatttttt tggtttttttg 45720
gggtggtttt tggttttttt tatttgtttt tgtgttttttg gtgaggtgtt ggggttgggt 45780
ttagtttttg taggaatttt ggggatagta tggagttggt aatttttttat taggtgtttt 45840
tttatttgtt tagagtttat ggttgtgagg ggtttttttt tgtggttttg atttgtaatt 45900
ttttagtggt tagtgatgtt gagtattttt ttgtgtttat tgtttatttg tatatatttt 45960

tgtagaaatg tttgtttaag ttttttattt gtgtttgatt tggatgattg ttgttgttga 46020
gtgttaggag atttgtagat taatatttag atattaattt tatatttgat ttatgatttg 46080
ttgttatttt atggattgtt ttttttttat gttgttagtg ttttttgata tgtaattttt 46140
tttttttttt ttgagatgga gtttgatttt gttgtttagg ttggagtgta gtggtatgat 46200
tttggtttat tgtaattttt gtttttttgg ttttagtgat ttttttgttt tagttttttg 46260
agtagttggg attataggta tttgttatta tatttagtta atttttgtat ttttagtaga 46320
gatggggttt tattgtgttg attaagttgg ttttgaattt ttgattttaa gtaatttatt 46380
tgttttggtt ttttaaagtg ttggagttat aggtgtgagt tattgtattt aggttgatat 46440
gtaaatattt taaattttta tgatgtttta ttttatttat ttgttttttt gttgtttgtg 46500
tttttggtgt tatatttaag aaattattgt taaatgtaat gttaggaagt tttttttttg 46560
tgtttttttt taagagtttt gtggttttag tttttgagtt taggtttttg atgtaagttg 46620
agttgatttt tgtatgtggt gtaagggttg gtttagtttt atgttttggg tttttgattt 46680
attttttttt tttttttatg tttagttggt tttgttgggt ggtggggagg agtggggaag 46740
ttttgggttg ggtttgtatt tgattatttt tttttaggtt agttagggag ttttagtttt 46800
tgtttaggat ttggttggat gtgtttttta ttgggaaagt ttgggttgtt tttttaggtt 46860
gatggtaggg ttagtttggg gtgttttgag gtttgttttg tggatatgtt ttttgtttta 46920
ggtggtgtgg ttgtttggtt tgtgtgtgag attagttgtt tgtgttttag gttatggagg 46980
ttgagtgttt ttagtttgtt tttttgtttg gttttttttt tggggaagtt tttgtagttt 47040
atttttgtt tttgtttttg ttatttgtgt ttttgtttgt tttttgtttg gaggtggtta 47100
tttttggggt tatttttat gatttggata taagttttta ttttgaagtt attatttaaa 47160
tttttgtgtt ttaaattttt tttatttatt atatgggttt ttattgtgat taatttagta 47220
gttgatgaag ttttttttgg ggttttttta gtaatgggga gttggttttt ttggaggttt 47280
ggtttttttt taagtggaag tggggtgtga gggtgttagt tttttttttgt tgtttggtgt 47340
tttaggttgg tttgttattt ttggaagtat ttgttatttt tatatagtat tttatattta 47400
ttttttttgtt ttttattttt tttttttaagg ggttattttt gttttttttt ttatttaatt 47460
ttatttatgt ggtttgttta gtaatttttg atttttaata tgataaaata aatttttttt 47520
gttggttatt tatttattta tttagtattg ggtgtttttt gtggatttgg tgttggggtt 47580
ttgtggagga taaagttaga tatagttttt gtttttatgg gattgtataa gtgtaagatt 47640
atattagtaa atgtgaaata taggaagtga taggtgtgat aaaggggatt agtggtagga 47700
tagaatttgt.ggtttgtagg attaggttag gagggttgtt ttggggggat atttttgggt 47760
tgagtgtaga aggatgaggg gagtaaatta ggtttaaatt tagtaggtag aggtgattgt 47820
tgtaggtaat tggtaatgtg tttaaaggtt ttggggtgtg gggggttgag gttggtagta 47880
tggtaggaag taagattggg gttgaaagag attgattgtt atgttgtgaa atatatattt 47940
ggtttttatt tttatttttt ggtatataat ttttaaaatt tttggaattt ttaaagtgat 48000
gtttttttgg atgtttatga ttgatagatt agttggtagt tttaggatgg tttttaggga 48060
agattaggta gaattataag gtttagtatt attttgtaat ttttaggtag gggagagggg 48120
ttgaaggtta agtagattat tagtggttaa tgattgaatt aattatgttt ttgtaatgag 48180
gtttttgtga atatttagaa ggatggggtt ttgggagttt ttggatggat gagtatgtgg 48240
aggtttttgg agggtggagt gtttggggag tatatggaag ttttgtgttt tttttttata 48300
ttttgtttta tatatttttt tttttgtatt ttttgtaata tttttataa taaattagta 48360
aattttatga gttttaggaa tatgtgtaaa agaaaaaaaa aagaataaat tatagttgtt 48420
gtagtaagtt aattttatta gtattggttt ttatttgttg attttatttt tggtaatgtt 48480
tttttttttt gggtttttgg gatttttttg gttttattat tttttttgta gtggttttgt 48540
tggttgttgt ttttaggtta gtttattttt atttggtttg tttttattag gggttgaggt 48600
ttttttgttt attttagttt tttggtttgt tttttgtttt gggtattttt ttattttgaa 48660
tggttagagt ttttgaagtt gtttgttgtg tgtttttgtt tggttttatg tattttgtat 48720
ggtgtttttt agggtttttt attggtaagg gtattggtat agattttggg gttgttttgg 48780
tttaagttta ttttgatttg gagtttgagg gtttttgagg gggttttgaa ggttagggta 48840
ggtaaggttg gtatgttttg ggtagtgggg gttgggtatg ggaggggatt gtgggtgggg 48900
agattttttt taattagtgg tttatgtttg gagattgatt tgggttttag gttatgtttt 48960
gaaattggga tagagagtgg tgtagatttt agtttggaga tttttttttgt ttgtggatgt 49020
ttttatattt ttatttgtgg agttattttt tttttttttt ttttaaatgg ggaaatttttg 49080
atttgaatat tgaataaata atatattttt gaaattagtg aagaggtatt ggttttgaaa 49140
ttttgttatg aaagtttgaa aatgtttttt ggattgattt gttggaatag atgggtgtga 49200
tggggttgtg gggttttagg tggtaggagg tgtttttgtg tttagggtat tttgatgttt 49260
agagtttttt tgtgtttagt gtgtttttgt atttagggta ttttttatgtt tagggtgttt 49320
ttgtgtttag tgtgtttttg tatttagggt atttttatgt ttagggtttt tttgtgttta 49380
gggtgttttt gtatttagag tatttttatg tttagggtgt ttttgtattt agggtgtttt 49440

tgtttttagg gtgttttgtg tttagggtgt ttttgtgttt agtgtgtttt tgtatttagg 49500
gtgtttttat gtttagggtg tttttatgtt taggtattgt tttttttat ttttttaatt 49560
ttttttattt aataggtgtt agttgtgaaa tgtttgttgt gtgtgttttt tggttgagtt 49620
tattttgtgt ttttaagttt ttgttagata taggtaggtt gttttagttt ttttggtaaa 49680
tgttttgttg ttgttgtttg atatggggtt ttattttgtt atttaggttg gagtgtagtg 49740
gtgttatttt gttttattgt aatttttgtt ttttaggttt aagtgatttt tttgttttag 49800
ttttttaaat agttgggatg ataggtgttt gttattatgt ttggttaatt tttgtatttt 49860
tagtagagat ggggttttat tatgttggtt aggttggttt tgatttttg attttaagtg 49920
atttatttag tttggttttt taaagtgttg ggattatagg tatgagttat tgtgtttggt 49980
ttgtttggtg aatgtttatt gaggtttatt atgtgtagtt ttttttttag gtgtttggtt 50040
tgtaatgagg agtaataaag atatagttgt tggttgtttg ttatttaata gttggaattg 50100
agaggtagtt tataaatgag taaatttata ggtggtttta gatgaggatt gtgaagaagt 50160
taagaatagg ttgttggggg tgtatatttg tgtgtgtggt gaggggatta atggaagttt 50220
tttttgagtg gtgatattga gttgaagttg taatgattag aaggatttag ttaggttaaa 50280
atttaaggga agagtaattt ggtaaaggaa atagtaggtg tgaaggtttg aggttggagt 50340
ttggggaagg tgggagtggt tggagtgtgt ttttagagat gggttatggt gatattaatg 50400
tgataggtag ggttaggttg tgttgggttt tggaggtttt ggtgagggtt tgatttttatt 50460
tttattatgg tgggatttta gtgttttgtg gttgttgaga atggttaggg gtaggtaggg 50520
aggtttgtta gggaggaggt tgttgtaggt tttgtagttg gagagggagg attaggattt 50580
ttttagagag gaagttggag agggaggatt tttttgattg ttggatggat ttatttgttt 50640
ggttgttttt gttagtattt agtagaggtt attggatgtt gataggtggt tggttttgtt 50700
tagttttgta ttgttttgtg ttgttttggt ggttgttggt tttaggagga gtgtttttgg 50760
tgtttgagtt tagtggtttt ggttgtggtt tggtatattt ggttgtgggt gaggttaagg 50820
tggtttggat tttgggaagg ttgggtattt ggtagtttta aggtaggagt tggggtgttg 50880
gaggaggaag tatgtttatt ttttgttttt ttttgttttg tgggatgttg agtttttggg 50940
tagggatatg gtgagttttg gtgtgatgta tttaatattt gttggtgttt ttatttttaa 51000
gtaggaattt tggtgtttgg tgtttgagga ttgttgttta ttgttttatt attttttagg 51060
ttttttttta gtttttgatt tggggtaagg tttagatatt tagaatattt ttggttggtt 51120
tttggagttg gggtgaggta gaattttttt tttatattta tagtattagt taaggttttg 51180
gggtttggat attattttg ggaggggtga gtaggataag tgaggttttt agatttgggg 51240
tggttttgga ttggttaggt aggtgggtgg gggttgtagt ttatgttttt gttttaggta 51300
atatagttga gttgtagttt ttgttttatt tttagttggg gtttttttgtg tttatttttt 51360
gttttttttt ttttttttgg aatggggggt ggatatttta agattagttt tttagttatt 51420
tttttttgttt tttgtttttt ttttttttggg atagattttt gtgattgttg ggtgtttttg 51480
atgtttttgtt tttttttgtgt tttgtgttgt gggtgagaga gggttttggg gggatttttt 51540
tttttttggag tatttgttgt tttgggtgtt tttggcggtg ggtttttatt ttatattgtg 51600
gagttggtgt aggaaggaag gtgttgggag ttagttttaa tttatgtgtt ggttgtggtt 51660
tttaaggtgt ggatggtagg aggtggtgag atagggattt attgattatg ggattgatat 51720
tttttttatgt tgatgtgggt tttgagttat aattttatta gtttttgagt tttggtgttg 51780
tttttggtga tggtgtggat ttagttaggt ttttgggtag ttttttttttgg atttttttttt 51840
tgtttttttt tgggtagtgt ttttgtttat ttggaaagga tgggtaggat ttggagataa 51900
tagggattta aagaagttgg gagtggggt atttgtgtag tttattttgt agaatagata 51960
atttggttaa agtttaaagt ttgttgttag gagttttgtt tgtttttttag agagtatatg 52020
gtttgattat tatggggatt gggggagagg tataggtatg tttgtttggt agtagaaggg 52080
aattttgggt ttgggttttt ttattttgtg gtgttgtttt tttttagagt tgtattgttt 52140
tttttattgt ttttgtttgg ttttagtgtt tatgtggttt gtggttttta gtttgggttt 52200
ggtttttttgt aatttatttt gtttagtttt atatagtttg tttttttagt tgggaattgg 52260
agtttatttg gagttttagg tagtagattt gatgtagtga tgtagggttg ttagttatgt 52320
tagtaggttt tgttgtttat agagggatgg gtattgttgt tgataataat taaagaatta 52380
tggttttagg ttgggtatag tggtttatat ttgtaatttt agaattttgg gaggttgagg 52440
taggagaatt aattgaattt aggagtttta gattaggttg ggtaatatag taaggttttg 52500
tttttataaa aaataaaaaa taataataat aataaaaatt atagttttag aatgattgat 52560
tttagtatgt ttataaagtt aagggttttt agtgttttgg gtttatgtta ttgtgttaga 52620
gggagagagg aagaggttgg ggtgatgggt attggggttt atgtttttttt tggaatgggt 52680
ggttttggat tgtgtttgtg tgatatgttt gttgagtggg ggtggtgttt attgaggatt 52740
ggatgttgag gtgtgggtgg ggggaggtta tgaagtattg ggtatttttt gtgtattagt 52800
gttttgagat tgtagagagg ggatttgttt agtttgggta gggaggtggg tggtagtgag 52860
gaaggggaga ggaatttggg tgtagaaagt ttttttttta gtaggtggtg ttgtaagttt 52920

ttgagagttt agtttttgtg gggtttgtat ttttattgaa tttgtgggta gaggttgttt 52980
gggtgtagtt tttgttaatt gtgattgttt attgtgggtg aggggaagtg gagtgtagag 53040
aatgtattgg agggtagatg attagttttt gtataggtat gggggtgttt ttttttttgt 53100
ataggtatgg ggggtttgag tttttggggt ttttgtttta attgggtggg gaaagggagg 53160
gttggaagtt gagttagggg gaaaggggggt ggttttttgt tatttttttt attttatttt 53220
ttttattttt ggttgatagg ttttaatttt ttttatattt ttttaatttt ttgttgtaaa 53280
attttttaaa tatagaaaag atgaaagagg gttgagtgtg gtggtttatg attgtaattt 53340
tagtattttg ggaggttgag gtaggaggat tatttgagtt taggagttta agattagttt 53400
gggtaatata gtgagatttt attttataa aggatataaa aaattagtta ggtatggtgg 53460
tgtaggtttg gagttttagt tatttggaag gttgaggtga gaggattgtt tgaatttggg 53520
aggttgaggt tgtagtgagt tgggattgtg ttaatgtatt ttagtttggg tgatagaata 53580
aaattttgtt ttaaaaataa aataaaataa aattataaaa aagaaattat gaaagatttt 53640
aaagtaaata tttatgtatt tattatttag attttatagt aattatttg ttgtttgttt 53700
tattataaat ttttttggtt attagtattt atgttggttt tgatgtattt aaaataagtt 53760
gtagaaatta gtatataata ttttgttaat attttagttt gtattttatt aattagagtt 53820
tggtatttgt ttgtgattat ttttgttttt ttttgaagta aaatgtatat attttgagat 53880
gtataggtgt gaaatgtttt gtttttttgaa ttttaataaa ggtatttatg tgtattttag 53940
tttgattaga atatagaata ttattattat tttagtaagt tttttatgtt tttttaggtg 54000
tattatagtt tttatttttt ttagggtaat tataattgtg tttttttttt attatgaaat 54060
aattttgtta gtttgagtgt tttttagagt tatttatgtt gttgtaggtg ttagtgattt 54120
gttttgtttt attgttgagt aatgtttatt gtgtgaatat tttgtgggtt tttagttatt 54180
tttttgttga aagattttttg gttgttttta tttggaggtt tttatgagta aatttgttat 54240
gttggttttt tttgtataag tttttttgtg tatatgggtg tttatttatt ttgtagaaat 54300
atttagagta ggtatagggt aggtatatat ttagttttat aagaaattgt tagattttttt 54360
tttaaagtgg tttttttatt ttatattttt attagtaatg gatatgagtt ttagttgttt 54420
tatattttgg ttaatatttg gtgttgttat tttgtgaaat tttagttatt ttggtgggtg 54480
tgtagtggta ttatttttatt atggttttaa tttgttttgg tttgatgatt aatgtggttg 54540
agtatatttt tatgattggt tttttatata tttttttttt gtgaaatgtt tttttaaatt 54600
tgttgtttat ttttaaaattg ggttgtttga tttttttttt tggatattga ttttttgtta 54660
gatatgtgtt ttataaatat tttattttag tttgtggttt gtttgtttat tttttttttt 54720
tttttaagaa taaggttttg ttgtattgtt taagtaggtt tgaaattttt gggtttaagt 54780
tgttttttttg tttttgtttt tttaagagtt ggttttatag gtatgagtta ttatgtttgg 54840
tttgtttatt tttttagtgg tattttttaa taagtaaaag tgtgttattt taattttttat 54900
gaagtttaat ttaataaatt tttttttttg tggttttttt tttgtgtttt ttgtaagaaa 54960
tttttgttta ttttgttaaa ttgtaaagat atttatgttt tttttttagaa ggtttagagt 55020
tatatagttt ttatgaatta attttgtat atggtgtgag gtagagggtt agggtttatt 55080
ttgttgttgt tttatgtgtt tatttagttg ttttaaattt tattttttttt taaagttttt 55140
atttgtatt tttttggtga tttatgaggt tgtataattt tttaagattt ttgttggtta 55200
tttatgtatt ttttttgta aaattatttg tttatgtttt attttttttt aaaattatgt 55260
tgttttaaga ttgagtgatt tttgaagttt ttattttata atgtttgaaa gaatgaatat 55320
tttgtttttt agttataggt ttttgttttt tgttttgtag ttgtatagtg gggtaagatt 55380
tgggttttgt tttgttgttg ttttgttgtt tttttatttg attgtttttt tatgggtttt 55440
atttaaaaaa aaaattggtt gggtatagtg gtttatgttt gtaattttag tatttggga 55500
ggttgaggtg ggtagattat gaggttagga gattaagatt attttggtta atatggtgaa 55560
attttatttt tattaaaaat ataaaaaatt agttgggtgt ggtggtgggt gtttgtagtt 55620
ttagataatt gggaggttga ggtaggaaaa tggtgtgaat ttgggaggtg gagtttgtag 55680
tgagttgaga ttgtattatt gtattttagt ttaggtgata gagtaagatt ttgttttaaa 55740
aaaaaaaaaa aaaaaaaatg ttgttttata tgttttttat atattttgga tataagtttt 55800
ttattagata tatgtattat aagtttttttt ttttatattg tttttttttt tttggttttt 55860
ttaatgatgt tatttaatga gtaagagtat tttgttttta taaagtttaa tttattaatt 55920
ttttttagtt ttgatttatg atttttgtga tttatggagg aaatttttgt ttatattaag 55980
gttaaaagaa gttttttttat tttttagaaa ttttatagtt gtatatttta tgattttttt 56040
tattttttaa ttttttaaat ttttatattt tttaatttat tatagttgtt aggatgttgt 56100
atgatttatt ttaaattagt ttttgtattt ggtttgaggt tagggttaat ttttgttttt 56160
ttatatttat atttagtttt agtattgttt gttgaaaaga tatattttat tttattgaat 56220
tatttgatat ttttgttaaa aattattgat tgtatatgtg gttttatatt agtatatgta 56280
tgtatatata tgtaggtttt gtgttgattg tatgtgtggt tttatattag tatatgtatg 56340
tatatatata ttggttttat gttgattgta tatgtggttt tgtattagta tatatatatt 56400

agtttatat tgattgtata tgtggttttg tattagtata tgtatatata tatatgtagg 56460
ttttatgttg attgtatatg tggttttata ttagtatatg tatatttata tatgtaggtt 56520
ttgtgttgat tgtatatgtg gttttgtatt agtatatgta tatatatata tataggtttt 56580
gtgttgattg tatatgtggt tttgtattag tatatgtata tatatatatg taggttttat 56640
gttgattgta tatgtggttt tgtattagta tatgtatata tatatatata ggttttgtgt 56700
tgattgtata tgtggttttg tattagtata tgtatatata tatatgtagg ttttatgttg 56760
attgtatatg tggttttgta ttagtatatg tatatatata tatgtaggtt ttatgttgat 56820
tgtatatgta gttttatatt agtatatgta tatatatata tgtaggtttt atgttgattg 56880
tatatgtagt tttatattag tatatgtata tatatatatg taggttttat gttgattgta 56940
tatgtggttt tgtattagta tatgtatata tatatatgta ggttttatgt tgattgtata 57000
tgtggttttg tattagtata tgtatatata tatatgtagg ttttatgttg attgtatatg 57060
tggttttgta ttagtatatg tatatatata tatgtaggtt ttatgttgat tgtatatgtg 57120
gttttgtatt agtatatgta tatatatata tgtaggtttt atgttgattg tatatatata 57180
tgtggttttg tattagtata tgtatatata tatatgtagg ttttgtgttg attgtatatg 57240
tggttttgta ttagtatatg tatatatata tatgtaggtt ttatgttgat tgtatatgtg 57300
gttttatatt agtatatgta ttgtatatat gtaggtttta tgttgattgt atatgtggtt 57360
ttatattagt atatgtattg tatatatgta ggttttgtgt tgagatatta ttgtgttttt 57420
ttgatatttt gtttattttt ttgttgatgt tatattttga ttaatgtggt tttatagtaa 57480
gttttaatat aaagtttttt aaatatttaa aatattattt tgaatatttt aggtttttttg 57540
agttttttta taaatttaag aagtagtttg ttgatgttta taaaaatgta gtgtgaattt 57600
tggttgagat tgtgttgaat ttgtagatta attaaggaag aattggtatt ttaataatat 57660
tgagtttttt gtatagatgg tatattttttg ttttaaaggt tttttgtat ttttttttatt 57720
ttttattga aaaaaaattt ttatagaatt atattgtttt gtagtttttta agaaaaagat 57780
tttgtatgtt atttgttaaa tttattttta agaattttat ttttggtatt atttaagtt 57840
gaaatagatt ttaaatttta tttttaatta tttgttgtta gtatgtaaaa atataataga 57900
tttttgtatg taaattttat attttatgat ttttttaaa gttatttatt attttggtaa 57960
tggttttgta ggttttttag gatttaaata tatagtttta ttttttttttg atttgatgt 58020
tttgtttttt tttttttgttt tattgtattg gttagagttg ttagtttagt attgaatagt 58080
agtgattagt ggatattttt gtttgtgtta aatttataat gttaatttta gatttgttat 58140
atatgatttt tgttagattg aggaaatttt tttttatttt taatttgttg aaattttta 58200
atatgaatgg gtatgatttt agtaaatgtt ttttttgtat ttatggaaat gattattgtt 58260
ttgtttttta tttgtttaat aggataaatt ataatgattg atttttgttt tttgtttttt 58320
tttttttttt ttttaaagaa atagagtttt atttttgttt aggttgtagt atagtggtgt 58380
tattgtagtt tattgtagtt tgaattttttg gatttaagta atttttttat tttagttttt 58440
taagtagtag ggattatagg tatatgttat tatgttaagt taatttttaa tttttaaaat 58500
tttttgtata gatggggttt tattgtgttg tttaggttgg tttgtaattt ttgagtttaa 58560
gtaatatttt tatttttgtt ttttaaagtg ttgagattat aggtgtgagt tattgtgtag 58620
gtttgatttt ttaatattga attaatttttg tatttttgga agaagtttaa tttgtttatg 58680
agatattatt tttttatat attgttaaat ttgatttgtt tagtttttaa ggatttttgt 58740
gtgttgattt atgagggata ttggtgtggt atttttttttg tttgttagtt tgttttggta 58800
atgtttttgt tagttttatt ttggttttat aaaattagtt tggaagtgtt ttttttttt 58860
tgtttttttgt gtttttataa ggttgatgtt gttttttttt taaatgtttg atagaattta 58920
ttaagaaaat tgtttggatt tagaattttt tattaggaaa ggtgattgtt tgttttttaat 58980
tataaattta atttaatgta tagaaagagt tatttaggtt tttttatttt tttttgtttt 59040
atttttggta agttgtattt tgaaggaatt tgttttttttt aggtaagttg ttgaatttat 59100
ttatttaaag ttgtttgtaa tataagtata tttagggttt gtttttagat tttggtaata 59160
aagtaaatat tgtaataaag tatgttatat aaattttttg gttttttagt atatataaaa 59220
gttatgttta ggttaggtat ggtggtttat gtttgtaatt ttagtttttt gggaggttaa 59280
ggtgggtgga ttatttgagg ttaggagttt gagaatagtt tggttaatat ggtgaaattt 59340
tatttttatt agaaatataa aaattagttg ggtgtggtgg tatatgtttg tagttttagt 59400
tgtttgggag gttgaggtgg gagaattgtt tgaatttggg aggtggaggt tgtggtgagt 59460
tgagattata ttattgtatt ttagattggg tgatagggtg agattttatt ttaaaaaaaa 59520
aagaaaaaag ttatgtttat attatattat agtttattaa agtgtataag attattatgt 59580
tttttttgtta aaaaatgtta ataattattt gagtttttag taagttataa ttttttttt 59640
ggtagaaggt ttgttttaat gttgatggtt gttgattagg tggtggttgt tgaaagttga 59700
aggtagttgt ggtagtagtt tttttgtttt ttttttttt ttttttttt ttgagataga 59760
gttttgtttt attgtttagg ttggagtgta gtggtatgat tttagtttat tgtaattttt 59820
gtttttttggg tttaagtaat tttttttgttt tagtttttttg agtagttggg attataggtg 59880

```
tttgttatta tgtttggtta atttttgtat ttttagtaga gatagggttt tattttgttg   59940
gttaggttgg ttttaaattt ttgattttag gtgatttatt tgttttggtt ttttaaagtg   60000
ttgggattat aggtgtgagt tattgtattt ggttggtagt tttttaattg aagtttttga   60060
tatgggttga tttttttttt tatgaaagat ttttttgtgg tatgtattat gtttgatagt   60120
atttatttta taatagagtt ttttttaaaa ttagagttaa ttttttttaat tttggttatg   60180
gattgattaa ataagttgat gtaatatttt aaattttttg ttgttatttt aataatgttt   60240
atagtatttt tgtttggagt agattttatt tttaggaatt attttgtttg tttatttata   60300
agaagtaatt ttttatttgt ttaagtttga ttatgggatt gtagtaattt agttttattt   60360
ttaggtttta tttttaattt tagtttttttg ttattttttat tatatttgta gtgatttttt   60420
ttgttgaagt tttaaattgt ttatttatga gggttggaat taatttttttt taaatttttg   60480
ttaatgttga tattttaatt ttttttttgtg aattataagt gtttttaatg gtatttagaa   60540
tggggaattt tttttagaag gttttttaatt tattttgttt agatttatta gaggaattat   60600
taattatggt agatattgtt ttataaaata tattttttaa ataataagat ttgaaaatta   60660
aatttatttt tttttttatgg gttgtagaat gggtgttata ttagtaggta tgaaaataat   60720
atttattttt atatatattt ttgttagagt ttttgggtta taaggtgtat tgttaatgag   60780
taataatatt ttgaaaggat tttttttttt tgagtagtag gttttaatga tttttttttt   60840
ttttgatgg agttttgttg tgttatttag aatggaatgt ggtggtatga ttttggttta   60900
ttgtaatttt tgttttttttgg gtttaagtga tttttgtgtt ttagttttttt gagtagttgg   60960
gattataggt atatattatt atgtttggtt agtttttgta tttttttgtag aaatagggtt   61020
ttattatgtt ggttaggttg gttttgaatt tttgatttta ggtgatttat ttgttttagt   61080
tttttaaagt attgggatta taggtatgag ttattgtatt gggttttatt tgtttttttt   61140
ttgttgttgt tttttaattg agatagagtt ttgttttgtt gtttagattg gagtgtagta   61200
gtgttatttt agtttattgt agtttttgtt ttttaggttt aagtgatttt tttgttttag   61260
ttttttgagt agttgggatt gtaggtgtgt attattatgt ttggttaatt tttgtatttt   61320
taatagagat gaggttttat tattatgttg gttaggttgg ttttgaattt ttgaatttag   61380
gtgatttgtt tgttttggtt ttttaaaatg ttgggattat agttgtgagt tattgtgttt   61440
ggtttttttt tgttttttttg tttttttttta aattgagata gagttttttt ttgttgttta   61500
ggttggagta tagtggtatg attttggttt attgtaattt ttgtttttttg ggtttaagtg   61560
gtttttttgt tttagttttt taagtagttg gaattatagg tgtgtgatat tatgtttgaa   61620
taatttttgt atttttagta gagatgtggt tttattatgt tggttaggtt ggttttgaat   61680
ttttgatttt aagtgatttt tttgttttag tttttaaaag tgttgggatt ataggtgtga   61740
gttgttgtat taggttttaa tgggttttta atatttagta aattatgttg taaatagatg   61800
tgttgttaag tagggtttgt tattttattt ttgaagtata gatagaatag atttggtaga   61860
atttttaagg gttttaggat ttttggaatg gttaatgagt attggtttta atttatggtt   61920
aatagttgtg ttagtttttt atgagagtta gtttgttttt tgaagtttta aagataggta   61980
ttgatttttt tttttagtt atgaaaattt tagatgatat ttttttttag tagaaggtgg   62040
gatatttatg ttgaaatttt gttgtttagt gtagttattt ttattagtgt tttgagttag   62100
atttttgggg taatttgttg ttgtttttttt attagtattt gttgttttat tttgtatttt   62160
tatattatag agatggtttt ttttttaaa ttttatgatt tgatttttgt tagttttaaa   62220
tttttttttt ttagtttttt tatttttttt agtttttata ttattgaaga gagttagggt   62280
tttgttttgg attaggtttt tgtttaaggg aatgttgtgg ttgatttgat tttttattta   62340
gattatttaa atttttttta tattagtatt aaggttgttt tgtttttttt tttttatgt   62400
gtgtattgga attgtatttt taattttttt taataatttt tttttggtat ttataatttg   62460
gttagttatt tggtataaga gattttattt ttgtttgttt tggtttttga tatgtttttt   62520
ttatttagtt taattatttt tagtttttga tttaaagtga gagatgggtg attttttttt   62580
ttatttgaat atttagaggt tattgtaggg ttattaattg gtttaatttt aatattgttg   62640
agttttaggg aatagggatg tttgagtaga gggagagaga gtggggaata gttagttagt   62700
ggagtagtta gaatatataa tgtttattga ataagtttat tgttttattt ggatgtggtt   62760
tgttatgttt taaaaataat tgtaataatg gtattgaaga ttattgatta ttgggtgtgg   62820
tggtttatgt ttgtaatttt agtattttgg gaggttgagg taggtggatt atttgaggtt   62880
aggagtttga gattagtttg gttaatatgg tgaaattttg tttttattaa aaatattaaa   62940
attagttagg tgtggtggtg ggtgtttgta gttttagtta ttggggaagt tggggtagaa   63000
gagttgtttg aatttgggag gtggaggttg tagtgagttg agattatatt attgtatttt   63060
attgtatttt agtttgggta atagagtgag attttatttt aaaaaaaaaa aaaaagggtt   63120
gggtgtagtg gtttatgttt gtaattttag tattttggga ggttgaggtg ggtagattat   63180
gaggttaggg gattgagatt attttggtta atatgatgaa attttgtttt tattaaagat   63240
atgaaaaaaa tagttgggtg tggtggtggg tgtttgtagt tttagttatt tgggaggttg   63300
aggtaggaga atggtgtgaa tttgggaggt ggagtttgta gtgagttgag attgtgttgt   63360
```

```
tgtattttag atagagtaag attttgtttt aaaaaaaaaa aaataaagaa agaaaaaaga 63420
aaaaagagat tatagattat tatagtagat ataataataa tgaaaaagtt tgaattattt 63480
taagaattat taaattttta tatagagata tagtgttaat atgttgttgg aaaaatggtg 63540
ttagtagatt tatttgatgt agggttgtta tgaattttta atttgtaaaa agtgtagtat 63600
ttgtgaagta taatgaaatg aggtgtgttt gtgtttttttg ttattgtaat gttggtagga 63660
tttgtaagga aaatatttta ttttttttat tgtgatttta tgtttgttat ttttttttta 63720
ttagtagttt ggttaggagg ttattaattt agattttttt aaagaattag tattggattt 63780
ttttttttg gtgtttattt ttttgagttt ttttgttatt tttattattt ttttttttgt 63840
atttgtttta gtgtattagt tagggtttag ttaggaaata aaaattatat tggtaatttg 63900
aatagtaatt tgtaatataa agtgtttttta attgtaagag gtagttaatt attaaaggga 63960
ttataaaggg gtgatattgt gatataagaa agatgtattt gggttgggtt tggtgtagtg 64020
gtttatgttt gtaattttag tattttggga ggttgaggta ggtggatttt ttgagtttag 64080
gagtttgaga ttagtttggg taatatggtg aaattttatg tttattaaaa atataaaaaa 64140
ttattttggt gtggtggtgt gtgtttgtgg ttttagttat ttgggaggtt gaggtgagag 64200
gatagtttga gtttgggaag gtagatgttg tggtgagttg agattgtatt attgtatttt 64260
agtttggatg atagagggag attttgtttt aaaaattaat gtatttgttt gtttgtttgt 64320
ttatttattt atttatttat ttatttattt atttatttag tttttgtttt ttaattttgt 64380
ttggtatgta gtttttaaaa tttttgaaat ttttaaagtg atgggtgttt ttttgtttgt 64440
taaggaggtg attggtggtt ggaggttttt ggatagtttg gggataaggg ttggttgtta 64500
ggggatttag ttttgtgatt agaaggttag aatttgtagt tttttttatt tattttttgt 64560
gaggtaagag gggatgaagg ttgagttgat tattaatggt taaagatttt atgttatgtt 64620
tatgtagtga agtttttttg aaaatttaaa aggatgaggt ttgtagagtt ttaggttgtt 64680
gaatatttgt aggtgttggg agggtagtgt atttttttgt ggaagtttta tgttgttttt 64740
tttatatttt ttttatgtat tttttttagt tggttgttta tttgtatttt ttgaaatatt 64800
atttgtaata agttagtaat tttaagtaaa ttgatttttt gggttttgtg agttatttaa 64860
gtaaattatt gaatttgaga agggagttgt gaagatttta aatttgtagt taagttagat 64920
agaagttatg ggtaatttgg tgttttatta tttgtgattg atttttgaag ttgagggtag 64980
ttttgtgaga tggaattttt aatttatggg atttgtatta attttggttg gtgttagaat 65040
tgaattgaat tgtaggatat ttagttgggg attgttgagt attggagaat tgtttggggg 65100
ttggtgggag gaaattatat atttggtgtt agagttgaag tattgagtgt tgtgaatgag 65160
agtggagaga tagagtttgt tttgttttat gtaagaggat tttaaggagt atagaaagag 65220
taaatatgag aagtagttat tttagaagaa aatataggag gaaagttttg tgatattgga 65280
gttggtaatg ttttttttgga tgtgatattg aaaatataat aaaagaaata aatgataaat 65340
tggattttat taaaatttaa aatttttgtg tattaaggaa tattattaat ggtgtgaaaa 65400
ggaaatttat agaatggaag aaagtatttg aaaattgtat atttgataaa ggattagtat 65460
ttagaatata taaagaattt ttataattaa gttataaaat aaaaaaaaat aatttagtga 65520
atagatattt ttttatagaa gatatattaa tggttaagaa gtataggaag agatgtgtag 65580
tgttattaat tattagggaa atgaaaatta aagttataat aaaatatgat tttatatttt 65640
ttaaggtggt tgttagttaa aatatggata atagtaagtg ttggtaagga tatggagaaa 65700
ttggagtttt gtgtattgtt ggtgggaatg taaaatggtg tagttgttgt ggaaaagata 65760
taataattgt ttaaaaaatt aaatttagaa tgattataat atttagtgat tttattttta 65820
gatgtatatt ttaaagaatt gatagtaggg atttgaatag gtgtgttata tttttgtttg 65880
tagtagtatt tttttataa ttaaaagatg taggtaattt aggtgtttat tagtggatga 65940
atgaatagat aaaatgtggt ttatttgtat agtggaatat tattttgttt taaaaaggaa 66000
ggagaggtta ggtgtggtgg tttatgtttg taattttagt gttttggaag gttgaggtgg 66060
gtagattatt tgaggttagg agtttgagat tagtttggtt agtatggtga aattttgttt 66120
ttgttaaaaa tataaaaatt agttaggtat gggggtatgt gtttgtaatt ttagttatta 66180
gggaggttga ggtaggagaa ttgtttgaat tagggaggtg gaggttgtag tgagttgaga 66240
ttatgttatt gtattttagt ttgggtgata gagtaagatt gttttaaaaa aaaaaaaaaa 66300
aaaaaaaaaa aaggagattt tgatatatgt tatagtatag atgagttttg aagatatgtt 66360
aagtgataga agttagatat gaaaagataa atattgtatg attttatttt tatgaggttt 66420
ttagagtagt taaatttaga gagatagaaa gtgggatgga ggttgttaga ggttgggggga 66480
agggagaatg aatgggaagt tggtgtttaa tggggataga attgtagttt gggaagatga 66540
gatggatggt ggtgatggtt atataatagt gtgaatgtgt tttatgttat taaattgtat 66600
atttgaaaat ggttaaaatg gtaaattttt tgtgatggat attttaaggt atgtatgtaa 66660
atatatagaa gtagttatta ttattggggt tgatggaaaa gaaattaaga atttggagtg 66720
tttttttatat tttgttatgt tttatgttta ggttttgttg gagagtgtgt gattgttata 66780
gaaatttgta gtttattggt aataaaaagg tttttgttag aaggtgtaga aagagtggat 66840
```

ttgtaggatt agtttattgg gtagttgaga aatttattag agggtatggg tgggtaggag 66900
ttgggtgttt gagaagtttg ttgaggtttt attgggttag agttggggtg ttggagaaat 66960
ttagtataga gtaggtttta ttgggtatat gttattggta gttgtgtttt tagaggaaaa 67020
agagattttg gaattaggaa gagaagttgt tattttagta aggtttggta gtttttata 67080
gtgttgtttt ttgtggatag agtttgatat gattgtattt ggttaaggag aaagatttat 67140
aaggttttgg tttagtatta tggagtgggg tttggtagga tgtatgtaga gttaagaggt 67200
aatatatggg taattggtat attttttag atttttgtt tttttgttg ttttgttttg 67260
ttttgttttg ttttgttttg ttttgagaga gtattttgtt ttgttgttta ggttggagtg 67320
tagtggtatg atttaggttt attgtaattt ttatttttg ggtttaagtt attttttgt 67380
tttagttttt taagtagttg gagatgttta ttatgtttag ttaattttg tatttttagt 67440
agagataggg ttttattatg ttggttaggt tggttttgaa tttttgattt taggtgattt 67500
atttgttttg gttttttaaa gtgttgggat tataggtgtg agttatattg tatttggttt 67560
attttttag atttttaaag tagattttta ggtttttttt tttttttttt tttttttttt 67620
tttattttt ttttaatata agtatttata gttttttttt aagtattagt tttagtttat 67680
gtattttagt atgttgtgtt tttattatta ttttatttga aatattgtaa aattttttt 67740
gtgattttt ttttgattta tgggttattt agaagtatgt tgtttaattt gtaaatagtt 67800
aaggtttttt tgagtatttt agagttattg atttttaatt taattttatt ttggttagat 67860
aagtatattt tgtatgtttt taaaatttga gatttatggt ttagaatgtg tattttggtg 67920
aatgtattgt ttttatttga gaaaaaaata tgtatatata ttttagattt tttgagtata 67980
gagttttata attgttgatt aggttaattg ttagttatta gtgtttttta gattatttga 68040
tgtttgtttt ttagatgttg agagaatggt gtttgttatg gtttatttgt ttgtgttttt 68100
taaaatttat atgttggagt ttaattttg gtgtagtggt gttgggaatt ggggtattag 68160
ggaagtattg gtttatgagg gtagagttat tgtgaatggg attagttttt tttgttatgt 68220
ggggatatag taggaaaagg atatttttga agtagggagt tagtttttat tagatattga 68280
gtattttgat tttggatttt ttagtttta gaattgtgag tattataatt tgtttataaa 68340
ttatttagtt taaggtattt tgttttaata ttttgaatga attgaggtgg tgttaaaatt 68400
tttaattatg tatttaggtg tagtggttta tatttgtaat tttagtattt taggaggtta 68460
aggtgggtgg attatttgag gttaggagtt tgagattagt ttggataata tggtgaaatt 68520
ttgtttttat taaaaatata aaaattagtt gggtgtggtg gtaggttttt gtaattttag 68580
ttatttggga ggttgaggta ggagaattgt ttgaatttag gaggtggagg ttgtagtgag 68640
ttgagattgt attattgtat tttagtttgg gtaataagag tgaaattttg ttttaaaaaa 68700
aaaaaaaaag agtttttatt tatgattgtg gaattgttta tttttttttt taattttttt 68760
aggttttata ttatgtattt tgaagttttt ttggttaggt gtatatatat ttatgattat 68820
aatggtgttt tgatgaattg attatttaat tatgatgaat gtatttttt attttgtaat 68880
aattttattt tggatttaat tttattgata tttatgtagt tttttagtt tttttaaata 68940
tgttgtttgg gatgtataag aaggttggag aggtttatgg agaaatttat gatggtttga 69000
tttatagttt tttaatttta ttatggtgtg aatgtggtat gtatatagta gaaattgtat 69060
tttaagtatg tgtgaaatta ttgtgttttt tatttttagt atagtattta ataatttata 69120
tgagatagtt aatattttat tataaaatag gttttgtgtt tgttgatttt gtttaattgt 69180
aggttgaagt aagtgtttta attatgttaa ggtaggttag gttaagttag aatgtttggt 69240
aagttagatg tattaagtgt attttgaga tgatatttat tttttattta tgatgggttt 69300
gttaagatat aattttatta ttatttaagt agtattagta tgttttttt taaatagaaa 69360
atttaattt gtgtttatat tgaaatagta tttttgtag atagtatata gttgagtttt 69420
gttattttat ttattttgat agtttttgtt ttttaattgg aatatttagg ttattttgt 69480
ttaatatagt tatagatatg attgggttta gagttattat tttgtttttt gatatttatt 69540
tttttattt tgtttttttg gtttttttta tgttaattaa gtatttttta gaattttatt 69600
ttaatttatg tttttttttt tttttttttt ttaagataga gttttatttt gttgtttagg 69660
ttggagtgta atagtatgat tttggtttat tgtaattttt gttttttggg tttaagtgat 69720
ttttttgttt tagttttttt agtatattgg gattatagat gtttgttatt atggttggtt 69780
aatttttgta tttttagtaa agatggggtt ttattatgtt ggttaggttg gttttgaatt 69840
tttgatttta ggtgatttgt ttattttggt tttttaaagt gttgggatta taagtgtgag 69900
ttattgagtt tggtttgttt tgattttta attgtatttt tttgtattgt tattttagta 69960
gttattttat agattttatt gtatattttt agttttttat agtttattta gaattaatat 70020
tgttttttt tatggaaaaa aagtgtaaga attaggtaat tttatgggtt tatttattat 70080
tttttatttt gtatgtgatg gttgttagat atttattata tttatatata ttgtaaatta 70140
gataatataa taaggaaatt tttgttttaa atagttttat gtattttaaa gaaattaaaa 70200
gtaaaataga atttttttta tttgtttatg tatttgttat attttgtgtt ttttattttt 70260
ttttgaagag ttgagttttt atttggtttt atttttatg atttggaga atttgtttta 70320

tggtttttgt agtgtaggtt tgtagatagt taattttttt ttatttttat ttatttgtaa 70380
atttttattt tgttgttatt tttttttttt ttatttttat ttttattttt gagatagagt 70440
tttattttgt tttttatag gttatagtgt aatggtgtga ttttaaaatt tttgtttaat 70500
tttaatttat taatttttt tttttgggtt taaatgattt ttttgtttta gttttttaag 70560
tagttgggat tataggtatg tgttattata tttagttaat ttttgtattt ttagtagaga 70620
tgggatttta ttatgatggt taggttggtt ttgaattttt gattttaagt gatttgtttg 70680
ttttggtttt ttaaaatgtt gggattatag gtgtgagtta ttgtgtttag tttgttgtta 70740
tttttgaatt ataattttat tggttataga attttgagtt gttagttttt ttttttagga 70800
ttttaaaata tagggtttta gtgtttttgg tttttgttgt ttttgttaag gaattagtat 70860
ttatttagtt atttttttgt agattatatg ttgttttttt ttggatgttt ttaagatttt 70920
ttatttaagt ttgattgttt agtatttatt tttgggtaaa aaagttttta gaaattagaa 70980
atttatttag ttttattttt ttttgttagg tgtagatttt attttagttt ttatttattt 71040
gagatatttt atattgattt taggttgttg ttattttgtt tttttaaata ttttgtttag 71100
agttataatg ttttttgtag aattgattag atagtagtta ttttttgtga ttaggagttt 71160
tgattttttta gatttgttga tgggtttata aagtttttgt ttgtggtttt tagttggtag 71220
tggttttttt gttttgaagt tgttatgagt gtgtaaaatt tataggttta atttttttta 71280
ttagtatagt ttagaaaagt ggaatttttg gtttgggtgg ttatggtgtt ttagattatt 71340
ggttagatta gggtggtgat tgttagttgg ttgaatgtgg ggtttttagt ttagtatttt 71400
tggattttat tttaggagtt atggttgttt tagggttagg ggtttggttt tagtttttgt 71460
tttttttttt tttttttaa gttgtttagt attaggtttt aattagtatt ttttgtagat 71520
agggtgggtt ggttttttat tagaggggta gggttgggtg ggaagtttgt tgagtttttt 71580
tttttgtgta gtttatagtt ttggggttag ttttagaggg gtgtagaaag gagtttttaa 71640
aggtagaaat ttattttttt ttttgttttt gtgtttttag ggataaagga gaggtggttt 71700
tagttggggg gttattagtg gtatgtagga ggtttatatg attattgttt gttggggttt 71760
tttttttttt gtgagagaga tgagagagag gagagagagt gtgagttagg gtagtgtttt 71820
tttttagtaa tgtagatttt tgtatgtgtt ttttaggaga aattttgtta tttgttagag 71880
ttttagattt ggaagaagtt ttgttttttt tttttttttt agtgtagtgt gtgtgtgtgt 71940
gtgtgtgtgt gtgtttttat atgtattata ttttttttata gaggaaataa tttttgttta 72000
agtgtaaagg ttgggattgg agtaggtagt atgtgggtta aaggagtaga gaggaggaaa 72060
tgggggtggg ggtgagggtg ggaataggag gtgggtagat gttatgaggt atgtttttta 72120
taattagaag ggttggtgga gatgattaga atgttttaga tagatttttt attaaatttt 72180
taaaatttaa ttttgtttta tgaagtttga ggattttgta gagtgaaaga taaggaaggg 72240
tttttttgta ggggggtgtg tgtgtttgtg tgtgtttgtg tgttgtgtgt ttttatgtgt 72300
gtgtatgtgt ggatttgtgt ggtgtgtgtt tgtatatgtg tgtatgtgtg gatttgtgtg 72360
gtgtgtgttg agtgtgtata tgtgtgtgtt tgtgtgttgt gtatgtattt gtatatgtgt 72420
atatagattt tattttttttt tttttttggtt tattaagtgt tagaaagaat ttttgtgagt 72480
ttttttattt tttttaaggt tgtatatgga gggatttgtg taggtatatg tgtgtgtgta 72540
tataatatat atatatatat atatatatat atatatatat atatatgagt taggggtgag 72600
tatttttgtg gattgtattt aggtttggta ggaaattggg ttttgggaaa ttgtaggttt 72660
tatttttggt agaaaattat gtttttgttg ttgtttgttg aagggaatat taagtggggt 72720
tggggatagt ttatgttgag aagagtaaat tttgtttatg gtattgggga tattgagtgg 72780
tttttgtttt gggttttgga gggtttggga gatatgtttg tagttttggg tattttttaa 72840
ggttatgggt ggttatattt aaggttaggt tttttttttgt gtgagttgtg gttagtagga 72900
aggttgattt tagaggtggt tttagggttt ttggaggttt tgtttttgag atatgggtta 72960
aggttagaga gttgagaagg ttgttgttga ggattttggt tggtgtttag tttagtggtt 73020
ttttaggtgg gtttttgtgta ggggttgatg aagttagttg ggaaatttgt gaaataaagt 73080
ataggttttt taggtttgtt tagagttttg gtattagatg aggtgtagag aagggaattg 73140
agttgttaag tgtgaatagg agagaggagt tttaggatta tataggggtt ttgatatggt 73200
aggtgtttgg ttagtagtgg tgttgttttt attgttggat gtgggaaaat gtattgatat 73260
ttgttgatta gagttggggt gagtatttgg gtgattttgt agatgagata aaataagggt 73320
tgttgtgggg gaggggtttt tgttgggtta ttttgttgag gttttgtgtt attgttttgt 73380
ggttgttata ataaaggatt ataaatgagg tgatttaaaa tatttgaaat tggttatgtg 73440
tggtggttta tgtttgtaat tttagtattt tgggaggttg aggtggttgg attatgaggt 73500
taggagattg agattatttt ggttaatatg gtgaaatttt gtttttatta aaaatataaa 73560
aaaaaaaaaa aaattagttg ggtgtggtgg tgggtgtttg tagttttagt tgtttaggag 73620
gttgaggtag gagaatggta tgaattttgg aggtggaggt tgtagtgagt tgagattgtg 73680
ttattgtatt ttagtttggg tgatagagta agattttgtt ttaaaaataa aaataaaaat 73740
aaaaataaaa atatttgaaa tgtttttttt aaagtttttg gagttagaaa ttttagattg 73800

```
aggtgtttga aggtttattt tttttggggg ttttgaggga gattgtttta tgtttttttt 73860
tgagttttgt ggtggttagt aatttttggt atttttttggt ttgtagtttg gtttattttt 73920
tatttggttt ttattttgtg tttttttttg tgtttttgtt taaattgttt tttttgttgt 73980
ttttttaatt tatttttagg gataggtttt tattttgttt tttaggttgg agtgtagtgg 74040
tgtgattatg gtttattgta gttttagttt tttaggttta agtaattttt ttattttagt 74100
tttttaagta gttgggatat aggtgtatgt tattatgttt ggttaatttt ttaatttttt 74160
tgtagagatg agggtttagg ttggttttaa atttttgtgt ttaggtaatg agattttgtt 74220
ttttaaaata ttgggattag agatatgagt tgttgtgttt agtttatttt tgttttataa 74280
agatatttgt tattggattt agggtttatt ttgatttaat atgtttttaa ataatttaat 74340
tatattttta aagattttat gtttatataa agttatattt ataggtattg gggggttagg 74400
atttgtatat gtttttttggg ggaatataat ttaatttata atgggtttta tttatagaat 74460
gaatggtttt tttagggtgt gtgtaggtgg gtgaggggtt taggagaggg atttgtgtgg 74520
taagagggtt tgtgtttatg ttagagattt tagaaggaga ttggatgggg tggttgtatg 74580
gagtaagggg ttatagttat tgtttttaag tagttggtgg gttgtttggt agaaagggag 74640
tatttatttt gagttttttt aagatatgaa gttagtatta ggtatttgga aggtgttagt 74700
gagtagagtt tgattttttta tagaaagagg tgtttttttt ataatgttta tttgaggatg 74760
gtggaatagg ttattttata aggtagtgag tttttttatgt ttggaagtat ttaagtaggg 74820
gttatgtggg agttgtttag gagtgtatag aagggatttt agaggttttt gttaatatta 74880
ttttgtttg ttttatttta tgataatttg aattttagaa tgtaatgtta ttggggatat 74940
gtttattaga ggggttttta gtattaggtt ttatatatta tgtattattt ttagggtagg 75000
gaatgagagt aggttttatt tttttggtt gtttgtgagg attaggaggt ttgttgttga 75060
ggtagttttt tgtggaaata gtgttttagt tttgtaattt atttattgga gttttaatat 75120
tttaatgaaa tattgtgtta gatgggtttt gggtatgtgt gtgtagattg ttataagttt 75180
atgagaatgt atatatgtat attgtatgtg tgtgtatata tgtatgtata tatgtataag 75240
aatatgaaga tttgaggtag gtatagtttg tgagatagat tttttaggt tgaaagattt 75300
tttataaggg tttttagaat aaggttttag ttttggtagt ttttttttatt tttggtggga 75360
ggatttttat agagaagaag ggttaagaag tatagttttt gatgtttatg gtgtattaag 75420
ttttgtttta agtttgtgtt ttattttttta tttatttagt aaatttgttg tttttgtttt 75480
gtgttagatg ttgtttttag tattttataa atattgatgt atgtttgttt tatgataatt 75540
ttgtgaagta ggtagtgtta ttttgtttta tagatgagga aataggtgta gagggataaa 75600
gggatttgtt taaggtttgt ttttatttta tatgtatatg aatgtttatt tatgtggtag 75660
gtgttagttt ttttttgatg tgagttaggt tttgagttgg ttggggtttg ggttttattt 75720
tggttgtgtt ttgttgtggg tattaaggaa gtttttagta gttttatttt tttggtatga 75780
tttagtttta gggattggat ttttttaaat gtttttgttt agtttggagt ttttttttta 75840
gttgggggag ggttgaatga tttttttattt ttggaggttg ttggagtttt ttttgtttgt 75900
atgtggttgt tgttgggtgg ataggtttgt ttgtgggttg aggtttttag tagttggggt 75960
ggattatttt ttttttttaag aagttttggg agaagtagtt gagggggtta gtgttttttt 76020
gaggttaatt ttttgttttt tttagtgtag gtgtgaagtt atttttgttt gtgtgtttgt 76080
tatagatagt ggagggtggt gtaattgtga tgtttgtttg ttgtatttttg tgtttttgag 76140
gatgttgatt tttggttggt gttgggtgtt tgtttttttag ttattttgtt ttgatttggt 76200
gtggtaggtt tgagtattgt tttttttataa ggaaataggt ttagaggttt tgagttattt 76260
gtttgggttt ttttagttaa taattggttg agttaggatt tttatttagt tttgtgattt 76320
tagaagttta agtttttttt attatgttag ggttaggaaa ggattagaat gtttttttat 76380
gtgttgatta tttggatgat ttgtgtagtt tagtttgttg tagttgttta ataaaaatgt 76440
ggaatgaatg ggtttatatg ttttattgtg tgatttagag ttaatgattt tggttgattt 76500
ttttgttttt attttgtggt ttagtatttt tttttggggt gtgtgatttt ggaagtagtg 76560
attttttttta ttttattttg aatggtgttt gtgttgtagt aggggagtag ttggggattg 76620
ttagtgtttg ttgttggtgg gttgtgtgtt agattttgtt ttggatgtgt gggtgattgt 76680
tagttatatg tgtgtgaagg gttgtgtgtg taggatttgt gtaaatgtgt ttttttttgt 76740
tgttagttta tgggaggttg ttaagatttt gtttttgtat attagtttat attttttttg 76800
tggtttattg ttgtggttgt ttttttgtgt tatgtggttg taggtatagt ttttaagtgg 76860
atggggattg tgggttgaat gtgtattaga tgtttttgtt ttttggtggg tggagatttt 76920
taatttatgt attgtggttg ttgtatttga aaggtttttt atggtgaagg atttggattt 76980
gagtaatttt ttgtagaggt tttagaagtg tttttttggtt gggtgtggtg gtttatattt 77040
gtaattttag tattttggga ggttgaggtg ggtggattat aaggttagga gattgagatt 77100
attttggtta atatggtgaa attttgtttt tattaaaaat ataaaaaatt agttgggtgt 77160
ggtggtgggt gtttgtagtt ttagttattt gggaggttga ggtaggagaa tggtgtgaat 77220
ttaggaggtg gagtttgtag tgagttgaga ttgtgttatt gtattttagt ttgggtgata 77280
```

gagtgagatt ttgttttaaa aaaaaaaaaa aaatgaagtg tttttagttt ttttttttgt 77340
ggtgaggttt tgagttttta tttttagaag aaaatttggt tggggagggt atttttagat 77400
ttggatataa atgttaggag attagggttg tttttttagg tgattaatta tagagattta 77460
gggaaagtta tgtttggaag aggtgatatt gtgttttagt tttatttatt tttttatttt 77520
atagatattg tttggtgttt agtttgtatt gtgaggtttt ttaagtagtt ttgggtattg 77580
tgtatttggt atggttttta gttttatttt tttgttttgg gagatttggt ggggattttg 77640
ggttgagttt ttgtggttgt ggaattttgg tggtttattg tggtggttga gaatagggat 77700
gtggggttgg atgggttgag tgtaattttt agtggttttg tgaattgttt gggagttttg 77760
ggtagttttt ttttatttttg tgtttttgtt ttttgatgga aaaatggggg ttaggggggga 77820
gtgattttga aggtatttgt gggaattaag tgaggtgata aatgtagtgt ttttggtaga 77880
gaattggata tagagtttat ttttatgaag gggggttgttt ttttttttt tatgtttttt 77940
tttgttttta ttttttttt tgtattttttt tttttgttt ttttttttt ttttttttt 78000
ttttatatgt aggtatattt tatttggtgt tttaattgtt ttttttattt gtttttttgt 78060
aagtggtttt ttaagtggga atttaggttt ggaaagggtg ggtattttgt ttggtgggtt 78120
ttggtaggtt aggtgttgag tagttttagg agatataatt ttaggaaaag gtttatgtag 78180
gttggttgtt atgatggatt gttggtggga gaggggaagg tgttgtggta ggggaagttt 78240
tggaaatatt tattagtggt tttatttatg atttaggata tttgttaggt tattagaata 78300
tagttgagga agttgttggt tagatgttta gaagtattaa tttggtggtt gggttgtggg 78360
gggatttttt tggataggta gataaggtgt tggaggaagg ggttgatatt tatttagtgt 78420
ttgttatgta ttgagggtat aaagggtagg atggagagtt ggtttatgtt atttgtggaa 78480
tttttttaggt gtgggggtag atggggaagt aagggggttt gaagatatat gtgtttatta 78540
aatgttttta aattttaaga agaagaataa ggatatattt ataggggtag ggttgaaagt 78600
ttgatgtatg gtgttttggg gtttgttttt gttgtaatag ttatagtggt ttttttttat 78660
ttttttttt tttttatttt gttgtgaaaa tgtttaaaat atatggttaa gttgaaagta 78720
ttttataggg aatatattta gttattattt agattttatt attaatatta agttatattt 78780
gttttattat tttattgttt atttattttt tttttgtttg gttatgaatt tattttattt 78840
ttattttttg gtgtatttta aagtaatttt aggttatttt gattattttt ggaattattg 78900
ttagaaattt ttttttttt ttttttttt ttttttttt tttttttttga gatgggattt 78960
tgttaggtta tttaggttga agtgtggtgg tgtagttata gtatattgta gtttttaatt 79020
tttgagttta agtaattttt ttgttttagt tttttgagta gttgggattg taggtattta 79080
ttattatatt tagttttatt tttaaaattt tttattaatt ttttattttt tgtatttatt 79140
taggattatt ttttataaag ttagggtgtt gtaggagggg ttagaatttt aagttttaat 79200
tttggttttg ttattaatat tgtgtgattt ttagtaaatt attttgtttg tttaggtttt 79260
gtttttattt ttatgaaatg agggtgttaa attgtatgtt ataaataatg agggttatta 79320
gttattaagt ttttatttta ggttaggtat tttttatatg ttattatgag ttttttttaat 79380
ttaggtattg tttttaatgg atagtttagt taatggaagt ttagagaggt ggtgtaattt 79440
gttaaaagtt ttattattta gtgaatgttt ttatggggtt tgtatttagg agtttgatat 79500
agagtttagg ttttagtatt tggtgatgtt ttgggggtgt gagtagttta gtttattttg 79560
ggtatgtgtt tatttgttgt tttttttgtt ttatgtttgt gtttgttttt tttgaagttt 79620
agattgttat tttttagttt taaatattaa aattggttag gtatggtggt ttatgtttgt 79680
aatttagtaa ttttgggaga ttaaggtggg tggattattt gaagttagga gtttaagatt 79740
agtttggtta atatggtaaa attttatttt tataaaaaat ataaaaatta gttagatatg 79800
gtggtgtgta tttgtagttt tagttattta aggagaattt agttatttag ttattgaggt 79860
atgagaatta tttaaatttg ggaggtggag gttgtagtga gttgagattg tattattttt 79920
ttttaggtta ggtaataagt gtgagatttt gttttaaaaa taataaataa ataaaataaa 79980
ataaaaatta aaattaaatg ttaaaattaa tttataaaat aaattttggt taaagtagtt 80040
tgtttatttt gtttttaatta tgtatattgt attagatttt gttaggggtt tagtgtataa 80100
agatgagatt tttgttttgt tagataggtg ggatgatggt tatattttga gttagttaag 80160
gtattatttg gttgtgtaat aaaagttaaa atattagagg ttgagtagta tttggattta 80220
tgttttagtt taagtgttag ttgttatggg ggtttaggat agtttggatt tagtttattt 80280
ttttggtgta tttttgatat atagttttta ttttttgagt tgagatggtt ttagtttttg 80340
ttgttatgtt tgtattttag ttagagggaa gggaagaagg gagaggggaa agggtaagtg 80400
ttttttttta tttttaaggg tatgatttga aagttaaata ttttagtttt tttttattgg 80460
ttagaattta gttgtatggt tgtttttgtt attagggagg ttgagtaatg tattttttag 80520
ttgggttttt ttgtgtataa ttaaaatttt agttattata gaaaaaaggg agtttagata 80580
ttggggaaat aattagtatt tgtattatat gtattgatag ttttttttgt atgaatagag 80640
ttgtggtagg ttttatgtta gggagaaagt aagattggaa aagagtttat taaggaggtg 80700
gtatttgtat tgtgtttaag ggtaagaaa aatgtttttt aattaggagt tataaatgtt 80760

tggttgaagt gttattgttt ttttattttg gagttggaat agatgttatt agttaattat 80820
gatggttgtt gggtgtattg gttaatattt ataattttag tattttgtga ggttgagggt 80880
gggaagattg tttggggtta ggagtttgag attagtttgg gtaaattgta agattttgtt 80940
tttgtaaaaa aatataaaat gtagttgagt gtggtggtat ttgtagattt agttttagtt 81000
atttgagagg ttgagatggg aggattgttt gggtttagga gtttgaggtt gtagtgagtt 81060
atgattgtat tattgtattt tagtttgggt gatagagtag gatttgtttt taaaaataat 81120
aaaataaaat taaagtttta aaatggaaaa aaaattatga gtatataatt ttagatgtat 81180
ttttaagata gtaaatttgg gtgggtgtag tggtttatgt ttgtaatttt ggtattttgg 81240
gaggttaagg tgggtggatt atttgaggtt aggagtttga gattagtttg gttaatagtt 81300
ttatttgtat tataaatata aaattagttg ggtatggtgg tgggtatttg tgattttagt 81360
tatttggaag gttgaggtag gagaattgtt tgaatttagg aggtggaggt tgtagtgagt 81420
taagattata ttattgtatt ttagtttgga gaaaaaaagt aaaatttttgt tttaaaaaaa 81480
aaaaaaaaa gtaaatttag ggttaggttt gtagttatat ggttattttt ttttttgagt 81540
atggaggtag ttttagaaat ttgttttata ttatattagt gggtagttga ttgggttagt 81600
ttgttagttg aattttattt gttatagtta ttttttatag taaggggtat tttagatata 81660
ttttttttt tagggaagaa taagttgtag ttgtttgagt gttttattta gtggtgtgta 81720
tgtttttttt taattttgtg tttagtgatg ttttgtttgt agtttgaaat ttgttatagt 81780
gggtgtgttt atattatagg aattggtaaa tattatatat taggttttat tttttgtttt 81840
ttattagggt aggttgtttt tttttttgttg tttttgtttt ttgttttttt ttttttttga 81900
gatggagttt tgttttgttg tttaggttgg tgtggagtga tgtgattttt gtttattgta 81960
agttttgttt ttttggttta tattattttt ttgtttgagt ttttagaata gttgggatta 82020
taggtgtttg ttattatatt tggttaattt tttgtatttt tagtagagat ggggttttat 82080
tgtgttagtt aggatggttt tgattttttg attttgtaat ttgttttttt tggtttttta 82140
aaatgttggt attatgttat tgtgtttggt ttagggtagg ttgttagaaa tgtattagtt 82200
tgttagtttt agttttgttt gttttttatt tttttttttt tttttttttt ttttgagatg 82260
gggttttttt ttgttgtttt ttaggttgga gtgtagtggt gtgattttgg tttattgtaa 82320
tttttgtttt ttgggattaa gtgatttttt tgttttagtt ttttaagtag ttgggattat 82380
aggtgtttat tattatgttt agttaaattt tgtattttta gtagagatgg ggttttatta 82440
tattggttag gttggtattg aattttttat ttaaggtgat ttatttgttt tggtttttta 82500
aagtgttggg attataggtg tgagttattg tattgggttt tttttgtttt tttttaatga 82560
aaattttttt gaaagatttg tttattgatg tttgtgttaa taggtattgt ttgtgggata 82620
gagtttgttt tttttttgaa ggtttttttt tgtttgggtg attatttgta tgttattttt 82680
tgttgtttag aattaaagat gtagttatgt ttggggtagg ttgggaaaat ttgtgggaat 82740
tatagagttt gttgttatgt tgtgttttgg atatttgaag tttggtaggt aggtgggatt 82800
gagggtgggt attaggaaag tgatgtggtt ttataggaag gggatatagg ttttgagatt 82860
ttaggttttt aattggataa agttgttttt tttttttgga taaatgtttt agtttattta 82920
taattgagtg gttagttttt tttttttttt tttttttttt ttttttgaga tggagttttg 82980
ttttttttgtt taggttggag tgtagtggtg ttattttggt ttattgtaag ttttgttttt 83040
taggtttatg ttattttttt gttttagttt ttggagtagt tgggattata ggtgtttgtt 83100
attgtgtttg gttaattttt tgtattttta gtagagatag ggttttattg tgttagttag 83160
gatggttttg atttttttgat tttgtgattt gtttgttttg gtttttttaaa gtgttgggat 83220
tataggtgtg agttattgtg tttggttaag tggttagttt ttttagttgt tgggttttgt 83280
atattttgtt ggttttggag attatatgta ttatgtagat tttatttgtt ttttattttt 83340
agtgttgggg gaggggttaa gggtttgttg atgtgttgat atttggttat ttattggttt 83400
tgaatgttta tatttatttt tttgaaattg atgttttgta ttggaggttt tatgtggatt 83460
tgggggtaga ggtttgtatt ttagatggtg gggtggtttt tattaattag gtagattttt 83520
tgttttgttt tgttttttgg gttttgtagt tttagatgtg tgtttaagat ttgtggttat 83580
atggatggga taagttatag agatttagat tttgtggttt ttgtgattag agtagttgtg 83640
gtttttgtttg ggaatgtttt tattttgttg ttgaaggtga gaaatttttt tgtgttggag 83700
aatagttgtg ttgagaagtt ggtttatttg gtttgtagga ttgttggggt aagggaggtt 83760
tgttgttggt tttttttttt ttttttttgat tgggtgtttt ttgtttaggt tttttgtagt 83820
aaaagaattt tttaatttat tttgtttttt tattgagttt ttattttatg aagtttattt 83880
tttaatttta gttgattggt ttttattttt ttaagaggaa ggttaggggt agatgaggtt 83940
gaaggaggtt gatttttttg tgtttttgta gtttgagttg atgttgttgt atgtttatag 84000
ggttttttta gttttaaaa aggttttaat atgtttgtta tatgtttgtg gtttttttgg 84060
atgttatatt aatttagtaa ggtttgagtt agaggttgtt tttgaggttg tgaaaataga 84120
agaaggggttt agaggagaat tttttattt gatgatttta ggaaggtttt ttagaagaag 84180
tagtttttga gttgagattt gaagatagat taggtttgag ttaggttagg gagggtgtga 84240

ggggtttatt aggtagaggg aataggatag ggtaggtata gaggtttggt atttggtttg 84300
tggggaagtg tgagtttttg ggtatgatta aggtggggag aagtggtgtg agaggaggta 84360
gatgtggttg gtaggagatt ggtggttttg gttttgttga gttttatttt gtatgtatta 84420
ggtgatagag gttgtttagg atggtgggta gggaagtgat aggttatatg gttattttat 84480
taatagttgg gagtttagtt tggggggatt taagattttg gggaaggtat gagttagggg 84540
tttgttagag ttaggagtta gtgggagaag gaaatagtgt tgtttatgaa ttataaatag 84600
atatttttgt aatttttttt gatgtgatat aatttaaag tgggttattt agaggtggga 84660
aggtgatttt tggtttttta ggtttgggt ttgtttttt ttttttttt ttttttttt 84720
tgagatggag ttttatttg ttgtttaggt tggagtgtag tggtgtaatt ttggtttatt 84780
attattttg tttttgtgt ttaagtaatt tttttgtttt agtttttga gtagttggga 84840
ttataggtat ttgttattat atttggttaa tttttgtatt tttagtagag atggtgtttt 84900
attatgttgg ttaggttggt tttgaatttt tgattttagg tgatttgttt attttggttt 84960
tttaaagtgt tgggattata ggtatgagtt attgtgttta gttgagttta tttttaagtt 85020
tttttggtt tttagtgttt ttgtattagt ttattttttt ggtttttgt ttttaagagt 85080
ttttttaag attgtttta agtggatagg tttttagaat taaggagtag ttttaggagt 85140
ttttagttat agatatttta ggggggttgt gtggtttta gtttttggtt tggttttggt 85200
agttttggtt ttttgtgttg ttttttatat agtttggtta gagttggtta ggttttttgg 85260
ttgggttgag ttattgtggt tagtgattta tgtaggggga gaaaagtgag gagggggaat 85320
agagtgtgag ggggattgtg ttaataataa atttattagg tttttgaggt tattttatag 85380
gagggataaa tggtttattt aggttgattt ggttgttggt tggtttttag agatatttag 85440
ggtggggaag attttattat ttggggaaag taatattaga ttgtttggag gggtttagtt 85500
agtgtatttt gtgtttttg tttgtttttt atgtagagtg ttttgttttt aaggtggtag 85560
gttaagagtg tgagttgagg tttaggtgta aagaggggtg tggtatgggg gtattgtagg 85620
gtatggggtg tatgtatttt tatttatttt tttattgagt gttagggtaa tttgttaatg 85680
gggtatattt ggtgatataa aattttttgt tgatttatgg tagtttggaa tggagtggaa 85740
gtgttttatt tttggggaat ttttatatat atttggttgt gaatgttggt gtttttgtat 85800
tggagttttt ttggagtgtg ttgggtagag tggagaggtt ttttttttt ttttttttatt 85860
attttgtgga ggtgttaata tgtagttaga gatggttagt ggtagtgagg agaggggggt 85920
aggaagagat tagggttggg ggtttgttag tttatagttt tatttttgaa tatttatggt 85980
ttttttgttt aggttagtga ggttgggggt agagatgggt aggggttttt taatgttggg 86040
tattttttg ttttttata ttttggattt gtttttgga gaaagttttg gagtatttag 86100
aagttttggg gttgttggtt gaggtgggga gtagagttta gttgtgtttg gaggtttttt 86160
attttatttt agtttttagg gatagtttta gttgtgtagg tttaggttgt tgaggttggt 86220
tttagaaatt tttttataat tattattgga tggaaaatta gtttttaaa gggtttaga 86280
gttagttttg ttttttttt tataggtttt tatagttaga gtattgggtt tttgttagag 86340
atgtagaaga gatgatgttt ttagtttta tgtggtttgg tttttattgt ggatgttggt 86400
gattttagtt tttttttaa aattaagagg gttgatgttt tatattttag gtttttagaa 86460
agagttgtta tttatttata ttgttatatt ttttttttat tttttgttt tggaaagagt 86520
agagtggata tgtgtttttg tagaaattta tttatagttt gattgtagat gtattttgtt 86580
tttttattga gtttttgttt tatgaagttt gttttgtttg tatttttttt ttttttttt 86640
ttttttttt ttttttttt ttttttttt gttttttttt ttttttttt tttttgttt 86700
tatttttttt ttttttttt ttttttttt ttttttttt ttttttttt gtttttttta 86760
ttttttttt ttttttttt ttttttttt gaatagttaa agatagaatg aatggtatag 86820
gtaggtattg agtttttgt aattggaggt gtttaagtgt agggtggatg ggtatttata 86880
gggtaggtat tgaggaagga tttttttggg attgtggatg gtaggatagg ttttgtggag 86940
ttttttgagt gggtgtagaa gagggagggt atgttttgt tttttgtttt ttttgggttg 87000
ttaggttgtt attttaatt tttatttttt tttttttat ttgttgggtt ttatgggatg 87060
ggtagaggta ttgttagaat atggagttgt ggtttataga gggttagtag ggaggagagt 87120
tttttgggga ttatgttgtt ttagttattg tggtgatttt gaaaataaat gtttttttttt 87180
ttgtttgagt ttttgttgtt tttgttgatg tggaattagg gtaggttggt gatttagttt 87240
ttatttttgt attgtattgg tggtaagttg tttttagttt tagattttat agagtagggt 87300
gttttttta gaaaaatagt agggaggtta ggatattaag ggttttaggg atagtttgtt 87360
tttttggtt ggtatgagtt ttagttaagt tttttggtat tttgggggtg aggaggtatt 87420
agttgtgtgt tttagttatt ggttgttttg aggttgggtt ggtgttaggt tggttatttt 87480
tttttgtttt ttagtgtgat tttgtagagt ttttggaagg gttagagggt ttttttttat 87540
tattggtgtt tatgggatat tgtgttttta ttttattttt gtattttagt tttagatggg 87600
tgttaggaag ttggagattt tagttatttt atgattgtgt tttgtggggt tggagtagga 87660
ataggagtag gaggaatgtt ttggttatgg gattgtgtga agagagaagg attaggaaga 87720

aaatatgagt tattatgttt gggttatatt ttagtagagt tgttggtggt tttttaggta 87780
tttgaattta ttaattataa agtttattag gtgtgtaaat aaataaatgt tgtttaggag 87840
gtatagttag ggttttgttg gagagggatt attttggagg ggttatattt tttatttagt 87900
tttttagttt ttttgttttg tgtgtgtgta gtttttgatt attattattt ttgtttttgt 87960
tatttgtttt tagggtttta ttaggttgga tgttattgta ataaaatgta gtaatagaag 88020
gtttaggtag tgttgtagtt tttggtggta ttattagagt tagtaggtag tagggttggg 88080
gaattttaag ttgatttagt tttaagtgtt ggagtgggga gtttggatga ggtgggggtg 88140
gggaattggg gagttatgga gtagagtatt tgttgtgttg aaggttagga gtttttttgg 88200
gagattatga atttagaata ttaatatttt gtttaaaaag ttttttgttt tggagataag 88260
agtttaggtt gttgtggtta ggttttgttg taaatgtttt gtataaagta ggttgggggg 88320
attattggga agtttgggga atagattggt tatgtgtttt tttgagtgaa attttatggt 88380
tgtattgaag tggttaggta gagggtgtga tggggtgtgg tagttgtata ttggtttagt 88440
taggttgaat ggggaaatgt aaatttaggt ggtttgaaag agatttagtg gtttatagtt 88500
tggtggttgt ttttagtata ggtgatgtaa agagagaagg ggttttgggg gtttggttta 88560
ggtggaaatt atgtattgtt tgtgattttg ggtatgttga ggttggagta ggtttgtttg 88620
gtggttggta gaatgtatat gaagaattta tgtgtgtttt gaatgttttt tttgtttttt 88680
tgggtttatg ttttattagg agtttttga ttttgttatt ttgtaaagga aattgtgttt 88740
tgttattgta taggattgaa taggtgtgtg tggagttttt aaagttattg ttttaagggg 88800
ttttatggga taatgggaga gttgttgtga tgtttaagat taaagtgatg gttgggtgta 88860
gtggtttatg tttgtaattt tagtattttg ggaggttgag gaggatggat tatttgaggt 88920
taggagtttg agattatttt ggttaatatg gtgaaatttt atttttatta aaaaaataaa 88980
aattagttgg gtatggtagt gggtgtttgt aattttagtt atttgggagg ttgaggtagg 89040
agaattgttt tgatttggga ggtggaggtt gtagtgagtt gagattatgt tattatatta 89100
tagtttgggt gatagagtga gattgtttaa aataataata atgttaataa taaataaaat 89160
aataataata aaataataaa agtaaggatt tgaataaata tttgtatatt tatgtttgta 89220
gtagttttat ttttaattgt ttaaatgtgg aaggaatgga tatataaagt gtgtgtatat 89280
atagtggaat attatttagt ttttaaaagg aatggaattt tgttattata tgtgaatttt 89340
gaataatgaa ttttgaagat tttatgttat gtaagtttgt tttataagga taaatattgt 89400
atgattttat ttatgtgaag ttaaatttat agagaaattg gaatgatggt tgttagtagt 89460
taggggaagg aaggatgggg agttattgtt taatgaggat agagttttag tttgggaagt 89520
tggaagaagt tttggaggtg gatggtgttg atgggtgtat aatgtgaatg tatttaatgt 89580
tattaatatg gaaatttggt taaaatggta aatttgtttt ttttgttttt gttttttttt 89640
tttttgagat ggagttttgt tttgttgttt aggttggagt gtattggtgt gattttggtt 89700
tattgtaatt tttgtttttt aggtttaggt gatttttgtg ttttagtttt ttagagagtt 89760
gggattatag gtgtttgtta ttatgtttgg ttgatttttg tatttttagt agagataggg 89820
ttttattatg ttggttaggt tggttttgaa tttttgattt taggtgattt atttattttg 89880
gtttttttaaa gtgttgggat tataggtgtg agttatggtg gttggttaaa aatggtaaat 89940
tttatattat atatatttta ttatagtaaa aatgtattgt tttattagaa aaatgataga 90000
tttttataat atgaattaaa ttataagtta tgaaggaaaa attaagtaaa atattaaaaa 90060
attatttata agtaattata tttaaaataa aagataataa atatataatt tttattattt 90120
tttaggtttt ttgtaatatt taattattgt ttgttttgtt gtggttatgt ttttgaggtt 90180
gtatttgtat gatagaaata ttatatttgt ggtgattaaa tatttttttt tagttttatt 90240
tttaatgatg ttatatagat aatttgaaat tggttggttg ggtatgatgg tttatgtttg 90300
taattttagt attttgggag gttaaggtag gtagattatt tgagtttaaa attagtttgg 90360
ttagtagggt gaatttttat ttttagtaaa aatataaaaa ttagttagga gtggtggtgg 90420
gtgtttgtaa tttgagttat ttgggaggtt gaggtaggag aattatttga atttgggagg 90480
tggagtttgt agtgagttag gattgtttta ttgtatttta gattgggtga tagagtgaga 90540
ttttgttaaa aaagaaaaga aaagaaagaa attggttaag gtaggaatat tgatattata 90600
ggaataggta agtgttataa attgggaaat tgagtgttgt tgtttttgtt ggttttaggt 90660
ggttggttgt tgattgttaa ttggtatatt attggaggag ggaggtttag attgatttgt 90720
agagagatta gaggtagaaa tgtattatgg tttggatgtt gagggtgagg gaaagagagg 90780
agttaatagt ggtgtttgga gatttggttt gagtaattag gtggatggta gtattgtttt 90840
ttaagatgag ggttgtgggg aatttgaggg gttgtggaag gttttgttat tgatggattt 90900
agggtttggt agttttggga ggtttatttt gtgttaggtt atgtagagat aagattattt 90960
gggttttgtt tttgggaagt ttttgtttat gaggatatag tttgttattt aatggggtgt 91020
tttgtttgga gttttagtgt tgttgggaga aagtttttta tttttttttg tttttttgagt 91080
tttttttgtt tttttgttag ttgagttttt ttatggaggt tgtgtttgga ggggggttat 91140
ggatatggat gtgtgtgtta aatgttgttt ttttgtggat ttttttttta ttttgttttt 91200

atgttttgta agttttattt ttttggggtt tgatttgtgg ggttttttt tagatttgtt 91260
tttttttatt tttagaaagg tgtgtagggg ttttttatgg ttttttttt tgtttttatt 91320
ttttttttt tgggatagtg atttgttgga tgggtgggtt atttataggg tttgtgattg 91380
tttagatatt tttgttgtat gaagttttga ttttaaggta tgatgtagat gggaataagg 91440
tatgagtttt ttttggttgt tgtaataatt gttgaaattg ggtggtttat agtaagggga 91500
atgtgttttt ttatagtttt ggaggttaga agtttgaaat taaggtgtta gtagggttag 91560
tttttttga agattttagg ggaaaatttt tttttgtttt ttttagtttt tggtggtttt 91620
aggtgttttt tggtttgtgg ttgtattatt ttggttttg tttttgtttt tataaggttt 91680
tttttgtgt ttttgtattt tttttttgt tttttagaag gatatagtat tgaatttagg 91740
gtttatttta ttttgagatt tttgtttaaa tttttttgt agagattttt aaatagaatt 91800
aggttatatt tgaggtttta agtgggtata tttttgtttt ggggggatt attggtttat 91860
tgtaggtttt gttgtgtatt gaagatgtag gtggtttttt tggttaggtt tgtagttggt 91920
tttgtttttt tttgtgattg tggaagaatg tattttgttg gatgtatagg tttttatagt 91980
tttttgttt ttgttaaatt ttagtttagt ttttttttg ttgttggtgt gtattttttt 92040
tgtttagtgt tttttttggg attgtgtgtt taggttttga ggttttagaa aaggggggtg 92100
gagggttttt ttattatttt gtagtaatga ggttttatta aaattttgga gtttttagatt 92160
ggatttagtt tttagttatg attaaaggat agtttgggat atgattaaag ggttagagaa 92220
agatgattta ggttgagaaa gaggaggtta aggagtaatt ggattgtttt tgaatgtgga 92280
aaaggtaatt atgatttgaa gggtggtgtt tagttgtttt ttattttttt tgaggatagg 92340
aaaaaaaagg aaataaatga tgtataatta gagtagttgg ttagatatga gggtgaattt 92400
tttggttggg agtatggtta agttttgatt tgggtttttt gaaagagttt ggggaatttt 92460
tttatttgga ggtttttagg tttggttatg tggttttgag tgattatatt gaaggggtag 92520
tggttggggt tggagttggt gggttttgag tatagttatg aaggtatgtg tattttttgg 92580
ttgtttttt gttattgttt tttgggtttt tatttggtg aggtgtgtat tttggtggtg 92640
tttttttaggg aattaaatat gtttgttgta ttgtgtgtgg agatggagaa tgtataattg 92700
gttgatttg tgttaatttg gtggaatttt atgttagttt tgggaaagaa taattgtatg 92760
ggtgtgttta tatttattag gtgtttttta gaaaaatatt tgagaataat gttgtggttt 92820
aggatggttg ttgtgttgga tttggtattt ttttaggggg gttgtgttgt tgggttgagt 92880
tttttaggta ttggattttt aaattttttaa atatggtgtg gataggtggt ttagtagggg 92940
ttggattatt tgataggttt aggtgttgga gtttagataa gatatatttt ggtttggtgt 93000
ggaagatatg gggtgttatt aatggtagta atggttgtat ttttgaaatt tgggttttta 93060
ggttgatgag ggtgtgtatg tatttgaaat gtttgtggtt ttgtagtttt tatgtttata 93120
aatttatttg gttgaaaata gtttaaaata tttaaagtat gagggaggga gtgtttgttt 93180
tttttaaaaa ggaaggattt gattttattt atttaaaaag ttatttaaat ttagaatatt 93240
ttttgtaaga gattttttgt tttttgtttt tttagaatgg ttggagagtt ttagtatttt 93300
tgtatatttg ggatatttta gaggggtgg ggaggggtaa gtgggtagtg agtgatttta 93360
gatttaggat gagttgttag gtgtttttg gttatatatt taagggattg gagtgtagtt 93420
gtagtgttgt ggtttgttgt tttgggggtg ggggtgttgt tttatgttgt gaatttttat 93480
atggtttttg attttgggta gaggttgagg gtttaaggga tggggtgata gggagagtat 93540
gtaggagtgg gtttttggtt ttttagggtg agtggaagaa gtgttttttt tttttgtagg 93600
tgatagattt ggggggtttt ttttgaggat gagagtttgt tgttttttaa gttttgtgtt 93660
taatttaggt ttttaggttt attttagttt tttggttttg tttgttttgt ggatgatata 93720
gtttaagggt agagattgtt ggtttggagg gaaggttagg ttttaggtta gggtttagaa 93780
gggagggaga agtttttggg gtagtttttt ttttgtttat ttattgttta gttttttttt 93840
ttatattttt ttttggaaat gtttgttttt gataaggttt atttttttgtt tttaggaggt 93900
ttttattgtg gaggaaggga ggtgttgttt gtttttggtt tttttgatag ttgtgtttta 93960
tttttgtttt gtgttttttt ttttggatag tgtttttttt agggtttatt taggagggtg 94020
tagtggtggt ttttggggtg gtggttgtgg tgggggtgtt agttgtaggg gtgtttttgg 94080
tgggtgggag ttggtggttt tttgttggtg ttatgggatt tgtatgtttg ttttgtgttt 94140
tttggttttt gagtttatag gttgggattt tgtttgttag ttgtgtgtgt tgttgtttaa 94200
tttttgtagg tgtagagtgt gtggtggtgg tgatagagaa ttttgtttgg ttgtttaaat 94260
atagtttttt gtagaaggat tttgtgtttg gggaagggga ggaatttttt tttttttggg 94320
tgtttgtttt ttttgttatg gtttggtttt tatatttgtt tatatttggt tgtagtgggg 94380
tgtttggggg gaggggttga ggttgtgttt tttgttgttt tttgggtgtg ggttaggtgg 94440
ggaggagggg ggtgttttgg ttgtgtgttt aggattgttt tttagtggtt atttgggttt 94500
tagtttttta ggtttggttt tgataggtgg gtggagtagt tagtgtgaga tagggaggtt 94560
ggtgtgggtg tgggaatttg atttgtttgg gaggtggggg tggggtgggg gtgtagtgtg 94620
tggggagggg ttggtgtttg tttttttttt ttatttattt agttgagtta gggggtttag 94680

```
gggttttttt ggtggttagt tttgtattgt aggagtgtgg gtgtggtgtt ttagttagtg 94740
tgtagggttt gggttttgtt gggggtgttt ttttgttgtt gtttttttgtg tgatttgttg 94800
tttattagtt attatgttgg attttgtggt taatgtgtag ttggatggga ttattttgga 94860
ttttgaaggt gggtgttggg ttggttgttg tggttgtgga tgtgttggag aggattttgt 94920
gggtgggttt ggtgtgggat gggggtgtgt tgaggggaga tgggagtgtg ttgaggggag 94980
atgggatttt taatttaggt gttttttttgt tgagagtgtt gtgtgttttt ggttttgtgt 95040
ttgtgttgtt tatgtggggg attttgttag gggtatttgt gtagattttg tgtgttttta 95100
taggattttg tgtttgtttt gtgtattgtt gtttgggttt tttttttttt attgttgttt 95160
gtgtttgtta agtgatagtg atttttttga gggtttgtga ggttgttttg gaatttttta 95220
ggatgtatag ttttattttg ggaaatttat tggttttttt tttttggttt tttttggtgg 95280
tttttgggtt ttgtttggat ttggtaatgg gatagggagg ttgtttttta tttttgattg 95340
agtggatagt tgtgttttgt ttgggtggat agtttttttt tttttatgt tagttttggg 95400
gttgttaagt tgtgtagttt gtgggttggg agtattgaat ggatatagtt taggttgtgg 95460
tagggtttag agtgggatgt tttatggttt ttatttaggt ttggggatat ttttatttgt 95520
tttttagaat tgggttgtgg gggatagaag gggtttgtgt gtgggtaggg agagtatttt 95580
ggtttttttt tgtttttggg gtttataaag tgtgttggga tttgtggggt tgttttgttt 95640
aagtttgggt ttggtgtttg tgtttttgag tttgtgagtg tgtgtgtttt tttgtgtttt 95700
tttgattgtt ggtgttgggg ttttgtgttt tgtgtttgtg ggagtaaata tagtaggtga 95760
aggggaagtt tatataatgg tttttagtgt tttgggggtag ggtttttgag gggtgggttt 95820
gtttttgttg ggatttggag tttttgtttt ttggagaggt ttttaggttg atttgggtag 95880
agtttttgg tgggttggga gggggaaagg ttgtgttgaa atgagtaaat tgtttaggtg 95940
ttaggttaag ttgggaggtg attagtttga ggtttttttt gttttatggt tagaattagg 96000
gttgatattt gggtgttttg agtttagttg tttatatggt ttatttgggg ttagttttat 96060
ttgagtgggg gaggtggggt tttttggggg attagaattt tggttggatg ttaagtagag 96120
tgttagtggt tgttttttag ggttgggttt gaggagggtg tggggtggtg aagggatggg 96180
agggggttgt gatttagtgg ttattggtgt tgtgtagagt gtgagttgga aatattgtag 96240
ttattttgtt agtttagtgg tgaaagtttt tttttaggtt ttatttttt gtatttttgt 96300
tttttagagg gaggggaggt ttgggtttgt agagttggga gggtttgttg tttttgtttt 96360
ttttttttat aatatttttt tatttggata tttttgggta tatgtttata ttgggggtttt 96420
tttaggttta ttgtgttttg ttgagttttt tgtagttttt gagtgaatgt gatttttttg 96480
tttttgtttt tttgtaattt ttttttgtga ttgttttttt aggggttttt tttgttttaa 96540
atgtttaagt ggtatgattt agttggtttg attatttttt agtaagtttt tatggagaga 96600
ggtttgtgt tgtgtagagt tttttttttg tttgtgggat tgaggttttt gtttttagtt 96660
tttaatagaa agtgttgggt ttttagtggg atttttgggg aagaattttt gtgttttaat 96720
gggagttttg tggtgggagg ggaggttagg gtttggggtt gtgtttgttg tatagttgtt 96780
attatttgta ttatgaaagt tgttagtgtt tttttttgg gtttttgggt gtaattttat 96840
ttttgttttt atgtgttttt atttggagtt gtttttgtgg ttgtttttta agttagtttt 96900
gtgattttgt aatttagttt aagataatgg gtttattgag attattttgg tgtagtagtt 96960
ggtaattttt tggttttggg ggaaggtttt ttagttttgg ggagtggggt tttaatttgt 97020
tggtttttg tgtttattag tttttttttt gtgtgttttg aatggttttg ttgggaattt 97080
tggttttaga gttattaggt ggtttgagtt gataggtgtg agagagtgtg tgtgtgtatg 97140
agtgtgtatg tgtatggggg ttgatttggg gtatggaaag gtggtttttt ttggtgttta 97200
aggagtttgg agtatagttg gagggtgtgg gggtgtgtat atgggagttg gataattttg 97260
ggtggataga tagatgtggg gaagggatga ttgaaggagg tggaggagag agtgtgattt 97320
agtttagtta ggggtgatgt ggataggtag tttttgaatt agggtagaga aaagttatta 97380
ttagttagta ggggagaagt tagtatggag gaggtggatt ttgagggaga gtaggaattg 97440
gattgtaaga ggaaggagag ttttttggtt agtagtagtt agtagtagtg gggggaggttg 97500
gaatgagttg gttggagagg gggttggggt ataaggaggg gtttgtttgt gaagattata 97560
tgggttaggt tgtggagggt taggtatgtt tgttgggagt gtagttggtt tatgggaagt 97620
atttggagtg gttgggaatg ggtgtaggag tagtgttgtg ggagtatagg tttttttttt 97680
ggggtggttt atttggtgtt ttggtttttg taaggtaggt tgaaagggtg gggaggaaat 97740
tgttagtttt ttatagtgtt gggatggtgg ttttagggtt tttgaggtta gtggatgtgg 97800
gtgtttgtta ttatgtgggt tgttgagggg tggagatttt aggggttatt ttaaagtagg 97860
atgagttttg agttatggta tttttggggg tagtttttta attgagtaga tgtttaggtt 97920
tggaattttg taatagaggt tatagggttt tgattagggt gttttgggag gtttagaatt 97980
agtggtagta tatagggtag atggtaagtg atttggtatg gggaaagagg taggtgttta 98040
ggttggtata gtatatttgt aaggaatagg tagatgggaa gttgtttgtg ggtttgtgtg 98100
tgtgtttgga gttaaaattt tgttaatgtt ttatgttttg ggtatattta tttttttttt 98160
```

ggggagtatt ttttttttta ttttttttt ttttgtttgt tttttttatt tagggttttt 98220
tttattttt ttgttttggg gattgagggt attatggttt tatgttttat tattgatgag 98280
ttgtataggg atttagtttt tttgttgttt aggttgggtt ttttaggttt agggttttta 98340
ggaatggaga gggtattagt gtttttttatg gatttaaatt ttttgtattt tgtttttgtt 98400
ttttttttaa gataggtttt tgagttttaa ggttttaggg ttttgtggag gttgttatgt 98460
agtagtaagg agaatgtttt gtatttggtt gatgagattt ttagagtttt attttttatt 98520
tttttattgt ataaatgggt ttttaggtga ttgtagtatt tgttattgtt tgtaataggg 98580
tgataagagg gatgattttt ttttttttt ttttttttggt tggtggaggt atggggttgg 98640
tggatggtat gtgtttttgt gaatttaggt taaatttgtt attgtaaata tgattataat 98700
ttgggttttt gtgtaataaa agttttttta agtattagtt gttggtttgt tttgtttagt 98760
ggtgtttgtt gtaattagat ttgtatattg agaaagaatt taaaagtttt tgatgtttgt 98820
tgaaataatt tggtttagga tttatgtgtt tagattttag agttgtgtgg tatttgagtt 98880
ttttttgagt ttttattgtt gtttgaggag gatttttaga tttgtgtttt ggaggtagag 98940
taggttgtgg gatgggtttt tgggtgggaa ggattatgtg gatatgtttt tttgtttgag 99000
agttttaata tttttgggat gtgggagttg gtgtgttggt aggatttagg tgtttttttt 99060
ttttttagag aaaaaggttt tgttgtttgg taataggtgt agatttgttt ttaattaatg 99120
ttagtaggtt ttttgtgtga tgaattttgt tttttagtta agatttaagg tattttgtga 99180
atattgtttt tttgtagttt gagtttttgt ggtgggaggt aggagttatg gggagtgggg 99240
gtaggttttt tatatgggtt ttatagttat tggtagtatt gatttgatgt tttttgagtt 99300
tagagtttag ggttagatag atttattgtt ttgattatga gttggtttat ttagaggggg 99360
gtggatatag tatttaggta gtagatgtat tgtgattagt tttgtagtgg ggttgtgggt 99420
tttttgggtt ggatgtttgg gaagaggtag gtggaggtaa atgttaggat atttttgtag 99480
tgattgggtg attgtaggtt ggaaatgttt tttgtgggtt gtggttgttt aggaaggttt 99540
tgaatggggt tagtggatag agtttgtatt tagaggggta gtgttttgga ggagtgaggg 99600
gtatggtagt gtagggatgt ttaggttgtt tttattttgt tattggaaag ttgggtggtt 99660
ttggtttttt tagttttttt gtttgttttt tttgtttgtaa agtggggtta gaaatagttt 99720
tttttgaggg ttgttggggg attttgagat gtagtttatg gtgttgagta tgggttttgt 99780
tttttatggg tgtggtgggt gttgtggttg gtgtggtatt tgggtgggaa aagggggtat 99840
ttgtaaagga taggtaggtt tggatgttta aatatgtaga tttggggatg ggaggtttta 99900
ggtaagggtt tgtgtgatgt tattgtatga atgaggttgg atagtatggt tattattaat 99960
tatggaaagt atggtagata ttagtgttag tagtgtttgt ttgggggttt ttttgagttt 100020
gataggtatt taggttttta gtgtagttat gggatttttt ttttttttgtt tggttgatat 100080
tttggaggtt ggtgttatgt tggttgtagg ttttttttat tgggaggttg ttttggtttt 100140
ttttgttatt atttattttt atttttattt tggagtgtta tagatgggtg gattatatgt 100200
ttatttgtag ggttaagtgt tgataatgag gtgagtattt gttggatgat tggattatga 100260
ggatagtggg ggtgttggta gtttgtgttt ggttttgtag gttggttttt ttttgaaaag 100320
tggttgtggt agagtgtgta ttttagggag tggtgtttgt tgttttgaat atttttattt 100380
agtaggtttt tgtaagggtt tagttaagtg tggtgtagag ttgggttggt agttggaata 100440
gttttatttt gtttagtggt gagattaagg gttgatgggt taaggttgtg tgttgtttgt 100500
tttagtttgtg gatttattta ttgagtattt ttatattttt atattgggtt tggggtgtta 100560
ggaggtggag gggggattgg gtgtttggaa aaataagttt attgagagtt tgagttttta 100620
ggggttagta ttaggtagtt attatggtgt ttggtatata gtggttattt ggttttgttg 100680
gttgttttgg tggtggagag tgtgtatggt ggttgtatgt gatgggtatt atttagggta 100740
atatagatat gtagattgtg tatttgtttg taggagttag ggattttttta gtttggtaat 100800
ttgtgatttt tgtttatttg ttgggagaga ggtgttgttt tttgatagtt tggtgttggg 100860
agagtagaat tagggttttt ttttttttt tttattttt atttttttata tgtttaggta 100920
ttaggggagg ttatatttag ggatgagttt tggttattgg gtttagggtt ttattgttgt 100980
tatttttttt ttttgttagt tagttaaatg tttgtttatt attgtgttgg ggatggggtt 101040
ggtttgtttt tttgaagttt ttttggtgta tagtttaggt tggagttttg gatgattttg 101100
ttattttttt ttttgatttt ttttttttat tttgatttat tttagaaatt tagttaaaaa 101160
ttaagttttt gtgttttttt ttgataaagt tattgaggaa aaatggttaa atattgtttt 101220
ttttttttaaa gtttttttgg ttttttttaa atttggaggt tttgagttgt ttatgagatt 101280
aaagggtttg gaatttttttg gaatatagtt ttagaagtgg gaggatttgt tattttttta 101340
gttaaataga tttattagtt ggtttttaga tatttttagg tgtttggggt atttttgggt 101400
ttaggttgtt taatttggtt tgtagagttt tgaggttttg tttagattgg gttagtagat 101460
agttttagag attttaagtt tgttgttgtt tttaaaagtt ttgttaatgt ttagaaattt 101520
agagggataa gaagatgatt ttgatgttag ttgggattat aaatagtgag gtttgttttt 101580
ttttttattt atgtttgttt ttattgtttt tgtagttggg tttttagggg attgtttgtg 101640

gttatggggg aggttgggaa tgtggggttt agatttttatt gggtttttg tattttttg 101700
ttttttttg tttattttt tttttggaaa tttttgaata tttttttata gtttttggta 101760
ttttgttatt tttggtattt ttgggttaat tgggaaagtt attatttgtt taatatttag 101820
aaaattaagg ggtttggttt taaatatatt gtagtgtttt tttttttata tggtattgaa 101880
ttttttgtgt ttttaaagtt aggagtttgt gttttggatg tgggtgatgg agttttgtag 101940
atatttggag tggtttttt gggagagttt tttttgtttt tttatttatt tgttttttt 102000
tttttgtttt aagttttggt tatagttttg gaggggttat ttgagtgtag gttgtttta 102060
ggtgtatagt tggagggggg tagttatttt atattttttt gtttgttagg ggtatttta 102120
gggatttagg ttttgaggga tagtggaagg gtggggtggg gagtaggttg ttgtttttgtg 102180
ttatttgggt gttatgtaga ttttgaatgg gtagtttttg ggagttggtt ttttttttt 102240
atttttttta tagtgttgtt tttttgaggg gttggagttt tttggagttt tttgtagttt 102300
gtggtaggtt tttttgattt tgtgtttggt tttgtagttt ttaatttata gttagtggta 102360
tgaagaatat ttttggttgt ttttatttgt ggtttttgta ggtagtagtt ttttggggta 102420
gggtttgggg gttttggtat ttttttgtgg gatttttttgg gtatgaggga gggagttatt 102480
tgggttatag atagattttt tttttgtatt taggaggttt aggatggatt tgttttattt 102540
tttttttgg tgggaagaga gtatttttgg ttatggggaa tgtggtttgg atagagttat 102600
ttagtttttt tttttttta ggtagggttt tgaaagatgg agatggtttt gtttttgtta 102660
ggttaatagt tagtagtttg gtggattttt atgtagttta ggtaggattt tttttgttt 102720
ttatttttta ttttatgtgt aggaagttgg ttttaagtag ggatttttgg ttttgttttt 102780
tatgtgggtg ggtagttttt ttgtgagggg taggttaggt ttttgtttta tgtttttttag 102840
agattgatgt tatttatagt aagagtatat aagtttttt ggtttaaat taggtttgga 102900
ggggtggttt tttttttgt tttttgtttt ttgtttttta ttaaaggtta gggaatggag 102960
tgttttgtat gtttgttggg gttgggtttt ttttttggt ttaaaatgaa gggttggaat 103020
ttttggtgta gattgtatgg tgagaggtgt tttttgggat ttatttgagg agtattattg 103080
ttgaatgttt tgttaggtta ggagtattgt tgggggtggg agaggtagtt ggtttatttg 103140
tttatttaat agttatttat gggtatttta agggttagtt ttgttttagg tattgggggt 103200
ttaggtatgg gtttaggaga aggggtagaa tgggagggtt ttttggagga aggtttttta 103260
attagagatt ggggtaggag tttgttaggt aggtgatgtt ggttagtttt ttttgttatt 103320
ttttttttt ttttttttt ttttttttt ttttttttt gagatagagt tttgttttttg 103380
ttgtttaggt tgtagtgtag tggtgtgatt ttggtttatt gtattttttg tttttgggt 103440
ttaagtgatt tttttgtttt agttttttga gtagttgaga ttttaggtat gtattattat 103500
gtttagttaa ttttttgtat ttttagtaga gataaggttt tattttgttg gttaggttgg 103560
ttttaaattt tttattttag gtgatttgtt tattttggtt ttttaaagtg ttgggattat 103620
aggtgtgagt tattgtgttt ggtttttttt tgttattttt gtagatttat agtgtgtgtg 103680
gaagattgta tgtttgtgtt tggtatagtt gtttttggta tttaggtggg aggtggggga 103740
gagggttgtt ggtgttagta taggttatgg tatgagatag gtttttgtag ttaagtgggg 103800
tgtattgtag tattggaggg gttttgttgt gtatgagatt taggttttgt atttgttttt 103860
tttttttagga ggtttggagg gttgggaggg attagggtgg tgaaataatt taggtgggag 103920
agaggtattg ttggagaggt agagggagtt gtaggtttgt agtagtgagt tttaaatttt 103980
gggtttttat tagaaaattt tagaaagggt tgttttgttt tttttttttaa attataaaag 104040
aaatttttgg ttgtttgggt tttttggggt tgggtggggg aggagatttt ttatttgtat 104100
ttttgtaggt ttagtattgt gtagagggag gagggtggag gtaaaggtga gggaggaggg 104160
tggaagtaaa ggtggggttt ttttttagtt ttgttgtttt gtttggttgt tgttttttaga 104220
tttttatttt tggtttggaa atatggaatt ttgggatagt tggggtaggg ggtatagaat 104280
ggagatttttg gtgagggagg aattttttttt ttagttttgg ttgtggttat agtaaggtta 104340
ggttgggagg attttattta aatatttata ttttttttaa tatttaaatg atgaagtagg 104400
tttttgtata tagttgtttt ggagttagtt gttgtttgtt ttattttttt ttttttttt 104460
ttttttttt ttttttttt ttttttttt ttttttttt ttttttttt ttttttttgt 104520
atttttttgtt tttgatttaa gtttaggttt ttttagttgt tttttttta tttataggta 104580
tttttttgtga ttgagttttt ttttgttatt ttttataatt attttttta attgagtatt 104640
ttttaggtgt taggttttgg gttggtattt tgtatataag gtgtttttta atttttatga 104700
gagtttaata aggtagattg ggttgttgag gttttagtgt tggaaggttg gtttgggttt 104760
ttttagtttg tatttgtatt tatttattat tttttttattg tggagatttg tttttttggtt 104820
tttggttgag tatgatggaa atttttgttt attagttttt ggagagtggg gatggttgag 104880
ggttttgatt ttggggtttg atgggggttg tttttgtttt atttagttt ggttgttttt 104940
ttatttaatg attgagtttg taaaattaga ataatagtgt tatttggttg gtattttggg 105000
tggttgggag gtgtgggaga gggtgtatgg aggggttgag tgaggtgtta gtattgtggg 105060
gatttttgag ggtagttttt ttaggtttag ggtggagtgt aggtagaggg tgtgatggtt 105120

```
tagtgtgttg gtttatattt ggttttgttt ttgtttatat ggtaatgtgg gggagttatt 105180
agttttttag tgttttagtt ttttatttgt gtaatgggga taagagtaaa ttattgtttt 105240
taggtatggt aagtatttat atatattgat tattgtagaa tgaatatagt gagttttttt 105300
taggttttta ttatatgtta ggtagggtgt taggttttag tggagagggg ataatgatgg 105360
gtaagttggt ttttgttttt aagggttgtt gagtagggag gaaggtggtg gttgttttga 105420
ttgtgatttg gagtggggtg atttgtaaag aggttggtgg gaggttggtt gggggttggt 105480
tgggggttgt ttttttttt gtttgtgttt tgtggtaggg tattaaattg gttttttta 105540
tggatttttg aggttttgat tgtgggattt tgggtaggtt ttgggatttt gtgattttgt 105600
ttttgtttgt tatttaggtt tttgttttta gtagtgttta gtatatggtg atttttgtat 105660
gtttttagt gtgtgattga ttttggagtt gagatttggt agttgaggtt ggatattgtt 105720
tttggataat ttttggggag gtgggggtag ggtggggttg tgagttggta attttgagtt 105780
tttagtagtt tttgaatgga ggttttttt ttgtaggatt tttggggagg aaggagggga 105840
tgggggagat gaggtttgtg agtttttagg agggtagagg tttatatttg tagtgtttta 105900
gaaatatttt ggggtgttta ggtttattta gtttttttga gttagaatta ttaagagttg 105960
gggattagta gtttattttt tagtaagatt tagaaatgat tttttgaat agtttgagtt 106020
aagaatgata tatttggaat tagaatatta gaattataga ggaattgaga gaatgggttt 106080
aaaatgtgta gggattttag ttatatggtg ttatatggag gaaagtattt aatattatta 106140
ttgtatgaat ttgtttaaat atatatatat atatatatat atatatatat atatatatat 106200
atattttta gtaaagagtg gaaagatatt tgttagaata ttgatggaag ttatttgggt 106260
tgtgggttta tagattttt tttttgttt ttgtgtattt tgtaaatttt tggttttgtg 106320
tttaaatttt ttttatagtt aggaataaag taatagatgt tatttttaa agtttatggg 106380
tttgtgtttg taattttaat attttgggag gttgaggtag gaggattttt taagtttagg 106440
agtttgagat tagtttgggt aatataggga gatttttatt ttttttttt ttttttttg 106500
atagagtttt attgtgtttt aggttgaagt ggtggtggta tgtttttggt ttattgtaat 106560
ttttgttttt taggtttagg tgatttttat gttttagttt tttgagtagt tggattatag 106620
atatttatat ttgtaggatt tgagaatttt ataagaggaa agtttttatt tggaggttgt 106680
tggggatttg gagttgttag tttatatatt ttttagtaga gatagggttt tattatgttg 106740
attaggttgg ttttaaattt ttgtttttaa gtgatttatt tattttggtt ttttaaggtt 106800
ttaggattat aggtgtgagt tattgtgttt ggttatggga gatttttttt taaaataaga 106860
ataaaaataa aaataaataa aagtttattg ttaaaaagaa aaatatatta tttaatttaa 106920
tttttttta ggattgagag atgaggaagt agaggttgga aagtgttata tttttggtt 106980
gttggtagtt aggggatttg aggtttgggt tggttttttt gtgttttagg tttggggtgt 107040
taggtggagt ttttgttttg tttaaaggtt taggtgtttt tgttagtatt gtttgttttt 107100
gtatttttgg ttataaggaa tgtgggttaa aggtagggtt tttgattttt gtttaggagg 107160
gtgaagtggg ttggtttttt ttgtttttgt tttaaggttt ttgtggttgt gtttttggaa 107220
attgttggtt tgagttagtg tgtggagttg ggatagattt tattgtggtt tttgtggggt 107280
tattttatgt ttgggttgga agggattttt tggtttagtt ttttttgttt tttattagga 107340
aattgagatt agagaggtta tttgtttggt ttggggtggg gtgtagatgg tgtttgtgat 107400
ttttgtatgt tatgtgtaga tagtttttta gttgtgggtt ttttatgtaa gtagttatgt 107460
tggggataat ttggttgaaa tttgtgattt aatgaagaga agaatgattt tttttgtagt 107520
tttgtgtttt ataggttttt ttttgttttt attttttttg tagaggggtt ttatgttggt 107580
gggtgggttt ttgttgttgt aatgggttta ggtagttttt gggtagtgga ggtttggttg 107640
ttttttgtt taatgttttt tgtagtttat gttgtttggg aagggtgggg aagtatttag 107700
ttattgggga ttttataaga tgggtatttg ggagatgttg attggtaggt taggtttttg 107760
tggtttatag gtaggttaga ggttatagtg ttgagttggg ttgagttggt agtttagggt 107820
agtgggggtg ggggtatttt gtttagttat gggttttttt ggtataaggg tttggagttt 107880
attgtaaata attttttag ttttttttt ttttataggt tattagtaga attgaggggt 107940
tttttgttga tgaggtgtgg tttgggaggg gttagttgtt tgtttatttg atttttgtgg 108000
ataggtgatt ataggttttt tgttttaggg ggttggggga aagattgggg aggttttttt 108060
ggttttttta tttggaagga aaatatgggt ataggggaga tattttatag ggatatattt 108120
tagttagtat tttttggttg tgttggggggt atagtgtttg ttttgtttat aagaggggtt 108180
agttttttt aggatttaat tttagttttt agtgtagggt agatgagtat ttagaggttt 108240
agaggattta tattatgaga gagaagttgt gattatatag gtattgaagg ttaagtagaa 108300
gagtgggata tatgggtgat ttgaaagttt tagggttagt gtggttagtt ttagatagtt 108360
ggggagtttt gggtattagg tttagtaggg gaggagggtt tttgtgatag tttgttgggg 108420
ttgttgtaat aaagttttat gaattttggg ggttgaagat ggttttagag gttagaagtt 108480
taaaattaag gtgttgatta ggttgatttt ttggggtttt tgtgggaggt tttggtttag 108540
gtttttttt tagtttttgg tggtttttgg gtgtttttgg gtatgtagat gtgttatgtt 108600
```

```
atttttgtt ttgtttttat atggtttttt ttttgtgttt ttgtgttttg taaggatgtt 108660
tgttattgta tttagagttt attttaaatt taggatattt ttttttgag atttttattt 108720
gtatttgtaa agattttatt ttaatttttg ttatattat aggtttgagg gttagggttt 108780
tggtatgttt tttgggggtt attatttagt ttattgtggt agttgatttt aggggaagta 108840
gaatgtttta agtttggggt gagtggaggg aatgttgttg ggtggggagt gggagattag 108900
gtttggagat attgtggatt tttttgtttt gatgagggtg tgatgaggtt tttattttag 108960
gtttttgaa tggtggttgt ttttgaggag attttttttt ttggatttgt ttttttagaa 109020
attttatttt aggtttaggt tttaaggtgg atggtaagag gtggttgttt ttgttatatt 109080
tgatgggtga gtgtgtgaat ggggttggga gtaggatatt tgttattttt gtggatagat 109140
tttagaggtt tatgaatttg ttgttgggga aggtgtttga tttagtttga tttttttattt 109200
ttattttttt tttttgtttt tttttatta gttttgtttt tagagtttta ttttttttaaa 109260
ggtaggtgtt gtttttgga tttttagta gttattttt atttagatt tttttttgt 109320
aggtttttt ggttttagtg tttattttt tttgggtttt tgtttttta ttgtgaggtt 109380
tttgggtaga agtgtggggt ttttttgttg tgtttggttt ttgtggtata ttaggggttt 109440
ttttaggggt ttgttttgtt tgttaggttt ttagggagtt ttttaagttt ttagatgggt 109500
ttgatggagt tttttaatgt gttgttttag agtaggaggg gttttggttg agtttgagag 109560
tttttttagt tatattttgg gttgtttggg gtagttaggt agtttgtttt ttggaggtgt 109620
tgtttttttt tggttatttt ttttggaatt ttttgtgttt agttgtggtt ttttttatat 109680
agtatattta ttattttttt ttatttgtga ttttttttg ttaattttt tatgtttttg 109740
agaatggaga ttaggttttt tttattttgg agtttagtag aatttaaatt aggtagattt 109800
gtgtaggttt atgtggggt tttgtggtta ttgtggtttt gttgttattt ttagtttagt 109860
ttggtgtgtt ttggattatg ttatttagtg ttggtttttt aaatttttt tttggttttg 109920
taggtttggg aagtagttgg tttttgggtg tagttgtgat tggagtttga attttgtttt 109980
ttggggttat tgtttttgt aattaggttg tagtttatat tggtttgggt agaggttatt 110040
ggttgtttgt tttggggtta taatatagag agttttgtga ttaatggagg ttttatttgg 110100
gttaagtggt ttttggtgtt aggtgttttt tttttttttt ttttttttt gtattttttta 110160
agtgtttgta atattttttt taatggataa tgagatgagg agggagggag gggtggtttt 110220
tttgtttagg tagattagga atggttattg tttgtgtgtg tagtgatagg tttgggaata 110280
gataagggag agtttgtttg ggaggttgga tttttatatt gatgttgttt gtgtttggta 110340
gttttgtttt gggtttttaga atagatattg attaatatgt tttttagggt ttttaggttt 110400
gggttttggt ttttggtaga gttttttatt tatgggaaga agtattgagt agttttgttg 110460
gtttttgta gggagttggg atgtattttt aagagtagta tgtgtgaagt ttggggtttg 110520
ggtaaaggag tgtttgggga agagtttgtt gttgttggag ggttggaagt agaggggaag 110580
gaggggtatg tggtaggttg aggaggtggt tggtgatttt gtggtgggga tgaggggggtt 110640
atgagtagat taggagttag tattatgttt ttttgttttt agaattgagg atggagagtt 110700
gttagagagg ttttggtggg gtgtttgggg ttatttaaag ggtgtgggtg gattaaagtt 110760
gaggaaagtt ttattttggg ggattttgta tttaattttt gaggtttta gagttgtagt 110820
ttgtggtttt ttgaagttaa ttattttta gggttttgtt tttgtttttta gtgatagtta 110880
tttattaggg agtttttgat ttaaggagat ttgaaaggtt atttggtttt tttttattt 110940
ttaggtaggt tatatttaga tttttttata tagatttgtt ttatttttt gttgtttttt 111000
attagggatg gagggtgggt ggtttttta tttaggtttt ggtggtttta ttttagttag 111060
gaaatttttt tttttattt gttttatatt ttttttttg tggtttttgt ttattttttt 111120
ttaatttgtt tttagtggag tagttagtag ttggtttaga atttggggggt gggggggagtg 111180
ttagattagt gtagtatatt tgagagtgtg ttagaggtgt ttttattttt atagttgatt 111240
tagtaaagaa aagaggaagt ttagtaattt agtgttattt taaagtttgt ttggaatgat 111300
tttgtggttg ttggaattag gggtatgtgg tagatgatgg gagtaggtat agagaggttt 111360
ttgttttgga ttagttgttg ttgtttttttt ggaggttggg ttgttttttta agaagaggat 111420
ttaaggtttt gtgagtggtg gtttttttg gagtttttat tgtggttttg gttttgtttt 111480
gtgtaaagag agatggtttg aagtagaagt tgttatgtgg ttgttttagg taggtgtttg 111540
tagttttagt tatttgggag gtggaggtag gagaatggta tgaatttggg aggtggagtt 111600
tgtagtgagt agagatggta ttattgtatt gtagtttggg tgatagagta agattttgtt 111660
ttaaaaaaaa aaaaaaaaaa aaggagtaga gaaatttttt ttagaaggtt tgtagttatt 111720
ttttttttat gttttagagg taaaaaaaaa aaaaattgta tattttgttt tttttttttt 111780
tttttgagat ggagttttgt tttgttgttt aggttggagt gtagtggtgt gattttggtt 111840
tgttgtaagt tttgtttttt gggtttatgt tattttttg ttttagtttt ttgagtagtt 111900
gggattatag gtgtttgtta ttatgtttgg ttaatttttt gtgtttttag tagagatggg 111960
gttttattgt gttagttagg atggtttgga ttttttgatt ttgtgatttg tttgtttttg 112020
tttttaaag tgttgggatt ataggtgtga gttgttgtgt ttggttatat gtttttgttt 112080
```

ttgaataggt ggttttttga ttttgggtgt ggttatttta agattttgtt ttggtggggt 112140
ataattttgg ttttaggtag ggtgattgta taatttattg ttttttgggg gatatgttag 112200
tgaaagggat ttttgaatga ttatggtggg ataatagatg tgagttgggt ttgttttagg 112260
aaggttggat gtatgggttt tttgagttag aggagataat agtagaaagt tttgtggttg 112320
atgggattga gtaggagaag ggttggtgga ggttggggta ataatgtatt tgtttattgt 112380
gaagggtttg tgtgagttga tggttgatga gagggtagtg gggtgaggtt gtttttttgtt 112440
aggttagatt agttttagga tatttatatt ttttttttag aattggtgat ttagtggtta 112500
gaaggggttt tagaattgat gggatattga gggagttgaa tggaatggtt ggagtggtta 112560
tagtaggttt taggtttttt tttgtttttt ttttgtgttt tgtttttggg gtttatattt 112620
tggggttgtt gtaatagagt gttataattt gggggtttat aaatatagtt tttggtggtt 112680
tttggagatt tttggtgttt ttttggtttgt agatgtatta ttattatttt tgatttttgg 112740
tttttgtttt tatgtggttt tttttttttg tttgtgtgtt tttttttttt tttgtttttt 112800
ataaagatgt ttattattgg atttagggtt tattaaattt agaatgattt tattttgaga 112860
tttttatttt aattatattt gtaaagattt gtttttttaaa tgtgattatg tttgtaggtt 112920
ttaggagtta ggatttaaga tatattgttt tgggggatat tgttgaattt attatattta 112980
ataaatttag agttggtgtt aatgtttttt gtggatatta tagtagattt tggaggtttt 113040
ttgtaaagga atttgtagga tttttttttt tttttggagg gatgagattg tagttgggtt 113100
ggggaaagtt gttggtttaa gttggggttg gtttagttgg tgttggtttg ttaggttgtt 113160
agagagtttt atttagggag aaattaatat tataaagttg ggggataaga gaaggtttgg 113220
aattttaagg gttggttgtt gaggttagtt gtttattttt ttgtgtttta ttttataggg 113280
atttaggttt gttagtgttt tgtttttgga aggaatgttg tgggttgttg tgttttatag 113340
gtagggtgga gggttttttt gtagttagtg agttgttttt tttgtagtgt gtagagggaa 113400
ggaggaggtt tgtttatttt ttttggtttt agttgaaaat ttaattatag gttttggtat 113460
taggtagatt tgtttatttt tatattatta ttgtagagtg agttaggttt tttgggtggg 113520
gttgtgatga gtaggaagga tttttggagt gtttgggatt ttttgggttt agggttgtta 113580
gaggtagaga gaaagaagtt ttttttttaat atttgagtaa agatagttat tagaatttat 113640
tagagtggtt ttttgtggtt tttttttggg tgttgatttt tttggtgtat ttgaagtttt 113700
tagggatatt attgatgttt tttgtatttt tatttagggg ttagtggaaa tggttttttt 113760
tttttttgtt tagaggtatt gtggatgatg attttaggtt ttagatttta gttattgggt 113820
tatgtgtgta gataagtttg ggtagttagt aatttttggt tattaagttg gtttttttgtg 113880
gattgggttg gtattggagg tgggttttag ttgttagtat tgtggtttag ttaggtttgg 113940
ttttgggaag gtagaggggg aggggattgg gaaagggatt gagttggggg tggggtggta 114000
ttttggtttt ttttttttag atttttggta ggtttgaatt atttggttgt ggtttttttaa 114060
ggagggagtg tttttttttgt tttgatgtgg attttgtttt ttgtttatgt tgtttttgtt 114120
tttaaataaa tgtggttttt tgtggggagt ttgggggttg tttttttattg atattgttgt 114180
tttttggttt tttttttgtag gttttgttta gttttattgt gtttattttt aagtttttttt 114240
ttttggatgg ttttgttttt tgagaatata tttttttttta ttattttaat tgtgtttttt 114300
tttttatttg gagtttgttt tatttgtttt aggagttatt tttttttttt attaagtagt 114360
ttaggttaat tttggagggt taatttgtgt ttttttagaa gtaggttttg agatagggat 114420
tttagtggga ggggatatta ggggatgttt gagtgtggga atgggttagg tagggaattt 114480
agttaataaa gggtgtttta ttagtttatt gtgattatag gtggttgttt agtttttata 114540
ttgggtagta tggggagatt gtagattgtg tttttagttt tgttttattt ggggttgggt 114600
aagggagttg gggtttttat attttagttt ttggttaagg attgtattgt ttattttttt 114660
tagtttttttg agggtttggg tattaggaaa ggttttgggt ggagatatgg aaataggtta 114720
tgtgaagtta gtgggtgtga ttggtagtag taaggttagg atagttttgg ttattagtaa 114780
ggtgattata tattttagta tgtatagttt ttatagatat atatttagga gatggtaatt 114840
atattagtaa tagttatttt tgttgtgtgt tatgtttgta tttagggaat tttatttttt 114900
atttatttat ttattttttat ttgagatgga gttgtgtttt tgttgtttag gatagagtgt 114960
aatggtataa ttttggttta ttataatttt tgtttttttgg gtttaagtta ttttgtttta 115020
gtttttttgag tagttgggat tataggtatg tattattatg tttgattatt tttgtatttt 115080
tagtagagat ggggtttttt tattttggtt aggttggttt tgaattttttg attttaggtg 115140
atttatttat tttagttttt taaagtgtta ggattatagg tatgagttat tgtgtttggt 115200
tttaggggta ttttattatt ttattatttt ttatagtagg tgatattgag gatattgagg 115260
tttagagtag attgtggtta tgttattttt ttgtttggtt tttttgtttt tagttttgaa 115320
ataaagagta tggtttttgt gtaggtttat atgttttgtt tttagttttg gagtagtttt 115380
taagtttttt gaaggtatat taggttttttg ggttttgttt tttaagtttt ggataaatag 115440
gaggtgaagg agtttttgta attggatttt attttgttag tgttaaggtt ttgtttgatt 115500
atttatttta ttttatatag attagggatt tgtgttaagg tttagtgtta taggagagtg 115560

aagataagga ggtgtttatg gtgtatatgt tgttggattt attttgtaga ttttaagggt 115620
tatggggagt tatttgttag gtgaggtttt tttgtggttt ttttttgtag gtttagggta 115680
gtatataggg aggtgttttt ttattttttg gtattatttg tttggttttt ttgagttttg 115740
attttggttt ttagatagtt agtgtttttt tagggataga atttgttttg ttgttgttta 115800
gaggagattt agtttgatta gggttagtgt ttttggaatt tgagtttgga attttatttt 115860
gttttgggta gaggttatgt tgtttttttt tatgagagtt gagttgtgta tgagattatt 115920
ttgattttgg gttttattat ttttttgtat gatatttgtt ttaggttgga gtagggtttt 115980
gtggtttgtt tggtgttttt tttttagatt gaggtttgat atgtatatta ggaagtgttt 116040
gatttggttt taggagttgg gagtggtttg ttaggtagtt tgtggttaga gtaggggttt 116100
taataatggt tttttgtgtg gtatattttt ttttaaatag gtttaggttt aggttgtttt 116160
tgttttgatt ttgttatggg tttggagggg aaagtttgta tttggagggt ggtttattgt 116220
ggtttttttt tgtgtagtag ttgagattgt aggtgtttga gaataggaga agttttgtgt 116280
ttattttggg ttgtgagatg ttttttttaga ggtggggatg gggatgggtt agtatgtagt 116340
tatttatatt ttttagtttg ttggtattaa ggtttgtgta tgtgtgtgtt tttttttttt 116400
taattttgtt tttttaatat ggagttttgt tatttgttta ggttggtttt ggatttttgg 116460
tttaagtgat tttttagttt taggttttta aagtgttggg attataggtg tgagttatta 116520
tgtttagtta gtattaaggt gtgtttttta agagtaatat tggttgggtg tagtggttta 116580
tgtttgtaat tgtagtattt tgggaggttg aggtaggtgg attatgaggt tagtagattg 116640
agattatttt ggttaatatg gtgaaattgt gtttttatta aaaatataaa aaaattagtt 116700
agatgtggtg gttgggtatt tgtagtttta gttatttggg aggttgaggt aggagaatgg 116760
tgtgaatttg ggaggtggag tttgtggtga gttgagattg tgttattgta ttttagtttg 116820
ggtgatagag tgagattgtt ttaaaaaaaa aaaaaaaaaa gaataatatt gatggtttta 116880
tttttttgaat atgttaagtg atttttatgt agtataagta ttttagaagg taggaaaatg 116940
taatgaataa tatttattgt tatttatttt atttagagat tgttattttt tgtttgggtg 117000
tgagtgtgtg tgtgtgtgta aattgtatat gtgtgtatga aatggaatta ggattttttgt 117060
atatattttg tgttttgtta tttttattta atatttttgg tattttttat ttttaaaaat 117120
gtgattttaa tgatggtgta ggtttttgat atatgggttt agaaaatttg tggaaatttg 117180
gattgttttt aggttttgtt agtttaatgt tgatgtaaat gtttttgtat gtaaattttt 117240
ttgtattttt gattattttt ttgggataaa tttttagaag tgggttaaag tatatgaatg 117300
gtttttaggt ttttggagta agttgagatt ggtggggatt ggagaaggga tttagggagg 117360
tttttgggag tttgtgagat tggagtttgg ttttagggga tttgaattta tagatgaagg 117420
agatggttgg taaaggtggg aatttttttgt tttatgggtg ggggggtgat attgttttat 117480
tttttagtga tagtatgggg attttttttaa tatgatttgg ttttgtattt ttttgatatt 117540
gtgtatggtt tatagttgtg atgtttagtt gggagttttt tttgtgtttt ttagttgtat 117600
taattttttg tagatagagg aggaagttgt ttggtgtttt tttttgttgt gtgggttttt 117660
tgaatttgtt tagttgggtt tgtggatttg tgtagttatg tatggggttt taaaatttgt 117720
aaaatgataa gtttatgaat atttttgttt ttgtgggtga atgtggatat tttttggttg 117780
tgtttttttt tatagggaag atggagatgt gtaaagtgat aaggatttgt tatttttggg 117840
taggttatgt tggttttatt gtgaggatgg tggtaggggg tttttttagga ggtttgtata 117900
ttgggaagag aggggtgatt gtgtgtgatt gtaggattta gggtgtagtt ttaggatttt 117960
gatgggggag gagatgttgg ggagttttgt tttgttagtt tggtgataga tattttttaa 118020
tgtttttttgg ttgggtataa tttataatgt tttttgtggg gaggtaattg tttatgaggt 118080
atttttggta gggatgattt tgttttattg ttatatattt tagtaatgaa ataagaattt 118140
agagtgatgt gatttttttg atgatttttt taattatttg gggtttttgt gtgattttaa 118200
gaagggggaa ggaggtagtt tttgtttttt tgggttttttg atgtggtagt tttggaatgg 118260
ttatgtgttt gttggtttat agtgggttat ttgttgaatg tggttgtggt gggattgggg 118320
ttgggttgga gagtgggtga ttatattagt tagttttatt ttgggttatt gtgtagttag 118380
gggttttatt tttttagggg ttttgttgtg agtgttttgg ggttgttgga ataaattatt 118440
ggaaattaag tggtttaata aagattgatt tgtttatggt tttggaggtt ataagtttga 118500
tattaaggta ttggtaggat tatatttttt ttgatagttt gaggggagga tttttttttg 118560
ttttttttag tttgtgttgg tttaggtgtt ttttggtttt ggtagtattt gtagttttttg 118620
tttttattat gtagtttttt ttattgtgtt tttttgtgtt ttaagttttt ttttgttttt 118680
tttttataag gatattgagt attggattta gagtttattt taaatttagg atagttttat 118740
tttgggattt ttaattatat ttgtaaagat ttttttttta aataaggtta tatttgtggg 118800
tttttggggt taggtattag atatgttttt ttgggggtta ttagttaatt ttttatagaa 118860
ttttagatga aagtgaattt ttataggttg aaatgggggt gaaggtttag tgtgtttttt 118920
ttttttggga gttttttttag ggtttgtgtt tattttgtga ttttgtgatt tagttttttt 118980
tttgagatga agttttgttt tgttgtttag gttggagtat tgtggtatga tttgggttta 119040

ttgtaatttt tgttttttga atttaagtga ttttttttgtt ttagtttttt aagtagttgg 119100
gattataggt gtttattttt atgtttagtt aattttttgt gtttttagta gagatagggt 119160
tttattatgt tggttaggtt ggttttgaat ttttgatttt aggtgatttg tttgttttgg 119220
gtttgttttg gtttttttaaa gtgttgggat tataggtatg agttattatg tttggtattt 119280
agtaggtttt ggttgttttt tttttttgag atagagtttt atttgttat ttaggttgga 119340
gtgtagtggt gtgattatgg tttattgtag ttttgatttt ttggttttaa gtgatttttt 119400
tattttagtt ttttgagtag ttgggattat aggtatatgt tattattttt ggttaatttt 119460
tgtattgttt agtggagttg gggttttgtt atgttgttta ggtttgtttt gaatttttgg 119520
gtttaagtga tttgtttatt ttggtttttt aaagtgttgg ggttataggt gtgagttatt 119580
atgtttggtt tagtaggttt tttagaagtg tttgaggttt ggtgaaggta tggtgttatg 119640
gagtagagaa tatttaattt tgtttttggg gagttggaat atattttttg tagttaattt 119700
taatagttat gatgtggtta ggatgatatg gggagggggt ggtgtggatt gtgtgggagg 119760
ttagtttttt gggaaggggg ttggagtggg tttggttttg aggaggattt ttaagaggag 119820
gaggaggtgg tggtatgttt gtttgggggg atggtgtgag ttagggtggg tattttttta 119880
tttaggtgtt attttttttga tattttagtt gggatatggt tttttgggg aagatggata 119940
ttttagagt ggggtatttt ttggggaaga tggatatttt tggaatgggg tgtttttttgt 120000
tttattgtt tttttagtat atagttgtat ttttttgtag ttttggaggt tgtgagattt 120060
tttggttgtt atttagaata aatttgtttt tgagtagtgt tgttttgatg gtggggtggt 120120
ttttgtgggt ggaatagttt ttgttgtttt agagtttatt gagagttagt gggtatggga 120180
gtggtgttgg attgaaatat ttagagggat tatagagtta ggtagtaagt atagaggtta 120240
gttagtgggg agggagggat ttatttatga aaagatatta agtattgaaa gagaggagag 120300
gatggttggg gattggtata tatgtgtatt ttgtgtagat ggttaaggtg gtgaggtgtt 120360
agttatgtag ataattggag gaaggaggag tgttttaggg gaggggttag ttttggtttt 120420
agaatgattt gtttgtgtat gtgggtttgg ttggaataaa ttattgtatt tgtattgtag 120480
atttaattgt aagtttatat tagggtagtt tttgttttgg tgtagtgttt ttgattatga 120540
gttggttatg gtgggggtga ttttaagggg atttttaagg aggggggttta ttggattttt 120600
tagggaaggt tatttgtttt tttgggttgg atttgggata aggtttttttt ttgtttagtt 120660
tttgtttttt aatttgattg tatggaagga tgttgaggat atttggaagt tggtttttgt 120720
tttatagtat ttataggata gaggtatatt atgtggggat agtttggata gtgtttagga 120780
ggtagggagt agattagagg attgtgggag gttttgggaa ttgtatgagg atttaggtat 120840
tgagttttgt attttttggt attagtttga tgtatagttt ggttttgtgt ttattttgta 120900
ttttattttt tttatttgta tgggggtggt aataatattt tttatagttt aggtttaagt 120960
gggtgtagat gtgatgagtt tggtaagttt atttattat atttagtttt gggtattgaa 121020
ttttggattt tttggttggt agaagtggat atttaatttt tttttgggt tttggggttt 121080
ttgtagaatt agaatatagt tagtagtttg tatgtagggt gtagagggat agagggatat 121140
tatgttggga gtttatattt ggtatatttt tgattttggg gtttagtgtt ttgttgtttt 121200
gtggttatgt gattagttag aggttatggt tggatttata ggtgaatata gtttgtggat 121260
ttgattttgt ttggttttgt ggtgttttat tttattttta gtttttagttt gtggagatag 121320
agttttgttt tgttagttag gttgagtgta gtgatatgat tttagttttg taatttttgt 121380
tttttgggtt taagtgattt ttttgtttta gttttttgag tagttgggat tataggtgag 121440
tgttattatg tttagttaat ttttttatttt tagtaaagat ggggttttat tatattagtt 121500
aggttgttta gtgttttaaa aagggtttgt tttgttttgt gttagtattt aaagattagg 121560
agatattata taaagattta gatttttatt tttttgagaa gtgaatagtt ttggttatgt 121620
tgggtttata ttttttaggg tagtgatgga gttgagatag ggtttgtgat aattgagagt 121680
tttttggagt ttatatggtt ttttgtttag tttttggtgg aggttgagtt tagtgattta 121740
ggattttgtt tgtgtttatt aagaaggaga ggtgtttgtg gtttagagag ggtgtttata 121800
ggagtgtttt gtttaggaag ttggtagttg aggtggttag ggttaggggg ttgggttttt 121860
ttttttttt tttagtgttt gagtgtattt ggaggaagag agatgggtga ggggaaggta 121920
ttgaaattgg ttttggagtg ttattattgg gggttgggga ttagttgtta ttttttttt 121980
tgtgggtagg ggaggtagtg gtgggatttt atgagatttg gattttgtgg tttggtgagg 122040
tagggagtgg ttggggagat attgtgggtt tttataggg tgtgttgtgg gtttggtttg 122100
gggaaggagt tggtgaatgt gtttgttatg tgggtttttt tttgttttgg ttttgataat 122160
taggagtggt tggtttttat ttggagtgtg gttgtgggga ttgtggaggt ggtagttttt 122220
taggtattat ttttattgtt tttgagggag tagatttttgg tggagaggta ggatggttgt 122280
agggtgggag ttggtggttg gtattattgt atgggtttgt agtagtggat gggaagatgt 122340
aggagaggtt gatgattgag aaagttggtt tttgtggatt aagttggggg agttggataa 122400
ttaagtggtt agaattttgg gttttggtat tagtttgatg tatagtttgg ttttgtgttt 122460
atttgtgtt ttattttttt tatttgtatt gggggtattg gtattttta tattttaggg 122520

ttaagtggtt gtagatgtga tgagtttggt aggtttggtg ttttgttggt attgaaagtt 122580
ttagtgtttg tttttagtgt tattgatggg gttttgtttt tgttgtggtt ttgattggtt 122640
atagttgtgg ttatttatta ttggaggtat gttttttgtt gtggttgatg gtagaggtgt 122700
gtttgttggt ggtgttttag tgtgtgtttt ggttggaggt gaggagggat aaaaatagtt 122760
gtgataagaa ggaaattaag gttgggtatg gtggtgtatg tttgtaattt tagtattttg 122820
ggaggttgag gtgggtggat tgtttgaggt taggagtttg agattagatt gattaatatg 122880
gtgaaatttt attttatta aaaatataaa aattagttag gtatggtggt ttgtgtttat 122940
aattttagtt attggggagg ttgaggtagg agaattgttt gaatttggga ggtgttgttg 123000
tattttagtt agggtaatag agtaagattt tattttaaaa aaaaaaagaa ggaaataaaa 123060
gttttttttg gaattagaat gtttttatgt agatgtagtt ggaggggag gttgtgtggt 123120
ttgtgttgtg gttatggtaa gatatgatgg gtagtatttt tttattttag tttgtatttt 123180
tgttttagaa tttatgatgg ttttttagg atggggttgt ttttttgtt tgttgagata 123240
tgaaggatgg gaagagattt ttggttttta ttgttgtatt gtttgttttt ttttgttgtt 123300
ttttagtagg aaatatgttg gagtgttttt tagttattta tttattaatt gtattttttt 123360
tttttatttt ttagttttga aaagattttt tgaggttgag gaggttgaga tatttaattt 123420
tattttattt tggagggttt agattttttt attttgagtt attgtggtta gttttattta 123480
gaaattttag gatttgggtg tgaagaattt agaattttttg gtttgttatg tggatttttt 123540
aagttaatgt ttttttaagg tgtttttgtg gagggtggag tttttgggtt ttaaggtggt 123600
tgagttggtg ttttggtgta ttgagttgtt tattgatatt ttgtttaagt aggtggagaa 123660
tgttggggtt attggtttgt tttggtttgg gtttaagagg gttgaggtgt tgggttataa 123720
gatgttagaa ttggtttttt ggaggatgga gattattatt gttaaatttt aggagttagt 123780
ttattggagg atggagtttt ttgtttttaa ggtttttgag gtgtttattg tttttgttat 123840
tgatgtagtt tttaagaggg tggagattta gatgtttaag tttgttgagg tgtttattgt 123900
ttttagttta gtttagattt tggagaattt agagtttgtt tttgtgtttt agttgtagag 123960
taggttggag tttaagtttt agtttttttgt ggttgaggtt atattttgga gttaggaggg 124020
tgagttgtag agtgttaggt tttggatgtt gtggtaatgg ggggtttggt tgttggtttt 124080
gttatagaat tttggaggaa gaagttggat atggggtgga gggtgttatt ttggagattt 124140
agaaagattg gaatgtatgg gggtgggggt ttgtaagttt aggagaggtg gttttttttg 124200
tttgatggga atatgttaag atgttttaag tgggatttga atgagagttt ggaaagattt 124260
tttgggtttt aaggagtttt ttttatgggt tattgtgggt tttgttggga tttttgtttg 124320
ttgtgtgttt ttggagggtt tgggaggttt agagaatgag gagttatgta ggaattaggg 124380
taggtttatg taggggtgta ggatagggtg gagttgagat ttttagaagg gttgttatat 124440
tttggtggtt tttgtttgag gaattgtagg ttgtttggat atgtttagta gaggttgagg 124500
gtttggggaa gggaggaggg atgtatgttt tttgaggttt gtattggagt ttatggtttg 124560
gttttttttt tttaggaggg atggttgtgg tagagggagg ggaatatgag gagtatttta 124620
gtttgtagga taaaattttt agggtattgg gaaggggtgt ttggattagg gggttgtgtg 124680
atagttgtat atggttttat ttgggggtgt ttttttggtg agaggagaga ggttatgggg 124740
ttttagtagt gttttgggag ggtttaagtt tatagttgtg ttgatatgga gtttataggt 124800
aggggattgg tgttagtttt gtgggatata gggtttgttt ggaaatgtta ggttgtggga 124860
tatggttggg ttttttttta gaagtgtgtt ttttggtttt agtagggatt gtggttttat 124920
ggggttatag taaggtgtta gttgggttag aagtttttta agagttaggt ttgattttgg 124980
gtataggatt agaatttagt aatgtttagt aagttttttt taggttggat aaatagtata 125040
gtttgggttt ttgatttagt tgttgttttt tatttttaat tgtggtgaaa tatataatat 125100
ataatgtgta atttagtta ttttgtagtg tatgttttag tggtatttag tatatttatg 125160
gtgttgtgta gttgttattg ttgtttattt ttggtatttt ttttattttt ttaaattgag 125220
ttttgttttt gttagattaa gtttttatg ttttttttt agttgttggt agttattatt 125280
ttatttttg tttttattat gaatttattt gattatttta ggaattttgt gtgtgtggaa 125340
aaatataata attgtttatt ttgtgtttgg tttatttat ttagtatgat gttttaaagg 125400
tttatttgtt gtgttgtagt atggggtaga attttttttt ttttagaggt tgaataatat 125460
tttgttgtgt gatgtatttg tgttgtgtag ttgtttttt attggtggat ttttgggttg 125520
tttttatatt tttttaagtt ttttttttta tttttttttga tttttttttg atttttttga 125580
gatagggttt tgttttgttt tttaggttgg tgtgtagtgg tgtgattatt gtttattgta 125640
atttttgttt tttgggttta agtgattttt tagttttagt tttttaagta tttaggatta 125700
taggttgtgt gttattatgt ttggttaatt tttaaatttt ttttgtagag ataggttttt 125760
attaggttgt ttaggttggt tttaaatttt tgggtttaaa tgagtttttt gttttggttt 125820
tttaaagtgt tgggattata ggtatgagtt atttaatttt tttttggttt ttttttttga 125880
atgagagttt tttagtggtt agtgggtttg ggggttggga ggatgtgggg tttggagagg. 125940
gtgtgggttt ggggggagat tagtgttttt tatgttttta tgggtatgtg gagtgtatat 126000

```
ttgttttggt gggtttgggg tttgggattt tgtatttttt atagaggggg tattggaata 126060
tattgagtag ttagaggtta gtgggttttg gaaggttttt tttggttttt agtgtgtgag 126120
ttttgtgttt ttttttttat ttttttaagt ttgattttga agagttattt tgttttagtt 126180
atttagattt agtgatagtg gttgttggtg gtatggtgtt aggttaaagg tatgggtagt 126240
ttgtagagtg gtaggaggtt gttttttttt ttgttttttg ttggtgtagg atttttgttt 126300
gatgtgttgg atatattttt attttgggtt gatgtgttta tatttagttg agttaaatag 126360
gatgtggttg gggaggtggt tgttattgag ttatttaaat tttggtgatg gttggtgttg 126420
gatgtatagg gatgtggttt tggttttggg ggataggtag ggggatgttt atggggatgt 126480
ataagaatat tttttttttag gggtagatat agtttagttt ttaaattgtg ttagagattg 126540
tgttgggagt taggtttagg gatataattt gtgggttttg ttttttggag tttattgagt 126600
tgtgagtagg atggttggga tataaggagt gtttagggag tttttttagt agtggaagta 126660
taattttatt aggttaggaa ggggagagaa aggtaggtta tgtggaggta gtagtgttta 126720
gagttgttag gaatttggtg ttttgggggg atggtggttt tttttatgga ataggatgta 126780
tatgttggag ttggggtatg ggagaaaatt attagtattt tagatttggt tatttatgga 126840
tagggatttg tgtaggtggg agtttagttt atgtggagag gtttgtgggt tgggttgata 126900
gttttgaggg tttaagtaag tttaggtgga ggaaatgtag ggatagatga gggtagggtt 126960
tttttaatgg aggagtgttt ttgaaatttg atgggagtgg ttttttgggg ggtttaggtt 127020
tgagttttat tagttttttt tttgaggtat tatttgtttg tttttttgtt tttttttttt 127080
gtgttatgat tgttttggat atggatttag tttttttttaa tttagttttt tgtttatttt 127140
tttttttaggt ttatttttttt ttgttttttt atgtggggtt gggttgggag tttttttgagt 127200
tttgatatgt atagttttga ttaattttttg tggtttttttt gtttttttgag tattagggat 127260
ttagttggta ttatggggaa aggaattatg gagggagttt tgtgtttttt gagtagatgt 127320
ttagtttttt ggttttttgtt tgtgggaggg tatttgtgtt gtggtttgtt tttattttag 127380
agttgtgggt gtagggtagg gttttttggg gttttagttg aattgttttt agattgattt 127440
ttttaaagta tagtattgtt gagattttttt tttgtgttga gttttttttg tttatttatt 127500
ataggttttt ttatggttga tttatttttt tgtttagttt atattttttt ttttttttagt 127560
tttttttgtt ttggtttttt tttggttttt tatatttttt tttataattt ttttgttttg 127620
atttttttgaa ttttgatttt tgtttttttt tatattattg ttttggtgaa tttttatgta 127680
tttttttaaaa tttggtttaa atattttgtg tttggtttgg gatataaatg atttgtgtat 127740
aaattttttt tttttttgtta gatttttttt tttaaatgtg aaagtagttt tttagattat 127800
aaaaagtaat tgagtatttt tttttaaaa tgtaggaagt atagaaatga gaaagaattt 127860
aaaatttatt tgtaagttag tgatatttat gtttgaggtt ttgttgtata gtttttaaaa 127920
ttttttttat gtttttatta gattttgagg gtagggttgt tgtttttgtt tttttttgtg 127980
ttttttttt gttttttgtt ttttgagatg gagttttttt ttattattta ggttggagtg 128040
tagtggtatg attttagttt attgtaattt ttgtttttta ggtttaagtg atttttttgt 128100
tttagttttt tgagtagttg ggattatagg tgtgggttat tatgtttggt taatttttgt 128160
atttttagta gagatggggt tttattatat aggttaggtt ggttttgaat ttttgatttt 128220
gtgatttatt tattttagtt tttttgattt tttaaagtgt tgggattatg ttattgtgtt 128280
tagtttgttt tggtttttat aaagaatttt ataaggggtt aggtgtagtg gtttatgttt 128340
gtaaatttag tattttggag gttgaagtag gtagattgtt tgagtttagg agtttgggat 128400
tagtttgggt aatatagaga aattttattt ttataaaaaa tataaaaaat tagttaggta 128460
tgatagtatg tgtttgtagt tttagttatt ggggaggttg aggtgggagg attatttaag 128520
tatgggaggt tgaggttgtg gtgagttgag attgtgttat tgtattttag tttaggagat 128580
agagtgagat tttgttttaa aaaaaaaaaa aaaaaaaaaa aaaattttat aagatattaa 128640
gtaagtgatt tggatataat aggtgtgtta tgaaattatg ttgaaggagt gagttattgg 128700
tggtgagttg ggatggtttt tttgtggttt ttgtaggtag atgtagtgat ttttatgggt 128760
tgtttttaga gattaagtag aaattttttt ggttaatttt atttttagga tatttggttt 128820
tttttttgga ggttgtgttg gagggtttag tgggtgggtg ttggttttag tgtggggggtt 128880
ttggaaggag ttagtatttt aggagggagt gtgggtattt agatggttga aggggaggga 128940
gaggaagaga ggtgttgtag agggtagagg tatttttgtt tgttttttttg ttttagggtt 129000
tggtgaggtt tgagttttat gttggtgggg tggattttttg gtttttgagg ttttggtttt 129060
gtttttggat ttggtatttt ttttgttgtt tttttataga agggtgttta ttttttgggg 129120
ttatttttgg tttttttagg ttttgggtgg ggatggtttt gttttttttt ttggattttt 129180
gttttttttt tgttttttaa tatgggtgtt tttgggtgga gtggggtggg gttgggttgt 129240
agtatttagt attgagtttg taagggttgg ttgttggttt agagtttttt ttattagagt 129300
tatttttttt tggtttttta gttatattag ttaagttttt ttagagttgt tttataaggt 129360
gggtgtttag tgtttgttta tgggtttggt tttttgtttt tttgggatgt ttattggggg 129420
taagagggag gttaggttta gtgtttagtt tagttttggg tatgttttag ttttgtttta 129480
```

tgagttaatt ggttttgaat tattatttta tttttttag ttttaatttt ttgatttgtt 129540
aaagaggtat ttgtagaggt ggttgggggt tgagttttta tgtatttatt tattttaaga 129600
attaagaagg gagtttatag tggttttggg gttatttgtg aatatggatg tgaaattggg 129660
gtggggtaga tatggtagta tgttttagta gttttttgt gatttagagg gtttggaaat 129720
ttttgttatg tgggtggagt gttgagggag tagggggtta ggtttttttg agttgatgat 129780
ggtgggtgtg gtttttgta tatttggttt gtattgggtt gtgtgtttta ggggtagaat 129840
agggaggaat tttagtatag atagtagggt tgttagtata gtgttgagta tagggttaat 129900
taaagtatag ggtttttgg aattatttaa tatagagtag tgaagaggtg gttttgggga 129960
aagtggggtt agagttgagt ttgagtgggg ggtggggagg gtgggtatta gtttgtaagg 130020
gagggatatt tattgggttt tagaagtttt tggtggggat tagggattgt gaagttgggg 130080
agagggtgat tattgtgatt tagggaggaa gggttttata gatagtttag tttttggttt 130140
tgtgtttata ggatatttta tataggtttt gagtttattt ttggattgtg gattttttga 130200
ggtagggtgt gtaggtttgt ttagtattag gtgtaaggta ggggtttttg gtaagtgttg 130260
agggatttat tggtgtttga tgaagtagtg agtttggatt gtgttatgtt ttgggttttg 130320
agtttgtagg atttttttgt ggggttgtgt gtttttgttt ttggaggtgt ttagaaggta 130380
ttgatgtttt tgttgggatg ttttatttgg aaggttttgt tgttttattt tttttttatt 130440
tttttgagtt gtttgtagag ggtatttagg tttttttta ttttgggatt gggtttttt 130500
ttttttttt ttttaagaat aagtattttt ttttttgaa ttgtttttgg agtttttttag 130560
atttaggttt agagtgggaa gttttgtatg tgttggggga ataggaatta atgtgggagg 130620
attggttagg tttggtgtgt ttttttgtg gtgggatggg gtggggttag agaatagagt 130680
ttggttttag agtttaggaa gagaattttt ttttttgttg gtttgttaag tattgtgtag 130740
tggtgagagg aaaggagggt tggtttggga atgtagtggg tgttgagtag ttgtggaata 130800
ttttaaatag agtgtagttg tggggaaggg tggggttagg aggtaggtgt tgggggtggt 130860
tttttgtttt tgttttttgt agttttgtgg ttttgattat ttagtttggt tttaggatgg 130920
agttttggtt tttagatata tggtattggg tgaggttgtt ttttagtatt tgttttagga 130980
agttagagta gttgttagaa gagagaggag ttgtgtttgt atttgttgtt ttttaggttt 131040
gggatagttg ttgggattga gggtttgtat aatggtgagt ttgtttggag gggtgtgttg 131100
tggttagata gttatgtttt gtgtggtgtg gatagagggt atatttagtt ttggaggagt 131160
gtatttagat tttttgtttt ggggtttaaa gtgtgggtag tttttaatta gattttggat 131220
tttagtagat agtttgggag gatgttatgt ggtttatttt gatatattgt tttatttgtg 131280
atttatttt tgagagttgt agggaggtgt ttttttagtt ggattttatt tttttttgg 131340
ggttttttgt gggtgttata agtgatttttg ttttttttag tttaaggggg gtttttgggg 131400
tatgggttgt aattgatagg ttagagtagt ttatggttag ggttgagttt tttttttgtt 131460
tttttgaat attgggttta ggaaggggtt aaagtggaga tgttttgatt aaagttgagg 131520
gaggtatgga gttttttga ttttgttttt ttattttatt gttgttattg ggtgtatttt 131580
gtggagtttt aggattttta aagaatttag agtggagatt tttggtttta tttagtgtag 131640
gtgaggggga aagtgatttg tttatagtta tatggttatt attagtatat ttgggttaag 131700
tttgatttta ttttatggtt tttgtttgaa atttggaatt ggaaatggtt ttggaaatta 131760
ttgattttat ttgttgtttt ttaaaatggt ttgtagaggt taagatatag atttttgtaa 131820
tgttttgttt atttgtgtgt ggggaaatgg tagaggagta ttggttttgt ttttatattg 131880
tttgggttgt tgtttttgtt ttgtttagtg gtttttttt ttggtttgtt tttttattgt 131940
ggaagtgggt ataattttt ttgtgtagtg tggttagagt ggatagtgat gatttgtgaa 132000
gtagggtgga gtgtttgggg gatttgtgtt aaataagtat ataaataaaa gttaatataa 132060
tttttattta attagtaagt aggttttgtt tgaagttttt gggtagtatt ttatgttatt 132120
tttaggtttt aggttggtgg tgttgttttg tgtttttttt atttgtaagt tggtgtttag 132180
aagttttgtt ttgtgttttg gtttttattt tttttgtgat tttttttttt ttttatttta 132240
aaaagtaatg tatgatgggg tgtggtggtg ggtatttgta gttttagttg tttgggaggt 132300
tgaggtagga gaattatttg aatttaggag gtggaggttg tagtgagttg agattgtgtt 132360
agtgtagttt agtttgggta atagaataag atttttattt gggaaaaaaa aaaaaaagtg 132420
atgtttagtt atttttagat agaaaatgaa gattatatga aatttggaaa gaagtttttt 132480
tttttattt tttaattttt ttttaagggg tggttatgtt gatagttggg tgtggtattt 132540
ttttgaattt tatttttgta gagttattta gggtttgtgt aaaaaaagtt aatgtgtaaa 132600
tatagatttt ttaagaatat aaaggaaatt agatttttgt tattagtttt tattttttt 132660
atttaataat attgttgtta tttgtagatg tagtatgttt tttttttttt tttttttttt 132720
tttttttga tagagtgtgg tttaggttgg agtgtagtgg tgtgatttta gtttattgtg 132780
gtttattgta atttttattt tttgtttttt gtttttaagt gatttttttg ttttagtttt 132840
ttaagtagtt gaaattatag gtgtttgtta ttatatttgg ttaattttg tatttttagt 132900
agagatgggg ttttattatg ttggttaggt tgtttttgaa tttttgattt tgagatttgt 132960

ttattttggt tttttaaagt agtatgtttt ttttaatggt tgtgttatta gttgttttat 133020
ggttttgtta ggtttgttta gtttttttatt gtgattgaga ggttgtaata gatgtttttg 133080
tatttgtata tgtgtatata tgattgaagg atgagttttt agtgaatttt tgtgttaagg 133140
atgtgtgtat ttaagttttg atttgttggg tatagtggtt tatatttgta attttagtat 133200
tttgggaggt taaggtgggt tgattatttg aggttaggag tttaagatta gtttggttaa 133260
tatggtgaaa ttttattttt attaaaaata taaaaattag ttgggtatgg tggtttgtgt 133320
ttgtagtttt agttatttag gaggttaagg taggagattt gtttgaattt agtaggtaga 133380
ggttgtagtg agttaagatt gtgttattgt attttagttt gggtgataga gtgagatttt 133440
ttattaaaaa aaaaaaaaaa aaaaaaaaat tgatttttgt ttttgaattg gtttttaaag 133500
tggttattag aatttttttag gatttttttt tttgttgatt tgtttagttg tttgttgggt 133560
tttgtgtagt gatggtttag tagggaggtt ttatgttttt tatttttggt tgagggttaa 133620
atgtttttta attggtgttt tattttgtgt attttggtta agtgagaggg attatttttt 133680
tgtatgttat aggttagggt tttgtgtttt tttttttattt ggaaggtgta tttttttttt 133740
gttgaaattt tttaaaaaga gtttttttgta agttaggtgt gatggttaat atttataatt 133800
ttagtatttt gggaggttga agtgggtgga ttgtttgagt ttaggagttt gagattagtt 133860
taggtaatat ggtaaaattg tatttttgta aaaattagtt gggtgaggtg gtgtgtttgt 133920
agttttggtt atttgggagg ttgatgtggg aggattattt gagtttggga gtttgaggtt 133980
gttgtgagtt gtggttatat tattgtattt tagtttgggt gatagagtga gattttgttt 134040
aaaaaaaaaa aagagttatt tgtatatttt tattatagta ggtatgagta ttttgttttt 134100
agttgtttat ttttgatttt gtttttggga tgtttttttta tatagaatat tttaattttg 134160
atattgttaa gtttttgttt tttttttttag tttttggaat ttatattttg tttagaggtt 134220
ttttgtgttt tgaaattata ggaaagaatt ttatttatgt ttttttttag tatttttgtg 134280
gttttatttt tatatgttga aattatgaat ttattggaat gtattttggt tttagtagtg 134340
aggtaggggt agatggtatt gtttggatat atagtagtga gtttggtttg gagtgggtgg 134400
ggtgtgtgtg tgtgtgtgtg tgtgtgtgtg tgtgtgtgtt atttttatta gttttggtgt 134460
ttgtattgtg ttggtttagt aaaaataatt tggatgtatt ttttttattt tttgtttttt 134520
ggaatatttt aagtagtaat ataataattt ttttgttaaa atttttttaag aatttatttg 134580
tgaaattatt tgagtttgat atttttgggg gagtttagta ttttaaaaat ttaaattttt 134640
tttatgggtt tgtgagtttg ggagtttttt tttttttttta aaatttatta ttattttttt 134700
tttagaaaag tgtttttttag tttatttaaa atttgttagt ttattttgag ggggttgttt 134760
tagtttgttt agtgaggata gtgtgtagtg tggtgattga tatgattgat tagtagagta 134820
tgattgatta gttggtgtgt aaatatgatt gattgtggtt agtgttttgg gatgttttat 134880
gttgaggtat atgtgtgtag tgtagggtga tgtgtatttt gagaggggat gaagttatag 134940
gatttttgta tttttaggag agagtttttt tttttaaggt tgtagttttg agtaggtagt 135000
gatgggattg ggtttagggg gttgagtggt tttagtaggt taagggtttt aggaattatg 135060
agaagtttgg agttgtttag agagtagttt ttaaaattag tttggattgt agtttttaga 135120
aagatatgtg attgatatta tgaattagga tatatttata tatggggtgg gggtggggat 135180
gggatgtatt aatgaaatta aatttttgtg atggaatttt tatttttatt atgtgtgtgt 135240
tatatttagt attttttttt tttttttttt tttttttttt tttttttttt ttttttgttt 135300
gtttgttttg gagagaggga aggttttttt ttattttttt ttaaatagtt ttattgagat 135360
ttaagtaagt tattatataa tttattaatt tatttattta tttatttttt tgagatggag 135420
ttttattttt gttgtttagg ttggagtgta..gtggtgttat tttggttgat tgtaattttt 135480
gtttttttggg tttaattaat ttttttgttt tagtttttttg agtagttgag attatagata 135540
tgtattatta tgtttggtta attttgtatt tttagtagag atgggttttt tttatgttga 135600
ggttggtttt gaatttttga ttttaggtga tttatttgtt ttagtttttt aaagtgttgg 135660
gattataggt atgagttatt gtgtggttta atttatttat ttaaagtgta taattttatg 135720
gtttttagtt tatttagaga gtagtgtaat tattgttatt ataattaatt ttagaaaatt 135780
gaaaatttga gaaatttaga aaattttttt tttaagaaga aattttgtag tttttagtta 135840
ttaattttgt tttttttttt tatatttttt ttttatttgt gtggattttt tttttttttt 135900
tttttttttt tgagataggg ttttattttg ttatttaggt tggggtgtaa tggtttaatt 135960
tttgtttatt gtaattttta ttttttaggt ttaagttatt tttttattat ggtttttttga 136020
gtagttgaga ttataggttt gtgttattat gtttggttaa tttttgtatt ttttgtagag 136080
atgggttttt attatgttgt ttaggttggt tttgaatttt tgggtttaaa tgattttttt 136140
gttttggttt ttttaaagttt tggaattata ggtgtgagtt attgtgtttg gttgatttta 136200
gatatttttt gtatagagaa ttgtatgttt ggtttttgtg tttggttttt ttatttggtg 136260
ttatgttttt aaggtttatt tatattgtgg taggtgttag tgtgtttttg agtaatattt 136320
tattatggta tggattattt tattgtggat agatatgttg tttttattag ttgagggata 136380
ttgggttgtt gttattgttg gtgagtgtat tttttttaaa ttaaaaaaaa aatgttggtt 136440

attatttatt agattgtttt tttggtttat agggtgggat aagttggttt gggtgagggt 136500
taggaggtgg ttaggtggag gtggttggag tggttttggg ttgtttttgg gtgtagtagg 136560
tagaggtgta atggtagagt ttgagtaggt tttttgggtg gatttttatg ttggtaatgt 136620
ttgtttattt aagaagtttt ggttaggtgt ggtgttttat atttgtaatt ttagtatttt 136680
gggaagttga ggtaggtaga ttatttgagg ttaggagttt gagattagtt tggttaatat 136740
ggtaaaattt tgtttttatt aaaaatataa aaattagttg ggtgtggtgg tgtgtatttg 136800
tagttttagt tatttggtag gttgaggtag gagaatttgt ttgattttag gaggtagagt 136860
ttgaatttag gaggtggagg ttgtagtgag ttaaaattgt gttattgtat tttagtttgg 136920
gtgatagagt gagattatgt tttaaaaaaa aaaaaaaaaa gaaaaaaaaa gaaattttta 136980
gtgttgtttt gtgtttggta ttgaagttga gggaaggtag tattagtatg agtttttagt 137040
ttattgggtt tttgtttgga gttgatgtgt gattaaggta tatgagggaa gtatggtgag 137100
gtggatggtg tggaggtata aggagtaaaa taaggtagat gtagtggttt agtggttttt 137160
ggtggttgtg aggagtgtgg gtaggatagt tttaggaggg tatttggaga gtagggttgg 137220
ttggggtaag gtgtgttagg tatttgttag gagtaaaatg taaggggata ttaaagagtt 137280
taggaattaa tatagatagt atttaatgta gtattttta atattgaaat gatgtatagt 137340
aatttttatg atgaataaat gttggaattt taaattaaga taggtttagt tttgtatgtg 137400
tatagttttg ttagagtttg tttagtgtgg aatttgattt gtttaaaatg ttgttttaaa 137460
atattgtttg atttttggat tttttggtat tgttttaatt tttgtatttg gatgaaggtt 137520
ttattttaat tttagttgga gagggttaag ggttggtgaa tttggttttg gatttgtaga 137580
gtttgtggta tttgagaggt ggggttagga ataggaagga gttggtgaga atggtatttg 137640
gtatgggatg gggttagatt agagtagtat tgtggttttt tttaatttgt ggttttgttg 137700
ttggatgttt ggtgttggtg ttgagttttt ggttttttga atggagtgtg atgaataatg 137760
gggttgggga gtttttgggt tttttttga agtagtagtg aagttaggga tatgttggta 137820
gtaggtaggg agagggatgt ttggtttgtg tttggagtta gttgttttat ggaggagttg 137880
ggtgaggatg tggtgggagg aagatttttt tagttttgta tttttgaag tataattgag 137940
tgtttgtgtg gtttgggggt tattttggtt tgaggatagt tttgttttgt ttagttaggt 138000
tttgaagagt tattgggata tggatagtag ttttgttggg tggtaggttg tttattttg 138060
tttatttaat gttggaattg gatgtagagt atttggatgg ttagttttag agtgtttttg 138120
atatttttag ttattttatt ttttagatta ggataagatg tgggatttta atttgagtt 138180
ttttatgggt tttattttat gtgtttttat aagttattgg gtttattttt tggttttagg 138240
gttgtttggt tatttggtat tttggttttt ttatttgtat aatgaaaata atagttttag 138300
tttttttgag gagttataag atgtttttg tgtgaaaaat tgggatttgg tattagatta 138360
tttggatttg aattttggtt ttattttttt taatttgtgg ttttgggtta atttggtttt 138420
tttgttttta gtttttttat ttatagagtt attttattga gttgttggga ggatgaatat 138480
aaggaagtgt atttatgtgt ttagagtatt gtgattttat ttattattat taaatgaggt 138540
atataataag tgtttaatag atgtttagtg ttattttatg gattttgtgt gttttgtgtg 138600
ttttagtag ttaagttttg gaggtgggga agttgttgat gagttgtatg aggtaattag 138660
aaagaggagg ttataggtgt aagggtttgg gtgatatttg tggatagaga gaggttgatg 138720
gattttggta gaggggaagg gggtttttgt atatagaaaa tgtttttgt atttaagatt 138780
tttgtgattt tggaatagag gtagtagagg tttgtaaatt ttaaattata aatttagaaa 138840
gtatagaata tttgtagaga ggattagaaa tattatatat tttagtgtgt atagaatttt 138900
ttatatagtt aaataaaagt atgtttttaa ttgaagttgt tggagagttt ttgaggattt 138960
tatattagat aagggttggt aagtggaagt ttaatatgta ggagttattt tttttgttta 139020
taaatgtttg ttgttttttt tgtttatatt tgtatttatg tgagggtatt tataggttta 139080
ttgtttagtt ttatgttttg tttgttatta aaaaattttt taagaatttt ttagttgtaa 139140
atttttttat tggagggggg gttttaggaa tttagttttg tttggggttg gggtatttta 139200
gtatttgttg tataggtgtt ggttattaaa ggttatgtgt tgtatttgtg ggtttttggg 139260
gtttggtggt atttgggtat atagtttagt ggtggttttt tatttgttag atattgggag 139320
tgggggggttt tttattttttg tttttggtg gtggggggag tgttttgagt tgttatatat 139380
tagaatttat tgatttatat gttgtggtat tttatagttt tttgatagtt ttttagtttg 139440
agagattatt aaattgagtt tttggagagg gtggttgttg aggtttggat ataggtgtgg 139500
ataggatagt tttgttttat tttggagttt tgttttggtt tagtttttttg tgggtttgaa 139560
gtttaggttg ggttagtttt tgttttttag atggttggaa aggtagttta gttttatggt 139620
tttttgtta ggttttgaga gttgtttggt ttttagtttt gttttttttt tttttaaga 139680
tggagtttta ttttgttgtt taggttggag tgtagtggtg ttattttggt ttattgtaat 139740
ttttattttt tgggtttaag taattttttt gttttagttt tttgagtagt tgggattata 139800
ggtgtgtgtt attatggtag gttagttttt gtatttttag tagggattgg tttttattat 139860
gttggttagg ttggttttga atttttgatt ttaagtgatt tgtttatttt ggttttttaa 139920

agtgttggga ttataggtgt gagttgttat gtttagtttt tagttttgtt ttgatttttt 139980
tttaaggttg tgaggatgtt tatgtttttt ttttttttta tggtttattg atgtttttag 140040
atgttttgta aagttatttt ttttttggga gttttttagt tggtgatggg ggattttttg 140100
gggattattg tttaggtagt tatgtaggtt tgtgggttgt gtttttaggt tggattgaga 140160
tttggtgtat ggggtgtttt tggtttgagt tgaggttttt ggaagttata gttaggaggt 140220
agtaagggat ggttgttatg tttttttttt tttgtttttt agggttttttg tgggatagat 140280
ttttttgttt gagtttttttg gttgtttata ttttggtttt ttaaagttta gtttgaagtt 140340
ttaggtattt tataatttgt ttttttagt agtttgtttt gttttttttt tgttgatgtt 140400
tttgaaattt ttttgttttt ttttattttt tggttagaga gagattggag attttgtggg 140460
aatttttgag gggagggaga aggttgttgt ttgaatggag gtggtttttt tttttgtttt 140520
tttttaggtg ggttatattt gtaggtttag ttttttgttg tggtagtgtt tggggtgttg 140580
gttgtttttt gggtatgagt tgggagttga gggaggtgtt tttaggattg gagtggtaat 140640
ttagttggag gaggagtaat aggtttgtgg ttagtagtag ttgttgtagg gattttggtg 140700
ttgtatttgt gttttagttg atttttttttg tttttttagg gtgggttggg gatggtggtt 140760
tgttgtttag tttaggaaga agaggggttg taattagtag tagttatttt aggggatttt 140820
agtgttgtat ttgtgattta gttgattttt tttttttttt tagggttggt tgggaatggt 140880
ggtttgttgt ttagtttagg aggaagaggg gttgtggttt ttatttttaga ggtttttttt 140940
ttggtttttt ttttggtgtt ggtttaattg taggtttgtt ttaggttgtg ttggtatttg 141000
ttgattgatg tttagtggat gttgatagga gagggttggg atggggaagt agtaagtttt 141060
ggttattgtg ggtttgggtt atagatggtt gaggtatttt ttggttattt ttaggaagta 141120
aggttttatg tagtttttga tttatttatt gtttgtgtgt atttaggag gttgtatttt 141180
ttaaggtttt tattaggtaa ttttattggg ttgggtgttg ttgtaaaatt atttattggg 141240
tgtggtggtt ttttggtggg gggtatggtt ggggaggatg tggtggagga gaagttagtt 141300
ttgtggttgg ggggagggtg gattatttgt tttttgtttg ttttttgaat tggagtttat 141360
ggtttttttt ttaggtgttt gggttatgga agaggttata gttttgtttt attttttgtgg 141420
tagtttaggg gagttttggt tagttttgtg tttttttggga gggtgatagt ttggttaggt 141480
tgtagggtag gattggaatg tagattttttg tttggttatg ttgttgtttg gttgttttgt 141540
ttggggattt tttgtttagg aagagagaag tgggttggat agtttggttt agtgattgga 141600
agtttagttt-tttatgttta gtttagtagg aagtttgttt ttttggaata tttgtgtgtt 141660
tttgtatgtg tttttttggt tggtggtttt gtgaggtttt tattgggttg tgttatgttt 141720
tttggttatt ttggttttgg tgtttggttt ttgtgaaata gagatatttg ggtagttttt 141780
tatttgtgtt tgtaagtttg tgtggtttta gtgatggttg atttgtttt atttagagga 141840
tttgggaatt tttagtttaa ttgtttagag gagttttttt tagtgggggag agtttgagtt 141900
gtttttttgg tggatagttt tttagatttt aggtggattt ttttgttggt tgtttttttt 141960
gttgttttttg tttttttgtt ttttatttgt tttttatttt tttttttgta gtagttagtt 142020
ggtatgggga tttttttttt tttttgggat tgtgaatgag atgatttttt tggttttttt 142080
gggtttagat ttttttggta atagttagtg aaatttttgt tttaggtggg aggagaaggt 142140
ttagtatgtg gttataataa tggttttaat tatattattt ttaaatgttg gtttttattt 142200
attttttgtt ttgtttaata gttattttta ttattttatt ttttagatta ggaaattaag 142260
gtgtagagag ttttagttat ttgtttgagg tttttatagt taagtgtttg gatttgaatt 142320
ttggagtagt ttggttgttg agttgttttg gagatggggt attgtgtagt ggttggggta 142380
ggggtggagt tttgttgttg ttgatttttg ggttatttgg agtgataata tagtttagtg 142440
tttgggtttt tgtttgagat tttggttgga tgttaggagt ttgagatttt ttagtttttt 142500
tttgtgtaag gtgttatggg gtgaaatgaa agaaaatttt agttagaagg tgtttttttt 142560
tttttttagt tttttagttg ttttggattt tgaaataaga tattgtttgt ttgtggggtg 142620
agtaggaata gattggaatt gtattttttt tttttttagtt atagttgtta ggagtttatt 142680
tttttttgtt tttttttttt gttgtttttt tttataagat tgggttggat gttgttggga 142740
aagttaatag gaagtttttt ggttgtggtg gaggtcgggg tttgtgtttt tttggggatt 142800
gagatgaggg ttttaggttg gttttggtaa gagtagggag gtttatgttt ttgtttgttg 142860
gttttgtttt ttgttttggg agaaggtatt tatatgtttt agaatgtatt ttttgtgtgt 142920
atagggatgg tttttgggtt atttatttga ggtttattga tttttttttt tggaattgga 142980
gtggtttttt tatggtggat tgggttgagt tttttgtttt ggttgatgtt tttttttagg 143040
tattttttttt tattgtgtta gggtgtaggt tgttagtgga tgggttttta tttttgggga 143100
ttggtagttg ttatgtttat tttatagttg agatattttg gttgaaggtt ttgttaagtt 143160
ttatggagtg tagggatttt ttttgagatg gttttttttt gttgtttagg ttggagttta 143220
atggtttaat gttttgttta ttgtgatttt gatttttttag gtttagtagt ttttttattt 143280
tagtttatta agtagttggg attataggtg tgtgttatta tgtttggtta atttttttaat 143340
tttaattttt ttttgtagaa atggagtttt attatatttt ttaggttgaa ttttaatttt 143400

tgggtttaaa tgattttttt gttttggttt gttaaagtgt tgggatttta ggtatgagtt 143460
attgtgttta gttggggttt ttttttaatt ttttttggtt ttttttaggt gtaggatttg 143520
ggttttagtt ttatgtgttt ttgaatagtt ttgggttttg ttttgtattt tttgggtgtg 143580
tggttaggtt ttttgtgata ttagtaggag atttaggtga agaagtaggt agaatggaat 143640
gtttgtttat ttagttttgg ggttaggtgg ggtggttagg atagtagtag tggttggtgt 143700
ggtttttgg gaggttagtt tattggtgtg gttagtgaat ggggagggga gttttttttt 143760
ttgtttttt ttatttttgg tttttgggtt tttagggtta ggtttttaga gttttaggtg 143820
gaggatggag gttagggaag ttggggtaag agtgggttgg gggtgggtgg ttaggtggat 143880
tttggaaaat tatttttgt aggttttgtt ttttttaggg agtatttagg gttggttttg 143940
gggagttggg ttaatggtta ttttggatgg tgtaggggtt tatttttatg aaattgggtt 144000
tatttttatg aaattgggtt tatagaagtt tttggttttg gttttgtttt gtttttggt 144060
ttttgttttt aaagtaggat ttaggatagt tttgaatttt ttgttgaaat gtagtttttt 144120
gggttttaat ttagtttttt agaattagag tttttggggt gggtttttg tttgtttagt 144180
agttttagg ggatatttgt gtttgttaga gtaagtttaa taagtattgt tgtaaatttt 144240
ttgtttttat agtttttgg tttgtttttt ttttttgtt gggatttgta ttaggattaa 144300
atgttaatat tagggattag gttatatttg tgaattttga gataatattt gtattttttt 144360
tttttttgag gttgttttgt ttgttttgtt atttaggttg aagtataatg gtgtgatatt 144420
tatttatggt aatttttatt tttagggttt aagtaatttt tttgttttag tttttttgagt 144480
agttgggatt ataggtttat attattatat ttggttaatt tttgtatttt tagtagagat 144540
ggagttttat tatgttggtt aggttggttt tgaattttg gttttaagtg atttgtttgt 144600
tttagttttt taaagtgttg ggattatagg tgtgagttat tgtgtttggt ttgatattta 144660
tattttgatt aagttgttgt ggaaatattg agttttttt gatagttttg tttatagagt 144720
gaatgtttag gaattggtga ggtatttttt atttttattt agatggtggt ttggaggtag 144780
tgtttttgat tttgtgatgg gatttgtttt ttttgtttaa gagattttag agatgtggtt 144840
tttgaatgtg ggggttttg ttggggtggg aatgggagtt ggttttggtt attgtttagt 144900
gatttattt tagtgttttg attttagtat ttttgatttt agtgtttttt tttttttggt 144960
tttgtgtgga gattatgtgg tttgtgttga gtaatttttt attgttttg ttgtgttttt 145020
gttgtaggga agtttttta gggagttagt tgatatattt ggtttttatt atttgtttt 145080
gaataggaat gaaaggagag agtgagtaag aaagtggtag gaggtggggg ttgtagagta 145140
ggggaaggtt agagagaggg gaggaaagat ttggaaagtt taggtatgtg ttttggaagt 145200
aggtgttagt gatgagttta tttggagggt ggttgtttgt tttgagtttt gtttggggtt 145260
agtagggaat atttgttag ggtatgttgt tgggagtttg tttttggttt attttttgtt 145320
gttttgagtg ttgagtagat ttgtttttat tttagggata atgtaaggag gttgttattt 145380
tattttttat tttattaata ttttatgaat tataagtgat tatttattgt gttttgatgt 145440
attttaggtg ttgggtttga agttttatgt gttttatggg atgtatttgt attattgttg 145500
tagaaaatgg ttttttgttat tattgtagag aaggtattga ggtatagaga ggttgaggaa 145560
ttggttttag gttatatagt tagaagtggt tgattttggt tgtgaatata gggttgtttg 145620
atttaaagt ttgagttggt ttgtagtgag gtttatggtt tttgtgttat tgtttgagtt 145680
tttggttgag ttttttggtt tagtgggggg tttagtgatt tattagttgt tttattatat 145740
gttgtggata tttttatggt ttatttgggt gggagggaag gggttttagg aggtgggagg 145800
ggtataggga atatagttta gtttgtttgg gtttaatatt ttgattttta aggtattttt 145860
tgagggtagt atgaggttaa gtttttgttt ttttttagga ttttgttttt gtgagggttt 145920
tggggttttt tgttttttt tgggggtttt gttggttagg attatagaga agttttttgt 145980
tgttgatttt tttgtttttg tattttttgg taagttattt tatgatttag ttgttttgtt 146040
tgtaaaatgg gttgaataat attgttttgt tttggagggt ttttgggagg attaaatgag 146100
atttgttgta tagaagtttt ttgtaagtta tgttgattgg tggttgttgg gtttttaagt 146160
gaatgggtat ttgggtattg agtgtgtgga tttagtattt ggaaggaggg gttgggggt 146220
atttagaatt tttgggaggt ttggattttt gttttgttta gattttgagt tttttttttgg 146280
ttagtttaat ttttattagt agggtgaggg taattaggat ttatgtatag ttgggtgaag 146340
ttgagtgatt atatgtggaa gtttagtgtt ttttatgtaa agtgggtagg tggttaaagt 146400
gagttttgtt ttagtttttt tatttttggg gttgtttttg gtgttttgtt ttttttttaga 146460
gttttattga tttgtattat gtgatttggg tttgggaaga tgggtttagt tgtgggaggt 146520
tgtgtgggtt ggggattgag agtgttggag gagggttttt taggaaaatt tagttgtata 146580
ggaagttggg ttagtggttt ttggaagttt ttgggttttg taagttggat tttttagatt 146640
tgtggttgtg gggttttttt ttgtttgtag ttgagagttt ttgatttgta tttgagggtt 146700
ttagaggttt gggaggggga tgtgattgtg tttttagggt gaagtttatt tgagtgttat 146760
tttggatata gtttttattt ttttttggtt ttttttttgg ttatggggat gttgtttggg 146820
gggggttttt ttatatggaa ggagttgtag ttgagttggt gggttttgta tatttttaag 146880

gttgtttgat ttttagtttt taagtggggt tgtatttatt atttaggatt tggagtttta 146940
gttagagagg gattttttat tgtttttttt tttgtttttt gggaaaggta gggtttttgt 147000
gttttttttt atttagggat ttggtatgtt gggaggttaa gaggaagttg tttttttgttt 147060
atttattttt taattttttt agttagtaga gttttatttg tgtgtttaga atttgtgtgt 147120
ggagttagaa gttataaggt tggtttagtt gtgtttgatt ttggttgtgt ggttttggat 147180
agttttagtt tttttggagt tgagtatttt gatttgtaga ggagaggttg ttgtgttggt 147240
tttgtagggt tttgtgtaga ggggagtgtg tgtggttgtg agtttaggtt atgtttttag 147300
attggaggat aggattggaa tagtgggtta ggatggttag agggttttag ttttaggagg 147360
aggtttttat atttttttagt atttttatat taggaaatgt agttaggttt ttaggttttt 147420
tatgttagga agtagtattt tgtttttttat ttttggttgt ttgggtttga gaagttgagt 147480
atttttttttg aatttgagtg tttttttgatt tttgtttggg tagttgtttt ttttgaattg 147540
attgtttgtt ttatatagtt ttttgtggtt tgtttgatag ggtggttagt gagtttttttg 147600
tatagtatat tttttttagg agtgttgttt gtgatgatgg tgtaaagggt gttttttttga 147660
gtttttggag gtatgtagtg gtgtaatagt gtgagtttag gagttttggt gagtttttgg 147720
atggagaatt tgtttagatt atttttttagt ttaaattttt tttgggtgtt tgttgtgttt 147780
ggggtgttga tttattgtgg ggtggtttgt gagattttat gttatttggt tatttttttttt 147840
agtttttgtt tattgtattg gttttttggt ttggttgttt ttggaatatg agggtttatt 147900
tttatggaag aatatttatt tgtttttttttt tggaaagttt tttttttagat tttttggttg 147960
ttttatttag gttttgttga agtgttgtta ttttggagag gttttttttgt tatttgtagt 148020
taaaatagtt tttttatgtt tgtgggtggt ttttttatggg gtatggatga gtattggaga 148080
gttttgttgt ttatttgttt atatgttttt tgggagggta ggggatattg atttgtgttg 148140
tgggtgtttt gtatgttatg ggtgtttagt gaatgtttgt taagtattag gggagtataa 148200
aggagggaag tttttgttta attggagtag ttagggaaga ttttttttggag gaagtggtat 148260
ttggtttgtt tagaatgtgg tgatttgttt gtatgtagat attagattag agggtaaagg 148320
ttattattat aatttagtag atagttataa gtagttaagg aattgttatg atatggattg 148380
tttgtgattg gagttaagaa ggagttagag atagaggttt ttgtgttttg gtgtgagttt 148440
ttgggggagg ttgagttttt agaatgtggg gttgtttgtg tagaggttgg aggtgggaga 148500
tatatttgtg agtgttggtg agtgtggggt gagttattgg ggttggatta gaagatggga 148560
atttttaaat ttattaatta gttttggtgt tttgggagtg ataggagatg gggtttttgg 148620
gggattaatt tagtataagt ggatgagatg ggatgtaggg gaaaggataa agtttttttga 148680
ggttttggga agttgagtta gggtagagtt atgatagggt gagttttaga agtaatataa 148740
gaaaaaatag ttagattggg tttttttttt attgtgggag atgttttattt ttattttttt 148800
tgttttttgg gttttggtaa tgggttgggt atagtgaatg ggttttattt gtaggagtgg 148860
ttatattttt tgggtatagt tgttttatat attgtattga gatgggattt gaggttggga 148920
agaatagttt tttttgtagg ttgatattgt tagtttttgg ggagggaagg atggtagggg 148980
gtttggagta tttttttttg ttatttatat tttgtgtttg tttgtagggt tttgagtttt 149040
gtttgttttg ttttagtttt ggttaggaat ttgttggagt ttataggggt agttaggttg 149100
gaataggagg agattaggtt ggtaggggag ttgagagggg gttttggata gtatttaggg 149160
ttgagttatt ttgggtttgt ttgatttgtt atttggagtt gtttttgttt gtttattatg 149220
tgtaaggttt tgatttattt gggtttgttt aattagtgtt tttttttagg tttagttagt 149280
ttggtttttg tttgatttat ttattgttat tagtgttatt tttgtattat gtaattagtt 149340
tttgtagtat gttagttatt aagtttaggg ttttttgtgt atttttttgta atttttatag 149400
taatttggga ggtatagaga ggttgagttg ttggtttata gttatatagt aggtatgggg 149460
tattgttagg atttagttgg atttttgatt ttagggtttt tttagggttt tgggtgggat 149520
attggtaagg aagtaaggta tgaatattgg ttttgttttg gatagagttt ggggatggtg 149580
gtttggtagg tttagttgat atagtgattt tggtgtaggt ttaattattg gtaaagtgtt 149640
taagtatata tagaatttta attggggaga taggtgaagt ggttaaggga gggttgtgag 149700
tgataggtgg atattgtatt tattggtaga ttagttgatt ttggtgtttt ttggagtttt 149760
ttaatttttt tatttagaaa aagaggaatt aatagtttta tttttaaatt gggtgggagt 149820
attatatttg aaattgaagt aaagttggtt gtgttatagg tttggtagat gttagttgtg 149880
taggtaattt agtgattatt tgaagtaggt tgtgtggtgg tatttttatt ttgtagatgt 149940
agtttagaga ggttagggta ttaattttgg gttatatagt gatagtttag gttatagaga 150000
gtagtgttgg atgggagggt gttggtagtt ttaggtatag gttgggaggg ttgtggggtt 150060
agtagaggga gggtatattt tttagttggt ggggtttttt tgtgttttag tatttatgtg 150120
ggtttaaagg agttggaagg agggaggtta gagttattta tgggtttaga agttgataag 150180
taggttattg attttgaatt tgttttattg tatgtaaatt ggggataaat ttggggattt 150240
aatgatataa tgtaagaagt agatatgtta tagttttggt tatatagtta gtgtataata 150300
agtggtaagt tgttagagga ggtagttggt gagaatggga gtattttagt agtttttttgt 150360

tttttgtttt tggttgtaga tgtatttgtt tttggttatt tgtgttttag ttttgtgttt 150420
tttggtgtgt gtgaggttaa gtttttgggg tggggatttg ggggtgtgtt tgtagttttt 150480
gaggttaaga ttaaggttga ggttgaggtt gaggttgagg ttattttggt gaggaggaaa 150540
ttgtaggtgg agaagtttgt tgattgtgat tttgatttga ttgtaatttt gatggtgttg 150600
atgttgttag tatgtaggtt ttttgttttt gttatgattg gtttttgtag tttatttggt 150660
tgagaggtgt gttggttttt gtaggttgta aaatggggat attattgttt gtttgggtta 150720
tagtgtgttt gttgattgta tttggggagg gattgagggt tgattgtgtt gtggggatgt 150780
tggtagagaa ttagagaggg tatttaaaaa ggttaatagt ttggtgggga gaggaggtgt 150840
aaggttggtt tttgtttttt gggttttgat tgaaagggaa taggggatat atttggggat 150900
agtttttttt ttggaggaag aagggtttag ggtgttggtg tggagtttgt atagatgggg 150960
ataatgagtt ggagggtgtt gggtaggagt tggttagtaa taggttgatg agggaggttt 151020
tgttggttgg gtttggtttt taagtgagta ttttgagttg tggggattta gggggtttta 151080
gagattttag agttgagatt tgggattata gaggggtggg ttgtgtttag ggtaatatag 151140
tggatggggt agatgttgtg aaatttgttt ttttgttttt tttttttttg ttatagaatt 151200
ggattttttt agtttatggt tgttaaattt tttagatgtt tttgggtttt atgggtgttt 151260
tatattggtt tttttgtagg atagtagatt ttgggggttg gttttgatgg tatttattat 151320
ggggtatttt atgtaagtat agtgttttta tgtttagttt tttagtttgt ggagtaggtt 151380
tgtgtttggt tagttttatt ttgtggttag aggagggaga tagtggtttt ggagatagga 151440
gttggtttgg ggttgttttt ttagttttg ttttttagt ttattttttt gttttgtggg 151500
tgttggttat ttttttttg ttagtttat ttttagtttt ttttgtttga gttgtgattt 151560
gttagtaggg ggtagtggag gttttgaaag tttttggaga ggattttgaa gtagtttta 151620
taaatttgtt tttggaaagt ttttttgtt atatttttag tggtttttt tttttaata 151680
ttttttttgg ggttgttatg tagtgggggg tggaggaatt tattaggttg tggatagagt 151740
agagttatgt tgatataaat tttggttgaa tttatatgtt ttttatagtt agtggttgat 151800
tattggaatt agggaagtga ttttatagta agggtttgta gagtagttat tatgtatttt 151860
tgattttggt ttttggattt agttttggaa aagaagttag attttagtta tttggagttg 151920
tttagggaag attttagttt ggtgttggtt gatagtttat tggaattttt tatttgtttg 151980
gttaggtgat gttaaggggt agaggttaga agtgttaaga agtaggttga gggtggtggt 152040
ttatgtatgt aattttaata ttttgggagg ttaaggtggg tgaatttttt aaggttagga 152100
gtttaagatt aggttggtta atatggtgaa attttatttt tattgaaaat ataaaaatta 152160
gttaggtatg gtggtgtatg tttgtaattt tagttatttt ggaggttgag gtaggagaat 152220
tttttgaatt tgggaggtag aggttgtagt gagttgagat tgtgttattg tattttagtt 152280
tgggtaatag agtaatattt tgtattaaaa aaaaaaaaaa gaagaagaat aagaagtgtt 152340
aagaagtatt tgtttttttg attgaaaagt aatttttgtt gggtgtggtg gtttatgttt 152400
gtaattttag ttttgggagg ttgtggtggg agagttgttt gaggttaggt gtttgagatt 152460
agtttgggta atagaggtgt taggggtagt ggtgtttgtt tgtagtttta gtttttttagg 152520
aggtttaggt aggaggattg tttgagttag gagtttgagg ttgtagtgag ttatgattat 152580
attattgtat tttagttttt gggtgatagg atgagatttt gttttaaaaa ataaaatata 152640
aataaaaaat taatgtttat tttttgtgat tttaaaaatt atatttattt gtgataaaat 152700
aattaaatta gaaaatgtaa aggaaatgat taataataat tgtgtttttt agataaatat 152760
tgttaatatg tttatgtttt tttttagttt ttttttatat agatatttaa tatttattaa 152820
tttgttttga attaaagatg gtttttttttg ttttgtatga gaattatttt agttaggtat 152880
ggtggtttat gtttataatt ttagaaagtt gaggtaggta gattgtttga gtttatgagt 152940
ttgagattag tttgggtaat atggtgaaat tttattttta taaaaaaata taaaaattag 153000
tttggtgtgg tggtatatgt ttgtgttttt agtttttttag gaggttgagg taggagaata 153060
gtttgagttt gggaggtaga ggttgtagtg agttaagatt gtgttattgt attttagttt 153120
aggtaatggg agtgaaatgt tgttttaaaa aaaaaaaaaa aaaaaaaaaa gttgttatgt 153180
tgttattttt tttagggatt tttatttttt ttatttttttg tttaagattt gttttgtaaa 153240
aggatgaggt taaatagtta tgggagggtg gtattggatg tttaaaaaaa tgttttgtgg 153300
ttggtgggat ttagaaatag gttggggagg ttttagtaga gggtggattt tagttggggg 153360
tggggggtag taggggggtt ttttatatgt tttttttgtg tgttttatgg ggttgtttgt 153420
gaggttgtgt gaattagagt ttggttggga agttatatgt tatttggatt ttgatttttt 153480
ttggtagtat gattttagtt aatatgttta attatgtttt tttttgtaaa atgggttaat 153540
agaaatgttt atttgagata tgatgagaaa taaaggtgtt tatagagtaa agtgtttaga 153600
atggttttgg tatgatagga gtttatatgt tagtgttgtt gttggtattg ttattattat 153660
ggtggtattg gttgttttgt tttttttttg tttttgttat tttttttttt tagtaggttt 153720
ttatttattt gtttatttat ttatggttga ggttttgttt gtgttaggtt tggtgttgag 153780
aatgggggta gagagatggg ggttgtgttt tgttttttga tttaagggtt ttttttgtaa 153840

tgtgaagttt ttggagattg gatttttagg ttgttttatt agttttttt taggttagtt 153900
taattttttt gtgtttgttt tttgagttgt tagaggaaga gagtgggagg aaggatggag 153960
tggggttttt gtggggattg agtggggggt gtgtgaggtt ttggaaatgg tgtttgtggt 154020
atttatgtag tattagtttt tgttgtagtt gttgtttgga gaatattttt ggattttagg 154080
atattgatga tagaaggtgg aatgtttttt ttggaagtta ttaagagggg attattgtgg 154140
tggttgagta gagttgttga tttgtgagtt gtggtttgat tatagttatt gtttttatga 154200
gtgaggtttt ttgtggtttt tttttagga atttttggtt ttttggtagg ggatagttta 154260
gggttggggg tgggtagtgg tgtttgtagg ttttttttgga gttagagaat tttttgttgt 154320
tgtggtgggg ttgttttttt ttgttttttt tttttgttg tgttttttagt tgttttgata 154380
ggatgttttt ggtttgagag atagaaattt taattttgaa tggttttagt ttttaagaga 154440
atttggagtt tttttgtgta aaatgagatg ttttgagtat tgaggatgtt gtgggggatt 154500
ttttatttta ttatttttag ttgtggtgtt ttttttggtt ataggatgtt gtagttatgt 154560
tgtagggaag gagagttttt tttttttgtga gtttagggag tttttttatg tagttttttt 154620
attggttatt gtttgagatt tgatttggtg gtttttgttt taaggagtag aaaggggggtt 154680
tgttaggtta gggtggggtt gggtgtgagg atttttttttg gttaataggg tttgtttagg 154740
tgtttgtttt gttggggtag tagggaaggg atttatatgt ggtattggga tatggagagg 154800
tttttgtgtt tgtgatggtt aaaagggatg gtgttttagt tatgttttta tttattttga 154860
ggtttagtat ggttatgttg gaaatagtag tattgttttt tttattgttt tggtttgttt 154920
ttaataggtt agaggggtgg ggttggagtg ggtgtttgat ttgtggttgg atttagttgt 154980
ttgtggatag gttgtgagtg aatttgtaga ggaagggaga tgggaatgtt gggatttttt 155040
tttgtatttt ttgaattttt gtttttttgtg agttagatgg ggagtgtatt tttgtttttt 155100
ttttttgggt atttttttgtg ttttttgttt taggtatttg atgttgtttg tttatttttt 155160
ttttttgttt tttttgttgg tttttttttat atggttttaa ttaggatttt ttgtttgttg 155220
gtggatgggt agagaggggga gtttgttgtg gggttttttt tgattgggggg atttttagt 155280
atagagtttt ttggtttttg tagggatgtt gtgtgtgttt gtgtgagttt ggtgtgttag 155340
aagtgttaag ttgtagttgt tttgtgtgtt ttttatattt tttattttt aaaggttttg 155400
ggtttggtgt ttgtgggttg gggatttgtg ttggggtttt taggaggttt tttggtgggag 155460
aggtagggtt tttttttttt taatttgggt aattttgttt tggtttgttg tttgtggaaa 155520
gtgttttagg atatggtggt aggattggtt ggggttttgg ttttaggttt agttttagtt 155580
tttgttagat ttattttttt tgtgtttttg tttattttgt ttatttgtag ggtttgtagg 155640
agtatttttga gagttggtag ggtggtttgt ttggtgtttg tatagggttt gttggtgggt 155700
ggttgaggtt gtggtgaggt ttttatggtt tttttgtttt gtatattttt tttttgttgt 155760
gttgttttgt tagtatatga gtttgtattg tttttgagtt tgtagtttgt tttttttatt 155820
aggtggttaa gtagggtggt agttagggtg gtagagtttt attggtttgt aggggatttt 155880
ttgtgggttg taggtagttt tttgtggttg aaggtgtttt ggtaggattt gtattatgat 155940
ttttgttaaa gtttatgttt aaggtatttg tgggtatttt tgttttagat gtgagggttg 156000
gtattttttt ttgtatgtga agtaaaggtt tgtttgaagg agtgtgtgga agtttgggtg 156060
tgatgaaggt aaggattggt ggtttatttt ttttgtattt ggattttggg tggtaggttt 156120
ggggttttta gtttttaatt taggagggta gttagttgtt ggaattaaga gtgaatttta 156180
gttgttgttg ttatagttgg agggaagagg ggaaaggagg tgttgtaggg gagtagagat 156240
tttattttt ttttgttgat attaggttgt tggtgttgga tggggttttg gtaattgtgg 156300
taggagtggt gatgtttgat gatggtaata agggtttttt gaggattttg agttgtttta 156360
agggtttttt atttggagta agtatttttt ttaattttt taataatatt gtgaaattga 156420
ttttgttgtt atttttattt tattgatgag gaatttatag tttagagatg ttaagtaatt 156480
tgtttaagtt tttttttgag tatggtagag ggagggtttg aatgtggaaa gtttggtttt 156540
agtgttttta tttttaatta ttgttattta ttttttttggt tagttttttg attgtaaaat 156600
ttgaggttta gaggagtgta gtgatttggt tagttttgta tggtaggtta gtggtagagt 156660
tgagtagtgt tttttatgtt ttgattttgt ggtttttttga ggtggtttat ttttaaatat 156720
gtttttttatg gtggttttgt tttttttttt tttttgaatt tgttagtttg tgtttaggta 156780
gggggagaag tagagtaatt atgttgggtg ggttgttgtg ttttagtgtt gtttgtttta 156840
tatttgattg gtagtagagt ggtttttttt tggtgtggtt tggaggtgtt agtggttatt 156900
tattagtata gtttagtttt atatatgtta tttttaaagg atgttgatat agaatgaagt 156960
agttaggggga ggtggatgtt ttggtatgag ggtggtgttt ttggtaattt gtagtgttgt 157020
tgtttgtggt tgttttttggt tggtatttta tttttttttg gttgttttgt agttatttga 157080
gggaggttag tatgttgttt ttattttagg atagtgtgaa tgtagtagtg taagtttgtt 157140
agtgttgtat tgtgggattt ggaggttttt gtttttgagt tttgtttttg tgtaattttt 157200
aaagtatgga tttttttaggg gtttttattt ttttatttgg aaaaatttta tattgtttag 157260
ggtttgaggt agggttagag tatggagatg ttgttggagg attaagggag aattgagtgt 157320

gtgttttttt ggttttagtt ttgtgtgtgt gtttatgtat gggtattttg ttatttatat 157380
tggtttttat ggttgggtgg gagttgagtt gagtttggtg gttgtttttt tattttgtgg 157440
gtttggttgg ggagtgtgag gatgtttgtt ggaagttaat gtgtatgaaa gagttaatgg 157500
tggattttat ttttttagtt ttttttttag ttttttttagg gaaagggtag gaatagagag 157560
gttttatttt tagaggaagg ggtgggtggg gatatagtgt tgtttggtat agtttttttt 157620
gagttatttt gtatgtttta ttttattgat ttttgtagtt tagttgttta gagtggattt 157680
agaaggtgga gagagggttt ttaataagta gaggatggag tgaatgtgag atttattatt 157740
gggatttggt aaagtaaaga gttttatttt ttttttgtag atgggatggg ggtggggttg 157800
gagttagtgg ggtgggggta tttagtaaga gttttttttt atttttttat ttttttttgtt 157860
atgggttttt ttttttatgt agattttttag tttggaatga ttagtgttta gttttttttg 157920
tttttttttt gttttgttgg agggagtgat ttggttttttg gttttggtta tttatatttt 157980
ttgttttgtt gtattagttt tgtggtaggg gttgtggtgg aggtgggtaa aagttgtttt 158040
tgggtagtta gtttttattt tgggagtgat tgaaagtttt tgggggtttt tgtagggtga 158100
gttgtttatt ttggttttgg taaggatatt taggagaggg ttagggtgtt tgtatttggg 158160
gtatagtata aagaatagtt gtaagtgttt tttaggtagg agggtgttga agtattggga 158220
agttgggatt tagaagtatt gtttggggtg gtgagtggaa gatgtaggat atagatttat 158280
gtgattgtgt agattatttg gttttatttt tttggtttat agtggaggaa attagaggtt 158340
gagagaggtg aagtgagttt tttggggtta tatagtttag ttaggtttag ggtgttgatt 158400
ttttgttttg ttttttttgtg tttatttggt tggtattagt atttttgttt tgatattttt 158460
gtgtggtagt tgttgtgatt ttgtgatttt tttttatttt attttagtgg ggttttgtga 158520
gggaaagaag aatgttttat gtagaagtgg ttggaaagta agaagtttgg agagaagtga 158580
ggtttttgtg gtttggtttg agtttaggat ttatggtttg gtttttaggg atggagtatt 158640
gatttttttt ttttatattt ttattttatg tttttaggag ttttttatgg ggaagttgga 158700
atggatttgg gtttagagat tgtaaattta ttttgttgga tgtgtggttg ttttatgta 158760
attaattttt ttatggagaa gttaggggat ggtttttagt atttttgggg gtttttttagt 158820
gaggtgggag gattttgaga ggttaggagg ttgtttaaga ttttttgttt tttagggatt 158880
aggtgagggt ttgttgattg ttttgatttt ttgatgtttt tggtggggag atttagagg 158940
ggtttgttgt tattattgtt agtggttttg gtttgtttag ttattttttt ggggtggtta 159000
aattttatgt tgtaaaagtt taagtgaatt ttagtagtgg tttgggagaa ttatttgtta 159060
tgagttaagg atggagtttt tggggaggat tttttgttgt tttttaattt tttttgggta 159120
tatttttttg tagttttatt ttttgtaaga aagttaggtg tttttttgaag tttttttttg 159180
gttttaggtt agaggtgata gtgggggttt tttgtttgtg ttggtgattg tggttggttg 159240
ttggtgtttg gttttgtttt gtattgtgta tttattgaag gtggtattat ttgggttttt 159300
ttggtggtta tgtatgggtg agtagtttat gatatatttg tttttagtat tgtgtgggtt 159360
ggggttttta gaggggttga gggtttggat ggttttgtag gggggtgttt ttggttttgt 159420
gattgaaagg agttgaaggt ttggagaagt tgttgggttg aggttagttt ttatagattt 159480
ggtgaggagg gaatagatgt ttatggagtt gttttaggta ttgtatatat tgttttattt 159540
tgtgttgata gttaagaggt tggtttttttg ttgattttta ttttatagat aatgtgtttt 159600
tttagttttt tagtggtgga gttaggattt gaatttatgt aggtagtttt ttgggtttgt 159660
agttttattt tttatttttt tttaggattt atgttttggg gttggtagga taatgatttt 159720
tagagttgag tatattttaa tttttagtat ttgggattat gttaggtgat atggtagggt 159780
tagttaaggt tattaattag ttggttttaa aatagggaga tgattttgga tgatttatat 159840
gggtttgttg ttattataag agtttttaaa agtgtaatag attatttgag ggtgtagtg 159900
ggagaggttt ggaaggagga aggaggttat aagttaagaa atgtaggtga atagaaaatg 159960
taggaaaatg tagttttttt tgtagttttg ggagggtgtg tagttttgtg gatatttttt 160020
ttgtagttta gtgaggtgga gtgtgggatt aatttgtgtt gtttgatatt ataagtgtgt 160080
ggttatttgt tatagtagtg atgggaattg aatgttatta ggtagtagtg tttttatttt 160140
ggaagaatta ggataaattt ttttgtggat tttaagtttt gggagatttt gggttttttt 160200
ggattgtttt agttttgtaa ttataggaaa ggtttttttat gtttgtttgg gagaagaaaa 160260
ttttgtagta aatttatttt tttttttgat aaatgaaggt gagggtgtgg gttggatata 160320
tgtttttttt ttgtggattt tttttgtttt aatatttaga attttgtagt tgaaggagat 160380
tttgggggt tgtttgtttg atttgtaagt gaagaaatgg aggtttaggg aagttaagtg 160440
attggtttag ggttatgtag agttatgttg gaatattaag ttagatagtt tagtttttgt 160500
tttaaagtgt tttttttttt ggttggggag gagggttgga gggagtggat tttgagtaat 160560
agtagtattg gtaggggttg gaagagtagt tattgtattt aggtgttgga gtttttggtt 160620
attttttgta ggaattaggt gatttattat ggtataggtt ttttttttgtt tatttgtagt 160680
tttggggggtt ttagggatta gaggatattt tggtttgagg gttggggttt gagagaagag 160740
atggaagtat ttttagtttg gtttgttttt gtggtgtttt gtaggtatat ttggtagttt 160800

aggggtttta gagaatttgg ttggtgggtg tttttatttt gttgtagttg tattttgtgg 160860
ttagtagttt gtaggatttg ggttgttgtt tgtggttttg aaatagttat tttttgagtt 160920
gagtggggat taggtgagta gatttttata ttttaattgt tgtgattttt ttgttttgga 160980
ggttggtggg tagtttggtt ggtgttttat ggtattttat gattggtttt gttattaggt 161040
ttgtgagttg ttttttttt tttgggtatt ttgaatggtt tttttgagag ttttgttttg 161100
ggtttttgtt tttgtaggtt tttgttgttt ggggtgtttt ttttttttgg aagttgggtt 161160
tttttgggga tttattaaga agggttttgt tgagtgttgt taaattgttt tatttggttt 161220
ttatttgttt tatttattat tttattttaa tttttatgtt ttttttttgg ttttaagatt 161280
tgagtaggta gggtttgtga ggtattttag gtttttgat gttgttagta tttaggggat 161340
tgttagtata tatgtaggga gggaaggaat gaaggaagag atgatttggg gagtttttta 161400
gtttttattt gtaattggta tattgtgata aaatatttat ggtattttat tttttttata 161460
tattttttt ttaaatttat ttttatttga gatggagttt tgttttgtta tttaggttgg 161520
agtgtagtag tataatttg gtttattgta atttttattt tttgggttta agtgagtttt 161580
ttgttttagt tttttaagta gttgggatta taggtgttta ttattatgtt tggtgaattt 161640
ttgtattttt agtagagatg gggtttttatt aagttggtta ggttggtttt gaatttttga 161700
ttttaggtga tttgtttgtt tttgtttttt aaagtgttgg gattataggt gtgagttatt 161760
gtatttggtt tttgtatatt tataaaagag aatttgttta gaaaattttt tagatgtatt 161820
tttagggttt taaggtatag atatatgaag tttgttagtg agtagttgtt gtttattttt 161880
ttttttttt taatttagag tggtttgtgt tttaagtgga tagttgtttt ttttttttg 161940
gtttttgtt gtttatgttt ttatagaagt agggagaaga ggtaggaagg tgatgtgtga 162000
ttatttgttg ggtgtttgta tagtgttgtt tgtataggtg ttatttttt tatttgttat 162060
tttttttgtt gagtttttt ttttttttgt agatgagaga gtattgtttt ttagaggtta 162120
agaaatgggt ttagagttat ttagttgatt agtgtgtagt tagatttgaa ttatattttg 162180
ttgattttta tgtttgtagg tttggtttga tgagatggat agttgggtgg tggagatttt 162240
gagattttgg atggttttta aatagggttt gtagtttttg gatagggaga gtttggtata 162300
aagggtttgt ggagtgagtt gggggagaag gtggtttagg gtttttgttt ttgaataaat 162360
agatggaggg gttattttag tgttttttta gtttttggtg tgttgggttt ttggttttgg 162420
gaggagagaa tggagttggt ttttattttg ggttgggttg gtttgggttt aatttagata 162480
agtttttttag tttgttatga gtgatatttt tgtttgtttt ggtgtttttg ttaaaaatat 162540
gaagggtttt agaattttta ttttggtttg ttttttttt agatttggag tgggtttggg 162600
gttgataata gtttttatgtt agatagtttg agtagttatt agtgttatta ggtttgatat 162660
tgtggttttt gttttggagg gaaaaataag gttttttttg ggggattttt tgttagtttg 162720
tgaaaatagg tttggagtta gttagtgttt ttatgtttag atattttgtg tggtttttaa 162780
agtgttttta tgttgttagt ttatatgatt tttttaaaa ttttataagg gagggtagga 162840
agtttgagta atagtttgtt aagtatttat atagtaggtg gttttattgt tttttattta 162900
tagataagta aattgagttt tagggtggtt attgtttgtt ttttaagtta tttagttatt 162960
tagttatgga agaggttatg atttttata ttttagtgaa gttgaagatt ttttttttg 163020
tttttaaaaa tgtttatttt ttagggtagt gaatatttgg tgtttttgtt tagtgtaaga 163080
ggtgaatgtt tgttattttt gtttttttt ttttattaga tttgattttt tttttgtgat 163140
gggaatattt tgtaggtagt ttgttttgtt tttatagatt ttgagaaatg gggtttaaat 163200
tgttggttgt gggtgggata ggtgatattg tagtgaagag gtggagtatt tttgtagtgg 163260
ggaagaaata tattttttt attttttag gtttagtgtt tgggggtttg tgaattaaat 163320
ttatttaaga taaattaatg agagaaaaga tagatttaat tatatatgta tgggatttta 163380
ttgaaaaatg tgatttaaaa gagtaggtag aaggtggggt ttgtgattgg tttgaaggta 163440
gtgagttatt ttagttgatt gtttatagtt agttatagat tagatttttt gttttattgt 163500
ttttttttt tttatttgtt ttgttttttt tgagttagag ttttgtattg ttgtttgggt 163560
tggagtgtaa tggtatgatt ttggtttatt ttaattttg ttttttgggt ttaagtgatt 163620
tttttgtttt agtttttga gtagttagga ttataggtat ttattattat gtttggttaa 163680
ttttttgtat ttttagtaga gatagggttt tattatgttg gttaggttgg ttttaaattt 163740
ttgattttgt gatttgttta ttttggtttt ttaaagtggt gggattatag gtatgagtta 163800
ttgtatttag ttaattagtt ttaaaaaaaa aaaaaaaaaa aaaagaattt agggtttgta 163860
tattatttta ataggaaaaa aggaagggt gaaagtatat attatgggtt gggtgtgatg 163920
gtttatgttt gtaattttgg tattttggga ggttgaggta ggtggattat ttgaggttgg 163980
gaatttaaga ttagtttggt taatatggtg aaattttgtt tttattaaaa atataaaaat 164040
tagttgggta tggtggtgta tatttgtaat tttagttatt tgggaagttg aggtaggaga 164100
attgtttgaa tttatgaggt ggaggttgta gtgagttgag attatattat tgtattttag 164160
tttgggtaat agagtgagat tgtgttttaa aaaaataaat aaaaaaaaaa aagtatatat 164220
tatggtaaaa taaatgagtt tttggaaaga taaataggtt tttaggagtt gagatgggag 164280

atgtgaagtt ttgtgataat gtttttttag gtgtggtgtg gaaattattt atattttttta 164340
gggaagagtt agttgttttt agggagtaga tttgggtttg tatattattt taatagggga 164400
aatggaaggg gtaaaagtat atattatggg ttgggtgtaa tgtgtttagt gtaggaggta 164460
gatgtggttt atggttgttg ttgaaattag tgtagggttt gattgttttg tattgagtgt 164520
tgaggatttt ggttttgtat tagttatgag tttgtagttt tgggatatat ttgatgttgg 164580
tttaaggggga aatgaggttg gaggggtagg gtggttggat tggatgtggg tttggggtta 164640
ttgttgattt ataaagtggt tattatatgg gaataataga gttggttata gattataatt 164700
agtaatatta tttaaaagta aggttatatt tttaataata agttatattt taagttatta 164760
ttaatttata aaatatattg ttggtataat gatagttttt taagaaggat ataaaaaggt 164820
atgttgggtt gggtgtagtg atttatgttt ataattttag tattttgaga ggttgaggtt 164880
ggtggattat tttaagttag gagtttgaga ttagtttggt taatatggtg aaattttgtt 164940
tttgttaaaa atataaaaaa aaattagttt ggtgtggtgg tgtatgtttg taattttagt 165000
tatgggggag gttgtggtag aagaattatt taaaatttgg gaggtggagg ttgtagtgag 165060
ttgagattgt gttattatat tttagtttgg gtaatagagt tagattttgt tttaaaaaaa 165120
aagaaaaaaa agaaaaaaag taaaaaggta tgttgaaggt atttgtttgt ggaaagttgt 165180
aatttgatgg gaggtagggt gagtattttt tagaaaggtg gaaatgggtt tttttgttat 165240
agagatttat aaatatagat tgaggtttgt agtttaggag atttaaaaga ggtaaaggtg 165300
aataagagtt aaggttttta agttagggtt tgattttggg ggtgggggtt atatatgatg 165360
gaggatgtta ggagggtttt ttggaggtgg tgatatttat taggggggat tttaggtggg 165420
gtttgtgaga tagtaggatt tggttgaaga gtgtaggttt tttagatagg agtgggaatt 165480
taggtttgaa aatgagttgg ttagattgta ggggtttttg aatgtaaggg aagaatttta 165540
atttaatag aggagtttgg gaagttgttt gggaaaagga gtgataaagt gggaggggtt 165600
ttttagtaga gttttgtatg ggtaggaggg agtgagatgg gaggtgggtg ttggtaaggg 165660
atgattttag tggtttaggg gagtagttat agggtggaaa ttaattttgt ggggtttggg 165720
taggaaggga ttgatttgag atattgggaa gaaatagtta gtaggatttt ggtgagaggg 165780
ttgggggttg gggagggggg tgtggagagt gggagtttga gtttttgaaa atagatagtg 165840
ttttaagggg gagatttgag gtaagatgtg gagaggtagt tgagttggga agttggaatg 165900
gaggtgtggt tattttagga gattaagtta gggggtgagg ggattttggg gagttattta 165960
gattttagta ttagggaata tggaggggag aaggaggggga gaagaattag ttggtgtgtg 166020
ttaggtattg tttttgaggt tagatggggt ggggttggat attggttatg aggatttttt 166080
taagagttgg gttaatagtg agatgggtgg atattagatg gtttgggtta agaagtaagt 166140
gggtggtggg aagttagaag ttgtggtgta gatttatttt gagaggttgg gttgtttaga 166200
tagggagaaa gtttatagtt tgaggaggta gataggttaa aggaagaggg tttgtttgtt 166260
tttgagattg gggtattttg aatgtttttt taggttaagt gagaagaaat tagaagagga 166320
atatgttatt taaatgtgtt gaaatagttt agtagttgtg ttgttttata gaaaatgtgt 166380
agaggttttt ttgttttatt atgtttgttt ggggatggtt tttatttggg ttatttaggg 166440
tttttttagt ttgttaattt aagggtggta ggagttgtgg gtgtttttag ttttttttta 166500
aggttggttt gtgggtagaa atgtgggttg ttttggggtg ttatagttat aaatgtatat 166560
tagtttttag aattatattt ttgtttttta gttttttttt ttgtattttt atgtagaagt 166620
gtggttgtta gtttataaag gatagggagg gatattgttt ttggtatttg atgggaagta 166680
tttgttgtat tttattgggg tgttgtttag gatggattgg aaaagagttg gtaggaaggt 166740
tgagttttgt gtttataatt tggtttggtg gtggttgagg agtttggtag gagtagagtg 166800
taggatttgg gaattggggg ttggtgtatg tgtgtatgta tgtgtgtgtg tgtgtgtgtg 166860
tgtgttgggt gggtagggag gaagttgtga aattatattt tttttttttt gttgttgtgt 166920
tgttgtgtgt tttagtagta tgtgggtgtt attatatttt ttagtaggtg ttaattttta 166980
agattgtttt gggttttttt tttagttggt tgagttggag gtgggagttt taattgtttt 167040
ttgtggtttt tagagtggga ttttgttgtg ggataggttg gttaatggtg ttttttttt 167100
tgtgattttt ttgttgggtg ggttatgagg aaggattgtg ggtgttgttt atagataggt 167160
ggatatgtgg taaggatatt ttgggatttt tttttgatg tttttgaag ggggtatttt 167220
tttagttttg agatgagttt ttgtggatgt gggaagttta ttattttaag agtagtagtt 167280
ttggaaaatt taatatagaa ttttgagtag gggtgggaag gggttttgtt ttgtttattg 167340
gttgtttggt agagttttgt ataaggaagt gtttgtgttg ttgtgggtgg aggaatggat 167400
tgagggttat atttgttttt tgttgttgtt gtaattgttt attataaatt tagtggttta 167460
aagtaataga ggtttttttt tttatagtgt taagggttag aagttgatta gttttattgg 167520
attaaagtta aggtgttggt agaatttatt tttgtttttt ttagttttgg gtgggaggtt 167580
ttgttggagt tttttggttt gtggttgtat tttttagtt tttgtttta ttttttata 167640
gtttttttt tttttgtagt tagattttt tttgttttt tttttttt ttttgagatg 167700
gagttattta ggttggagtg tagtggtata attttggttt attgtagttt ttgttttttg 167760

ggtttaagtg attttttgt tttagttttt tgagtagttg ggattatagg tatgtgttat 167820
tatatttggt taatttttgt attttagta gagatagggt tttgttatgt tggttaggtt 167880
ggttttgaat ttttgatttt aggtgatttg tttgttttag tttttaaag tgttgggatt 167940
gtagttatga gttattatat ttggtttgtt tttttttaa aggatgtttg tgatttgggg 168000
tttatttggg taattttttt atttaatat ttttagttat atttatagag tttttgttgt 168060
tatatgaggt aatatagttt gggaagggag agttatttag tttattttag gggtttgtgg 168120
tgtattttag ggttttttg atttaagat ataaagtaag aaaataaatt ggtttaaggg 168180
gaaaaaagga tatgttgaat tttgttgttt taaatgtata ttttttatt gtgttaaaat 168240
gtatagaata taaaatttgt tattagtaat attgagtata tttatagtgt tgtgtaatta 168300
ttagtattgt ttagtgttag aattttttta ttattttaaa gggaaatttt gtatttatga 168360
aggatttatt ttttatttgt tttttttagt ttttggtagt tattagaatg tttttgttt 168420
ttataaattt atttttaata agtgtaattt tgtgtgattt taaaataaat aaatatgagt 168480
atgatgagtt gtttattgga aggatattta tgtggggagg ttggtgtgtg gagtgtgtag 168540
gtttttggat gggtaggagt tgaaggggtg tggatgtgtt gtttttttt ttttttgttt 168600
ttttttggg gttattgttt gagtattttt ttttgtaaat ggttttaaat aatgttttag 168660
tttttatgtt tgtattgttt tttagtttta ggatttttta ttaaaaaata ttttaagttt 168720
tagatttatt tttgggaaaa ttttaatggt tttattttt tttttgagtt agttagattt 168780
tatggtttgt ggtgggttgt ttttgagttt tgagtatttg tgagttaggg aagtaggata 168840
ggtatattta gggaagggga agagttgttg ttagttatag taattgatat ttttggaatt 168900
gtttaagaga tatttagtgt gtgtttaaaa tatttatttt tttttttgtt tgttttttga 168960
gatgaagttt tgttttgttg tttaggttgg agtgtagtgg tgtgatttta gtttattgta 169020
atttttttt tttgggttta agtgattttt ttgttttaat tttttgagtg gttgggatta 169080
taggtatata ttattatgtt tggtgaattt ttgtattttt agtagtgatt gggtttttatt 169140
atgttggtta ggttggtttt gaatttttga ttttaggtaa tgtgtttgtt ttggtttttt 169200
aaagtgttgg gatgataggt gtgagttaat atatttggtt atatttattt tttaggagaa 169260
gttttgtatt ttttgaaagt gaaatgtttt taggtgggtg atattgatgg tgggatttgt 169320
ggttttatta gtgttttatg agagatgttg ttgtgtgttt tattaagttg tattttattg 169380
tttttaaat gtggtttgta gatatggtgt tgagaatgat ttgggaggtt taaattataa 169440
tatggtttaa attaatagtt ttttgatgat ttggtattat tttaggatta aattgagatt 169500
atttgaggat gggtgtggtg gtttatgtgt gtaattttag tattttagga ggttgaggtg 169560
ggtggattaa ttgaggtttg tagtttgaga ttagtttggt taatatggtg aaattttatt 169620
tttattaaaa atataaaaag tagttgttgg atatggtggt gtatgtttgt agttttagtt 169680
atttgggagg ttgaggtggg agaattgttt gaatttagga gatggaggtt gtagtgagtt 169740
aagattatgt tattgtgttt tagtttgggt gatagagtaa gattttgttt tttggtaatt 169800
aaaaaaaaaa aaaaaaattg agattttttg agttgtattg tttagtatga tagttattgg 169860
ttgtatgggg ttatttgaat atttgaaatg tggtgatttt aagtggagag gtgttgtgtg 169920
tgtgaagtgt attttgggtt ttagagtttt agtaaggggg aaagaaaaga atattaaata 169980
tttgttattt ttatattgat tatatgttga attgataata ttttgggttt tgttaagtga 170040
atgatattaa aattaatatt atttgttttt atattttta atgatttttt ttttttttt 170100
ttttttttt tttttaaat atggggtttt atattgttgt ttaggttgga gtgtagtggt 170160
atgattttgg tttattgtaa tttttgtttt ttaggtttga gtgatttttt tgttttagtt 170220
ttttaagtag ttgggattat aggtggttgt tattatgttt ggttaatttt tgtattttta 170280
gtagagatag ggttttgtta tgttggttag gttggttttg aatttttgat tttaggtgat 170340
ttgtttattt tagtttttta aagtgttggt atgataggtg tgagttattg tgtttggttt 170400
gtttttatat tttttattgt aggtgttgga aaatgtaagg ttgtgtttgt ggtttatgtg 170460
ttgtttttgt tggagggttg gattgagggt tagttgagaa ggttttgggg ttgtttttatt 170520
tgaatgttga gtgtgtgttg agtggtagag aatttgaga agtgttgaag gatagaggtt 170580
ttgtgtgggg gatgtagttt taggttggag atgtttttt tgtgttttgg agatttttat 170640
ttggtgatag taagaggaat tattgtaaga ggggtggtta ttttagtaaa gtatttagga 170700
tgttggtttt ttagtttagg aatttagagg tagaggaggg gaggaggttg ggtttggagg 170760
agagtagggt tttgggtttt gggaaaagtt tggggggtta gaaagttggg ggatagattg 170820
aggttagagg taaagagaag aaggtttgga gaggagggtt aaggaagaag ggttttgttt 170880
atggggaggg agaggtttat ggggtagggg tgtggtggga gttgtatttt tgaggttttt 170940
tgttgatttt gatatttggt tagaaaggag tttttttgg tattttttgt tagtggtttt 171000
tggagttgtt ttttaggggt tagtgatgag ggtgttttgg gttgggttgt agatagtgga 171060
aaagataggt tttttttatt tttagtgttg gggaaattag ttttttggag gaagggggag 171120
ttgtattttt tgagtggttg tattttttgg tttagatgtg tgtgttttgt tgtaatttg 171180
gtgattggtg ttggtggtgt ttattttatg gatggggaag gttaaatttt atagatattg 171240

```
agtgataggt ttagggtttt ggattttagt gtttgattta gaatttaggt ttttgatttg  171300
ttattagttt tttttttatt tagatgggaa tagggatttt gggtatgtag ttttttttta  171360
gatatttttt tggttttggt ttggtgtttg gagattagta gtagtagttt tttatggttg  171420
tttattgtga gggtggggag ggttgtggga ttaggaggtt ttagggggaa agttggttat  171480
gaggagtggg aagtgatggg ttagatgtta gtgattgttg gttggggagt tgttggatgt  171540
ggttggtttg tttggttagt gtggtgggtg ttttggggtt ttagttattt ttagttatgt  171600
gtgggggtgt gggatgggaa aggtaagagt gtgtggagtt tagttagtat ttaatatgag  171660
gttttgtggg ggttgtttga gggtgtagtt tttagaattt agtttttggt ttttagaagg  171720
tgttattaaa ggtttttttt tatttgagtg aatttttttt agtttttagt aagtttttgg  171780
aatgagttta tttttaggtg ttttttttta gtgtgggagt ggttatgttt ttgttgggag  171840
tgatttattt gggtttagag gttgtggtat tgagatgggt ttggtagatt ttagtgttta  171900
ggtttagttt ttatagtgtt agtttttttt tttggggatt tttttgtttg tgttttttttg  171960
ggtttagta tattttaggt ttgtagggag gggagagga agagattgat ttattggtta  172020
ggttttttag gggttggaga ggttggagag gtaggagttg gattagattt gaatttagag  172080
gtttttggag gaagagttta gggtgagttg tgtttttatt ttttgttttt tttttttttgt  172140
tttttttttt ttttatggtt ttagttagta agtatttggg gtagagggga taaatttagg  172200
tggttgtgtt ttagttttgg ttgtaggttt gaatggtttt ttggggtggt tggttatgtt  172260
ttttgagagt ttagttgtgg tgatgtttga gtaggtaggt gggggagtat ttaggaagta  172320
ggggtgttag gtagagtata aggagagagg gtgtttaggt tagttttagg atttggttga  172380
gaggaggggg ttttttatgg gtattgtttt tggtaagtat agggataagg gtaaggatgg  172440
tatggttaga ggtttttggg agtttttttt tttttttttt ttttagtagt tttttttttat  172500
tttttttagg gattttgtta ttttttttta gtgtgtggta gtttttttggt gttttttttg  172560
tgtttttagg ttgtttttgt gttgggtttg ttgttgggtg tttttatttt tgtttgtttt  172620
tttttttttgt tttttttgtt ttgttttttt ggagtaattt tttattgagt tttttttttt  172680
aggtagttga ggtttttttt tatttttgtt ttgtgttttg ggaggttttt tgttttgtga  172740
ttataaagtt tttttgattt tttgtttggt tttgagttat gtgatttggt gggtgggttg  172800
tggtttttgg tgtgtttagt gtagtttgat gttttgttgt tggggtgagt tttgtttttt  172860
tgtttttttt agttttgtat tattggtttg gggtttttta ggtggtgtgg ttgtgaggtg  172920
gatttgatg gttatggtgg tggtgttggg agttatgttg tttttgggtt ttggtttgag  172980
gttggtagga ttgagtgggg tttttaggag aggggtggtg gggagtttga tttttatgtg  173040
gggattagat ttttggtttt aaaatagaag aatagggttt tgtgtggtta tagttatttt  173100
tttgtaaata tttggttaat taagttgagt ggttaagttt ttttgttgtt tggagttttt  173160
tggaatatgt tttttttttg taagggggttt ttttggtttt taggagggtt aggaagaaat  173220
ttgaattggt tattgttttt tttaaaatta ttttgtttaa gttattattt ttttgggttt  173280
ttgaagattg gttggttgga ggttggagat agttttaatg tttgaaatgt tgtaattgaa  173340
gtttttttgtg gtgttggtat tgggatttag ggagttgttg ttatagtgta gttttggatt  173400
tttggatgtg ttggatatgt gtagggtgtt tttgggagga gtggggaggg agggtgttgt  173460
tggtggggtt ggtttgtgtg tgttttgttt ttttataatg gtatgttgtg tgttggttat  173520
gtagaggtat gttagtgagt aggggttgag ggattttttt aatggatttg tttttagagg  173580
gttttttttat gttgggagaa ttttagagat taaattatgt agttaatggg gtggtttttg  173640
gttttaaagt agggaggggt gaggagtttt gtaggtaatg ttatttgttt ttgaaatgtt  173700
gttttagtga ttttggggat tgatttagtt tttagtttgt tgtattttat ttttggtttt  173760
tttttttttg tttttttatg tgaaatttgt tgtgttttgg ttagaggttt ggggaatagt  173820
tttttgttta tttaagataa gtttgtaatt tttttttttgg agaaaaatat gttatttgga  173880
agtaggttga gtagtggttt tttggtaggt ttgtttgggg ttgtggagat agtatttgtt  173940
ggattaggat ggagttggaa tttggtttgg attttatatg agaaaatttg ttggaagagg  174000
aggaagtaga aaaaggtttt atttttttaat aatgttgtgg gttttttttt tttatttatt  174060
gttattttgt tttgaaattt aatggggtta tgtgtgtttt tatgaatgtt atttttttttg  174120
gttaattttt ttggtagttt taggagtttt tttttaggaa aaggaagaag gtgtttgttt  174180
agtatttagt atatgagttt ttgttggaaa ggtgggattt ttgttttttt ttgggttttt  174240
tttaggttga gatgtggtta aggaaggttt gtggtgtggt ttgttttttt taatgtttgt  174300
ttttggagat ttgagttgtt gttgattttt agtttgtttt ttttgttttt tggttatttt  174360
tgtgttgggg tggttgagat attattgtat tggttatttt gtgtttttat tagtttttgt  174420
ttttgtaggt tgttattgtg atggtgatgg ggttaattta gttgtggtgg ggaggattta  174480
ttgtgtgtgt ttgggggggtg gtgtggggta ttttgggatg agaagatgtg atttttgttt  174540
tgagagtttt gttttaatgg aaggtggggg tatagttgta ggtagaagtt attaagtatt  174600
gtggttagat tagtatttta tgttaaaaat ggttttgttg gttgggtgta gtggtttatg  174660
tttgtaattt taatatttta ggaggttaag gtaggtagat tatttgaggt taagagtttg  174720
```

agattagttt ggttaatatg gtgaaatttt gtttttatta aaaatattgg ttaatttggg 174780
tatggtggtg tgtgtttgta atggtagtta tttgggaggt tgagatagga gaattatttg 174840
aatttgggag gtggaggttg tagtgagttg agattgtgtt attgtatttt agtttgggta 174900
ataagagtga aattttgttt taaaaaaaaa aaaaaaaagt tttatttggg tttgggattt 174960
gtttttgaa tattgggttt attgtgtttt ataaggattt ttattttgga agttttagat 175020
ttatttgggt aattagagtt tttgggttga ttattttta gttttggtta gtttttggg 175080
ttattgtatt tttttaattt tttgttgtgt tatggttggt ttaagttgtt tttattagat 175140
aatgtgaggt atttttatag gaggggataa ttttgttttt aggagttttt ttttttgtt 175200
tttaggtttg gtgtagtgtg tggtttttt gttggtgtta aaggttttaa ggttttttta 175260
agaagtttgt tattattatt agtagttttt gttatttggt tttttgtat atgtaagttt 175320
ttttatata ttttggatgt ttagggtttt tgttttttt ttttggttgg gagtgtggga 175380
agagggtttt atttaatgt ttgagagtag ggttaatggt tagggtaagt tgtgggggtt 175440
tttttttgtt ggtttttttt tgtagttttt attttaatat agagtattga tattatttgg 175500
gttttgggaa gatttggagg ttgattattt tggaggtttt tagtgtttta tagttatttt 175560
ttggggaaga ggttttttga gggatttgtg gtttttggtt gggattttag ttttagagtt 175620
ttgtttttg gtgttgagtt ttgatttgtt tattgagtgt tagtttggtt tttggatagg 175680
tagggatttg tttttggagt ttagtagagt tttgggaagt ttttttagga gggtttttga 175740
gggtggtggt tatgtttagt ttgttgtatg tgtgagtttg tttgatgagg aattggttgg 175800
gaaggtgtgg gtttaattat aggtttatga gggttttttt tgtttttatt taggaagtag 175860
tggagatttt ggtatattta gtatttgggt ttgtgtttgt ttagttgttg gtggaattat 175920
gggagttttg ttttggggtt tgtagagtag tagtgggaat tttttttatt ttagtttttt 175980
ttagagttgg ttgtttaaat ttgttgttgt ttagtttttt tttttttttt tttattaatt 176040
ttgggaattg tatattgttt attttattgg gttttggagt gggtgtagga agagagagtt 176100
taatatttag aggtttgggt tttttgtagg tgtttgagtt gttgtggttt gagttgggtt 176160
ttttgttgga attattatag tagatttaat gatttgtttg taggagttgt tttattttag 176220
ttgattttgg ggttgtggga aggggttggg ttttggtttt gttgggggtg gggggttagt 176280
gtaaagggtt tgaaaagtgg tagaggtgat ggtttatttg ttttttttt ggttggttgg 176340
tttaaatgtg attggttttt ttttttttt ttttttttt ttttttttt tataaataat 176400
attaaatgtg atttgttaat gtatttattt gggtttagta gttggattat gggtagaagt 176460
attttagatt tgttattttt tttaagttaa gggtggggaa tattttttgt gagattttgg 176520
tttttgagtg tttaagattg tttttggaat tttaattttg ggttggtttt aggggattga 176580
tgttgatgga gttgttgata ttgatttttg gtttttgtat ggtttttagg aagggggttt 176640
tttgtttttt gttttgttga gtttggttgg tagtattttt tttttaggtt tgtgtttttg 176700
gtagggtttg ttgtttttgt tttgttttgt gggtgttagg ttttaagtat taggtgattt 176760
ggttattgaa tggttattga atagttattt ttgttggtgt ttttagtgga atgtgtatgg 176820
atgttttttt ttttttagat ttttgttttt tattttttag tttttgtttt aggggttatt 176880
ataggattag gaggtatgta gatgtgggtg gtatatttat tgggtagatt tagttttgaa 176940
tttgtatgtt ttttttaagg gggtttttag tgggtggggt tgatggtgtt gttttttttt 177000
ttgttattag aatggatatg aggattgtgt tttgttgatt tatgttttga gtttttagat 177060
gttataggtg tttgtttaat gagtgtttga atgaatggtt agtattgtgt gattgtagat 177120
ttttaatttt tatttaaata gtttgtggtt tttgtgaggt tattttgaga tagttttata 177180
agaattttta ttttttttgt atgttttgta taaggttttt ttgggggggtt ttttagaaaa 177240
agggttaaag agtagtattt ttttggggat ttgaatatga ttttttttgg ggtaggggat 177300
tgttttggaa gatttttggt attgtttttt atgtgttatg tgattaggag ggtttttgggg 177360
taggatggtg ttttttgtgt tttgtgtgtt ggggttggag gaaatattgt ttgtggttgg 177420
tgttatttgt tagttttgaa gattaatgat gggttggata gtggttttat tgagtgggag 177480
ggagaattgt tgttgtttgt agttgggggg ttgggtgggt gggagttgag gttagggaga 177540
atagtgtaga tttggtatgg agaggtttgg aggaggagta gggggtttta gttatttagg 177600
gtgtagggat gttatatgag gagtaggtgt ggtgtgtgtt tttttgtggg tttttttattt 177660
tttggttttg ttatttgtat taggttttgt ggtgagaggg tatagagtgg aggaaggtgt 177720
tgtggttagg gtaggttata tgttgtgtta atggattgtt gttatttgtt gtttttgtgg 177780
ttttgttgtt aggatttttt agtattaggt ttttattatt aggtatgggt tttataggta 177840
ttattggttt tttgaagtta gggatagatt ttatagataa tattgagttt ttgtagttag 177900
ggatgggttt ttatggttag tattagattt tagggattag tattgggtat ttatagttag 177960
ggataggttt ttatggttag tattaggttt tagggattag tattgggtat ttatagttag 178020
ggatgggttt ttatggttag tattagattt tagggattag tattgggtat ttatagttag 178080
ggatgggttt ttatggttag tattaggttt tagggattag tgttgggttt ttatagttaa 178140
ggataagttt ttatggttaa tattagattt tagtgattag tattagatgt ttatagttag 178200

agatgggttt ttatggttag tattaggttt tagggattag tgttgggatt ttggatgatt 178260
gaaggagggt atggttgatt aaagttgtag gagttagttt ggttattttg aggtattaag 178320
ttattattgt ggttttaggg gatttagatt tttttatttg tttttttgaa ttttggaatt 178380
agatatattt gggtttagtt tttgtttttg gggttgtgtg tttgggatgg ttgttttatt 178440
tttttgaagt ttagtgagat ggggatggtt gtatagggtt tggtatagat ggtatttgat 178500
ggtatagggt tatttgtttg tatttatata gttttggttt ttgttttggt tgtgtattga 178560
gagttttatg tttagatttt tgttgagttt ttaagttttt ggttatgttt taggtttttt 178620
gtgagaagta ttattgtatg gattatgttt tgggatttta ttttgggttg ggaagtttgt 178680
tttattttttt taagtttttt ttagaggtgt tttatgtttt attattttta ttgtttgagg 178740
ggttagatag gggaatttag tgtgggaggt ttagaggtgt tggagtagat gttaagtgag 178800
ttttgtgttt atgtgttggg ttttggtaga tattttgaat gagtttttttt tttaggaagg 178860
agattggggt tttagtttaa gttgtggttt taaattttgt gattaagagt ggagaaaagg 178920
taagattttt tttttagtt ttttagaata gttgatagtg gtggaaatat ggggtttata 178980
ttgaattttt ttgtaatttg ttgaagagaa tttagtttat atttttaggg gtggaggttt 179040
ggttgatttg gtagtgtgtt tgtttttttg gttatagttt attttggatt gggtattttg 179100
gtgttttttt ttagtgatgt agtgaagtta ttatgatgtg tttagttggt gagaaagtgt 179160
tttttgtaat gttttatttt ttttagaggg tgaataagtt ttgtttatta taaataatta 179220
tttatagaat atatgaggtt taatttaatt ttgggttatg gtagtgtttt ttgggtaaat 179280
ttatgtgtta ttttagttag agtttgtgtt tttataggtt ttttggattt attttgaagg 179340
ggggttggat tttttgtttg ttttgatgag agggttgtag gatagttttt agtgagtttt 179400
tttgagggtt gtaatttttt tttttggtat ttttttgttt gttaggaatg ttttaagttt 179460
ttagtagaag tttatttatt atagttgttt tttattttgt ttattatttt ttttgttttt 179520
atttatttag tagattttgt gtatagttta gtgtaggatt tagggtggga aggtgttttg 179580
ggtgggttgg agtaaggttt ttagttaggg tggggtttta aggtgggttt atagttagga 179640
ttgtgatttg ggaggttttt ttttgttttg ttttagtttt ggtattgttg ttgtttttgt 179700
gtttagtgga gatttattgg ttttaggttt taaaatttta gtgaggtttg tagataggga 179760
ggagttttat ttgagggtag agtgattgtt gggtttattt gtttgttatg aattatgtgg 179820
ttttagattt gttatttttt tttttttaga gttatggttt tttgtttgaa aaatggataa 179880
tgatggttat tatgttaggg attattttga ggataaatgg ggatgaaatt gaagaggttt 179940
agtatggaat ttggatatag aagtgttaat tgtgtgttat tagttgtggt ggttgttaag 180000
gtttaattat atggtagtta tgggttgagt ggtgggtggg aagtggggag taggagtatg 180060
taagatgggt atttttgaag tttttgattt agtgagataa gtatatattt agttttaaga 180120
tggagtgttt taggaaggtg gagattgtaa aggttttgtg gtgtaggagt gtgagttgta 180180
tttttgggtt ttttagaatt ttattagagt ttagggagga ggttagttta gatatagggg 180240
taatgggaga gatgggagtg agaaaatggt ttggtgtttg gaagtggagt ttggaaaggt 180300
gggtgtgggg atagaggagg gttttggggg ttatgggttt atgtgtttag gaggtggagg 180360
tagtgaatgg ggttatggtt ggttttatat gtggtattat tagttttgtt gagtttgtat 180420
ttgtttttta ggtttttttga gtgttattat tatttggtat tttttggttt gatttttgtt 180480
ttttgtttgt ttgggaagag tgagattatt tgtttaaagt tttttagttg tttttaatta 180540
ttttggttgt tttttggttt tgtttagggt tagttgttga tttgaattgt ttggtgttat 180600
ttttttagat ttttggggtt ttgttttggt attaatagtt atttttattt ttattattta 180660
tttgtatatt tatttagtag tgttttttgg gtatttattt agtaatatta aggtgttgag 180720
atgtggttgt ggttaagatt tgggtttggt ttgtgaggat tttgtagttg ttattttttt 180780
agagtatagt tttagttagg tatattgtgg ggtttagaaa gagaaattaa tatattgtgt 180840
ttgtttattt tgagttgtta taaggaatag ttgaggttgg ggggttattt gatttatggt 180900
tttgtaggtt gtataagtgg tgttagtatt tgtttggttt ttggggaggt tttaggaagt 180960
ttttttttat ggtagaaggt gaagggggag ttggtatgtt atatggtgag agagagaatt 181020
tttagatttt tttttttttt gaaatggagt tttgttgtgt tagttaggtt ggagtgtagt 181080
ggtataattt ttggtttatt gtaattttag ttttttgagt agttgggatt ataggtgtat 181140
gttattgtgt ttggttaatt tttttgtatt tttattagag atggggtttt attatgttgg 181200
ttgggttggt tttaaatttt taattttgtg atttatttat tttggttttt taaagtgttg 181260
ggattatagg tgtgagttat tttatttagt ttttttagat tttatagtaa ttagattttt 181320
tggattttgt gattgtgggg agggtattaa attatttatg agggatttat ttttatgatt 181380
taagttttat ttttttttagg ttttattttt aatattaggg attatatttt agtatgagat 181440
ttggaggggga taatgtttaa attatattat atgtgatttt tttgtgtgtg tgttagtttt 181500
tatatttggt taagtagtag aagttttttt tttgtttttt gttgagagag tggtggggga 181560
gaaatgattt agggatggga ggttggtttt ttgaggggtt ttggtgtttg tggagtggga 181620
gtgtgggaag gtttttatgt tgtgtgtgtt gtgttgggtt ggttttttat agtttattgt 181680

ggtgtttttt gaggattgtt ggggaggaaa gagttaatta atttggggat ttagttgttg 181740
aagttattat taaatttgag tagtttaggg gttttttttt ttttgggttt tatgttattt 181800
agtatagtgg ggtttaattg aatttttttt tatttttaa ggtttttggt tggtgtggtt 181860
ggggtttggg atatggggag tgagagggtt tgggtttttt ttagatttgg agtgggttta 181920
ttagaaatgg tttaggaaat tgtgggggtt atttttagta gttgggggtt tttgtggtta 181980
tttttttttg gttttgttga gtagttttgg tttttggagt ttttagtaag ggatgtgggg 182040
gtttgttagg ttagggtttt tattgttgtt gttatgtata gttgtatttt gtatttgggg 182100
agtggggtgt tgagtggggt gtttgggagg taggtttttt tttgtttttt ttgtttagag 182160
atttggggga tgagttgagt gagatttttt tggtttttga ttggttgtgt ttttgttgtt 182220
atttttattt ttttttaagt ggaggtttat gatttttttt tttatttaaa agggttattt 182280
agtgatttag tagattttag tttgtgttat ttagggtttt tttaaaaatt tgtggggagg 182340
ttgggtttgg tggtttatat ttttaatttt agtattttgg gaggttgagg tgggtggatt 182400
atttgaagtt aggagtttga gattagtttg gttaatatgg agaaatttta tttttagtaa 182460
aaatataaaa aattggttgg gtgtggtggt atgtgtttgt aattttagtt atttgggagg 182520
ttgaggtagg agaattattt aaatttagga ggtggaggtt gtagtgagtt gagattatat 182580
tattgtattt tagtttggat gatagagtga gattttgttt taaaaaaata aaaataaata 182640
aataaaaata aaaatttatg gggggatttt tgtgtttatt ggttggtgtg aggagagggt 182700
gaggagttgt agagaaggta ttaggttgag gattgttttt atttttgggt tatagaattg 182760
aaggagggga tatatgaaag atgtttttag atttgggttg gaggttgggg atatttgagg 182820
gtaaggatga tgttgttttt gtttttgttt gtagaggagt gtatagggtt tattagtttt 182880
ttgttgttgt ttaagtgata gttttatgga gatagaattt atatattgtg atgttgattt 182940
ttttaaaatt tatagtttaa tggttttgtt gtatttataa agtgattgta ttagggttat 183000
ttagagaaat agaattaata gaaggtgtgt atgtgtgttt agataaagag atttattatt 183060
atgaggagtt ggtttatttt attatagagg ttggtaagtg ttaagattgg tagagtaaat 183120
tagtaggttg gagatatggg agagtttatg ttgtggtttt ggtgtttatt tgaaggttta 183180
agatttagga gagttaatgg tgttgtttta gttttgagat ttaggaaaag tgtatgtgtt 183240
agtttgagtt tgaagtagga aaaatttgat gttttaggtt gaaggtggtt aagtgggaag 183300
aattttttttt tatttaggtt ttgtgttttt ttatttagat ttttaggttt ttgttataaa 183360
attataaaga tttgattaga ataaaatttg agaaaaaatt tttttttttt attggttttg 183420
gatttattgg ttgggatttt ggaaattaga ttgatagaga ttaataggaa ataagtataa 183480
taatttaagt aagttttatt tgatattgag tttttataag gaaatgaaga tttagagaaa 183540
ttgttagttt gagtggtttt tgttttgttt tgagataaag ttttattttg ttgtatgttt 183600
aggttggaga gtagtggttt gattatggtt tattgtagtt ttgatttttt gggtttaagt 183660
tggggtttag ttgttggttt tgggagttgt tttgaattta tttttaagtt ttagtttaga 183720
tttttaagta gttgggatta taggtatgtg ttattttgtt tggttaattt tttattttttt 183780
gtagagatag ggttttatta tgttgtttgg gttggtttta aattttttggg tttgagtaat 183840
tttttttgttt tggtttttta aagtgttggg gttataggtg tgagttattg tgtttggttt 183900
aggtttgaat agtttttatt tgttttgttt gtttttttaaa tttgtttgtt gtttgttttg 183960
agtagttttt ttgtttattt tttttgtttg tttttttgttt aggttggagt gtagtggtat 184020
gatttttgtt tattgtaatt tttgtttttt gggtttaagt tatttttatg ttttagtttt 184080
ttaagtagtt gtgattatag gtgtgtgtta ttatatttgg ttgattttttg tatttttagt 184140
agggatgggg ttttattatg ttggttaggt tgtttttttaa tttttgattt taagtgattt 184200
ggttattttg gtttttttaaa gtgttgggat tgtaggtgtg agttgttgtg tttagtttag 184260
gtttgagtgt ttttatgttg ggtttgatga agagtggaaa gttgtgggaa attgtgatag 184320
agtaataaaa gatgagttga atagtgagtt tgtggggggtt ttggtaagat ttgtgtattg 184380
gggttttttt tagattttttt gtttttggag atgaggttgt ttttttttgt tgggtgtagg 184440
gagggtattt tttatgtgag gggtttatga tttattttag agaaggggta ggttagtgag 184500
ttttttttgg atttgttatt ttttaaattt ttttagttga aaatatttat tatgttaagg 184560
tattgtattt tggagtgtgt gttttaagtt ttgttgtttt taattgattg ggtgaggttt 184620
atttttatta gagtatatag tttgttttat ttaggttttt gattgtattt tttttttgtt 184680
tgttttgaga atatttatta gtagtgtttg tggttgtagt gtttatttta agataaattt 184740
gttgggaagt attttgtttt tattattatt tttatattat tttagtatat tgattttgga 184800
aataaaagat attattttgt ttatagtatt ttatttttag tagtggtatt ttttttaaaa 184860
aaaaatggta atttttagtt gggtgtggtg gtttatgttt gtaattttag tatttgggaa 184920
agttgaggta ggttgattat ttgaggttag gagttggaga ttagtttggt taatatggtg 184980
aaattttatt tttattaaaa atataaaaat tagttaggtg tggtggtagg tatttgtaat 185040
tttagttatt tgggaggttg aggttggaga attgtttgaa tttgggaggt agaggttgta 185100
gtgagttgag attgtgttat tgtattttag tttggatgat agagtaagat tttattttaa 185160

aaaataaata aataaataaa atttaaaaaa aaatagtaat ttttgattgt tgaaaatgtt 185220
aaattttaga gaatatagta tttttataag tgatgttaat attgttttg attagttgtt 185280
ggttgaagat ttatttgatg aatttgattt ttttagaata gatgatttag atgattttgt 185340
tgttttgttt agaaataatt gtaggaataa tttaaaaaat tttttttttt tttaattttt 185400
ttgttttttg agatggattt tttttgttgt ttaggttgga gtgtagtggt atgattttgg 185460
tttattgtaa tttttgtttt ttgggtttaa gtgatttttt agttttagtt ttttgagttt 185520
agttgggatt ataaggtgtt tgttattata tgtggttaat tttttgtatt tttagtagag 185580
atggtgtttt attaggttga ttaggttagt tttgaatttt tgattttaaa taattggttt 185640
atgttggttt ttttaaagtgt tgggattata ggtgtgagtt attatgtttg gttagttttt 185700
atatttaatt ttttatataga gtatttagtt agatttgttt tagttttaaa gagtgtgttt 185760
atgtaaaatt aagtgagtgt tggtagtgag ttgaattttt taaaaggtaa aagggttaaa 185820
tattaaattt atttaaaaat atttttatag atatatttag aataatgttt gattggttat 185880
ttaggtgttt tatagtttag tttagttgat atagaaattt tttttttttt ttttgagatg 185940
gagttttgtt ttgttgttta ggttggagtg tagtggtgtg attttggttt attgtaagtt 186000
ttgtttttta ggtttatgtt attttttttgt tttagttttt tgagtagttg ggattatagg 186060
tgtttattat tatatttggt taattttttt ttttgtattt ttagtagaga tggggtttta 186120
ttgtggtttt aatttttttga ttttgtgatt tatttgtttt agtttttttaa agtgttggga 186180
ttataggtat gagttattgt gtttggttga tataggaaat taattattat agttatatag 186240
ttattgttat taattttagt atattttatt atttttaaaa gagattttgt ttttattagt 186300
taatttttat ttttaatttt gggtttttag tttttagtta ttgtttattt gtttttgttt 186360
ttatgaattt atttgtttta gatagtttgt ataagtggaa ttgtatagta tgtggttttg 186420
gtgtttggtt tttttttattt ggtagaatat ttttaaggtt tatttatatt gtagtaggtg 186480
ttggtgtttt attttttttt atggttgaat aatattttat tgtggataga ttatattgtg 186540
tatttttttta tttgttgatg gatatttggg ttgtttttgt tttttggtat aatttttgttg 186600
tgaagaattt tgtagtgagt gttggtttat aggtttttgt gtgaatatgt ttttattttt 186660
tttgggtaat tgtaagatta tgtggtttag ttaattagtt tttaagttat gtgtttgttg 186720
agggatttag gtttggtggg ggtgtattgt tgtgtttggt tagttaggtg ttgtgagttt 186780
gtgttgtagt tatttttttg tgttgtgttt tgaggttgtt ttgttggtat tgggtattgt 186840
gagttttagg aggttaaagt ttttttttgtt ttattttgag tttaggtttt gttttatgaa 186900
ttaggatgaa tggatattga attaatgtag ggatgggtgg ggttggggtg gggatgtaga 186960
gtgattttgg gggtttgtag ttgggttttg gttgttgatt tttagttttt tttgtttgtt 187020
ggagtttaga ataagttaga aaggggaaat gtaaagtttg agtgtttttt ttgtggtaga 187080
ttggtaggtt tgggtgggtg agttatttgt agttagttgt agtgtgagag aggaagtggg 187140
aggggaggga gagggtgagt ttggtgtaat ttatttttgg aaggaggttg ggttgaggtt 187200
gggggaagtg atttttattt ttttgagagg attttttttt ggggttaatt ttaaattaag 187260
ggagataaga ttttttttggg ggaggttgga agtgttgggg tttttaggtg ggtaggtttt 187320
ttagggttaa tgttttgggg aagttataag ttattatgtt tttggattgt tatatatata 187380
tatatatata tatatatata tatatatata tatatatata tatgagtaaa ttgtaatata 187440
aatgtgtgtt tatgaaagtg tttattttaa attattatat agtttgtaaa gtgaaggttt 187500
tggtttgtat ttgggtattt attgtttagt tagttggtag gtagtttgta ttgtagtttg 187560
ttattatttt ttagagatgt agttgaggag tttagttggt ttggagggtt tagtgggtgt 187620
agttttagga aagtggttag gtttgggtta ggtttttttt tttttttagt tttgggagaa 187680
attattatat gaatgtagtt tttttttgga gtttgggttg gaagggtttg ggaggggata 187740
ggtttttatt ttaggagttt ttaggttttg gttgttgttt gtgttagttg tgtgagtgtg 187800
agtgagttag gggttattat tgggtggaag gaagaagtgg gtattttttt taggaggtta 187860
ttgttggagt tttatagtag gttaatttta ggttttgggt ttttgggagg aggagaaaga 187920
agagaagtag gaggtgtgga gggtaggtta ggattagaag gtatataggg ttggggtagt 187980
gtgggagatt ttgggggttt ttttatttaa ttagttaata ttatagtgtt tataagaata 188040
tgttattatt gtagttttat tattttgttt tgattttaga aggtataatt gatgatttta 188100
gggaatggag aatttgagga aggttttgta ggagagggtt agatgtgatt gagtaaatgt 188160
gagttgtttg taaattgtag agtgtatgta tagtattggt tagttttggg gttagtgttt 188220
ttttggtgtt tagtttggtt tttgtatttt ttagtgtttg gttgggtttt gtgggggtta 188280
gggttgggtt tggtatatta gaggattttt attaagtttt tggtagtgtt ttagttatta 188340
aatttagttt gttagttatt aaatttgttt tgttggtttt tgaattattt ttttgttggg 188400
gttggttatg ttatgttagg ttttttatgt aggatggtag atggatagat gttgggatta 188460
agttattttt gggttgtgag atgtttgatt ttttgttggt gttttagaag atgttagttt 188520
tttgtggtat gtttttttatg tggtgtttgg ggattagtgg tttttaaggg gtttttttgt 188580
tttagtgtgg ggagtggttt ggttaaggtt ttagtgtttg gttggagtta ggtataggtt 188640

taggtgtatt ggttggaggt tttttgttta tgggttttgt ttgttttgtg tttttggttt 188700
ttattgtgtt ttttgtgttt ttgggagaga ttttaggggt tttgagttta gtattggggt 188760
tggggagtgg gtggggaaga gggtggggat tagggttttt ggttgtagaa tagttaggtt 188820
ttaagttttt attttatttt tttttattg ggtgttttat tttatattga ttattttttg 188880
aagaaaattt tttgaattgg tttttttattt tggttgtttt aggaagttta tttatttttt 188940
tgttttaatg ttttgtggtt ttagaaggat ggtggaagtt ggaaattatg agttttattt 189000
ataggtggtt atatattgaa gttgtggaga tgttgtggga aaggattttg gttagtgtgg 189060
ttggtataga gggagtggtg ttattggggt agatgggaat tgtggttgtt tttatgttgg 189120
tttgggagag atgaggggtt ggatgaaagg atagtagatt ggttatttat ttatttattt 189180
atttatttat ttatagtgtt ttattgggtg ttttggatgt gtaaggtttt gtgggtagtg 189240
aatgtgatag attttagata gtaattggag ggttatgtgt gtaagaattg agggatttag 189300
gttgggtgtg gtgttttaag tttgtaattt tagtattttg ggaggtagag gtgggtggat 189360
tatgaggtta ggagattgag attattttgg ttaatatggt gaaattttgt ttttattaaa 189420
aatataaaaa attagttggg tgtggtggtg ggtgtttgta attttagtta tttaggaggt 189480
tgaggtagga gaatggtgtg aatttgggag gtagagtttg tagtgagttt agatagtgtt 189540
attgtatttt agtttgggtg atagagtgag attttatttt aaaaaaaata aaataaaata 189600
aaataaaata aaaattgagg aatttagtgt agatgagtta ttttgggaaa ataagtggga 189660
gttttaggaa tagtatgggg aagggatggt tttggtaggg aggattttg tgggtagttt 189720
tgttggaaga gttgaaaggt agttagagag gttgtgtgtg gatggaagtt gaagagtagg 189780
ttaggggtgt gtgttttgtt aataaagatt ttaagtattt attgattagg tataatatta 189840
tgtgttttat aaataaatat tttatttgat ttttagtatt tttatgagat agatgttatt 189900
attttatttt atatgtaaga atattgaggt atagagtggt taagttattt gtttagggtt 189960
atatagtatg ttggtggtag gtaggatttt ggagttatag gaatttggta tattttaggt 190020
gtaatggaaa ttagttatgg ttttatagag ggaaattatt agattagtaa attttatata 190080
tgtttttttgg tgttttgagg aggagggatt tggatagggg tagttaatgg tggtggttgt 190140
gagatgtgtt gggttaatat tgatgagagt ttgggatgga tgagtggatt tggagtaatg 190200
gaaatggagg gtttaggttt ggtttttgga gatttagttg taatagttgt tggttggaga 190260
ggttgtttat ttagatgggg gatggggtgg gaggtaggtg agatgagagt tttagagttt 190320
agtttggggt atggttattg ggtttaagat gtttttagat gttggggttt gatagtgata 190380
gtggttgtag atatttagga tttagtagag ggtttggtat agtggggttt agggatatgt 190440
ttgtgagtta ggttggtatt taagtgtgaa tttggataaa gaaggtggta ttgttggtat 190500
tgaatgttta ggggttgggt gaagttagtt agggagaggg gtttttaagg tattaggatt 190560
gattttgtgg gtgagggtgg aggaggagat ggtagaggtt gagagaatag ttagagatag 190620
gaggaaggtt agtagagtta tgtatagaag tttggagaag atttgaggaa aggaagggtt 190680
gggtggttgt tattttttgt agttaggagg taagggtgat ttttgtgtgt tttatttttt 190740
agaggattgg tggttttatt tggttagaat tttaagttta gagatgtatt tgagttttat 190800
gttatggtgt aggtagtagg agttatggag taggtttgtg gggttatggt ttgtttgttg 190860
tttttttggtg tttttatttt gagtttaggg gttgtttgga tgttttgtgt tgtatttttt 190920
taggtggtgt tttagagagg atgaaagtgg ttattttaag gtgtgattag gtagtggttt 190980
atgtatattg ttttttttttg gggagagttt gggatggaag ggtatgtgtg tgtttttgag 191040
atttttttgaa taggttttat gtatttattt atttatttta ttatttattt atttatttat 191100
ttatttattt atttatgtat ttatttattt atttatttat ttatttattt gttaatttat 191160
ttatttattt atttatttat ttatttattt atttatttat agttatttat ttatttattt 191220
atttatttat ttatttattt atttatttat ttatttattt gtttatttat ttatttattt 191280
atttatttat ttatttattt atttatttat ttatgtattt atttatttat ttatttattt 191340
atttatttat ttatttattt atttatttat ttatttattt atttatttat ttatttattt 191400
atttatttat ttatttattt atttatttat ttatttgttt atttatttat ttatttattt 191460
atttatttat ttatttattt atttatttaa ttatttattt atttatttat ttatttatta 191520
tttgtttatt tatttattta tttatttatg tatttattta tttatttatt tatttattta 191580
tttgtttatt tatttattta tttatttatt tatttattta tttatttatt tatttattta 191640
tttatttttt tatttattta tttatttatt tatttattta tttatttatt tatttattta 191700
tttgtttatt tatttattta tgtatttatt tatttattta tttatttatt tatttattta 191760
tttatttatt tgtttattta tttatttatt tatttattta tttatttatt tatttattta 191820
tttatttatt tatttattta tttatttatt tatttattaa tttatttatt tatttattta 191880
tttatttatt tatttattta gttatttatt tatttgttta tttatttgtt aatttattta 191940
tttatttgtt tgtttattta tttatttatt tatttattta tttgtttatt tatttattta 192000
tttatttatt tatttatata tttatatatt tatttattta tttaattatt tatttttatt 192060
tttattttag aaagtatttg aagttgttgg ggtgtgtatt tggaaaatgt gttataagtt 192120

tttataggtt tttttttggt tttttattt ttttgattgg ttttttttt ggatatgtag 192180
gattttgagt agttttttg ggaggtgggt gtagtttagg taggttgttt gtaaagttag 192240
gattgaggga gttatattta aggtattgtg ggtttttaag gtgatggtat tgtgggttta 192300
ttgtttttag attttttaa tttttaggtt tttgagtagg aggattttg gggtagggtt 192360
tggagatggg aagagaaaat aatttagtt ttttaatttt tgttagttta ggtgggtgtt 192420
gtatggattg ggggtatttg gaatagttgg tttttttatt ttttttttt atttttttag 192480
ttttttggt tataattaga tagttgtgtg tgatgttttt ttttttttg aaggggttat 192540
ttagttaggt ttttatttat ggtttaggaa tgtgtttttt tttgtttgtt ttttattttt 192600
aagtaggttg tgtttttagt tttttgttgt ttttgaaggg tttgggtatg gggtagtttt 192660
tgtagtattt ttagggtttg agttgatttg tatagtagaa gggaattggg ggaaagtttt 192720
tttttattt agtatttggt aggggttat tttgttgtat tttagagatt tttgtttaag 192780
ttttgttatt ttagatgatt ttataagttg ggggaggtgg agttttagtt ttatagttt 192840
ttgttgttgt tttattgttg agttttgttg tgtatttgtt gataggtttg ttgatagtag 192900
aaggggttgg agagagggaa gttttgggtt ttgttttttt taatttttgt ttaggggata 192960
tttttgataa gaggaaattt tagtttgtga tagatggtta gtgtttattt atttatgggg 193020
gaattttttt tttttttgt aagtttgtag atttgttagg aattaaagga atttttggtt 193080
gataggttat tgtggatatg gttggagggt tggtatgggt tggattttgg ggttgttttg 193140
aggtaggggt tgtttttgtt ggaaggtttt ttggggtggg gaaggtatta gggttatttt 193200
ttgatttttt ttttgtagtg gatttatttt agagtttgag ataaggtaag ggggtagtag 193260
aaaatagaat ataaaatagt ttttttatt atatattgta ttagaagtta gaggatttat 193320
ttttggtttg tttttttgt agtttaagtt ttgagagtgg tttttagga agtgaatggt 193380
gatggtttgt aggttagagt ttgttttggt ttaagattga tgttttaata ggttgtggag 193440
tttgggtgaa aaaatttagg ttagggaggt ggtgggttgt aaatgaaaga atagaggagg 193500
gttttttta gaaagtggtg ggtggtgtga ggagtttttg gattttgttt aaatttgatt 193560
ttattgattg gggttaattg aaattgtgtt ggtagaaagg taaaattgtt tgagattttt 193620
tttttataa tagaggagag gtttgtttga aagggtttgg tgagggaaaa gttggaggaa 193680
ttttggttgg tagtaaaagt ggggattttt gttttttgtt ttgggggtgt tttggttttt 193740
gaatgaatga aagaggaggt tggatggaga tagatttata ttatttgttt tttttaggtt 193800
ttgtaaagga gtatataggg tttttgttgg tttttttatt ttgttagtta ttaagaagga 193860
gaaaatatta ataggggaat tgtaaagttg tagttttgtt taagttttat agtggaaatt 193920
ggaaagagtt ttagtttgga gaaaaatgta gtttttttgtt tttgtaaatt tgtgtttttt 193980
tttgggaggt tagagttatt gtgtttgatt tttaagtagg gttttagagt gttttaggag 194040
gggaaggtag agttttagat ttttggtatt ttaggtttta attaggtttt gaggagatgt 194100
tttttgtttt atataaagtg tgaagtgtaa tttgatttgg agttagttaa taaattagat 194160
gagttgtgtg tattttaaaa atttagtttt tgatttttta tttatatata tttgtataat 194220
ttggttattt atatttagtg tttttatttt tagaagagaa taggaagttt ttagtgatat 194280
ttatggagtg tttgtagtag tttgggtagg ttagtaggaa tataatgtag tgaaaagtag 194340
agttgagatg ggaaaatatt ttgtttttag agttttttag ggatgatatt ttttattgtt 194400
tttgtttatt tgttttggag tttattttgt aaatagtaat ttgtttttat ttgagagttt 194460
tttttgttg gagtttaggg atggagagta gttggtttta gtttttttt tttatttttt 194520
tgtttttag atttattttt tattttgaat aattgttttt aatttgtttt ttttttttt 194580
tagttgtaga atttagaatt ttgtgtgtgt ggttaaaagt ttagagagtg ttgtaggtaa 194640
tgttgagata attttattaa attttgtaag tttttattat attaagtttg tttgtttttt 194700
ttaattttta ttttatttta ttttatttta tagggatggg gttttgttat gttgtttagg 194760
ttggttttga atttttgggt ttaaatgatt tttttgtttt ggttttttag agtgttaagt 194820
tattttttta attgggattt atgggtttag ggattttttg atgaattgtt taaaatatgt 194880
gtgtgtttgt ttgtttgttt ttaatgtata tttttttagg gagagttttt atttttttatt 194940
agaggtttgt ggtatatata tgttttgtat tattgtatta gattgtgaat tttttatatg 195000
ttaaagattg ttttaaattt gtttggaagt tttggttaat aaatatttaa ataaatgaat 195060
tttaaatttt tatgtgttat tattgttttt attttgatta tttttaagtt tgtagtgtta 195120
ttttttgtt attgtgaagt agatatttaa taaatatttg ttaaatgaat taatgttatg 195180
gttttggttt attaggattt taggattatt gtttattatt attgtttata gttttttat 195240
tattttgttt tagagtagtt ttttttttga ggtttgttta tttttgtatg tgtgtttagt 195300
ttttagtttt gttgttttat tattttttt ttttttattttg tatttttttga attgttaata 195360
gttaataata gttttttttt ttatttattt atttatattt gaagtgagaa gttattattg 195420
aggtttttgt ggattttgtt agtttaattt ggggtggttt tttggaggtg gagtttttag 195480
atgagggatg gatattttgg tgtttgttgg gtagatttat ttgggagttt tggtaggggt 195540
tttagggtag ttttgttttt ttattttggt tgtgggattt ttggtgtatt agggtttttt 195600

```
aagtaagtat ttgtgttttg ggagagttat aggtaggttt gagagggttt ttggaatttg  195660
ggatttttag tttggtttag tagttggtat tattattttt tttgtaaggg aagttatgga  195720
aattaagatg ttaatgtgtt ttgataggtt tggggtggtt ttaagttttg ggaagagttt  195780
tggtaatttt ttttggttgg tgggtagaat ttttttttgg tagtttaggg ttttgttttt  195840
agtttttttgg gggagagtaa ttgagggagg agttggaggt taaggtttta gtgttgagtt  195900
gtttgtattg tagttgtatt ttttgggttt tggttgtagg aattttatat ttttttagtt  195960
tttttatggt ttttgaagat ttggtttggg gagggggtta gtagtttggg gtttatttat  196020
ttggttttaa attttggttt tattgtttat ttgttttgtg atttttataa ttgataaaag  196080
ttttttttggt tttggttttt ttatttgtga aatgggataa taatagtggt tattttatag  196140
gattgtgtga tgatttatta gttataatag tttttgagta gtgtttagag taatatttgg  196200
ggtttggtgt agtggtttat gtttataatt ttagtatttt gggaggttaa ggtgggagga  196260
ttgtttgagg ttaggagttt gaaattagtt tgattaatat tagtgagatt tattttttaa  196320
aaaaaaaaat tagagtaatg tttggtatag agagtttttta gtaaatttttg gttgttgtga  196380
tgagttttgt tttttattt gtttatttat ttattagatt ttttgttggg gtttgttgtg  196440
agttttatgt tgttttgaag aaagttgggt tggggtgagt gtgatggatg aggtgtttgg  196500
ttttataaat tttgtgtggg ggggttgtgg tgagaggaaa ttgtatatat tattatttag  196560
ttattattgt ggggagattg tttggatgta gagagtaggt tttgtgtggg agtggggagg  196620
gtaagttttg gttgtgaggt agggtttttt taggatttag tagggatttg aaggattttg  196680
taggtattta gggagatagg agaggaggag ggagtagttt tggttggata ttgttttttt  196740
agtattgttt gtattaggga tttatttagt tttaagaagt ttttttgtgg gggataggga  196800
gtatttgtta gtttatagga tttgaagtgt ttttatgggt ttaagttttt gggaaggttt  196860
gtaggtggtt gtagggttgt tgtaggggtg ttggttttag ggtttggatt taggggagtt  196920
tgtagaggga gggtagttgg aggttgtttt agtgtgtatt gttatgaggt aaagtaagga  196980
gattgttggg tttatgttgg gttggggtgg atggaggtaa ggaagttttt gttggggtaa  197040
gggtattagt tgtagatgtt ggtagttttt ttttggatat gggtttggaa ggttgatagg  197100
gtgtggtgag tgttattggt ttttttgttg tggtttggtt agtggtttta taggtttttg  197160
tgggtaggag gaggatgatt ttgtatttttg tttggttatt attggtagtg atagataagg  197220
tgtgtgggga tgtggttatt ttggtttgtg ttttgagagt taggtgtggg ttgggtttgg  197280
ggaagatagg ggaatggtat tgatttgatt ttgagtattt tgttttgggg tgtaggtttg  197340
gttgttattt ttttggagtt gggaatggta ttgggtggtg gtttagggtt ttttgtttta  197400
gtggtattat ttttgtattt ggttagtaag gttgatggga tttgtgtgga tatttttggg  197460
atgtagatta ggtagtatga tatataggga ggggtaggta gagaagggtg gtttttgggt  197520
tttggttttt aagttaggtt ttgtaagttt gttgtgttaa gtttttttttt ggtattaggt  197580
taggtgggtt agggttagtt ttggggttgt gatgagtgtg agtgttttta aagtgtgtta  197640
tgtttaatag ttgttttttt tttgggtatt gtttttagt tagatatttt atagtttttg  197700
ttttagttat agatagtaat ttgatttttt tatttttggt aggttttggg gttggggtaa  197760
gaatttgtta ttaagtgtag atgtttttag agggtgttgg tttagggttg tgtggattat  197820
agttttttta tttttgtttt agatttattt gtaattttgg agaaaagatg gtttagtgga  197880
tgtttgtggg gaggagggag ttggttttta ggatgagtag atttgggggt agtttttatt  197940
atgggattgg tgatttattg ttattagtgt tagtgaatgg tagaggggtt ttggtataag  198000
gtttgggttt agaatgggaa tttgtagaga aattgttaag gtttttttgtt atttgatatt  198060
tttgtttggt tgttatttta gaaatatttt tgttgtaaga agatgggggga gtttgtttgg  198120
ggtatggttt ggtgatatta atttgagaat tttaggttta agtgttggtt tttgggtttt  198180
agttgttttt tgagttgtaa agttggtttt tgggtatttg tggaaaatat tggatttttt  198240
attttatttt attttatatt ttgggatata tgtgtagaat gtgtaggttt gttatatagg  198300
aatataagtg ttatagtggt ttgttgtatt tattggattt tttaatttag ttttattaga  198360
aatgattgtt ttttttttt tttttttgttt ttttttttt ttttttttt ttttttttt  198420
tttttgtttt ttttttttt ttttttttt ttttttttt ttttttttttg tttttatggg  198480
gatataattt ttatattata ttatttgttg atttagaaag tatagttgag tggtattttt  198540
ttataatttt ttataagtat tatttttaat tttagaatgt ttttttttatt ttaaaaggaa  198600
atgttatttt tggtatatag ttattttttt ttttttagtt tttggaaatt attaatttgt  198660
gttttgtttt tatagatgta tttattttga atatatgatt agaattttaa aagatgtgat  198720
tttttgtgtt tggttttttg tagttggaat gtttttagag tttatttgtg ttggtgtatg  198780
tttgtgtttt tttatggttg agaaatagtt tgttgtgttg atttagtatg ttgggttttt  198840
ttatgtattt gttggtggat gtgttagtta tttttggagt tgggtgtgtt gtgttattta  198900
tgggttgtat taggagttgg ggatattatt gagtaagtta tagttttggg tatttaggag  198960
tttgtagagt aggagttggg agggaggtta ggaggtgtat ggtttgtttg ttaggttgga  199020
gagatttgta aaaagttatt ttgagttaga aattagagtt tgaggttttt gatttttagt  199080
```

```
tgtggagttg ggagggaagt tttgtatttt gttttgattg tggggtgatg tgttagttat   199140
tgtttaaatt agaatttttt tgagagttga tgtgggtatt gttgataatt agtttggata   199200
tttgaaagaa tggaaagtag ggatttagat agatttttgt ataattgtgt ttgtagtagg   199260
gtgaggtttg gaggtgtgat ggggatttta atggggtagt tgttgttttg ttggagttta   199320
tggtttttgg ggggggttat aggtgttaat tgtatattat ataagtagga gtgtgaaatt   199380
ttagggttgt taaatttagt gagggttttg tgttggtttg gttatttata gtggtatttt   199440
aggaaaaaaa attattttat ggatttagt tttttttatta gtaaagagta ggttggattg   199500
gaattttgta ttatttagta tagttgttat ttatgaaatg tggttattta aatttaaata   199560
aaaatagaaa ttttgttttt tagttggatt agtttagtgt taagtgttta gaagttattt   199620
gtgaatatat tgtggtaatt ttttaaaaaa tgaaaattag aattattgta gtattttata   199680
attttatttt tgggtgtgga tttaaaagaa tgtatagtat tttaaagagt tagagatatt   199740
tgtatattta tatttatagt ggttttgttt atagtagtta gtgggtagaa ataatttgaa   199800
tgtttgtggt taaatgaatg gagaaattaa aggtgggata ggttgggtgt ggtggtttat   199860
gtttgtaatt ttagtatttt gggaggttga ggtgggtgga ttatttgagg ttataagttt   199920
aagattagtt tgattagtat ggagaaattt tgtttttatt aaaataaaat tagttgggta   199980
tggtggtggg tgtttgtaat tttagttatt tgagaggttg aggtaggaga attgtttgaa   200040
tttggggagg tagaggtttt ggtgagttga gattgtgtta ttgtatttta gtttgggtaa   200100
taagagtaaa attttgttta aaaaaaaaaa aaaaaagggt ggaatatata aggaatttga   200160
tttagttgta aagaggaagt taggatattt gtaatagtaa gggtgaattt tttgggtatt   200220
atgttaagtg aaataatttg gttataaaag gataattttg taggatttta tttatattag   200280
gtttgtagag tagttagatt tagaaaaata gtagaatgga ggttgttaga ggtttggggg   200340
atggggttgg ggagttgttt aatgaggata gtttgtttta taagatgaaa aagttttgga   200400
gatttgttat ataataatgt gaatgtattt aatattattg gattgtatat tttaaaatag   200460
tgattgtggt aaatgttatg ataattaaaa ggaaaagtta tgtggttagt ggttattatg   200520
ttgagtagtg taggttttgg atggtgttgt ggttatagat aatggagtgg gtgattttta   200580
ttattgtttt tagttgtttg agtattatgg tgttttgggg ttaggatgaa agtgtttttg   200640
ggtatggggg tttttgtttt ttttatttgg gagttttgaa ggtagatttt aaagtttatt   200700
tggttaggta ggagagtgag gtgggttttt gtattgtttt ttgtgtgtgt gtttgtttgt   200760
gggttagttg gggtttgtaa ggttgttttt tgagggaggg gttgtaggaa tggtttttat   200820
ttgtgtagaa ggtaagttat tgttttatgt taatggtaaa gatggggttt ggttgtgttg   200880
tattattatt ttttaagtgg ggtttttggt gggtaggatg tttggtttga tttgagtttt   200940
tgtagtgagt gttggtgttt gtggagggat ttagttgtag ttgagttggt ttttttttttg   201000
ttttgggttt taggggtgtt gggggtgaga aaggagagtt tagggggtag tgtttttttt   201060
taggttttgt tggggagggt tgagggatgt tggggggagg tgggggtggg taggggttgg   201120
ttttagtttt aggtaaaatg gtttttagat ttataggtag atggaagtga tttttggtta   201180
tgtgggtggg gattgaattg ttgttttgta aggtaaggtt ttatgtttgg ttttaggaag   201240
tggttttaaa gttatatggt ttatggttag atgtagggtt gtgtttttgt ttatttaaag   201300
gttttggaag tttaggggag agtatttttg aagggggagt aggagtagga gtaaagtttt   201360
gaaggttttt aggttgtgtg gtttgggtgg gggttatgtg ggggtataga gagaggagtt   201420
ttggttttta ggtttgtttg tgggtattgt ttttagtagt ggagatgtgt ggattttttt   201480
ttggtatttt ttaataagga attatattgt tttttttgttt ttttaatttt ataagatagg   201540
aatttttgtg ttttgtggag gttgagaaga tggggaggtg tttgtgagga taaattagtt   201600
tttagttttt ggtttttaga gttttttttt tttttatttt tgatgtttta ggtttgggga   201660
gttttttttt gatatttttt gattgtttta gtgaaatagg ttgagaggga tggtttgaat   201720
tggttgggga tgtaggttgt agttaggggg tttagtgggt atttttttgag tttggttttt   201780
gttttgtaag tggaagggta gttgaattga agttttttat tgtttttttt ggtttggaag   201840
tttgtttttt atttttttggg tagagtgagg agtgggtgtg ggttgggagg gttgtttttt   201900
gtttgggagg gttgtttatt tgtttgttat gggggatgag tttttttgtag tttttttgtgt   201960
tgtggttttg gtttttatgt tggttttatt tttttgtggt tgtttttgtt gggtgtttat   202020
tggttttagg gaaaagttat tttttttttg gaggattggt gggaggggtt ttgtttggga   202080
gtgtgttgtg tggtttttgt tttagtgtag gatgtttgag gttagatttt ttttgggagg   202140
taggatgggg taaaagtttt tttgtaggta gagtagggtt tggatgggtt taagtaggga   202200
tgggggtata gaatagtagt tgtttagttt gtgtgggagt tgagggttat taagggtagt   202260
tttagtttgt ggggattagt agaggtatta tttttgtgat tagtgagttg tttgtatttg   202320
tatgtgtatg tattagtgag tgtggtgagt ttgaggtgtg ttgggatttg gtttgtgtat   202380
tagttagttt tgatgggaat tagttttatt ttgggtattt ttggaatttt attgtgtggg   202440
agtatttggg tttttagtag gtgtttttttg ggtgtttgtt tttagtttta ggtttttgat   202500
ttgtgtgtgg tgttttaata gtagttagga gtagggttat tgaagggatt tatagataga   202560
```

agaggtttta gtgattttgg gaagtttagt tttatgtgtt ttatatattt aagagtaaaa 202620
tttggttttg gtttgtgggt gtgtagtttg tgggtgttgg ttaggtatat aagatggttg 202680
tttgggttag tttaggggga ggtagatgtg gtgtaggggt ttttttttt tatgagttta 202740
ggaatagttt tgtatttagg tatttgtttt gtatttagta ttttttttt ttttaggatt 202800
gttggtttgt tttatgggat tgaatatata attttatggg gattgatgtt ggttttattt 202860
ttttttttta taagattatt tatggggatg tgggaggtgg ggatatatgt tggttttatt 202920
tttgtttttt ataagattat tttttttgtg ttttggtttg tggattggtt tttaaggttt 202980
ttgagtggta gtaggtggta tagtttattt gagtagaggt gaaggagagg gttaggttta 203040
tttgggtggt ttgaggtagt gtggagggtt aggagaggag agtatttttt atttttgttt 203100
gagtagtttt ttagggtatg gagataggag tttagggtta gttttgtttt ggagtgtagg 203160
gggtttttag tttggtatag tttttagaag aggagatgaa aaagtttggt tttgggagtt 203220
tggggatgga gttttgttgg ggaggtagtg tggttgtagg atggtagttt tttagaggat 203280
attatggtta gggatattta tttgggggaa tttttagggg ttgtttgggt aagttgttta 203340
gtttttttga gtttagtgt tttagttttt ttatttaagg atgggtgtga taattatttg 203400
gtaggtgttt gtgtgtgagt ttttgttttg ttttaatttt tttgttgttt ttttttagt 203460
tattgaggtg gtttttagtt gtgttggtga tatggttgat atttttagag atgttgggtt 203520
taagtaggtg tttgtattat ggaatgagaa ggttttggtg gattttggtt atgtggggat 203580
tgattttatt ttggagtaga tgtgttggaa ggttatgaag tagggttttg agtttaatat 203640
tatggtggtt ggtgagtttt tattttgtat gttatttaat taatgttgga aattagtttt 203700
agtttttagg gtggtttata agtttttggg tttggttttt tttttgtaa aatggggtag 203760
taattttgat tttaaggatg gattgttagt gatattgtgt gtgtatgtat gtgtttggga 203820
ggagtttata gtttttagtg gtttttaga ggggtttgtt ttgtatattt agggttttg 203880
agatgatttt ggggagttta ggttggtttg gagtaggttt tgatgagtgg ttgttgtttt 203940
tgagtaatga tgttagtttg tgtagagggt tttgtggtag ttgtttttt attgttgttt 204000
tagtttggtt ttttaggttt tttattgtat tttattgtat tttggttaga ggttttttag 204060
tagtttagtt ttagttttgt tttggaattg gggttttgtg gtttttatat tttttggtgg 204120
ttttggagag ttggaggtta ttatgggagg ttgttgatta gtatgatttg ggtagggtga 204180
gggggttagg ttggagttta tagtgggttg tttgttgttt ttaaagtttt ttaggtttta 204240
tttaaatttt atagggattt tgtgaagggg gtgttttgtt ttttaggtag aagtgaggtt 204300
tagggaaggt tgttaaggtt ttttagttta tagggtagag aattgtagtt attgttttga 204360
tttttagaat tgtgttttgt ttattttatg gggtttaggg aggggtttgg ggaagtgttt 204420
ttgttaggtt atattttaaa taaaggtgag tttgtggggt ttttgtaagg tgtttgggta 204480
ttttttaggt tgggtttgtt tttttgattt ttgttagttg tgtatgtttt ttttgtttag 204540
ttttgtggat gtaaatttat atttgttagt ttgatatttt ttttttttt tttgtattga 204600
tttgggtttt ggttttttg gggttttggt gttttttttg tttttgtag ttttatggga 204660
tttggttgtt tatggttttg ttgtgtttat agttgtttgg aagttggtag tttgttttaa 204720
gggagggtgg aggtgtgggg ttttttttta tggtttttat tttgttttgt ttgttgtggg 204780
ggatgtgttt gtatttgag gatttagggt tgttttttgg gaagatggtt ggagagaaag 204840
agaggggaga ggtttaaagg tagtggttag gttgggattt atttttgtgg gttatggagg 204900
ggttgttttg tttttagtt agggataggg tgagaaggat ttgggaattt ttgtgtttta 204960
gttatggtat aggttttttt ttggagtttt ggttttaagg agagggagag gtagtttagt 205020
agtttttgtt ttttgtggtt gttattgtga tagttttgtt ttttgggaat ggtgtgtagg 205080
aggttatttt atttgtttgg gtttttttgt ttttttaatt ttgagaagaa tagtggtttt 205140
gatttttat aaaattattt ttttttaggg atgttgagtg aggaataaga tgtggggtag 205200
ttttgttttt ttttttttt ttttttatgg tagttgtgtt tatttgtaga ggtgtaggta 205260
aggtggaggg gtatttgttt ggaggtttat agattttggg gagaatttta gttttgtttt 205320
aatttagtgg ggtgaggttt gtgtgttttt ttttgggttt tttttttgtg agttttagtt 205380
tttttgttta taaaatggga ttattttata aagtgagatt aagtgagtta ggttttgggt 205440
agggttttta ataggttgtg gttgtttgag aatttgtagt agtggtgatg gtggttgtgg 205500
tgatgaggat gatgggggtg gtaatgatgg tgatgatgat ggggtgatgg tgttagtgga 205560
gatgatgggg atgatggtga tggtggtggt gatagtggtg ggatggtagt ggtgatgtgg 205620
gtgatggtga ttgtgatggt ggtggtggtg gtgatggagg tgatggtgat agtgattatg 205680
agggtgatgg ggatggtggt ggtggtgatg gtgatagtgg tgggatggta gtgatgatgt 205740
gggtgatggt gatggtggtg atggtggtgg tggtgattgt ggtggtggtg gtgatgatgg 205800
tggtgatgat ggtgattgtg gtgatggtga tggtggtaat tgtgatggtg gtggtggtga 205860
tggtggtgat gatggtgatt gtggtgatgg tggttatgat ggtggtaatt gtgatggtgg 205920
tggtgatggt ggtgatgggg atgatgatgg tgattgtgat gggggtggtg gtggtgattg 205980
tgatggtggt gatgtgggtg gtggtggtga ttgtgatggt ggtgatgtgg gtggtggtgg 206040

tgattgtgat ggtggtggtg gtggttgtga tggtggtgat gtgggtggtg gtggtgattg 206100
tgatggtggt gatgtgggtg gtggtggtga ttgtgatggt ggtggtgatg gtggttgtga 206160
tggtggtgat gtgggtggtg gtgattgtga tggttgtgat tgtgatggtg gtggtggtga 206220
tggtgatgat ggtggtgatt gtgatggtgg tgatgggagt gatgttggtg attgtggtgg 206280
tgaaggggggt gatggtggtg attgtgatgg tggtgaaggg ggtgatggtg gtgattgtga 206340
tggtggtgaa gggggtgatg gtggtgattg tggtggtggt aatggtgatg gtggtaaagg 206400
ggtgatggtg gtgattgtgg tgatgtgggt ggtggtggtg atgtgggtgg tggtggtgat 206460
tgtgatgggg gtgatgatgg taatgtgggt gatggtgatt gtggtggtgg tggtgatggt 206520
gatgattgtg atggtggtga agggggtgat ggtggtgatt gtgatggtgg tgattgtgat 206580
ggtgatgtgg gtgatggtgg tgttgatggg ggtgatgatg ttagtagtgg tggtgatagt 206640
gatggggatg atggtggtgt tgatggtgat agtggtggga tggtagtggt gatgtggttg 206700
atgatggtgt tggtgttgat gattgtgatg atagtggtgg tggtgatgag ggtgatggaa 206760
gtggtgatga tggtgggagt gatgggatga tggtaatagt agtgatggta ttgtgatgat 206820
ggtgttgatg gggatggtgg tgatgataag ggtgatgatg atggtgatga taataataaa 206880
agtaataata ataaagggat atattttgta gtattttagt tggtattttt tagatttaag 206940
gtgttgtggt tggaatattg atataatttt tgatttgagg ttggttagga tatagatttg 207000
gtggatagag agagatggat gaggagtgtt tgtttttggg ttattaaata gtttaggtta 207060
gggattttta gtattagttt ttgttttttt aggagtttat atttttttta gaaaggtgtt 207120
attaaagtta gaatattagt gtaatttagg gtgtttttttg tgttgtgttt ttttgagata 207180
gggttttatt gttgtttagg ttggagtgta gtggtatggt tttgatttat tgtagttttt 207240
atattttggg tttaagtgat ttttgtgttt taggtatgtg ttattatatt tggttaattt 207300
ttgtgttttt tggtagagat agggttttat tatgttggtt aagttggttt tgaattttttg 207360
attttaagta atttattttt tttagttttt taaagtgttg ggattatagg tgtgagttat 207420
tttgtttggt tgatttttgt tttttaaaa gatagggttt ttgttatttt ggagtggtaa 207480
tgattttagt ttattgtagt tttgtatttt tgggtttgag taattttttt attttagttt 207540
tttgtgtagt tgggattata ggtgtttgtt attatattta gttaatgttt atattttgat 207600
gtagagatag ggtttttttg tgttgtttag gttggttttg aatttttggg tttaagtaat 207660
tttttttgttt tagtttttta aagtgttggg attataggta tgagttatta tatttggttt 207720
agggtatttt ttttggtgtg ttgttagtgt agtttaaggt agttttgtgt agggtaaagt 207780
aatggggttt tgagtttgga ggggtgtgtg tgtgtgtgtg tgtgtgtgtg tgtgtgtgtg 207840
tgtatgtgtg tgtgtgttat atgtgatttg tttgtgtgtg ttgtgagagt tgtgtgtgag 207900
gtatgtgtaa gaggtatgtt tgaggtgagt tgtgtgtgtg ttgtgagttg tgtatgtgaa 207960
gtgtgtgtgt tgtgaggtgt gtgtatgagg tgtatgtgag ttgtgtgtgt tgtgttgtat 208020
atgaatgtta ttttttttatt tttgtttgtt ttagttattt gtggattggt tgtttgtata 208080
tatggttatt gtttttttgag tggtattatt ttgtaggtta gtgtttttta gtatatggtt 208140
ttatagattt tatgggatat gtgtgagttt gtttgatgtt tatattagtt ttatgggggga 208200
aggattgttt attttatttt gtagattagg aaattgagat atggtgaggt taggtgattt 208260
gtttaggttt tggttggtta gggaggagta gtttgaatat ttgggttttg gttgtaatta 208320
ttttggtgaa ttgtaggaat tttggttatt tggtgtattt tgggtagttt tgggtttttt 208380
tgttagtttg gtttttgttt tgtttaaaat ttgagttatt aggagatttt ttgtttagaa 208440
agtataaggg gtataagaga tgttgatttt tttaggtttg gtttattttt taggagagga 208500
ttgtggtttg ggttgtgttg gtattagagt ttggttgtgt gggtttatat agttattgga 208560
tatttggttt gagggtgaag tttttggggg ttgtttggta gggatattgt tgggaggtag 208620
tttaggtatt gttgaatttt agattggaga gttttgtgtt tgggtgggag gggttttatg 208680
tgtagatttt gttggttttt ttggagagat ttttgattgg ttttttattt tttttttagg 208740
gtagagtggt ttgggtaaat ttattttaat taatattttt tttaaattta aaattagttg 208800
gaagttggtg tagtttattt tagaggagtg tatttttaag attattgaga ttaagtttat 208860
tatgtatggt tagtggttgg gagtgggttg ggggtgtagg atgtttttgt tttttttggag 208920
tatagggggtt gggggttaag attattatat atagttagtg gttaggggtg ggttgggggt 208980
gtaggatttt tttgtttttt tggagtatag agtggggggt taagattatt atatatagtt 209040
agtggttggg agtgggttgg ggatgtagga tgtttttgtt tttttggagt atagggggttg 209100
ggggttaaga ttattatata tggttagtgg ttggggatgg gttggggatg tgggatattt 209160
atgtttttttt ggagtattgg tgtggtgtga gggtgtttga gttaggtaat tttaggtttt 209220
taagatgggg atttgttgtg tttattattt ttagtatgtt gagtatgttg gggttttggt 209280
tgtgatgggt tagtgttttg gatttttgtg ggttttgtta tattgagttt tttatagata 209340
attgagtttt ttattaaaag tttgataggt aaggggtttt taggggaatt gttattggtt 209400
tgtgttggag ttggttgagg aaggttatta attttttggt gttttttttgg ttgttattgt 209460
tgtttatttg gggttgtttt ggggttagtt aggtgtgggg tgtttgtgag ggtttttttgt 209520

```
ttttttttg atttgtgtt tgtggttttg tgtagatatt gaggagaaag gtgtttggat 209580
gaagttgata gtgattgata tattagggtt tggggattat attaataatg agaattggtg 209640
aggtttttt agggggagga gtattagtgg gggttttagg gtttttgga ttttatttag 209700
agttagtttt agaggtttt tggtttgtgg gggtgtaggg tttatttttt gggatttgat 209760
atgttgttaa agttgtatgt tttaggggtt tgaaaatttt ggattaaagt agaattattt 209820
ggggaatttt gttgttttta atttattatt tgaggttagt ttggggaggt tggtttggga 209880
ggttgagggt aaggttgtgg tatttttgtt tagtaagtta gattttttag ggtatgtatt 209940
ttattggaag gtggggtgtt gggttttgag ttttgggaat gtatgatttg tttggggagg 210000
gtattttatg tgtttgttga tttgttttta tttttatgt agttggtagt ttattatgaa 210060
gtttattaat gattagtatg agaaatattt gtaggaggag gttaatatta attgtaagaa 210120
gtgtattttg gatatttgtg tttattgttg tttttatttt atttttgtta ttggttattt 210180
gtatgttttt gtagtgttgg tgttttatg ttgggtgttt tgggtttttt ttgtttttg 210240
ggattgtaag atggtgttgt ttgttttgtt gttgggtgta gaggggttga gttaggggtt 210300
atggttgtta gttatgaagt tgggggtgtg ttatgtttgt atttttaaag ttttatattg 210360
atttttatgt aagtgtttg tttagttttt attttttagt tgtgttttgg ggagttgagg 210420
gttaggtagg ttttgggttg ttttggagtt tggtggtttg tgttttgttt ttttaggtta 210480
ggttttggta ggtggagtgg gagtttttag ttaagtattt gtagtggtga ttgatttttt 210540
tatttgggtt ttgttttatg ggattttta gagggtttgg tgtgttttta gatgggaagt 210600
atagagatta gtgttggttt tttttagggt tggatggggt tgttgttggt gttggtgaga 210660
gttttttttt tttgttggtt ttgtttgtat ttgatgttgt ttttagagat tagtaagggt 210720
gggaggttgt atgtattttg gtaggtggat tttttatggt tttatggatt ttttgttttt 210780
agttttttgt gtatttttg ttagatattt gtggtttatt ttgagttagg gttgggagga 210840
ggaggattag aggttagtag gagtggttgg tggaggtagt tgtgggtggt ttgtttgtat 210900
gggtgggtgg agaaggtttt ttgtatttgt tgttttagat gtattttttt gggtggagga 210960
agtttagggg tttggggaaa ttatatttg tgggtattag gtgtaggaag gagtttggtt 211020
ggtgggtagt gggttttatt tagagtttgt gggaaaattt aagttggagt ttttgagttt 211080
atttttggt attatttttt gtgggttagt aggagttagg ggtagggggt tagtttgttg 211140
tttatgggtt tttttgaat tgaatatttg ttttttttagg tagaatggtt tatttgtggt 211200
tttgtggtag ttgttttagt tgttttaagt tttgttttag ttgaggttga tggtaggagg 211260
tagaagtttt ttttgatgtt agttgggtaa gttgagggtg ggtgggtggt ttgtggtatt 211320
tttgttattt tttagtattg atttttggt tgggatttta gttaggtatg ttgtttggtt 211380
ttgtttaagg tgtttagttt tatggggtag gggtgggttt tggttatttt tgtttaaatt 211440
tttgtttgga gtgggttttt ttttagttat gtgtgtgatt gatttatttt tattgtggga 211500
aaagtttggt tttagaattg gggaagatgg tgttttagtt ttagggtttt gttagttttt 211560
gttatttata gttttttta tattagattt taggtttatt ggggttaagt aggtaagggg 211620
agtagtttgt ttggtgtagg ggagtagttt gtttggtgta ggggtattgt atattggtgt 211680
tagtttttat tttttttgtt ttttttttt tagttaaagt tttatagtaa gatggttgtt 211740
gagttaatga tagtttttt gtgtggatat gtaggggtgt ggttgggatt ttagaattta 211800
tggatgaggg tgtttggagt tggttttggt ttgtttggtt tgtgtagtgg tgttatttgt 211860
gtttgggata tatttgtgtt ttttttttgt tttatattta tagtgtggtt gatttggttt 211920
ggggttttgt ttttgttagt agggattttt gtgagttttg agttagtatt tgagagtgtt 211980
gatatttgtt tgggggtggg tgtttttta ttgttttgtt tttttagatg agttttatgt 212040
atattttttt gttttagttt taggttttg gatattgagt ttatgaaatg tttgagtaag 212100
gtggttaata ttgtttttgt tattgttaag gtggatatat ttattttgga ggagagggtt 212160
tattttaaat agtgggtagg gttttatttt tatttgtttt agtttttttg tgtaatttgg 212220
agatgttgta ggtttggtag gggttatttt gtttaaagga gttatattgt tagggagatt 212280
gaattgttgg ttattttttt tagtgggtgg ttggtttggt ggtttttggt ttaggtggtt 212340
gttgggggtt gtttttgaga tggttttttt agtttttta tattttaaaa gtagttttgt 212400
gggagtgtta atttttagt agagttttg aggggggttt tggagaagat ttggattagg 212460
tagatggtga atttttttt tagggagtga ggttgagtag gggttgtgtg atatggttta 212520
ttttgtttta tgtttttttt ttttttttt gagaaagggt ttggtttagt tgttaaggtt 212580
gtaatgtagt ggtataatta tggtttattg tagtttttat tttttaggtt tagtttttga 212640
gtagttggaa ttatagatgt atattattat gtttggttaa tttttgtatt tttagtagag 212700
atgaggtttt gttatgttgt ttaggttggt ttttaatttt tgggtttaag ggattttttt 212760
gttttagttt tttaaagtgt tggggttata ggtatgagtt attttatttt gttttgtttg 212820
gtttttatta gatggaagag aagttgggtt aggtgtggtg gtttatgttt gtaattttag 212880
tattttggga ggttgaggtg ggtagattat ttgaggttag gagtttgagg ttaatatgat 212940
gaaattttat ttttattaaa aatataaaaa tattagttgg gtatggtggt gggtgtttgt 213000
```

agtttagtt atttgggagg ttgaggtagg agaatttttt gaatttggga ggtggaggtt 213060
ttagtgagtt gagattgagt tattgtattt tagtttgggt gatagagtga gattttattt 213120
taaaaaaaaa aaaaaaaaaa aaagttttag gagggatggt ttttgttatt tttttttttg 213180
ttattgtttg aggtttttgt tgtgggatag ggaggggtat ttaagtaaga gggtagtttt 213240
gggttttttt ttttgatagg gtagtatatt agagttatat ggggtgtttt ttaaaatgaa 213300
attgaggtgt ttgggttgtg gtattatatt tgttttggtg ggtgtgggtg gggtttgtaa 213360
tttattttgt tttggggtta ggtgttaggg attttttttag tatgtgtagg ggaagatagt 213420
ttatataaag tgggtgtgtt tgttggaggt tatatatggg ttgtggtttg tgtttgggta 213480
ggtgagtagt tgtttattgg gatgtttttg ttgtatgtat ttatgttggg ttgttggtag 213540
ggagttgaga ggttattgta ggtggggttt ttgttggaat tggggattgt gatgagggtt 213600
ttttaggaag ggttttagga ggggaggttt tggtaatttt gagtattttt ttggggttgt 213660
gatgagtagg agaagagagg tgggaggtgt ttaggatgat gattttaagt ttttggggag 213720
ttgtagtgtt gtttaggata gtaggtgtat ttgtagttgt tttttttttt tttaggaggt 213780
ataggagttg gaggtgattg gtgttatagt tttttagagt ttgtttttga atttgagttt 213840
ggggtagtat atagtgtgga ggttatgtgg taaatattag tgggtgggta gagtttgtta 213900
tagggatggg tttatttttt tttttttttta ttttagatta ttgtagattt gttgtttaat 213960
ggtattgatg tgtattttta gaaggaattt gatgaggatt tggaggattg gttggtgaat 214020
gagaagtttt gggtgagtgg atttattagg atgttgtttt taggggattt ttagtttttg 214080
ttgaagggtg ggttgggggt ttggaggatt ttaagttatg aaattatatt tttggggtta 214140
gagggtattg agtttaggtg tttgtattta gtgttgttag gttgaggttt ttgtttttgg 214200
gggattttag gtttagggta gtttttttttg ggggtttagg ggagggaatt gtttttttgt 214260
atatgtgtaa ttaatattgt ttgtttgttt tttaggagat gattttattt gttgtggtgg 214320
gtagtgatta tgagtattag gttaatggta agaggatttt tgggaggaag attaagtggg 214380
gtattattga aggtatttgt tgtaggtgtt ggggtttag atagatggga agatagtttt 214440
ttttgttgat ttagagtttg tgggttatgt ttgagttagg gatttgtgga attttattta 214500
ttgttttgtt ttatttagag ggagggggttt ttttgttttt atttaatttt tgggaaagtg 214560
agagggtaag tggtttgtgt agatgttttt attttagatt tttttttggg tagggaaaga 214620
tttagggaga taggagttgg gatggggggtt gttaagtttt ggattttggt gtaggttttt 214680
ttataggtat ttgtgtggtt ttggataaat tgtttaattt ttttgggttt agaaaagggg 214740
tgggttgtag ttttgtgagt tagatggatt tttggttttt tattttgttg aagtttttgg 214800
gatttttata ttttagtttt ttgagtggtt gttgtaatta attagtataa atgaggggat 214860
ttaaaataat ataaatgtat ttttatagtt ttagaggtta gaggtttgaa attaggggtt 214920
gttttttttt gaggttttga ggattagttt tatttatgtt tttttttttt tggtggtgtt 214980
tggtattttt agttgtattg taaaggtatt atatgtttta atttttgttt tattgttata 215040
gggttttttt ttttgtgttt ttgttttgtt tttttttttt tttttttttt tttttttttt 215100
ttgtttgaga tggagttttg ttttgttatt taggttggag tgtagtgttg taattttagt 215160
ttattgtaat ttttgttttt tgggtttaag taattttttt gtttttagttt tttgagtatt 215220
tgggattata gttatgtatt atttatttga tttaattttt gtatttttag tagagatagg 215280
atttgttat gttggttagg ttggttttaa atttttgatt ttaggtgatt tgtttatttt 215340
gtttggtttt ttaaagtgtt gggattatag gaatgagtta ttgagtttgt tttttttttt 215400
ttttgttttt ggataaggat gtttgttttt ggatttaggg tttattttaa gtggatgatt 215460
ttatttggag atttttaatg aatttataaa gaaagatgtt ttttaagtaa ggttataatt 215520
atgggtttta aggtttggat tttgattatg tttttgaatg gatgggagtt tttggggggt 215580
tatagtttaa tttatttttg gtggtgatat tttgggaggg atgggtttga atgttttttt 215640
tttttttttt tttttgtttt tttatttttt attagaatgg aagaggggaa ggtgtagagg 215700
gaaatgtagt aggaaaagtt attttgtttt gggagagtat ttggttgaaa ggtttagtag 215760
agtaggaagt ataagttttt taattttta gggttttagt ttttattatt tataaagtgg 215820
gtgtagtgtg ttaagatttt agtgagttga gagattgttt taaagattat agagtttttt 215880
gggaagttgt tgttttaaaa aaatggttat aatgataatt attaggaggt attagatatt 215940
tttgtgttat tggttagata tgggttattt tgttttatgt ttgaagtttt ttgtatttat 216000
tttttttgta gagttgaaga ggtgttgtga gtagaaaatt tgttaaggga tttagaatgg 216060
gaggtggttt tagaatttat agttttagtt ttgtattaga tttttttgag atgggagttt 216120
atgtttggag gagggtgtgg agaggtatat gggttttttt tttgtttgta tttttttttt 216180
attattattt ttgttgggtt tattttgtgg gagttgttta gttgggtgta gtttgggtgt 216240
tttttagtat ttgtttgtgg tgttaggttg gtttgttttt ttttggtttt tttgtttgta 216300
gttttttttt ttttttttgt ttaaagagtt tttgttgttt aggaaatttt ttatgaaagg 216360
taggtttggg agagttaggg atggatgggt ttgggatggg ttggtggttt tgttatgggt 216420
gtgtttttgt tgtattttgg tggttaattt tgagtattgt aggtggttgg tgatagaaat 216480

tgttaggaaa tgtataagag agaggttttt gtattttttt gatttgtgtg ttgttgagta 216540
ttattggttt tagttgagga agagtttgga atttgttagg atagttgtga ggtggtgggg 216600
tttatttttt agtagtttta tatgaggatt ttggaaatgt gtttttagta ttgttgggtt 216660
tttatattag gggttttatg agtttatagt tgatttgata tttggattag taagggttgt 216720
gttttgtttt tgttttttat ttggtgaata gtattttgag gtattttttt tatttttaga 216780
aattatttta agtattgtta atagaaatgt ggggtgtaat tgattatgtt atgaatttat 216840
ttaattttta tgtttttgag aggtgggtga tgttattatt tatattttat ggatgaggaa 216900
attgagggaa tgagagtaat ttgtttgagt tagggagtgg tgattatata ttttaatttt 216960
agtttgagag ttttattttt tttgttggtt tttgttgttt gttagtgtta gatgagagtg 217020
ttgttggtta gtttgtagga ggttttagtg tggatattgt tttatatagg gttgtggtag 217080
gagggtttag gtaaaggtaa ttggtatagt attgagagta tttgagggtt gggtaaggtg 217140
gaggttttgg ttttgtttgt tggttttatta agtaaaaatt agatattttt aatttgggga 217200
aaagttatgt ggttttgtga tggggtagtt tggagttagg tgtatgggtt ttgtattttg 217260
ttttagtttt atgtattgtg gagttgagat agatatagtt tttgtgtttt ttatgtttta 217320
tttttttttt tttttttttt tttttttttt tttttttttt ttgagatgaa gttttatttt 217380
tttgtttagg ttggagtgtt gtggtgtgat tttggtttat tataattttt atttttttggg 217440
ttttagtgat tttttttgttt tagctttttg agtagttggg attataggtg tttggtatta 217500
ggtttggtta atttttgtat ttttaataga gatgagattt tattatgttg gttaggttgg 217560
ttttgaattt ttgattttgt gatttattta ttttagtttt ttaaagtgtt gggattgtag 217620
atgtgagttg tggtgtttag tgtttatgtt ttatttttgt aagataatgg ggataataaa 217680
ggaattataa gagatatttg taggtgtgtg aagatgaagt ttattatatg ggagggtatt 217740
gggtgttttt atttgttttt tttgtttggt tttttatttt tagtgatgtt gtttattgtt 217800
tttttttgtta aaaggttttt tgttatataa tagttatata tgttaggtat tgtgtgttta 217860
tataggagtt atgaagtata gtaataaaat gattattttg agatggttat tagtttgagt 217920
ttgagggtat ttgtgggtgt ttgtggtttt ttgtttagtt tttttgagaa gtggttattg 217980
ttttgagttt gggtttgtat gtattgtgtg aagtgtatat gtttttgagt aatgtttagt 218040
attgttgatt tttgagtttg tgaatggttg tttatagtat gtagtttttg tgtgtgtttt 218100
tgtgtgtgtg tgttttgagt gtatataagg tggatggggt tgtgggggta atattttgtg 218160
ggttataggt ttttttttaaa attttagttt tgatttttgt tttattttat ttttagtagt 218220
ttagtttttt gtttgttttt aggtttttgt tgggatgttt tgtttttttt ggttatttta 218280
gtggtgtggg ggtttattgt tttggttttt ttattgtggt ttttttttat gtttttattt 218340
ggtttggtta ttgtgttatt ttttattagt taggaatttt ttggagagta ggagttgagt 218400
tgtttaggag taggagttgg tgttggttat tttagtattt tttagattga gggtagtgtt 218460
aggtgtttta tatttagttg tagagtaggt ggtttttttg tttttttgtt tatggggttg 218520
tttttagatt ttgttttttgg tattagtatt tttgggtagg gggagagagg tggggtttgg 218580
gtagagtttt tttattggga tattaaagtt tttttttgttt ttttttttag ttgaaaatat 218640
tatatattgt gagtttgttt atttgtggga ttttttttatt aggtgagaga tagggtgttt 218700
gggtggggtt gatggtttta tttttaagag ggttttatag tgggtggggg tagtgggtgt 218760
ggggttgaag ttttgggtag agtgggtgtt ttttgttatt tgtttgtttt gtttttggga 218820
agattttgga agtttatttt ttgaaaaaga aaatttattg ggaaatgaag taggtagtga 218880
agattttttt taattttttgt gaagttggtt ggatgggaag gttgagtttt gatttattgt 218940
ggtttggttt gttagagttg tttggtggtt attaagggtt tagtgaggtt ttatgtgtag 219000
gttttgttgg tagtgtgggg tgtttttagt tttgatttta ttggtttttg atttttattt 219060
tttttatttg gttgaggttt tttttttgttt tttttgagtt taggtaagga ggggaagttt 219120
tgatggagag ggtgttgggt gggtggtttt tggagtgtgg gttttggttt gggtaggttt 219180
tttttttagta agtagttggt atggatgatg ggaatgttat taagaggagg ggtttttagg 219240
ttggtggggg taggggtggg gggatttggg gagttgtttt gttattgtgg ggatattggg 219300
tttttttaatt tttttttaga ttattttttt tgagttgtag aggaatgaaa atttgagttt 219360
aggatttttt tttttttgggg ataagggtaa tttagttttt tgttttaggt tatttgtttg 219420
ttataggtta tagttttgga ggttggtggt tatttattgt ttttattttg ttagtatttt 219480
ggatattggt ttggagtaat aatgttttag ttttgttttt ttgagtttat gtattgtttt 219540
tgggttttgg atttagggtt atagggtgga tttagaggga gatatatgta ttggaatatg 219600
tgtgttttga ttgagtttgg gtttattagg gggattgatt tttttttttaa aatgtgtatt 219660
agatttggtt taatatggtg ggtttggggg attttatggg gagttaagta gttgggatgg 219720
ggagtggggg tgggggtagg tgggtttgag tttgaggtag gttgagtagg gtttttgttt 219780
tgttgttttt attttgttgt gtttatttta ttgatttgtt tgttttttat ttttatagga 219840
tgtatatgta gaatattaag gatattatta gtagtattta ttttgaggtg tattgtgtga 219900
agtgttttaa tgagggtagt agtgttatgg ttaatggtat ggaggagaag gagttagaag 219960

```
ttttggagat gtagatgtta ttttgtttat ttttgggatt ttgtttttaa gttatttttg  220020
ttttttttta ggttttttta ttattttatt ttattttata tgattttttt tatttgttat  220080
tgtttttttat ttttttgata tgttgttagg aaataaggga aggggttttt ttttgagtga  220140
gttagtgatg aggttgtggt tttttttgagg ttgtggggag gttgtattgg agttataggt  220200
aggggtgaga gtatttattg aattgatatg atttttttgtt tttaggtttg gtttttttgag  220260
ggtttagaag agtagttttg gtgtgtagat tatttgtttg tgtggggttt ttagtgttgg  220320
aggttttggg gtgggggtta ggttttgtat ttgtagagga gtttagtggg ttgtatgttt  220380
tttttttattt ttattggttt tgtttttttgg agtgtgttag agtttagggg agaatgtagt  220440
ttattaggag tatttggatt ttttgtttgt tatatggtgt ggttattatt tagtttttat  220500
ttttgttttg tttttaaggg tagaaaattt tagggttttt tgttattgat tgtttagtta  220560
ttttaagttt tttggtagtt gttttttttg gagtagaaag tgttttttatt ttagttattt  220620
gtagattgtt tggttagatg tggggatagg ttggaatgag ggaggtgttt ttattttttt  220680
gttatttttt ttttatgtta tttttgtttt tattgggttg taggtgttgt tgatgtgttg  220740
ggatttgatt gaggataaaa aggaaggaga gatgattttt attttttttat tttttagttt  220800
tgaaaaggtt taagtaagtg agtttggtgg aggagttgag ggagggagtt atggaaggtg  220860
ttagaaggaa ggttggtggg ggtatgtgtg ggttgtggtt tgggttggtt agagtggtgt  220920
gagttgtttg gtgtttgttt tgtttaagtg attagggaag tgtgtgtgtg tttatgtgta  220980
tgtgtgtttg tttgtttgtt tagtgtttgg gtttggttta agattggggtt gtagttatat  221040
taatgtttag ttagttattt ttgttattta ttttttttttgg tttaggtttt gttgattttt  221100
tgagttgggg aggtgggagg ttaggtgagt ttgattttgt tgatttattt gtgtttgggt  221160
ttttttttttt agagtttatg gtaatgaatt tttagaaagg agagaatggg tgttaggggt  221220
gtatagttta tagttgaaga gtaaggtttt gtggtagtat ggtttggttt tttatttttt  221280
gtttttatga ggggatttat gggggttgtt tttgtagggt atagatgatt aaagtttttt  221340
tttgtttttt gttatttgtt ttgtttttgg ggagaaagag gggtttgatg agattttatt  221400
taggtgtata tattattagg tgtatttgta ggtattgggt tggttgtttg tagttaggag  221460
aaggttagtg agaaggagtg tatgagtgtg agtgtgtgtg tatggaagtt ggggtattgg  221520
gtgtttgatt tttttttttat ttaagagagg aaggattttt tattattttt attggtgaga  221580
tagtttattt tgttaatttg ttatgttttt gttaaaatta agatttgaa ggaaatgagt  221640
ggttagtgtt agggtttagg ttatgtggtt tgtttagttt taatgttatt tggtggtggg  221700
gtggggtggg ggtgggtagt agtattttag ttttgagatg ttttattttt ttttttttgta  221760
attagatttt gaaaaattgt ttgttttatt aggttttgtt ttttaatggt tttttgttat  221820
gtgtttttttg agaaatttgt ttttttgtat aaatgataaa gtgttgaaaa tgtatttttt  221880
gaaataaatg ttttaaatgt agaaattta                                    221909
```

<210> 10
<211> 221909
<212> DNA
<213> Artificial Sequence

<220>
<223> chemically treated genomic DNA (Homo sapiens)

<400> 10

```
tgggttttttg tatttgaaat atttatttta ggaaatatat ttttaatatt ttgttattta     60
tataaaaaga taaatttttt gggaggtatg tagtaaaagg ttattgagga atagagtttg    120
atgaaatgaa taattttttta aagtttggtt atagagaagg aaagtgaagt attttaaggt    180
tgggatgttg ttgtttattt ttattttatt ttgttattaa gtgatattga ggttgggtag    240
gttatatggt ttgggttttg gtgttggtta tttatttttt ttaaaatttt ggttttggta    300
aaaatatggt aagttggtaa agtaaattgt tttattagtg ggggtggtga ggggtttttt    360
tttttttggg tggggaggga gttagatgtt tagtgtttta atttttatgt atatatattt    420
atatttatat attttttttt gttgattttt ttttggttgt aagtagttag tttggtgttt    480
gtagatgtat ttggtgatgt gtgtatttga gtggagtttt attaggtttt ttttttttttt   540
taggagtaag ataggtaata ggaagtagga agggattttg gttatttgtg ttttgtaaag    600
atagttttta tgggtttttt tgtggggata gagggtgagg ggttgggttg tgttgttatg    660
aaattttgtt ttttagttgt ggattgtgta tttttgatgt ttgttttttt tttttttaggg   720
gtttgttatt atgggttttg aggggagggg tttaggtatg ggtagattaa tagagttagg    780
tttgtttggt tttttatttt tttagtttag agagttagta aggtttgggt taggaagggt    840
```

```
gagtggtagg ggtggttggt tgggtattaa tgtaattata gtttagtttt gggttaaatt 900
tagatattag atagatagat ggatatgtat atatatggat atatatatat ttttttggtt 960
atttgggtag ggtaggtgtt gggtagttta tgttattttg attagtttaa gttatggttt 1020
atatgtgttt ttgttaattt ttttttggt atttttatg gttttttttt ttagtttttt 1080
tattagattt atttgtttag atttttttaa aattggggga tgagggggta ggggttattt 1140
ttttttttt tttattttta attagatttt agtgtattag tagtatttgt agtttggtgg 1200
gggtgggggt ggtgtgagag ggggatggta gggagatgaa gatgtttttt ttattttagt 1260
ttgtttttgt atttggttaa gtagtttgtg gatggttgag ataaaggtat tttttgtttt 1320
aggaggggta gttgttaggg ggtttggagt ggttgggtag ttggtggtag gggattttgg 1380
agtttttat ttttaggagt agggtagggg taggggttga gtgatggtta tattatgtgg 1440
tgggtagggg gtttaggtgt ttttggtggg ttgtattttt ttttgggttt tgatatattt 1500
taggggatag ggttgatggg gatggagggg agtgtgtagt ttattgggtt tttttgtaag 1560
tgtgaggttt ggttttatt ttaaggtttt tggtattgag aattttatat agatggatga 1620
tttgtatatt gaagttgttt ttttgagttt ttggggagtt aggtttgggg atagagggtt 1680
atgttaattt agtgggtgtt tttattttg tttgtggttt tagtgtagtt tttttataat 1740
tttggggagg ttgtggtttt attattgatt tatttggagg gaggtttttt tttttgtttt 1800
ttggtagtat gttgaagggg tggggaatga tgataaatgg agaaaattat ataaaataaa 1860
atggggtggt gggagggttt gggggggat ggaaatgatt tgggggtagg attttgggg 1920
tgggtagggt ggtgtttata tttttggggt ttttggtttt ttttttttta tgttgttggt 1980
tatggtgttg ttgtttttgt tgaggtgttt tatatggtat gttttgaagt ggatgttgtt 2040
ggtgatgttt ttgatgtttt gtatgtgtgt tttgtggggg tgggggtgg gtgggttagt 2100
gaggtgggtg tagtggggtg ggggtagtgg ggtaggggtt ttgtttggtt tgttttggat 2160
ttaggtttgt ttgtttttat ttttattttt tattttgatt gtttggtttt ttatggggtt 2220
ttttaggttt attatgttgg attagatttg gtatatgttt tgggggaagg gttagttttt 2280
ttggtaagtt tagatttggt tagaatatat atattttagt gtatgtgttt ttttttgggt 2340
ttattttatg gttttgggtt tagggtttag ggatagtgta tagatttaga gggatagggt 2400
tggagtattg ttgttttagg ttggtgttta gggtgttggt agggtggaag tagtgggtga 2460
ttattggttt ttaggattat ggtttgtggt aggtaggtgg tttggggtaa agggttgggt 2520
tgtttttgtt tttaggaaaa aaaagttttg ggtttggatt tttatttttt tgtagtttag 2580
gaggaataat ttggagggag gttggaaagt ttagtatttt tataatggta gaatggtttt 2640
tggaattttt ttattttgt ttttgttaat ttggaggttt tttttttga taatattttt 2700
attatttatg ttagttgttt gttgaggaag aatttgttta ggttagggtt tatattttag 2760
ggattattta tttagtattt tttttattag ggtttttttt ttttgtttgg atttaaggga 2820
aataaggaga agttttagtt aggtgaaagg ggtaggggtt agaggttagt gggattggag 2880
ttggagatgt tttatgttgt tggtagggtt tgtatatgag gttttattgg gtttttagtg 2940
gttattgggt agttttaatg ggttagatta tagtggatta aagtttagtt tttttattta 3000
gttaattttg taggggttga aaaggatttt tattgtttgt tttatttttt agtgggtttt 3060
tttttttaga agatgggttt ttaagatttt tttgagggta gggtaggtag gtggtagggg 3120
gtatttattt tgtttagggt tttggtttta tatttattgt ttttatttgt tgtagggttt 3180
ttttaggggt gaagttgtta gttttattta agtattttgt tttttatttg atgagaaggt 3240
tttgtaggta ggtaaattta tagtgtgtgg tgtttttaat taggagagga gataggaggg 3300
gttttaatgt tttagtggag aaattttgtt tggattttat tttttttttt ttgtttagaa 3360
atgttggtgt taaaagtaga gtttgagggt agttttatgg gtaggagggt aggggaatta 3420
tttgttttgt agttgagtat aggatatttg gtattgtttt tagtttgggg gatattggag 3480
tgattagtat tagtttttgt ttttggatag tttagttttt gtttttttaga aagtttttgg 3540
ttaatgggag atggtatagt gattaggtta aataggagta tggaaggaag ttatagtgga 3600
gaagttaggg tagtgggttt ttgtattatt ggggtgatta gggagagtag gatgttttag 3660
taggggtttg aagataggta gggggttggg ttgttgggaa tgggatgaag taggaattgg 3720
agttgggatt ttaaagaaaa tttgtggttt atagggtgtt atttttatgg ttttatttat 3780
tttgtgtgtg tttagagtat atatatatag ggatatatat aggggttatg tattgtggat 3840
gattatttat aagtttaaga attagtaatg ttaaatattg tttagggata tgtgtatttt 3900
atataatata tataagttta agtttgggat agtggttatt ttttagggag attgggtagg 3960
agattatggg tatttataag tgtttttggg tttgagttgg tggttatttt agagtggtta 4020
ttttattgtt gtgttttgta atttttatat gaatatgtgg tgtttggtat gtgtaattgt 4080
tatataatag aaggtttttt aataaggagg gtagtgaatg gtattattgg aggtggggag 4140
ttgggtaggg aaggtaaata aagatatttg gtgttttttt atgtgatgaa ttttgttttt 4200
atatatttgt aagtgttttt tgtgattttt ttgttatttt tattatttta tgaaaatggg 4260
gtgtagatgt tgggtattat ggtttatgtt tgtaatttta gtattttggg aggttgaggt 4320
```

gggtggatta tgaggttagg agtttgagat tagtttggtt aatatgatga aattttgttt 4380
ttgttaaaaa tataaaaatt agttaggttt ggtattaggt gtttgtaatt ttagttattt 4440
aggaggttga ggtaggagaa ttattggaat ttaggaggtg gaggttgtgg tgagttaaga 4500
ttatgttatg gtattttagt ttgggtaaaa gagtgaaatt ttgttttaaa aaaaaaaaaa 4560
aagaaaaaga aaaagaaaag aaaagaaaat gaggtataga gaatgtaaga attgtgtttg 4620
ttttagtttt atagtgtata aggttggagt gggatgtaga atttatatat ttggttttag 4680
gttgttttat tgtagagtta tatggttttt ttttaagtta aaaatgttta gtttttattt 4740
ggtaggttag taggtagggt tggggttttt attttgttta gtttttaagt gtttttagtg 4800
ttatgttggt tgtttttgtt taggtttttt tgttatggtt ttgtgtaggg tggtgtttat 4860
attagaattt tttgtagatt gattagtagt atttttattt aatattgatg ggtagtgaga 4920
gttggtagga agaatggggt ttttaggttg gagttggagt gtgtgattgt tattttttgg 4980
tttgggtaag ttatttttat tttttagtt tttttattta taaaatgtgg ataataatgt 5040
tatttgtttt ttagggatat gaggattaaa tgagtttatg gtgtgattaa ttatatttta 5100
tatttttatt gataatattt ggggtggttt ttaaggatgg gaaagatatt ttaagatgtt 5160
gtttattaag tgggaggtaa gaataaggta tagtttttat tagtttgggt gttaggttag 5220
ttatgaattt atggggtttt tgatataaaa atttagtagt gttgaaggta tatttttagg 5280
gtttttatat aagattgttg gaaagtgggt tttattattt tataattgtt ttgataggtt 5340
ttagattttt ttttagttgg agttagtggt gtttaataat gtataggtta gagaggtgtg 5400
gggatttttt ttttgtgtat tttttgatag tttttgttat tggttatttg tggtatttaa 5460
ggttggttat tagggtgtag tagaggtatg tttatagtaa agttgttggt ttgttttagg 5520
tttatttatt tttaattttt ttaaatttgt tttttataaa aagtttttta aatagtaaga 5580
atttttttggg tagagaggaa gagaggaatt gtaggtagag gggttaaggg gaggtaagtt 5640
agtttgatat tataggtgga tattggaaaa tatttaggtt gtatttagtt gggtagtttt 5700
tgtagggtgg gtttggtagg ggtggtggta ggggaggagt gtagataggg gaaaggtttg 5760
tgtgtttttt tatattttttt tttaggtatg aatttttatt ttgagggagt ttggtgtagg 5820
gttggagttg tagattttag agttgttttt tgttttgggt tttttggtag gtttttttgtt 5880
tatagtattt ttttaatttt gtaaggaggg tgggtgtggg gagttttgga tatgaaatga 5940
gataatttat gtttagttag tggtatagga gtgtttgatg ttttttggta attgttatta 6000
tgattatttt tttagagtaa tagtttttta aaaagttttg tggtttttgg gatagttttt 6060
taatttatta aaattttggt atattgtatt tattttgtgg atgatgaaaa ttgaggtttt 6120
ggaagattaa gagatttgtg tttttttgttt tattgggttt tttagttaag tgttttttta 6180
gaataaagtg gtttttttttg ttgtattttt ttttgtattt ttttttttt tattttaatg 6240
agaaatgaag ggatagagag aggaagggga aggaaatgtt tgggtttatt tttttttgggg 6300
tgttattgtt aggagtgggt tgaattgtgg tttttttaggg gtttttattt atttaaagat 6360
atggttaagg tttaggtttt ggaatttatg attgtgattt tatttggaaa atgttttttt 6420
ttatagattt attaaggatt tttaaatggg attatttatt tagggtaggt tttaagttta 6480
aagataggta tttttgttta aagataagaa ggaaagaggg taggtttggt ggtttatttt 6540
tgtaatttta gtattttggg aggttaagta aggtgggtag attatttgag gttaggagtt 6600
taagattagt ttggttaata tggtgaaatt ttgtttttat taaaaatata aaaattaagt 6660
taggtgggtg gtatgtgatt gtaattttag atatttggga ggttgaggta ggagaattat 6720
ttgaatttag gaggtagagg ttgtagtgag ttgagattgt aatattgtat tttagtttgg 6780
gtgatagaat gagattttgt tttaaataaa aaaaaaaaaa aaaaaaaagg aagaggaggg 6840
atgggataga gatataggga agaaggtttt gtgataatgg ggtagagatt agagtatgtg 6900
atgtttttat aatataatta ggaatgttag gtgttattag aaggaggaag gtatggatga 6960
ggttggtttt tagggtttta gaaggaaata gtttttgatt ttggattttt ggtttttgga 7020
attgtgaaaa tatgtttgtg ttgttttaag tttttttgtt tgtgttaatt ggttataata 7080
gttatttaag agattaaggt gtgagggttt taggaatttt agtagggtaa ggggttgagg 7140
gtttatttgg tttatagagt tatagtttat tttttttttta ggtttagaga ggttgggtga 7200
tttgtttagg gttatatagg tgtttatgaa agagtttgta ttaggattta gaatttggtg 7260
gtttttattt tagtttttgt tttttttgaat ttttttttgt ttggggggag gtttggggtg 7320
gaggtatttg tataggttat ttgttttttt atttttttaa gggttgaatg gggataagga 7380
gattttttttt tttgggtggg gtaaggtagt ggatgaggtt ttatgagttt ttggtttagg 7440
tgtggtttat aggttttggg ttagtagaag agattgtttt tttatttgtt tggagttttg 7500
gtgtttgtgg tgagtatttt tgatggtatt ttatttggtt ttttttttaa ggattttttt 7560
gttgttgatt tggtatttgt ggttattgtt tattatagta aatgggatta ttttttgggg 7620
aatgggtaga tggtattggt tatatatgtg tgggaaggta attttttttt ttgggttttt 7680
gggaggagtt gttttgagtt tggggttttt taaaaatgag agttttagtt tgatagtatt 7740
gggtatagat atttgggttt agtgtttttt ggttttaaga atataatttt atggtttaag 7800

gtttttaaa tttttaattt atttttagt aggaattggg ggtttttgg gaataatatt 7860
ttagtggatt tatttatttg gaatttttg tttattagtt ggtttttga gtttttatta 7920
aattttttt gggggtatat gttgatgttg ttggatagta ggtttgtggt gatttgggat 7980
aaggagggag agagatgggt ttatttttgt ggtaagtttt atttatttat tggtgtttgt 8040
tatatgattt ttatattgtg tgttgtttta ggtttgggtt taagggtagg ttttgggggg 8100
ttgtgatatt aattattttt aatttttgtg ttttttggag ggaagagggg tagttgtagg 8160
tgtatttgtt gttttgagtg atgttgtaat tttttagggg tttaaggtta ttgttttgag 8220
tatttttat ttttttttt ttgtttatta tagttttaag aaagtattta gggttattga 8280
gattttttt tttgaaattt tttttaaaaa atttttgtta tagtttttag ttttagtagg 8340
ggttttgttt gtagtaattt tttagttttt tgttagtagt ttgatatggg tatgtgtaat 8400
agaaatattt tagtgagtgg ttgtttgttt gtttaggtat agattatagt ttgtgtgtag 8460
tttttggtag atatatttat tttgtgtgga ttgttttttt ttgtatatgt tgggaaggtt 8520
tttgatattt ggttttagga tggagtaggt tataggtttt atttatattt attgaggtag 8580
atgtggtgtt gtagtttagg tattttggtt ttattttgaa aggtatttta tgtggttttg 8640
atgtgttgtt ttgttaggag gaagagttta gggttgtttt tttgtttgga tgttttttt 8700
tattttatag taggggtttt agatggtggt ggagaagaag gtggtagaga ttattttttt 8760
tgaaattttt ttttttttt ttttttttga gatggagttt tgttttgtta tttaggttgg 8820
agtgtagtgg tttaattttg gtttattgaa gtttttgttt tttgggttta agagattttt 8880
ttgtttagt tttttaagta gttgggatta taggtgtttg ttattatgtt tgattaatat 8940
ttttgtattt ttagtagaga tggggtttta ttatgttggt tttaaatttt tgattttaag 9000
tgatttgttt gttttggttt tttaaagtgt tgggattata ggtgtgagtt attatatttg 9060
gtttagtttt tttttattt aataaaaatt aggtagggtg aggtgaggtg gtttatgttt 9120
gtaatttag tattttggga ggttgaggta ggaggatttt ttgagtttaa gggttagaga 9180
ttagtttggg taatatggta aaattttgtt tttattaaaa atataaaaat tagttagata 9240
tggtggtgtg tgtttgtaat tttagttatt tggggattga gtttgggagg tggaggttgt 9300
agtgagttat gattgtgtta ttatattgta attttggtaa ttgagttaga ttttttttta 9360
aaaaaaaaaa gggggggtat aaaataaggt gggttatgtt gtatggtttt tgtttggttt 9420
tatttttgg aagagagatt tattatttgt ttggtttagg tttttttaa aattttttt 9480
agagatttg ttggagaatt ggtattttta taaagttgtt tttgagatat gggggagttg 9540
gagaggttat tttagggatg gtttttagta gttatttggg ttagggatta ttaggttagt 9600
tatttgttgg ggagagtgat tagtggtttg gtttttttga tagtgtgatt tttttagatg 9660
gggtggtttt tgttaggttt gtagtgtttt taggttgtat agaagggttg gggtaagtag 9720
agatggaatt ttatttgttg tttgaagtgg attttttttt ttagggtgag tgtgtttgtt 9780
ttggtgatga tagggatgat gttgattatt ttgtttaggt gttttataaa tttgatgttt 9840
aggggtttga ggttgaagta gagggatgtg tgtgaggttt atttgggga gtggggtagt 9900
ggggggtat ttattttaa gtaggtgttg atatttttag gtgttggttt ggggtttatg 9960
ggggttttg ttggtaagga tagggttttg ggttgagttg gttatgttgt gagtgtgggg 10020
tagaggaagg atatgggtgt gttttagata taggtgatat tattgtatag gttaggtggg 10080
ttagggttag ttttaagtat ttttgtttat gagttttggg gttttaatta tatttttata 10140
tgtttatata gaagggttgt tgttggttta gtagttgttt tgttgtgggg ttttggttgg 10200
gaggggagag gtaagagaaa tagaagttag tattagtgtg tggtgttttt gtattaggtg 10260
ggttgttttt ttgtattagg tgggttgttt tttttgtttg tttggtttta gtgagtttgg 10320
ggtttgatgt ggggaaagtt gtgggtggta ggggttggta gggttttgga attggggtat 10380
tatttttttt agttttggag ttgagttttt tttatagtga gaataaattg gttatatata 10440
tggttgggga gaagtttgtt ttgggtagaa gtttaagtag agatggttaa ggtttgtttt 10500
tgttttatgg gattggatgt tttgggtggg attaggtaat atgtttagtt ggagttttag 10560
ttaaagggtt agtgttgggg agtggtagaa gtgttataga ttgtttgttt gtttttggtt 10620
tgtttagttg gtattaggag aggtttttgt tttttgttat tagttttagt tgaggtaaag 10680
tttggagtag ttggaatagt tgttatagga ttgtaggtga gttgttttgt ttgggagggt 10740
agatgtttag tttaggagag gtttatggat agtgggttgg ttttttgttt ttgatttttg 10800
ttggtttata gaggatgatg ttagagggtg ggtttgaaaa ttttggtttg ggttttttta 10860
tagattttgg gtgaggtttg ttgtttatta gttaggtttt tttttgtatt tggtgtttat 10920
agagtatggt ttttttaaat ttttgggttt tttttgttta gggaagtata tttgggatag 10980
taggtgtgaa ggattttttt tatttattta tgtagataag ttatttatgg ttgtttttat 11040
tagttatttt tgttggtttt tggttttttt tttttagtt ttggtttagg gtgagttata 11100
ggtgtttggt aggggtgta tagggagttg aggatagagg gtttgtgagg ttatgaagag 11160
tttatttgtt agggtatatg tagtttttg tttttgttga tttttagggg tgatgttagg 11220
tgtggataga gttggtggga ggaaagggtt tttattaata ttaatagtag ttttgtttag 11280

ttttggaagg ggttagtatt gatttttgtg ttttttgttt aagaatgtat taaatttttt 11340
aaggggtttt atgaggtagg gtttggatga gaaggttagt tattgttatg ggtgtttggt 11400
tgggggtttt tattttattt gttagagttt ggtttgagaa ggtaggatat agattattag 11460
gttttagaat agtttagggt ttgtttgatt tttagttttt taagatgtag ttgaaggatg 11520
ggggttgggt aggatatttg tataggggtt agtgtgaggt tttggaggtg tagatatggt 11580
atatttttaa ttttatagtt ggtggttatg gtttttgatt tagttttttt gtatttagta 11640
gtagggtaga tagtattgtt ttgtagtttt aggggataga gggaatttag ggtatttggt 11700
atgaggatgt tgatattgta gggatgtatg agtggttggt ggtggggatg aagtagaggt 11760
agtagtggat gtgggtgttt gggatgtgtt ttttgtggtt gatgttgatt tttttttgta 11820
ggtatttttt gtattggtta ttgatgaatt ttatgatggg ttgttagttg tgtgggggat 11880
ggggatgagt tagtaggtat atgagatgtt ttttttaagt aggttgtgta tttttaggat 11940
ttagagttta atattttatt ttttaatgaa atgtatgttt tgggaggttt ggtttgttga 12000
gtggagatgt tatagtttta tttttggttt tttaaattag tttttttggg ttggttttag 12060
gtgatgagtt ggggatagta agatttttta ggtaattttg ttttggttta gaatttttag 12120
gtttttgaga tgtgtggttt tggtagtatg ttaggtttta ggaggtgggt tttgtatttt 12180
tgtgggttgg gaaattttta gggttgattt tgggtggggt ttagggagtt ttgaagtttt 12240
tgttagtgtt tttttttttg gaggggtttt attagttttt gttgttgatg tggtttttga 12300
atttggtgt gttaattatt gttagtttta tttggatgtt ttttttttta atatttgtat 12360
agagttatgg atgtagagtt agaggggaga tggaagattt ttatagatat tttatatttg 12420
attgatttg gggtaatttt aggtaggtag tgatgataat taaggagata ttgggaaatt 12480
aatggttttt tttagttaat tttggtatag gttggtgatg gtttttttgg gagtttttg 12540
tttgttagat ttttggtgga aaatttagtt gtttgtgggg agtttagtgt gatggggttt 12600
ataggggttt gggatattga tttattatag ttagagtttt aatatgttta atatgttggg 12660
gatggtgggt atagtgagtt tttgttttgg ggatttggga ttgtttgatt tgggtatttt 12720
tatattgtat tagtgtttta ggaaggtatg ggtattttat atttttagtt tatttttagt 12780
tattgattgt gtgtgatggt tttgattttt aatttttgtg ttttaggaag gtaggggtgt 12840
tttgtatttt tagtttattt ttggttattg attgtgtgtg atggtttgga tttttattt 12900
tgtgttttag gaaggtagag gagttttgta tttttagttt gtttttggtt attgattgtg 12960
tgtgatggtt ttgattttta atttttgtgt tttaggaagg taggggtgtt ttgtattttt 13020
agtttatttt tggttattga ttgtgtgtga tggatttgat tttgatggtt ttggggatgt 13080
gtttttttga ggtgggttgt attgattttt ggttgatttt ggatttgaag agggtgttga 13140
ttaaggtgga tttatttaag ttgttttgtt ttggggagag gatgggaggt tggttagggg 13200
tttttttgga gagattagta gggtttgtat atggggtttt ttttatttag gtatgaggtt 13260
ttttagtttg agatttagta gtgtttgggt tattttttgg taatattttt gttaagtagt 13320
ttttgaggat tttattttttg agttgggtgt ttagtgattg tgtaaattta tatagttggg 13380
ttttgatgtt agtataattt aggttatagt ttttttttga gaggtgagtt gggtttggga 13440
gagttggtat tttttgtgtt ttttgtgttt tttggatagg aggtttttttg gtggtttaga 13500
ttttggatag gatagaggtt gggttggtgg aggagtttag ggttgtttag ggtgtattag 13560
gtggttggga tttttgtagt ttattagggt ggttgtaatt aaagtttagg tgtttaaatt 13620
gttttttttt gattggttag ggtttgggta agttatttga ttttattgtg ttttagtttt 13680
ttgatttgta aaatgggata aatagttttt tttttgtgga gttggtgtga gtattaggta 13740
ggtttatgta tgttttatgg ggtttgtggg gttatgtgtt agggggtgtt ggtttgtagg 13800
gtggtgttat ttgagggata gtgattgtgt gtgtagatag ttagtttata ggtgattgag 13860
ataaatgagg atgaggaagt agtatttata tgtaatataa tatatataat ttatatatat 13920
tttatatata tattttataa tatatatatt ttatatgtat aatttataat atatatataa 13980
tttattttaa atatattttt tatatatatt ttatatataa tttttataat atatataaat 14040
aaattatata taatatgtgt gtgtgtgtgt gtatatatat atatatatat atatatatat 14100
atatattttt ttagatttgg agttttattg ttttattttg tatagagttg ttttgggtta 14160
tgttagtaat atattaaaaa aaatatttta ggttaggtgt ggtggtttat gtttgtagtt 14220
ttagtatttt gggaggttga ggtaggagga ttgtttgagt ttaggagttt aagattagtt 14280
tgggtaatat agagagattt tgtttttata ttaaaatgta aatattggtt aggtgtagtg 14340
gtgagtattt atagttttag ttatgtagga ggttgaagta ggagaattgt ttaagtttag 14400
gagtgtgagg ttgtagtgag ttaggattat tgttatttta gagtgataga gattttgttt 14460
tttaaaaaaa taaaaattgg ttaggtgaga tggtttatgt ttgtaatttt agtattttgg 14520
gaggttgagg agggtggatt atttgaggtt aggagtttga gattagtttg gttaatatgg 14580
tgaaattttg tttttattaa aaaatataaa aattagttag gtatggtggt atatgtttga 14640
ggtatgagaa ttatttgaat ttaggatgtg gaggttgtag tgagttaaga ttatgttatt 14700
gtattttagt ttgggtaata atgagatttt gttttaaaaa aatataatat aaaaaatatt 14760

ttaaattata ttgatgtttt gattttgata atattttttt aggaaaagtg tgggtttttg 14820
ggaggataga ggttggtgtt gggaattttt ggtttgaatt gtttagtagt ttaaggatgg 14880
atattttttg tttatttttt tttgtttatt aggtttgtgt tttggttaat tttaggttag 14940
gagttatgtt aatgttttag ttatgatatt ttagatttag gagatgttaa ttgggatatt 15000
gtaaggtgta ttttttttatt attgttattt ttattattat tattattatt attattattt 15060
ttattattat tattattttt attagtatta ttattataat attattattg ttattattat 15120
tattttatta tttttgttat tattattatt tttgttattt ttattattat tattattgtt 15180
attataatta ttagtattag tattattatt aattatatta ttattattat tttattatta 15240
ttattattaa tattattatt atttttatta ttattattat tattattaat attattattt 15300
ttattaatat tattattatt tatattatta ttataattat tattattata attattatta 15360
ttattttttt tattattatt ataattatta ttattattat tattattata attattatta 15420
tttatattat tattattatt tttattataa ttattattat tatttatatt attattatta 15480
tttatattat tataattatt attattattt ttttattatt attattatta ttattattat 15540
aattattatt attattttt ttattattat tataattatt attattattt tttttattat 15600
tattataatt attattatta ttttttttat tattataatt attagtatta tttttattat 15660
tattattata attattatta ttattattat tattattatt attattataa ttataattat 15720
tataattatt attatttata ttattattat tataattatt attgttatta ttattataat 15780
tattattatt atttatatta ttattattat aattattatt attatttata ttattattat 15840
tataattatt attattatta ttataattat tattattatt tatattatta ttattataat 15900
tattattatt atttatatta ttattattat aattattatt attattttta ttataattat 15960
tattattatt tttattatta ttattattat tattattata attattatta ttataattat 16020
tattattata attattatta ttattattat tattattatt attattataa ttattattat 16080
tattattatt ataattatta ttattattat tattattatt attattatta taattattat 16140
tattattatt attattatta ttattattta tattattatt attattttat tattgttatt 16200
attattatta ttattatttt tattattttt atgattatta ttattattat ttttattatt 16260
attattatta ttattataat tattattatt tatattatta ttattatttt attattatta 16320
ttattattat tattattatt tttattattt ttattagtat tattatttta ttattattat 16380
tgttattatt atttttatta tttttattat tataattatt attattattg ttgtaaattt 16440
ttgaataatt ataatttgtt ggggatttg tttggggttt agtttgtttg attttatttt 16500
atgggatgat tttattttat ggatgaggaa gttaaggttt atagaggagg agtttagggg 16560
aagatatata agttttattt tgttgagtta ggataaagtt gggatttttt ttggggtttg 16620
tggattttta aataggtgtt tttttatttt gtttgtattt ttgtaggtgg gtatagttgt 16680
tatgggaggg aggagggagg agagtagagt tgttttatgt tttgtttttt atttggtatt 16740
tttgggaggg agtgatttg tggggaatta ggattattgt ttttttagg gttgaaaggg 16800
tgaagaggtt tagataggtg aagtggtttt ttgtgtatta tttttagagg gtaaggttgt 16860
tatagtggta gttatggaag gtgagggttg ttgggttgtt tttttttttt tttggggtta 16920
aagttttgga agaaggtttg tgttatggtt gggatgtaga ggtttttagg ttttttttat 16980
tttgtttttg gttagggagt agggtagttt ttttgtagtt tatagagatg agtttagtt 17040
.tggttattgt ttttgggttt tttttttttt tttttttta gttatttttt tgggaggtag 17100
ttttgaattt ttagggtata agtatgtttt ttataatagg tagaataggg tgggggttgt 17160
aggaggggat tttatattt tgtttttttt tgaagtaggt tgttagtttt taggtagttg 17220
tgagtatagt agggttatga gtagttgggt tttatgaggt tataggaagt agagaggata 17280
ttaagatttt aggggagtta gggtttagat tagtgtagaa gaggaaagaa aggtgttggg 17340
ttggtaggtg tgggtttgtg tttatagagt tgggtaggaa ggatgtgtat ggttggtaaa 17400
ggttagagaa ataggtttgg tttgagaggt gtttagatat tttgtagaaa ttttgtaagt 17460
ttattttgt ttagggtgtg atttggtggg ggtatttttt tagatttttt tttggatttt 17520
gtggagtggg tagaatatag ttttgggagt taaaatggtg gttgtggttt tttgttttgt 17580
gagttaagga atttggtag tttttttga gttttgtttt tgtttggaaa atgggatgtt 17640
tttttatag ggtttttgtg aggtttgaat gggatttggg gggttttgga aataataggt 17700
ggtttattgt agattttagt ttggtttttt tattttgttt aggttgtgtt gattgatgat 17760
tttttgtggt gatttttagt tttttggggt tattaggggg tgtggaagtt atgaggtttt 17820
aattttaggg tagagttgaa gttgagttgt tgggaagttt ttggttgggg tgtggtgggg 17880
tgtggtgggg agtttggagg gttaggttgg agtaatagtg aggggatagt tgttatagaa 17940
tttttttgtat aagttgatgt tattgtttag aggtagtgat tatttattag gatttgtttt 18000
aggttggttt gggtttttta aagttatttt aggggttttg gatgtgtagg ataggttttt 18060
ttgaggagtt gttggaggtt gtgggttttt tttaggtgta tgtatgtata tgtaatgtta 18120
ttggtaattt gtttttgagg ttagggttgt tgttttattt tatagaaggg aagattaagt 18180
ttaaaggttt gtgggttgtt ttgggggttg aggttggttt ttggtattgg ttgagtggta 18240

tgtaaggtga ggatttattg attattatga tgttgaattt gaagttttgt tttatggttt 18300
tttggtgtat ttgttttagg atggagttaa tttttatgta gttgaagttt attggggttt 18360
ttttgttttg tgatgtaggt gtttgtttga gtttggtatt tttgggggtg ttggttatgt 18420
tgttaatgta gttgggagtt gttttagtgg ttggggaagg aatagtagag gagttagagt 18480
ggaatagggg tttatatata ggtgtttgtt aggtggttgt tatatttatt tttggatggg 18540
gaaattgagg tattgaggtt tagaaaggtt aagtgatttg tttaggtgat ttttgagaat 18600
ttttttgggt ggatgttttt ggttgtggtg tttttaaaa agttgttatt ttgtagttat 18660
gttgtttttt tagtaaagtt ttattttttag atttttaagg ttaggttttt ttattttttt 18720
ttttaggagt tgtgttaagt tggggttttt ttgtgtttta aggtagggtt gattttgaat 18780
ttttgttttt gtattttgga gggttgttta gataagggtg gagggtgttt ttttttttttg 18840
gtttttata ttgttttggg ttatttaggt ggatttggtt ttttttttta tttttattta 18900
gataagttgt attgtttgtt gttatttaaa ggttttagaa gttgatttat gagttaagat 18960
ataggaggga tggttttgta agaaatgggg atagaattag tgtgtgtttt tgtttttttat 19020
atttttatgg atggttttgt aagaaagggg gatagaatta gtgttggttt ttatggagtt 19080
gtgtgtttaa ttttatagaa tgagttagta gttttgggag aaggaaaagt gttgggtgta 19140
gggtgggtgt ttggatgtag agttgttttt gggtttatgg aggagggggg tttttgtatt 19200
atatttgttt tttttggat tgatttaggt ggttgttttg tgtgtttggt tagtgtttat 19260
gggttgtata tttataggtt aagattaagt tttattttttg ggtatgtggg gtatgtggag 19320
ttgaattttt tgaaattatt aaagtttttt ttgtttgtag gtttttttga tggttttgtt 19380
tttggttgtt gttaagatat tatatatggg ttagaggttt ggggttgggg gtggatgttt 19440
ggaagatgtt tgttgaggat ttaggtgttt ttatatagtg gggttttagg ggtatttagg 19500
atggggttgg tttttattag ggttggttgg tatatgggtt aggtttagt atattttaga 19560
tttgttatat ttattggtgt atgtgtatgt aggtgtagat aatttgttgg ttatagaggt 19620
ggtgttttttg ttagtttttta taggttggag ttgtttttgg tggtttttag ttttttatgta 19680
ggttgggtgg ttgttgtttt gtgtttttgt ttttgtttgg gtttatttag gttttgtttt 19740
gtttgtgggg agatttttgt tttattttgt tttttaggga ggatttggtt ttaggtattt 19800
tgtgttaaga tagggattat atggtatgtt tttaggtagg gttttttttg ttagtttttt 19860
gggaggggag tggttttttt ttgggattgg tgggtattta gtaggggtag ttatagggga 19920
gtggagttgg tgtgggagtt agggttatag tatgggaggt tgtagggggt ttatttttttg 19980
tgataggtaa gtgagtagtt tttttagata ggaggtagtt tttttggttt atgtttattt 20040
tttattttgt ttagaaggtg ggaggtaggt ttttaggtta ggaggggtag tgggagattt 20100
tagtttaatt gtttttttat ttgtggggta agaattgaat ttaggaaatg tttattgagt 20160
tttttggttg tagtttgtat ttttagttga tttaagttat ttttttttggt ttatttttatt 20220
ggagtggtta gagggtgttg gagggaggtt ttttaggttt gaggtattag aggtgagaga 20280
ggaaaagatt ttggaagtta gaaattgggg gttggtttat ttttataggt attttttttgt 20340
ttttttagtt tttatagggt ataggaattt ttattttatg gaattggggg aataaaggaa 20400
taatataatt ttttgttggg aaatgttaag ggggggttta tatattttta ttgttgagag 20460
taatatttat aggtggattt ggaggttagg gttttttttt ttgtgttttt atgtggtttt 20520
tatttaggtt atgtagtttg gaagttttta gggttttgtt tttgtttttg tttttttttt 20580
agaaatattt tttttgggt ttttaggatt tttaaataaa taaagatata gttttgtatt 20640
tggttatgga ttatgtggtt ttggggttgt tttttgaagt tagatatggg gttttgtttt 20700
gtggggtagt agtttagttt ttatttatgt gattgaaaat tgtttttatt tgtttgtggg 20760
tttgagagtt attttgtttg ggattgggat tagtttttat ttgtttttat tttttttttag 20820
tattttttaa ttttttttag taggatttgg ggggaggtgt tgtttttttga attttttttt 20880
tttgttttta atgtttttgg agtttagagt gagggagggg ttggtttagt tgtagttggg 20940
tttttttata ggtattagta tttattgtag aaatttaaat taaattaaat attttgttta 21000
ttaggggttt tgtttgagaa ataataatat aatgtagttg agttttattt ttgttattga 21060
tgtagggtag tggtttgttt tttgtatagg tgagagttat ttttgtaatt tttttttttga 21120
gaggtggttt tgtaggtttt ggttggtttg taggtaggta tgtatgtaga gagtaatgta 21180
agagtttgtt ttgttttttt atttggttag gtgagtttttg gggtttgttt ttaggatttt 21240
taagtgggaa aagtagagat ttttatattt agagatattt ttattttggt tttaggatat 21300
tatggtgttt agatagttgg aaataatggt aaagattatt tattttattg tttgtgatta 21360
tgatattgtt tgaggtttgt gttgtttagt gtggtagtta ttggttatgt gattttttttt 21420
tttaattgtt ataatgtttg ttatggttat tattttgaag tgtgtagttt agtggtgtta 21480
agtatatttg tattgttgtg taataaattt ttagaatttt tttattttgt aaaataaatt 21540
gtttttatta aataattttt tagttttatt ttttaagttt ttggtaattt ttattttgtt 21600
gtttttttga atttgattat tttatggatt tggtataagt ggaattttat aggattgttt 21660
ttttgtgatt aaattatttt atttagtgta atatttgaaa ggtttatttt tgttgttgta 21720

ggtattttaa ttttttttt atggttgagt taaattttt gtatgtttta ttttttttt 21780
ttttttttt agatagagtt ttgttttgt tgtttaggtt ggagtgtaat ggtgtgattt 21840
tggtttattg aaatttttgt tttttaggt ttaagtaatt tttttgtttt agttttttaa 21900
gtagttggga ttataggtat ttgttattat gtttggttaa ttttgtttta gtagagatgg 21960
ggttttttta tgttggttag gttggttttg aatttgtgat tttagatgat ttatttgttt 22020
tggtttttta aagtgttgag attataggtg tgagttatta tgtttggttt gttttgtttt 22080
tgattttttt atttatttga ttgtgggtat ttgggttgtt tttatttgtt ggttattgtg 22140
aatagagttg ttatgaatgt gggtgtataa atatttttag ttttttaaga tgttgtgtat 22200
tttttgaat ttatatttag aagtggaatt atgggatgtt atggtggttt tagtttttgt 22260
tttttgagga attgttatag tgtgtttata agtgattttt gagtatttga tattgagtta 22320
gtttaattga ggaatggaat ttttattttt atttaaattt aaatagttat attttataaa 22380
tggtaattat attggatggt atagaatttt agtttaattt atttttttatt aatggggaaa 22440
ttgaggttta tagagtgatt tttttttttg aaatgttatt gtaagtggtt aggttagtat 22500
aagattttta ttgaatttgg taattttgaa attttatatt tttgtttgta tgatatgtgg 22560
ttaatatttg tgattttttt taaagattgt gggttttagt aagatggtag ttgttttgtt 22620
gagattttta ttgtattttt aggttttgtt ttgttatgaa tatggttgtg taaggatttg 22680
tttgggtttt tgtttttttat tttttgggt atttagatta attattagta gtatttatgt 22740
tgatttttag aaaggttttg atttggatag taattgatat gttattttat agttaaagta 22800
aaatgtagag ttttttttt ggttttgtag ttgggagtta gaggttttag gttttggttt 22860
ttggtttaaa gtgatttttt gtaagttttt ttagtttggt aaataggtta tgtatttttt 22920
gatttttttt ttagtttttg ttttgtgagt ttttgggtgt ttgaggttgt ggtttattta 22980
gtgatatttt tagtttttag tgtagtttgt gagtagtata atatatttgg ttttagaaat 23040
aattaatata tttgttaata gatgtgtgga gaaatttaat gtgttgagtt aatatagtgg 23100
attattttt agttataaaa aggtatgagt gtgtgttaat atagatgaat tttgaaaata 23160
ttttaattgt aagaaattaa gtataaaaag ttatattttt tagggtttta gttatatgtt 23220
tagaataagt atatttgtag ggataaagtg tagattggtg atttttaggg gttgggggag 23280
ggggagtgat tgtgtattga ggatggtgtt tttttttggg gtgaggaaaa tgttttggaa 23340
ttagaggtga tgtttataga aggttgtgaa aaaatgttat ttaattgtgt tttttgaatt 23400
ggtggatggt gtaatatgga aattatattt ttatgaggat gggagaggaa gggaaagaag 23460
ggaaggaggg agggggaagg aaatagggaa ggaaggaaaa aagggagggg aggagggagg 23520
gatggaagaa gggaaagaag gtagttattt ttagtggagt tggattgaag agtttaatgg 23580
gtgtagtaaa ttattatggt atttgtattt ttatgtaata aatttgtatg ttttgtatat 23640
gtattttaga atgtaaagta aaataaaata aagagtttag tgttttttat aagtgtttag 23700
gagttagttt tatagtttag aggatagttg aggtttagag gttaatattt aggtttgagg 23760
tttttaagtt ggtgttgtta gattatgttt tagataaatt tttttatttt tttgtagtaa 23820
gggtgttttt agggtggtag ttaggtggag gtgttaggtg gtgggagatt ttgatagttt 23880
ttttgtaggt ttttattttg aatttaggtt ttatgttagg gtttttttgt tatttattgg 23940
tgttggtggt agtaggttat tggttttgtg atggggattg tttttaaatt tgtttatttt 24000
ggggattagt ttttttttt ttgtaggtgt ttattgggtt attttttttt tagagttgta 24060
ggtgggttta aggtagggat gaaagggttg taatttatgt agttttgagt tagtattttt 24120
tgggaatatt tgtatttagt gataaatttt tattttaatt ttaggatttg ttgaggatga 24180
gggaattaga ttgttgtttg tggttgggat gggggttgta ggatatttgg ttgggaggta 24240
gtgtttagag aggaggtagt tattggatat gatatatttt aggggtattt atatttatta 24300
tagttttagg gttgattttg gtttatttgg tttggtgttg agagagggtt tgatatagta 24360
ggtttgtaaa gtttgatttg gaaattaaga tttaggggtt gtttttttttt gtttattttt 24420
ttttgtgtgt tatgttgttt ggtttatgtt ttagaaatgt ttatataagt tttattagtt 24480
ttgttggtta aatgtagaga tggtgttgtt ggggtaaggg atttgagtt attgtttaat 24540
gttattttta gtttagggg agtgatagtt gggtttgtat tttaggataa agtgtttagg 24600
gttaagttaa tgttattttt ttgttttttt taaatttaat ttatatttgg tttttagagt 24660
atggattgaa atggttatat ttttatatat tttgtttgtt attgttaatg gtggttaagt 24720
agaatgtaag gttatttttt ttttgtttat ggaggtttgt gaagttattg attaggttat 24780
ggtaggggag ttggtggtat ttattatatt ttgttagttt tttaggtttg tgtttaggag 24840
aaagttgttg gtatttatag ttggtgtttt tgttttggtg aagatttttt tgtttttatt 24900
tattttggtt tagtgtgggt ttagtagttt ttttattttg ttttgtaata atgtatattg 24960
gagtagtttt tagttgtttt tttttgtag gttttttga atttagattt tggggttagt 25020
atttttgtgg tagttttatg gttatttgta ggtttttta gaaatttgag tttatggggg 25080
tattttaggt tttataaatt aatagatgtt ttttgtttt tataaagggg tttttaaag 25140
ttgagtgagt ttttgatgta ggtaatgtta ggaggatagt gtttggttag ggttgttttt 25200

ttttttttt ttatttttt gggtgtttgt aaagtttttt agatttttgt tgaattttgg 25260
ggaagttttg ttttgtaatt agggtttgtt ttttttgttt ttatatagag tttgtttttt 25320
gtgtttagat agttttttta tgatggtaat tgaataatga tgtgtgtggt tttttttttat 25380
tataattttt ttatatagggg tttgtgaagt tagatatttt atttattatg tttattttag 25440
tttggttttt tttagagtag tgtagggttt atggtaagtt ttggtaaaaa gtttggtgaa 25500
tgaatgaata aatgaagaaa taaagtttat tatagtagtt gaggtttatt gggggttttt 25560
tgtgttaggt gttattttaa tttttttttt tagagagtgg gttttattaa tgttggttag 25620
gttggtttta aatttttggt tttaagtgat ttttttattt tggtttttta aagtgttggg 25680
attatagata taagttattg tattgagttt taggtgttat tttaaatgtt gtttaggagt 25740
tattataatt gatggattat tatataattt tataaggtag ttattgttat tgttttattt 25800
tatagataag gaaattgaga ttgagagggt ttttgttaat tatgagggtt gtaaagtgag 25860
taagtggtag agttaggatt tgaagttagg taggtgagtt ttaggttgtt gatttttttt 25920
ttaggttagg tttttgggaa ttatggaaag gttggagaag tatggggttt ttgtagttag 25980
gatttagaag gtgtagttgt ggtgtaggta gtttagtatt gaggttttgg tttttagttt 26040
tttttttagt tgtttttttt tagggagttg gaggtagagt tttgaattgt taggagaaga 26100
ttttatttgt tagttaggga aagttgttag ggtttttttt agggtttaga gttattttag 26160
gtttattaaa gtatattagt attttagttt ttataatttt ttttgtaaga aagatgatgg 26220
tattagttat tgggttgggt tgggggtttt agattttagg ggttttttta ggtttgttta 26280
tggttttttt aggatataga tgtttatttg agaaatttta gtgtattagg ggttttatag 26340
ttaaaataaa gaggtagagt tgttttgggg tttttgttaa agtttttagg tagatttatt 26400
tagtagatat taaaatgttt atttttttatt tgaaaatttt atttttagga ggttgtttta 26460
ggttgggttg ataaaattta tagagatttt aatgatgatt ttttatttta ggtatgagtg 26520
agtgggtgga gggagaagtt attgttaatt attaatagtt taggaaatgt aggtaaagga 26580
aagagaatgg taaggtgata gggttggaaa ttagatatat gtgtagaggt gagtagattt 26640
tagagaaaga attgttttgg aataagatgg taaagaaatt atagataata atggtagatg 26700
atgattttgg ggttttagtg gattaggatt ataatattga tttatttgat aagtatttat 26760
tgagtgttta ttttatagta ataaaaagat gatattatag gtttgagagt agttagaatg 26820
aaaatagtgg tggtatatgg gaatttgaag tttatttatt taaatgttta ttagttgaga 26880
tttttggata gatttgaagt agtttttaat atgtaaggaa tttataattt gatatagtag 26940
tataggatat gtgtgtgtta taaatttttg gtgaaaagta aagatttttt ttagaaaagt 27000
atatattgaa gatagataga tagatatata tgtattttga atggtttatt aaagaatttt 27060
taggtttgtg gattttagtt aaaaaaataa tttggtattt tgggaggttg aggtgggagg 27120
attgtttgag tttaggagtt taagattagt ttgggtaatg tagtgagatt ttatttttat 27180
aaaatgaaat aaaataaaat gagaattaaa aaaaataaat aaatttggtg tgatgagaat 27240
ttatagaatt taatagggtt attttagtat tatttgtagt atttttttaaa tttttgatta 27300
tatatataaa gttttgggtt ttatagttag agaagaggga gagtaggtta aaaataatta 27360
tttaaggtgg aagatgagtt tgagaaataa agaggtaaga ggagaagatt gaggttagtt 27420
gtttttttatt tttgagtttt ggtaggagga agtttttaag tgagaataag ttgttgtttg 27480
tggagtggat tttagagtgg gtggatagag atgataagga atgttatttt tgggggggttt 27540
tgaaaatagg atattttttt attttaattt tgttttttat tgtattgtgt ttttattgat 27600
ttatttaagt tattataaat attttataaa tgttattggg gatttttttgt tttttttttaa 27660
agatggaaat attgaatata aatgattaaa ttgtgtaaat gtgtgtgggt gggggattaa 27720
ggattgaatt tttaaaatgt atatagttta tttaatttgt taattaattt taaattaaat 27780
tatattttat attttgtatg ggataggaag tgttttttta aagtttggtt aggatttggg 27840
atgttggaaa tttgagattt tgtttttttt ttttgagata ttttgaaatt ttgtttagaa 27900
attgagtata atggttttgg tttttttagag aaagatatgg atttgtaaag gtaaagagtt 27960
gtattttttt ttgagttgga gttttttttg gtttttatta tggggtttaa ataaaattgt 28020
agttttatag tttttttgtt ggtgtttttt ttttttttggt gattaataag gtggaagagt 28080
taataagaat tttgtgtgtt tttttgtagg atttggaagg gataggtggt gtgagtttgt 28140
ttttatttag tttttttttt tatttattta ggggttgagg tgtttttgag gtagggggtg 28200
ggggttttta tttttattgt tggttaaggt ttttttagtt tttttttttgt taaatttttt 28260
taagtaaatt tttttttttat tgtggaggaa ggaattttaa gtagttttgt tttttttgtta 28320
gtatggtttt aattggtttt agttggtaaa gttaaatttg gataaggttt aagggttttt 28380
tatattattt gttgtttttt ggaagaggtt ttttttttgtt tttttatttg taatttatta 28440
tttttttagt ttgagttttt ttatttaggt tttgtggttt attgagatat taattttggg 28500
ttagagtaag ttttgatttg taaattatta ttatttattt tttaaagagt tatttttaga 28560
atttaggttg tagggagggt aggttaagga tgaatttttt gatttttggt gtggtgtgtg 28620
gtgggggggg ttattttata ttttgttttt tgttgttttt ttgttttgtt ttaggttttg 28680

aagtggattt attgtagagg gagggttagg gaatggtttt gatgtttttt ttattttaag 28740
gaatttttta gtaggagtgg tttttatttt aaagtaattt taagatttag tttgtgttag 28800
tttttttagtt atgtttatag tggtttgtta attagagatt tttttagttt ttggtaaatt 28860
tgtaaattta taaagagaaa gaaaagtttt tttgtgggtg ggtggatatt ggttatttgt 28920
tataggttgg aatttttttt tattagggat gtttttaag taggggttgg ggagggtagg 28980
gtttagagtt tttttttttt tagtttttttt tgttattagt agatttgtta gtagatatat 29040
agtagagttt agtagtgagg taataatagg ggttgtgggg gttggggttt tgtttttttt 29100
aatttatgaa attatttgaa ataataaaat ttagataagg gtttttgggg tgtaataggg 29160
tggttttttg ttaggtgttg ggtagaaaaa aagtttttttt ttaatttttt tttgttgtgt 29220
agattagttt gggttttggg gatgttgtag aggttgtttt atgtttaggt tttttagaga 29280
tagtaggggg ttggaggtat ggtttgtttg ggggtagggg ataggtaggg aggggtatat 29340
ttttgggtta tgagtaggag tttgattaaa tgattttttt aggagggaga aagatattgt 29400
atataattgt ttgattatag ttagaggggt tggaaaggtg ggggaaaggg gtgaaggggt 29460
taattgtttt aggtgttttt aatttatgta gtatttattt ggattgataa gagttgagag 29520
attggaattg tttttttttt ttgtttttag gttttgtttt aggagttttt ttgtttagga 29580
gtttgggagt tgaggaggtt tggaagtagt gggtttatag tgttattgtt ttgggaattt 29640
atagtgtttt ggatgtggtt tttttggttt tagttttgta ggtagtttat ttggattgta 29700
tttatttttt agaagagttg tttagaattt tgtatgtttg aaagaaagat taattaaaga 29760
agtgaggaag ttaaagagag gtttatgaaa gtttatgatg tattttttaa gtgtatattt 29820
tagtaatttt aaatgttttt tgaagtaagg atggggatgg atggttggat aaatggatgg 29880
gtgtatgggt gtgtgaatgg gtgagtgggt gggtggatgg atggatagat ggatgaatgg 29940
atgggtaggt ggatgaatag gtaggtgaat ggatgggttg gtggatgaat gagtaaatgg 30000
atgaatgatt ggatggatgg gtggatgagt agataggtgg atgagtgaat tggtggatga 30060
gtggatagat ggatgagtgg atgagtagat gagtgaatgg gtggatgagt ggatgggtgg 30120
gtgagtggat gagtagataa gtggatgggt ggatgagtgg atgggtagat ggatgggtga 30180
gtggatgtgt ggatgagtgg atggatggat gagtggatgg atggataagt ggatgggtag 30240
atgagtggat ggatagatga gaggatgggt agatggatgg gtgagtggat gggtgggtga 30300
gtagatgggt ggataagtgg atggatagat gagtggatgg gtggatgagt ggatggatat 30360
atgggtggat agatgggtga gtggatggat gatgagtgga tgggtggatg agtggatggt 30420
tgagtggatg gataaatggg tgaatgagtg gatgggtaga tgagtggatg gatagatgag 30480
tgaatgagtg gatgggtgga tgagtggatg gatagatggg tgaatgagtg gatgggtgga 30540
tgagtgaatg agtgaatgag tggataggtg gatgagtgga tgggtggatg agtgtataga 30600
tagatgggtg gatgggtgga tgagtggatg ggtgggtgag tggatgggta ggtggatgag 30660
tggatggata gatgggtaaa tgagtggatg ggtggatgag tgaatgattg tagataggtg 30720
gatgagtgga tgggtgaatg agtggataga tgggttgata ggtggatgag tggatgggtg 30780
gatgggtggg tgtgtggatg ggtgagtgag tggatgggtg ggtgggtggt aagatgggtg 30840
gataggtata tggagtttat ttaaggagtt ttagaaatat atatatattt ttttatttta 30900
gatttttttt aggaaaaagt agtgtgtatg gattgttatt tggttatatt ttgagatagt 30960
tatttttatt ttttttggaa tattgtttgg ggggatgtag tatagggtat ttaagtaatt 31020
tttgagttta gggtagagat attaggaagt agtaggtagg ttgtgatttt gtaggtttgt 31080
tttatggttt ttgttgtttg tattatggta tgaagtttag atgtattttt ggatttgggg 31140
ttttgattag atggagttat taattttttg aagaatgaaa tatatagggg ttatttttgt 31200
tttttagttg tagggaatga tgattattta gttttttttt tttttaaatt tttttttaggt 31260
ttttgtgtat ggttttgttg attttttttt tgtttttggt tattttttta gtttttgttg 31320
tttttttttt tgtttttatt tatggagtta gttttggtgt tttgagagtt tttttttttta 31380
gttgatttta tttagttttt gagtatttaa tgttaataat gttatttttt ttgtttagat 31440
ttatatttga gtgttagttt gatttataga tgtgtttttg ggttttgttg tgttaggttt 31500
tttgttgggt tttgggtatt tgtagttatt gttattgtta agttttaata tttgaaggta 31560
ttttgaattt aatgattgtg ttttaggttg aattttggag tttttatttt atttgttttt 31620
tattttattt tttatttaag tgaatagttt ttttagttaa tagttattgt agttagattt 31680
ttaggggttg ggtttgggtt ttttattttt attattttga atttatttat ttattttaaa 31740
tttttattaa tgttaattta atatatttta tagttattat tattagttgt ttttgtttga 31800
attttttttt tttaaagtat taggggggtgt gtgtaaaatt tattaatttg atgatttttt 31860
tttatgaagt tgtgattggt ttttattgta tttgggatat attagatttt tgtggtttta 31920
gaattttgtt tattattgat gtgttgtgtg attttgagta agtaatttaa ttattttgtg 31980
ttttaatgtt tttatatgtg aaatgggata ataatatttg ttttataaga atgttgggga 32040
ttaaatgaga tatttattta tgaagtatat agtgttgtgt ttggttagta aatgtttgaa 32100
gtttttgtta ataaaatata tgtttttaat ttattttttta gtttttattt atgtatagtt 32160

```
tttttggttg ttttttagtt tttttaataa ggttatttat agaggttttt tttgttggga 32220
ttattttttt tttatgttgt ttttagaatt tttatttgtt tttttgggat agtttattta 32280
tattgggttt tttagttttt gttttgtttt gttttgtttt gttttttttg agatggagtt 32340
ttattttgtt gtttaggttg gagtgtagtg gtattatttg ggtttattgt aagttttgtt 32400
ttttgggttt atgttatttt tttgttttag ttttttgagt agttgggatt ataggtgttt 32460
gttattatgt ttggttaatt ttttatattt ttagtagaaa tggggtttta ttgtgttagt 32520
taggatggtt ttgatttttt gattttgtaa tttgtttgtt tttgtttttt aaagtgttga 32580
gattataggt ttgagatatt atgtttagtt taggtttttt agtttttata tatgtggttt 32640
tttgattgtt gtttggggtt tgttgtattt attatttata aaattttata tgtttgaagt 32700
atttagtaag atgttgtgaa tgagtgaatg aataaatgaa tgaatggtta gtttgttgtt 32760
tttttgtttg gtttttttgtt tttttagat tagtatgggg gtagttatgg tttttgttta 32820
ttttggtaat attgtttttt ttgtgttggt tgtgttggtt agagtttttt tttatggtgt 32880
ttttgtggtt ttggtgtgtg gttgtttgtg aatgagattt atgattttg gtttttatta 32940
tttttttgga gttataaagt attaaggtgg aaggatggat gggttttttg ggatagttgg 33000
ggtgggaggt tggtttagga aatttttttt aggaaatggt tagtgtggga tgaggtgttt 33060
ggtgggaggg gagtggagtg gggatttggg gtttggttgt tttgtagtta gggattttaa 33120
ttttatttt ttttttttgtt tattttttaa ttttagtgtt gggtttaggg tttttggggt 33180
ttttttttagg aatatagaag atatagtaag gattagggat ataggatagg tagagtttat 33240
gggtaggagg tttttagtta gtatatttga gtttatgttt ggttttaatt agatattgag 33300
attttggtta ggttattttt tatgttgagg taggggggtt ttttagaggt tattggtttt 33360
taagtattgt gtggaagata tgttatggag agttagtatt ttttgagata ttagtagaag 33420
gttagatgtt ttgtggttta aggatgattt ggttttagtg tttatttatt tgttgttttg 33480
tatgaaaagt ttgatgtgat atggttggtt ttagtggaag agtgatttgg gaattggtag 33540
agtggattta gtaattggta aattggattt agtaattgag atattgttag gggtttggta 33600
gaagtttttt aatgtattag atttagtttt gatttttata gagtttagtt gagtattaaa 33660
aggtgtagag gttgagttga gtattagggg aatattgatt ttagggttga ttggtgttgt 33720
gtgtgtattt tatagtttat agatggttta tatttatttg attgtatttg attttttttt 33780
gtgaggtttt ttttagattt tttgtttttt aaagttatta gttgtgtttt ttgaaattag 33840
agtaaagtaa taaaattgta gtaatagtgt gtttttatga atgttgtgat gttagttggt 33900
taaatgaggg agtttttagg gtttttttata ttgttttgat tttgtgtgtt ttttggtttt 33960
ggtttatttt ttatattttt tgtttttttt tttttttttt tttttgagga tttagggttt 34020
ggggttgatt tattgtggga ttttaatggt gattttttgg aagaaatgtt tgtttttttt 34080
ttttatttag tagtgatttt tggtttgttt atgtttatgt agttaatata ggtagtagtt 34140
ggaatttgga agtttttggg gtgggagttt gttttttttt aggtttttttt agtttgaatt 34200
ttgggaagga gttatattta tatggtaatt ttttttaagg ttgggagggg gaagggggttt 34260
ggtttgggtt tgattatttt tttgaggttg tgtttattgg gtttttttaag ttagttgggt 34320
tttttggttg tatttttgag ggatggtaat agattatagt gtaaattgtt tattggttgg 34380
ttgggtagtg ggtgtttaga tgtgggttag ggtttttgtt ttataggttg tgtggtggtt 34440
taaagtgggt atttttatgg gtatatattt gtgttatagt ttatttgtgt gtgtgtgtgt 34500
gtgtgtgtgt gtgtgtgtgt gtgtgtgtgt gtgtgtgtga tagtttagaa atatggtaat 34560
ttatagtttt tttaagatgt tggttttggg agatttgttt atttgggggt tttagtattt 34620
ttagtttttt ttaggaaggt tttgtttttt ttggtttgaa gttggtttta gggaagaatt 34680
tttttagaaa aataaaagtt atttttttta gtttagttt agtttttttt taggggtgag 34740
ttatattaaa tttatttttt tttttttttt ttattttttt ttttatgttg taattagttg 34800
taagtggttt atttatttag gtttgttagt ttattataga aaagatattt gaattttgta 34860
tttttttttt ttgatttgtt ttgggtttta ataggtaaag gaaattggaa gttagtagtt 34920
aggatttagt tgtaggtttt tggggttatt ttgtgttttt attttagttt tatttatttt 34980
tgtgttgatt taatgtttat ttattttagt ttatggagtg aggtttggat ttaagataaa 35040
ataaaggggg ttttggtttt ttggggttta tagtgtttgg tgttggtgag ataattttag 35100
agtatagtat agagaaatgg ttgtgatatg gatttgtagt atttggttgg ttaagtatag 35160
taatgtattt ttattaggtt tgggtttttt agtagatata tggtttaaag gttggttggt 35220
taaattatat gattttgtaa ttatttaaga gaaatgaaaa tatgtttata taaaaatttg 35280
tagattagta tttattgtag aattttttat aatagaatta tgttaaaaag tagaaataat 35340
ttaaatgttt attagtagat gaagagatat atagtgtggt ttatttatga tggaatatta 35400
tttggttatg aaaaagaatg aagtattagt atttgttata atgtggatga gttttgaaaa 35460
tattttatta agtgaaagga gttagatatt aaggttatat attgtatgat tttatttata 35520
taaattattt agaataggtg aatttataga gatgaaggta gatgagtggt ggttaggggt 35580
tgagagtttg gagttgggaa tggggattgg ttaatggggg tagggttttt tttggggatg 35640
```

atgaaatgtg ttgggattaa tggtgatggt tgtgtaattg tgatggttaa ttttttgtgt 35700
tagttgggtg tagtggttta tgtttgtaat tttagtattt tgggaggttg aggtgggtgg 35760
attatgaggt taggagattg agattatggt gaaattttat ttttattaaa aatataaaaa 35820
aaaaaattag ttgggtgtgg tggtgggtat ttgtagtttt agttatttgg gaggttgagg 35880
taggagaatg gtgtgaattt gggaggtgaa gtttgtagtg agttaagatt gtgttattgt 35940
attttagttt gggtgataga gtgagatttt gttttaaaaa aaaaaaaaaa aatttttgtg 36000
ttaattggat tgggttatgg ggtatttagg taattggtta aatattattt tgagtgtgtt 36060
tgtgaaggtg tttttggatg agtttaatat ttaatttttt tattttttag aaagtttagt 36120
ttattgttag tatttattta attttatata aatatgtttt ttgaggttga agtaaatttg 36180
attggatgtt ttgtgtgaaa attaaatata aaaattggtt aggtgtggtg gtttatgttt 36240
gtaattttag tgttttggga agttgatatg ggttaattat ttgaggttag ggtttgagaa 36300
ttagtttggt taatttggtg aaatattgtt tttattaaaa atataaaaaa ttagttatgt 36360
gtggtggtag gtattttgta attttagtta aatttaggag gttaagattg gagaattatt 36420
tgaatttagg aggtagaggt tgtagtaagt tgagattgtg ttattgtatt ttagtttggg 36480
taatagagag agtttgtttt aaaaaataaa aaaattaaaa agaaaaaaaa tttttaaat 36540
tgtttttgta gttattttta agtaaaataa tagagttatt tgagttgttt attttggaaa 36600
aattagattt attaaatgaa tttttagtta ataattgatt aagaataatg ttaatattat 36660
ttgtaggaat gttgtgtttt ttaggatttg atatttttag tgattgagaa ttattatttt 36720
tttttaaatt ttatttattt atttattttt tgagatggaa ttttgtttg ttgtttaggt 36780
tggaatgtaa tggtgtgatt ttggtttatt gtaattttg tttttgggt ttaagtaatt 36840
ttttggtttt ggttttttaa gtagttggga ttataggtgt ttattattat gtttggttaa 36900
tttttgtatt tttagtagag atgggatttt attatgttgg ttaggttggt ttttaatttt 36960
tggttttagg tgattagttt gttttagttt ttttaagtgt taggattata ggtatgagtt 37020
attatattta gttgagaatt attatttttt tttaaaggaa atattattat taaaaatgga 37080
atgttataaa tagaatgatg ttttttgttt ttaaagttga tatattagag tgatgtgaaa 37140
ataataataa aagtaagata tttttttggta aatttatttt ggggtgaatg ttgtagttat 37200
aagtattgtt ggtgagtatt tttggggtaa ataggaaaag ggtgtaatta ggagtttggg 37260
taaagtagat tgtgtgtttt gatgggggtg ggttttattt gattagttga aggtaatagg 37320
gtttggaatg tatattttaa gatatagtgt tttggtatag taaatatttt tagttgaagg 37380
aatttaagaa atggtaggtt taagaagaat ttattgattt gtttttttt tgaagtagat 37440
tataagtttt ttatgtaaga ggtgtttttt ttatatttag tggaaaggaa tagttttatt 37500
tttagagata gaggatttga gaggggtttt agtatatagg ttttgttgag atttttatgg 37560
gtttattgtt tagtttattt tttgttgttt tgttatagtt ttttatgatt ttttattttt 37620
tattaaattt agtataaaaa tgtttaggtt taggttaggt atagtagttt atatttataa 37680
ttttagtatt ttggaaggtt aaggtggtta gattatttga ggttaggagt tgaagaatag 37740
tttggttaat atggtaaaat tttattttta ttaaaaatat aaaaattagt tgggtgtggt 37800
ggtatatgtt tgtagttgta gttatttggg aggttgaggt atgagaatgg tttgaattta 37860
ggagatagag gttgtagtga gtagagatta tgttattgta ttttagtttg ggtaaaagat 37920
aagtaaaaga aataaataaa aaaattgttt aaaataagta ataaataaat ttaaaaaata 37980
agtaaaataa ataaaaattg tttaggttta ggttgggtat agtggtttat atttgtaatt 38040
ttagtatttt gggaggttaa ggtaggagga ttgtttgagt ttaggagttt gagattagtt 38100
tgggtaatat agtgggattt tgtttttata aaaaataaaa aattagttag gtggggtggt 38160
atatgtttgt agttttagtt atttgggagt ttgagttggg atttaagagt gggtttagag 38220
tagtttttgg ggttggtagt tgggttttag tttgagttta ggaagttaag gttgtagtga 38280
gttgtgattg ggttattgtt ttttagtttg gatgtgtgat agagtgagat tttgttttaa 38340
aataaaataa aaattgttta ggttaatggt tttttttgggt ttttattttt ttatgaaggt 38400
ttggtgttaa gtaaaattta tttgaattgt tatgtttgtt ttttgttaat ttttgttagt 38460
ttaattttta gggttttagt taatgaattt aaaattgata gaaggaaagg gtttttttt 38520
agattttgtt ttgattaggt ttttgtaatt ttgtgataaa ggtttgaagg tttgaatagg 38580
aggatataag gtttgagtga gagagaattt tttttgttta attgttttta atttggaata 38640
ttagattttt tttgttttag atttaaattg atatatgtgt tttttttggg ttttgggatt 38700
ggaatgatgt tattggtttt tttgggtttt gggtttttag gtggatatta gagttgtaat 38760
atgggttttt ttgtgttttt agtttgttga tttattttgt taattttggt atttgttaat 38820
ttttgtaatg aggtgagtta attttttata ataataaatt tttttattta aatatatgta 38880
tatatttttt attggttttg ttttttttgga tgattttaat ataattattt tgtgaatata 38940
gtaaaattat tgaattgtag attttaaaaa ggttaatgtt atagtatgtg aattttattt 39000
ttataaagtt attatttaaa tagtaataag aagttggtga gtttgtgta ttttttgta 39060
aataagagta gggatagtgt tgtttttgtt tttggatgtt tttagttttt agtttgagtt 39120

taggaatatt ttttatgtgt tttttttttt aatttgtag tttaagggtg ggggtggttt 39180
ttagtttggt atttttttg tagtttttta tttttttttt atattagtta atggatatag 39240
aggtttttt atggattttt attttattt atttattttt attttttga gatagagttt 39300
tattttgttg tttaggttgg agtgtagtgg tgtgattttg gtttattgta atttttgttt 39360
tttggatttg agtgattttt ttgttttagt tttttgagta attgggatta taggtatgtg 39420
ttattatatt tagttaattt tttgtatttt tattagaggt agggtttttt tatgttggtt 39480
aggttggttt tgaattttg attttaggtg atttatttgt tttagttttt taaagtgttg 39540
ggattagagg tgtgagttat taagtttagt ttttttatag atttttaaaa gggttttggg 39600
taatatagat tgggatttgt taagttatta aatgattttt ttaaataaag aaggaagtta 39660
taaatttta tttaaaagga gtgggggatg gtaatagaaa tatagttagt tagaagttgg 39720
aagggtttta tttaatttat ttttaaggtt tttgggtaga agggataagg gaaaatttgt 39780
tttttaaata ttttatttgg tatttatttt tttggatgta agatgtagtt gtgtatagtg 39840
gtagtagtga ggattttggt ttggtaggtt tttatatttt ttgttgaggg ttttaggggt 39900
tagggttgtt taataggatt aagaggaaat gattatagga gtttttagtt gttgggggtg 39960
gtttttgtgg ttttttgggt tatttttggt gaatttattt taaatttgga agaggtttaa 40020
gttttttgt tttttgtatt ttaaatttta gttatgttag ttaggagttt tgggggatga 40080
gaaaaaattt aattaagttt tattgtgttg ggtgatatag gatttagaag gagaggggtt 40140
tttggattgt ttaagtttaa tgataatttt agtagttggg tttttaagtt aattagtttt 40200
ttttttttta gtagtttttg gggagtatta tggtgagttg tgaggagtta gtttagtatg 40260
gtatatataa tgtaggggtt tttttgtatt tttattttat aaatattagg gtttttttggg 40320
ggattagttt tttattttg agttattttt ttttattgt ttttttaata aggaatagag 40380
aaggagtttt tattgtttaa ttagatgtag aaattgatgt atgtatagag aggttatgtg 40440
tgatatggtt tggatgttgt ttttttttaaa ttttatgttg aaatgtgatt tttgatgttg 40500
gaggtaaggt ttgggggggg tggggtttgg gttatggggg tggatttttt atggatggtt 40560
tggtgttttt tttatggtta taaagtttgg gagatttgat tgttatagag tttgggaggg 40620
ttgggtgagg tggtttatat ttgtaatttt agtattttgg gaggttaagg tgggtggatt 40680
ataaggttag gagtttggga ttagtttgat taatatggtg aaattttatt tttagtaaaa 40740
atataaaaaa attagttggg tgtggtggta tgtgtttgta attttagtta tttaggaggt 40800
tgaggttgta gtgagttgag gattgtgtta ttgtatttta gtttggttga tatagtgaga 40860
ttttatttta aaaaaaaaaa gagtttggga gttttttttt ttgttatgtg atgtgttagt 40920
ttttttttta tttttatta tgaggaaaag ttttttgggg tttttttaga agttaagtag 40980
atgttggtat tatttgtata gtttgtagaa ttgtgagtta aataattttt tagttttggt 41040
tattttttat agtaatttaa aatggatgaa tataatgtat tagtttttt ttttggattt 41100
tatagtgtgt ttggttgggg ttgtgttttg gggaaatggt aattgtgagg tttttataga 41160
ttgggtttgg gttttaatta tagttgtatt ttagtgtttt gatattgttg agtggatgtt 41220
taaaaagtat tgttgggtga atgtgtgagt gaatgatgaa gatggaggtg gttgttggtg 41280
ttagggtaag gttttaggag tttggggagg tggtattagg tagtttaggt taatggttaa 41340
ttttggatag ggttaggagg tagttagaat ggttaaggat agttaaggga ttttaggtaa 41400
gtggttttat tttttttagg tgggtaggaa gtaggaatta gattaagggg tgttaggtgg 41460
tagtggtatt taggggggttt gaggagtagg tgtaggttta gtagggttgg tggtgttatg 41520
tgtggggttg attgtggttt tgtttattgt ttttattttt tggatgtatg ggtttatggt 41580
ttttaaggtt tttttttgtt tttatgttta ttttttttagg ttttattttt agatgttaag 41640
ttatttttt attttattt tttttgttgt ttttatattt gagttgattt ttttttgag 41700
ttttggtgaa atttagaga gtttagaggt gtagtttata tttttgtgtt ataaggtttt 41760
tgtagttttt attttttgg gatattttat tttagagttg ggtgtgtatt tattttattg 41820
gattagaaat tttaggaatg tttattttgt atgtttttgt ttttttattt ttgtttgtta 41880
tttagtttgt ggttgttatg tggttgggtt ttggtagtta ttgtagttgg taatatataa 41940
ttagtatttt tgtgtttagg ttttgtgttg agttttttta gttttatttt tgtttgtttt 42000
taaaatggtt tttgatatgg taattattat tatttatttt ttagataaaa gattatagtt 42060
ttaggagagg aggaatggta ggtttgaggt tatgtggttt gtaataggta aatggatttg 42120
gtagttattt tatttttgag tggggttttt ttttgtttgt agattttgtt gaggttttag 42180
agtttggagt tagtgagttt ttattgagta taaggatagt ggtggtgttg gggttggaat 42240
aaggtaggga ggggtttttt aaattatagt tttggttgtg gatttatttt gggattttgt 42300
tttggttggg aattttgttt tagtttattt gggatatttt tttattttga gttttgtatt 42360
gggttgtgta tggggtttgt tgaatgagtg agggtaggag ggatggtgag tagggtggaa 42420
agtgattatg gtgggtaggt ttttgttgga agtttgaaat atttttggtg agtaggaaaa 42480
tattagggga agaggttata gtttttagaa gggtttgttg gggattgttt tgtagttttt 42540
ttattgggat aggtagaaaa tttagttttt ttttagggtg agtttaggaa gtttgtgggg 42600

gtataggttt tagttagggt ggtgtgtggg tttatttagg ggatgttgtt gtggtttaga 42660
gttaggttaa attttatgta ttttgtaaat aattatttat aataaataag gtttatttat 42720
tttttaagaa gagtaaaata ttataggagg tattttttta ttaattaaat atgttatagt 42780
gattttattg tgttattgag ggagggtatt agggtgtttg gtttagagta aattgtggtt 42840
agggggatgg gtatattgtt aaattggtta gatttttatt tttgaaaatg tgggttaaat 42900
tttttttaat aagttataag gaaatttagt gtgaatttta tgtttttatt attattggtt 42960
gttttggaag attgggagga ggggtttgt ttttttttta tttttggtta tagggtttga 43020
ggttataatt tggattggga ttttaatttt ttttttgggg aagaggttta tttagggtgt 43080
ttgttaaggt ttagtgtata aatatggaat ttgtttaatg tttgttttgg tatttttggg 43140
tttttttatat tgggtttttt tatttgattt tttaagtagt gagagtgatg ggatatgggg 43200
tgtttttagg ggagatttgg ggagatggag taagtttttt agtttaaggt gaggttttga 43260
ggtatggttt atgtagtgat gtttttatg ggaggtttga ggtatgatta ggagtttgga 43320
gatttagtag aggtttgggt atgggatttt tggtatgtag ttagagtgag ggttgaggtt 43380
gtatggatgt aagtaggtga ttttgtgtta ttgggtgtta tttgtgttgg gttttgtgta 43440
gttgttttta ttttattgag ttttagggag gtgaggtaat tattttaggt atatagtttt 43500
gggggtaaag gttggattta ggtgtgtttg attttagaat ttaagaagat aagtagagga 43560
gtttgagttt tttgagattg tagtgatggt ttagtgtttt aggatggtta ggttggtttt 43620
tgtagtttta gttaattgtg ttttttttta gttatttagg gtttagtgt tggtttttga 43680
gatttggtat tggttatagg ggtttatttt tggttgtgga tatttagtgt tggttattgg 43740
gatttggtat tggttgtggg gatttgtttt tggttgtggg gatttagtgt tggtttttga 43800
gatttggtat tggttatggg gatttgtttt tggttgtggg tatttggtgt tggtttttga 43860
gatttggtat tggttatggg ggtttgtttt tggttgtggg tatttggtgt tggtttttga 43920
gatttggtat tggttatgga ggtttgtttt tggttgtggg tatttggtgt tggtttttga 43980
gatttggtat tggttgtggg ggtttatttt tggttgtagg gatttagtat tgtttatggg 44040
gtttgttttt ggttttgggg agttagtagt gtttgtgggg tttatatttg gtggtgggga 44100
tttggtgttg gaggattttg atagtaaaat tatagaggtg ataggtaata gtaatttatt 44160
agtatagtgt gtggtttgtt ttagttatgg tatttttttt tattttgtgt tttttttatta 44220
taaagtttag tgtaggtaat gaagttaaga ggtggagaat ttgtaggagg atgtatatta 44280
tatttgtttt ttgtgtggtg tttttgtatt ttgggtggtt gagattttttt gttttttttt 44340
taggtttttt tgtgttaggt ttgtgttgtt ttttttggtt ttggttttta tttatttggt 44400
tttttagtta tagatagtag tgattttttt ttttatttaa tggggttatt gtttagttta 44460
ttattagttt ttagggttgg tagatgatat tggttatggg tagtgttttt tttggtttta 44520
gtatataagg tataaaagat attatttgt tttaagattt ttttagttat gtggtgtatg 44580
ggaagtggtg ttgggaattt tttagggtag ttttttgttt tgggggagat tgtgtttaaa 44640
tttttgagga aatgttgttt tttggttttt tttttgaaaa atttttaag gagattttgt 44700
gtggagtgtg tagggagaat ggagattttt gtgagattgt tttgggatag ttttatagaa 44760
attataaatt atttggataa aagttgaaaa tttgtggtta tataatgttg attatttatt 44820
taaatatttg ttgagtaagt atttatgatg tttgggaatt taggatgtgg attgataaga 44880
tataattttt atgtttgttt tagtgataga gagagagatg gtattattaa ttttgtttgt 44940
tgggggtttt tttggagggg gtgtgtaggt ttagggttga atttgtttag tggatgtgtt 45000
atttatgttt gtgtattttt tggttttgtg gtggtttttg gaataaaggt tgagagataa 45060
aagataagga tttgggggag ggaggatgtt tatgtatatt ttattgggga tattagtaag 45120
gatgattgtt tagtgattat ttagtgattg agttatttgg tgtttggagt ttggtattta 45180
tagagtaggg tgggggtagt aggttttgtt gggggtatag gtttgagaag ggagtgttgt 45240
tagttaggtt tgatgggata gagagtgaag ggtttttttt ttgaaggtta tgtagaggtt 45300
ggaggttaat gttagtaatt ttattaatgt tagtttttttg agattgattt agagttgggg 45360
ttttaagagt agttttgggt gtttaggagt taagatttta tagagggtgt tttttatttt 45420
tagtttaggg aaagtgataa atttggagtg tttttgttta tggtttaatt attgaattta 45480
aatgaatgta ttgataaatt gtatttagtg ttatttatag ggaaaaggga gggaaaaaaa 45540
aaaaagaaag agagttaatt gtgtttaggt tggttggtta gggggaggat agatgggtta 45600
ttatttttgt tatttttttag gtttttttgta ttgatttttt atttttagta aaattaaggt 45660
ttagtttttt tttgtagttt tggagttggt tgggatgaag taatttttgt aggtagatta 45720
ttaggtttat tgtgatggtt ttagtaggga atttagttta ggttatagta gtttaggtgt 45780
ttgtagaagg tttagatttt tgggtgttaa gttttttttt tttgtatttg ttttggggtt 45840
tggtggggtg ggtagtgtgt gatttttagg gttgatgaaa gagaggagga agaggttgag 45900
tggtggtgag tttgaatagt taattttggg aggggttgag gtggggaagg tttttattgt 45960
tgttttatag gttttggagt aaggttttta tgattttatt agtagttggg taggtatagg 46020
tttaggtgtt gagtgtgtta ggatttttgt tgttttttgg gtgggggtga gagggatttt 46080

```
tatgggtttg taattagatt tgtgtttttt tagttaattt tttattaaat aaatttatat    46140
gtatagtgga ttgggtatgg ttgttatttt tgggagtttt tttgggaaag ttttttaagg    46200
ttttgttgag ttttgggaat aggtttttat ttgtttggga attgggttgg tatttaataa    46260
ataaattaaa gtttggtgtt ggaaggtaga attttgggat tagagtttta gttaagggtt    46320
ataagttttt taggaagttt tttttttaaa gagtggttgt gggatgttgg gagtttttag    46380
ggtggttagt ttttaggttt ttttaaagtt taggtggtat tgatgttttg tgttaaggtg    46440
agggttgtag agaggggttg gtggggagga gtttttatag tttattttgg ttattgattt    46500
tatttttaag tattgaaata aagttttttt tttatatttt tggttaggga gagagggtgg    46560
aagttttaaa tatttggggt gtatgggaaa ggtttatatg tatagaagaa ttagataatg    46620
gaagttgttg gtggtggtga taggtttttt aaaggggttt tgagattttt gatattggta    46680
gggaagttat atattgtatt aggtttgggg gtagggagaa ggggttttta aggatggggt    46740
tgtttttttt tgtggaagtg ttttgtattg tttgataaga gtagtttgga ttagttgtgg    46800
tataatagga agttgggaga atatggtggt ttaggaggtt ggttagggtt ggggagtggt    46860
taatttagag atttggttg tttaagtggg tttagggttt ttggggtgaa agtttttgtg    46920
gagtatagtg gatttagtgt ttggaggata gattttaggt ttagataagg tttttttttt    46980
ttttttttga gatagagttt tatttttgtt gtttagattg gagtgtagta gtataatttt    47040
agtttattgt aatttttatt ttttaggttt aagtgatttt tttgttttag ttttttgagt    47100
agttgttatt ataggtatgt gttattatgt ttggattagt tagtattttt agtagagata    47160
gggtttttatt atgttggtta ggttggtttt gaatttttga ttttaggtga tttgtttgtt    47220
ttggtttttt gaagtgttgg gattataggt atgagttatt gtgtttggtt aataaggtta    47280
tttttgatat ggaatgttgg tttgattata gtgtttgatg gtttttgttt gtaattatgt    47340
ttttgttttt tattaaagta gggtttttag ggtagaagtt gtattttttt atttagagt     47400
gttttatgtt atttttttagg tgtatatagt gggttttttt tattgtagtt gaattggttt    47460
tattattatt ataatgatag tttatagggg tgggggttag tgggaatata aagtggttaa    47520
tgtaatagtg ttttagttat tttgatatag aaatggttag gggatagaag gggtaagttg    47580
ggagttagta gtaatttagg tttttagaga tgagtattaa aaaaggtgag ttatattata    47640
ggtttttttt gattatattt tagtttaaaa gagatttaag gagaaataag aattttattt    47700
ttttaataag gatttgtata ttagatgtta agtaaatatt tttttttttt ttttgaaaag    47760
gagtttttgg ggttgttggg ggagttggtt aaagagaatg atatttatgg aaatatatgt    47820
ggttttatta agttttaagg taaggtggta atgaatggag gaggaaaatt tatagtatta    47880
ttagggaatg gggttttttt ttgttttttt tttttttaat gggttttttt atgtgggatt    47940
taaattaaat tttaatttta ttttgattta gtaggtgttg tttttatagt tttgaataga    48000
tttgttagaa aattattgtt taatttgttt ttgggtggta tattttttttt taggaaagga    48060
attataaatt tattttagat gagtagagag ttgtttttta aatttttgat taaaatatag    48120
tagattttat atgaaaaagt aaagagagag gggttaggga tgaagtgtag taggttggag    48180
gttgagttag tttttagagt tattgggatg gtgttttagg agtagatggt attgtttata    48240
aagtttttttg tttttttttg ttttgaggtt ggggattatt ttgttggttg tatgatttag    48300
tttttggggt tttttagta tgaaaagatt ttttgaaagt aggtttgtta gggagatttt    48360
ttagttttttg tttattgata tgtttttgta tggttgatat gtagtatgtt attgtagaga    48420
agtaaagtat atgtagatta gttttgttag tagtattttt ttttttttgtt tttttagga    48480
atgtttttgta tatatttaat atatttagga atttagagtt gtgttgtagt agtagttttt    48540
tggattttag tgttggtgtt gtggggagtt ttggttatgg tattttgagt attgagatta    48600
tttttagttt ttagttagtt ggtttttggg agtttagagg ggtgataatt tgagtggagt    48660
gattttagag aaagtggtgg ttaatttgaa tttttttttg gtttttttgg aagttaggga    48720
aattttttgt gaaaaggaaa tatgttttaa gaagttttgg gtagtaggag aatttagtta    48780
tttagtttag ttggttaaat atttataaag agatagttgt gattatataa agttttattt    48840
ttttatttg aggttgaaaa tttggttttt gtgtggagat tgagtttttt gttatttttt    48900
ttttggggat tttgtttggt tttgttggtt ttgggttggg gtttagggat agtgtggttt    48960
ttggtattgt tattatggtt attagggttt gttttgtggt tgtgttattt gagagttttt    49020
gagttagtgg tgtaaggttg ggaagaataa aggagtagga tttattttag tagtggaatg    49080
ttgggttgtg ttggatgtgt tgagagttgt ggtttgttta ttgggttata tggtttagag    49140
ttgagtagag gattaaaggg gttttgtggt tgtggaataa ggagtttttt agagtgtggg    49200
gtagaggtag ggagggattt tgattgtttg ggaagggagg tttggtgggg aattgtttta    49260
agggggtagg gtgaggggag taggaggagg gggtaggtag agataggggt gtttagtggt    49320
aggtttggtg tagaagtaat ttgaaggtat gggagggatg ttaaggagtt gttatatgtt    49380
aaaggaaagt gatagggttt ttgggaagag tgagggggag ttgttaggaa gaagagaggg    49440
gaagaggttt ttagggattt ttggttatgt tgtttttgtt tttgtttttg tgtttgttag    49500
aggtggtgtt tgtgaggagt tttttttttt tagttaggtt ttgaaattga tttggatatt    49560
```

```
ttttttttt gtgttttgtt tgatattttt gtttttagg tgttttttta tttatttgtt 49620
tagatattgt tatagttggg tttttaggga gtatggttag ttattttaga aagttattta 49680
gatttgtagt tagggttgga atatagttat ttgggtttgt ttttttatt ttaggtgttt 49740
gttggttggg attatggaag ggagaaggag taggagagga ggggtagggg atgggggtgt 49800
agtttatttt gagttttttt tttgagagtt tttggattta gatttgattt agtttttgtt 49860
tttttagttt ttttagtttt tgggggattt ggttagtgag ttagtttttt ttttttttt 49920
tttttgtagg tttgggatgt gttgggattt agaggggtat aagtaagggg gtttttagag 49980
gagggagttg atattatagg ggttgggttt ggatgttggg atttgttaga tttattttag 50040
tgttatgatt tttgaattta agtgagttat ttttagtagg ggtgtggtta tttttatatt 50100
aggagagagt gtttgggagt gggtttgttt tgagggtttg ttgggggttg ggagggattt 50160
atttaaatgg aaagaagttt ttggtgatat tttttgaggg ttaggggttg aattttggaa 50220
gttatgtttt taggtagttt ttatgggatt ttatgttgag tgttgattgg gttttatata 50280
tttttatttt ttttattttg tatttttata tgtggttggg ggtgattgaa gttttggggt 50340
atttgttata ttggttaaat aggttagtta tgtttagtgg ttttttagtt agtagttatt 50400
ggtatttgat ttgttgtttt ttatttttta tggttaattt ttttttaag gtttttttggt 50460
tttatggttt tttttatttt tatagtgaat ggttgtgggg agttgttgtt attggttttt 50520
gggtgttagg ttagagttgg gggagtgttt gggagaagat tgtatgtttg gagtttttgt 50580
ttttatttga atgggaggga ggttggtggt agattagaga tttgggtttt gagttaaata 50640
ttgaaattta aggttttggg tttgttattt aatatttgtg gaatttgatt tttttattt 50700
ataaaatggg tattattaat attagttgtt aaaattatag taagatatat atgtttaggt 50760
taggaagtgt ggttatttag aaaatatagt ttttttttt tttaagaagt taattttttt 50820
agtattggga gtgagaagag tttgtttttt ttattgtttg tagtttagtt tagggtattt 50880
ttattattgg tttttaaagg gtagttttag aaattgttga taaaggatgt tagaaggaat 50940
tttttttgg ttaggtgtta gggttaatag aaagttttga ggatgtagtt tttattatgt 51000
ttttgttttg tgggtttttt ttttttttgtg ggtagaattt ttttttttg gtttttttt 51060
ttgaattttt tttttttgt ttttggtttt aatttgtttt ttagtttttt ggtttttag 51120
gttttttttta gggtttaaga ttttgttttt ttttaagttt agttttttt ttttttttgt 51180
ttttggattt ttgagttggg aggttagtat tttgggtgtt ttgttgaggt ggttgttttt 51240
tttgtagtgg tttttttttgt tgttattaaa tgagggtttt tagagtatag agaaggtatt 51300
tttggtttgg ggttgtattt tttatataaa gtttttgttt tttagtattt tttaaagttt 51360
tttattgttt agtatatatt tagtatttag gtagagtagt tttagagttt ttttggttgg 51420
tttttggttt aatttttttag tagaaatggt atatgggtta tagatgtaat tttatatttt 51480
ttagtattta taataaaaaa tgtaagaata agttaggtat agtggtttat gtttgttata 51540
ttagtatttt gggaggttga ggtgggtaga ttatttgagg ttaggagttt aaaattagtt 51600
tggttaatat ggtaaaattt tgtttttatt aaaaatataa aaattagtta ggtatggtag 51660
tagttatttg taattttagt tatttgggag gttgaggtag gagaattgtt taaatttggg 51720
aggtaaaggt tgtagtgagt tgagattgtg ttattgtatt ttagtttggg taatagtgtg 51780
agattttata tttaaaaaaa aaaagaaaag aaaagaaaaa ggaaattatt aaaaaatgta 51840
agaataggtg atattaattt taatattatt tatttaatag agtttaaaat attattaatt 51900
taatatgtaa ttaatgtaaa aatgatagat atttgatatt ttttttttt ttttttgtta 51960
ggattttgaa atttagagtg tattttatat atatggtatt tttttatttg gaattattat 52020
atttaggtg tttaaatggt tttgtgtagt tagtggttat tatgttggat agtgtagttt 52080
ggagagtttt aattttttt ttttttttg gttgttgggg aatagagttt tgtttttgtta 52140
tttaggttgg agtgtaatgg tgtgattttg gtttattgta atttttgttt tttgagttta 52200
agtaattttt ttgttttagt tttttaagta gttgggatta taggtgtata ttattatgtt 52260
tggtggttat tttttgtatt tttagtagag atggggtttt attatgttgg ttaggttggt 52320
tttaaattat agattttaat tgatttgttt gttttagttt tttgaagtgt tgagattata 52380
tgtgtgagtt attatgttta tttttggata gttttaattt gattttagga taatattagg 52440
ttattagaga gttgttgatt tggattatat tatgatttga atttttttaga ttattttttgg 52500
tattatgttt ataagttata tttagaggat aataggatat agtttggtga aatatatagt 52560
ggtatttttt ataagatatt ggtgaggtta tagattttat tattagtgtt atttgtttgg 52620
ggatatttta tttttagaaa atgtagagtt tttttttaaga aatgaatgtg gttaggtgtg 52680
ttggtttata tttgttattt tagtattttg ggaggttaag gtgagtatat tgtttgaggt 52740
taggagttta agattagttt gattaatatg gtgaaattta gttattatta aaaatataaa 52800
aatttgttag gtgtggtagt gtgtgtttgt agttttagtt atttaggagg ttgaggtagg 52860
agaattgttt gaatttggga ggaggaggtt gtggtgagtt gagattatgt tattgtattt 52920
tagtttgggt gatagagtga gattttgttt taaaaaataa ataaaaaaag aaatgaatgt 52980
tttaggtata tgttaagtat tttttagatg atttaaaga tattaattat tgtgattgat 53040
```

```
gatgattttt tttttttttt gggtgtgttt gttttgtttt tttagtttat aggtgtttag   53100
gatttaaagg tagtttgtta taggttatgg gatttggttg gtttaaaagg gagagtgagg   53160
ttattggaat tttttagga gtgagtttga agtttggaat gtttttggt ggagggtttt   53220
gaagttagga ggtgatgtag atgtgggggt tgagatatta tttggggtta tttgtaaaga   53280
gggatattta ggtagtgatt ttaaggaaag agtaagggga ggagagggtg gtatatttat   53340
gtttttttaa tttttgtttg tttggaggtt tatgtatttt atatgttggt tttttttgtat   53400
ggatattttt ttaataagta atttattgtg tttatgttta tttattttaa agttatatag   53460
aattgtattt attaaaaatg aatttatgga gatagaaagt attttggtgg ttgttaaggg   53520
ttggagaggg tgaatgggga gtgagttttt tatggatatg gggttttttt ttggggtgat   53580
gaaaaagttt tggtgttaga tagtgttgat ggttgtatga tattgtgaat gtatttagtg   53640
ttattaatgg taaattttat gttttgtgta ttttagtata ataaaaaaat atatatttaa   53700
agtaatagaa tttaatgtgt tttttttttt ttttgagtta gtttgttttt ttgttttata   53760
ttttaaaatt agaagggttt tgagatatat tataggtttt tagggtaggt tgaataattt   53820
tttttttta aattgtgtta ttttatgtgg taatagggat tttgtagatg taattgaaga   53880
tgttgagatg aagagattat ttaggtgggt tttaaattat aagtgttttt taagagggag   53940
gtaggttagg tgtgatggtt tatggttgta attttagtat tttgggaggt tgaggtaggt   54000
agattatttg aggttaggag tttaagatta gtttggttaa tatggtaaaa ttttgttttt   54060
attaaaaata taaaaattag ttaggtgtag tagtatatgt ttgtaatttt agttatttgg   54120
gaagttgagg taggagaatt gtttgaattt aggaggtgga ggttgtagtg agttaagatt   54180
gtgttattgt attttagttt gggtgatttt gttttaaaaa aaaaaaagag gaaggtagag   54240
ggagatttga ttgtagagaa ggaggaggtt gtaggaggat ggaggtagag gttggaggga   54300
tgtagttgta agttaaggaa ttttagtaga gtttttttatt taaagttgga agaggtagga   54360
atggattttg ttaatatttt gattttagtt tggtgagatt gattggtttt tgatttttag   54420
tattgtaaag gaaggagttt ttgttgtttt aagttattga gtttgtgata agtagttata   54480
gtggtaatag gaagtgaatg tagtttttag tttatttttt tgtttatagt aatataggtg   54540
ttttttttgtg tagaatttttg ttaggtagtt agtgagtggg ataggatttt tttttgtttt   54600
tatttggggt tttgtgttgg atttttttaag gttgttgttt ttgagatagt gaatttttta   54660
tatttataga gatttatttt aaagttgaga aagtgttttt tttaaggggt gttagaaaga   54720
aggttttaag gtgtttttgt tatatgttta tttgtttgtg ggtaatattt atagtttttt   54780
tttgtgattt atttaataga agggttatag gggagggagt attattggtt agtttatttt   54840
atagtaaggt tttattttgg aagttatagg ggatagttgg gatttttatt tttaatttag   54900
ttaattggga gaagagttta gaatagtttt ggaggttgat atttgttagg aagtgtggtg   54960
atatttatgt gttgttgaaa tatatagtaa tatagtagta gagaggaggg gatgtggttt   55020
tatagttttt tttttattta tttagtatgt atgtatatat atatatatgt gtgtgtatat   55080
atgtattaat ttttagtttt taggtttgt attttgtttt tgttaagttt tttggttatt   55140
attaagttag gttgtgagta tagagtttag ttttttttgtt aatttttttt taatttattt   55200
tgggtaatat tttaatggga tgtagtagat gttttttatt aaatgttaag agtaatattt   55260
tttttttattt tttgtgagtt ggtggttgtg tttttgtatg ggggtgtgga ggaaagggtt   55320
ggggagtagg aatgtggttt tgagggttgg tatgtatttg tagttgtgat gttttgaggt   55380
agtttatatt tttatttata gattaatttt gagaaggggt tgggagtatt tatagttttt   55440
gttatttttg ggttggtagg ttggggaaat tttgagtagt ttgagtgaag gttgttttta   55500
ggtaggtatg gtgggataga ggggttttttg tatatttttt gtgaggtagt gtagttgttg   55560
ggttgtttta gtatatttgg gtgatatgtt ttttttttgg ttttttttta tttggtttgg   55620
gagaatgttt aggatgtttt aattttaaaa ataagtaaat tttttttttt tgatttattt   55680
attttttttaa attgtgaatt ttttttttgt ttgaatagtt taatttttta aagtaggttt   55740
atattatggt ttttagtttt ttattgttta tttatttttt agtttgagtt atttggtgtt   55800
tgtttgtttt attattaatt tagtttttga aaaaattttt atggttagta tttagtttta   55860
ttttatttga ttttgagggt agtgtttgat atgtattagt tggttttttt tttttttttt   55920
tttttttttgt atttttttagt gttgggggttt agatagtttt taagggtttt tttgtttttt   55980
gatttagttt tttggagtga ttatattttt gttttagttt tttagtttag ttgttttttt   56040
atattttgtt ttaggttttt tttttggaat attgtttgtt tttagagatt taagtttttta   56100
tttttttatat tttttttttt gatttttagt ttttttatta aagttttatt ggttgttttt   56160
ttttggtgtt ttaaattagt ttttttttgt ttagatttta taaggttagt ttttatttta   56220
tgattgtttt tttggattat tgggattatt ttttattaat atttattttt tattttgttt   56280
tttttttgttt atgtagggtt ttgttggagg atttttttttg ttttattatt tttttttttta   56340
aatagttttt taggtttttt tgttaaagtt gaaatttttt ttttgtattt gaggattttt   56400
gtaatttggt taatttattt ttaagtttga atttttattt ttgtttaagg agtttatgtt   56460
ttttagttag gttttattgt tttgtaaatt ttatttaaaa tttttttttgg taaatgttat   56520
```

tatttttagg aagttttttt gatatttttt attatatgtg atttttattt ttagagttag 56580
attttgattt ggagattttg atttttattt atttttgttt tttttgagtt ttttaggttg 56640
taggttttag tttatgtttg tgggtttttg tagtaggagg gtttattttt attttttttaa 56700
gaagtgtttg ttttattttt tgttaagttg taatttttta taagtaggta tttttaatat 56760
gtttttttat tttttttttt tttttttttt ttttttgaga tggagtttgg ttttgttgtt 56820
taggttggag tgtagtggtg tgattttagt ttattgtaat ttttattttt taggttttaa 56880
gtgatttttt tgttatagtt ttttttgtag ttgggattat aagtatgtgt tgttatgtta 56940
agttaatttt tttttgtatt tttagtagag atagggtttt attatgttgg ttaggttggt 57000
tttaaatttt tggtttgaag tgatttgttg attttagttt ttttaaagtgt tgggattata 57060
ggtgtgagtt attgtattta gtttaatatg tttttttata ttttttttga aaagttatta 57120
ttatattaat agtgtgtttt ataaattgat aataatttaa aatgtgattt attattaaaa 57180
gtgtgatttt atttttaaat aatattgttg gttgtggttt gtagttagtt ttattatttt 57240
tgtgtgatga ttattttgta gattagtaat ggttttaggt ttatatttaa tttaattatt 57300
ttgttttttt aattttattt tttttgagt taatattaag tgtgtttttag agttgtaaat 57360
ttatggttga tataaaatta ggattttttaa tatttaatat aaagtaattg ggttttgtat 57420
tggttttaat agtaattata aattatgttt gtttttttgta ttgggtatat tgtatttagt 57480
ttataatatg tgtttttgtt tttttatttt tttttattaa gatggtatat aggtttaaat 57540
ttgtttttttg ggagtaattg attttttttt ggaagatgtg agtagttttt atattatatt 57600
taagaagata ttgttataag attttatatt ttttatttta gtttttaagg gtttatttat 57660
ttttttaaaa atttatttgt tttgttataa tatgtgtttt ttttttttta tttgtttttt 57720
taagatatag ttttattttg ttgtttagat tggaatgtaa tggtatgatt ttagtttatt 57780
gtaattttttg ttttgtgggt ttaagtaatt tttttgtttt agttttttaa gtagttggga 57840
ttataggtgt gtattattat atttggttaa tttttgtatt tttagtagag atggggtttt 57900
attatgttgg ttaggttggt tttgaatttt tgattttagg tgatttattt gttttggttt 57960
tttgaagtgt tgggattata ggtgtgagtt attgtatttg gtttataata tgtgttttta 58020
tttttttttt tttttttatt aagatagtat atgggtttta agtttttttt tttttttttt 58080
tttttttaag attagttggt tgggtgtagt ggtttatgtt tgtaatttta ttattttggg 58140
aggttgaggt aggtagatta tgaggttagg agtttgagat tagtttggtt aatatagtga 58200
aattttgttt ttattaaaaa tataaaaaat tagttaggtg tggtggtggg tgtttgtaat 58260
tttagttatt tgggaggttg aggtagggga attgtttgaa tttgggaggt ggaggttaga 58320
gtgagttgag attgtgttat tgtattttag tttaggtaat agtgtaagat tttgatttaa 58380
aaaaaataaa ataagtggga agaggggaga tagtggaata aggagtttaa tttgtaattg 58440
attgtgaata attaattgag ataatttatt atttttggat tagttatggg ttttattttt 58500
tatttgtttt tttgagttat gtttttttagt gaaattttat atgtatgtga ttaaatttgt 58560
tttttttttt attaatttat tttgagtgag tttaatttat agatttttaa atattgaatt 58620
taagagggta gaggaaatgt attttttttt tgttataaaa atattttatt tttttattgt 58680
agtgttgttt gttttattta tagttggtaa tttggatttt gtttttttaaa gtttatgaaa 58740
gtaggataga ttgtttgtag gatgtttttg ttataaaagg agggttaggt ttggtaggag 58800
gaaagaaatg gaagtggtaa atgtttattt tttgtgttag atagaggtat taggtgttta 58860
ttgttttggg gagtaaatat ttttaaaaat ggaaagaaaa atttttagtt ttattggggt 58920
gtaaaaagtt atgatttttt ttgtggttaa atggttgggt ggtttgggag ataagtggta 58980
gttattttga agtttagttt atttgtttgt aaatggaggg taatgaaatt atttgttgta 59040
tgggtgtttg ataggttgtt gtttaaattt tttgtttttt tttatggggt tttggggaag 59100
attatatgag ttagtaatat aaaagtattt tgaaaattat atgaagtgtt taagtatggg 59160
ggtgttggtt gattttaggt ttgtttttat aagttggtag aaaatttttt agagagggtt 59220
ttgtttttttt ttttggggta ggggttgtag tgttagattt gatggtattg gtggttgttt 59280
aggttatttg gtatgagatt gttgttagtt ttagatttat tttaagtttg ggagggaagt 59340
aggttggggt gggggtttg aaattttttta tatttttggt agagatatta gggtaggtaa 59400
aagtgttatt tatggtaaat taagaagttt gtttgaattg ggtttaaatt agtttagttt 59460
agaatggagg ttagttttat tttttttttt tagaattagg agtttagtat attagaggtt 59520
gagaaaatgt tggaatagtt tttttatttg tttgtttaga gatagggatt ttaagttatt 59580
tttttttttta gtttatttta taaatttttt gtgttagatt ttttttgttt aggagttata 59640
ggttttattt gggagttatt tagggtttta aggtttttat tatttagttg tttattttat 59700
taggttaggt ttgtaggtat ggagattagt aggatgtggt ttaaatttgg ttgtatgttg 59760
attagttgag tgattttggg tttatttttt aatttttggg aggtaatgtt tttttatttg 59820
taaaaagggg aaagggttta gtgaggagag tgataagtaa aggagatggt atttgtgtag 59880
atggtattgt gtgggtgttt agtggatgat tatgtgttat tttttttgttt tttttttttg 59940
tttttgtggg agtgtgggta gtagggagtt aagaggaagg gagtagttgt ttgtttggga 60000

tgtaggttgt tttgaattgg aagagaaaaa ggaataagta gtagttgttt attgataagt 60060
tttatgtatt tgtgttttaa aattttaaag gtgtatttga aagatttttt gggtaaattt 60120
tttttatag atatatgaaa gttaggtgta gtggtttatg tttgtaattt taatattttg 60180
ggaggtgaag gtaggtggat tatttgaagt tgggagtttg agattagttt ggttaatttg 60240
gtgaaatttt atttttatta aaaatataaa aatttgttgg gtgtggtggt gggtgtttat 60300
aattttagtt atttaggagg ttgaggtagg agatttgttt gaatttgaga ggtggaggtt 60360
gtagtgagtt aagattgtgt tattgtattt tagtttaggt gatggagtga gattttattt 60420
taaataaaaa taaatttaaa aaaaaggtat atgaaaaagg taaaatgtta tgagtgtttt 60480
gttgtaatgt gttagttata aatgggaatt ggaggatttt ttaagttatt ttttttttta 60540
ttttttttt tttgtatat gtattaatag tttttgggt gttggtagta ttaggggatt 60600
tggggtgttt tataggtttt gtttgtttag attttagagt taaggaggag atataaaagt 60660
taaaataggg tggtaagtgg aataaatggg gattagatgg ggtaatttgg tagtatttgg 60720
taaggttttt tttaatgagt ttttaaagag gtttagtttt taggggaagg aggtgtttta 60780
ggtagtgggg gtttgtagag gtgaaggttt agggtggggt ttttagggaa gttgtttagg 60840
atgtttgaaa ggaggggagt aatttatgag tttggtgata gggttggtta taaagtgtta 60900
tgaagtatta attaggttgt ttattagttt ttaggataga aagattatag tagttaaggt 60960
gtggggattt gtttatttga tttttattta gtttagaaaa tgattgtttt agggttatag 61020
gtaatagttt aagttttgta agttgttggt tatagggtgt agttgtggta aggtggggat 61080
gtttattagt taagtttttt aggattttg ggttgttgga tatgtttgta ggatgttatg 61140
ggggtaggtt agattagggg tatttttatt tttttttta ggttttagtt tttaggttag 61200
agtgtttttt ggtttttggg gtttttaggg ttgtagatag atagggagag gtttgtgtta 61260
tggtgggtta tttggttttt gtaggaagtg gttagaggtt ttagtatttg gatatagtag 61320
ttattttttt aatttttgtt aatgttgttg ttgtttgggg tttatttttt ttaatttttt 61380
tttttaatta gggaggaaag tattttggag taggaattgg gttatttagt ttgatatttt 61440
agtatgattt tgtatgattt tgggttagtt atttaatttt tttgggtttt tattttttta 61500
tttgtaaatt aggtagataa ttttttaaag ttttttttag ttatagaatt ttaagtgtta 61560
aaataaaaaa aatttataaa aaggaaatat gtgtttagtt tatattttg ttttatttg 61620
ttaaaagggg aaatggattt gttatggggt ttttttttt taaatagata tgggagattt 61680
tttttatgat tgtagaattg gagtagttta gaggagttta gaatttttta gagtttgaga 61740
tttatagggg aatttatttt aatttttttg ggatagaagt attgttgttt aatggtattt 61800
aatttttatt attgttgtag taaatgatta tatatttgtg atgttaaata atatagatta 61860
gttttatatt ttatttatt aggttatagg gaaggtgttt atagggttgt gtattttttt 61920
gaggttgtag ggagattgt gttttttgt atttttgtt tatttgtatt ttttggtttg 61980
tggttttttt tttttttta agtttttttt attgtatttt ttagatggtt tattgtgttt 62040
ttaaaaattt ttgtgatgat agtgggtttg tgtggattat ttaggattat tttttttattt 62100
taagattagt tgattagtga ttttagttgg ttttgttatg ttatttaatg tagttttagg 62160
tgttggggat taggatgtgt ttaatttgg gagttattat tttgttaatt ttaagatata 62220
ggttttggag ggagatgaag ggtaggatta taggtttagg aggttgtttg tgtgggtttg 62280
aattttggtt ttattattaa ggaattgggg gagtatatta tttgtaaaat gggggttggt 62340
ggggaattag ttttttggtt gttagtgtag agtgaggtag tgtgtgtagt gtttggagta 62400
gttttgtgag tatttgtttt ttttttgtta aatttataag aattgatttt ggtttaatgg 62460
tttttttaaa tttttagttt tttttggtta taaggttagg agtatttttt tgtagggtta 62520
tttaagtttt tagttttttt gaaagttttg gtttatatga tgttggaagt agatgtgttg 62580
tgggttgttt atttatgtat ggttattgga gaggtttgga tgatgttgtt tttaatgggt 62640
gtatagtgta gggtagggtt aggtattaat aattagttat agttattagt gtaagtaagg 62700
ggtttttatt gttattttg gtttggggtt agggagaagt tttgggaggt atttgatttt 62760
tttataggga gtggggttat aggagaatgt atttgaaagg ggttgggggg tagtagaaag 62820
ttttttttag aaatttatt tttggtttgt gatagatgat ttttttaggt tattattggg 62880
gtttatttgg gtttttgtag tatggagttt ggttatttta gaaggatgat tgggtaggtt 62940
agggttattg gtgatggtga tggtaggttt ttttgaggtt tttttattag aagtattagg 63000
aggttagagt agttgataag tttttatttg gtttttgagg ggtagaaggt tttgagtagt 63060
tttttgattt tttgaggttt ttttatttta ttgagagatt ttttgagagta ttgagagtta 63120
ttttttggtt tttttgtgag agagttggtt atataaaggt agttatatat ttagtaagat 63180
gaatttataa tttttgagtt taggtttatt ttggtttttt tatagggagt ttttgggggt 63240
gtggggtggg ggtgtagaag ggaggggttg gtgttttatt tttgggaatt aggttatgag 63300
ttttggattt aggttaaatt atagaggttt tatttttttt taggtttttt gtttttttaat 63360
tatttttgta tagggtattt ttttttttt tgtagggttt tattggggtg ggtgaggag 63420
gagttataga attatagtag ttgttatatg ggggtgttaa ggtagggatg ttgatgttaa 63480

ttgaatgggt atgggaaggt agagtaggag gttggtgttt tgggtttggt taggttgtgt 63540
gattttaggg aatttatttt attttttttg gtttttagtt ttttttattg taaattgagg 63600
gagtgaggtt aggtgatttg tataattgtg tgaatttgtg ttttgtattt tttatttatt 63660
attttaaata atgtttttaa attttagttt tttaatgttt tggtattttt ttgtttgaga 63720
aatatttatg gttgtttttt atgttgtgtt ttaaatatag gtattttggt ttttttttgg 63780
gtgtttttat tagggttaga atgggtagtt tattttgtag gaatttttaa ggatttttag 63840
ttatttttag gatgggggtt ggttgtttgg aagtggtttt tgtttatttt tgttgtagtt 63900
tttattatga ggttagtgta gtaaaatagg aggtgtaggt ggttggggtt aggggttagg 63960
ttattttttt tagtggggta ggagaggggt gagaaaagtt ggatattgat tattttaggt 64020
tgagggtttg tgtggaggag gggtttatg gtaaaggaag tgggaggatg ggaaggagtt 64080
tttgttggat atttttattt tgttggtttt agttttgttt ttgttttatt tgtggggaga 64140
aagtgaggtt ttttgttttg ttaaatttta gtaatgaatt ttatatttat tttatttttt 64200
gtttattgga aatttttttt ttattttttg gatttgtttt gaatagttgg gttgtagaag 64260
ttggtggaat gaaatatgta gggtggttta gaggggattg tgttgaatag tgttgtgttt 64320
ttgtttattt ttttttttga agataaggtt tttttgtttt tgtttttttt ttgggaaagt 64380
tggagagggg gttgaggaaa tgggatttat tattaatttt tttgtgtatg ttggttttta 64440
gtaggtattt ttatattttt taattaggtt tgtagggtgg aaaggtagtt gttgagttta 64500
atttaatttt tatttagttg tggaagttag tgtggatggt aaagtattta tgtataaata 64560
tatatatgag gttgaggtta agagaatatg tgtttagttt ttttttggtt ttttagtaat 64620
gtttttatgt tttagttttg ttttaagttt taaatggtgt aaggtttttt taggtggagg 64680
aatggaagtt tttaagaagt ttgtgtttta gggattatat agaggtaggg tttaggagta 64740
aaggttttta gattttgtag tgtggtgttg gtaagtttgt gttgttgtat ttatattgtt 64800
ttgggatgaa ggtgatatgt tgattttttt taggtggttg taggatagtt aggaggaggt 64860
gagatgttag ttaaaggtag ttatagataa tagtattatg gattattaga aatgttattt 64920
ttgtgttaaa gtgtttgttt tttttagttg ttttattttg tgttaatatt ttttgggagt 64980
gatatgtgtg agattgggtt gtgttgatga gtgattattg gtatttttag attatattaa 65040
ggaggggtta ttttgttgtt aattaggtgt agggtaggtg gtattggggt atgatgattt 65100
gtttggtatg gttgttttat tttttttttt gtttgagtat aggttggtag gtttagagga 65160
gaggaggaaa tagagttatt atggggggtg tgtttagggg tgagttattt taagaagtta 65220
tagaattagg gtatgggaga tattgtttag ttttgttatt aatttgttgt gtgaagttgg 65280
ttaagttatt gtattttttt gggtttttagg ttttgtaatt agagggttgg ttgaagaggt 65340
aaatgatagt ggttagggat gggggtattg aagttagatt ttttgtattt ggattttttt 65400
tttgttatgt ttagagggag gtttgggtag gttatttaat atttttgggt tgtgggtttt 65460
ttattggtga aatgggaata ataatagaat taattttata gtgttgttgg gaggattaaa 65520
ggggatattt atttaggta aagagttttt aagatagttt ggggttttta ggaagttttt 65580
gttattatta ttggatatta ttatttttgt tatggttgtt agggttttgt ttagtattgg 65640
tagtttgatg ttggtagagg aagggtgagg tttttgtttt tttgtagtgt tttttttttt 65700
tttttttttt tagttatggt aataatagtt gaagtttatt tttagtttta gtgattggtt 65760
atttttttga attaaggatt gaaggtttta ggtttgttat ttaaggttta ggtgtaagag 65820
gggtgagtta ttggtttttg tttttattat atttaggttt ttatatgttt ttttaaatag 65880
gtttttgttt tatgtgtaag agaagatgtt aatttttatg tttggggtgg gggtgtttat 65940
aaatgttttg gatgtgagtt ttgatggggg ttatggtgta ggttttgtta gggtattttt 66000
aattataggg gattatttgt agtttataga gggtttttttg taggttaatg ggattttgtt 66060
atttggttg ttattttgtt tagttatttg gtaaggaaaa taggttgtag atttagaggt 66120
agtatgggtt tatatgttag tgagataata taataaggga aggatgtgta ggatgaggga 66180
gttatggaga ttttattgta gttttagtta tttattagta gattttgtgt ggatgttggg 66240
taggttattt tattaatttt taggatgttt ttgtaggttt tgtgggtgga tagggtgaat 66300
ggaggtataa ggagggtaag tttgataggg gttggggttg ggtttggagt tagaatttta 66360
gttagttttg ttattgtatt ttagggtatt ttttgtagat agtaggttaa agtaaggttg 66420
tttagattaa aggggaaggg gttttgtttt ttttattaag ggttttttgg agattttaat 66480
atagattttt agtttataga tattaaattt agggtttttg agaagtgaga aatgtgaaaa 66540
atgtatagaa tagttgtagt ttagtgtttt taatatgtta gatttatata ggtatatata 66600
gtgtttttgt agaggttaga aagttttgtg ttgggaagtt ttttgattgg gggaggtttt 66660
atagtaagtt tttttttttg tttatttatt aatgggtgga aggttttaat taggattata 66720
tgggaagggt tagtgggagg ggtagagagg gaaaatgaat aggtaatatt aagtgtttgg 66780
ggtggggggt gtaggggata tttaggaggg gagggtaggg gtgtattttt tatttaattt 66840
atagggaatg ggggtttggg aggtgtagga gaaggttttg gtatttttat tttttttttt 66900
ttgtagattt atttatagtt tgtttatggg tagttagatt tagttatagg ttagatgttt 66960

gtttagttt tatttttttg gtttattgaa agtaagttag gatagtgagg gggatgatgt 67020
tgttattttt ggtatggtta tgttgggttt tggggtgggt gggaatgtgg ttggggtgtt 67080
atttttttg gttgttatag gtataaaagt tttttgtgt tttagtgtta tatgtgggtt 67140
tttttttgt tgttttaata ggatggatat ttaagtagat tttgttggtt gggggggtt 67200
tttgtattta gttttatttt gatttagtga atttttttt tgttttttgg ggtaggagtt 67260
attaggttgg gttttaggta atggttagtg aaggggttgt atggaggggt tttttaggtt 67320
tataggaagg gaggtttttt tttttgtaa tgtggttgta gtattttgtg attgagaggg 67380
atattatggt tggagatggt gggatggagg attttttata gtatttttga tgtttagaat 67440
atttatttt gtataaaaaa gttttaaatt tttttgaggg ttgaagttat ttagagttgg 67500
ggtttttgtt ttttaggtta aaagtatttt gttggagtag ttgagagtat agtgggaggg 67560
gaggggtagg agggaatagt tttgttatgg tgatagggga ttttttgatt ttaagagggt 67620
ttgtagatat tattatttat ttttagtttt gaattgtttt ttgttaaggg gttagaaatt 67680
tttgggaggg ggattatagg gggttttgtt tatgagaata gtgattgtgg ttaggttata 67740
gtttataagt tagtaatttt gtttagttgt tgtggtggtt tttttttgat ggtttttaga 67800
aaaagtgttt tattttttgt tattagtgtt ttgaaattta agaatatttt ttaaatagta 67860
attgtagtaa aagttaatgt tgtgtgagtg ttgtaggtat tatttttagg gttttatata 67920
tttttgttt agttttata aaaatttat tttatttttt tttttgtttt tttttttaa 67980
tagtttagaa aatgagtatg gaagggttaa gttggtttga ggaggggttg gtggaatagt 68040
ttgagggttt agtttttaag aattttatat tgtaaaggga atttttgggt tagggagtag 68100
ggtatagttt ttatttttt atttttgttt ttagtattag gtttgatata gatggggttt 68160
tggttatgaa taaatgaata agtgaatgag agtttgttga gggaagaagg tgatagggat 68220
aagggagaaa taaagtagtt aatgttgttg tgatgatgat gatgttaatg gtagtattga 68280
tatgtgggtt tttgttatgt taggattgtt ttaagtattt tgttttatga atatttttgt 68340
ttttattat gttttgagtg gatatttta ttagtttatt ttatagaggg gaatatagtt 68400
gagtgtattg gttaaggtta tgttgttagg aaaggttaga gtttaggtag tatgtggttt 68460
tttagttggg ttttggttta tatggtttta taggtagttt tatgaaatat gtagagagag 68520
tatgtgaggg gttttttgt tgtttttat ttttagttgg ggtttatttt ttgttgaaat 68580
tttttaatt tgtttttaag ttttgttagt tatgaaatat tttttgaat atttagtatt 68640
atttttttat ggttatttgg ttttattttt ttatagggta agttttgggt gaaaagtaga 68700
aaggatgaaa attttgaga gaggtgataa tgtggtaatt ttttttttt ttttttttt 68760
tttgagatag tattttattt ttgttgttta ggttggaatg tagtggtatg attttggttt 68820
attgtaattt ttattttttg ggtttaagtt attttttgt tttagttttt tgaggagttg 68880
ggagtatagg tatgtgttat tatattaggt taatttttgt gttttttgt agagatggag 68940
ttttgttatg ttgtttaggt tggttttaaa tttgtgggtt taagtaattt gtttgtttta 69000
gtttttgggg attatagatg tgaattattg tgtttggttg aggtggtttt tatataaagt 69060
agagaaaatt atttttagtt taaaataaat taataagtat tgagtgttta tgtagaaaaa 69120
gattagaagg aaatgtgaat atgttaatag tgtttgtttg gaggatgtaa ttattattga 69180
ttatttttt tatattttt aatttaatta ttttgttata agtaggtgtg atttttaaaa 69240
ttataaaaga taaatgttgg ttttttgttt gtgttttgtt ttttgagata gggtttatt 69300
ttgttattta ggagttggag tatagtggtg tggttatagt ttattgtagt tttaaatttt 69360
taatttaagt aattttttta tttgagtttt ttgaggagtt gggattatag gtaagtgtta 69420
ttatttttag tatttttgtt gtttagattg gttttgaatg tttggtttta agtgattttt 69480
ttattgtagt tttttaaagt tgggattata ggtgtgagtt attatgttta gtaaagattg 69540
tttttaatt aggaagatag atgtttttta gtatttttg ttttttttt ttttttttt 69600
tttgatatgg agtattgttt tgttgtttag gttggagtgt agtagtatag ttttggttta 69660
ttgtaatttt tgttttttgg gtttaagaga ttttttgtt ttagtttta gagtagttgg 69720
gattataagt gtgtattatt atatttggtt aatttttgta tttttagtag agatggggtt 69780
ttgttatgtt gattagtttg gttttgaatt tttgatttta aggatttat ttgttttggt 69840
tttttaaagt gttgggatta tatgtgtgag ttattatttt tggtttgttt tttagtattt 69900
ttaattttg tttttggta ttatttggtt aagtagatga aaagttttag tgagttgtta 69960
gttggtatta ggttggggtt tttttaggt agttttaagt ggttaggatt tggtttttt 70020
tttagagttg ggtttagaaa ttaagattgg gaatgtgtga tggttgtttt gtgggttttt 70080
gttatgaggt tatttttttg gttttagtga ttggttgttg gttgtaggga gtatgtgggt 70140
ttagttaggg tttgtgttaa tatggttttg ttttatttat ggtttagtgg gttttttat 70200
tttttgttgt atgataattt taggaaaaat gttaggaggg agggagttgt tgggggtgtg 70260
ataggagaga tttttaggg atagatttgt aaaaattgtt ttaaagtttt ttttaaggat 70320
ttttaaggtt tttattgttt tttattgata ggttatggtt tgggtagaga aagttgggga 70380
tggagttggt ggggggaggg tggttagtat ttgtaaggta aggagatgag ttgggagaat 70440

```
aggggttggg agggtagttt taggttagtt tttgttttta gaattattgt tttttttttt   70500
tggttataaa gtgaggttgg ttagatatag gtttatttta taggttggga ggttgggtgt   70560
gaaagtattg tgtttgtatg gggtatttta tggtgaatat tattagagtt aatttttagg   70620
gtttgttgtt ttatagggaa gttagtgtaa ggtgtttatg gaatttagaa gtatttaaag   70680
gatttgatag ttatgagttg ggaggattta gttttgtagt aggagaaaga gaggtagaga   70740
gataggtttt gtaatatttg ttttatttgt tgtgttgttt tgagtatagt ttattttttt   70800
gtggttttga gttttagttt tgaggttttt aagatttttt gggtttttgt agtttaggat   70860
gtttatttga aaattaggtt tagttagtag ggtttttttt gttagtttat tattggttag   70920
tttttgttta gtatttttta gtttattatt tttatttgtg taaattttat attagtattt   70980
taagtttttt ttttttttagg aaggagattg tttttagatg tgttttttgt ttttttttga   71040
ttaaagttta gaaaatagga attagttttg tgtttttttt ttttattgaa ttgttggttt   71100
ttttggatgt ttttttgat tttttgttaa tatttttata atataattag tttttagttt   71160
tttttaaat gtaattggtg agtatgttgt agtttaggtg gatggtgatg tttttatttt   71220
gtaatttgtg agagttagta tattttttag ttaggtgggt tgtaggagtt agttgtggtg   71280
agggtaggag gtttgtgtgt tgatggtatt ggtattatta aaattatggt tagattaagg   71340
ttatggttaa tgagtttttt tatttgtaat ttttttttta ttaaggtggt tttagtttta   71400
gttttggttt tagttttggt tttggtttta ggagttgtag atatattttt aagtttttat   71460
tttaggagtt tggtttttata tatattggag gatatggagt tggggtataa atagttaaga   71520
ataggtatat ttgtaattag gagtaggggg tggagggttg ttgagatgtt tttattttta   71580
ttggttgttt tttttgatgg tttattattt attatatatt gattgtataa ttagggttgt   71640
gatatgtttg tttttttatat tatattatta aatttttaaa tttgttttta atttatatat   71700
gatgaggtaa atttagagtt aataatttgt ttattagttt ttgaatttat gggtggtttt   71760
aatttttttt tttttagttt ttttgggttt atatgagtat tgaggtataa gggaatttta   71820
ttagttggga agtgtgtttt tttttgttg attttatagt tttttagtt tgtgtttgaa   71880
gttgttaata ttttttttatt tagtattgtt ttttgtagtt tgagttgttg ttgtgtgatt   71940
tagggttagt gttttaattt ttttggatta tatttgtaag atgaggatgt tgttgtatag   72000
tttattttaa atggttatta agttgtttgt atagttaata tttgttgagt ttgtgatatg   72060
attagttttg ttttaatttt gaatgtggta tttttattta gtttgggggt ggaattgtta   72120
gtttttttttt ttttggataa ggaagttgaa aggttttaga aggtattaag gttagttgat   72180
ttgttagtag gtatagtgtt tatttgttat ttatagtttt tttttgattg ttttgtttgt   72240
ttttttagtt gagattttgt gtgtatttga atattttgtt aatggttgaa tttgtattag   72300
ggttattgtg ttagttgggt ttgttaggtt attgtttttg agttttgttt aaggtagggt   72360
tagtgtttat attttgtttt tttgttagtg ttttatttaa ggttttggaa aggttttgga   72420
gttagagatt tggttaaatt ttggtaatgt tttatatttg ttgtgtgatt atgggttagt   72480
gatttagttt ttttgtgttt tttgggttgt tgtgagggtt ataggagatg tatagagggt   72540
tttgagtttg gtggttggta tgttgtaaag gttagttatg tggtgtaagg atggtattga   72600
tgataataaa tgaattgaat gaaaattagg ttaattgggt ttggggaggg gtgttggtta   72660
aatagatttg agtggattag gatttgtat atgatgagta agtgggaata gttttaaata   72720
gtaagttggg taggtttagg atgatttagt tttgggtgtt gtttagggtt ttttttttggt   72780
tttttttgttg gtttggtttt tttttgtttt agtttgattg tttttatggg ttttaataag   72840
tttttgatta ggattgaggt agaatagata gggtttaaga ttttgtaggt aggtatagag   72900
tgtggatggt aaaaggagat gttttaggtt ttttgttgtt tttttttttt tgaaggttga   72960
taatgttaat ttatagaaga agttgttttt tttggttttg gattttattt taatatagtg   73020
tatgaggtag ttgtgtttaa ggggtgtgat tatttttgta gatggaattt atttattgtg   73080
tttagtttat tgttagagtt tggaggatgg gggaagtgag gtggggtatt ttttatagtg   73140
aagggaaaat ttagtttagt tatttttttt tgtgttgttt ttggaattta ttttgttatg   73200
gttttatttt aatttagttt tttaaagttt tagagagttt tgtttttttt tttgtatttt   73260
atttgttta tttgtgttgg gttagttttt tagaagtttt gttttttgtt atttttaggg   73320
tattaaggtt ggttggtggg tttagggatt tttattttttt gatttagttt tagtggttta   73380
ttttatattt attggtattt ataggtgtgt tttttatttt tggtttttgt ataggtggtt   73440
ttatattttg ggagtttagt ttttttttaag gatttatgtt agagtatagg gatttttgtt   73500
tttgattttt ttttagtttt aattatgagt agtttatgtt atagtaattt tttagttgtt   73560
tgtagttgtt tgttaagtta tgataatggt ttttgttttt taatttaatg tttgtatata   73620
gataaattgt tatattttaa gtagattaga tattattttt tttaggaagt ttttttttgat   73680
tgttttagtt ggatagaggt tttttttttt tgtgtttttt taatgtttaa taggtattta   73740
ttgagtattt gtgatatgta aggtatttat aatataagtt agtgtttttt gttttttttag   73800
agggtatatg aataaataaa tggtaagatt ttttaatatt tatttatgtt ttatagaaag   73860
ttatttatag gtatgggagg gttgttttaa ttgtgggtga tagggaggtt tttttaagat   73920
```

ggtagtgttt tagtaagatt tgaatgaggt ggttaggagg tttagggagg gattttttag 73980
gagggaataa gtgaatattt ttttgtgagg atgagttttt atgttttagg agtagttagg 74040
ttaggggatt ggtgtagtga atagggttg aggggagtgg ttaggtgatg tagggtttta 74100
taggttgttt tatgatgagt tagtgtttta agtatagtag atatttaggg aaggtttagg 74160
ttggggagtg atttgaatag atttttattt taggagttta ttggggtttt tgggtttatg 74220
ttgttgtgtt attgtatgtt tttggggttt tgggggggta ttttttatat tattattata 74280
ggtagtattt ttgggggagg tgtgttgtgt aggggatttg ttggttattt tgttgggtaa 74340
attatagggg attgtgtagg ataggtggtt agtttaggag ggataattat ttaagtaggg 74400
gttagagggt atttggattt gggaggagtg tttaattttt taggtttagg taattagagg 74460
tgggggtgg ggtgttgttt tttgatatgg gaggtttggg ggtttggttg tattttttgg 74520
tgtggaggtg ttgggaggtg tggaggtttt tttttgaggt tgggattttt tggttatttt 74580
gatttattgt tttgatttta tttttagtt tgagggtatg gtttgaattt atagttgtat 74640
atattttttt ttgtataggg ttttgtgggg ttggtatagt agtttttttt ttatagatta 74700
ggatgtttag ttttaggagg gttgggattg tttaagattg tataattagg gttagatata 74760
gttgggttaa ttttgtgatt tttggtttta tgtatagatt ttaagtatat aggtaaaatt 74820
ttgttggttg ggagggttgg ggggtagatg agtgggggt agtttttttt tagtttttta 74880
atatgttagg tttttgggtg ggagggggta taaagatttt atttttttta agagatagag 74940
aaagaggtag tggagggttt ttttttggtt gaggttttag gttttgagtg gtgaatgtaa 75000
ttttatttgg aggttggaag ttaggtggtt ttgggggtgt gtgaggtttg ttagtttagt 75060
tgtagttttt tttatgtaag ggaatttttt ttaggtagtg tttttatgat tagagagggg 75120
gttaggaaga ggtgagggtt gtatttagag tgatatttgg gtgggtttta ttttggggat 75180
atggttatat tttttttttg gatttttgaa gtttttaggt gtgggttaag ggtttttagt 75240
tgtaggtagg agagggtttt atagttatag atttggggag tttagtttgt aaagtttaga 75300
ggtttttgga ggttgttggt ttggtttttt gtgtagttgg gtttttttgg gaggtttttt 75360
tttggtattt ttagttttta gtttgtatga ttttttatag ttgggtttat tttttaggt 75420
ttgggttata tggtgtaggt tagtggggtt ttgggggagg gtagggtgtt aggaatagtt 75480
ttgggggtgg ggggattgga gtagagttta ttttggttgt ttgtttattt tgtatgaagg 75540
atgttagatt tttatatgtg gttatttgat tttgtttaat tatgtgtaag ttttggttat 75600
ttttatttta ttgatgggaa ttaaattggt taaggagaag tttagaattt gagtagagta 75660
ggggtttaag tttttttagga gttttaggta tttttttagtt tttttttttg agtattgagt 75720
ttatatattt agtgtttgga tgtttattta tttggggggtt tggtgattgt tagttagtat 75780
ggtttataaa gggtttttgt gtaataggtt ttatttgatt tttttaaaaa tttttttgagg 75840
taggataatg ttatttaatt tattttatag ataaagtaat tgagttataa aatggtttgt 75900
taaaagatat agaggtagaa agattaatag taaagagttt ttttgtgatt ttgattagta 75960
aggtttttag aaaaaagtaa gggatttga ggtttttata ggaatagagt tttggaggag 76020
aataagagtt tagttttatg ttatttttaa ggggtatttt gaaggttaag gtattgggtt 76080
taagtaggtt ggattgtgtt ttttgtattt tttttatttt ttgaagtttt ttttttttta 76140
tttaggtggg ttgtggaggt gtttataata tataatggaa tagttggtag gttattgggt 76200
ttttattga gttaaggggt ttgattagag gtttaaatag tggtatagaa attatgagtt 76260
ttgttgtaaa ttagtttagg ttttggagtt aagtagtttt gtgtttatag ttagggttag 76320
ttgtttttag ttgtgtggtt tgaggttagt tttttaattt ttttgtgttt tggtgttttt 76380
tttgtagtag taataggaat tgttttttat ggtgatagtg tagatatatt ttatgaagtg 76440
tataaagttt tgagtttagt gtttggagta tgttaggatg tagtaagtgg ttatttatga 76500
tttatgaaat attaataagg tgagggtgg aatgatagtt tttttgtgtt gtttttaaaa 76560
tggaaataaa tttgtttagt gtttaaagta gtagaaagtg agttaaggat aggtttttag 76620
tgatatgttt tggtagagtg tttttttgttg gttttaggta gggtttaggg tgggtggtta 76680
ttttttagat gagtttattg ttggtatttg tttttagagt atatgtttgg atttttttaga 76740
tttttttttt ttttttttttg atttttttttt atttgtaat ttttattttt tgttattttt 76800
ttatttattt ttttttttta tttttgttta aaaataaata atggaggtta aatgtgttag 76860
ttggtttttt ggaaaggttt ttttgtagta ggaatgtgat gggggtagtg gaggattgtt 76920
tagtgtaagt tatgtgattt ttgtgtggag ttggggaagg ggaggtgttg agattgaggg 76980
tgttaggatt gaggtattga gatggggtta ttggataatg gttagggtta gtttttgttt 77040
ttattttaat aggggttttt atatttaggg gttatatttt taaagttttt tggataagag 77100
agataaattt tattataggg ttagaaatgt tgtttttagg ttgttatttg ggtgggggtg 77160
agagatgttt tgttggtttt taaatattta ttttgtggat aaaattatta aggggaattt 77220
aatgtttta tagtagtttg gttaagatat aggtattagg ttgggtgtag tggtttatgt 77280
ttgtagtttt agtattttgg gaggttgagg tgggtggatt gtttgaggtt aggagtttga 77340
gattagtttg gttaatatgg tgaaatttta tttttattaa aaatataaaa attagttagg 77400

```
tgtggtggtg tgagtttgta gttttagtta tttaggaggt tgagatagga gaattgtttg    77460
aattttggag gtggaggttg ttatgagtgg atattatatt attgtatttt agtttggatg    77520
atagagtgag taagatagtt ttaagaaaaa aaaagatgta ggtattgttt tagggtttat    77580
aagtataatt tgatttttga tgttggtatt taattttgat gtagatttta gtaggaaaag    77640
ggagatgaat tagaaggttg tggggatgag gggtttatag tagtgtttat tagatttgtt    77700
ttggtaggta taagtgtttt ttggaggttg ttagataagt agagggttta ttttagagat    77760
tttggttttg gagggttggg ttagggttta ggaagttgta ttttggtagg gaatttagag    77820
ttattttgag ttttattttg gaagtaagag ttagggggta gaatagaatt ggaattaaaa    77880
gtttttgtgg gtttagtttt atggggatgg gtttggtttt atggggatgg atttttgtat    77940
tatttgaggt gattattagt ttggtttttt gggattagtt ttgagtgttt tttggggaaa    78000
gtgggatttg tagaggatgg ttttttagga tttgtttgat tatttatttt tagtttgttt    78060
ttattttagt ttttttgatt tttatttttt atttagaatt ttggagattt ggttttggag    78120
gtttagagat tagaaatgag gggagataag gggagaagtt ttttttttta tttgttggtt    78180
atgttaatgg gttgattttt taggaagtta tattagttat tgttattgtt ttggttgttt    78240
tgtttggttt tgaggttggg tgagtaagta ttttgttttg tttgtttttt tatttgagtt    78300
ttttgttgat gttatgagag gtttgattat gtgtttagga ggtataggat agagtttaga    78360
gttgtttaag aatatataag gttgaggttt gggttttgta tttgggaagg gttaggaagg    78420
gttaaaggaa aattttagtt gagtataatg gtttatgttt ggaattttag tattttggta    78480
ggttaaggta ggaggattat ttaagtttag gagttggagt ttagtttggg aaatatagta    78540
agatttatt tttataaaaa aaaattaaaa ttaaaaaatt aattaggtat ggtggtatat    78600
atttgtagtt ttagttattt ggtaggttga gatgggagga ttgttgagtt taggaggttg    78660
agattatagt gagtgggata ttgggttatt gaattttagt ttgggtgata gagagagatt    78720
gttttaaaaa aaatttttat attttatgga gtttagtaga atttttggtt agggtgtttt    78780
gattgtgaaa tgagtgtggt agttgttaat ttttgagggt gaagatttat ttattagtaa    78840
tttgtatttt ggtatggtga gaggaagtgt ttggggaaag atgttggttg aggtagagag    78900
tttagtttaa tttattgtgg aggagttatt ttagttttaa agagaaagat taataaattt    78960
taggtgaatg gtttaaaagt tatttttgta tatgtagagg gtgtattttg ggatatgtgg    79020
atattttttt ttaggatagg aagtaaaatt agtaggtaga gatgtgagtt tttttgtttt    79080
tgttggagtt aatttggagt ttttattttg gtttttaggg aagtatagat ttttattttt    79140
attgtagtta aaagattttt tgttgatttt tttagtagta tttagtttag ttttgtgggg    79200
agaggtggta gggggaggag atagagaaag atgagttttt ggtagttgtg attgggggga    79260
gagaaatgtg gttttaattt gtttttgttt atttatagga tgggtggtgt tttattttaa    79320
aatttagggt agttgaggga ttgaaggaag agggggatat tttttaattg gggttttttt    79380
ttattttatt ttatggtgtt ttatatagga aagagttgga aggttttggg tttttggtat    79440
ttagttaaag ttttaggtag gggtttagat attgggttgt gttattattt taggtagttt    79500
gggagttagt gatggtagag ttttattttt attttgatta ttgtatagta ttttgttttt    79560
aggatagttt agtagttagg ttgttttagg gtttaaattt agatatttaa ttgtgggggt    79620
tttaggtaag tggttgaagt tttttgtgtt ttgatttttt aatttggaaa atgggatgat    79680
gaaagtaatt gttgggtagg gtaaggggta agtgaaagtt agtgtttgag agtgatgtgg    79740
ttaggattgt tgttgtgatt atatattgag tttttttttt ttatttgaga taagggtttt    79800
attgattgtt gttagaaaga tttagattta gaaggattag gaaagttgtt ttatttataa    79860
ttttagaggg aaggggaggt ttttgtgtta gttggttgtt gtaggggaga gggtgggaga    79920
tagatggggg atagaggagt aggggtagta ggaggaatgg ttggtaggga agtttgtttg    79980
agatttaaag gattgtttat taggaggata gtttaggttt tttttattga gaaagatttt    80040
tttggatagt tgggttgagg atttttaggt tttttgggtg gagtagaatt agttattatt    80100
gaagttatat aggtttgtaa gtataagtgg gggattgttt aggtattttt gttttatagg    80160
ggttaggtat taaggttagg gtggttagag gatatgatgt agtttagtag gggttttgta    80220
gaattattag ttaagaagat atatgtagaa gtatgtaagt gttttgagga ggtaaatttt    80280
ttgttggatt gaatatgggg agttgagttt ttaattattg agttgggttg tttggtttgt    80340
tttttttttt ttgaatagaa ggtttttaga tagagtagtt aggtaatagt atggttaagt    80400
aggggtttgt attttagttt tgttttgtag tttgattagg ttgttgtttt tttaggagat    80460
atgggattgg ttagggtttt tttaggttgt tataggaata aggtagggtt gtgatttttt    80520
ttatggttta ggtatttggg ggagggatta tgagttttag tttaggggat agataggaaa    80580
tggatagttt gttttttttt taattatgga gttggttttt tttttattgt attttttta    80640
gttatgtttt ttattagaag attattgtat ttaatgggtg gttttgtagt gatatttagt    80700
ttaatgaagt tatttggtgg gggttttggg ggatgtagtt ttttgaaata tatatgggta    80760
gtgggtgagt tggaggttgt gtggggtttt gtttttgggg gatggttagg aggtgtttta    80820
attatttgta atttgagttt atagtgattg ggatttgttg tttttttatt ttagtttttt    80880
```

tttattagta tttgttaagt gttagttagt aggtgttggt atggtttaag gtaggtttgt 80940
agttagatta gtattggggg agggattggg agagagattt ttagagtgga agttatagtt 81000
tttttttttt ttaggttggg taatagatta ttatttttag ttagttttgg ggaaggggga 81060
ggggttagtt gggttgtagg tatagtgttg gggttttttg aggtggttgt tgttgattat 81120
ggtttttttt ttttttgggt tgggtaatag attattattt ttggtttatt ttagggaagt 81180
gggaggggtt agttggggta taggtatagt gttggggttt ttgtggtggt tgttgttgat 81240
tatgggtttg ttattttttt tttagttggg ttgttatttt ggttttgaaa gtgttttttt 81300
tagtttttaa tttgtgttta ggaggtagtt gatattttaa gtgttgttat agtaggaggt 81360
tgggtttgta agtgtgattt gtttggggag gggtagaggg aaagattatt tttgtttaaa 81420
tagtggtttt tttttttttt ttgggggttt ttatagggtt tttagttttt ttttggttaa 81480
gaggtaagga gaggtaggaa agttttagga atattggtag agaagagata gggtaggttg 81540
ttagaggaag taggttgtag ggtgtttaag gttttaggtt gggttttggg aggttagggt 81600
atgggtaatt aggaggttta ggtagggaaa tttgttttgt agggatttg ggagataggg 81660
gagaggggat atgatagttg ttttttgttg ttttttggtt gtggttttta ggggttttag 81720
tttagattag aagtatttta tgtattgggt tttggtttag tttggaagta tggtttatag 81780
atttatatga ttatttgggt agtgattttt aaaaggtttt ttgttattag ttggggagtt 81840
tttgagggga gggtggtttt gtagagtatt tggagatatt aatgggttgt gaggaaagga 81900
ggaggtgtgg atatttttat ggttttaaga gaaaattaag gtagagttgg aggttgggtg 81960
tggtggttta tatttgtaat tttagtattt tgggaagttg aggtgggtag attatttgag 82020
gttaggagtt taagattagt ttggttaata tggtgaaaat tggtttttgt taaaaatata 82080
aaaattagtt tgttatggtg atatgtgttt gtagttttag ttatttagga ggttgaggta 82140
ggagaattgt ttgaatttag gaggtggaga ttgtagtgag ttaagatggt attattgtat 82200
tttagtttgg gtaatagagt aaaattttat tttaagaaaa aaaaagagta gagttggagg 82260
ttaggtggtt tttagagttt gataaggagg ttgtggggtt gggttgtttt tttggttatt 82320
tgaggggtag ggattggttt agtttgagtt ttagatttat aagaggttga attggagtgg 82380
gattttgaga tgaggtaggg ttattttgtt tatatttgtg tttaggtttt aatagttatt 82440
tttttagga atttagtttg atggtttttt aggttgagag gttgttaagg ggttgtaagg 82500
tgttgtaatg tgtgagttgg tgggttttga tgtgtgatag tttggggtat tttttttatt 82560
gttagggaat aggaatgagg agttttttat ttttagtgtt tggtaggtag gaggttgtta 82620
ttggattgtg tgtttaggtg ttattagatt ttgagggttt atggatatgg tatatgattt 82680
ttaatgatta atgtttgtgt agtaggtgtt gaggtgtttt aattttgagt aaggttgggt 82740
ttttggaatt ttttttttgg taggaggatt tgtagttgag gaatttttaa gaaatttttt 82800
ggtagtaaat aaagtatggg gttggatggt ggatttatga gtgtttttat atgaatgtgg 82860
atatagatag aaaagatggt aaatatttgt gaataaaagg aatggttttt gtgtattgaa 82920
ttttattta ttggtttttg tttggtataa agtttttagg aatttttaa tagtttagt 82980
tagaaatgtg tttttgtttg gttatgtgga aaattttata tatattaaga tatatgatgt 83040
ttttggtttt ttttatgaat gttttatgtt ttttgggttt atgatttgga atttgtagat 83100
ttttgttgtt tttgttttag agttataagg attttggatg taggaaatat tttttatgtg 83160
taagggtttt tttttttttt gttagggttt attggttttt ttttgtttat aggtgttatt 83220
taaattttg tatttgtggt tttttttttt taattatttt gtgtaatttg ttaatagttt 83280
ttttattttt agagtttggt tgttggagat atataaggta tatagaattt gtgaaatgat 83340
attgagtatt tattaggtat ttattgtgtg ttttgtttgg taataataag taaaattata 83400
atgttttggg tatatgagta tattttttta tgtttatttt tttagtaatt taatgagatg 83460
gttttataaa tggggaaatt gaggataggg aggttagatt ggtttaaagt tataggttag 83520
gaggggtagg gttaaaattt aaatttagat agtttggtat taaattttag tttttttatat 83580
gaaaggtatt ttgtaatttt ttgagaaggt tgggattatt atttttattg tatagatgag 83640
gaaattaaga tgttaaatgg ttagatagtt ttggagttag gaggtgggtt tagtggttta 83700
tgggagtata tggaatggag tttatgggag gtttgaggtt gggattttgt attttatttt 83760
ggtttaaggg atgaggtgat tgggagtgtt agagatattt tgggattggt tatttaggtg 83820
ttttgtattt agttttagtg ttgagtgggt gagggtggat aatttgttat ttagtagggt 83880
tgttgtttgt attttggtgg ttttttaaag tttggttaag taaagtaaga ttgtttttgg 83940
attagagtga tttttaagtt atataagtat ttagttatgt tttggaggat gtaggattgg 84000
gaaggttttt ttttattgt atttttattt agttttttta tggggtagtt ggttttaggt 84060
atagattaga tgtttttttt tttgtttgtt gttggtatgt ttttggtttt attgttgttt 84120
tagggagggg tttaagagtt ttttggtttt gttatttatt atattttgtt tagagggttg 84180
ggggtttagt attaatgtta agtatttagt agtaaaatta tgggttggaa agagttatag 84240
tgttgttttg atttggtttt attttatatt gggtgttgtt tttattagtt ttttttttgtt 84300
tttggtttta tttttaggt attataagtt ttgtaggttt aaagttaaat ttattagttt 84360

ttgatttttt ttagttggga ttagggtgag gtttttgttt aggtgtaaaa gttaaggtag 84420
tgttaaaaaa tttaggaatt aaataatatt ttgaagtaat attttaaata aattaaattt 84480
tatgttgaat aaattttgat agggttgtgt atgtgtaggg ttgggtttgt tttaatttaa 84540
aattttaata tttgtttatt atagagatta ttatgtatta ttttgatatt gaaaaatatt 84600
gtattaaata ttatttgtgt tgatttttga gtttttttggt gttttttttat attttgtttt 84660
tgataagtgt ttgatatatt ttgttttagt tggttttgtt ttttaggtgt ttttttgggg 84720
ttgttttgtt tatgtttttt gtagttatta ggagttattg agttattgta tttgttttat 84780
tttgtttttt atgtttttgt attgtttatt ttgttatgtt tttttgtgt gttttggttg 84840
tatgttagtt ttagatagga gtttggtggg ttgggagttt atattaatgt tgttttttttt 84900
tggttttggt gttgggtata gggtagtatt ggagattttt tttttttttt tttttttttt 84960
ttttttgaga tatagtttta ttttgttatt taggttggag tgtagtggtg taattttggt 85020
ttattgtaat ttttattttt taggtttaag ttttgttttt tggaattaag tagatttttt 85080
tgttttagtt tattgagtag ttgggattat aggtatatgt tattatattt agttaatttt 85140
tatattttta gtagagatgg ggttttgtta tgttggttag gttggttttg aatttttgat 85200
tttaggtgat ttgtttgttt taattttta aagtgttggg attataggtg tgaggtatta 85260
tgtttggttg agattttttg aataaataaa tattattggt atggagattt gtttagggaa 85320
tttgtttaga ttttgttatt gtattttgt ttgttgtgtt tagaggtaat ttagggttgt 85380
tttagttgtt tttatttggt tattttttgg tttttattta ggttggtttg ttttattttg 85440
tgggttaaga aaatgattta gtgggtggtg attagtattt ttttttaat ttgaaaagaa 85500
tatatttatt agtggtggta gtaatttaat gtttttttagt tgatgaaggt aatatgtttg 85560
tttataatgg aatggtttat gttatggtgg aatattattt agggatgtat tggtatttgt 85620
tataatgtgg atggattttg gaaatatgat attaagtgga gaagttagat ataaaggtta 85680
aatgtgtaat tttttgtata agaaatgttt agaattggtt aggtgtagta gtttatgttt 85740
gtaattttag aattttggga ggttaaggta ggaggattat ttgagtttag gagtttaaga 85800
ttagtttggg taatatggtg aaattttatt tttataaaaa atataaaaat tagttgggta 85860
tggtggtata ggtttgtagt tttagttatt tgggaggtta tggtgggagg atggtttgag 85920
tttgggaggt ggaggttgta gtgagtagag attgagttat tgtattttag tttgggtgat 85980
agagtgagat tttattttaa aaaaaaaaaa aaaaaaaaaa agaatttata tagataggaa 86040
ggaaatatgg aggaggaggg taggattgat agttaaaggt tatggggttt tttttaaga 86100
aaaaaatttt ttaaattttt taaattttta atttttttaaa attggttgtg gtgatgatgg 86160
ttgtattatt ttttgaatag attgaaaatt atggaattat gtattttaaa tgggtggatt 86220
gggttatgtg gtgatttatg tttgtaattt tagtattttg ggaggttgag gtgggtggat 86280
tatttgaggt taggagtttg agattagttt taatatggag aaattttgtt tttattaaaa 86340
atataaaatt agttgggtgt agtggtatat gtttgtaatt ttagttattt gggaggttga 86400
ggtaggagaa ttggttgaat ttaggaggtg gaggttgtag ttagttgaga tggtgttatt 86460
gtattttagt ttaggtgata agagtgaaat tttgttttaa aaaaataaat aaataaataa 86520
attggtggat tgtatagtag tttatttgga ttttaataaa gttgtttaaa aaaaaatgaa 86580
aaaaagtttt tttttttttt aaaataaata aataaaaaga aaaaaagaga gaggggaaaa 86640
aaaaaaaaaa aaagaaaata ttgagtgtag tatatgtatg gtaaaggtaa gaattttatt 86700
atggaaattt ggttttatta atatatttta tttttatttt tattttatat ataggtgtgt 86760
tttggtttgt ggtgttaatt gtatgttttt ttggaggttg tagtttagat tggttttgaa 86820
agttgttttt tgggtaattt taaatttttt ataattttg aagtttttgg tttgttggga 86880
ttatttagtt ttttagattt agttttgtta ttatttattt aagattgtag ttttggggag 86940
gggggttttt ttttaagggt gtaggggttt tgtggtttta ttttttttta gaatatatat 87000
tattttgtat tgtatatatg tgttttaata tggagtattt taggatgttg gttatagtta 87060
gttatgtttg tatattgatt gattgattgt gttttgttga ttaattatgt taattattgt 87120
gttgtatatt gtttttatta agtgagttaa agtaattttt ttggaataag ttgatggatt 87180
ttaaataagt taaaagatat tttttagga aaaaaatagt aatgaatttt aagaagaaag 87240
aaaaattttt aaatttatag atttatggaa aaagtttaag tttttaaagt gttagatttt 87300
tttaaaagta ttaggtttag atggttttat gagtgaattt ttgaaaagtt ttaatagaga 87360
gattattatg ttgttgttta aaatgtttta gaaggtagag ggtaaaagaa atgtatttaa 87420
attgttttttg ttaagttagt ataatgtaga tattaaaatt gataaaaata gtatatatat 87480
atatatatat atatatatat atatatgttt tatttgtttt agattaagtt tgttgttgtg 87540
tatttaagta atattattta tttttatttt gttgttgaaa ttaaaatata ttttaatgga 87600
tttatgattt taatatgtaa aaatgaaatt atagaagtat tagaagaaaa tgtgggtgaa 87660
atttttttttt ataattttag aatgtaggag atttttaaat aggatgtgaa ttttaaaagt 87720
taaaagggaa agtagagatt tgataatatt aggattaaaa tgttttgtgt agaagaatat 87780
tttagaaata aaattaaaag taggtagttg gggataagat atttgtattt gttgtgataa 87840

agatgtataa ataatttttt ttttttttta gataggattt tattttgtta tttaggttgg 87900
agtgtagtgg tatgattata gtttatagta gttttaaatt tttagattta aatgattttt 87960
ttatattagt tttttgagta gttgggatta taggtgtatt attttattta gttaattttt 88020
gtagagatgt ggttttgtta tgttgtttag gttggttttg aatttttaag tttaagtaat 88080
ttgtttattt tagtttttta aagtgttggg attataggtg ttagttatta tgtttggttt 88140
ataaagaatt ttttttaaaa aattttaata agaaaaggat atatttttta aatgaaaaga 88200
gaatataggg ttttggttta taatatatga agagataatt ttttttgttt gattgagatg 88260
tataaaataa aatattaatt ggagagtatt tggtttttag ttaggggtaa ggggtatagg 88320
atttttttgt tggattatta ttgtataggg tttagtagat agttgagtag gttgatgagg 88380
ggaggggttt taaaaaattt tagtaattat tttagaggtt aatttaggaa taaggattaa 88440
tttttttttt tttttttttt ttttgatagg gagttttgtt ttgttattta ggttggagtg 88500
taatggtatg attttggttt attgtaattt ttgtttgttg ggtttaagta agttttttgt 88560
tttagttttt tgagtaattg ggattatagg tataagttat tatgtttagt taatttttgt 88620
atttttagta gagatggggt tttattatgt tggttaggtt ggttttgaat ttttgatttt 88680
aagtgattag tttgttttgg ttttttaaag tgttgggatt ataggtgtga gttattgtgt 88740
ttagtagatt aaaatttaaa tgtatatatt tttgatatag aaatttatta agagtttatt 88800
ttttagttat gtgtgtatat gtataagtgt gagaatattt gttgtagttt tttaattata 88860
gtgggaagtt ggataagttt ggtagggtta tgggatagtt gatgatataa ttattaaaag 88920
gaatgtgtta ttttgggagg ttgaggtggg tggattttaa ggttaggagt ttaagagtag 88980
tttggttaat atggtgaaat tttgttttta ttaaaaatat aaaaattagt taggtgtggt 89040
ggtaagtatt tgtgatttta gttatttggg aggttgaggt aggagaattg tttgaaagtg 89100
gaaggtgaaa ggtggaggtt atagtgagtt atagtgagtt gagattgtat tattgtattt 89160
tagtttgggt tatgttttgt taaaaaaaaa aaaaaagaaa aagaaaaagg aatgtgttat 89220
atttataagt ggtaatagtg ttgttaaatg gaaaaaataa aagttggtag tagaagtttg 89280
atttttttttg tgtttttgaa aagtttatat ttatatattg attttttta tataagtttt 89340
gaatgatttt gtaggaataa aatttaggag aatgttatat ttagttgtta atatggttat 89400
tttttgggag agagttgggg aatgggagag aaaaattttt ttttaaattt tatgtgattt 89460
ttattttta tttaagaatg attgggtatt gttttttttt ttttttttta gatgggagtt 89520
ttgttttgtt gtttaggttg gattgtattg gtataatttt agtttattgt aatttttgtt 89580
ttttgggttt aagtgatttt tttgttttag tttttttaagt agttgggatt ataggtgttt 89640
gttattatgt tttgttgtgt attgtttttt aggatgaaga aagggaaagg ttatagggag 89700
aatgggagtt agaatataga gtagggtttt taaatattag tttgtgggtg agagagatat 89760
ggggtgatat tattggtttg gaatttaggg atggtatgag gtgttgttta ggggttttag 89820
ataaggtttg tttgttgatt aagtaagggt tgtgttggtt tttgtttatg tgtttgttta 89880
atatgaattt tttaggtgtt ttattttgtt ttatgaatta ttattgttta ttttaattat 89940
gttgtatagg aagaattgtg tttattttta taatgagaaa atggattaga gaggaaagtt 90000
attaggtagg gtaggggtgg tagtttaggt agtgtgaggg taaagttggt gttttttttgt 90060
tattttttta tatatgagtg ggtaaggtat tataggagtt tatattttaa tttttgtggg 90120
ttattttgga aggtggtaga tggaattagt gattttttagg gttattttta gttttaggtt 90180
ttagataggg gttgtggagt ggaattgagt ttgatttagg tgtgttgatg gtagttatgt 90240
aattgtggat aaattatttt ttttttttatt tgtattggat gagattggga atttttattt 90300
tgggtttttt aagagttttg gggttttata gagtatattt agtagtagta gtgggatggg 90360
gggatagggt taggggagtt ttatgttttt tttaatttta attagagtat ttttatttta 90420
atttttttttt gggtttagtg tttagagaga atgggaagga agtttagttt tggttatggg 90480
ttattttagt ttgttagtta tagtttatat tttaggagtt tttttttggat tgaaggagat 90540
agagttattt ataatattta tagaaaattt tggagaggaa gtggagttta attgaggaag 90600
tgttttttttg tagtttttag aaatgaggtt ataggtgaga tagtgtgtta gggtggatta 90660
tatggtattt ttttaggttg tttgttggaa tttagggttt ggttaggggt tgtttatatt 90720
ttgggttttg aggtggggag tttgagtgtg tttttttagg gttgaatgtg ttttttttgttt 90780
atattgtata gagtatggtt gtttggttgt ggtgtgtttt tttggatagg tttgttattg 90840
tgtagatttt taattttggt agttgttttg ggtttgggag gtggtgggta tagatatagt 90900
tttttttttt tttggtagtt gttttgattt tttgggataa gtgttgagag gtggttttat 90960
ttagtgttat gtgtttgggg gttggggttt tattttgggg ttaggttggg tggttagggt 91020
tatagagttg taggggggtgg agtagagaaa ttgtttttag tgtttgtttt ttggttttat 91080
ttttttttgt agttgtgttt tatttaaggt atttttatagt tgtttggtat ttgttgtatt 91140
tttggattag tttttttttt tttttattat tgtatggtgt ttggtaggtt ggtgaggggga 91200
ggggtttttt ttttgggttt tggagttagg ttttgttttt tggttttatt ttgttttatt 91260
atgaaggaga tatattgggt ttggttaatt tttttgtatt gatttttgtt tttttagtat 91320

atgtgggatt ttttatttta gatttaggtt tgagaggttt tagggatagt ttggagaagg 91380
agggtatttg tttttagaag gggaaagaga aagagattta attttaaagt aaaaggggat 91440
ttgggtgttt tttgtaagta gtttaggaaa atgggaggag gtgggagtag tgggattttt 91500
tgagtaaggt atttggtag gaatgttagt gtttttgga tatttttaga agtagaagta 91560
tatagtttta tagaaaaatt ttgtaggttt aggatttgag gtatgatatg atttaaattt 91620
attgttttat tagatattag tgagtttttt gatgtttatt aaggtttttt gttttatatt 91680
tggtattgga tgaatttgta tattttgttt tagggagttt atagtttagg gataggttta 91740
gagtttatgt ggggtgtttt gtaaatatgg ggttagaagt tgggttgttt atgcggtttt 91800
tttttttaa attatagtgg ttattttttt tttaatttta tagtttttga tttttattag 91860
gaatttttgg ggtttagtag gtgttttttt tttgtaggtt gatgtttgtt tttttattt 91920
tttatttagg tttagttttg attttatttt ttttaaagtt atttttttat tgttttgtgt 91980
tgggtggttt tagggagttt tgtgtttga ttaattttat atttaatgtt gtattggtag 92040
ttttgttgtt tgtgttgaga ttttttttg ttttgttttt ggaatatatg gtttggtgtg 92100
gattaagtat gtgaagagtt atgtttatta ttattagttt gggggggttt gatttttgt 92160
tttttaatg ttttatttat atgataaaga tttttagatt ttttgggtta taggaaaatt 92220
attgagatat gttgttatgt ttattttgtt ttaattttat atttatgttt ataagtggtt 92280
ttagaattat tgtggatttt tttttggtt tttggggtgg gtggatatat gagagtttag 92340
ttttagtta tttttgtaga tgttttttg atagattaag aaattgaggt tgggaggggt 92400
ggaatgatag tttagaatta gttgatttat ggggtagagt tgggatatgt ttagaattga 92460
gttgggtatt gggtttggtt tttttttgt ttttagtgag tattttaaga aggtagagaa 92520
ttagatttat gaatgagtgt tgagtatttg ttttataagg tagttttgga gaaatttggt 92580
tggtgtggtt gaggagttgg agggggtaa ttttggtggg gggggttttg agttagtagt 92640
tagtttttgt aggtttagta ttgggtgttg tagtttagtt ttattttatt ttatttagag 92700
gtatttatgt tgggggtag gaaaagagta gggatttagg agaggaggta aggttgtttt 92760
tatttagagt ttgagaggat tagggatgat tttaaaggat gagtgttttt ttgtgggagg 92820
atagtaagga gggtgttaag tttaggggta gggttagagt tttggaaatt gagaatttat 92880
tttattagta taaagtttag attttattag gttttggggt ggaggagtag gtagggatgt 92940
ttttgttttt tgtagtgttt tttttttttt tttttttttt taattattta ggtgtttata 93000
tttttttttg aaatgttgat tttttttaga atttttatat tgaagttaat atttatttat 93060
tgagtttttt agtataattt ttagaggaga gattaggtgt tttgggggta aagttgatta 93120
ggaaagtttt tgtttggttt ttagggatga tttatggggg ttattatatt tgtttataag 93180
aattataaaa aagttatttt aatttattat tagtggttta tttttttagt ataattttat 93240
ggtatattta ttatgtttaa gttatttatt tggtatttta tgggattttt tttttttttt 93300
tttttttttt tgagataggg ttttattttg ttttttaggt tggagtgtag tggtataatt 93360
ttagtttatt gtagttttaa ttttttatgt ttaggtggtt tttttatttt aatttttta 93420
gtagttggga ttataagtgt atgttattat gtttggttaa ttttttgtat tttttgtaaa 93480
gatagggttt ttttatgttg tttaggttag ttttaaattt ttgggtttaa gtaatttgtt 93540
tgttttggtt tttaaagtgt tggatttata ggtatgagtt attgtatttg gttttttatg 93600
ggatttttta taaaaattaa gataggttgg gtatggtggt atgattttag tattttggga 93660
ggttagggaa gttgaggtgg gtgcggttatg aggttaggag tttgagatta gtttggttta 93720
tatggtgaaa ttttgttttt attaaaaata taaaagttag ttaggtgtgg tagtttatgt 93780
ttatagtttt agttatttgg gaggttgagg tagaagaatt gtttgaattt gggaggtaga 93840
ggttgtagta agttgagatt atgttattgt attttagttt gggtgataga gagagatttt 93900
atttaaaaa ataaaaaata aaaaaaaat ataaaaaaaa ataaagataa tagttttatt 93960
tttagaattt agtgggggta tgaaaggaat tttgaagatt atatagtaaa attttaagta 94020
tggatattgt tggtttatag gtgaatttta ggtttttttt tatttttgta tttttttgtat 94080
tttaaagaaa gggtatttga ttatttttta tgatttgaaa aattattttt atatttaaaa 94140
aaaaaagttt agtagaggaa agaaagttta tatataaatt atttatattt taagttaaat 94200
atgaggtatt tggattaggt tttgagggat gtataggagt ttattaaggt agtgatgtaa 94260
agagaagtag aggttagggt ttaggaggtt agggtaaagg ggttgtgagg aaaggtgtga 94320
gagattaagg gaagattaaa gtaggagaag ttgggaagga gagaggtgtg agttgagtag 94380
gagagtgaat taattgtgga ggggtttatg gtgagtgagt agagaaggtt tagtatggga 94440
agagatttta gtagtgttgt gttttgggga agttagtttg ggagtaattt agttggggtt 94500
ttaggagatt ttgttttgta tttatagttt tggggtggag ataaattata gtatagatgt 94560
tttttgtag gtagaagtta aggggttggg tgtttgttta gagggtatgg aattttttt 94620
atgatttttt tttttatggt gttagttgga tttttggtgt ttgggggata gagggggttgt 94680
agagattggt tagagttgtg tatgttgggg tttagggaat ttttagttta gttttatatg 94740
gaggggtaga aggggatggg tttgaaggaa agatgggtag gaagttgagt tggagagaat 94800

tggatttgtg tttagaatgg ttgtgatata gagagggagg atggagaagt aagtaggtgg 94860
tgttttggag gaagaattag tggggtttag gtttgggttt tttgagaagt tatttttatt 94920
ggattttaag agtatttttt tattgggaag gttttgtttt tgtttgtttt tgtatttttt 94980
ttatttggat ttgtttaggt ttttaggatt gttagtttag tttatggatt tttttatgtg 95040
agttggattt ttatttgtat aggttttttgt ttgtgggtgg ttgggtttgg gatgttgatg 95100
gttttttttt atgttttggt tttaatgtgt atattttatt ttatggagaa agttattgtt 95160
tttttagagt gttaggtttt tggtagtttt gaatgttgtt gtttttatgt ggtttgtttt 95220
tttttttttt ttttggtttg atgaaattgt atttttattg ttggggaagt tttttggata 95280
tttttttgtgt tttagttatt ttgtttatgg tttggtgagt tttagaaagt agagtttgta 95340
ggttgtgttt ttgggtttgg tttttggtat agtttttggt ataatttagg ggttaaattg 95400
tgtttgtttt tggaggagaa tgtttttgta tatttttatg gatattttt tatttgtttt 95460
ttagagttag gattatgttt ttgtgtattt agtattagtt attattgaga tttagatggt 95520
ttggtgataa ttgttttttt agttatattt tgtttgattt agttgagtgt gaatatatta 95580
atttaggatg ggggtgtatt tggtatgtta aataaaggtt ttgtattaat agaaggtagg 95640
ggaggaaata gtttttttgtt attttgtaaa ttgtttatgt ttttggtttg atattatgtt 95700
attagtaatt attgttattg agtttgggtg gttggggtag ggtggttttt tagagttaag 95760
tttagagaga tgggagaggg aatatgggat ttgtatattg agggttggaa aggatttttt 95820
aaggtttatt agtttttggt tatttagtgt gttttgatgt ttttttttgta ggaaatgtag 95880
agtttaggt tttagatttg ttaagataaa tgtgtatttt atatgtttat gaaagtatga 95940
ggagtattgg tttttttttta aatttatatt tttttaggt tttatatttt tttagttttt 96000
aagtttattg attattggag aatttttatt taaaaagaaa aattaaaaag gggttgggtg 96060
gtttatgttt ataattttaa tattttggga ggttgaggta ggaggtttat ttgaatttag 96120
gagtttgaga ttagtttggg taatttagtg agaatttgtt tttataaaaa aaatttaaaa 96180
attagttagg tatggtggtg tgtaatttgt ggttttaggt atttgggagg ttgaggttgg 96240
ggaattattt gagtttaggg ggtagaggtt gtagtaagta gtgattatat tattgtatgt 96300
tagtttggag gatagagtaa gatttttgttt taaaaaagtt aaagaaaagt taaagaagat 96360
ggaaagaggg atttagagag atgtggaagt aatttaaggg tttattgatg gaggaatagt 96420
tatataatat aagtgtattg tataatagaa tattatttag tttttgaaag gaaggaaatt 96480
ttgttttatg ttatagtatg atggatgaat ttttaggatg ttatgttgag tgaaatgagt 96540
tagatataaa atgggtagtt attgtatttt tttatatata taaggttttt agagtggtta 96600
aataaattta taataaagat agaaaataga atggtggttg ttagtagttg ggggaggggt 96660
atggggagtt tggtttaatg gggatggagt ttagtttggg aggatgaaaa gggtattgga 96720
gatggatagt ggtggtagtt gtgtaatatt gtgaatgtat tgaatgttgt tgaaatgtat 96780
attgtaaagt ggttaaaatt gtatattgta tgttgtgtat tttattatgg ttagaaataa 96840
gaagtagtag ttaaattaaa aatttagatt gtgttgtttg tttaatttaa agaaggttta 96900
ttgagtatta ttgagttttg attttgtgtt tagaattaga tttggttttt gaggggtttt 96960
taatttagtt ggtattttat tatagtttta tggagttatg atttttgttg gggttagggg 97020
gtatgttttt gagaagggat ttaattatgt tttatagttt ggtattttta aataggtttt 97080
gtgttttata ggattggtat taatttttttg tttgtaagtt ttatgttagt atggttgtgg 97140
gtttgagttt ttttaggatg ttgttggagt tttatggttt tttttttttt gttagaaggg 97200
tatttttaaa taaaattgtg tgtagttatt atatagtttt ttggtttagg tatttttttt 97260
tagtgttttg agaattttgt tttgtaggtt aaggtgtttt ttatgttttt tttttttttgt 97320
tataattatt tttttttgggg gaggaggatt agattatggg ttttaatgta agttttagga 97380
ggtatatgtt tttttttttt tttttaagtt tttggttttt gttgggtata tttaggtagt 97440
ttgtaatttt ttaagtagga gttattagaa tgtgatagtt tttttagggg ttttaatttt 97500
attttgtttt atatttttgt atgagtttgt tttggttttt gtgtggtttt ttattttttta 97560
agttttttag gtttttttagg gatatatagt agatagaggt tttagtaagg tttatggtga 97620
tttataaagg aggtttttttg aaatttagaa aattttttta aatttttatt taagttttgt 97680
ttgggggtatt ttggtatgtt tttattagat agagagagtt atttttttttg agtttgtaga 97740
tttttatttt tatgtatttt ggtttttttg ggtttttagg gtagtatttt ttattttata 97800
tttagttttt ttttttaaga ttttgtggta aagttagtaa ttaggttttt tattattata 97860
gtatttaaga tttagtgttt tgtgatttat ttttttggtt ttggggtgta gttttagtta 97920
tagggggttg gggtttgggt tttagtttgt tttgtagttg agatataggg gtaggttttg 97980
aatttttttaa ggtttgggtt gggttggggg tggtgggtgt tttagtaggt ttgggtattt 98040
ggatttttat ttttttgggg gttgtgttgg tggtaggggt agtgggtatt ttggggattt 98100
tggaggtagg gggttttatt ttttggtggg ttgattttttg gggtttgatg atggtgattt 98160
ttgtttttttg aggggttggt tttggtgttt tgtggtttaa tattttggtt tttttgagtt 98220
tgaattggga tgggttgatg gttttggtgt tttttatttg tttggatgag atgttaatgg 98280

atagtttagt gtgttgggat attggtttgg ttgttttggg gtttgagagt tttatttttt 98340
gtagggatgt tttgggggag tgttggttta gggagtttat atggtgggtt gagggttttg 98400
agttttttat gtttaggttt tggaattttt gggtggagtt ggttatagtg gtttggagta 98460
ggggagtttg gatttttttgg ggtggggtgg agttgggtgt tttgattttt ttgattttaa 98520
aagattttttt taaggttgaa aaataaagag aggggatgta attggtgaat ggatggttag 98580
ggaatatttt ggtatgtttt ttgttaagga gtagtagagg aaggtaggtg atgtggtggt 98640
gagggttggg ggtttttttt tgttttttat gttttagtaa gtggaaggga tagttttatt 98700
ttaggagaat tattatgagt tttggggtag agatgtaggt tggagtggga agatgttgtt 98760
tgttgtgttt tgttatggtt atggtgtaaa ttatatagtt ttttttttta gttatgttta 98820
tataggaatg ttttagtttt agagaagatt tttatttttt ttttttttttt tttgagatgg 98880
agttttgttt tgttgttttg gttggagtgt agtggtattt tttgggttta agtgattttt 98940
ttattttagt ttttttagta gttgggatta taggtatggg ttattatgtt tggttaattt 99000
ttgtattttt agtagagatg gggttttatt atgttggtta gtttggtttt ggatttttga 99060
ttttaagtga tttatttgtt ttggtttttt aaagtgttgg gattataggt gtgtgttatt 99120
gtgtttggtt ttgatttttt ttttgttgtg gttgttttta ttttttttta tttttagtta 99180
aggtatatat tgggatatta ttagtaaata tatttttatt attagttata gtgggggata 99240
tgtttttaat aataagtgat tgtaattgta attaattaga attgtagtag gaataaaatt 99300
ttattaatga tattaagggt ggatattgga gtttttagtg ttggtgaggt gttgggttta 99360
ttgagtttat tatatttgta gttgtttaat tttgggatgt aaagaatatt agtattttta 99420
gtatagatgg ggaaagtggg gtatggggta gatatagagt tagattgtat attaggttga 99480
tattagagtt taaggtttta attatttggt tgtttggttt ttttagttta gtttatagga 99540
attaattttt ttagttatta gtttttttttg tattttttta tttattatta taagtttgtg 99600
tagtgatgtt aattattagt ttttgttttg tagttatttt gtttttttat tagggtttgt 99660
ttttttggag gtagtgggga tggtatttgg gaagttgtta tttttatagt ttttataatt 99720
gtattttaaa tgagagttag ttgtttttgg ttattagggt tagagtaggg agggatttat 99780
atggtaggta tatttattag tttttttttt aggttagatt tatagtgtat ttttgtggag 99840
atttatagtg tttttttagt tgttttttgt tttattaggt tatagagttt gggttttatg 99900
ggattttatt attgtttttt ttgtttatag gaaaagggat gatagttggt ttttagtttt 99960
taataatagt gttttaggat taattttagt gtttttttttt tatttgtttt ttttttttta 100020
aatgtattta ggtattgggg aggggaagag gaagtttaat tttttaattt tggttgtttt 100080
agttgttagt tttttgggta aggtattttt gtgggtgttt tttttaagtt ataggtattt 100140
tttttttttg gtggatatag atgaaatttt gggttattaa gtttaatttt tattagaagt 100200
tgggtaagga gttgtgtgga ttttagagaa tttttagttg ttataggttt tgttttagtt 100260
ttgttattgt tttagaggat atgggtttgg tgtggttaga gttgtttatt ttttaagaga 100320
gtagaaattt ggatttttat atgatatttt ttgattttta aatgttggta taaagtaaaa 100380
taagtttttt ttaaaatatt gagtagtttg gttgatgtgg tgaaatttta tttttattaa 100440
aaataaaaaa ttagttgggt atggtgatat ttgtttatag ttttagttat ttaggaggtt 100500
gaggtaggag aattgtttga atttgggagg tagaggttgt agagttgaga ttgtgttatt 100560
gtatttagtt tgattgatag agtgagattt tgtttttata aattaaaatt aaaaataaaa 100620
taaaatattg tagggttaaa taaaattaaa tttataggtt gtatttgttt atgggtttag 100680
ttatgatttt tagttgatta tgtggttgta gggtagtagg atattgagtt ttagagttag 100740
gggtgtgtta ggtgtgggtt tttgatatgg tgtttttttg ttttttttgta ttttgtatgt 100800
aaattgttgg ttgtgttttg attttgtaga agtttagggg ttttaaggaa aggttgaatg 100860
tttattttta ttagttagaa agtttagggt ttaatgttta gaattgaatg taatgagatg 100920
ggtttattaa gtttattata tttatatttg tttaaatttg ggttgtaaag aatattatta 100980
ttgtttttgt atagatgggg aaagtgaagt atggggtaga tatagagtta gattgtatat 101040
tgggttgatg ttagaaagtg tagagtttaa tgtttgggtt tttgtgtggt ttttagggtt 101100
ttttgtggtt ttttgatttg tttttttgttt tttgagtatt gtttaaattg tttttatatg 101160
gtgtgttttt gttttatggg tgttgtagag taggggttgg tttttaggtg tttttagtat 101220
tttttttgtgt ggttaagttg gagaatagaa attagatagg agaaggtttt gttttaggtt 101280
taatttaaga aaataaatga tttttttttgg gagatttggt gaattttttt tttgggagtt 101340
tttttggagt tatttttatt atggttaatt tatgattgaa gatattgtat taaagtagag 101400
gttgttttgg tgtgagttta tgattgagtt tatggtgtgg atgtaatgat ttattttagt 101460
taggtttatg tgtataagtg gattatttta gaattggagt tggttttttt tttagggtat 101520
ttttttttttt tttagttgtt tgtgtggttg gtgttttatt attttggtta tttgtgtaaa 101580
atgtatatgt gtgttggttt ttaattattt ttttttttttt tttaatgttt ggtgtttttt 101640
tatggatggg ttttttttttt tttattagtt ggtttttgtg tttattgttt ggttttatga 101700
tttttttggg tgttttggtt tagtattatt tttatgttta ttagttttta gtaagttttg 101760

agatagtagg ggttgtttta tttgtagggg ttattttatt attaggatgg tgttgtttaa 101820
aggtgagttt gttttagata atagttagga gattttatag tttttagaat tgtaagagaa 101880
tataattgtg tgttagaaga atagtgagaa taggaggtgt tttattttgg aggtgtttat 101940
tttttttagg agatgtttta ttttgggggt gtttattttt tttaaggaaa ttatgttttg 102000
gttggaatgt taagaaggtg atatttaaat gaagaggtgt ttattttggt ttatgttatt 102060
tttttaaatg agtatgttat tgtttttttt tttttttggg aatttttttt aaagttaagt 102120
ttattttagt tttttttttg gggagttggt tttttatata gtttatatta tttttttttt 102180
atgttatttt ggttatgtta tgattgttga gattggttat gaggggtgtg ttttggtttt 102240
ttgggaatag agttgaatgt tttttatttt atgatattat gtttttatta ggttttaggt 102300
gtttttagaa aatttattaa gttaggtgtg gtggtttatg tttgtaattt taatattttg 102360
ggaagttaag gtggatagat tgtttgagtt taggagttta agataaattt gggtaatatg 102420
gtaaagtttt gattttatta aataatataa aaattggtta ggggtggtgg tatatgtttg 102480
taattttagt tatttgagag gttgaggtga gaggattatt tgaggttagg agattaaggt 102540
tgtagtgagt tatgattata ttattatatt ttagtttgga tgatagagtg agattttgtt 102600
ttaaaaaaaa aaaataatta aaatttatta aatgttaggt gtggtggttt atgtttgtaa 102660
ttttagtatt ttgggaggtt aaggtaggtt taaggtaggt ggattatttg aggttaggag 102720
tttgaaatta gtttggttaa tatggtaaaa ttttgttttt attaaaaata taaaaaatta 102780
gttaggtatg ggggtgggtg tttgtaattt tagttatttg gaaggttgag gtagaagaat 102840
tatttaaatt tgggaggtag aggttgtagt gagtttagat tgtgttatag tattttagtt 102900
tgggtgataa agtaagattt tattttaaaa aaaaggttgg attataggat tataggatag 102960
gtataagttt tgagggagtt tttaaggagg agggatgtat tagattttta tttttatttt 103020
agtttgtgaa gatttatttt tgtttgaggt tttgtagagg gttgattagt gatttttaaa 103080
aagatatgtt taatatttaa ttttaagaat ttatgaatgt gattttattt ggaaaaaggg 103140
tttttgtaga tgtgattagg gattttggga tgagattatt ttggatttgg gatgggtttt 103200
aaatttagtg tttggtgttt ttataggaga aaggtagaag gggatttggg atataaagag 103260
atatggtggg gagggttatg tgatgagagt agagattgta gatgttgtta gagttaagga 103320
gtgtttgggt taatgtaagt tggaggaggt aaggaagggt tttttttttg agttgttgga 103380
gggagtgtgg ttttgttaat attttgatgt taaatttgtg gtttttagaa ttatgagtga 103440
attaattttt gttgggttat ttggttttta gtaatttgtt ttagtagttt taggatattt 103500
ataatgagat ttttgggaag atgaaatttt tggttgtata gtggtttagg gtgaggttag 103560
ttggtgtggt tatttatttt ttagtttagt tttaatttta ttataattat atttgataaa 103620
tggtttattg tgggttaatg gatatgtggt tattttagag ttgttatatt aggagtttag 103680
aggagtgaga gttgtttttt tttttttttt tggagttgta taaaaatttt agatggttgg 103740
agaagttatt ggaggaatta tattatttta gatttttgtt ttattgttgg gatatgtggt 103800
aatgaaataa gattattttt gttagggatg ttttatgggt aattgttttt ttatggagga 103860
tattgtgagt tgtatttagt tgggaaatat taggagatgt ttgttattag gttggtagaa 103920
tagggttttt tagtattttt tttttattta gagttttaag attgtgtttt gggttttgta 103980
gttatatatg gttgtttttt tttttttaat gtgtaggttt tttgggaagt tttttgttat 104040
tatttttata gtggaattgg tataatttgt ttaggggtag taggttttttg ttatttttata 104100
tgtttttatt ttttttgtgg agggagatgt agttaagggg tatttatatt tatttataga 104160
agtgagagtg tttgtgagtt tgttattttg tagattttag aattttgtat gtgattgtat 104220
agatttatag gtttaattgg gtagatttaa aggatttatg tagtagggag gagtattgga 104280
tagttttttt ttttgtttgt aaggagttaa tgtaattgaa ggatatagaa gaggttttta 104340
gttgagtatt ataattgtgg attgtgtata gtgttagaaa gatgtggagt taggttatgt 104400
tgggaggatt tttatgttgt tattggaagg taaagtagtg ttattttttt atttatagag 104460
taggaggttt ttattttttgt tagttatttt ttttatttgt gaatttaagt tttttaaagt 104520
tagattttaa ttttgtaagt ttttaaaagt ttttttgggt ttttttttta atttttattg 104580
atttaatttt gttttaaaag tttgaagatt gtttatgtgt tttggtttat ttttgggaat 104640
ttattttaag gaaataatta gaagtataga aagatttatg tatgaggata tttgtattag 104700
tattaaattg ataaaatttg gaaatagttt gggttttttgt agattttttta aatttatatg 104760
ttaaaggttt gtattattat taaaattata tttttgaaga tgagaaatgt tgagaatatt 104820
aagtaaaaat agtaggatat aagatgtata taaagatttt aattttattt tatatatata 104880
tgtgtagttt gtatgtatat atatatttat atttgggtag aaaataatga tttttggatg 104940
gaatgaatgg tggtgggtgt tgtttattat atttttttgt tttttgagat gtttatatta 105000
tatggggatt atttagtatg tttagggaat aaaattattg atattgtttt tttttttttt 105060
tttttttgag atagttttgt tttgttgttt agattggagt gtagtggtgt aattttggtt 105120
tattgtaagt tttatttttt aggtttatgt tattttttttg ttttagtttt ttgagtagtt 105180
gggattatag gtgtttggtt attatgtttg gttaattttt ttgtattttt agtagagata 105240

```
tgattttatt gtgttagtta ggatggtttt aatttgttga ttttatgatt tgtttgtttt  105300
ggttttttaa agtgttgtga ttataggtgt gagttattgt atttggttaa tattgttttt  105360
aaaaaatata ttttggtatt ggttgggtat ggtggtttat atttataatt ttagtatttt  105420
gggaatttga ggttagagga ttgtttgagt taggagtttg agattagttt gggtaaatgg  105480
taagatttta tgttggaaaa ataaaattaa aaaaaaaaaa atatatatat atatagattt  105540
tgatgttaat aagttggagg gtgtgggtaa ttgtatgttg gtttattttt atttttattt  105600
ttggaaaaat attttatagt ttaggatggg tatagggttt tttttatttt taagtattta  105660
tggttttagt tgttatataa aaagaggtta tagtgggtta tttttttagat gtaaattttt  105720
ttttttaggt ttatggtggg gttgaaatag gagtagtttg gatttgggtt tatttagaga  105780
ggaatgtgtt gtataaggga ttattgttaa ggtttttgtt ttgattatag gttgtttggt  105840
aaattgtttt tagtttttga aattagatta agtatttttt ggtgtatata ttaggtttta  105900
gtttggggag ggaatattaa gtgagttata gagttttgtt ttagtttggg ataaatgtta  105960
tgtaaagaaa tggtagagtt taaggttaga ataattttat gtgtagttta gtttttgtag  106020
gagggagtaa tgtggttttt gtttagggta gggtggaatt ttaggtttag gttttaggaa  106080
tgttagtttt ggttaaattg ggtttttttt gagtagtagt aaagtaggtt ttgtttttga  106140
agaaatattg gttatttagg agttagggtt agggtttagg gaagttggat agatggtgtt  106200
aaggagtagg gaagtatttt tttgtatgtt attttgagtt tgtaggaaaa agttatggga  106260
aaattttatt tggtagataa tttttataa tttttaggat ttatagagtg agtttagtag  106320
tatgtatgtt atgggtattt ttttgttttt attttttttgt gatattggat tttggtatag  106380
gtttttgatt tgtgtagggt ggggtgggta gttaggtagg gttttggtgt tggtagagtg  106440
gggtttagtt gtaaaggttt ttttattttt tatttattta ggatttgggg gatgaggttt  106500
aaaggtttgg tgtgtttttta aggagtttaa ggattgtttt agggttggag ataggatgta  106560
tgggtttgta tagggattgt gtttttttatt ttagggttga gagtaggaga attaaatagg  106620
agagtgatat aattatagtt tattttgagt tttagtgttt ttagtgttat ttgttgtagg  106680
aggtgatggg gtaataaggt gtttttgagg ttaggtgtgg tggtttatgt ttgtaatttt  106740
aatatttttgg gaggttgagg tgggtggatt atttgaggtt aggagtttga gattagtttg  106800
attaaaatgg agaaatttta tttttattaa aaatataaaa atagttaggt gtggtggtgt  106860
atgtttgtaa ttttagttat ttgggaggtt gaggtagaat agtttgaatt taggaggtgg  106920
aggttgtggt gagttgagat tgtgttattg tattttattt tgggtaataa gagtgtaatt  106980
ttattttaaa taaaaataaa taaataaata aaaaataaga tttttgggt gtaggtatgg  107040
tatgtggtag gggtgattgt tgttggtgta attattattt tttgggtgtg tgtttgtggg  107100
ggttgtgtat gttgaaatgt gtggttattt tgttggtgat taaggttgtt ttggttttat  107160
tgttattaat tatgtttatt gattttatat ggtttatttt tgtgttttta tttagagttt  107220
tttttagtgt ttaagttttt ggaaggttgg aaggagtgag tagtgtagtt tttggttaag  107280
aattgaagta taaaaatttt agttttttta tttaatttta ggtgggataa ggttgggggt  107340
atggtttgta gtttttttat gttgtttggt atgggggttg ggtagttatt tgtggttata  107400
atgagttggt gaggtatttt ttattggttg gatttttttgt ttggtttatt tttatattta  107460
ggtatttttt ggtgtttttt tttattgaaa tttttgtttt aggatttgtt tttggaggga  107520
tataaattag tttttttagaa ttggtttggg ttgtttggtg gaggagggag gtggtttttg  107580
gggtaggtgg agtaggtttt ggatagggag gaggatatag ttggggtgat ggggaggggt  107640
gtgtttttag gaagtaaaat tgtttaaaag gaaaggttta aaaataggtg tagtgaagtt  107700
gagtggagtt tgtaaaggga agttaggaaa tagtagtgtt agtggagggt ggtttttagg  107760
tttttttatag agagttatat ttgtttaggg atagggataa tgtgaataga aggtaaagtt  107820
tatattaagg tggaggaggt gttttttttt tagagagttg tagttagata gtttaaattt  107880
gttaaaggtt tgggagggaa gaattagggt attattttat ttttagttta attttttttt  107940
tagttttttt tttttttgtt tttttaaggt taggtttggt tgggttatag tgttagtagt  108000
taagatttgt ttttagtgtt ggtttagttt gtaggaggtt agtttggtgg ttaaaggtta  108060
ttgattgttt aggtttattt gtatatgtgg tttaatgatt gaggtttggg atttggggtt  108120
attatttata gtatttttgg ataaggagga aaagaattat ttttattaat ttttggatgg  108180
aggtgtaggg agtgttaata gtatttttga aaattttaaa tgtattaggg gggttagtat  108240
ttagggaaga gttataggag gttattttga tggattttga tgattatttt tgtttaagtg  108300
ttaggaggag gttttttttt ttttgttttt ggtaattttg ggtttaagaa gttttaggta  108360
ttttggaggt tttttttgtt tattatagtt ttatttaaga ggtttggttt attttgtaat  108420
gatgatgtga gaatgagtag gtttgtttag tgttagagtt tgtggttgga tttttggttg  108480
aggttaagag aaataaataa gtttttttttt tttttttgta tgttatagga agagtagttt  108540
gttggttgta aggggatttt ttattttgtt tatggggtat agtagtttat agtatttttt  108600
ttagaaatgg agtattggta ggtttgggtt tttgtaagat ggaatataaa ggggtgggta  108660
gttgatttta atagttagtt tttgggattt taaatttttt tttattttttt aattttgtga  108720
```

tgttaatttt tttttggata aagtttttta ataatttggt aagttagtat tagttgaatt 108780
agttttagtt tgaattagta gtttttttta gtttagttat agttttattt ttttagaagg 108840
gaaaagaagt tttatggatt tttttataag gaatttttag aatttgttat ggtgtttgta 108900
ggggatattg gtattaattt tgggtttatt aaatgtagtg ggtttaatag tgtttttttaa 108960
aatgatatgt tttaagtttt aatttttgga atttgtgaat atgattatat ttggaaaatg 109020
gatttttgta gatgtaatta aggtaagggt tttaaggtga gattattttg aatttagtgg 109080
gttttaaatt taatggtgag tatttttata aaagatagaa aaggagaaag atatatagat 109140
agggcgagaa agttatgtgg agatagggt tagagattag agatgatggt gatgtgtttg 109200
taggttaagg aatgttagga atttttagga gttgttggaa gttatgtttg taagttttta 109260
ggttgtggta ttttgttata gtaattttag gatatagatt ttaggaatag aatatagaaa 109320
agaaatggga ggaggtttag agtttgttgt ggttatttta gttattttat ttgatttttt 109380
tagtgttttg ttaattttga agttttttt agttattgag ttattggttt tgaaaaggag 109440
atatgggtgt tttggagttg gtttagtttg gtaggaggta gttttgtttt attgtttttt 109500
tattagttat tagtttatat aggtttttta tagtgggtaa gtgtgttgtt gttttagttt 109560
ttgttggttt tttttttgtt tggttttatt agttgtagag tttttttgttg ttgttttttt 109620
tggtttaaga ggtttatata tttagttttt ttgggataaa tttagtttat atttgttgtt 109680
ttattgtagt tatttaaggg ttttttttat taatgtgttt tttaagggat agtaagttat 109740
atggttgttt tatttggggt tagaattgtg ttttattaga gtaaggtttt ggagtaatta 109800
tatttagggt tagggagtta tttgtttaag aatagaaatg tgtggttggg tgtggtggtt 109860
tatgtttgta attttagtat tttgggaggt agaggtgggt agattatgag gttaggagat 109920
tgagattatt ttggttaata tagtgaaatt ttgtttttat taaaaatata aaaaattagt 109980
tgggtgtggt ggtgggtgtt tgtagtttta gttatttggg aggttgaggt aggagaatgg 110040
tgtgaatttg ggaggtggag tttgtagtga gttgagattg tgttattgta ttttagtttg 110100
ggtgatagag tgagattttg ttttaaaaaa aaaaaaaaaa agtgaaatgt gtagtttttt 110160
ttttttttat ttttgaaata tgaaggggag gtgattgtag gtttttttggg aaagattttt 110220
ttattttttt tttttttttt tttttttaag atggagtttt gttttgttgt ttaggttgtg 110280
gtgtagtggt gttattttttg tttattgtaa gttttgtttt ttgggtttat gttatttttt 110340
tgtttttgtt ttttgagtag ttgggattat gggtgtttgt ttgggatagt tatgtagtag 110400
tttttgtttt aagttatttt tttttatgta gagtagaatt aggattatag taaaggtttt 110460
agaggaggtt attgtttata gggttttggg tttttttttt gaaaggtagt ttagttttta 110520
agagggtaat agtgattgat ttgggatgaa ggtttttttg tgtttgtttt tgttatttat 110580
tgtgtgtttt tggttttggt agttataggg ttattttagg tggattttaa agtggtgtta 110640
agttgttgga tttttttttt tttttattaa attagttatg ggaatgggag tatttttggt 110700
atatttttag atgtgttgta ttggtttagt gtttttttta tttttgggtt ttaaattagt 110760
tgttggttgt tttattagga gtagattgag gggaaatggg tggaagttgt aggggaagag 110820
atgtgaggta gataaagagg aagaattttt tggttggggt ggggttgtta gggtttgggt 110880
gggagggttg tttatttttt gtttttggtg gaaggtagta gaagggtggg atggatttgt 110940
gtgggagggt ttaggtgtgg tttgtttggg ggtaggagag ggattagatg atttttttagg 111000
tttttttggg ttaaagattt tttgataaat ggttgttgtt gaaggtagga gtgaggtttt 111060
gggaggtgat tgattttgaa aaattatagg ttgtagtttt gagggtttta aaggttaagt 111120
atggagtttt ttaaaatgag gttttttttta gttttaattt atttatgttt tttaggtggt 111180
tttagatatt ttgttagggt ttttttggta gttttttgtt tttgattttg gagatagaga 111240
aatgtgatgt tggtttttgg tttgtttata gtttttttgt ttttattata gggttgttag 111300
ttattttttt agtttattgt gtgttttttt tttttttta tttttgattt tttaataata 111360
gtagattttt ttttaaatat tttttttgttt agattttggg ttttatgtat gttgttttta 111420
gaaatgtatt ttagtttttt gtgaggagtt agtaaggttg tttggtgttt tttttttatgg 111480
atgggaagtt ttgttgggga ttaggatttg gatttggaga ttttgaggag tgtgttggtt 111540
agtgtttgtt ttgaggtttg ggatagagtt gttaggtata ggtagtattg gtgtgagggt 111600
ttagtttttt ggataagttt tttttttgttt gtttttagat ttgttattgt atatataggt 111660
ggtgattatt tttgatttgt ttgggtagag aagttatttt tttttttttt tttgttttat 111720
tgtttattag gggaaatgtt ataagtattt aaaaaatgta aaaaaaaaaa aaaaaaaaaa 111780
aaagtgttta atattagggg ttatttggtt taggtggagt ttttattaat tatggggttt 111840
tttgtgttgt ggttttaagg tgggtagttg atggtttttg tttgagttaa tatgagttgt 111900
agtttaatta tagggggatgg tgattttagg gagtagagtt taggttttgg ttatagttgt 111960
atttaggagt tagttgtttt ttagatttgt agagttaggg gagaggtttg gaggattggt 112020
attgaatgat atgatttaaa gtgtgttaag ttgggttgga ggtgatggta gagttatagt 112080
ggttatagag tttttatgtg aatttatatg gatttgtttg gtttaggttt tgttgaattt 112140
tagggtgagg aggatttggt ttttattttt aaagatatga agaggttgat aaagaagagt 112200

tataggtaaa agggggtggt gggtgtgttg tgtaggaggg gttgtaattg gatgtagaag 112260
attttagaag gggtaattag agagaggtag tatttttaga agatgggttg tttggttgtt 112320
ttaaatagtt tagggtgtga ttgaggggat ttttggattt agttggggtt ttttttgttt 112380
tgagataatg tattgggaaa ttttattaag tttatttaag aatttgaaaa gtttttttgga 112440
aatttagtgg gtagggtggg tttttgggag gatttttggt atattgtgag agttagatat 112500
agtgaggggg ttttatattt ttgtttagaa gttttatggt agaggggtag aagtttaggg 112560
aggagtaggt gttgagatta gaggggttta taagagaggg gtttggggta gggggtagtt 112620
gttgagaagt ttaggaaatg gtgtttgttt ttggggaagt gaagttttgg gaatagggtt 112680
ggtgaagggg ggtgggaagg gggaggtggg ggtggagggt taggttagat taggtatttt 112740
ttttagtagt agatttatga gtttttggaa tttgtttata gaagtgataa gtgttttgtt 112800
tttagttttg tttatatatt tatttattaa gtgtggtagg gatagttgtt ttttattatt 112860
tattttggga tttggattta agtggaggtt tttggagggg tagatttagg ggaaagggtt 112920
tttttagaag tagttgttat ttagaggatt tgagatgaga attttattat gtttttatta 112980
gatagaggag atttgtggtg tttttgagtt tggtttttta tttttttattt agtagtgttt 113040
tttttgttta ttttaagttt agaatatttt gttttttttta agattagttg ttgtagtgga 113100
ttgaatagtg atttttaaaa gatatgttag agttttagtt tttggatttg tgaatgtgat 113160
aggagttgga atagggtttt tgtagatgta ataaaggatt ttaagaggag agtattttgg 113220
atttagggtg ggttttaaat gtaatgataa gtatttttat gagatataga gatatagaga 113280
aaaggttatg tgaagatgga gtagggagtg gtgtgatgta tttatatgtt taggagtatt 113340
tgggagttat tagaagttgg gagagaggtt tgggttggag ttttttgtag aggttttaaa 113400
ggattagttt ggttgatatt ttgattttgg atttttggtt tttagaatta tttttagttt 113460
ttaaagtttg tggggttttg ttgtagtagt tttagtaaat tgttatgggg gttttttttt 113520
tttgttaagt ttggtattta aagtttttta gttatttggg gttaattata ttgattttgg 113580
gatttttagg ttatttgtgt gttttatttt tttgttttaat ttttagtgtt tgtgtggtta 113640
tagttttttt tttgtgatat gagtttttttg ggtttttgag tgtttatttg ttttgtattg 113700
aaggttgaag ttaaattttg gagagaattg atttttttttg tgggtagggt aggtattgtg 113760
tttttagtat agttagaggg tattggttga aatgtgtttt tgtaaagtgt ttttttttatg 113820
tttgtatttt ttttttaagt ggggaagtta gagaggtttt tttgattttt tttttagttt 113880
tttggggtag gaagtttgtg gttatttgtt tgtaaggatt aggtggatag ataattagtt 113940
ttttttaggt tatattttat taatagaggg ttttttaatt ttgttggtgg tttatgagag 114000
aaggaaaaat tgaaggagtt gtttatagta aattttaaat ttttgtgtta gagaggttta 114060
tggttgagta aggtattttt attttattg ttttgagttg ttaatttagt ttagtttagt 114120
attgtgattt ttgatttgtt tgtgggttat gggagtttgg tttgttaatt agtatttttt 114180
agatatttat tttgtgaagt ttttagtggt tgagtgtttt tttgtttttt ttggatagtg 114240
tgggttatag gggatattgg gtaggaagat agttaagttt ttattgttta gaaattgttt 114300
gggtttattg tagtaatagg gatttattta ttagtatgag gttttttttgt aggaggggtg 114360
ggggtaggag ggagtttgtg gggtataggg ttgtaggagg agttattttt tttttttatta 114420
aattgtaggt tttagttaag ttatttttag tatggttgtt tgtgtgggaa gtttatagtt 114480
gagggattgt ttgtatgtgg tatgtaggag ttataagtgt tatttatatt ttattttggg 114540
ttaggtagat gatttttttg gttttagttt tttgatgagg aataggaagg gttggattga 114600
aagatttttt ttagtttgga tatggaatga ttttatgagg gttatggtga ggtttgtttt 114660
agttttgtat attagtttgg gttagtagtt tttaaaggta tagttgtagg ggttttggag 114720
tagggataag gaggattagt ttattttatt tttttgggta gaggttaagg attttgtttt 114780
tggtttatat tttttgtggt taaaaatgta gaagtaagta gtgttggtag aggtatttag 114840
atttttgggt aaagtagggg ttttatttga tattttaggt ttggagtatg agaaaattag 114900
tttaggtttt aggtttttttg attgttagtg attagggaat gtgatatttt ttggtttttg 114960
ttttttttatt ttttaatttt agggggagat taggttgggt gatgtgtttt ttttttttgat 115020
agtaaatttt tatttatttt tatttttatt tttgttttaa gaggggggttt tttatggttg 115080
ggtgtagtgg tttatgtttg taattttgga atttgggag gttgagatgg atggattatt 115140
tgaggatagg agtttgagat tagtttggtt aatatggtga aattttgttt ttattaaaaa 115200
atatgtgagt tggtaatttt aagttttttag tagtttttga atggaggttt ttttttttgta 115260
ggatttttaa attttataaa tatgagtgtt tgtaatttag ttatttagga ggttgaggta 115320
tgagaattat ttgaatttgg gaggtagagg ttgtagtgaa ttaagaatgt gttattgtta 115380
ttttagtttg ggatatagtg aaattttgtt aaaaaaaaaa aaaaaaaaag gtgggggttt 115440
ttttatgttg tttaggttgg ttttaaattt ttgggtttaa ggaattttttt tgttttagtt 115500
ttttagagtg ttgggattat gggtatgagt ttatgaattt taaaaaataa tatttattgt 115560
tttgtttttg attgtaaaag gaatttaggt ataaagttga aaatttgtaa aatgtataaa 115620
aataaaggga aaaaatttat aaatttataa tttaggtaat ttttattaat attttgataa 115680

atgttttttt atttttatt ggggagtgtg tgtgtgtgtg tgtgtgtgtg tgtgtgtgtg 115740
tgtgtgtgtg tttaaatagg tttatatagt gatgatgttg ggtgtttttt tttatgtggt 115800
gttatgtggt tgaggttttt atatattttg ggtttgtttt tttgattttt ttgtagtttt 115860
gatgttttga ttttagatat gttattttttg gtttggattg tttaggagaa ttattttttgg 115920
atttgttaa aaaatggatt gttggttttt aatttttggt ggttttggtt taggagggtt 115980
gggtaagttt gggtattttg gagtgttttt ggggtgttgt aggtgtgaat ttttgttttt 116040
ttgaagattt ataagtttta ttttttttat tttttttttt ttttttgaga attttataag 116100
aggaaagttt ttatttggag gttgttaggg atttggagtt gttagtttat agttttattt 116160
tgtttttattt tttttgggag ttatttaagg ataatgttta gttttagttg ttaagtttta 116220
gttttagggt tagttatata ttgaaggata tgtagaagtt gttgtgtgtt gggtattgtt 116280
gaggataaag gtttgaatga taagtgggaa taaagttata aggttttaaa atttgtttga 116340
ggttttatag ttagggtttt ggaaatttat gaaaaaagtt agtttaatat tttgttatgg 116400
aatataggta ggagagggag tagttttttag ttagtttttta gttagttttt tgttagtttt 116460
tttgtgaatt attttatttt gggttatagt tagggtagtt gttgtttttt tttttgtttg 116520
atagttttttg agggtaggag ttagtttgtt tattattgtt ttttttttat tagggtttgg 116580
tattttgttt ggtatgtagt agaagtttaa gaagggttta ttgtgtttat tttgtaatag 116640
ttaatgtgta tgagtgttta ttatatttga agatagtgat ttatttttgt ttttattgta 116700
tagatgggaa attgaggtat tgagaggttg atgatttttt tatattgttg tgtgggtaag 116760
ggtaaggtta gatgtgagtt ggtatgttgg gttattgtgt tttttgttta tattttgttt 116820
tgggtttggg aaggttattt ttggagattt ttatgatgtt gatattttgt ttagtttttt 116880
tgtgtatttt tttttatgtt ttttagttat ttaaagtatt agttaggtag tattgttatt 116940
ttggttttgt aaatttagtt attgagtgga agagtagtta ggttgaagtg gggtagaggt 117000
agtttttgtt agattttagg gttgggattt ttagttattt ttattttttta aggattagta 117060
gatagagatt tttattgtgt ttagttaggg gttagggaat aggtttttat agtgggggag 117120
tgatggatgg atgtaggtgt ggattgggag gatttgggtt ggtttttttag tgttggagtt 117180
ttagtagttt aatttatttt gttgggtttt tgtggggatt aaaagatatt ttgtatataa 117240
agtattagtt tagggtttgg tatttaggag gtgtttaatt aaaggaaatg gttatgaaaa 117300
ataataagga gaagtttagt tataggaggt gtttgtgggt aaggagagaa tagttggaag 117360
ggtttggatt tgggttagag ataaagaatg taagagggag ggagggagag aaggagggag 117420
ggagagaaag agggagggaa agaaagaggg agggagagag ggagggtagg gtaggtaata 117480
gttggtttta gggtagttgt gtgtaggagt ttgttttatt atttaaatat taggagagat 117540
gtaaatattt gagtgaggtt tttttagttt ggttttgttg tggttatagt tagggttgga 117600
gaaagagttt ttttttttatt agggttttttg ttttgtgttt tttattttag ttgttttaag 117660
atttatgtt tttgggttag gagtaggggt ttgagagtag taattaggta gggtagtggg 117720
gttagggagg gattttgttt ttgtttttat tttttttttt tgtttttgtt tttattttttt 117780
ttttttgta tagtattggg tttgtaaagg tgtgaatgaa ggattttttt ttttatttga 117840
ttttaagggg tttaggtggt taagggtttt ttttgtagtt tagaaaaaaa agtaaagtag 117900
ttttttttga ggttttttgg tggaggttta gggtttaaag tttgttgtta taaatttgta 117960
gtttttttttg tttttttaat agtgtttttt ttttatttgg gttattttat tattttggtt 118020
ttttttagtt ttttaggttt tttggggagg aggatagatg tagagtttgg attttgtgtg 118080
tagtagggtt tttttaatgt tgtggtgtat tttgtttggt tgtgggagtt tgttttatgt 118140
tatgatttgt gttggtatta atagtttttt tttttatttt ttatttgagt gttgagggta 118200
gttgtgttag gtatagatat atagtttttt atatatatta tgagtttgta gaaatggtaa 118260
gagaaggttg agtgtagtgg tttatatttg taattttagt attttgggaa gttaaggtgg 118320
gtggattatt tgaggtaagg agtttgagat tagtttggtt aataggatga aattttgttt 118380
ttattaaaaa tataaaaaat tagttgggtg tggtggtgtg tgtttggagt tttagttatt 118440
taggaggttg aggtaggaga attgtttgaa tttaggaggt ggaggatgta gtgagttgag 118500
attatgttat tgtattgtag tttgggtaat aagagtgaaa ttttgtttta aaaaaaaaga 118560
aagaaagaaa aagaaaaaaa gaaaaggaaa atggtaagag aagttggtta gtattatttg 118620
tttggtaaat ttttattttg gtttttggtt aggaagtttt tttttaggaa gttttttttgt 118680
tttatttttt tttttgagtt tatgtttaga tttttagtgt ttagaataga gttggttttt 118740
ggggtattta tggatggttg ttggataaat aaataagtta attgtttttt ttattttttag 118800
tgatgttttt ggtttggtag ggtatttagt agtaatgttt tttgagtgga ttttaaggag 118860
tatttttttat tgtgtagttt gtattaggga ttttagtttt ttattttaaa ttaaaggaaa 118920
aaatttaatt ttagtaaata tatagagtat tttatttttt gatttttaat gggggtaag 118980
aggtagagga tagaggagaa gattattttt ttaggtttgg tttggagtta aaaagatttg 119040
tatgttttta ttgtagataa tattggtttt tgggggatat agggtagggg tttggtttgt 119100
tttttgtagg gaaattgttt atttatgtgg aaagtggggt taagggtttt tgtttggaat 119160

```
tagttttttg tatatggggt ggggggtggg gatagaagga gattttgttt ggattgtatg  119220
gaaatttatt agattgttgg ttgttgattt ggtaggagta gagttatttt tattttttag  119280
ggttttgttt gggggagggg agaggttagg tggttttgtt taggttatat tttttatggt  119340
taggaatgtt ttttttttat tagaaagaaa gatgagatag gtttattttg aatttttttgg 119400
atataggaag ggggtttatt tgtggtttag gtggtttttt tttttatatt taaggggttt  119460
tataaggagg tgttaggatt tttaggtttt gttttaggga attgttgttt gtaaaggtta  119520
taggtagaga tagttaggaa tgttttttat gttattggtt atgggttgga ggttgtgaaa  119580
ttaggtatag aattagaaag atttgttata ggttgtaaag ggttttagag agttttagtt  119640
ttttagaggg gtagtattgt agaaggggtg agaaggaaaa attagttttt aggaattgtt  119700
tatttagagt ttatataata tttaggtggt atgagataat ggtttatttt ttattttatt  119760
ttttgttgt tttttaagat ttgagttttt gggggtgttt ttagtagata gggggatgtg   119820
gggtaattgt ttttttttaa ttgtatattt ggaggtaatt tatatttaga tgattttttt  119880
gaggttgtga ttagagtttg gggtagggga ggggaagtgg gtggatggga aggtgaggga  119940
gatttttta ggaaagttgt tttaggtgtt tgtaaggttt tattgtttat atttaaggta   120000
taggttttg gttttgagga tataggaaat ttgatgttat gtggggagga gggtattgta   120060
atgtatttaa ggttaggttt tttgattttt tggatattgg ataaatgatg gttttttttag 120120
ttggtttaga gatgttagga gtgatagaat gttaaaggtt gtagggggat atttagaaat  120180
ttttgagaaa gaagatggat aaggaaaaat aaaaaaatat gagaagttta gtagagtttg  120240
agttttgtat ttttggtttt ttttatggtt atgggtaatt ttttggagat ttagttatgg  120300
gagtagtgga ggtaggtatg ggtggggggg ggggtgggtt ttattgttta tggttttggt  120360
tgatattgag gttatttttt tgttttttta agtttttggg tattggtagg atttttaaaa  120420
atgataataa atttgaagtt tttagaattg tttgttggtt tagtttaaat ggagttttgg  120480
ggttttgtag attaaattaa atagtttgag tttagaaatg ttttaggtgt ttgggggtgt  120540
ttaggggtta attggtgggt ttgtttggtt ggagagatga taaatttttt tatttttggg  120600
attgtgtttt agagagtttt ggattttttg gttttataag taatttagag tttttaaatt  120660
tgggaaaaat taagagggtt ttggaaagaa aggtagtgtt tgattatttt tttttagtgg  120720
ttttgttaag ggagagtata ggaatttggt ttttggttgg gtttttggga tgaattaaaa  120780
taaaaagaaa aagttggggg agggggtggt ggaattgttt ggggttttag tttaggttat  120840
atattaagga gattttagag gagtaggtta gttttatttt tggtatgatg atggatgggt  120900
atttggttgg ttggtaaagg gaagagatgg taatagtgag gttttgaatt tagtggttaa  120960
gatttatttt tgagtgtgat ttttttgat gtttgggtat gtaagaaatg ggaggtggag   121020
aaaggaggag gagattttag ttttgttttt ttggtattag gttattaggg aatagtattt  121080
ttttttaat gggtaggtag aggttatagg ttgttaggtt gagaagtttt tggtttttat   121140
agataaatgt atagtttgta tatttgtatt attttgggtg atgtttatta tgtatggtta  121200
ttatgtatgt tttttattat taagatagtt gatgagatta ggtggttatt gtgtattggg  121260
tattgtggtg attgtttgat attgattttt gggggtttag gtttttggtg ggtttgtttt  121320
tttaggtatt tagttttttt tttatttttt gatattttgg gtttagtgta ggggtatggg  121380
ggtgtttagt ggatgggttt atgggttggg taagtagtat gtagttttgg tttattagtt  121440
tttggtttta ttattagata agatgagatt gttttagttg ttagtttagt tttgtattat  121500
atttgattgg gtttttgtag aggtttgttg gatgagaatg tttagggtag taggtattat  121560
tttttgggat gtatattttg ttataattgt tttttaggga aaggttagtt tgtagagttg  121620
aatgtaggtt gttagtgttt ttgttgtttt tatggtttgg ttatttagta agtgtttatt  121680
ttattgttag tgtttggttt tgtagatggg tatatggttt atttatttgt gatattttag  121740
ggtgggggtg ggggtagatg gtggtaggga agattaggat aattttttag taagaggaat  121800
ttgtagttaa tatgatgtta gtttttagaa tgttagttag gtaggaaaag gggggtttta  121860
tggttgtatt gggagtttga gtatttgttg ggtttagagg gatttttagg tgggtgttat  121920
tggtattagt gtttgttgtg ttttttataa ttagtggtga ttatgttgtt tggtttttatt 121980
tatatagtgg tattatatag gtttttgttt gagatttttt atttttagat ttgtatattt  122040
aagtatttaa atttatttgt tttttgtaaa tgtttttttt ttttgtttag atgttatatt  122100
agttgtgata tttattatat ttgtaggagg taggatttgt gtttagtgtt atgggttgta  122160
ttttagagtt ttttagtaat ttttaaagag agttgttttt aattttattt tgtaggtagg  122220
gaaataggtg aagaaattga agaaattgaa gttgtttagt tttttaatgg tagagtgagg  122280
gtggtttggg tatttttata ttgttatatt ttttattttt ttaaagtatt gttttttttgg 122340
atgtaaattt tgtttattag ttttatttaa aatttttttg gatagttata gtttatagaa  122400
agtattttta gtttgtagtt atttagttat tgtaggggtg ttttggtgtt tattttttatt 122460
tgttttttttt tggatattta gtttagagaa tttatagttt tgttgtaagg ttgattatag 122520
tgtatttgtt gtttgggtgt tgtgtttgtt tttttttaaa tgaattaatt tgtagttggg  122580
atagtgggtt tgtttggttt tgggttttga atttaaagag tattaagtta gtgttgttag  122640
```

tggttgtggg atttgtgtaa gaggtttgtt tttatttttt atggtttttg ttttttatta 122700
taaaagttta aattgtagag aggtagtatt tataggatgt tttgagtttt ggttgaaagg 122760
tagggtttat tatgtaagag gtttattggt attaattagg gataggtttg tatttattat 122820
taagtaatgg ggtttttttt tttagagagg aaagaggtat ttgaattttg ttagtgtgtt 122880
agtttttatg ttttggaggt gttgggattt ttagataaga aggtatattt atatggtttt 122940
ttttatttag aagtttgttt tatagtttgt tttgttttta gaatatagat ttgggggttt 123000
tttttgggtg atagtggggg tttgggagaa gtttagatgt tatatagttt tgggatttga 123060
gtatgtgggt tttgggttag attgttttaa taaatattaa ggatttttgg attttttttt 123120
ggtgtgtaaa tttggttata gtaaatattg ttgagtaaag taggttaata gttggtattt 123180
ggaaaggttt ttgttatata aagatttgag ttgtaattgt gtttgtgata atagatttgg 123240
tttgaattta tgaaagtata tgttgtttgt tagttttgtg tttttattaa ttagaaggga 123300
gaaagaggaa agggttattt tttttgttat tttattatgg gtagtggtgg gtgttgtagt 123360
tgtttggagg tttgtttgta tagtggaaaa gtagaaaatg agattttaaa aattttatta 123420
gttaaatata aaatattttt tttgttgtta tgtggtggtt tttatagagt tttaaaattt 123480
tgaagtttgg agatttattt tgaaggggga gtaaaggtgg gatgtgggaa gtttgagttt 123540
atggaagata ttgatatttt ttttattttt gggaattttg agtttgaagg atttggtttg 123600
aatagtggaa ggattgaatt tttgtgtaat ttattggtga tggaatataa agttatggtg 123660
tttttggttt ttaagatggg agggatgggg aaggttttgg gtgaagggga taggtgggga 123720
aggaaaaggt gaagagaaag gtgtttttta gggagggggt agatgtgttt aggatatggg 123780
atattggtag aattttaatt ttgagtatat atataggttt atggatggtt ttttgtttat 123840
ttgtttttta tgggtgtgtt gtgttggttt gggtatttgt tttttttttt atgttaggtt 123900
atttgttgtt tattttgtat attgttatta gttttaagtt ttttagaata ttttgattag 123960
ggtttttgtgg tttttgttat aggattttag gtttagatgt ttgtttgatt ggaaaattgt 124020
ttttagagat gttgtggttt agagtttgtt ttgttttggg gtggtttttg gagtttttgt 124080
tttttagtaa tttatatggt gataggtatt tatatttgtt ggttttagga attttggggt 124140
tattatttta gtgttgtaag gagttggtag tttttttttt attttttttgg tttattttgt 124200
aggaattaag atattaggtg agttgttttg ggaaagagat ttgtgttttt atggtgttgt 124260
ttttgtgttt atttttagtt attttagatg tttttttgtgg attagttgta tttttggtgg 124320
gtatgtttgg ttttttgtag tttggtttat atgattttta taggtaggtt tttttttatg 124380
ttttagtttt ttttttagtt agtttatttt agtttttttt attgttgttg gttgttgttg 124440
gttagaaggt tttttttttt tttgtaattt aatttttatt tttttttaag gtttgttttt 124500
tttatattga tttttttttt gttagttagt ggtgattttt ttttattttg atttggaagt 124560
tgtttgttta tattattttt ggttgggttg aattatattt ttttttttat tttttttaat 124620
tatttttttt ttatgtttgt ttgtttattt aaggttgttt aattttttata tatatatttt 124680
tatatttttt aattatattt taggtttttt gggtattagg gagggttatt tttttatatt 124740
ttaggttagt ttttatgtat atgtgtattt atgtatatat atattttttt atgtttattg 124800
atttgggtta tttgatggtt ttaagattag aatttttagt aaaattattt aggatatata 124860
agggggagat tgataaatat agggaattag tagattgagg ttttattttt tgagattgag 124920
aaattttttt ttagagttaa aggattgtta attgttgtat taggatggtt ttaatgagtt 124980
tattgttttg gattaaatta tagggttata aaattggttt gggagatagt tgtagaagta 125040
gtttagatg gaggtatatg ggggtaaggg tggagttata tttagagatt taaggaaggg 125100
gtattaataa tttttatagt gtaaatggtg atagttgtat aatgaatata attttaaatt 125160
ttggtttttt tttttgttat agggtttttg ttgagatatg agagtttttt tttagaaatt 125220
ttattggggg tttgatattt tttgttagga attgagagta gagattttga ttttgtaggt 125280
aggaggagag ttttgtataa tatagggttt ttttttataa gggtttattg gaaagtggtt 125340
agattggtta agttgtgtta tttggatatt tgggataagg aaggttttttg gaggagtggt 125400
tgtagggagg agttgtaaag aggtaggggt agggaggtta tatttatttg gggattgtag 125460
gaggtttaat ggaatatagt ggatttaggg agattttagt atgagtatgt gtttaaagat 125520
gtttagatga ggaggtgttg tgggggaggg gggtgggaat agtaagtttt tttagttttg 125580
tagatttaga tttttttttt ttttgggaag tagggatgta aagggataga gtttgaaaaa 125640
gggtttttat tattaagttg ataaagtaat tatgatgttt ttagtttata ttttgtatag 125700
tgttagtggt tattggatta taatttttttt ttgtttttttt gttgttttat attttttttta 125760
ggtttagttt tgaagaatag ttattggtat tttgtttggt gtttagttaa agttttggtt 125820
ttttaggagg ttttgttttt tttatttaga tagggttgat tttaggtggg ttgtgtgggt 125880
agttgggttt aggatattta gatgttagtt ttggttttgg ttatggagtg gggaggattt 125940
taggttggtt atttttttagt ttggtttgat atttggatag tttgtttatt ttaatatagt 126000
tttttttttt tttggtttat tagagggttt tgtttaagtt agtttaggag ttttttttgag 126060
gggtgggggt tttaggtttt ggtagaggta ggtttgtttt ttagaagttt tgttttagag 126120

```
tgttggagat tattgtgtga gttttttttt tgtttgttgt atttattttt gtggatgtag 126180
gatgtagagt tttagtattg gtagttaaga ggatgtagga aagtgtatgt atttataggt 126240
ttagagatgt ggatgttaga tttaggtttg gatagagtag ttttgtaagt tttaatatat 126300
tttgtaaatt ttgaagatag gagagagtta aaatattttt tttgtttgta tgtaggtttt 126360
ttttgttttt tatggtttga ttttgggagg tggatgagga tatttttagg tttggatggg 126420
ggttatggga tattttattt tagattttgt tatgatttgg gttgtgtttg tttggtgttt 126480
ttggtttatg ggttgtataa tttggtggtt ttgaaattgg tgtgggggag gggagggttg 126540
tttatttgag taggatgtgg ttgtttattt agttggaggt gaggaatgat ttttttattt 126600
tgttgttggg tttaagtggg gtttgagagt tgttggggag agttaaaggg aggggattga 126660
tggatttttt agagtgaaat tgtgtgtttt ggagagtttt gaggtagttt tgtgagtttt 126720
tgaggaggtt gttgttgttt ggtaaatata aataataata aaaggaagga aatttaggtg 126780
gtagtgtgta gaatgagtat agggtttgt gagggtgtgt agggtttatg tgggtgtttt 126840
taatagggtt ttttatgtag gtggtgtggg tatggggttg ggggtgtgtg gtgtttttag 126900
tgggagggtg tttggattag gggttttgtt tttttttagt gtatttttgt tttttttttag 126960
tgtattttttg ttttgtgtta ggtttatttg tagggttttt tttagtatgt ttgtggttgt 127020
agtagttagt ttagtattta tttttgaagt ttgaaatgat tttatttagt tgtgtgttga 127080
ttgtggggtt tgatatgatg gttggtgggt agtgggttgt gtggagggta gtggtgagga 127140
agtgttttttg gtggggtttg ggtttgtgt gttggttggg gtgttgtgtt tgtgtttttg 127200
tagtgtagag ttagttgttg gaggagtttt taggtttttt ggtttagttg aatgaatggg 127260
ggaggaaggt gggtgttggt ttttttttgt gtgttgtgtt tttgttttgt ttttgttttt 127320
tgggtggatt aggtttttgt atttgtattg gtttttttgt tttgtattgg ttgttttgtt 127380
tgtttgttaa ggttaggttt gggggttgg ggtttgagtg gttgttgggg gatagttttg 127440
ggtatatgat tggagtgttt tttttttttt tgtttggttt gtgtttaggg gatggtgaga 127500
gatgtggttt tagtttttttt ttttgggtgt tttgttgtgg ttagatgtgg gtggatgtgg 127560
aggttgggtt atggtgagga gggtgggtgt ttagagggga agagattttt tttttttttt 127620
gggtgtaggg tttttttgta ggaggttgta tttgggtagt taaataaagt tttttgttat 127680
tgttgttgtg tgttttgtgt ttgtaagggt taaatggtag tgtatgtggt tggtaggtag 127740
gattttggtt tgtgggttta agagttgagg ggtgtagggt gaatatgtga gtttatggt 127800
attagtgaga ggttattaat ttttatttat tgagggtatt tttgtagtta atatttttat 127860
tatgattatt gttttgggggg ttattgttgt atttttttgg gtgagttttg gagaaggtat 127920
tgtttgggag aaggggtata gggtggggat ggaatatggt tgttagagaa gttagggatg 127980
ggtgatgttt tttttttttt ataataaggg tttttttgaga gtaggaagta gattttgtta 128040
ggagtagatg tttttgaagg aaggtgtgaa ggaaggagtt aggtagtgag tgagtagaaa 128100
gggagttgtt ttaagggttt tttttttttt tttgggtttt aatttgaggt ttagtttttt 128160
ttttaggtta gtggtttttta tttttaaatt atattattta taaggtaggt agggttgagg 128220
ggttggggta gatttgggga tttgggttag atatagaatt tgagaagtaa taggttttta 128280
tttttaagaa gggttttttta ggtttgttat ttataagaga gagaggtgtt tttttttattt 128340
gttttggaaa gttagaaatt tatttttgta tgtttttttt gttattttgt tttttagatt 128400
tttggttttt gtttagagtt aggggttatg tgagaattta tagtatggaa taatattttt 128460
atttttgaag tagtaggttg taatgttata attgtatttt ggttttttga atgtgtggtt 128520
ggggagtgtt tgatagttta ttttgggttt gaggttgttt gttgtttatt tgtttttttt 128580
tattttttttt gaaatatttt aaatgtgtag gagtgttgag atttttttagt tattttggag 128640
aggtgggggg taggggggttt tttgtggaaa atgttttagg tttgggtggt tttttaagta 128700
gatgaaatta agtttttttt ttttaagaaa agtaggtatt tttttttttta tgttttggat 128760
attttgaatt atttttaatt aagtgagttt gtggatatgg gaattgtaaa attatagata 128820
ttttagatgt gtgtatattt ttgttggttt gggagtttgg gttttagaaa tgtagttatt 128880
gttgttatta atagtatttt gtatttttta tgttaggtta gggtatgttt tgtttgaatt 128940
ttagtatttg ggtttattgg atagtttagt ttttattggg ttatttgttt atgttgtatt 129000
tggggatttg gggtttagt atttaagaaa tttagtttaa taatatagtt tttttgggaa 129060
gatgttgggt ttggtataat agttatttta agttatagta ttatttttga gtgttttttt 129120
aaaaagtatt tggtgagtgt ggatatattt atgtaattgt tttttttttag ggttggtatg 129180
gagttttatt agattagtat agggttagtt aattgtatat tttttatttt tatgtatagt 129240
gtagtaaata tatttagttt tttggggagt gttgttaggg tgtatgtttt attaaaatgg 129300
gaatttaagg gataatggta ggagagtagt tagaaagtgt gtatgttttt gtgattgtgt 129360
ttagagttta ttggttttag ttttagttat tgttttttta atgtaattat ttagagttat 129420
atgattaggt ttagaaattt ttaggtaagg aggttttttg agttttttta ggaaatttaa 129480
attagggttt ggttatattt ttagttagga agtttatttt tgtatttaat taattgtttt 129540
aattgtatat tatttatttt tttttttttt ttgtttttag aggagatggg aaatagttgg 129600
```

gtattatttt ttaagttata attatttttt ttatatttaa ggataattta gttgtttttt 129660
ggtttttttt tttttagttt aaattatttt tttttagttt tttaattata ttttgagttg 129720
tttttttgatt atagttaagg gttgggttta gtttggggtt ttaggatttt aatgaggttt 129780
tattgttata ggatagtgag gaggtttttt attttttttt tttagggttt tagggtttgg 129840
atatatagtt ttagaagggg tattgggtag agaagatgta tgttggtagt agagaggaaa 129900
ttgagttgaa atttggtaag aataaaagaa ttataaaggt ttgtgtattt agtaaggtgt 129960
atttttttat ggttataaaa gaaggtaaga ttgattgtag gtttggttaa agggattgtt 130020
tgtgttttta gtgtataatg agatttatag tgggttggtg atttttttta aaataaagat 130080
atgtttattt ggaattttaa atgtgatttg attttgttta agggttttg tagaaagaat 130140
ttaggtaagg attttaagat aaggtgggtt ttaaatttaa tgttgtgttt ttttaagaga 130200
tagaaaagaa agtatagaga tatagaaaaa ggttttgtga agatagaggt agagattgga 130260
gtgatgtagt tataggttaa ggaatatttg gggttattag aagttggaag aggtaaagaa 130320
aggttttttt ttggagtttt tagaggaagt tggttttgtt ggtattttga ttttagattt 130380
ttggttttta gaattgtgag aggatatatt ttttttgtta taagttattt agttttgata 130440
gttgttatag tagttaggag aaatttgtgt tttattttta tttgtattat gttttgaggt 130500
taaagtttta tatagtaaag atgtttaagt aattataaat tttgtgggta gtttatttgt 130560
ttagtagatt attgttttaa gggaaggggg atggggatag ggaagagagt tataggaaat 130620
ttttgtatgt ttttttggaa atgggaagga gtaggtttgg agagaaattt tataggttag 130680
gttttgggga aatgaagttt ataaagtgtg ggaatggagt ggggaggaaa tttatgagga 130740
agtagtgttt ggtgtatata tttatattta tgattttttt ttaggtatag tttttatgga 130800
gaggtttagt tggtagggaa gtaaaagagg tttggagagt gaaggaaggt gaggggtttt 130860
tttttagtaa tattgaggtt ttagatggga tattttatta ggtggtaggt tgtgttttta 130920
tagatagaga ttttttaagg gtagggttta gatggtttta tttttgtgtg gtttgatata 130980
aagtaggttt tttagagttg ttgagttttg ggtttgttag tggtagagtt ttttatagtt 131040
ttttaagttt ttatagtttt ttattttagg aaatggtgtt attatttatt tagttgttta 131100
agttaagttt ttgggtatta ttgttgattt tttttttttt tttattttta gtatttaaat 131160
tatggtgtgt ttttattttt agtttttttg tgaattagtt tgaatttttt tttttagta 131220
gtgtgttagt taatggttaa taattggtta tttggggtta gtaaaagtaa taatatttag 131280
ttttttgatt tgtagtgttt gtttattttt gtggtgttaa tatttttgtt ttggttaatt 131340
tttttttttt tttttttttt ttgatagggt tttattttgt tatttggttt ggagtgtagt 131400
ggggtaattt tggtttattg taaattttat tttttaggtt taagtgattt ttttgtttta 131460
gttttttaag tagtttggat tataggtatt tgttattatt tttggttaat ttttgtattt 131520
ttattagaga tgaggattta ttttgttggt taggttggtt ttgaatttag atgatttgtt 131580
tgttttggtt ttttaaagtg ttgggattat aggtatgagt tattgtgttt agttggttaa 131640
ttttaagtta tttgtatgat attattggag gtgagattga gaagagatgt ttagttatta 131700
tgaatatagt gtttttatta tataaatata attttaagaa tataattgta ataggataga 131760
taatagttaa atgttgtaaa aaatttaaga ggtgatgaaa gttgtgtatt tattattttt 131820
tgttttaaat ataattattt ataagtaatt ttttaatgtt ttgtttaatt tttttttat 131880
aatttgtaat ttaatttata ttgtaaaaat ttattatttt tttaatagag taatgtattt 131940
ttattgtgat aaaatatatg taatataaaa tttattattt ttggttagtt attatggttt 132000
atgtttgtaa ttttagtatt ttggaaggtt aaggtgggtg gattatttga ggttaggagt 132060
ttaagattag tttggttaat atggtgaaat tttgttttta ttaaaaatat aaaaattagt 132120
tgggtgtggt ggtaaatgtt tataatttta gttttttggg aggttgaggt atgagaattg 132180
tttgaatttg ggaggtggag gttatagtga gttaagatta tgttagtgta ttttagtttg 132240
ggtgatagag tgagattttg ttttaaaaaa aaaaaaaata aaaataaaaa agataaattt 132300
attattttaa ttaagttttt atgttagtga tattaagtat atttatattg tatatttatt 132360
aatattattt attttgaaa tttttttaa ttttttaaat tgaaatttg tttttattaa 132420
ataataattt tttattttt tttttttag ttgttggtaa ttgttatttt aattttttta 132480
tgaatttgat tttatataag tgaaattata taatatttgt ttttgtgaga taggtttatg 132540
tagtatgagg tttttaaggt ttattattta aggtttatat gtagtgatag aattttgttt 132600
tttttgaagg ttgaatgata ttttattgtg tgtatatata ttttgtatat ttattttttt 132660
tatatttgag taattgagag taaagttgtt ataaatatgg gtgtgtaaat atttgtttaa 132720
gtttttgttt ttattatttt attattatta ttttatttat tattagtatt attattattt 132780
tagatagttt tattttgttg tttgggttgt agtgtagtgg tgtgattttg gtttattgta 132840
atttttgttt tttagattgg agtaattttt ttgttttagt tttttgagta gttgggatta 132900
taggtatttg ttattatgtt tggttaattt ttgttttttt agtagagatg ggttttattt 132960
gtgttggtta gggtagtttt gaattttttgg ttttaagtga tttatttttt ttagtttttt 133020
aaagtgttgg gattatagat gtgagttatt gtgtttagtt attattttag ttttaaatat 133080

tatagtaatt ttttattgt tttatgagat tttttgagat agtggtttta gggattttat 133140
atatatttgt ttgattttgt gtaatgataa agtgtagttt tttttgtaga atggtagagt 133200
tgggagattt ttgatggggt atgagtttaa aggggtagag gggatgttta gaatatatat 133260
ggatttttta tgtgtgtttt gttagttatt aagtagattt gttttagttt tgatatgttt 133320
aggattatag atagtgtatg gtttttattt gggttaagtt tttggaattt tttttttttt 133380
tatattattt gtattgggag tagttattag gttgtggatt attaagtttt ttttaggtta 133440
tttgagtttg tattttttta tttagtttga ttgagttggt gtgtagttat tatattttat 133500
tatatttttt gtttggttgt tttaatgtaa ttataaggtt ttatttaaag aagtatatga 133560
ttaatttgtt ttttagattt tttagtgatt tttttagttt gttttgtgta gaatatttat 133620
agtaaaattt gattatagtg gtttgagttt ttatttttaa ggtaggggat tttttaaata 133680
aagtattagt gttttaaatt tatgattttt taaggaggtt tttggttttt agtatagtaa 133740
atgttttgtt ttatggtttt ttggtttttt atttttattt tatttgagtt ttttgttttg 133800
gtatttggga ttgaattagt ttggagtttt ttagttttgt tgtttgttgg ttttgatgat 133860
attattaggg attgtagtat tgtttggatt ttttgttgtt atattttatt gtaataatat 133920
ttaatttgat ggaattttgg gagtaagtgg taggggtagg gatggtggtg gttaggggtt 133980
gtatatatat aggatagaag gattgaagga ttgggtggaa agtatgattt ttttaagatg 134040
attttttttt agtagaattt tggttgtgtt ttttgaatag tatttgttta tttgtatatt 134100
tggtgagttt tgtgattagt aaatttagat gtttgggagg ttattagtgg ttttgttgag 134160
gtataattta gatatgatag tttatgtttt ttttttgatt tttttttttt tatatagttt 134220
tatgattaga gtgttttttt tatttttgtt tttattttag ttttgtaaga tatagttgtg 134280
aaatggttaa agtttttaat tttttagtat ttgtttgaga ttggagtata aaaatgagat 134340
ggggatatag tattttatgg atattagtga tggaaaaaaa tttttttgatt ttttgggag 134400
ttttgtaaag ttatattagg ggataagaag gagtggttag tttggtattg atttagtttt 134460
aaagtagtta gtgattaaag tatatagttg gtattttttt atttttaggg tattaggagg 134520
tttggttaga atttatgtta gttaggagaa ataggttgtt tttggggttt ttggtgtttt 134580
ggttttttta ttgttttttt gaagaaggta ttttgttttg tggggtttga ggttggaggt 134640
aatttgttat tgatgtagtg tagggatgag ggttgagtta ttagtttgtt ttagttttat 134700
gttagtagga ataatataggga tttaagtaga gaagaaagta tttattttta aagttattat 134760
agtgattgag gtgatgtggt ttttagggag tttttttttt tgttggtttt ttgtggattg 134820
tggttttatg ttttaataat gtttttgttt attttgtaag gtttagtagg tggaaggaaa 134880
gagggtggag gttgggagtg gtggtttggt aatttgggaa gggtagggag taggaatatg 134940
tttttttttt tttgtatttg tttagaaggt tttatagggt ttgttttgtt atttataatt 135000
ttagggagat tttttttttaa tgtttgtttt ataaatgttt atttatttta tatttgaata 135060
tttttagtta tagggagttt agtatttatt tatgttattt attttatttt tggttgttta 135120
aaaaagagga aagagagaga aagaaaagat aggaaagata gagaaagggg gaaagagaga 135180
gagaggaaag aaagaaagag gaaagaataa gataggaagg aaagaaaaag gaagggaatg 135240
gaagggaagg aagagaggga agaaagagag aaagagagag aaagaggtat aaataaggta 135300
ggttttatgg agtagagatt taataaggga gtaaaatata tttgtagtta ggttgtagat 135360
ggatttttat aggagtgtat atttatttta tttttttttgg aatagaaaag tggaaagaag 135420
atgtagtggt gtgggtgggt ggtggttttt tttgggggtt tggaatgtgg ggtattagtt 135480
tttttggttt tgagaaggag attaagatta ttaatattta tagtggagat tgaattatat 135540
aagggttggg gatattgttt tttttgtatt tttaataaag atttgatgtt ttaattatgg 135600
gaatttgtgg aagaagaaat aaaattggtt ttggggtttt ttagagaatt ggttttttat 135660
ttagtggtgg ttgtgaagaa atttttggag ttagttttag tggtttgggt ttgtatagtt 135720
gagattgttt ttggaagttg agtggaagtg gggagttttt aggtatagtt gggttttgtt 135780
ttttatttta gttagtagtt ttaggatttt tgggtgtttt agagtttttt ttagagggta 135840
aatttagagt atgagggagt ggggagatgt ttaatattgg gggattttttg tttgtttttg 135900
tttttagttt tgttgatttg aatagggagg ttatgggtgt ttaggggtag aattgtgggt 135960
tggtaagttt ttagttttgg tttttttttg tttttttttt tttattgtta ttgattattt 136020
ttggttatat gttgatattt ttataggatg atggagaaga gagaagagga attttttttgt 136080
tttgtttagt atattttaaa gaggttttag tgtaaaggta ttgatgttta tagttaaatg 136140
tgtgtggggg ttttttagga gtgggatgtt tttattttgt tttagattgt tatgaattga 136200
tagaaggttt tatgttatta aatatatttt attaataaat tattttaata tttagtaaaa 136260
aaataaataa aaatgtatgt attttgtgtt ttgtagtgtt tttatgttgt attttttttt 136320
gtatttgagt tttggtttgt gtttttggtt tgttgttttg ggaatagaat gttttgtgtg 136380
ggaggtagat aggaagtgtg gggtgtgttg gttgggtttt tttagataat ttggtattgt 136440
tttttttaga taatgaaatt ttttttattt tgagtgtttt tggaagttag ttggtagtta 136500
gattaattta aatgaattat ttgttttttt tgtgaagtga ttttagggat ttaatgggtt 136560

tgttattaat atagtttttt ttgtattttg tttttttttt ttattttttt ttttttgtat 136620
gagttattgg ttatgatgat ttggtttagt taagggattt ggttagtttt ggttaggtta 136680
tgtgagagat agtataaagg attaaggttg ttggaattag gttagaggtt gagggttata 136740
tggatttttt ggagtgtttg taattgggga tttttaaggt tgttttttgg ttttggggat 136800
ttgtttattt ggggatagtt ttaagggaga tttttaaaag tagggagtta aaagggtgaa 136860
ttaatataga aatattgagg gttagggaga atttgaagat gggtttggtt gggtatagtg 136920
gtttatgttt gtaattttag tattttggga ggttaaggtg ggtggattat ttgaggttag 136980
gaatttgaga ttagtttggt taatatggtg aaatattatt tttattaaaa atataaaaat 137040
tagttgggtg tggtggtagg tgtttgtaat tttagttatt taggaggttg aggtaggagg 137100
attgtttgaa tgtaggaggt agaggtggta gtgagttaag attatattat tgtattttag 137160
tttgggtaat agagtgagat tttattttaa aaaaaaaaaa aaaaaaaaaa aaaagatagg 137220
tttaaggttt ggagagttag aagttatttt tttattttg ggtggtttat tttgagattg 137280
tattatgtta gagaaagttg tgggagtgtt tgtttataat ttataagtaa tattgttttt 137340
tttttttatt agtttttggt tttagtaggt ttttgatttg tgtttttttt agggttttgg 137400
gtttttttaa gttggatttt tggttgttgg tgggatgatt atgtgatttg ttattttttt 137460
gtttattgtt ttgggtagtt tttgttattt ggtgtatata gagtggagtt tagtaaggtt 137520
ggggttgttg atttttttgtt agttatattt gttttttttt atgttatttt tttttgtttt 137580
ggttatgttt aggggtttat atttttttat aagttaggtg ttagattttt gtgtttgttt 137640
tattttgttt tttttgtttg gtgggttttt tatatttttt ttggtttgat ttaggtttga 137700
tttgttttta ggttttagtt tagaggttgt ttttttttagg aagttttttt gggattgtta 137760
gatgaaggga ttttttttttg gattttttttt ttatttttat agttttagga atagttttta 137820
atttaggttt tattgagtta gtgtggtatt taggagagtt ataagtgtgt gatgaatgtg 137880
ttgaagtttt tttaaaagtt gaaggagttt tgtgaatata tagtagtatt agtttagatt 137940
gtgaaggtat aggggagtta gtttttttta gttttgtttg tttttggttt tttttttgag 138000
gaggtgggag ttagttagtt gggattagga ggtgggtttt atggagtaga gatttaataa 138060
gggaataaaa taaattagag aatttttttg ttgtaaggaa tttagataag gagtatttag 138120
ttagaggagg aaggaagaga ttggtagtag atttttttttg ttttagtagt tttgtaagtt 138180
agatgggtta atttttttagt atagttgttt tttagtatag agaaattttt tattttttagt 138240
aatagggtgg gaatattttt agatagggtt atggttattt tggttatagg gattatggag 138300
tttgagtttt tgtggtttgt tttatttatg tggttataga ttttgagtat atatttggag 138360
ttatagagtt taaagggtag ggtagggtag ggaatttgtt tgattggtga aggttatttt 138420
attatttgga gtataggttt ttgtttttaa atttatatgg agtttttgat atggggtatt 138480
gattttaagg aggtgaatgt ggatatttaa ggttagtgaa tggttaggtg ttaatatatt 138540
gataggtttt tggttttttt tttaatattg agggtaggaa ataaatgggg tttatatggt 138600
gtatatggtt tttagagtta gtaaggtgtg taagatttgg tagttgagaa aattgattat 138660
ttggttgggt gtggtggttt atgtttgtaa ttttagtatt ttgggaggtt gaggtgggtg 138720
gattatgagg ttaagagatt gagattattt tggttaatat ggtgaaattt tgtttttatt 138780
aaaaatataa aaaattagtt ggatgtggtg gtaggtgttt gtagttttag ttattttgga 138840
ggttgaggtg ggagaatggt gtgaatttgg gaggtggagt ttgtagtgag ttgagatagt 138900
gttattgtat tttagtttgg gtgaaagagt gagattttat ttttaaaaaaa aaaagaaaag 138960
aaaagaaaag aaaattgatt atttgattat gaataagtta gaatatttat ttagaaaaaa 139020
aaaatagttt tgtttaattg gggatttaaa attttaggat ttatgttttt tttttataga 139080
gttatattat tttttaatg tttattttg attttatttg tttgttaagt tttaggtatt 139140
tagaatatag tatgatagta agtttgtga tttttatagg ttttttaat ttattttagg 139200
tatagttata tttttagttt tgagtagtag ggagtgatat gtagatggtt atttagatag 139260
agaaagattt ttaaaaggag ggtaggtttt gttttgtagg tagtatttat tgatatagat 139320
attagtaggt aaattttta ggaagatttt tattaagaaa gaatgaaaga ggtttagtgt 139380
ggtggtttat gtttgtaatt ttagtatttt gggaggttaa ggtgggtgga ttattttagg 139440
taaggagttt gatattagtt tggttaatgt ggtgaaattt tatttttatt aaaaatataa 139500
aatttagttg gatgtggtgg tgggtatttg taattttagt tatttgggag gttgaggtag 139560
gagaattatt tgatttagg aggtagaggt tgtagtgagt taggattata ttattgtatt 139620
ttagtttggg gggtaataga gagagatttt gttttaaaaa aaaaaaaaaa aaaaaaaagg 139680
atgaaagata aataaggttg aaattggtag gttggtatat ttttaataat ttattttagg 139740
ttgggtgtag tggtataatg ttagtatttt gggaggttga ggagggtaga ttgtgaggtt 139800
aggagattga gattattttg gttaatatgg tgaaattttg tttttattaa aaatataaaa 139860
aattagttgg gtgtggtggt gggtgtttgt agttttagtt attttggaag tttaggtagg 139920
agaatggtgt gaattaggga ggtggagttt gtagtgagtg gagattgtat tattttatgt 139980
tagtttgggt gatagagtga gattttgttt taaaaaaaaa aagaaataaa aaataaaaat 140040

aataaaaaaa aaataattta ttttagtagg gagtaggagg taaggtttga tatgtagtgt 140100
ttgttaattt ttgtggtgta aatatattta ttgtggtaaa ttttaagtta taaataggat 140160
attattaaat atagagttgg gaaaagatat gtatattatt ggataaaatg tttaggtagt 140220
tatggtttat ttttttttgg gaaggggggt atgtttagaa tgttttttat tatggagaat 140280
gattgtggta aatagggttt aattggtagg ttgatttaat taattgttta ttgatgtgat 140340
gtggggtagg tttttgaggt tgtttttatg tttagggaag aaggtggtta tgtggttata 140400
gatttggttt tgggtttgtt tttttttttt tttttgaga tagagtttg ttttttttt 140460
ttaggttgga gtgtaatggt gtgatttgg tttattgtaa tttttgtttt ttgggtttaa 140520
gtaatttttt tgttttagtt ttttaagtag ttgggattat aggtgtttat tattatgttt 140580
agttaatttt gtatttgtag tatagatggg gttgttggtt aggttagttt taaatttttg 140640
atttaggtg atttatttat tttggttttt taaagtgtta ggattatagg tatgagttat 140700
tgtgtttatt tgggtttgtt attttgagga tatatttgga gttatgtgtt tatgattttt 140760
tttttatttt aaaattttga ttttatttta ttgtttttag agataggttt tgttttgtta 140820
tttaggttgg agtgtagtgg tgtaattata gtttattgta gttttgaatt tttaggttta 140880
agtgattttt ttattttagt tttttgagta gttggggttg ggtttatagg tgttattata 140940
tttagttata ttttatattt ttttgtagag atagggtttt gtaatttgtt taaattggtt 141000
ttaaattttt ggttttaagt aattttttta tttttagttt tataaagtgt tgggattata 141060
gatgttagtt agtgtattta gtagttatta tgattgattg gtgatgtttg ttttagtttt 141120
aggatgaaag agtagtagta ttttagttaa gtatttgtgg tttttgattg gaagatgttt 141180
tttttgtttt ttaagtatag tgtaaatgtt atttttttgg taagtttttt tttaatttta 141240
tttttttttt ggtatagggt ttgttatagt tttgtttata taaagagagt tattagtgta 141300
tgtggtatag atgttagttg ttttttttaat atttgagttt tttttttttt atagtaattg 141360
aagttttggt tgtgtatagg gaagtttagt tgaagaatat attgtttagt tttttgatg 141420
ataggagtgg ttatataatt gggttttggt tagtgggaag gagttgaagt gtttaatttt 141480
taggttatgt ttttaaaaat aaagagggat atttgttttt tttttttttt tttttttttt 141540
tttttttggt tggagtgtag atatgatggt aggagttgga attattttag tttaagagat 141600
ggaagttgtg tgttgaggat gtattaaagg aatgaattag gtttgggttg ttttgggttt 141660
ttataatgat tgatatttgg attgggatgt gggtttgagt gttgtttagt ttttgatgtt 141720
ttagtttttg ttatatagtt agatggtgtt ttgattaatt taaggtatgg ttattatttt 141780
atttgtttgg tagggtaggg gttttatttt tatatgttga gtttttggta gaatttggtg 141840
taatatatat agttaaagta aaatgaatag gttattttaa ttaaggttta ttttgtgggt 141900
tggttttggt atttggtttt aatttttatt ttattttatt tatttattat ttttgagata 141960
gagttttata tttgttgttt agtttagaga ggagtggtgt gattttggtt tattgtaatt 142020
tttatttttt aggtttaagt gatttttgtg ttttagtagt tgggtagttg ggtttttttt 142080
gagtagttgg gattataggt gtatgttatt atgtttggtt aatttttgta tttttttgtag 142140
agatggggtt ttattatgtt ggttaggttg gttttgaatt tttgatttta agtgatttat 142200
ttgttttggt tttttaaagt gttgggatta taggtatgag ttattgtgtt tagttagttt 142260
tggtatttgg aattggaaaa taatagtttg agttttagag gggataaata taaatatgag 142320
gtagaaggaa tagtaagtaa atatgtgttt agagtaggtt gggttgttta tatttttaaa 142380
atattgttag gtgttgaggt ttgggttttg tgttagattt ttgggtgtag atttttgtggg 142440
gatatttatt gggtagtggg atttttggta agttatatta tttttttgag tttttgttga 142500
ttaagttgtt tattgagaat aatatttaga ttgaaaggat ttataataat atgtgaaaag 142560
tgtttggttt aaaataggag tttggtaatt ggtagttttt attatttata atatatagtt 142620
taatgttttt gttttatagg aatgaaaata ggatttagat aggtaaaatg atttgttgag 142680
ggttgtatag tgttaatgat agagttagga ttagggtttg gggttttgat tttttttgta 142740
gtattttggt tttgtgagaa gtggttttgg gtgaatataa ggagtgagga gttggtgaga 142800
aattttgaaa atgaagttgg gtgtggtggt ggatgtttgt ggttttagtt atttaggagg 142860
ttgaggtagg aggattgttt gagtttggga gttggaggtt gtagtgtgtt atgattgtat 142920
tattgtattt tagtttaggt gatttagtaa gattttattt tagaaaagga aaggaaagga 142980
gaggggaggg gaagggaggg aatttttgat agtgatttta gagataatta ggatgatttg 143040
aaattatttt gaaatgtatt aaaaaataaa aataaaatgg atttatggtt aggtgaagga 143100
gggatagatg gatggtgagg tgataaagta ggtgtagttt ggtgttagtg gtagaattta 143160
ggtgatggtt gaatgtgttt tttgtaaaat gtttttaatt tgattatgtg ttttgaatat 143220
ttttataata gaatggaggg aagagaaagg taagagggag ttattgtagt tgttgtagta 143280
ggagtagatt ttaaaatatt atgtattaga tttttagttt tgtttttgtg gatgtgtttt 143340
tgtttttttt tttgggattt aaggatattt aatgagtgta tgtgttttta agtttttttg 143400
tttttttatt tgtttttata tttggaagat tttataggtg gtgtgggtta gtttttttatt 143460
ttgtttttttg tgtttttagt gtatgataga tattgagtga gtgttagttt tttttttttga 143520

tattttgttt gtttgtttag agaggttttt ttatagttta gttattaggt tgatgttttt 143580
ggatgtttgg ttgatagttt ttttagttgt gttttagtga tttggtaggt gttttgagtt 143640
ataaataagg ttattagtgg atgtttttaa agtttttttt attatggtgt tttttttttt 143700
tttattaata gtttgttatg atagttagtt tgtatgagtt tttttttagg gttgtgtttt 143760
ttgaggttgt ttagtgtttg gtttgttaga gtttattaga tagggtgttt atttttttta 143820
ggtttgagtt tttatttaga gaattattta tagaaggata atggagaaag gtagttgggg 143880
tattaaatgg agtgtgtttg tatgtggggg gggaggggag ggggagggaa agtaggaaga 143940
gaagatataa gaggagaaat aagaataaga ggaaatatag aaggaggagg agtagttttt 144000
tttatggggg tggattttgt gtttagtttt ttattaagga tattatattt attattttgt 144060
ttggttttta tgaatatttt taaggttgtt ttttttttggt ttttattttt ttattaggaa 144120
gtggaggtat agagtgagaa agagttgttt aaggttttta ggtagtttat ggagttattg 144180
ggagttatat ttaatttgtt taattttatg tttttgtttt tagttattat aataaattat 144240
taaggtttta tagttatagg agtttagttt ggagttttta ttgggttttt taaggtaggg 144300
agatggggtt ggaggttatg ttaggtgtat ggtgtttaga gttgtttagg aagttttgtg 144360
gtgtgggttg ggtattaggt agtgtttgta ggatggagaa atgaatgagg ttgagatata 144420
gtgttgtttt ttttaaatgt gatttttttt gagtttttgt gattggttat ttgagagggt 144480
agttttggtt ttttggtgtt tatgtttggg tttagagatg tttttttttag ttaggttttt 144540
ttttgaggat ggaaatttag aattttatta taggaggaag agttgggagt attttatttt 144600
tttttttttt ttgagatgga gttttgtttt gttgtttagg ttggagtgta gtgatgtgat 144660
tttggtttat tgtaagtttt gtttttttggg tttatgttat tttttttgttt tagttttttg 144720
agtagttggg attataggta tttgttatta tgtttggtta atttttttgta tttttagtag 144780
agatggggtt ttattgtgtt agttaggatg gttttgattt tttgatttg tgatttgttt 144840
attttggttt tttaaagtgt tgggattata ggtgtgagtt attgtgtttg gttagggagt 144900
atttttaaga tttttgtgga gggttgttta ggtttaagtt ttttattatg gaaggttttt 144960
tagatgtggt agttatggtg tatgggttga gggtttttat ttattaagaa gtaggagtat 145020
ttgatgtata tttagtttat gatttttatt tatttggggg ttgtgtttgt agttatgtga 145080
tataagggaa tagttatagt agtgggttat aaagagagtg tgggttaatg tgtaggggta 145140
gaattttgat agttttttat gggttggtag tagggagggg tatatttgta taaattttat 145200
atgtatagtt ttttatatat atgtggttga taattattta tatatttaag atgggatttg 145260
gtgtatagtt tattagtaat aggtgttggt aatttttagt tgtttttttg ttgtggtgta 145320
gatattattt aaggtgggat ggagagagtt attgttttta aggttatata ttttaaaggg 145380
agatgttggg ttatggaatg aaaataggag gattagttag agttattggt tttgagttat 145440
ataatgggat atataggttt atttatttta tatttttatt gagtaattat aatgagttaa 145500
gttgtataga ttatttaaat aattggtatg tagaaaagta ttttaatttt tttttggttt 145560
tgatgtgatg ggaagaattt ggatttttgg agttatagag ttggatggaa attttggttt 145620
agttaattat tggttggggg agtttgggta agtgatttaa ggtttttgag tttgtttttt 145680
tgtaaaagag tagtgtttgg gtttgttatg ttaggttagg gtaagatgat tgaagagtaa 145740
gtgtttagta ttggttaggg gttaatgttt ttaaggatgt aaagtgtaat aagtaggtgt 145800
tatgattgta ttgtttttta ttgtttgtgg taaatatata agtaaagatg gttttgtgtt 145860
tgtgttgggg gaggtaggga attggtttta ggaggatgtt ggttttttta gttgtttttt 145920
ttaaagtttt ttgggaggag aaatgattta ttttagttgt tgggagtttt aatttataag 145980
taggtttgtt tatttggtag tggttgtatg taggtaggag gggttttggt agtttttaag 146040
agtgaggggt tgtttagttt ttttttagtt ggaaaggagg ttttaggttg ggtaggagta 146100
tttggggaaa tttagttttt ggagttaggt tatattaggg agatgaggtt gttagaagtt 146160
tttttggtgt ttatagtaaa atataattaa agtgggattt agattttaat tagtttaagg 146220
tttgatttat attagaggga aattagtgtt tgttgtatga gtgagtgttt atgtgtgtgt 146280
aaggtagaga tagattttgg gtaagttttt ttattttttt atgtttgttt ttttatttat 146340
aaaatggggt aatattattt attttatagg gttgttatga aataaatatg tattaatatt 146400
tgtaaagtgt tgagaatagt atttggtata aaataagagt gatagatttg ttaaataagt 146460
aaaaaataag atataagttt aaagtagagt ttggtatatt gtgagtatta agagttgtgt 146520
tttttgattt tttttttttg tggagatttt tttattagaa gtgggaggga ttgttgaggt 146580
tggagttttg ttttagggat ttttgtgggg agttttttag tttagaggga tttgttttat 146640
ggattatatt tgttttgggt ttttgtgttt ttgtgtatgt gtgtatatat gtgtgtatat 146700
atatgtaatg tgtgtgtgtg tatttttatg gatttgtgat aatttgtata tatatgttta 146760
gaatttattt gatatagtgt tttattaaga tgttggagtt ttgatgaatg gattgtgggg 146820
ttgagatatt gtttttatag gaagttgttt tagtagtaag ttttttggtt tttatagata 146880
attaaaaaga atggagtttg tttttatttt ttgttttaaa aatgatgtat agtgtgtggg 146940
gtttgatgtt ggaggttttt ttagtagata tatttttaat aatattatat tttgaaattt 147000

aagttgttgt aaaataagat aagtaaagat ggtattggta gggattttg aggtttttt 147060
tatgtatttt tgaataattt ttatatggtt tttgtttgaa tgtttttagg tatggggaat 147120
ttattatttt gtgaaatagt ttgttttatt gtttttaagt gaatattgtg agaaaaatat 147180
tttttttgt aaagagttaa gttttatttg ttagtatttt ttaggtattt ggtattagtt 147240
ttatattttg ggggaattta aaataaatgt ttttttttta ttagataatt tattaattat 147300
ttggagatag tggttgtagt tttttgtttt atgtagttat tttatttagt ttttttttgg 147360
aattttgat gtgaatataa gttttttgt tatgtaggtt tttttttgg gtttttatt 147420
tatttgtgta tattttggag aggttgtttg ttttgtgggt gagatttatt gtgggttgaa 147480
ttgtgttttt ttaaaagata tatgtaagtt ttgatttttt agtatttatg aatgtgattt 147540
tatatggata tagggttttt gaagatgtaa ttgggttatt tgagggtata ttgaattaga 147600
gtgggtttta aatttaatga taggtgtttt tataagatag aggtaggttg ggtatagtag 147660
tttatgtttt taattttagt gttttgggag gtaaagtttt attgtttgag tgtaggagtt 147720
tgagattagt ttgggttttt gtttttataa aaaaattaaa aaattagtta ggtatggtgg 147780
tgtgtgtttg tgttttagtt atttggaagg ttgaggtggg aggattgttt gagtttggga 147840
ggttgaggtt gtagtgagtt atgattatgt tattgtattt tagtttggag gatagaatga 147900
gaatttgttt ttaaaaataa attaaaaaga taatagagag ggtagtttgg atagagatat 147960
aagaagagat atagggtgaa ggttaggtgg ggggtaggtt gagttgtaag ttgaggaata 148020
ttaggggttg ttggttgttg tagagtttgg agaaaggtat gggatagttt tttttggaat 148080
ttttaggagg aatgaatttt tagatatttt ggtttgggat ttttggtttt agaaatttta 148140
gggaagatat tttggatgtt tttgtttttg tttttgtttt tgtttttgag atggagtttt 148200
gttttgttat ttaggttgga gtgtaatggt gtgattttgg tttattgtaa tttttatttt 148260
taaggtttat gttattttt tgttttagtt ttttgagtag ttgggattat aggtatttat 148320
tattatgttt ggttaatttt ttttttttt ttgtattttt agtagagatg gggttttatt 148380
atgttagtta ggatggtttt gatttttttga ttttgtgatt taattgtttt ggttttttaa 148440
agtgttggga ttataggtat gagttattgt atgtggttga ttttggatgt tttgagttat 148500
tttatttgtg gtttttttgtt atgatagttg taggatagta atatagggtt ttagtagggt 148560
ggtttggtaa gggttttttt tttatggtag tttttatttt gttttatttg tagggttatt 148620
tagatgttta ttttggtttt ggttagtaag tgttagtgta tttttttatg tttagtagta 148680
agggtagtat tgttgttgat taagtatttg ttatgttggg gtttttgtgt ggttttgggg 148740
tttttttttt ttgtttatat ttggtagttt agtttttttt tttgtatttt atttgatgtt 148800
ggggttttgg gtaagtttgg gggatttgtg ttttatttta tagattttt agttgatttt 148860
attagttttt gtatagggtt tgtttgaagg gttgttgggt tgggtgttaa ttagagtttt 148920
tagtagtagt ttttttggtt ttttgatttt ggtttatgtt ttaggggtag ggtttttaag 148980
ggttttgggg ttgtttttga ggttagtttt tttgttgatt gtagtttata tagaggaggg 149040
tttaattttg agtgtggttg tttgtgattt tgagagatgt ttagagttgt agatgtgttt 149100
tttaagtttt ttagagtttg ggataggagt tatttaatgt ttttagtgtt ataggtaaaa 149160
tttgtttttt ttaatataaa ttgtttttag ttttatttaa tgttttttt aatgagtagt 149220
aataaaaata tggtttttttg ttaaaggtgg ggtttatagt tttttagaat ttggtttttt 149280
gttaggttta ggtataattt ataggagtgt ttgtttttga tttgtgtgtg tgtgtgtgtg 149340
tgtgtgtgtg tgtgtgtgtg tgtgttgtgt gtatgtgtat atgtgtttgt gtaagttttt 149400
ttatgtgtag ttttgggaag ggtggagagg tttatggaaa ttttttttgg tatttagtaa 149460
gttagaaaaa aggaaaatga gatttgtgta tatatgtgta agtgtatata tagtatatag 149520
gtatatatat atatatattt agtatatatt atataggttt atatgtgtat atatgtgtag 149580
gtatatatta tataggttta tatgtgtata tatatggggg tatatagtat ataggtatat 149640
ataggtatat atattttttt gtaggaaagt ttttttttgt tttttatttt gtagggtttt 149700
taggttttat gaaataagat tgaattttgg aagtttgata aaaggtttgt ttggagtatt 149760
ttggttattt ttattagttt ttttagttgt ggaaggtatg ttttgtggta tttatttatt 149820
ttttgttttt atttttattt ttgtttttgt tttttttttt ttgttttttt agtttatatg 149880
ttgtttgttt taattttaat ttttgtattt ggatgggaat tatttttttt gtagaagggt 149940
gtaatgtatg tggggatata tatatatata tatatatata tattgtattg gaggagagaa 150000
gagaggtaga gttttttttta gatttagagt tttagtgagt gatgggattt tttttagaga 150060
atatgtgtag aagtttgtgt tattggaaga agatattatt ttgatttata tttttttttt 150120
ttttttttgt ttttttttata gagaaggaaa ggtttagtta aatagtggtt atgtgggttt 150180
tttgtatgtt attggtagtt ttttagttag gattattttt tttttatttt tggaagtata 150240
agaatagaga gaagggtgag tttttgtttt tgggggtttt tttttgtatt tttttggagt 150300
taattttagg gttgtgggtt gtataggaag gagggtttaa taggtttttt gtttaatttt 150360
gttttttttag tgaggagttg atttattttg tttgtaaaag atgttggttg ggatttgatg 150420
ttgggtaatt tgaggagaag agaaggggga taggggttga ggttaggttt ttggttttga 150480

aatagttata atttttgggg tagagtttaa gggtgttagg ttggagattt tatatttggt 150540
tagttggtag ttattatttt ggtttagtta gtggtttagg gtattatagt tatttagatt 150600
aggaatttta tttttttgga ttgtattaat gaggaaggtt gagtttgtag gttttatata 150660
tttatggtaa ttttaaggta gagaggttat tgttaattga gggttatggg taggagtttt 150720
gtgagtttat tagtaagttt ggaagattaa ggtttttggt tatagagagt agttgttatt 150780
tggttaattt tgtagaaaat attatagttt tggataaaat atttgaaaga ataaaataat 150840
aatagtttgg agttagtata gaatatttta agtaggtaga agttggagta gagtttatat 150900
ttgatagagg ggaataaaat tagataagtt tttggttttt ggaggttttt ttgtttagag 150960
gtaggtattg agtaattaaa tttggataaa aagttttaaa agtatttagg gaaaaataat 151020
atgtggttta taggggaata gttagatgaa tgttgatttt ttgatagaaa taatggaggt 151080
taaagatatt ggaattttat attttaaagt tttgaaagaa aaaattgata atttagaatt 151140
ttataattag tgaaattatg gtttaagaat gatagtaggt tgggtatagt ggtttatatt 151200
tataatttta gtattttagg aggttaaagt aggtagatta tttgaggtta ggagtttgag 151260
attagtttgg ttattatggt gaaattttat ttttattaaa aatataaaaa ttagttgggt 151320
atgatggtat atgtttgtag ttttagttat ttgggaggtt gaggtaggag aattgtttga 151380
gtttgggagg agaaggttgg tgagttgaga ttgggtagga gttttgaaat tatattattg 151440
tattgtagtt tgtggggaga tagagtgaga ttttgtttta aaaataaaaa taaaaataaa 151500
aaaagaaaga atgatagtaa aataaagatt tgtagataag tgaaaatgaa agggaattag 151560
ttgtttgtaa atttgtatta taaaaattgt gaagtaagtt ttttaagatt atgggaaatg 151620
agattagatg ggaatttagt tttttaggaa ggaatgaaga gtataggata tagtaaatat 151680
atgggtaaat agaaaagatt ttgttttatt tttaattttt ttaaaatgta tggggttgtt 151740
taaagtaaaa atttttttat tgtattgttt agtttataat gtgtatggat gtagtaaata 151800
tttaataatt attatatata ggatggaaag tggtaaatga atttatagga ttgtttagtt 151860
tttatatttt tttttatga agaaaaataa tgttaatttt aagtagattg tgaaaagtta 151920
agaatgtgta atagaattta taaagtaatt attaaaatag taatataaag agatataatt 151980
aaaaagttaa aataggttgg gtttagtggt ttatgtttgt aattttagta ttttgggagg 152040
ttagggtggg tggattgttt gaggttagga gtttgaggtt agtttggtta gtatggtgaa 152100
atttgttttt tattaaaaat ataaaaatta gttggttgtg gtggtaggtg tttgtaattt 152160
tagtatatta gggaagttga ggtaggagaa ttatttgaat ttaggaggta gaggttgtag 152220
tgagttaaga ttatgttatt gtattttagt ttgggtaata gagtgagatt ttgttttaaa 152280
aaaaaaaaaa aaaaaaaaat ataaattaaa atagaatttt aaaaaatatt taattaatat 152340
aaaagaagtt aagaaagtaa gatgaaagaa ataaatatta aagagtaagg tagtagtttt 152400
aaatttagtt atatttataa ttatgttaaa tagaaatagt ttgaatattt taattaaaag 152460
gtggaggttg ttagaatgaa taaaatagta agatttaatt atatattatt tataaaagat 152520
gttgttttag tataaatata ggttgaaatt ttttgtttgg aaaaagatat attgatgttg 152580
tttgaatgat gatggagtta tgttttgata aatttattat aagtggaaaa taaatattat 152640
tttaaaaatg tatttaatat atttaattta ttgaatattt tagtttagtt tagtttattt 152700
taatatgatt agaatattta ttttagttta tagttgagta aaattaataa atataaagtt 152760
tattttataa taaagtgttg attattttat gtaagttatt gaatattgta ttgaaagtga 152820
gaaatataat ggttttatat atatttaaag tatagttttt attgtatata tattatgttt 152880
gtattatggt aaagttgaaa aattgtaagt taaattatta taagtttttt tataagtttt 152940
tttagttttt ttatgtattt taaatagtat gtttaagaag gttggagagg ttatataaat 153000
attaataaag ttgagtttaa gataaaatta ttatgagata aaggaatata tttattatgg 153060
ttaaatgatt agtttattga gatattatta taattataaa tgtgtatgta tttaattaaa 153120
ggagttttaa aatatatgat ataaaatttg aaagaattaa agggagaaat gggtaatttt 153180
ataattatag gtggagattt tttttttttt tttttgagat ggagttttat ttttgttgtt 153240
taggttggag tataatggtg taattttggt ttattgtaat ttttattttt tgggtttaag 153300
tgattttttt gttttagttt tttgagtagt tgggattata ggagtttgtt attatgttta 153360
gttaattttt gtatttttag tagagataag gttttattat gttgtttagg ttggttttaa 153420
atttttgatt ttaggtgatt tatttgtttt ggttttttga agtgttggga ttataggtgt 153480
gagttattgt gtttggatgt atagttggag attttaatat tattttagtt tatttaggat 153540
gttaaaataa aatgttttag gttgggtaat ttataaatag attgtagtgt ttatagtttt 153600
agaggttggg aagtttaaaa ttagggtgtt tagtgtttgg tgagagttag tttttttgttt 153660
tagagatgtt tttttttgt tgtgttttta tatggtagaa gggattaatt ttatttatga 153720
tgattttgtt tttatgagtt aatatttttt tgatgtttta atttttaata ttattgtatt 153780
agagattaag ttttaatata tgaattttgg gggatataaa taggtaggtt atagtaaata 153840
ttattttttt aatatttgag aaataagtat tagataattt gaagaatatt ggtaattagt 153900
aattgattta attagtagtt ataaagtttt atatttagga agtttagaat atatatatat 153960

```
attttttttt taagtgaaaa tagtgtattt attaagatat atattttggg ttataagttt 154020
taaattttaa aagtatgtaa aatatgtttg tttggttaaa gtaagattaa attagaaatt 154080
aatagtttta agatatttag aggagttttg attatttgta aattaaataa tatattttta 154140
aataatttat gagttaaaga ggaaattata agaaaaattt tataatattt taaatgaaat 154200
gataatgaaa atatagtata ttaaaatatg taggttgaag ttagtgttta gagggaaatt 154260
gtaagtgttt atattagaaa agaagtaaaa aaaaaaaaaa aaaaaaaaaa aaaaagattt 154320
aaggatttgt tttaagaatt tggaaaaatg ggttaggtgt agtgtagttt atgtttgtaa 154380
ttttagtatt ttgggaggtt aaggtaggtg gattatttga gattaggagt ttaagattag 154440
tttggttaat atggtgaaat tttgttttta ttaaaaatat aaaaattagt tgggtatggt 154500
gggtattttt agttatttag aaggttgagg taggagaatg gtttgaattt gggaggtgga 154560
ggttgtagtg agtttagatt atgttattgt attttagttt gggtaataga gtaggatgtt 154620
tttttaaaat aaaataaaat aaaataaaat aaaataaaat aataaaaaaa ataaagaatt 154680
tggaaaaata tgttagttat ttatgtattg ttttttaatt ttgtatatat tttgttaggt 154740
tttgttttgt gatattggat tgaggtttta tgagtttttt tttttggtta aatatagtta 154800
tgttaggttt tatttatagg ggataatatt gtggggagtt gttaggtttt gttgaggtag 154860
tagttttttt ttttggtttt agggtttttt tttttttttga aggtatagtt gttagtggtg 154920
tgtgtttagt ggggtttgtt ttgtattgag ttttttttagt attttagttt tagtttaatg 154980
ggatttttagt aaattttttta gatatttagt ttttgtttat ttatattttt tagtaggttt 155040
tttggttatt tagtaagttg attttgtaga tttatttttt ttatattttt tggtaagaat 155100
ttttttgtta ttagtaggtt gtaggttttt gtggtagtta tatatttttt aataaggttt 155160
gaatgtaaga tgtggtaagg tgtggaggat gttttgagtt tttggttttt tttttattaa 155220
ttttagtggt agtaattgtt tttgtgtgtt tgtatatatg ttttaaagta tttattatag 155280
aaaatttatt attttaatta tttttaggtg tgtagtttgg tggtatgaag tatatttata 155340
ttgttgtgta attattatta ttatttattt tattttttta aattgtaatt ttattttttat 155400
taaatattaa tttttttattt attttttttt tttttagttt ttggtaattt ttattttattt 155460
ttttgttttt ttgaatttga ttattttagg gattttataa aagtgaaatt atatagtatt 155520
tgttttttta tgtttggttt ttgttattta gtatgttttt aaggtttatt tatgttatag 155580
tatgtgttag aatttttttt tttttttttt tttttttttt ttttgaggta gttttgtttt 155640
gttgtttaag ttggagtgta gtggtatggt tttggtttat tgtaattttt attttttttag 155700
tttaagtgat tttttttgttt tagttttttt agtagttggg attataggta tatgtttttta 155760
tgtttggtta atttttgtat ttttagtaga gatagggttt tgttatgttg gttaggttag 155820
ttttaaattt ttgattttaa gtgatttgtt tattttggtt ttttaaagtg ttgggattat 155880
aggtgtgagt tattatattt ggtttttttt tttttttttaa ggtagaataa tattttattg 155940
tatagatgga ttatattttg tttgtttatt tatttgttgg tggatatttg ggttgtttgt 156000
atttttttggt tatgggaaaa gtgttgttat aaataagaat gtagtatatt tgtttaagtt 156060
tttgttgtta gtttttttgga gtatatattt agaagtggaa ttattggatg ttatggttat 156120
tttgagttta atttttttgag taattgttat atttttttttta tagtagttgt attattttat 156180
atttttatta gtagtgtatg gggttttaat ttttttatat ttttgttaat atttgttgtt 156240
atttatgttt tggttaatag ttattttaaa gggtgtgaaa ttatatttta ttgtggtttt 156300
gattttttatt tttttgatga ttagtgatat tgtgtatttt tttttgtgtt ttttggttat 156360
tggtatgttt tttgtggaaa aatgtttgtt tattgggttg tttttttttg ttttgtggtt 156420
tagttgtagg agtttttttat atattttgga tattagtttt ttattggata tatgattttt 156480
aaatattttt ttttattttg tggatttttt ttttatgtta ttggtagtat tttttgatgt 156540
ataaaagttt taagttttgg tgaagtttaa tttattattt attttttttg ttgtattttt 156600
ggtgttatat ttaagaaagt attgttaatt ttaatgttat gaagtttttt ttttatgttt 156660
tttttttaaag tagttgtttt ttatatttgt ttttttttata ttttttagag ttttttttgta 156720
taaggtaaaa taaattttat tttttttattt ttatttataa tatttagtgt tttagttttg 156780
atattagatg tgtggttttt ttttgttgat ttttaagtaa ttttttttaata tttagtagtt 156840
tttaattggg tgttttgtaa tttagtttaa ttttgatatt aattagagtt agtgtagatt 156900
ttataggtta agggttttat tttataagat tgtttttaat tttagagatt agttgtaagt 156960
ggtaagatat taggttattt ataattttttg tttgatttgg ttataaattt gaggttttta 157020
taattttttt tttaggttta ataatttgtt tgagtgattt atagaattta ggaaattagt 157080
ttatttaaaa ttgttgattt attataaatg atattttaaa ggatataaat gaatagttaa 157140
ttggaagaga tgtataagaa aggtatgaag gaagtggtat ggagttttta tgggagggta 157200
tattgttttt ttagtatttg taggtattta ataatttgaa attttataaa ttttattttt 157260
ttgggttttt agggaggttt tattatataa gtatgatatg aaatttttgg ttattgatga 157320
ttaatttaat ttttattttt ttttgttttg taggaggtgg atgggaaagg ttgtaagttt 157380
tagtttttta attataaggt tgggtttttt ggtaattagt ttttattttt aggttgttta 157440
```

ggagttttta gttattagtt gtttttttag taaataaaaa gatatttatt attttggaga 157500
ttttaagagt tttaggagtt atatgttaaa taaagttggg ggatagagat tagatagata 157560
gatagataga tagatagata gatagataga tagatagata gatagatata ttgatttttg 157620
agatagagtt tttttttgtt atttaggttg gagtgtagta gtgtaatttt ggtttattat 157680
aatgtttgtt tttttgggtt taaattattt ttttatttta attttttaag tagttggaat 157740
tgtaggtata tattgttatg ttaggataat tttttgtatt tttggtagat atgaggtttt 157800
attatgttgt ttaggttggt tttaaatttt tgagtttaag ggatttgttt gttttagttt 157860
tttaaagtgt tgggattata aatatgagtt attgtattga gtttagttta aatatatttt 157920
ttttatatta taatgttatt tttttgtgat ttttttaata gttaattatt ttttatagtt 157980
aagaatattt tatattataa gttgttattt aaattgttgg tgtggttttt gtttttttgat 158040
tgggttttga ttaatgtgtt gaagtaagta taaggaagga agtaatgaag ataatagaga 158100
aatttaagga aatggatatt agaaaagggg agtttaatgt tggtttttta aaaagattta 158160
gattgataat tttttagtta ggttgttgat gagagggaga tggtgaatat aaagttataa 158220
taaaaggaat gaagtgtttt ttttatggat tttattgata ttatagtgat aaggaatata 158280
ggtatatttt attttattgt gttttataga tattgtattt tttataaatt gaaggtttgt 158340
ggtagttttg tattaagtaa gtttgttggt attatttttt taataatatg ttaatattgt 158400
gtttttgtgt aggagtttga tgatttttga aatagtttaa atttttttat tattattata 158460
tttgttatgg tgatttgtga tttttttttt tttttttttt tttttgtttt tttttttttg 158520
agatagagtt ttgttttgtt tggagtgtag tggtgtgatt ttggtttatt gtaagttttg 158580
ttttttgggt ttatgttatt tttttgtttt agttttttga gtagttggga ttataggtat 158640
ttattattat gtttggttat ttttttgta tttttagtag agatggggtt ttattgtgtt 158700
agttaggatg gttttaattt tttgatttta tgatttgttt gttttggttt tttaaagtgt 158760
tgggattata ggtgtgagtt attgtgtttg gttttttttt tttttttttg agatggagtt 158820
ttgttttgtt gtttaggttg gagtgtagtg gagtgtagtg gtgtgatttt ggtttattgt 158880
aatttttatt ttttaggttt aagtgatttt tttgttttag ttttttagt agttgggatt 158940
ataggtattt gttattatgt ttggttaatt ttggtatttt tagtagagat gaggttttat 159000
tatgttggtt aggttggttt taaatttttg attttaggtg atttatttgt tttggttttt 159060
taaagtgttg ggattatagg tatgggttat tgtgtttagt gattagtgat ttttaatgtt 159120
attattgtag ttgtttttgg ggtatgatga gttatattta gataagatag tgaatttatt 159180
taataaatgt tgtatgtttt gattgtttta ttgattggtt gtttttttgtt tttttttttt 159240
ttgtttaggt atttttattt tttgagattt aataatattg aaattaggtt aattaataat 159300
tttataatgg tttttaagtg tttaagtgaa aggaagagtt atttgttttt tattttaaat 159360
taaaagttag aaatgattaa gttgagtgag gaaggtgtgt tgaaagttaa gataggtaaa 159420
agtgagattt tttatgttaa atagttagtt aagttgtgaa tgttaagaaa aagttattga 159480
aagaaattaa aagtgtgatt ttagtgtata tatgaagaag aagaaagtaa aatagtttta 159540
atgttgatat gaagaaagtt tgagtggttt ggatagaaga ttaaattagt tataatattt 159600
ttttaaataa aagtttaatt tagggtaaag ttttaatttt ttttaatggt atgaaggtta 159660
agagaagtga ggaagttgga gaaggaaagt ttgaggttag tagaggttag gttatgaagt 159720
ttaaggaaag aagttatttt tataatgtaa aagtgtaagg tgaagtagta ggtgttgatg 159780
gagaagtggt agtaagttat ttagaagatt tagtttagag tattgatgaa ggtggttata 159840
ttaagtaata gaattttagt atagatgttt tattttttat tggaagaaga tgttatttag 159900
gattttttata gttagagggg agaagttaat gtttgttttt aaagttttaa aggataggtt 159960
gatttttatg agaggttaat gtagttgttg attgtaagtt aaagttaata tttattgatt 160020
gttttaaaaa ttttagggtt tttaagaatt ttgttaaatt tattttgttt atgttttaga 160080
aatggaataa taggttttat ttgatagtat atttgtttat agtatggttt attgaatatt 160140
aaaaatttat tgaggtttgg tgtggtaatt tatgtttgta attttagtat ttttgaaggt 160200
tggggtgggg ggattatttg aggttaggag tttgagatta gtttggttaa tatggtgaaa 160260
ttgtattttt attaaaaata taaaaattat ttaggtatgg tgttatatgt ttgtaatttt 160320
agttatttgg gaggttgagg taggagaatt gtttgagttt aggaggtgga ggttgtagtg 160380
agttgagatt gtgttattgt attttagttt gggtaataga gggagatttt gttttaattt 160440
gaaaaaaaat aaaaaaataa aaaagggtta agtatggtgg tttatagttg taattttagt 160500
attttgggag gttaaggtag gtaaattatt tgagtttagg agtttaagat tagtttggtt 160560
aatatggtgg tgaaatttta tttttattaa aaatataaaa attagttagg tatggtagtg 160620
tatatttgta gttttagtta tttaggaggt tgaggtagga gaattatttg aatttgggag 160680
gtggaggttg tggtgagttg agatagtgtt gttgtatttt agtttaggta ataaagtgag 160740
attttgtttt aattaaaaaa taataataaa aaaaaaataa atagggtttg gtgtagtggt 160800
ttatgtttgt agttttaatg ttttgagagg ttgaggtagg tggattattt gaggttagga 160860
gtttgagatt agtttggtta atatggtgaa attttgtttt tataaaaaat ataaaaatta 160920

gttaagtatg gtggtgtgtg tttgtaattt taattattta ggaggttgag gtataagaat 160980
tatttgaatt tgggaggtag aggttataat gagttgagat tatgttattg tattttattt 161040
tgggtgatat agtgagattt tattaaaaaa aaaaaaaaat tattgagatt tattgtttag 161100
gaaaaaagaa tttttttaaa atattattgt ttattgataa tgtattttgt gatttaagag 161160
ttttgatgga gatgtatgta aagatgaatg ttgtttttat gtttgttaat gtaatattta 161220
ttttgtagtt tatggagaag ggataaattt gatttttaag ttttattatt taagaaatat 161280
attttataaa gtagtatttg ttatagttag tgattttttt aatggatttg ggtaaagtaa 161340
attgaaaatt ttttggaaag gatttttat tttagatgtt attaagaata tttgtgattt 161400
ataagaagag gttgaaatat taatattaat aggagtttgg aagaagttga ttttaatttt 161460
tatggatgag tggtttaaga ttttagtaga ggaagttatt gtagatgtgg tggaaatagt 161520
agagaattag aattagaagt ggagtttgga gatgggattg gattgttgta attttatgat 161580
taaatttgaa tagatgagga gttgtttttt atggatgagt agataaagtg gtttttggag 161640
atgggattta ttttaggtga agatgttgtg aatattgttg aaatgataat aaaagattta 161700
gaatattata ttaatttatt tgattaattt gtggttaggg ttgaaagaat tgattttaat 161760
tttgaaggaa gttttattgt gggtaagatg ttattaaata taatgtgtgt tatagaaaaa 161820
tttttatga aagaaagagt taatttatgt taaaaatttt agttaagaaa ttgttggtta 161880
ggtgtagtgg tttatgtttg taattttagt attttgggag gttaaggtag gtggattatt 161940
tgaggttagg agtttgagat tagtttggtt aataaggtga aattttgttt ttattaaaaa 162000
tataaaaatt agttaggtgt ggtggtaggt atttgtagtt ttagttattt gggaggttga 162060
ggtaggagaa ttgtttgaat ttgggaggta gaggttgtag tgagttgaaa ttgtgttatt 162120
gtattttagt ttgggtaata gagtaagatt ttgttttaaa aaaaaagaaa aagaaaaaag 162180
aaataaagaa attgttgtta tagttatttt taatttttag taattattat ttggttagta 162240
gttattaata ttgaggtaga tttttttatta agaaaaagat tatggtttgt tgaaggttta 162300
gatgattatt agtatttttt agtaaaaaga tataatgatt ttatatattt taatagatta 162360
tagtatagtg taaatataat tttttttttt tttttttgag atggagtttt gttttgttat 162420
ttagtttgga gtatagtggt gtgattttag tttattgtaa tttttatttt ttaggtttga 162480
gtaatttttt tattttagtt ttttaagtag ttgggattat aggtatgtgt tattatattt 162540
agttaatttt tgtattttta atagagatga ggttttatta tgttggttag gttgttttta 162600
aattttaat tttaaatgat ttgtttattt tggtttttta aagggttagg attataggta 162660
tgagttatta tgtttggttt aaatataatt tttatatgta ttgggaaatt aaaaagtttg 162720
tgtgatgtgt tttattgtaa tatttgtttt attgttgagg tttggaagta aattttaggt 162780
atgtttgtat tatgaataat tttaaatgag taaatttaat aatttatttg aaagggataa 162840
atttttttaa agtataattt attaaaagta agataagggg aagtaaaaaa atttgaataa 162900
ttttttttat atattaaatt gaatttgtga ttaaaaataa ataattattt tttttagtag 162960
aaaattttag gtttagatgg tttttttggt ggattttatt aaatatttaa ggaagaaata 163020
atattaattt tatgaaaata tagaaaataa agagaaggga atatttttaa attaatttta 163080
tgaagttaaa atgaggttga taaagatatt attgaaataa attaataaat aaagaaaata 163140
ttatattaat atttttatg aattaatatg taaaaatttt taaaaattgg gtaaattaaa 163200
tttagtaata tataaaaagg atgatatttt ataagtaagt tggatttttt ttagaaatgt 163260
aaggttggtt taatattaaa aaattaggtt tgtatagtgg tttatatttg taattttagt 163320
attttgggag gtagaggtgg gagtattgtt taagtttaag aattgtagat tagtttgagt 163380
aatataatga gattttattt gtataaaaaa ttttaaaaat taaaaattag tttggtatgg 163440
tggtatgtgt ttatagtttt tgttatttag gaggttgagg tgggaggatt gtttaagttt 163500
aggagtttag gttgtagtga gttatgatgg tattattgtg ttatagtttg ggtaagagtg 163560
agattttgtt tttttaaaaa aaaaaaaaaa aaaaatgggg ggtaaaaatt aattattgta 163620
atttatttta ttaaatgaat aaaagataaa ataatgatta tttttataga tgtagaaaaa 163680
gtatttatta aaattatatt tatttatgtt aaaaaatttt agtaaattaa gaatagaagg 163740
gaattttttt aatttgataa aggttatata tgataaattt aaagttaata ttataagttt 163800
ggtataggta aggatgttta ttaattatta ttatttaata ttgaattggt agttttaatt 163860
agtgtaataa agtaagaaaa aaaaataagg tattaagatt agaaagaagt aaaattatgt 163920
atttaaattt taaaagattt ataaaattat tattagagta ataaatgatt ttggaaaaga 163980
ttataggata taagatttat atataaaaat ttattgtatt tttatatatt agtagtaaat 164040
aattaaaaat gaaatttaaa atttatttta gtttaaaata gtgttaaaag taaaattttt 164100
aggaataaat ttaataaatg gtatgtaaga tttttttttt gaaaattata aaataatgta 164160
attttgtaga gatttttttt tagtagagaa ataaaagaaa tataaaagga tttttaaaat 164220
ggagatatat tatttatata aaagatttaa tattgttaaa atgttaattt tttttaattt 164280
gatttataga tttaatataa ttttaattaa aatttatatt gtatttttgt agatattaat 164340
aagttatttt ttaaatttat aaggaaattt aaaggattta gaatatttaa agtaatattt 164400

```
tagatatttg agagatttta tattaagatt tattgtaaag ttatattaat taagatgtgg 164460
tattagtaaa ggaatggata aaatattaag aaaatataat agtgttttaa tatagggttt 164520
atatgtatat agtatatata ttaatatagg attatatata tagttaatat aggatttata 164580
tgtatatagt atatatatta atataggatt atatatatag ttaatatagg atttatatgt 164640
atatatgtat atatattaat ataggattat atatatagtt aatatggggt ttatatgtat 164700
atatgtatat atattaatat aggattatat atatatatat agttaatata gggtttatat 164760
gtatatatgt atatatatta atataggatt atatatatag ttaatatagg gtttatatgt 164820
atatatgtat atatattaat ataggattat atatatagtt aatatagggt ttatatgtat 164880
atatgtatat atattaatat aggattatat atatagttaa tatagggttt atatgtatat 164940
atgtatatat attaatatag gattatatat atagttaata tggggtttat atgtatatat 165000
gtatatatat taatatagga ttatatatat agttaatata gggtttatat gtatatatgt 165060
atatatatta atataggatt atatatatag ttaatatagg gtttatatgt atatatgtat 165120
atatattaat ataagattat atatatagtt aatatggggt ttatatgtat atatgtatat 165180
atattaatat aggattatat atatagttaa tatggggttt atatgtatat atgtatatat 165240
attaatatag gattatatat atagttaata tggggtttat atgtatatat gtatatatat 165300
taatatagga ttatatatat agttaatatg ggtttatat gtatatatgt atatatatta 165360
atataggatt atatatatag ttaatatagg gtttatatgt ataaatgtat atatattaat 165420
ataggattat atatatagtt aatatagggt ttatatgtat atatgtatat atattaatat 165480
aggattatat atatagttaa tatagggttg atatgtatat attaatatag gattatatat 165540
atagttaata tagggttaat atgtatatat gtatatatat taatatagga ttatatatat 165600
agttaatata gggtttatat gtatatatgt atatatatta atatagggtt atatatatag 165660
ttaatgattt ttagtaaagg tgttaggtaa tttaatagag taaggtatgt ttttttaata 165720
aatggtgtta gaattggata tgagtataga aaaataagaa ttaattttga ttttaaatta 165780
aatataaaaa ttgatttaaa atggattata taatattttg atggttataa taggttaaaa 165840
aatgtaaaaa tttaaaaaat taaaaaataa aaggaattat agaatatata attataaaat 165900
ttttagaaaa taagagaatt ttttttgatt ttagtgtagg taaagatttt ttttataggt 165960
tataaaaatt ataaattgaa attggaaaaa gttgataaat tggattttat gaaaataaaa 166020
tatttttgtt tattaaataa tattattaag aaaattaaaa ggaaaggagt aatgtggaaa 166080
aaaaaattta tgatatatat atttgataaa ggatttatat ttagaatata taaaaaatat 166140
atgaaataat attttttttt tttttttttt ttgagataga gttttgtttt gttatttagg 166200
ttggagtgta gtggtgtaat tttggtttat tgtaagtttt gtttttttagg tttatattat 166260
ttttttgttt tggtttttttg attatttggg attataggtg tttgttatta tgtttggtta 166320
attttttgta tttttagtag agatggggtt ttattgtgtt agttaggatg gttttgattt 166380
tttgattttg tgatttgttt gttttggttt tttaaagtgt tgggattata ggtgtgagtt 166440
attgtgtttg gttaattttt tttttaaatg agatttatgg ggaaatagtt aaatgaagaa 166500
ataataaagt agtaataaaa taaaatttaa gttttatttt attgtgtagt tgtaggataa 166560
agagtagaag tttgtagttg gggaataagg tgtttatttt tttaagtatt ataaaatgaa 166620
ggttttagaa attatttgat tttgaaataa tataatttta aaaagaagta agatgtggat 166680
agataatttt ataaaggaaa atatgtaaat gattaataag gattttggaa aattgtgtga 166740
ttttataagt tattaagaaa atataaaata aaagttttaa aaaaaaatga agtttggaat 166800
aattgaataa atatataaaa tgataataaa atgaattttg atttttttatt ttatattata 166860
tgtaaaagtt aatttataaa agttatataa ttttaagttt tttaaaggaa aatataaata 166920
tttttgtaat ttggtagggt aggtaaaggt tttttataaa gaatatagag agagaattat 166980
aaagaaagga gtttgttaaa ttagatttta taaaaattaa agtgatatat ttttgtttat 167040
tggaaaatat tattaagaaa atgaataggt tgggtatggt ggtttatgtt tgtaaaattg 167100
gtttttgggg aggtagaggt aggaggatag tttgagttta ggagttttag atttgtttgg 167160
gtaatatagt gagattttgt ttttgaaaaa ggaaagaaaa tgaataggta agttatagat 167220
tggaataaaa tatttgtaaa atatgtattt gataaaagat tagtatttag aagaaaaagt 167280
taagtaattt aattttaaaa tgagtaatag atttgaaaag atattttata aaggaaaaat 167340
atatgaagag ttaattatga aaatgtgttt aattgtatta gttattaggt taaagtaaat 167400
taaagttata atgagatgat attattatat gtttattaga atgattaaga ttttatagag 167460
tgatagtatt aaatgttggt taggatgtgg agtaattaga atttgtattt attgttggtg 167520
ggagtgtgaa atggaagggt tattttggga aaagatttgg tagttttttta taaaattaaa 167580
tgtatattta ttttatattt attttaggtg tttttataga ataaatgaat gtttatatgt 167640
atagaaaaat ttgtataaga agaattagtg taataggttt atttataaga gtttttagat 167700
ggaaatagtt aggggttttt taataagaga atgattaaaa atttatagaa tgtttatata 167760
gtggatgtta tttagtaata aaatagaata aattgttggt atttgtagta atatgggtag 167820
ttttaagaaa tatttaaatt ggtaaaatta ttttgtggtg gaaaaaaagt ataattgtgg 167880
```

```
ttgttttggg gaaggtgggg attgtgatgt atttggaggg gtatgaggaa tttattgggg 167940
tgatagtgat gttttatgtt ttggttaggt tgggatatat gtggatattt ttgttaaaat 168000
ttaaagaatg gaatgtttta tatttgtgta ttttagggta tatatatttt attttaaaga 168060
aaaataaaaa taattataga taaatgttgg attttagtta atgaaatgta aattgaggtg 168120
ttgataggat gttgtgtgtt gatttttgta gtttatttta agtgtattaa aattagtgtg 168180
gatgttggtg gttagaggga tttgtggtga agtaaatagt aaaatggttg ttgtggaatt 168240
taggtggtag gtgtatgggt gtttatttta gaatttttta tggttttttt tttgtggttt 168300
tgttttgttt tgtttttgag atagggtttt gttttgttgt ttaggttgga gtgtattggt 168360
ataattttag tttattgtag ttttaatttt ttaggtttaa gtaatttttt tattttaatt 168420
ttttgagtag ttgggatttt aagtttgtat tattatgttt gattaatttt ttgtattttt 168480
tatagagatg ggattttatt atattgtttg ggttggtttt gaatttttgg gtttaagtga 168540
tttttttatt ttaatttttt aaagtgttgg gattatagtt atgagttatt atatttagtt 168600
tttttttatt ttttttatgt ttgaaaagtt ttatggtaaa aagttggaaa aatgtaaaaa 168660
agattgaaat ttattaatta aaaataggaa aaataaaata aaagagatgg taggagatta 168720
tttttttttt ttttaattta gtttttaatt tttttttttt ttgtttagtt aggataggaa 168780
ttttaagggt ttgggttttt tgtgtttgtg tggggaagga ggtattttttg tgtttgtgtg 168840
ggggttggtt atttgttttt tggtgtattt tttgtatttt attttttttt gtttgtagtg 168900
agtagttata gttggtggaa attatattta ggtagttttt gtttataaat ttagtgaaaa 168960
tgtaaatttt gtaggagttg gatttttgag agtttgtggt gttatttgtt ggaggaaagg 169020
ttttttgtat ttgagttttt tttttttttt ttgttgttat ttgttttttt gtttaggttg 169080
ggtgggtttt ttttttgtaa ttttggggta ttggtgtatg ggaaatgttt agtgttttat 169140
aatttttttt tatttatatt ttaatattta gtttttagtg gatattgttt ttgtttgata 169200
aatatattat ataggtatag tttaagatta tttattttag gaggagtata agttttagtg 169260
tttattattt tagttttttt tttttttttt ttgatgtagt gatataggtt taggatattg 169320
aagatttttg attttataaa tatgttgaag ttaattattt tgaagttgta gtttttatta 169380
ttattattat tttttatttt ttgtaaagat aaagttttgt tatgttgttt agtttggttt 169440
ggaatttttg gatttaattg atttttttgt tttggttttt taaagttttg ggattgtagg 169500
tgtgagttat tgtatttagt ttgaagttgt agttttttaa ttattgttaa tagtaatatt 169560
tattttttttg tgagtagtag ggtttgttgg tatggttgat agttttgtat tattgtattg 169620
aatttgttgt ttgggatttt aggtgagttt tgatttttag ttggagggat aagttgtgtg 169680
gggttgggta gggtgaattg tgggagatta agtttagatt gagggttata agttatatga 169740
gtattggggt taggtaggga tagtgaagga gatagtgtaa ttttgaggaa agataatgtt 169800
atagaatgga gaggtttagg tttggggttt tttttttgttg ttggataggt atgtttgtgt 169860
ttttttttta gtttttgtga tggttaagtt atgtgttttt tgagaggtaa gtgaggtttt 169920
tgatagtagg ttttaggttt taattaaatt gtttgttttg tagggtgagt tgtataggtg 169980
tttttatttt tgattttttt gaatttttgt tgtttttaag ttttatttat ttttttttagg 170040
tgaatagaaa tattgtttag gggagaatgg aggaagaatt taggaaaggt tgtttgagag 170100
tttggttgag tttatgttat tgttaagagt agtgttaaaa tttaggagtt ggtgagatta 170160
tggtttaggg tttatattaa tatgaggggg tgttagtttt atgattaatg agtttttgtt 170220
ttattatttt ttgttattta tattttgagg gttatagtta gtatgtgaat tggagttggt 170280
ttttggtatt tttttttttta tattagtttt ataatgtgag gtgggggttt gtttttagag 170340
gtatttggag tagtaggtgt tttaagaggg ggaagttttt ttgaagtttt tttttattta 170400
tggtatagga tgtagagggg atagagtatt aggaatattt agtgattata ggggtttgtt 170460
ttgaaggaga ggaggtagga ggtagggaga gtggttggga ggttagtttt ggagtgttta 170520
tttttttattt tgagggagag gaaggaagta gaaggtgagt atagggagtt ttagttgggg 170580
atggagtggg ggttgtagtt tgattgtgtt gtttggagtg ggggtatgga ttgtagtttt 170640
tatttatttg tttggttagt ttgaagttat tttaagtttg gagattttat ttgttttgtt 170700
tatttttttt agggatggtg tttaaatttt gaagttttgg ttggtgttgt aaatatggga 170760
gggaggtttt gttttatttt agttttaaga gttagttaag aatgttttgg atatttgaat 170820
tttgttttga gttagaggtt gggaagaaat ttgaaggatg gtgggatggt ggatagtagt 170880
ttttaggtat taggtattag ggtttttgtt tggggatggg ggtattagta ggtgttaagt 170940
gtattatatt agggtttatt atgtttttgt ttagagattt agtattttat gaaataggaa 171000
ggaatgaggg atgagtgtgt tttttttttt aatattttag tttttgtttt gaggttgtta 171060
ggtatttaat tttttttagga tttaaattat tttggtttta tttatggtta agtgtattaa 171120
attatagtta ggattattgg gtttagatat taaaagtgtt tttttttaaag ttggtagtta 171180
ttagggtagt atagggtagt gtaaggttgg gtagggttgg ttatttgtta gtatttagtg 171240
gtttttgttg gatgttgatg ggggtagtta ggtaggtggg tttatttagt aattaaagag 171300
attttttttt tttaattttt tttttaaaga gattttgatt tttttttttt agttgtaggg 171360
```

tttgtagtag ttttttttt agtaggtttt tttgtttgtt tttggttatt tttagtagtt 171420
atagggtatt gaggttttat tatagtgaga ataaaattaa gtttttatta gggtttttaa 171480
ggtttggtat gatttggttt tgtttgttgt attggtgtta ttatggtttg tttttgaaaa 171540
tatattttag ttatttttat ttttttagg ttttagtttt gggtttttat atttgttgtt 171600
ttttttgtta ggttgttttt tttttggatt ttggtttggt tgggtttttt tggttattat 171660
agttttagtt tagtgttatt atttagaaag ggtttttatt aatttttttg ttatatatat 171720
agatatgtat ttttagtaat ttgttttttag tttttttata gtttttattt aaaattattt 171780
atggatttgt ttatttgtgg attgtttttt agttttaatt gttaagtaat aagtgattag 171840
tgattgtgtt tttgttgttt tttattatag gttaagtatt taagagaggg gttgtatata 171900
gtaggtttta ataaatattt attagatggg ttaggtatgg tggtttatgt ttgtaatttt 171960
agtattttgg gaggttaagt tgggtggatt atttgaggtt aggagattgg gattagtttg 172020
attaatatgg tgaaatttta tttttattaa aaatataaaa attaattagg tatggtggtg 172080
gatgtttgtt attttagtta tttgggaggt tgaggtagga gaattgtttg aatttgggag 172140
gtggaggttg tagtgaggtg agatggtgtt attgtatttt agtttgggtg atggagtgag 172200
atttgtattt aaataataat aataaaatat ttgttagaag aattaaagta gtttgtttgt 172260
gtttaatagg aatttaagag tataagatga atttaattaa aggatatata tagtaggtat 172320
tttatagttg atatttgttg gtggagggag attaggaaag tggggagagg tagtgtttgg 172380
gtatggggat attttgggta tggggatgtt ttggatatag ggatatattg ggtatgggga 172440
tattttgggt ataggatgtt ttgggaatgg ggatattttg ggtatgggga tattttgggt 172500
atggggatgt tttggatata gggatatttt gggtatgggg aaattttggg tatggggatg 172560
ttttggatat agggatatat taggtatggg gatattttgg gtatggggat gttttggata 172620
tagggatata ttgggtatgg ggaaattttg agtattggga tgttttaggt atggggatgt 172680
tttttgttat ttgaaatttt gtagttttat tatatttgtt tgttttagta aattggttta 172740
aagagtattt ttaagttttt atggtaaagt tttagagtta gtgttttttt gttgattttg 172800
agggtgtgtt gtttatttag tgtttaagtt agagtttttt tatttagaaa aaaaaagagg 172860
agggtggttt tatggatgga ggtatgaggg tgtttatagg tagagggagt ttttgggttg 172920
agatttgtgt tattttttgt tttaatttta aggtgtagtt tggagtttaa attagttttt 172980
aaatgtgggt tgttggttaa gaggaatttt tttatttatg attttttttt atatttagtt 173040
tttattgttt agagtatgtt agttttgttt gttttggttt ttaggatttt tttagggatt 173100
tttaggtttt aagttagggt gggtttgaat taaggtagtt ttagaattta tgttaatgtt 173160
tttgttagtg ggagattttg gggagtattg tgtgggatgt atggggttag gtgggggtat 173220
atagtgagtg gttttagaag ttttggttgt ttggagtggg ggggtgttta gagtaagaga 173280
tgggttagga agttggagtg agtaaggagg ttttagtttt tggtgggagt aggttgggta 173340
aaaataaatt aatttgaggg tagtagttag tgagattatt ataggagggg tggtggggtt 173400
ggaggggttt taggagttta ggagagggag gtattgttag aggtaaggtt ggtagatgag 173460
agttagtgtt ggtaggatta atttgttata gtagttatgg tttatttttt ttttttttt 173520
ttatatatgt ttttggggtt tatggaattt attagtttat tataaaggat attataaagg 173580
atatagggga agagatgtgt agggtaaggt atgggggagg gatgtagagt ttttatgtgt 173640
tttttaggtg ttttattttt taggagtttt tatatgttta tttatttaga agtttttgga 173700
attttatttt tttgagtgtt tatggaggtt ttattatgaa ggtatgattg atttaattat 173760
tggttgttga tgatttgttt aatttttagt ttttttttt tatttggagg ttgtggggtg 173820
gtgttgaatt ttgtgatttt atttggtttt ttttgggaat tattttgaag ttgttagttg 173880
gtttgttaat tatgagtatt taaaaagata ttattttgga gattttaagg attttaggag 173940
ttgtgtgtta ggaaatggag atgaaaatta agtgtatatt ttataatatg atagttagtt 174000
ttttttaatt ttagttttat tttttgttgt gttgttggtt ttagtttttt gtgttttagg 174060
gtttttgaat atattgttgg ttgtttgtag tgattgtttt tgtttgttgg gtttgagttt 174120
ggtttatttt ttttattttt ttgtgtttag tttgaaggtg ttttttttgag gtagtttttt 174180
tgatttggtt ttatgaattg taggttttgt tttgttattg gttttttttg ttatatttgt 174240
tattttttgt gttttatgtt tattgatgtg gttttgtatt tgtgtagttt tatgagggta 174300
aggattgtgt ttggttttgt tttttatggg attttagtgt taagtttata gggagtattt 174360
aatgttgaat gaatgaatga gtgattggta agggaggttt attttgttat gttggggggtt 174420
aaaggttgtt gggtggatta tatgggtgag gttgggtggg gagggaagta gagatgattt 174480
tttggaagaa ggggtaggag gtggggaggt gggtgtgggg tgttgtataa ggatggtaag 174540
tgtttttagg ggtgataagt taatttagag tattaggtag tagaaaaagg ttgatatttt 174600
tatgttttat ttttatttag ggagaggtta ggtttttggg aaagttagtt ttttgttgtt 174660
aggagagttt taagggaagt tttattagtt gttgagttgg ttatagtgga aatttatgtg 174720
gtgggtggga ggggtttgag gtatagggt ttgggtggtg gtttaggat ggagatttgt 174780
gtttagatta tgaggggtgg ttttagggat gattattttt aaataggaag tagataaagg 174840

```
tatagatggt agaagtggag gtagagaatg ggttgtaggg gtttttttag agggagagtt 174900
gagtaagggg ataggttgga aatatttagt ttttatggtt tgaagtatag atagttggtt 174960
ttatatgtag gttgggtagt tatattattt agaataaggg gtatgtttgt agggtaggtt 175020
ttaggatgtt ttgggttggt tttgttatta gtttagaaag atggtttagg ttttttttggt 175080
agggagtatg tttagttagg ttttgggtag gagttgagat tttttggttg gtttgagagg 175140
ggatgattga gtgtaggttt agtttgggat tttttttattt ttttttgtta tttagtggaa 175200
ttagttgggt gtgagaaaag aagagaagtg aattaagagt ttagagtatg aggttggatt 175260
agtttttata ttatatgtaa aaattaattt aatttgtatt aaagatttaa atttaagagt 175320
taaaattata aaatttttag aagaaaatat aggggaaaag tttttttgatg ttgtatttgg 175380
taatgatttt ttggatatgg tgttaaaagt ataggtaata gaagaataaa tagataaagt 175440
ggaatgttat agaagtttaa aatatttgta tattagtttg ggtgtggtga tttatgtttg 175500
taattttggt attttgggag gttgagatgg gtggattatt tgaggttagg agtttgagat 175560
taatttagtt aatatagtga aattttgttt ttattaaaaa tataaaaatt agttgggtgt 175620
ggtggtgggt gtttgtaatt ttaattattt aggaggttga ggtaggaaaa ttgttggaat 175680
tagggaggtg gaggttgtag tgagttaaga ttatgttatt gtattttagt ttgggtaata 175740
gagttagatt ttattttaaa aaaaaaaaaa aaaattgtat attaaagggt attaataatg 175800
tggggagaag gtaatttatg gaatggtagt aaattatagg ttagatatgg gattagtatt 175860
tggatattaa tttatagatt ttttaatatt tagtaataat aattatttaa attaaatata 175920
ggtaaaggat ttggatagat atttttgtaa gaatatgtgt aaatgagtaa taaatatgaa 175980
aagatgttta atattattaa ttattgggga attgtaaatt aaaattataa ggagagattt 176040
tttataatta tgagttttgg gtaggtagga agatgtttgg tggggagttg ttggttttat 176100
gttgttttta gaatttttgt aggggttaag tttgattttg atattttatt aggagtatag 176160
aaatgagtgg aaaaggttaa aggttatttt aaagggttag aggggtttag taaggagttt 176220
gggatatgga attatgttgt gtatttagat atatggggat aataggggga tgaaaggtgg 176280
tttttaaaat ttatatttat tttagagttt atgaatatgg tttatttgga aaaagggttt 176340
ttgtagatgt tatgaagtta aggattttga gatgggatta ttttgggtta tttagtgggt 176400
tttaaatttg agaaagattg tttgtatgag agatagatag ggggagattt agggtataga 176460
aggaggtttt gtgataatgg aggtagaggt tgagtggtgt ggttatgagt taaggaatgt 176520
taagggtttt gggagttgtt agaagttgtt agaggtgaga agtaggtttt tttttggagt 176580
ttttggaaag aattaggttt gttgatgttg tgattttgga tttttggttt ttagaatggt 176640
gagagaagaa atgtttggtg ttttgagtta tttagtttgt ggtatttggt gatggtagtt 176700
ttaggaaatt attataggga ttagagtttt tatggggttg ggtaggaagt gggtgtagag 176760
tgtgttaggt taggtttttt attttggaga tggaaaagta gagttggaga ggttgagatt 176820
ggataggtat tgagtgttta ggggtaagaa tttttagggg atggtagagg ttattgggga 176880
tagtatagtg agggatagga gggaggggtg agggttgagg tattgttttt taggaggttt 176940
tggtttttttg tttattgatg atttaggaat tttgtaagag gagttgggtt ttattttgtg 177000
ttgtattgta ttagatggat tttggtgggg atagatgttt agtgatagtg tggttaagtt 177060
tttttagga ttttgggttt gtatttatga ttatgtatag atggttattt tttttgtttg 177120
tagttttttt ttgtggtttg tttttagttt tttagtttaa tagagttttt tagtggaaag 177180
ttttttttt ggagattttt tttttgttga aatataagtt tttggaataa gataaaattg 177240
tttaatttgt aaaagttata aagttgtttt agaataaaaa aaaagttttg atttttggtt 177300
ggaaggagtg gtttttttatg ttttagtggt tagttattat tttatgattt atgttggttt 177360
tgttgataaa tttttttagta ttttttttagg ggaaggttat agtttttagg gagggtggga 177420
gagtatttag gtttttgttt ataaattaga ttggagttag atattgttga gggagggata 177480
agagggtagg ttttggggtt tttaagtttt ttttggggtt ggtagattat tgtggattta 177540
ttagggtagg attatttttt tttataaatt tttttttgggt agttgtaggg tagtttgagt 177600
tggatatgtt ttatatgtta gtttagtggg gtatgaggta tttatagtta tgtttggaag 177660
ttagtgggtt ggtatttgtt gggtaggttt ttgttatata ggtatagtgt tttttttatg 177720
gttttttttt aggttagagt atgtttgggg attagagtag tttttgtagt ggtttgtagt 177780
ttgataatgt ggtgtgagtt atgttttgta gagaagaaga gtttttttagt gagggaagag 177840
gtgtttataa aatttaggat atgtgtagaa atttgtagga ggtgggggtg ttgtttaggg 177900
gttgtgttgt ttttattttt tatttgtag atgatggaat tgtggtttag agttgttaag 177960
tggtttgttt aaggttattt agtagggata ggaaggaagt tatttttttta gggattgtgt 178020
gggagtttag tgataattaa agattagtgt taaagtttgt ttaggatgag aattttatta 178080
agggaaatag ttttgggatt gttgtgttta tttggaaggt agttagttta taattgagtt 178140
tttaaaaatg tatgtttttt tatatgaaaa tatatttgaa tgggattttg tagaaagtat 178200
tttttattaa ggtatagttt gaggggaggt ttttgtttta tttttggttg tgttgttttt 178260
agttaagggt agtagggatt agagtggttt ttggggtata gtattggttg tttttaaaga 178320
```

gtgttttgtt tagtttatga aaagtttgtg ttttgtgagt ttgggagggt gttattattt 178380
ttagtttttt agggagtatt atttttaaat ttatgttttt ttgtttagtt tttagatttt 178440
ttgttggtta tttatttggg ttttggttag attatttata aagtgaaggt tgaatttgag 178500
gtttttgag gtttttgtta gttaggtggg tggagtgatg ttttttggg gttggtggtt 178560
taggaggtag gggatttatt gtggtttgtt taggttgttt ggtggggtgg ttattttgag 178620
gatggtttta ttgtgtaagt ttgatgaagg tttttttag tgttttttgt tttgtggttt 178680
ttgttgggtg gagagtagga tgttgggttt tatatttatt tgttttagtg ggtttgagtt 178740
tttgagttta aatagataag atgtagtttt attgttattt aagtttttg ttgtttttt 178800
tggtttttt ttgaggtttg tttgttgtgt ttgattgggt gtgtttagtg gtataatata 178860
tatttgtttt attagtttta atgtgataat taagatgtgg ttttgaggtt gggtgttgtg 178920
gtttatgttt gtaattttag tattttggga ggttgaggtg ggtggattat gagattagga 178980
gattgagatt attttggttg atatggtgaa attttatttt tattaaaaat ataaaaaaat 179040
atggtgggtg tttatagttt tagttgtttg ggaggttgag gtaggagaat ggtatgaatt 179100
agggaggtgg agtttgtagt gagttgagat tgtgttattg tattttagtt tgggtaatag 179160
agttagattt tgttttaaag aaaaaaaaaa aaaaagatgt ggttttggga gtagtggtgg 179220
aggggattat atgttttaga gaggagttat tgagatgggt ttttttttt taggtttttt 179280
agggattttt taatatatat atagtggtaa ttaatagatt agggtttaag gtatgtaggg 179340
gttgatattt ttagtgattt tttgttttat ggtttgtagg ttattttaga atatttaaag 179400
tttggagttt ggtttagtta ttttatgttt tttagtgttg ggtttagagg gtggtatgag 179460
tggtagaaat tgttattatt gttaattatt gatgatggtg ttggttatgt gagatggtag 179520
ttatggtatt gtatgtgagt gaagggatta aggaggttgg gtttgggagg gagggtttgg 179580
aaagttgggg tgaattaaag gtaggggagt ttttttttt tttatttgtt tgtttatgtt 179640
ttgatgggtt tgtgtttgtg gtaagggggt tgttagtgtg aatggatata tgtatatata 179700
tgtatatata tgtgtatata tatgttaggt ttttttgagt gttgggagtt tttttttta 179760
taattaattg ttaggaaggt atagaaatgt tattttaggt aggagattga ggtagtttg 179820
aagatagatt ttggttggaa gtaaaaaata aattatatag tggataaatt aattattagt 179880
taggtttaag tgttgttgtt ttagatttga gttagggggg taggtttttt atattttttt 179940
aaggttttgg tgttaatagt tttagtattg atagtggtta tgatatgtaa gaatggtata 180000
ttggaaaatg aaagggaagt agttaggaga gaggagagtt gagtttaaat aaaagttaga 180060
tttaggagag tttagttttt gagatggagt ttattttatg agtttggaat tgttaaaaag 180120
aaaagaaata tttttgtgaa aaatgagtaa aagtgttttt tgtatgttta tttttttagg 180180
tataaataaa gttttgagtt tagggtagtg gggggtggtt gatggggagg gggttgtttg 180240
tgtatttgag atttggtttt tttttatttt tatggatata tttagtagtg gtgggagttt 180300
tggtgttttg tttagaggtg gtttttttt ttttattttt gttgtttttt tttttttagt 180360
ttttttttt ttttttttt tttttaaaaa tatttttgt ttggtttaat tttttaatt 180420
tgttgtaatt atgaatgtat ttgaattatt atatttttat gtgttgatta tatgtttgtg 180480
ttttaaattg aagtagtatt gttttttaa aatgggagga aaaagggagt aaaggagaga 180540
aatttaaaaa taggaggtag gagagagggt ttgaagtggg ttttgaggag tttggtagat 180600
tattgggttt gtttgtgaga gttttagagt tttggttgtt tggtttggtt ggtggttgta 180660
gaggttttga gtgtggtttt tggtagtttt ttttttttat ttttttagt ttttgttgtt 180720
ttgaggtttg ttgttttttt ttaatattta tttggagatg tttaatttag atttttggaa 180780
attaatttat agagttaaat ttttgatgat ttttaaagtt ttttattttt tttgttttgt 180840
gatgttatga ttttttggg tttaagggtt taaaaagttt agtttttgtg tgtttgtttt 180900
gttttttttt gttttgggtt ggttttagtt tttttttatt ttgtatttta atgtggttgg 180960
gtaggaggaa tggttgtgtt ttaggttagt gtgttgagaa aaggttttgg agtatttgtt 181020
taggaggaat tttgagaagt tattttttt tgttttatta gtttgtagtg atagaggtag 181080
ttgttgtttt tgtttaggta gagagggtga atgggatggg gatttttgg aagattttta 181140
ttatatatgt atgagttagt gagtttttag ttgtttttg ggagggagtg agtttttttt 181200
tttgggaatt tggggtttta ttgggggatt agtgttgttt tttattgttt atttggggtt 181260
tttagaggtt gtttatttat ttggggggtt tttattagta tatggttagg gtttatgttg 181320
aggttaaggg tttagtagag taggtggggg tttataatgt gggtttttgg ggtgggaatt 181380
aggggtattt tttttgtttt taagttttat tttaagggtt tatttaaatt tgagatatgg 181440
aggttggttt tgttggttta agtttgttta aagagaatgt gtgtttgtgt taaggttgag 181500
aatttaggta atttttttga aaattttaga ttgttagttt ttgttgagaa agggaggatg 181560
tgtggttgta ttatggaagt ttttttgttt ggttgtatag ggttggtata agatagtttt 181620
gtttttaga taaaatattt tttagtttgt gttgttttt aaggtttta ataaatttat 181680
aatatttgta ttttttatt ttgatatatt ttgtaggttt ttgagtttat aatttttgtt 181740
ttatagagtt aggtgagtta tttgtgatta ggtgaggttt atgtatagtt tttggttaga 181800

tgatagagtt aaaggttttg aagtgttatt ttaggttata gttttgtttg ttttttgttt 181860
gggataggat tttaaatgta attttttttgg tagaaaggat atttttttgg ggggtgggga 181920
ggattatttt tagttttttg ggagatatta agtaattatt tttttttaaa gagtttggag 181980
gatagtttta taggtagtta gatagtgagg gaggtgggga tagtatttta gttgaggtgg 182040
gaggttgggg aggaagggag ggattatagt tatgagagaa ggttgaataa tggttttagg 182100
gatagtatgt tttaattttt agatttgtaa atgggatttt gtttggaata agagtttttg 182160
tatttgtgat ttagttgtag attttgttgt ggtgagagta ttttggatgg tttaggtggt 182220
ttttaaaggt agtttggtg tttatataag atgggggtag agggagattt gatatagata 182280
gggatggtta agtggagatg gggtttggag agatttgtag atgttggtgt tgaaggtaag 182340
ggttatgtag ttataagtta agggatattt atagttatta gaggtttaaa ggggtaggtg 182400
atgaaggtag tggtttttaa tttttttggt atttgggatt ggttttaggg aagataattt 182460
ttttatagat tagggagtgg ggagatggtt ttgagatgat ttaagtgtat tatatttatt 182520
attttatttt attttatttt attgagatag agttttgttt tgttatttag gttggagtat 182580
agtggtatta ttttagttta ttgtaatttt tgttatttgg gtttaagtaa ttttttttgtt 182640
ttagtttttt aagaagttgg gattatagat gtttgttatt gtgtttggtt aatttttgta 182700
tttttagtag aaatggggtt ttattatatt ggttaggttg attttgaatt tttgattttg 182760
tgatttgtta gttttggttt tttaaagtgt tgggattata ggtataagtt attgtgtttg 182820
gtttttattt tttatttttt gagatagagt tttattttgg gattataggt gtgagttatt 182880
gtatttggtt ataagtatgt tgtattgatt gtgtatttta tttttattat tattataata 182940
tataatgaga taattatata atttattata atgtagaatt agttggagtt ttgagtttat 183000
tttttttgtaa ttagatggtt ttatttggtg gtgatgggag atggtgatag attattaggt 183060
attagatttt tataagaagt atgtaattta gatttttttat atgtgttgtt tataatatgg 183120
tttattttttt tataagaatt taatgttatg gttgattaga taggaggtag agtttgggta 183180
gtaatgtgag taatggggag tgattgtaaa tatagatgaa gttttgtttg tttgttagtt 183240
atttattttt tgttgtgtag gttagttttt aataggatat atggggttgg gaatttttgt 183300
tttagagttt ttagagggtt atgtttagtg gatattttga ttttagtttg gtgattttga 183360
ttttggatgt gtggtttgta gagttgtgag aggatggttg ggtgttgttg tgatttagtt 183420
agtttgtggt tatttgttat agtagttgta ggaaattgat gtagtgttat agatggagtt 183480
agggtttagt ttattggagt tgttttttgt tattttattt tttttttaaaa tattgtgtta 183540
agttaattat taataattat tgattttgat gggtaggaat tgtagaggtt ttttggtttg 183600
tttttttaat tttttttttta atttgaaat aatattttttt gttggttttt ttgattataa 183660
atgtaatatt ttttttttgta ttgagtttag aaataagaga aaaagtaaaa agtagaaatt 183720
ttagttgggt aaggtggtat gtgtttgtaa ttttagtatt ttgggaggtt gtggtggaag 183780
gattgtttga agttaggagt ttgaggttgt tgtgagttat aattgtatta ttgtattttg 183840
gttgggtaaa aagagtgaga ttttgtttta aattaaaaaa aaaaaaaaag aagaggaaga 183900
aaatttatta attttatttg agatttttta tatgaatttt tgttgtgggg tagggaagtt 183960
taggaaggtt ttatgaggtt ttgatttagt ttggttggtg gtgggaattt gggaatagtt 184020
ttgagtgagg ggttgggtta tgattgagtt tatttattta gttatttttt gtatgttgtg 184080
tatattatgg gtttgatggt tagggttgga gtatttgttg agtatagttt tgatttttag 184140
ttttatttag tatggtttgg tttgaggttt atttttttttt tgtgtttgaa aatttaaata 184200
ttttggttta ggtgggggat tatttgaaat ggaaaatttg gtagtattga ttggttttga 184260
agaggtagga gttagttatg agttggttgg gaggtgagtg tagaggtggt gaggtaaggt 184320
tagtagaggg tgggggaggt gagggttaat tttttattgt gtatggtgta agtattaagg 184380
gggtatttta ttaggttagt gtagatttag gtttttgtgg tggatttttt gtggtttttt 184440
tttttagagg aatattgaag aggtttttgt ggttttgttt tagatttaga ggtatatttg 184500
tatattaaat gtattgagta attttatagt gagggagttg ggtttggttt aatttatttt 184560
tttttaaatt ggattaaagg ttgttgtttt aggaaggtag ggtttttagg tttatggttt 184620
tgggaaattt tggttgtgtt agtttatgag tttagttttt agttatttta gtttgtttat 184680
attttggagt gggtattgtt atttaaaagt ttttttttaga tagtttttag ggtggtattt 184740
ttgtttttta tttttttatag atttttggtt atttttttgt gatattagta ttattggttt 184800
atagtttttt ttaattgtaa agtggggttt agtggtatag gttgggtttg gtagggtggg 184860
aaggaagagt gaaggttatt ttgtttttaa tagtattggt tgggttttgg aaaggttgga 184920
atttatagat aggaaggaag tgttatattt atttaatttg ttttgtgtta gttagggggt 184980
attttgtaga aagaagttag gtggttgtgg ttgggtgtag tggtttatgt ttgtaatttt 185040
agtattttgg gaggttgagg taggtggatt ataaggttag gggttttaga ttagtttgat 185100
taatatggtg aaattttgtt tttattaaaa atataaaaat tagttgggta tggtggtgta 185160
tgtttgtaat tttagttatt tagaaggttg aggtaggaga attgtttgaa tttgggaggt 185220
ggaggttgta gtgagtttag attatgttat tgtattttag tttgggtgat agtgtgagat 185280

```
tttattttaa aaaaaaaaaa aagaagttag gtggttattt tttagttttt ggtttggttt 185340
aggaattgtt ttttttttaa gatgttatta aatgtttttt ttattaggaa gttttttttg 185400
gttgtttttt ttatttttgt ttttgtttta gaatatggtt tggtttttat ggataatttt 185460
gatattagag tttaatagag attttttttt ttatggaaat ttatatttta aattgaatat 185520
gtgtgtagga taagtttttg tttttatgtg aaataaaagt gaaaggtgtg gattatttgt 185580
tttttttat tttttttat aataatttgg gttttatagg ggtttagtag gagttaggtt 185640
tagtttagtt gttttaggtg tattattttg ggtattagtg tttttgtttt tttttatgaa 185700
gtatagtttt atttttttggt atttagtggg gaggggatat atattaggta ttttttaggg 185760
tggattgtgt tttttatatt aggttaggag gttttggggt tggtttagtt atttagaaaa 185820
gggagtaggt ggtttaggtg atattgatgt gggtagtagg ggtttttggg aaagggtttt 185880
tgtaggattg gttttatttt gttttaggag tatatattgg ggaagggtgt tgtgtggagg 185940
tggagagaat gtatttttgg ttgaaagtat gttttttgat gttttgggtt tggatttgtt 186000
gttatttttt atgttttttt atttaatata tgttttagta aatttttat gttgatgagt 186060
gttaagttat agaggatgta taatttttg ttttaaaaag tgattttttt tgttgggtgt 186120
ggtggtttat atttgtaatt ttaatatttt gggaggttaa agtgggagga ttatttgagg 186180
ttaagagttt aagattagtt tgggtaatat agagaaattt tatttttata aaaaaaaaaa 186240
aaaaaaaaaa aagtttaggt atagtggttt atgtttgtaa ttttagttat ttgggaggtt 186300
gaggtaggag aattatttga atttgggagg tagagattgt agtgagttaa gattatgtta 186360
ttgtatttta gtttgggtaa tagagtaaga ttttatttta aaaaaaaaaa aaattttttt 186420
ttttgaaata gggttttatt tttttttgttt aggttggagg atagtgatgt gattataatt 186480
tattgtagtt ttagtttttt gggtagttgg aattataggg ttgtattatt atgtttagtt 186540
aatttttgt atttttagtt aagatggggt tttgttatgt tgtttaggtt ggtttggagt 186600
atttgagttt aagtgatttt tttatttgg ttttaaaagt attgggatta taggtatggg 186660
ttattatgtt tagtttgaaa atattttttt taagaattag ttagttatgg tggtgtataa 186720
ttgtagtttt agttatttag gaggttaagg taggaggatt ttttgagttt ggtaggttga 186780
ggttgtagtg agttataatt gtgttattgt attttagttt gggggataga gaaagatttt 186840
gtttagaaag aaaaaagaaa aaaaaaagat ttttttattt attttttgtt tatttttaga 186900
tttaattatt gaagtttgtt tttttgaatt aaaaatgaag gagttttgtt ttggattttt 186960
ttttgttgat tttgttattg tatttgttag tagagttagt taagattttt taggttggtt 187020
ttagtttggt ttgtttatag ttttttaagt aagatggatt tttaggttga ggttagttgt 187080
attttttttt ggaatttttt tttatttttt attgtgtttt agattttggt tgtttttaaa 187140
taaatataat ttagttattt ttgtttttgg ggttgttgta gggtttattg gtgtaaattg 187200
tttgggtagg tttgttgttt taggagtttt tttaggttag tgtggaagga aattaagata 187260
atggggagaa gtagtttggt tgggtttaat tttattttta tatgtttgtg tttagttaat 187320
tatttgggtt gtagtttgat aggagttgga gttggtgttt tatttttaga gggggttttt 187380
gattgttttt gtagagtgag ggaggtttgg gggtgtagag gttaagggtt tttgtagagt 187440
ttgttttagg ttttttgagt ttaagttttt ttgtagttaa tggaagattt ttttgagagt 187500
gggtgttttt tattttgttt gtttagtagg tgtttagtaa ttgtttattg gataaataat 187560
gaatttgtgg atttaatgtg aagatgattt aaattagtgt ttttttatag aattttttgt 187620
aatgatgtta atgttttgta tttgttgtag ttaatatggt agttatatgt ggtagtgaat 187680
gtttgtaatg tggaagggtt attaaggtaa agagttttgt atttatgtta taatttaaat 187740
ttaaatagtt ataaatgttt agtgtgtgtt ttgttatata gatttttttt gttatttttt 187800
tttttttttt agagatattg tatttgttat taattttta tttgggaggt aaatggagaa 187860
atgttatttt ttagttaatg taaaagaagg ttaatatttg tattttaagt tagtttttg 187920
ggtaattttt ttaagtattt tttattaagg ttagtttagt aatatttttt gattgtttgt 187980
tatgtattag gaaagtattt tgttgtggtt ttggtttgag gttatggagg atatagaaat 188040
aattaaggtt aagttgatag aaggggtgtg ataagatggt ttaaagttat tgagttagtt 188100
aggaggttta ggagtatgga agttttgttt tttagaggat aagtggtaat ttatttaggg 188160
ggggtttgag tatttagagt taggagtgta gaagtagggg gttttaggtt ggaaagtggt 188220
attagggtta ggttgggtgg tagtatgaat ttatttttga ggttttgggg gtggaaatta 188280
agggttttat gagtaagaag atttggttta ggtttaatgg aaatgattgg gtggagggat 188340
tttattattt ggtttttagt atatagggag ggaagtggta attgtttttt aaatgttggt 188400
tgtggagggg aaggagttat agtagggtag gggtggggag gatggtattt gttggtttaa 188460
ggagggatga tggaggttta attaagatat gattgtgatt ggggtagttt gtttaagggt 188520
tagtatgtag agggtgagtt gatgagattt tggtagtttg ttgagatttg ggggtgttgg 188580
ggaggtagag gttaaggtta attttgagat tttttgtttg taaatttggg tgggtggtaa 188640
ggttgatatt agagaaatag aggaagagtg ggtagttatg ggtgagagag gaagtaatga 188700
attaggggtg tttgtaagag tttaggggta aggtttttgg gtagttaaga tgttggtggt 188760
```

tttttttatg attagttttt tttatgtggt atatttttgt ttgtatggtt gggtatgggg 188820
tatttgtttg tttgatatat tttgagtagt tattttgtgt attgaggtaa tgtggtatgg 188880
tgttttgggg aaatagtgag gaggtattgt ataggtttta ttttttggt atttttttgtt 188940
ttttggggat ggtggtagtt aaataagtga tagttaattg ttaggagagt ttggttttga 189000
tttattaggt ggagttaggt attttgttg gaagaggtat gggaattagt ataggatgaa 189060
gggaggtagt gagtattagt ttaggttttg tttttatagg gttttttttt tatatttatt 189120
tgtgtaggtg tttttgtga gggtagagat atttgtagtg tttgagggag atttttttgtg 189180
ggtggggttg tttatttata gtggttaggt gggaggtgta ttagtgtttt gtggttgtta 189240
taatggatta tagttgtggg gtttgaaaaa atggaagtgg gtttttttt atgttggagg 189300
ttagaggttt aaaattaagg tgttagtaag gttatatttt ttagggaggt tttggggtgg 189360
ggtggggggg gggttttttt ttgttttttt ttagatgttt tttggtttgg ggttgtgtta 189420
ttttattatt tgtttttgtt tttatgtggt tttttttggt gtgtttgtgg ttttttttgtt 189480
tttttaagg atattagttg ttggatttag ggtttatttt aatttagaat gattttattt 189540
taagattttt aattatttat atttgtaaag attttatttt taatttttag gttatatttt 189600
gaggtttttg gtggatatga gtttgtgggg gatatggttt aatttaggat agaagggatg 189660
atgggggtat tggggaaaag gttatatatt ttagaggtat tggaagatgg aagagtagga 189720
tttagtgata tgggatgtta ggtagaggag ttggttttaa ggatttagt tggggtttga 189780
gggttagagg gagggaagtt taggtattat tgagatgaaa tgttttgtgt attgtgggaa 189840
atagggtaaa agtttgttgg atgtttagtt agagaggtgg ttgtttgagg tatagaaaat 189900
gagtgaatat gggagagttt attagaaggt tatttgggag ggtgtggtgg taatatttag 189960
aagggttgag aagtgtattt ttatagttta gttaaattta tttggggaaa tatatgtttt 190020
tggtagtatt agtaatagag aagatttttg gtgatgttat tagtgggtta tgggatgggt 190080
aaatttgtt ttttatata gaggaatatt tgttagttgt taaaataagt gaattaaggt 190140
tttgtggatt tatatggata agtttttatg ttgttatgtt gtgggtatag gtagggtagt 190200
tattgatttt agtgaggtat taaggatgtg atgttgatga tggtatttaa gaggagatag 190260
atttaggttg tttagttata tgatgttgat gattgtatgt aagaggagat taggttggat 190320
gtggtggttt atatttataa ttttagtatt ttgggaggtt aaggtaggtg gattatttga 190380
ggttaggggt atgagattag tttgattaat atggtgaaat tttgtgttta ttaaaaatat 190440
aaaaaaaatt agttagatat ggtggtgggt gtttgtaatt ttggttattg aggaggttga 190500
ggtagaagaa ttgtttgaat ttaggaggta gaggttgtag tgagttgaga tggtgttatt 190560
gtattttagt ttgggtgata gagtgagatt ttattttaaa aaaaaaaaaa aggagattta 190620
ggttttttag atatattatt gtttagtata gatgtgtttg tttggggtta gattttaatt 190680
tagaggggtg ggtattgtag taggagtggg gttaggatgg agaaggttat ttagggttgt 190740
ggttatattt gtaatgtttt atttttaag tgggatggtg agtatatagg tgttttatta 190800
ttttattttt tatatttttg ttttgtagtt ttaagaattt tatttttttt ttaaaaggaa 190860
agaaagtgtg tgagttgatt aaagtgttga ttatggtgtt atttaagttt ttgttagaaa 190920
tgttattttt ggtttagttt taaatataat tttgagaaga gtttttgttt ttttggggag 190980
ggtaagagtt ttagatatag gagattggtt agatgtggat ttatagagaa gttgggttgg 191040
gaggatggga tggtagatag ttgtttgggg agtgttaaat ggggaggttg gagatgttgg 191100
ggtgtttagg aatgaatttt tgtttggatt agggttttag taaaagggag gtagggagaa 191160
agaaagaagt taagaagatg aggaaagggt gttttgggtt agagtttata gaggtttata 191220
gagggaggat tttgtgtgtt agtaggtagt tttatgtaaa tttagattat tagtttgtag 191280
ttttgggatt tagtaaatta tgttttaggt ttttaagttg agttgtgatt gtggttgtta 191340
agtgttgata gtatagaatt agttagtttg agtagaggga agtaattgtt gggggaggtt 191400
gtgtttatgt agtttagggt gttaggtttt taagatgtaa tttttataag aattgggggga 191460
ttttttttat ttattttatt agaagttggt taatgagagg gaaggagatg gagaggtaga 191520
ttagatgtgg agttggagaa aagtttttta gaaatatagt agtggttggg ttgtttagtg 191580
gttagtgttt aatatgattg tgatgggtgg agtttttttt aattatagtg ttgaattatg 191640
tagttgttta ggggttttta gttagagtat tattttttttt tttttatata tagttttatt 191700
ttgtattgta aggggtttta tatgtattta atgtggatat ggtatttttt ttattttttat 191760
tttttgtaaa aagttatttt ttttattgtt taggtttggt ggtgtatagt ttatgggtat 191820
tgtatttatt tttggaaggt agatttagga ggtgtttgtg ttgtttttat agaagtaagt 191880
ttaggattag aagtaggttt tgggtttaga atttgaggt tttaggtgtt agtgtggttt 191940
ggaattgttt agttttatag ttattagtta tatgtagata taagtggaaa tttatttaaa 192000
ataaataaat aattttatgt aagtaaatga gtgaataaag taaatgtaaa tatttatatt 192060
ttgagtgtgt aatagattta ggtggttaag tggttattat ataggatggt ggagtgtaga 192120
tgtatagaat agatttatta ttatagaagg ttttattaga tggagttggt ttagagtatg 192180
gaggtggtgg ttttagatgg gtatatgttt ttgtttttgt aggtgtttttg ttttggtagg 192240

atggttgttt tattttgttt tgaaggtagt tttggtttat aggtgtttta tttgtgtgga 192300
tttgattttg ggtttgtgtt ttttggtaat tttgtttttt ttagaatggt taatttatga 192360
aggtatagtt tttgtttgtt tgttattatg atggtagtta ggggtttggt atttagttgg 192420
tttttagtat atatgtatag gtgattatgt tatttttgtt ttttttagag ttttttgttt 192480
tttaggggag gtaagtttat ttattgttgt gatttattat tttttttaaa tagaaaagtt 192540
attagatggg tatttgtttt agtttggttg ttggaagtga tagttaagaa taatgtaggt 192600
ttttagaaat agaggtatta gtatggttat attggggtta ggtaggggag attagggata 192660
tttagtgggg agaagatgat gtgtagattt agttttgggg ggtaagggtt ggaggtagag 192720
ttttgatttt tttgttttat tttttgtttt aggttgtatt tagtgttggt agagtttttt 192780
taagtttata agttgtaatt tgtgttttgg atggttagtt ttagagtttt gatgggtgta 192840
tttgtttttt ttttttattg gttagtgtgg aaaattttta aatatatgga aaaagtataa 192900
ggagtagtat agtgattatt gttagaggtt tatttatttt tttttagata atattggatg 192960
tatttgtttt attttggagt atttttttatt ttgtaaggtt gtagggagtt tatagatatt 193020
gaattaatat tgagaggttt atggttaaag ttatttgttt atggtatatt tgttttttta 193080
gtattttaaa ttaaattatt tagttttatt tattttttgt ttttttttt gttttgtata 193140
tatgggtttg ttttttgagt ttgggggtgg ttgtttttgt ttttataagg attattttta 193200
gtagggtagt gttgataggt ttggaagtta tttgagttta aagttgttaa agattgtgtt 193260
tttttgggaa atgggtaagt ggttttattt atttagggaa gttaggtggg gaggtaggtt 193320
ttagattaga atgaagggat tttagtataa atatatttat gagttgggtg gggttagagt 193380
ggagtgagtt atttatttgg gttattggag ttattaagtt tttggagttg gttattggag 193440
gtttgtgtgt tttttaaaga tagagataaa gggtaaaggt tatataaggt tattggtgtt 193500
ttttttttgg gtggatgatt aatattagga atgtttttgt ttttgttttt gttgtgagtt 193560
ttagagttag aaatggggtt tatttgggga tagaggtttt attttttttag gtaattttgg 193620
atgttggagg gatgatagtt attgttgttt ggttgttgga ggggttttgg gttaaatttt 193680
tatatatatt ttttttttaag ttttattatt tttgttttgt agataaggaa tttgagtttt 193740
tatagagtgt tattttttttg tggttatata gtgaatgaga gttgtatata ggtgagttta 193800
tgtttattag attttaagtt gggttgtatt taagtgtgtt tttgttggat ggagtttttt 193860
ttttttttta tgtttatttg ttttatgaat tagggttttt ttatattagg gtttataatt 193920
tatgggtagt tttgatttag ttttttttat ttttagggtt gggtgtagtt gggttggatt 193980
tggggatgga gtgggatagg ttatttttat tttttgtttt aggagggaat aaaaatagaa 194040
ttttagatta atgtagtttt ttgtggtatg tgtttttttt gtttatatat aggttttggt 194100
ttggggttgt ttgttggaga ggtttatggt aggtaagttt taatttgagg agatttgttg 194160
aggtgttaag ttaagtaaat ttgaaaggtt gttaggagga gtggttttgg gagtataggt 194220
ggttatttta ttgttgtaga gtatggtatt ttggggatgt tttttgggta atgttatata 194280
gttatttttt agagagtttt agggatatat aattagttta gttttattaa tagtgagata 194340
agggattagg tagatttaga tattttttttg tatttgatta ttatgttggt tgttggttaa 194400
agatgttagt ttttttttgg aatttttttag aaaggttttt gtagtattta tttatttatt 194460
tatttattta tttattttat ttatttattt atttatttat ttatttatgg attttattta 194520
ttttattatt tttttatata tttatttatt tatttattta atttatttat ttagtttttt 194580
atttatttat ttatttatta atttatttat ttgtttattt aatttattta tttattaatt 194640
tatttattta ttatttattt atttatttat ttatttattt attttttttat ttatttattt 194700
attttttttt ttatttattt tataatttta tttttaaatt gtgattagat ggaaatgggt 194760
gtagttagtt tgtttttaaa ggtttagagt ggaggtagag tgaattgttt atttggtttt 194820
tgtgtttagt agtttaggtt tgtatatatg ttttataatt ataggatatt agatttagta 194880
attggtagta gaatgagtat agtgtatttt ttgtggaatt agtattgata gggtatttat 194940
ttttattaag tttgagtttt tttggtgttg gagggaattt ttagatgggt tttaatttta 195000
gttgaattta tttttttaggg aaagtaatat tgtgagaaat gtgtgtgata taaggtttta 195060
aagagggtgt gggttgtgat ttttggagta attagggagg ttttatggag gagatgagga 195120
taaagttgag ttttgaagga gggattggag gatgttgagt agggatattt atttttgttt 195180
gtgagggttt gatggtaggt tgtgagttgt attttagttt tgatttattt aatttataat 195240
tttagtaggt aggtgttttt gttttatttt atagaggagg aagtgaggtt tagggttggg 195300
taaattgttt aaggttgtat agttgtggaa tttattttta gtatttttag tttgttttag 195360
ttatgtttgt gattttattt agtatttttt ttttggtgtt tatttatttt gtttattgtt 195420
aagtaataaa aattttatta ggaaaattta ggggttttttg gttagttttt ttagatagaa 195480
aggtagattt tggtgtagat ttttaggaag taaggatgga agggttagta tttgtttttt 195540
agaaatgata gttttttttaa gattaggaga gatggtttag agtgtagttt tggtgggttg 195600
tttaggatta ggaggggaga ggagtgggga ggaggatgaa ttttattttt aaggttgttg 195660
agtggtatat agtgagtttg gatttttttag ttttagttga gtttattttt atgatggtta 195720

```
tgagtatttt ttttatatat ttgattggaa gttgtattgt atataatgta tatggggtta 195780
ttattttttg ttttatatat ttttatgtat atattattag tgtattattg ggatattttg 195840
ttgtttgggg ttgagagtga attagaattt tgtttataaa tttagtatat tttattgtgg 195900
aaataatggg gggtaaggag gggttgttta gtaaagaata ggatatatgt ttttgaggat 195960
ggagttattt ttggttgaga tatttttgta ggggatgttg gtgataaagg ttgttttatt 196020
tttttttttg ggttagatgg atattatttt tgtttttttgt tttttagtat taggtaaaag 196080
gaaaatagaa gaggttttat ttaggttaaa tattttaggg ttttttattt gtaggaaata 196140
tgttggttta ttttttttaa atttagtatg ggggtggaga aatggtgggg aaaatagtat 196200
ttgttttaga ttttttgggt ggagtttttg ttttatttat attgtttatt tttttgattt 196260
tttaaaatgg gtatttggtg tttggtagtt tttggtatag agaagatgta aagtaaattg 196320
tttataagaa gggggtgagt gtagtttgag tattttttgt ttttgaataa attgttaggt 196380
ttagtgtagt tgttgttgtt attgttgttt ttaggaaggg atttttggtt atgagtggtt 196440
ttagtttagt tttttggatt taattattta ggtttgtgtt tgagtttagt aaagaggtag 196500
ttagaaaagt ttttaagata tttaatgatt ttgtggtttt agttgagaag tgagttattt 196560
ttatgtgatt ttaaaataaa atatatttag aaggagagaa gataattgaa atttagttgt 196620
ttggagttat tttagatgaa agtatttgag tttttgggag gtaaaggagg taggaaagtt 196680
tggagttaga tgattagaat ttgaatgtag attttgttag gtttttgttg tgggttttgg 196740
gagatgattt ttattttttg agttttagtt tttttgggga tagagatggt gtttatttat 196800
aggttgtttt gagttaatga ggttatatgg tgtaagtatt agtatagtgt ttggtataaa 196860
attaaaattt aataagtgat attattatta ttattattat tattattatt tttgagatgg 196920
agttttgttt tgttatttag gttggattgt agtggtatga ttttagttta ttgtaatttt 196980
tgttttttaa gtagttgaga ttataggtat ttgttattat gttttggtta atttttgtat 197040
atttggtaga gatagggttt tattatgttg gttaggttgg ttttgaattg attttaggtg 197100
atttgtttgt tttagttttt taaagtgttt ggattatagg tgttagttat tgttatttgg 197160
ttgtgatatt attataatat taatattata atagttggtt aggtatggtg atttatgttt 197220
gtaattttag taatttggga ggttgatgtg ggttagttat ttgaggttag tttgagatta 197280
gtttgattaa tatagggaaa ttttattttt attaaaaata taaaaattag ttgggtgtgg 197340
tggtatttgt ttgtaatttt agttatttgg gaggttaagg taggagaatt gtttgaattt 197400
aggagatgga ggttgtagtg agttgagatt atattattgt attttagttt gggtgataga 197460
gtaagatttt gttttaaaaa ataataataa taaataaata aaatatattt tgatgagata 197520
tggtaaaaga tagtagaagg taggagtttg gggatttatt atgggttggt gttttaagta 197580
gaaagaaatg tagtgtttat aagttagaga gtagatgttg aagataggag atgagtgggg 197640
tttttttggtt ttggagagga aagggtgagt ttaggtagaa agagtaaatt tagggtgttt 197700
tgtgtagtgt tttttttgtg gaattagttt agggtattag tagtatagtt ttttttttagt 197760
agtggagatt aggttaattg tttagtgttt tttttgtttt tttgttttaa ttttattaag 197820
tatagttttt ttttgggtat agtagttttg taggggtgga tattgattta tgtatttagg 197880
agtttagagg tttttttttt tttatttatt tatttagaga tggagttttt ttttgttgtt 197940
taggttggag tgtagtggta ttaatttggt ttattgtaat ttttattttt taggtttaag 198000
tgattttttt gttttaattt tttgtgtagt tgggattata agtatgtatt attatgtttg 198060
gttaattttt gtatttttag tagagatggg gttttattat gttggttagg ttggttttga 198120
atttttgatt ttgtgatttt tttattttgg ttttttaaag tgttgggatt atagatgtga 198180
gttattatgt ttggttaggg gttttttttta atggattttt tttaatgtat ataatttggg 198240
gaatttagat ataagaaaga tatagagtat gaaggttttt tattggtttg tttattttta 198300
gaggtggggt tttaggtggt ttaggttggt tttaaatttt tggttttaag taattttttt 198360
gttttggttt tatgttggga ttataggtat aagttattat gtttagttat tttttttttt 198420
atttgggttg ttatgttaga atttggtaag ttataagatg gaaatgttta aataggtatt 198480
tagaatttga gtagatatta tttgatgtat tattgttgtt attttaatga ttgaaatgta 198540
tttattatat ttgttttttt aaaggttatt ttagattttt atagatttta tttgtgatgt 198600
aagatagtta ttttaaattt ttttgtgggt ttgaatagat tagattttttg aatttttggg 198660
tagagtaggg atggggttgt tttagaggag ggtgatattg ttatattttt gttttgtttt 198720
tgtgttgata atgaatgagg tttggatagt tttttttttta ttaaaagtta tattttttta 198780
aaaaggagtt ttttaggatg aggtgttatg ataatttaat tgtgtataaa tatttatttt 198840
taataatgat agaagttatt tatagaaaag aatttatttt tggttgggta tggtggttta 198900
ggtttgtaat tttagtattt tgagaggtta aggtaggtaa attatttgag gtttggagtt 198960
tgagattagt ttgattaata tggtgaaatt ttgtttttaa tgaaaatata aaattagttt 199020
gtatgtttgt aattttagtt atttaggaag ttgaggtaga agaagtattt gagtttgaga 199080
ggtggaggtt gtagtgagtt gagattgtgt tattgtattt tagtttgggt gatagagtga 199140
aatttttttt taaaatatat atatatatat atatatatat atatatatat atatatataa 199200
```

atttatttt atttttttt ttgaaatttt tgagagaggt taggtgtagt ggtttatatt 199260
tgtaatttta gtagtttggg aggttgaggt gggaggatgg tttgaggtta ggagttggag 199320
attagtttgg gtaatatagt aagattttat ttttattaaa aaataataaa agtttaaaaa 199380
gtttttgaga tagtttttgg ggaaaggtgg gttatggaga tttttatttt ggttatttgt 199440
gttttttttg tttgtttttt tgtggttttt ttttattttg agtttggggt gggggtggtt 199500
tgagtttagg atgggggtgg tttgagtttg gggtgggagt ggtttgagtt tgggataggg 199560
tggtttgagt tggggtgggg gtgttttaag tttgggttgt gggtattttg agtttggggt 199620
ggggatgttt tgagtttggg gtgggggtgt tttgagtttg ggttgagggt ggtttgagtt 199680
tgtgttgggg gtggtttgag ttgggatggg ggtggtttga gttgggatgg gggtggtttg 199740
agtttggggt gggggtgttt tgagtttggg atgggggtat tttgagtttg ggatgggggt 199800
gttttgagtt gggatggggg tgttttgagt ttgggatggg ggtggtttga gttgggatgg 199860
gggtggtttg agtttggggt gggggtggtt tgagtttggg gtgggggtgt ttaggatgta 199920
taggttttat tttttttttt tgaggtttag tttgtttata ggagttgtga agggttggat 199980
taaagttaaa ggtttaaatt ttagatttat ttttagattt agtggataat taaaatattt 200040
aagtttattt ttaaaggaag ttgagataag gagataatgg ttattttttt tttttttttt 200100
tttttgtttt tggtttttgt tatttggagt tgtgttttga agaggtatat tgggtattag 200160
gtttttttgg ttttgttttt atttttgtt tagatataga ataatttttt tttattttta 200220
aggttgatat gtggggttgt ggattaaagt taatttattt atttatgagg ggagtaattt 200280
ttttgtttta ggaatttgtt tttataggag aataaatatt gtagaaggta ttgtttttag 200340
tggtggtgta tgtttgtaat tttagttatt taggaggttg aggtagaaga attgttagaa 200400
tttaggaggt ggaggttgta gtgagttaat attatgttat tgtattttaa tttgggtgaa 200460
aagattaaga ttttgtttta aaataaaaaa agaatagtat tttgtgttaa ggttaggggt 200520
tttagttatt ttttttgtga agtggaaggg gtattattag tttttgtgag aaggaaatag 200580
tttatttaat tataaaatgt taattaaagg aggtagtata ttggttttgg tgtggtgttg 200640
ttggtttttg tttggggtag gaggagggtt gggggagagt taaaagttaa agaaggttta 200700
tggaggttgt tttttttttg gtgattagtt tttttttga tggtaagttg ttaatagtat 200760
ttggttttta ggttggggaa ggtgagatta gtttatattt gtttaggggt ttgttttgtt 200820
tgtatgggta gtaggtgttt attttgttag atggttttgt ttattatagg gtattagtgt 200880
tagttaagga tttatagagt ttttttgtaa aggtttatta gtgggtattg ggatttttggg 200940
ttttatttat atagggtttt tatataaaat ataggatgtt tagttaaatt tggattttag 201000
ataaataatt aataattttt tgaagagata aggattttgt tgttttattt aagttggagt 201060
ttagtggtat gattatggtt tattgtaatt ttgaattttt ggatttaagt aatttttttaa 201120
ttgtagtttt ttaaagtatt gggattgtag gtatgagtta ttaaggttag taaataatga 201180
attattttta ggtataagta tattttaagt ttgttgtggg atatgtttat attaaaaatt 201240
aatttttgtt tatttgaaat ttaaattgaa tttattgttg ggggagataa tggagtgatg 201300
gattgataga ggagtataga gatagttttt ttaaattttt agttgattaa agtttggttt 201360
ttagtttttt tgggttttta gagatgtatt aagggtggaa gtgattagga gggagataaa 201420
aatattgaag tttgagtgtt agtattgagt tgtgtggttt tggataggat tgtttttttt 201480
ttgagtttta gttttatatg ttataattgg ttaataatag ttataaagta tttgaagtta 201540
atggttggga gtttgtttta ggaggtaggt attattagga tgtagagaga gtgaggagtt 201600
tttaaagtta gtttaggtta ggtatagtag tttatgtttg taattttagt attttgagag 201660
gttgaggtga gagaattatt tgagtttaga agtttaaggt tgtagtgagt tgtaattgta 201720
ttgttgtatt ttaggttgaa tgatagagtt tttgttttta aaataaaata aaataaaata 201780
aaataaaata ataaagttag tttgtgtatt ggattgtatg ttgttttaaa gtatttaaag 201840
tgagagaggt taggaagtaa tgagatgtag aaaggtaggg ggaggaggtt tagaggtatt 201900
agagaagata gttgattttt atttttatat atttttgggg tattttgagt tttatttata 201960
tatttaggtg agaattttttg tatttgtgaa agtaatttga ggttgagaga attaatagga 202020
aattgtttta ttttttatgt agggtttttt tataaataag gttgagttgt agtttattat 202080
tgtagttgag ttttttgtag tagttgtggt ggggaaggtt ttttaggagg tttgagtaat 202140
ttagaaatat ttatagtata ttttttagtt tttttagagg tttttgttga gatgaaagtg 202200
ttttggtttg tggttaaaga gtttagtata ttgggtagtt atagtttttta ttatattgat 202260
ttggttatta ggtttttatg aatttagtga ggggagatgg gtttttttagg gaagtttgtt 202320
gatttttttta tattgttgta aatgtaaagt ttggtgattt tattattttt agtaattgtg 202380
atgtagtaat aataagaata gttatgttta ttgtttattg aaggtatatg atgagttaga 202440
tattgtgttg gtatatgtga tttttggagg taatatattt ttttttaagg aggaaatgat 202500
tgagttttgg agggagggtt ttttatttgg ttttaggttt ttttttgatt tgtgtttttt 202560
tttttttttt tgtttgtttt attgtagttt ttttggtttt tttgttgttt ttggaatgta 202620
ttaagtttgt ttttagttta gtttttgtat tgttggtttt tttttgtttt tttaatttta 202680

atttagttgt tattattttt gtgatttttt attatttatt ttaaagtagt tattagttat 202740
tttttatgta attattttat tttaattttt attatagttt ttagttttat tttgatagtt 202800
tttagaattg atgaatttat tgattttgtt tttttttatt gtatgtaaat tttaagaaat 202860
aagagttttt gtttgtttat tgttgtgttt tagtgtttgt tatagtattt ggtatgtagt 202920
aggtgtttaa tgaatatatt tttaaaagaa tgaatgattg gaaggagttt gaaagaatga 202980
tggtttgtgt taatagttta tttttaattt ttttagatgg gatggagatt attattatgt 203040
aattatgatt tttttgagtt tgtgtggtgg ttgatggtag gtgttagttt ggttggatta 203100
agggatggtt ggtaatgtat tgtttttggg tgtgtttgtg agggtgtttt tggaggagat 203160
gggtatgtga gttggtgggt tgagtggtaa agattttttt ttaatatgga tgatattatt 203220
ttattgttag ataataaaaa ggtagagaaa aggtgaattt tttttttttt ttggaggtgg 203280
gatgtttttt ttttttgttt ttggatttag aatttttagt tttttagtag tttttttaggt 203340
ttttaaattt ttggttttaa attaagagtt atattaatag ttttttagtt ttgaggtttg 203400
tagatttgga ttgagttatg atattggttt ttttgggttt ttagtttgtg gatggttttt 203460
tgtggtatat tttggttttt ataatggtat gagttaattt ttttaataaa ttttttttta 203520
tttatttatt atttattttt ttaattgttt atttgtttat ttatttattt atttattatt 203580
tattttttat ttatttattt attatttatt taattaatgt atttatgttt gtttgtttat 203640
tggttattta tatttattta ttaattattt atttatttat tatttatttg ttatttattt 203700
agttatttat ttatttattt atttattttt tttttttttt tgagatggag ttttattttg 203760
ttgtttaggt tggagtgtag tggtgtgatt ttagtttatt gtaattttta tttttttaggt 203820
ttaagtgatt tttgtgtttt agttttttga gtggttggga ttatagatat gtattattat 203880
gtttagttaa tttttttatt tttagtagag atggggtttt attttgttag ttaggttggt 203940
tttaaatttt tggttttaag tgatttattg gttttagttt attaatgtgt tggttattat 204000
aggtaagagt tattgaattt aatttattat ttgtttgttt gtttgtttgt ttgtttgtgt 204060
ttatttgttt gtttggttat ttattattta tttatttatt tgtttgtttg tttgtttgtt 204120
tgtttgtgtt tgtttgtttt tattttttttg tttgtttgtt tatttagtta tttgtttgtt 204180
tgtttgtttg tgtttgtttg tttttttttt tttttgtttg tttgtttgtt tatttagtta 204240
tttatttatt tatttatttt ttattggttg tttttttgga gagttttggt tgatgtagat 204300
ggtgttttgg taagtttagg ttgttattta gattattgtg gagggagtga tttaaataat 204360
agatatgtat tttttagttt gagaggttta aagttttgga ttaaggtgtt gttagggtta 204420
gtttttggtg aggttttttt ttttggtttg tatttatttt tttgttgtgt tttttgttat 204480
tttttttttg tttataagtg agggggggagg agagattttt ggtgtttttt ttttttttta 204540
taaggatatt aattttatta gattagggtt ttgttttatg attttattta attttaatta 204600
tttttttaaa agttttattt ttaattatag ttgtattggg agttagggtt ttaatgtagg 204660
aatttaaggg gaagggtata gtttggttta taataaatgg gaaattatgt ttatgattaa 204720
atttttttaga agatatggtt tattagtgtt gttaatgatt gtaagtaaag gttagagagg 204780
aaaggggttt ttgtggggtg gggggtggtg ggggtagttg gtgttgggaa taaggttttt 204840
tttttttgttt aatagtaggg aagtgaggtt ggttgtagat gttatttggg gtgggaagag 204900
aggggagttt ttgtttgtat attttgtgtg ggttttggtt tgattttttt ttgtgttttt 204960
tattttttgt gttttttttt gtgtgggaga aatgttgatt tttgttggtt taggtgttta 205020
ggtttttagg tttgttggtt ttgggttggg tttaattaat gagaggtttg ggaggaattt 205080
gggaggtgga agtaagggag ggtgtgggag tttttttattt tttttgtttt agatagtttt 205140
tttagtagga gaggtggttt tttgtgtagt tttagttttt gttagagttt tatttttgtt 205200
tgggttttta gtaggtgatt ttggtttttg gattttggtt ataggatttt ttttttttta 205260
tttttattta ggggtggtgg tggtattttg ttgttaattt ttaggttatt tttataatgt 205320
taagaatttt ttttttattt gtatggtttt tttttaaggt attgagaagg gttttaatta 205380
tatgtttata gggttaaata tttttttgtaa aatttataaa atggaaaata ttttttttat 205440
aattaattaa tgtttttgtt tatttttagt tttgagtttt tgtttgttat attttttttt 205500
gtgtgaggtg gaaatggagg tttgttgtta tgtgggggtt tttgttaagg agaagttgaa 205560
ttggaaagat atttagtttg gttttggttg ggttatattt atgtagtttt tatttatttt 205620
tatgtatagt ttagttattg tttgttattt tggtgtggaa atggatttta gtaatatttg 205680
ttttatttat tgttttaatt ttatttattg tgtagaatta aaaattataa gatatgaata 205740
tattttgtgg tgtttggtgt tggaaaaatg tgtgtagtgg agagaagtaa gatttgaaat 205800
gtatgggttt agaagtaaat ttgtggaaaa ttatttttat tattagaggt gaagttgtaa 205860
gtaaaggatt tagttttttt aatgtggagt taaaatagaa aagttttttt ggttagaaat 205920
atatgtataa atgttttata tgtagttaga aatttttttt atttttttat ttttttttttg 205980
atgggaatta tatttaaaaa aaaagaaaaa aataggattt attagaattt ttgtgtattt 206040
tgaataaaat aattgtagta gtattattaa ttttatgttg attgttattt ttgtatatat 206100
tttaattatt aaaaataaaa ataaaatttg attaattaag gagatttatt tgttttattt 206160

atttatttat ttttgagatg gagttttatt ttgttgttta ggttggagtg taatggtatg 206220
attttggttt attgtaattt ttgtttttg ggtttaagtt attttttgt tttagtttt 206280
taagtagttg ggattatagg tgtgtgttat tatatttggt taattttta tgtttttggt 206340
agagatgggg ttttattatg ttgattaggt tggttttaaa tttttgattt taagtgattt 206400
gtttatttta attttttata gtgttggaat tataggtgtg agttattatg tttagtttat 206460
gttttttttt gttgtttttt ttttttttt gagatatggt tttattttt tatttaggtt 206520
ggagtgtagt ggtatgatta tggtttattg tagttttgat tttttgggtt taagtaattt 206580
ttttatttta gtttttaag taattgggat tataggtgta tgttattatg ttttgttagt 206640
ttttgtattt ttagtagaga tggggttttg ttatgttgtt tggattggtt ttgaattttt 206700
ggtttaagt gatttatttg ttttggtttt ttaaagtgtt gggattatag gtatgagtta 206760
ttgtgtttag tttgattagt tttttgtttt tgaaatagaa aatgatatta taaagttatt 206820
atattgaaga agtaatgtta aaaatgtggg agaaatagaa ttttggagat atgattgata 206880
gttgattggt atttttttgt atgtagtgat gtttgtgata tttaatagtt gtttataatt 206940
tataatttgt tgttatttat ttttttattt taaatgaatt attgtattta tatttttaga 207000
gggttgtata agtttatatt attttgattt tttgttgatt gttttttgt tttttgtttg 207060
gttttattt tttttattat ttgtgtaatt agtttttgt ttttaagttt ttttgtttta 207120
aatgtttaag tggattaggt attgtggttt ttgtttgtaa ttttagtatt ttgggaggtt 207180
taggtaggag gattatataa atttaggagt ataagattaa tttgggtaat atagtaagat 207240
tttgttttta tatatatata aaaaatataa aaattagtaa ggtgtgatgg tatatgtttg 207300
tagttttagt attttgggag gttgaggtag gaggattgtt tgaggttggg agtttaggat 207360
tagtttgggt aataaagtaa gattttgttt ttataaaaat aaaagaaaaa attaggtagg 207420
tatatagtgt gtatttatag tttgagttat ttgggaggtt gagaagggag gattgtttga 207480
gtttaggagt ttaaggttgt tatgagttgt gattatatta ttgtatttta gtttgtgtga 207540
tagagtgaga ttttgttttg ggaaaaataa ataaataaat aaataaataa ataaataaat 207600
aaatgtttaa gtggttttta tttttggtt agattttatt atatatttta tgaataaatt 207660
gtatgtatga gaaaggtgag tagatgaata gggtagggtt aggggttaa ggggaagatt 207720
atagattatg tggagtgaga ttagtggtta aaaattgtta attatagtta ggtttggtgg 207780
tttatgaagg taattttaat ggttgggttt ggtggtttat gtttgtaatt ttagtatttt 207840
gggaggttga tgtgagttta ttatttgagg ttaagagttt gagattagtt tggttaatgt 207900
agggaaattt tgtttttatt aaaaatatta aaattggtta ggtatggtgg tgggtgtttg 207960
tagttttagt tatttgggag gttgaagtag gagaattggt tgaatttggg aggtggaggt 208020
tgtagtgagt tgggatttta ttattgtatt ttagtttggg tgatagagtg agattttatt 208080
ttaaaaaatt taaaaaaaaa aaaataaaaa attgtagatt ttaataagga agagttatag 208140
gatgatattt taggatgtgg gaaaaggtta tgttagtttt ggattttttt tgattaatgg 208200
gtattttagt tattaaaggg gtagatgtgt tttatttaat ttttatttag ttattagagt 208260
ataatatagg gatgtgttat agatagagat ggagattaat aggtagaggg gtgatgggga 208320
ggaagattga ggtaagagga ataaaatggg gattgtagag ggtaggtggt aggagtttga 208380
gggttttaga agaaattgtt gtataagtaa tgagttagta gggtgagtga tggttaggag 208440
aggtggttta agaaaataaa gagatatttt tgggggtata gatgtgtttt gaggggttga 208500
ggaagagatg gggaaggggt ggttttgtgg atttttggta tgattgttgt ggtgaaaagg 208560
aattaaagat tttgataagg ggatgatttt ttaaataagg aagttttggg tttttgtagg 208620
gggattagta gattgagggt aaatggtatt ttgtttatta aagttttaat aaaatgttaa 208680
ggtaagaata tttgtttggt tttgggggta attttagaaa tattagaaaa gggatttatt 208740
atgtgtaatt ttttttaat tatttggaga aatgtatggt tatttaagat agagaaaggt 208800
agttaaagta agtggaggtt aggtatagtg atttatgttt gtatttttag tgttttggga 208860
ggttgaggta ggaggattgt ttgaggttgg aaatttagta ttagtttagg taataataaa 208920
ataagatttt gttgttataa aaataaaaat aaaaattttt tgagatggag ttttattttt 208980
tttgtttagg ttggagtgtt gtggtatgat tttagtttat tgtaattttt gtttttaga 209040
tttaagtaat tttttgttt tagttttttg agtagttggg attataggta tgtgttatta 209100
tgtttagtta attttgtagt tttagtagag atagggtttt tttatgttgg ttaggttggt 209160
tttgaatttt tgattttttt ggttttttaa agtgttagga ttataggtat gagttattgt 209220
gtttggttgt tataaaaatt ttttaaaaat tagttaggta tgttaggtgt ggtggttttt 209280
atttgtaatt ttagtatttt gggaggttaa ggtaggtgga ttatgaggtt aggagtttga 209340
gattagtttg attaatatgg tgaaattttg tttttattaa aaatataaaa attagttagg 209400
tgtggtggta tatgtttgta attgtagtta tttaggaggt tgaggtagga gaattgtttt 209460
aatttaggag ttgggggttg tagtgagttg agattgtatt atggtatttt agtttgggta 209520
atagagtaag attttgtttt aaaaaaaaaa aaaaaaaaaa aattagttag gtatgtagtg 209580
tatatttgta gttttagtta tttaggaggt tgagaaggga ggattaattg aaattgggag 209640

gttgagattg ggaggttatg attatgttat tgtattttaa tttgggtaat atagtaagat 209700
tttgtttaat aataataata ataaataatg taagtgggta taagaattta tttatttatt 209760
tatttattta tttatttatg agatagggtt ttattttgtt gtttaggttg gagtgtattg 209820
gtgtaatttt ggtttattgt aagtttgtt ttttgggttt atgttatttt tttgttttag 209880
tttttttgagt agttgggatt ataggtgttt gttattgtat ttagttaatt ttttgtattt 209940
ttagtagaga tggggtttta ttatggtttt gattttttga ttttgtgatt tgtttgtttt 210000
ggttttttaa agtgttggga ttataggtgt gagttattgt ttttggtttt aagaatttat 210060
tttattttag tattttggag gttgaggtga gtggattatg aggttagaag attgagatta 210120
ttttggttaa tatggtgaaa ttttgttttt attaaaaaaa tataaaaaat tagttgggtg 210180
tgggggtggg tgtttgtagt tttagttatg tgggatgttg aggtaggaga atggtgtgaa 210240
tttgggaggt ggagtttgta gtgagtggag attgtgttat tgtattttag tttgggtaat 210300
agagtgagat tttattttaa aaaaaaaaaa aaaagaattt attttaagat agtaagtttt 210360
agggttgaag ttagttttta tttagtttta ggtttaaagt tataagtatg gttattgtaa 210420
tattttttatt taggatatta ttttttaggg gtttagattt atattattaa ggaggagtgt 210480
ttatattagt agttggggag agggtggttt tttataagat gtaatgttta ttttgaagag 210540
ttattatagt gataattatg aaatttatgt ttgagattta attttaagtg aaaagaatag 210600
aatttaatat ttattttatg cggagtataa aattttgaaa atatatgttt tttataatta 210660
aattaaattt tatgttatgt ttttgaggag ataagtattg aatggaatat agaagaaatg 210720
ttatagttaa tgattgttga agttttttttt ttaatttttt gtttatgttg tttggtaata 210780
agtaatatag aaaattgata ataaaaagta aaagagaaag aaaagatata gtgtatagtt 210840
tagattgttt tttttaaaaa attaaataat aataataaaa agtataggtt gggtgtggtg 210900
gtttatgttt gtaattttag tattttggga ggttaaggtg ggtggattat ttgaggttgg 210960
gagtttgaga ttagtttgat taatatgggg atattttgtt tgtattaaaa atataaaatt 211020
agttgggtat ggtgatgtag gtttgtaatt ttagttattt gggaggttga ggtaagagaa 211080
ttgtttgaat ttgggaggtg gaggttgtag tgagttgaga ttgtgttttt gtattttagt 211140
ttgggtaata agagtgaaat tttattttaa aaaaaaaaaa aaaaaagtat aaatgtaagt 211200
tgggtgtggt agtttatgtt tgtaatttta gtattttggg aggttggggt aggtggatta 211260
tttgaggtta ggagtttgaa attaatttgg ttaatatggt gaaattttat ttttattaaa 211320
aatataaaaa ttagttaggt atggtggtgt gtgtttgtaa ttttagttat ttaggaggtt 211380
gaggtaggag aattgtttga atttaggagg tagaggttgt agtgagttaa gattgagtta 211440
ttatatttta gtttgggtga tagagtgaga ttttgtttta aaaattaaat taaattaaat 211500
taaataaaat aaaaaaaaga agtggtataa tataaagttt tggtagttaa agtgttaatt 211560
tttagttatt aggaaggttt tgttatttaa tgatttattg ttttagttaa ggttagttat 211620
attttgggtt tttgatgttg gaggtaatat gatgttaata ttggtgagaa atttattatt 211680
ttttttttt tgttttttgg tgttggtttt tgtgtgtatt tgtagttttt ttattattgg 211740
tgtttttatg gtgtttatgt tattggttta ttttttagta ataatttgat agtttgatat 211800
ttttgtagat tttgtggtta tagagttaaa gtttttttgtt attagtgggt tttgtagata 211860
attttttaaa tttttttggg ttgagttagt taagttttta ataagttgga agatgatagg 211920
ggttggatgt aatttatttt taattggttt gggttatatg gatattattt tttttttggg 211980
atttgatttg ggatggaaat aggagttata gtttatggga aggttgtgaa aggaagtttt 212040
ggttttaggg tgtggattag gttgatttgt ggaataaggt tttaggggtg gtttatttgt 212100
ttgttttgtt tagttttttt tggtaattat atttatttgg ggtttatttt aaatttggta 212160
ggtaatgttg ttgtttttat tttagttgtt gttttaatat gttttgtttg gttaggatag 212220
gtgtttaggt atggtattta gggatatttt tggtttttgg ggtttaattt ttggttttta 212280
ggtgtagtgt tgggaaaagt agagggtttt ggttatggtg taggttttgt tataggttat 212340
ttaagtagtt tgggaggagt tggaaagaga gtaatgtatt tatttggata gagattgaag 212400
ttatattttt attgagaaaa gatgatattt ttgtttggat ttttttttg aaattttagg 212460
ttggggtggg gtaggagtta gtgagagagt ttagtgtttt aaaggggatt ttttggtttt 212520
ttgtgttttt taatttagta tttttgggga tttatatatt tagggtagag tggttattta 212580
gggttttttt tatttagata taaaggttat tgaaggtttt gggttaatat tgttttgagg 212640
atgattagtg atttgagttt ttgttagaga tagtaggtag tttgggttta agggttgttt 212700
agtgatttga agttgggatt attttatatt ttttgtgtta tttttgtttt ttgttttgga 212760
ggtttttagg ttgttaatag ttatgttttg gagaattagg ttagattagg ttgggaataa 212820
agtttagttg ttatagagtt gttgagaggt tattggtaag tagtgtttag gagttgtttt 212880
ttttttgtta gtaatagttt aatgtttttt tttatagggg ttgtaaatgg gtttatgttt 212940
agggtggggt tagggagata aagtagagag aaattgggtt gggagtagag agtgttatgt 213000
tttattttgg agatttaagg ttaggttatt ttgtagagtt ggtgtttaga gtttttagtg 213060
tataggagag gaggtaaata gaatttgata tttagtagtg tttggtagat gtttttggaa 213120

tgaatgatta aatttatatg gagagttgaa atgggttagg aattttaggg agatgggtag 213180
tttatagttt agtagggttg ttttagtaga gtaggttaat gttaggtttt ttggtaaatt 213240
ttttggggtt taggtataat tgttatatta ggaaaggggt aggggtgatt agtttattgg 213300
ttatgggggt attttattgt ttttatagaa gtttggtatt tataggtttg ggtgatatat 213360
gatttttatg tttggtatta tgatggttgt ttattaagtt tttttattta ttaggattga 213420
ttttttttgg ttagtgattt tagattatta tgatttggta gttgttgtta ggtttgtttg 213480
gttaaggatt tttttggaga ttgagttatt ttatatagta aagtgagtta tggggttata 213540
gtgttgtgtg ttgtgtataa gttttgaggg ttttatttag ggattggtat ttattgattt 213600
ttataaattt ttggtaattg tttagagttg ggatgttttt aaggtaatag ttttaataaa 213660
tagaggtgta ggaatttagt gtgttatttt ttgttttatt ttaaaggttt atatttttag 213720
gggtttggag ttttttttttg gagtttttt tattttaaag ttagttggat ttgtttagga 213780
attgttatta ttattttttg gttgggtttg tattgatgtg tattgaattt tttttattag 213840
gtttatttta ggtaaatttt agagtttatg taatattgtt tgtggataaa tattggggtg 213900
gaaatagggg ttatttttag gttttagtta tatggggttg ttagggttgt ttgggatggt 213960
aggagagtgt tagaggaggg tagtttaagg attaggtagg gggtgggaag tgtttttttt 214020
tatgggtttt taagtgagat gtttttttgta aatatttatt tgggattttt aagtagaggg 214080
ttggttttta tgttatttgg atttggattt tagattattg tttttaagtg gtaattttgg 214140
gttttttttgt atttgttttg gtttgagttg gttatatttg tggggggtgg ttttaggaag 214200
aaggtggatt gttgtaaatg taatgttttg ttgttaggtg tattgttttt ggttattaga 214260
atttttttgg gtaggttggg gagatttgat gggtaagtgt ttgtggtttg gtgtgagtgg 214320
gtagggtagg tggtttttgt ttgggtatgt ttttttattt ttgtaataga ttagggagag 214380
ggttggtttt aggatttagg tttagttagt tgttttataa ttgaggggtt ttggtggagg 214440
atataagtta gatagagtta tagattaatt gtatgaggtg gttttttttt tttagtttag 214500
gtttttttgaa tgtgttagag tagataagtt tggatgtgag gaggtaagag gtagtgagaa 214560
taggtgtgga gatggggttg ggggagaaga ggaagaaatt ttgtgtttgt gttttttttta 214620
tgtttgtttg ttattgggtt agttgattgg ggaagtgttt tttgagggtg taattttttg 214680
ttttttttgt aagttagttt ttggggtgtt gggtgagggt gtagttttgt ttattatggt 214740
gagttagtat attgaattat ggtagaagtt gtttttttgtg tgggtgtagt tttttttttt 214800
gtttaggatt tagaagttat gatagtatag agtttttttaa ttgtggttgg tgtttttttta 214860
ggaatttttt gaaagttata gagaaatttt agagtggttg ggtatggtgg tttatgtttg 214920
tgattttagt attttgggag gttgaggtgg gtggattatt tgaggtttgg agtttgagat 214980
tagtttggtt agtatggtaa aattttattt ttattaaaaa atataaaaat tagttgggta 215040
tggtggtata tgtttgtaat tttagttatt taggaggttg aggtatgaga attgtttgaa 215100
tttgggaggt agaggttgta gtgaattgag attgtgttat tgtattttag tttgggtaat 215160
agagtaagat tttattttaa aaaaataaaa aataaaaaaa gaatttttag agtaattttt 215220
aggtgttagt aggggtgtga tgtaatttta gttttgtttt tgtggtttat tggttttggg 215280
taagtttttt tgttgttgtt atttatagag agtttttagg ggagtttggg aatgatggta 215340
agttttatgt gatttagtat gtaaagggag ttttaaaatt tagaatttag agagttaggg 215400
tgggtatagt gtagttggtt ttagtgttga ggggttgggg gagagatttt gtattatgtt 215460
ttgattatag taagggaaga ttatggaggt ttttggaatg ttttagtaat ggtgtaattt 215520
tagtttttttt tatttttttg ttaatgataa ttatgattat gatagtagtt aataggttat 215580
agagtaggta ttttttagtt tttttaaata tgaatttgtt taatttttat aataatatag 215640
tattgtttta tttaataaat gaggtaggtt gggtgtggtg gtttatgttt gtaattatag 215700
tattttggga ggttaaggta ggtagattat gaggttagga gtttgagatt agtttgatta 215760
atatggtgaa attttatttt tagtaaaaat ataaaaatta gttgggtgta gtggtatgtt 215820
tttataattt tagttattta ggaggttgag gtaggagaat tatttgaatt tgggaggtgg 215880
aggttgtagt aagttgagat tatgttattg tattttagtt tgggagatag agtgagattt 215940
tgttttaaaa ataaataaat aaataaaatt aaaaaatatt atatatataa aaaaataaat 216000
gaggtaaatg agatgtatga ggtgagaatt ttgtgttata gggagaggtg tagtttggtg 216060
agatttaatt ttaagggggtt ggtggttttg agtttttaag tgtaattatt ggattatatt 216120
gtttgtagat tattattatt tttattttat tggaaggttg ggtgaggtaa ttgggaaagt 216180
ttggagtagt agtggagttg ttagagagaa tttgttttta gtgttttttt ggattttgta 216240
gttgttttgt gtgggttttt gttgtttttt gtattggaaa agattttttt atagatgggg 216300
ttttatgatg ttatttaggt tggagtgttg tgtttatttt taggtgttgt tatagggttg 216360
tagttttgaa tttttggttt taagtgattt ttttgtttta gtttttttgag tagttttttg 216420
ggtttaagtg atttttgtgt tttagttttt tgagtagttt tttgggttta agtgattttt 216480
gtgttttagt tttttgagta gttgggattt taagtgtatt tggttaagtt tttttttatt 216540
ttttattatt taagattttt ttttttttaa ggtttagttt aattttattt ttatttaaaa 216600

```
tttttggggt atttttgtag tatttggagg ttgaattgtt taggagggaa gggtgttaat 216660
gagttggtgt tttgttttta ttttgatttt tggtgagaat gtaagttttt tgtaggtaag 216720
gattatattt tattattatt tttgtattta tttagatttg gttatgtgtg tttagtatat 216780
aaagtgtttt tgatgattgt ttatagattt atggtatttg agattgtttt aatatatttt 216840
tttaaaattt tttttttttt tagaggttta tgaatgttta aattaggttt attgggaata 216900
atattatggt tattttgttt attttgaagt ttatatatag gtttggaagt aataatttg 216960
tatagtttag atgaaaaaat aaagattttt ttgttgtttt tggattgtta gagttttggg 217020
ggatttttaa tattttttgt tgtttttttt aattttatgg tgttgatggt ttgttggggg 217080
gttagtttga tttttgtgag tgtttgtatt tggaaaaggg ttggtgttaa gatagtttta 217140
ggatatttag aatgagggta agaggttatt tttattatta gtttttttta gttttatttt 217200
atataattgt aatatagtga tttagtatat tttgggttat tgttaagtag ggagtaaatt 217260
agaaatggtt tgagtttttt tttttttat ttttagaggg gtttgtttt ttaggtaaat 217320
ataaggaggt attgaggttg ttgtataaga gttggttttt gtttatttta taaattttat 217380
ttttatttat tgtaaaaggt tttgtttttt tttttaaaaa aaaaaaaaaa aaagaaattt 217440
agtgaggggt gggctaggtg tgtgtggagg agaggagaaa aggataagtg atggtttttt 217500
tattttttag taagttgtag gtgtagagat tttaagtttg ttttagggtt tttttggtat 217560
ttgtgtagga gggtttttgt agaaaaaatt taggatttat gatttgggag gatttggtt 217620
atttttattg tttggattta ttagtgggag gtaaagtgta agaaattaag ggtaaagggt 217680
tttttttggg tgggttagag atttttggga aagggttatg tattgtgtga tttttataat 217740
aattttaagt atttggagta tgttggggag gaagttttgg tgttaaaatt aagtatgtgt 217800
tatttggaag tttgtttttg tttattagta tattaattta tttatgtttt tgatagagtt 217860
gtgttttggg tgtgtagagg gttattttgt ttgtaataat tgggtgaaag ttgaaatttt 217920
aagttattga tttagaaggg tatttttggg gaagttttga gtaatttttt ttgagataag 217980
gtattttatt atttattttg gtttttaaat atggaaagtt agaggaaatt gataatggtt 218040
taaaggtagg gggatggttt tgatgatttt tttattttta attaaggttt atgattttgt 218100
tttttttatta ataaaaatta atttatggat ttttggtagg tatttttta tttgaatgaa 218160
ttaaaggagg gatattaaat aaatggtttt tagatttaga agaggagatg ttatttataa 218220
tttgaggatt gaggaggata aaggattttg tttgtttgga ttttttggag aaaattataa 218280
aggaaaaaaa aaaggaagga aaagataaat aatgaagatt tggatttttt ttaatttgtt 218340
tttgtggttt tggtgtgttt atttgtgaaa tggaatttta gagtgttaga gtgggagttt 218400
tttatgaaat gaaagttttt gaattggttt atggtgaggt ttaagttagg ttttttttgt 218460
ttatttaagg tgggttttgt tagggtggag tgaggatgtt agttgtgggt ggtgttttga 218520
gtgtaaaatg tgaatttgag ttgttaagtt tgggtgttgt tggatggttt tatttggagg 218580
ttgttttgtg ttttattaag tagaggagaa attgaagaga atttgaagaa aggatgttgt 218640
ggtttggatt attagtttta gaggttgaat ttaaggatat tttttttattt agttttgatt 218700
ttatttattt atatagatgt atagggattt aaagtatttg gttgatttgg aaagttagtg 218760
atttttgtga gggtgtaagg ggtgaggaag agggagtgtt ttttagggta gtttttttga 218820
tatttgagta agggttggag atagtgtagg ggatggttgg tttaggttga aagtggtttg 218880
atagtggttt tgttttggga ggaatttgat ttgtagtgtg tttgttgtgg ggatttgttt 218940
agttttgggg gtgttttgtt atttttggtt gtaggtattt aggtgagtag tgttggtttt 219000
gggatattga tttttttttta ttttgggtgt gaagtttatt tgagtaagat ttttttatgg 219060
tgtttttgtg gttgttgttt ttaggaaagt gtggtggtga ggtagtgggg tgtgtgtttt 219120
tagggtgtta attttgggtt ggtagtagga aggtgggaag agggggtggt ttgttgtttg 219180
gagtgtattg attgggtttt gttttggttt tgtttttgtt ttgttttttt ttggagatgt 219240
gtatagaatt ttggtttagt tggttgtggt tttgtttttg atttgttttt tttttgtttt 219300
gtgaatgtgt gtgttgagtt ttatttttgt ttatttttgt gttgtgttgt atttggtgga 219360
gtggggttgg tgggtgttgg gtttgggagg gggtgttttt gtgtggtagt gtagattaga 219420
ttagattagg ttaggttggg ggtgggggtt gtttgtgtgg tttgttgtgt ttgtagtttt 219480
gtatttttag tttttttgtgt tggtgttaat ggttggtttt gggagttgtt gtatttttta 219540
gttttgagtt tgggtggggt gggtttggtt aggtggaagt gggtggaggt gtgggtgttt 219600
tgggtggttt tgttgggtga ggtggtggtg ttgggtattg tggtggtgtt gggtatagtt 219660
gggggttgga gttggggtgt tggtttagtg ggttggggta gggtgggata ggatatttgt 219720
tttgttattt ttttggtaat taaaagggta gagtgtgttg attgagttgg agttttagtt 219780
tttttatttg taaatgggtg tgttggatag atgattattt gtttttttttg tgttgggtat 219840
tgtgtgtagt ttggtgtatt ggtgtagtgg ggtagatata gggagttgtt ttttataatt 219900
tgggtaatgt aggtagtggt gagaggatgt aaaatttgta tggaagaatt ttttttattt 219960
ggatattttt aaagttttta tgataaagtg gtgtttttaa tgtatagagg aagaagtgat 220020
taattatgat tggagggtgg tgttagagtt ttattgaggt gaaattaaaa tagggtttta 220080
```

```
aggaatgagt aggaattagt taggtagtta aggtgatgga aggtagttgt ttttgaaaga   220140
gggaagggtg tggtggagtt tgggaggttt gaggtattat agtgagtgtg gagagataga   220200
ggtagggttg ggtttttgta tagttgttaa tggaggttgt agaggatgag aggaaggtgg   220260
gaggttagtt gggaagtgtt tgttagtttt gggtaggggt gtagtattgt tttttttgt    220320
agttttttta aaaatgtata ggaggttgaa agtgttatta atagttttga aaaatttttt   220380
tttttttttt tttttttta tttttttgg gggtttaaga agtattttta tttttgttt      220440
gagtgtggga aatttatatg tatgagaatg gaggtttatg tggtattgtg ggtttgtttt   220500
ggtttatttg tttttttttt tgagtaattt gtttagttat agttttgtag agggagtagt   220560
ttagttagag gagttttttt ttattttagg tagagttgat tggattggag tggatattgg   220620
atttaattg agtttatttt ttttttttatt tgggggttag aggtttagga ggttttggga   220680
gggaagtgtt gtaagggagg ggagtaggga gtatggatga attgtgaata gtgttttgga   220740
ttttgggatg tttatatttt aattgtaatt tttatttata tgtttattta ttgtttgtat   220800
tagtattagt agattttggt ttaggttgtg ttttgatttg gttagtaagt attatgatga   220860
tattgttttt agtttagtta tgatgatatt gttatgagtt taatgtttgg gatttttaaa   220920
gtttagtggt gttttaagaa tttgggttat gggtgtattg ttggttttgt ttattttatt   220980
tagtataagt tagaggtgtt ttggagtata gttattttga ggatgttagt tttggttgtt   221040
ttttggtttt gggtaggtgg gttttttttgt ggatgtttat gtttttgttt ggggttataa   221100
tttataggaa tttgggtatt taggatttaa ggatatttgt gtggagtgat gttggatata   221160
ttatatgggt gttttttgtg tgatggtttt tggatatttg aatttttttt ttgattttaa   221220
gaaattagaa agaaggataa gagagaggga gggaaggttg ggaatgaggg ttttatatat   221280
ttttgagatg tatgaagttt aatttgttat gaaattagag ttaaaaaaaa aaaagaaaaa   221340
ttaaagagat gtatgaagtt taattgttta agttttaaat tttgttttta tttatttatt   221400
tttatttttt atttttttgag agggagtttt gtttttgttg ttgaggttgg agtgtaatgg   221460
tgtgatttta gtttattgta atttttgttt tttaggttta agtgattttt tagttttagt   221520
tttttgagta gttgagatta taggtgtttg ttatatttgg ttaatttttt ttttttgta    221580
attttttttt tttttagtag agatagggtt ttattatgtt ggttaggttg gttttaaatt   221640
tttaatttga ggtgattttt ttgttttagt tttttaaagt gttgggatta taggtgtgag   221700
ttattgtatt tggtttattt atttatttat gagatagagt ttttttttgt tatttaggtt   221760
agagtgtagt ggtatgattt tagtttatta taatgtttat tttttggatt taagtaattt   221820
ttttgtttta gtttttttaag tagttgggat tataggtata tgttattata attggttaat   221880
ttttgtattt ttagtagaga tggggtttt                                     221909
```

<210> 11
<211> 16
<212> DNA
<213> Artificial Sequence

<220>
<223> chemically treated genomic DNA (Homo sapiens)

<400> 11
cgtcgcaacg cgtacg 16

<210> 12
<211> 20
<212> DNA
<213> Artificial Sequence

<220>
<223> chemically treated genomic DNA (Homo sapiens)

<400> 12
cgcggtttcg attttaatgc 20

<210> 13
<211> 23
<212> DNA
<213> Artificial Sequence

<220>
<223> chemically treated genomic DNA (Homo sapiens)

<400> 13
actccgactt aacccgacga tcg 23

<210> 14
<211> 23
<212> DNA
<213> Artificial Sequence

<220>
<223> chemically treated genomic DNA (Homo sapiens)

<400> 14
acgaaattcc taacgcaacc get 23

<210> 15
<211> 23
<212> DNA
<213> Artificial Sequence

<220>
<223> chemically treated genomic DNA (Homo sapiens)

<400> 15
acgaaattcc taacgcaacc get 23

<210> 16
<211> 108
<212> DNA
<213> Artificial Sequence

<220>
<223> chemically treated genomic DNA (Homo sapiens)

<400> 16

cgtcgcaacg cgtacgactc aaaacttaat aactccgact taacccgacg atcgcgacga 60
aattcctaac gcaaccgctt aaaacttcgc attaaaatcg aaaccgcg 108

<210> 17
<211> 26
<212> DNA
<213> Artificial Sequence

<220>
<223> chemically treated genomic DNA (Homo sapiens)

<400> 17
gtagtagtta gtttagtatt tatttt 26

<210> 18
<211> 42
<212> DNA
<213> Artificial Sequence

<220>
<223> chemically treated genomic DNA (Homo sapiens)

<400> 18
cccaccaacc atcatatgtt cgaaatgatt ttatttagtt gc 42

<210> 19
<211> 17
<212> DNA
<213> Artificial Sequence

<220>
<223> chemically treated genomic DNA (Homo sapiens)

<400> 19
cgttgatcgc ggggttc 17

<210> 20
<211> 32
<212> DNA
<213> Artificial Sequence

<220>
<223> chemically treated genomic DNA (Homo sapiens)

<400> 20
catcatatca aaccccacaa tcaacacaca ac 32

## Description

FIELD OF THE INVENTION

The present invention relates to genomic DNA sequences that exhibit altered expression patterns in disease states relative to normal. Particular embodiments provide, inter alia, novel methods, nucleic acids, nucleic acid arrays and kits useful for detecting, or for detecting pre-cancerous colorectal lesions. Preferably, the methods, nucleic acids, nucleic acid arrays and kits for the screening of individuals to identify those at risk of developing colorectal carcinoma.

BACKGROUND

Incidence and diagnosis of cancer. Cancer is the second leading cause of death of the United States. Mortality rates could be significantly improved if current screening methods would be improved in terms of patient compliance, sensitivity and ease of screening. Current recommended methods for diagnosis of cancer are often expensive and are not suitable for application as population wide screening tests.

Hepatocellular cancer (HCC) is the fourth most common cancer in the world, its incidence varies from 2.1 per 100,000 in North America to 80 per 100,000 in China. In the United States, it is estimated that there will be 17,550 new cases diagnosed in 2005 and 15,420 deaths due to this disease. Ultrasound of the liver, alpha fetoprotein levels and conventional CT scan are regularly obtained in the diagnostic evaluation of HCC (hepatocellular cancer or primary liver cancer), but they are often too insensitive to detect multi-focal small lesions and for treatment planning.

In the United States the annual incidence of colorectal cancer is approximately 150,000, with 56,600 individuals dying form colorectal cancer each year. The lifetime risk of colorectal cancer in the general population is about 5 to 6 percent. Despite intensive efforts in recent years in screening and early detection of colon cancer, until today most cases are diagnosed in an advanced stage with regional or distant metastasis. While the therapeutic options include surgery and adjuvant or palliative chemotherapy, most patients die from progression of their cancer within a few months. Identifying the molecular changes that underlie the development of colon cancer may help to develop new monitoring, screening, diagnostic and therapeutic options that could improve the overall poor prognosis of these patients.

The current guidelines for colorectal screening according to the American Cancer Society utilizes one of five different options for screening in average risk individuals 50 years of age or older. These options include 1) fecal occult blood test (FOBT) annually, 2) flexible sigmoidoscopy every five years, 3) annual FPBT plus flexible sigmoidoscopy every five years, 4) double contrast barium enema (DCBE) every five years or 5) colonoscopy every ten years. Even though these testing procedures are well accepted by the medical community, the implementation of widespread screening for colorectal cancer has not been realized. Patient compliance is a major factor for limited use due to the discomfort or inconvenience associated with the procedures. FOBT testing, although a non-invasive procedure, requires dietary and other restrictions 3-5 days prior to testing. Sensitivity levels for this test are also very low for colorectal adenocarcinoma with wide variability depending on the trial. Sensitivity measurements for detection of adenomas is even less since most adenomas do not bleed. In contrast, sensitivity for more invasive procedures such as sigmoidoscopy and colonoscopy are quite high because of direct visualization of the lumen of the colon. No randomized trials have evaluated the efficacy of these techniques, however, using data from case-control studies and data from the National Polyp Study (U.S.) it has been shown that removal of adenomatous polyps results in a 76-90% reduction in CRC incidence. Sigmoidoscopy has the limitation of only visualizing the left side of the colon leaving lesions in the right colon undetected. Both scoping procedures are expensive, require cathartic preparation and have increased risk of morbidity and mortality. Improved tests with increased sensitivity, specificity, ease of use and decreased costs are clearly needed before general widespread screening for colorectal cancer becomes routine.

Early colorectal cancer detection is generally based on the fecal occult blood test (FOBT) performed annually on asymptomatic individuals. Current recommendations adapted by several healthcare organizations, including the American Cancer Society, call for fecal occult blood testing beginning at age 50, repeated annually until such time as the patient would no longer benefit from screening. A positive FOBT leads to colonoscopic examination of the bowel; an expensive and invasive procedure, with a serious complication rate of one per 5,000 examinations. Only 12% of patients with heme positive stool are diagnosed with cancer or large polyps at the time of colonoscopy. A number of studies show that FOBT screening does not improve cancer-related mortality or overall survival. Compliance with occult blood testing has been poor; less than 20 percent of the population is offered or completes FOBT as recommended. If FOBT is properly done, the patient collects a fecal sample from three consecutive bowel movements. Samples are obtained while the patient adheres to dietary guidelines and avoids medications known to induce occult gastrointestinal bleeding. In reality, physicians frequently fail to instruct patients properly, patients frequently fail to adhere to protocol, and some patients find the task of collecting fecal samples difficult or unpleasant, hence compliance with annual occult blood testing is poor. If testing sensitivity and specificity can be improved over current methods, the frequency of testing could be reduced, collection of consecutive samples would be eliminated, dietary and medication schedule modifications would be eliminated, and patient compliance would be enhanced. Compounding the problem of compliance, the sensitivity and specificity of FOBT to detect colon cancer is poor. Poor test specificity leads to unnecessary colonoscopy, adding considerable expense to colon cancer screening.

Specificity of the FOBT has been calculated at best to be 96%, with a sensitivity of 43% (adenomas) and 50% (colorectal carcinoma). Sensitivity can be improved using an immunoassay FOBT such as that produced under the tradename 'InSure™', with an improved sensitivity of 77 % (adenomas) and 88.9% (colorectal carcinoma.

Molecular disease markers. Molecular disease markers offer several advantages over other types of markers, one advantage being that even samples of very small sizes and/or samples whose tissue architecture has not been maintained can be analyzed quite efficiently. Within the last decade a number of genes have been shown to be differentially expressed between normal and colon carcinomas. However, no single or combination of marker has been shown to be sufficient for the diagnosis of colon carcinomas. High-dimensional mRNA based approaches have recently been shown to be able to provide a better means to distinguish between different tumor types and benign and malignant lesions. However its application as a routine diagnostic tool in a clinical environment is impeded by the extreme instability of mRNA, the rapidly occurring expression changes following certain triggers (e.g., sample collection), and, most importantly, the large amount of mRNA needed for analysis (Lipshutz, R. J. et al., Nature Genetics 21:20-24, 1999 ; Bowtell, D. D. L. Nature genetics suppl. 21:25-32,1999), which often cannot be obtained from a routine biopsy.

The use of biological markers to further improve sensitivity and specificity of FOBT has been suggested, examples of such tests include the PreGen-Plus™ stool analysis assay available from EXACT Sciences which has a sensitivity of 20% (adenoma) and 52% (colorectal carcinoma) and a specificity of 95% in both cases. This test assays for the presence of 23 DNA mutations associated with the development of colon neoplasms. The use of DNA methylation as colon cancer markers is known. For example Sabbioni et al. (Molecular Diagnosis 7:201-207, 2003 ) detected hypermethylation of a panel of genes consisting of TPEF, HICl, DAPK and MGMT in peripheral blood in 98% of colon carcinoma patients. However, this does provide a suitable basis for a commercially marketable test, as the specificity of such a test must also be sufficiently high.

The current model of colorectal pathogenesis favours a stepwise progression of adenomas, which includes the development of dysplasia and finally signs of invasive cancer. The molecular changes underlying this adenoma-carcinoma sequence include genetic and epigenetic alterations of tumour suppressor genes (APC, p53, DCC), the activation of oncogenes (K-ras) and the inactivation of DNA mismatch repair genes. Recently, further molecular changes and genetic defects have been revealed. Thus, activation of the Wnt signalling pathway not only includes mutations of the APC gene, but may also result from β-catenin mutations. Furthermore, alterations in the TGF-β signalling pathway together with its signal transducers SMAD4 and SMAD2 have been linked to the development of colon cancer.

Despite recent progress in the understanding of the pathogenesis of adenomas and carcinomas of the colon and their genetic and molecular changes, the genetic and epigenetic changes underlying the development of metastasis are less well understood. It is, however, generally well accepted that the process of invasion and proteolysis of the extracellular matrix, as well as infiltration of the vascular basement membrane involve adhesive proteins, such as members of the family of integrin receptors, the cadherins, the immunoglobulin superfamily, the laminin binding protein and the CD44 receptor. Apart from adhesion, the process of metastasis formation also includes the induction and regulation of angiogenesis (VEGF, bFGF), the induction of cell proliferation (EGF, HGF, IGF) and the activation of proteolytic enzymes (MMPs, TIMPs, uPAR), as well as the inhibition of apoptosis (Bcl-2, Bcl-X). More recently other groups have compared the genetic and molecular changes in metastatic lesions to the changes found in primary colorectal cancers. Thus, Kleeff et al. reported the loss of DOC-2, a candidate tumour suppressor gene, both in primary and metastatic colorectal cancer. Furthermore, Zauber et al. reported that in their series of 42 colorectal cancers Ki-ras mutations in the primary cancers were identical in all of the 42 paired primary and synchronous metastatic lesions. Similarly loss of heterozygosity at the APC locus was identical for 39 paired carcinomas and synchronous metastasis. The authors concluded that for Ki-ras and APC genes the genetic changes in metastasis are identical to the primary colorectal cancer. However, other groups have found genetic and molecular changes in metastatic colon cancers, that are not present in the primary cancers. Thus, the development of LOH of chromosome 3p in colorectal metastasis has been reported. In addition, using comparative genomic hybridization several alterations were found in liver metastasis that were unique to metastastic lesions (-9q, -11q, and -17q).

CpG island methylation. Apart from mutations aberrant methylation of CpG islands has been shown to lead to the transcriptional silencing of certain genes that have been previously linked to the pathogenesis of various cancers. CpG islands are short sequences which are rich in CpG dinucleotides and can usually be found in the 5' region of approximately 50% of all human genes. Methylation of the cytosines in these islands leads to the loss of gene expression and has been reported in the inactivation of the X chromosome and genomic imprinting.

The genomic sequence of the ALX4 gene and sequences derived therefrom by bisulfite treatment and the use of said sequence for diagnosing fully developed colorectal cancer has been disclosed by WO 2005/001141 and WO 2005/001140. These documents only disclose the use of tissue samples. The diagnosis of precancerous lesions in bodily fluids such as blood is not shown.

Zou et al., (2006) "Selection of methylated genes as candidate markers for colorectal cancer" AACR Meetings, and Ebert (2006) "Genome scanning analysis identifies ALX4 gene methylation as a potential marker for colorectal and other gastrointestinal adenocarcinomas" Gastroenterology, Vol. 130, no. 4, supplement 2, A54 disclose hypermethylationof the ALX4 gene in precancerous lesions but remains silent with respect to the Septin 9 gene.

Multifactorial approach. Cancer diagnostics has traditionally relied upon the detection of single molecular markers (e.g., gene mutations, elevated PSA levels). Unfortunately, cancer is a disease state in which single markers have typically failed to detect or differentiate many forms of the disease. Thus, assays that recognize only a single marker have been shown to be of limited predictive value. A fundamental aspect of this invention is that methylation-based cancer diagnostics and the screening, diagnosis, and therapeutic monitoring of such diseases will provide significant improvements over the state-of-the-art that uses single marker analyses by the use of a selection of multiple markers. The multiplexed analytical approach is particularly well suited for cancer diagnostics since cancer is not a simple disease, this multi-factorial "panel" approach is consistent with the heterogeneous nature of cancer, both cytologically and clinically.

Key to the successful implementation of a panel approach to methylation based diagnostic tests is the design and development of optimized panels of markers that can characterize and distinguish disease states. The present invention describes a plurality of particularly efficient and unique panels of genes, the methylation analysis of one or a combination of the members of the panel enabling the detection of colon cell proliferative disorders with a particularly high sensitivity, specificity and/or predictive value.

Development of medical tests. Two key evaluative measures of any medical screening or diagnostic test are its sensitivity and specificity, which measure how well the test performs to accurately detect all affected individuals without exception, and without falsely including individuals who do not have the target disease (predictive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present. In this context: Sensitivity = TP/(TP+FN); Specificity = TN/(FP+TN); and Predictive value = TP/(TP+FP).

Sensitivity is a measure of a test's ability to correctly detect the target disease in an individual being tested. A test having poor sensitivity produces a high rate of false negatives, i.e., individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high. Specificity, on the other hand, is a measure of a test's ability to identify accurately patients who are free of the disease state. A test having poor specificity produces a high rate of false positives, i.e., individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cancer, often involve a plurality of genes and proteins. Additionally, certain proteins may be elevated for reasons unrelated to a disease state. An example of a test that has high specificity is a gene-based test that can detect a p53 mutation. Specificity is important when the cost or risk associated with further diagnostic procedures or further medical intervention are very high.

Pronounced need in the art. It is generally accepted that there is a pronounced need in the art for improved screening and early detection of cancers. As an example, if colon cancer screening specificity can be increased, the problem of false positive test results leading to unnecessary colonoscopic examination would be reduced leading to cost savings and improved safety.

In view of the incidence of cancers in general and more particularly the disadvantages associated with current colorectal cell proliferative disorder screening methods there is a substantial need in the art for improved methods for the early detection of cancer, in particular colon cancer, to be used in addition to or as a substitute for currently available tests.

Background of the genes of the present invention. The human Septin 9 gene (also known as MLL septin-like fusion protein, MLL septin-like fusion protein MSF-A, Slpa, Eseptin, Msf, septin-like protein Ovarian/Breast septin (Ov/Br septin) and Septin D1) is located on chromosome 17q25 within contig AC068594.15.1.168501 and is a member of the Septin gene family. SEQ ID NO: I provides the sequence of said gene, comprising regions of both the Septin 9 and Q9HC74 transcripts and promoter regions.

It has been postulated that members of the Septin gene family are associated with multiple cellular functions ranging from vesicle transport to cytokinesis. Disruption of the action of Septin 9 results in incomplete cell division, see Surka, M.C., Tsang, C.W., and Trimble, W.S. Mol Biol Cell, 13: 3532-45 (2002). Septin 9 and other proteins have been shown to be fusion partners of the proto-oncogene MLL suggesting a role in tumorigenesis, see Osaka, M, Rowley, J.D. and Zeleznik-Le, N.J. PNAS, 96:6428-6433 (1999). Burrows et al. reported an in depth study of expression of the multiple isoforms of the Septin 9 gene in ovarian cancer and showed tissue specific expression of various transcripts, see Burrows, J. F., Chanduloy, et al. S.E.H. Journal of Pathology, 201:581-588 (2003).

A recent study of over 7000 normal and tumor tissues indicates that there is consistent over-expression of Septin 9 isoforms in a number of tumor tissues, see Scott, M., Hyland, P.L., et al. Oncogene, 24: 4688-4700 (2005 ). The authors speculate that the gene is likely a type II cancer gene where changes in RNA transcript processing control regulation of different protein products, and the levels of these altered protein isoforms may provide answers to the gene's role in malignancy.

The ALX4 gene is a putative transcription factor that belongs to the family of paired-class homeoproteins. This gene is part of a family of genes that includes the mammalian genes A1x3, Cart-1, MHox, and S8 and exhibits similarity to the Drosophila gene aristaless. It binds palindromic DNA sequences (5'-TAAT-3') as either homodimers or as heterodimers with other family members and strongly activates transcription from a promoter containing the homeodomain binding site, P2. ALX4 is expressed at several sites during development, including the craniofacial and limb-bud mesenchyme. Interestingly, ALX4 deficient mice exhibit body-wall defects, preaxial polydactyly, and a decreased size of the parietal plate of the skull, while mutations of the human homeobox gene ALX4 have been found in inherited defects of skull ossification. ALX4 is also expressed in various tissues whose development is dependent on epithelial-mesenchymal interactions and regulates mesenchymal-specific activities of LEF-1.

Methylation of the gene ALX4 has been previously disclosed in WO 2004/035803. In said document it was disclosed that ALX4 was methylated across all classes of colon adenoma and polyp classes, with no differentation between benign, malignant and pre-malignant classes thereof. The subject matter of the present invention differs from that of WO 2004/035803 in that the method of the present invention is practised on body fluid isolated from a subject. The technical effect of practising the method on body fluid samples is that it enables the differentiation between benign and pre-cancerous lesions. Thus the technical problem to be solved by the present invention is how to differentiate between harmless (i.e. benign) and potentially harmful (i.e. those undergoing malignant transformation) colorectal lesions. The person of skill in the art when taking the teachings of WO 2004/035803 into account would not be led to analyse the methylation of said markers in body fluids as opposed to e.g. a histological sample.

## SUMMARY OF THE INVENTION

The present invention provides a method for determining the presence or absence of pre-cancerous colorectal lesions in a subject comprising determining the expression levels of Septin 9 (including all transcript variants thereof) and ALX4 in a body fluid sample isolated from said subject wherein CpG methylation is indicative of the presence of said lesions. Alternatively, said invention provides a method for the differentiation of pre-cancerous from benign colorectal lesions. Various aspects of the present invention provide an efficient and unique genetic marker, whereby expression analysis of said marker enables the detection of pre-cancerous lesions with a particularly high sensitivity, specificity and/or predictive value. The inventive testing methods have particular utility for the screening of at-risk populations. The inventive methods have advantages over prior art methods (including the industry standard FOBT) because it enables the detection of colorectal lesions undergoing malignant transformation but prior to the development of cancer. Furthermore the high sensitivity and specificity as well as non-invasiveness of such a test is likely to result in increased patient compliance.

In one embodiment the invention provides a method for detecting and/or classifying cell proliferative disorders in a subject comprising determining the expression levels of Septin 9 (including all transcript variants thereof) and ALX4 in a body fluid sample isolated from said subject wherein CpG methylation is indicative of the presence or class of said disorder. In one example said expression level is determined by detecting the presence, absence or level of mRNA transcribed from said gene. In a further example said expression level is determined by detecting the presence, absence or level of a polypeptide encoded by said gene or sequence thereof.

In a further preferred embodiment said expression is determined by detecting the presence or absence of CpG methylation within said genes, wherein the presence of methylation indicates the presence of a cell proliferative lesion undergoing or having already achieved malignant transformation. Said method comprises the following steps: i) contacting genomic DNA isolated from a body fluid sample (preferably selected from the group consisting of blood plasma, blood serum, whole blood, isolated blood cells, cells isolated from the blood) obtained from the subject with at least one reagent, or series of reagents that distinguishes between methylated and non-methylated CpG dinucleotides within at least one target region of the genomic DNA, wherein the nucleotide sequence of said target region comprises at least one CpG dinucleotide sequence of bothSeptin 9 (including all transcript variants thereof) and ALX4; and ii) detecting and/or classifying cell proliferative disorders , at least in part. Preferably the target region comprises, or hybridizes under stringent conditions to a sequence of at least 16 contiguous nucleotides of at least one sequence selected from the group consisting of SEQ ID NO: 1 to SEQ ID NO: 2.

The method is novel as no methods currently exist that enable the early detection of potentially harmful colorectal lesions. For example, current methods used to detect and diagnose colorectal carcinoma include colonoscopy, sigmoidoscopy, and fecal occult blood colon cancer. In comparison to these methods, the disclosed invention is much less invasive than colonoscopy, and as, if not more sensitive than sigmoidoscopy and FOBT while enabling the detection of harmful lesions before reaching the carcinoma stage. The development of a body fluid assay represents a clear technical advantage over current methods known in the art in that it is anticipated that at least for colorectal carcinoma screening patient compliance for a single body fluid based test will be higher than the triplicate analysis of stool currently recommended for FOBT.

A particular embodiment of the method comprises the use of Septin 9 (including all transcript variants thereof) and ALX4 as a markers for the detection and/or classification of colorectal cellular proliferative disorders. The present invention is particularly suited for the detection of pre-cancerous colorectal cellular proliferative disorders undergoing malignant transformation. Said use of the genes may be enabled by means of any analysis of the expression of the gene, by means of mRNA expression analysis or protein expression analysis. However, in the most preferred embodiment of the invention, the detection, differentiation and distinguishing of colorectal cell proliferative disorders is enabled by means of analysis of the methylation status of Septin 9 (including all transcript variants thereof) and ALX4 and/or their promoter or regulatory elements.

The invention provides a method for the analysis of biological samples for features associated with the development of pre-cancerous cellular proliferative disorders, the method characterized in that at least one nucleic acid, or a fragment thereof, from both SEQ ID NO: 1 and SEQ ID NO: 2 is contacted with a reagent or series of reagents capable of distinguishing between methylated and non methylated CpG dinucleotides within the genomic sequence, or sequences of interest.

The present invention provides a method for ascertaining epigenetic parameters of genomic DNA associated with the development of malignant colorectal cellular proliferative disorders, the method has utility for the improved diagnosis, treatment and monitoring of said diseases.

Preferably, the source of the test sample is selected from the group consisting of cells or cell lines, histological slides, biopsies, paraffin-embedded tissue, body fluids, stool, urine, blood, and combinations thereof. More preferably, the source is selected from the group consisting of stool, blood plasma, blood serum, whole blood, isolated blood cells, cells isolated from the blood obtained from the subject.

Specifically, the present invention provides a method for detecting pre-cancerous colorectal cellular proliferative disorders or for differentiating between pre-cancerous and benign cellular proliferative disorders, comprising: obtaining a body fluid sample comprising genomic nucleic acid(s); contacting the nucleic acid(s), or a fragment thereof, with one reagent or a plurality of reagents sufficient for distinguishing between methylated and non methylated CpG dinucleotide sequences within at least two target sequences of the subject nucleic acid, wherein the target sequences comprise, or hybridise under stringent conditions to, a sequence comprising at least 16 contiguous nucleotides of SEQ ID NO: 1 and to a sequence comprising at least 16 contiguous nucleotides of SEQ ID NO: 2, said contiguous nucleotides comprising at least one CpG dinucleotide sequence; and determining, based at least in part on said distinguishing, the methylation state of both target CpG dinucleotide sequences, or an average, or a value reflecting an average methylation state of a plurality of target CpG dinucleotide sequences. Preferably, distinguishing between methylated and non methylated CpG dinucleotide sequences within the target sequences comprises methylation state-dependent conversion or non-conversion of at least two CpG dinucleotide sequence to the corresponding converted or non-converted dinucleotide sequence within a sequence selected from the group consisting of SEQ ID NO: 3 to SEQ ID NO: 10, and contiguous regions thereof corresponding to the target sequence.

Further embodiments provide alternative methods comprising: obtaining a body fluid sample having subject genomic DNA; extracting the genomic DNA; contacting the genomic DNA, or a fragment thereof, comprising the sequences of SEQ ID NO: 1 and SEQ ID NO: 2 or sequences that hybridize under stringent conditions thereto, with one or more methylation-sensitive restriction enzymes, wherein the genomic DNA is either digested thereby to produce digestion fragments, or is not digested thereby; and determining, based on a presence or absence of, or on property of at least two such fragments, the methylation state of at least two CpG dinucleotide sequences of SEQ ID NO: 1 and SEQ ID NO: 2, or an average, or a value reflecting an average methylation state of a plurality of CpG dinucleotide sequences thereof. Preferably, the digested or undigested genomic DNA is amplified prior to said determining.

Additionally, genomic and chemically modified nucleic acid sequences, as well as oligonucleotides and/or PNA-oligomers for analysis of cytosine methylation patterns within sequences from the group consisting of SEQ ID NO: 1 to SEQ ID NO: 2 are disclosed.

DETAILED DESCRIPTION OF THE INVENTION

Definitions:

The term "Observed/Expected Ratio" ("O/E Ratio") refers to the frequency of CpG dinucleotides within a particular DNA sequence, and corresponds to the [number of CpG sites / (number of C bases x number of G bases)] / band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about I KB, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG dinucleotide sequences) within a DNA sequence include "unmethylated," "fully-methylated" and "hemimethylated."

The term "hemi-methylation" or "hemimethylation" refers to the methylation state of a double stranded DNA wherein only one strand thereof is methylated.

The term 'AUC' as used herein is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "hypermethylation" refers to the average methylation state corresponding to an increased presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a decreased presence of 5-mCyt at one or a plurality of CpG dinucleotides within a DNA sequence of a test DNA sample, relative to the amount of 5-mCyt found at corresponding CpG dinucleotides within a normal control DNA sample.

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylation. Further epigenetic parameters include, for example, the acetylation of histones which, however, cannot be directly analysed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific blocking probes (also referred to herein as blockers) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific blocking probes covering CpG positions between the amplification primers.

The term "Ms-SNuPE" (Methylation-sensitive Single Nucleotide Primer Extension) refers to the art-recognized assay described by Gonzalgo and Jones, Nucleic Acids Res. 25:2529-2531, 1997 .

The term "MSP" (Methylation-specific PCR) refers to the art-recognized methylation assay described by Herman et al. Proc. Natl. Acad. Sci. USA 93:9821-9826, 1996 , and by US Patent No. 5,786,146.

The term "COBRA" (Combined Bisulfite Restriction Analysis) refers to the art-recognized methylation assay described by Xiong and Laird, Nucleic Acids Res. 25:2532-2534, 1997 .

The term "MCA" (Methylated CpG Island Amplification) refers to the methylation assay described by Toyota et al., Cancer Res. 59:2307-12, 1999 , and in WO 00/26401 A1.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y .; and Ausubel et al. (eds.), 1995, Current Protocols in Molecular Biology, (John Wiley and Sons, N.Y.) at Unit 2.10.

The terms "Methylation-specific restriction enzymes" or "methylation-sensitive restriction enzymes" shall be taken to mean an enzyme that selectively digests a nucleic acid dependant on the methylation state of its recognition site. In the case of such restriction enzymes which specifically cut if the recognition site is not methylated or hemimethylated, the cut will not take place, or with a significantly reduced efficiency, if the recognition site is methylated. In the case of such restriction enzymes which specifically cut if the recognition site is methylated, the cut will not take place, or with a significantly reduced efficiency if the recognition site is not methylated. Preferred are methylation-specific restriction enzymes, the recognition sequence of which contains a CG dinucleotide (for instance cgcg or cccggg). Further preferred for some embodiments are restriction enzymes that do not cut if the cytosine in this dinucleotide is methylated at the carbon atom C5.

"Non-methylation-specific restriction enzymes" or "non-methylation-sensitive restriction enzymes" are restriction enzymes that cut a nucleic acid sequence irrespective of the methylation state with nearly identical efficiency. They are also called "methylation-unspecific restriction enzymes."

The term "gene" shall be taken to include all transcript variants thereof (e.g. the term "Septin 9" shall include for example its truncated transcript Q9HC74) and all promoter and regulatory elements thereof. Furthermore as a plurality of SNPs are known within said gene the term shall be taken to include all sequence variants thereof.

Colorectal lesions which do not have malignant potential are histologically classified as benign and include hyperplastic polyps, hamartomas and inflammatory polyps. Colorectal lesions which do have malignant potential are histologically classified as neoplastic adenomas and include tubular adenomas (0%-25% villious tissue), tubuvillous adenomas (25%-75% villious tissue) and villous adenomas (75%-100% villious tissue).

The terms "pre-cancerous" or "pre-malignant" and equivalents thereof shall be taken to mean any cellular proliferative disorder which is undergoing malignant transformation, such as but not limited to neoplastic adenomas including those described above. Examples of such conditions include, in the context of colorectal cellular proliferative disorders, cellular proliferative disorders (such as but not limited to those commonly referred to as "polyps") with a high degree of dysplasia and the following classes of adenomas:

Level 1: penetration of malignant glands through the muscularis mucosa into the submucosa, within the polyp head;
Level 2: the same submucosal invasion, but present at the junction of the head to the stalk;
Level 3: invasion of the stalk; and
Level 4: invasion of the stalk's base at the connection to the colonic wall (this level corresponds to stage Dukes A).

Overview:

The present invention provides a method for detecting pre-cancerous colorectal cell proliferative disorders (e.g. neoplastic adenomas) or for differentiating between benign colorectal lesions and pre-malignant colorectal lesions in a subject comprising determining the expression levels of Septin 9 (including all transcript variants thereof) and ALX4 in a biological sample isolated from said subject wherein CpG methylation is indicative of the presence of said disorder. Said markers may be used for the early detection of colorectal cancers during the pre-cancerous stages of the disease both by detecting the presence thereof and/or differentiating between benign and malignant forms of colorectal lesions.

In a first embodiment the present invention is based upon the analysis of CpG methylation status of Septin 9 (including all transcript variants thereof) and ALX4. Preferably the methylation status of both Septin 9 (including any transcript variants thereof) and ALX4 are analyzed. It is further preferred that the sequences of said genes are as according to TABLE 1.

Bisulfite modification of DNA is an art-recognized tool used to assess CpG methylation status. 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, e.g., PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is converted in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analyzed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996 ). It is thus possible to analyze individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (e.g., Zeschnigk M, et al., Eur J Hum Genet. 5:94-98, 1997 ), is currently only used in research. In all instances, short, specific fragments of a known gene are amplified subsequent to a bisulfite treatment, and either completely sequenced (Olek and Walter, Nat Genet. 1997 17:275-6, 1997 ), subjected to one or more primer extension reactions (Gonzalgo and Jones, Nucleic Acids Res., 25:2529-31, 1997 ; WO 95/00669 ; U.S. Patent No. 6,251,594) to analyze individual cytosine positions, or treated by enzymatic digestion (Xiong and Laird, Nucleic Acids Res., 25:2532-4, 1997 ). Detection by hybridization has also been described in the art (Olek et al., WO 99/28498 ). Additionally, use of the bisulfite technique for methylation detection with respect to individual genes has been described (Grigg and Clark, Bioessays, 16:431-6, 1994 ; Zeschnigk M, et al., Hum Mol Genet., 6:387-95, 1997 ; Feil R, et al., Nucleic Acids Res., 22:695-, 1994 ; Martin V, et al., Gene, 157:261-4, 1995 ; WO 9746705 and WO 9515373).

The present invention provides for the use of the bisulfite technique, in combination with one or more methylation assays, for determination of the methylation status of CpG dinucleotide sequences within SEQ ID NO: 1 and SEQ ID NO: 2. It is particularly preferred that CpG positions of the gene ALX4 within bases 42,700-52,000 of SEQ ID NO: I (or said positions within the equivalent bisulfite converted sequences) are analyzed. It is particularly preferred that CpG positions of the gene Septin 9 within bases 1,000-4,250 or 93,850-96,000 of SEQ ID NO: 2 (or said positions within the equivalent bisulfite converted sequences) are analysed. Genomic CpG dinucleotides can be methylated or unmethylated (alternatively known as up-and down- methylated respectively). However the methods of the present invention are suitable for the analysis of biological samples of a heterogeneous nature, e.g., a low concentration of colorectal cells within a background of blood or stool. Accordingly, when analyzing the methylation status of a CpG position within such a sample the person skilled in the art may use a quantitative assay for determining the level (e.g., percent, fraction, ratio, proportion or degree) of methylation at a particular CpG position as opposed to a methylation state. Accordingly the term methylation status or methylation state should also be taken to mean a value reflecting the degree of methylation at a CpG position. Unless specifically stated the terms "hypermethylated" or "upmethylated" shall be taken to mean a methylation level above that of a specified cut-off point, wherein said cut-off may be a value representing the average or median methylation level for a given population, or is preferably an optimized cut-off level. The "cut-off" is also referred herein as a "threshold". In the context of the present invention the terms "methylated", "hypermethylated" or "upmethylated" shall be taken to include a methylation level above the cut-off be zero (0) % (or equivalents thereof) methylation for all CpG positions within and associated with (e.g. in promoter or regulatory regions) the genes selected from the group consisting of Septin 9 (including all transcript variants thereof) and ALX4.

According to the present invention, determination of the methylation status of CpG dinucleotide sequences within SEQ ID NO: 1 TO SEQ ID NO: 2 has utility in the detection of colorectal lesions undergoing malignant transformation and thus in the early detection of colorectal cancers.

Methylation Assay Procedures. Various methylation assay procedures are known in the art, and can be used in conjunction with the present invention. These assays allow for determination of the methylation state of one or a plurality of CpG dinucleotides (e.g., CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992 ). Additionally, restriction enzyme digestion of PCR products amplified from bisulfite-converted DNA is used, e.g., the method described by Sadri and Hornsby (Nucl. Acids Res. 24:5058-5059, 1996), or COBRA (Combined Bisulfite Restriction Analysis) (Xiong and Laird, Nucleic Acids Res. 25:2532-2534, 1997 ).

COBRA. COBRA™ analysis is a quantitative methylation assay useful for determining DNA methylation levels at specific gene loci in small amounts of genomic DNA (Xiong and Laird, Nucleic Acids Res. 25:2532-2534, 1997 ). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from micro-dissected paraffin-embedded tissue samples.

Typical reagents (e.g., as might be found in a typical COBRA™-based kit) for COBRA™ analysis may include, but are not limited to: PCR primers for specific gene (or bisulfite treated DNA sequence or CpG island); restriction enzyme and appropriate buffer; gene-hybridization oligonucleotide; control hybridization oligonucleotide; kinase labeling kit for oligonucleotide probe; and labeled nucleotides. Additionally, bisulfite conversion reagents may include: DNA denaturation buffer; sulfonation buffer; DNA recovery reagents or kits (e.g., precipitation, ultrafiltration, affinity column); desulfonation buffer; and DNA recovery components.

Preferably, assays such as "MethyLight™" (a fluorescence-based real-time PCR technique) (Eads et al., Cancer Res. 59:2302-2306, 1999 ), Ms-SNuPE™ (Methylation-sensitive Single Nucleotide Primer Extension) reactions (Gonzalgo and Jones, Nucleic Acids Res. 25:2529-2531, 1997 ), methylation-specific PCR ("MSP"; Herman et al., Proc. Natl. Acad. Sci. USA 93:9821-9826, 1996 ; US Patent No. 5,786,146 ), and methylated CpG island amplification ("MCA"; Toyota et al., Cancer Res. 59:2307-12, 1999 ) are used alone or in combination with other of these methods.

The "HeavyMethyl™" assay, technique is a quantitative method for assessing methylation differences based on methylation specific amplification of bisulfite treated DNA. Methylation specific blocking probes (also referred to herein as blockers) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific blocking probes covering CpG positions between the amplification primers. The HeavyMethyl™ assay may also be used in combination with methylation specific amplification primers.

Typical reagents (e.g., as might be found in a typical MethyLight□-based kit) for HeavyMethyl™ analysis may include, but are not limited to: PCR primers for specific genes (or bisulfite treated DNA sequence or CpG island); blocking oligonucleotides; optimized PCR buffers and deoxynucleotides; and Taq polymerase.

MSP. MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996 ; US Patent No. 5,786,146 ). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (e.g., as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

MethyLight™. The MethyLight™ assay is a high-throughput quantitative methylation assay that utilizes fluorescence-based real-time PCR (TaqMap®) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed in a "biased" (with PCR primers that overlap known CpG dinucleotides) reaction. Sequence discrimination can occur both at the level of the amplification process and at the level of the fluorescence detection process.

The MethyLight™ assay may be used as a quantitative test for methylation patterns in the genomic DNA sample, wherein sequence discrimination occurs at the level of probe hybridization. In this quantitative version, the PCR reaction provides for a methylation specific amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The MethyLight™ process can by used with any suitable probes e.g. "TaqMan®", Lightcycler® etc.... For example, double-stranded genomic DNA is treated with sodium bisulfite and subjected to one of two sets of PCR reactions using TaqMan® probes; e.g., with MSP primers and/ or HeavyMethyl blocker oligonucleotides and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (e.g., as might be found in a typical MethyLight™-based kit) for MethyLight™ analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

The QM™ (quantitative methylation) assay is an alternative quantitative test for methylation patterns in genomic DNA samples, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The QM™ process can be used with any suitable probes e.g. "TaqMan®", Lightcycler® etc... in the amplification process. For example, double-stranded genomic DNA is treated with sodium bisulfite and subjected to unbiased primers and the TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system. Typical reagents (e.g., as might be found in a typical QM™ -based kit) for QM™ analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

Ms-SNuPE. The Ms-SNuPE™ technique is a quantitative method for assessing methylation differences at specific CpG sites based on bisulfite treatment of DNA, followed by single-nucleotide primer extension (Gonzalgo and Jones, Nucleic Acids Res. 25:2529-2531, 1997 ). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (e.g., micro-dissected pathology sections), and it avoids utilization of restriction enzymes for determining the methylation status at CpG sites.

Typical reagents (e.g., as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for specific gene; reaction buffer (for the Ms-SNuPE reaction); and labelled nucleotides. Additionally, bisulfite conversion reagents may include: DNA denaturation buffer; sulfonation buffer; DNA recovery regents or kit (e.g., precipitation, ultrafiltration, affinity column); desulfonation buffer; and DNA recovery components.

The genomic sequence according to SEQ ID NO: 1 to SEQ ID NO: 2, and non-naturally occurring treated variants thereof according to SEQ ID NO: 3 to SEQ ID NO: 10, were determined to have novel utility for the detection of malignant or pre-malignant colorectal adenomas and the differentiation of benign colorectal lesions from those undergoing malignant transformation and accordingly to be of use in the early detection of colorectal carcinomas.

In one embodiment the invention of the method comprises the following steps: i) contacting genomic DNA (preferably isolated from body fluids) obtained from the subject with at least one reagent, or series of reagents that distinguishes between methylated and non-methylated CpG dinucleotides within Septin 9 (including all transcript variants thereof) and ALX4 (including their promoter and regulatory regions); and ii) determining the presence or absence of pre-cancerous (i.e. malignant or pre-malignant) colon cellular proliferative disorders or distinguishing between or among benign and pre-cancerous colon cellular proliferative disorders. Preferably the methylation status of both Septin 9 (including any transcript variants thereof) and ALX4 are analyzed.

Genomic DNA may be isolated by any means standard in the art, including the use of commercially available kits. Briefly, wherein the DNA of interest is encapsulated by a cellular membrane the biological sample must be disrupted and lysed by enzymatic, chemical or mechanical means. The DNA solution may then be cleared of proteins and other contaminants, e.g., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising neoplastic matter are suitable for use in the present method, preferred are cell lines, histological slides, biopsies, paraffin-embedded tissue, body fluids, stool, colonic effluent, urine, blood plasma, blood serum, whole blood, isolated blood cells, cells isolated from the blood and combinations thereof. Body fluids are the preferred source of the DNA; particularly preferred are blood plasma, blood serum, whole blood, isolated blood cells and cells isolated from the blood.

The genomic DNA sample is then treated with at least one reagent, or series of reagents that distinguishes between methylated and non-methylated CpG dinucleotides within at least two target regions of the genomic DNA, wherein the target region comprise, or hybridizes under stringent conditions to a sequence of at least 16 contiguous nucleotides of SEQ ID NO: 1 and to a sequence of at least 16 contiguous nucleotides of SEQ ID NO: 2, wherein said contiguous nucleotides comprise at least one CpG dinucleotide sequence.

It is particularly preferred that said reagent converts cytosine bases which are unmethylated at the 5'-position to uracil, thymine, or another base which is dissimilar to cytosine in terms of hybridization behavior. However in an alternative embodiment said reagent may be a methylation sensitive restriction enzyme.

Wherein the genomic DNA sample is treated in such a manner that cytosine bases which are unmethylated at the 5'-position are converted to uracil, thymine, or another base which is dissimilar to cytosine in terms of hybridization behavior It is preferred that this treatment is carried out with bisulfite (hydrogen sulfite, disulfite) and subsequent alkaline hydrolysis. Such a treatment results in the conversion of SEQ ID NO: I TO SEQ ID NO: 2 to SEQ ID NO: 3-6, (respectively) wherein said CpG dinucleotides are methylated or SEQ ID NO: 7-10 wherein said CpG dinucleotides are unmethylated.

The treated DNA is then analyzed in order to determine the methylation state of the target gene sequences (at least two genes or genomic sequences selected of SEQ ID NO: 1 and SEQ ID NO: 2, respectively). It is particularly preferred that the target region comprises, or hybridizes under stringent conditions to at least 16 contiguous nucleotides of at least one gene or genomic sequence selected from the group consisting of SEQ ID NO: 1 to SEQ ID NO: 2. It is preferred that the sequence of said genes according to SEQ ID NO: 1 to SEQ ID NO: 2 are analyzed. The method of analysis may be selected from those known in the art, including those listed herein. Particularly preferred are MethyLight™, MSP and the use of blocking oligonucleotides (HeavyMethyl™) as described herein. It is further preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more of SEQ ID NO: 3 to SEQ ID NO: 10 and sequences complementary thereto.

Aberrant methylation, more specifically hypermethylation of the genes and genomic sequences thereof according to Table 1 selected from the group consisting of Septin 9 (including all transcript variants thereof) and/or ALX4 (including their promoter and/or regulatory regions) is associated with the presence of cancer. Preferably the methylation status of both Septin 9 (including any transcript variants thereof) and ALX4 are analyzed. Accordingly wherein a biological sample presents within any degree of methylation, said sample should be determined as having malignant potential.

The method may alternatively be enabled by means of any analysis of the expression of an RNA transcribed therefrom or polypeptide or protein translated from said RNA, preferably by means of mRNA expression analysis or polypeptide expression analysis. Accordingly the present invention also provides assays and methods, both quantitative and qualitative for detecting the expression of at least one gene or genomic sequence selected from the group consisting of Septin 9 (including all transcript variants thereof) and/or ALX4 in a subject and determining therefrom upon the presence or absence of a neoplastic colorectal cell proliferative disorder, or differentiating between a malignant or pre-malignant and benign colorectal cell proliferative disorder.

Aberrant expression of mRNA transcribed from the genes or genomic sequences selected from the group consisting of Septin 9 (including all transcript variants thereof) and/or ALX4 are associated with the malignant transformation of colorectal lesions. The expression of both Septin 9 (including any transcript variants thereof) and ALX4 may be analyzed. Underexpression (and/or methylation) is associated with the presence of malignant or pre-malignant colorectal cellular proliferative disorders, and over-expression (and/or absence of methylation) is associated with benign colorectal cellular proliferative disorders. It is particularly exemplified that the expression of at least one of the transcript variants of the genes Septin 9 and ALX4 is determined.

To detect the presence of mRNA encoding a gene or genomic sequence, a sample is obtained from a patient. The sample may be any suitable sample comprising cellular matter of the lesion. Suitable sample types include cell lines, histological slides, biopsies, paraffin-embedded tissue, body fluids, stool, colonic effluent, blood plasma, blood serum, whole blood, isolated blood cells, cells isolated from the blood and all possible combinations thereof. It is exemplified that said sample types are stool or body fluids selected from the group consisting colonic effluent, urine, blood plasma, blood serum, whole blood, isolated blood cells, cells isolated from the blood.

The sample may be treated to extract the RNA contained therein. The resulting nucleic acid from the sample is then analyzed. Many techniques are known in the state of the art for determining absolute and relative levels of gene expression, commonly used techniques suitable for use in the present invention include in situ hybridization (e.g., FISH), Northern analysis, RNase protection assays (RPA), microarrays and PCR-based techniques, such as quantitative PCR and differential display PCR or any other nucleic acid detection method.

Particularly exemplifiedis the use of the reverse transcription/polymerization chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligonucleotide dT primer and/or random hexamer primers. The cDNA thus produced is then amplified by means of PCR. (Belyavsky et al, Nucl Acid Res 17:2919-2932, 1989 ; Krug and Berger, Methods in Enzymology, Academic Press, N.Y., Vol.152, pp. 316-325,1987). Further preferred is the "Real-time" variant of RT- PCR, wherein the PCR product is detected by means of hybridization probes (e.g. TaqMan, Lightcycler, Molecular Beacons and Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridisation to a labelled probe in the dried gel itself or on a membrane. The resulting signal is proportional to the amount of target RNA in the RNA population.

Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labelled (e.g., radioactive labels, mass labels or fluorescent labels). The size of the target RNA, not the probe, will determine the size of the detected band, so methods such as random-primed labelling, which generates probes of variable lengths, are suitable for probe synthesis. The specific activity of the probe will determine the level of sensitivity, so it is preferred that probes with high specific activities, are used.

In an RNase protection assay, the RNA target and an RNA probe of a defined length are hybridised in solution. Following hybridisation, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (Ausubel et al. 2003), particularly preferred are probes with high specific activities.

Disclosed is the use of microarrays. The microarray analysis process can be divided into two main parts. First is the immobilization of known gene sequences onto glass slides or other solid support followed by hybridisation of the fluorescently labelled cDNA (comprising the sequences to be interrogated) to the known genes immobilized on the glass slide (or other solid phase). After hybridisation, arrays are scanned using a fluorescent microarray scanner. Analysing the relative fluorescent intensity of different genes provides a measure of the differences in gene expression.

DNA arrays can be generated by immobilizing presynthesized oligonucleotides onto prepared glass slides or other solid surfaces. In this case, representative gene sequences are manufactured and prepared using standard oligonucleotide synthesis and purification methods. These synthesized gene sequences are complementary to the RNA transcript(s) of the genes of interest (in this case the genes or genomic sequences selected from the group consisting of Septin 9 (including all transcript variants thereof) and ALX4) and tend to be shorter sequences in the range of 25-70 nucleotides. Said oligonucleotides or polynucleotides may comprise at least 9, 18 or 25 bases of a sequence complementary to or hybridising to at least the mRNA transcript and sequences complementary thereto. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks. Said synthesized nucleic acids may be locked nucleic acids.

In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labelling of the cDNA can be accomplished by either direct labelling or indirect labelling methods. During direct labelling, fluorescently modified nucleotides (e.g., Cy®3- or Cy®5-dCTP) are incorporated directly into the cDNA during the reverse transcription. Alternatively, indirect labelling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labelled with Cy®3. The resulting labelled cDNA is purified to remove unincorporated nucleotides, free dye and residual RNA. Following purification, the labelled cDNA samples are hybridised to the microarray. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2nd ed., 1989 ). The microarray is scanned post-hybridisation using a fluorescent microarray scanner. The fluorescent intensity of each spot indicates the level of expression of the analysed gene; bright spots correspond to strongly expressed genes, while dim spots indicate weak expression.

Once the images are obtained, the raw data must be analysed. First, the background fluorescence must be subtracted from the fluorescence of each spot. The data is then normalized to a control sequence, such as exogenously added nucleic acids (preferably RNA or DNA), or a housekeeping gene panel to account for any non-specific hybridisation, array imperfections or variability in the array set-up, cDNA labelling, hybridisation or washing. Data normalization allows the results of multiple arrays to be compared.

Another aspect of the disclosure relates to a kit for use in the detection of pre-cancerous colorectal cellular proliferative disorders or the differentiation between malignant/pre-malignant and benign colorectal lesions in a subject according to the methods of the present invention, said kit comprising: a means for measuring the level of transcription of genes or genomic sequences selected from the group consisting of Septin 9 (including all transcript variants thereof) and ALX4.

The means for measuring the level of transcription may comprise oligonucleotides or polynucleotides able to hybridise under stringent or moderately stringent conditions to the transcription products of a gene or genomic sequence selected from the group consisting of Septin 9 (including all transcript variants thereof) and ALX4. Said oligonucleotides or polynucleotides may be able to hybridise under stringent or moderately stringent conditions to at least one of the transcription products of a gene or genomic sequence selected from the group consisting of Septin 9 (including all transcript variants thereof) and/or ALX4. Said oligonucleotides or polynucleotides may comprise at least 9, 18 or 25 bases of a sequence complementary to or hybridising to at least one of said sequence or sequences complementary thereto.

The level of transcription may be determined by techniques selected from the group of Northern Blot analysis, reverse transcriptase PCR, real-time PCR, RNAse protection, and microarray. The kit further may comprise means for obtaining a biological sample of the patient. Exemplified is a kit, which further comprises a container which may be suitable for containing the means for measuring the level of transcription and the biological sample of the patient, and may further comprise instructions for use and interpretation of the kit results.

In an example the kit comprises (a) a plurality of oligonucleotides or polynucleotides able to hybridise under stringent or moderately stringent conditions to the transcription products of at least one gene or genomic sequence selected from the group consisting of Septin 9 (including all transcript variants thereof) and ALX4; (b) a container, preferably suitable for containing the oligonucleotides or polynucleotides and a biological sample of the patient comprising the transcription products wherein the oligonucleotides or polynucleotides can hybridise under stringent or moderately stringent conditions to the transcription products, (c) means to detect the hybridisation of (b); and optionally, (d) instructions for use and interpretation of the kit results. It is further preferred that said oligonucleotides or polynucleotides of (a) comprise in each case at least 9, 18 or 25 bases of a sequence complementary to or hybridising to the transcription products and sequences complementary thereto.

The kit may also contain other components such as hybridisation buffer (where the oligonucleotides are to be used as a probe) packaged in a separate container. Alternatively, where the oligonucleotides are to be used to amplify a target region, the kit may contain, packaged in separate containers, a polymerase and a reaction buffer optimised for primer extension mediated by the polymerase, such as PCR. Preferably said polymerase is a reverse transcriptase. It is further exemplified that said kit further contains an Rnase reagent.

The present disclosure further provides for methods for the detection of the presence of the polypeptide encoded by said gene sequences in a sample obtained from a patient.

Aberrant levels of polypeptide expression of the polypeptides encoded by the genes or genomic sequences selected from the group consisting of Septin 9 (including all transcript variants thereof) and/or ALX4 are associated with the presence of colorectal cellular proliferative disorders. Furthermore said aberrant levels of expression are of use in the differentiation between benign and malignant/pre-malignant colorectal lesions. According to the present disclosure, under expression of said polypeptides is associated with the presence of colorectal lesions undergoing malignant transformation.

Any method known in the art for detecting polypeptides can be used. Such methods include, but are not limited to mass-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton and Lange, Norwalk, Conn. pp 217-262, 1991). Exemplified are binder-ligand immunoassay methods including reacting antibodies with an epitope or epitopes and competitively displacing a labelled polypeptide or derivative thereof.

Certain embodiments of the present disclosure comprise the use of antibodies specific to the polypeptide encoded by a gene or genomic sequence selected from the group consisting of Septin 9 (including all transcript variants thereof) and ALX4.

Such antibodies are useful for the analysis of colorectal lesions. In certain embodiments production of monoclonal or polyclonal antibodies can be induced by the use of an epitope encoded by a polypeptide of the genes Septin 9 (including all transcript variants thereof) and/or ALX4 as an antigene. Such antibodies may in turn be used to detect expressed polypeptides as markers for the early detection of colorectal cancer. The levels of such polypeptides present may be quantified by conventional methods. Antibody-polypeptide binding may be detected and quantified by a variety of means known in the art, such as labelling with fluorescent or radioactive ligands. The disclosure further comprises kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used in agglutination tests, or labelled for use a wide variety of assay methods. Labels that can be used include radionuclides, enzymes, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

The proteins may also be detected by means of western blot analysis. Said analysis is standard in the art, briefly proteins are separated by means of electrophoresis, e.g., SDS-PAGE. The separated proteins are then transferred to a suitable membrane (or paper), e.g., nitrocellulose, retaining the spatial separation achieved by electrophoresis. The membrane is then incubated with a blocking agent to bind remaining sticky places on the membrane, commonly used agents include generic protein (e.g., milk protein). An antibody specific to the protein of interest is then added, said antibody being detectably labelled for example by dyes or enzymatic means (e.g., alkaline phosphatase or horseradish peroxidase). The location of the antibody on the membrane is then detected.

In an alternative embodiment of the method the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly, the primary antibody to be detected by binding to its specific antigen. The antibody-antigen complex is then bound by a secondary enzyme conjugated antibody. In the presence of the necessary substrate and chromogen the bound enzyme is detected according to coloured deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesising the amino acid sequence and injecting it into an appropriate animal, usually a rabbit or a mouse (Milstein and Kohler Nature 256:495-497, 1975 ; Gulfre and Milstein, Methods in Enzymology: Immunochemical Techniques 73:1-46, Langone and Banatis eds., Academic Press, 1981). Methods for preparation of the polypeptides or epitopes thereof include, but are not limited to chemical synthesis, recombinant DNA techniques or isolation from biological samples.

In the final step of the method the diagnosis of the patient is determined, whereby under-expression (of at least one gene or genomic sequence selected from the group consisting of Septin 9 (including all transcript variants thereof) and ALX4) is indicative of the presence of a colorectal lesion undergoing malignant transformation. Preferably the expression of both Septin 9 (including any transcript variants thereof) and ALX4 are analyzed. The term under-expression shall be taken to mean expression at a detected level less than a pre-determined cut off which may be selected from the group consisting of the mean, median or an optimised threshold value.

Another aspect of the disclosure provides a kit for use in the early detection of colorectal cancer and/or differentiation between malignant or pre-malignant and benign colorectal lesions in a subject according to the methods of the present invention, comprising: a means for detecting polypeptides at least one gene or genomic sequence selected from the group consisting of Septin 9 (including all transcript variants thereof) and ALX4. The means for detecting the polypeptides comprise preferably antibodies, antibody derivatives, or antibody fragments. The polypeptides are most preferably detected by means of Western Blotting utilizing a labelled antibody. In another embodiment of the disclosure the kit further comprising means for obtaining a biological sample of the patient. Preferred is a kit, which further comprises a container suitable for containing the means for detecting the polypeptides in the biological sample of the patient, and most preferably further comprises instructions for use and interpretation of the kit results. In a preferred embodiment the kit comprises: (a) a means for detecting polypeptides at least one gene or genomic sequence selected from the group consisting of Septin 9 (including all transcript variants thereof) and ALX4; (b) a container suitable for containing the said means and the biological sample of the patient comprising the polypeptides wherein the means can form complexes with the polypeptides; (c) a means to detect the complexes of (b); and optionally (d) instructions for use and interpretation of the kit results. The kit may also contain other components such as buffers or solutions suitable for blocking, washing or coating, packaged in a separate container.

Particular embodiments of the present invention provide a novel application of the analysis of methylation levels and/or patterns within said sequences that enables the early detection of colorectal cancers. Early detection of cancer is directly linked with disease prognosis, and the disclosed method thereby enables the physician and patient to make better and more informed treatment decisions.

FURTHER IMPROVEMENTS

The present invention provides novel uses for the genomic sequence SEQ ID NO: 1 to SEQ ID NO: 2. Additional examples provide modified variants of SEQ ID NO: 1 to SEQ ID NO: 2, as well as oligonucleotides and/or PNA-oligomers for analysis of cytosine methylation patterns within SEQ ID NO: 1 to SEQ ID NO: 2.

An objective of the invention comprises analysis of the methylation state of one or more CpG dinucleotides within at least one sequence selected form the group consisting of SEQ ID NO: 1 to SEQ ID NO: 2 and sequences complementary thereto.

The disclosure provides treated nucleic acids, derived from genomic SEQ ID NO: 1 to SEQ ID NO: 2, wherein the treatment is suitable to convert at least one unmethylated cytosine base of the genomic DNA sequence to uracil or another base that is detectably dissimilar to cytosine in terms of hybridization. The genomic sequences in question may comprise one, or more consecutive methylated CpG positions. Said treatment preferably comprises use of a reagent selected from the group consisting of bisulfite, hydrogen sulfite, disulfite, and combinations thereof. In a preferred embodiment of the disclosure, the disclosure provides a non-naturally occurring modified nucleic acid comprising a sequence of at least 16 contiguous nucleotide bases in length of a sequence selected from the group consisting of SEQ ID NO: 3 to SEQ ID NO: 10. In further preferred embodiments of the disclosure said nucleic acid is at least 50, 100, 150, 200, 250 or 500 base pairs in length of a segment of the nucleic acid sequence disclosed in SEQ ID NO: 3 to SEQ ID NO: 10. Particularly preferred is a nucleic acid molecule that is not identical or complementary to all or a portion of the sequences SEQ ID NO: 1 to SEQ ID NO: 2 or other naturally occurring DNA.

It is preferred that said sequence comprises at least one CpG, TpA or CpA dinucleotide and sequences complementary thereto. The sequences of SEQ ID NO: 3 to SEQ ID NO: 10 disclose non-naturally occurring modified versions of the nucleic acid according to SEQ ID NO: 1 to SEQ ID NO: 2, wherein the modification of each genomic sequence results in the synthesis of a nucleic acid having a sequence that is unique and distinct from said genomic sequence as follows. For each sense strand genomic DNA, e.g., SEQ ID NO:1, four converted versions are disclosed. A first version wherein "C" is converted to "T," but "CpG" remains "CpG" (i.e., corresponds to case where, for the genomic sequence, all "C" residues of CpG dinucleotide sequences are methylated and are thus not converted); a second version discloses the complement of the disclosed genomic DNA sequence (i.e. antisense strand), wherein "C" is converted to "T," but "CpG" remains "CpG" (i.e., corresponds to case where, for all "C" residues of CpG dinucleotide sequences are methylated and are thus not converted). The 'upmethylated' converted sequences of SEQ ID NO: I to SEQ ID NO: 2 correspond to SEQ ID NO: 3 to 6. A third chemically converted version of each genomic sequences is disclosed, wherein "C" is converted to "T" for all "C" residues, including those of "CpG" dinucleotide sequences (i.e., corresponds to case where, for the genomic sequences, all "C" residues of CpG dinucleotide sequences are unmethylated); a final chemically converted version of each sequence, discloses the complement of the disclosed genomic DNA sequence (i.e. antisense strand), wherein "C" is converted to "T" for all "C" residues, including those of "CpG" dinucleotide sequences (i.e., corresponds to case where, for the complement (antisense strand) of each genomic sequence, all "C" residues of CpG dinucleotide sequences are unmethylated). The 'downmethylated' converted sequences of SEQ ID NO: 1 to SEQ ID NO: 2 correspond to SEQ ID NO: 7 to 10.

Significantly, heretofore, the nucleic acid sequences and molecules according SEQ ID NO: 3 to SEQ ID NO: 10 were not implicated in or connected with the detection, classification or treatment of cellular proliferative disorders.

In an alternative preferred embodiment, the disclosure further provides oligonucleotides or oligomers suitable for use in the methods of the invention for detecting the cytosine methylation state within genomic or treated (chemically modified) DNA, according to SEQ ID NO: 1 to SEQ ID NO: 2, SEQ ID NO: 3 to SEQ ID NO: 10. Said oligonucleotide or oligomer nucleic acids provide diagnostic means. Said oligonucleotide or oligomer comprising a nucleic acid sequence having a length of at least nine (9) nucleotides which is identical to, hybridizes, under moderately stringent or stringent conditions (as defined herein above), to a treated nucleic acid sequence according to SEQ ID NO: 3 to SEQ ID NO: 10 and/or sequences complementary thereto, or to a genomic sequence according to SEQ ID NO: 1 to SEQ ID NO: 2, and/or sequences complementary thereto.

Thus, the present disclosure includes nucleic acid molecules (e.g., oligonucleotides and peptide nucleic acid (PNA) molecules (PNA-oligomers)) that hybridize under moderately stringent and/or stringent hybridization conditions to all or a portion of a sequence selected form the group consisting of SEQ ID NO: 1 to SEQ ID NO: 2, SEQ ID NO: 3 to SEQ ID NO: 10 or to the complements thereof. Particularly preferred is a nucleic acid molecule that hybridizes under moderately stringent and/or stringent hybridization conditions to all or a portion of the sequences SEQ ID NO: 3 to SEQ ID NO: 10 but not SEQ ID NO: 1 to SEQ ID NO: 2 or other human genomic DNA.

The identical or hybridizing portion of the hybridizing nucleic acids is typically at least 9, 16, 20, 25, 30 or 35 nucleotides in length. However, longer molecules have utility, and are thus within the scope of the present disclosure.

Preferably, the hybridizing portion of the inventive hybridizing nucleic acids is at least 95%, or at least 98%, or 100% identical to the sequence, or to a portion thereof of a sequence selected from the group consisting of SEQ ID NO: 1 to SEQ ID NO: 2, SEQ ID NO: 3 to SEQ ID NO: 10, or to the complements thereof.

Hybridizing nucleic acids of the type described herein can be used, for example, as a primer (e.g., a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO: 1 to SEQ ID NO: 2 (such as allelic variants and SNPs), rather than identical, it is useful to first establish the lowest temperature at which only homologous hybridization occurs with a particular concentration of salt (e.g., SSC or SSPE). Then, assuming that 1% mismatching results in a 1°C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of oligonucleotides of length X (in nucleotides), as indicated by polynucleotide positions with reference to, e.g., SEQ ID NO:1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions: n to n (n + (X-1)); where

$$n=1, 2, 3,...(Y-(X-1));$$

where Y equals the length (nucleotides or base pairs) of SEQ ID NO: 1 (52626);

where X equals the common length (in nucleotides) of each oligonucleotide in the set (e.g., X=20 for a set of consecutively overlapping 20-mers); and

where the number (Z) of consecutively overlapping oligomers of length X for a given SEQ ID NO of length Y is equal to Y- (X-1). For example Z= 52626-19 = 52607 for either sense or antisense sets of SEQ ID NO: 1, where X=20.

Preferably, the set is limited to those oligomers that comprise at least one CpG, TpG or CpA dinucleotide. Examples of inventive 20-mer oligonucleotides include the following set of oligomers (and the antisense set complementary thereto), indicated by polynucleotide positions with reference to SEQ ID NO:1:

1-20, 2-21, 3-22, 4-23, 5-24, ............. and 52607-52626.

Preferably, the set is limited to those oligomers that comprise at least one CpG, TpG or CpA dinucleotide.

Likewise, examples of 25-mer oligonucleotides include the following set of 219885 oligomers (and the antisense set complementary thereto), indicated by polynucleotide positions with reference to SEQ ID NO: 1:

1-25, 2-26, 3-27, 4-28, 5-29, ....., and 52602- 52626.

Preferably, the set is limited to those oligomers that comprise at least one CpG, TpG or CpA dinucleotide.

The present disclosure encompasses, for each of SEQ ID NO: 3 to SEQ ID NO: 10, SEQ ID NO: 1 to SEQ ID NO: 2 (sense and antisense), multiple consecutively overlapping sets of oligonucleotides or modified oligonucleotides of length X, where, e.g., X= 9, 10, 17, 20, 22, 23, 25, 27, 30 or 35 nucleotides.

The oligonucleotides or oligomers according to the present disclosure constitute effective tools useful to ascertain genetic and epigenetic parameters of the genomic sequences selected from the group consisting of SEQ ID NO: 1 to SEQ ID NO: 2. Preferred sets of such oligonucleotides or modified oligonucleotides of length X are those consecutively overlapping sets of oligomers corresponding to SEQ ID NO: 1 to SEQ ID NO: 2, SEQ ID NO: 3 to SEQ ID NO: 10 (and to the complements thereof). Preferably, said oligomers comprise at least one CpG, TpG or CpA dinucleotide.

Particularly preferred oligonucleotides or oligomers according to the present disclosure are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758 5,565,552 , 5,567,810 , 5,574,142 , 5,585,481 , 5,587,371 , 5,597,696 and 5,958,773 . The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988 ) or intercalating agents (Zon, Pharm. Res. 5:539-549, 1988 ). To this end, the oligonucleotide may be conjugated to another molecule, e.g., a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers according to particular embodiments of the present invention are typically used in 'sets,' which contain at least one oligomer for analysis of each of the CpG dinucleotides of a genomic sequence selected from the group consisting SEQ ID NO: I to SEQ ID NO: 2 and sequences complementary thereto, or to the corresponding CpG, TpG or CpA dinucleotide within a sequence of the treated nucleic acids according to SEQ ID NO: 3 to SEQ ID NO: 10 and sequences complementary thereto. However, it is anticipated that for economic or other factors it may be preferable to analyse a limited selection of the CpG dinucleotides within said sequences, and the content of the set of oligonucleotides is altered accordingly.

Therefore, in particular embodiments, the present disclosure provides a set of at least two (2) (oligonucleotides and/or PNA-oligomers) useful for detecting the cytosine methylation state in treated genomic DNA (SEQ ID NO: 3 to SEQ ID NO: 10), or in genomic DNA (SEQ ID NO: 1 to SEQ ID NO: 2 and sequences complementary thereto). These probes enable the differentiation of pre-cancerous (i.e. malignant or pre-malignant) colorectal lesions from benign colorectal lesions (commonly referred to as benign). The set of oligomers may also be used for detecting single nucleotide polymorphisms (SNPs) in treated genomic DNA (SEQ ID NO: 3 to SEQ ID NO: 10), or in genomic DNA (SEQ ID NO: 1 to SEQ ID NO: 2 and sequences complementary thereto).

In preferred embodiments, at least one, and more preferably all members of a set of oligonucleotides is bound to a solid phase.

In further embodiments, the present disclosure provides a set of at least two (2) oligonucleotides that are used as 'primer' oligonucleotides for amplifying DNA sequences of one of SEQ ID NO: 1 to SEQ ID NO: 2, SEQ ID NO: 3 to SEQ ID NO: 10 and sequences complementary thereto, or segments thereof.

It is anticipated that the oligonucleotides may constitute all or part of an "array" or "DNA chip" (i.e., an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminium, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labelled probes are often used for the scanning of immobilized DNA arrays. The simple attachment of Cy3 and Cy5 dyes to the 5'-OH of the specific probe are particularly suitable for fluorescence labels. The detection of the fluorescence of the hybridised probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003 ). In such methods, enough labels are generated so that each nucleic acid in the complex mixture (i.e., each analyte) can be uniquely bound by a unique label and thus detected (each label is directly counted, resulting in a digital read-out of each molecular species in the mixture).

It is particularly preferred that the oligomers according to the invention are utilized for the early detection of colorectal cancer by differentiating benign colorectal lesions from those undergoing malignant transformation.

In the most preferred embodiment of the method, the presence or absence of a colon lesion undergoing malignant transformation is determined and/or the differentiation between malignant/pre-malignant and benign lesions is made. This is achieved by analysis of the methylation status of at least two target sequences each comprising at least one CpG position said sequences comprising, or hybridizing under stringent conditions to at least 16 contiguous nucleotides of SEQ ID NO: 1 and SEQ ID NO: 2 and/or complements thereof. The present invention further provides a method for ascertaining genetic and/or epigenetic parameters of the genomic sequence according to SEQ ID NO: I to SEQ ID NO: 2 within a subject by analyzing cytosine methylation and single nucleotide polymorphisms. Said method comprising contacting nucleic acids comprising SEQ ID NO: I and SEQ ID NO: 2 in a biological sample obtained from said subject with at least one reagent or a series of reagents, wherein said reagent or series of reagents, distinguishes between methylated and non-methylated CpG dinucleotides within the target nucleic acid.

In a preferred embodiment, said method comprises the following steps: In the first step, a sample of the tissue to be analyzed is obtained. The source may be any suitable source, such as cell lines, histological slides, biopsies, paraffin-embedded tissue, body fluids, stool, colonic effluent, urine, blood plasma, blood serum, whole blood, isolated blood cells, cells isolated from the blood and all possible combinations thereof. It is preferred that said sources of DNA are stool or body fluids selected from the group consisting colonic effluent, blood plasma, blood serum, whole blood, isolated blood cells, cells isolated from the blood.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Wherein the sample DNA is not enclosed in a membrane (e.g. circulating DNA from a blood sample) methods standard in the art for the isolation and/or purification of DNA may be employed. Such methods include the use of a protein degenerating reagent e.g., chaotropic salt e.g. guanidine hydrochloride or urea; or a detergent e.g. sodium dodecyl sulphate (SDS), cyanogen bromide. Alternative methods include but are not limited to ethanol precipitation or propanol precipitation, vacuum concentration amongst others by means of a centrifuge. The person skilled in the art may also make use of devices such as filter devices, e.g., ultrafiltration, silica surfaces or membranes, magnetic particles, polystyrene particles, polystyrene surfaces, positively charged surfaces, and positively charged membranes, charged membranes, charged surfaces, charged switch membranes, charged switched surfaces.

Once the nucleic acids have been extracted, the genomic double stranded DNA is used in the analysis.

In the second step of the method, the genomic DNA sample is treated in such a manner that cytosine bases which are unmethylated at the 5'-position are converted to uracil, thymine, or another base which is dissimilar to cytosine in terms of hybridization behavior. This will be understood as 'pretreatment' or 'treatment' hereinafter.

This is preferably achieved by means of treatment with a bisulfite reagent. The term "bisulfite reagent" refers to a reagent comprising bisulfite, disulfite, hydrogen sulfite or combinations thereof, useful as disclosed herein to distinguish between methylated and unmethylated CpG dinucleotide sequences. Methods of said treatment are known in the art (e.g., WO2005038051). It is preferred that the bisulfite treatment is conducted in the presence of denaturing solvents such as but not limited to n-alkylenglycol, particularly diethylene glycol dimethyl ether (DME), or in the presence of dioxane or dioxane derivatives. In a preferred embodiment the denaturing solvents are used in concentrations between 1% and 35% (v/v). It is also preferred that the bisulfite reaction is carried out in the presence of scavengers such as but not limited to chromane derivatives, e.g., 6-hydroxy-2, 5,7,8, - tetramethylchromane 2-carboxylic acid or trihydroxybenzoe acid and derivates thereof, e.g., Gallic acid (see: WO2005038051). The bisulfite conversion is preferably carried out at a reaction temperature between 30°C and 70°C, whereby the temperature is increased to over 85°C for short periods of times during the reaction (see WO2005038051). The bisulfite treated DNA is preferably purified priori to the quantification. This may be conducted by any means known in the art, such as but not limited to ultrafiltration, preferably carried out by means of Microcon(™) columns (manufactured by Millipore(™). The purification is carried out according to a modified manufacturer's protocol (see: WO2005038051).

In the third step of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). Preferably said amplificates are 100 to 2,000 base pairs in length. The set of primer oligonucleotides includes at least two oligonucleotides whose sequences are each reverse complementary, identical, or hybridise under stringent or highly stringent conditions to an at least 16-base-pair long segment of the base sequences of one of SEQ ID NO: 3 TO SEQ ID NO: 10 and sequences complementary thereto.

In an alternate embodiment of the method, the methylation status of pre-selected CpG positions within SEQ ID NO: 1 and SEQ ID NO: 2 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridizes to a treated nucleic acid sequence according to one of SEQ ID NO: 3 TO SEQ ID NO: 10 and sequences complementary thereto, wherein the base sequence of said oligomers comprises at least one CpG dinucleotide .A further preferred embodiment of the method comprises the use of blocker oligonucleotides (the HeavyMethyl™ assay). The use of such blocker oligonucleotides has been described by Yu et al., BioTechniques 23:714-720, 1997 . Blocking probe oligonucleotides are hybridised to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said blocking oligonucleotides is required to comprise a sequence having a length of at least 9 nucleotides which hybridizes to a treated nucleic acid sequence according to one of SEQ ID NO: 3 to SEQ ID NO: 10 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labeled amplificates have a single positive or negative net charge, allowing for better delectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas and Hillenkamp, Anal Chem., 60:2299-301, 1988 ). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut and Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallization. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut and Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the fourth step of the method, the amplificates obtained during the third step of the method are analyzed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of based-based methods such as, but not limited to, array technology and probe based technologies as well as by means of techniques such as sequencing and template directed extension.

In one embodiment of the method, the amplificates synthesized in step three are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide. The hybridizing portion of the hybridizing nucleic acids is typically at least 9, 15, 20, 25, 30 or 35 nucleotides in length. However, longer molecules have inventive utility, and are thus within the scope of the present invention.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within a sequence selected from the group consisting SEQ ID NO: 1 to SEQ ID NO: 2, and the equivalent positions within SEQ ID NO: 3 to SEQ ID NO: 10.

Said oligonucleotides may also be present in the form of peptide nucleic acids. The non-hybridized amplificates are then removed. The hybridized amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes (as detailed above) that are hybridized to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996 ; also see United States Patent No. 6,331,393) employing a dual-labeled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a non-extendible interrogating oligonucleotide, called a TaqMan™ probe, which, in preferred embodiments, is designed to hybridize to a CpG-rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393 , also known as the MethyLightTM™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

In a further preferred embodiment of the method, the fourth step of the method comprises the use of template-directed oligonucleotide extension, such as MS-SNuPE as described by Gonzalgo and Jones, Nucleic Acids Res. 25:2529-2531, 1997.

In yet a further embodiment of the method, the fourth step of the method comprises sequencing and subsequent sequence analysis of the amplificate generated in the third step of the method (Sanger F., et al., Proc Natl Acad Sci USA 74:5463-5467, 1977).

Best mode

In the most preferred embodiment of the method the genomic nucleic acids are isolated and treated according to the first three steps of the method outlined above, namely:

a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or blocking oligonucleotides, and further wherein
e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID NO: 3 to SEQ ID NO: 10 and sequences complementary thereto, wherein the base sequence of said oligomers comprise at least one CpG dinucleotide.

Step e) of the method, namely the detection of the specific amplificates indicative of the methylation status of one or more CpG positions of at least one sequences of the group comprising SEQ ID NO: I to SEQ ID NO: 2 is carried out by means of real-time detection methods as described above.

Additional embodiments of the disclosure provide a method for the analysis of the methylation status of genomic DNA according to the invention (SEQ ID NO: I to SEQ ID NO: 2, and complements thereof) without the need for bisulfite conversion. Methods are known in the art wherein a methylation sensitive restriction enzyme reagent, or a series of restriction enzyme reagents comprising methylation sensitive restriction enzyme reagents that distinguishes between methylated and non-methylated CpG dinucleotides within a target region are utilized in determining methylation, for example but not limited to DMH.

In the first step of such additional embodiments, the genomic DNA sample is isolated from tissue or cellular sources. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, e.g., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising neoplastic or potentially neoplastic matter are suitable for us e in the present method, preferred are cell lines, histological slides, biopsies, paraffin-embedded tissue, body fluids, stool, colonic effluent, urine, blood plasma, blood serum, whole blood, isolated blood cells, cells isolated from the blood and combinations thereof. Body fluids are the preferred source of the DNA; particularly preferred are blood plasma, blood serum, whole blood, isolated blood cells and cells isolated from the blood.

Once the nucleic acids have been extracted, the genomic double-stranded DNA is used in the analysis.

In a preferred embodiment, the DNA may be cleaved prior to treatment with methylation sensitive restriction enzymes. Such methods are known in the art and may include both physical and enzymatic means. Particularly preferred is the use of one or a plurality of restriction enzymes which are not methylation sensitive, and whose recognition sites are AT rich and do not comprise CG dinucleotides. The use of such enzymes enables the conservation of CpG islands and CpG rich regions in the fragmented DNA. The non-methylation-specific restriction enzymes are preferably selected from the group consisting of MseI, BfaI, Csp6I, TruII, TvuII, Tru9I, Tvu9I, MaeI and XspI. Particularly preferred is the use of two or three such enzymes. Particularly preferred is the use of a combination of MseI, BfaI and Csp6I.

The fragmented DNA may then be ligated to adaptor oligonucleotides in order to facilitate subsequent enzymatic amplification. The ligation of oligonucleotides to blunt and sticky ended DNA fragments is known in the art, and is carried out by means of dephosphorylation of the ends (e.g. using calf or shrimp alkaline phosphatase) and subsequent ligation using ligase enzymes (e.g. T4 DNA ligase) in the presence of dATPs. The adaptor oligonucleotides are typically at least 18 base pairs in length.

In the third step, the DNA (or fragments thereof) is then digested with one or more methylation sensitive restriction enzymes. The digestion is carried out such that hydrolysis of the DNA at the restriction site is informative of the methylation status of a specific CpG dinucleotide of at least one gene or genomic sequence selected from the group consisting of Septin 9 (including all transcript variants thereof) and ALX4. Preferably the methylation status of both Septin 9 (including any transcript variants thereof) and ALX4 are analyzed.

Preferably, the methylation-specific restriction enzyme is selected from the group consisting of Bsi EI, Hga I HinPI, Hpy991, Ava I, Bce AI, Bsa HI, BisI, BstUI, BshI236I, AccII, BstFNI, McrBC, GlaI, MvnI, HpaII (HapII), HhaI, AciI, SmaI, HinPII, HpyCH4IV, EagI and mixtures of two or more of the above enzymes. Preferred is a mixture containing the restriction enzymes BstUI, HpaII, HpyCH4IV and HinPII.

In the fourth step, which is optional but a preferred embodiment, the restriction fragments are amplified. This is preferably carried out using a polymerase chain reaction, and said amplificates may carry suitable detectable labels as discussed above, namely fluorophore labels, radionuclides and mass labels. Particularly preferred is amplification by means of an amplification enzyme and at least two primers comprising, in each case a contiguous sequence at least 16 nucleotides in length that is complementary to, or hybridizes under moderately stringent or stringent conditions to a sequence selected from the group consisting SEQ ID NO: 1 TO SEQ ID NO: 2, and complements thereof. Preferably said contiguous sequence is at least 16, 20 or 25 nucleotides in length. In an alternative embodiment said primers may be complementary to any adaptors linked to the fragments.

In the fifth step the amplificates are detected. The detection may be by any means standard in the art, for example, but not limited to, gel electrophoresis analysis, hybridization analysis, incorporation of detectable tags within the PCR products, DNA array analysis, MALDI or ESI analysis. Preferably said detection is carried out by hybridization to at least one nucleic acid or peptide nucleic acid comprising in each case a contiguous sequence at least 16 nucleotides in length that is complementary to, or hybridizes under moderately stringent or stringent conditions to a sequence selected from the group consisting SEQ ID NO: I to SEQ ID NO: 2, and complements thereof. Preferably said contiguous sequence is at least 16, 20 or 25 nucleotides in length.

Subsequent to the determination of the methylation state or level of the genomic nucleic acids the class of cellular proliferative disorder (benign or malignant) is deduced based upon the methylation state or level of at least one CpG dinucleotide sequence of at least one sequence selected from the group consisting SEQ ID NO: 1 to SEQ ID NO: 2, or an average, or a value reflecting an average methylation state of a plurality of CpG dinucleotide sequences of at least one sequence selected from the group consisting SEQ ID NO: 1 to SEQ ID NO: 2 wherein methylation is associated with a colorectal lesion undergoing malignant transformation. Wherein said methylation is determined by quantitative means the cut-off point for determining said the presence of methylation is preferably zero (i.e. wherein a sample displays any degree of methylation it is determined as having a methylated status at the analyzed CpG position). Nonetheless, it is foreseen that the person skilled in the art may wish to adjust said cut-off value in order to provide an assay of a particularly preferred sensitivity or specificity. Accordingly said cut-off value may be increased (thus increasing the specificity), said cut off value may be within a range selected form the group consisting of 0%-5%, 5%-10%, 10%-15%, 15%-20%, 20%-30% and 30%-50%. Particularly preferred are the cut-offs 10%, 15%, 25%, and 30%.

Prognostic Assays for cellular proliferative disorders

The present invention enables diagnosis of events which are disadvantageous to patients or individuals in which important genetic and/or epigenetic parameters within Septin 9 (including all transcript variants thereof) and ALX4 may be used as markers. Said parameters obtained by means of the present invention may be compared to another set of genetic and/or epigenetic parameters, the differences serving as the basis for a diagnosis and/or prognosis of events which are disadvantageous to patients or individuals.

More specifically the present invention enables the screening of at-risk populations for the early detection of cancers, most preferably colorectal carcinomas. Furthermore, the present invention enables the differentiation of malignant or pre-malignant lesions from those which are likely to remain benign (i.e. non-cancerous).

Specifically, the present invention provides for colorectal neoplasm detection assays based on measurement of methylation of one or more CpG dinucleotide sequences of eachSEQ ID NO: 1 and SEQ ID NO: 2 that comprise such a CpG dinucleotide sequence. Typically, such assays involve obtaining a sample from a subject, performing an assay to measure the expression of Septin 9 (including all transcript variants thereof) and ALX4, by determining the methylation status of SEQ ID NO: 1 and SEQ ID NO: 2, derived from the sample, relative to a control sample, or a known standard and making a diagnosis based thereon. It is particularly preferred that the methylation status of both Septin 9 (including any transcript variants thereof) and ALX4 are analyzed.

In particular preferred embodiments, inventive oligomers are used to assess the CpG dinucleotide methylation status, such as those based on SEQ ID NO: 1 to SEQ ID NO: 2, SEQ ID NO: 3 to SEQ ID NO: 10, or arrays thereof, as well as in kits based thereon and useful for the classification of colorectal cell proliferative disorders.

Kits

Moreover, an additional aspect of the present disclosure is a kit comprising: a means for determining methylation of at least one gene selected from the group consisting of Septin 9 (including all transcript variants thereof) and ALX4. The means for determining methylation comprise preferably a bisulfite-containing reagent; one or a plurality of oligonucleotides consisting whose sequences in each case are identical, are complementary, or hybridise under stringent or highly stringent conditions to a 9 or more preferably up to 18 base long segment of a sequence selected from SEQ ID NO: 3 to SEQ ID NO: 10; and optionally instructions for carrying out and evaluating the described method of methylation analysis. In one embodiment the base sequence of said oligonucleotides comprises at least one CpG, CpA or TpG dinucleotide.

In a further embodiment, said kit may further comprise standard reagents for performing a CpG position-specific methylation analysis, wherein said analysis comprises one or more of the following techniques: MS-SNuPE, MSP, MethyLight™, HeavyMethyl, COBRA, and nucleic acid sequencing. However, a kit along the lines of the present invention can also contain only part of the aforementioned components.

In a preferred embodiment the kit may comprise additional bisulfite conversion reagents selected from the group consisting: DNA denaturation buffer; sulfonation buffer; DNA recovery reagents or kits (e.g., precipitation, ultrafiltration, affinity column); desulfonation buffer; and DNA recovery components.

The kit may alternatively contain, packaged in separate containers, a polymerase and a reaction buffer optimised for primer extension mediated by the polymerase, such as PCR. In another embodiment of the invention the kit further comprising means for obtaining a biological sample of the patient. Exemplified is a kit, which further comprises a container suitable for containing the means for determining methylation of at least one gene or genomic sequence selected from the group consisting of Septin 9 (including all transcript variants thereof) and ALX4 in the biological sample of the patient, and most preferably further comprises instructions for use and interpretation of the kit results. The kit may comprise: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably up to 18 base long segment of a sequence selected from SEQ ID NO: 3 to SEQ ID NO: 10; and optionally (d) instructions for use and interpretation of the kit results. Alternatively, the kit may comprise: (a) a bisulfite reagent; (b) a container suitable for containing the said bisulfite reagent and the biological sample of the patient; (c) at least one oligonucleotides and/or PNA-oligomer having a length of at least 9 or 16 nucleotides which is identical to or hybridises to a pre-treated nucleic acid sequence according to one of SEQ ID NO: 3 to SEQ ID NO: 10 and sequences complementary thereto; and optionally (d) instructions for use and interpretation of the kit results.

The kit may alternatively comprise: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NO: 3 to SEQ ID NO: 10; (d) at least one oligonucleotides and/or PNA-oligomer having a length of at least 9 or 16 nucleotides which is identical to or hybridises to a pre-treated nucleic acid sequence according to one of SEQ ID NO: 3 to SEQ ID NO: 10 and sequences complementary thereto; and optionally (e) instructions for use and interpretation of the kit results.

The kit may also contain other components such as buffers or solutions suitable for blocking, washing or coating, packaged in a separate container.

Typical reagents (e.g., as might be found in a typical COBRA™-based kit) for COBRA™ analysis may include, but are not limited to: PCR primers for at least one gene selected from the group consisting of Septin 9 (including all transcript variants thereof) and ALX4; restriction enzyme and appropriate buffer; gene-hybridization oligo; control hybridization oligo; kinase labeling kit for oligo probe; and labeled nucleotides. Typical reagents (e.g., as might be found in a typical MethyLight™ - based kit) for MethyLight™ analysis may include, but are not limited to: PCR primers for the bisulfite converted sequence of at least one gene or genomic sequence selected from the group consisting of Septin 9 (including all transcript variants thereof) and ALX4; bisulfite specific probes (e.g., TaqMan ™ or Lightcycler™); optimized PCR buffers and deoxynucleotides; and Taq polymerase.

Typical reagents (e.g., as might be found in a typical Ms-SNuPE™-based kit) for Ms-SNuPE™ analysis may include, but are not limited to: PCR primers for specific gene (or bisulfite treated DNA sequence or CpG island); optimized PCR buffers and deoxynucleotides; gel extraction kit; positive control primers; Ms-SNuPE™ primers for the bisulfite converted sequence of at least one gene or genomic sequence selected from the group consisting of Septin 9 (including all transcript variants thereof) and ALX4; reaction buffer (for the Ms-SNuPE reaction); and labeled nucleotides.

Typical reagents (e.g., as might be found in a typical MSP-based kit) for MSP analysis may include, but are not limited to: methylated and unmethylated PCR primers for the bisulfite converted sequence of or genomic sequence selected from the group consisting of Septin 9 (including all transcript variants thereof) and ALX4, optimized PCR buffers and deoxynucleotides, and specific probes.

Moreover, an additional aspect of the present disclosure is an alternative kit comprising a means for determining methylation of at least one gene or genomic sequence selected from the group consisting of Septin 9 (including all transcript variants thereof) and ALX4, wherein said means comprise preferably at least one methylation specific restriction enzyme; one or a plurality of primer oligonucleotides (preferably one or a plurality of primer pairs) suitable for the amplification of a sequence comprising at least one CpG dinucleotide of a sequence selected from SEQ ID NO: 1 to SEQ ID NO: 2; and optionally instructions for carrying out and evaluating the described method of methylation analysis. In one embodiment the base sequence of said oligonucleotides are identical, are complementary, or hybridise under stringent or highly stringent conditions to an at least 18 base long segment of a sequence selected from SEQ ID NO: I to SEQ ID NO: 2.

Said kit may further comprise one or a plurality of oligonucleotide probes for the analysis of the digest fragments, preferably said oligonucleotides are identical, are complementary, or hybridise under stringent or highly stringent conditions to an at least 16 base long segment of a sequence selected from SEQ ID NO: 1 to SEQ ID NO: 2.

The kit may comprise additional reagents selected from the group consisting: buffer (e.g., restriction enzyme, PCR, storage or washing buffers); DNA recovery reagents or kits (e.g., precipitation, ultrafiltration, affinity column) and DNA recovery components.

The kit may contain, packaged in separate containers, a polymerase and a reaction buffer optimized for primer extension mediated by the polymerase, such as PCR. The kit may further comprise means for obtaining a biological sample of the patient. In an example the kit comprises: (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridise under stringent or highly stringent conditions to an at least 9 base long segment of a sequence selected from SEQ ID NO: 1 to SEQ ID NO: 2; and optionally (d) instructions for use and interpretation of the kit results.

The kit may alternatively comprise: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from SEQ ID NO: 1 to SEQ ID NO: 2; and optionally (d) instructions for use and interpretation of the kit results.

The kit may alternatively comprise: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence selected from SEQ ID NO: 1 to SEQ ID NO: 2; (d) at least one set of oligonucleotides one or a plurality of nucleic acids or peptide nucleic acids which are identical , are complementary, or hybridize under stringent or highly stringent conditions to an at least 9 base long segment of a sequence selected from SEQ ID NO: I to SEQ ID NO: 2 and optionally (e) instructions for use and interpretation of the kit results.

The kit may also contain other components such as buffers or solutions suitable for blocking, washing or coating, packaged in a separate container.

The disclosure further relates to a kit for use in detecting and/or providing a classification of colorectal lesions in a subject by means of methylation-sensitive restriction enzyme analysis. Said kit comprises a container and a DNA microarray component. Said DNA microarray component being a surface upon which a plurality of oligonucleotides are immobilized at designated positions and wherein the oligonucleotide comprises at least one CpG methylation site. At least one of said oligonucleotides is specific for the at least one gene or genomic sequence selected from the group consisting of Septin 9 (including all transcript variants thereof) and/or ALX4 and comprises a sequence of at least 15 base pairs in length but no more than 200 bp of a sequence according to one of SEQ ID NO: 1 to SEQ ID NO: 2. Preferably said sequence is at least 15 base pairs in length but no more than 80 bp of a sequence according to one of SEQ ID NO: 1 to SEQ ID NO: 2. It is further preferred that said sequence is at least 20 base pairs in length but no more than 30 bp of a sequence according to one of SEQ ID NO: 1 to SEQ ID NO: 2.

Said test kit preferably further comprises a restriction enzyme component comprising one or a plurality of methylation-sensitive restriction enzymes.

Said test kit may be further characterized in that it comprises at least one methylation-specific restriction enzyme, and wherein the oligonucleotides comprise a restriction site of said at least one methylation specific restriction enzymes.

The kit may further comprise one or several of the following components, which are known in the art for DNA enrichment: a protein component, said protein binding selectively to methylated DNA; a triplex-forming nucleic acid component, one or a plurality of linkers, optionally in a suitable solution; substances or solutions for performing a ligation e.g. ligases, buffers; substances or solutions for performing a column chromatography; substances or solutions for performing an immunology based enrichment (e.g. immunoprecipitation); substances or solutions for performing a nucleic acid amplification e.g. PCR; a dye or several dyes, if applicable with a coupling reagent, if applicable in a solution; substances or solutions for performing a hybridization; and/or substances or solutions for performing a washing step.

The disclosure further provides a composition of matter useful for the classification of colorectal lesions. Said composition comprising at least one nucleic acid 18 base pairs in length of a segment of the nucleic acid sequence disclosed in SEQ ID NO: 3 to SEQ ID NO: 10, and one or more substances taken from the group comprising: 1-5 mM Magnesium Chloride, 100-500 μM dNTP, 0.5-5 units of taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO: 3 to SEQ ID NO: 10 and sequences complementary thereto. It is preferred that said composition of matter comprises a buffer solution appropriate for the stabilization of said nucleic acid in an aqueous solution and enabling polymerase based reactions within said solution.. Suitable buffers are known in the art and commercially available.

Said at least one nucleic acid may be at least 50, 100, 150, 200, 250 or 500 base pairs in length of a segment of the nucleic acid sequence disclosed in SEQ ID NO: 3 to SEQ ID NO: 10.

**Example 1**

In the following study, the methylation status of plasma samples derived from patients with varying type and stages of colorectal lesions was analysed in order to confirm lesion specific methylation within the genes Septin 9 and ALX4.

The assays were MSP and HeavyMethyl assays as described above, methylation specific real-time assays for the analysis of bisulfite converted DNA. The assays were designed to be run on the LightCycler platform (Roche Diagnostics), but other such instruments commonly used in the art are also suitable. MSP and HeavyMethyl amplificates were designed to be detected by means of Lightcycler style dual probes. Each assay was run in triplicate on plasma samples obtained from a commercial provider.

**Study population**

Samples were collected from male and female patients ages 40 to 80 but predominantly ages 50 and older. Case report forms were reviewed by a physician and severity of disease determined. In total, serum was collected from 36 patients with adenomatous polyps (13 female, 23 male, median age 63.5, range 24-75) and 13 patients with hyperplastic polyps (5 female, 8 male, median age 58, range 20-73). Serum was also collected from 22 individuals undergoing colonoscopy for various other reasons without neoplasia or preneoplasia of the colon and rectum (negative control) as well as 5 patients with colorectal carcinoma.

Of the polyp serum samples 7 samples were taken from patients with polyps >1cm with dysplasia, 9 samples from polyps >1cm without dysplasia, 17 samples from polyps <1cm without dysplasia, 3 samples from polyps <1cm with dysplasia, 13 samples from hyperplastic polyps of which 11 were from polyps <1cm and 2 were from polyps >2cm. The colorectal carcinoma samples were obtained from Stage 1 (4 samples) and stage 3 (1 sample) patients.

**Plasma methylation analysis**

Plasma was processed first by extraction of free-circulating DNA using the Total Nucleic Acid DNA extraction kit (Roche Applied Science) and Roche MagNaPure device. Eluted DNA's from each patient were pooled and concentrated on microcon filters. DNA Methylation information was preserved by deamination of unmethylated cytosines using sodium bisulfite as described above. Bisulfite treated plasma DNA in each sample was quantified on the Roche LC 2.0™ device using a non-methylation specific assay for beta-actin. ALX4 methylation was determined by means of the MSP assay. Septin 9 methylation was determined using an assay based on the applicant's HM real-time PCR technology (see Cottrell SE, Distler J, et al.. NAR 2004; 32(1):e10). The 90% limit of detection of the *Septin* 9 assay was estimated as 21pg by a dilution series of methylated (SSS1 treated) DNA in a background of 50ng blood DNA (Roche human genomic DNA). The Roche LC 2.0 was also used to measure Septin 9 amplification. A plasma equivalent of 1.6 ml to 1.9 ml of DNA was added per PCR reaction and each plasma sample run in duplicate or triplicate.

**Assays**

Genomic region of interest: SEQ ID NO: 1; Assay type: MSP

Primers: cgtcgcaacgcgtacg (SEQ ID NO: 11); cgcggtttcgattttaatgc (SEQ ID NO: 12)

Lightcycler probes: actccgacttaacccgacgatcg - fluo (SEQ ID NO: 13); LC 640-acgaaattcctaacgcaaccgct-p (SEQ ID NO: 14)

Amplificate sequence:

Cgtcgcaacgcgtacgactcaaaacttaataactccgacttaacccgacgatcgcgacgaaattccta acgcaaccgcttaaaacttcgcattaaaatcgaaaccgcg (SEQ ID NO: 15)

Temperature cycling program:

| | | | |
|---|---|---|---|
| activation: | 95°C | 10min | |
| 55 cycles: | 95°C | 15min | (20°C/s) |
| | 60°C | 45sec | (20°C/s) |
| | 72°C | 15sec | (20°C/s) |

Genomic region of interest: SEQ ID NO: 2; Assay type: HeavyMethyl
Primers: GtAGtAGttAGtttAGtAtttAttTT (SEQ ID NO: 16); CCCACCAaCCATCATaT (SEQ ID NO: 17)
Lightcycler probes: Gtt cga aat gat ttt att tag ttg c-FL (SEQ ID NO: 18); LC-Red640-cgt tga tcg egg ggt tc-PH (SEQ ID NO: 19)
Blocker sequence: CATCATaTCAaACCCCACAaTCAACACACAaC-Inv 3' (SEQ ID NO: 20)
Temperature cycling program:

| | | |
|---|---|---|
| Activation: | 95°C | 10min |
| 55 cycles: | 95°C | 10 sec |
| | 56°C | 30 sec |
| | 72°C | 10 sec |
| Cooling: | 10° C | 30 sec |

**Results**

A replicate was determined as positive if it presented with greater than 4% methylation, a sample was determined as positive according to how many of the replicates presented methylation.

| Single gene analysis | | | | | |
|---|---|---|---|---|---|
| Septin 9 | | | | | |
| | | 2/3 positive | | 1/3 positive | |
| | Total sample | # | % | # | % |
| Normal | 22 | 1 | 4.5 | 4 | 18 |
| Polyp | 49 | 6 | 12 | 17 | 35 |
| CRC | 5 | 2 | 40 | 2 | 40 |
| | | | | | |
| ALX4 | | | | | |
| | | 2/3 positive | | 1/3 positive | |
| | Total sample | # | % | # | % |
| Normal | 22 | 4 | 18 | 14 | 64 |
| Polyp | 49 | 23 | 47 | 38 | 77.5 |
| CRC | 5 | 2 | 40 | 4 | 80 |

| Panel analysis (Septin 9 + ALX4) | | | | | |
|---|---|---|---|---|---|
| | | 2/3 positive | | 3/6 positive | |

(continued)

| Panel analysis (Septin 9 + ALX4) | | | | | |
|---|---|---|---|---|---|
| | Total # samples | # | % | # | % |
| Normal | 22 | 4 | 18 | 2 | 9 |
| Polyp | 49 | 26 | 53 | 24 | 49 |
| CRC | 5 | 3 | 60 | 3 | 60 |

| According to polyp histology | | | | | | | | | | |
|---|---|---|---|---|---|---|---|---|---|---|
| | | Slept 2/3 in 9 | | ALX 2/3 4 | | Septin 9 + ALX4 2/3 | | Septin 9 + ALX4 3/6 | | |
| Polyp Characteristics | # samples | # | % | # | % | # | % | # | % | |
| >or = 1cm | 18 | 3 | 17 | 10 | 55 | 13 | 72 | 12 | 67 | |
| <1cm | 31 | 3 | 10 | 13 | 42 | 13 | 42 | 12 | 12 39 | |
| Tubular or villous adenoma | 36 | 5 | 14 | 17 | 47 | 20 | 55 | 20 | 55 | |
| Tubular or villous adenoma +>1cm | 11 | 3 | 27 | 8 | 73 | 11 | 100 | 11 | 100 | |
| Hyperplastic polyps | 13 | 1 | 8 | 6 | 46 | 6 | 46 | 4 | 31 | |

Furthermore, of the 10 adenomas with intraepithelial lesions 80% displayed methylation in at least 3 out of 6 replicates of Septin 9 and ALX4.

**Conclusions**

It was determined that the sensitivity of a real-time PCR assay for detection of polyps larger than 1cm was 23%. Our results indicate that the *Septin* 9 biomarker is also highly specific (95%) in asymptomatic individuals over 50 years of age.

The results of combining Septin 9 and ALX4 in a marker panel indicate that sensitivity for detecting polyps can be considerably improved while maintaining high specificity. The marker panel detected polyps larger than 1cm with a sensitivity of 67%. Sensitivity for large adenomas (> 1cm) or adenomas with IEN showed even greater improvement (100%, 80% respectively). Specificity of the panel in asymptomatic individuals over 50 years of age was 91%. Patient compliance and performance of current screening strategies limit the effectiveness of tests available on the market today. An easily administered blood-based test for early detection of colorectal cancer followed by colonoscopy for positive individuals has the potential to be a very effective tool for reducing mortality from this disease.

Table 1

| Genomic SEQ ID NO: | Ensembl database genomic location | Associated gene transcript (s)* | Methylated bisulfite converted sequence (sense) | Methylated bisulfite converted sequence (antisense) | Unmethylated bisulfite converted sequence (sense) | Unmethylated bisulfite converted sequence (antisense) |
|---|---|---|---|---|---|---|
| 1 | Chromosome 11:44238570:44291195:1 | ALX4 | 3 | 4 | 7 | 8 |
| 2 | Chromosome 17:72786362:73008270:1 | (Septin 9 | 5 | 6 | 9 | 10 |

## Claims

1. A method for detecting pre-cancerous colorectal cell proliferative disorders or for differentiating between pre-cancerous and benign cellular proliferative disorders in a subject comprising determining the presence or absence of CpG methylation of ALX 4 and Septin 9 in a biological sample selected from the group consisting of whole blood, blood plasma and blood serum isolated from said subject wherein CpG hypermethylation as compared to a control sample is indicative of the presence of pre-cancerous colorectal cell proliferative disorders.

2. A method according to claim 1, wherein a pre-cancerous cell proliferative disorder is distinguished from a benign cell proliferative disorder said method **characterized in that** the presence of CpG hypermethylation is indicative of the presence of a pre-cancerous cell proliferative disorder.

3. The method of claim 1 further comprising:

   a) contacting genomic DNA isolated from a biological sample selected from the group consisting of whole blood, blood plasma and blood serum obtained from said subject with at least one reagent, or series of reagents that distinguishes between methylated and non-methylated CpG dinucleotides within at least two target regions of the genomic DNA, wherein said target regions comprise, or hybridize under stringent conditions to a sequence of at least 16 contiguous nucleotides of SEQ ID NO: 1 and a sequence of at least 16 contiguous nucleotides of SEQ ID NO: 2, wherein said contiguous nucleotides comprise at least one CpG dinucleotide sequence, and whereby detecting pre-cancerous colorectal cell proliferative disorders or differentiating between pre-cancerous and benign cellular proliferative disorders is, at least in part, afforded.
   or
   b)

      (i) extracting or otherwise isolating genomic DNA from a biological sample selected from the group consisting of whole blood, blood plasma and blood serum obtained from the subject;
      (ii) treating the genomic DNA of a), or a fragment thereof, with one or more reagents to convert cytosine bases that are unmethylated in the 5-position thereof to uracil or to another base that is detectably dissimilar to cytosine in terms of hybridization properties;
      (iii) contacting the treated genomic DNA, or the treated fragment thereof, with an amplification enzyme and at least one primer comprising, a contiguous sequence of at least 9 nucleotides that is complementary to, or hybridizes under moderately stringent or stringent conditions to a sequence selected from the group consisting of SEQ ID NO: 3, 4, 7 and 8 and complements thereof, and at least one primer comprising, a contiguous sequence of at least 9 nucleotides that is complementary to, or hybridizes under moderately stringent or stringent conditions to a sequence selected from the group consisting of SEQ ID NO: 5, 6, 9 and 10 and complements thereof, wherein the treated genomic DNA or the fragment thereof is either amplified to produce at least two amplificates, or is not amplified; and
(iv) determining, based on a presence or absence of, or on a property of said amplificates, the methylation state or level of at least one CpG dinucleotide of a sequence according to SEQ ID NOs: 1 and 2, or an average, or a value reflecting an average methylation state or level of a plurality of CpG dinucleotides of sequences according to SEQ ID NOs: 1 and 2, whereby at least one of detecting pre-cancerous colorectal cellular proliferative disorders disorders or differentiating between pre-cancerous and benign cellular proliferative disorders is, at least in part, afforded.

**4.** The method of claim 3, wherein treating the genomic DNA, or the fragment thereof in (ii), comprises use of a reagent selected from the group comprising of bisulfite, hydrogen sulfite, disulfite, and combinations thereof and/or wherein contacting or amplifying in (iii) comprises use of at least one method selected from the group comprising: use of a heat-resistant DNA polymerase as the amplification enzyme; use of a polymerase lacking 5’-3’ exonuclease activity; use of a polymerase chain reaction (PCR); generation of an amplificate nucleic acid molecule carrying a detectable label and/or wherein determining in (iv) comprises hybridization of at least one nucleic acid molecule or peptide nucleic acid molecule in each case comprising a contiguous sequence at least 9 nucleotides in length that is complementary to, or hybridizes under moderately stringent or stringent conditions stringent conditions to a sequence of at least 16 contiguous nucleotides of at least SEQ ID NO: 1 and at least one nucleic acid molecule or peptide nucleic acid molecule in each case comprising a contiguous sequence at least 9 nucleotides in length that is complementary to, or hybridizes under moderately stringent or stringent conditions stringent conditions to a sequence of at least 16 contiguous nucleotides of at least SEQ ID NO: 2, wherein said contiguous nucleotides comprise at least one CpG

**5.** The method of claim 3 or 4, further comprising in step (iv) of embodiment b) the use of at least one nucleic acid molecule or peptide nucleic acid molecule comprising in each case a contiguous sequence at least 9 nucleotides in length that is complementary to, or hybridizes under moderately stringent or stringent conditions to a sequence selected from the group consisting of SEQ ID NO: 3, 4, 7 and 8 and complements thereof and at least one nucleic acid molecule or peptide nucleic acid molecule comprising in each case a contiguous sequence at least 9 nucleotides in length that is complementary to, or hybridizes under moderately stringent or stringent conditions to a sequence selected from the group consisting of SEQ ID NO: 5, 6, 9 and 10 and complements thereof, wherein said nucleic acid molecule or peptide nucleic acid molecule suppresses amplification of the nucleic acid to which it is hybridized.

**6.** The method of claim 1, further comprising:

a) extracting or otherwise isolating genomic DNA from a biological sample selected from the group consisting of whole blood, blood plasma and blood serum obtained from the subject,
b) digesting the genomic DNA of a), or a fragment thereof, with one or more methylation sensitive restriction enzymes, contacting the DNA restriction enzyme digest of b), with an amplification enzyme and at least two primers suitable for the amplification of a sequence comprising at least one CpG dinucleotide of a sequence according to SEQ ID NO: 1 and at least two primers suitable for the amplification of a sequence comprising at least one CpG dinucleotide of a sequence according to SEQ ID N0: 2, and
c) determining, based on a presence or absence of an amplificate the methylation state or level of at least one CpG dinucleotide of a sequence according to SEQ ID NO: 1 and a sequence according to SEQ ID NO: 2, whereby at least one of detecting pre-cancerous proliferative disorders or differentiating between pre-cancerous and benign cellular proliferative disorders is, at least in part, afforded.

**7.** The method according to claim 6 wherein the presence or absence of an amplificate is determined by means of hybridization to at least one nucleic acid or peptide nucleic acid which is identical, complementary, or hybridizes under stringent or highly stringent conditions to an at least 16 base long segment of a sequence according to SEQ ID NO: 1 and at least one nucleic acid or peptide nucleic acid which is identical, complementary, or hybridizes under stringent or highly stringent conditions to an at least 16 base long segment of a sequence according to SEQ ID NO: 2.

**8.** Use of a treated nucleic acid derived from genomic SEQ ID NO: 1 and a treated nucleic acid derived from genomic SEQ ID NO: 2, wherein the treatment is suitable to convert at least one unmethylated cytosine base of the genomic DNA sequence to uracil or another base that is detectably dissimilar to cytosine in terms of hybridization in a biological sample selected from the group consisting of whole blood, blood plasma and blood serum, the nucleic acid for detecting pre-cancerous colorectal cellular proliferative disorders or for differentiating between precancerous and benign cellular proliferative disorders.

**9.** Use of two nucleic acids in a biological sample selected from the group consisting of whole blood, blood plasma and blood serum, the nucleic acids comprising at least

a) 16 contiguous nucleotides comprising at least one CPG dinucleotide, of a treated genomic DNA sequence selected from the group consisting of SEQ ID NO: 3, 4, 7 and 8 and sequences complementary thereto and 16 contiguous nucleotides comprising at least one CPG dinucleotide, of a treated genomic DNA sequence selected from the group consisting of SEQ ID NO: 5, 6, 9 and 10 and sequences complementary thereto or
b) at least 50 contiguous nucleotides comprising at least one CPG dinucleotide, of a DNA sequence selected from the group consisting of SEQ ID NO: 3, 4, 7 and 8 and sequences complementary thereto and at least 50 contiguous nucleotides comprising at least one CPG dinucleotide, of a DNA sequence selected from the group consisting of SEQ ID NO: 5, 6, 9 and 10 and sequences complementary thereto

wherein the contiguous base sequence optionally comprises at least one CpG, TpG or CpA dinucleotide sequence for detecting pre-cancerous colorectal cellular proliferative disorders or for differentiating between pre-cancerous and benign cellular proliferative disorders.

**Patentansprüche**

**1.** Verfahren zum Nachweisen präkanzeröser Kolorektalzellproliferationserkrankungen oder zur Differenzierung zwischen präkanzerösen und gutartigen Zellproliferationserkrankungen in einem Individuum, bei dem die Anwesenheit oder Abwesenheit von CpG-Methylierung von ALX 4 und Septin 9 in einer biologischen Probe bestimmt wird, die ausgewählt ist aus der Gruppe bestehend aus von dem Individuum isoliertem Vollblut, Blutplasma und Blutserum, wobei CpG-Hypermethylierung im Vergleich zu einer Kontrollprobe auf das Vorhandensein von präkanzerösen Kolorektalzellproliferationserkrankungen hinweist.

**2.** Verfahren nach Anspruch 1, bei dem eine präkanzeröse Zellproliferationserkrankung von einer gutartigen Zellproliferationserkrankung unterschieden wird, wobei das Verfahren **dadurch gekennzeichnet ist, dass** die Anwesenheit von CpG-Hypermethylierung auf das Vorliegen einer präkanzerösen Zellproliferationserkrankungen hinweist.

**3.** Verfahren nach Anspruch 1, das weiterhin folgende Schritte umfasst:

a) Inkontaktbringen genomischer DNA, die aus einer biologischen Probe isoliert wird, die ausgewählt ist aus der Gruppe bestehend aus Vollblut, Blutplasma und Blutserum, welches von dem Individuum mit mindestens einem Reagenz oder einer Reihe von Reagenzien gewonnen wird, die zwischen methylierten und nicht-methylierten CpG-Dinukleotiden innerhalb von mindestens zwei Zielregionen der genomischen DNA unterscheiden, wobei diese Zielregionen eine Sequenz von mindestens 16 zusammenhängenden Nukleotiden der SEQ ID NO: 1 und eine Sequenz von mindestens 16 zusammenhängenden Nukleotiden der SEQ ID NO: 2 umfassen, oder an diese unter stringenten Bedingungen hybridisieren, wobei diese zusammenhängenden Nukleotide mindestens eine CpG-Dinukleotidsequenz umfassen, und wodurch der Nachweisen von präkanzerösen Kolorektalzellproliferationserkrankungen oder die Differenzierung zwischen präkanzerösen und gutartigen Zellproliferationserkrankungen zumindest teilweise ermöglicht wird.
oder
b)

i. Extrahieren oder anderweitiges Isolieren genomischer DNA aus einer biologischen Probe, die ausgewählt ist aus der Gruppe bestehend aus von dem Individuum gewonnenem Vollblut, Blutplasma und Blutserum;
ii. Behandeln der genomischen DNA aus a), oder eines Fragments davon, mit einem oder mehreren Reagenzien, um Cytosinbasen, die in ihrer 5-Position unmethyliert sind, in Uracil oder in eine andere Base umzuwandeln, die hinsichtlich ihrer Hybridisierungseigenschaften ungleich Cytosin nachweisbar ist;
iii. Inkontaktbringen der behandelten genomischen DNA, oder des behandelten Fragments davon, mit einem Amplifikationsenzym und mindestens einem Primer, der eine zusammenhängende Sequenz von mindestens 9 Nukleotiden umfasst, die zu einer Sequenz ausgewählt aus der Gruppe bestehend aus SEQ ID NO: 3, 4, 7 und 8 und Komplementen davon komplementär ist oder an diese unter mäßig stringenten oder stringenten Bedingungen hybridisiert, und mindestens einem Primer, der eine zusammenhängende Sequenz von mindestens 9 Nukleotiden umfasst, die zu einer Sequenz ausgewählt aus der Gruppe bestehend aus SEQ ID NO: 5, 6, 9 und 10 und Komplementen davon komplementär ist oder an diese unter mäßig stringenten oder stringenten Bedingungen hybridisiert, wobei die behandelte genomische DNA oder das Fragment davon entweder amplifiziert wird, um mindestens zwei Amplifikate zu erzeugen, oder nicht amplifiziert wird; und

iv. Bestimmen, basierend auf der Anwesenheit oder Abwesenheit von Amplifikaten oder auf einer Eigenschaft der Amplifikate, des Methylierungsstatus oder -niveaus von mindestens einem CpG-Dinukleotid von einer Sequenz gemäß SEQ ID NOs: 1 und 2 oder eines Mittelwerts oder eines Werts der einen durchschnittlichen Methylierungsstatus oder -niveau einer Vielzahl von CpG-Dinukleotiden von Sequenzen gemäß SEQ ID NOs: 1 und 2 widerspiegelt, wobei mindestens eines von einem Nachweisen von präkanzerösen Kolorektalzellproliferationserkrankungen oder der Differenzierung zwischen präkanzerösen und gutartigen Zellproliferationserkrankungen zumindest teilweise ermöglicht wird.

**4.** Verfahren nach Anspruch 3, bei dem das Behandeln der genomischen DNA oder des Fragments davon in (ii) die Verwendung eines Reagenz umfasst, das ausgewählt ist aus der Gruppe bestehend aus Bisulfit, Hydrogensulfit, Disulfit und Kombinationen davon, und/ oder bei dem das Inkontaktbringen oder Amplifizieren in (iii) mindestens ein Verfahren umfasst, das ausgewählt ist aus der Gruppe bestehend aus: Verwendung einer hitzebeständigen DNA-Polymerase als Amplifikationsenzym; Verwendung einer Polymerase ohne 5'-3'-Exonukleaseaktivität; Verwendung einer Polymerasekettenreaktion (PCR); Erzeugung eines Amplifikat-Nukleinsäuremoleküls mit einem detektierbaren Label, und/ oder bei dem das Bestimmen in (iv) die Hybridisierung von mindestens einem Nukleinsäuremolekül oder Peptid-Nukleinsäuremolekül umfasst, das jeweils eine zusammenhängende Sequenz von mindestens 9 Nukleotiden Länge umfasst, welche zu einer Sequenz von mindestens 16 zusammenhängenden Nukleotiden von mindestens SEQ ID NO: 1 komplementär ist oder unter mäßig stringenten oder stringenten Bedingungen an diese Sequenz hybridisiert, und mindestens ein Nukleinsäuremolekül oder Peptid-Nukleinsäuremolekül das jeweils eine zusammenhängende Sequenz von mindestens 9 Nukleotiden Länge umfasst, die zu einer Sequenz von mindestens 16 zusammenhängenden Nukleotiden von mindestens SEQ ID NO: 2 komplementär ist oder unter mäßig stringenten oder stringenten Bedingungen an diese Sequenz hybridisiert, wobei diese zusammenhängenden Nucleotide mindestens ein CpG umfassen.

**5.** Verfahren nach Anspruch 3 oder 4, ferner umfassend in Schritt (iv) der Ausführungsform b) die Verwendung von mindestens einem Nukleinsäuremolekül oder Peptid-Nukleinsäuremolekül, das jeweils eine zusammenhängende Sequenz von mindestens 9 Nukleotiden Länge umfasst, welche zu einer aus der Gruppe bestehend aus SEQ ID NO: 3, 4, 7 und 8 und Komplementen davon ausgewählten Sequenz komplementär ist oder an diese unter mäßig stringenten oder stringenten Bedingungen hybridisiert, und von mindestens einem Nukleinsäuremolekül oder Peptid-Nukleinsäuremolekül, das jeweils eine zusammenhängende Sequenz von mindestens 9 Nukleotiden Länge umfasst, welche zu einer aus der Gruppe bestehend aus SEQ ID NO: 5, 6, 9 und 10 und Komplementen davon ausgewählten Sequenz komplementär ist oder an diese unter mäßig stringenten oder stringenten Bedingungen hybridisiert, wobei dieses Nukleinsäuremolekül oder Peptid-Nukleinsäuremolekül die Amplifizierung derjenigen Nukleinsäure, an die es hybridisiert ist, unterdrückt.

**6.** Verfahren nach Anspruch 1, das ferner die folgenden Schritte umfasst:

a) Extrahieren oder anderweitiges Isolieren genomischer DNA aus einer biologischen Probe, die ausgewählt ist aus der Gruppe bestehend aus von dem Individuum gewonnenem Vollblut, Blutplasma und Blutserum;

b) Verdauen der genomischen DNA aus a), oder eines Fragments davon, mit einem oder mehreren methylierungssensitiven Restriktionsenzymen, Inkontaktbringen des DNA-Restuktionsenzyms-Verdaus aus b) mit einem Amplifikationsenzym und mindestens zwei Primern, die für die Amplifikation einer Sequenz geeignet sind, die mindestens ein CpG-Dinukleotid einer Sequenz gemäß SEQ ID NO: 1 umfasst, und mindestens zwei Primer, die für die Amplifikation einer Sequenz geeignet sind, die mindestens ein CpG-Dinukleotid einer Sequenz gemäß SEQ ID NO: 2 umfasst,
und

c) Bestimmen, basierend auf der Anwesenheit oder Abwesenheit eines Amplifikats, des Methylierungsstatus oder -niveaus von mindestens einem CpG-Dinukleotid von einer Sequenz gemäß SEQ ID NO: 1 und einer Sequenz gemäß SEQ ID NO: 2, wobei mindestens eines von einem Nachweisen von präkanzerösen Zellproliferationserkrankungen oder der Differenzierung zwischen präkanzerösen und gutartigen Zellproliferationserkrankungen zumindest teilweise ermöglicht wird.

**7.** Verfahren nach Anspruch 6, wobei die Anwesenheit oder Abwesenheit eines Amplifikats mittels Hybridisierung an mindestens eine Nukleinsäure oder Peptid-Nukleinsäure bestimmt wird, die zu einem mindestens 16 Basen langen Segment einer Sequenz gemäß SEQ ID NO: 1 identisch ist, komplementär ist, oder unter stringenten oder hochstringenten Bedingungen an dieses hybridisiert, und an mindestens eine Nukleinsäure oder Peptid-Nukleinsäure, die zu einem mindestens 16 Basen langen Segment einer Sequenz gemäß SEQ ID NO: 2 identisch ist, komplementär ist, oder unter stringenten oder hochstringenten Bedingungen an dieses hybridisiert.

**8.** Verwendung einer behandelten Nukleinsäure, die von genomischer SEQ ID NO: 1 abgeleitet ist, und einer behandelten Nukleinsäure, die von genomischer SEQ ID NO: 2 abgeleitet ist, wobei die Behandlung geeignet ist, um mindestens eine nicht-methylierte Cytosinbase der genomischen DNA-Sequenz in Uracil oder eine andere Base umzuwandeln, die in einer biologischen Probe hinsichtlich ihrer Hybridisierungseigenschaften ungleich Cytosin nachweisbar ist, wobei die biologische Probe ausgewählt ist aus der Gruppe bestehend aus Vollblut, Blutplasma und Blutserum, um präkanzeröse Kolorektalzellproliferationserkrankungen nachzuweisen oder zwischen präkanzerösen und gutartigen Zellproliferationserkrankungen zu differenzieren.

**9.** Verwendung von zwei Nukleinsäuren in einer biologische Probe, die ausgewählt ist aus der Gruppe bestehend aus Vollblut, Blutplasma und Blutserum, wobei die Nukleinsäuren mindestens Folgendes umfassen:

a) 16 zusammenhängende Nucleotide umfassend mindestens ein CpG-Dinukleotid, von einer behandelten genomischen DNA-Sequenz, die aus der Gruppe bestehend aus SEQ ID NO: 3, 4, 7 und 8 und dazu komplementären Sequenzen ausgewählt ist, und 16 zusammenhängende Nukleotide umfassend mindestens ein CpG-Dinukleotid von einer behandelten genomischen DNA-Sequenz, die aus der Gruppe bestehend aus SEQ ID NO: 5, 6, 9 und 10 und dazu komplementären Sequenzen ausgewählt ist,
oder
b) mindestens 50 zusammenhängende Nukleotide umfassend mindestens ein CpG-Dinukleotid von einer DNA-Sequenz, die aus der Gruppe bestehend aus SEQ ID NO: 3, 4, 7 und 8 und dazu komplementären Sequenzen ausgewählt ist, und mindestens 50 zusammenhängende Nukleotide umfassend mindestens ein CpG-Dinukleotid von einer DNA-Sequenz, die aus der Gruppe bestehend aus SEQ ID NO: 5, 6, 9 und 10 und dazu komplementären Sequenzen ausgewählt ist,

wobei die zusammenhängende Basensequenz gegebenenfalls mindestens eine CpG-, TpG- oder CpA-Dinukleotid-Sequenz aufweist, um präkanzeröse Kolorektalzellproliferationserkrankungen nachzuweisen oder zwischen präkanzerösen und gutartigen Zellproliferationserkrankungen zu differenzieren.

## Revendications

**1.** Procédé pour la détection de troubles prolifératifs de cellulaires colorectanx pré-cancéreux ou pour la différenciation entre troubles prolifératifs cellulaires précancéreux et bénins chez un sujet, comprenant la détermination de la présence ou de l'absence de la méthylation des CpG de ALX 4 et de Septine 9 dans un échantillon biologique choisi dans le groupe consistant en sang total, plasma sanguin et sérum sanguin isolé à partir dudit sujet, dans lequel l'hyperméthylation des CpG telle que comparée à un échantillon témoin est indicative de la présence de troubles prolifératifs cellulaires colorectanx pré-cancéreux.

**2.** Procédé selon la revendication 1, dans lequel un trouble prolifératif cellulaire pré-cancéreux est distingué d'un trouble prolifératif cellulaire bénin, ledit procédé étant **caractérisé en ce que** la présence d'une l'hyperméthylation des CpG est indicative de la présence d'un trouble prolifératif cellulaire pré-cancéreux.

**3.** Procédé selon la revendication 1, comprenant en outre:

a) la mise en contact d'ADN génomique isolé à partir d'un échantillon biologique choisi dans le groupe consistant en sang total, plasma sanguin et sérum sanguin obtenus à partir dudit sujet avec au moins un réactif, ou une série de réactifs qui font la distinction entre dinucléotides CpG méthylés et non-méthylés dans au moins deux régions cibles de l'ADN génomique, tandis que lesdites régions cibles comprennent, ou hybrident dans des conditions stringentes à une séquence d'au moins 16 nucléotides contigus de SEQ ID NO: 1 et une séquence d'au moins 16 nucléotides contigus de SEQ ID NO: 2, tandis que lesdits nucléotides contigus comprennent au moins une séquence de dinucléotide CpG, et ce par quoi la détection de troubles prolifératifs cellulaires colorectaux pré-cancéreux ou la différenciation entre des troubles prolifératifs cellulaires pré-cancéreux et bénins est procurée, au moins en partie,
ou
b)

(i) l'extraction ou l'isolement d'une autre manière d'ADN génomique à partir d'un échantillon biologique choisi dans le groupe consistant en sang total, plasma sanguin et sérum sanguin obtenus à partir du sujet;
(ii) Le traitement de l'ADN génomique selon a), ou d'un fragment de celui-ci, avec un ou plusieurs réactifs pour convertir les bases cytosine qui sont non-méthylées dans leur position 5 en uracile ou en une autre base qui à la détection diffère de la cytosine en termes de propriétés d'hybridation;
(iii) la mise en contact de l'ADN génomique traité, ou du fragment traité de celui-ci, avec une enzyme d'amplification et au moins une amorce comprenant, une séquence contiguë d'au moins 9 nucléotides qui est complémentaire de, ou hybride dans des conditions modérément stringentes ou stringentes à une séquence choisie dans le groupe consistant en SEQ ID NO: 3, 4, 7 et 8 et leurs compléments, et au moins une amorce comprenant une séquence contiguë d'au moins 9 nucléotides qui est complémentaire de, ou hybride dans des conditions modérément stringentes ou stringentes à une séquence choisie dans le groupe consistant en SEQ ID NO: 5, 6, 9 et 10 et leurs compléments, tandis que l'ADN génomique traité ou son fragment est soit amplifié pour produire au moins deux produits d'amplification, ou pas amplifié; et
(iv) la détermination, sur la base de la présence ou de l'absence de, ou d'une propriété desdits produits d'amplification, de l'état ou du niveau de méthylation d'au moins un dinucléotide CpG d'une séquence selon SEQ ID NOs: 1 et 2, ou d'une moyenne, ou d'une valeur reflétant un état ou niveau de méthylation moyen d'une pluralité de dinucléotides CpG de séquences selon SEQ ID NOs: 1 et 2, ce par quoi au moins l'une parmi la détection de troubles prolifératifs cellulaires colorectaux pré-cancéreux ou la différenciation entre des troubles prolifératifs cellulaires pré-cancéreux et bénins est procurée, au moins en partie.

**4.** Procédé selon la revendication 3, dans lequel le traitement de l'ADN génomique, ou le fragment de celui-ci selon (ii), comprend l'utilisation d'un réactif choisi dans le groupe comprenant bisulfite, hydrogéno bisulfite, disulfite, et leurs combinaisons et/ou dans lequel la mise en contact ou l'amplification dans (iii) comprend l'utilisation d'au moins un procédé choisi dans le groupe comprenant: l'utilisation d'une ADN-polymérase résistante à la chaleur comme enzyme d'amplification; l'utilisation d'une polymérase dénuée de l'activité de 5'-3' exonucléase; l'utilisation d'une réaction en chaîne par polymérase (ACP); la production d'une molécule d'acide nucléique comme produit d'amplification, portant un marqueur détectable et/ou dans laquelle la détermination dans (iv) comprend l'hybridation d'au moins une molécule d'acide nucléique ou molécule d'acide nucléique de peptide dans chaque cas comprenant une séquence contiguë d'au moins 9 nucléotides de long qui est complémentaire de, ou hybride dans des conditions modérément stringentes ou stringentes à une séquence d'au moins 16 nucléotides contigus d'au moins SEQ ID NO: 1 et au moins une molécule d'acide nucléique ou une molécule d'acide nucléique de peptide dans chaque cas comprenant une séquence contiguë ayant au moins 9 nucléotides de long, qui est complémentaire de, ou hybride dans des conditions modérément stringentes ou stringentes à une séquence d'au moins 16 nucléotides contigus d'au moins SEQ ID NO: 2, tandis que lesdits nucléotides contigus comprennent au moins un CpG.

**5.** Procédé selon la revendication 3 ou 4, comprenant en outre dans l'étape (iv) du moch de réalisation b) l'utilisation d'au moins une molécule d'acide nucléique ou molécule d'acide nucléique de peptide comprenant dans chaque cas une séquence contiguë d'au moins 9 nucléotides de long qui est complémentaire de, ou hybride dans des conditions modérément stringentes ou stringentes à une séquence choisie dans le groupe consistant en SEQ ID NO: 3, 4, 7 et 8 et leurs compléments, et au moins une molécule d'acide nucléique ou molécule d'acide nucléique de peptide comprenant dans chaque cas une séquence contiguë d'au moins 9 nucléotides de long qui est complémentaire de, ou hybride dans des conditions modérément stringentes ou stringentes à une séquence choisie dans le groupe consistant en SEQ ID NO: 5, 6, 9 et 10 et leurs compléments, tandis que ladite molécule d'acide nucléique ou molécule d'acide nucléique de peptide supprime l'amplification de l'acide nucléique auquel elle est hybridée.

**6.** Procédé selon la revendication 1, comprenant en outre:

a) l'extraction ou l'isolement d'une autre manière d'ADN génomique provenant d'un échantillon biologique choisi dans le groupe consistant en sang total, plasma sanguin et sérum sanguin obtenus à partir du sujet,
b) la digestion de l'ADN génomique de a), ou d'un fragment de celui-ci, avec une ou plusieurs enzymes de restriction sensibles à la méthylation, la mise en contact du produit de digestion de l'enzyme de restriction d'ADN de b), avec une enzyme d'amplification et au moins deux amorces appropriées pour l'amplification d'une séquence comprenant au moins un dinucléotide CpG d'une séquence selon SEQ ID NO: 1 et au moins deux amorces appropriées pour l'amplification d'une séquence comprenant au moins un dinucléotide CpG d'une séquence selon SEQ ID NO: 2, et

c) la détermination, sur la base de la présence ou de l'absence d'un produit d'amplification de l'état ou du niveau de méthylation d'au moins un dinucléotide CpG d'une séquence selon SEQ ID NO: 1 et d'une séquence selon SEQ ID NO: 2, ce par quoi au moins l'une parmi la détection de troubles prolifératifs pré-cancéreux ou la différenciation entre des troubles prolifératifs cellulaires pré-cancéreux et bénins est procurée, au moins en partie.

**7.** Procédé selon la revendication 6, dans lequel la présence ou l'absence d'un produit d'amplification est déterminée au moyen de l'hybridation à au moins un acide nucléique ou un acide nucléique de peptide qui est identique, complémentaire, ou hybride dans des conditions stringentes ou fortement stringentes à un segment d'au moins 16 bases de long d'une séquence selon SEQ ID NO: 1, et au moins un acide nucléique ou un acide nucléique de peptide qui est identique, complémentaire, ou hybride dans des conditions stringentes ou fortement stringentes à un segment d'au moins 16 bases de long d'une séquence selon SEQ ID NO: 2.

**8.** Utilisation d'un acide nucléique traité dérivé de SEQ ID NO: 1 génomique et d'un acide nucléique traité dérivé de SEQ ID NO: 2 génomique, dans laquelle le traitement est approprié pour la conversion d'au moins une base cytosine non-méthylée de la séquence d'ADN génomique en uracile ou une autre base qui à la détection diffère de la cytosine en termes d'hybridation dans un échantillon biologique qui est choisi dans le groupe consistant en sang total, plasma sanguin et sérum sanguin, l'acide nucléique étant destiné à la détection de troubles prolifératifs cellulaires colorectaux pré-cancéreux ou pour la différenciation entre des troubles prolifératifs cellulaires pré-cancéreux et bénins.

**9.** Utilisation de deux acides nucléiques dans un échantillon biologique choisi dans le groupe consistant en sang total, plasma sanguin et sérum sanguin, les acides nucléiques comprenant au moins

a) 16 nucléotides contigus comprenant au moins un dinucléotide CpG, d'une séquence d'ADN génomique traité choisie dans le groupe consistant en SEQ ID NO: 3, 4, 7 et 8 et des séquences complémentaires de celles-ci, et 16 nucléotides contigus comprenant au moins un dinucléotide CpG, d'une séquence d'ADN génomique traité choisie dans le groupe consistant en SEQ ID NO: 5, 6, 9 et 10 et des séquences complémentaires de celles-ci ou

b) au moins 50 nucléotides contigus comprenant au moins un dinucléotide CpG, d'une séquence d'ADN choisie dans le groupe consistant en SEQ ID NO: 3, 4, 7 et 8 et des séquences complémentaires de celles-ci, et au moins 50 nucléotides contigus comprenant au moins un dinucléotide CpG, d'une séquence d'ADN choisie dans le groupe consistant en SEQ ID NO: 5, 6, 9 et 10 et des séquences complémentaires de celles-ci,

tandis que la séquence de bases contiguës comprend en option au moins une séquence dinucléotidique CpG, TpG ou CpA pour la détection de troubles prolifératifs cellulaires colorectaux pré-cancéreux ou pour la différenciation entre des troubles prolifératifs cellulaires pré-cancéreux et bénins.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005001141 A
- WO 2005001140 A
- WO 2004035803 A
- US 5786146 A
- WO 0026401 A1
- WO 9500669 A
- US 6251594 B
- WO 9928498 A, Olek
- WO 9746705 A
- WO 9515373 A
- US 5514758 A
- US 5565552 A
- US 5567810 A
- US 5574142 A
- US 5585481 A
- US 5587371 A
- US 5597696 A
- US 5958773 A
- US 20030013091 A
- US 09898743 B
- WO 2005038051 A
- US 6265171 B, Herman
- US 6331393 B


### Non-patent literature cited in the description


- **LIPSHUTZ, R. J. et al.** *Nature Genetics,* 1999, vol. 21, 20-24
- **BOWTELL, D. D. L.** *Nature genetics suppl.,* 1999, vol. 21, 25-32
- **SABBIONI et al.** *Molecular Diagnosis,* 2003, vol. 7, 201-207
- **ZOU et al.** Selection of methylated genes as candidate markers for colorectal cancer. *AACR Meetings, and Ebert,* 2006
- Genome scanning analysis identifies ALX4 gene methylation as a potential marker for colorectal and other gastrointestinal adenocarcinomas. *Gastroenterology,* vol. 130 (4
- **SURKA, M.C. ; TSANG, C.W. ; TRIMBLE, W.S.** *Mol Biol Cell,* 2002, vol. 13, 3532-45
- **OSAKA, M ; ROWLEY, J.D. ; ZELEZNIK-LE, N.J.** *PNAS,* 1999, vol. 96, 3428-6433
- **BURROWS, J. F., CHANDULOY et al.** *S.E.H. Journal of Pathology,* 2003, vol. 201, 581-588
- **SCOTT, M. ; HYLAND, P.L. et al.** *Oncogene,* 2005, vol. 24, 4688-4700
- J.P. Egan. Signal Detection Theory and ROC Analysis. Academic Press, 1975
- **GONZALGO et al.** *Cancer Research,* 1997, vol. 57, 594-599
- **EADS et al.** *Cancer Res.,* 1999, vol. 59, 2302-2306
- **GONZALGO ; JONES.** *Nucleic Acids Res.,* 1997, vol. 25, 2529-2531
- **HERMAN et al.** *Proc. Natl. Acad. Sci. USA,* 1996, vol. 93, 9821-9826
- **XIONG ; LAIRD.** *Nucleic Acids Res.,* 1997, vol. 25, 2532-2534
- **TOYOTA et al.** *Cancer Res.,* 1999, vol. 59, 2307-12
- **SAMBROOK et al.** Molecular Cloning, A Laboratory Manual. Cold Spring Harbor Press, 1989
- Current Protocols in Molecular Biology. John Wiley and Sons, 1995
- **OLEK A et al.** A modified and improved method for bisulfite based cytosine methylation analysis. *Nucleic Acids Res.,* 1996, vol. 24, 5064-6
- **REIN, T. et al.** *Nucleic Acids Res.,* 1998, vol. 26, 2255
- **ZESCHNIGK M et al.** *Eur J Hum Genet.,* 1997, vol. 5, 94-98
- **OLEK ; WALTER.** *Nat Genet. 1997,* 1997, vol. 17, 275-6
- **GONZALGO ; JONES.** *Nucleic Acids Res.,* 1997, vol. 25, 2529-31
- **XIONG ; LAIRD.** *Nucleic Acids Res.,* 1997, vol. 25, 2532-4
- **GRIGG ; CLARK.** *Bioessays,* 1994, vol. 16, 431-6
- **ZESCHNIGK M et al.** *Hum Mol Genet.,* 1997, vol. 6, 387-95
- **FEIL R et al.** *Nucleic Acids Res.,* 1994, vol. 22, 695
- **MARTIN V et al.** *Gene,* 1995, vol. 157, 261-4
- **FROMMER et al.** *Proc. Natl. Acad. Sci. USA,* 1992, vol. 89, 1827-1831
- *Nucl. Acids Res.,* 1996, vol. 24, 5058-5059
- **BELYAVSKY et al.** *Nucl Acid Res,* 1989, vol. 17, 2919-2932
- **KRUG ; BERGER.** Methods in Enzymology. Academic Press, 1987, vol. 152, 316-325

- **SAMBROOK et al.** Molecular Cloning: A Laboratory Manual. 1989
- Basic and Clinical Immunology. Appleton and Lange, 1991, 217-262
- **MILSTEIN ; KOHLER.** *Nature,* 1975, vol. 256, 495-497
- **GULFRE ; MILSTEIN.** Methods in Enzymology: Immunochemical Techniques. Academic Press, 1981, vol. 73, 1-46
- **KROL et al.** *BioTechniques,* 1988, vol. 6, 958-976
- **ZON.** *Pharm. Res.,* 1988, vol. 5, 539-549
- *Nature Genetics Supplement,* January 1999, vol. 21
- **YU et al.** *BioTechniques,* 1997, vol. 23, 714-720
- **KARAS ; HILLENKAMP.** *Anal Chem.,* 1988, vol. 60, 2299-301
- **GUT ; BECK.** *Current Innovations and Future Trends,* 1995, vol. 1, 147-57
- **GUT ; BECK.** *Nucleic Acids Res.,* 1995, vol. 23, 1367-73
- **HEID et al.** *Genome Res.,* 1996, vol. 6, 986-994
- **SANGER F. et al.** *Proc Natl Acad Sci USA,* 1977, vol. 74, 5463-5467
- **COTTRELL SE ; DISTLER J et al.** *NAR,* 2004, vol. 32 (1), e10